# EUROPEAN PATENT SPECIFICATION

(11) **EP 1 573 000 B1**
(45) Date of publication and mention of the grant of the patent: **07.05.2008**
(21) Application number: 03810884.1
(22) Date of filing: 08.12.2003
(51) Int. Cl.: C12N 9/90, C12N 15/61, G01N 33/573

(54) **CRYSTAL STRUCTURE OF GLUTAMATE RACEMASE (MURI)**
KRISTALLSTRUKTUR DER GLUTAMATRACEMASE (MURI)
STRUCTURE CRISTALLINE DE GLUTAMATE RACEMASE (MURI)

(30) Priority: 20.12.2002 US 435167 P; 20.12.2002 US 435272 P; 20.12.2002 US 435087 P; 20.12.2002 US 435527 P
(43) Date of publication of application: 14.09.2005
(73) Proprietor: AstraZeneca AB, 151 85 Södertälje (SE)
(72) Inventor: ANDERSON, Marie, SE-437 93 Lindome (SE); FISHER, Stewart, Lindsay, Framingham, MA 01701 (US); FOLMER, Rutger, Henk, Adriaan, 437 42 Lindome (SE); KERN, Gunther, Waltham, MA 02453 (US); LUNDQVIST, Rolf, Tomas, SE-413 07 Gothenburg (SE); NEWTON, David, Trevor, Ottawa, Ontario K4A 2S4 (CA); XUE, Yafeng, SE-436 42 Askim (SE)
(86) International application number: PCT/US2003/038977
(87) International publication number: WO 2004/061097

(56) References cited:
- WO-A-02/14261
- HWANG KWANG YEON ET AL: "Structure and mechanism of glutamate racemase from Aquifex pyrophilus" NATURE STRUCTURAL BIOLOGY, vol. 6, no. 5, May 1999 (1999-05), pages 422-426, XP002286544 ISSN: 1072-8368 cited in the application
- HWANG KWANG YEON ET AL: "Crystallization and preliminary X-ray analysis of glutamate racemase from Aquifex pyrophilus, a hyperthermophilic bacterium" ACTA CRYSTALLOGRAPHICA SECTION D BIOLOGICAL CRYSTALLOGRAPHY, vol. 55, no. 4, April 1999 (1999-04), pages 927-928, XP008031528 ISSN: 0907-4449
- DE DIOS ALFONSO ET AL: "4-substituted D-glutamic acid analogues: The first potent inhibitors of glutamate racemase (MurI) enzyme with antibacterial activity" JOURNAL OF MEDICINAL CHEMISTRY, vol. 45, no. 20, 26 September 2002 (2002-09-26), pages 4559-4570, XP002286546 ISSN: 0022-2623
- GLAVAS S ET AL: "The inhibition of glutamate racemase by d-N-hydroxyglutamate" BIOORGANIC & MEDICINAL CHEMISTRY LETTERS, OXFORD, GB, vol. 7, no. 17, 9 September 1997 (1997-09-09), pages 2265-2270, XP004136426 ISSN: 0960-894X
- KIM WOO-CHANG ET AL: "Isolation of peptide ligands that inhibit glutamate racemase activity from a random phage display library" JOURNAL OF BIOMOLECULAR SCREENING, vol. 5, no. 6, December 2000 (2000-12), pages 435-440, XP008032389 ISSN: 1087-0571
- TANNER MARTIN E ET AL: "The synthesis and stability of aziridino-glutamate, an irreversible inhibitor of glutamate racemase" TETRAHEDRON LETTERS, vol. 35, no. 24, 1994, pages 4073-4076, XP002286565 ISSN: 0040-4039
- GLAVAS SUZANA ET AL: "Active site residues of glutamate racemase" BIOCHEMISTRY, vol. 40, no. 21, 29 May 2001 (2001-05-29), pages 6199-6204, XP002286547 ISSN: 0006-2960
- PUCCI M J ET AL: "THE ESCHERICHIA COLI DGA (MURI) PROTEIN SHARES BIOLOGICAL ACTIVITY AND STRUCTURAL DOMAINS WITH THE PEDIOCOCCUS PENTOSACEUS GLUTAMATE RACEMASE" JOURNAL OF BACTERIOLOGY, WASHINGTON, DC, US, vol. 176, no. 2, January 1994 (1994-01), pages 528-530, XP001035226 ISSN: 0021-9193

### SEQUENCE LISTING

<110> ASTRAZENECA AB et al.
<120> CRYSTAL STRUCTURE OF GLUTAMATE RACEMASE (MURI)
<130> ASZD-PWO-007
<140> US03/38977
   <141> 2003-12-08
<150> 60/435,272
   <151> 2002-12-20
<150> 60/435,167
   <151> 2002-12-20
<150> 60/435,087
   <151> 2002-12-20
<150> 60/435,527
   <151> 2002-12-20
<160> 76
<170> PatentIn version 3.1
<210> 1
   <211> 768
   <212> DNA
   <213> H. pylori
<221> CDS
   <222> (1)..(768)
<400> 1
   atg aaa ata ggc gtt ttt gat agc ggt gtg ggg ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg cga ttg ttt gat gaa atc atc tac tat ggc      96
   Lys Ser Leu Leu Lys Ala Arg Leu Phe Asp Glu Ile Ile Tyr Tyr Gly
               20                  25                  30
   gat agc gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cat gag att gaa     192
   Gln Phe Gly Leu Glu Ala Leu Asp Phe Phe Lys Pro His Glu Ile Glu
       50                  55                  60
   tta ttg att gtg gca tgc aac acc gcg agc gct ctg gct tta gaa gag     240
   Leu Leu Ile Val Ala Cys Asn Thr Ala Ser Ala Leu Ala Leu Glu Glu
   65                  70                  75                  80
   atg caa aag tat tct aaa atc cct att gtg ggc gtg att gag cca agc     288
   Met Gln Lys Tyr Ser Lys Ile Pro Ile Val Gly Val Ile Glu Pro Ser
                   85                  90                  95
   att tta gcg atc aag cgg caa gtg gaa gat aaa aac gcc cct att tta     336
   Ile Leu Ala Ile Lys Arg Gln Val Glu Asp Lys Asn Ala Pro Ile Leu
               100                 105                 110
   gtg cta ggg aca aaa gcg acg att caa tcc aac gcc tat gac aac gcc     384
   Val Leu Gly Thr Lys Ala Thr Ile Gln Ser Asn Ala Tyr Asp Asn Ala
           115                 120                 125
   ctg aaa caa caa ggc tat ttg aac att tcg cat tta gct act tct ctt     432
   Leu Lys Gln Gln Gly Tyr Leu Asn Ile Ser His Leu Ala Thr Ser Leu
       130                 135                 140
   ttt gtg cct ttg att gaa gaa agt att tta gag ggc gaa ttg tta gaa     480
   Phe Val Pro Leu Ile Glu Glu Ser Ile Leu Glu Gly Glu Leu Leu Glu
   145                 150                 155                 160
   act tgc atg cat tat tat ttc act ccc tta gag att tta ccc gaa gtg     528
   Thr Cys Met His Tyr Tyr Phe Thr Pro Leu Glu Ile Leu Pro Glu Val
                   165                 170                 175
   atc att tta ggt tgc acg cat ttt ccc tta atc gct caa aaa att gag     576
   Ile Ile Leu Gly Cys Thr His Phe Pro Leu Ile Ala Gln Lys Ile Glu
               180                 185                 190
   ggc tat ttc atg ggg cat ttt gcc ctt cca acg ccc ccc cta ctc atc     624
   Gly Tyr Phe Met Gly His Phe Ala Leu Pro Thr Pro Pro Leu Leu Ile
           195                 200                 205
   cat tcg ggc gat gct att gta gaa tat ttg caa caa aaa tac gcc ctt     672
   His Ser Gly Asp Ala Ile Val Glu Tyr Leu Gln Gln Lys Tyr Ala Leu
       210                 215                 220
   aaa aac aat gca tgc aca ttc cct aaa gtg gaa ttt cat gcg agc ggc     720
   Lys Asn Asn Ala Cys Thr Phe Pro Lys Val Glu Phe His Ala Ser Gly
   225                 230                 235                 240
   gat gtg atc tgg cta gaa aga caa gct aaa gaa tgg ctc aaa ttg taa     768
   Asp Val Ile Trp Leu Glu Arg Gln Ala Lys Glu Trp Leu Lys Leu
                   245                 250                 255
<210> 2
   <211> 255
   <212> PRT
   <213> H. pylori
<400> 2
   Met Lys Ile Gly Val Phe Asp Ser Gly Val Gly Gly Phe Ser Val Leu
   1               5                   10                  15
   Lys Ser Leu Leu Lys Ala Arg Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Glu Ile Glu
       50                  55                  60
   Leu Leu Ile Val Ala Cys Asn Thr Ala Ser Ala Leu Ala Leu Glu Glu
   65                  70                  75                  80
   Met Gln Lys Tyr Ser Lys Ile Pro Ile Val Gly Val Ile Glu Pro Ser
   85 90 95
   Ile Leu Ala Ile Lys Arg Gln Val Glu Asp Lys Asn Ala Pro Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Ile Ser His Leu Ala Thr Ser Leu
   130 135 140
   Phe Val Pro Leu Ile Glu Glu Ser Ile Leu Glu Gly Glu Leu Leu Glu
   145 150 155 160
   Thr Cys Met His Tyr Tyr Phe Thr Pro Leu Glu Ile Leu Pro Glu Val
   165 170 175
   Ile Ile Leu Gly Cys Thr His Phe Pro Leu Ile Ala Gln Lys Ile Glu
   180 185 190
   Gly Tyr Phe Met Gly His Phe Ala Leu Pro Thr Pro Pro Leu Leu Ile
   195 200 205
   His Ser Gly Asp Ala Ile Val Glu Tyr Leu Gln Gln Lys Tyr Ala Leu
   210 215 220
   Lys Asn Asn Ala Cys Thr Phe Pro Lys Val Glu Phe His Ala Ser Gly
   225 230 235 240
   Asp Val Ile Trp Leu Glu Arg Gln Ala Lys Glu Trp Leu Lys Leu
   245 250 255
<210> 3
   <211> 768
   <212> DNA
   <213> H. pylori
<221> CDS
   <222> (1)..(768)
<400> 3
   atg aaa ata ggc gtt ttt gat agc ggt gtg ggg ggg ttt agc gtt tta 48
   Met Lys Ile Gly Val Phe Asp Ser Gly Val Gly Gly Phe Ser Val Leu
   1 5 10 15
   aaa agc ctt tta aaa gcg caa ttg ttt gat gaa atc atc tat tat ggc 96
   Lys Ser Leu Leu Lys Ala Gln Leu Phe Asp Glu Ile Ile Tyr Tyr Gly
   20 25 30
   gat agc gct aga gtg cct tat ggc act aaa gac ccc act acg atc aag 144
   Asp Ser Ala Arg Val Pro Tyr Gly Thr Lys Asp Pro Thr Thr Ile Lys
   35 40 45
   caa ttt ggc tta gag gct ttg gat ttt ttc aaa cca cac cag att gaa 192
   Gln Phe Gly Leu Glu Ala Leu Asp Phe Phe Lys Pro His Gln Ile Glu
       50                  55                  60
   tta ttg att gtg gca tgc aac acc gca agc gct ctg gct tta gaa gag      240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc      288
   Met Gln Lys His Ser Lys Ile Pro Ile Val Gly Val Ile Glu Pro Ser
                   85                  90                  95
   att tta gcg atc aag caa caa gtg aaa gat aaa aac gcc cct att tta      336
   Ile Leu Ala Ile Lys Gln Gln Val Lys Asp Lys Asn Ala Pro Ile Leu
               100                 105                 110
   gtg cta ggg aca aaa gcg acg att caa tcc aac gct tat gac aac gcc      384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt      432
   Leu Lys Gln Gln Gly Tyr Leu Asn Val Ser His Leu Ala Thr Ser Leu
       130                 135                 140
   ttt gtg cct ttg att gaa gaa agt att tta gag ggc gaa ttg tta gag      480
   Phe Val Pro Leu Ile Glu Glu Ser Ile Leu Glu Gly Glu Leu Leu Glu
   145                 150                 155                 160
   act tgc atg cgt tat tat ttc act ccc tta aag att tta cct gaa gtg      528
   Thr Cys Met Arg Tyr Tyr Phe Thr Pro Leu Lys Ile Leu Pro Glu Val
                   165                 170                 175
   att att tta ggt tgc acg cat ttt ccc ttg att gct caa aaa att gag      576
   Ile Ile Leu Gly Cys Thr His Phe Pro Leu Ile Ala Gln Lys Ile Glu
               180                 185                 190
   ggc tat ttc atg gag cat ttt gcc ctt cca acg ccc ccc cta ctc atc      624
   Gly Tyr Phe Met Glu His Phe Ala Leu Pro Thr Pro Pro Leu Leu Ile
           195                 200                 205
   cat tcg ggc gat gct att gta gaa tat ttg cag caa aaa tac gcc ctt      672
   His Ser Gly Asp Ala Ile Val Glu Tyr Leu Gln Gln Lys Tyr Ala Leu
       210                 215                 220
   aaa aac aat gca cac gca ttc cct aaa gtg gaa ttt cat gcg agc ggc      720
   Lys Asn Asn Ala His Ala Phe Pro Lys Val Glu Phe His Ala Ser Gly
   225                 230                 235                 240
   gat gtg atc tgg cta gaa aga caa gct aaa gaa tgg ctc aaa ttg taa      768
   Asp Val Ile Trp Leu Glu Arg Gln Ala Lys Glu Trp Leu Lys Leu
                   245                 250                 255
<210> 4
   <211> 255
   <212> PRT
   <213> H. pylori
<400> 4
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
   35 40 45
   Gln Phe Gly Leu Glu Ala Leu Asp Phe Phe Lys Pro His Gln Ile Glu
   50 55 60
   Leu Leu Ile Val Ala Cys Asn Thr Ala Ser Ala Leu Ala Leu Glu Glu
   65 70 75 80
   Met Gln Lys His Ser Lys Ile Pro Ile Val Gly Val Ile Glu Pro Ser
   85 90 95
   Ile Leu Ala Ile Lys Gln Gln Val Lys Asp Lys Asn Ala Pro Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Val Ser His Leu Ala Thr Ser Leu
   130 135 140
   Phe Val Pro Leu Ile Glu Glu Ser Ile Leu Glu Gly Glu Leu Leu Glu
   145 150 155 160
   Thr Cys Met Arg Tyr Tyr Phe Thr Pro Leu Lys Ile Leu Pro Glu Val
   165 170 175
   Ile Ile Leu Gly Cys Thr His Phe Pro Leu Ile Ala Gln Lys Ile Glu
   180 185 190
   Gly Tyr Phe Met Glu His Phe Ala Leu Pro Thr Pro Pro Leu Leu Ile
   195 200 205
   His Ser Gly Asp Ala Ile Val Glu Tyr Leu Gln Gln Lys Tyr Ala Leu
   210 215 220
   Lys Asn Asn Ala His Ala Phe Pro Lys Val Glu Phe His Ala Ser Gly
   225 230 235 240
   Asp Val Ile Trp Leu Glu Arg Gln Ala Lys Glu Trp Leu Lys Leu
   245 250 255
<210> 5
   <211> 768
   <212> DNA
   <213> H. pylori
<221> CDS
   <222> (1)..(768)
<400> 5
   atg aaa ata ggc gtt ttt gat agc ggt gtg gga ggg ttt agc gtt tta 48
   Met Lys Ile Gly Val Phe Asp Ser Gly Val Gly Gly Phe Ser Val Leu
   1 5 10 15
   aaa agc ctt tta aaa gcg caa tta ttt gat gaa atc atc tat tat ggc 96
   Lys Ser Leu Leu Lys Ala Gln Leu Phe Asp Glu Ile Ile Tyr Tyr Gly
   20 25 30
   gat agc gct aga gtg cct tat ggc act aaa gac ccc act acg atc aag 144
   Asp Ser Ala Arg Val Pro Tyr Gly Thr Lys Asp Pro Thr Thr Ile Lys
   35 40 45
   caa ttt ggc tta gag gct ttg gat ttt ttc aaa ccg cac cag att gaa 192
   Gln Phe Gly Leu Glu Ala Leu Asp Phe Phe Lys Pro His Gln Ile Glu
   50 55 60
   tta ttg att gtg gca tgc aac aca gcg agc gct cta gct tta gaa gag 240
   Leu Leu Ile Val Ala Cys Asn Thr Ala Ser Ala Leu Ala Leu Glu Glu
   65 70 75 80
   atg caa aag cat tcc aaa atc cct att gtg ggc gtg att gag cca agc 288
   Met Gln Lys His Ser Lys Ile Pro Ile Val Gly Val Ile Glu Pro Ser
   85 90 95
   att tta gcg atc aag cga caa gta aaa gat aaa aac gcc cct att tta 336
   Ile Leu Ala Ile Lys Arg Gln Val Lys Asp Lys Asn Ala Pro Ile Leu
   100 105 110
   gtg cta ggg aca aaa gcg acg atc caa tcc aac gct tat gac aat gcc 384
   Val Leu Gly Thr Lys Ala Thr Ile Gln Ser Asn Ala Tyr Asp Asn Ala
   115 120 125
   ctg aaa caa caa ggc tat ttg aat gtt tcg cat tta gcc act tct ctt 432
   Leu Lys Gln Gln Gly Tyr Leu Asn Val Ser His Leu Ala Thr Ser Leu
   130 135 140
   ttt gtg cct ttg att gaa gaa agt att tta gag ggc gaa ttg tta gaa 480
   Phe Val Pro Leu Ile Glu Glu Ser Ile Leu Glu Gly Glu Leu Leu Glu
   145 150 155 160
   act tgc atg cgt tat tat ttc act ccc tta aag att tta ccc gaa gtg 528
   Thr Cys Met Arg Tyr Tyr Phe Thr Pro Leu Lys Ile Leu Pro Glu Val
   165 170 175
   att att tta ggt tgc acg cat ttt ccc tta atc gct caa aaa att gag 576
   Ile Ile Leu Gly Cys Thr His Phe Pro Leu Ile Ala Gln Lys Ile Glu
   180 185 190
   ggc tat ttt atg gag cat ttt gcc ctt tca aca ccc ccc cta ctc atc 624
   Gly Tyr Phe Met Glu His Phe Ala Leu Ser Thr Pro Pro Leu Leu Ile
   195 200 205
   cat tcg ggc gat gct att gta gga tat ttg cag caa aaa tac gcc ctt 672
   His Ser Gly Asp Ala Ile Val Gly Tyr Leu Gln Gln Lys Tyr Ala Leu
   210 215 220
   aaa aaa aat gca cac gca ttc cct aaa gtg gaa ttt cat gcg agc ggc 720
   Lys Lys Asn Ala His Ala Phe Pro Lys Val Glu Phe His Ala Ser Gly
   225 230 235 240
   gat gtg atc tgg cta gaa aaa caa gct aaa gaa tgg ctc aaa ttg taa 768
   Asp Val Ile Trp Leu Glu Lys Gln Ala Lys Glu Trp Leu Lys Leu
   245 250 255
<210> 6
   <211> 255
   <212> PRT
   <213> H. pylori
<400> 6
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Glu
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Arg Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Lys Ile Leu Pro Glu Val
                   165                 170                 175
   Ile Ile Leu Gly Cys Thr His Phe Pro Leu Ile Ala Gln Lys Ile Glu
               180                 185                 190
   Gly Tyr Phe Met Glu His Phe Ala Leu Ser Thr Pro Pro Leu Leu Ile
           195                 200                 205
   His Ser Gly Asp Ala Ile Val Gly Tyr Leu Gln Gln Lys Tyr Ala Leu
       210                 215                 220
   Lys Lys Asn Ala His Ala Phe Pro Lys Val Glu Phe His Ala Ser Gly
   225                 230                 235                 240
   Asp Val Ile Trp Leu Glu Lys Gln Ala Lys Glu Trp Leu Lys Leu
                   245                 250                 255
<210> 7
   <211> 749
   <212> DNA
   <213> H. pylori
<221> CDS
   <222> (1)..(747)
<400> 7
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg caa ttg ttt gat gaa atc atc tat tat ggc      96
   Lys Ser Leu Leu Lys Ala Gln Leu Phe Asp Glu Ile Ile Tyr Tyr Gly
               20                  25                  30
   gat agc gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att aaa     192
   Gln Phe Gly Leu Glu Ala Leu Asp Phe Phe Lys Pro His Gln Ile Lys
       50                  55                  60
   tta ttg att gtg gca tgc aac aca gcg agc gct cta gct tta gaa gag     240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc     288
   Met Gln Lys His Ser Lys Ile Pro Ile Val Gly Val Ile Glu Pro Ser
                   85                  90                  95
   att tta gcg atc aag caa caa gta aaa gat aaa aac gcc cct att tta     336
   Ile Leu Ala Ile Lys Gln Gln Val Lys Asp Lys Asn Ala Pro Ile Leu
               100                 105                 110
   gtg cta ggg aca aaa gcg acg atc caa tcc aac gct tat gac aac gcc     384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt     432
   Leu Lys Gln Gln Gly Tyr Leu Asn Val Ser His Leu Ala Thr Ser Leu
       130                 135                 140
   ttt gtg cct ttg att gaa gaa agt att tta ggg ggc gaa ttg tta gaa     480
   Phe Val Pro Leu Ile Glu Glu Ser Ile Leu Gly Gly Glu Leu Leu Glu
   145                 150                 155                 160
   act tgc atg cgt tat tat ttc act ccc tta aag att tta cct gaa gtg     528
   Thr Cys Met Arg Tyr Tyr Phe Thr Pro Leu Lys Ile Leu Pro Glu Val
                   165                 170                 175
   att att tta ggt tgc acg cat ttt ccc ttg atc gct caa aaa att gag     576
   Ile Ile Leu Gly Cys Thr His Phe Pro Leu Ile Ala Gln Lys Ile Glu
               180                 185                 190
   ggc tat ttt atg gag cat ttt gcc ctt tca acg ccc ccc cta ctc atc      624
   Gly Tyr Phe Met Glu His Phe Ala Leu Ser Thr Pro Pro Leu Leu Ile
           195                 200                 205
   cat tcg ggc gat gct att gtg gaa tat ttg cag caa aaa tac gcc ctt      672
   His Ser Gly Asp Ala Ile Val Glu Tyr Leu Gln Gln Lys Tyr Ala Leu
       210                 215                 220
   aag aaa aat gca cac gca ttc cct aaa gtg gaa ttt cat gcg agc ggc      720
   Lys Lys Asn Ala His Ala Phe Pro Lys Val Glu Phe His Ala Ser Gly
   225                 230                 235                 240
   gat gtg atc tgg cta gaa aaa cag gct aa                               749
   Asp Val Ile Trp Leu Glu Lys Gln Ala
                   245
<210> 8
   <211> 249
   <212> PRT
   <213> H. pylori
<400> 8
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Ser Ile Leu Gly Gly Glu Leu Leu Glu
   145                 150                 155                 160
   Thr Cys Met Arg Tyr Tyr Phe Thr Pro Leu Lys Ile Leu Pro Glu Val
                   165                 170                 175
   Ile Ile Leu Gly Cys Thr His Phe Pro Leu Ile Ala Gln Lys Ile Glu
               180                 185                 190
   Gly Tyr Phe Met Glu His Phe Ala Leu Ser Thr Pro Pro Leu Leu Ile
           195                 200                 205
   His Ser Gly Asp Ala Ile Val Glu Tyr Leu Gln Gln Lys Tyr Ala Leu
       210                 215                 220
   Lys Lys Asn Ala His Ala Phe Pro Lys Val Glu Phe His Ala Ser Gly
   225                 230                 235                 240
   Asp Val Ile Trp Leu Glu Lys Gln Ala
                   245
<210> 9
   <211> 768
   <212> DNA
   <213> H. pylori
<221> CDS
   <222> (1)..(768)
<400> 9
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg caa cta ttt gat gaa atc atc tat tat ggc      96
   Lys Ser Leu Leu Lys Ala Gln Leu Phe Asp Glu Ile Ile Tyr Tyr Gly
               20                  25                  30
   gat agt gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att gga     192
   Gln Phe Gly Leu Glu Ala Leu Asp Phe Phe Lys Pro His Gln Ile Gly
       50                  55                  60
   tta ttg att gtg gca tgc aac aca gcg agc gct cta gct tta gaa gag     240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gaa cca agc     288
   Met Gln Lys His Ser Lys Ile Pro Ile Val Gly Val Ile Glu Pro Ser
                   85                  90                  95
   att tta gcg atc aag caa caa gta aaa gat aaa aac gcc tct att ttg     336
   Ile Leu Ala Ile Lys Gln Gln Val Lys Asp Lys Asn Ala Ser Ile Leu
               100                 105                 110
   gtg cta ggg aca aaa gcg acg atc caa tcc aac gct tat gac aac gcc     384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt        432
   Leu Lys Gln Gln Gly Tyr Leu Asn Val Ser His Leu Ala Thr Ser Leu
       130                 135                 140
   ttt gtg cct ttg att gaa gaa agt att tta gag ggc gaa ttg cta gaa        480
   Phe Val Pro Leu Ile Glu Glu Ser Ile Leu Glu Gly Glu Leu Leu Glu
   145                 150                 155                 160
   act tgc atg cgt tat tat ttc act ccg tta gag atc ttg cct gaa gtg        528
   Thr Cys Met Arg Tyr Tyr Phe Thr Pro Leu Glu Ile Leu Pro Glu Val
                   165                 170                 175
   gtt att tta ggt tgc acg cat ttt ccc tta atc gct caa aaa att gag        576
   Val Ile Leu Gly Cys Thr His Phe Pro Leu Ile Ala Gln Lys Ile Glu
               180                 185                 190
   ggc tat ttt atg gag cat ttt gcc ctt tca acg ccc ccc cta ctc atc        624
   Gly Tyr Phe Met Glu His Phe Ala Leu Ser Thr Pro Pro Leu Leu Ile
           195                 200                 205
   cat tcg ggc gat gct att gtg gaa tat ttg cag caa aaa tac gcc ctt        672
   His Ser Gly Asp Ala Ile Val Glu Tyr Leu Gln Gln Lys Tyr Ala Leu
       210                 215                 220
   aaa aaa aat gca cac gca ttc cct aaa gtg gaa ttt cat gcg agt ggc        720
   Lys Lys Asn Ala His Ala Phe Pro Lys Val Glu Phe His Ala Ser Gly
   225                 230                 235                 240
   gat gtg atc tgg cta gaa aaa cag gct aaa gaa tgg ctc aaa ttg taa        768
   Asp Val Ile Trp Leu Glu Lys Gln Ala Lys Glu Trp Leu Lys Leu
                   245                 250                 255
<210> 10
   <211> 255
   <212> PRT
   <213> H. pylori
<400> 10
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Gly
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Ser Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Val Ser His Leu Ala Thr Ser Leu
   130 135 140
   Phe Val Pro Leu Ile Glu Glu Ser Ile Leu Glu Gly Glu Leu Leu Glu
   145 150 155 160
   Thr Cys Met Arg Tyr Tyr Phe Thr Pro Leu Glu Ile Leu Pro Glu Val
   165 170 175
   Val Ile Leu Gly Cys Thr His Phe Pro Leu Ile Ala Gln Lys Ile Glu
   180 185 190
   Gly Tyr Phe Met Glu His Phe Ala Leu Ser Thr Pro Pro Leu Leu Ile
   195 200 205
   His Ser Gly Asp Ala Ile Val Glu Tyr Leu Gln Gln Lys Tyr Ala Leu
   210 215 220
   Lys Lys Asn Ala His Ala Phe Pro Lys Val Glu Phe His Ala Ser Gly
   225 230 235 240
   Asp Val Ile Trp Leu Glu Lys Gln Ala Lys Glu Trp Leu Lys Leu
   245 250 255
<210> 11
   <211> 749
   <212> DNA
   <213> H. pylori
<221> CDS
   <222> (1)..(747)
<400> 11
   atg aaa ata ggc gtt ttt gat agc ggt gtg gga ggg ttt agc gtt tta 48
   Met Lys Ile Gly Val Phe Asp Ser Gly Val Gly Gly Phe Ser Val Leu
   1 5 10 15
   aaa agc ctt tta aaa gcg caa att ttt gat gaa atc atc tat tat ggc 96
   Lys Ser Leu Leu Lys Ala Gln Ile Phe Asp Glu Ile Ile Tyr Tyr Gly
   20 25 30
   gat agc gct aga gtg cct tat ggc act aaa gac ccc acc acg atc aag 144
   Asp Ser Ala Arg Val Pro Tyr Gly Thr Lys Asp Pro Thr Thr Ile Lys
   35 40 45
   caa ttt ggc tta gag gct ttg gat ttt ttc aaa ccg cac cag att aaa 192
   Gln Phe Gly Leu Glu Ala Leu Asp Phe Phe Lys Pro His Gln Ile Lys
   50 55 60
   tta ttg att gtg gca tgc aac aca gcg agc gct cta gct tta gaa gag 240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc      288
   Met Gln Lys His Ser Lys Ile Pro Ile Val Gly Val Ile Glu Pro Ser
                   85                  90                  95
   att tta gcg atc aag caa caa gta aaa gat aaa aac gcc cct att tta      336
   Ile Leu Ala Ile Lys Gln Gln Val Lys Asp Lys Asn Ala Pro Ile Leu
               100                 105                 110
   gtg cta ggg aca aaa gcg acg att caa tct aac gct tat gac aac gcc      384
   Val Leu Gly Thr Lys Ala Thr Ile Gln Ser Asn Ala Tyr Asp Asn Ala
           115                 120                 125
   cta aaa caa caa ggc tat ttg aac att tcg cat tta gcc act tct ctt      432
   Leu Lys Gln Gln Gly Tyr Leu Asn Ile Ser His Leu Ala Thr Ser Leu
       130                 135                 140
   ttt gtg cct ttg att gaa gaa agt att tta gag ggc gaa ttg tta gag      480
   Phe Val Pro Leu Ile Glu Glu Ser Ile Leu Glu Gly Glu Leu Leu Glu
   145                 150                 155                 160
   act tgc atg cgt tat tat ttc act ccc tta aag att tta cct gaa gtg      528
   Thr Cys Met Arg Tyr Tyr Phe Thr Pro Leu Lys Ile Leu Pro Glu Val
                   165                 170                 175
   atc att tta ggt tgc acg cat ttt ccc ttg atc gct caa aaa att gag      576
   Ile Ile Leu Gly Cys Thr His Phe Pro Leu Ile Ala Gln Lys Ile Glu
               180                 185                 190
   ggc tat ttt atg gag cat ttt gcc ctt cca acc ccc ccc cta ctc atc      624
   Gly Tyr Phe Met Glu His Phe Ala Leu Pro Thr Pro Pro Leu Leu Ile
           195                 200                 205
   cat tcg ggc gat gct att gta gaa tat ttg cag caa aaa tac acc ctt      672
   His Ser Gly Asp Ala Ile Val Glu Tyr Leu Gln Gln Lys Tyr Thr Leu
       210                 215                 220
   aag aaa aat gca cac gca ttc cct aaa gtg gaa ttt cat gcg agt ggc      720
   Lys Lys Asn Ala His Ala Phe Pro Lys Val Glu Phe His Ala Ser Gly
   225                 230                 235                 240
   gat gtg gtt tgg cta gaa aaa cag gct aa                               749
   Asp Val Val Trp Leu Glu Lys Gln Ala
                   245
<210> 12
   <211> 249
   <212> PRT
   <213> H. pylori
<400> 12
   Met Lys Ile Gly Val Phe Asp Ser Gly Val Gly Gly Phe Ser Val Leu
   1               5                   10                  15
   Lys Ser Leu Leu Lys Ala Gln Ile Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
   50 55 60
   Leu Leu Ile Val Ala Cys Asn Thr Ala Ser Ala Leu Ala Leu Glu Glu
   65 70 75 80
   Met Gln Lys His Ser Lys Ile Pro Ile Val Gly Val Ile Glu Pro Ser
   85 90 95
   Ile Leu Ala Ile Lys Gln Gln Val Lys Asp Lys Asn Ala Pro Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Ile Ser His Leu Ala Thr Ser Leu
   130 135 140
   Phe Val Pro Leu Ile Glu Glu Ser Ile Leu Glu Gly Glu Leu Leu Glu
   145 150 155 160
   Thr Cys Met Arg Tyr Tyr Phe Thr Pro Leu Lys Ile Leu Pro Glu Val
   165 170 175
   Ile Ile Leu Gly Cys Thr His Phe Pro Leu Ile Ala Gln Lys Ile Glu
   180 185 190
   Gly Tyr Phe Met Glu His Phe Ala Leu Pro Thr Pro Pro Leu Leu Ile
   195 200 205
   His Ser Gly Asp Ala Ile Val Glu Tyr Leu Gln Gln Lys Tyr Thr Leu
   210 215 220
   Lys Lys Asn Ala His Ala Phe Pro Lys Val Glu Phe His Ala Ser Gly
   225 230 235 240
   Asp Val Val Trp Leu Glu Lys Gln Ala
   245
<210> 13
   <211> 768
   <212> DNA
   <213> H. pylori
<221> CDS
   <222> (1)..(768)
<400> 13
   atg aaa ata ggc gtt ttt gat agc ggt gtg gga ggg ttt agc gtt tta 48
   Met Lys Ile Gly Val Phe Asp Ser Gly Val Gly Gly Phe Ser Val Leu
   1               5                   10                  15
   aaa agc ctt tta aaa gcg caa att ttt gat gaa atc atc tat tat ggc      96
   Lys Ser Leu Leu Lys Ala Gln Ile Phe Asp Glu Ile Ile Tyr Tyr Gly
               20                  25                  30
   gat agt gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att gga     192
   Gln Phe Gly Leu Glu Ala Leu Asp Phe Phe Lys Pro His Gln Ile Gly
       50                  55                  60
   tta ttg att gtg gca tgc aac aca gcg agc gct cta gct tta gaa gag     240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc     288
   Met Gln Lys His Ser Lys Ile Pro Ile Val Gly Val Ile Glu Pro Ser
                   85                  90                  95
   att tta gcg atc aaa caa caa gtg aaa gat aaa aac gct cct att tta     336
   Ile Leu Ala Ile Lys Gln Gln Val Lys Asp Lys Asn Ala Pro Ile Leu
               100                 105                 110
   gtg cta ggg aca aaa gcg acg att caa tct aac gct tac gat aac gcc     384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt     432
   Leu Lys Gln Gln Gly Tyr Leu Asn Val Ser His Leu Ala Thr Ser Leu
       130                 135                 140
   ttt gtg cct ttg att gaa gaa aat att tta gag ggc gaa ttg cta gaa     480
   Phe Val Pro Leu Ile Glu Glu Asn Ile Leu Glu Gly Glu Leu Leu Glu
   145                 150                 155                 160
   act tgc atg cgt tat tat ttc act ccc tta aag att tta cct gaa gtg     528
   Thr Cys Met Arg Tyr Tyr Phe Thr Pro Leu Lys Ile Leu Pro Glu Val
                   165                 170                 175
   atc att tta ggt tgc acg cat ttt ccc ttg atc gct caa aaa att gag     576
   Ile Ile Leu Gly Cys Thr His Phe Pro Leu Ile Ala Gln Lys Ile Glu
               180                 185                 190
   ggc tat ttt atg gag cat ttt gcc ctt tta acg ccc ccc cta ctc atc     624
   Gly Tyr Phe Met Glu His Phe Ala Leu Leu Thr Pro Pro Leu Leu Ile
           195                 200                 205
   cat tct ggc gat gct att gta gaa tat ttg caa caa aaa tac gcc ctt     672
   His Ser Gly Asp Ala Ile Val Glu Tyr Leu Gln Gln Lys Tyr Ala Leu
       210                 215                 220
   aag aaa aat gca cac tca ttc cct aaa gtg gaa ttt cat gcg agc ggc     720
   Lys Lys Asn Ala His Ser Phe Pro Lys Val Glu Phe His Ala Ser Gly
   225                 230                 235                 240
   gat gtg atc tgg cta gaa aaa cag gct aaa gaa tgg ctc aaa ttg taa     768
   Asp Val Ile Trp Leu Glu Lys Gln Ala Lys Glu Trp Leu Lys Leu
                   245                 250                 255
<210> 14
   <211> 255
   <212> PRT
   <213> H. pylori
<400> 14
   Met Lys Ile Gly Val Phe Asp Ser Gly Val Gly Gly Phe Ser Val Leu
   1               5                   10                  15
   Lys Ser Leu Leu Lys Ala Gln Ile Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Gly
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Asn Ile Leu Glu Gly Glu Leu Leu Glu
   145                 150                 155                 160
   Thr Cys Met Arg Tyr Tyr Phe Thr Pro Leu Lys Ile Leu Pro Glu Val
                   165                 170                 175
   Ile Ile Leu Gly Cys Thr His Phe Pro Leu Ile Ala Gln Lys Ile Glu
               180                 185                 190
   Gly Tyr Phe Met Glu His Phe Ala Leu Leu Thr Pro Pro Leu Leu Ile
           195                 200                 205
   His Ser Gly Asp Ala Ile Val Glu Tyr Leu Gln Gln Lys Tyr Ala Leu
       210                 215                 220
   Lys Lys Asn Ala His Ser Phe Pro Lys Val Glu Phe His Ala Ser Gly
   225                 230                 235                 240
   Asp Val Ile Trp Leu Glu Lys Gln Ala Lys Glu Trp Leu Lys Leu
                   245                 250                 255
<210> 15
   <211> 768
   <212> DNA
   <213> H. pylori
<221> CDS
   <222> (1)..(768)
<400> 15
   atg aaa ata ggc gtt ttt gat agc ggt gtg gga ggg ttt agc gtt tta       48
   Met Lys Ile Gly Val Phe Asp Ser Gly Val Gly Gly Phe Ser Val Leu
   1               5                   10                  15
   aaa agc ctt tta aaa gcg caa att ttt gat gaa atc atc tat tat ggc       96
   Lys Ser Leu Leu Lys Ala Gln Ile Phe Asp Glu Ile Ile Tyr Tyr Gly
               20                  25                  30
   gat agc gct aga gtg cct tat ggc act aaa gac ccc acc acg atc aag      144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att gaa      192
   Gln Phe Gly Leu Glu Ala Leu Asp Phe Phe Lys Pro His Gln Ile Glu
       50                  55                  60
   tta ttg att gtg gca tgc aac aca gcg agc gct cta gct tta gaa gag      240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gaa cca agc      288
   Met Gln Lys His Ser Lys Ile Pro Ile Val Gly Val Ile Glu Pro Ser
                   85                  90                  95
   att tta gcg atc aaa caa caa gtg aaa gat aaa aac gct cct att tta      336
   Ile Leu Ala Ile Lys Gln Gln Val Lys Asp Lys Asn Ala Pro Ile Leu
               100                 105                 110
   gtg cta ggg aca aaa gcg acg att caa tct aac gct tac gac aac gcc      384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt      432
   Leu Lys Gln Gln Gly Tyr Leu Asn Val Ser His Leu Ala Thr Ser Leu
       130                 135                 140
   ttt gtg cct ttg att gaa gaa agt att tta gag ggc gaa ttg cta gaa      480
   Phe Val Pro Leu Ile Glu Glu Ser Ile Leu Glu Gly Glu Leu Leu Glu
   145                 150                 155                 160
   act tgc atg cgt tat tat ttc act ccc tta aag att tta ccc aaa gta      528
   Thr Cys Met Arg Tyr Tyr Phe Thr Pro Leu Lys Ile Leu Pro Lys Val
                   165                 170                 175
   atc att tta ggt tgc acg cat ttt ccc ttg atc gct cac caa att aag      576
   Ile Ile Leu Gly Cys Thr His Phe Pro Leu Ile Ala His Gln Ile Lys
               180                 185                 190
   ggc tat ttt atg ggg cat ttt gcc ctt tca acg ccc ccc cta ctc atc      624
   Gly Tyr Phe Met Gly His Phe Ala Leu Ser Thr Pro Pro Leu Leu Ile
           195                 200                 205
   cat tcg ggc gat gct att gtg gga tat ttg caa caa aaa tac gcc ctt      672
   His Ser Gly Asp Ala Ile Val Gly Tyr Leu Gln Gln Lys Tyr Ala Leu
       210                 215                 220
   aag aaa aat gca cac gca ttc cct aaa gtg gaa ttt cat gcg agc ggc      720
   Lys Lys Asn Ala His Ala Phe Pro Lys Val Glu Phe His Ala Ser Gly
   225                 230                 235                 240
   gat gtg atc tgg cta gaa aaa cag gct aaa gaa tgg ctc aaa ttg taa      768
   Asp Val Ile Trp Leu Glu Lys Gln Ala Lys Glu Trp Leu Lys Leu
                   245                 250                 255
<210> 16
   <211> 255
   <212> PRT
   <213> H. pylori
<400> 16
   Met Lys Ile Gly Val Phe Asp Ser Gly Val Gly Gly Phe Ser Val Leu
   1               5                   10                  15
   Lys Ser Leu Leu Lys Ala Gln Ile Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Glu
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Lys Ile Leu Pro Lys Val
                   165                 170                 175
   Ile Ile Leu Gly Cys Thr His Phe Pro Leu Ile Ala His Gln Ile Lys
   180 185 190
   Gly Tyr Phe Met Gly His Phe Ala Leu Ser Thr Pro Pro Leu Leu Ile
   195 200 205
   His Ser Gly Asp Ala Ile Val Gly Tyr Leu Gln Gln Lys Tyr Ala Leu
   210 215 220
   Lys Lys Asn Ala His Ala Phe Pro Lys Val Glu Phe His Ala Ser Gly
   225 230 235 240
   Asp Val Ile Trp Leu Glu Lys Gln Ala Lys Glu Trp Leu Lys Leu
   245 250 255
<210> 17
   <211> 768
   <212> DNA
   <213> H. pylori
<221> CDS
   <222> (1)..(768)
<400> 17
   atg aaa ata ggc gtt ttt gat agc ggt gtg gga ggg ttt agc gtt tta 48
   Met Lys Ile Gly Val Phe Asp Ser Gly Val Gly Gly Phe Ser Val Leu
   1 5 10 15
   aaa agc ctt tta aaa gcg caa tta ttt gat gaa atc atc tat tat ggc 96
   Lys Ser Leu Leu Lys Ala Gln Leu Phe Asp Glu Ile Ile Tyr Tyr Gly
   20 25 30
   gat agc gct aga gtg cct tat ggc act aaa gac ccc acc acg atc aag 144
   Asp Ser Ala Arg Val Pro Tyr Gly Thr Lys Asp Pro Thr Thr Ile Lys
   35 40 45
   caa ttt ggc tta gag gct ttg gat ttt ttc aaa ccg cac cag att aaa 192
   Gln Phe Gly Leu Glu Ala Leu Asp Phe Phe Lys Pro His Gln Ile Lys
   50 55 60
   tta ttg att gtg gca tgc aac aca gcg agt gct ctg gct tta gaa gag 240
   Leu Leu Ile Val Ala Cys Asn Thr Ala Ser Ala Leu Ala Leu Glu Glu
   65 70 75 80
   atg caa aag cat tcc aaa atc cct att gtg ggc gtg att gag cca agc 288
   Met Gln Lys His Ser Lys Ile Pro Ile Val Gly Val Ile Glu Pro Ser
   85 90 95
   att tta gcg atc aaa caa cag gta aaa gat aaa aac gcc ccc att tta 336
   Ile Leu Ala Ile Lys Gln Gln Val Lys Asp Lys Asn Ala Pro Ile Leu
   100 105 110
   gtg cta ggc aca aaa gcg acg att caa tct aac gct tac gat aac gct 384
   Val Leu Gly Thr Lys Ala Thr Ile Gln Ser Asn Ala Tyr Asp Asn Ala
   115 120 125
   ctg aaa cga caa ggc tat ttg aac gtt tcg cat tta gcc act tcc ctt 432
   Leu Lys Arg Gln Gly Tyr Leu Asn Val Ser His Leu Ala Thr Ser Leu
   130 135 140
   ttt gtg cct ttg att gaa gaa agt att tta gag ggc gaa ttg tta gaa      480
   Phe Val Pro Leu Ile Glu Glu Ser Ile Leu Glu Gly Glu Leu Leu Glu
   145                 150                 155                 160
   act tgc atg cgt tat tat ttc act ccc tta aag att tta cct gaa gtg      528
   Thr Cys Met Arg Tyr Tyr Phe Thr Pro Leu Lys Ile Leu Pro Glu Val
                   165                 170                 175
   atc att tta ggt tgt acg cat ttt ccc ttg atc gct caa aaa att gag      576
   Ile Ile Leu Gly Cys Thr His Phe Pro Leu Ile Ala Gln Lys Ile Glu
               180                 185                 190
   ggc tat ttt atg gaa cat ttt gcc ttt cca acg ccc ccc cta ctc atc      624
   Gly Tyr Phe Met Glu His Phe Ala Phe Pro Thr Pro Pro Leu Leu Ile
           195                 200                 205
   cat tcg ggc gat gct att gtg gaa tat ttg cag caa aaa tac gcc ctt      672
   His Ser Gly Asp Ala Ile Val Glu Tyr Leu Gln Gln Lys Tyr Ala Leu
       210                 215                 220
   aag aaa aat gca cac gca tta cct aaa gtg gaa ttt cat gcg agc ggc      720
   Lys Lys Asn Ala His Ala Leu Pro Lys Val Glu Phe His Ala Ser Gly
   225                 230                 235                 240
   gat gtg atc tgg cta gaa aaa caa gct aaa gaa tgg ctc aaa ttg taa      768
   Asp Val Ile Trp Leu Glu Lys Gln Ala Lys Glu Trp Leu Lys Leu
                   245                 250                 255
<210> 18
   <211> 255
   <212> PRT
   <213> H. pylori
<400> 18
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Arg Gln Gly Tyr Leu Asn Val Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Lys Ile Leu Pro Glu Val
                   165                 170                 175
   Ile Ile Leu Gly Cys Thr His Phe Pro Leu Ile Ala Gln Lys Ile Glu
               180                 185                 190
   Gly Tyr Phe Met Glu His Phe Ala Phe Pro Thr Pro Pro Leu Leu Ile
           195                 200                 205
   His Ser Gly Asp Ala Ile Val Glu Tyr Leu Gln Gln Lys Tyr Ala Leu
       210                 215                 220
   Lys Lys Asn Ala His Ala Leu Pro Lys Val Glu Phe His Ala Ser Gly
   225                 230                 235                 240
   Asp Val Ile Trp Leu Glu Lys Gln Ala Lys Glu Trp Leu Lys Leu
                   245                 250                 255
<210> 19
   <211> 768
   <212> DNA
   <213> H. pylori
<221> CDS
   <222> (1)..(768)
<400> 19
   atg aaa ata ggc gtt ttt gat agc ggt gtg gga ggg ttt agc gtt tta       48
   Met Lys Ile Gly Val Phe Asp Ser Gly Val Gly Gly Phe Ser Val Leu
   1               5                   10                  15
   aaa agc ctt tta aaa gcg caa tta ttt gat gaa atc atc tat tat ggc       96
   Lys Ser Leu Leu Lys Ala Gln Leu Phe Asp Glu Ile Ile Tyr Tyr Gly
               20                  25                  30
   gat agc gct aga gtg cct tat ggc act aaa gac ccc acc acg atc aag      144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att aaa      192
   Gln Phe Gly Leu Glu Ala Leu Asp Phe Phe Lys Pro His Gln Ile Lys
       50                  55                  60
   tta ttg att gtg gca tgc aac aca gcg agc gct cta gct tta gaa gag      240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc      288
   Met Gln Lys His Ser Lys Ile Pro Ile Val Gly Val Ile Glu Pro Ser
                   85                  90                  95
   att tta gcg atc aaa caa caa gta aag gat aaa aac gcc ccc att tta     336
   Ile Leu Ala Ile Lys Gln Gln Val Lys Asp Lys Asn Ala Pro Ile Leu
               100                 105                 110
   gtg cta ggg aca aaa gcg acg att caa tct aac gct tac gat aac gct     384
   Val Leu Gly Thr Lys Ala Thr Ile Gln Ser Asn Ala Tyr Asp Asn Ala
           115                 120                 125
   ctg aaa caa caa ggc tat ttg aac gtt tcg cat tta gcc act tct ctt     432
   Leu Lys Gln Gln Gly Tyr Leu Asn Val Ser His Leu Ala Thr Ser Leu
       130                 135                 140
   ttt gtg cct ttg att gaa gaa aat att tta gag ggc gaa ttg tta gaa     480
   Phe Val Pro Leu Ile Glu Glu Asn Ile Leu Glu Gly Glu Leu Leu Glu
   145                 150                 155                 160
   act tgc atg cgt tat tat ttc act ccc tta gag att tta cct gaa gtg     528
   Thr Cys Met Arg Tyr Tyr Phe Thr Pro Leu Glu Ile Leu Pro Glu Val
                   165                 170                 175
   atc att tta ggt tgc acg cat ttt ccc tta atc gct caa aaa att gag     576
   Ile Ile Leu Gly Cys Thr His Phe Pro Leu Ile Ala Gln Lys Ile Glu
               180                 185                 190
   ggc tat ttc atg ggg cat ttt gcc ctt cca acg ccc ccc ata ctc atc     624
   Gly Tyr Phe Met Gly His Phe Ala Leu Pro Thr Pro Pro Ile Leu Ile
           195                 200                 205
   cat tct ggc gac gct att gta gaa tat ttg caa caa aaa tac gcc ctt     672
   His Ser Gly Asp Ala Ile Val Glu Tyr Leu Gln Gln Lys Tyr Ala Leu
       210                 215                 220
   aag aaa aat gca cac gca ttc cct aaa gtg gaa ttt cat gcg agc ggc     720
   Lys Lys Asn Ala His Ala Phe Pro Lys Val Glu Phe His Ala Ser Gly
   225                 230                 235                 240
   gat atg atc tgg cta gaa aaa caa gct aaa gaa tgg ctc aaa ttg taa     768
   Asp Met Ile Trp Leu Glu Lys Gln Ala Lys Glu Trp Leu Lys Leu
                   245                 250                 255
<210> 20
   <211> 255
   <212> PRT
   <213> H. pylori
<400> 20
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
       50                  55                  60
   Leu Leu Ile Val Ala Cys Asn Thr Ala Ser Ala Leu Ala Leu Glu Glu
   65 70 75 80
   Met Gln Lys His Ser Lys Ile Pro Ile Val Gly Val Ile Glu Pro Ser
   85 90 95
   Ile Leu Ala Ile Lys Gln Gln Val Lys Asp Lys Asn Ala Pro Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Val Ser His Leu Ala Thr Ser Leu
   130 135 140
   Phe Val Pro Leu Ile Glu Glu Asn Ile Leu Glu Gly Glu Leu Leu Glu
   145 150 155 160
   Thr Cys Met Arg Tyr Tyr Phe Thr Pro Leu Glu Ile Leu Pro Glu Val
   165 170 175
   Ile Ile Leu Gly Cys Thr His Phe Pro Leu Ile Ala Gln Lys Ile Glu
   180 185 190
   Gly Tyr Phe Met Gly His Phe Ala Leu Pro Thr Pro Pro Ile Leu Ile
   195 200 205
   His Ser Gly Asp Ala Ile Val Glu Tyr Leu Gln Gln Lys Tyr Ala Leu
   210 215 220
   Lys Lys Asn Ala His Ala Phe Pro Lys Val Glu Phe His Ala Ser Gly
   225 230 235 240
   Asp Met Ile Trp Leu Glu Lys Gln Ala Lys Glu Trp Leu Lys Leu
   245 250 255
<210> 21
   <211> 768
   <212> DNA
   <213> H. pylori
<221> CDS
   <222> (1)..(768)
<400> 21
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg caa tta ttt gat gaa atc atc tat tat ggc      96
   Lys Ser Leu Leu Lys Ala Gln Leu Phe Asp Glu Ile Ile Tyr Tyr Gly
               20                  25                  30
   gat agc gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att aaa     192
   Gln Phe Gly Leu Glu Ala Leu Asp Phe Phe Lys Pro His Gln Ile Lys
       50                  55                  60
   tta ttg att gta gca tgc aac aca gcg agc gct cta gct tta gaa gag     240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gag cca agc     288
   Met Gln Lys His Ser Lys Ile Pro Ile Val Gly Val Ile Glu Pro Ser
                   85                  90                  95
   att tta gcg atc aaa caa caa gta aaa gat aaa aac gcc cct att tta     336
   Ile Leu Ala Ile Lys Gln Gln Val Lys Asp Lys Asn Ala Pro Ile Leu
               100                 105                 110
   gtg cta ggg aca aaa gcg acg att caa tct aac gct tat gac aac gcc     384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt     432
   Leu Lys Gln Gln Gly Tyr Leu Asn Val Ser His Leu Ala Thr Ser Leu
       130                 135                 140
   ttt gtg cct ttg att gaa gaa agt att tta gag ggc gaa ttg tta gaa     480
   Phe Val Pro Leu Ile Glu Glu Ser Ile Leu Glu Gly Glu Leu Leu Glu
   145                 150                 155                 160
   act tgc atg cgt tat tat ttc act ccc tta aag att tta cct gaa gtg     528
   Thr Cys Met Arg Tyr Tyr Phe Thr Pro Leu Lys Ile Leu Pro Glu Val
                   165                 170                 175
   att att tta ggt tgc acg cat ttt ccc ttg atc gct caa aaa att gag     576
   Ile Ile Leu Gly Cys Thr His Phe Pro Leu Ile Ala Gln Lys Ile Glu
               180                 185                 190
   agc tat ttt atg ggg cat ttt gcc ctt cca acg ccc ccc cta ctc atc     624
   Ser Tyr Phe Met Gly His Phe Ala Leu Pro Thr Pro Pro Leu Leu Ile
           195                 200                 205
   cat tct ggc gat gct att gtg gaa tat ttg cag caa aaa tac gcc ctt     672
   His Ser Gly Asp Ala Ile Val Glu Tyr Leu Gln Gln Lys Tyr Ala Leu
       210                 215                 220
   aag aaa aac gca cac gca ttc cct aaa gtg gaa ttt cat gcg agc ggc     720
   Lys Lys Asn Ala His Ala Phe Pro Lys Val Glu Phe His Ala Ser Gly
   225                 230                 235                 240
   gat gtg atc tgg cta gaa aaa caa gct aaa gaa tgg ctc aaa ttg taa     768
   Asp Val Ile Trp Leu Glu Lys Gln Ala Lys Glu Trp Leu Lys Leu
                   245                 250                 255
<210> 22
   <211> 255
   <212> PRT
   <213> H. pylori
<400> 22
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Lys Ile Leu Pro Glu Val
                   165                 170                 175
   Ile Ile Leu Gly Cys Thr His Phe Pro Leu Ile Ala Gln Lys Ile Glu
               180                 185                 190
   Ser Tyr Phe Met Gly His Phe Ala Leu Pro Thr Pro Pro Leu Leu Ile
           195                 200                 205
   His Ser Gly Asp Ala Ile Val Glu Tyr Leu Gln Gln Lys Tyr Ala Leu
       210                 215                 220
   Lys Lys Asn Ala His Ala Phe Pro Lys Val Glu Phe His Ala Ser Gly
   225                 230                 235                 240
   Asp Val Ile Trp Leu Glu Lys Gln Ala Lys Glu Trp Leu Lys Leu
                   245                 250                 255
<210> 23
   <211> 768
   <212> DNA
   <213> H. pylori
<221> CDS
   <222> (1)..(768)
<400> 23
   atg aaa ata ggc gtt ttt gat agc ggt gtg gga ggg ttt agc gtt tta        48
   Met Lys Ile Gly Val Phe Asp Ser Gly Val Gly Gly Phe Ser Val Leu
   1               5                   10                  15
   aaa agc ctt tta aaa gcg caa cta ttt gat gaa atc atc tat tat ggc        96
   Lys Ser Leu Leu Lys Ala Gln Leu Phe Asp Glu Ile Ile Tyr Tyr Gly
               20                  25                  30
   gat agc gct aga gtg cct tat ggc act aaa gac ccc acc acg atc aag       144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att gga       192
   Gln Phe Gly Leu Glu Ala Leu Asp Phe Phe Lys Pro His Gln Ile Gly
       50                  55                  60
   tta ttg att gtg gca tgc aac aca gcg agc gct ctg gct tta gaa gag       240
   Leu Leu Ile Val Ala Cys Asn Thr Ala Ser Ala Leu Ala Leu Glu Glu
   65                  70                  75                  80
   atg caa aaa tat tcc aaa atc cct att gtg ggc gtg att gag cca agc       288
   Met Gln Lys Tyr Ser Lys Ile Pro Ile Val Gly Val Ile Glu Pro Ser
                   85                  90                  95
   att tta gcg atc aaa caa caa gta aaa gat aaa aac gcc ccc att tta       336
   Ile Leu Ala Ile Lys Gln Gln Val Lys Asp Lys Asn Ala Pro Ile Leu
               100                 105                 110
   gtg cta ggg aca aaa gcg acg atc caa tct aac gct tat gat aac gcc       384
   Val Leu Gly Thr Lys Ala Thr Ile Gln Ser Asn Ala Tyr Asp Asn Ala
           115                 120                 125
   ctg aaa caa caa ggc tat ttg aac att tcg cat tta gcc act tct ctt       432
   Leu Lys Gln Gln Gly Tyr Leu Asn Ile Ser His Leu Ala Thr Ser Leu
       130                 135                 140
   ttt gtg ccc ttg att gaa gaa agt att tta gag ggc gaa ttg tta gaa       480
   Phe Val Pro Leu Ile Glu Glu Ser Ile Leu Glu Gly Glu Leu Leu Glu
   145                 150                 155                 160
   act tgc atg cgt tat tat ttc act cca tta gag att tta cct gaa gtg       528
   Thr Cys Met Arg Tyr Tyr Phe Thr Pro Leu Glu Ile Leu Pro Glu Val
                   165                 170                 175
   atc att tta ggt tgc acg cat ttt ccc ttg atc gct caa aaa att gag       576
   Ile Ile Leu Gly Cys Thr His Phe Pro Leu Ile Ala Gln Lys Ile Glu
               180                 185                 190
   agc tat ttt atg gag cat ttt gcc ctt tca acg ccc ccc tta ctc atc       624
   Ser Tyr Phe Met Glu His Phe Ala Leu Ser Thr Pro Pro Leu Leu Ile
           195                 200                 205
   cat tct ggc gat gct att gtg gaa tac ttg caa caa aaa tac gcc ctt       672
   His Ser Gly Asp Ala Ile Val Glu Tyr Leu Gln Gln Lys Tyr Ala Leu
   210                      215                      220
   aag aaa aac gca cac gca ttc cct aaa gtg gaa ttt cat gcg agc ggc      720
   Lys Lys Asn Ala His Ala Phe Pro Lys Val Glu Phe His Ala Ser Gly
   225                 230                 235                 240
   gat gtg atc tgg cta gaa aaa cag gct aaa gaa tgg ctc aaa ttg taa      768
   Asp Val Ile Trp Leu Glu Lys Gln Ala Lys Glu Trp Leu Lys Leu
                   245                 250                 255
<210> 24
   <211> 255
   <212> PRT
   <213> H. pylori
<400> 24
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Gly
       50                  55                  60
   Leu Leu Ile Val Ala Cys Asn Thr Ala Ser Ala Leu Ala Leu Glu Glu
   65                  70                  75                  80
   Met Gln Lys Tyr Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Ile Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Glu Ile Leu Pro Glu Val
                   165                 170                 175
   Ile Ile Leu Gly Cys Thr His Phe Pro Leu Ile Ala Gln Lys Ile Glu
               180                 185                 190
   Ser Tyr Phe Met Glu His Phe Ala Leu Ser Thr Pro Pro Leu Leu Ile
   195 200 205
   His Ser Gly Asp Ala Ile Val Glu Tyr Leu Gln Gln Lys Tyr Ala Leu
   210 215 220
   Lys Lys Asn Ala His Ala Phe Pro Lys Val Glu Phe His Ala Ser Gly
   225 230 235 240
   Asp Val Ile Trp Leu Glu Lys Gln Ala Lys Glu Trp Leu Lys Leu
   245 250 255
<210> 25
   <211> 768
   <212> DNA
   <213> H. pylori
<221> CDS
   <222> (1)..(768)
<400> 25
   atg aaa ata ggc gtt ttt gat agc ggt gtg gga ggg ttt agc gtt tta 48
   Met Lys Ile Gly Val Phe Asp Ser Gly Val Gly Gly Phe Ser Val Leu
   1 5 10 15
   aaa agc ctt tta aaa gcg caa tta ttt gat gaa atc atc tat tat ggc 96
   Lys Ser Leu Leu Lys Ala Gln Leu Phe Asp Glu Ile Ile Tyr Tyr Gly
   20 25 30
   gat agc gct aga gtg cct tat ggc act aaa gac ccc acc acg atc aag 144
   Asp Ser Ala Arg Val Pro Tyr Gly Thr Lys Asp Pro Thr Thr Ile Lys
   35 40 45
   caa ttt ggc tta gag gct ttg gat ttt ttc aaa ccg cac aaa att gaa 192
   Gln Phe Gly Leu Glu Ala Leu Asp Phe Phe Lys Pro His Lys Ile Glu
   50 55 60
   tta tta att gtg gca tgc aac aca gcg agc gct ctg gct tta gaa gag 240
   Leu Leu Ile Val Ala Cys Asn Thr Ala Ser Ala Leu Ala Leu Glu Glu
   65 70 75 80
   atg caa aag cat tcc aaa atc ccc att gtg ggc gtg att gag cca agc 288
   Met Gln Lys His Ser Lys Ile Pro Ile Val Gly Val Ile Glu Pro Ser
   85 90 95
   att tta gcg atc aaa caa caa gtg aaa gat aaa aac acc cct att tta 336
   Ile Leu Ala Ile Lys Gln Gln Val Lys Asp Lys Asn Thr Pro Ile Leu
   100 105 110
   gtg cta ggg aca aaa gcg acg atc caa tct aac gct tac gat aac gcc 384
   Val Leu Gly Thr Lys Ala Thr Ile Gln Ser Asn Ala Tyr Asp Asn Ala
   115 120 125
   ctg aaa caa caa ggc tat ttg aag gtt tcg cat ttg gcc act tct ctt 432
   Leu Lys Gln Gln Gly Tyr Leu Lys Val Ser His Leu Ala Thr Ser Leu
   130 135 140
   ttt gtg cct ttg att gaa gaa agt att tta gag ggc gaa ttg tta gaa 480
   Phe Val Pro Leu Ile Glu Glu Ser Ile Leu Glu Gly Glu Leu Leu Glu
   145 150 155 160
   act tgc atg cgt tat tat ttc act cca tta gaa atc tta cct gaa gtg      528
   Thr Cys Met Arg Tyr Tyr Phe Thr Pro Leu Glu Ile Leu Pro Glu Val
                   165                 170                 175
   gtt att tta ggc tgc acg cat ttt ccc ttg atc gct caa aaa att gag      576
   Val Ile Leu Gly Cys Thr His Phe Pro Leu Ile Ala Gln Lys Ile Glu
               180                 185                 190
   ggc tat ttt atg gaa cat ttt gcc ctt cca acg ccc ccc cta ctc atc      624
   Gly Tyr Phe Met Glu His Phe Ala Leu Pro Thr Pro Pro Leu Leu Ile
           195                 200                 205
   cat tct ggc gac gct att gtg gga tat ttg cag caa aaa tac gcc ctt      672
   His Ser Gly Asp Ala Ile Val Gly Tyr Leu Gln Gln Lys Tyr Ala Leu
       210                 215                 220
   aag aaa aac gca cac gca ttc cct aaa gtg gaa ttt cat gcg agc ggc      720
   Lys Lys Asn Ala His Ala Phe Pro Lys Val Glu Phe His Ala Ser Gly
   225                 230                 235                 240
   gat gta att tgg cta gaa aaa cag gct aaa gaa tgg ctc aaa ttg taa      768
   Asp Val Ile Trp Leu Glu Lys Gln Ala Lys Glu Trp Leu Lys Leu
                   245                 250                 255
<210> 26
   <211> 255
   <212> PRT
   <213> H. pylori
<400> 26
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Lys Ile Glu
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Thr Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Lys Val Ser His Leu Ala Thr Ser Leu
       130                 135                 140
   Phe Val Pro Leu Ile Glu Glu Ser Ile Leu Glu Gly Glu Leu Leu Glu
   145                 150                 155                 160
   Thr Cys Met Arg Tyr Tyr Phe Thr Pro Leu Glu Ile Leu Pro Glu Val
                   165                 170                 175
   Val Ile Leu Gly Cys Thr His Phe Pro Leu Ile Ala Gln Lys Ile Glu
               180                 185                 190
   Gly Tyr Phe Met Glu His Phe Ala Leu Pro Thr Pro Pro Leu Leu Ile
           195                 200                 205
   His Ser Gly Asp Ala Ile Val Gly Tyr Leu Gln Gln Lys Tyr Ala Leu
       210                 215                 220
   Lys Lys Asn Ala His Ala Phe Pro Lys Val Glu Phe His Ala Ser Gly
   225                 230                 235                 240
   Asp Val Ile Trp Leu Glu Lys Gln Ala Lys Glu Trp Leu Lys Leu
                   245                 250                 255
<210> 27
   <211> 768
   <212> DNA
   <213> H. pylori
<221> CDS
   <222> (1)..(768)
<400> 27
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg caa att ttt gat gaa atc atc tat tat ggc      96
   Lys Ser Leu Leu Lys Ala Gln Ile Phe Asp Glu Ile Ile Tyr Tyr Gly
               20                  25                  30
   gat agc gct agg gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac aag att gaa     192
   Gln Phe Gly Leu Glu Ala Leu Asp Phe Phe Lys Pro His Lys Ile Glu
       50                  55                  60
   tta ttg att gtg gca tgc aac aca gcg agc gct cta gct tta gaa gaa     240
   Leu Leu Ile Val Ala Cys Asn Thr Ala Ser Ala Leu Ala Leu Glu Glu
   65                  70                  75                  80
   atg caa aag cat tcc aaa atc cct att gtg ggc gtg att gaa cca agc     288
   Met Gln Lys His Ser Lys Ile Pro Ile Val Gly Val Ile Glu Pro Ser
                   85                  90                  95
   att tta gcg atc aaa caa caa gta aaa gat aaa aac gcc cct att tta        336
   Ile Leu Ala Ile Lys Gln Gln Val Lys Asp Lys Asn Ala Pro Ile Leu
               100                 105                 110
   gtg cta ggg aca aaa gcg acg att caa tct aac gct tat gac aac gcc        384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt        432
   Leu Lys Gln Gln Gly Tyr Leu Asn Val Ser His Leu Ala Thr Ser Leu
       130                 135                 140
   ttt gtg cct ttg att gaa gaa aat att tta gag ggc gaa ttg cta gaa        480
   Phe Val Pro Leu Ile Glu Glu Asn Ile Leu Glu Gly Glu Leu Leu Glu
   145                 150                 155                 160
   act tgc atg cgt tat tat ttc act cca tta gag atc ttg cct gaa gtg        528
   Thr Cys Met Arg Tyr Tyr Phe Thr Pro Leu Glu Ile Leu Pro Glu Val
                   165                 170                 175
   gtt att tta ggc tgc acg cat ttt ccc ttg atc gct cac caa att gag        576
   Val Ile Leu Gly Cys Thr His Phe Pro Leu Ile Ala His Gln Ile Glu
               180                 185                 190
   ggc tat ttt atg gag cat ttt gcc ctt tca acg ccc ccc cta ctc atc        624
   Gly Tyr Phe Met Glu His Phe Ala Leu Ser Thr Pro Pro Leu Leu Ile
           195                 200                 205
   cat tct ggc gat gct att gtg gaa tat ttg cag caa aaa tac gcc ctt        672
   His Ser Gly Asp Ala Ile Val Glu Tyr Leu Gln Gln Lys Tyr Ala Leu
       210                 215                 220
   aag aaa aac gca tgt gca ttc cct aaa gta gaa ttt cat gcg agc ggc        720
   Lys Lys Asn Ala Cys Ala Phe Pro Lys Val Glu Phe His Ala Ser Gly
   225                 230                 235                 240
   gat gta att tgg cta gaa aaa cag gct aaa gaa tgg ctc aaa ttg taa        768
   Asp Val Ile Trp Leu Glu Lys Gln Ala Lys Glu Trp Leu Lys Leu
                   245                 250                 255
<210> 28
   <211> 255
   <212> PRT
   <213> H. pylori
<400> 28
   Met Lys Ile Gly Val Phe Asp Ser Gly Val Gly Gly Phe Ser Val Leu
   1               5                   10                  15
   Lys Ser Leu Leu Lys Ala Gln Ile Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Lys Ile Glu
       50                  55                  60
   Leu Leu Ile Val Ala Cys Asn Thr Ala Ser Ala Leu Ala Leu Glu Glu
   65 70 75 80
   Met Gln Lys His Ser Lys Ile Pro Ile Val Gly Val Ile Glu Pro Ser
   85 90 95
   Ile Leu Ala Ile Lys Gln Gln Val Lys Asp Lys Asn Ala Pro Ile Leu
   100 105 110
   Val Leu Gly Thr Lys Ala Thr Ile Gln Ser Asn Ala Tyr Asp Asn Ala
   115 120 125
   Leu Lys Gln Gln Gly Tyr Leu Asn Val Ser His Leu Ala Thr Ser Leu
   130 135 140
   Phe Val Pro Leu Ile Glu Glu Asn Ile Leu Glu Gly Glu Leu Leu Glu
   145 150 155 160
   Thr Cys Met Arg Tyr Tyr Phe Thr Pro Leu Glu Ile Leu Pro Glu Val
   165 170 175
   Val Ile Leu Gly Cys Thr His Phe Pro Leu Ile Ala His Gln Ile Glu
   180 185 190
   Gly Tyr Phe Met Glu His Phe Ala Leu Ser Thr Pro Pro Leu Leu Ile
   195 200 205
   His Ser Gly Asp Ala Ile Val Glu Tyr Leu Gln Gln Lys Tyr Ala Leu
   210 215 220
   Lys Lys Asn Ala Cys Ala Phe Pro Lys Val Glu Phe His Ala Ser Gly
   225 230 235 240
   Asp Val Ile Trp Leu Glu Lys Gln Ala Lys Glu Trp Leu Lys Leu
   245 250 255
<210> 29
   <211> 768
   <212> DNA
   <213> H. pylori
<221> CDS
   <222> (1)..(768)
<400> 29
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gtg caa tta ttt gat gaa atc atc tat tat ggc      96
   Lys Ser Leu Leu Lys Val Gln Leu Phe Asp Glu Ile Ile Tyr Tyr Gly
               20                  25                  30
   gat agt gct agg gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac aag att gaa     192
   Gln Phe Gly Leu Glu Ala Leu Asp Phe Phe Lys Pro His Lys Ile Glu
       50                  55                  60
   tta ttg att gtg gca tgc aac aca gcg agc gct cta gct tta gga gag     240
   Leu Leu Ile Val Ala Cys Asn Thr Ala Ser Ala Leu Ala Leu Gly Glu
   65                  70                  75                  80
   atg caa aag tat tcc aaa atc cct att gtg ggc gtg att gag cca agc     288
   Met Gln Lys Tyr Ser Lys Ile Pro Ile Val Gly Val Ile Glu Pro Ser
                   85                  90                  95
   att tta gcg atc aaa caa caa gta aaa gat aaa aac gcc cct att tta     336
   Ile Leu Ala Ile Lys Gln Gln Val Lys Asp Lys Asn Ala Pro Ile Leu
               100                 105                 110
   gta cta ggg aca aaa gcg acg att cga tcc aac gct tat gac aac gcc     384
   Val Leu Gly Thr Lys Ala Thr Ile Arg Ser Asn Ala Tyr Asp Asn Ala
           115                 120                 125
   ctg aaa caa caa ggc tat ttg aat att tcg cat tta gcc act tct ctt     432
   Leu Lys Gln Gln Gly Tyr Leu Asn Ile Ser His Leu Ala Thr Ser Leu
       130                 135                 140
   ttt gtg cct ttg att gaa gaa aat att tta gag ggc gaa ttg cta gaa     480
   Phe Val Pro Leu Ile Glu Glu Asn Ile Leu Glu Gly Glu Leu Leu Glu
   145                 150                 155                 160
   act tgc atg cgt tat tat ttc act cca tta gag att tta cct gaa gtg     528
   Thr Cys Met Arg Tyr Tyr Phe Thr Pro Leu Glu Ile Leu Pro Glu Val
                   165                 170                 175
   gtt att tta ggt tgc acg cat ttt ccc ttg atc gct cac caa att gag     576
   Val Ile Leu Gly Cys Thr His Phe Pro Leu Ile Ala His Gln Ile Glu
               180                 185                 190
   ggc tat ttt atg gag cat ttt gcc ctt tca acg ccc ccc cta ctc atc     624
   Gly Tyr Phe Met Glu His Phe Ala Leu Ser Thr Pro Pro Leu Leu Ile
           195                 200                 205
   cat tct ggc gat gct att gtg gaa tat ttg caa caa aaa tac gcc ctt     672
   His Ser Gly Asp Ala Ile Val Glu Tyr Leu Gln Gln Lys Tyr Ala Leu
       210                 215                 220
   aag aaa aac gca tgc gca ttc cct aaa gta gaa ttc cat gcg agc ggc     720
   Lys Lys Asn Ala Cys Ala Phe Pro Lys Val Glu Phe His Ala Ser Gly
   225                 230                 235                 240
   gat gta att tgg cta gaa aaa cag gct aaa gaa tgg ctc aaa ttg taa     768
   Asp Val Ile Trp Leu Glu Lys Gln Ala Lys Glu Trp Leu Lys Leu
                   245                 250                 255
<210> 30
   <211> 255
   <212> PRT
   <213> H. pylori
<400> 30
   Met Lys Ile Gly Val Phe Asp Ser Gly Val Gly Gly Phe Ser Val Leu
   1 5 10 15
   Lys Ser Leu Leu Lys Val Gln Leu Phe Asp Glu Ile Ile Tyr Tyr Gly
   20 25 30
   Asp Ser Ala Arg Val Pro Tyr Gly Thr Lys Asp Pro Thr Thr Ile Lys
   35 40 45
   Gln Phe Gly Leu Glu Ala Leu Asp Phe Phe Lys Pro His Lys Ile Glu
   50 55 60
   Leu Leu Ile Val Ala Cys Asn Thr Ala Ser Ala Leu Ala Leu Gly Glu
   65 70 75 80
   Met Gln Lys Tyr Ser Lys Ile Pro Ile Val Gly Val Ile Glu Pro Ser
   85 90 95
   Ile Leu Ala Ile Lys Gln Gln Val Lys Asp Lys Asn Ala Pro Ile Leu
   100 105 110
   Val Leu Gly Thr Lys Ala Thr Ile Arg Ser Asn Ala Tyr Asp Asn Ala
   115 120 125
   Leu Lys Gln Gln Gly Tyr Leu Asn Ile Ser His Leu Ala Thr Ser Leu
   130 135 140
   Phe Val Pro Leu Ile Glu Glu Asn Ile Leu Glu Gly Glu Leu Leu Glu
   145 150 155 160
   Thr Cys Met Arg Tyr Tyr Phe Thr Pro Leu Glu Ile Leu Pro Glu Val
   165 170 175
   Val Ile Leu Gly Cys Thr His Phe Pro Leu Ile Ala His Gln Ile Glu
   180 185 190
   Gly Tyr Phe Met Glu His Phe Ala Leu Ser Thr Pro Pro Leu Leu Ile
   195 200 205
   His Ser Gly Asp Ala Ile Val Glu Tyr Leu Gln Gln Lys Tyr Ala Leu
   210 215 220
   Lys Lys Asn Ala Cys Ala Phe Pro Lys Val Glu Phe His Ala Ser Gly
   225 230 235 240
   Asp Val Ile Trp Leu Glu Lys Gln Ala Lys Glu Trp Leu Lys Leu
   245 250 255
<210> 31
   <211> 768
   <212> DNA
   <213> H. pylori
<221> CDS
   <222> (1)..(768)
<400> 31
   atg aaa ata ggc gtt ttt gat agc ggt gtg gga ggg ttt agc gtt tta       48
   Met Lys Ile Gly Val Phe Asp Ser Gly Val Gly Gly Phe Ser Val Leu
   1               5                   10                  15
   aaa agc ctt tta aaa gcg caa att ttt gat gaa atc atc tat tat ggc       96
   Lys Ser Leu Leu Lys Ala Gln Ile Phe Asp Glu Ile Ile Tyr Tyr Gly
               20                  25                  30
   gat agt gct aga gtg cct tat ggc act aaa gac ccc acc acg atc aag      144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att gga      192
   Gln Phe Gly Leu Glu Ala Leu Asp Phe Phe Lys Pro His Gln Ile Gly
       50                  55                  60
   tta ttg att gtg gca tgc aac aca gcg agc gct cta gct tta gaa gag      240
   Leu Leu Ile Val Ala Cys Asn Thr Ala Ser Ala Leu Ala Leu Glu Glu
   65                  70                  75                  80
   atg caa aag cat tcc aaa atc cct att gtg ggt gtg att gag cca agc      288
   Met Gln Lys His Ser Lys Ile Pro Ile Val Gly Val Ile Glu Pro Ser
                   85                  90                  95
   att tta gcg atc aaa caa caa gta aaa gat aaa aac gcc cct att tta      336
   Ile Leu Ala Ile Lys Gln Gln Val Lys Asp Lys Asn Ala Pro Ile Leu
               100                 105                 110
   gtg tta ggg aca aaa gcg acg att caa tcc aac gct tat gac aac gcc      384
   Val Leu Gly Thr Lys Ala Thr Ile Gln Ser Asn Ala Tyr Asp Asn Ala
           115                 120                 125
   ctg aaa caa caa ggc tat ttg aac gtt tcg cat tta gcc act tct ctt      432
   Leu Lys Gln Gln Gly Tyr Leu Asn Val Ser His Leu Ala Thr Ser Leu
       130                 135                 140
   ttt gtg cct ttg att gaa gaa aat att tta gag ggc gaa ttg tta gaa      480
   Phe Val Pro Leu Ile Glu Glu Asn Ile Leu Glu Gly Glu Leu Leu Glu
   145                 150                 155                 160
   act tgc atg cgt tat tat ttc act cca tta gag att tta cct gaa gtg      528
   Thr Cys Met Arg Tyr Tyr Phe Thr Pro Leu Glu Ile Leu Pro Glu Val
                   165                 170                 175
   gtt att tta ggt tgc acg cat ttt ccc ttg atc gct cac caa att gag      576
   Val Ile Leu Gly Cys Thr His Phe Pro Leu Ile Ala His Gln Ile Glu
               180                 185                 190
   ggc tat ttt atg gag cat ttt gcc ctt tca acg ccc ccc tta ctc atc      624
   Gly Tyr Phe Met Glu His Phe Ala Leu Ser Thr Pro Pro Leu Leu Ile
           195                 200                 205
   cat tct ggc gat gct att gtg gaa tat ttg caa caa aaa tac acc ctt      672
   His Ser Gly Asp Ala Ile Val Glu Tyr Leu Gln Gln Lys Tyr Thr Leu
       210                 215                 220
   aag aaa aat gca tgc gcg ttc cct aaa gtg gaa ttt cat gcg agc ggc      720
   Lys Lys Asn Ala Cys Ala Phe Pro Lys Val Glu Phe His Ala Ser Gly
   225                 230                 235                 240
   gat gtg gtt tgg cta gaa aaa cag gct aaa gaa tgg ctc aaa ttg taa      768
   Asp Val Val Trp Leu Glu Lys Gln Ala Lys Glu Trp Leu Lys Leu
                   245                 250                 255
<210> 32
   <211> 255
   <212> PRT
   <213> H. pylori
<400> 32
   Met Lys Ile Gly Val Phe Asp Ser Gly Val Gly Gly Phe Ser Val Leu
   1               5                   10                  15
   Lys Ser Leu Leu Lys Ala Gln Ile Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Gly
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Ile Val Gly Val Ile Glu Pro Ser
                   85                  90                  95
   Ile Leu Ala Ile Lys Gln Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Asn Ile Leu Glu Gly Glu Leu Leu Glu
   145                 150                 155                 160
   Thr Cys Met Arg Tyr Tyr Phe Thr Pro Leu Glu Ile Leu Pro Glu Val
                   165                 170                 175
   Val Ile Leu Gly Cys Thr His Phe Pro Leu Ile Ala His Gln Ile Glu
               180                 185                 190
   Gly Tyr Phe Met Glu His Phe Ala Leu Ser Thr Pro Pro Leu Leu Ile
           195                 200                 205
   His Ser Gly Asp Ala Ile Val Glu Tyr Leu Gln Gln Lys Tyr Thr Leu
       210                 215                 220
   Lys Lys Asn Ala Cys Ala Phe Pro Lys Val Glu Phe His Ala Ser Gly
   225                 230                 235                 240
   Asp Val Val Trp Leu Glu Lys Gln Ala Lys Glu Trp Leu Lys Leu
                   245                 250                 255
<210> 33
   <211> 765
   <212> DNA
   <213> H. pylori
<221> CDS
   <222> (1)..(765)
<400> 33
   atg aaa ata ggc gtt ttt gat agc ggt gtg gga ggg ttt agc gtt tta      48
   Met Lys Ile Gly Val Phe Asp Ser Gly Val Gly Gly Phe Ser Val Leu
   1               5                   10                  15
   aaa agc ctt tta aaa gcg caa cta ttt gat gaa atc atc tat tat ggc      96
   Lys Ser Leu Leu Lys Ala Gln Leu Phe Asp Glu Ile Ile Tyr Tyr Gly
               20                  25                  30
   gat agc gct aga gtg cct tat ggc act aaa gac ccc acc acg atc aag     144
   Asp Ser Ala Arg Val Pro Tyr Gly Thr Lys Asp Pro Thr Thr Ile Lys
           35                  40                  45
   caa ttt ggc tta gag gct ttg gat ttt ttc aaa ccg cac cag att aaa     192
   Gln Phe Gly Leu Glu Ala Leu Asp Phe Phe Lys Pro His Gln Ile Lys
       50                  55                  60
   tta ttg att gtg gca tgc aac acc gca agc gct ctg gct tta gaa gag     240
   Leu Leu Ile Val Ala Cys Asn Thr Ala Ser Ala Leu Ala Leu Glu Glu
   65                  70                  75                  80
   atg caa aag cat tcc aaa atc cct gtt gtg ggc gtg att gag cca agc     288
   Met Gln Lys His Ser Lys Ile Pro Val Val Gly Val Ile Glu Pro Ser
                   85                  90                  95
   att tta gcg atc aaa cgg caa gtg aaa gat aaa aac gcc cct att ttg     336
   Ile Leu Ala Ile Lys Arg Gln Val Lys Asp Lys Asn Ala Pro Ile Leu
               100                 105                 110
   gtg cta ggg aca aaa gcg acg att caa tcc aac gcc tat gat aac gcc     384
   Val Leu Gly Thr Lys Ala Thr Ile Gln Ser Asn Ala Tyr Asp Asn Ala
           115                 120                 125
   ctg aaa caa caa ggc tat ttg aat gtt tcg cat tta gcc act tct ctt     432
   Leu Lys Gln Gln Gly Tyr Leu Asn Val Ser His Leu Ala Thr Ser Leu
       130                 135                 140
   ttt gtg cct ttg att gaa gaa agt att tta gag ggc gaa ttg cta gaa     480
   Phe Val Pro Leu Ile Glu Glu Ser Ile Leu Glu Gly Glu Leu Leu Glu
   145                 150                 155                 160
   act tgc atg cgt tat tat ttc act cca tta gag att tta cct gaa gtg     528
   Thr Cys Met Arg Tyr Tyr Phe Thr Pro Leu Glu Ile Leu Pro Glu Val
                   165                 170                 175
   gtt att tta ggt tgc acg cat ttt ccc ttg atc gct caa aaa att gag        576
   Val Ile Leu Gly Cys Thr His Phe Pro Leu Ile Ala Gln Lys Ile Glu
               180                 185                 190
   ggc tat ttt atg gag cat ttt gcc ctt tca acg ccc ccc cta ctc atc        624
   Gly Tyr Phe Met Glu His Phe Ala Leu Ser Thr Pro Pro Leu Leu Ile
           195                 200                 205
   cat tct ggc gat gct att gtg gaa tat ttg caa caa aat tac gcc ctt        672
   His Ser Gly Asp Ala Ile Val Glu Tyr Leu Gln Gln Asn Tyr Ala Leu
       210                 215                 220
   aag aaa aac gca tgc gcg ttc cct aaa gtg gaa ttt cat gcg agc ggc        720
   Lys Lys Asn Ala Cys Ala Phe Pro Lys Val Glu Phe His Ala Ser Gly
   225                 230                 235                 240
   gat gtg gtt tgg cta gaa aaa caa gct aaa gaa tgg ctt aaa ttg            765
   Asp Val Val Trp Leu Glu Lys Gln Ala Lys Glu Trp Leu Lys Leu
                   245                 250                 255
<210> 34
   <211> 255
   <212> PRT
   <213> H. pylori
<400> 34
   Met Lys Ile Gly Val Phe Asp Ser Gly Val Gly Gly Phe Ser Val Leu
   1               5                   10                  15
   Lys Ser Leu Leu Lys Ala Gln Leu Phe Asp Glu Ile Ile Tyr Tyr Gly
               20                  25                  30
   Asp Ser Ala Arg Val Pro Tyr Gly Thr Lys Asp Pro Thr Thr Ile Lys
           35                  40                  45
   Gln Phe Gly Leu Glu Ala Leu Asp Phe Phe Lys Pro His Gln Ile Lys
       50                  55                  60
   Leu Leu Ile Val Ala Cys Asn Thr Ala Ser Ala Leu Ala Leu Glu Glu
   65                  70                  75                  80
   Met Gln Lys His Ser Lys Ile Pro Val Val Gly Val Ile Glu Pro Ser
                   85                  90                  95
   Ile Leu Ala Ile Lys Arg Gln Val Lys Asp Lys Asn Ala Pro Ile Leu
               100                 105                 110
   Val Leu Gly Thr Lys Ala Thr Ile Gln Ser Asn Ala Tyr Asp Asn Ala
           115                 120                 125
   Leu Lys Gln Gln Gly Tyr Leu Asn Val Ser His Leu Ala Thr Ser Leu
       130                 135                 140
   Phe Val Pro Leu Ile Glu Glu Ser Ile Leu Glu Gly Glu Leu Leu Glu
   145 150 155 160
   Thr Cys Met Arg Tyr Tyr Phe Thr Pro Leu Glu Ile Leu Pro Glu Val
   165 170 175
   Val Ile Leu Gly Cys Thr His Phe Pro Leu Ile Ala Gln Lys Ile Glu
   180 185 190
   Gly Tyr Phe Met Glu His Phe Ala Leu Ser Thr Pro Pro Leu Leu Ile
   195 200 205
   His Ser Gly Asp Ala Ile Val Glu Tyr Leu Gln Gln Asn Tyr Ala Leu
   210 215 220
   Lys Lys Asn Ala Cys Ala Phe Pro Lys Val Glu Phe His Ala Ser Gly
   225 230 235 240
   Asp Val Val Trp Leu Glu Lys Gln Ala Lys Glu Trp Leu Lys Leu
   245 250 255
<210> 35
   <211> 29
   <212> DNA
   <213> Artificial sequence
<220>
   <223> Homo sapiens
<400> 35
   aaatagtcat atgaaaatag gcgtttttg 29
<210> 36
   <211> 28
   <212> DNA
   <213> Artificial sequence
<220>
   <223> Homo sapiens
<400> 36
   agaattctat tacaatttga gccattct 28
<210> 37
   <211> 26
   <212> DNA
   <213> Artificial sequence
<220>
   <223> Homo sapiens
<400> 37
   gcgaattcga tcagaatttt ttttct 26
<210> 38
   <211> 26
   <212> DNA
   <213> Artificial sequence
<220>
   <223> Homo sapiens
<400> 38 26
   ataagtactt gtgaatctta tactag 26
<210> 39
   <211> 858
   <212> DNA
   <213> E. coli
<400> 39
   atggctacca aactgcagga cgggaataca ccttgtctgg cagctacacc ttctgaacca 60
   cgtcccaccg tgctggtgtt tgactccggc gtcggtgggt tgtcggtcta tgacgagatc 120
   cggcatctct taccggatct ccattacatt tatgctttcg ataacgtcgc tttcccgtat 180
   ggcgaaaaaa gcgaagcgtt tattgttgag cgagtggtgg caattgtcac cgcggtgcaa 240
   gaacgttatc cccttgcgct ggctgtggtc gcttgcaaca ctgccagtac cgtttcactt 300
   cctgcattac gcgaaaagtt cgacttcccg gttgttggtg tcgtgccggc gattaaacct 360
   gctgcacgtc tgacggcaaa tggcattgtc ggattactgg caacccgcgg aacagttaaa 420
   cgttcttata ctcatgagct gatcgcgcgt ttcgctaatg aatgccagat agaaatgctg 480
   ggctcggcag agatggttga gttggctgaa gcgaagctac atggcgaaga tgtttctctg 540
   gatgcactaa aacgtatcct acgcccgtgg ttaagaatga aagagccgcc agataccgtt 600
   gtattgggtt gcacccattt ccctctacta caagaagaac tgttacaagt gctgccagag 660
   ggaacccggc tggtggattc tggcgcagcg attgctcgcc gaacggcctg gttgttagaa 720
   catgaagccc cggatgcaaa atctgccgat gcgaatattg ccttttgtat ggcaatgacg 780
   ccaggagctg aacaattatt gcccgtttta cagcgttacg gcttcgaaac gctcgaaaaa 840
   ctggcagttt taggctga 858
<210> 40
   <211> 285
   <212> PRT
   <213> E. coli
<400> 40
   Met Ala Thr Lys Leu Gln Asp Gly Asn Thr Pro Cys Leu Ala Ala Thr
   1 5 10 15
   Pro Ser Glu Pro Arg Pro Thr Val Leu Val Phe Asp Ser Gly Val Gly
   20 25 30
   Gly Leu Ser Val Tyr Asp Glu Ile Arg His Leu Leu Pro Asp Leu His
   35 40 45
   Tyr Ile Tyr Ala Phe Asp Asn Val Ala Phe Pro Tyr Gly Glu Lys Ser
   50 55 60
   Glu Ala Phe Ile Val Glu Arg Val Val Ala Ile Val Thr Ala Val Gln
   65 70 75 80
   Glu Arg Tyr Pro Leu Ala Leu Ala Val Val Ala Cys Asn Thr Ala Ser
   85 90 95
   Thr Val Ser Leu Pro Ala Leu Arg Glu Lys Phe Asp Phe Pro Val Val
   100 105 110
   Gly Val Val Pro Ala Ile Lys Pro Ala Ala Arg Leu Thr Ala Asn Gly
   115 120 125
   Ile Val Gly Leu Leu Ala Thr Arg Gly Thr Val Lys Arg Ser Tyr Thr
   130 135 140
   His Glu Leu Ile Ala Arg Phe Ala Asn Glu Cys Gln Ile Glu Met Leu
   145 150 155 160
   Gly Ser Ala Glu Met Val Glu Leu Ala Glu Ala Lys Leu His Gly Glu
   165 170 175
   Asp Val Ser Leu Asp Ala Leu Lys Arg Ile Leu Arg Pro Trp Leu Arg
   180 185 190
   Met Lys Glu Pro Pro Asp Thr Val Val Leu Gly Cys Thr His Phe Pro
   195 200 205
   Leu Leu Gln Glu Glu Leu Leu Gln Val Leu Pro Glu Gly Thr Arg Leu
   210 215 220
   Val Asp Ser Gly Ala Ala Ile Ala Arg Arg Thr Ala Trp Leu Leu Glu
   225 230 235 240
   His Glu Ala Pro Asp Ala Lys Ser Ala Asp Ala Asn Ile Ala Phe Cys
   245 250 255
   Met Ala Met Thr Pro Gly Ala Glu Gln Leu Leu Pro Val Leu Gln Arg
   260 265 270
   Tyr Gly Phe Glu Thr Leu Glu Lys Leu Ala Val Leu Gly
   275 280 285
<210> 41
   <211> 29
   <212> DNA
   <213> Artificial Sequence
<220>
   <223> Homo sapiens
<400> 41
   aaatagtcat atgaaaatag gcgtttttg 29
<210> 42
   <211> 28
   <212> DNA
   <213> Artificial sequence
<220>
   <223> Homo sapiens
<400> 42
   agaattctat tacaatttga gccattct 28
<210> 43
   <211> 822
   <212> DNA
   <213> E. faecalis
<400> 43
   atgagcaatc aagaagccat tggattaatt gattctggcg ttggtggatt aactgtttta     60
   aaggaagcgc taaagcaatt accaaatgaa cgattaattt atttaggaga tacagcccgt    120
   tgcccatatg gtccacgacc agccgaacaa gtcgttcagt ttacttggga aatggccgat    180
   tttttattga aaaaacgaat aaaaatgcta gtaatcgcat gtaataccgc gacggctgtc    240
   gcattagaag aaattaaagc tgccttgcca attccagttg ttggtgttat tttacctggc    300
   gcacgagcag ccgttaaagt cacaaaaaat aacaaaattg gtgtcatagg taccttaggg    360
   acaatcaaaa gtgcttccta tgaaatcgcc attaaaagta aggcaccagc aattgaggtg    420
   actagtttag cttgccctaa atttgtcccc attgttgaaa gtaatcaata tcgttcttcc    480
   gtagcaaaaa aaattgtggc agaaacactt caagcactac aattaaaagg acttgatacg    540
   ttgattttag gttgtaccca ttacccgttg ttacgtccgg tgattcaaaa tgtgatgggg    600
   agtcatgtga cattaattga ctcaggagcc gaaacagttg gcgaagtcag catgcttctc    660
   gattattttg acattgccca cacgcctgaa gcgcctacac agccccatga attttataca    720
   actggttctg caaaaatgtt tgaagagatt gcaagcagtt ggcttggtat agagaactta    780
   aaagcacaac agattcactt aggaggaaac gaaaatgatt ag                       822
<210> 44
   <211> 273
   <212> PRT
   <213> E. faecalis
<400> 44
   Met Ser Asn Gln Glu Ala Ile Gly Leu Ile Asp Ser Gly Val Gly Gly
   1               5                   10                  15
   Leu Thr Val Leu Lys Glu Ala Leu Lys Gln Leu Pro Asn Glu Arg Leu
               20                  25                  30
   Ile Tyr Leu Gly Asp Thr Ala Arg Cys Pro Tyr Gly Pro Arg Pro Ala
   35 40 45
   Glu Gln Val Val Gln Phe Thr Trp Glu Met Ala Asp Phe Leu Leu Lys
   50 55 60
   Lys Arg Ile Lys Met Leu Val Ile Ala Cys Asn Thr Ala Thr Ala Val
   65 70 75 80
   Ala Leu Glu Glu Ile Lys Ala Ala Leu Pro Ile Pro Val Val Gly Val
   85 90 95
   Ile Leu Pro Gly Ala Arg Ala Ala Val Lys Val Thr Lys Asn Asn Lys
   100 105 110
   Ile Gly Val Ile Gly Thr Leu Gly Thr Ile Lys Ser Ala Ser Tyr Glu
   115 120 125
   Ile Ala Ile Lys Ser Lys Ala Pro Ala Ile Glu Val Thr Ser Leu Ala
   130 135 140
   Cys Pro Lys Phe Val Pro Ile Val Glu Ser Asn Gln Tyr Arg Ser Ser
   145 150 155 160
   Val Ala Lys Lys Ile Val Ala Glu Thr Leu Gln Ala Leu Gln Leu Lys
   165 170 175
   Gly Leu Asp Thr Leu Ile Leu Gly Cys Thr His Tyr Pro Leu Leu Arg
   180 185 190
   Pro Val Ile Gln Asn Val Met Gly Ser His Val Thr Leu Ile Asp Ser
   195 200 205
   Gly Ala Glu Thr Val Gly Glu Val Ser Met Leu Leu Asp Tyr Phe Asp
   210 215 220
   Ile Ala His Thr Pro Glu Ala Pro Thr Gln Pro His Glu Phe Tyr Thr
   225 230 235 240
   Thr Gly Ser Ala Lys Met Phe Glu Glu Ile Ala Ser Ser Trp Leu Gly
   245 250 255
   Ile Glu Asn Leu Lys Ala Gln Gln Ile His Leu Gly Gly Asn Glu Asn
   260 265 270
   Asp
<210> 45
   <211> 801
   <212> DNA
   <213> S. aureus
<400> 45
   atgaataaac caataggtgt aatagactct ggtgtcggag gtttgacagt agctaaagaa      60
   attatgcgtc agttgccaaa tgagacgatt tattacttag gtgatattgg gcgatgtcca     120
   tatgggccaa gaccaggaga acaagtaaaa caatatacag ttgaaatcgc tcgtaaatta     180
   atggaatttg atataaaaat gctcgtgatt gcttgtaata ctgcaactgc tgtagcttta     240
   gaatatttac aaaagacctt atcaatctca gtgattggcg taattgaacc aggtgctaga     300
   acagcaataa tgacgactag aaatcaaaat gtattagtac taggaacgga aggcacaatt     360
   aaatctgaag catatcgaac acatattaaa cgtataaatc cacatgtaga ggtacatggc     420
   gttgcctgtc caggttttgt gccacttgta gaacaaatga gatatagtga tccaacaatt     480
   acaagcattg ttattcatca aacactgaaa cgttggcgta atagtgagtc tgatactgtc     540
   attttaggat gtacccacta tccattgctc tataaaccta tctatgatta ttttggtggt     600
   aaaaagacag tgatttcgtc tggattagaa acggctcgtg aagttagtgc attgctaaca     660
   tttagtaatg aacatgcaag ttatactgaa catccagatc atcgattttt tgcaacaggt     720
   gataccacac atattactaa cattatcaaa gaatggctaa atttatctgt caatgtggaa     780
   cgtatatcag tgaatgacta g                                               801
<210> 46
   <211> 266
   <212> PRT
   <213> S. aureus
<400> 46
   Met Asn Lys Pro Ile Gly Val Ile Asp Ser Gly Val Gly Gly Leu Thr
   1               5                   10                  15
   Val Ala Lys Glu Ile Met Arg Gln Leu Pro Asn Glu Thr Ile Tyr Tyr
               20                  25                  30
   Leu Gly Asp Ile Gly Arg Cys Pro Tyr Gly Pro Arg Pro Gly Glu Gln
           35                  40                  45
   Val Lys Gln Tyr Thr Val Glu Ile Ala Arg Lys Leu Met Glu Phe Asp
       50                  55                  60
   Ile Lys Met Leu Val Ile Ala Cys Asn Thr Ala Thr Ala Val Ala Leu
   65                  70                  75                  80
   Glu Tyr Leu Gln Lys Thr Leu Ser Ile Ser Val Ile Gly Val Ile Glu
                   85                  90                  95
   Pro Gly Ala Arg Thr Ala Ile Met Thr Thr Arg Asn Gln Asn Val Leu
               100                 105                 110
   Val Leu Gly Thr Glu Gly Thr Ile Lys Ser Glu Ala Tyr Arg Thr His
           115                 120                 125
   Ile Lys Arg Ile Asn Pro His Val Glu Val His Gly Val Ala Cys Pro
       130                 135                 140
   Gly Phe Val Pro Leu Val Glu Gln Met Arg Tyr Ser Asp Pro Thr Ile
   145                 150                 155                 160
   Thr Ser Ile Val Ile His Gln Thr Leu Lys Arg Trp Arg Asn Ser Glu
                   165                 170                 175
   Ser Asp Thr Val Ile Leu Gly Cys Thr His Tyr Pro Leu Leu Tyr Lys
               180                 185                 190
   Pro Ile Tyr Asp Tyr Phe Gly Gly Lys Lys Thr Val Ile Ser Ser Gly
           195                 200                 205
   Leu Glu Thr Ala Arg Glu Val Ser Ala Leu Leu Thr Phe Ser Asn Glu
       210                 215                 220
   His Ala Ser Tyr Thr Glu His Pro Asp His Arg Phe Phe Ala Thr Gly
   225                 230                 235                 240
   Asp Thr Thr His Ile Thr Asn Ile Ile Lys Glu Trp Leu Asn Leu Ser
                   245                 250                 255
   Val Asn Val Glu Arg Ile Ser Val Asn Asp
               260                 265
<210> 47
   <211> 822
   <212> DNA
   <213> E. faecium
<400> 47
   atgatacgat tgacagataa tcgccctatc ggatttattg attcaggtgt cggcggcttg     60
   actgtagtaa aagaagccct gaaacaatta ccgaatgaaa atattttatt tgtaggagac    120
   acagcacgct gcccatatgg ccctagaccc gcggaacagg taatccagta tacttgggaa    180
   atgacggatt atctggtgga gcaaggaatc aagatgctgg tgatcgcctg caataccgca    240
   actgcggtgg ctttagaaga aatcaaagct gctctttcta ttccagtcat cggtgtgatc    300
   cttcccggta ctagagcggc agtaaaaaaa acacaaaata aacaagttgg cattatcggt    360
   acgattggta cggtaaaaag tcaagcttat gaaaaagcac tgaaagagaa agtaccagaa    420
   ttgactgtga caagtcttgc ttgtccaaaa tttgtttcag ttgtcgaaag taatgaatac    480
   cattcatcgg tggcgaaaaa aattgtggca gaaacattag ctcctttaac cactaaaaaa     540
   atcgatacat tgattttggg atgcacccat tatccattat tacgccccat cattcaaaat     600
   gtaatgggag aaaatgttca actgatcgat tctggagcag aaacagtagg tgaagtatct     660
   atgctgttag attatttcaa tctgagcaat tcaccgcaaa atggtcggac attatgccag     720
   ttttatacaa ctggctctgc caaacttttc gaggaaatag ctgaagactg gcttggaatc     780
   ggacacttaa atgtagaaca tatcgaattg ggaggaaaat aa                        822
<210> 48
   <211> 273
   <212> PRT
   <213> E. faecium
<400> 48
   Met Ile Arg Leu Thr Asp Asn Arg Pro Ile Gly Phe Ile Asp Ser Gly
   1               5                   10                  15
   Val Gly Gly Leu Thr Val Val Lys Glu Ala Leu Lys Gln Leu Pro Asn
               20                  25                  30
   Glu Asn Ile Leu Phe Val Gly Asp Thr Ala Arg Cys Pro Tyr Gly Pro
           35                  40                  45
   Arg Pro Ala Glu Gln Val Ile Gln Tyr Thr Trp Glu Met Thr Asp Tyr
       50                  55                  60
   Leu Val Glu Gln Gly Ile Lys Met Leu Val Ile Ala Cys Asn Thr Ala
   65                  70                  75                  80
   Thr Ala Val Ala Leu Glu Glu Ile Lys Ala Ala Leu Ser Ile Pro Val
                   85                  90                  95
   Ile Gly Val Ile Leu Pro Gly Thr Arg Ala Ala Val Lys Lys Thr Gln
               100                 105                 110
   Asn Lys Gln Val Gly Ile Ile Gly Thr Ile Gly Thr Val Lys Ser Gln
           115                 120                 125
   Ala Tyr Glu Lys Ala Leu Lys Glu Lys Val Pro Glu Leu Thr Val Thr
       130                 135                 140
   Ser Leu Ala Cys Pro Lys Phe Val Ser Val Val Glu Ser Asn Glu Tyr
   145                 150                 155                 160
   His Ser Ser Val Ala Lys Lys Ile Val Ala Glu Thr Leu Ala Pro Leu
                   165                 170                 175
   Thr Thr Lys Lys Ile Asp Thr Leu Ile Leu Gly Cys Thr His Tyr Pro
   180 185 190
   Leu Leu Arg Pro Ile Ile Gln Asn Val Met Gly Glu Asn Val Gln Leu
   195 200 205
   Ile Asp Ser Gly Ala Glu Thr Val Gly Glu Val Ser Met Leu Leu Asp
   210 215 220
   Tyr Phe Asn Leu Ser Asn Ser Pro Gln Asn Gly Arg Thr Leu Cys Gln
   225 230 235 240
   Phe Tyr Thr Thr Gly Ser Ala Lys Leu Phe Glu Glu Ile Ala Glu Asp
   245 250 255
   Trp Leu Gly Ile Gly His Leu Asn Val Glu His Ile Glu Leu Gly Gly
   260 265 270
   Lys
<210> 49
   <211> 335
   <212> DNA
   <213> E. saccharolyticus
<400> 49
   gcatgtaata ccgcaacggc ggtagcgtta gaagaaatta aagcgcaatt agatattcca 60
   gtcgtcggtg tgatcttacc tggtactcgt gctgcagtta aagctacgaa aaatcgtcaa 120
   atcggtatta taggaacagc gggtacaatt aaaagtagtt cgtatgagca agcaattaaa 180
   atgaaagtgc ctgaagcatc ggtgactagt ttagcttgtc ctaaatttgt accgattgtt 240
   gaaagtaatc aatttcaatc atcggtagct aaaaaaattg ttgctgagac gttattacca 300
   ttgcaacata aaaaattaga tacgttgatt ttagg 335
<210> 50
   <211> 111
   <212> PRT
   <213> E. saccharolyticus
<400> 50
   Ala Cys Asn Thr Ala Thr Ala Val Ala Leu Glu Glu Ile Lys Ala Gln
   1 5 10 15
   Leu Asp Ile Pro Val Val Gly Val Ile Leu Pro Gly Thr Arg Ala Ala
   20 25 30
   Val Lys Ala Thr Lys Asn Arg Gln Ile Gly Ile Ile Gly Thr Ala Gly
   35 40 45
   Thr Ile Lys Ser Ser Ser Tyr Glu Gln Ala Ile Lys Met Lys Val Pro
   50 55 60
   Glu Ala Ser Val Thr Ser Leu Ala Cys Pro Lys Phe Val Pro Ile Val
   65 70 75 80
   Glu Ser Asn Gln Phe Gln Ser Ser Val Ala Lys Lys Ile Val Ala Glu
   85 90 95
   Thr Leu Leu Pro Leu Gln His Lys Lys Leu Asp Thr Leu Ile Leu
   100 105 110
<210> 51
   <211> 344
   <212> DNA
   <213> E. mundtii
<400> 51
   gtaatcgcat gtaataccgc aactgcggtc gcattagaag aaatcaaagc aacactctcg 60
   attccagtga tcggtgtgat tttgccagga acgagagcgg cagtcaagca gacgaaaaat 120
   catcgagtag gggtgattgg aacaattggt accgtcaaaa gtgctgctta cgagacggca 180
   ttattggata aagcacccga actgaaagtt accagcttgg cgtgtccaaa gtttgtttca 240
   gtcgtagaaa gtaaagaata ccgatcatca gtcgctaaaa aaatcgtggc tcaaactttg 300
   cttccattag aattaaaagg gatcgatacg ttgattttag gttg 344
<210> 52
   <211> 113
   <212> PRT
   <213> E. mundtii
<400> 52
   Val Ile Ala Cys Asn Thr Ala Thr Ala Val Ala Leu Glu Glu Ile Lys
   1               5                   10                  15
   Ala Thr Leu Ser Ile Pro Val Ile Gly Val Ile Leu Pro Gly Thr Arg
               20                  25                  30
   Ala Ala Val Lys Gln Thr Lys Asn His Arg Val Gly Val Ile Gly Thr
           35                  40                  45
   Ile Gly Thr Val Lys Ser Ala Ala Tyr Glu Thr Ala Leu Leu Asp Lys
       50                  55                  60
   Ala Pro Glu Leu Lys Val Thr Ser Leu Ala Cys Pro Lys Phe Val Ser
   65                  70                  75                  80
   Val Val Glu Ser Lys Glu Tyr Arg Ser Ser Val Ala Lys Lys Ile Val
                   85                  90                  95
   Ala Gln Thr Leu Leu Pro Leu Glu Leu Lys Gly Ile Asp Thr Leu Ile
   100                 105                 110
   Leu
<210> 53
   <211> 340
   <212> DNA
   <213> E. casseliflavus
<400> 53
   atcgcatgta ataccgcgac agcggtcgcc cttgaagaaa tcaaagaaca actaacgatc 60
   ccagtgatcg gcgtgatcct gcctggcagt cgagcagcag tcaaagcaag caaaaaccaa 120
   cgaatcggtg tcatcgggac aaacggaacg atcaaaagtg actcttacaa gcgcgcgctt 180
   catggcaaag cgccccatgc gtccgtcgtc agtttggctt gcccgaagtt tgtgccgatc 240
   gtagaaagca aacaatacca tagctcggtc gccaagaaaa tcgtggcaga aacgttgcgt 300
   ccattgaaaa acaaacggct agatacgttg attttaggtg 340
<210> 54
   <211> 112
   <212> PRT
   <213> E. casseliflavus
<400> 54
   Ile Ala Cys Asn Thr Ala Thr Ala Val Ala Leu Glu Glu Ile Lys Glu
   1               5                   10                  15
   Gln Leu Thr Ile Pro Val Ile Gly Val Ile Leu Pro Gly Ser Arg Ala
               20                  25                  30
   Ala Val Lys Ala Ser Lys Asn Gln Arg Ile Gly Val Ile Gly Thr Asn
           35                  40                  45
   Gly Thr Ile Lys Ser Asp Ser Tyr Lys Arg Ala Leu His Gly Lys Ala
       50                  55                  60
   Pro His Ala Ser Val Val Ser Leu Ala Cys Pro Lys Phe Val Pro Ile
   65                  70                  75                  80
   Val Glu Ser Lys Gln Tyr His Ser Ser Val Ala Lys Lys Ile Val Ala
                   85                  90                  95
   Glu Thr Leu Arg Pro Leu Lys Asn Lys Arg Leu Asp Thr Leu Ile Leu
               100                 105                 110
<210> 55
   <211> 337
   <212> DNA
   <213> E. flavescens
<400> 55
   atcgcatgta ataccgcgac agcggtcgcc cttgaagaaa tcaaagaaca actaacgatc 60
<210> 56
   <211> 112
   <212> PRT
   <213> E. flavescens
<400> 56
   Ile Ala Cys Asn Thr Ala Thr Ala Val Ala Leu Glu Glu Ile Lys Glu
   1               5                   10                  15
   Gln Leu Thr Ile Pro Val Ile Gly Val Ile Leu Pro Gly Ser Arg Ala
               20                  25                  30
   Ala Val Lys Ala Ser Lys Asn Gln Arg Ile Gly Val Ile Gly Thr Asn
           35                  40                  45
   Gly Thr Ile Lys Ser Asp Ser Tyr Lys Arg Ala Leu His Gly Lys Ala
       50                  55                  60
   Pro His Ala Ser Val Val Ser Leu Ala Cys Pro Lys Phe Val Pro Ile
   65                  70                  75                  80
   Val Glu Ser Lys Gln Tyr His Ser Ser Val Ala Lys Lys Ile Val Ala
                   85                  90                  95
   Glu Thr Leu Arg Pro Leu Lys Asn Lys Arg Leu Asp Thr Leu Ile Leu
               100                 105                 110
<210> 57
   <211> 341
   <212> DNA
   <213> E. cecorum
<400> 57
   atcgcatgta ataccgcgac tgcagcagct ttaacccaaa ttaaggaaca attagacatt     60
   ccagttgtcg gtgtgatttt acctggaact agagctgctg tcaaaaatac aaaatcgcaa    120
   cgaattggga ttatcggcac acaaggaacc atccaaagtg gcagttatga acaagccatt    180
   ctttctaaag taccgactgc tcaacctgtg agtttagcgt gtcctagatt tgttccgata    240
   gtagaaagta atcaagcaaa ttcaagtgtg gcaaaaaaaa ttgtcgctca aacactacaa    300
   ccgatgacga aaaaaaacat cgatacgttg attttaggtt g                        341
<210> 58
   <211> 112
   <212> PRT
   <213> E. cecorum
<400> 58
   Ile Ala Cys Asn Thr Ala Thr Ala Ala Ala Leu Thr Gln Ile Lys Glu
   1 5 10 15
   Gln Leu Asp Ile Pro Val Val Gly Val Ile Leu Pro Gly Thr Arg Ala
   20 25 30
   Ala Val Lys Asn Thr Lys Ser Gln Arg Ile Gly Ile Ile Gly Thr Gln
   35 40 45
   Gly Thr Ile Gln Ser Gly Ser Tyr Glu Gln Ala Ile Leu Ser Lys Val
   50 55 60
   Pro Thr Ala Gln Pro Val Ser Leu Ala Cys Pro Arg Phe Val Pro Ile
   65 70 75 80
   Val Glu Ser Asn Gln Ala Asn Ser Ser Val Ala Lys Lys Ile Val Ala
   85 90 95
   Gln Thr Leu Gln Pro Met Thr Lys Lys Asn Ile Asp Thr Leu Ile Leu
   100 105 110
<210> 59
   <211> 339
   <212> DNA
   <213> E. raffinosus
<400> 59
   atcgcatgta ataccgcgac ggcagtagct ttggaagaaa ttaaaagaac cgtagatatt 60
   cccgtaatcg gtgttataca gccaggatct cgcgcagcgt taaaggcaag cgaaaatggg 120
   cgcgtgggaa ttatcggaac cattggaaca gtaaaaagtg gttcttataa acacgaacta 180
   caggaaaaag ctcctgatac ttatgtttct agtttagcat gcccaaaatt tgtaccgatt 240
   gttgaaagta atcaatttaa tagctcggta gcgaaaaaaa ttgtttctca aacattaact 300
   cctttgaaaa aggaaaagtt ggatacgttg attttaggt 339
<210> 60
   <211> 112
   <212> PRT
   <213> E. raffinosus
<400> 60
   Ile Ala Cys Asn Thr Ala Thr Ala Val Ala Leu Glu Glu Ile Lys Arg
   1 5 10 15
   Thr Val Asp Ile Pro Val Ile Gly Val Ile Gln Pro Gly Ser Arg Ala
   20 25 30
   Ala Leu Lys Ala Ser Glu Asn Gly Arg Val Gly Ile Ile Gly Thr Ile
           35                  40                  45
   Gly Thr Val Lys Ser Gly Ser Tyr Lys His Glu Leu Gln Glu Lys Ala
       50                  55                  60
   Pro Asp Thr Tyr Val Ser Ser Leu Ala Cys Pro Lys Phe Val Pro Ile
   65                  70                  75                  80
   Val Glu Ser Asn Gln Phe Asn Ser Ser Val Ala Lys Lys Ile Val Ser
                   85                  90                  95
   Gln Thr Leu Thr Pro Leu Lys Lys Glu Lys Leu Asp Thr Leu Ile Leu
               100                 105                 110
<210> 61
   <211> 341
   <212> DNA
   <213> E. malodoratus
<400> 61
   atcgcatgta ataccgcaac cgcagtggct ttagaagaga ttaagaagaa cgttgatatt      60
   cctgttattg gtgttatcca accaggatca cgtgctgcat taaaagcaag taaaaatagt     120
   cgtgtaggta tcatcggaac actaggaact gttaaaagtg gatcttataa acatgagctg     180
   caagaaaaag caccagaaac gtatgttgct agtctggcct gcccaaaatt tgtgccaatc     240
   gttgaaagta atcagtttaa tagttctgta gccaaaaaga ttgtttcaca atctctggca     300
   cccttaaaaa aggaaaaatt agatacgttg attttaggtt g                         341
<210> 62
   <211> 112
   <212> PRT
   <213> E. malodoratus
<400> 62
   Ile Ala Cys Asn Thr Ala Thr Ala Val Ala Leu Glu Glu Ile Lys Lys
   1               5                   10                  15
   Asn Val Asp Ile Pro Val Ile Gly Val Ile Gln Pro Gly Ser Arg Ala
               20                  25                  30
   Ala Leu Lys Ala Ser Lys Asn Ser Arg Val Gly Ile Ile Gly Thr Leu
           35                  40                  45
   Gly Thr Val Lys Ser Gly Ser Tyr Lys His Glu Leu Gln Glu Lys Ala
       50                  55                  60
   Pro Glu Thr Tyr Val Ala Ser Leu Ala Cys Pro Lys Phe Val Pro Ile
   65                  70                  75                  80
   Val Glu Ser Asn Gln Phe Asn Ser Ser Val Ala Lys Lys Ile Val Ser
                   85                  90                  95
   Gln Ser Leu Ala Pro Leu Lys Lys Glu Lys Leu Asp Thr Leu Ile Leu
               100                 105                 110
<210> 63
   <211> 338
   <212> DNA
   <213> E. solitarus
<400> 63
   gcatgtaata ccgcaacagc tgtggcttta gatgagatta aagagcaact gcaaatccct 60
   gttgtgggag ttattatgcc gggaaccaga gcagctgtta aagcgactaa aaatcatcgt 120
   attggtgtga ttggcacaaa aggaacagtt aaaagtgcct cttacaaacg agcaatcaaa 180
   gaaaaaaatg aaaatacaaa agtaacaagt ttggcttgtc cgaagtttgt tcccattgtg 240
   gaaagtaatc aaattcattc ttcagtggca aaaaaaattg tatttgaaac actattaccc 300
   ttaaaaaata aacatttaga tacgttgatt ttaggttg 338
<210> 64
   <211> 111
   <212> PRT
   <213> E. solitarus
<400> 64
   Ala Cys Asn Thr Ala Thr Ala Val Ala Leu Asp Glu Ile Lys Glu Gln
   1               5                   10                  15
   Leu Gln Ile Pro Val Val Gly Val Ile Met Pro Gly Thr Arg Ala Ala
               20                  25                  30
   Val Lys Ala Thr Lys Asn His Arg Ile Gly Val Ile Gly Thr Lys Gly
           35                  40                  45
   Thr Val Lys Ser Ala Ser Tyr Lys Arg Ala Ile Lys Glu Lys Asn Glu
       50                  55                  60
   Asn Thr Lys Val Thr Ser Leu Ala Cys Pro Lys Phe Val Pro Ile Val
   65                  70                  75                  80
   Glu Ser Asn Gln Ile His Ser Ser Val Ala Lys Lys Ile Val Phe Glu
                   85                  90                  95
   Thr Leu Leu Pro Leu Lys Asn Lys His Leu Asp Thr Leu Ile Leu
               100                 105                 110
<210> 65
   <211> 341
   <212> DNA
   <213> E. hirae
<400> 65
   atcgcatgta ataccgctac tgcggttgct ttagaagaaa tcaaggcggc acttcctatt 60
   ccagtcattg gtgtgatctt acctgggaca agagcagctg ttaaacaaac aagaaataaa 120
   caagtaggga ttatcggaac cctcggaacg atcaaaagtc gtgcttatga aacagcgctg 180
   aaaacgaagg tacctgaact tgccgtgact agtttggctt gtccaaaatt cgtttcggta 240
   gtggaaagta atgaatatca ttcgtcagtg gcaaaaaaaa tcgttgccca gacactagcg 300
   ccattggtta ctaagaaaat cgatacgttg attttaggtt g 341
<210> 66
   <211> 111
   <212> PRT
   <213> E. hirae
<400> 66
   Ala Cys Asn Thr Ala Thr Ala Val Ala Leu Glu Glu Ile Lys Ala Ala
   1 5 10 15
   Leu Pro Ile Pro Val Ile Gly Val Ile Leu Pro Gly Thr Arg Ala Ala
   20 25 30
   Val Lys Gln Thr Arg Asn Lys Gln Val Gly Ile Ile Gly Thr Leu Gly
   35 40 45
   Thr Ile Lys Ser Arg Ala Tyr Glu Thr Ala Leu Lys Thr Lys Val Pro
   50 55 60
   Glu Leu Ala Val Thr Ser Leu Ala Cys Pro Lys Phe Val Ser Val Val
   65 70 75 80
   Glu Ser Asn Glu Tyr His Ser Ser Val Ala Lys Lys Ile Val Ala Gln
   85 90 95
   Thr Leu Ala Pro Leu Val Thr Lys Lys Ile Asp Thr Leu Ile Leu
   100 105 110
<210> 67
   <211> 29
   <212> DNA
   <213> Artificial Sequence
<220>
   <223> Homo sapiens
<400> 67
   aaatagtcat atgaaaatag gcgtttttg 29
<210> 68
   <211> 28
   <212> DNA
   <213> Artificial Sequence
<220>
   <223> Homo sapiens
<400> 68 28
   agaattctat tacaatttga gccattct 28
<210> 69
   <211> 26
   <212> DNA
   <213> Artificial sequence
<220>
   <223> Homo sapiens
<400> 69 26
   gcgaattcga tcagaatttt ttttct 26
<210> 70
   <211> 26
   <212> DNA
   <213> Artificial Sequence
<220>
   <223> Homo sapiens
<400> 70
   ataagtactt gtgaatctta tactag 26
<210> 71
   <211> 29
   <212> DNA
   <213> Artificial sequence
<220>
   <223> Homo sapiens
<400> 71
   aaaatgctag taatcgcatg taataccgc 29
<210> 72
   <211> 26
   <212> DNA
   <213> Artificial Sequence
<220>
   <223> Homo sapiens
<400> 72
   tgggtacaac ctaaaatcaa cgtatc 26
<210> 73
   <211> 765
   <212> DNA
   <213> Aquifex pyrophilus NA sequence
<400> 73
   atgaagatag gtatctttga cagtggtgtg gggggactta ctgttctaaa ggctataaga 60
   aatagataca gaaaggttga tatagtatac ctcggtgata ccgcaagggt tccctacggc 120
   ataaggtcta aagatacgat aatcagatac tcccttgagt gtgcgggctt tttaaaggat 180
   aagggtgttg atataatcgt cgttgcctgc aataccgcaa gtgcttacgc tcttgaacgt 240
   ttaaagaaag agataaacgt tcccgttttc ggcgttattg aacccggggt taaagaagcc    300
   ttaaaaaagt caaggaataa aaaaatagga gttataggaa ctcctgcaac cgtaaaaagc    360
   ggagcctacc agagaaagct tgaagagggg ggagctgatg tttttgcaaa ggcctgtccc    420
   ctattcgttc cccttgcgga ggaaggtctc cttgaggggg agataacaag aaaggttgta    480
   gaacactacc ttaaggagtt taaaggtaag attgatactc tgattttagg atgtacccat    540
   tacccccttc ttaaaaagga gataaagaag tttttgggag acgttgaagt cgttgactct    600
   tccgaagccc tttccctttc cctccataac tttataaagg acgatgggtc ctcatccctt    660
   gagttatttt ttacggacct ttccccaaat ctccagtttt tgattaaatt aatactcggt    720
   agggattacc cggtaaaact tgcggagggg gtttttacac attaa                    765
<210> 74
   <211> 262
   <212> PRT
   <213> Aquifex pyrophilus amino acid sequence
<400> 74
   Met Lys Ile Gly Ile Phe Asp Ser Gly Val Gly Gly Leu Thr Val Leu
   1               5                   10                  15
   Lys Ala Ile Arg Asn Arg Tyr Arg Lys Val Asp Ile Val Tyr Leu Gly
               20                  25                  30
   Asp Thr Ala Arg Val Pro Tyr Gly Ile Arg Ser Lys Asp Phe Thr Thr
           35                  40                  45
   Ile Ile Arg Tyr Ser Leu Glu Cys Ala Gly Phe Leu Lys Asp Lys Gly
       50                  55                  60
   Val Asp Ile Ile Val Val Ala Cys Asn Thr Ala Ser Ala Tyr Ala Leu
   65                  70                  75                  80
   Glu Arg Leu Lys Lys Glu Ile Asn Val Pro Val Phe Gly Val Ile Glu
                   85                  90                  95
   Pro Gly Val Lys Glu Ala Leu Lys Lys Ser Phe Thr Arg Asn Lys Lys
               100                 105                 110
   Ile Gly Val Ile Gly Thr Pro Ala Thr Val Lys Ser Gly Ala Tyr Gln
           115                 120                 125
   Arg Lys Leu Glu Glu Gly Gly Ala Asp Val Phe Ala Lys Ala Cys Pro
       130                 135                 140
   Leu Phe Val Pro Leu Ala Glu Glu Gly Leu Leu Glu Gly Glu Ile Thr
   145                 150                 155                 160
   Arg Lys Val Val Glu His Tyr Phe Thr Leu Lys Glu Phe Lys Gly Lys
   165 170 175
   Ile Asp Thr Leu Ile Leu Gly Cys Thr His Tyr Pro Leu Leu Lys Lys
   180 185 190
   Glu Ile Lys Lys Phe Leu Gly Asp Val Glu Val Val Asp Ser Ser Glu
   195 200 205
   Ala Leu Ser Leu Ser Leu His Asn Phe Ile Lys Asp Asp Gly Ser Ser
   210 215 220
   Ser Leu Glu Leu Phe Thr Phe Phe Thr Asp Leu Ser Pro Asn Leu Gln
   225 230 235 240
   Phe Leu Ile Lys Leu Ile Leu Gly Arg Asp Tyr Pro Val Lys Leu Ala
   245 250 255
   Glu Gly Val Phe Thr His
   260
<210> 75
   <211> 19
   <212> DNA
   <213> Homo sapiens
<400> 75
   tgatgcaaca aatggacga 19
<210> 76
   <211> 18
   <212> DNA
   <213> Homo sapiens
<400> 76
   ttacaatttg agccattc
   18

## Description

### BACKGROUND OF THE INVENTION

Certain species of Gram negative bacteria are important human pathogens, and the prevalence of their association with human disease is increasing. Extensive antibiotic resistance has developed in gram-negative bacteria through three basic mechanisms, alteration of drug target, drug inactivation, and thirdly, reduction of cell membrane permeability either due to altered porins or the acquisition or induction of efflux pumps (Waterer, *Ibid.).* Species such as *Pseudomonas aeruginosa, Acinetobacter* spp., *Stenotrophomonas maltophila,* and members of the *Enterobacteriaceae* are particularly problematic in intensive care units (Waterer and Wunderink, Crit. Care Med. 29: 75-81 (2001)).
Chronic pulmonary infection with *Pseudomonas aeruginosa* is the major cause of lung function decline and mortality in cystic fibrosis patients and is also a major problem in severe burn victims (Lyczak et al. Clin. Microbiol. Rev., 15: 194-222 (2002); and Lyczak et al. Microbes Infect. 2: 1051-1060 (2000)).

The microbial etiology of urinary tract infections has been well established. *Escherichia coli* remains the predominant uropathogen (80%) isolated in acute community-acquired uncomplicated infections, followed by *Staphylococcus saprophyticus* (10-15%), *Klebsiella, Enterobacter,* and *Proteus* species (Ronald, A. Am. J. Med. 8; 113 (Suppl.) 1A:14S-19S (2002)).

Certain species of Gram positive bacteria are important human pathogens and the recent development of broad spectrum antibiotic resistance among these organisms has been identified as a critical human health issue (McDevitt and Rosenberg, Trends in Microbiol. 9: 611-617 (2001)). Members of the Enterococci, including *Enterococcus faecalis* and *Enterococcus faecium,* are agents of endocarditis, and urinary tract, bloodstream, and wound infection (Harbath et al. Antimicrob. Agents Chemo. 46: 1619-1628 (2002)). Species such as *Staphylococcus aureus, Streptococcus pneumoniae,* and *Streptococcus pyogenes* are major causes of respiratory tract infections, including sinusitis, otitis media, bacterial meningitis and community-acquired pneumonia and represent a leading cause of morbidity and mortality world-wide (Paradisi et al. Clin. Micro. Inf. 7(Supp14): 34-42 (2001); and McIntosh, K N. Engl. J. Med., 346: 429-437 (2002)). The development of resistance to current, commonly used antibiotics has risen steadily over the past two decades with rates exceeding 60-80% in some countries (Applebaum, P.C. Clin. Infect. Diseases, 34: 1613-1620 (2002)). The prevalence of these resistant organisms has limited treatment options in the clinic and the use of "last resort" antibiotics such as vancomycin has increased dramatically as a result. The emergence of Enterococci species that harbor resistance genes capable of high level glycopeptide resistance (Harbath et al. *Ibid*; and Linares, H. Clin. Micro. Inf. 7(Suppl. 4): 8-15 (2001)) has demonstrated that Gram positive pathogens are capable of developing resistance to all known therapies and that future infections may be untreatable without the development of new antibacterial agents.

*Helicobacter (H.) pylori* infections are one of the most common bacterial infections in humans and are the causative agent of peptic ulcers, gastric MALT lymphoma, dyspepsia, gastroesophageal reflux disease, and other diseases of the upper gastrointestinal tract, and has been linked to the development of gastric adenocarcinoma. Establishment of the bacteria in the upper gastrointestinal tract causes continuous gastric inflammation and induces a vigorous systemic and mucosal humoral response which causes tissue damage, but does not eradicate the bacteria (Sauerbaum and Michetti, N. Engl. J. Med. 347(15): 1175-1186 (2002)). An approach to disabling or killing *H pylori* would be beneficial.

Hwang et al. (Nature Structural Biology, 6(5): 422-426 (1999)) report the crystal structure of MurI from *Aquifex pyrophilus,* determined at 2.3 Å resolution.

### SUMMARY OF THE INVENTION

The structure of bacteria includes a peptidoglycan layer, located between the cytoplasmic and outer membranes of the cell wall, which is crucial to the structural integrity. Peptidoglycan is a large polymer, an essential component of which is the D amino acid, D-glutamate. The glutamate racemase enzyme (MurI) catalyzes the reversible interconversion of L-glutamate to D-glutamate and, thus, plays an important role in cell wall synthesis and bacterial growth. Because peptidoglycan is unique to bacteria, it and the enzymes involved in its biosynthesis are of interest as targets for designing or identifying antibacterial drugs.

Described herein are the three-dimensional structure of MurI (MurI) from Gram negative, Gram positive, and atypical bacteria such as *Escherichia (E.) coli; Enterococcus (E.) faecalis; Enterococcus (E.) faecium; Staphylococcus (S.) aureus;* and *Helicobacter (H.) pylori;* binding domains of MurI; conserved sequences of MurI; methods of identifying or designing agents that bind MurI (e.g., binding agents, ligands, drugs, or inhibitors that partially or totally inhibit MurI activity, proteins, small organic molecules); methods of crystallizing MurI; computer-assisted methods of identifying, screening, and/or designing agents that bind MurI; the use of the crystals in the preparation of a medicament for the treatment of bacterial infections, pharmaceutical compositions and packages; methods of treating bacterial infections in subjects comprising administering inhibitors of MurI; and methods of conducting business.

Gram negative bacteria include, for example, Escherichia species, *Haemophilus influenzae, Klebsiella pneumoniae, Moraxella catarrhalis, Vibrio cholerae, Proteus mirablis, Pasteruella multocida, Acinetobacter baumanii, Bacteroides fragilis,* Treponemal species, Borrelial species, Deinococcal species, Pseudomonas species, Salmonella species, Shigella species, Yersinia species and *Porphyromonas gingivalis.*

Gram positive bacteria include the bacteria of Bacillus species, Staphylococcal spp., Streptococcal species, Enterococcal species, Lactobacilli, Pediococci, and Mycobacterial species. More specifically, Gram positive bacteria include, for example, *B. subtilis, S. aureus, E. faecalis,* and *E. faecium.*

Atypical bacteria include, for example, Helicobacter species, *Campylobacter jejuni,* and *Aquifex aeolicus.*

### BRIEF DESCRIPTION OF THE FIGURES

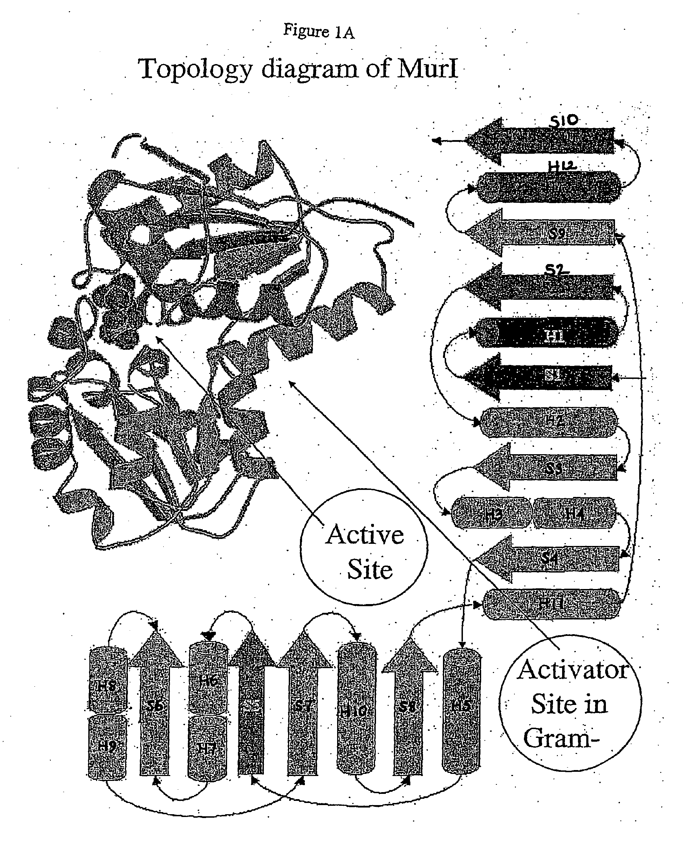

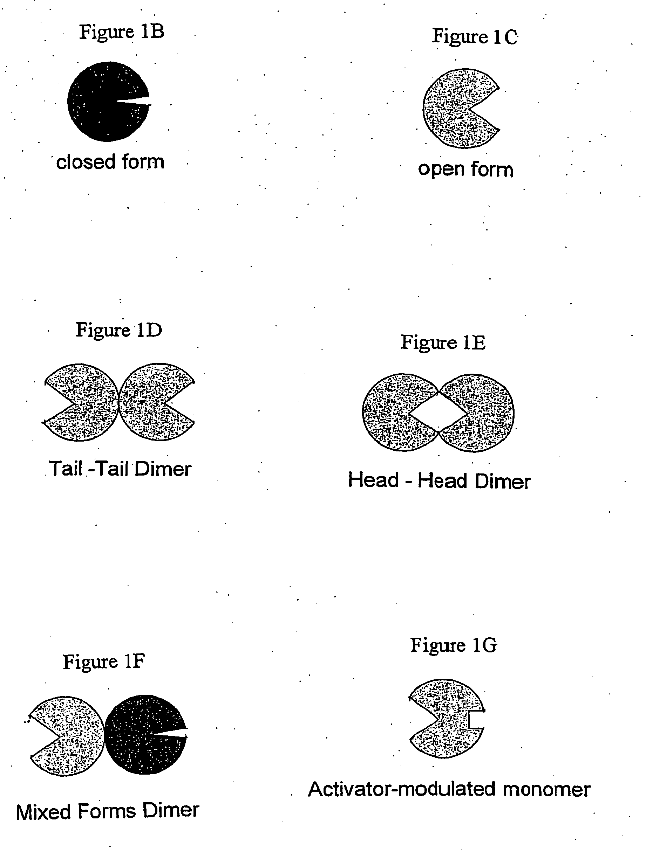

Figures 1A-1H present topology of the MurI fold and various conformations of the enzyme. Figure 1A illustrates the three dimensional structure of one domain of MurI depicting structural elements and illustrates the topology of the MurI fold. Figures 1B and 1C provide a cartoon depiction of MurI in an open (1B; black) and closed (1C; gray) conformations. Figures 1D and 1F depict the tail-tail structure of Gram positive MurI in different conformations. Figure 1E depicts the head-head structure of atypical MurI. Figure 1G depicts the structure of Gram negative MurI having both a substrate binding site (left side of 1G) and an activator binding site (right side of 1G).
Figure 2A-2O depict the structural elements which are conserved in MurI across all MurI, across Gram-positive and atypical bacteria, or all Gram-positive bacteria by specific amino acid residues. Structural motifs such as helices (H) beta sheets (E), loops (S) and turns (T), are indicated.
Figure 3 shows the distance (Å) between the active site cysteines of Murl crystal structures in different conformations. Measures of conformational movement are shown in columns 3-5, and the angles associated with conformational movement are shown in columns 6-7.

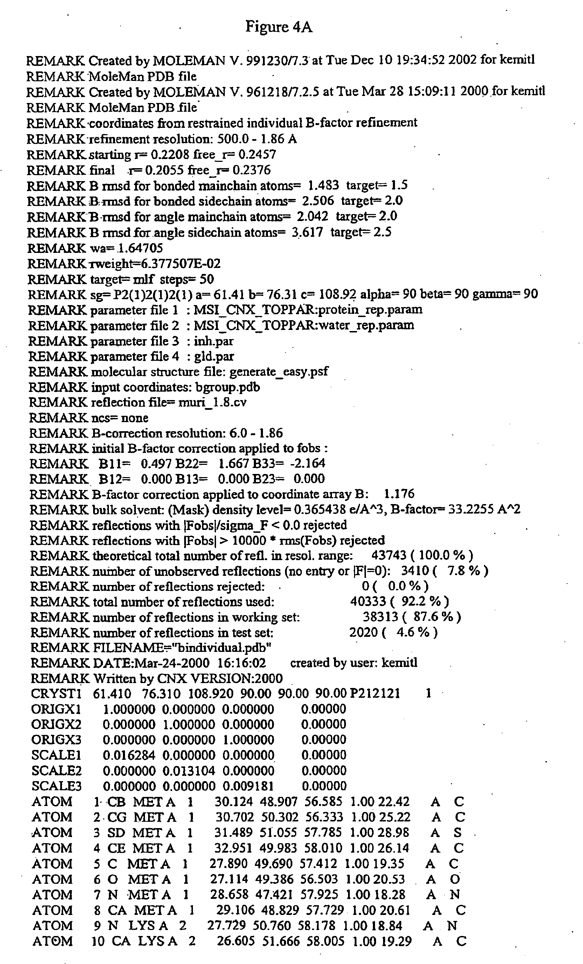

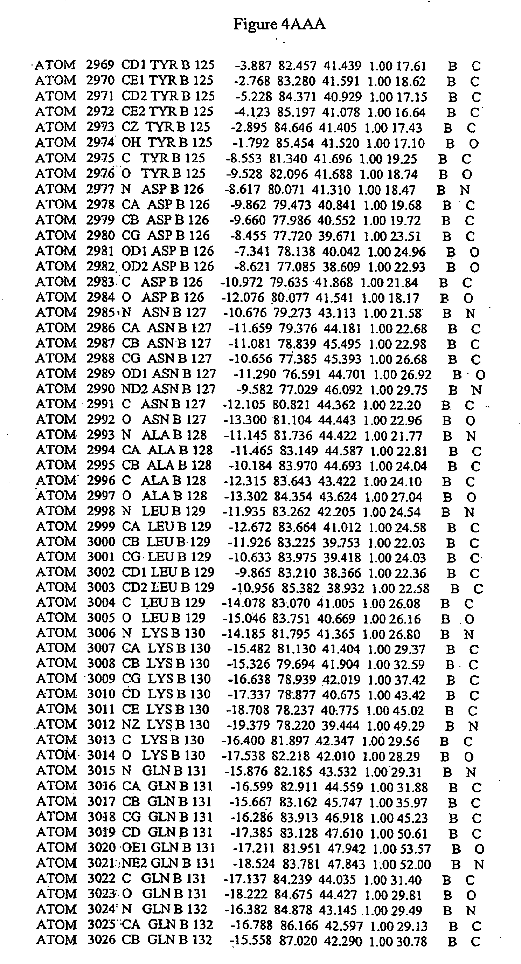

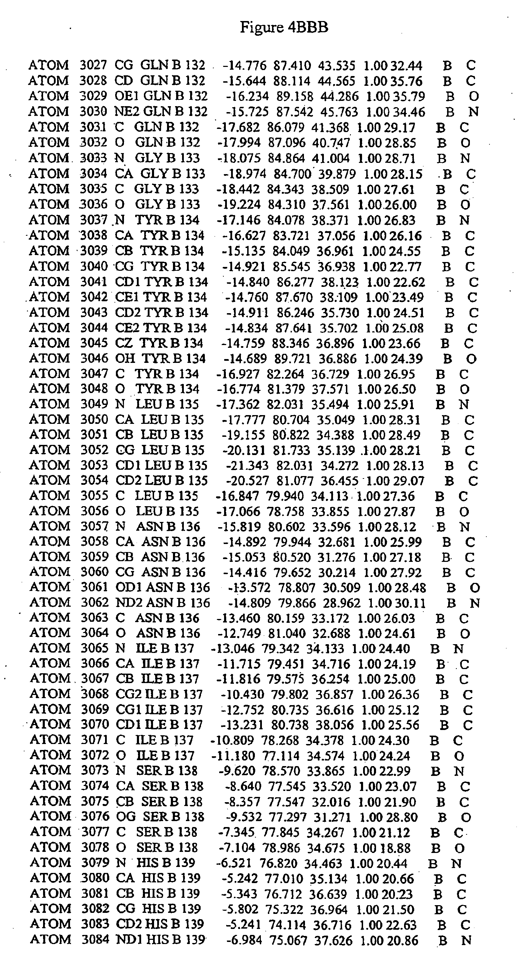

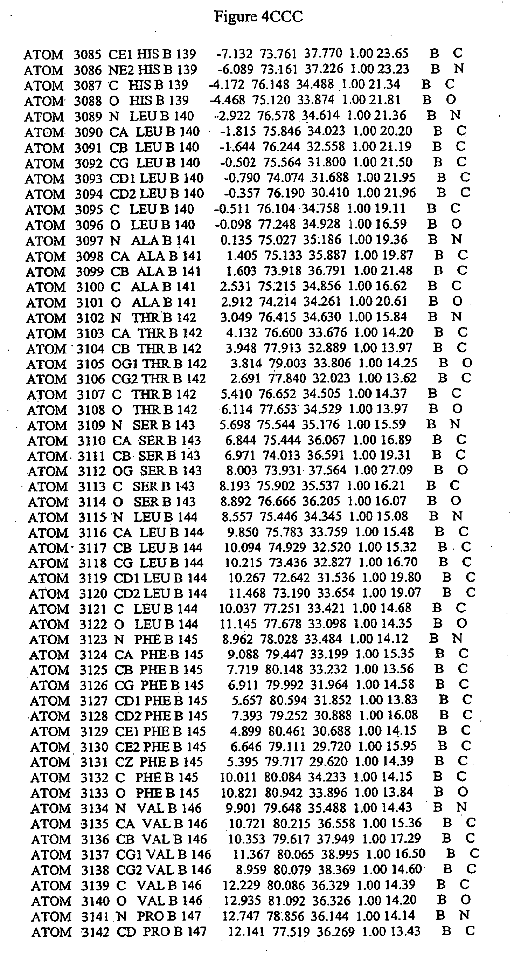

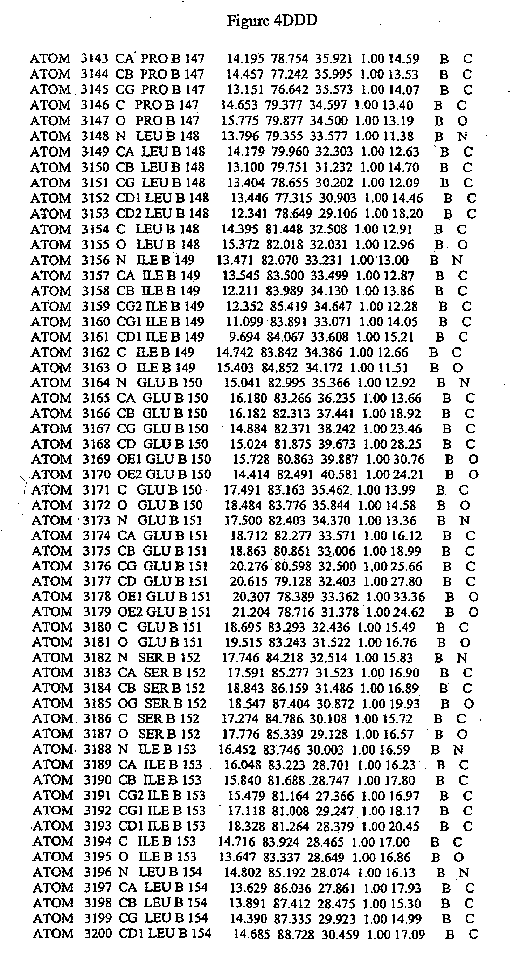

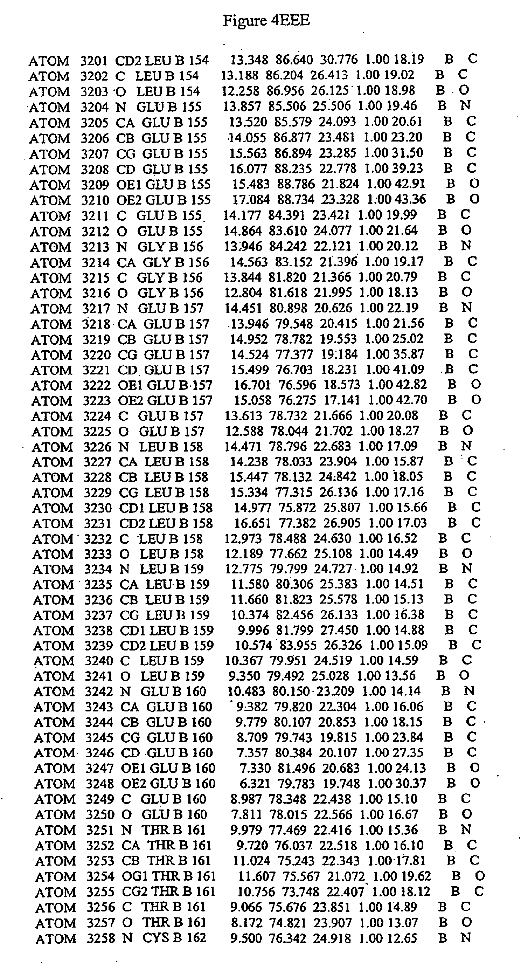

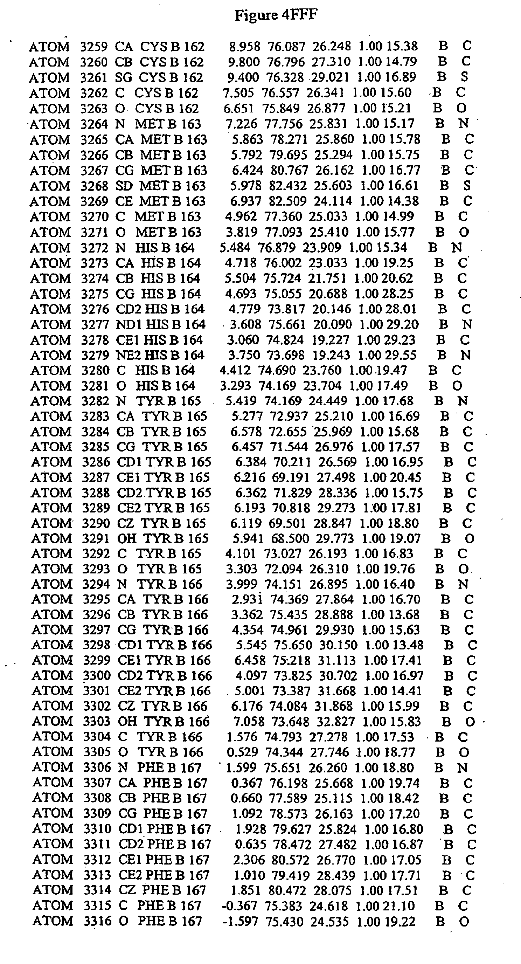

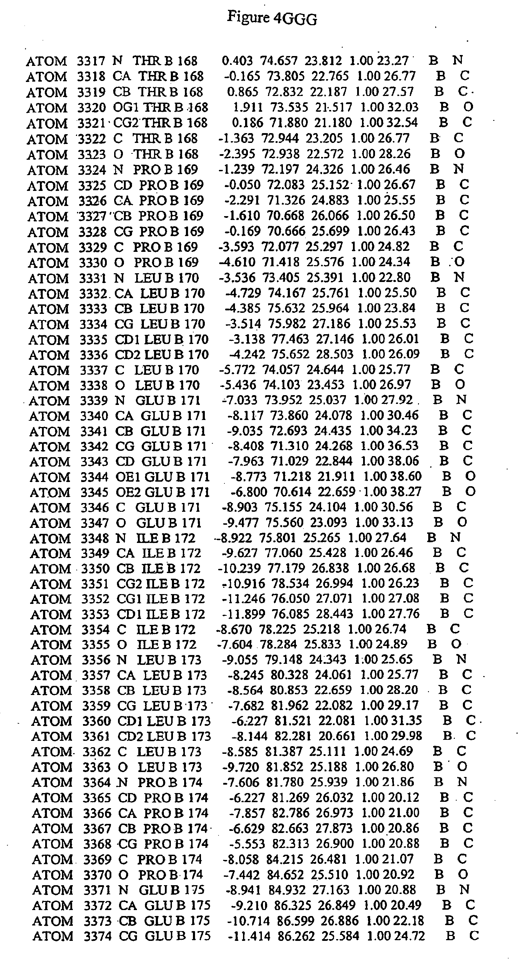

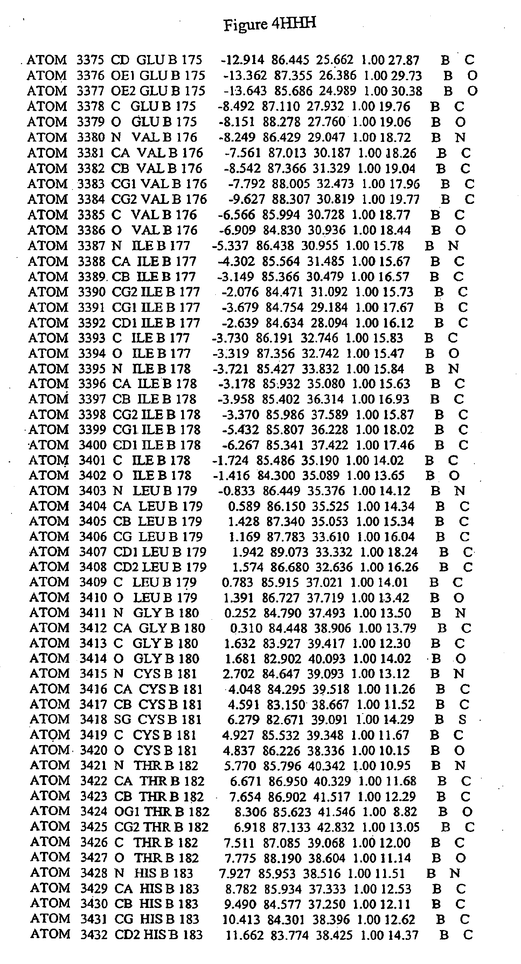

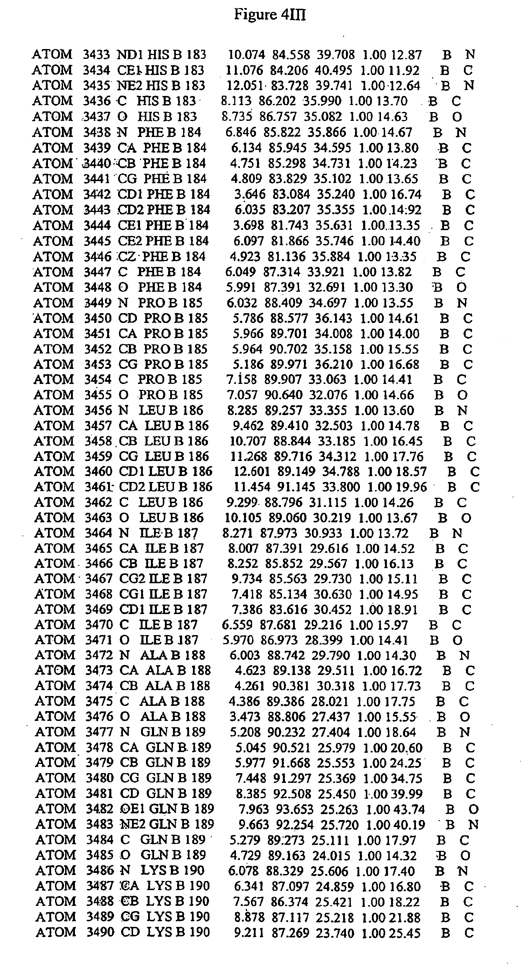

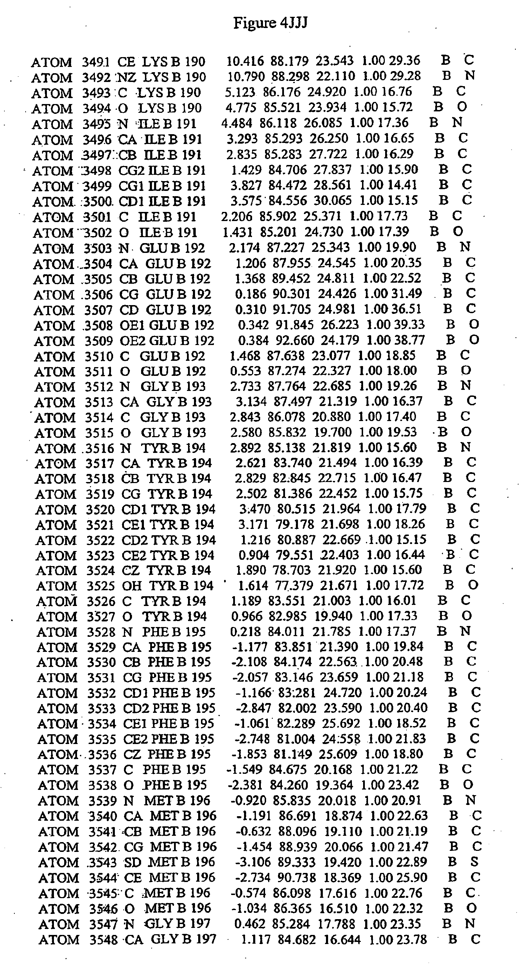

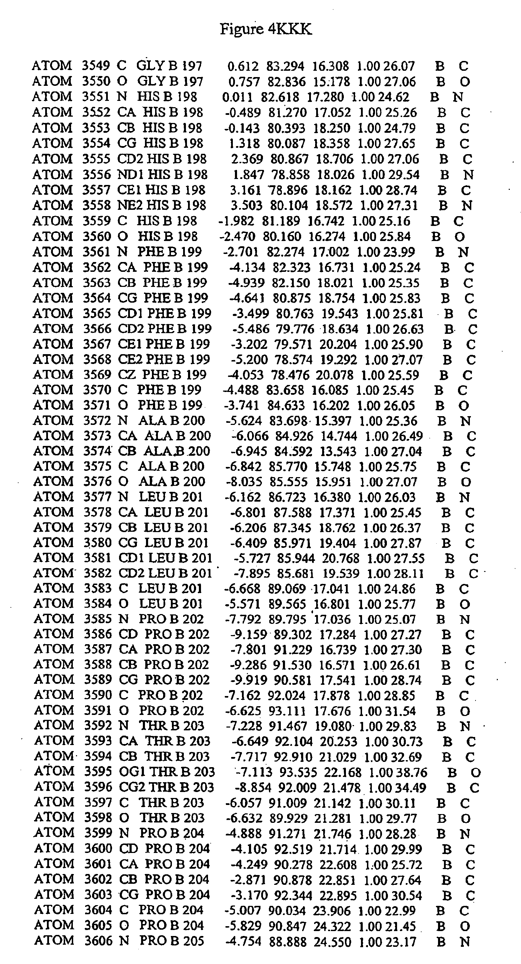

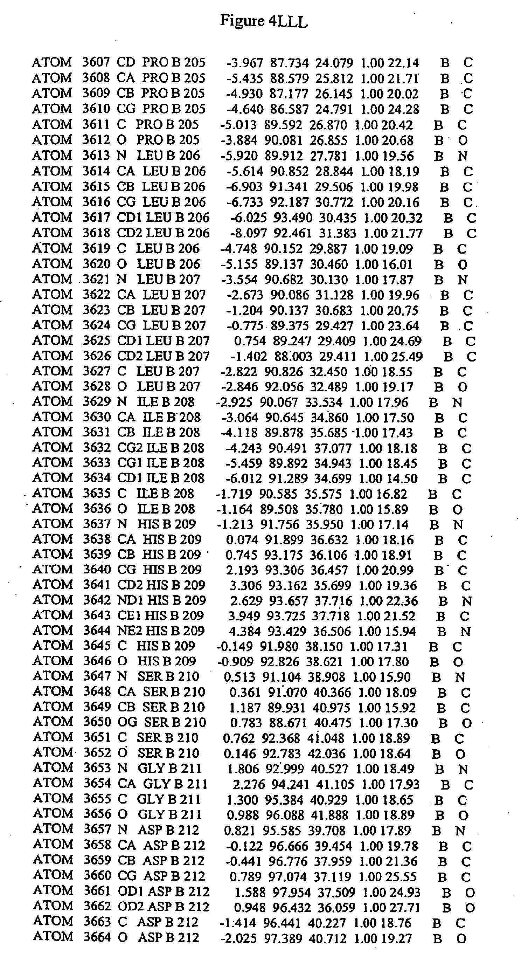

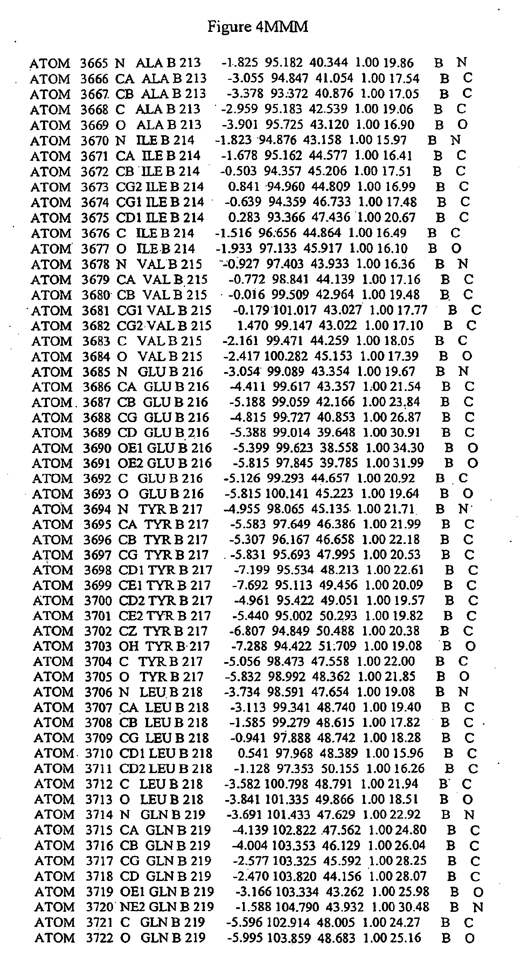

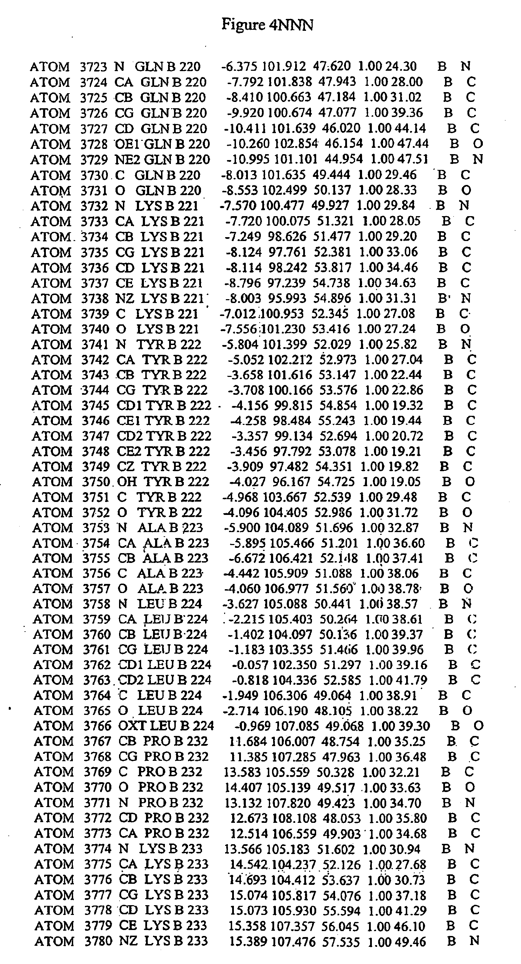

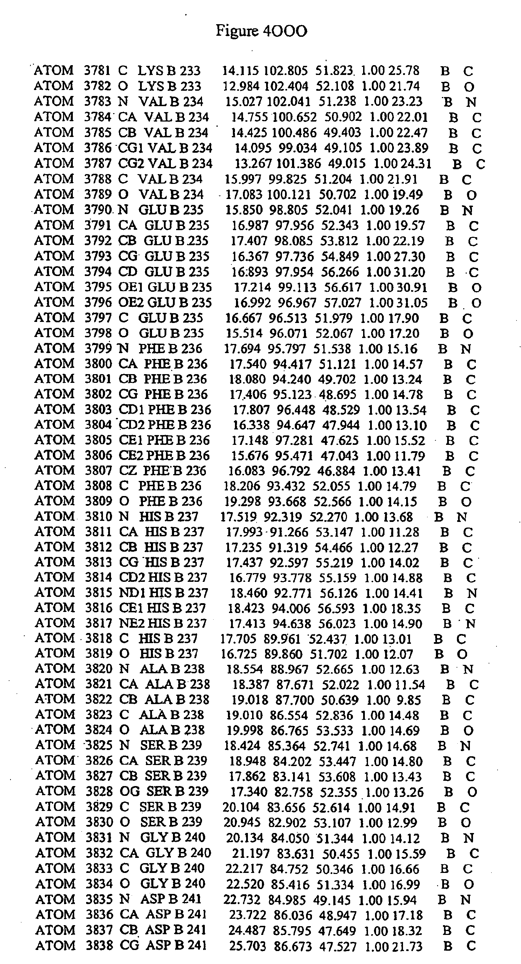

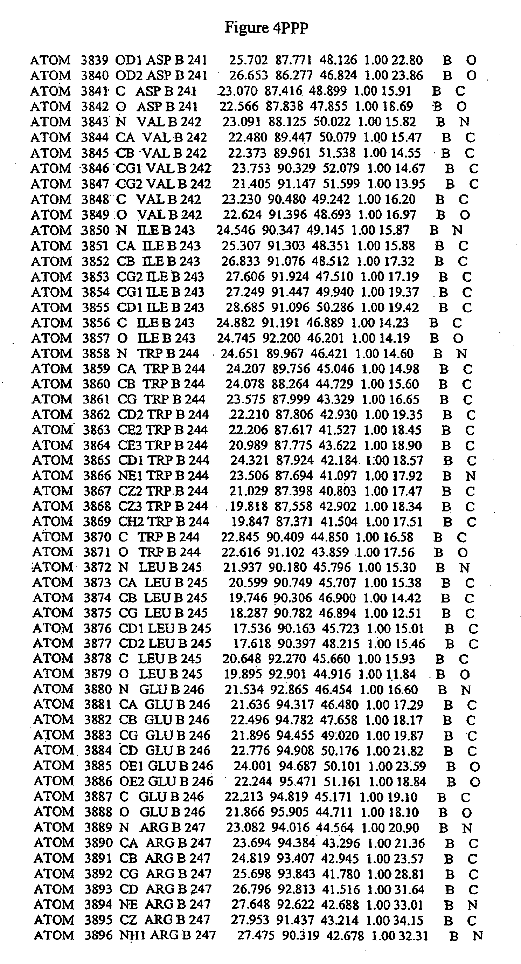

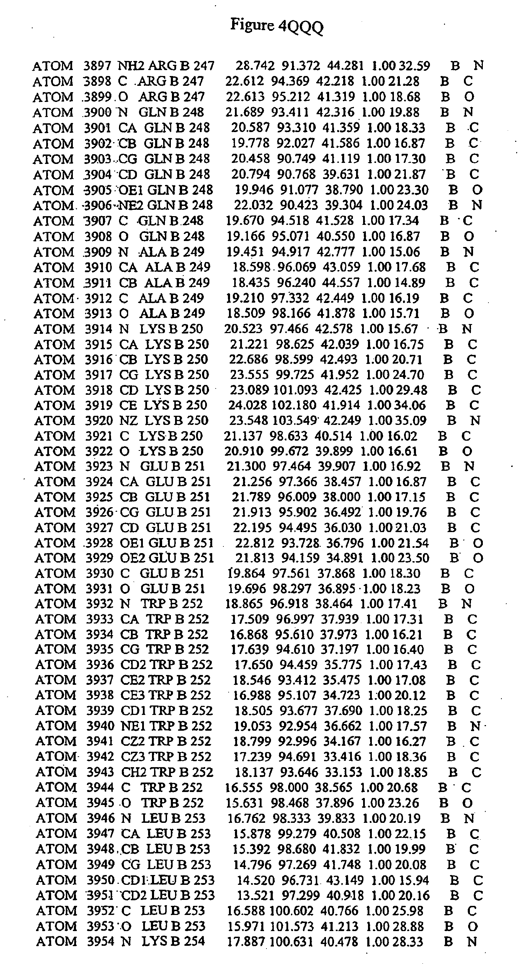

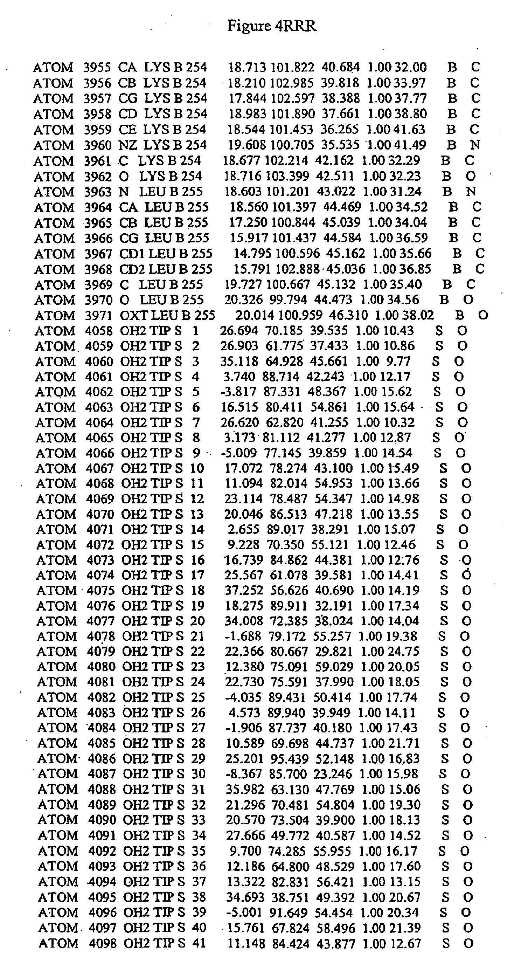

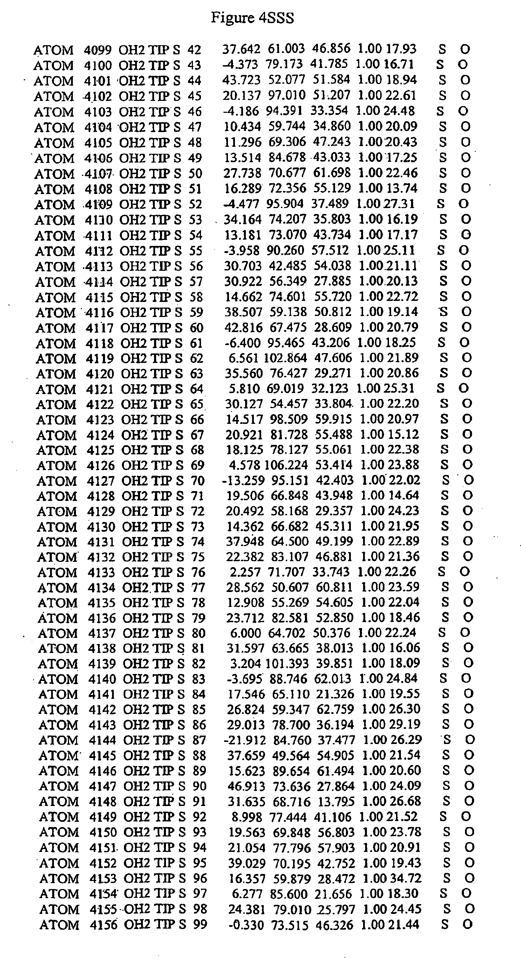

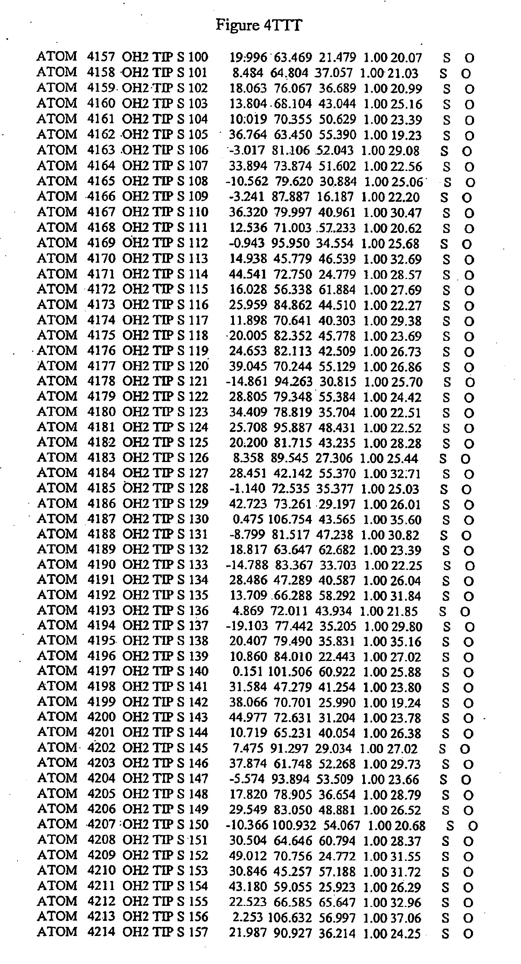

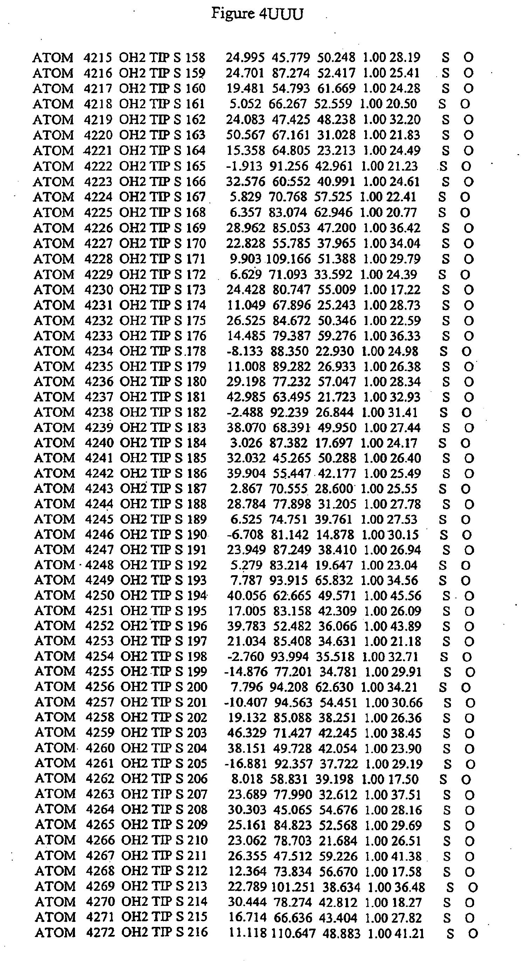

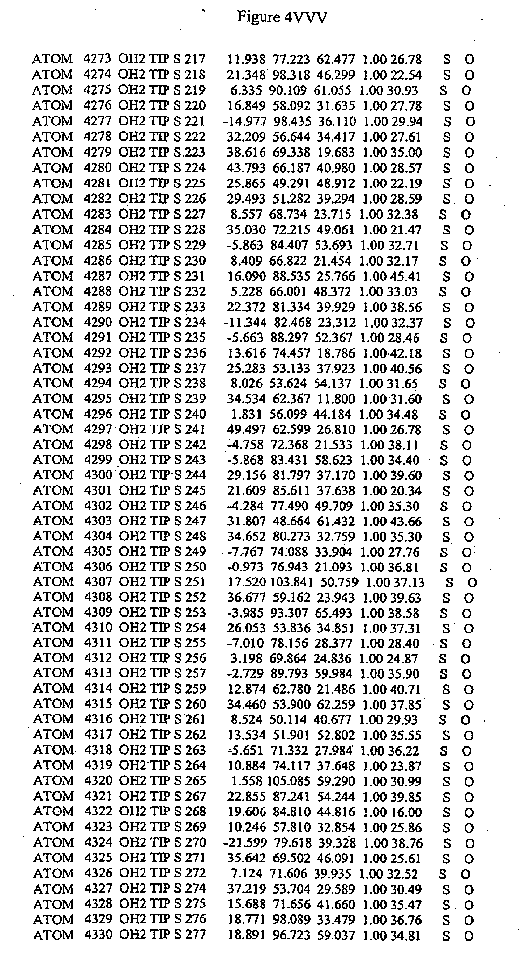

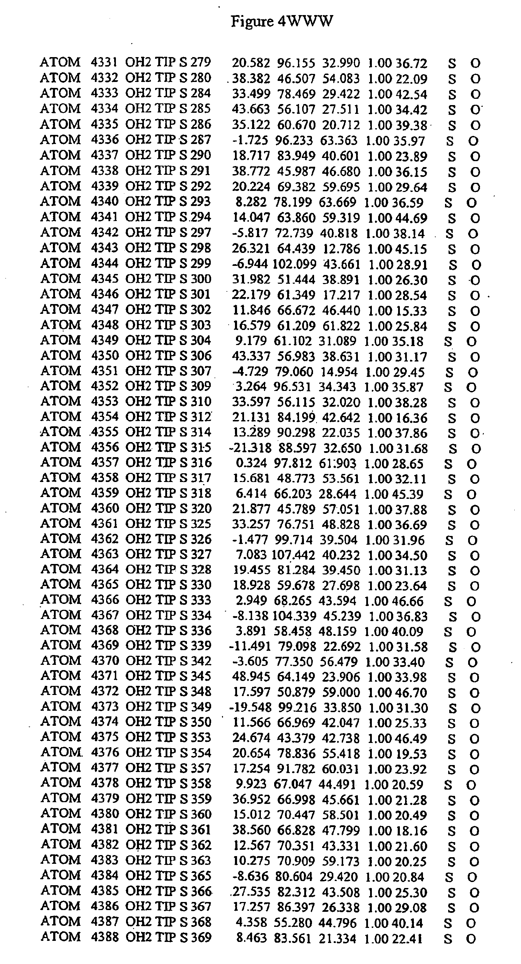

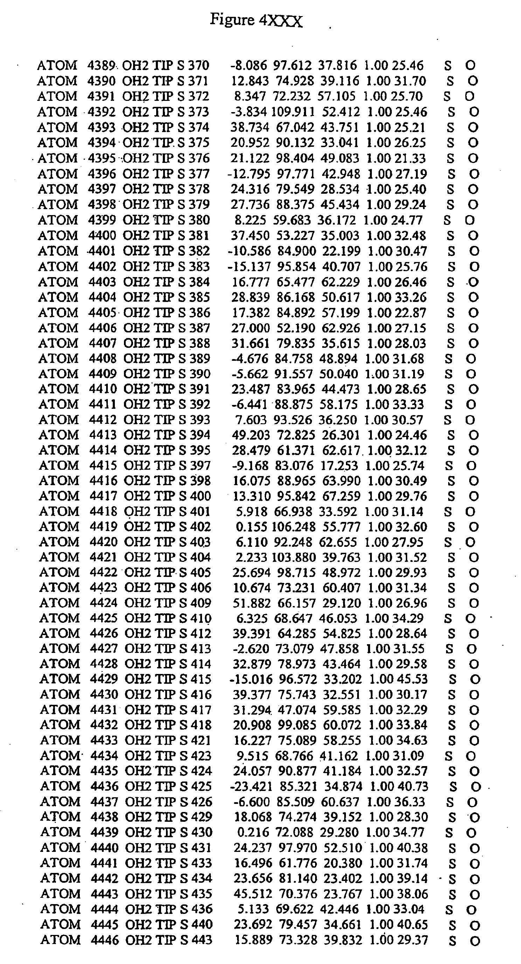

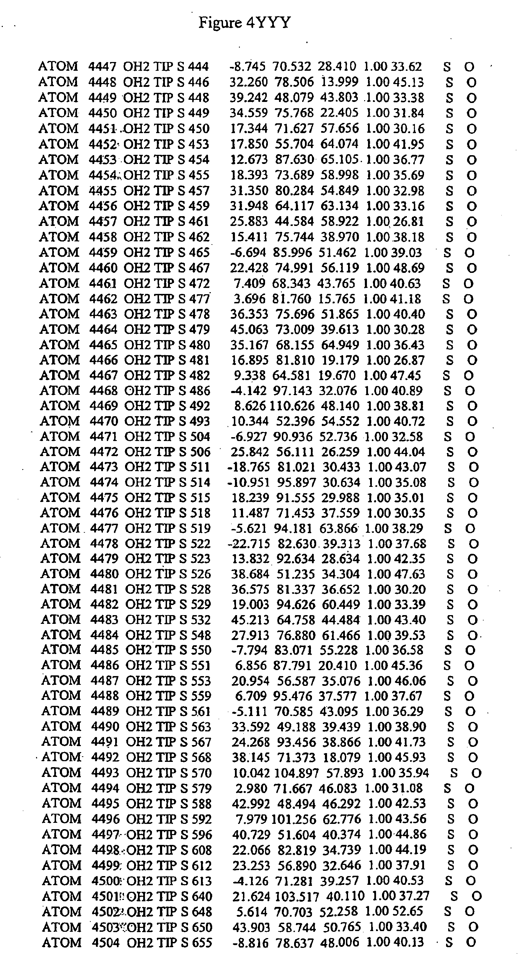

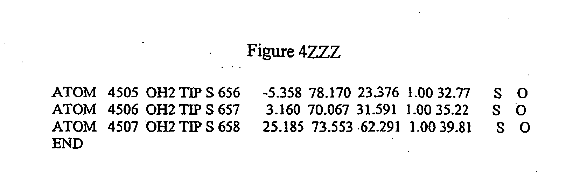

Figure 4A-4ZZZ show the listing of the three-dimensional atomic coordinates of a model derived from a *H. pylori* MurI (MurI) crystal structure. The coordinates presented are those of both chains of the dimeric protein. In the figures, the atom listing is preceded by the heading CRYST1, which is followed by the 3 dimensions (see Figure 4A) of the crystallographic unit cell. The next three values define a matrix which converts co-ordinates from orthogonal Angstrom coordinates to fractional coordinates of the unit cell. Each row labeled ATOM gives the (arbitrary) atom number, the label given to each amino acid main chain, each atom type, the amino acid residue type, the protein chain label (A comprises the first molecule (chain) and B comprises the second molecule (chain)), and the amino acid residue number. The first three numbers in the row give the orthogonal X, Y, Z coordinates of the atom. The next number is an occupancy number and would be less than 1.0 if the atom could be seen in more than one position (the amino acid could be seen in more than one orientation). The final number is a temperature factor which relates to the thermal amplitude of vibrations of the atom. At the end of the listing, there are lines of data indicating the ordered water molecules (TIP or WAT) included in the model.
Figures 5A-5ZZZ show the listing of the three-dimensional atomic coordinates of the crystal structure of MurI (MurI) from *H. pylori* complexed with D-glutamate.
Figures 6A-6AAAA show the listing of the three-dimensional coordinates of the crystal structure of MurI from *H. pylori* complexed with glutamate and the pyrimidinedione inhibitor, compound A.
Figures 7A-7AAAA show the listing of the three-dimensional coordinates of the crystal structure of MurI from *H. pylori* complexed with the pyrimidinedione inhibitor, compound A.
Figures 8A-8OO show the listing of the three-dimensional atomic coordinates of a *E. coli* MurI (MurI) crystal structure complexed with glutamate.
Figures 9A-9OO show the listing of the three-dimensional coordinates of the crystal structure of MurI (MurI) from *E*. *coli* complexed with activator.
Figures 10A-10NN show the listing of the three-dimensional coordinates of a model derived from a crystal structure of MurI (MurI) from *E*. *coli.*
Figures 11A-11OO show the listing of the three-dimensional coordinates of the crystal structure of MurI (MurI) from *E*. *coli* complexed with glutamate and activator.
Figures 12A-12OOO show the listing of the three-dimensional coordinates of a model derived from a crystal structure of MurI (MurI) from *E. faecalis.* The coordinates are both chains of the dimeric protein.
Figures 13A-13OOO show the listing of the three-dimensional coordinates of crystal structure of MurI (MurI) from *E. faecalis* complexed with D,L-glutamate.
Figures 14A-14MMM show the listing of the three-dimensional coordinates of a model derived from a crystal structure of MurI (MurI) from *S*. *aureus.*
Figures 15A-15MMM show the listing of the three-dimensional coordinates of the crystal structure of MurI (MurI) from *S*. *aureus* complexed with D-glutamate.
Figures 16A-16JJ show the listing of the three-dimensional coordinates of a model derived from a crystal structure of MurI (MurI) from *E. faecium.*
Figures 17A-17II show the listing of the three-dimensional coordinates of the crystal structure of MurI (Murl) from *E. faecium* complexed with tartrate.
Figures 18A-18JJ show the listing of the three-dimensional coordinates of the crystal structure of MurI (MurI) from *E. faecium* complexed with citrate.
Figures 19A-19JJ show the listing of the three-dimensional coordinates of the crystal structure of MurI (MurI) from *E.faecium* complexed with phosphate.

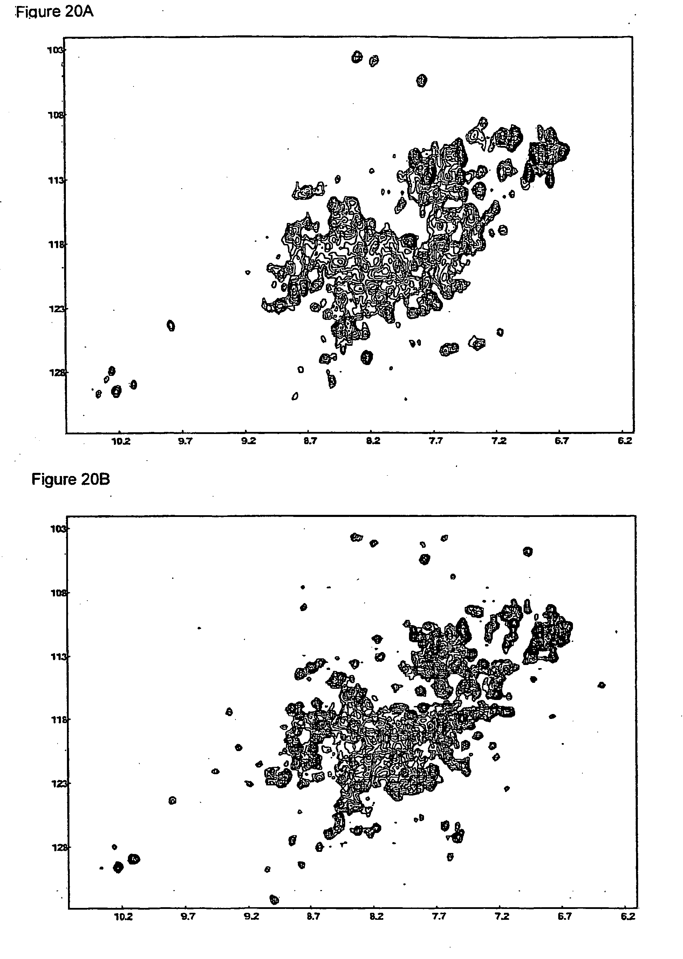

Figure 20A provides a nuclear magnetic resonance (NMR) [¹⁵N, ¹H] correlation spectrum of 0.3 mM ¹⁵N-labeled *H. pylori* MurI protein.
Figure 20B provides a nuclear magnetic resonance (NMR) [¹⁵N, ¹H] correlation spectrum of 0.3 mM ¹⁵N-labeled *H. pylori* MurI protein, to which 0.6 mM D-glutamate was added.

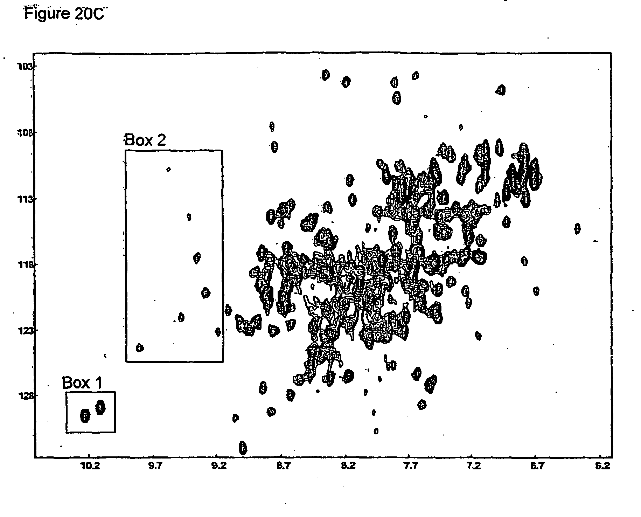

Figure 20C provides a nuclear magnetic resonance (NMR) [¹⁵N, ¹H] correlation spectrum of 0.3 mM ¹⁵N-Iabeled *H. pylori* MurI protein, to which 1.8 mM D-glutamate was added. Box 1 shows the two tryptophan side chain amide groups. In this spectrum with saturating conditions of the D-glutamate, the tryptophans take up a unique conformation. In Figures 20A and 20B, multiple conformations are visible (i.e., more than two peaks). Box 2 shows signals which are shifted to low-field NMR frequencies. Such signals are indicative of a high degree of structural content.

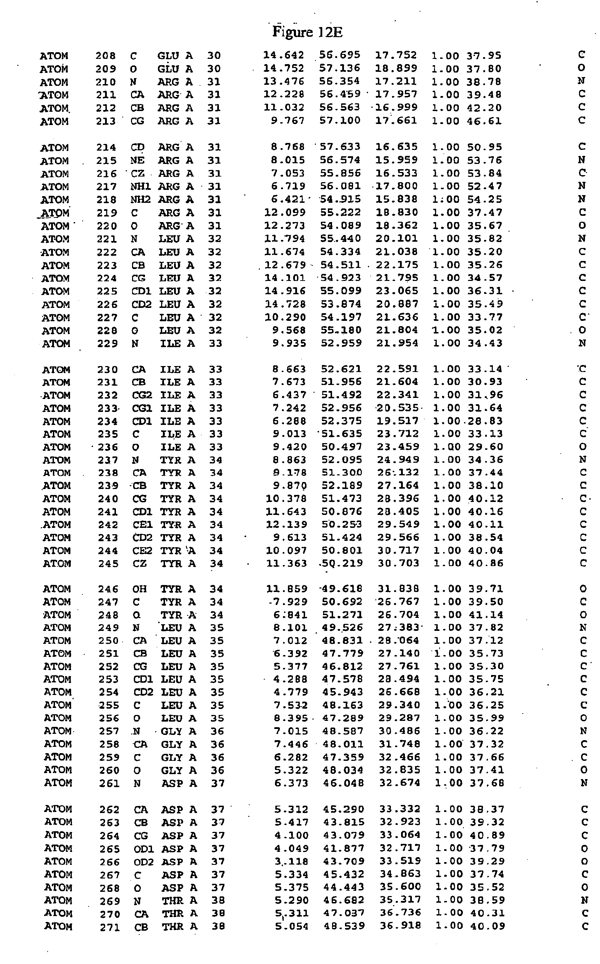

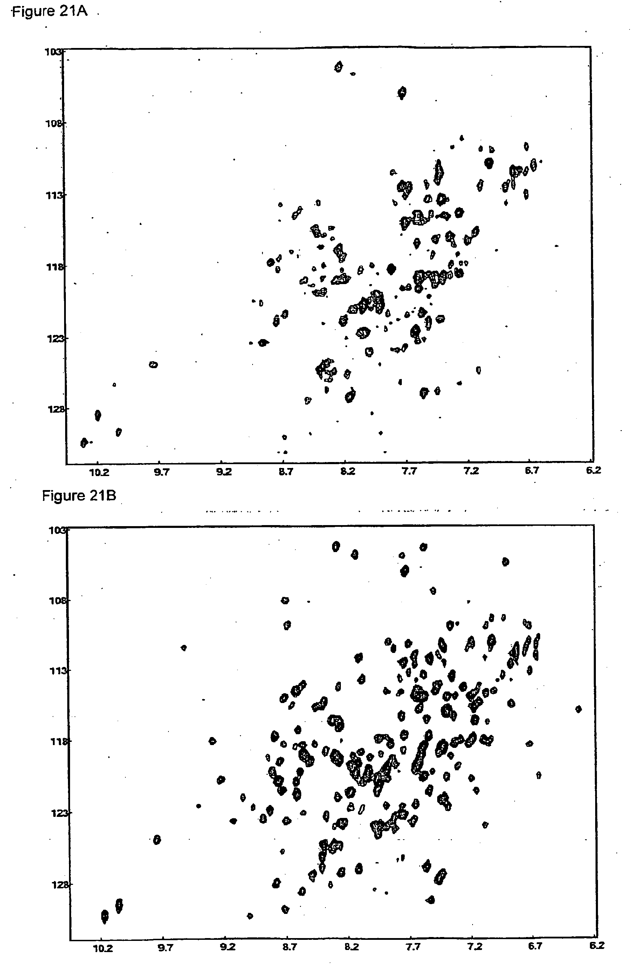

Figure 21A provides a nuclear magnetic resonance (NMR) [¹⁵N, ¹H] correlation spectrum of 0.3 mM ¹⁵N-labeled *H. pylori* MurI protein, to which a saturating amount of compound A was added.
Figure 21B provides a nuclear magnetic resonance (NMR) [¹⁵N, ¹H] correlation spectrum of 0.3 mM ¹⁵N-labeled *H. pylori* Murl protein, to which a saturating amount ofD-glutamate was added.

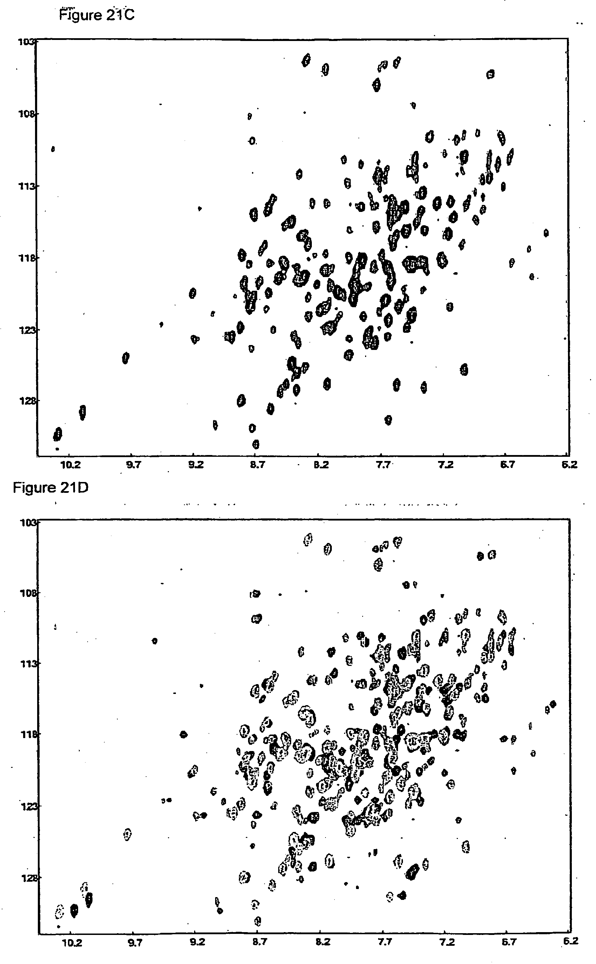

Figure 21C provides a nuclear magnetic resonance (NMR) [¹⁵N, ¹H] correlation spectrum of 0.3 mM ¹⁵N-labeled *H*. *pylori* MurI protein, to which saturating amounts of D-glutamate and compound A were added.
Figure 21D provides an overlay of the spectra shown in Figures 21B and 21C.

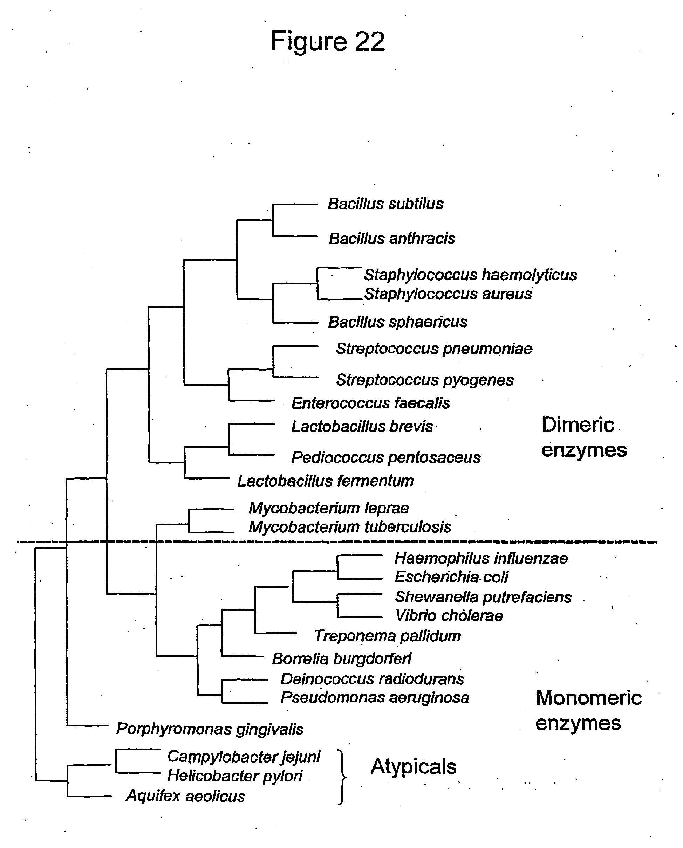

Figure 22 provides a pylogenetic analysis of MurI orthologs indicating dimeric (Gram positive), monomeric (Gram negative), and atypical dimeric structures by genus and species.

### DETAILED DESCRIPTION OF THE INVENTION

### I. Overview

One of ordinary skill in the art would recognize that solving crystal structures of proteins such as MurI requires a stable source of high-quality protein.

As described herein, MurI of three classes of bacteria (Gram negative, Gram positive, and atypical) has been crystallized and the crystal structures (three-dimensional structure) determined.

### II. Polypeptides, Crystals and Space Groups

We disclose an isolated polypeptide of a portion of MurI which functions as a binding site when folded in the proper 3-D orientation.

The terms "peptide", "polypeptide" and "protein" are used interchangeably herein. These terms refer to unmodified amino acid chains, and also include minor modifications, such as phosphorylation, glycosylation and lipid modification. "isolated" (used interchangeably with "substantially pure") when applied to polypeptides means a polypeptide or a portion thereof which, by virtue of its origin or manipulation. By "isolated" it is further meant a protein that is: (i) synthesized chemically; (ii) expressed in a host cell and purified away from associated and contaminating proteins. The term generally means a polypeptide that has been separated from other proteins and nucleic acids with which it naturally occurs. Preferably, the polypeptide is also separated from substances such as antibodies or gel matrices (polyacrylamide) which are used to purify it.

Each of the isolated polypeptide sequences can be a native sequence of MurI, or a sequence that is at least 70%, 75%, 80%, 85%, 90%, 95%, 98% or 99% homologous to the amino acid sequence represented by any one of SEQ ID NOS: 2-34, 40, 44, 46, 48, 50, 52, 54, 56, 58, 60, 62, 64, 66, and 74.

### A. Gram negative - E. coli

One of ordinary skill in the art would recognize that solving crystal structures of proteins such as MurI (also referred to as MurI) requires a stable high-quality protein.

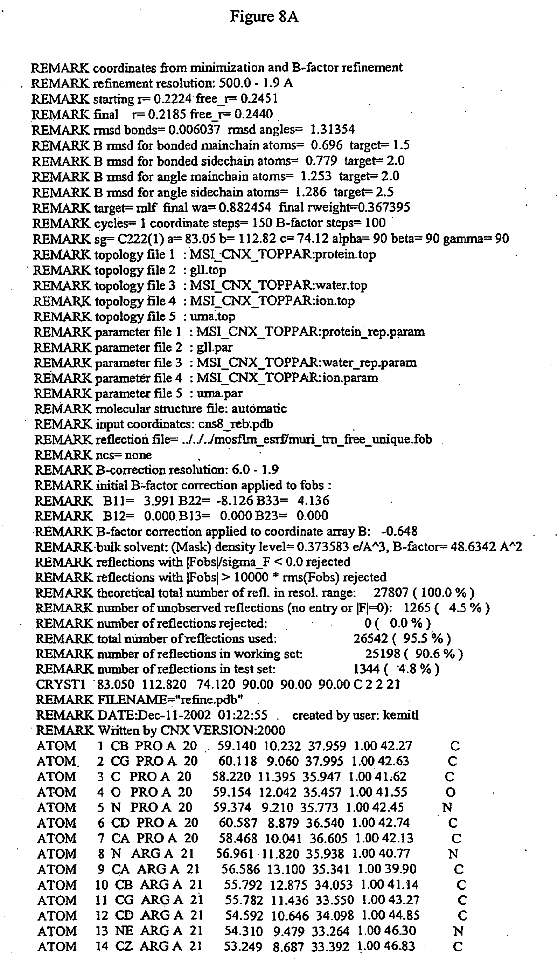

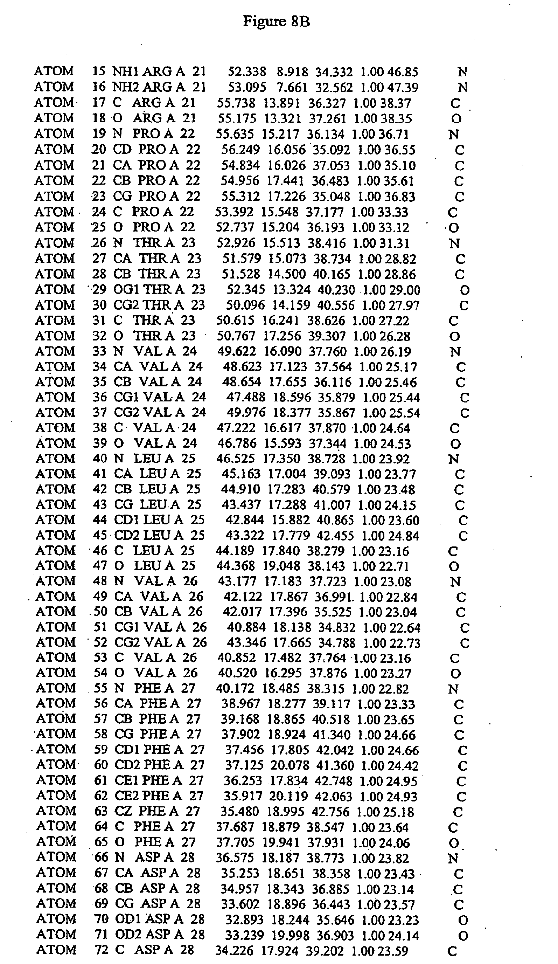

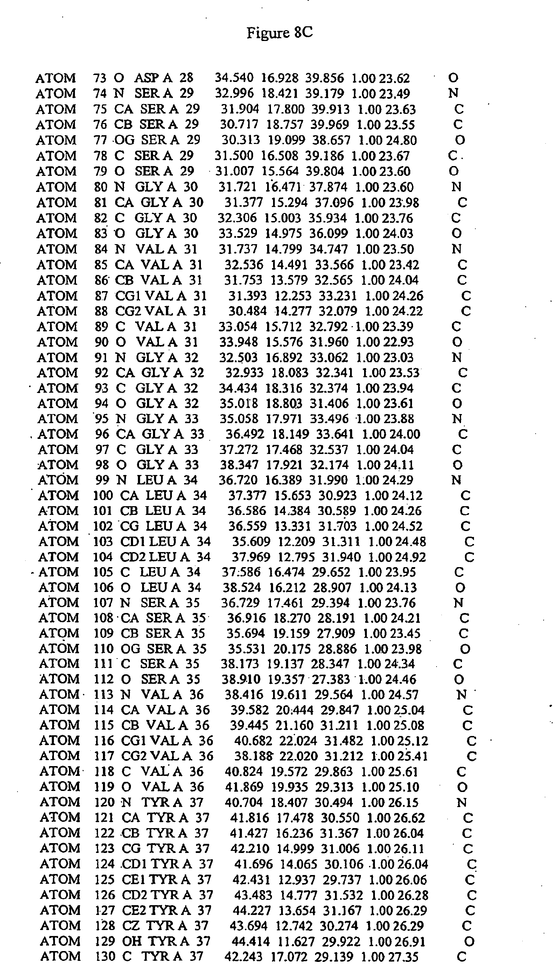

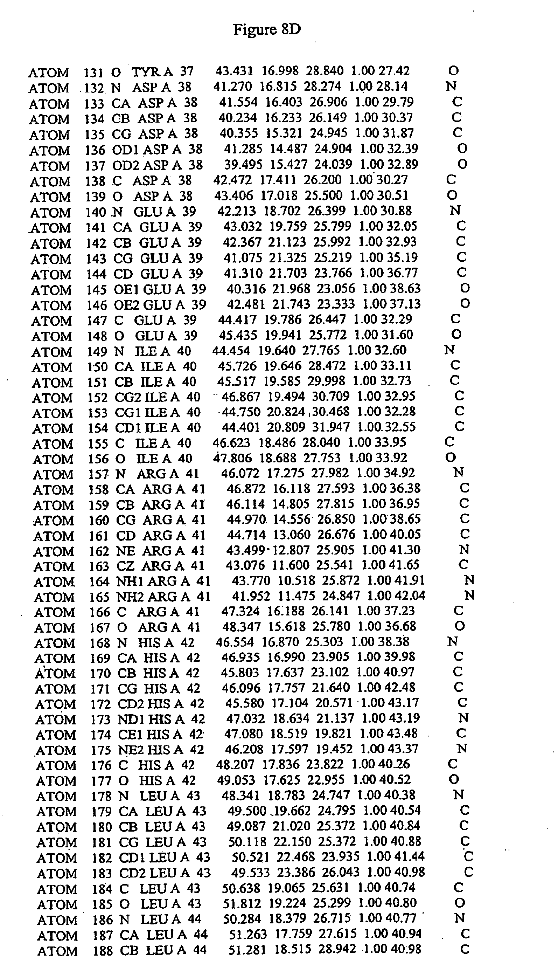

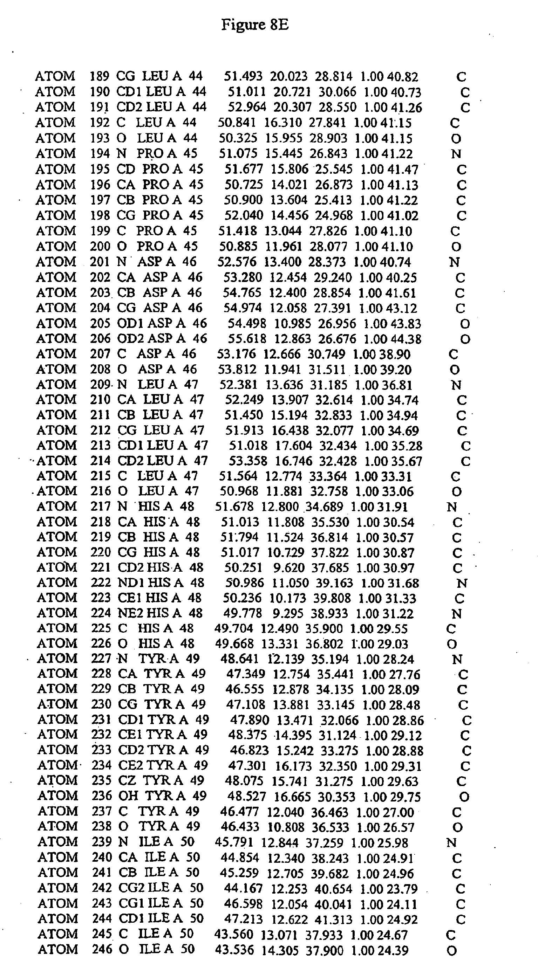

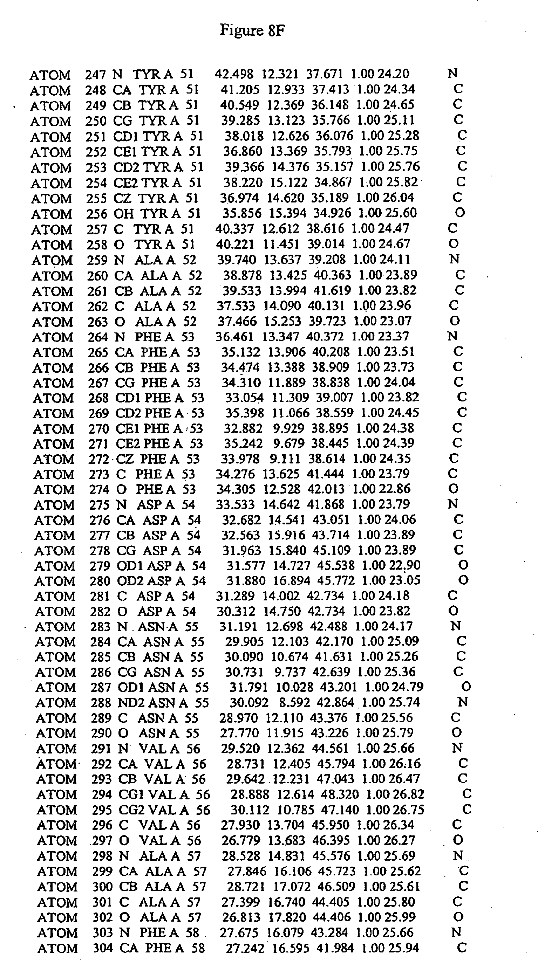

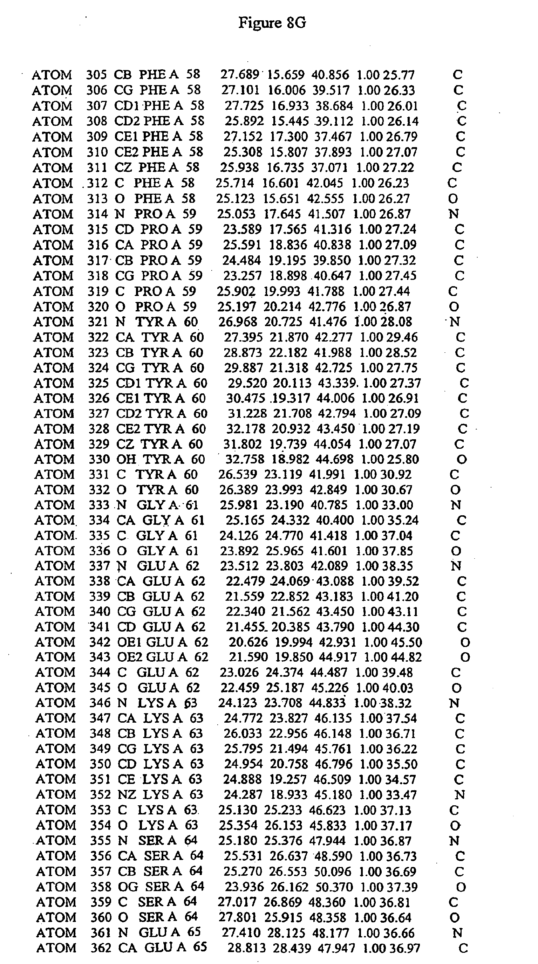

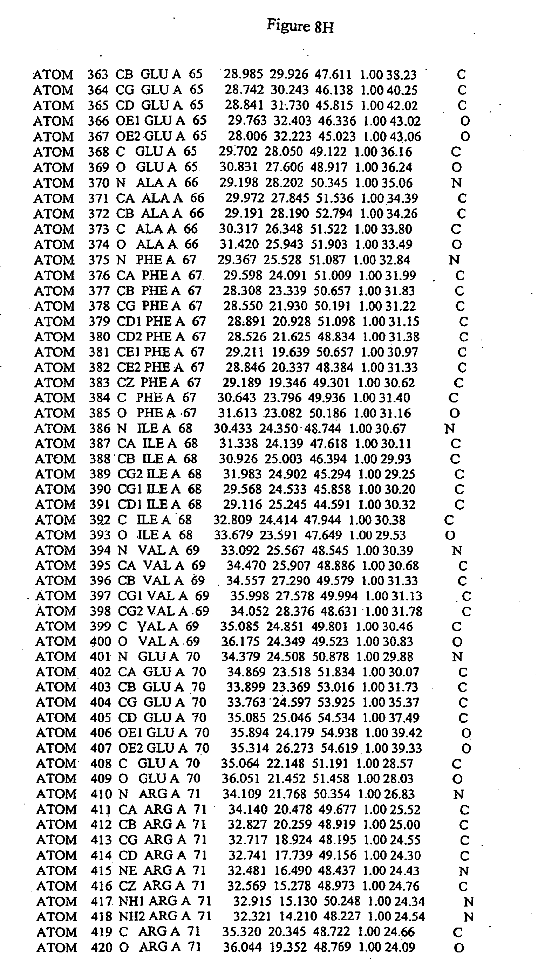

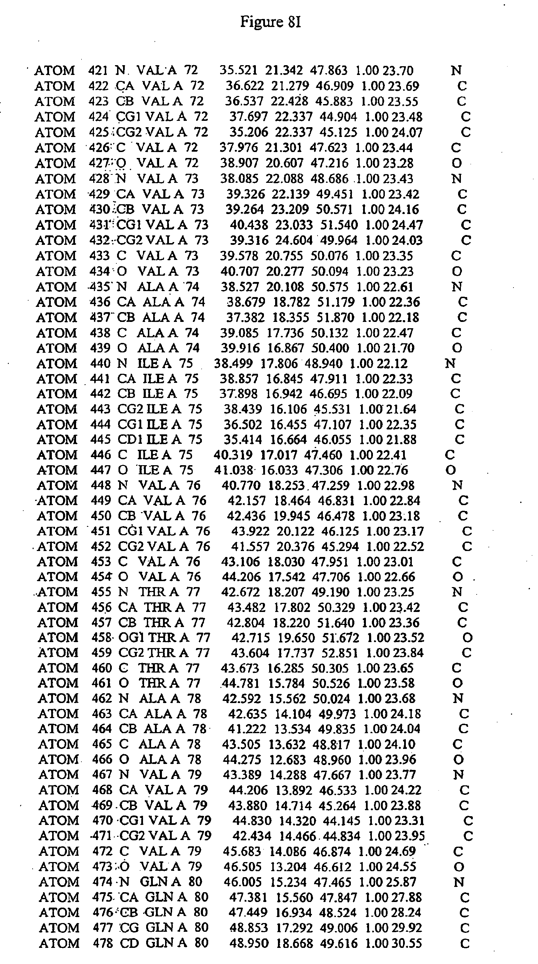

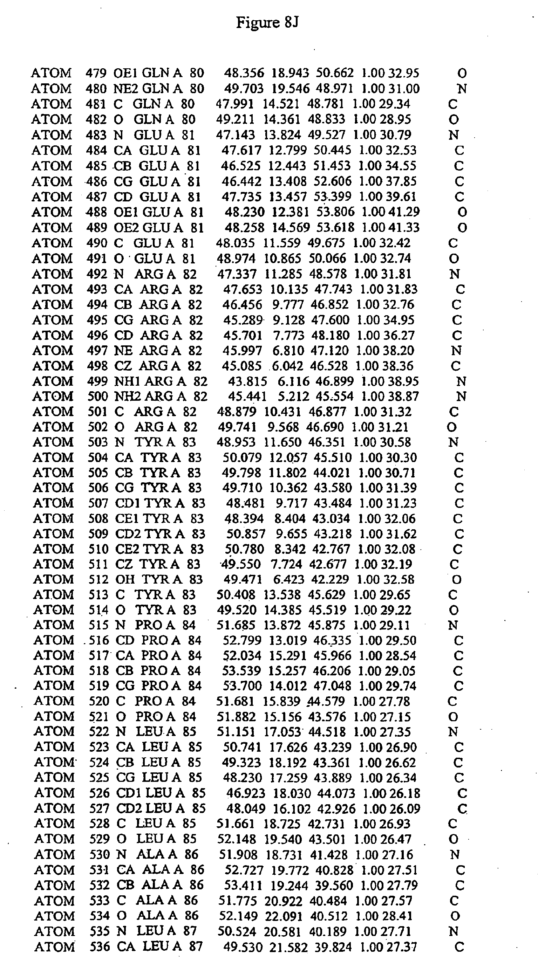

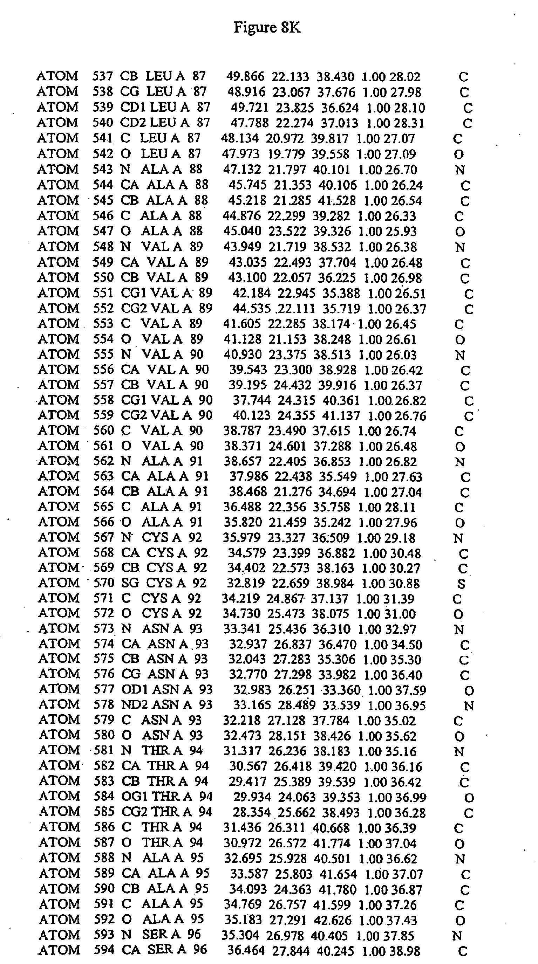

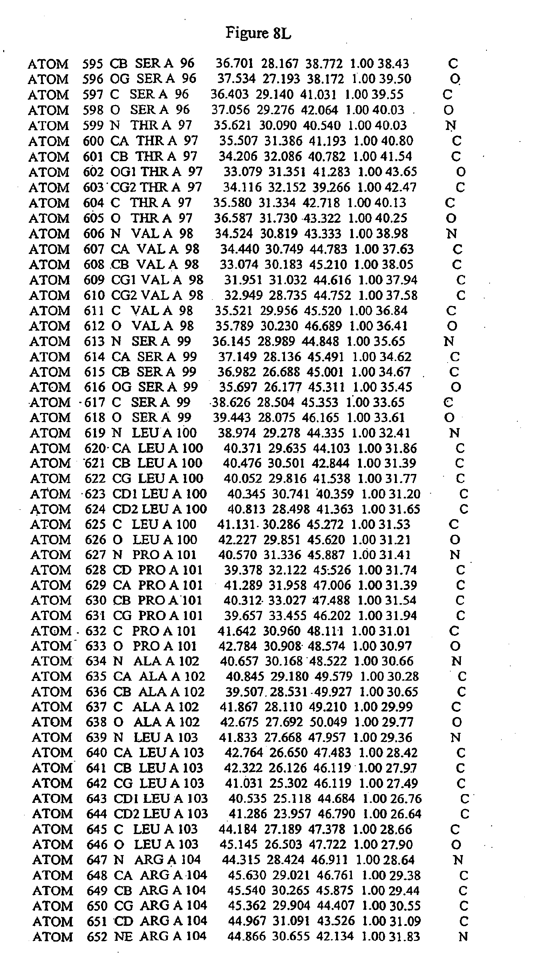

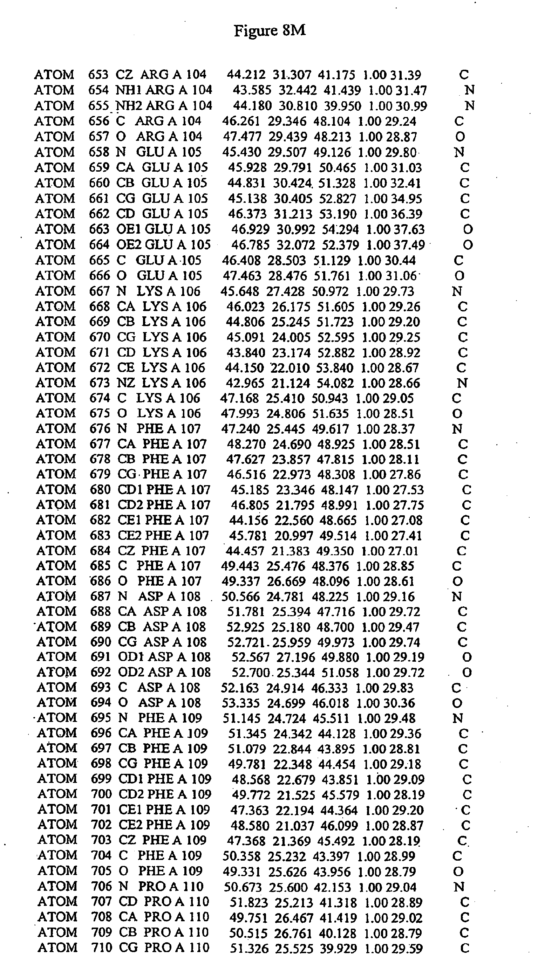

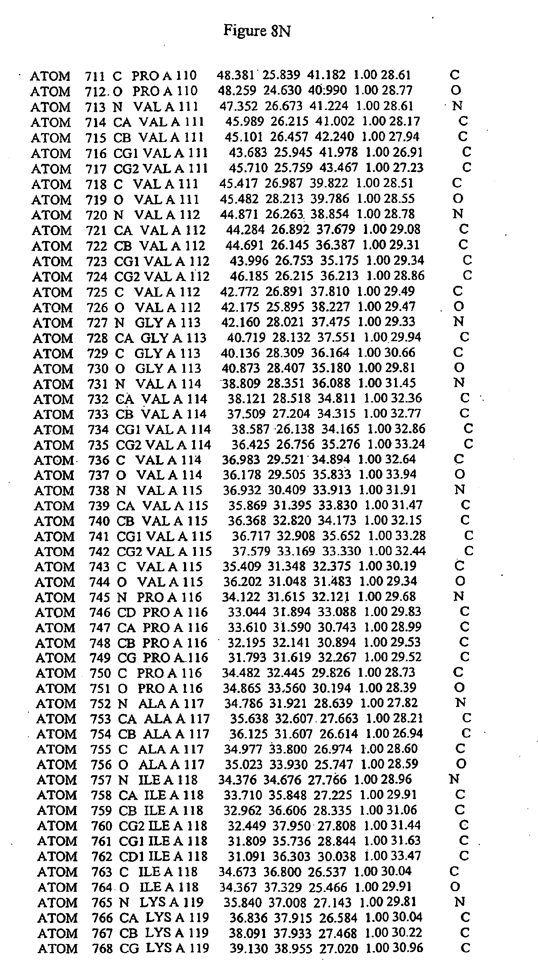

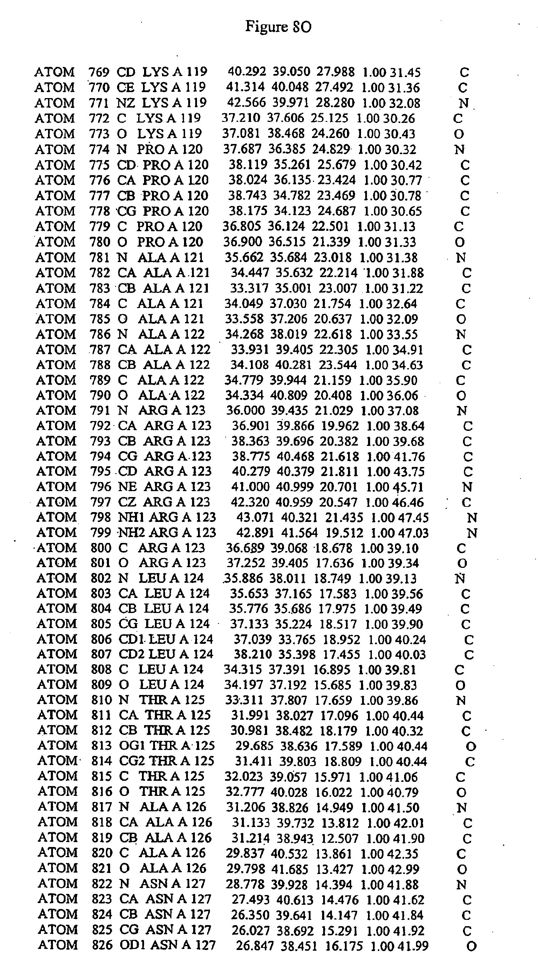

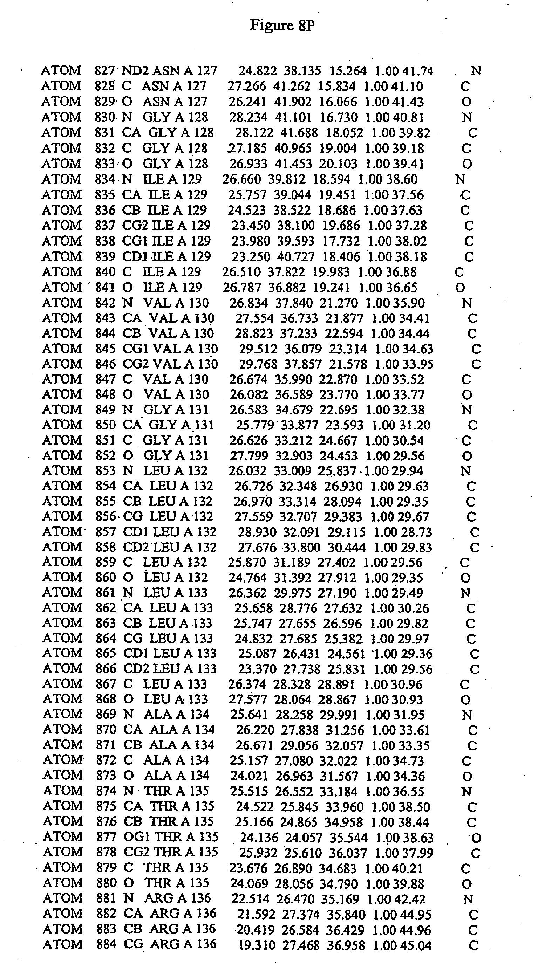

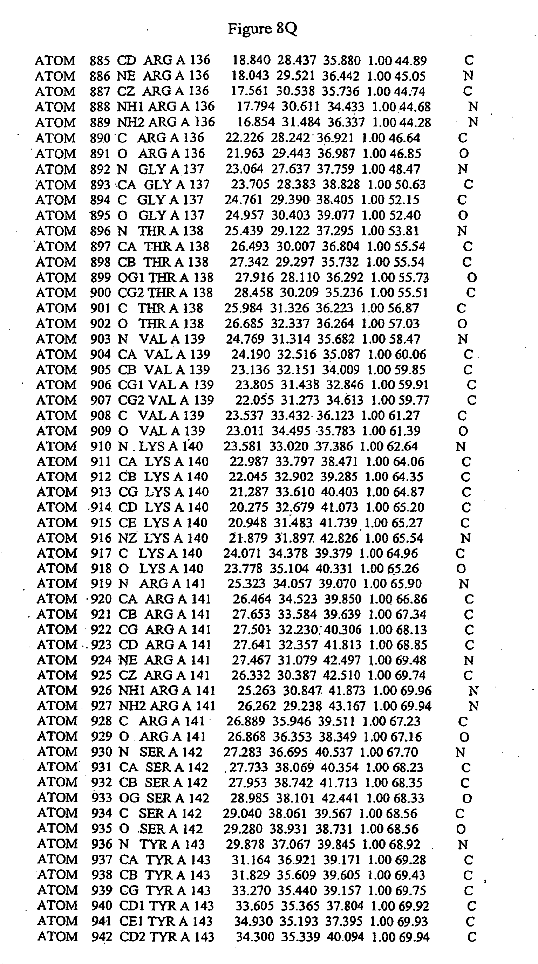

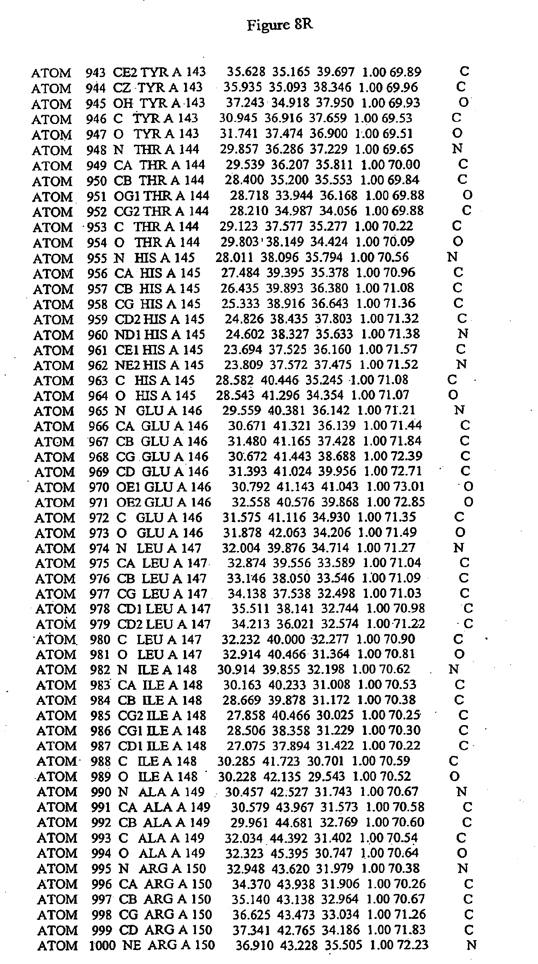

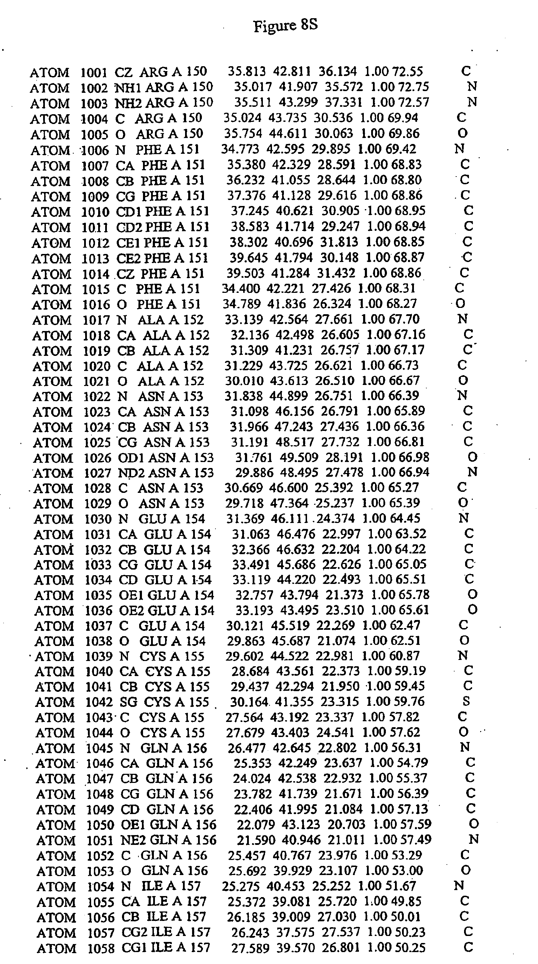

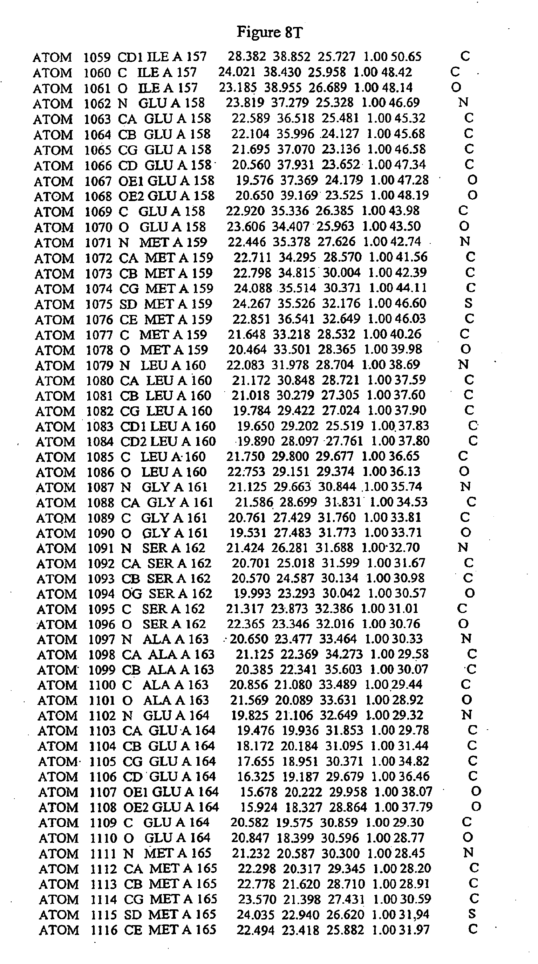

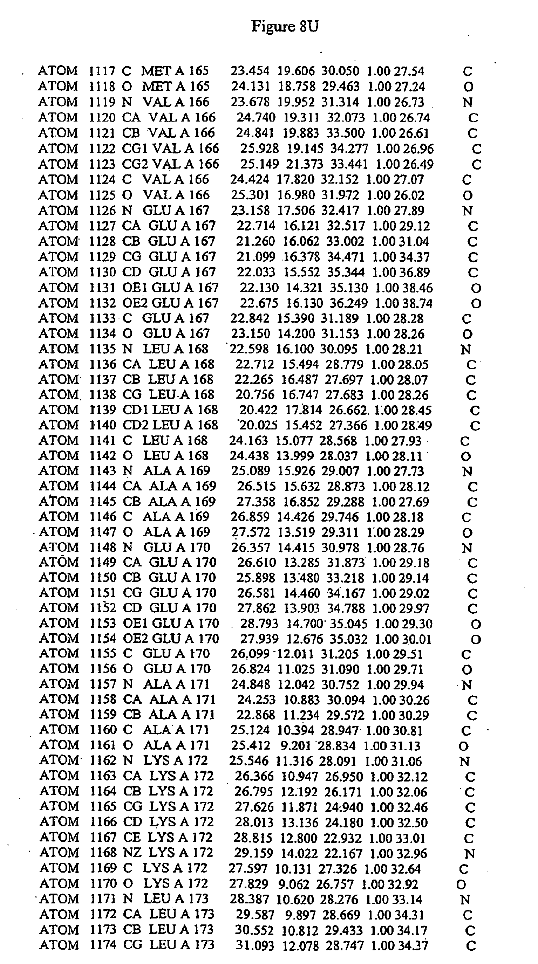

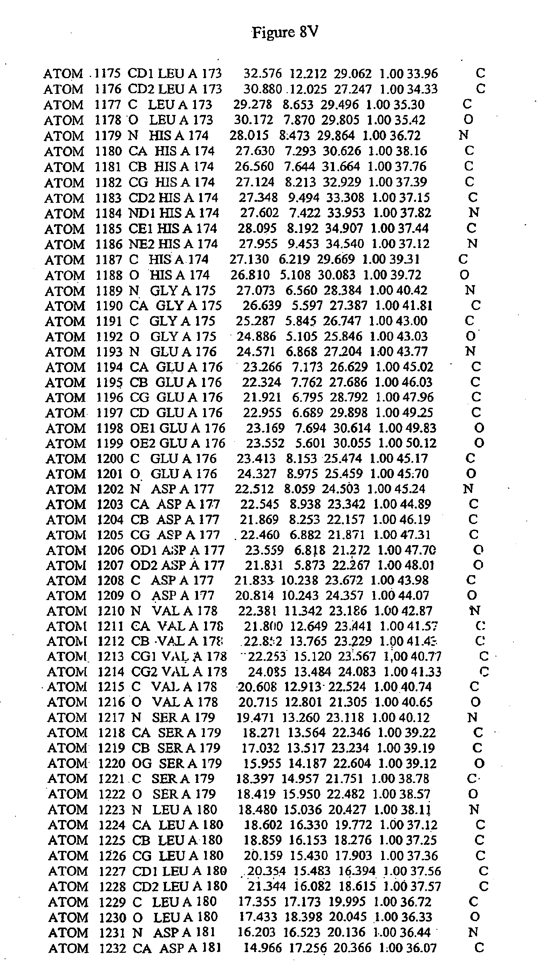

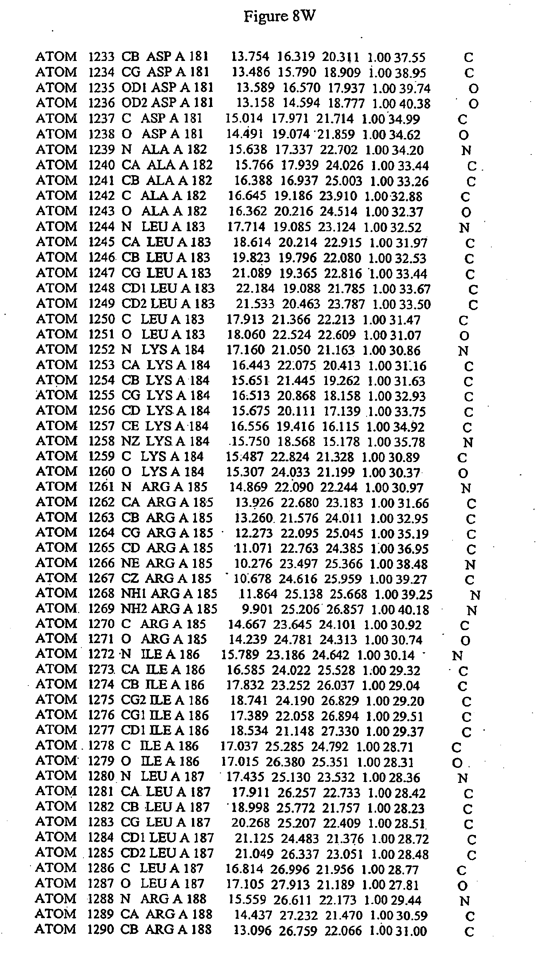

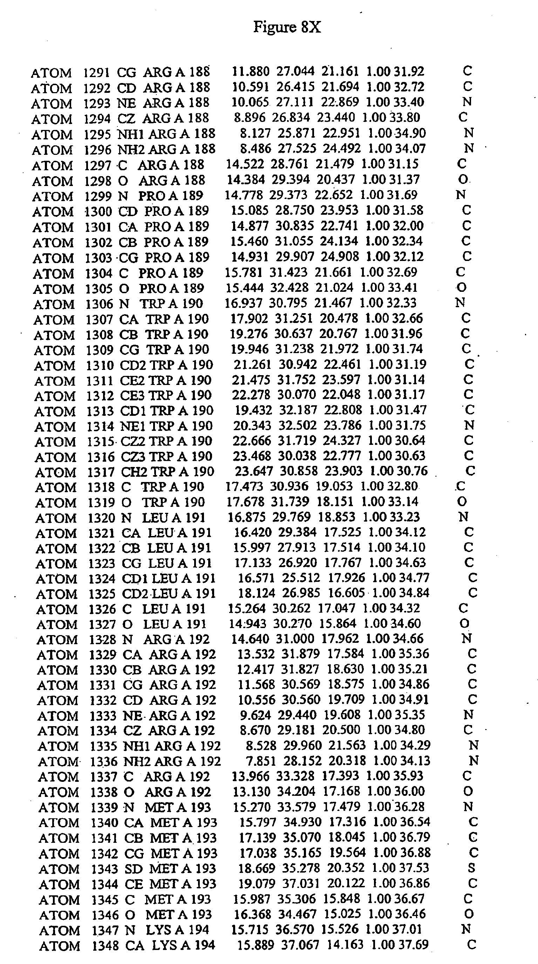

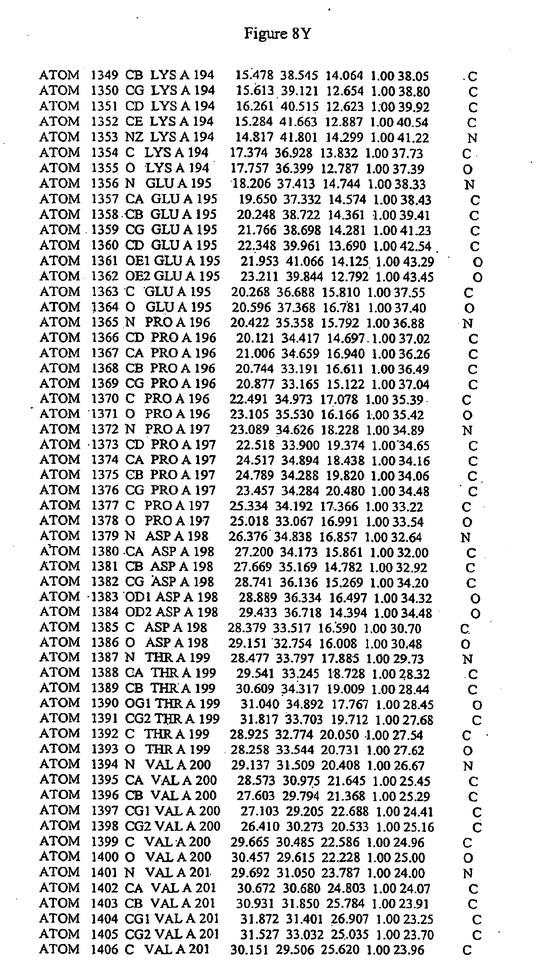

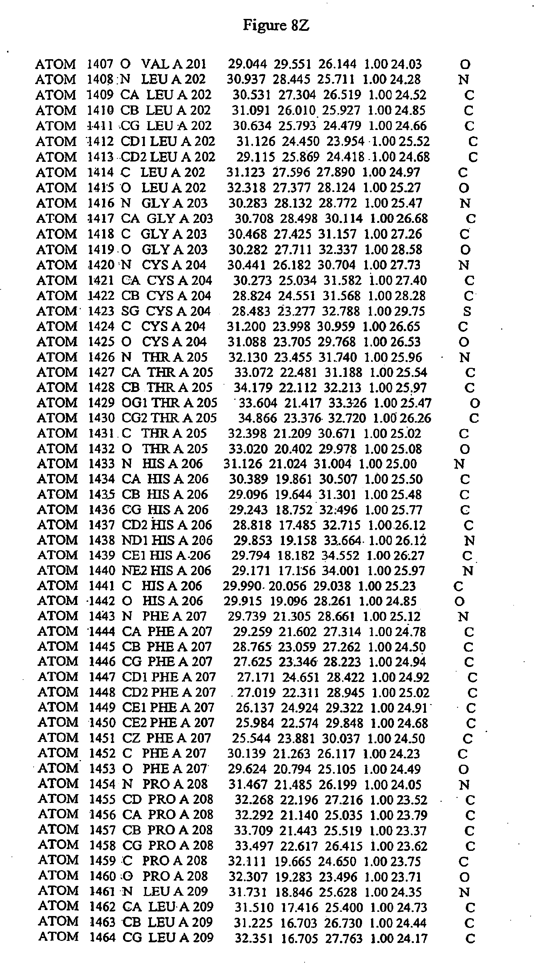

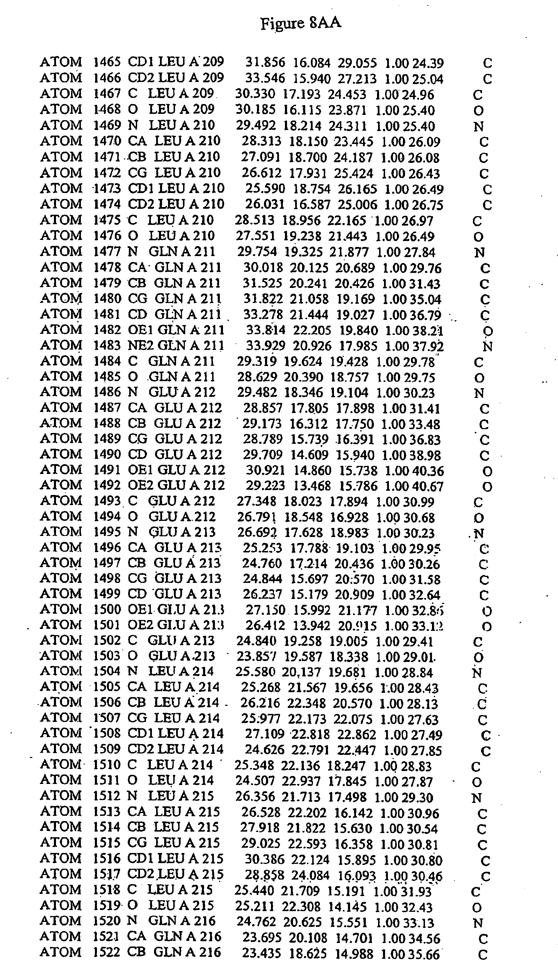

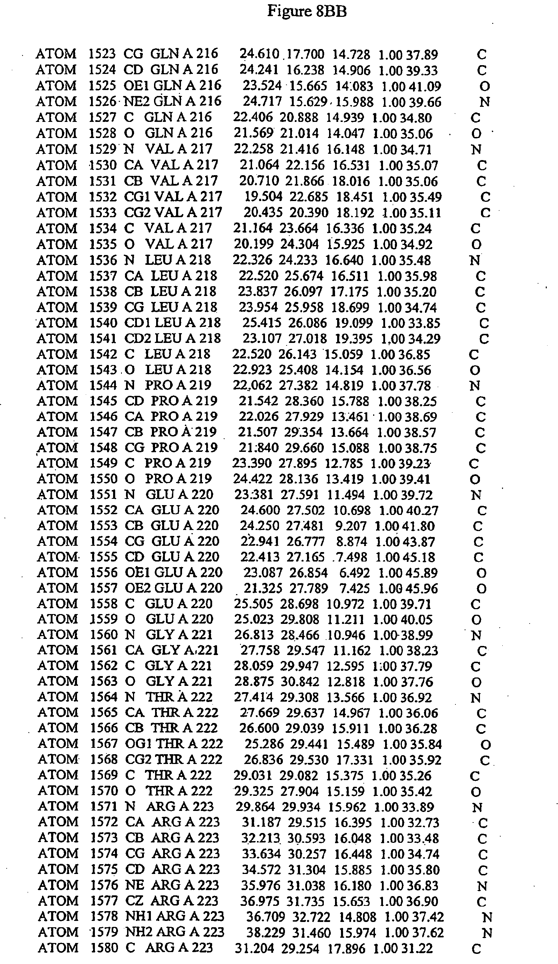

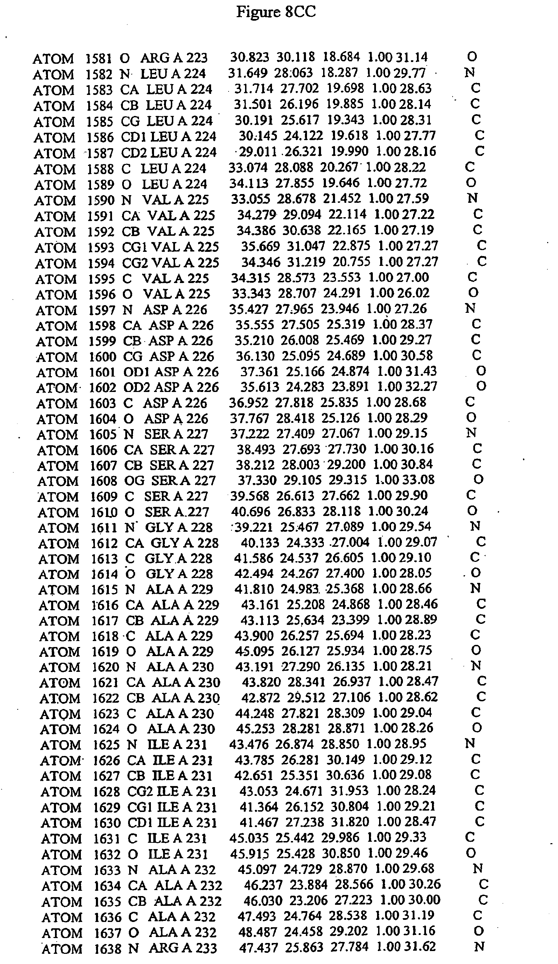

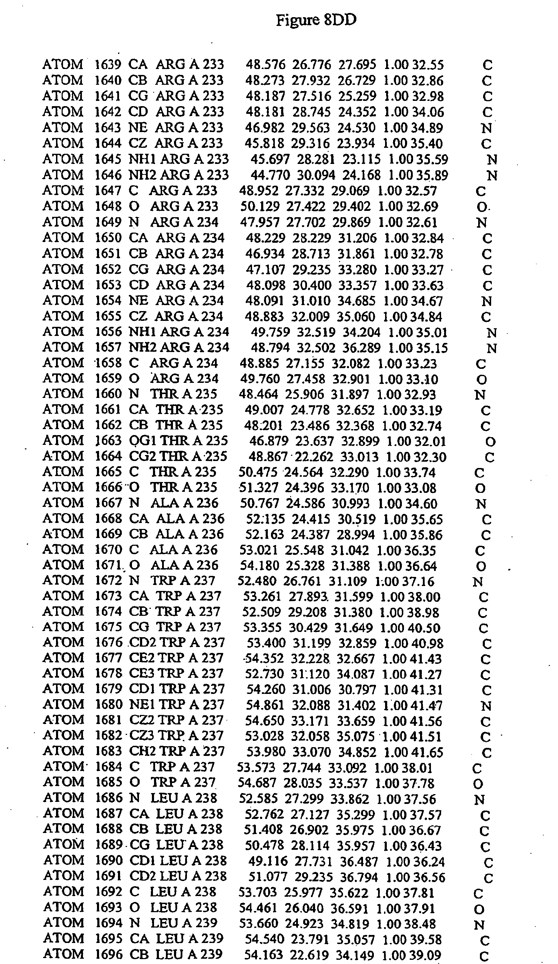

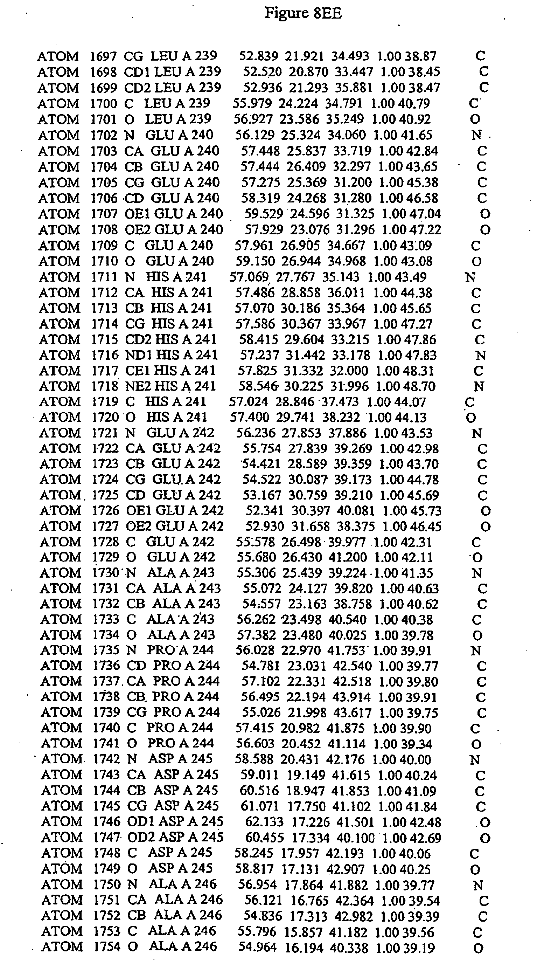

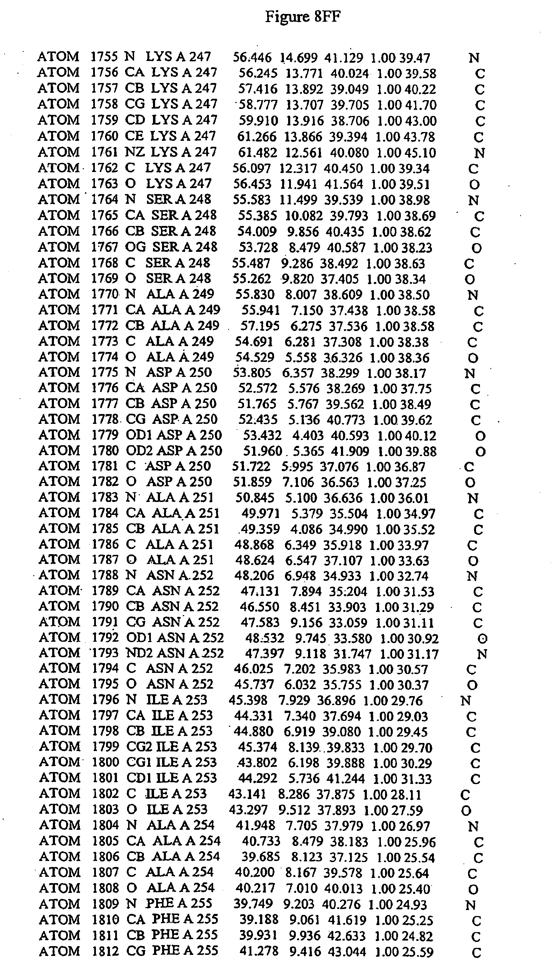

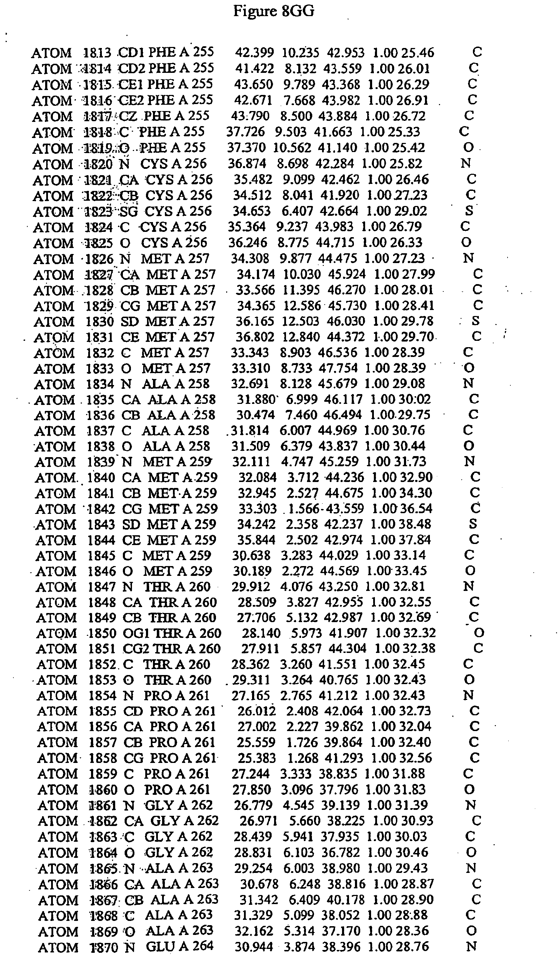

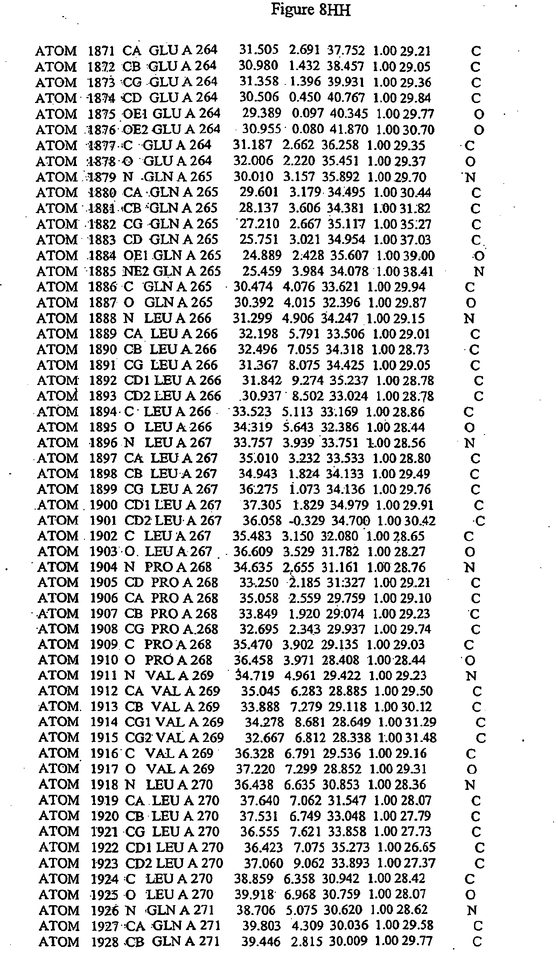

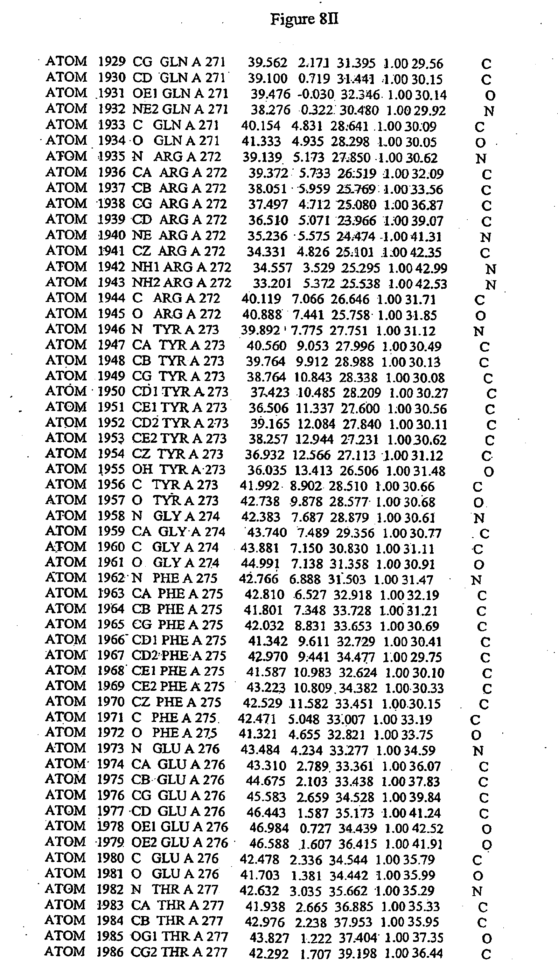

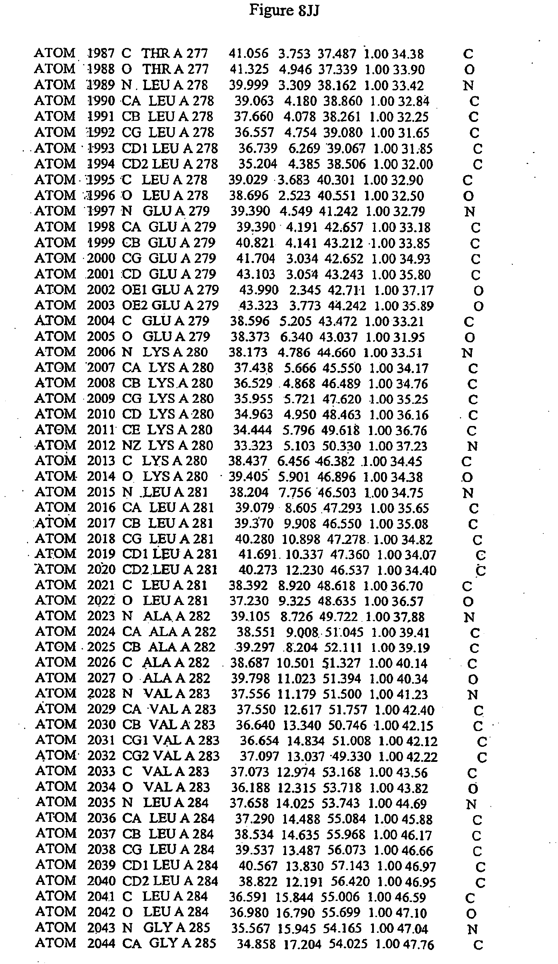

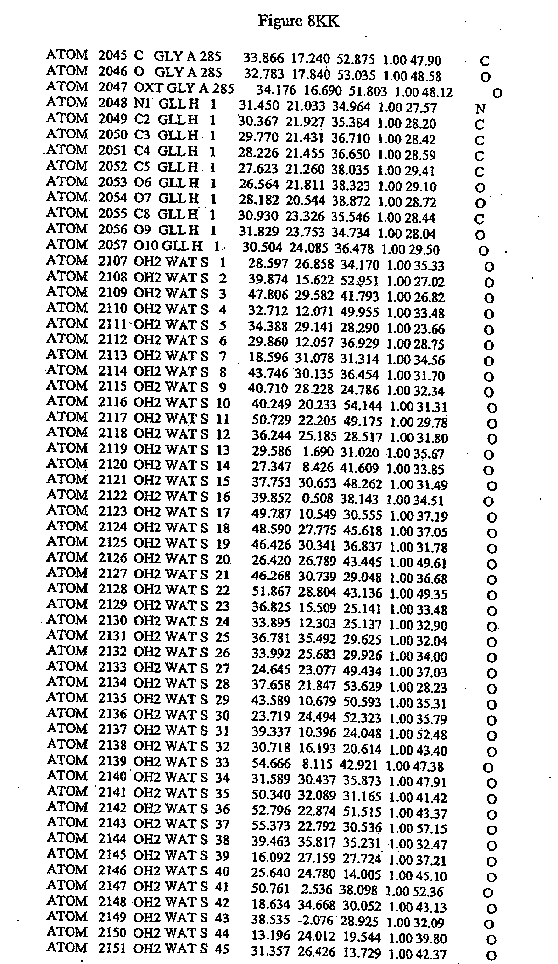

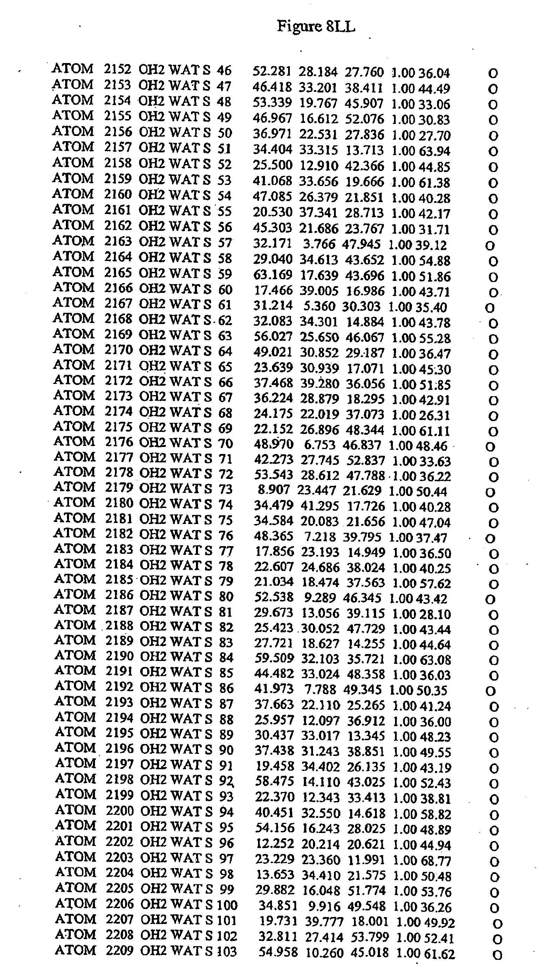

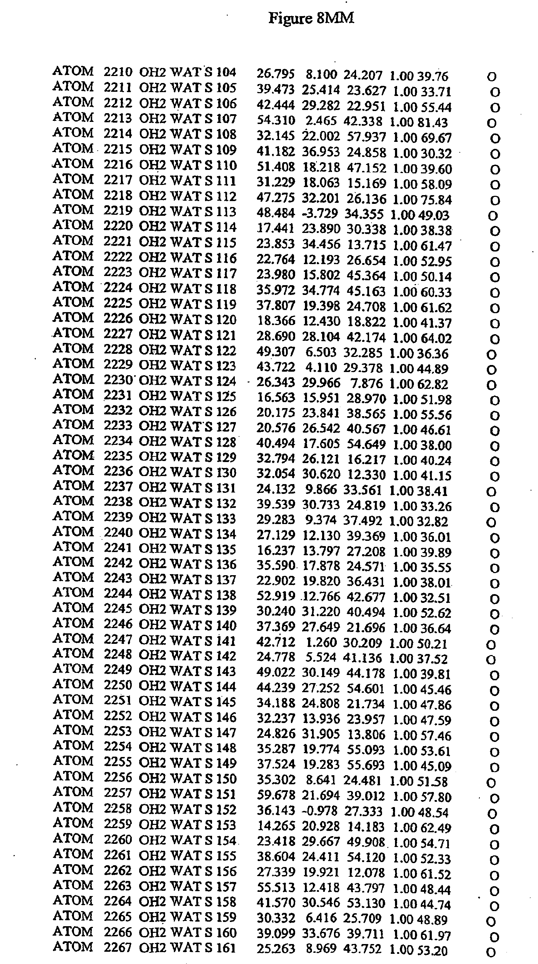

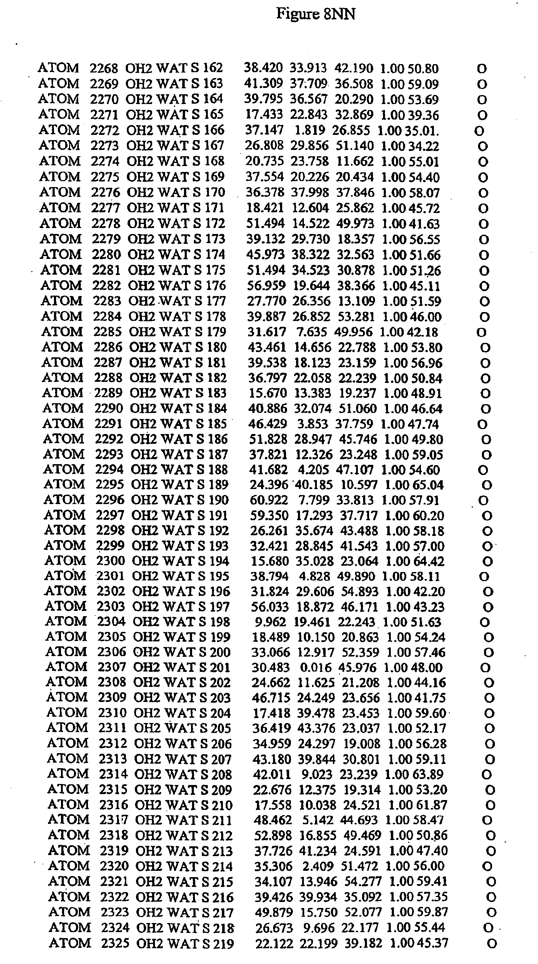

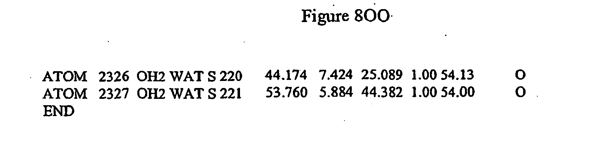

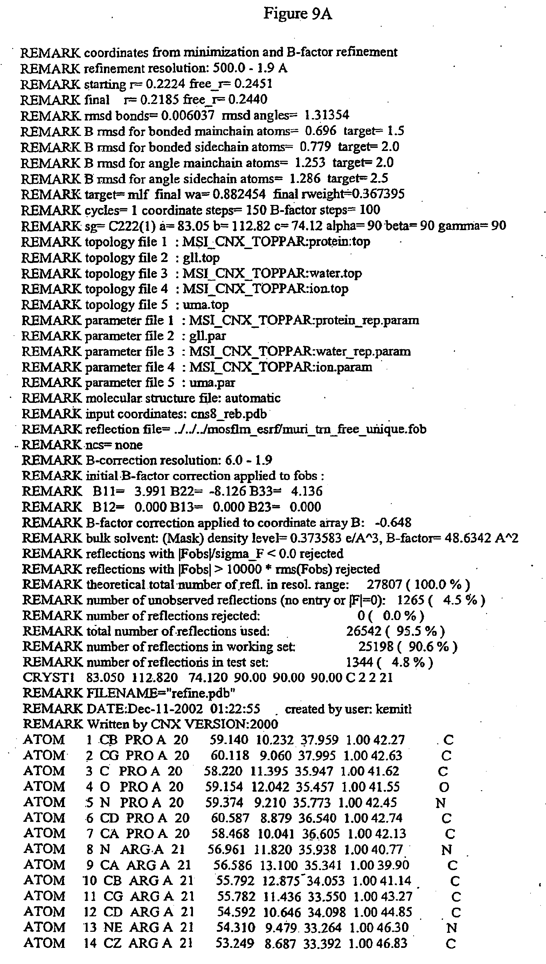

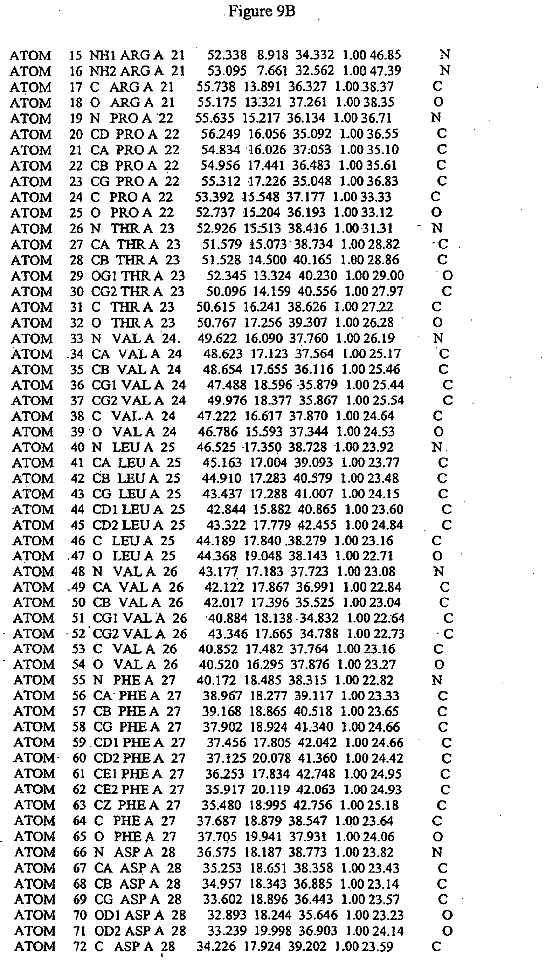

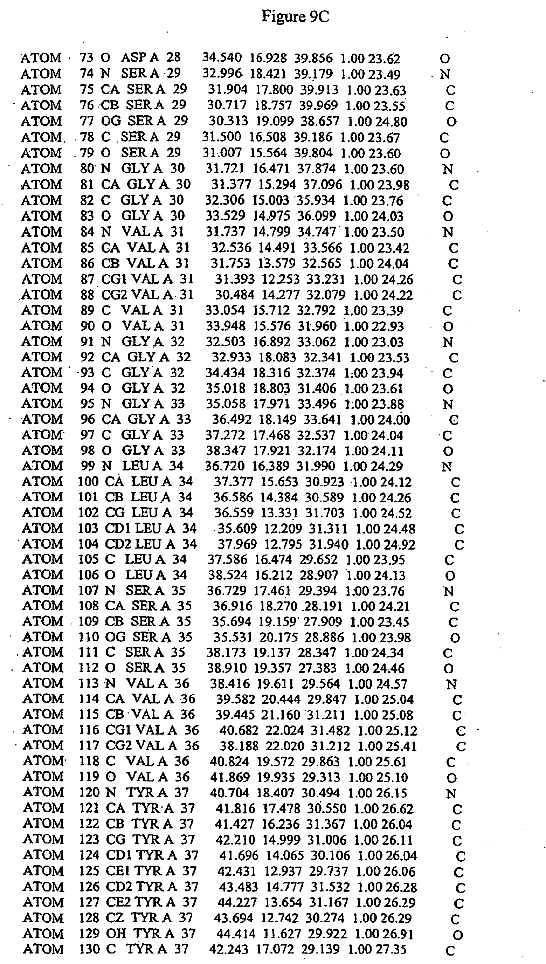

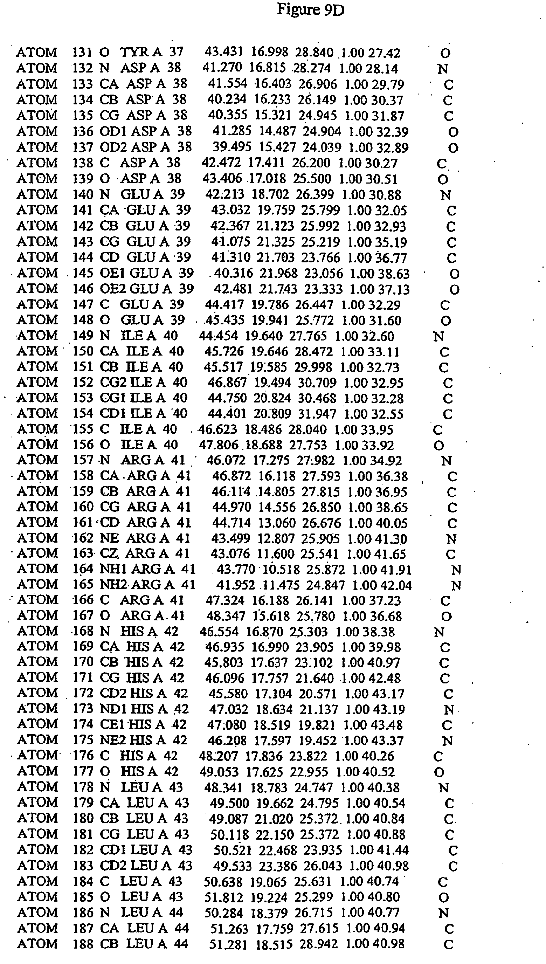

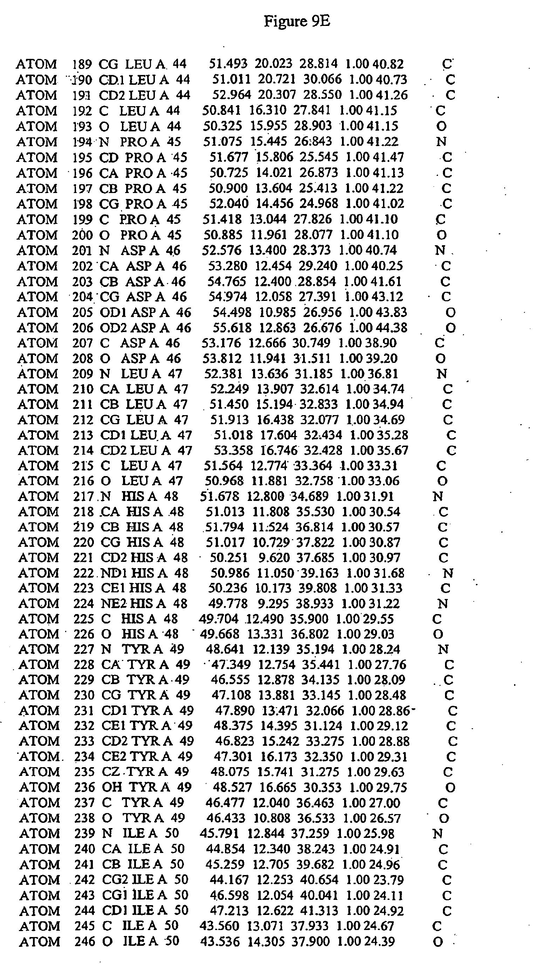

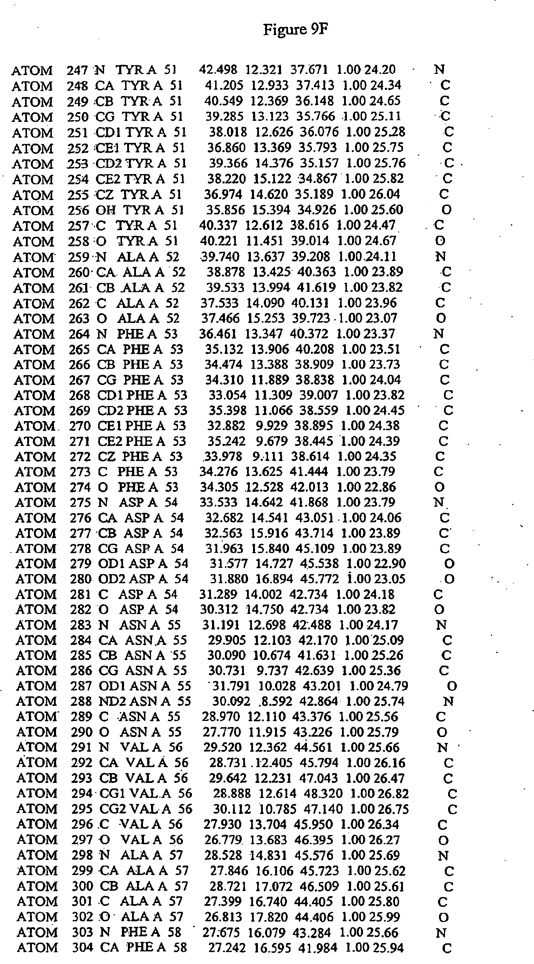

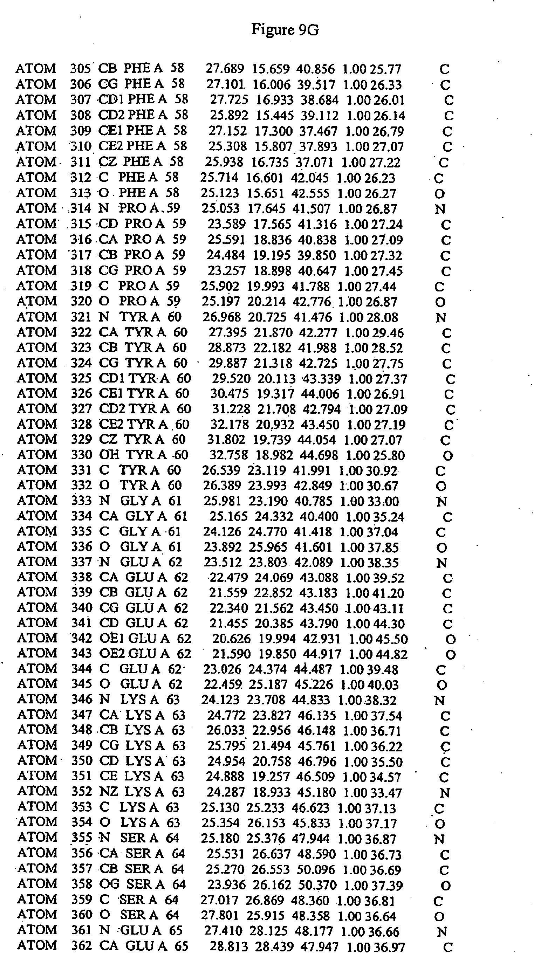

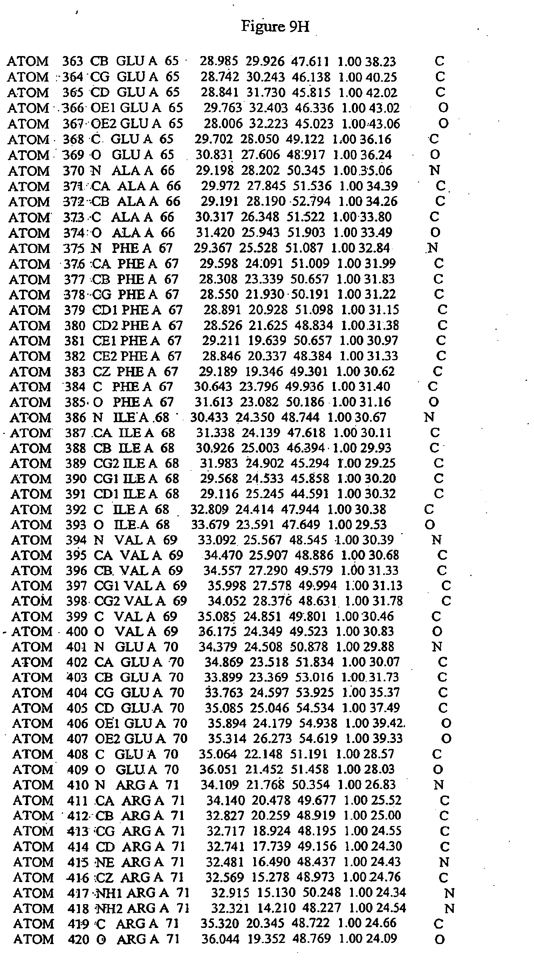

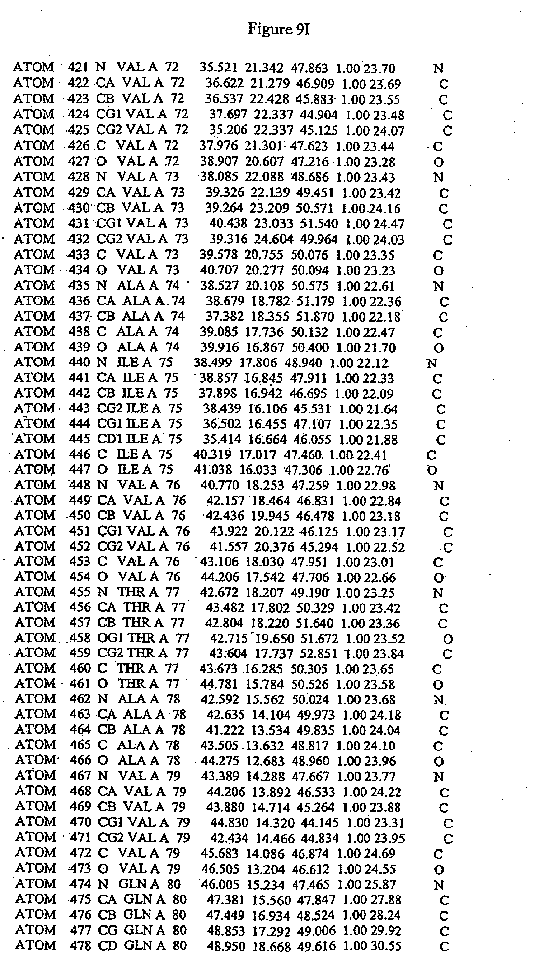

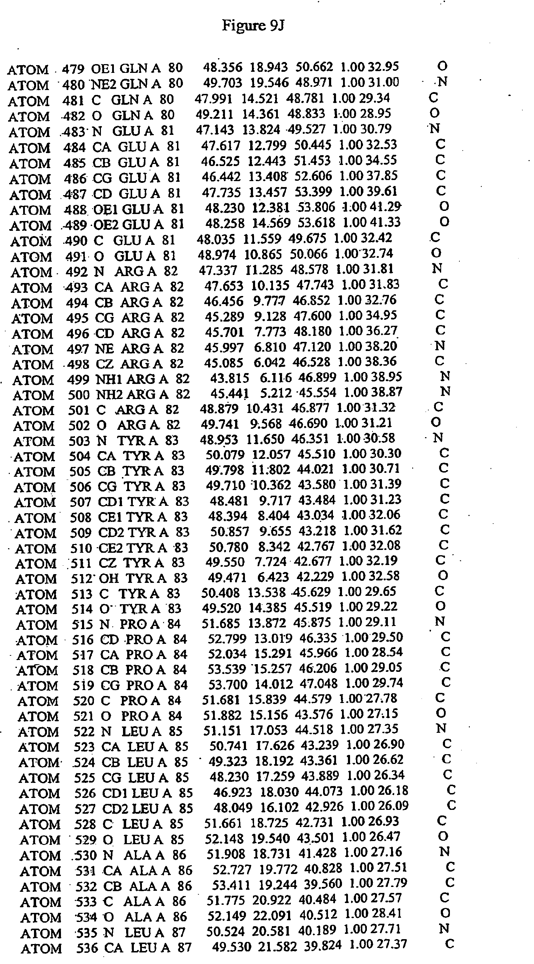

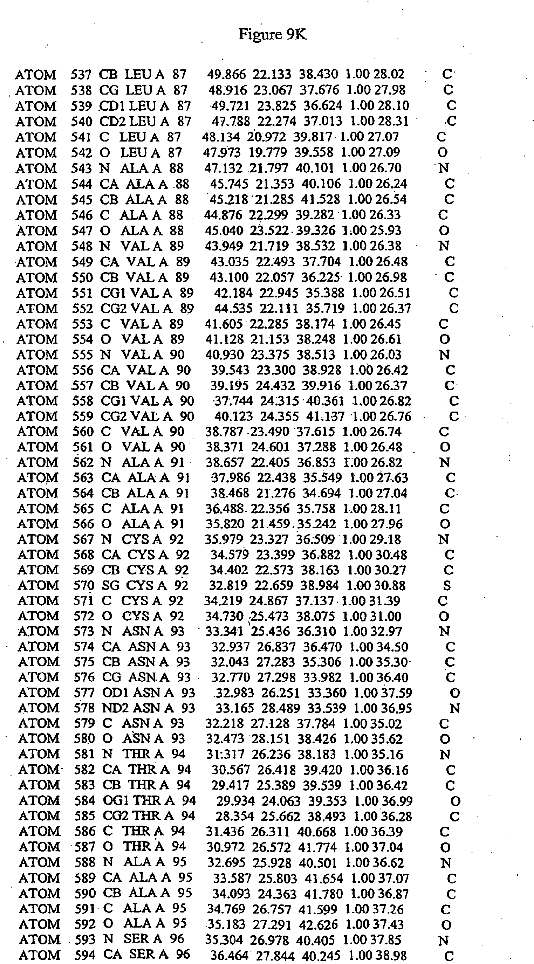

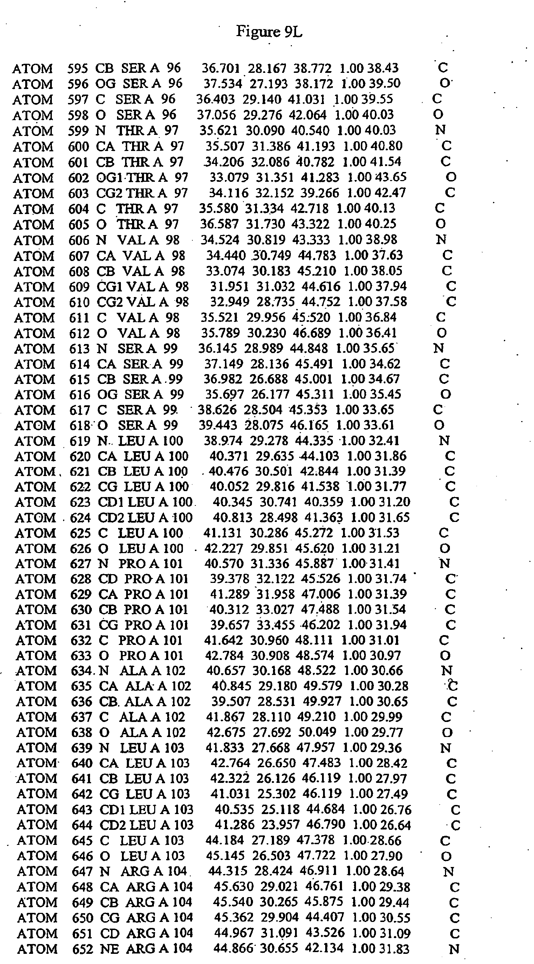

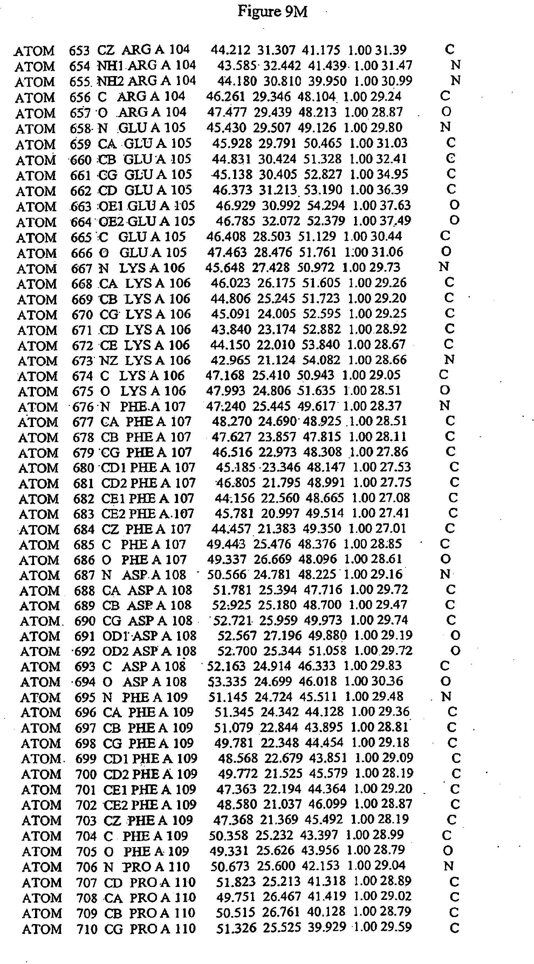

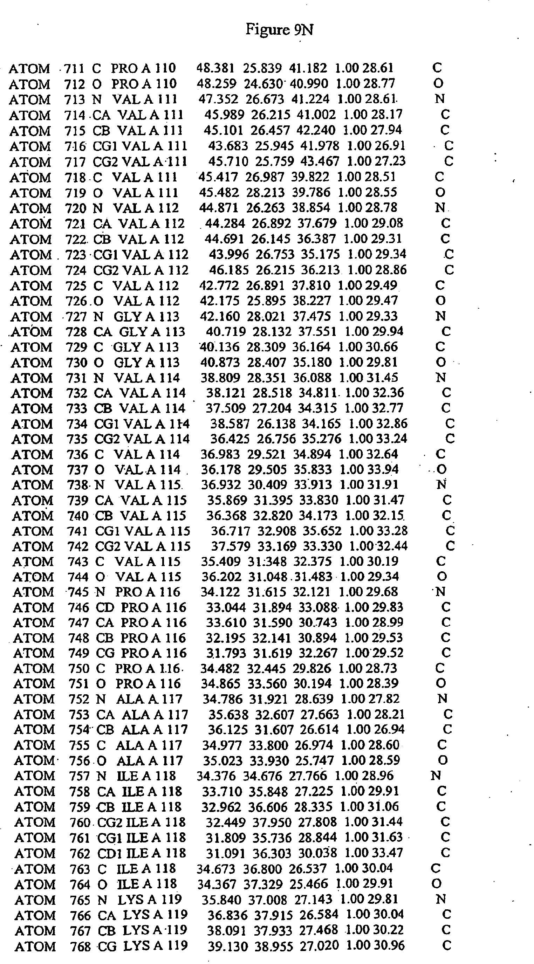

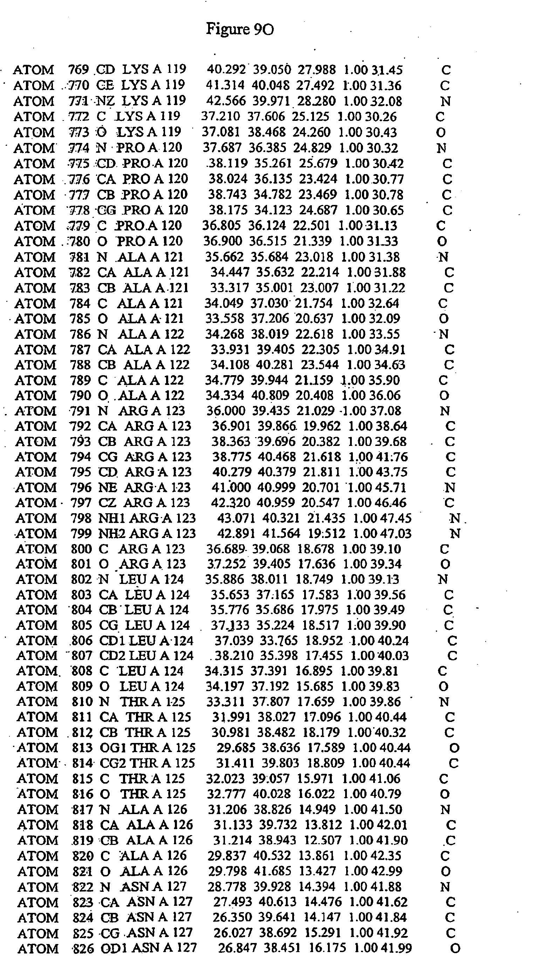

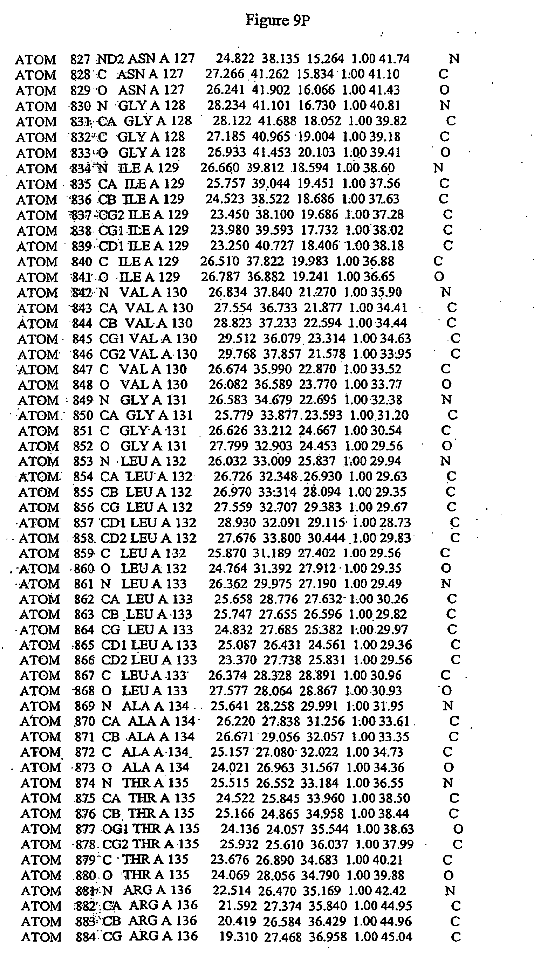

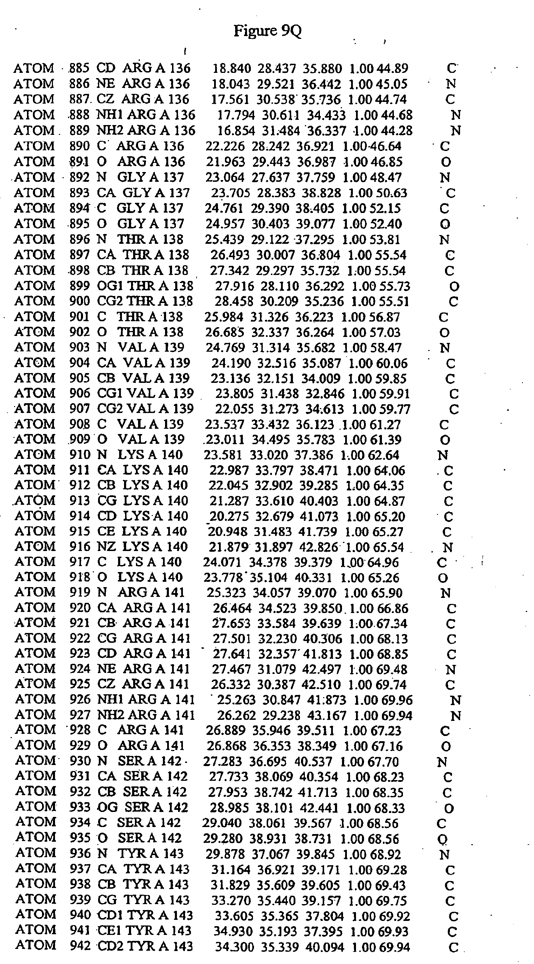

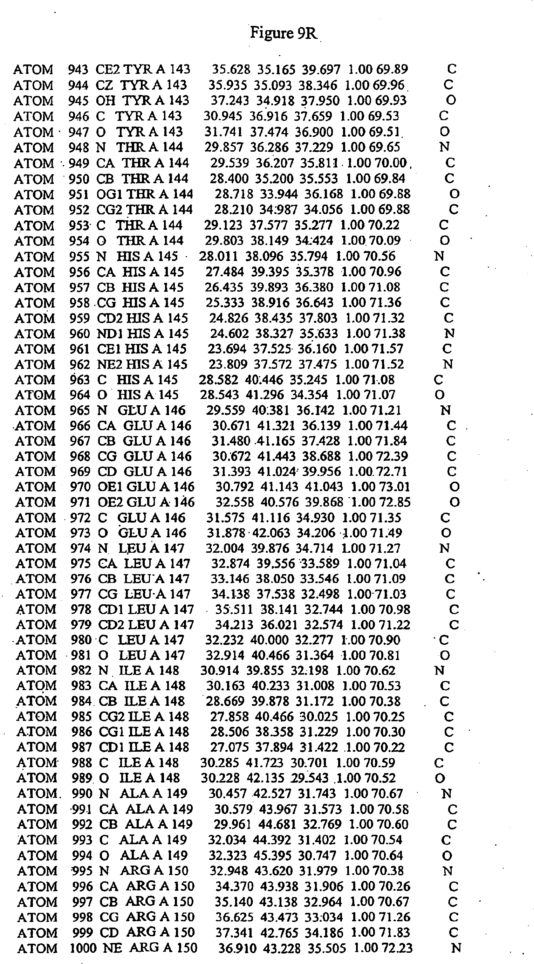

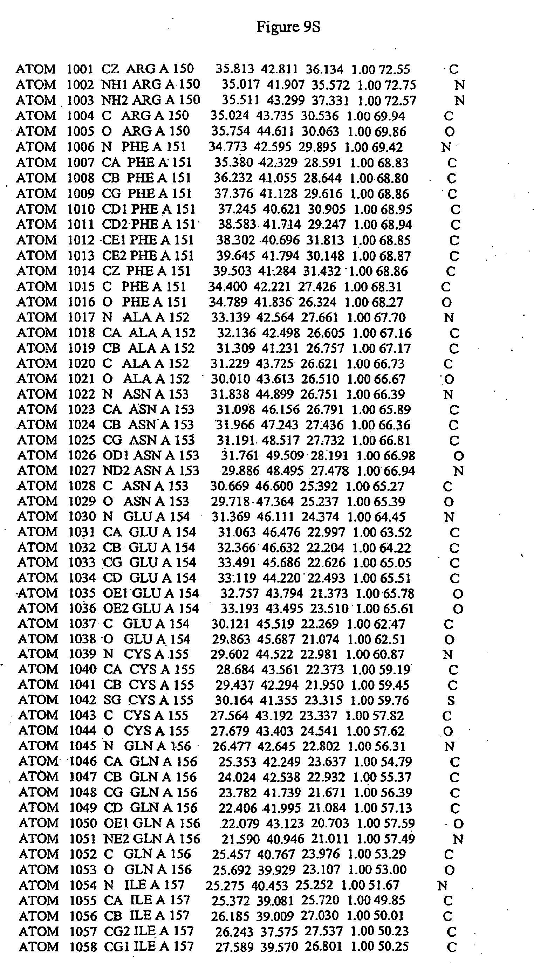

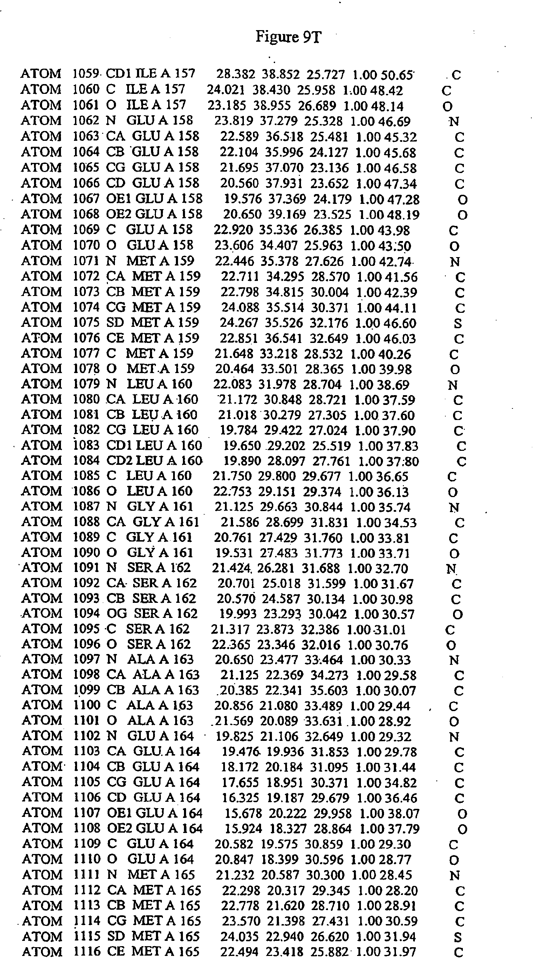

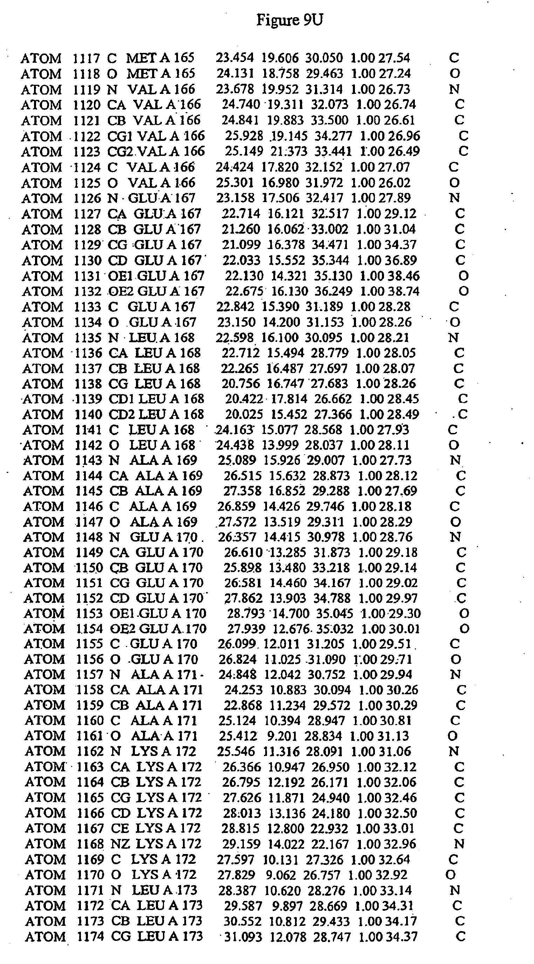

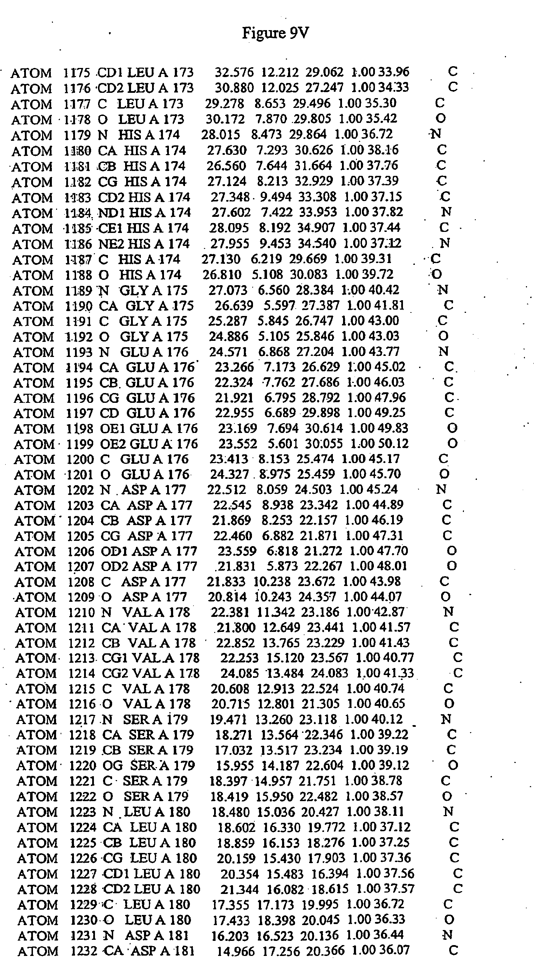

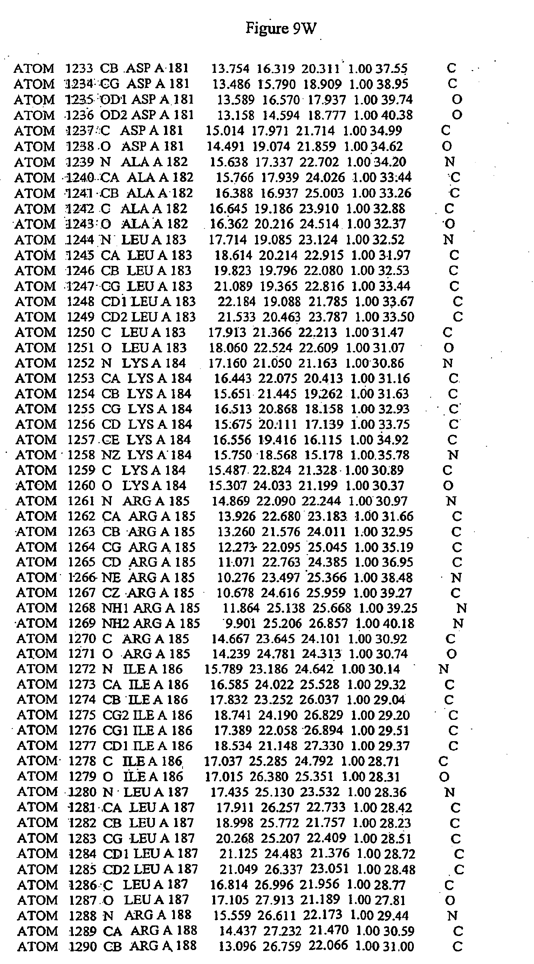

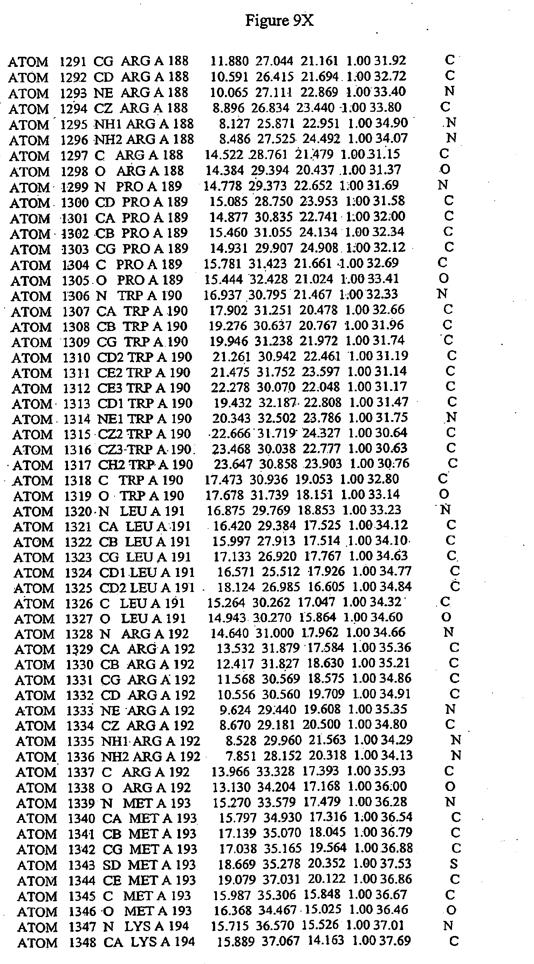

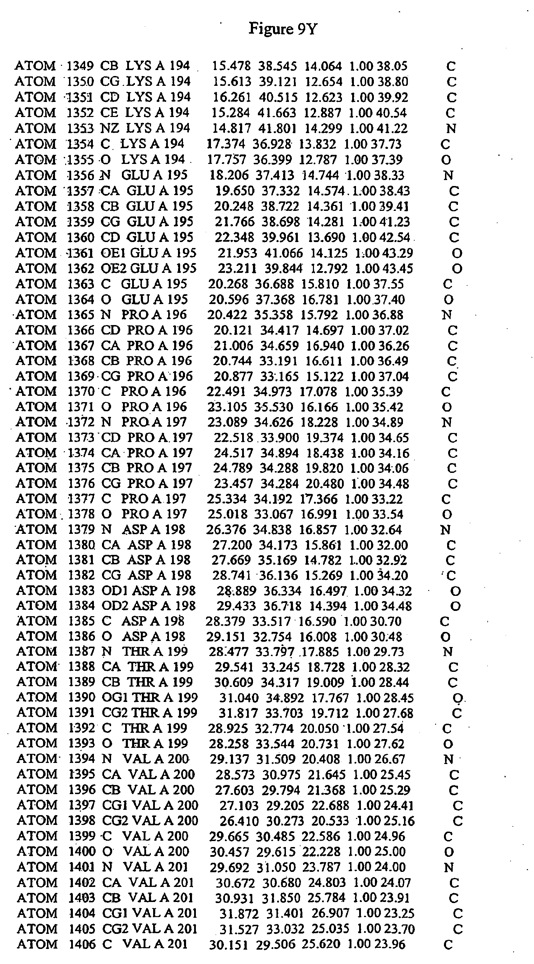

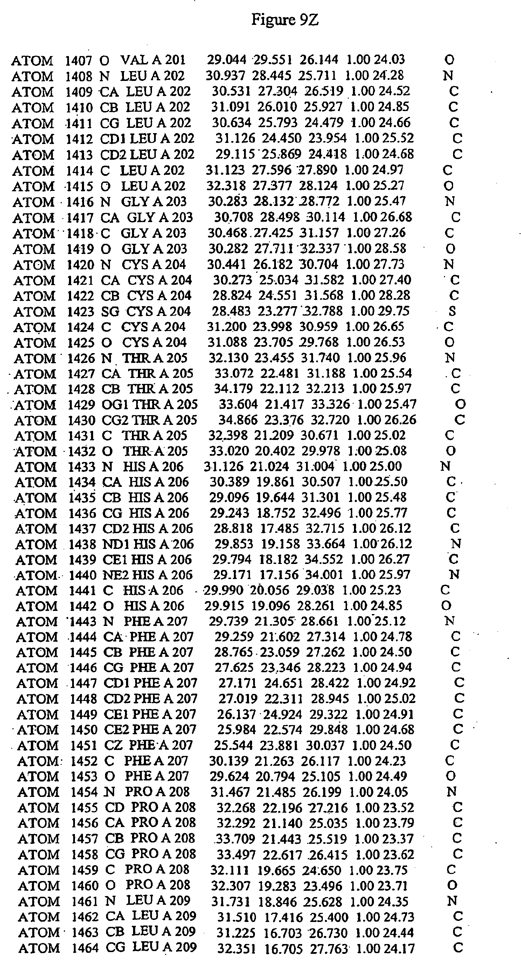

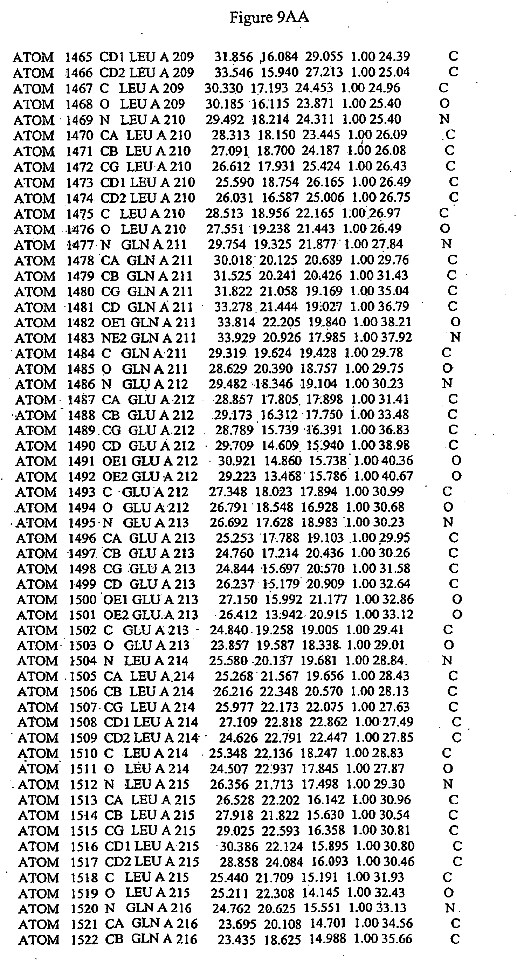

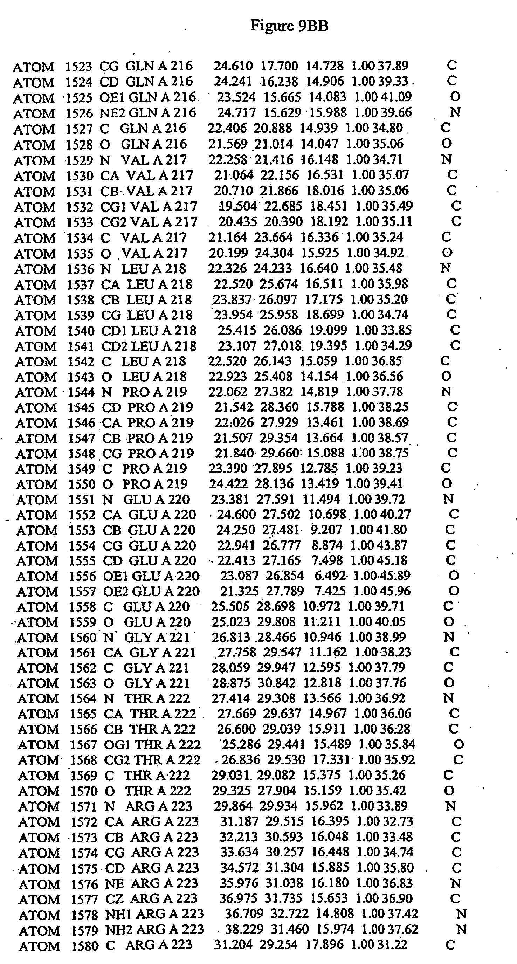

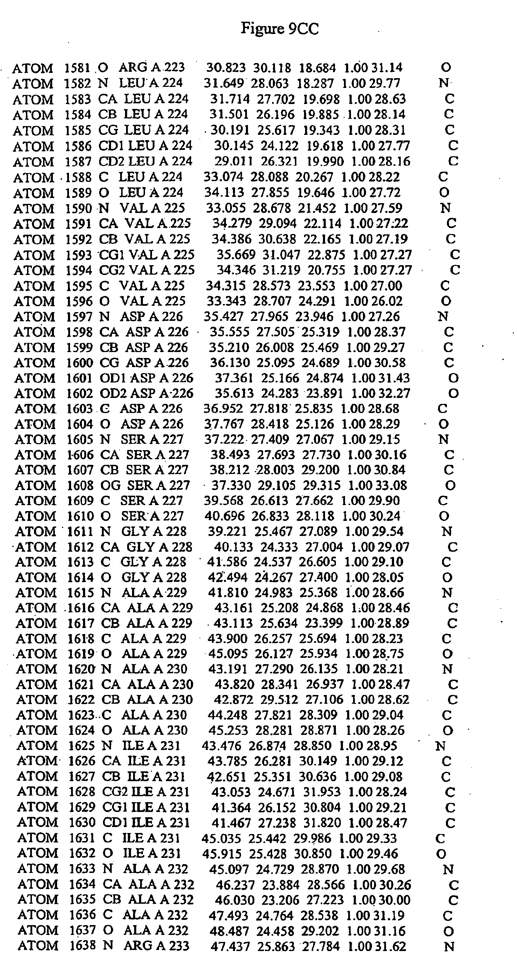

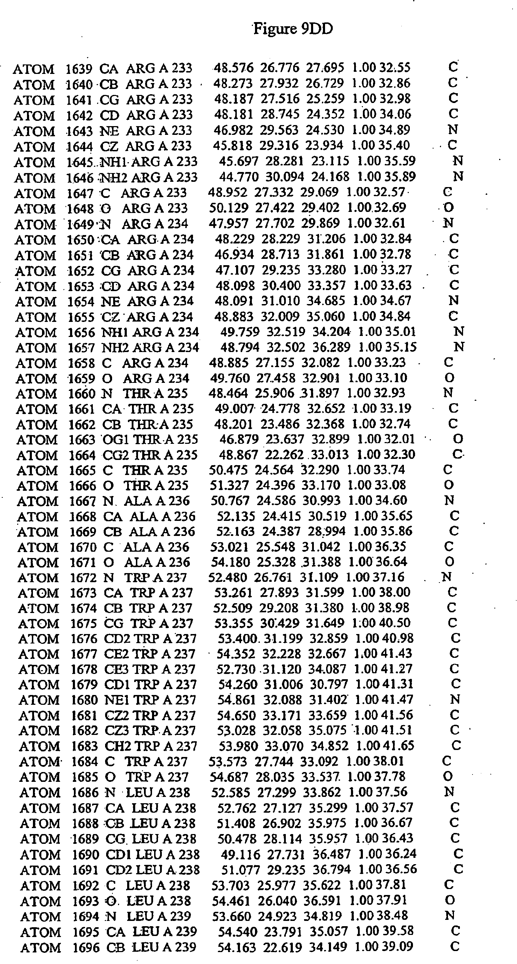

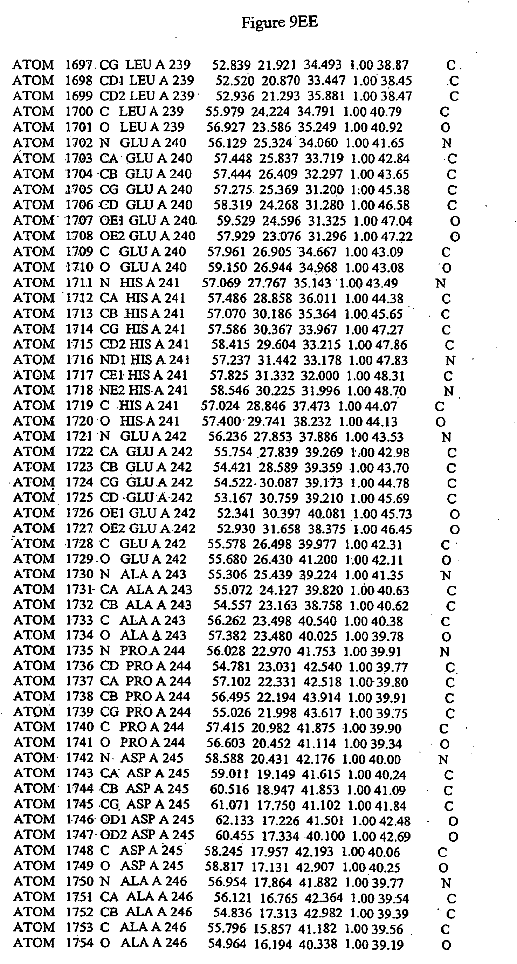

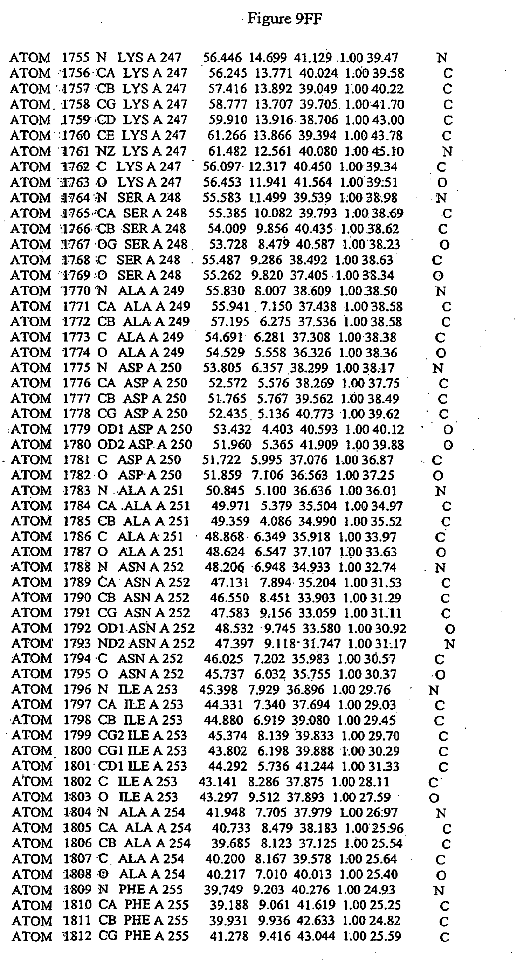

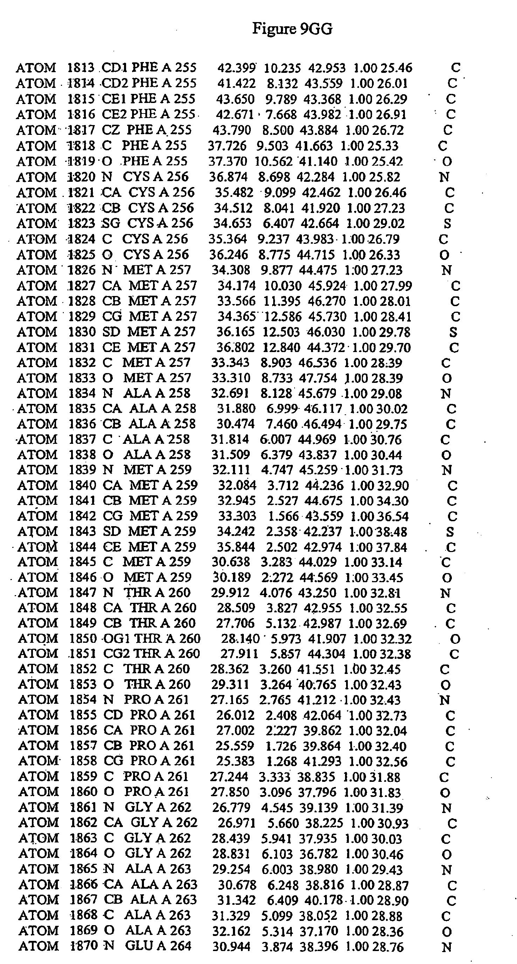

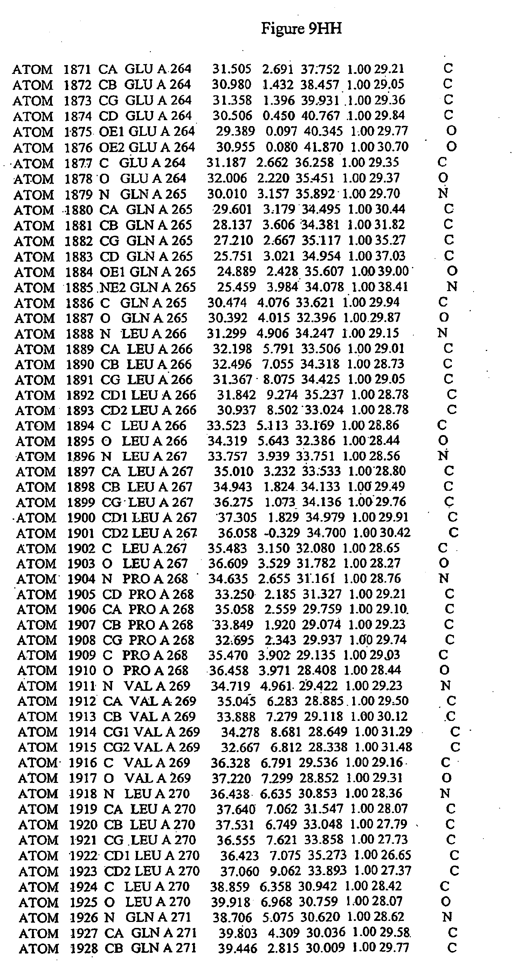

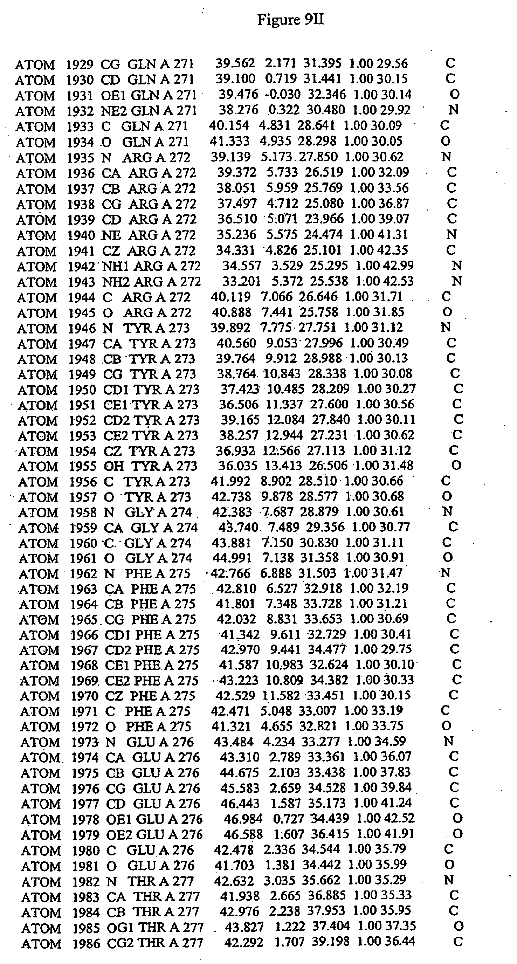

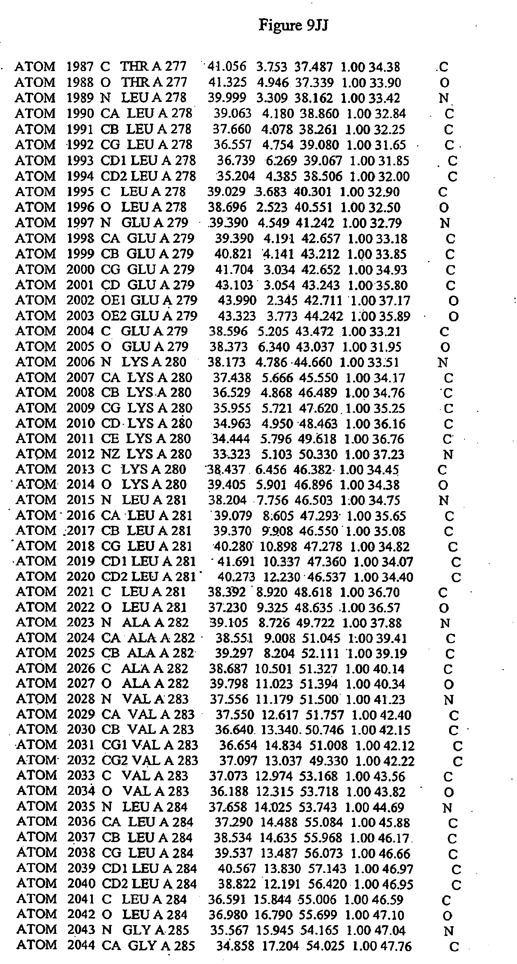

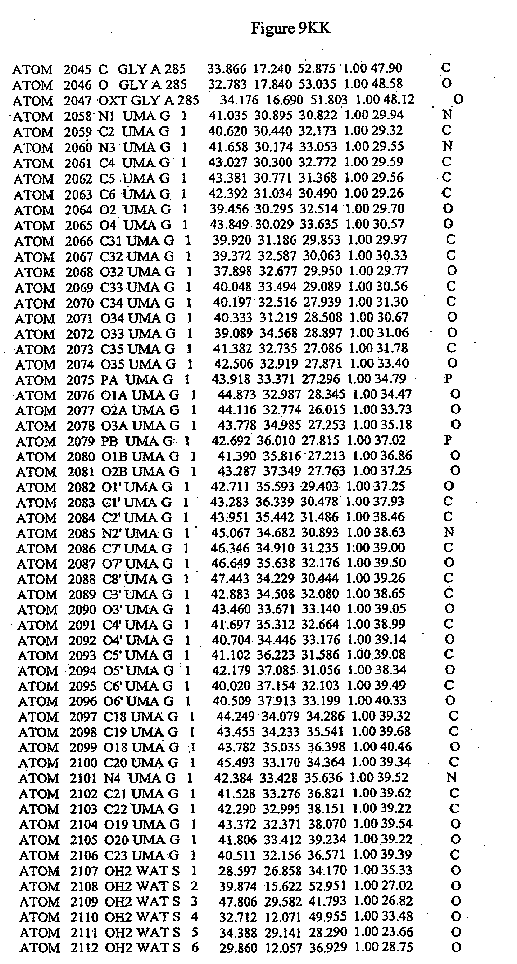

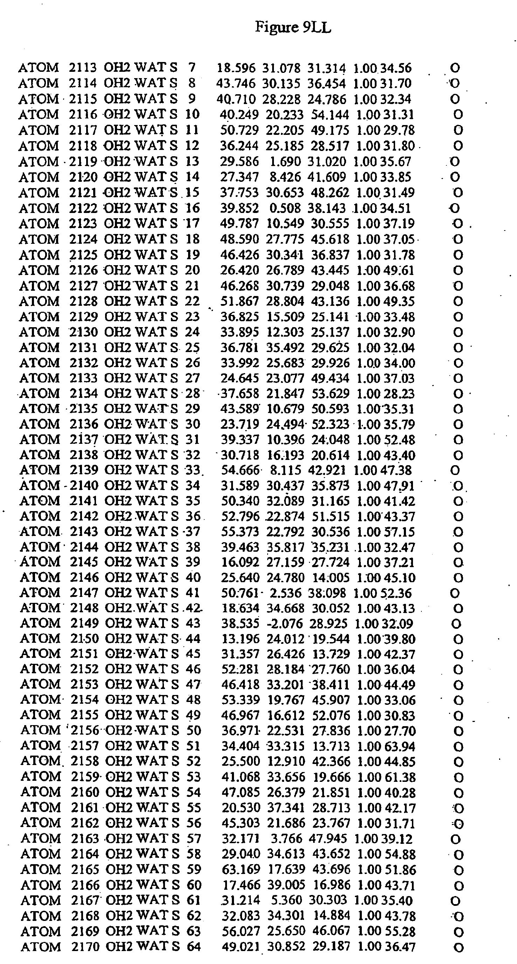

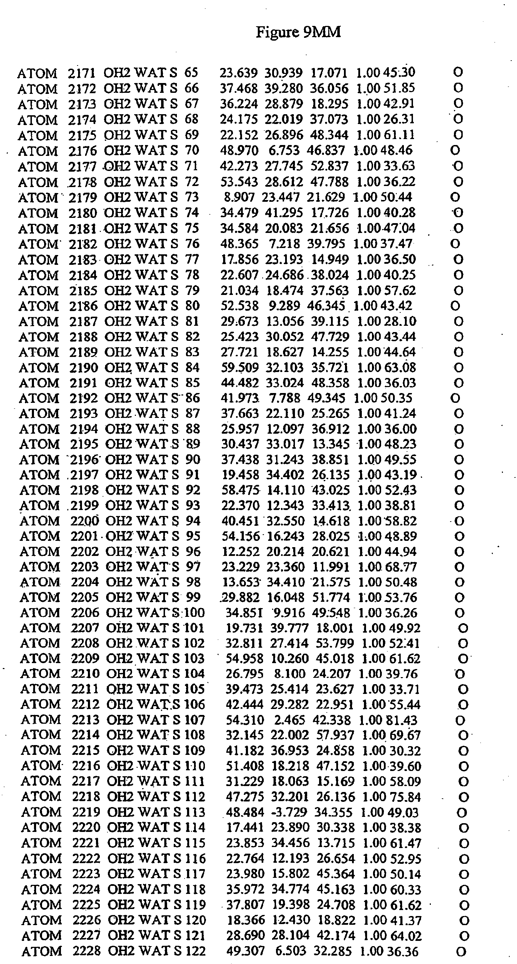

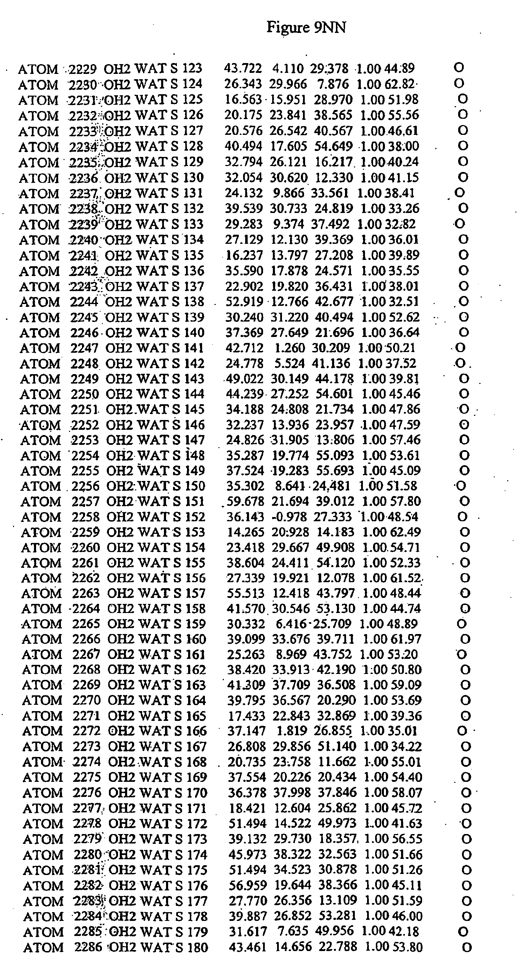

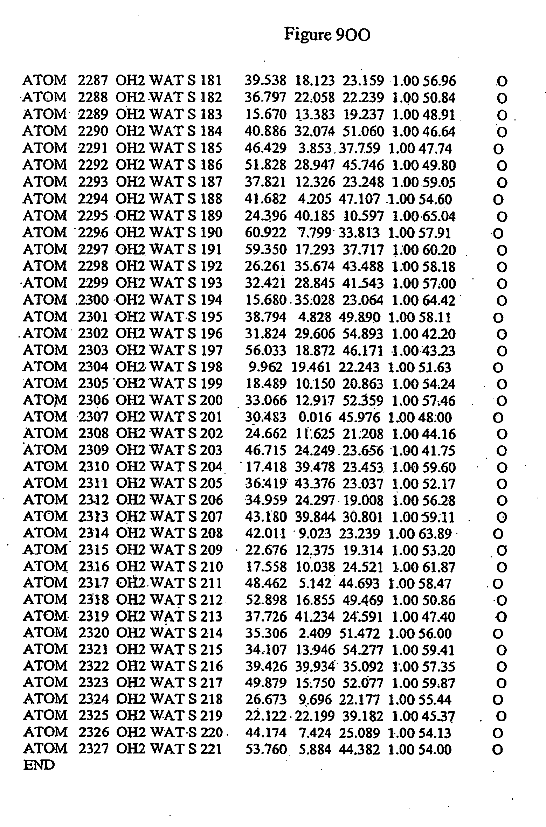

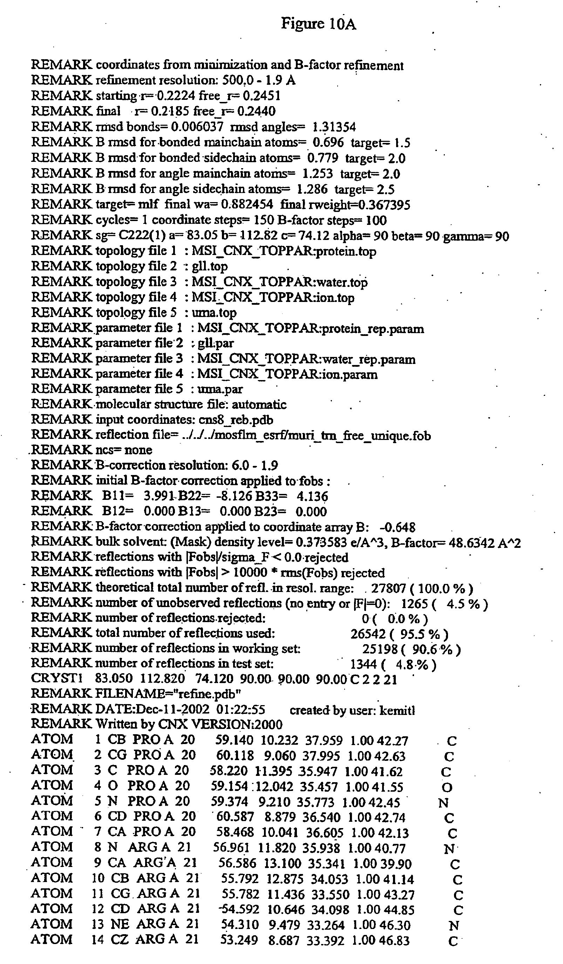

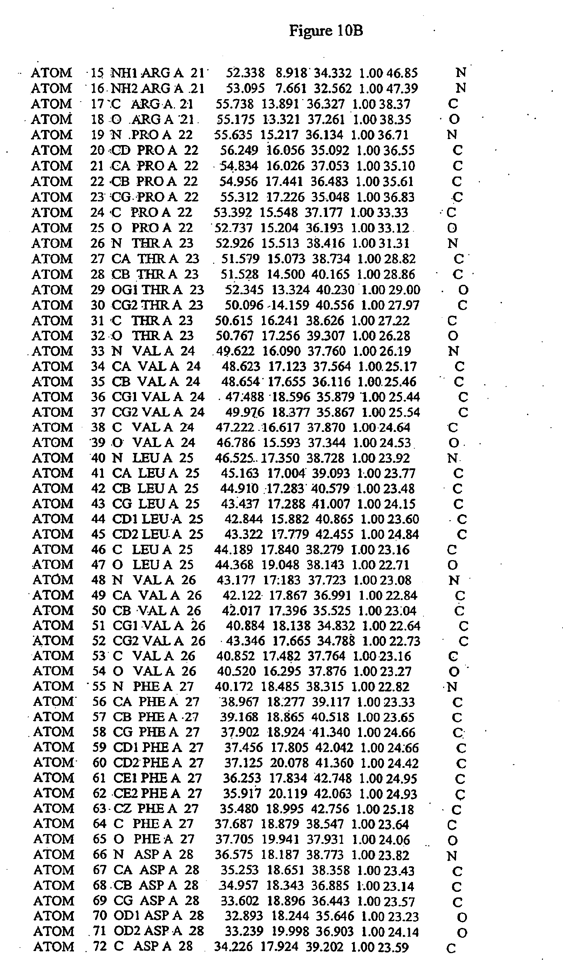

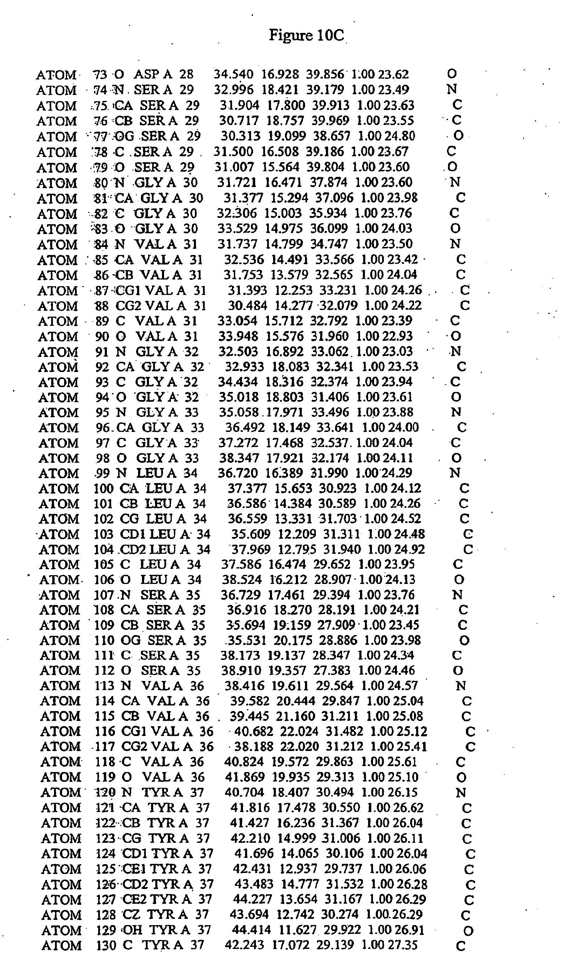

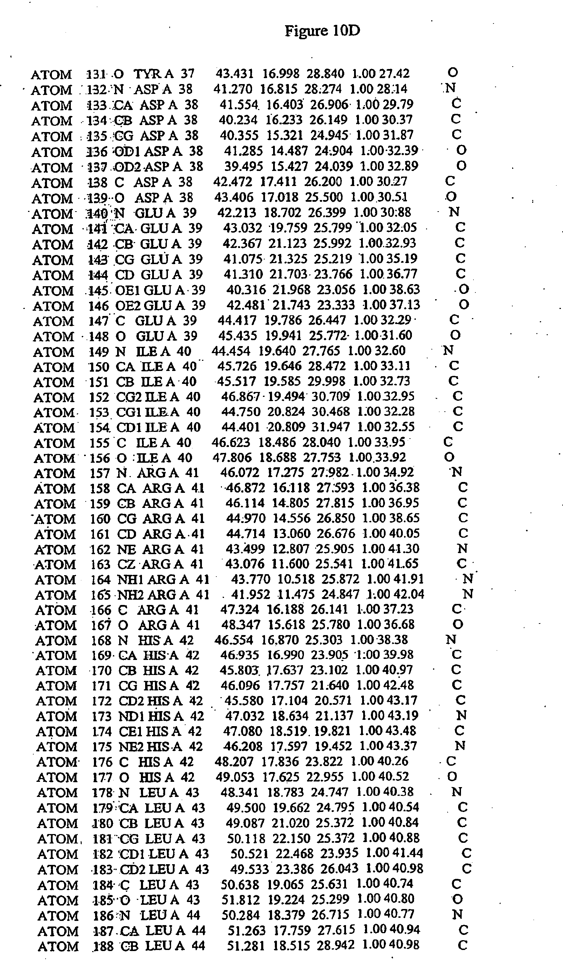

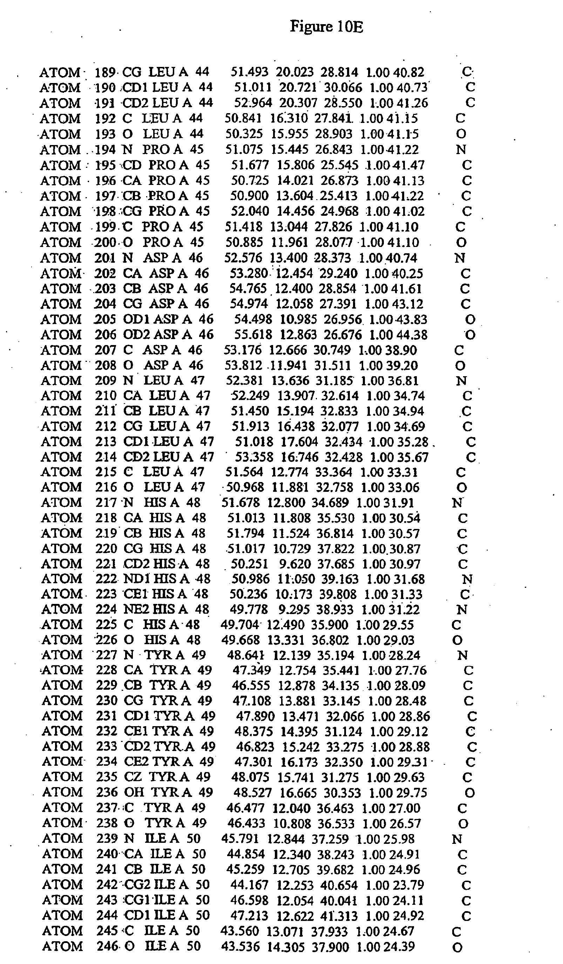

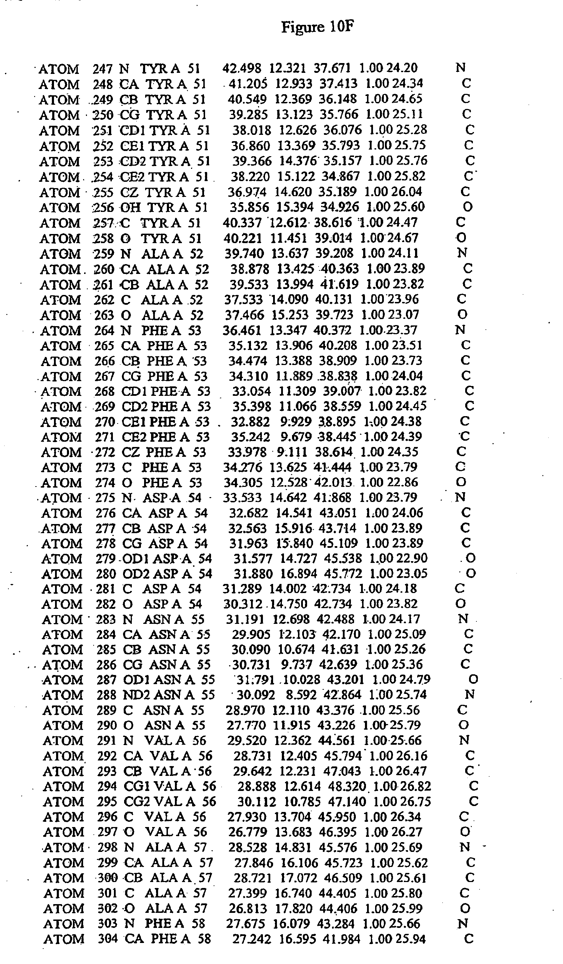

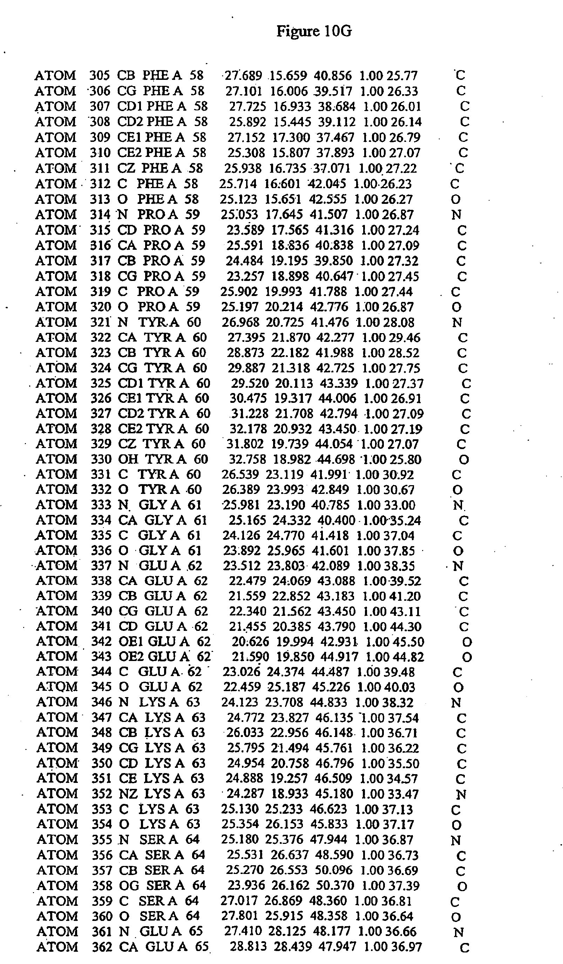

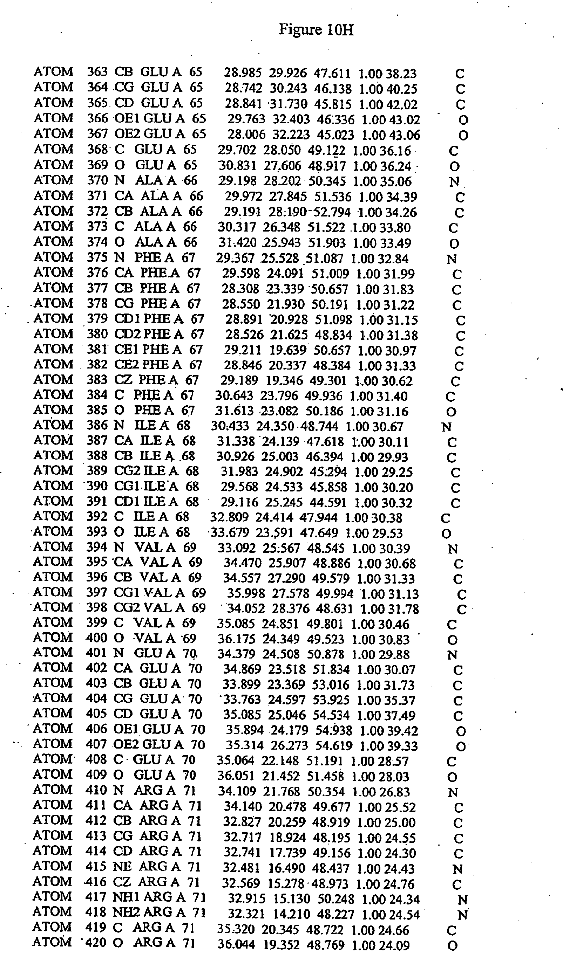

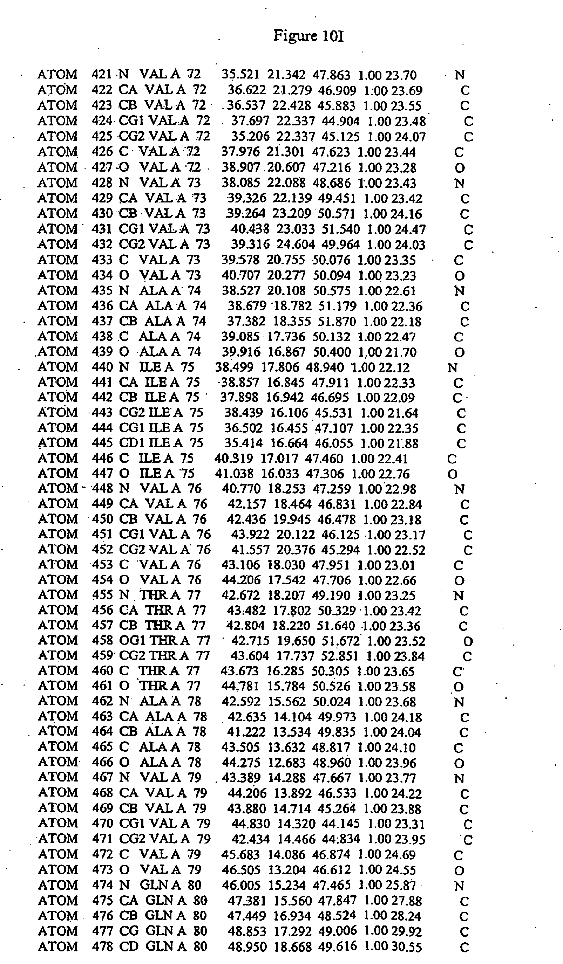

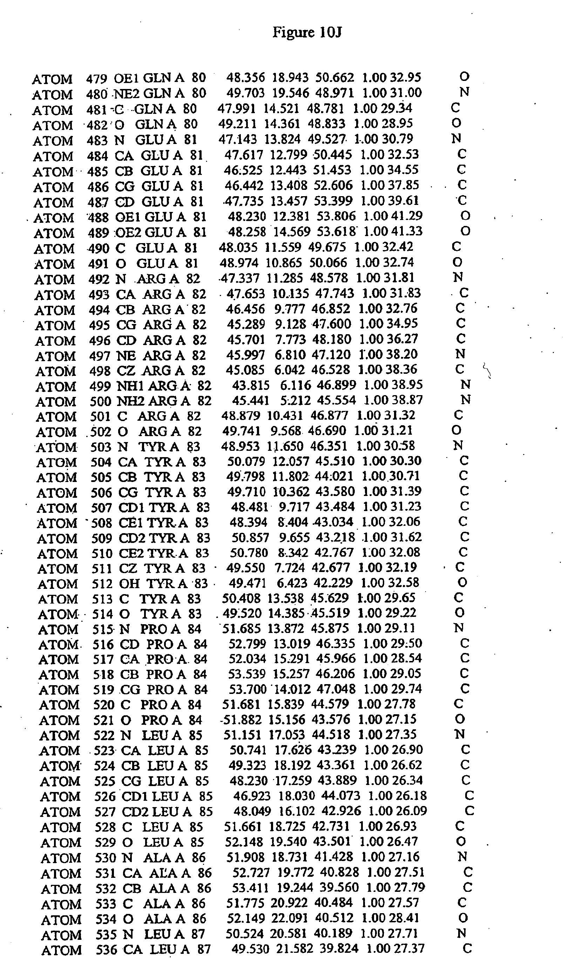

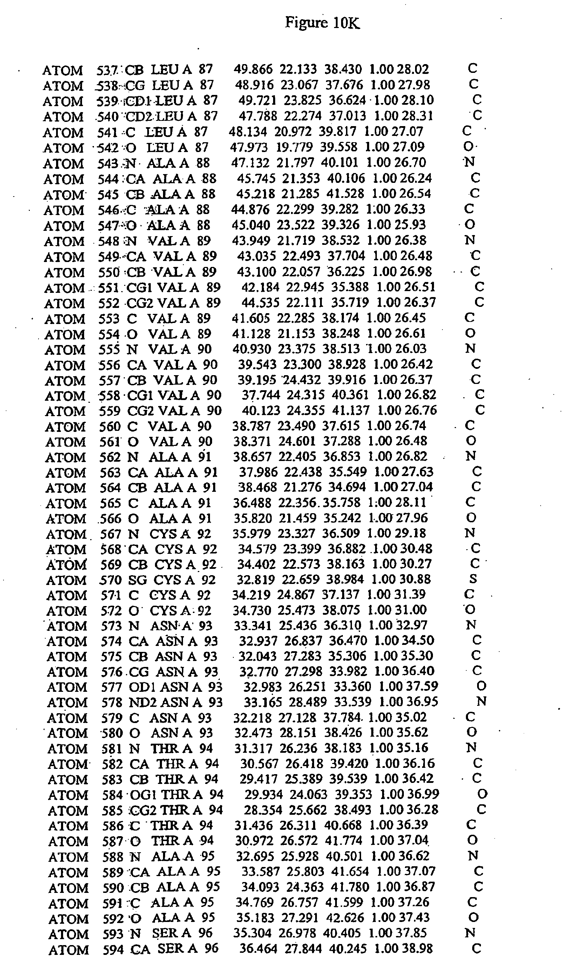

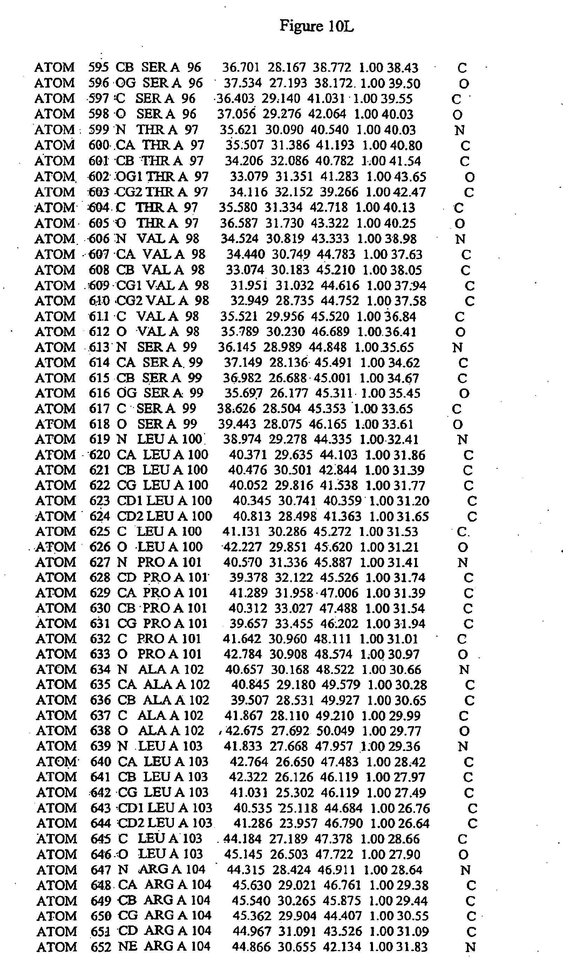

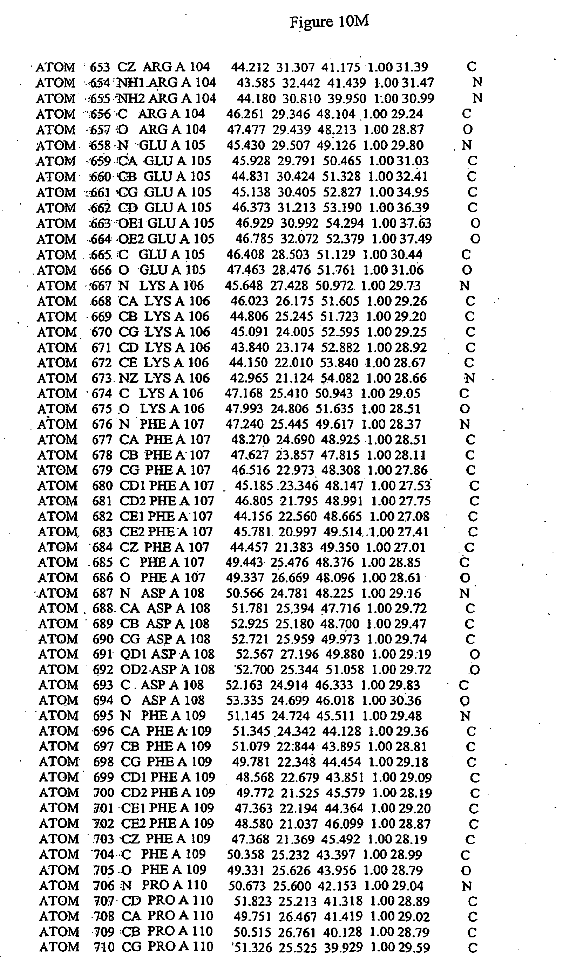

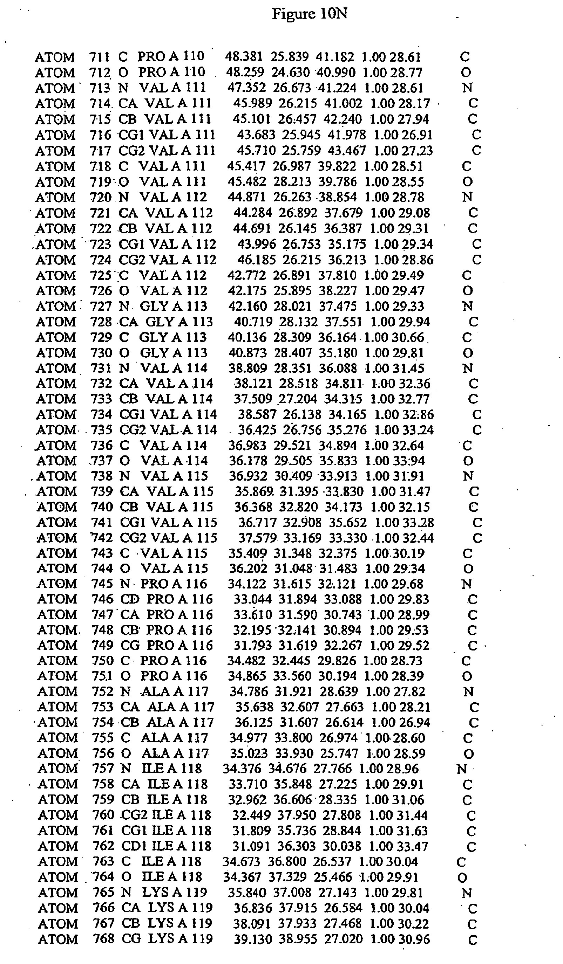

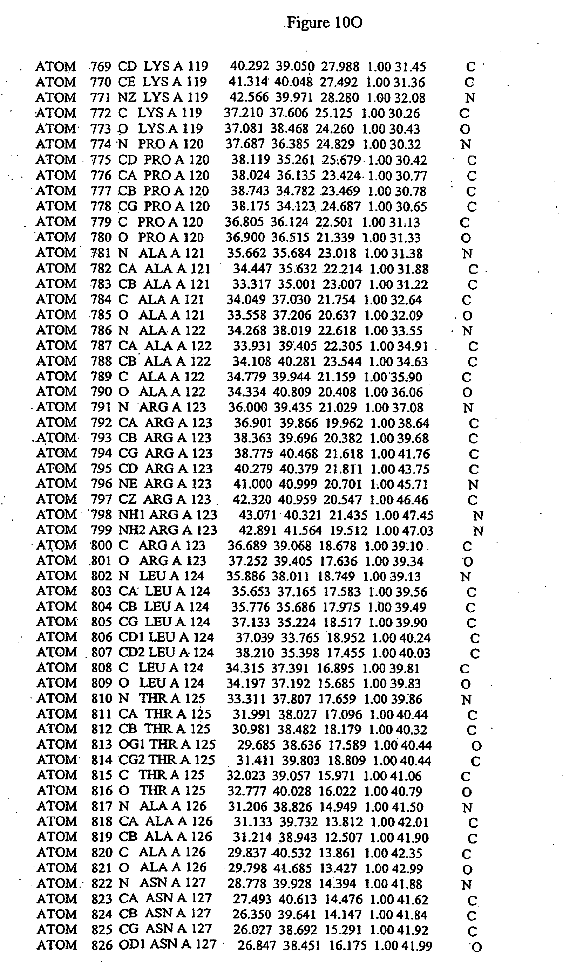

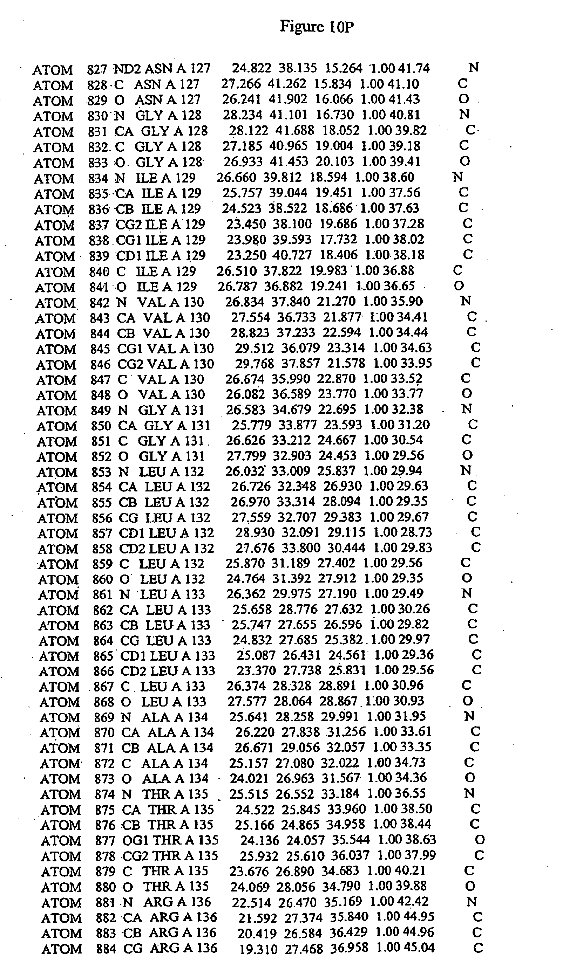

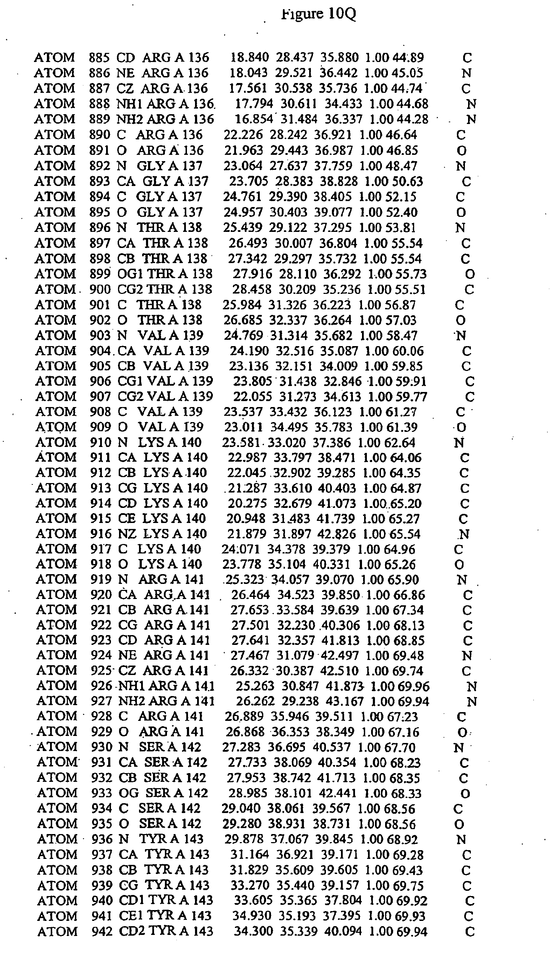

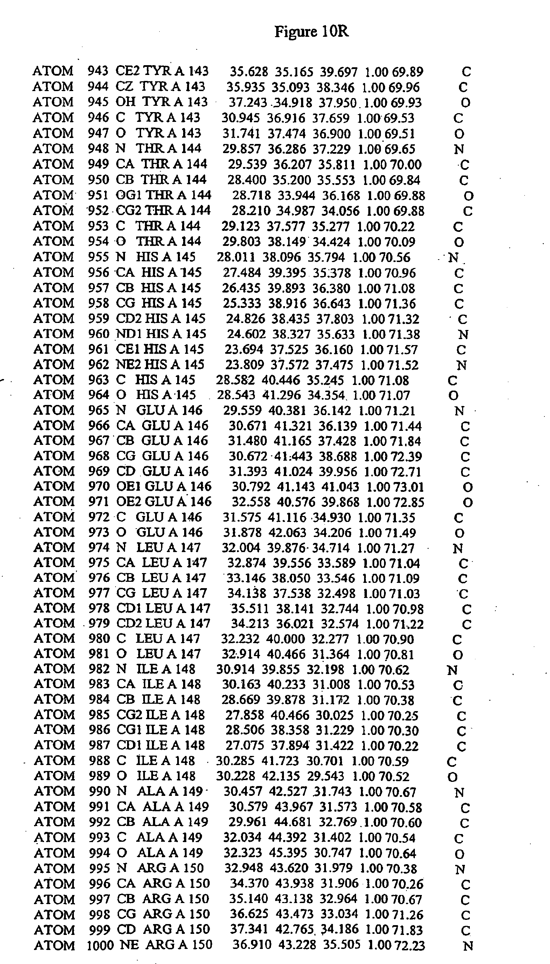

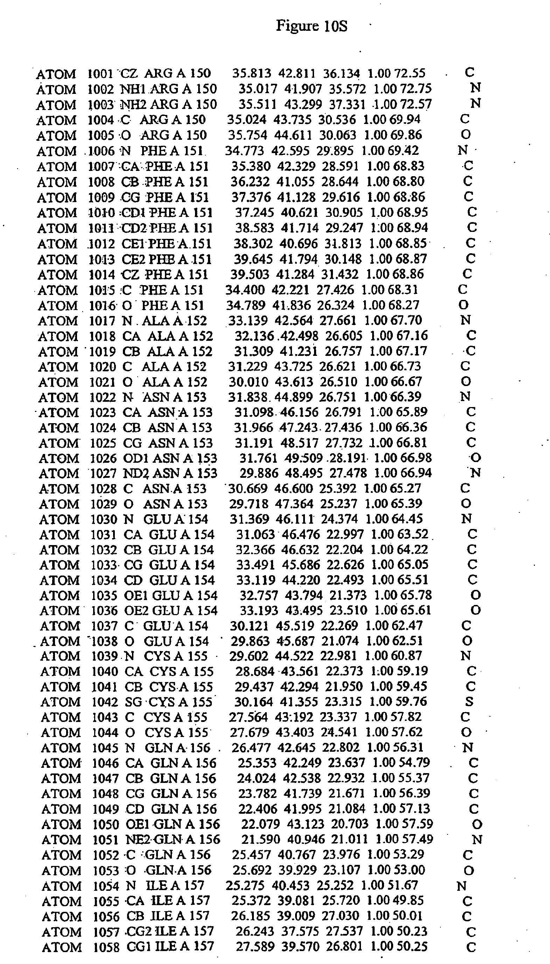

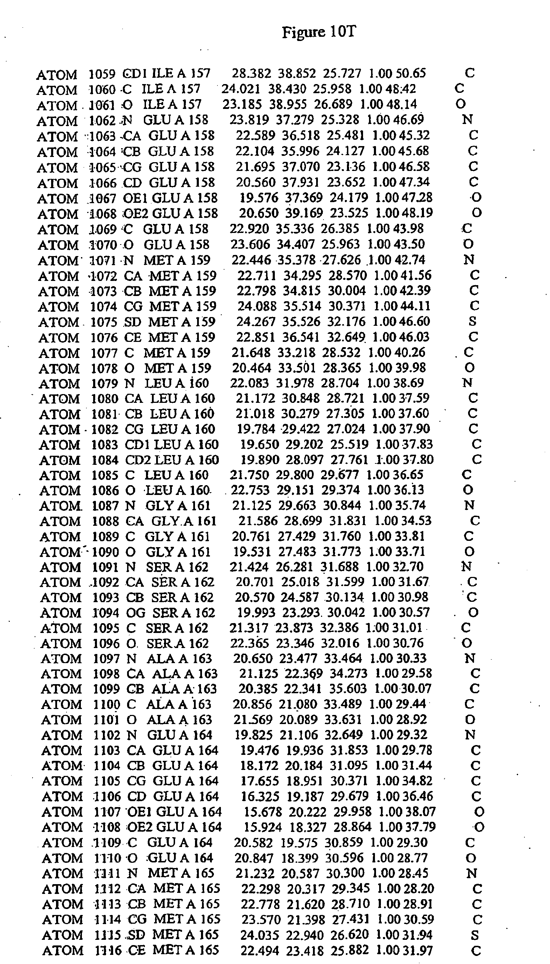

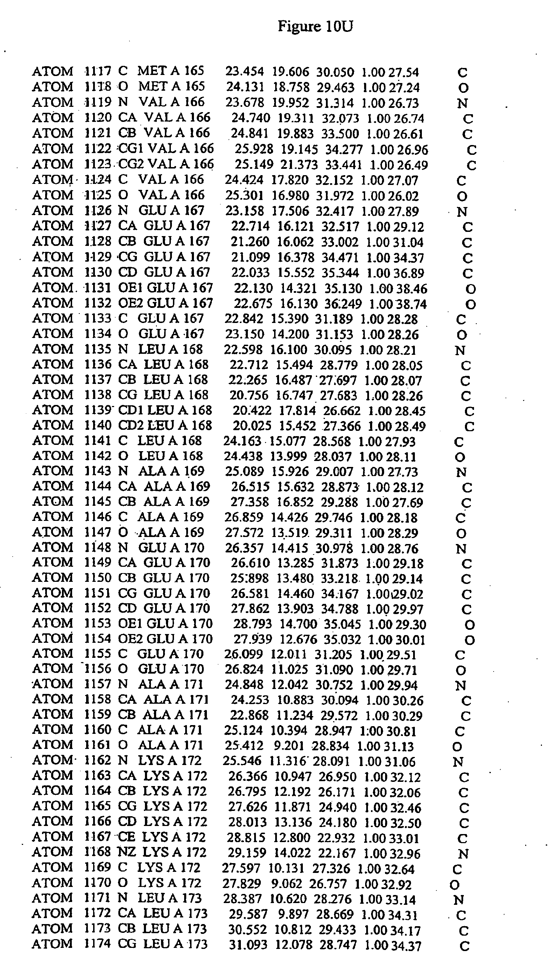

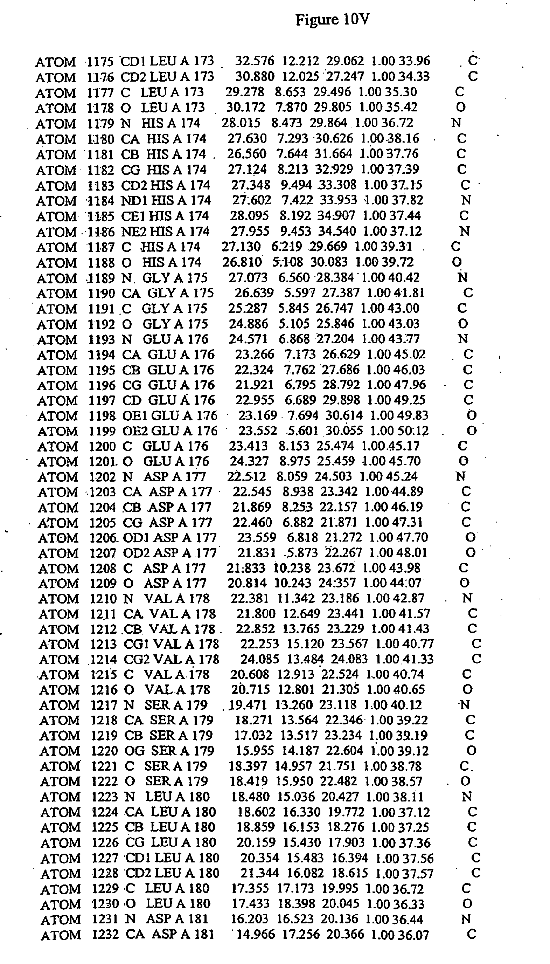

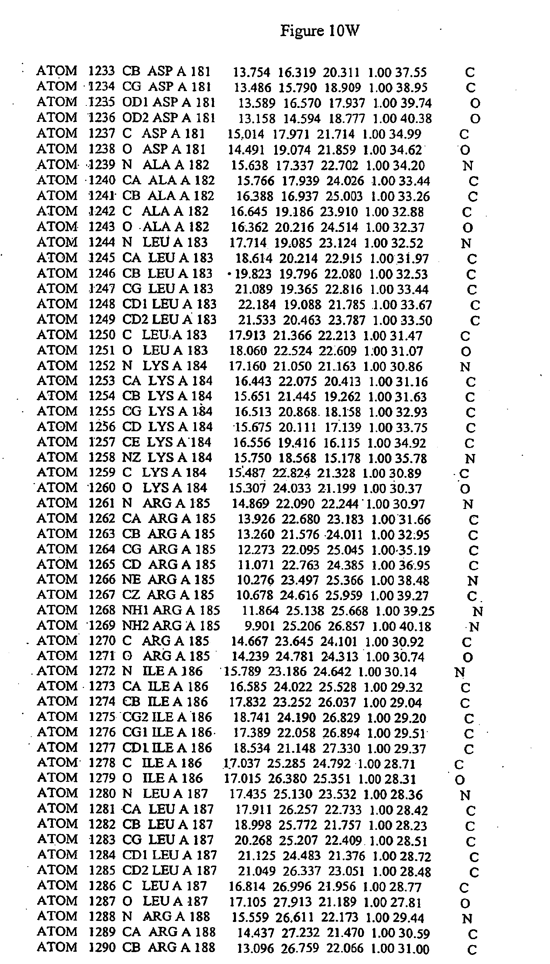

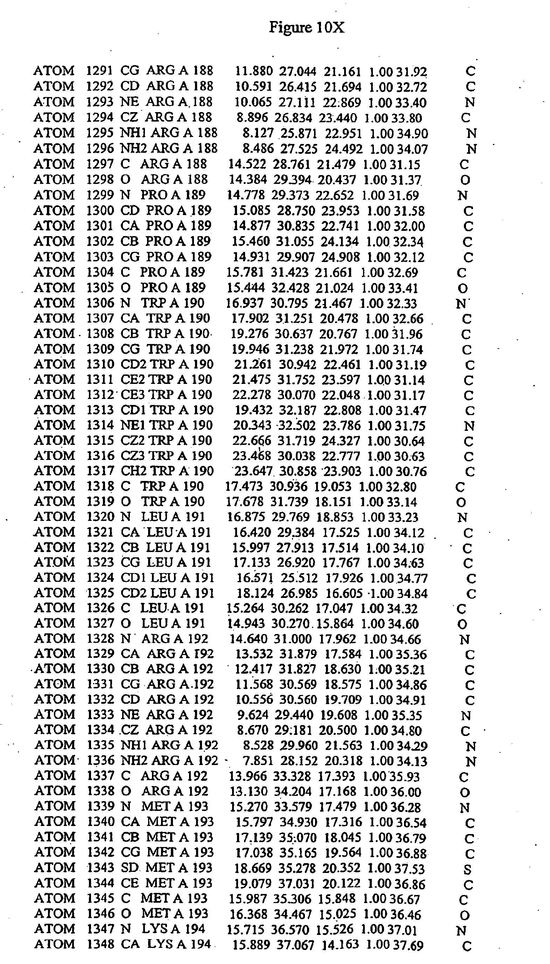

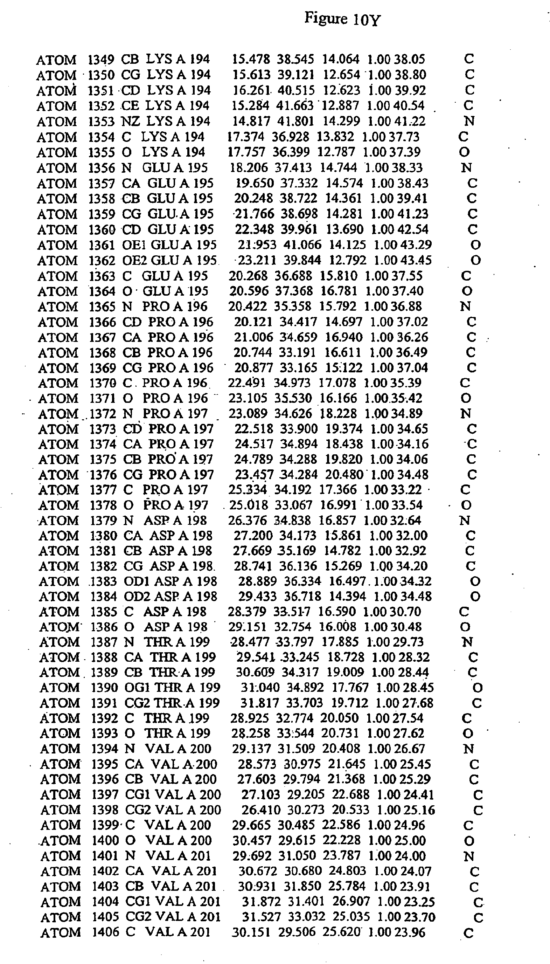

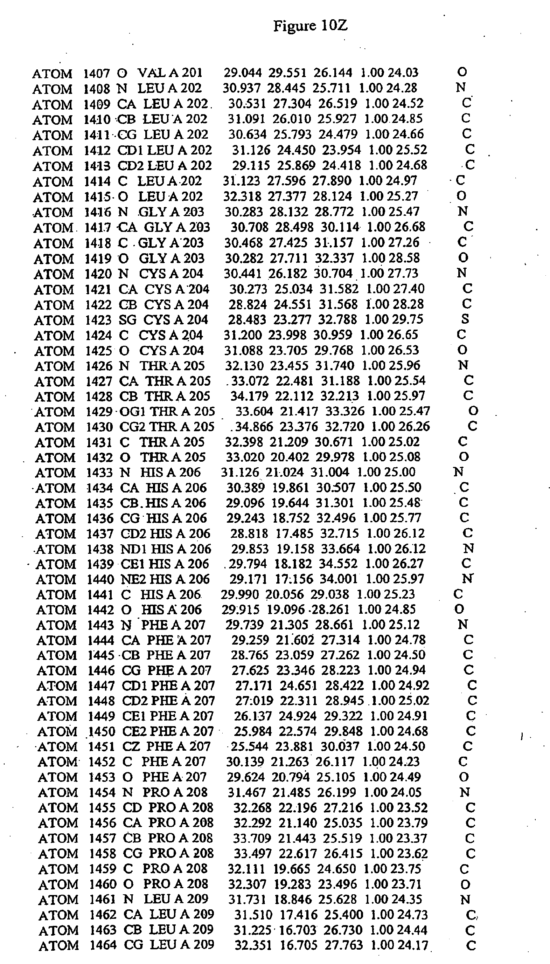

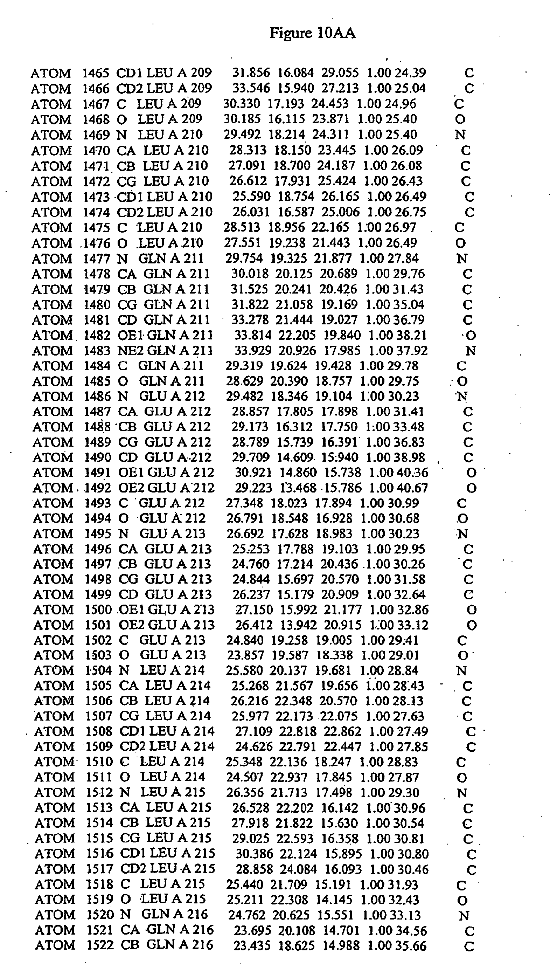

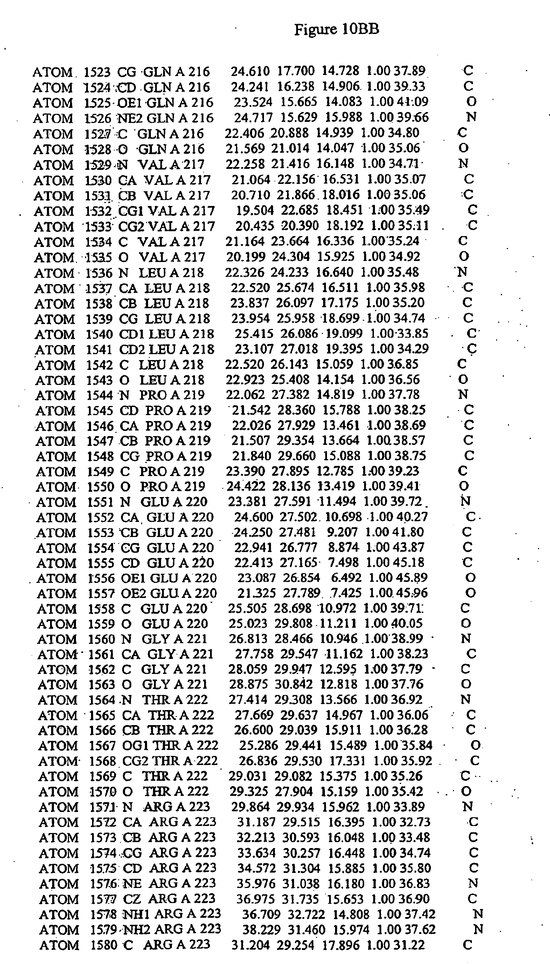

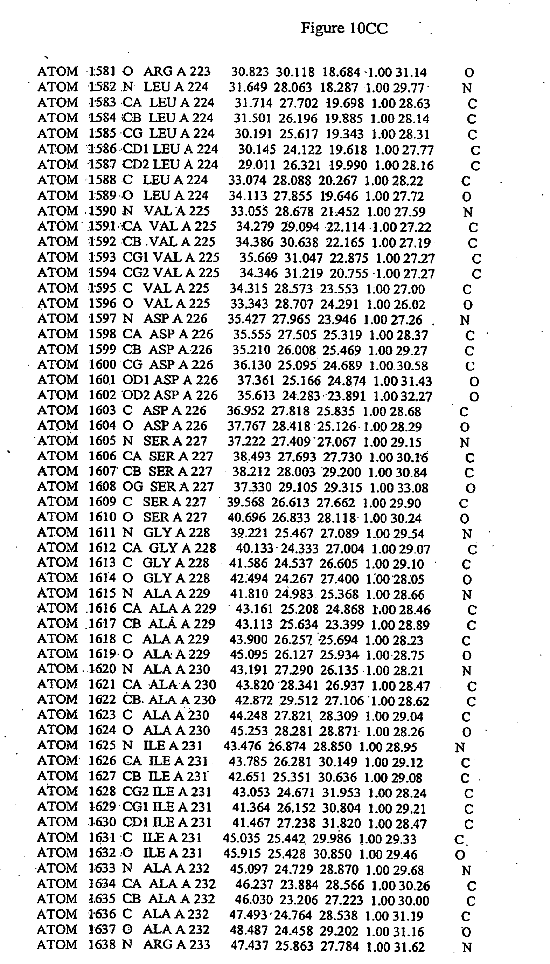

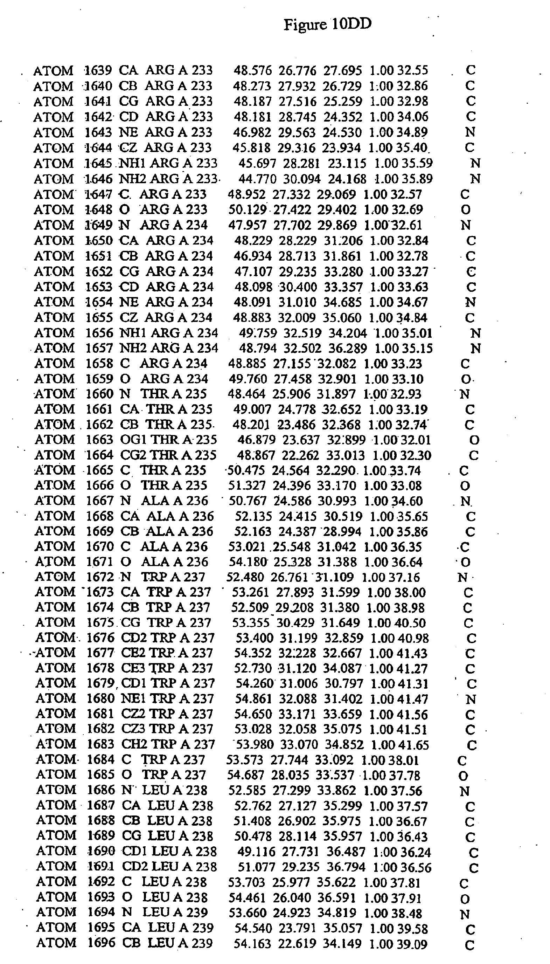

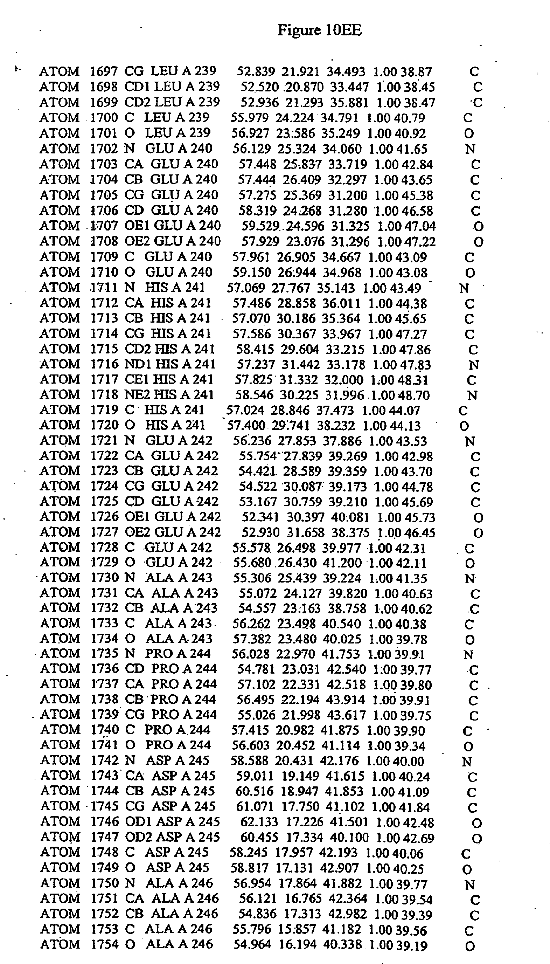

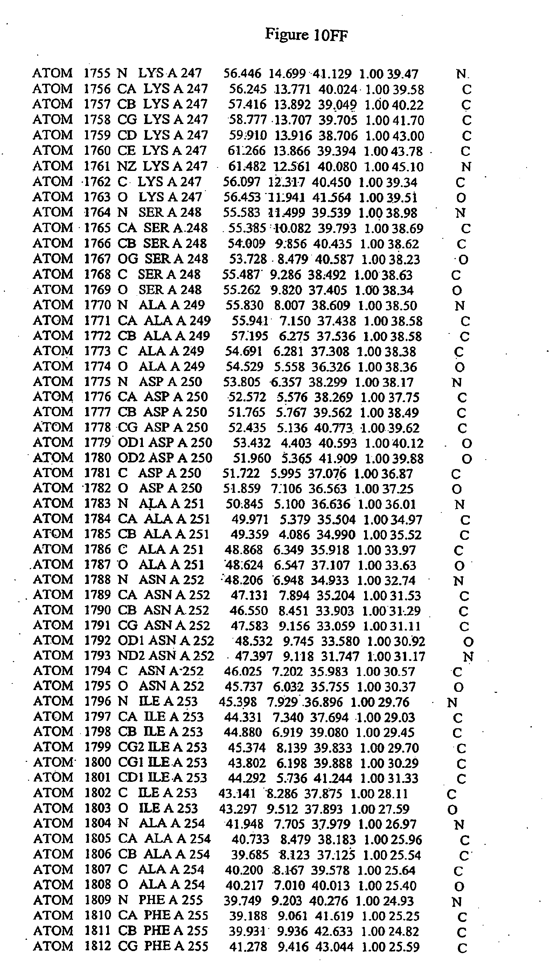

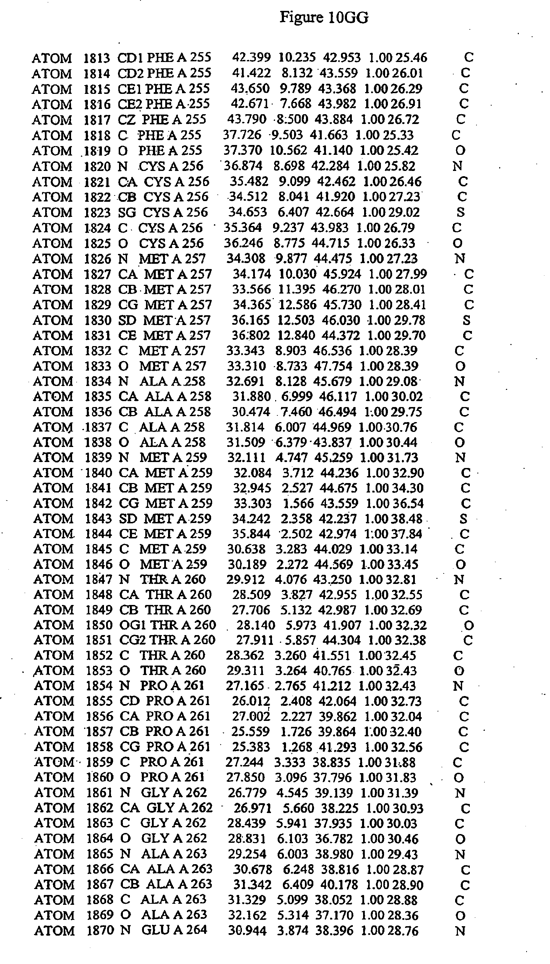

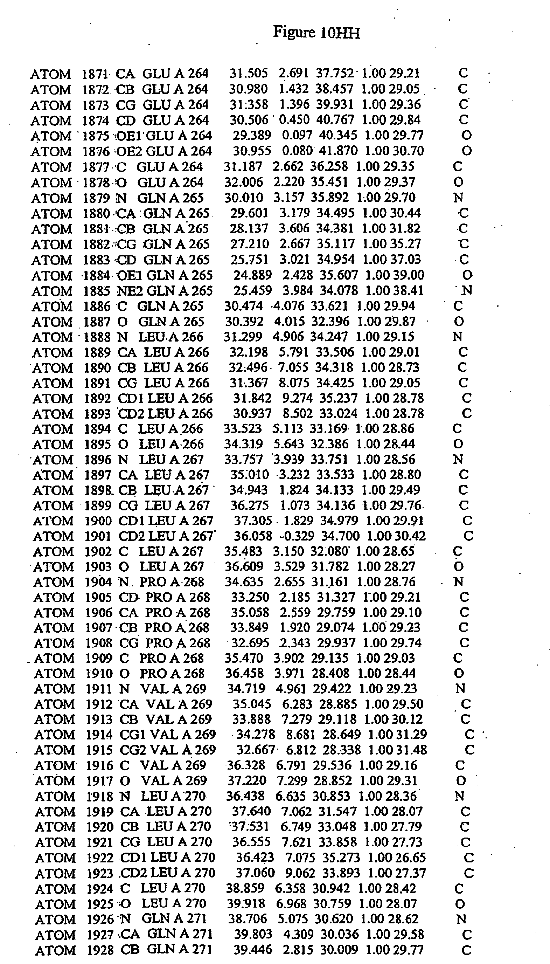

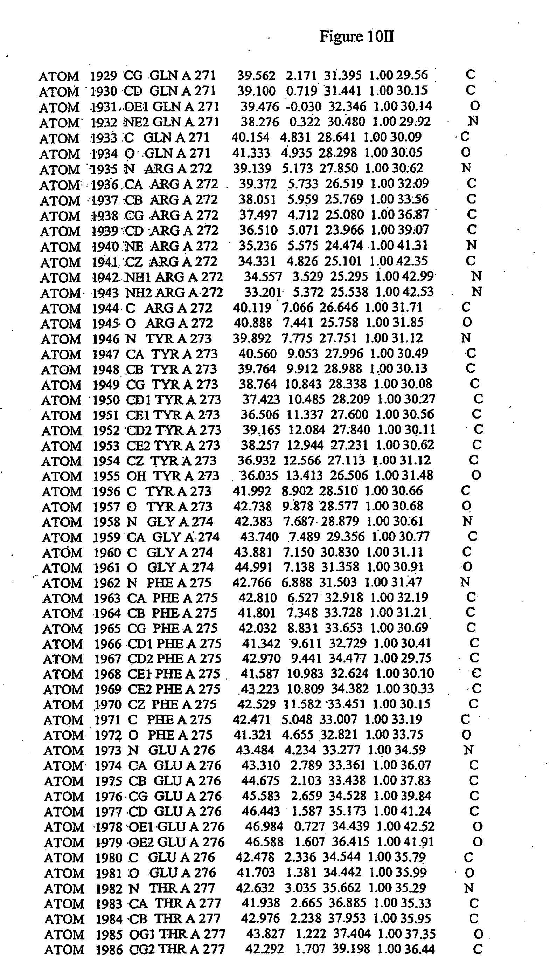

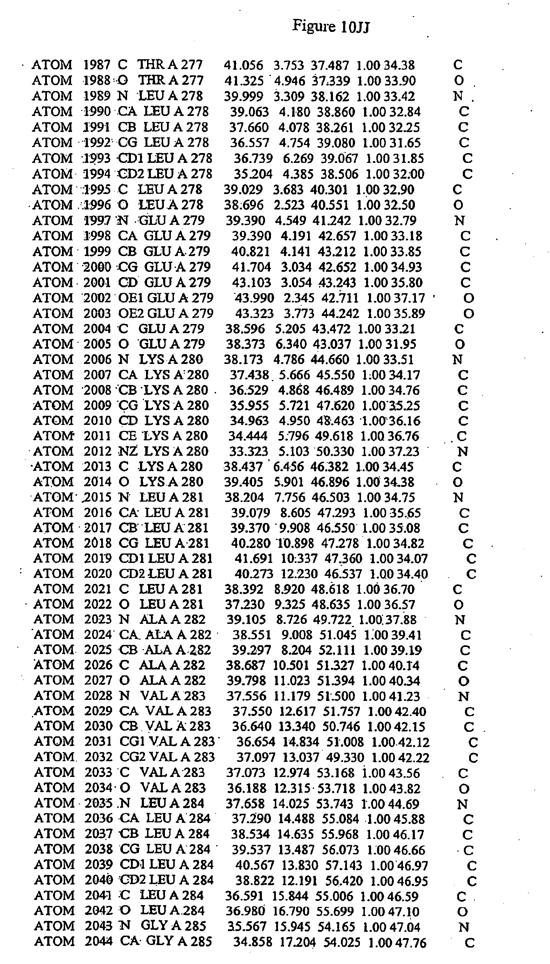

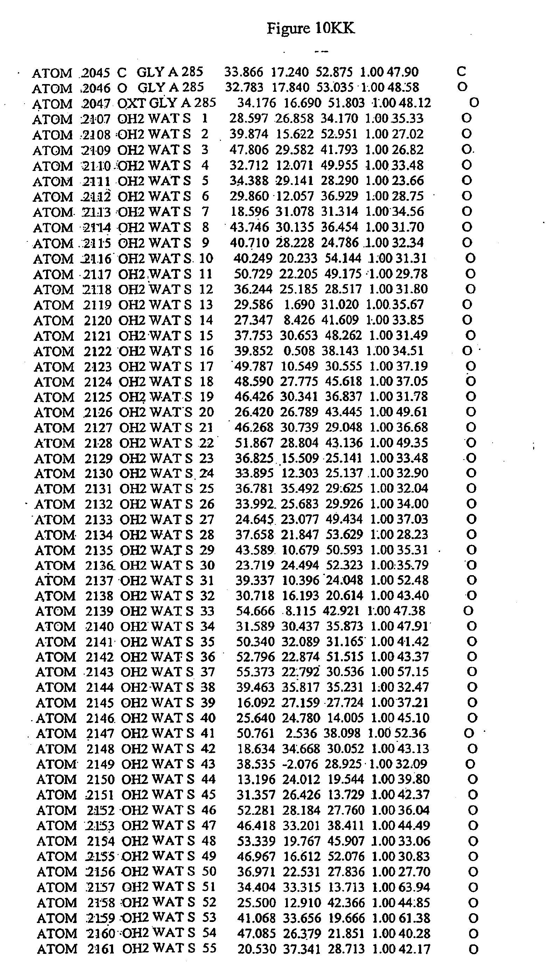

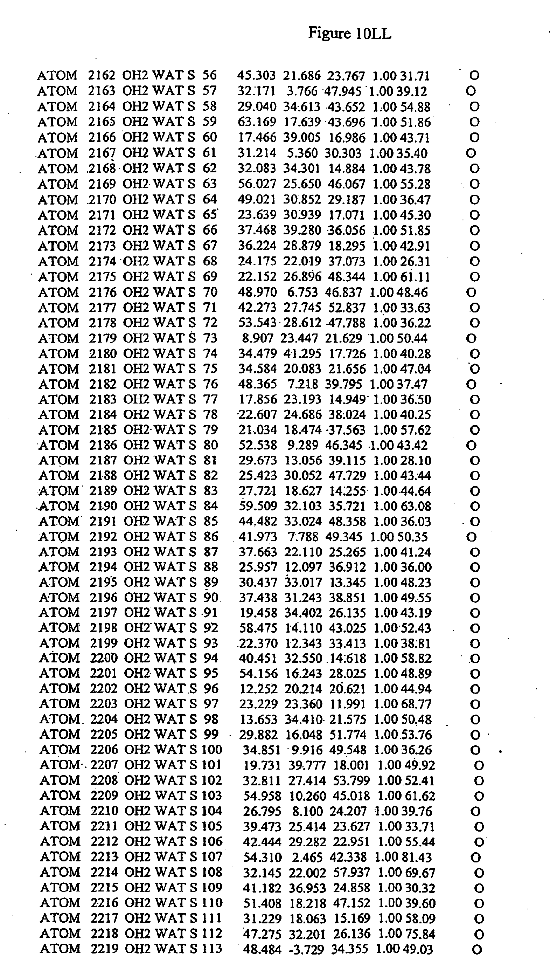

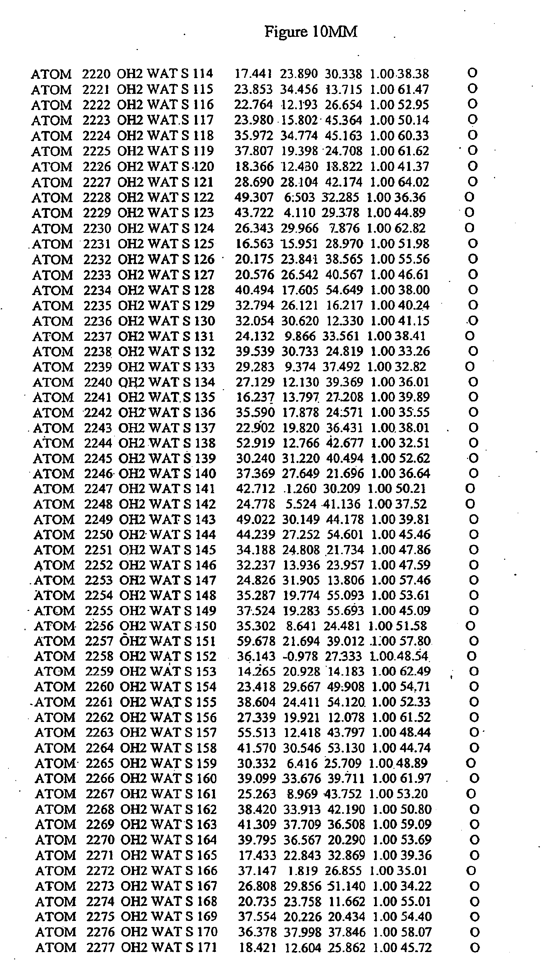

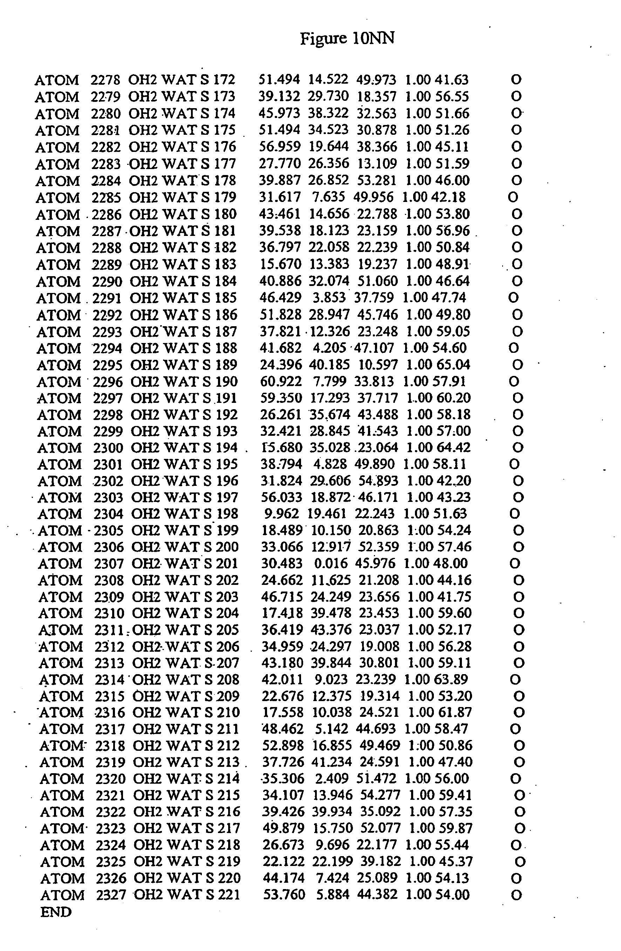

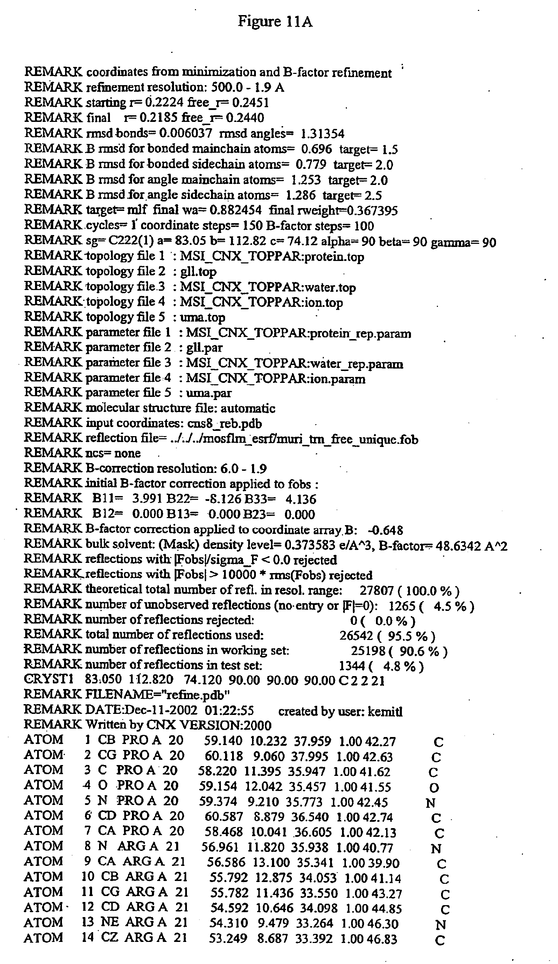

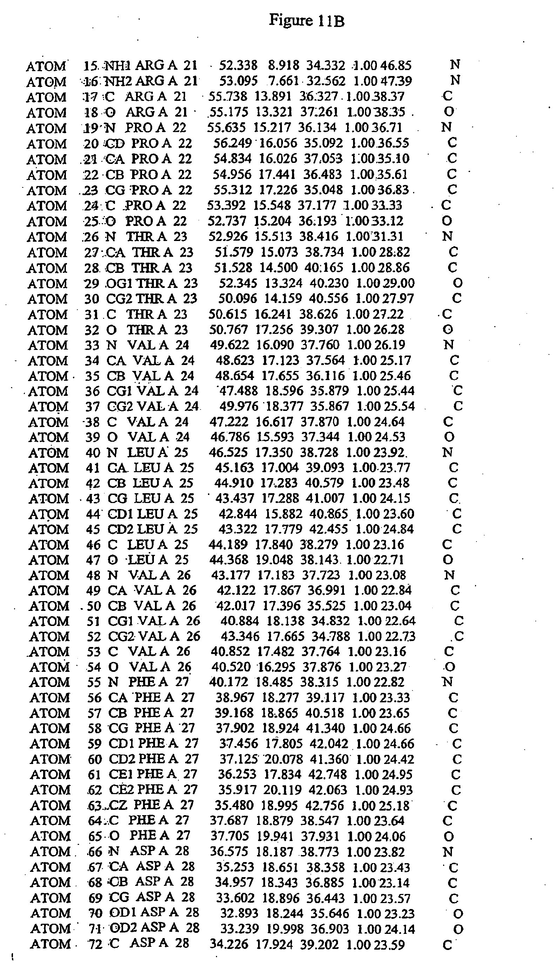

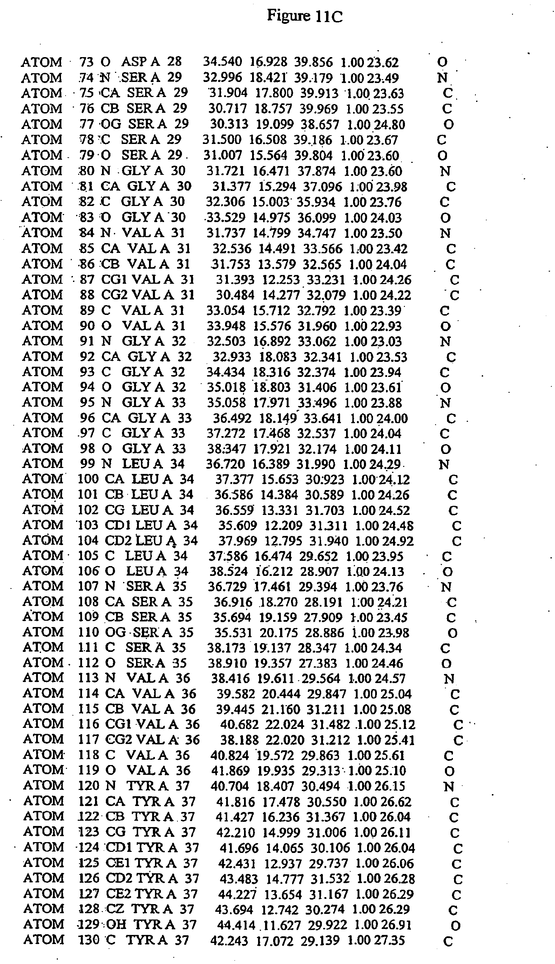

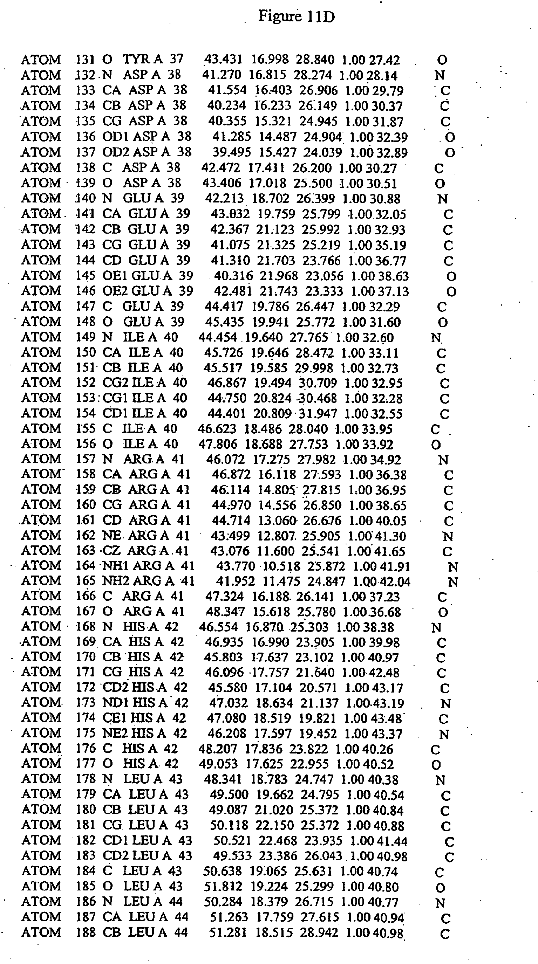

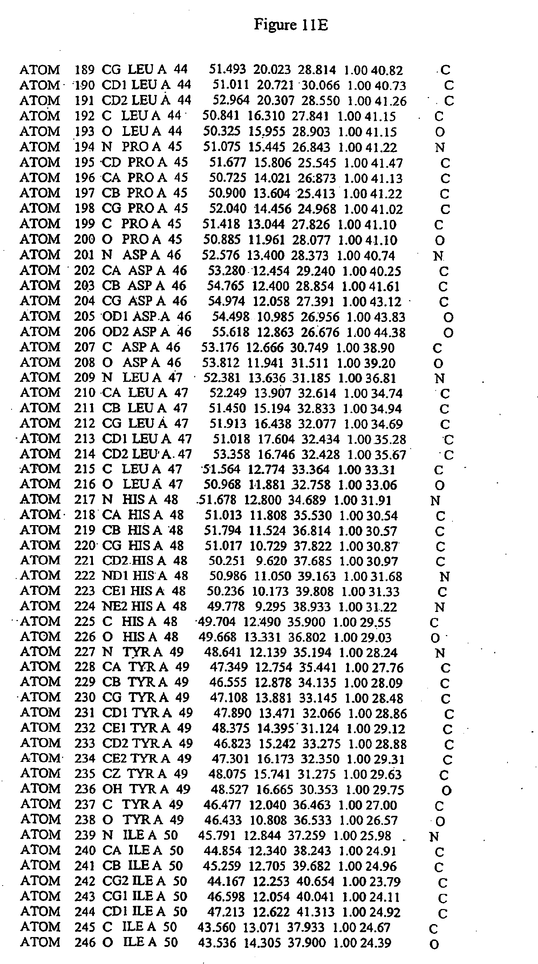

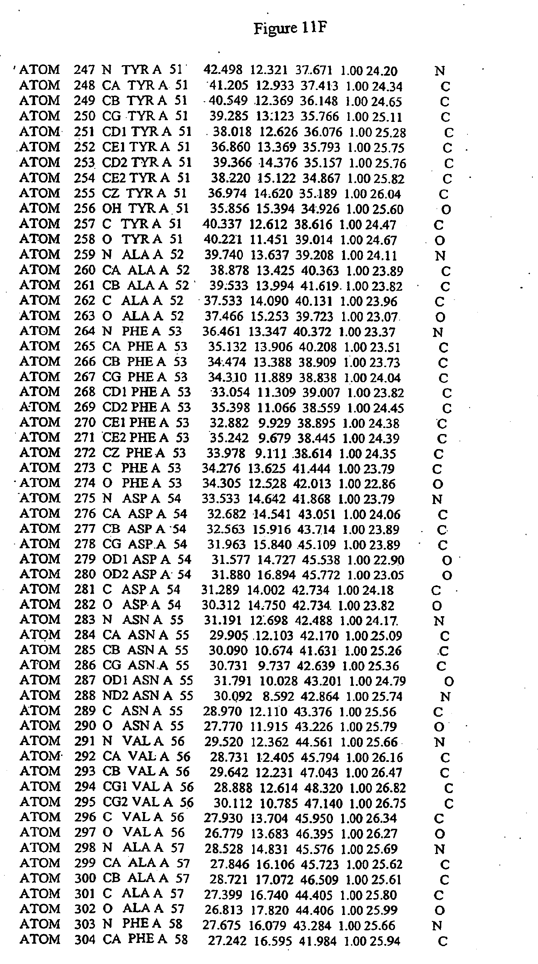

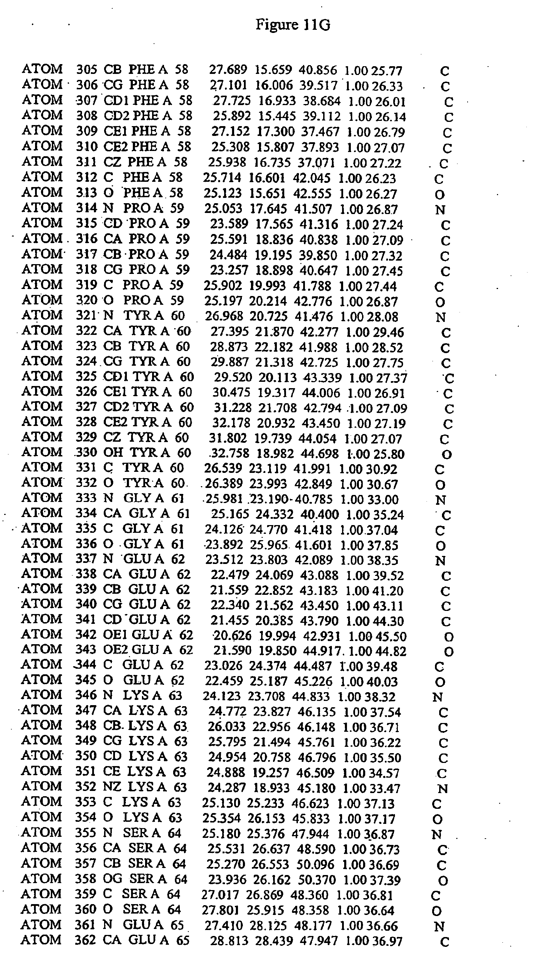

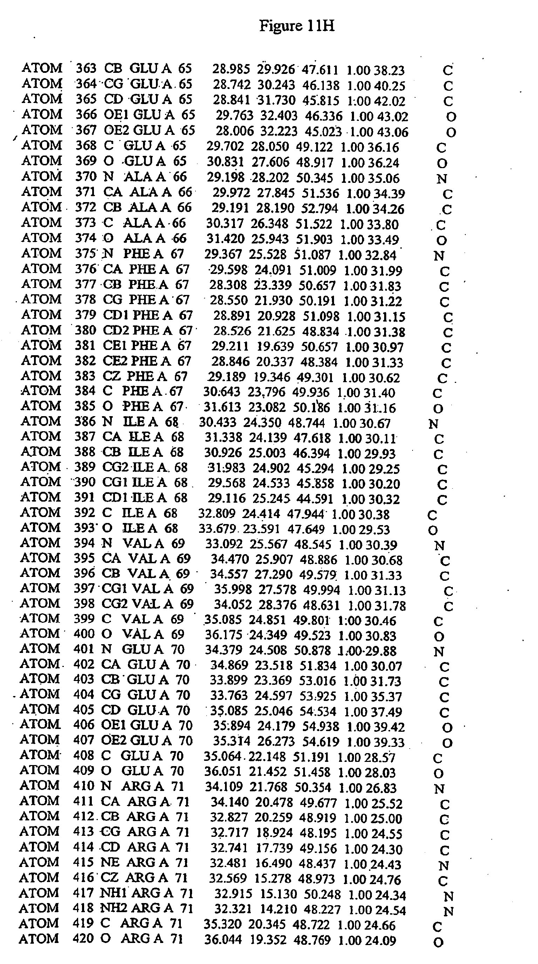

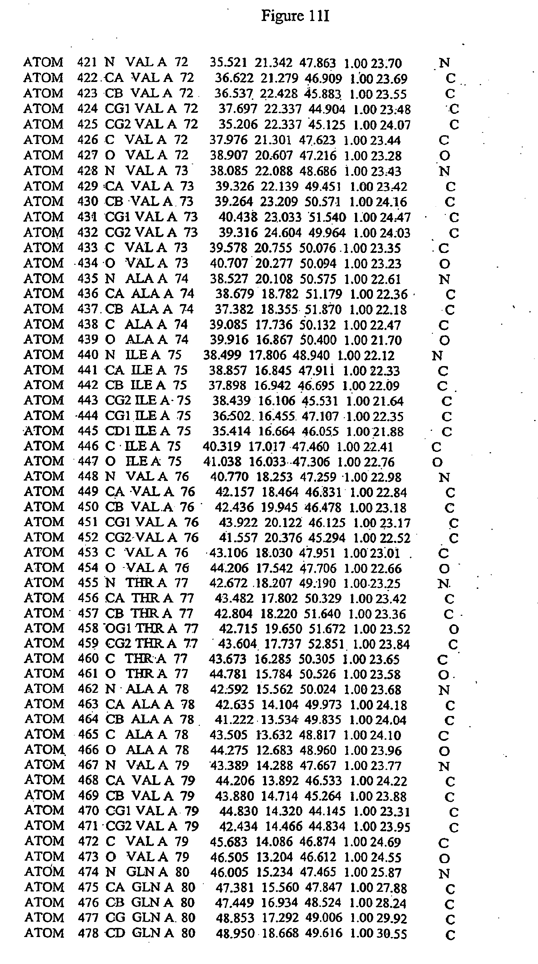

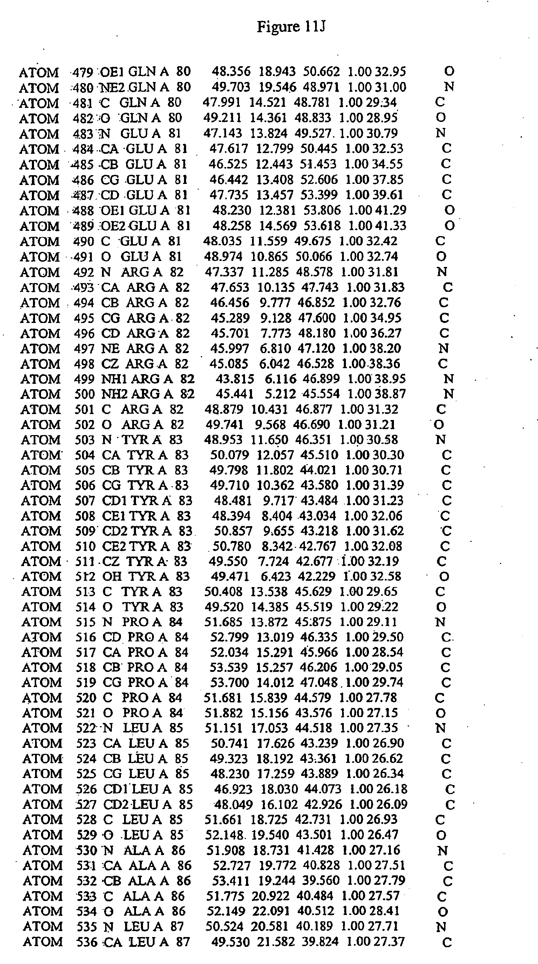

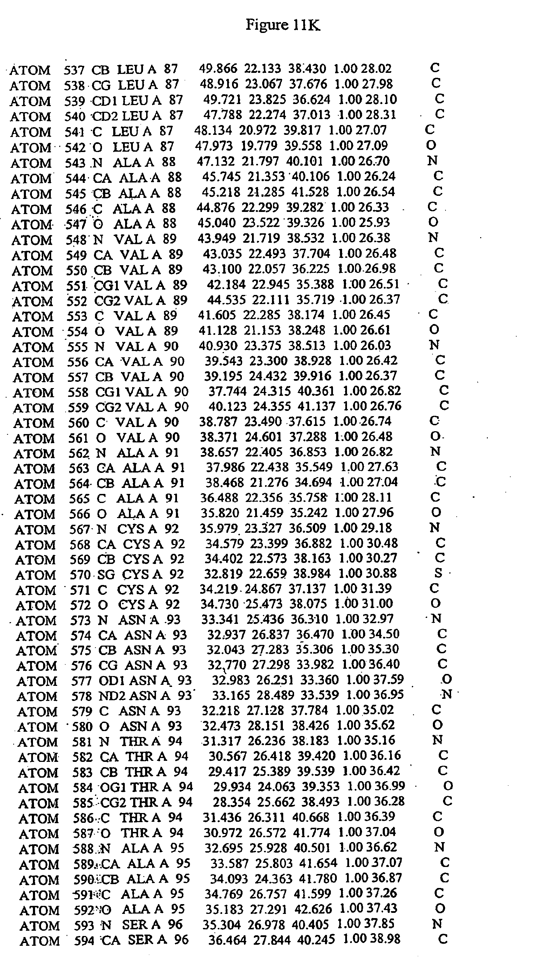

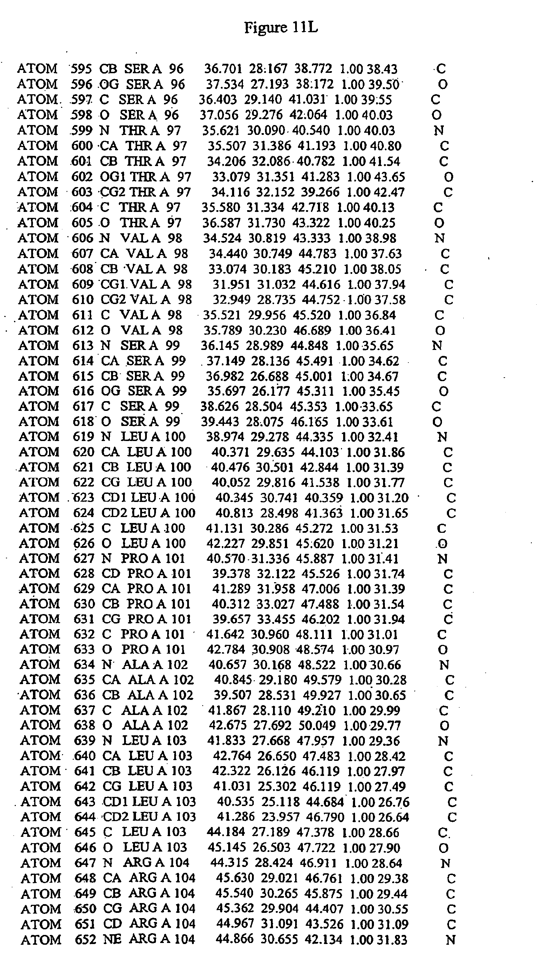

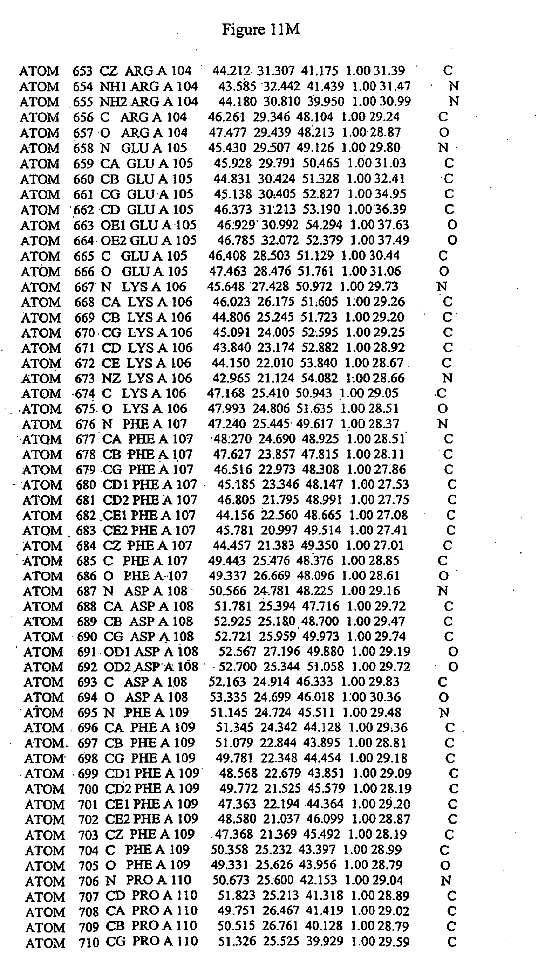

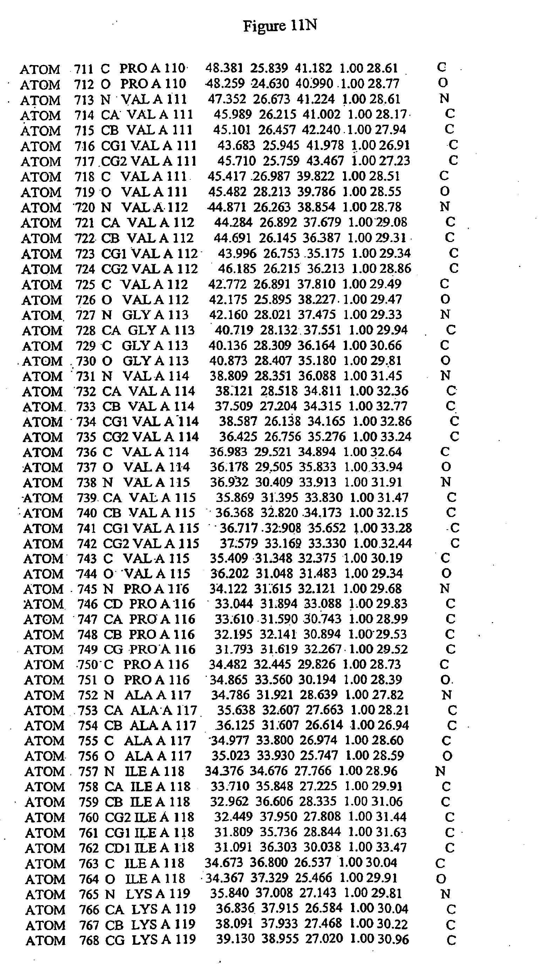

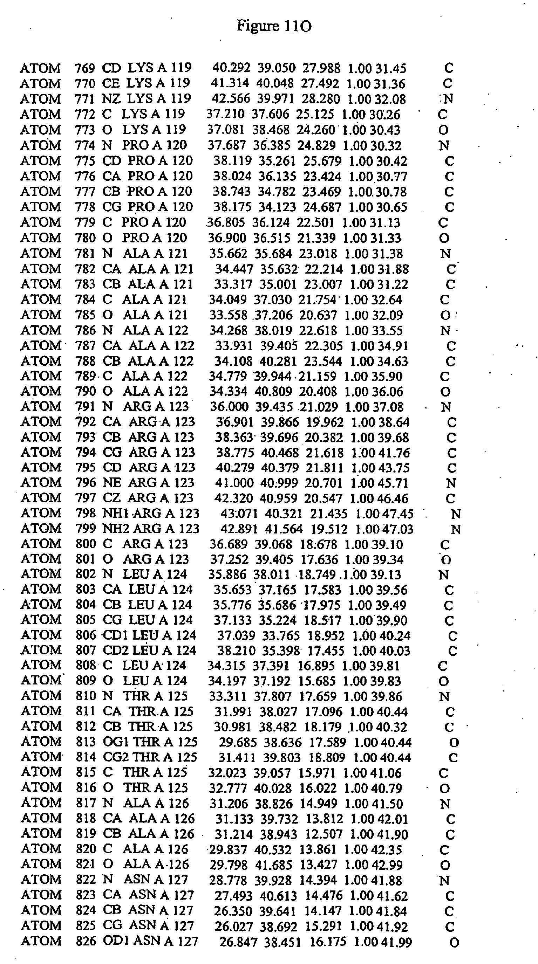

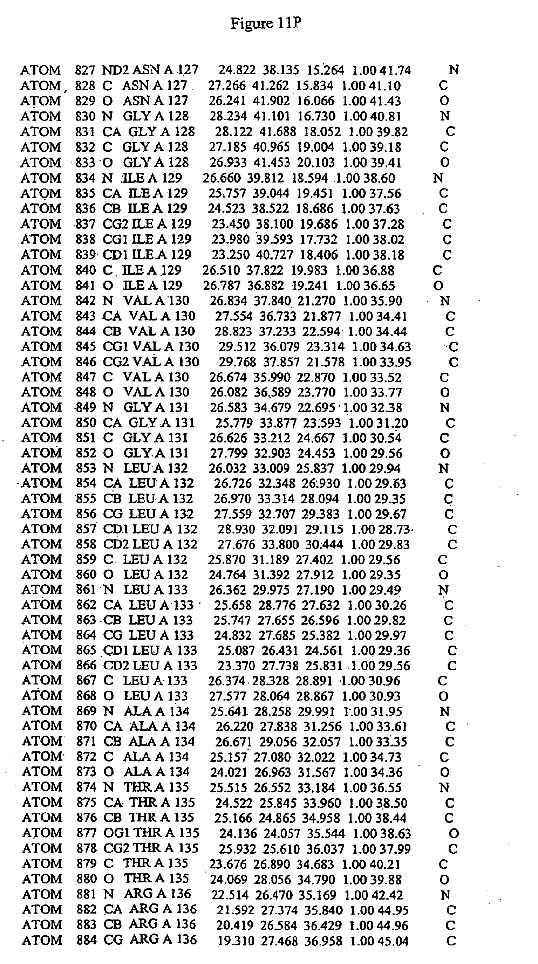

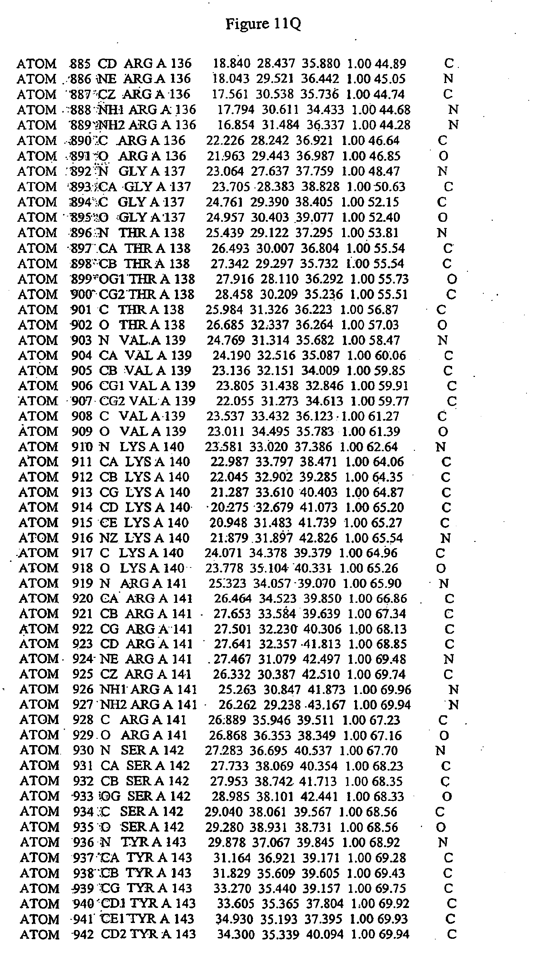

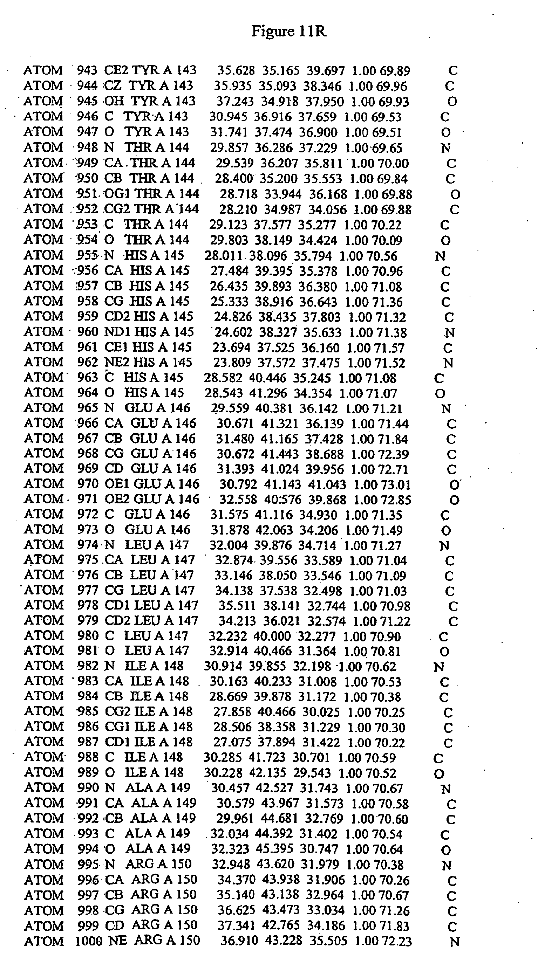

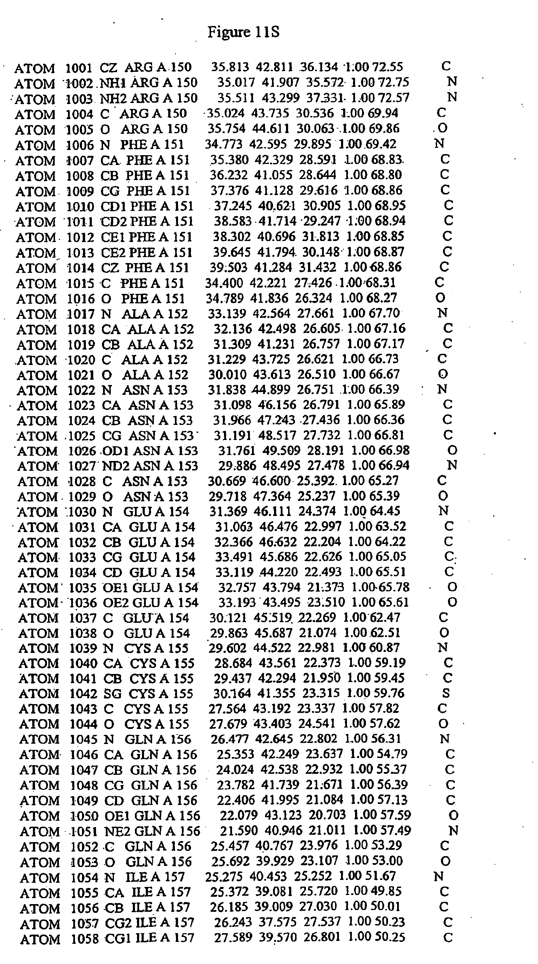

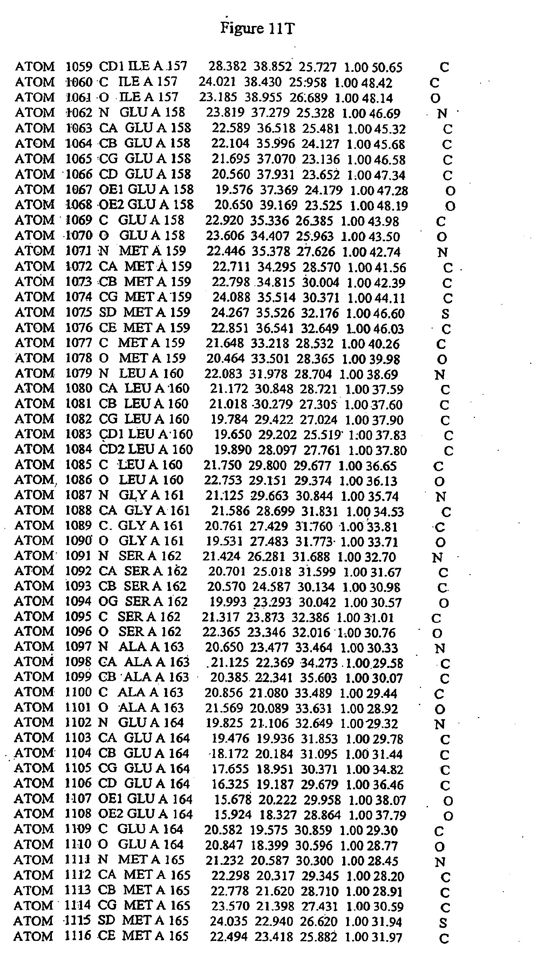

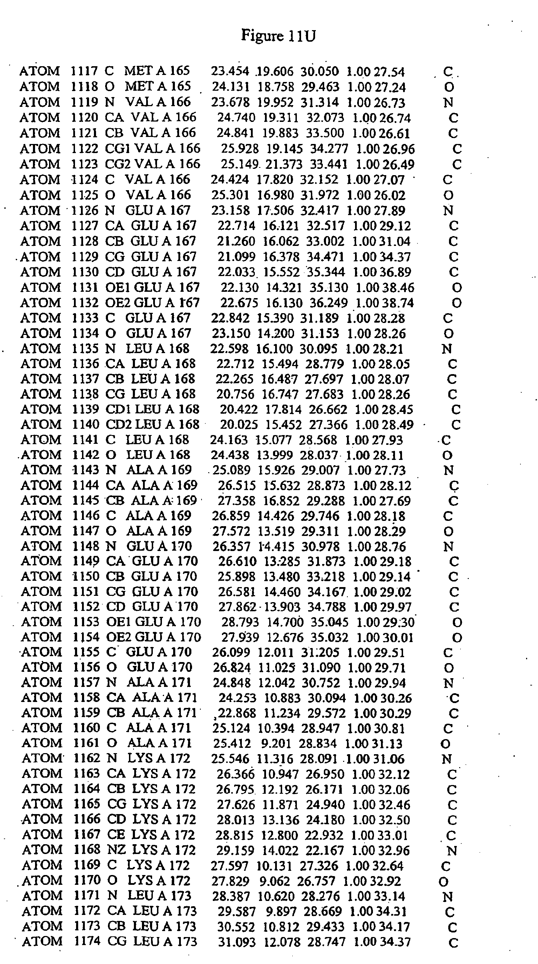

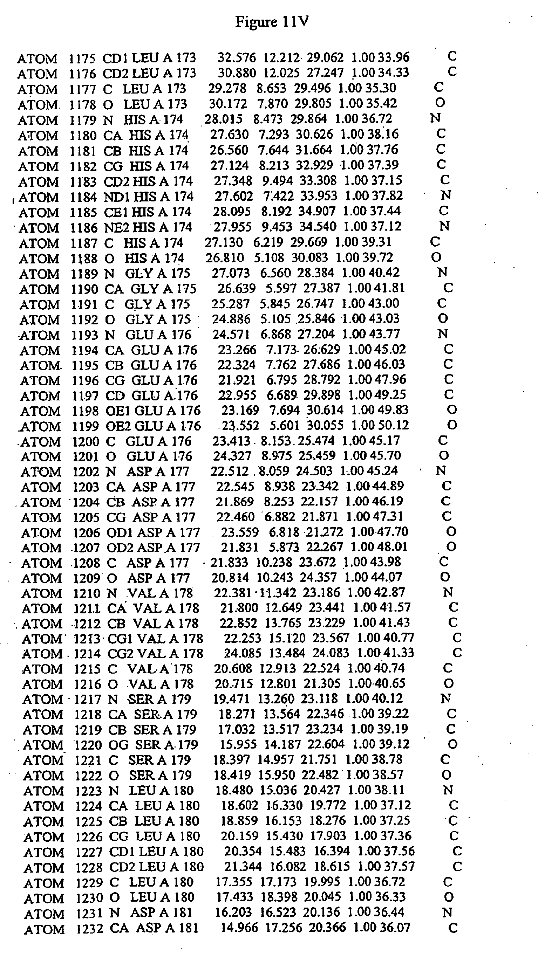

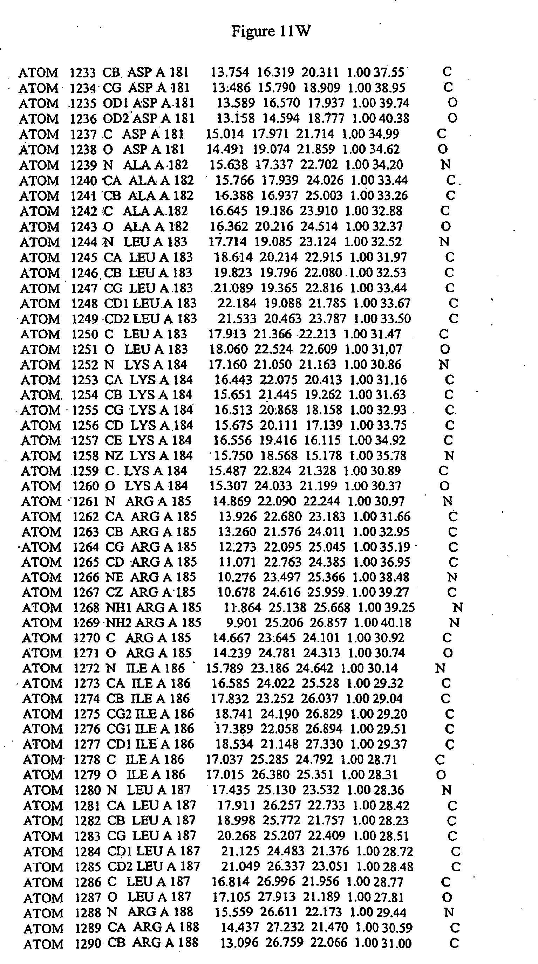

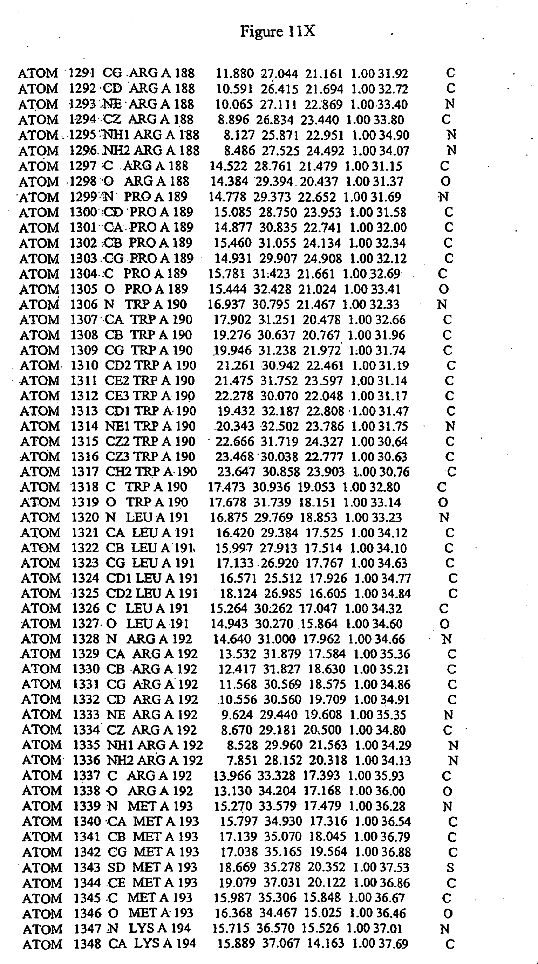

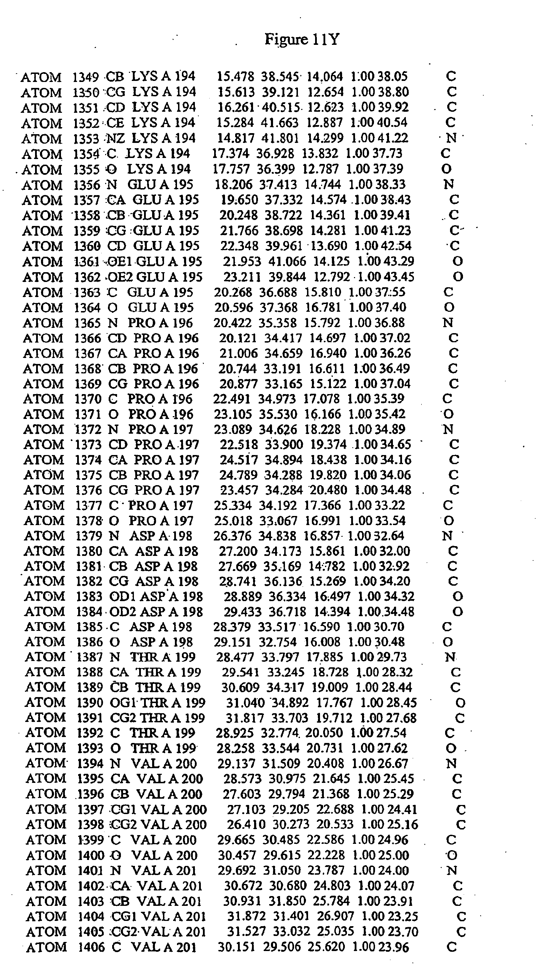

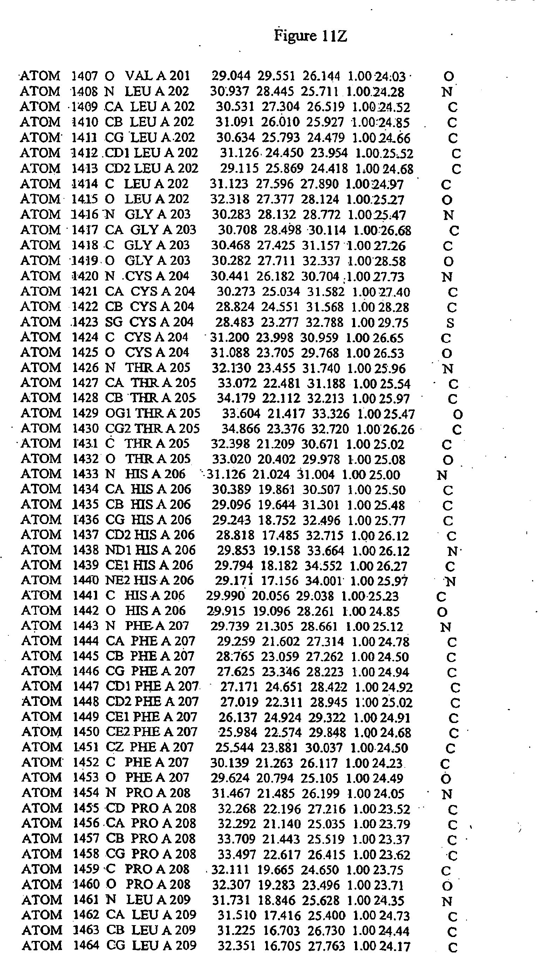

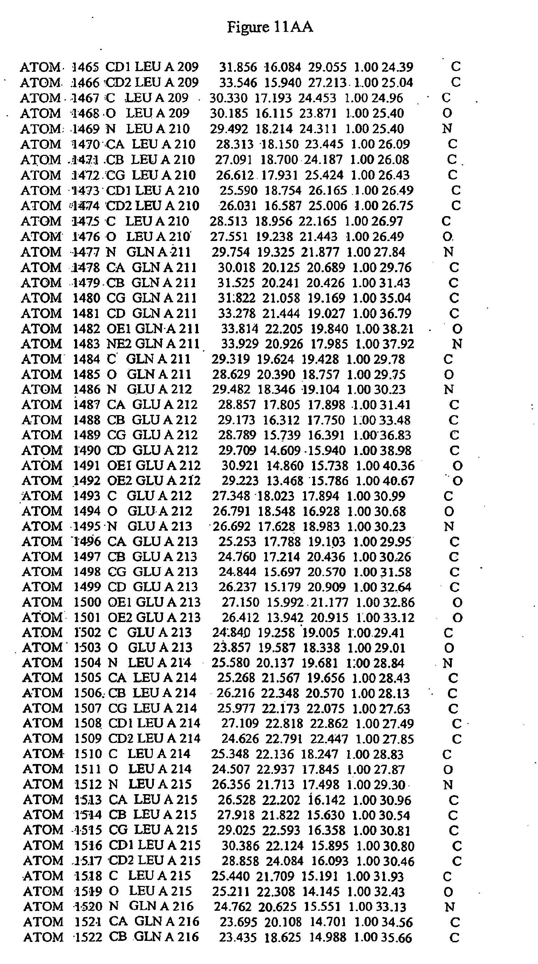

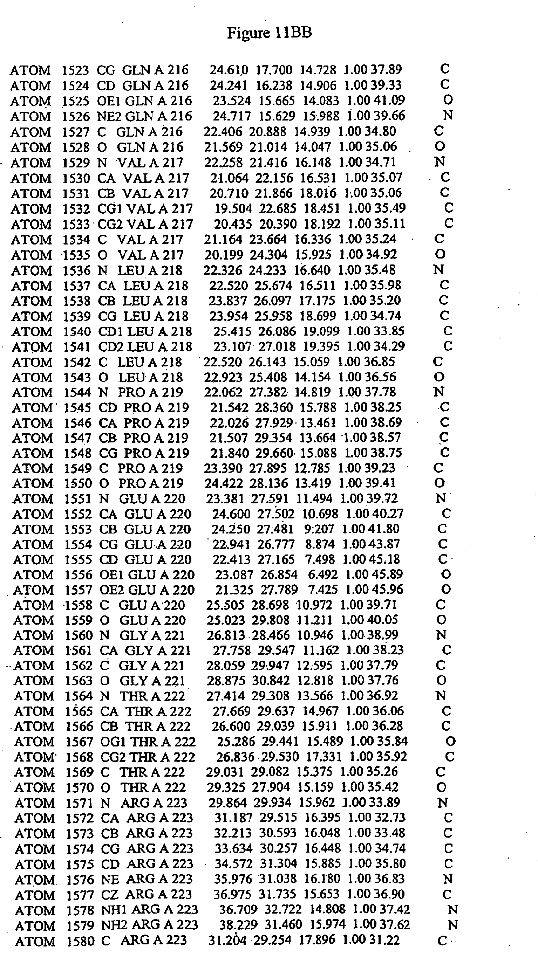

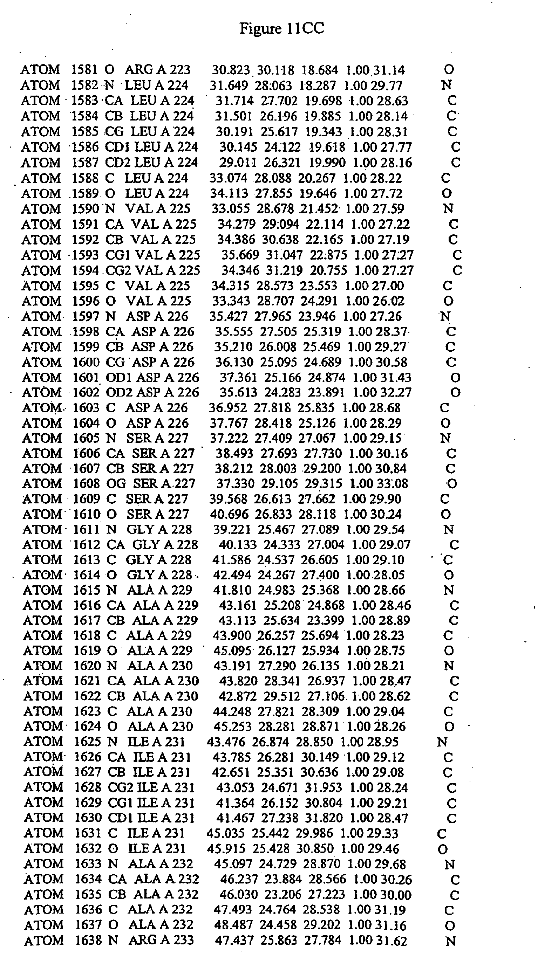

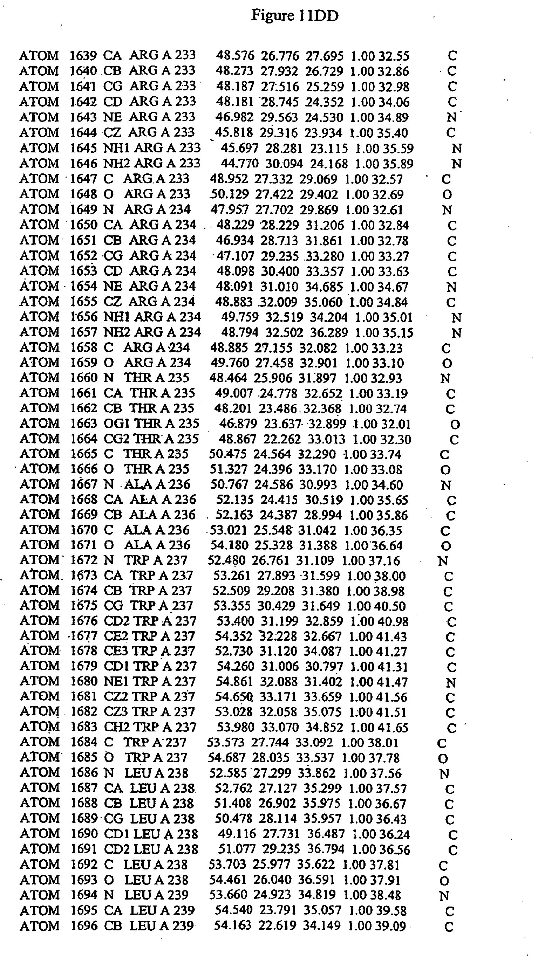

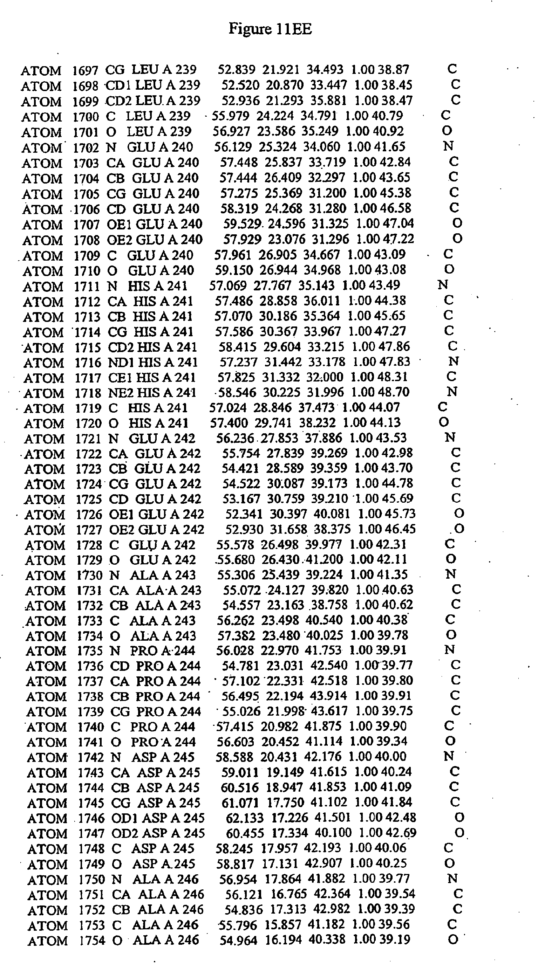

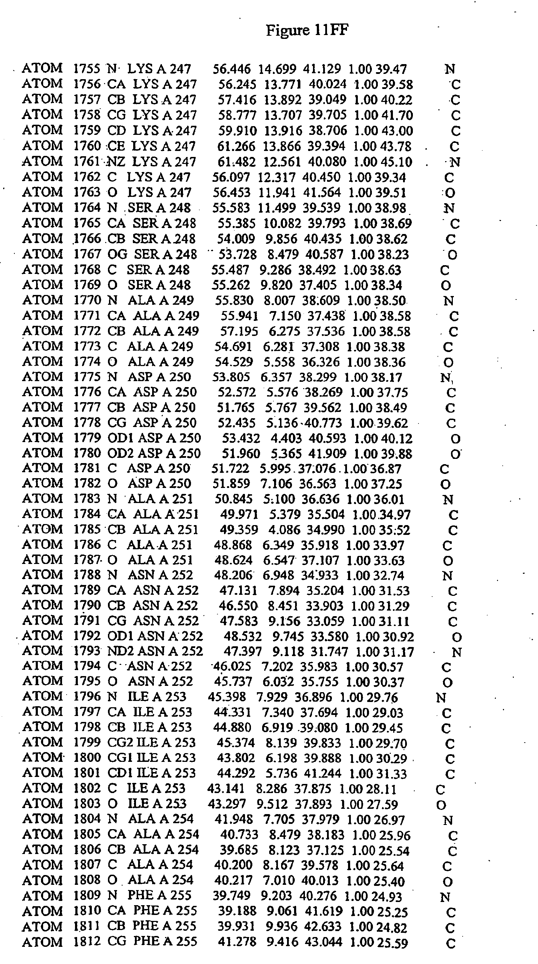

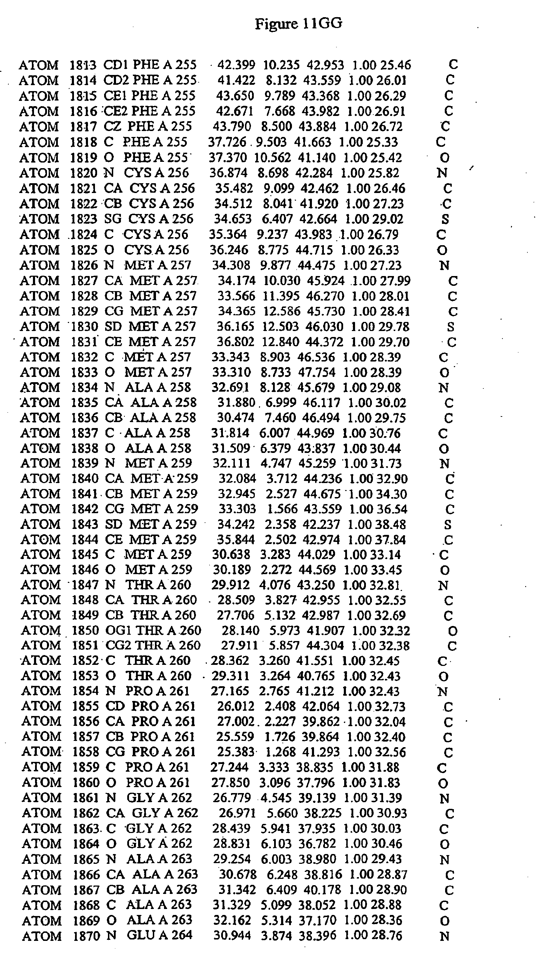

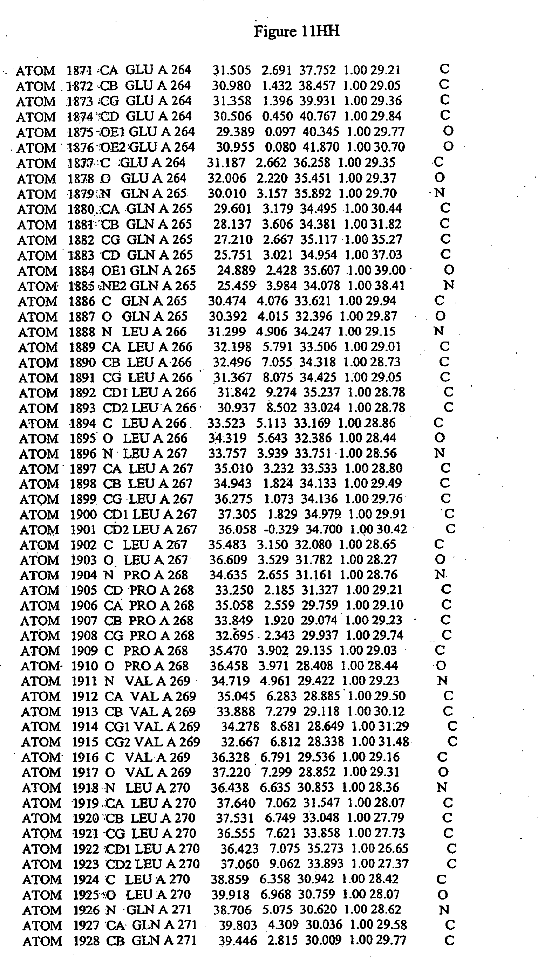

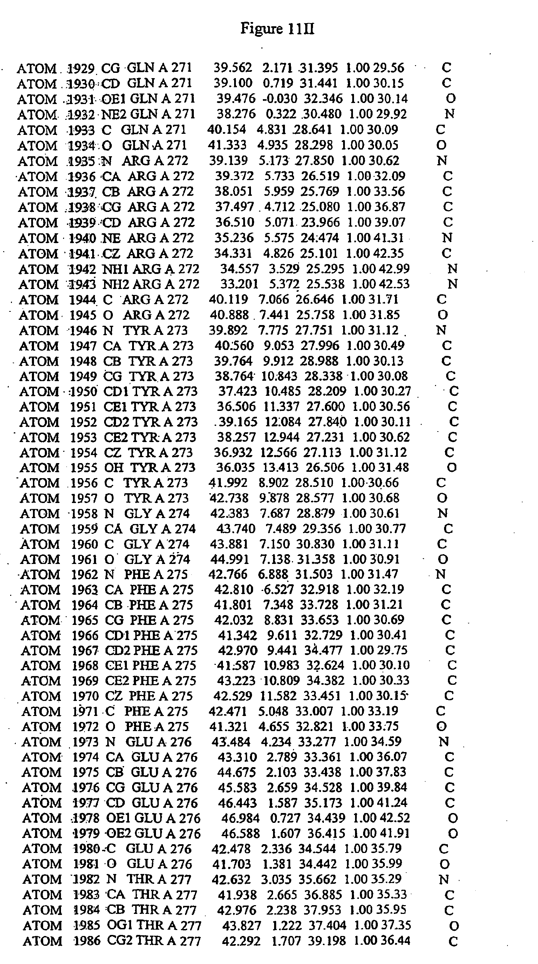

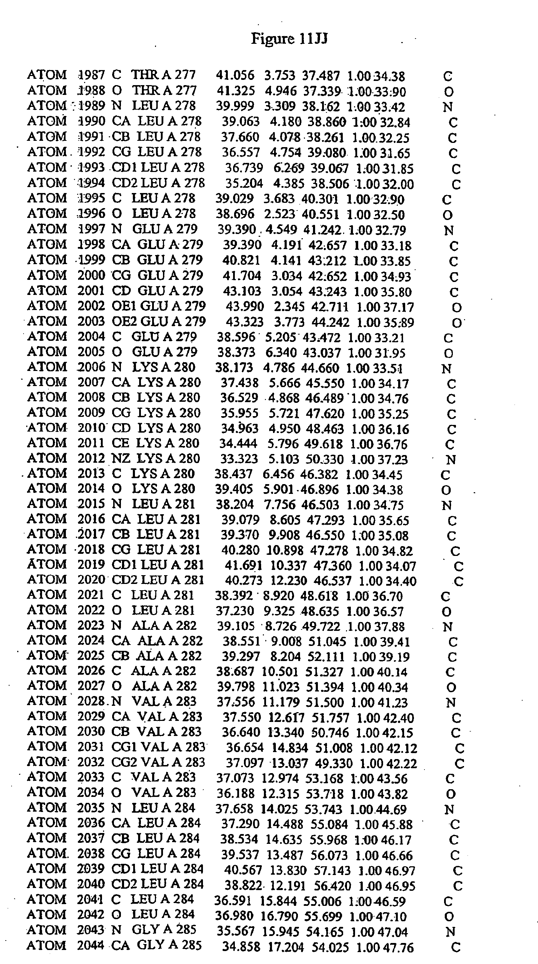

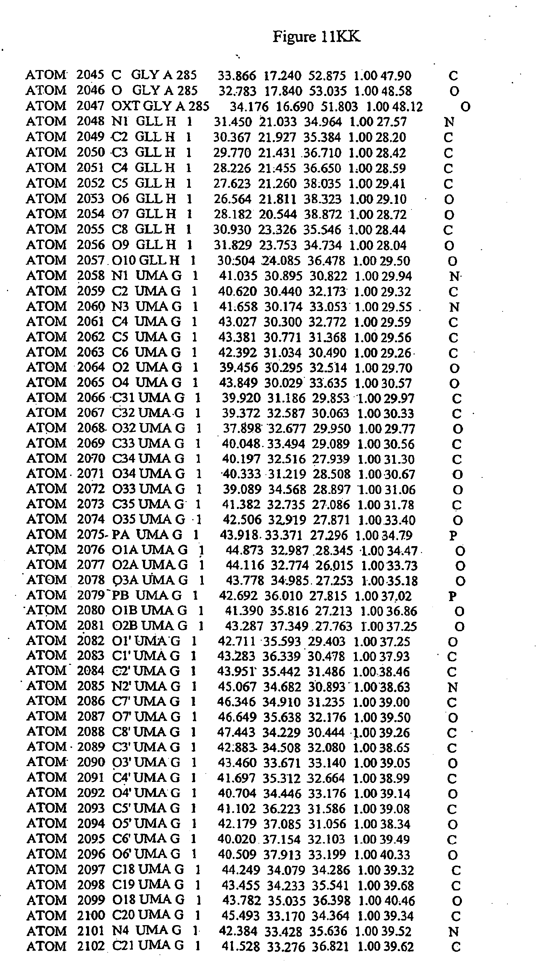

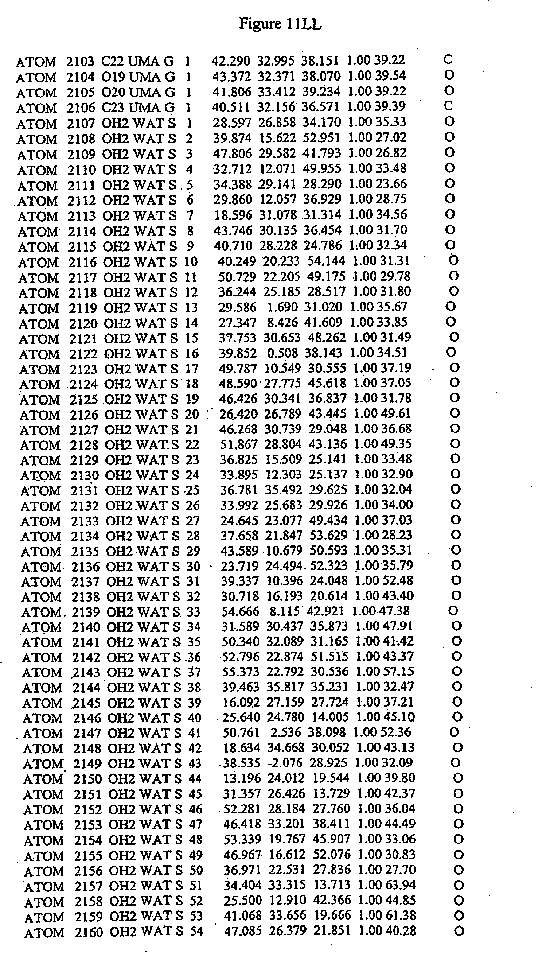

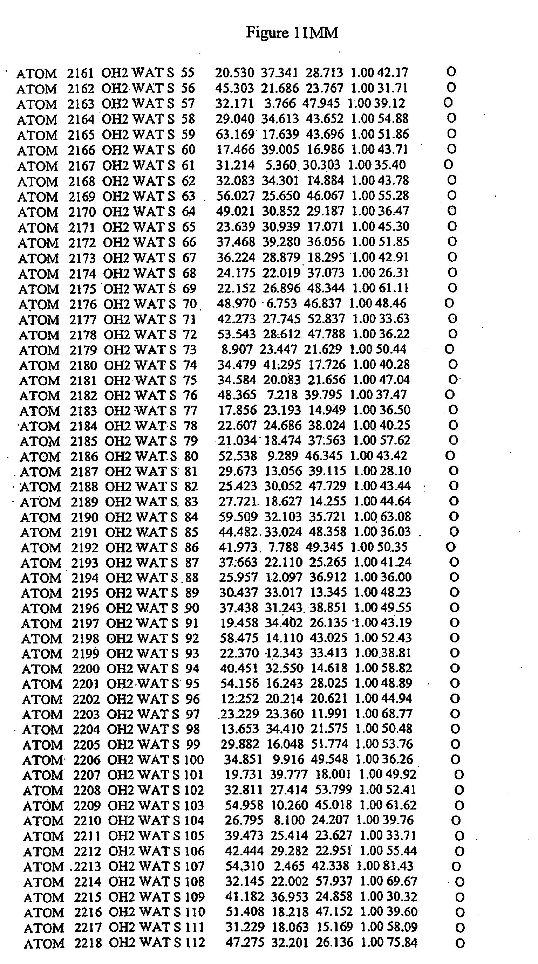

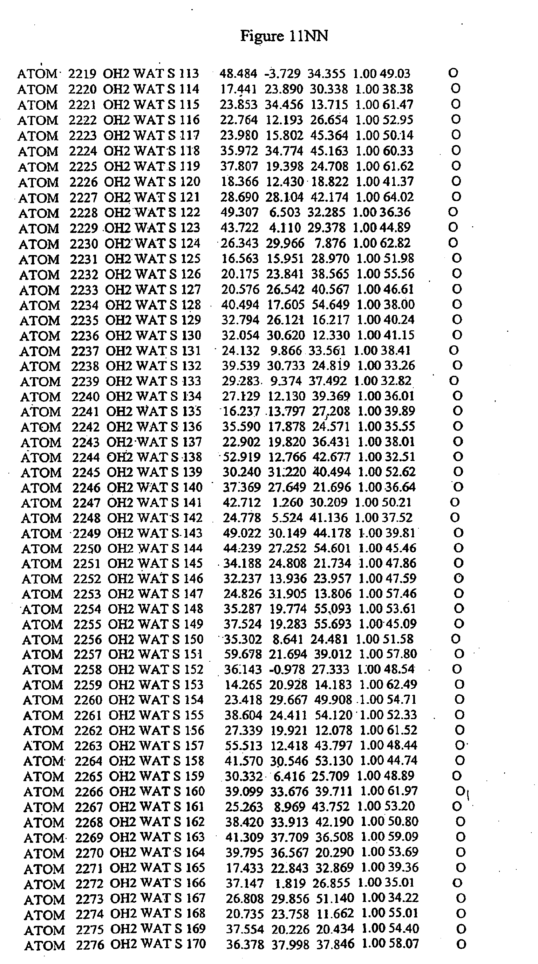

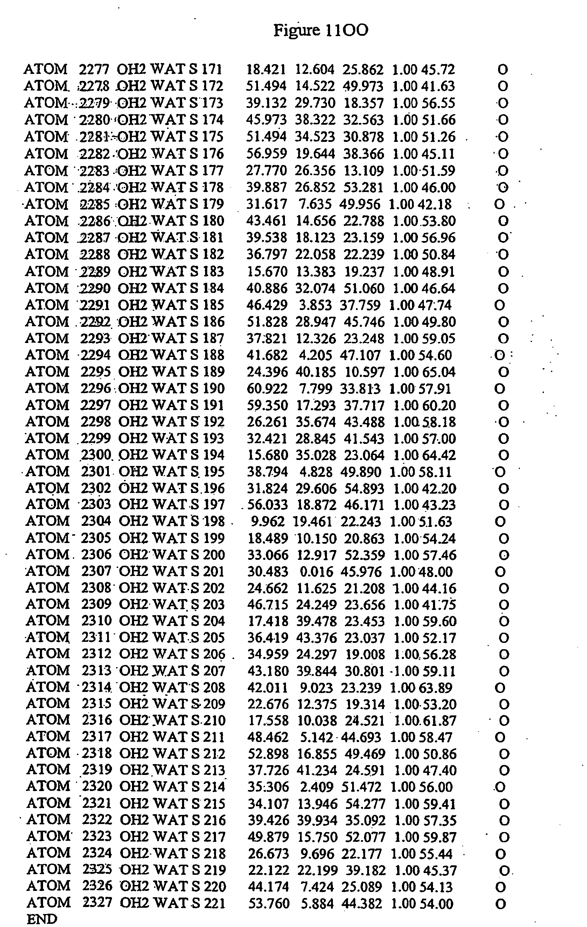

One embodiment relates to a crystal of MurI. In one embodiment, is crystallized *E*. *coli* MurI complexed with L-glutamate. One embodiment is crystallized *E*. *coli* MurI complexed with glutamate characterized by the structural coordinates depicted in Figure 8. One embodiment is crystallized *E*. *coli* MurI complexed with activator characterized by the structural coordinates depicted in Figure 9. One embodiment is crystallized *E*. *coli* MurI characterized by the structural coordinates depicted in Figure 10. One embodiment is crystallized *E*. *coli* MurI complexed with glutamate and activator characterized by the structural coordinates depicted in Figure 11.

One embodiment of the crystallized complex is characterized as belonging to the orthorhombic space group C222ₗ and has cell dimensions of a = 83.05 Å, b = 112.82 Å and c = 74.12 Å, wherein α = 90°, β = 90°, and γ = 90°. Another embodiment of the crystallized complex is characterized as belonging to the monoclinic space group P21 and has cell dimensions of a = 70.04 Å, b = 74.13 Å and c = 70.10 Å, wherein α = 90°, β = 107.25°, and γ = 90°.

One embodiment is a crystal of *E. coli* MurI, wherein the MurI is at least 70%, 75%, 80%, 85%, 90%, 95%, 98%, or 99% homologous to the amino acid sequence represented by SEQ ID NO: 40, or portions thereof. The crystals may diffract from about 0.8 Å to about 3.5 Å.

The MurI protein exists as a monomer, but inspection of the electron density map identified additional molecular symmetry. Each monomer has a pseudo two-fold symmetry that divides the monomer into two domains with very similar alpha/beta type folds. The binding site is found in the interface between the two domains. On the opposite side of the monomer, but still in the interface, is the binding site for an activator, such as UDP-*N*-acetylmuramyl-L-alanine (UDP-MurNAc-Ala), wherein the activator acts as a wedge to stabilize an active conformation in which the two domains are properly oriented to promote binding of substrate.

In the present invention, a substrate can be a compound such as L-glutamate, D-glutamate which MurI reversibly converts compounds from the R- to S-enantiomer. Thus, a substrate can also act a product. The substrate can be a naturally-occurring or artificial compound.

In the present invention, an inhibitor can be a compound which also may undergo a catalytic reaction, or which binds to the substrate binding site or another site on MurI and which competes with substrate turnover of glutamate. Inhibitors of the present invention can be a compound such as L-serine-O-sulfate, D-serine-O-sulfate, D-aspartate, L-aspartate, tartrate, citrate, phosphate, sulfate, aziridino-glutamate, N-hydroxyglutamate, or 3-chloroglutamate. The inhibitor can be a naturally-occurring or artificial compound.

In the present invention, an activator can be a compound such as UDP-MurNAc-Ala. The activator can be a naturally-occurring, or artificial compound.

The activator has a compact structure when bound to the protein. It folds back on itself into a two-layered structure where two phosphate groups act as a bridging link. The uridine ring stacks against the muramic acid ring. There is almost a perfect shape match between the protein and activator as it resides on top of the two connecting loops between the two domains, in sequence corresponding to residues 112-116 and 225-228. In the absence of activator, the two loops act as an interface between the two domains.

However, the binding is not entirely driven by favorable van der Waal interactions. There are a number of polar interactions and at least one strong salt bridge between Arg104 and the carboxylate group of the alanine motif of the activator. The latter salt bridge could be a key interaction for the activator in order to be able to lock the two domains into the proper orientation, explaining the need for the terminal alanine residue in order for the molecule to work as an activator.

Other extensive polar interactions are found between the uracil ring and main chain atoms of the protein, residues 113-115. There are also a number of hydrogen bonds from the hydroxyl groups of the two sugar rings. Many interactions between inhibitor and protein are mediated by water molecules. In contrast, the two phosphate groups make very little contact with the protein since they are facing towards the solution. However, on each side of the diphosphate group is a positively charged residue, Lys 119 on one domain, and Arg233 on the other domain, which provides another example of domain-domain stabilizing interaction.

A further embodiment relates to a *E*. *coli* MurI in which the substrate binding site comprises two conserved cysteine residues, denoted Cys92 and Cys204 in the amino acid sequence represented as SEQ ID NO: 40. A further embodiment comprises a substrate binding site of *E*. *coli* MurI wherein the binding site additionally comprises one or more of the following amino acid residues: Ser29, Thr94, Thr135, Thr138, Glu170, Thr205 and His206 as represented by the structural coordinates of Figure 8. In one embodiment, the substrate binding site of the *E*. *coli* Murl complexes with L-glutamate and comprises amino acid residues Cys92 and Cys204 as well as amino acid residues within 5 Å of the Cys92 and Cys204 residues as represented by the structural coordinates of Figure 9. In one embodiment, the substrate binding site of the *E*. *coli* MurI additionally comprises one or more of the following amino acid residues: Ser29, Thr94, Thr135, Thr138, Glul70, Thr205 and His206. In this embodiment, the substrate binding site complexes with L-glutamate and comprises amino acid residues Cys92 and Cys204 and at least one (i.e., one or more) of the following amino acid residues: Ser29, Thr94, Thr135, Thr138, Glu170, Thr205 and His206, which can be present in any combination. In a further embodiment, the substrate binding site includes amino acid residues Phe27, Asp28, Ser29, Gly30, Va131, Gly32, Gly33, Ser35, Val36, Asp54, Ala57, Ala57, Phe58, Pro59, Tyr60, Gly61, Glu62, Lys63, Ile68, Val90, Ala91, Cys92, Asn93, Thr94, Ala95, Ser96, Thr97, Val114, Val115, Leu133, Ala134, Thr135, Arg136, Gly137, Thr138, Val139, Thr144, Ala163, Val166, Glu167, Glu170, Leu202, Gly203, Cys204, Thr205, His206, Phe207 and Ser227of SEQ ID NO: 40.

In a further embodiment, the substrate binding site of the MurI comprises two hydrogen bond TRIADs, which occur close to the conserved cysteine residues of the binding site. Specifically, on one side (Cys204) of the binding site, the TRIAD is Glu170-Thr205-His206 and on the other side (Cys92), the TRIAD is Thr94-Thr135-Thr138. Thus, in a specific embodiment, the substrate binding site of *E. coli* MurI complexes with L-glutamate and comprises amino acid residues Cys92 and Cys204 and additionally includes at least one (i.e., one or more) of the following: amino acid residues Ser29, Thr94, Thr135, Thr138, Glu170, Thr205 and His206.

The threonine residues have features of interest as they relate to the binding site of the *E. coli* MurI. On one side (Cys204) of the substrate binding site, Thr205 is H-bonded to His206 and His206 is further H-bonded to GIu170. The hydroxyl oxygen (O) of Thr205 is 2.8 Å away from the amino nitrogen (N) of the substrate (i.e., glutamate) and 5.3 Å from the sulfur (S) atom of Cys204, which is 4.3 Å from His206. On the other side of the substrate binding site (Cys92), Thr94 is H-bonded to Thr135 and further H-bonded to Thr138. The hydroxyl O of Thr94 is H-bonded to one of the carboxylate oxygen atoms of the substrate and is 3.3 Å away from the S atom of Cys92. Analysis showed that the three hydroxyl oxygens form a triangle, all within less than 3.2 Å from one another.

The two TRIADs may play important roles in altering the pKa of the two substrate binding site cysteine residues (in addition to that of the neighboring hydrophobic core), facilitating the proton transfer during catalysis or both.

### B. Gram positive

### 1. E. faecalis

One of ordinary skill in the art would recognize that solving crystal structures of proteins such as *E. faecalis, E. faecium,* and *S*. *aureus* glutamate racemase (MurI) requires a stable source of high-quality protein.

One embodiment relates to a crystal of MurI from a Gram positive bacterium.

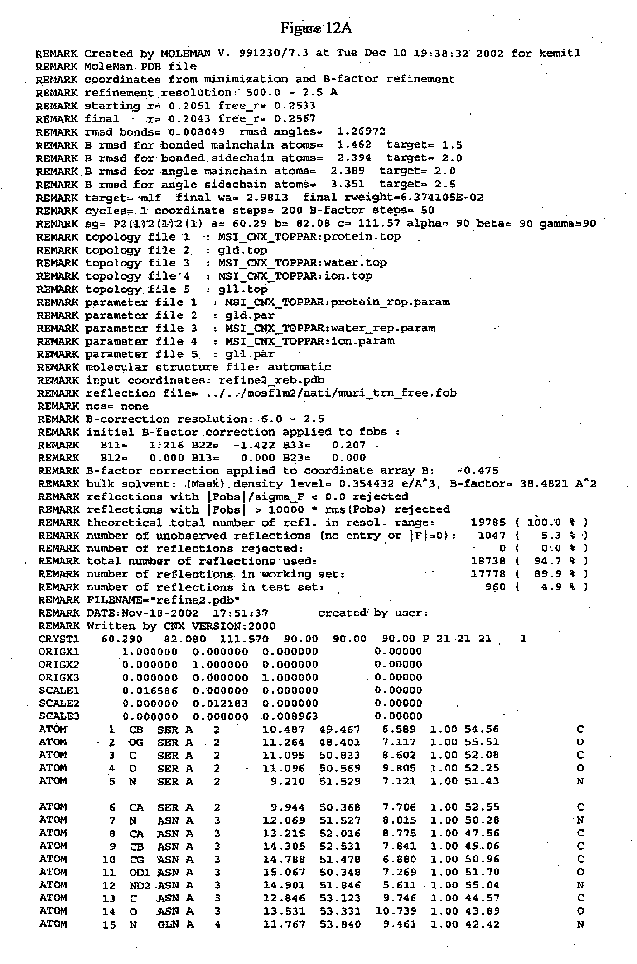

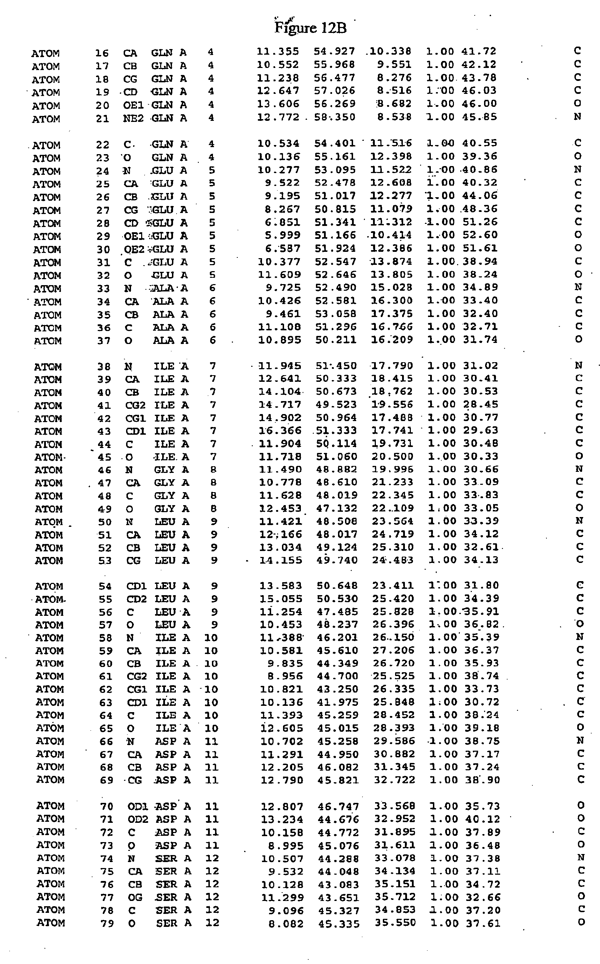

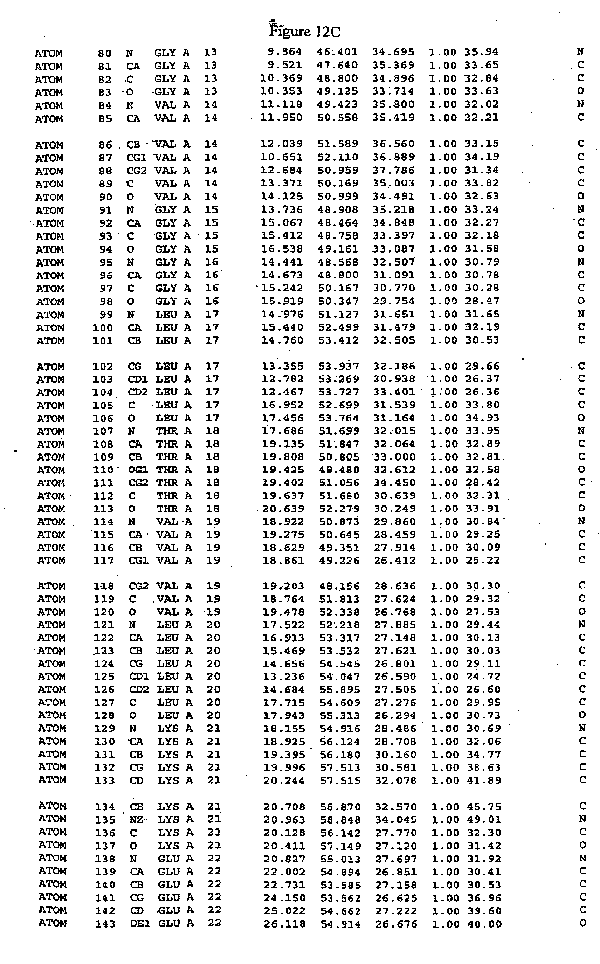

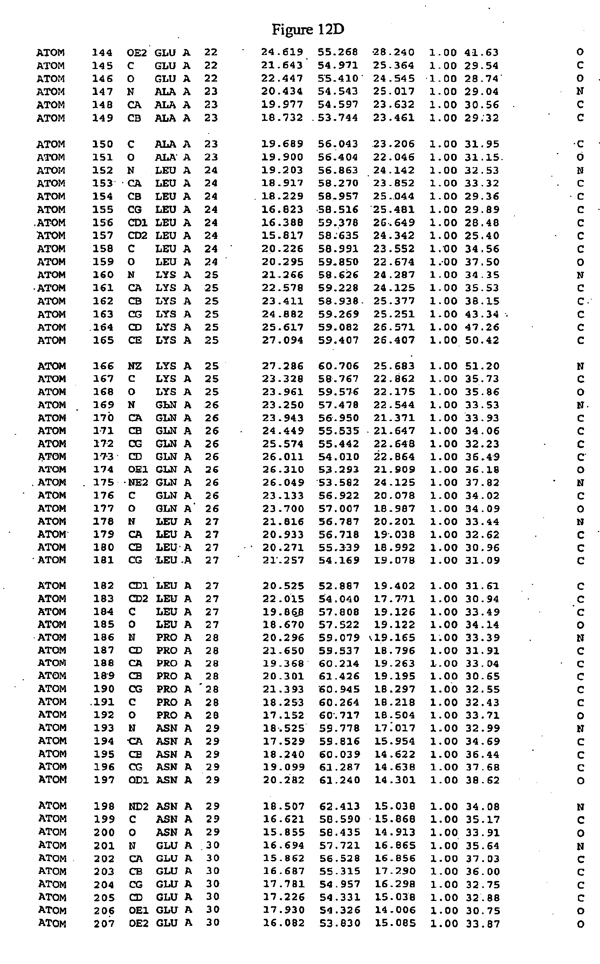

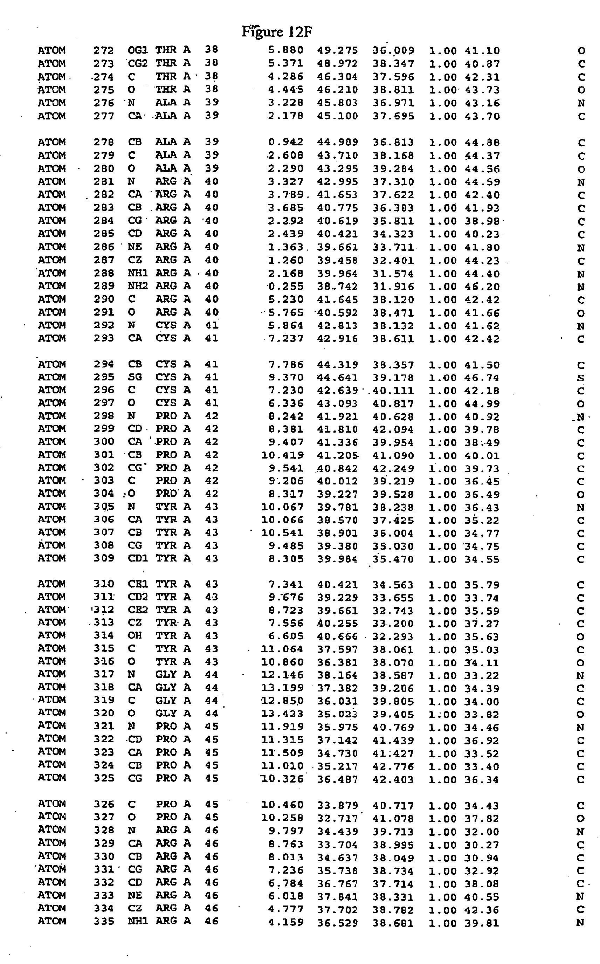

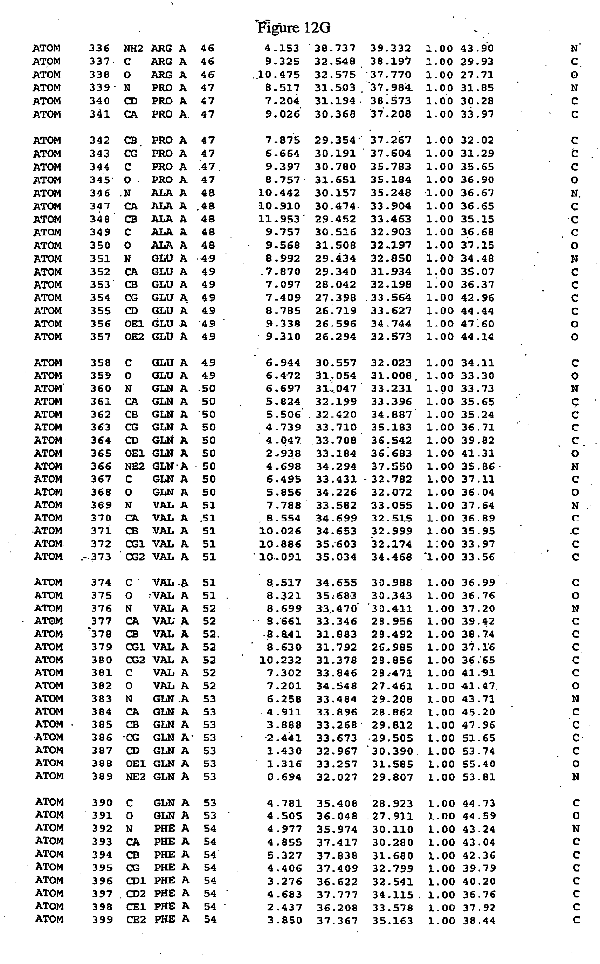

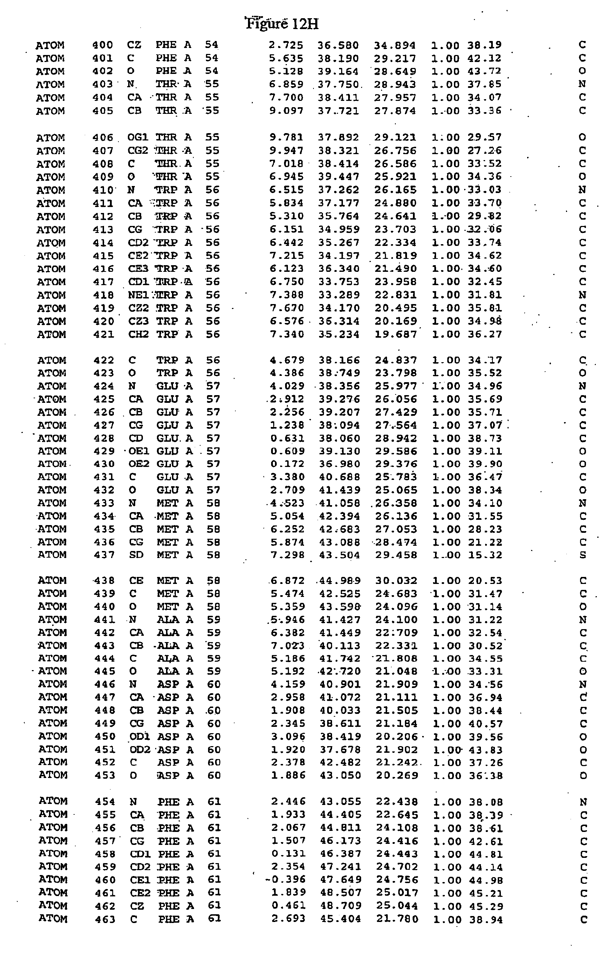

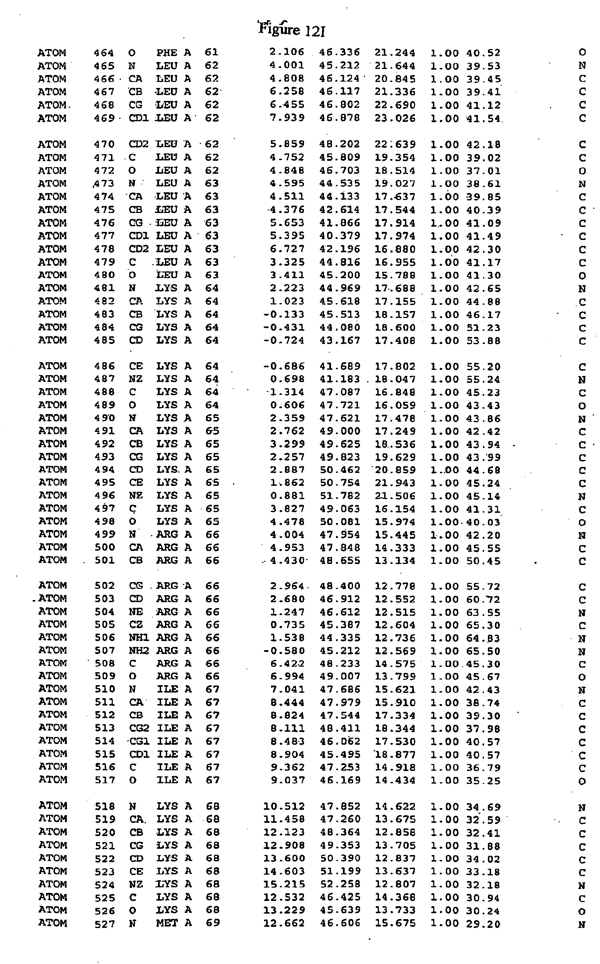

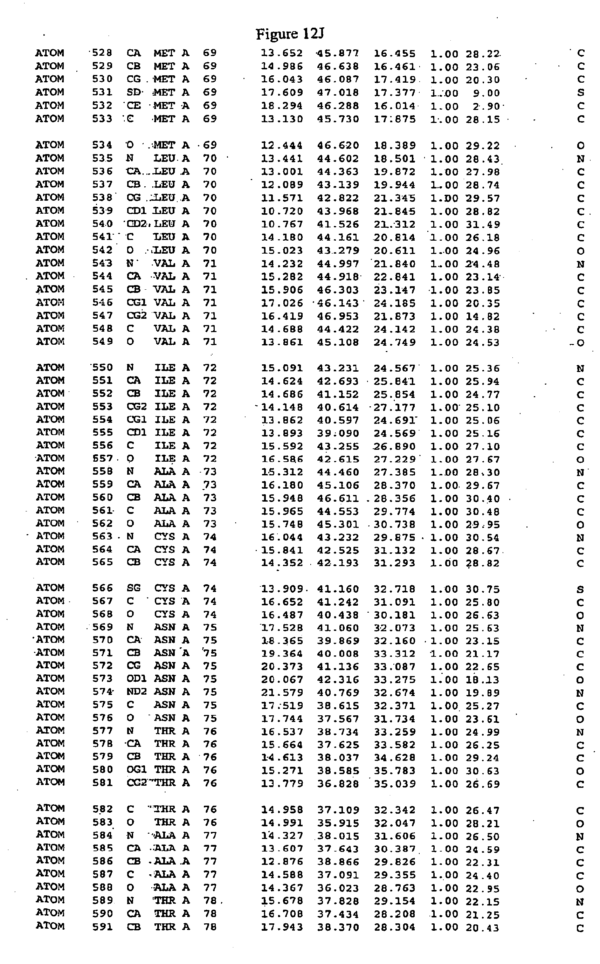

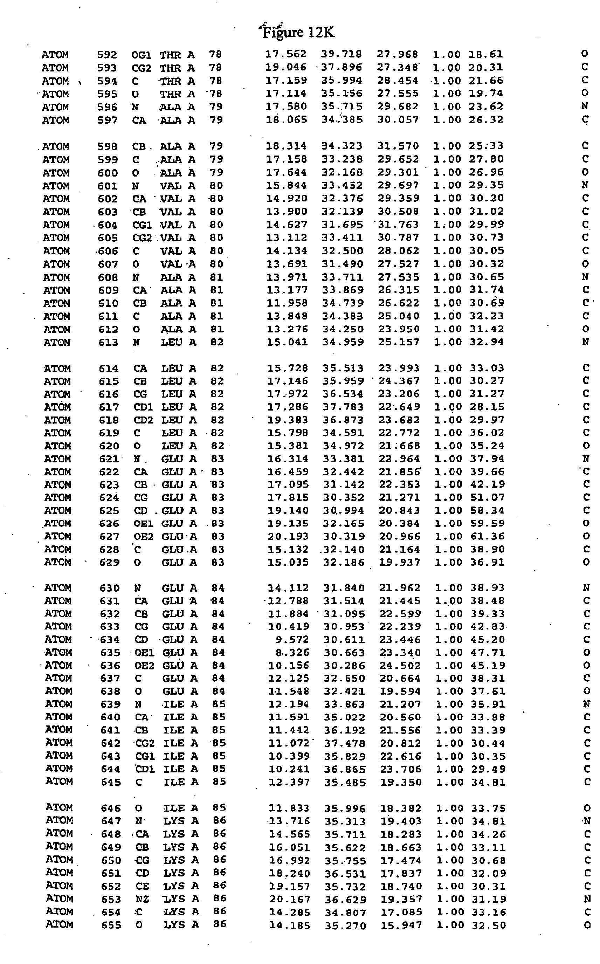

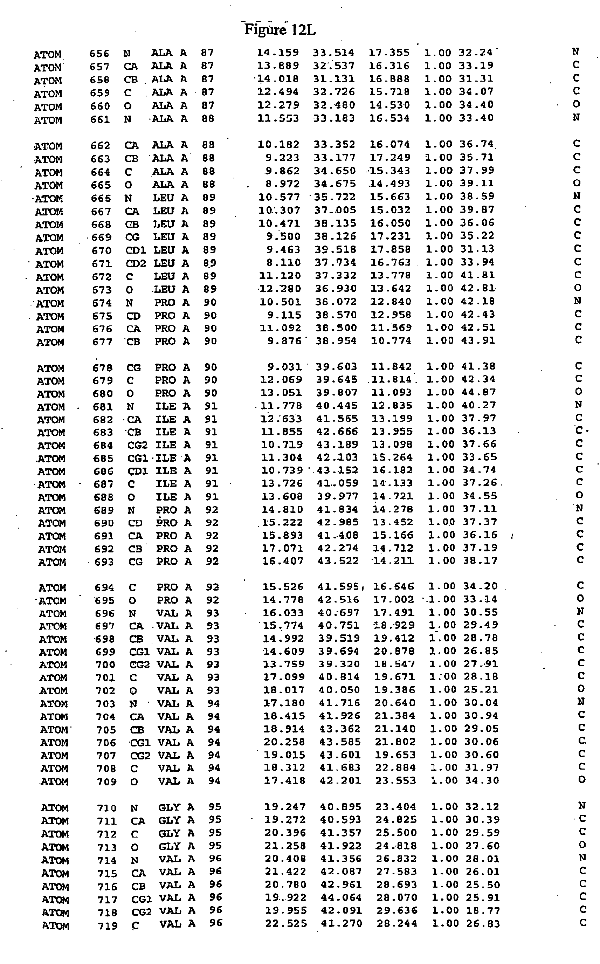

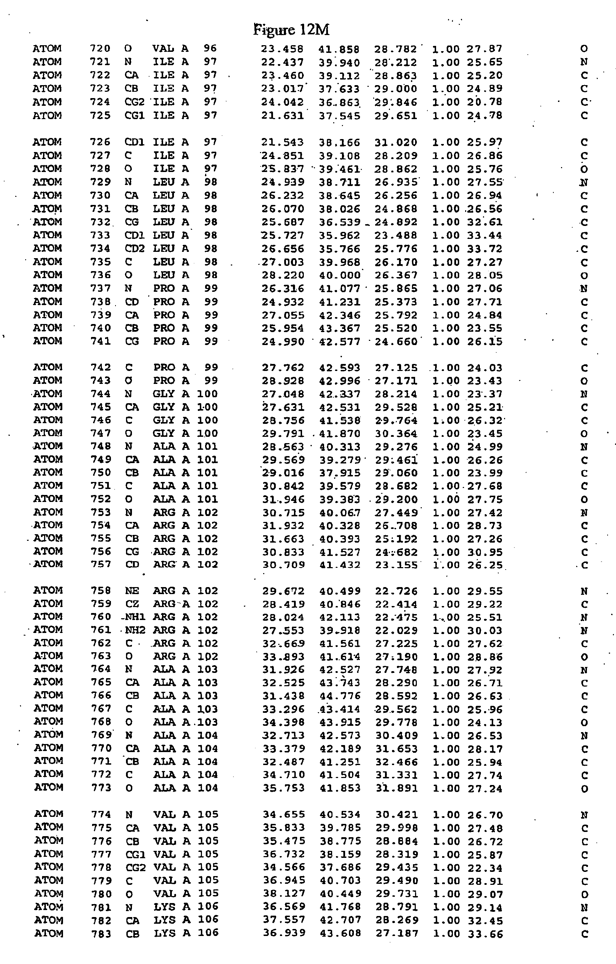

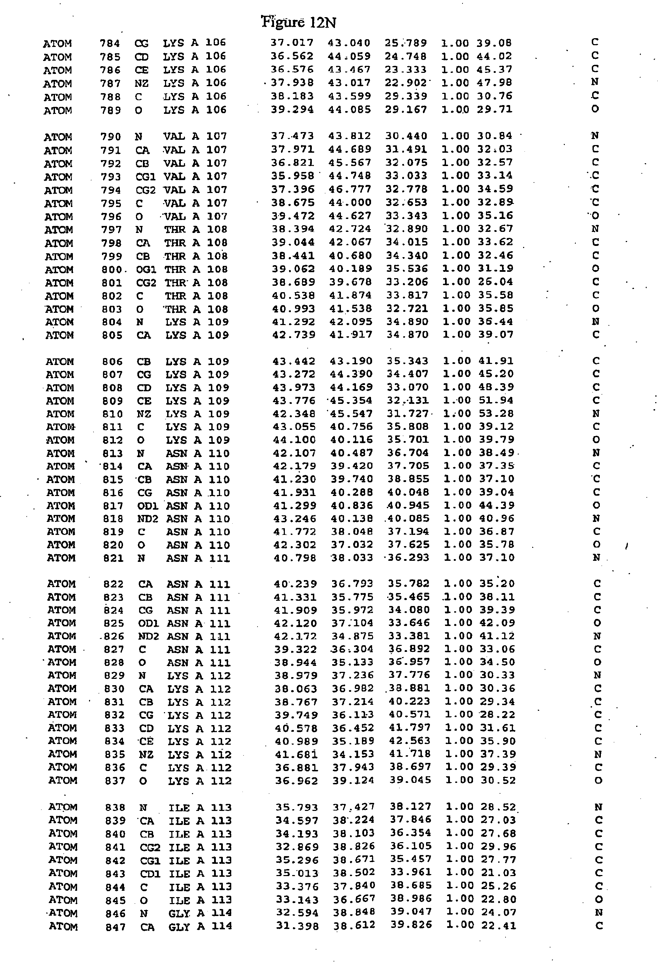

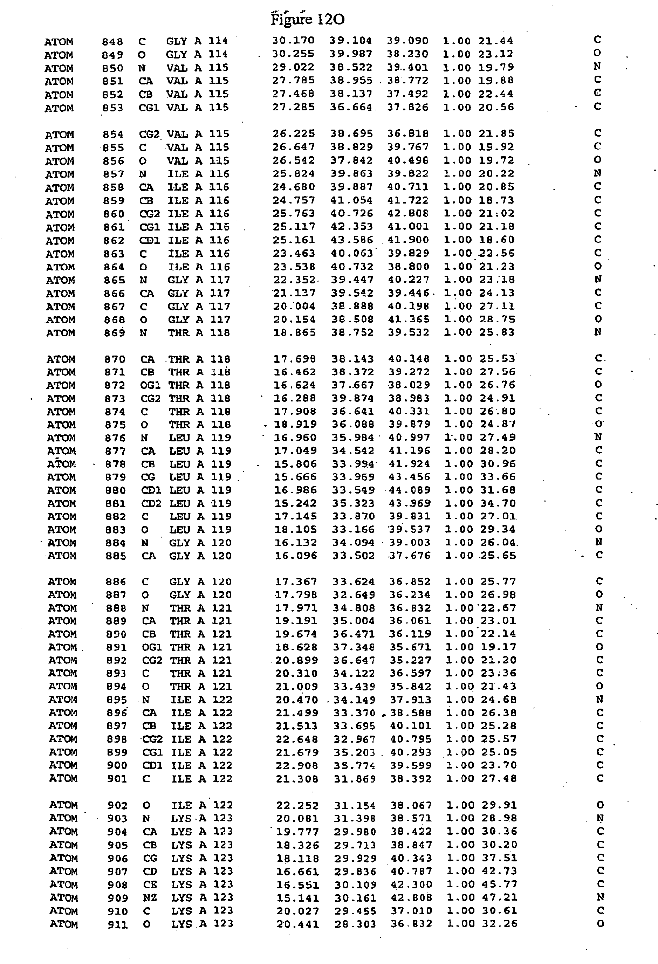

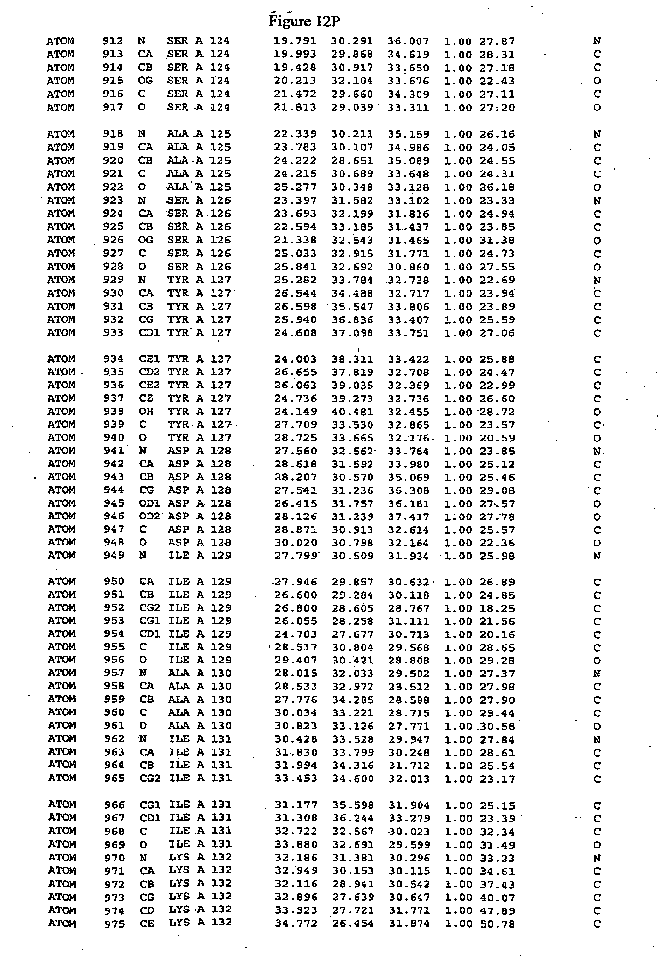

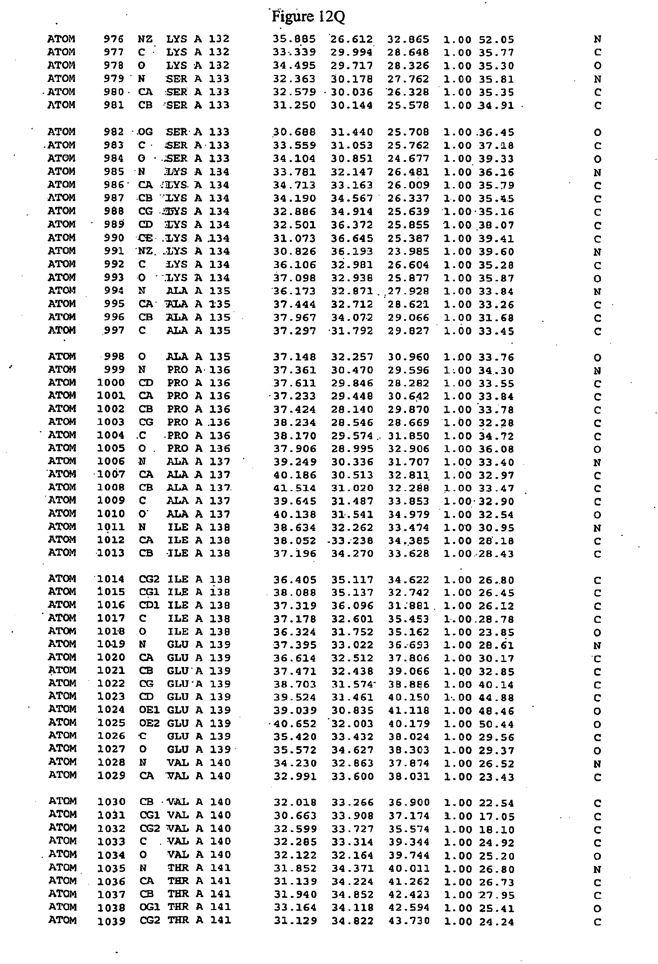

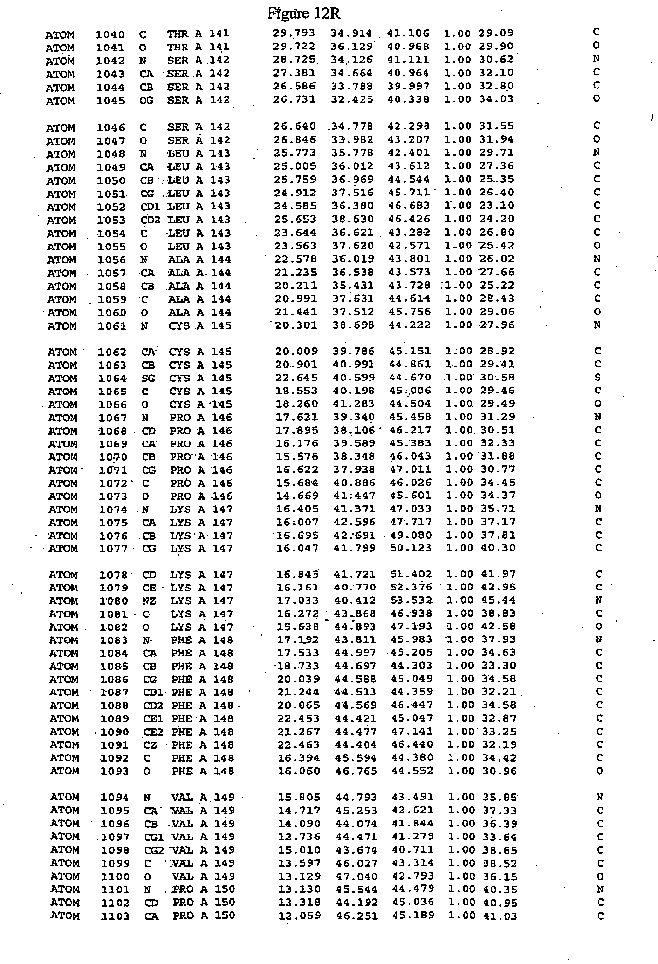

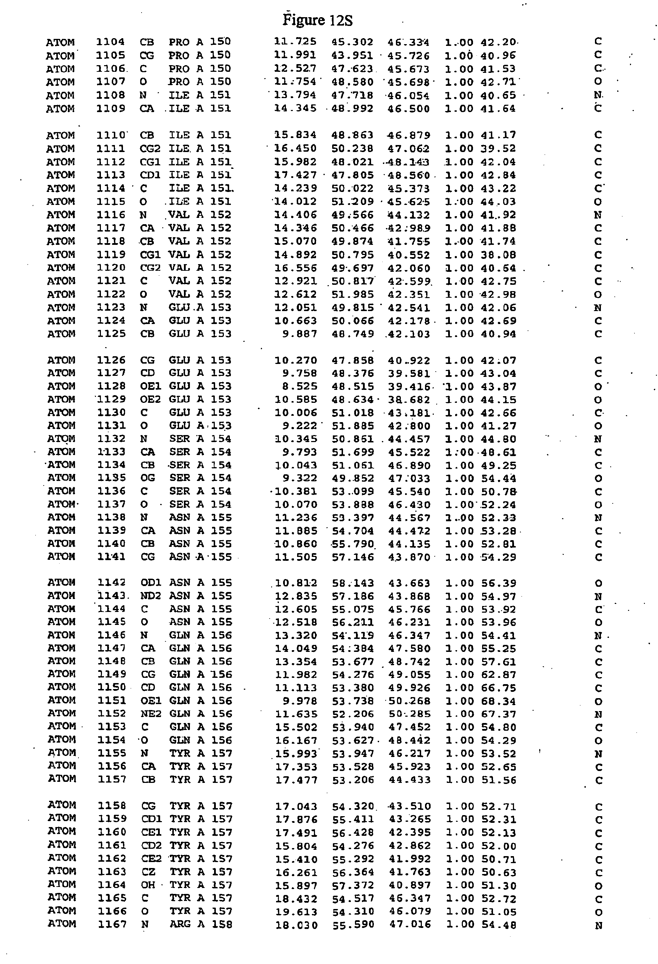

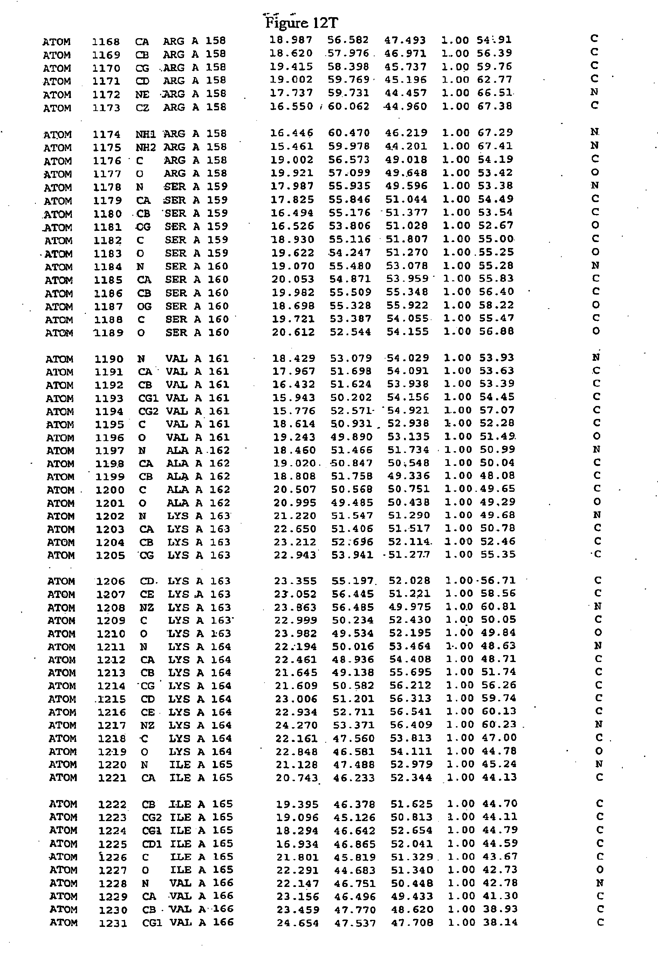

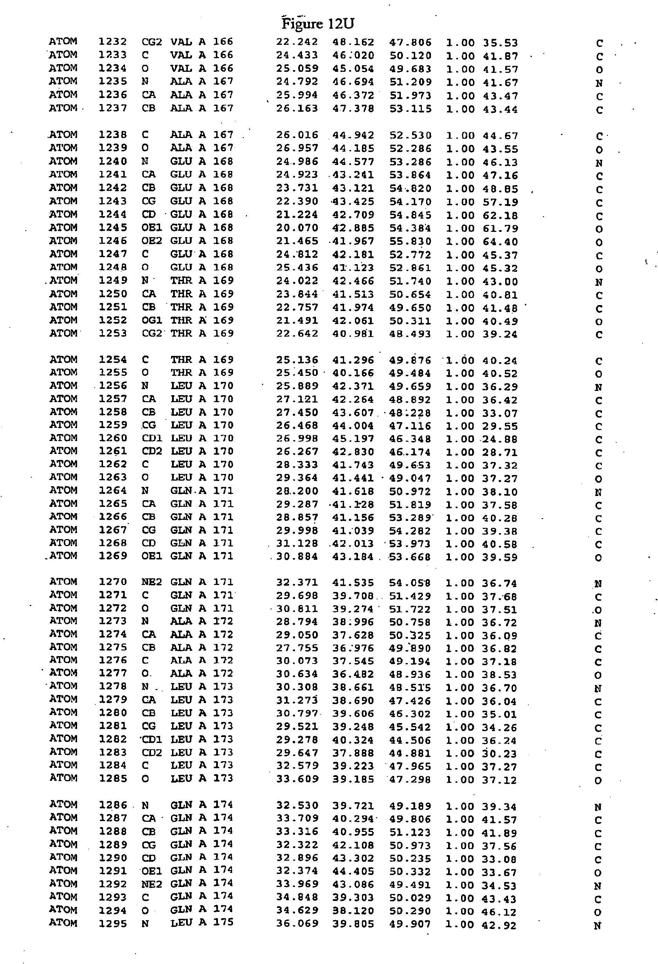

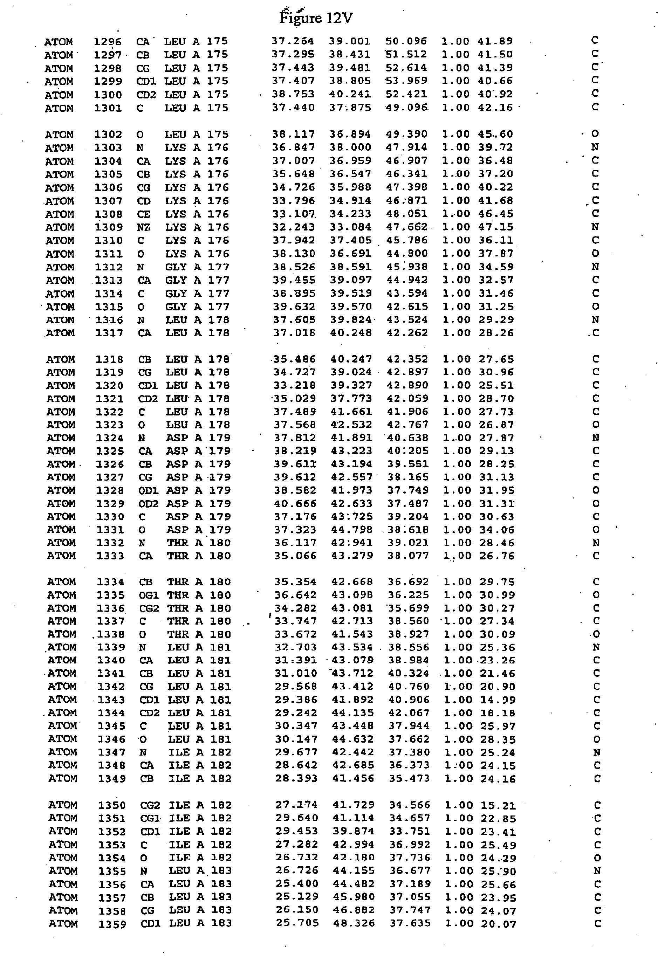

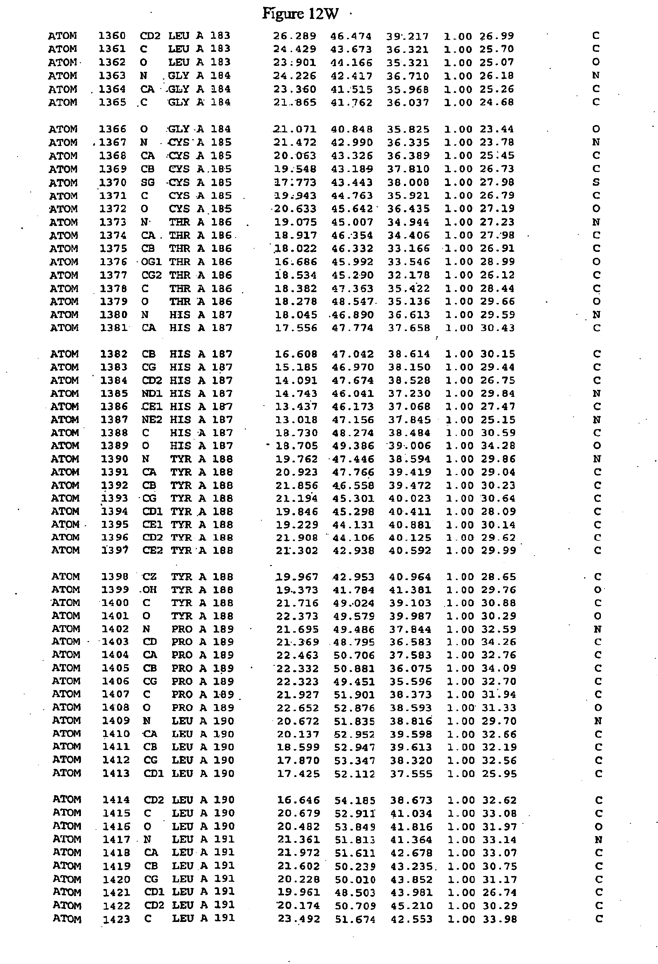

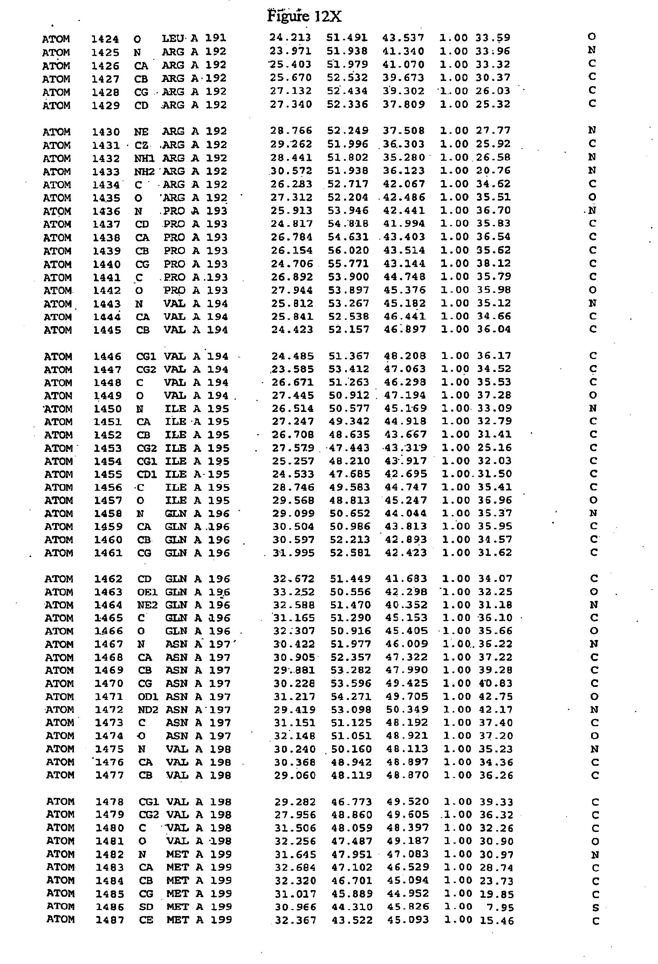

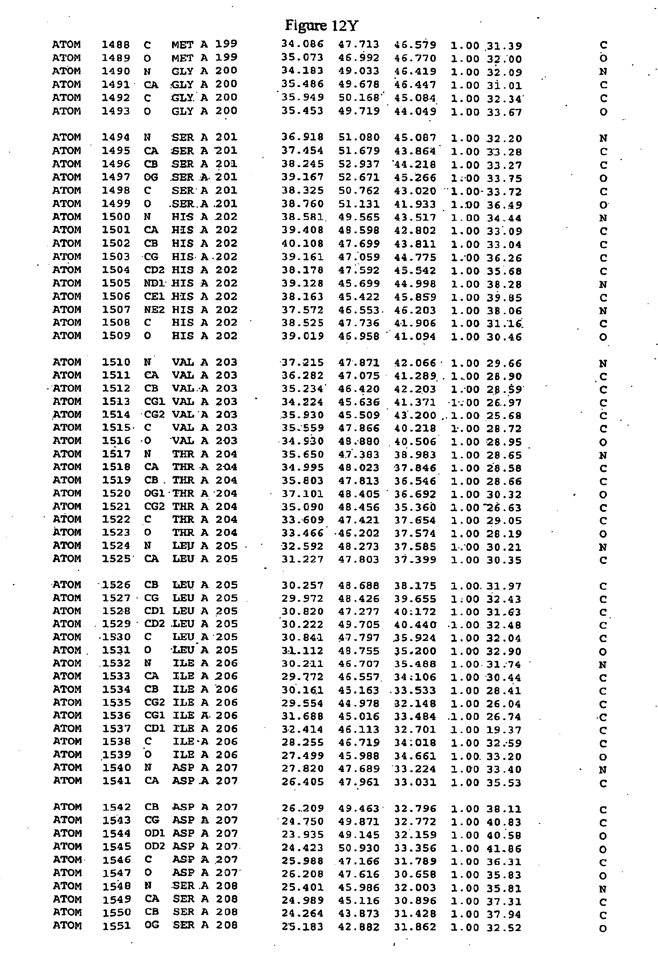

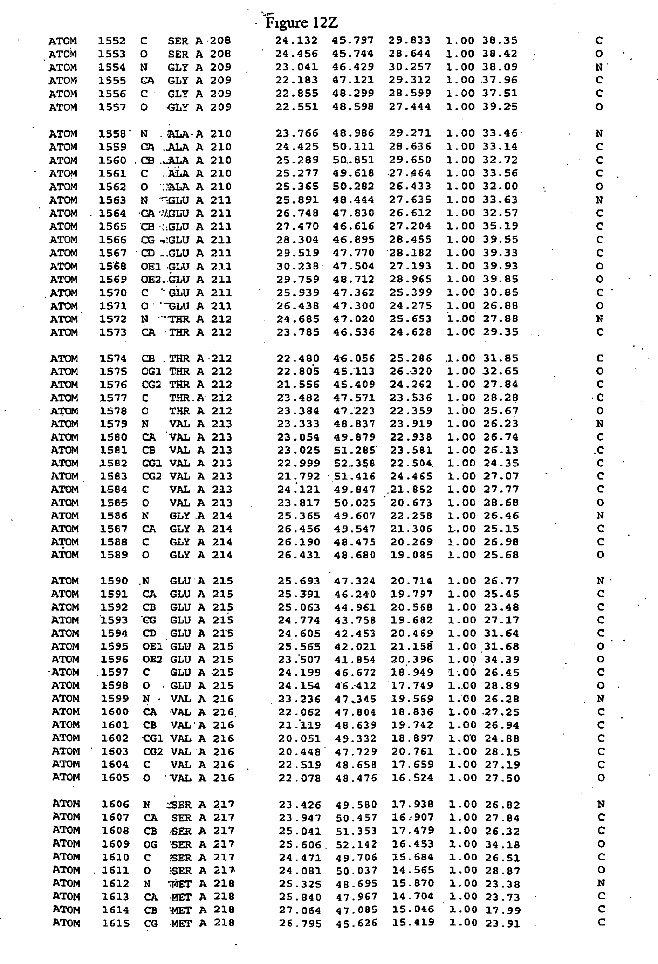

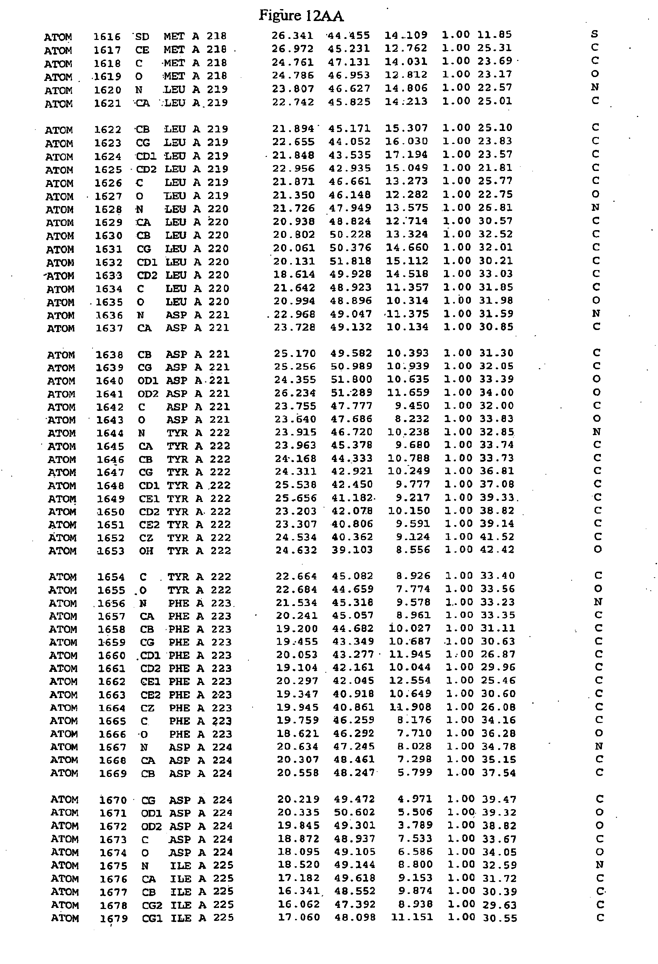

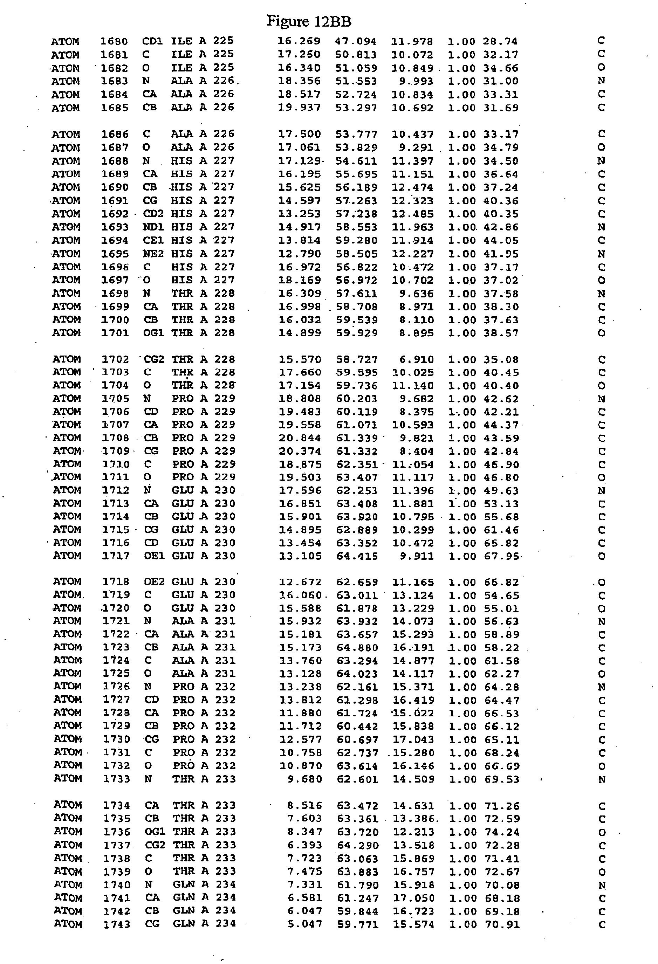

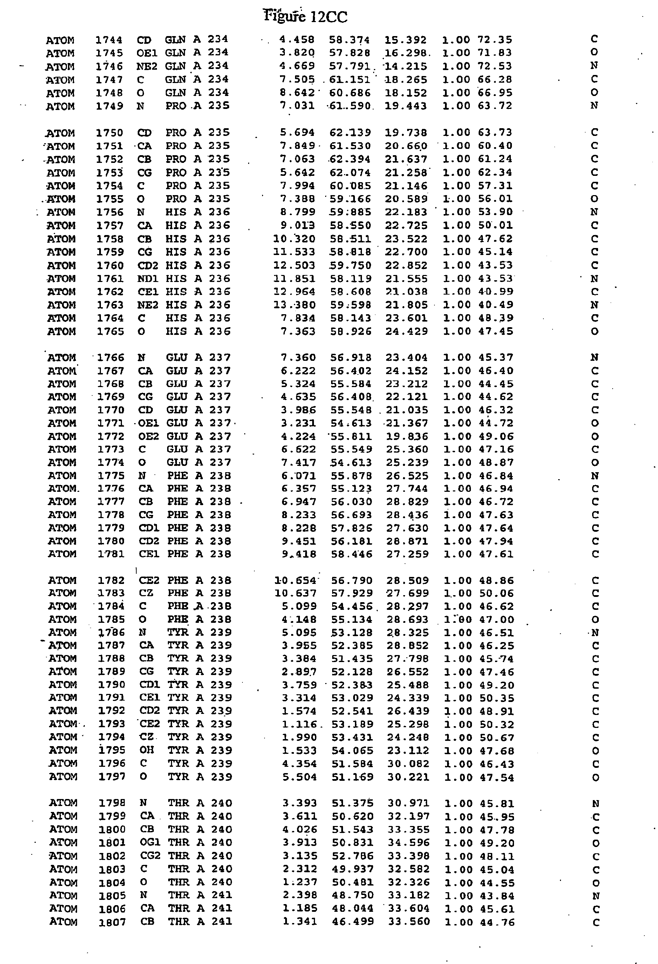

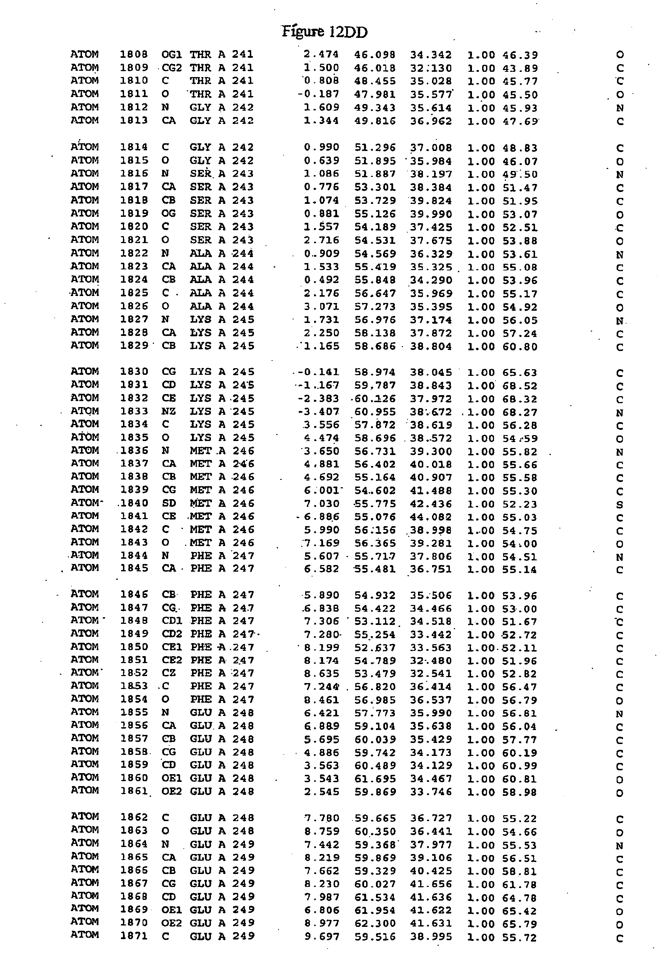

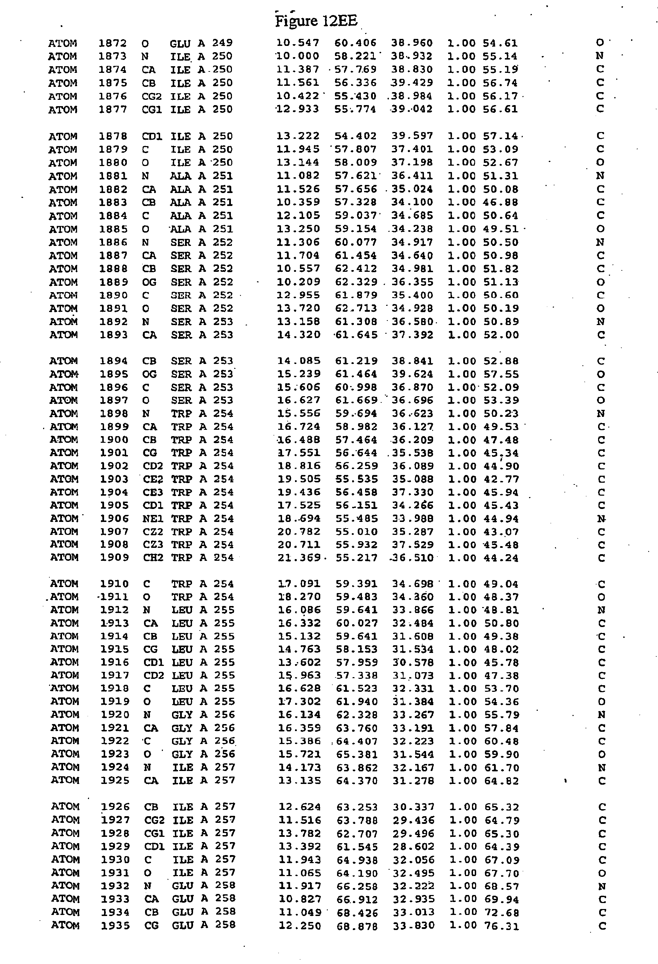

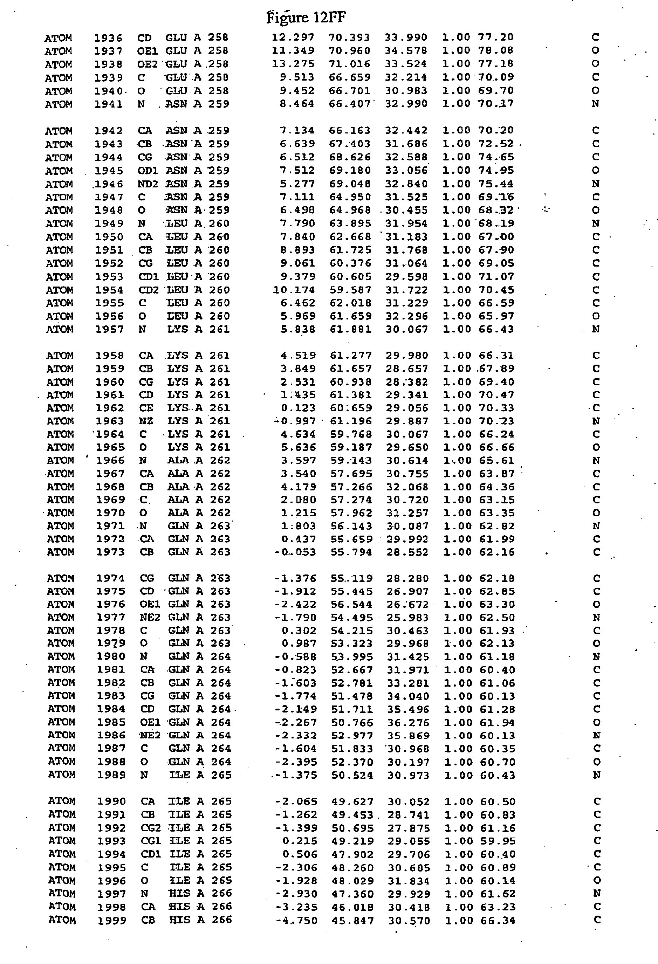

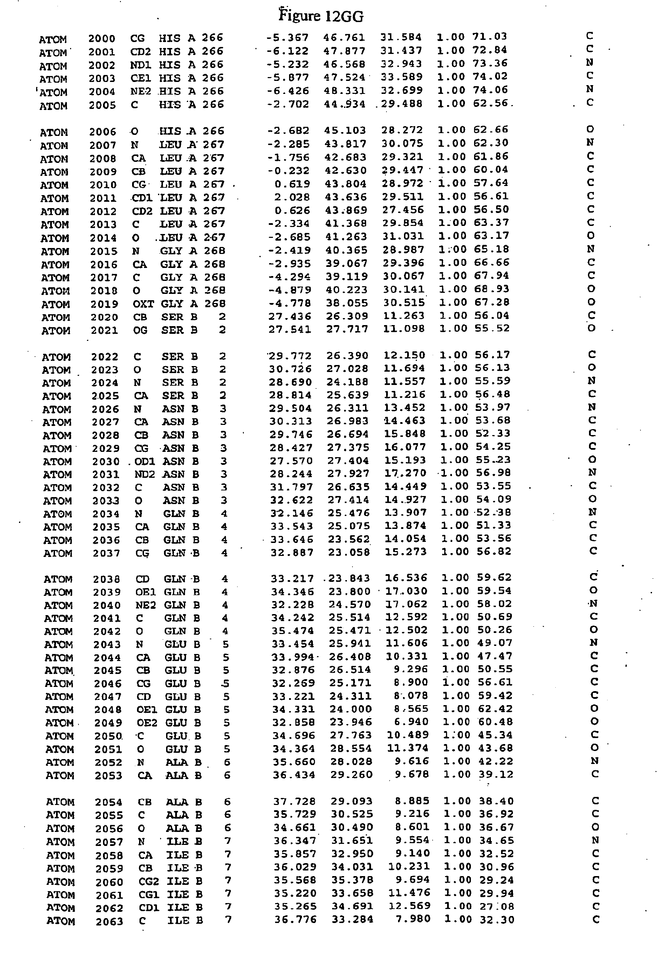

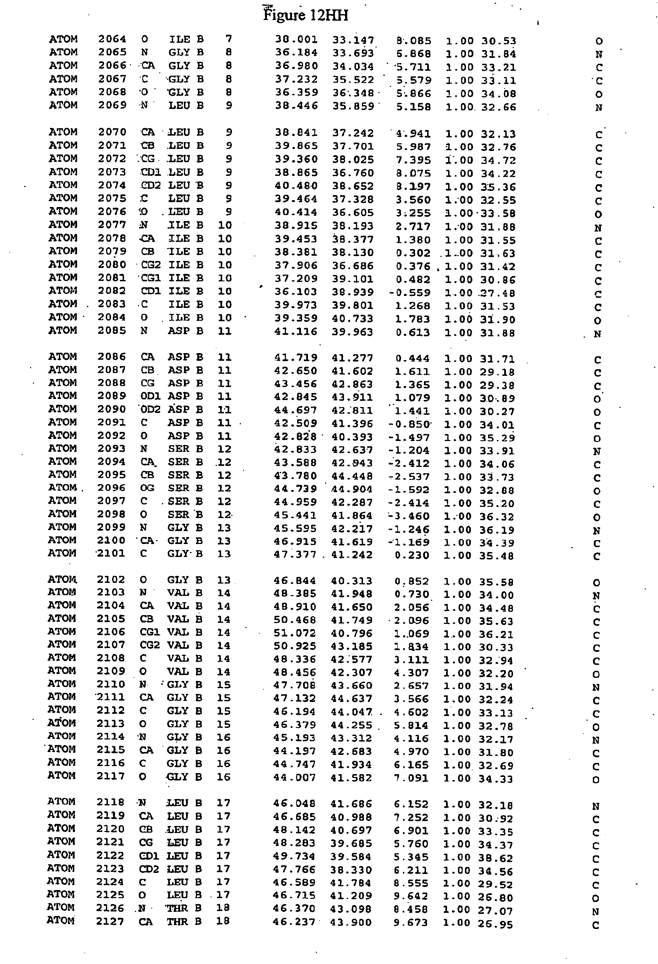

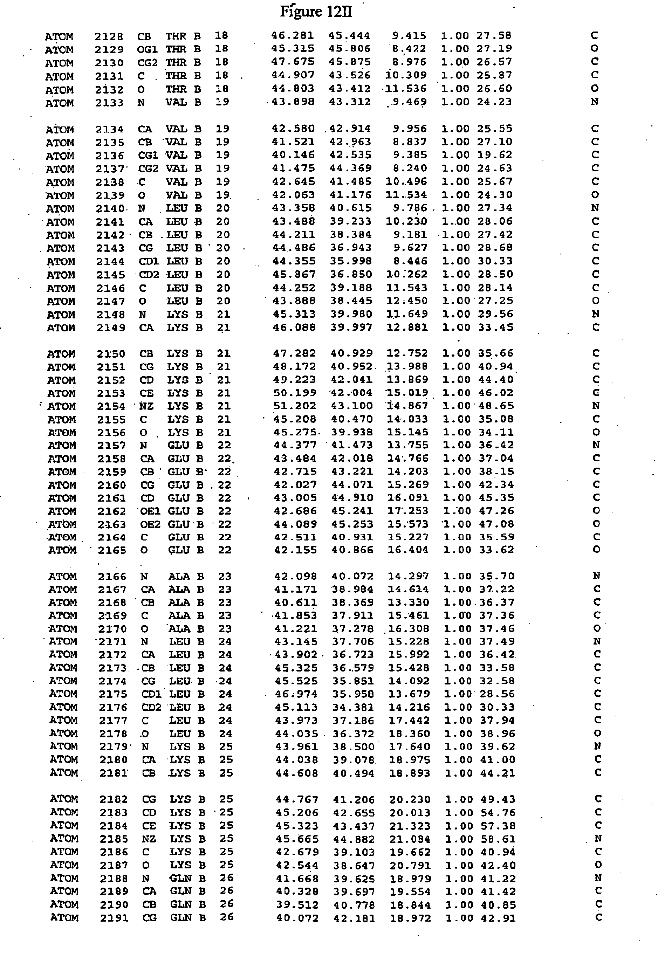

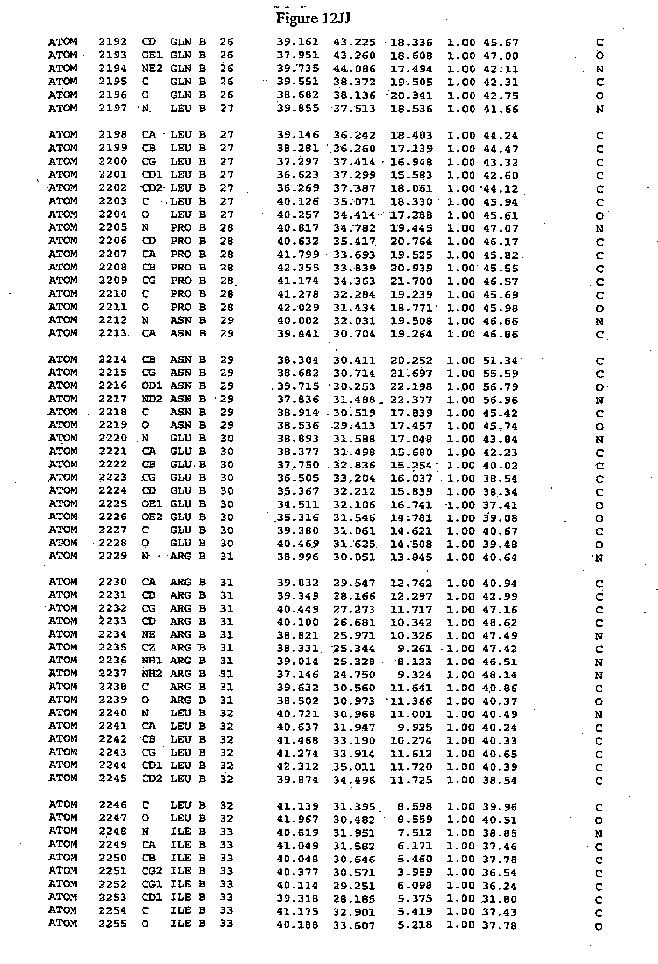

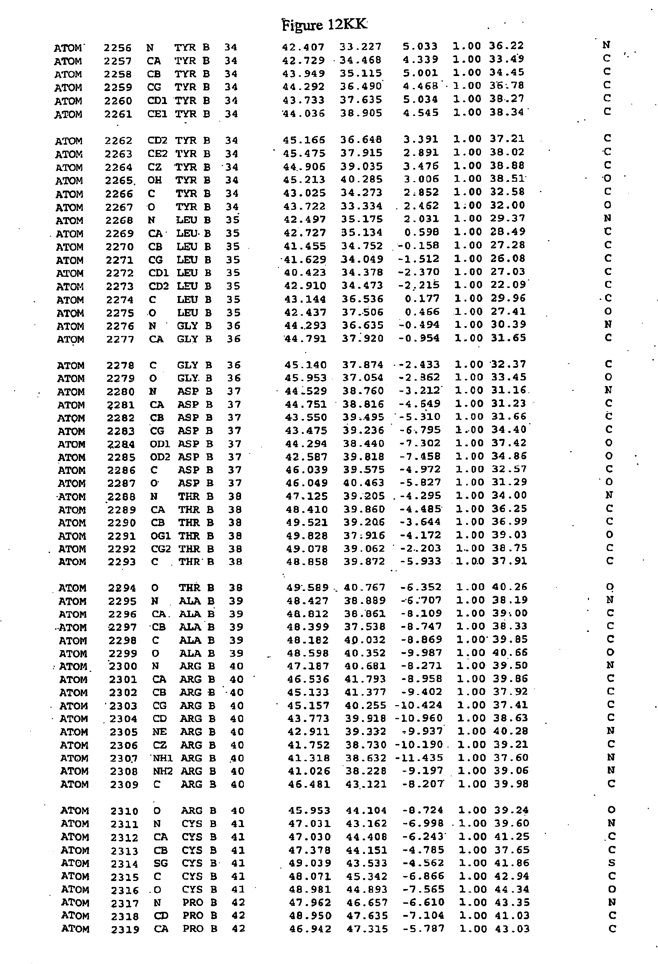

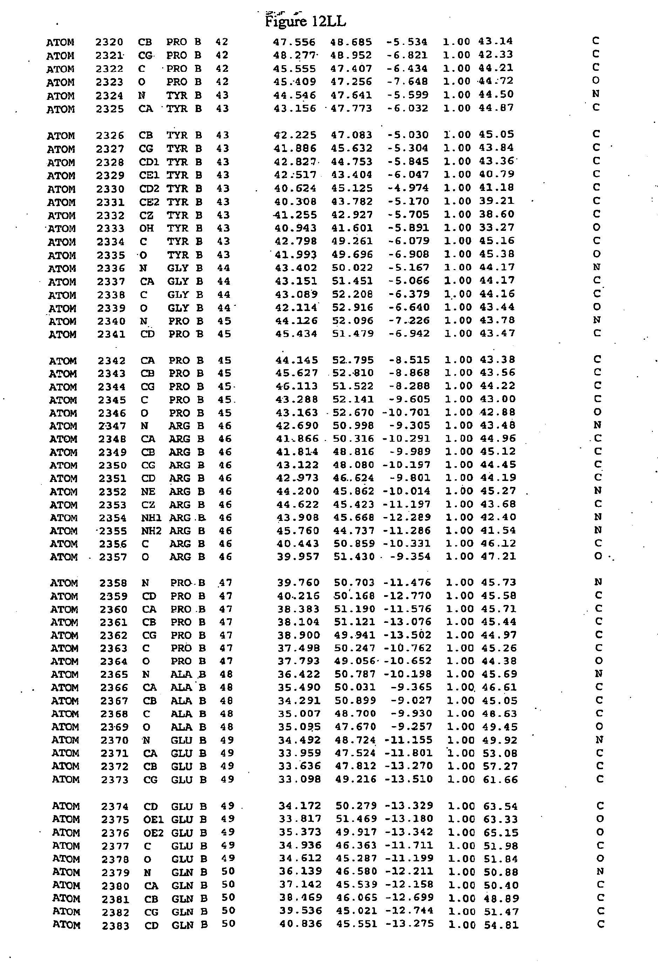

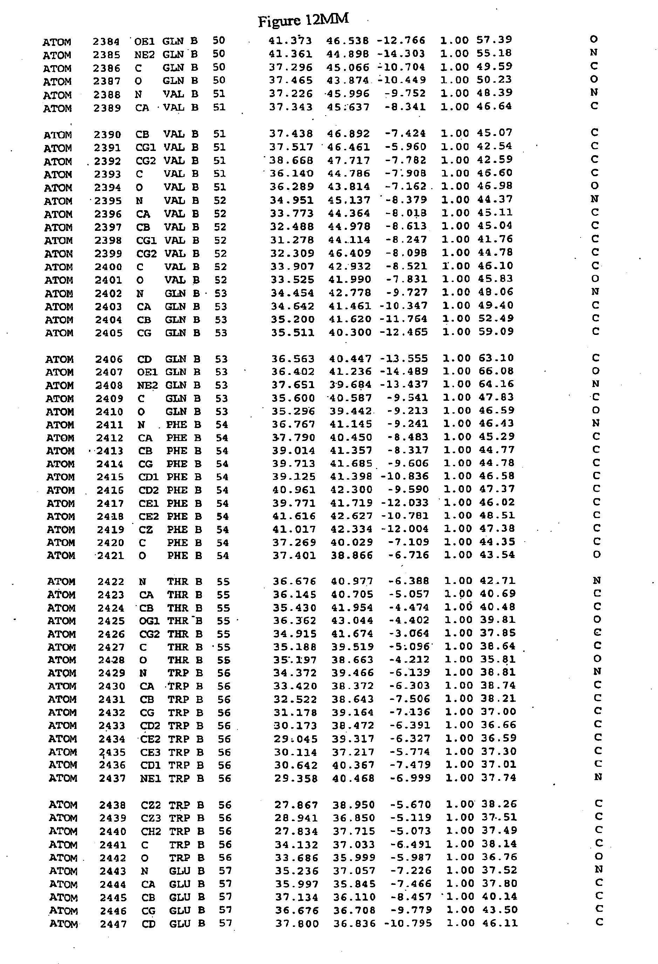

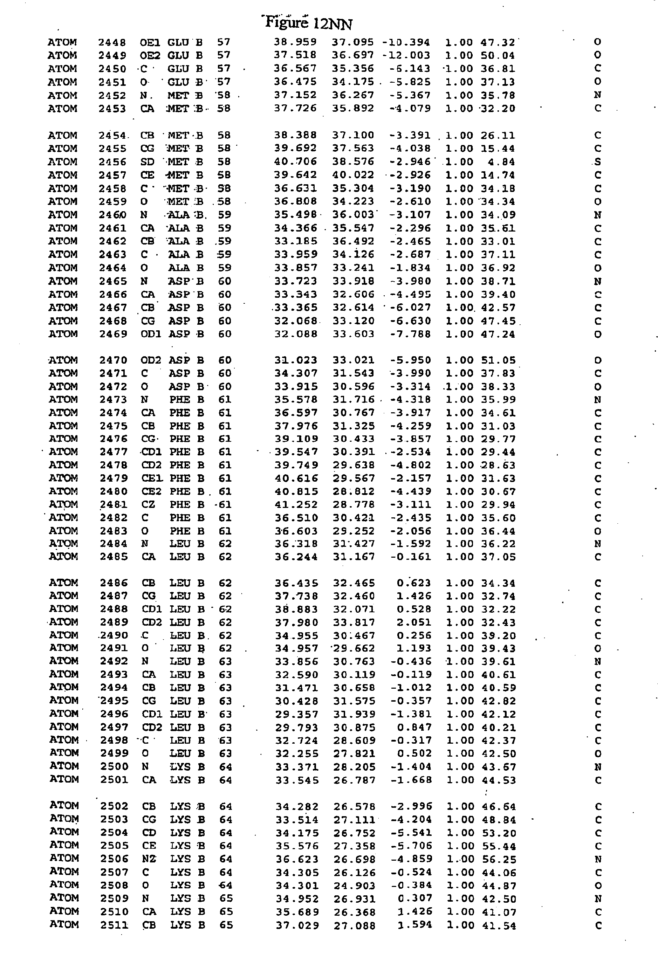

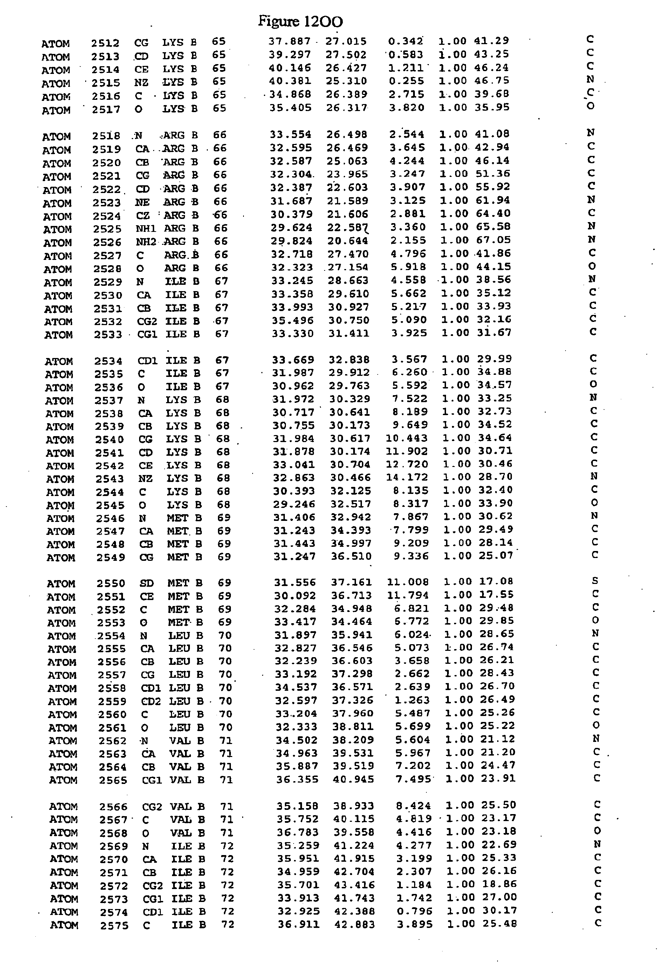

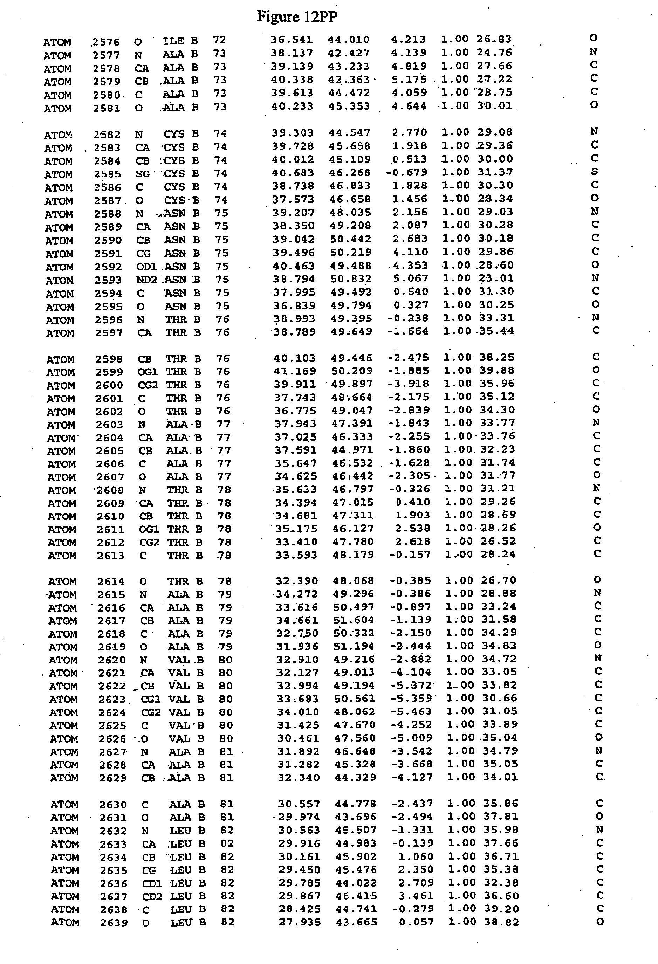

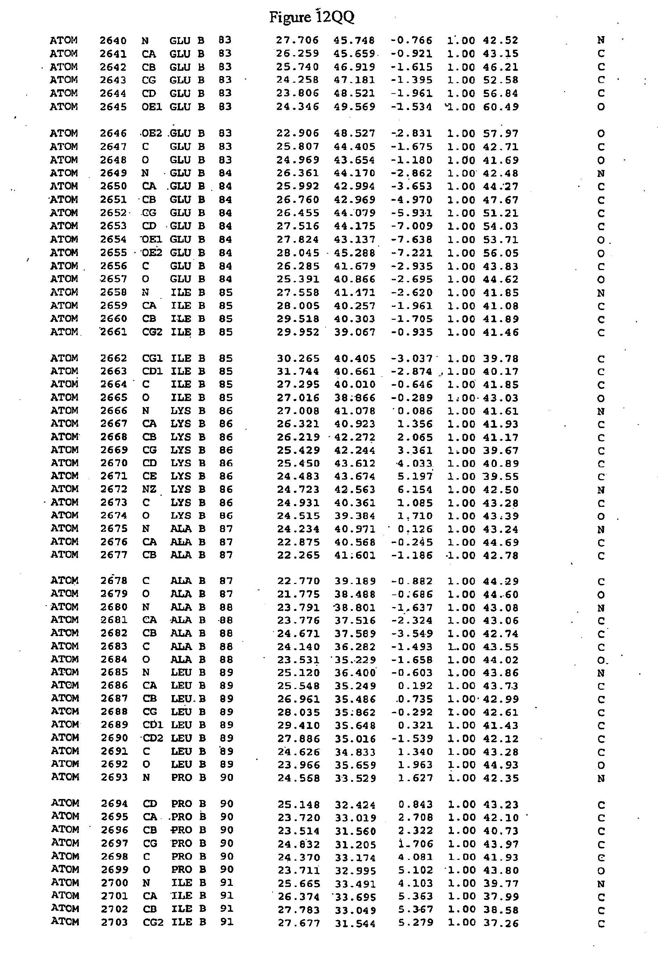

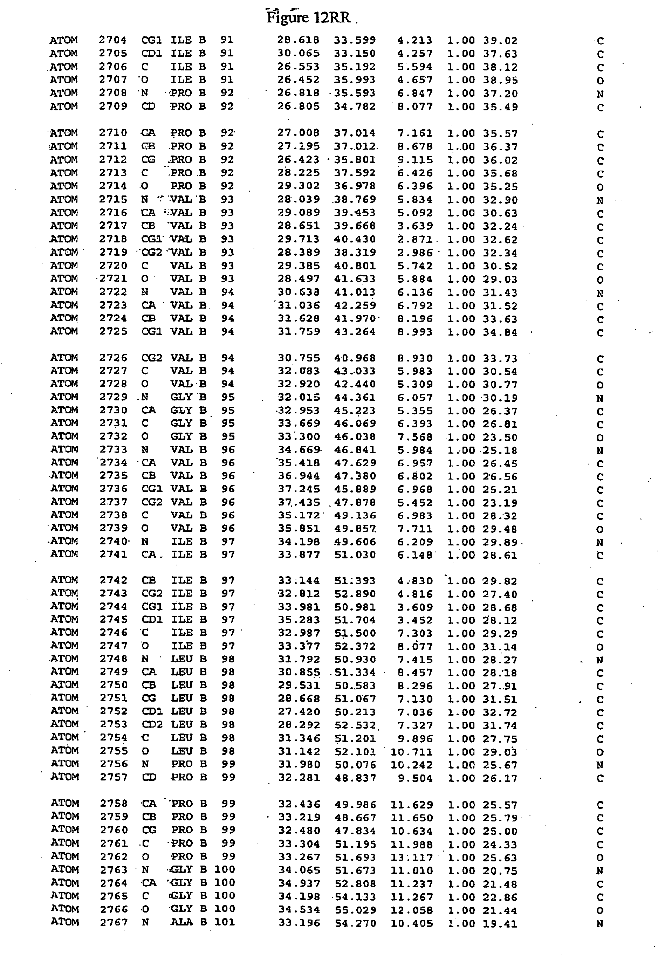

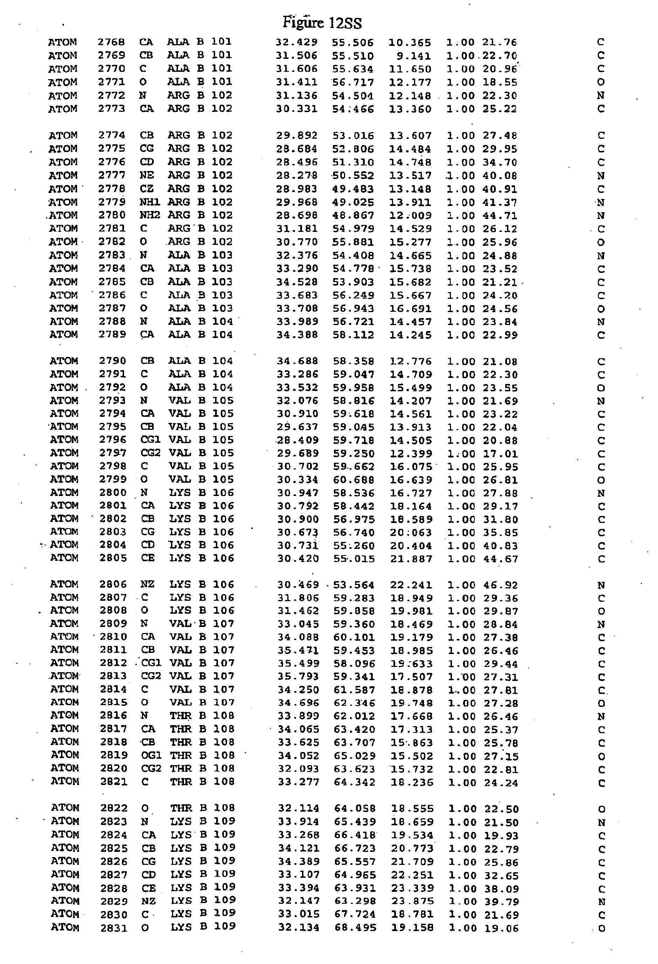

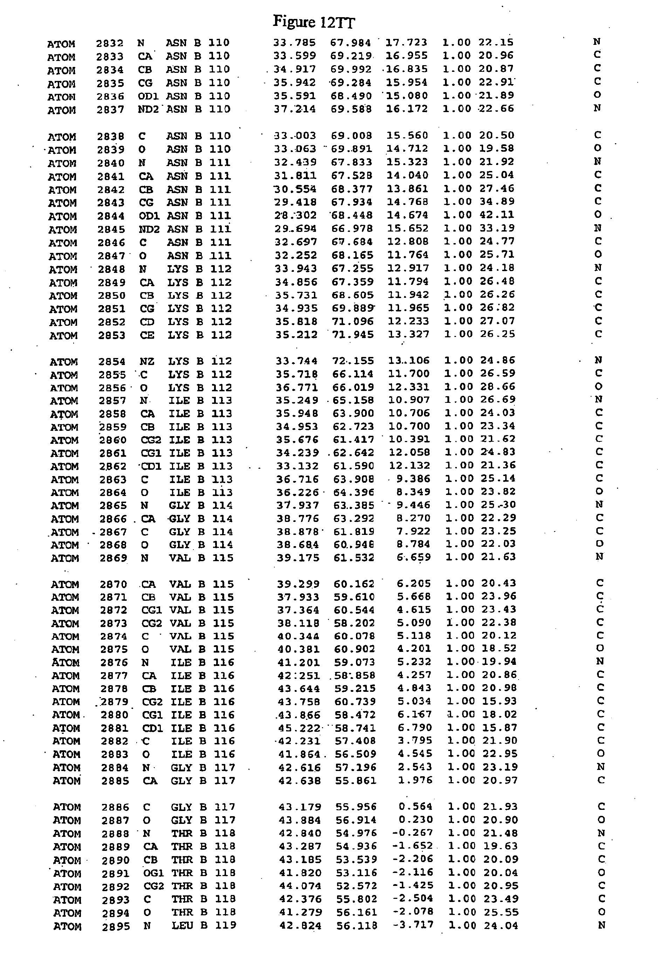

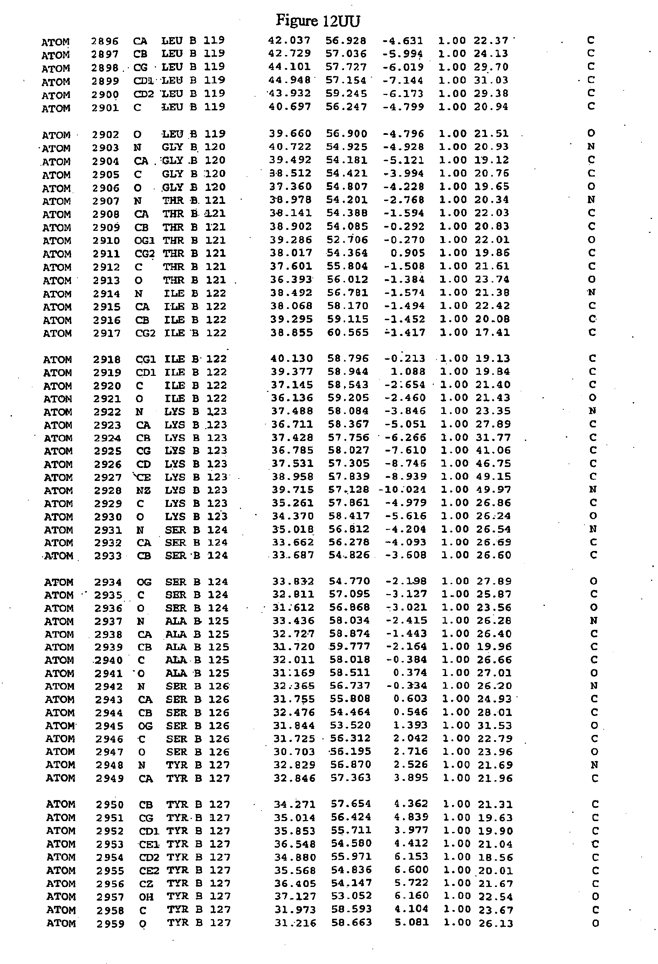

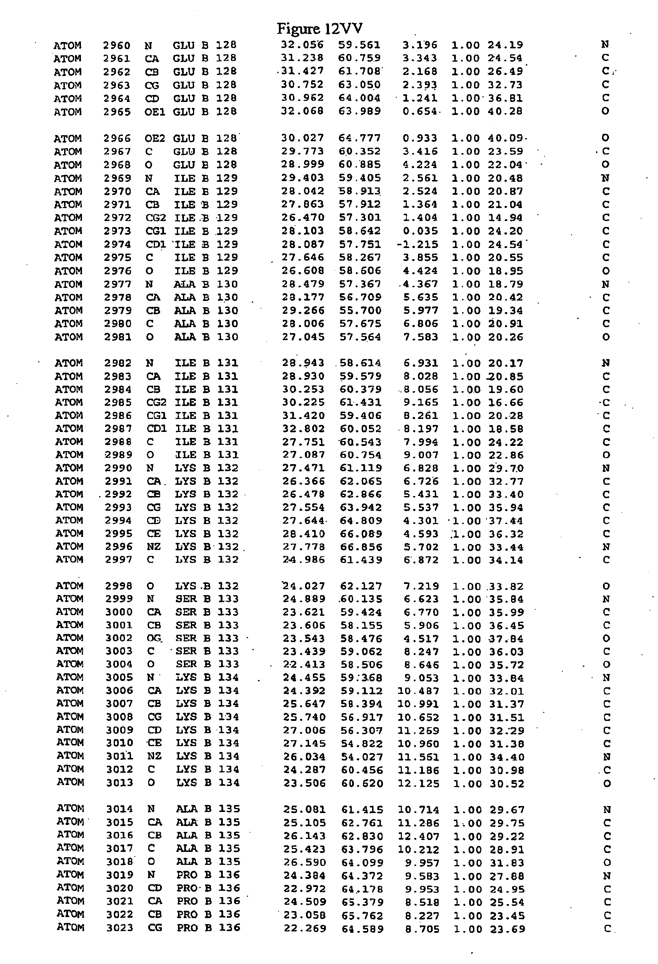

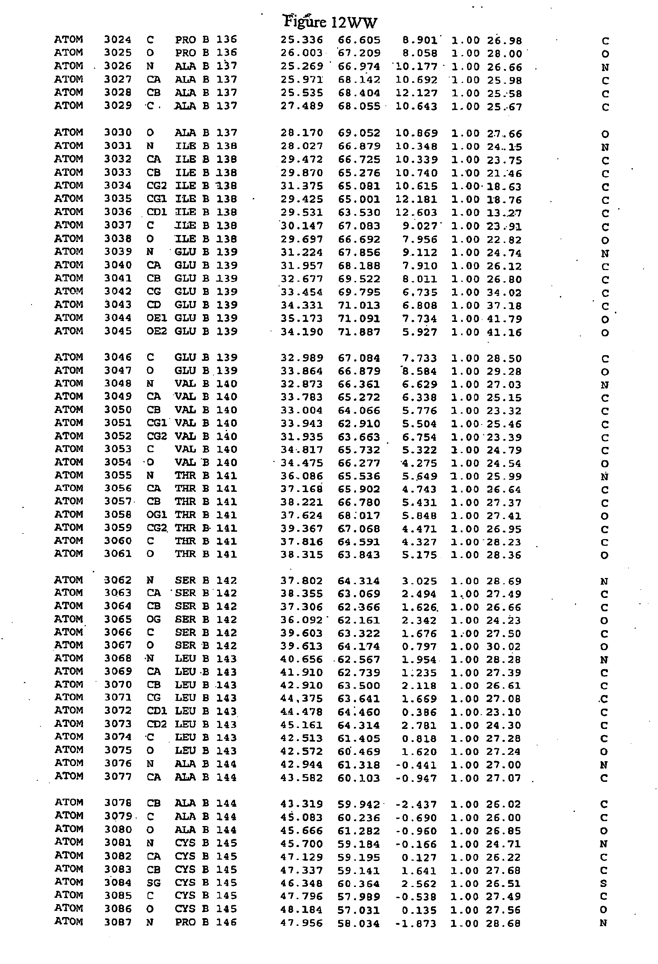

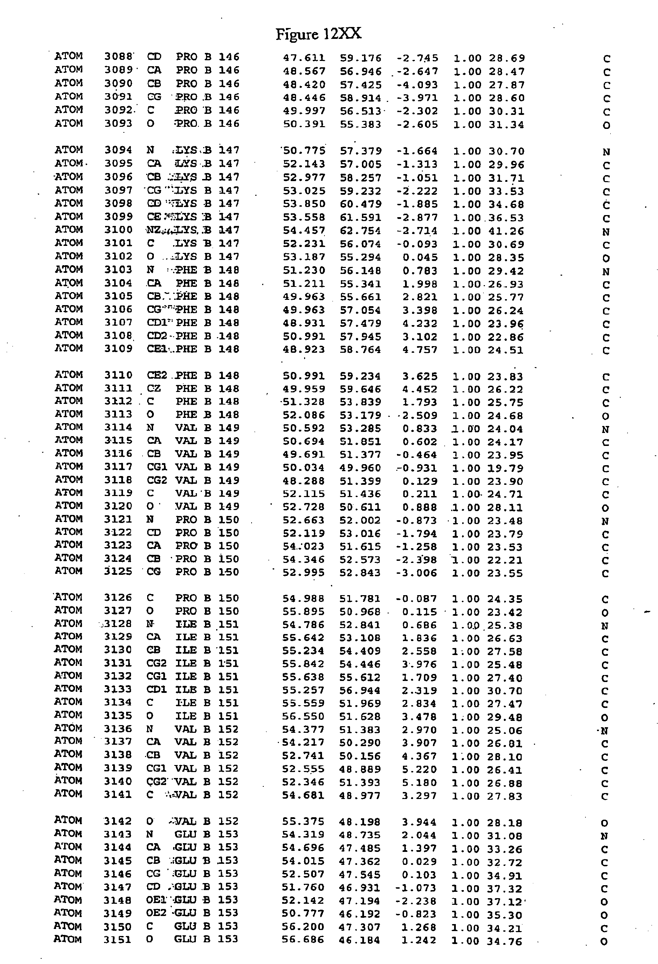

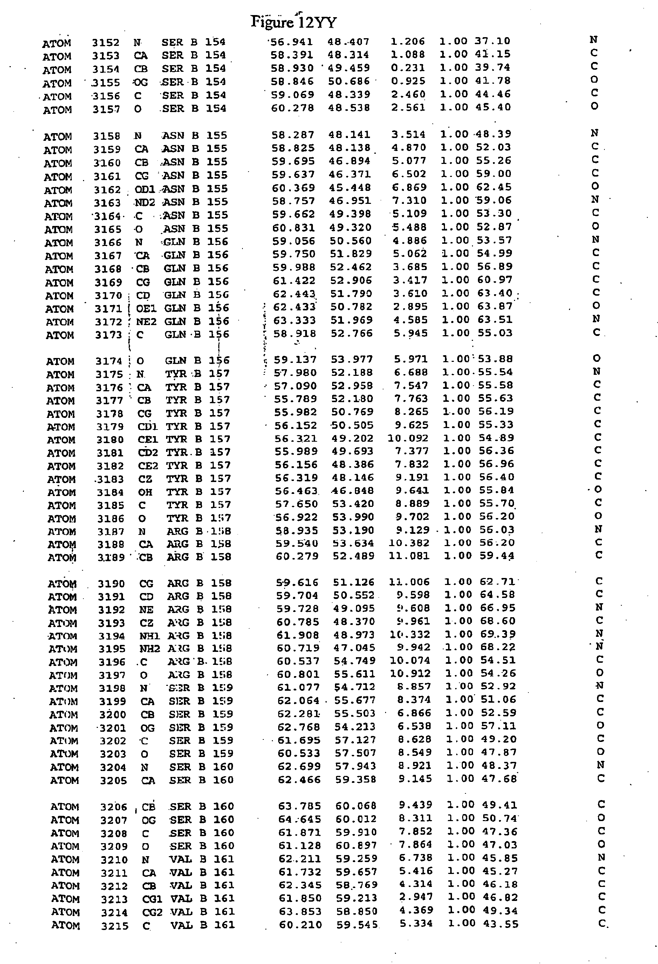

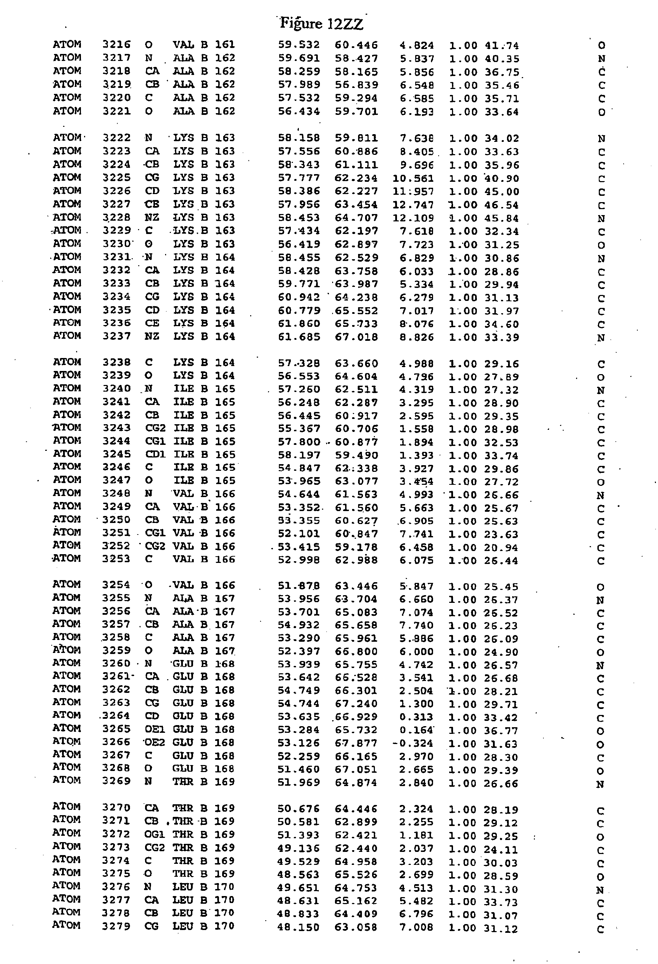

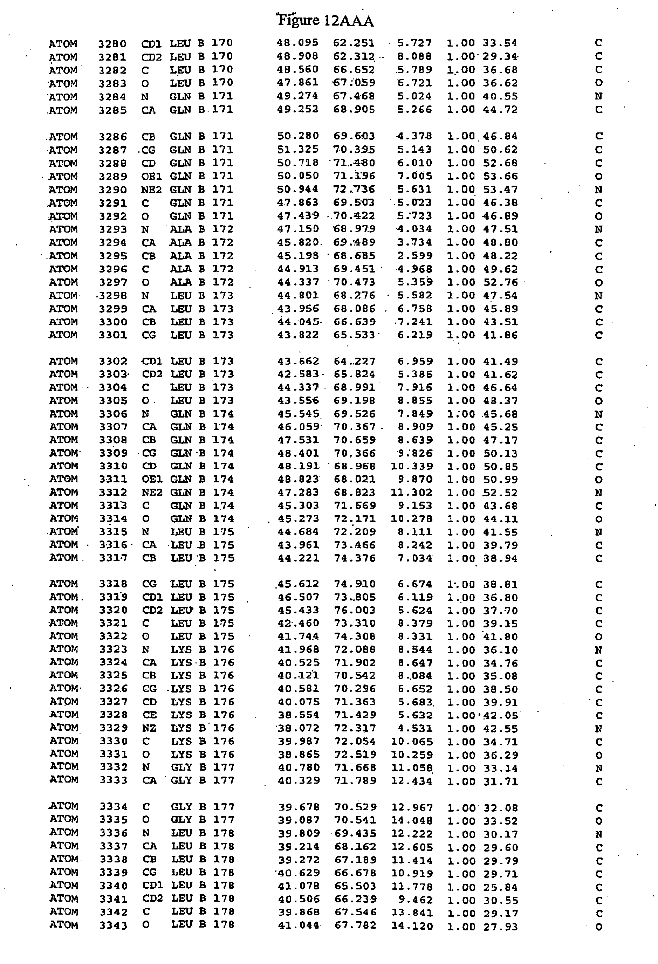

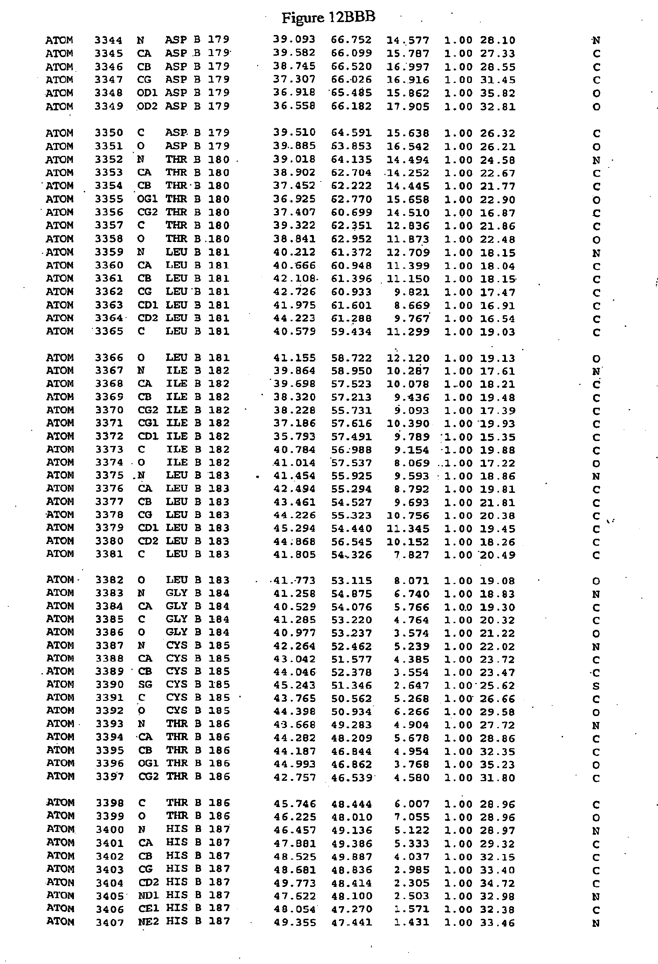

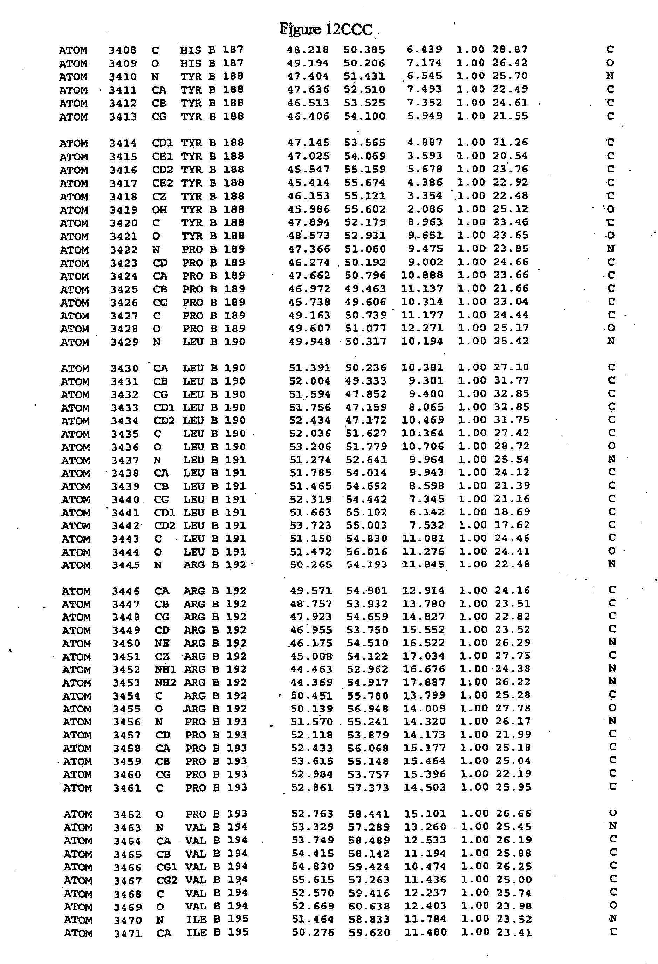

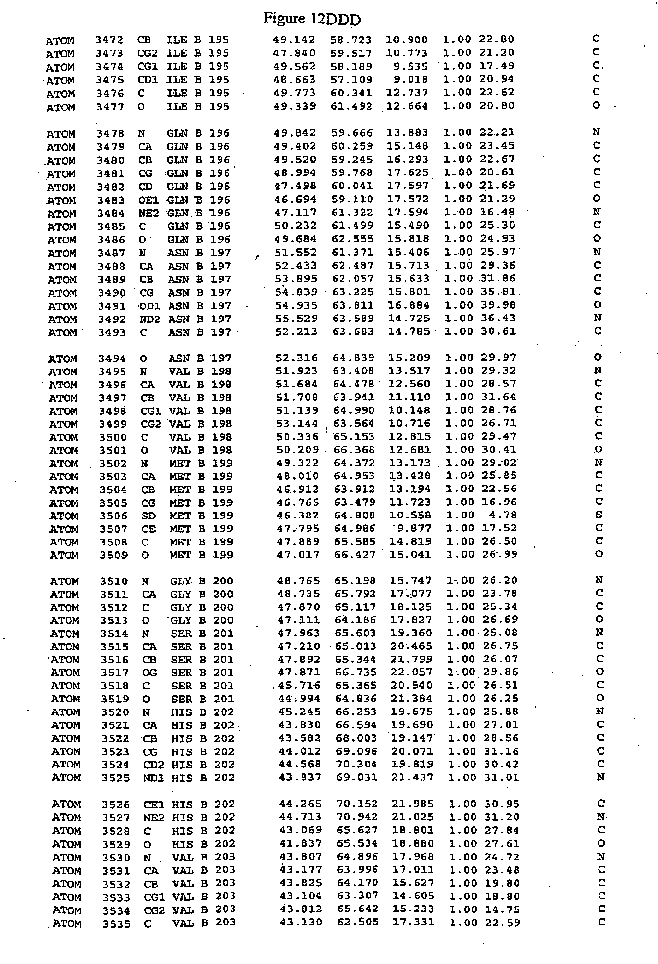

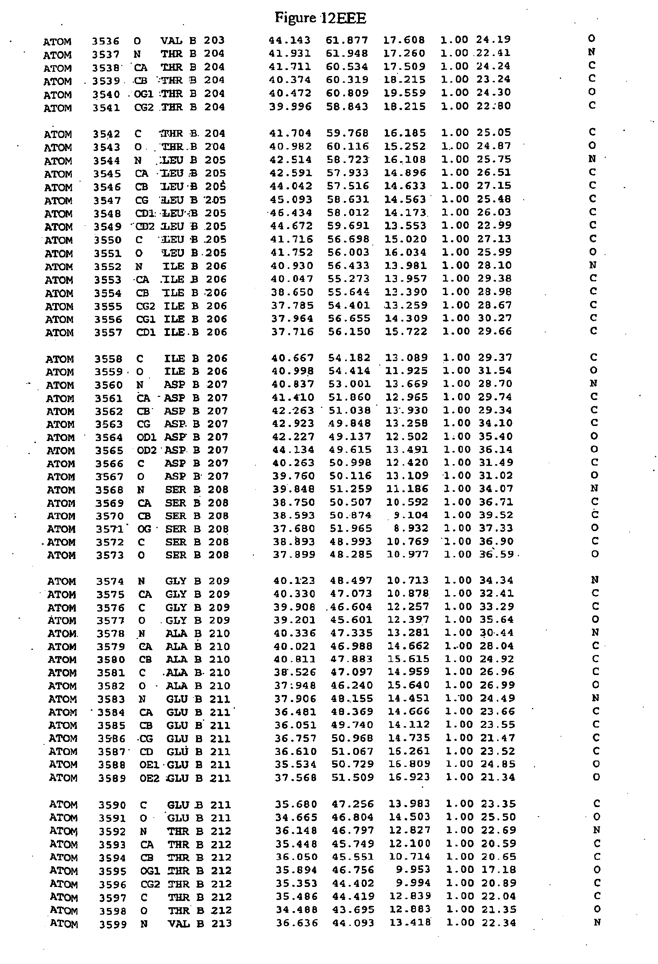

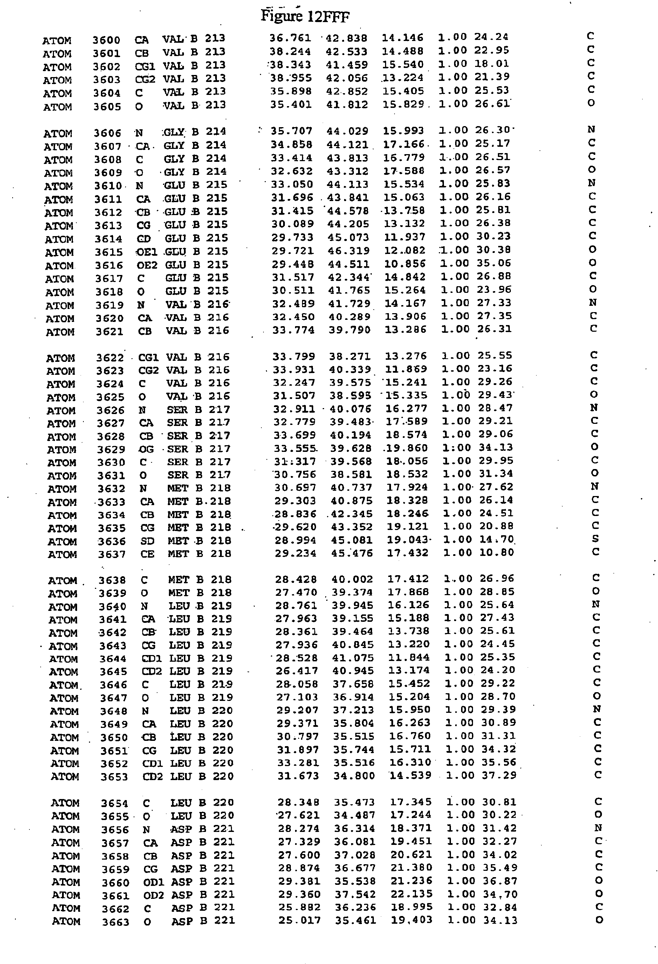

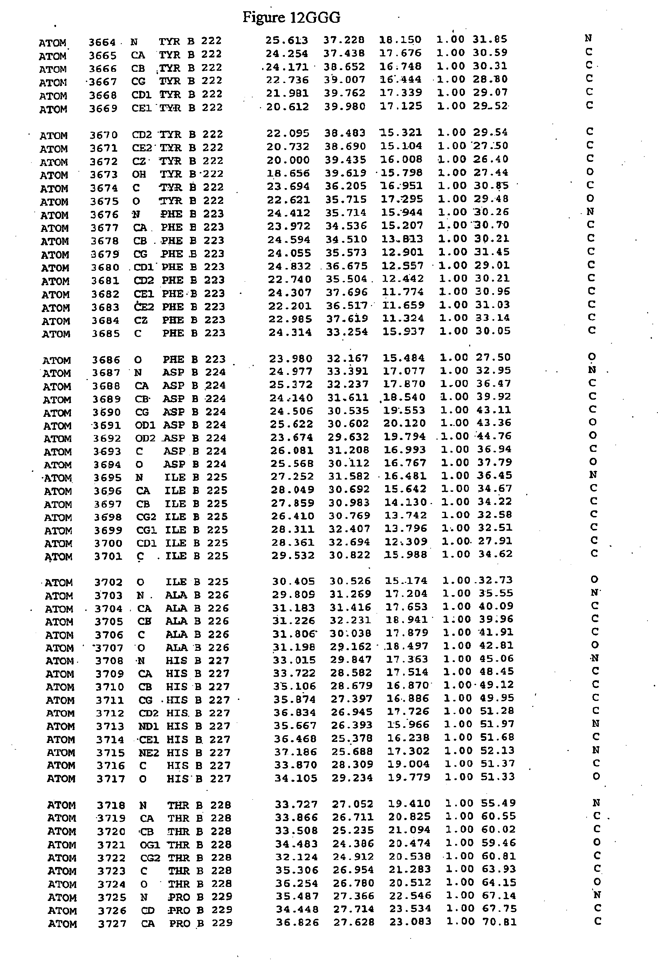

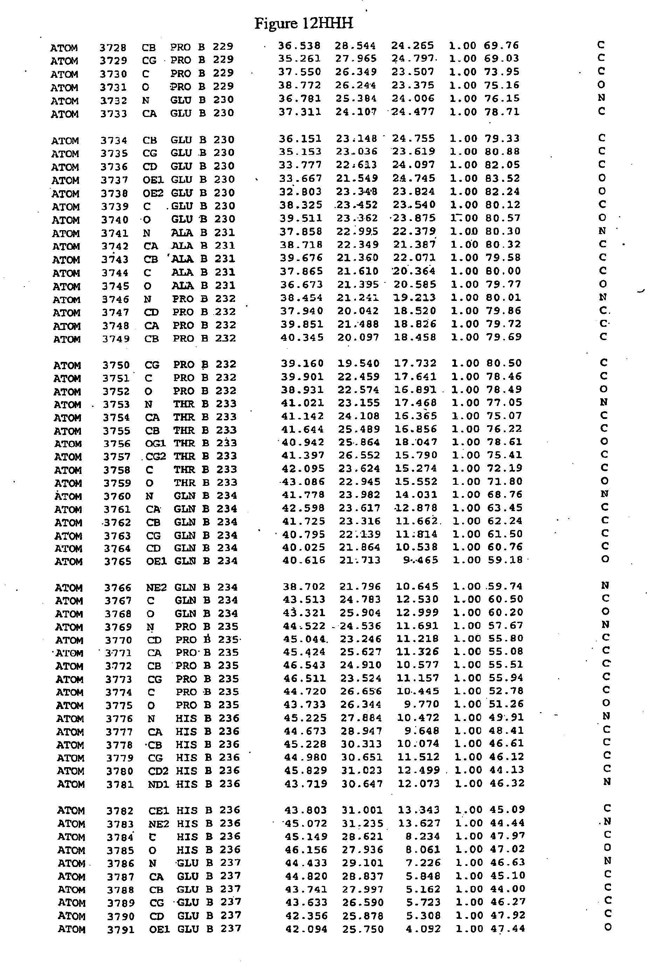

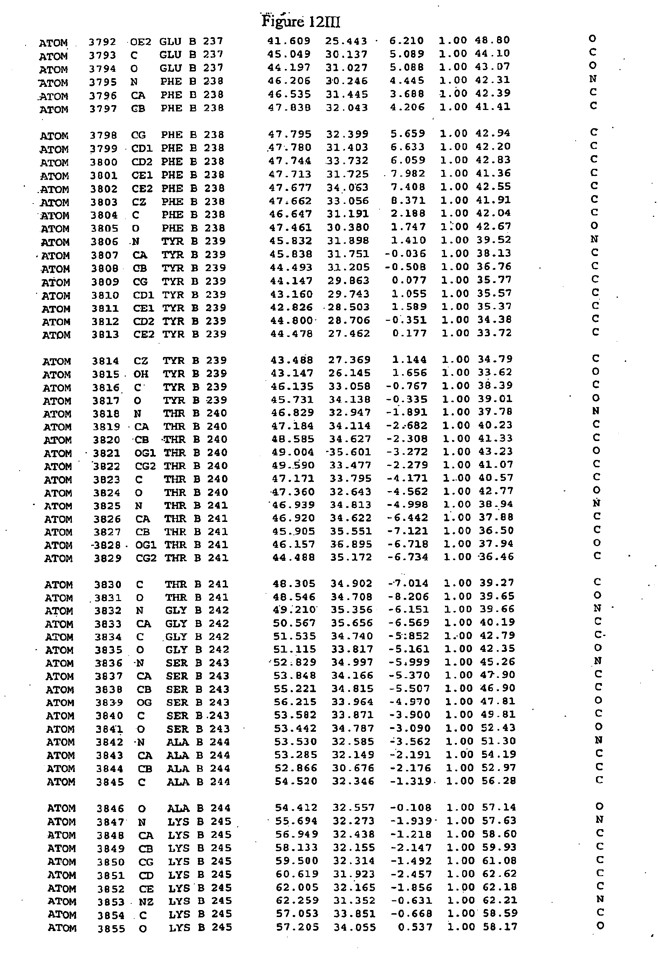

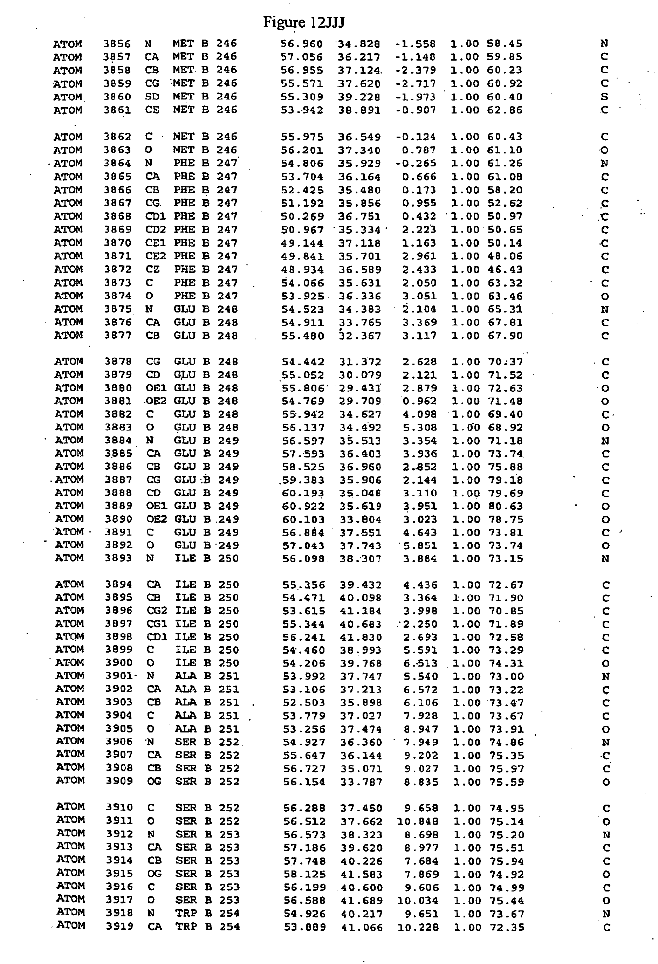

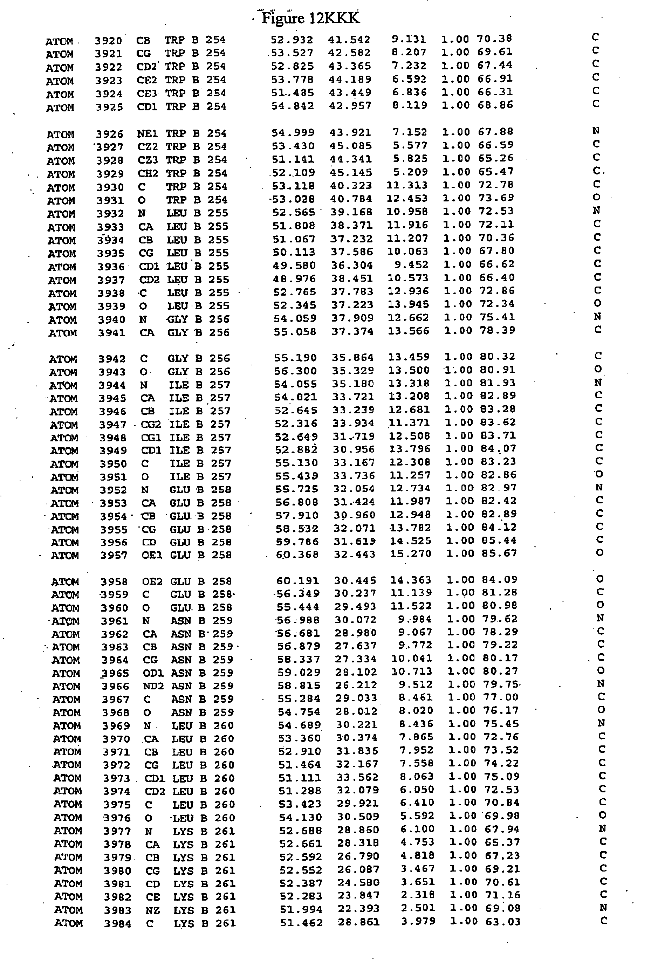

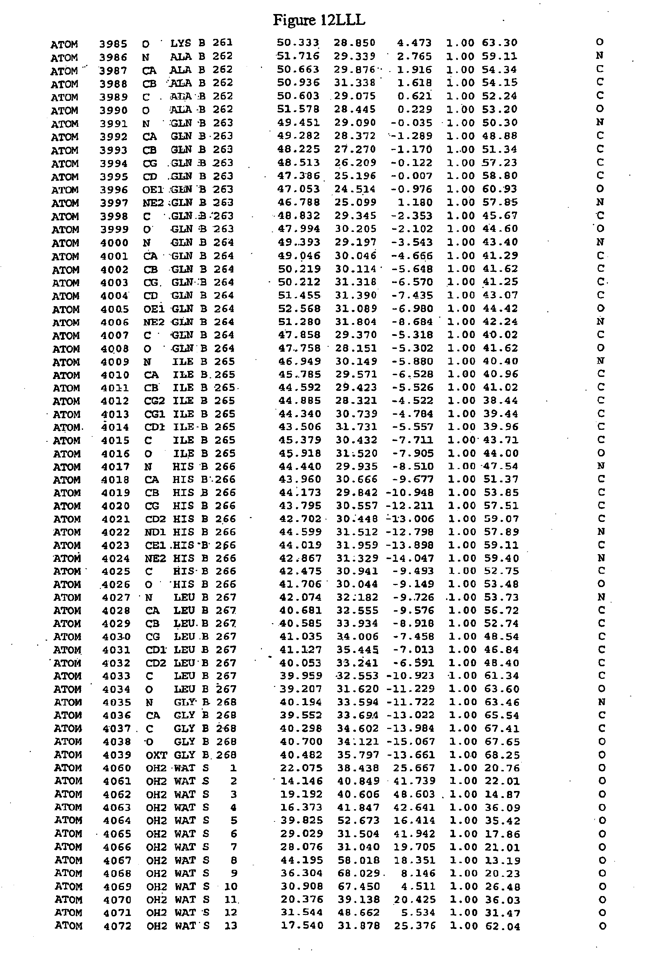

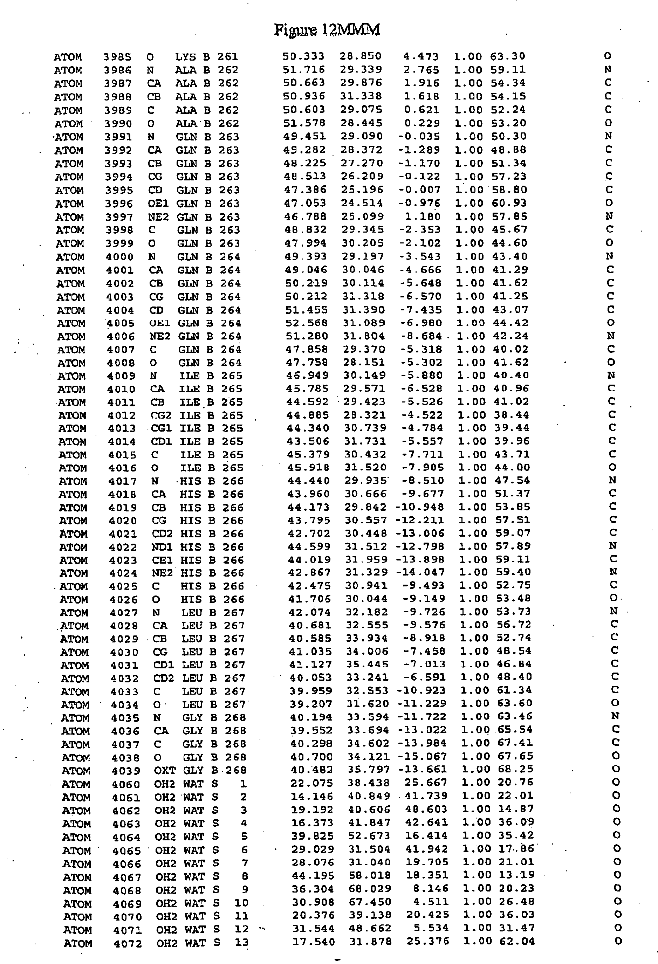

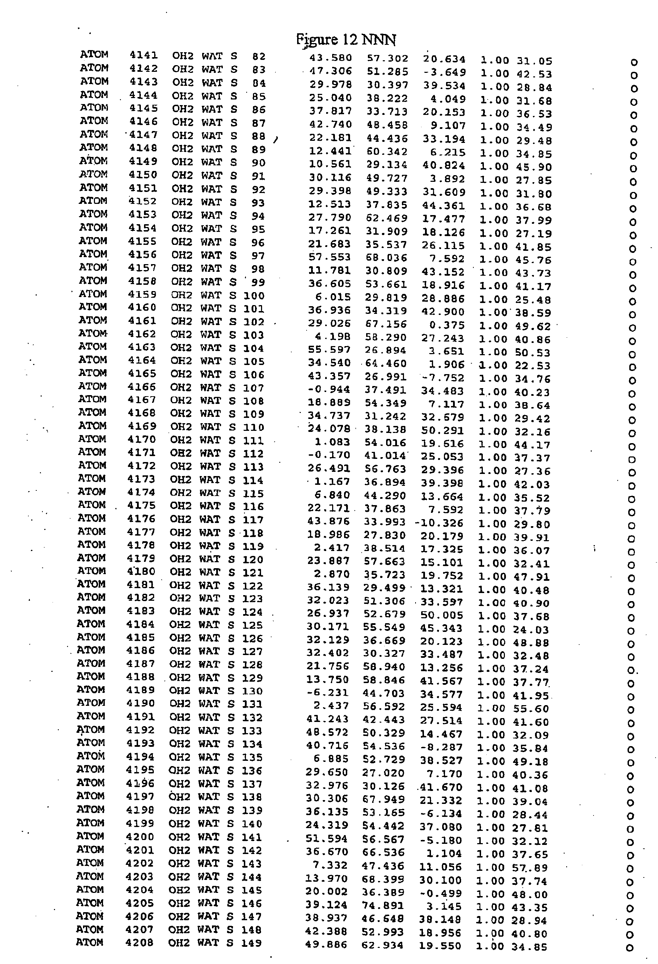

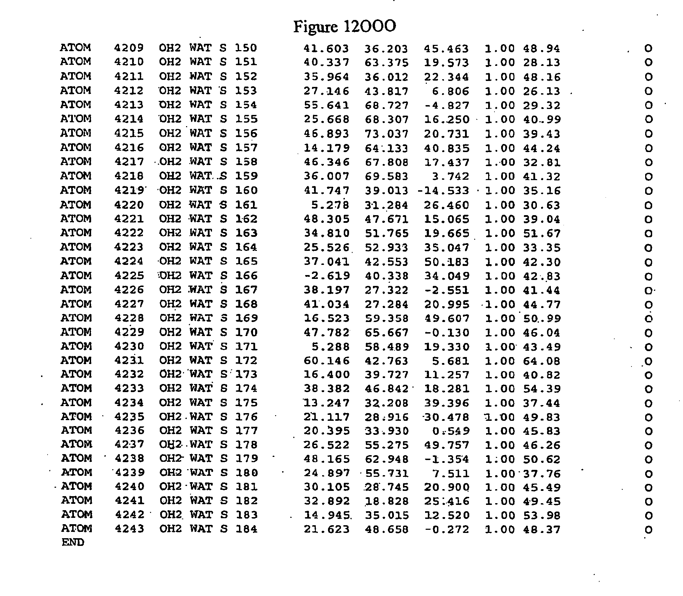

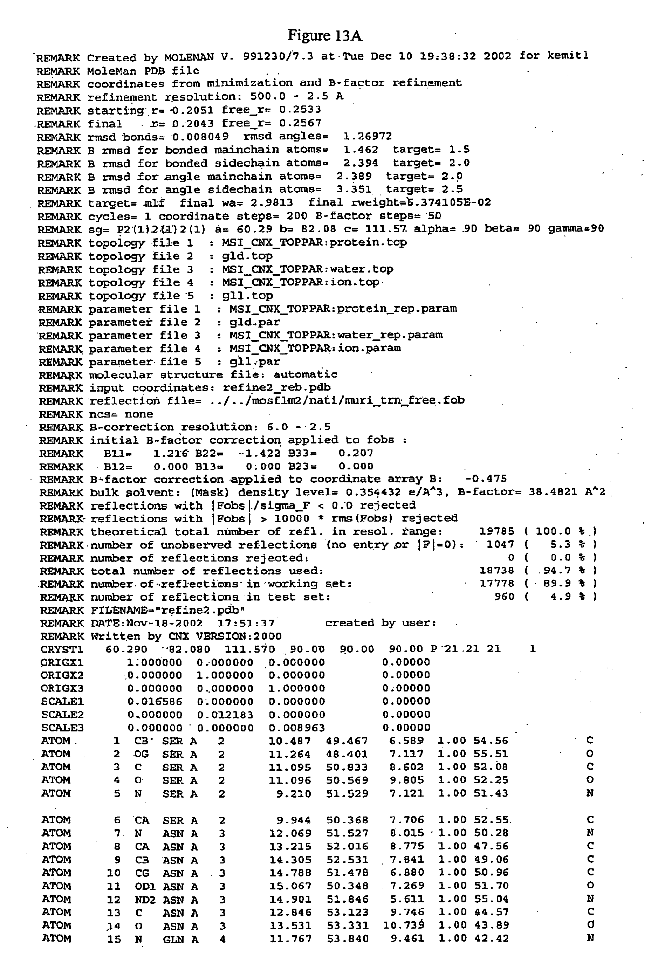

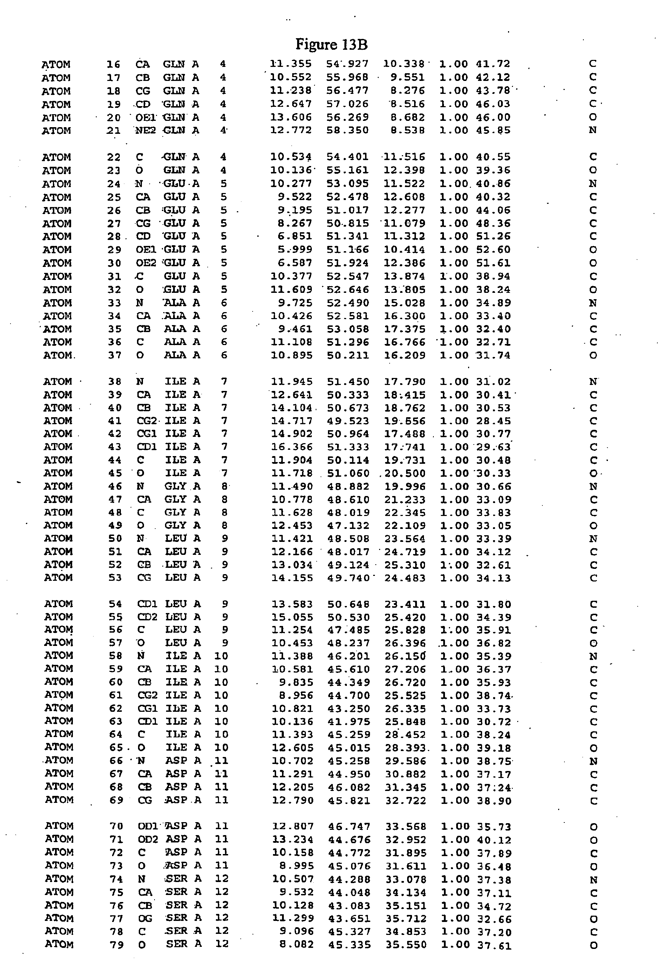

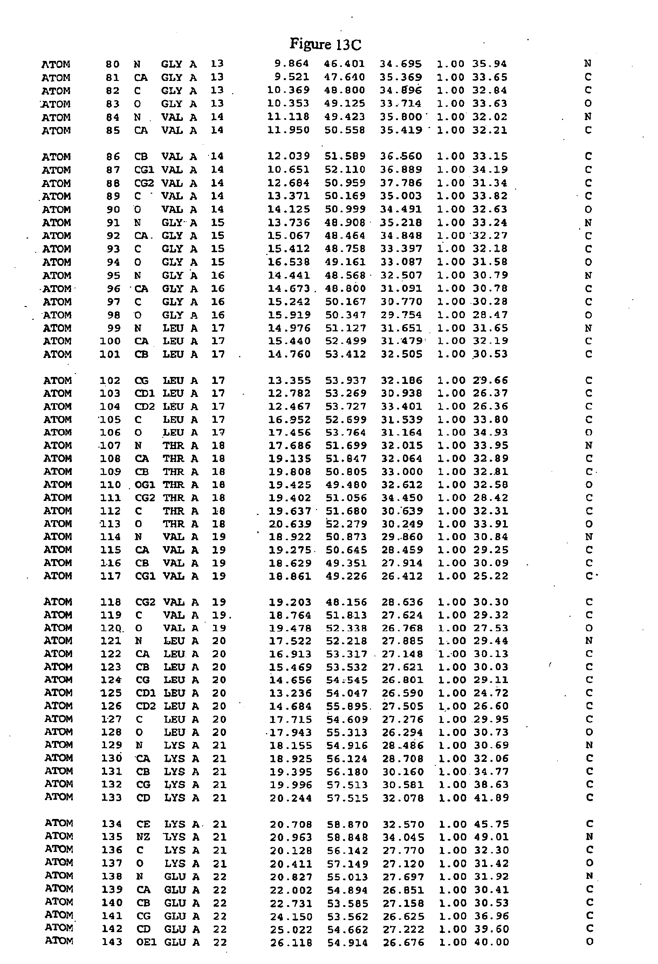

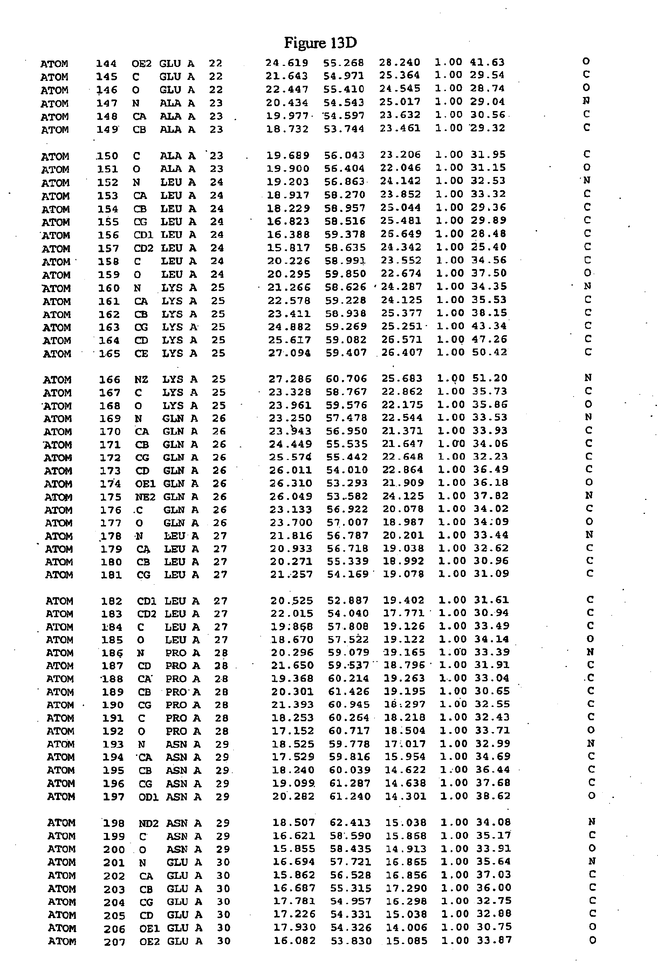

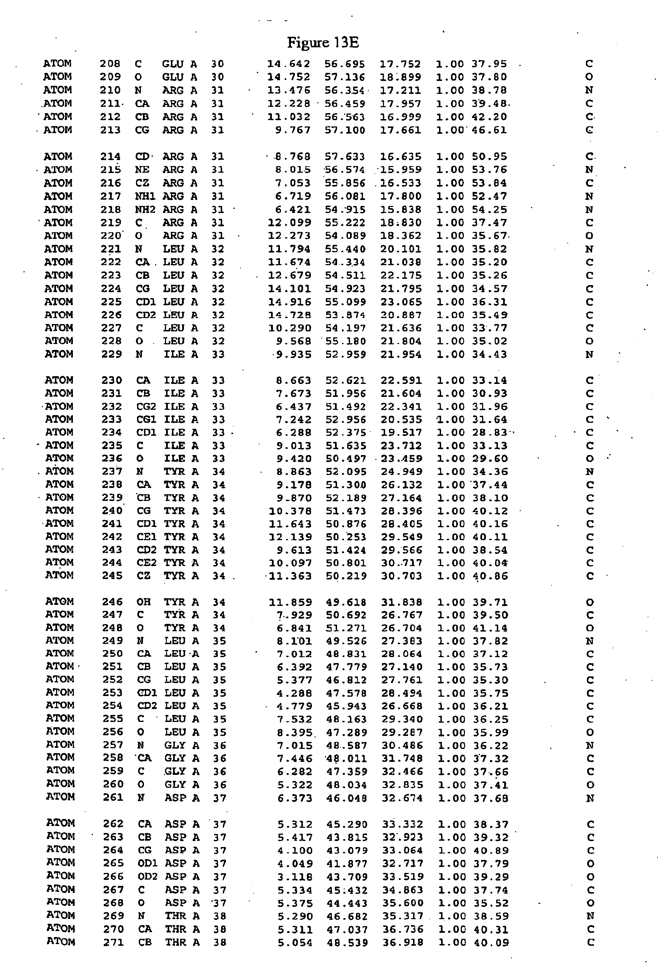

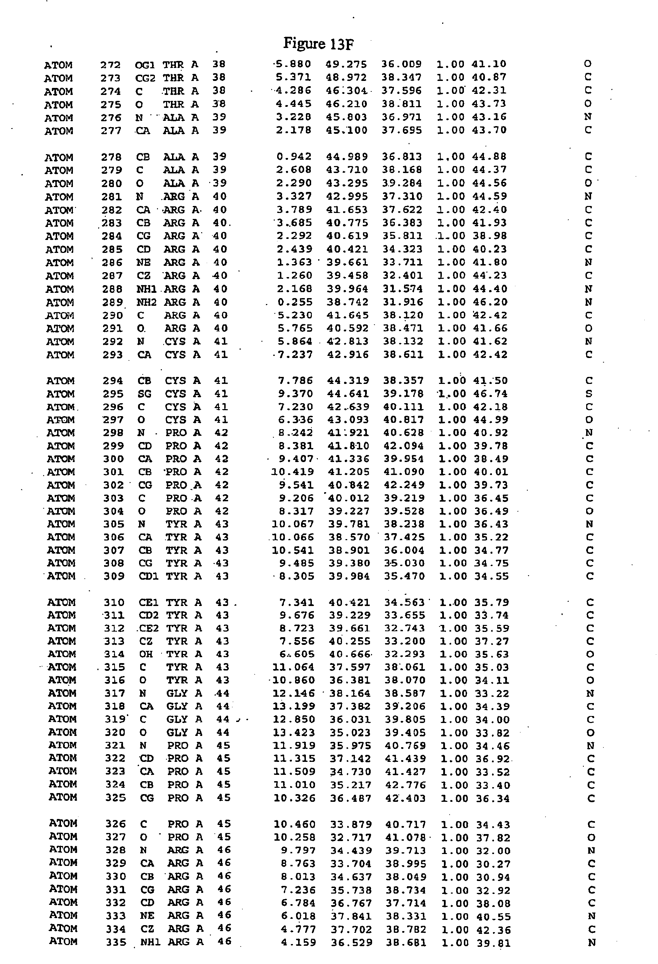

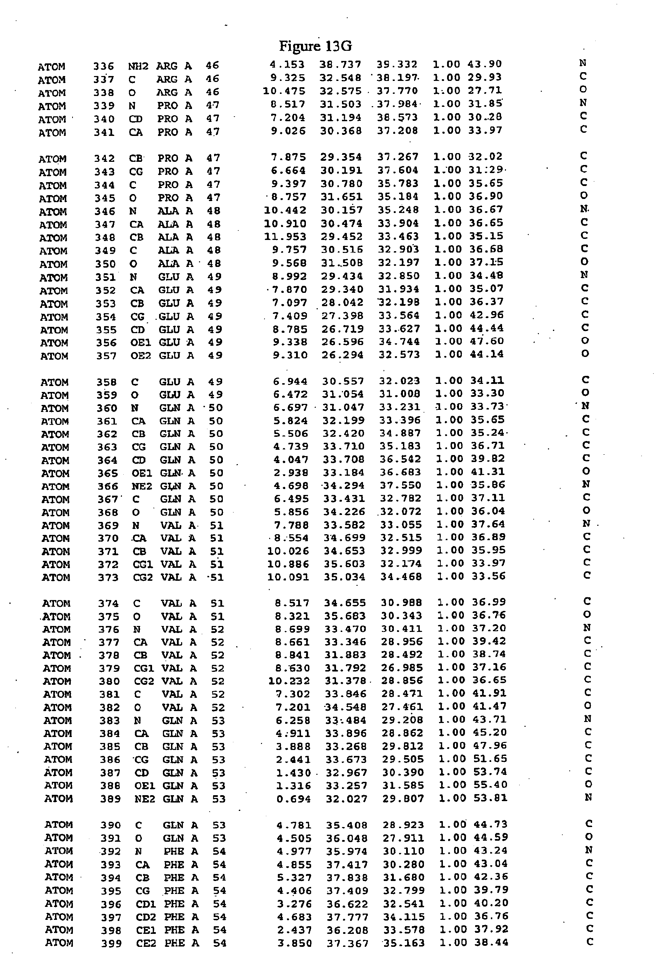

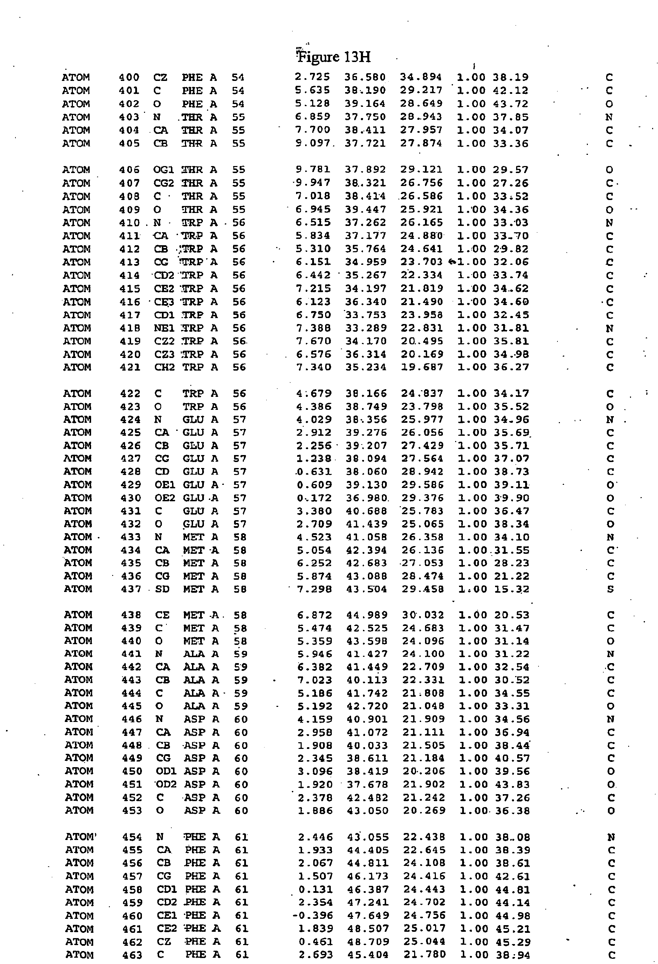

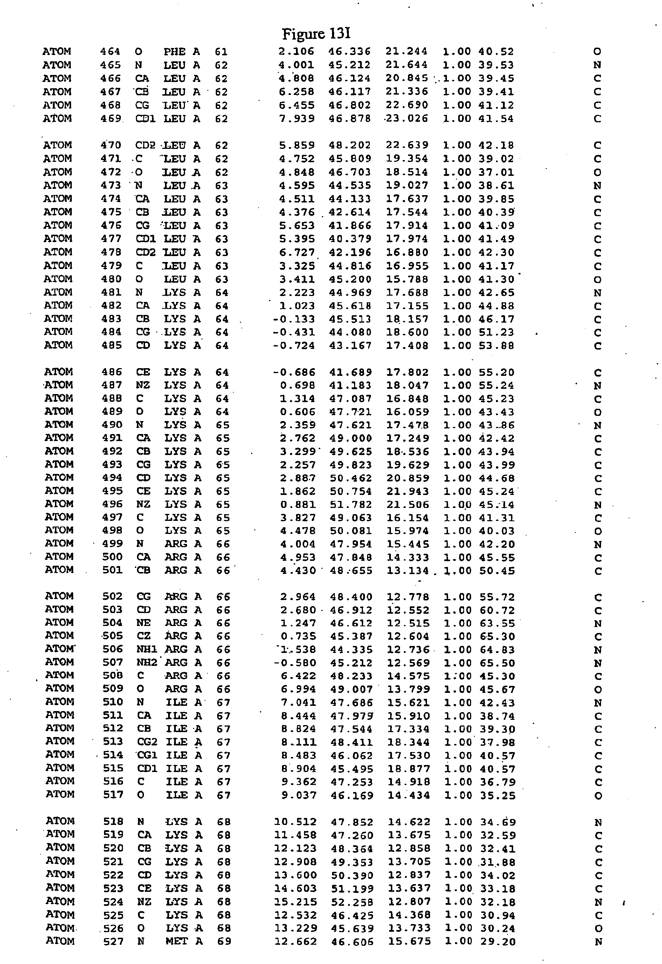

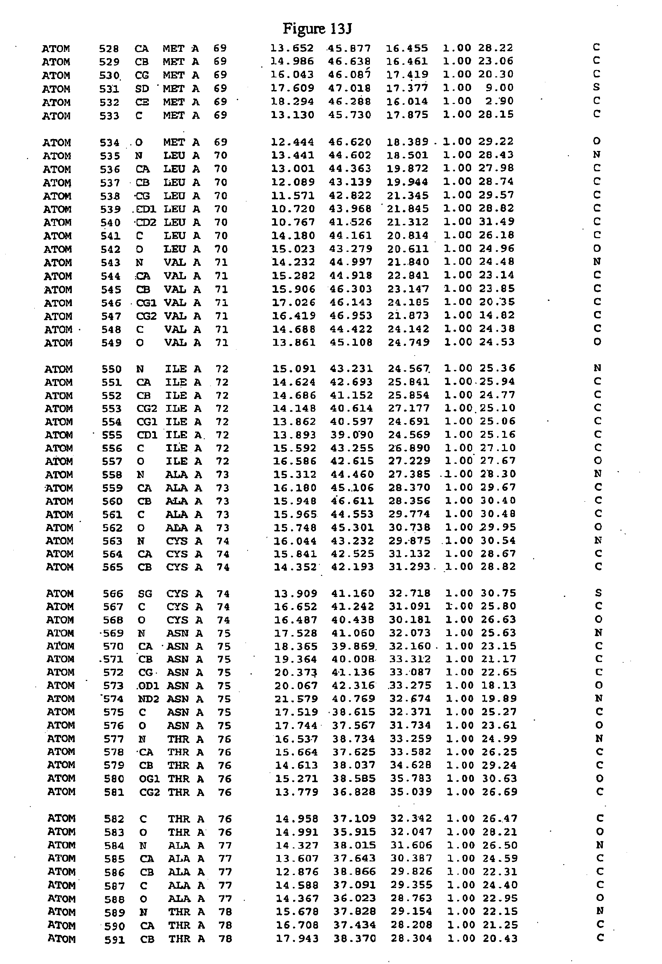

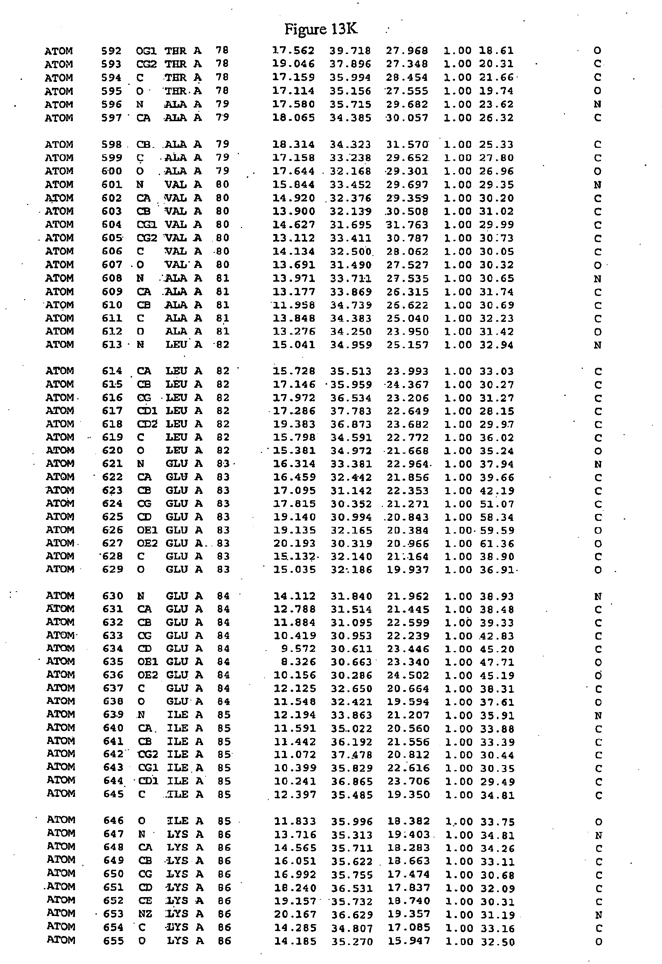

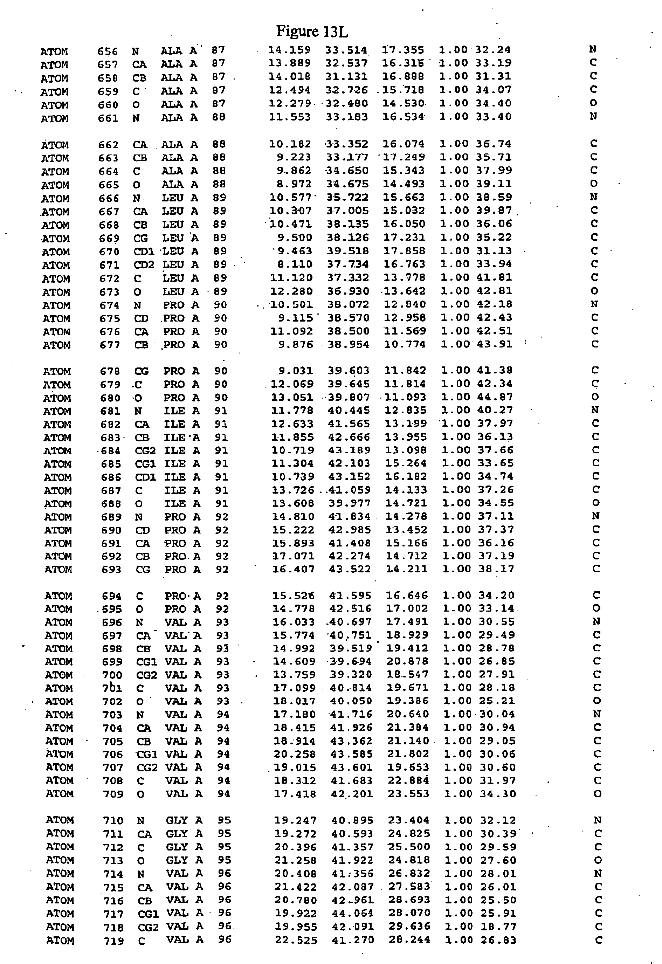

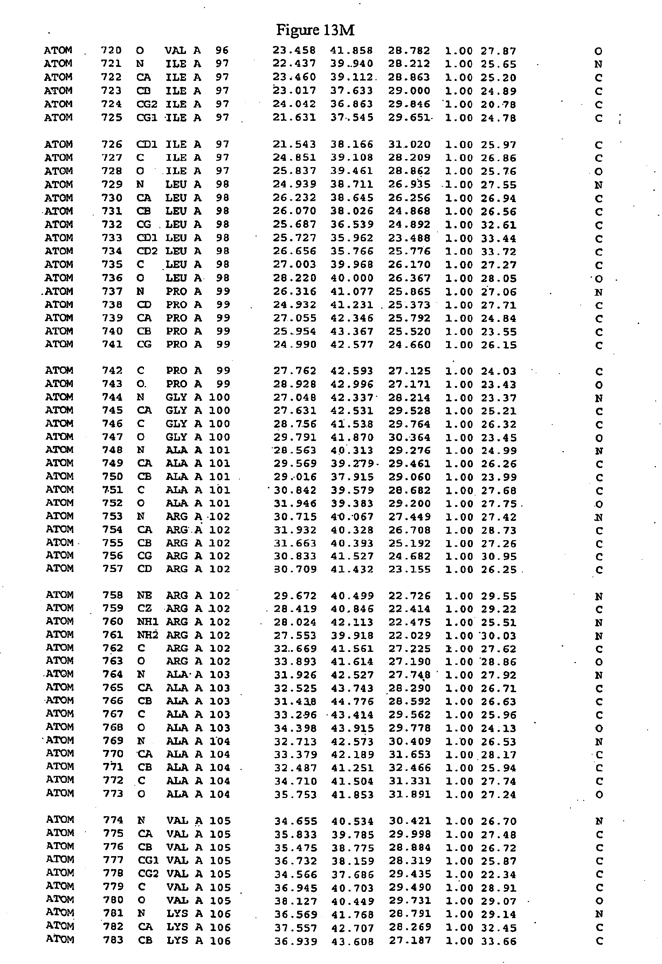

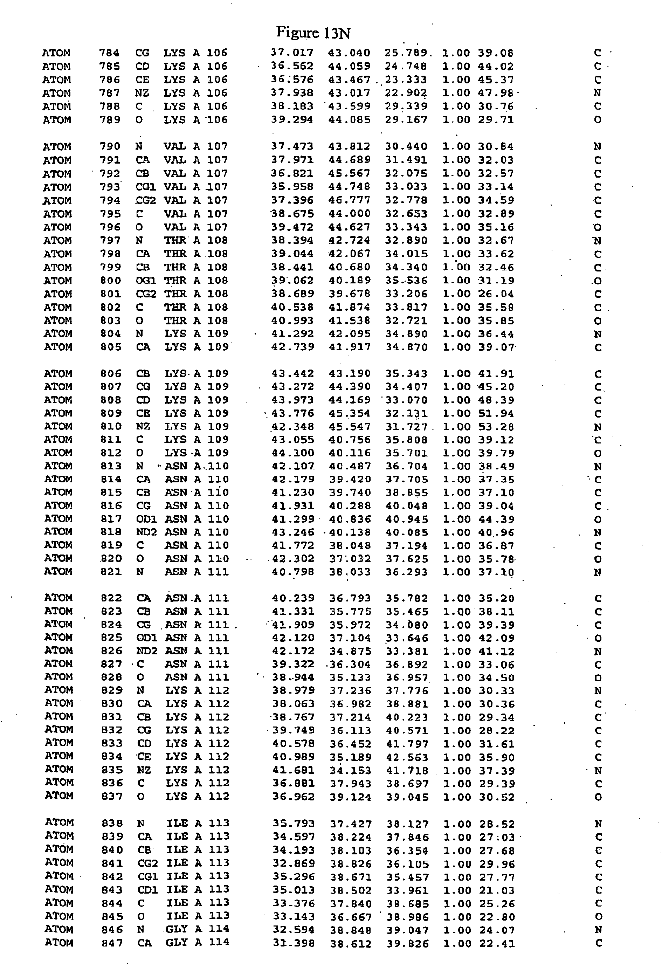

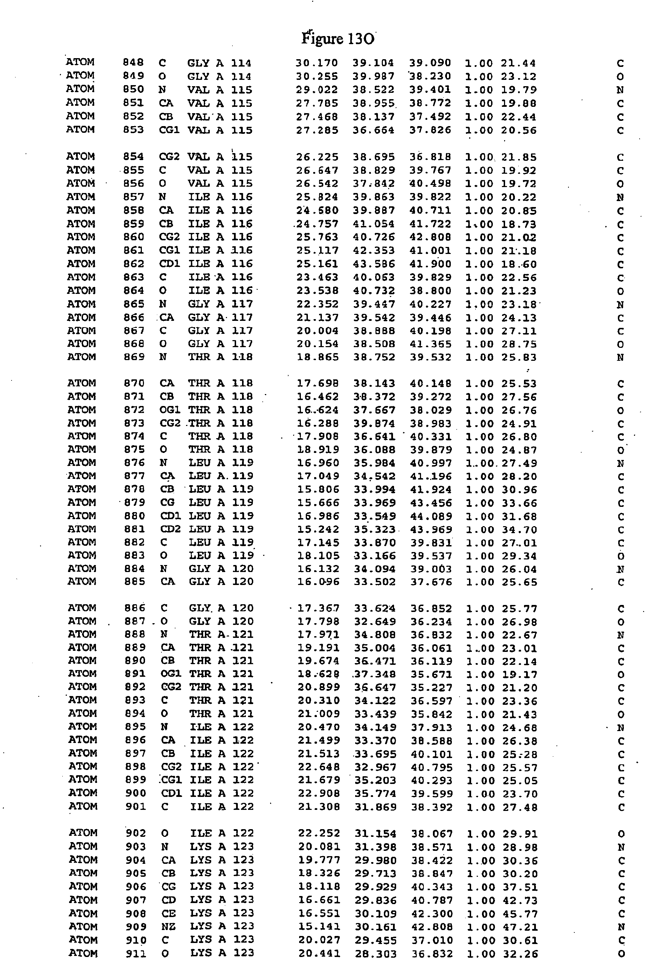

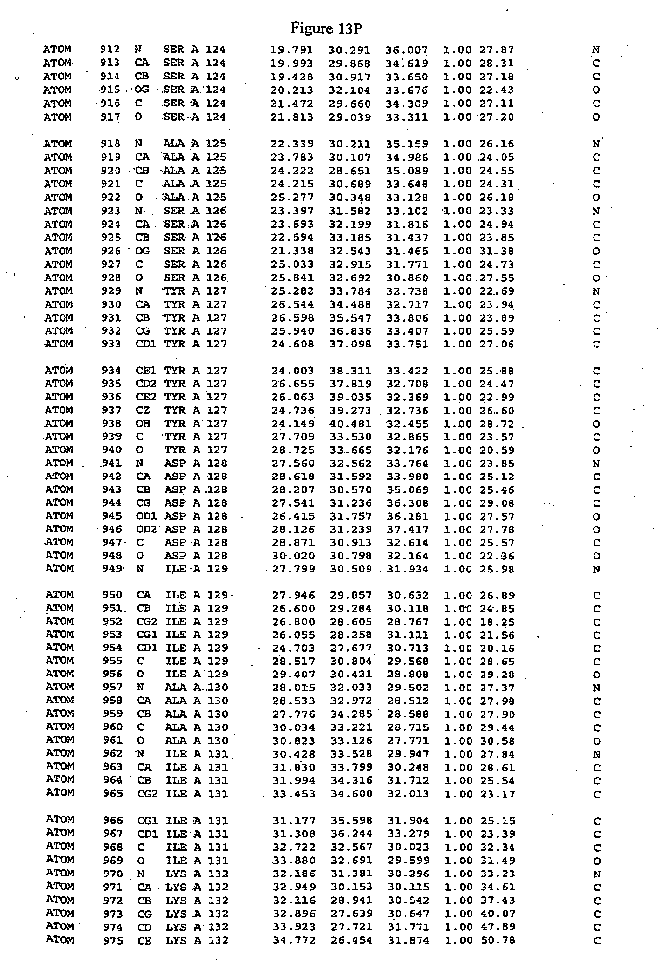

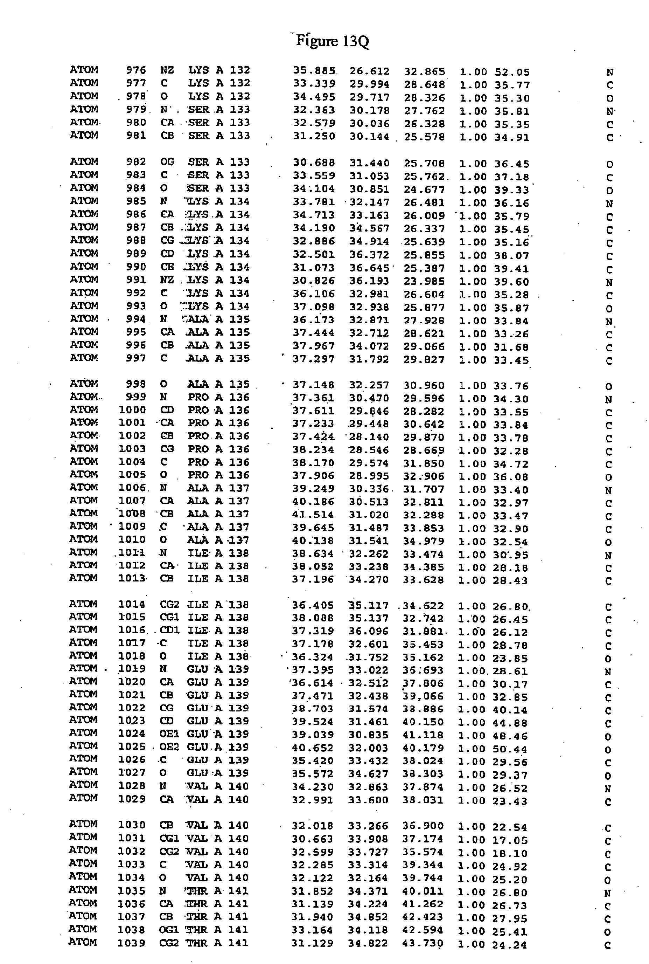

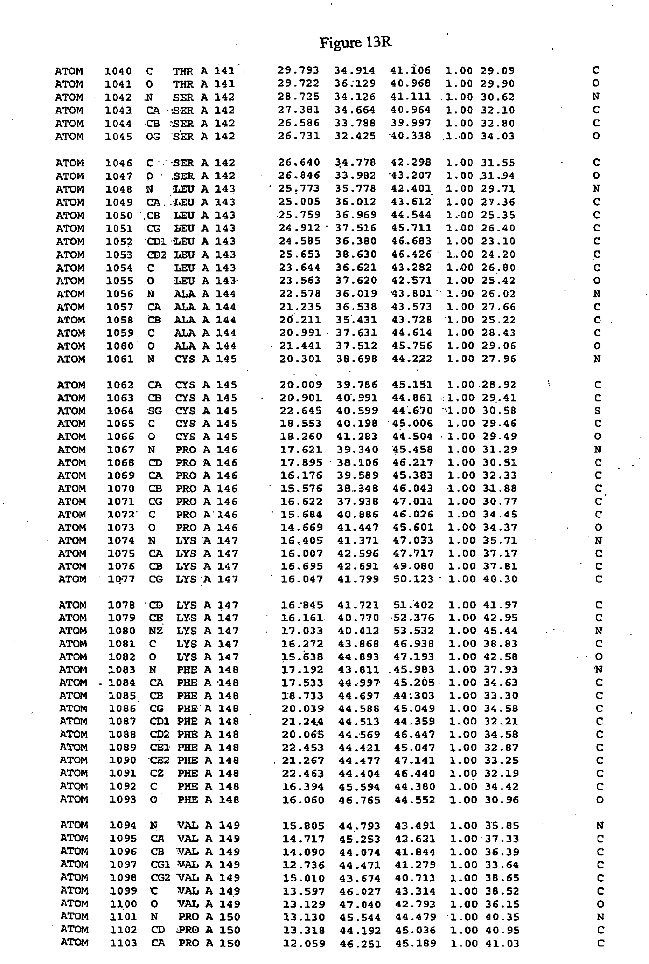

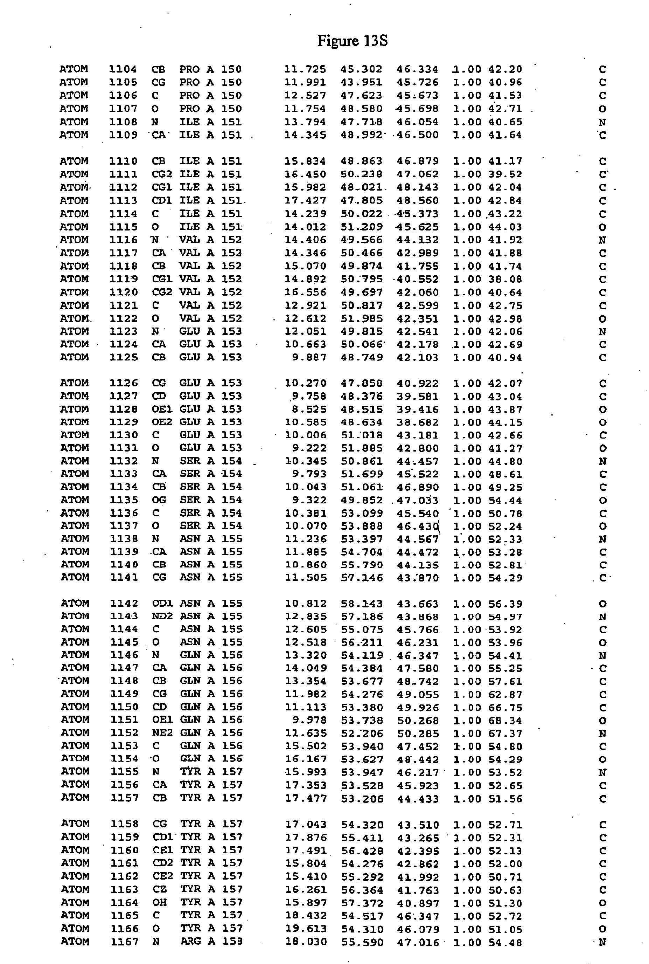

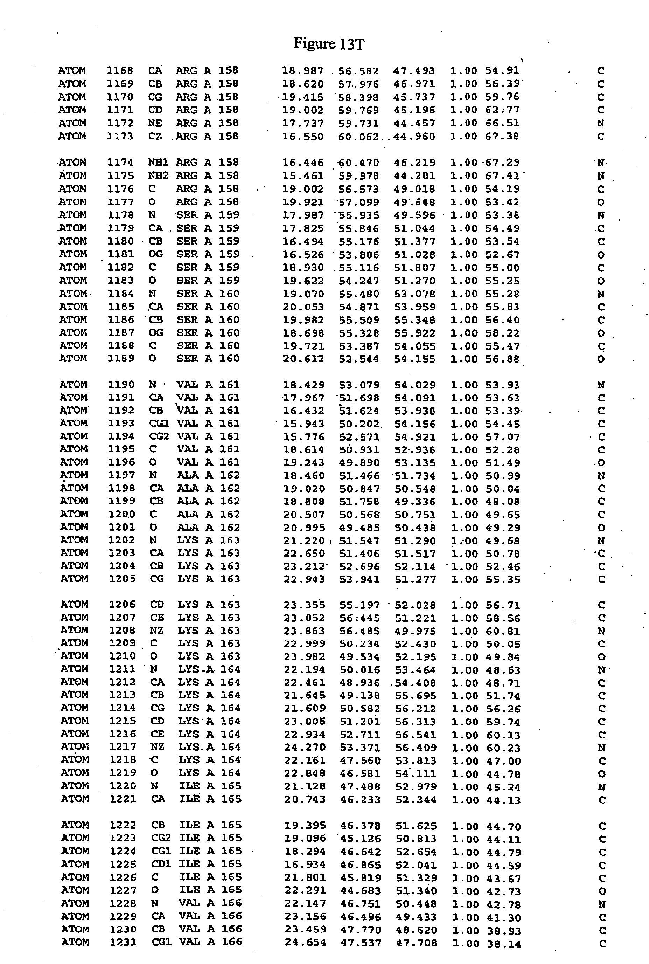

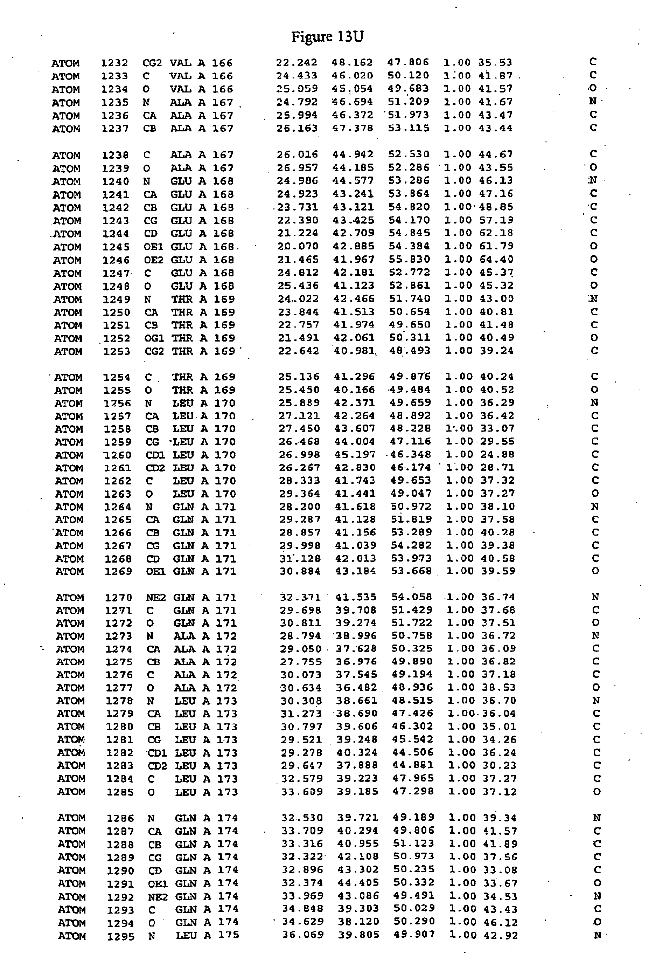

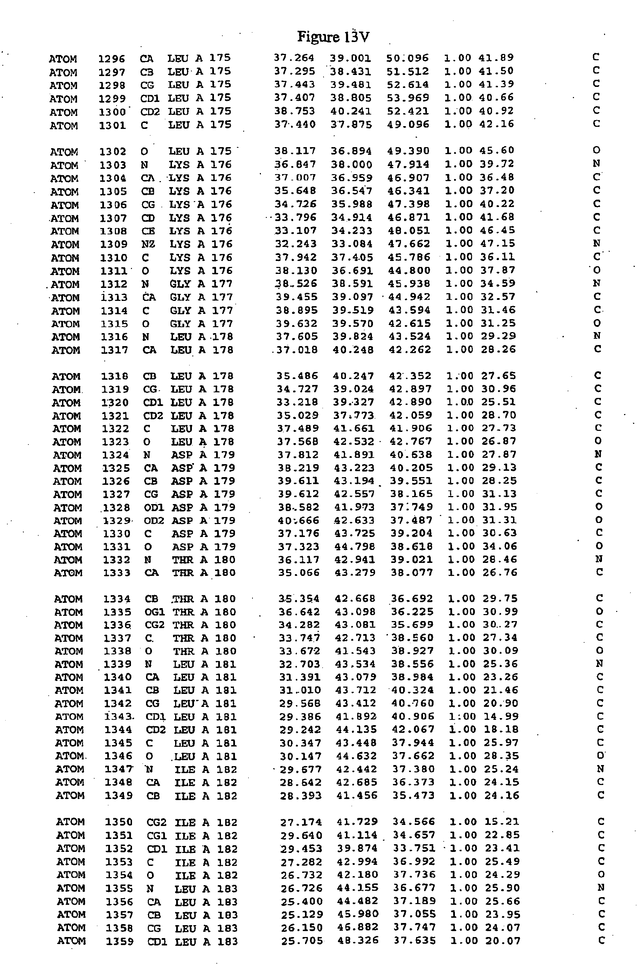

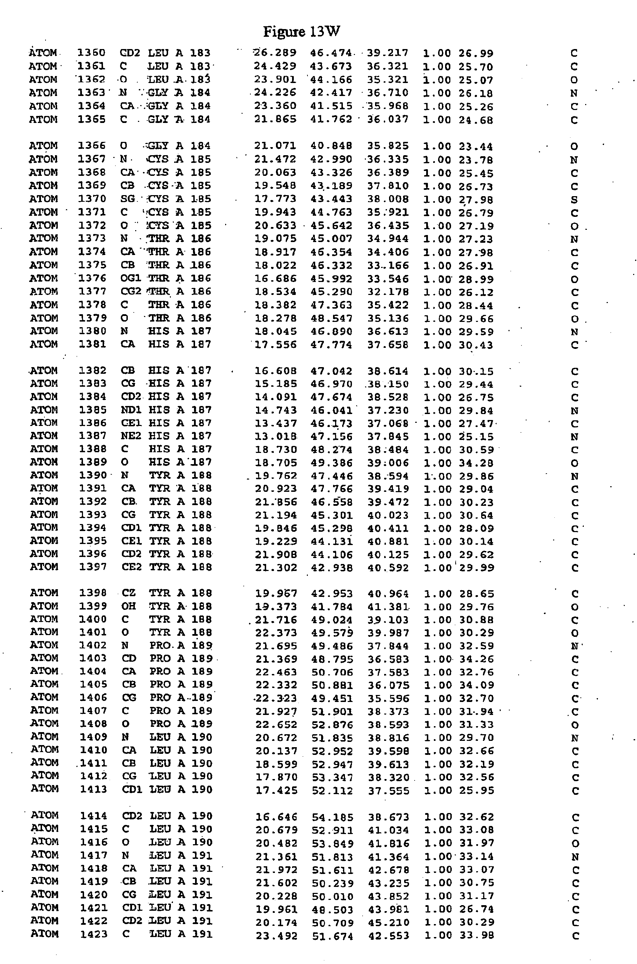

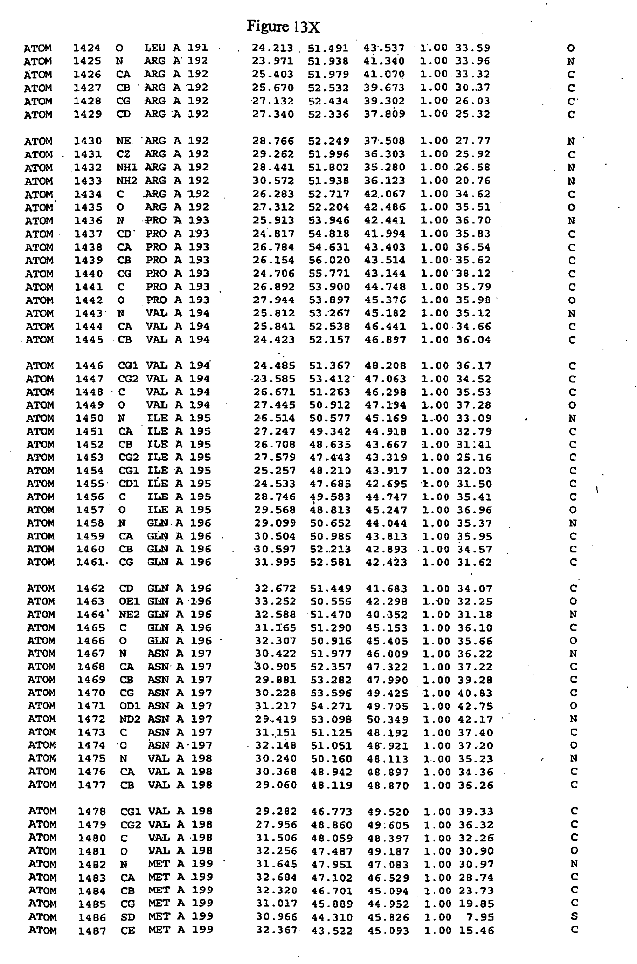

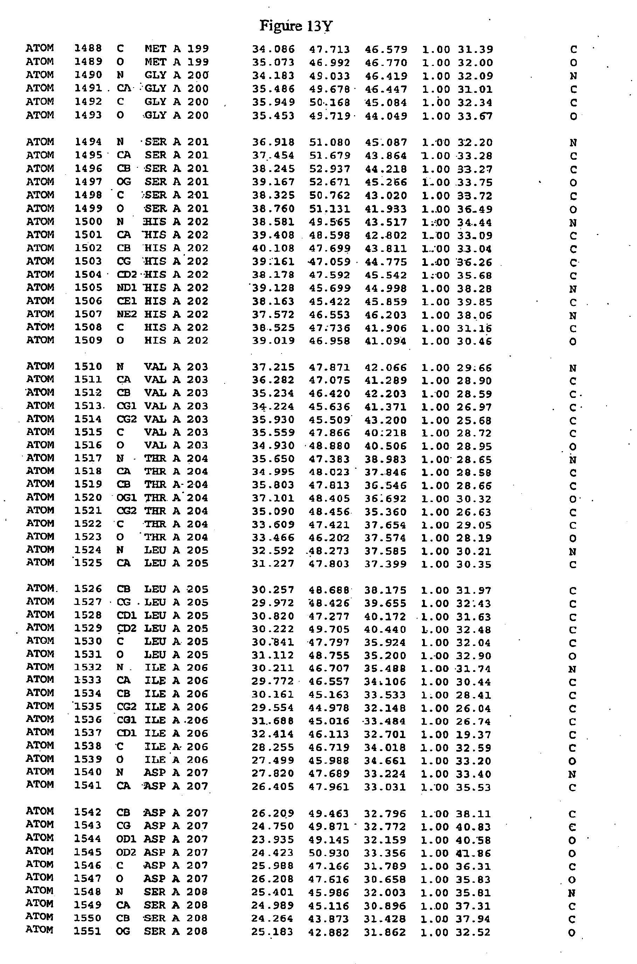

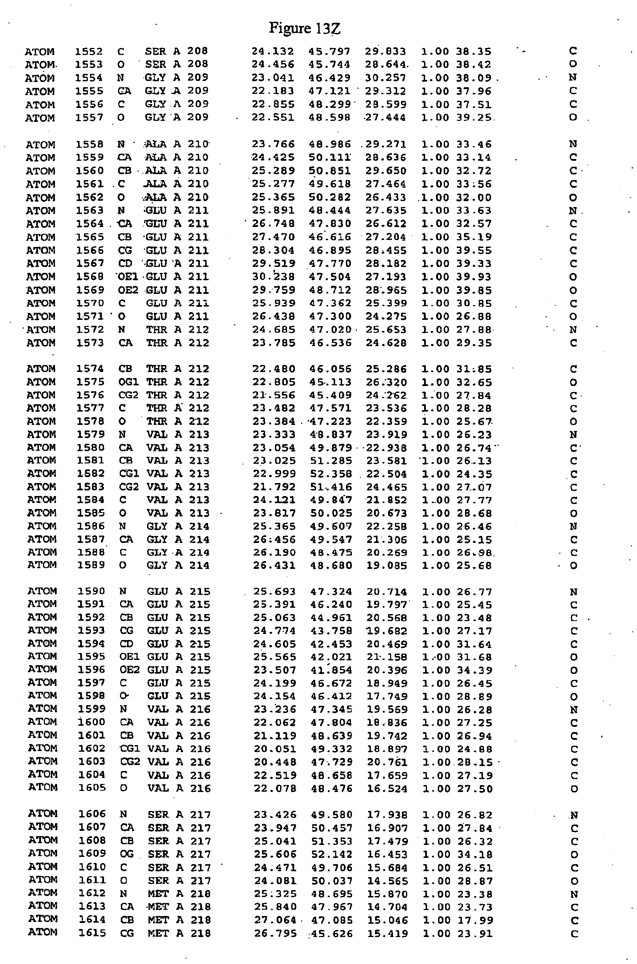

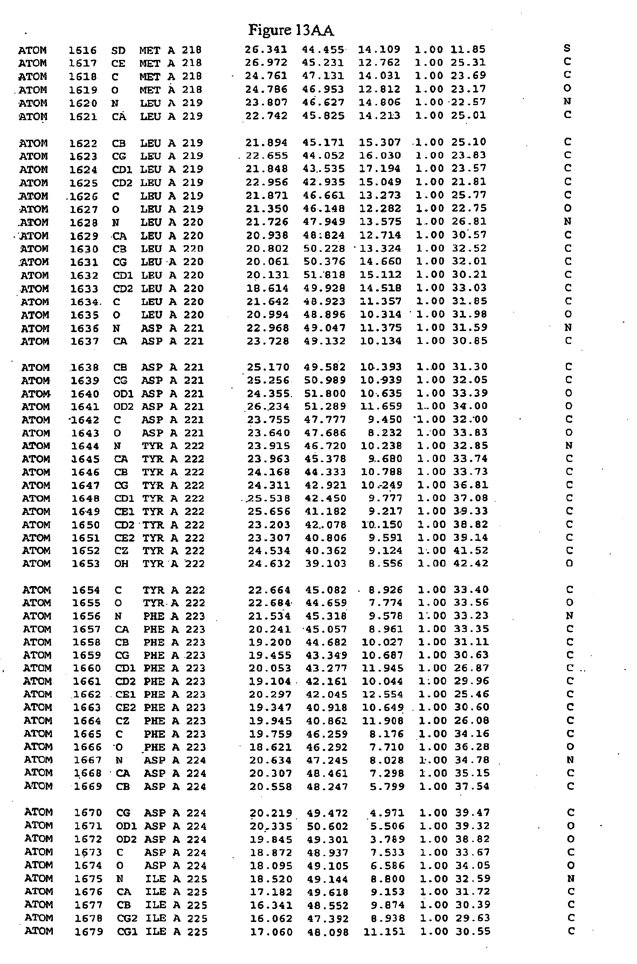

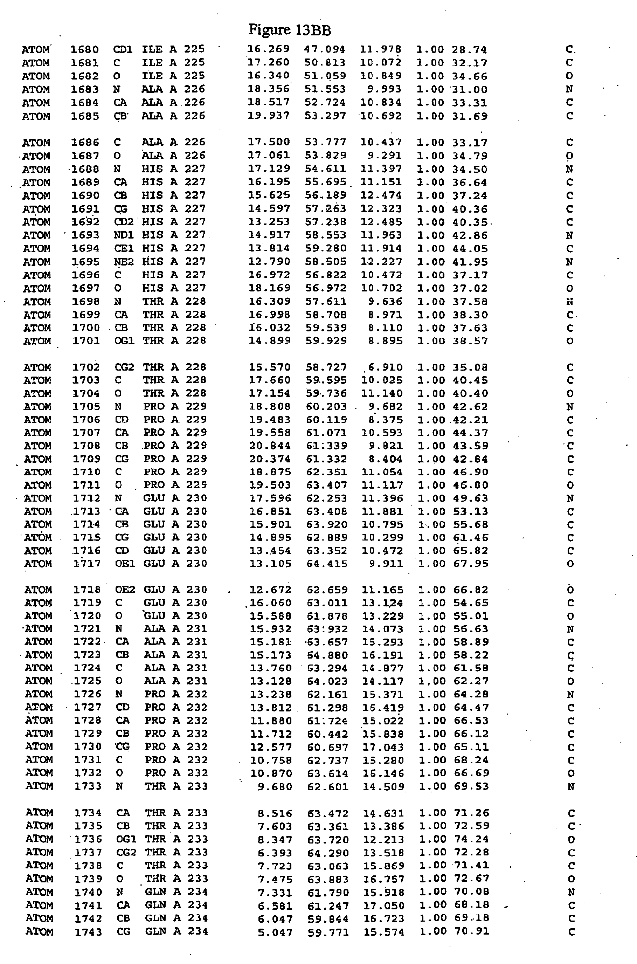

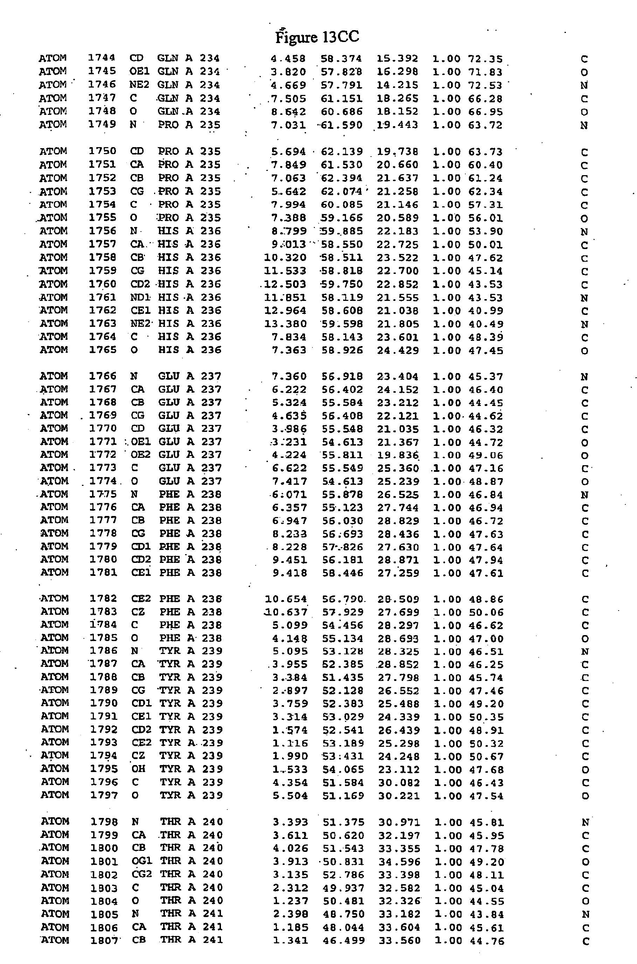

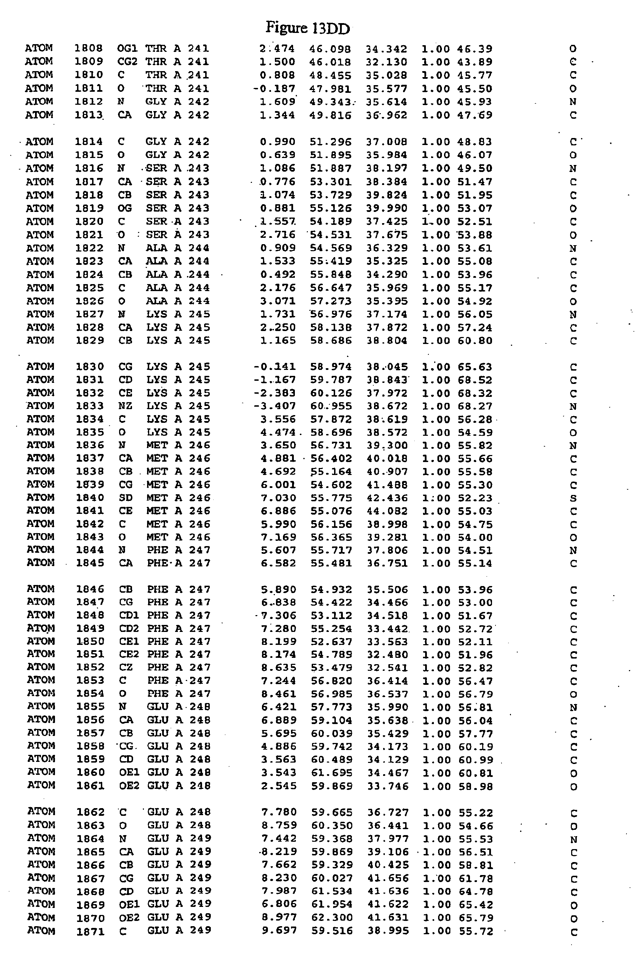

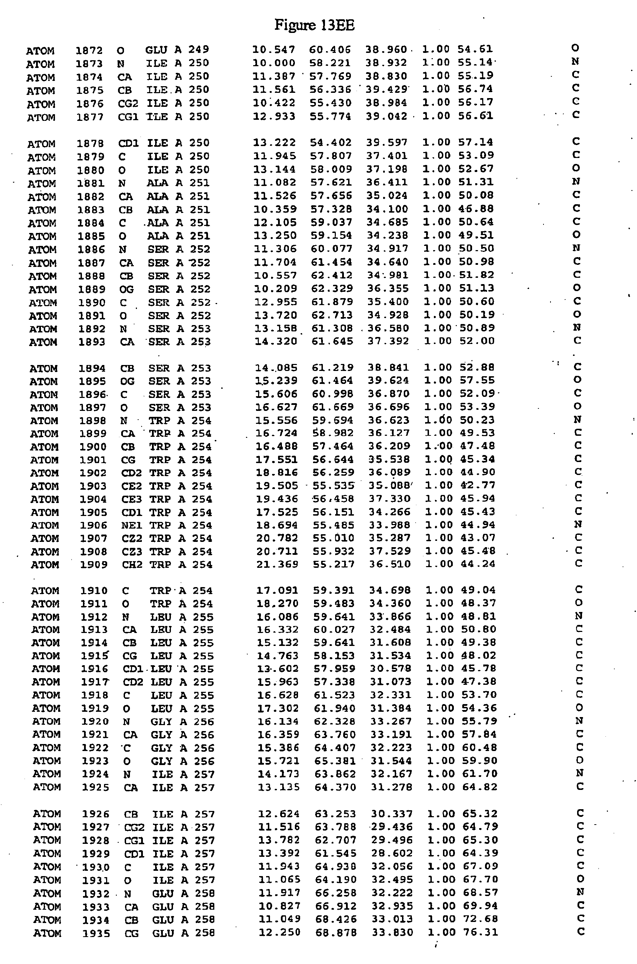

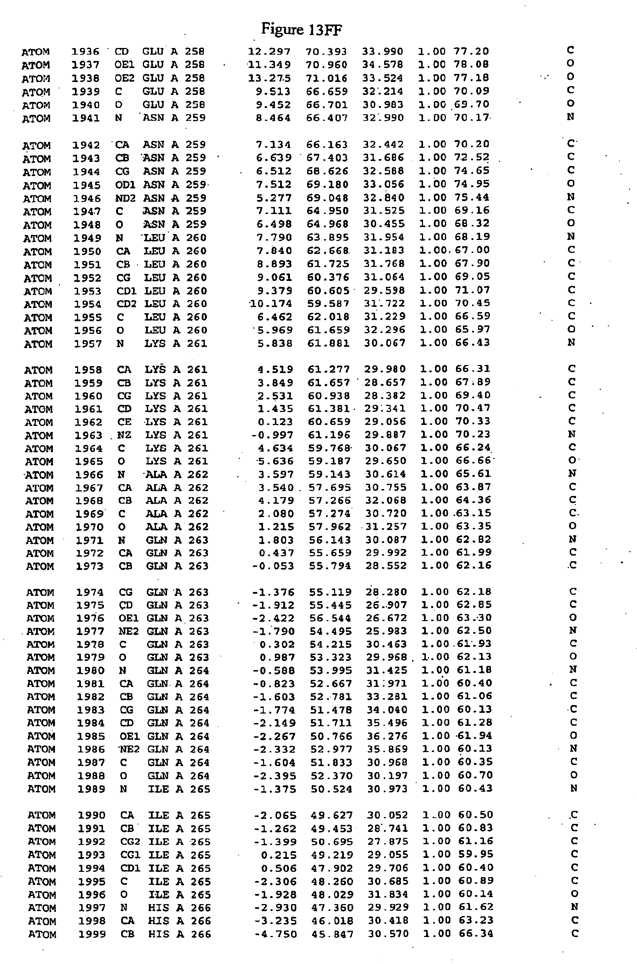

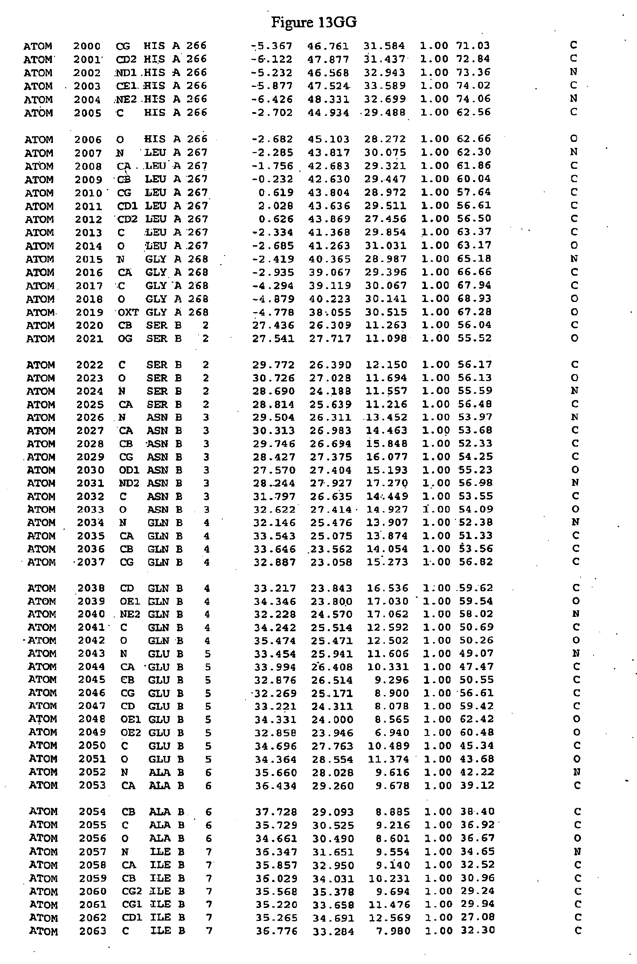

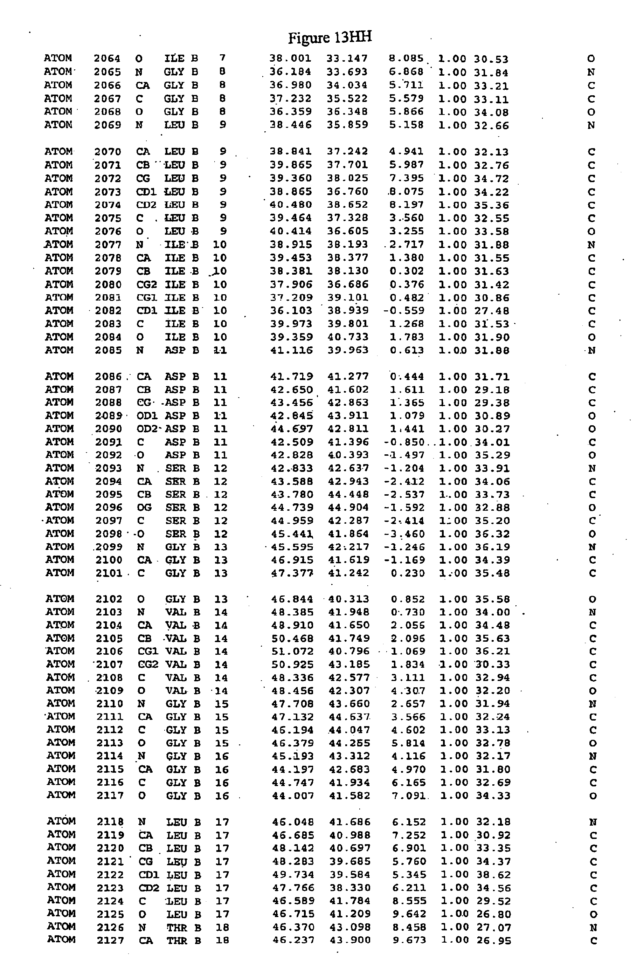

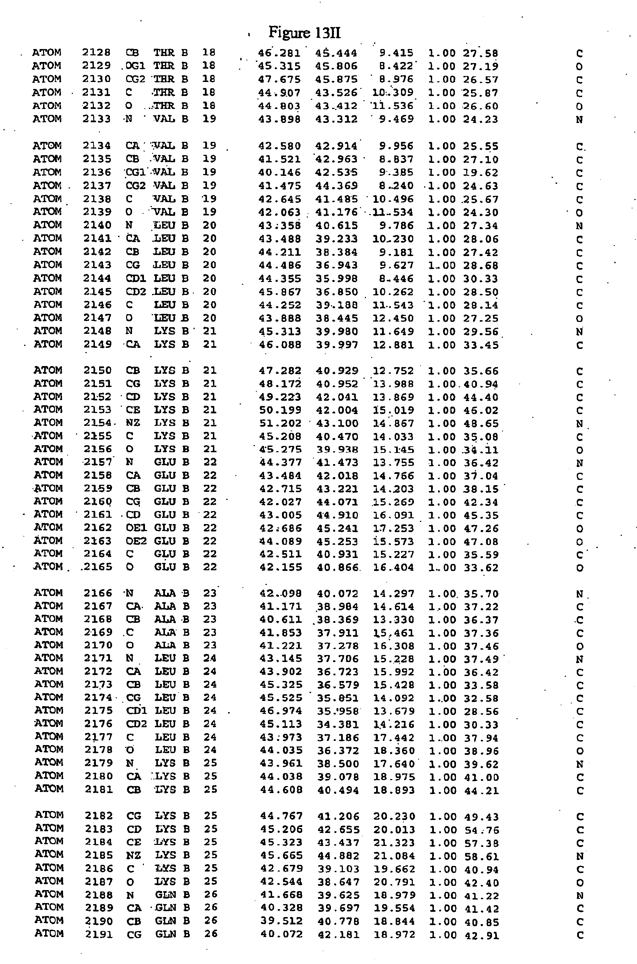

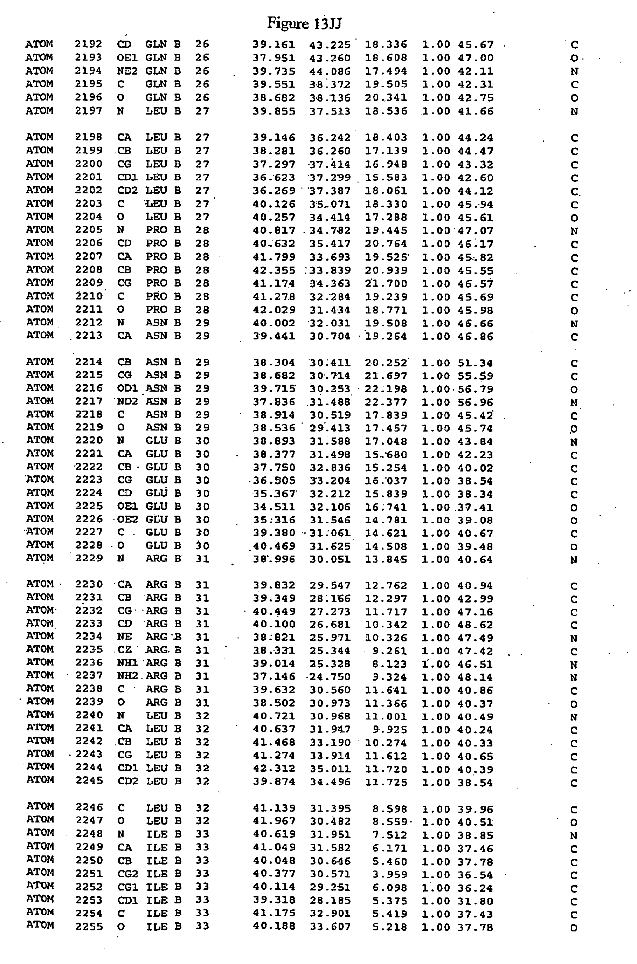

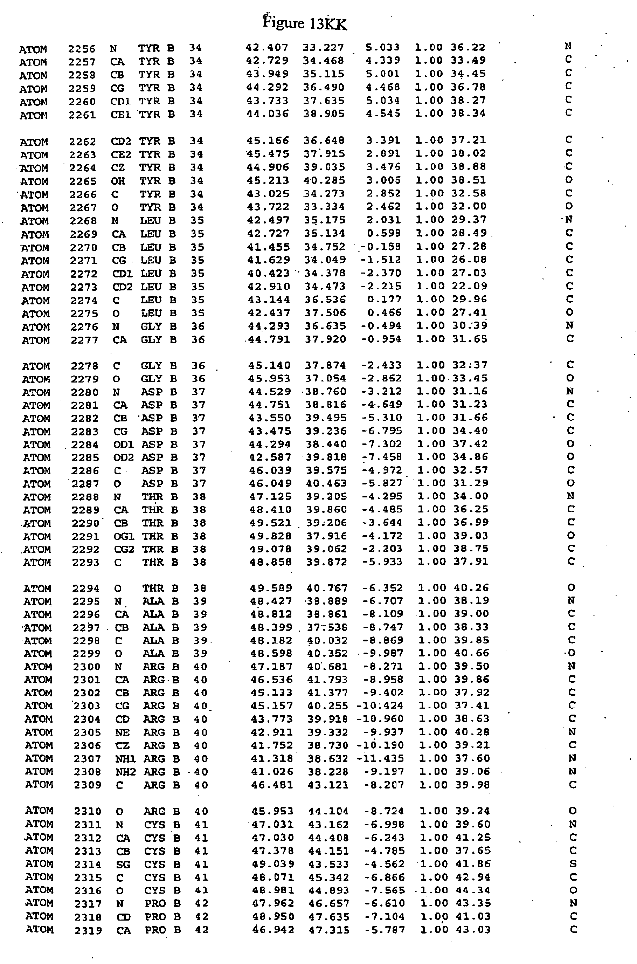

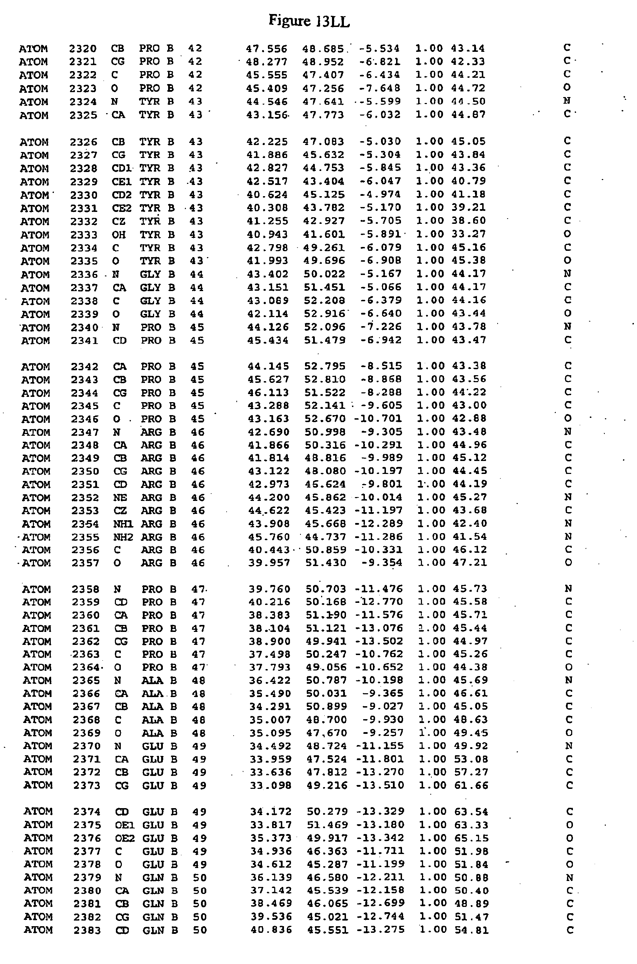

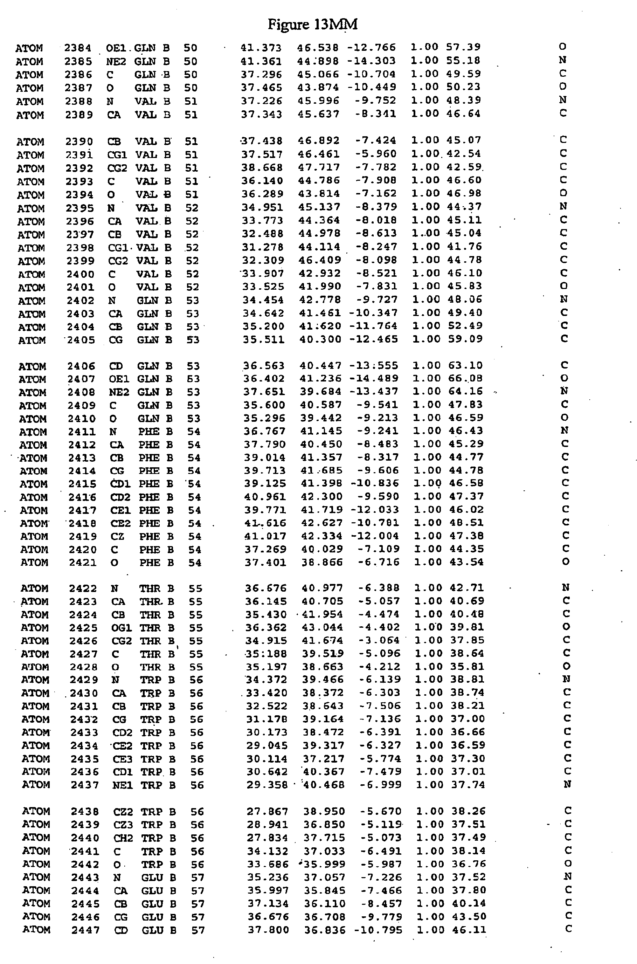

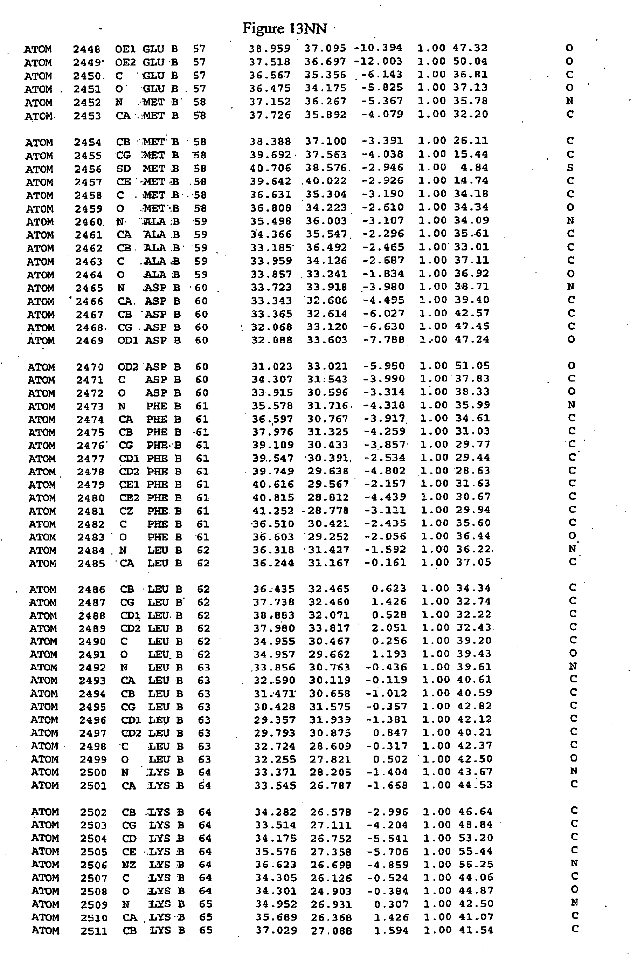

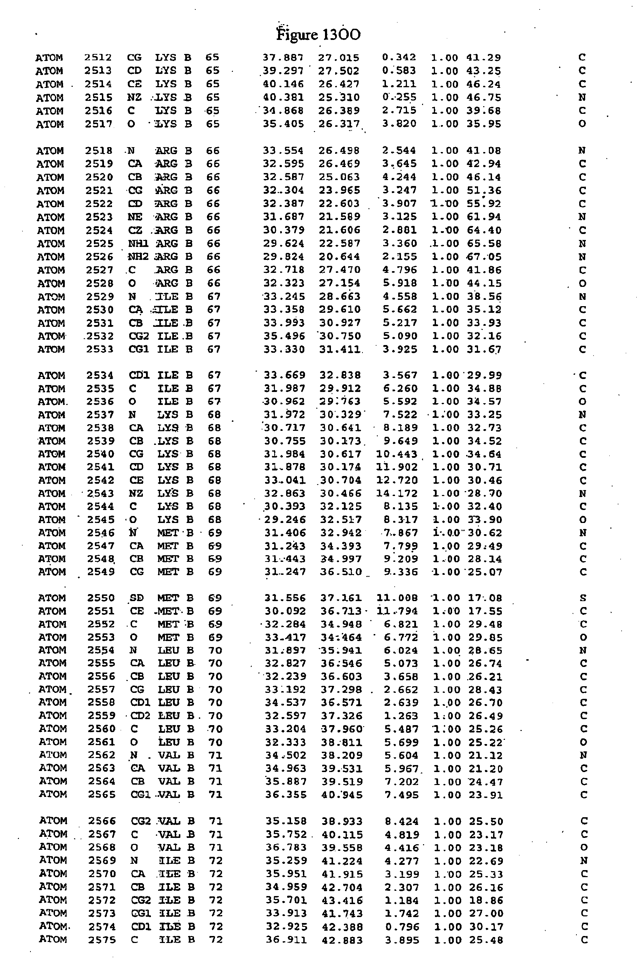

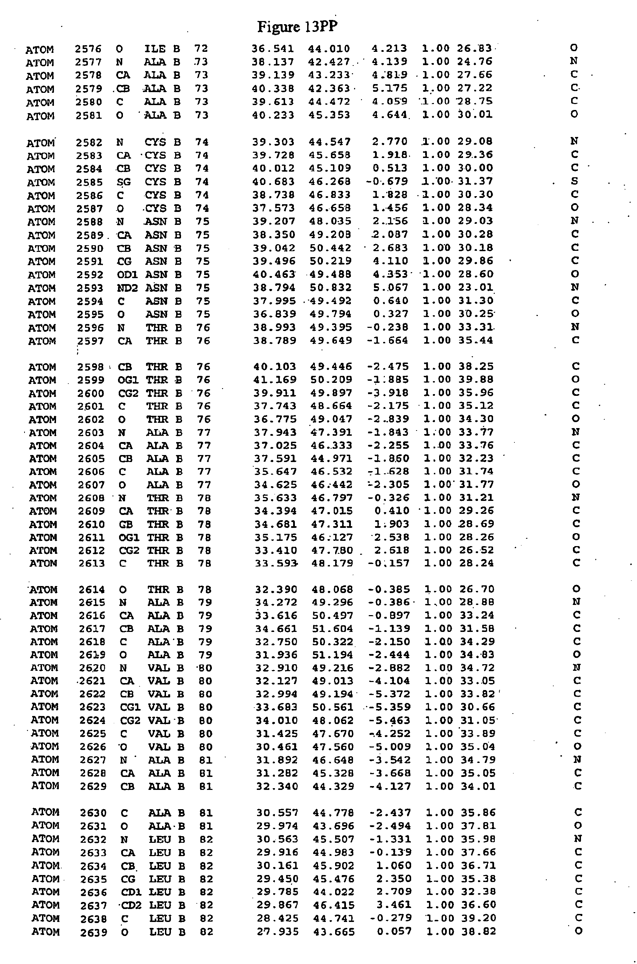

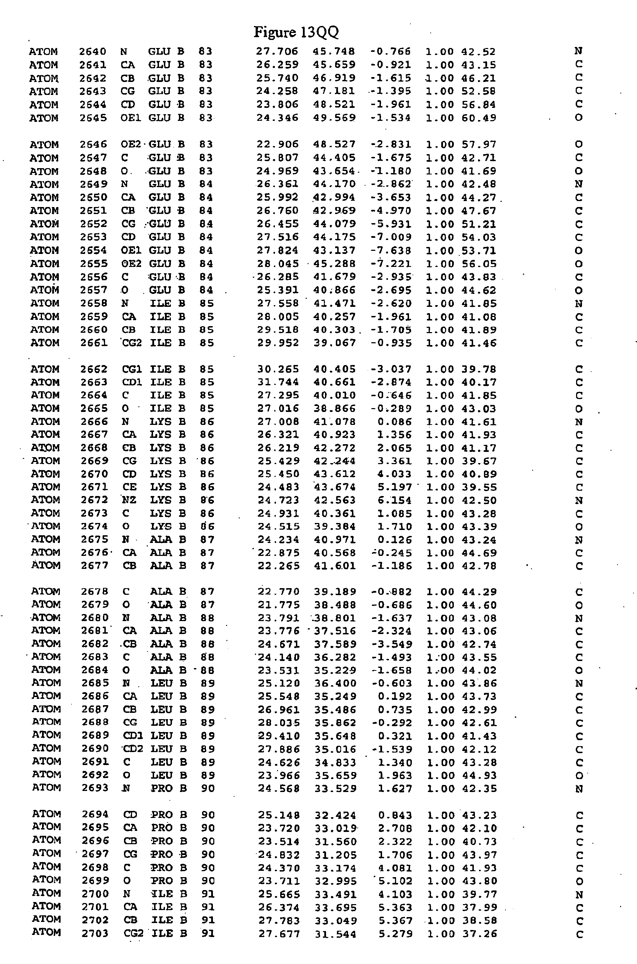

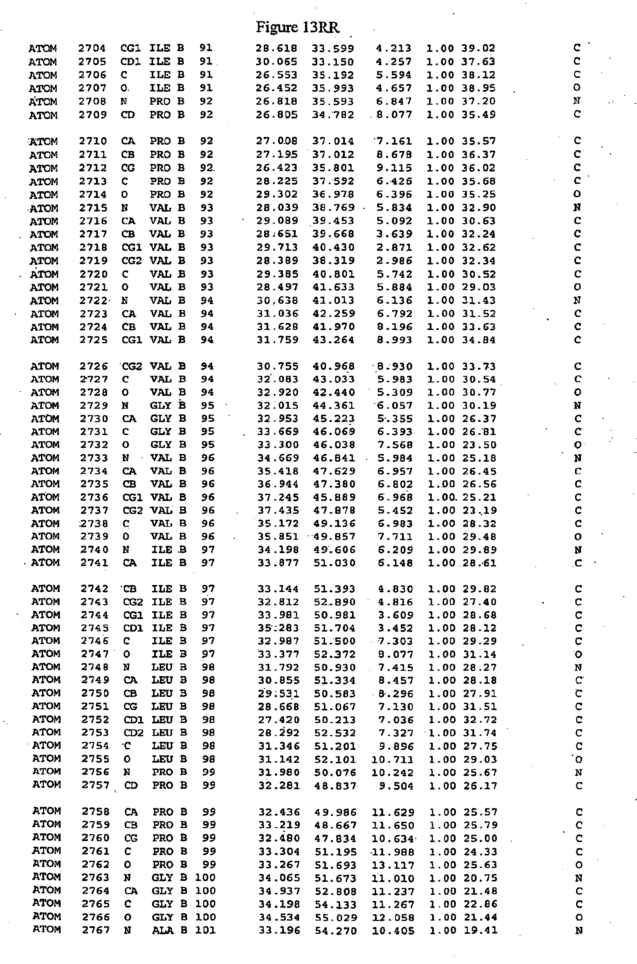

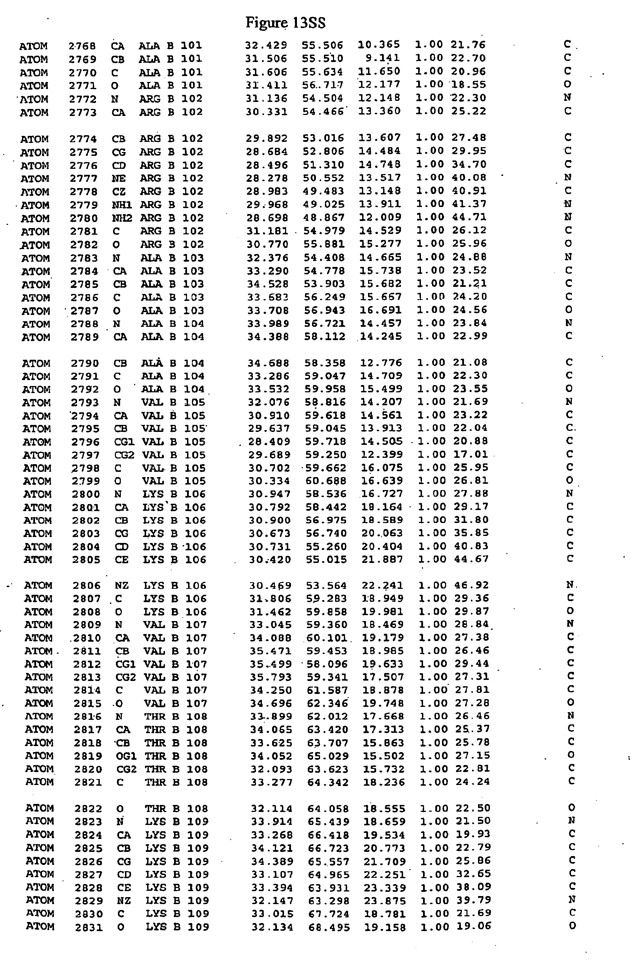

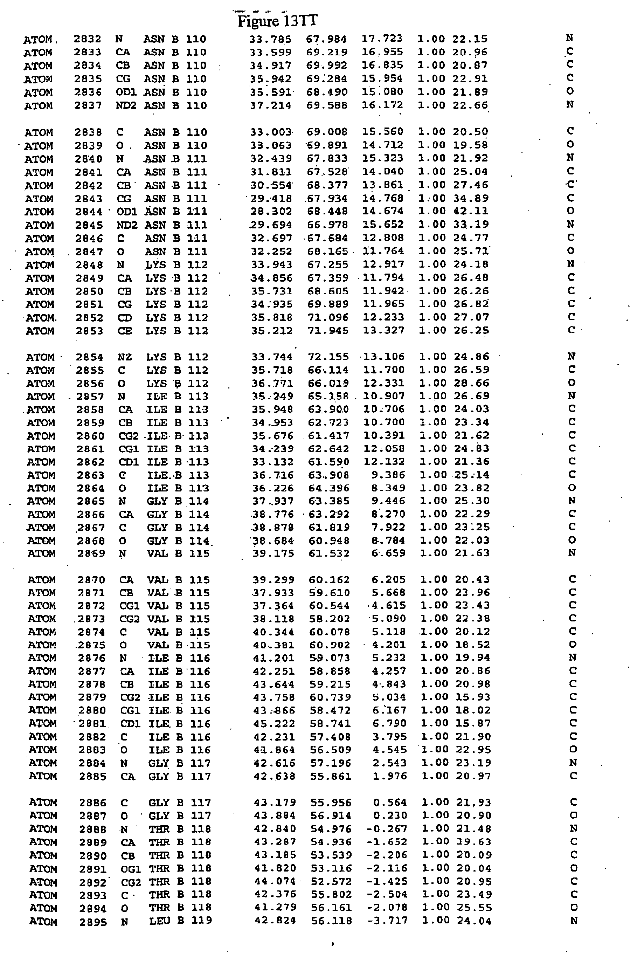

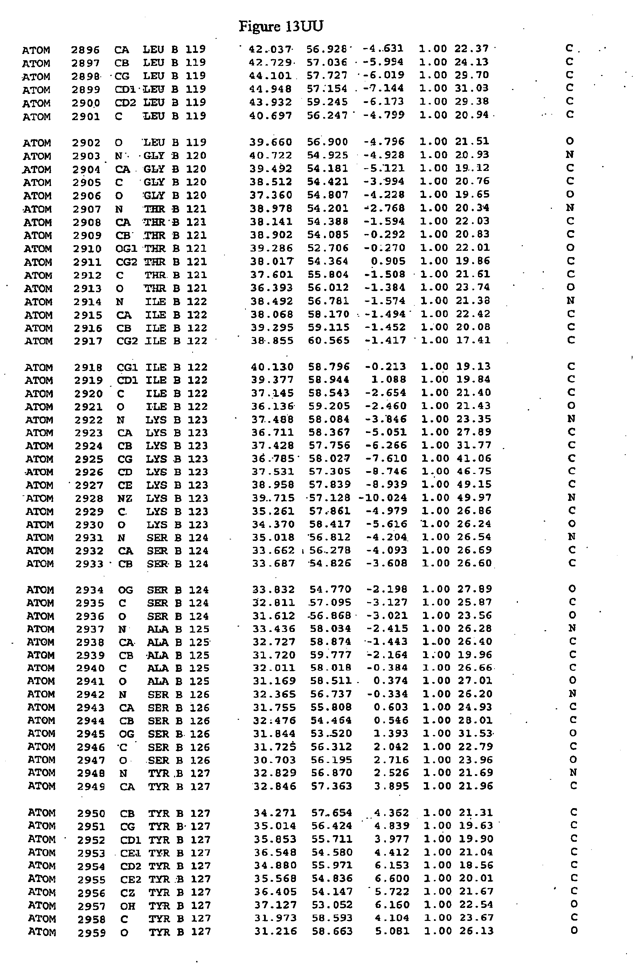

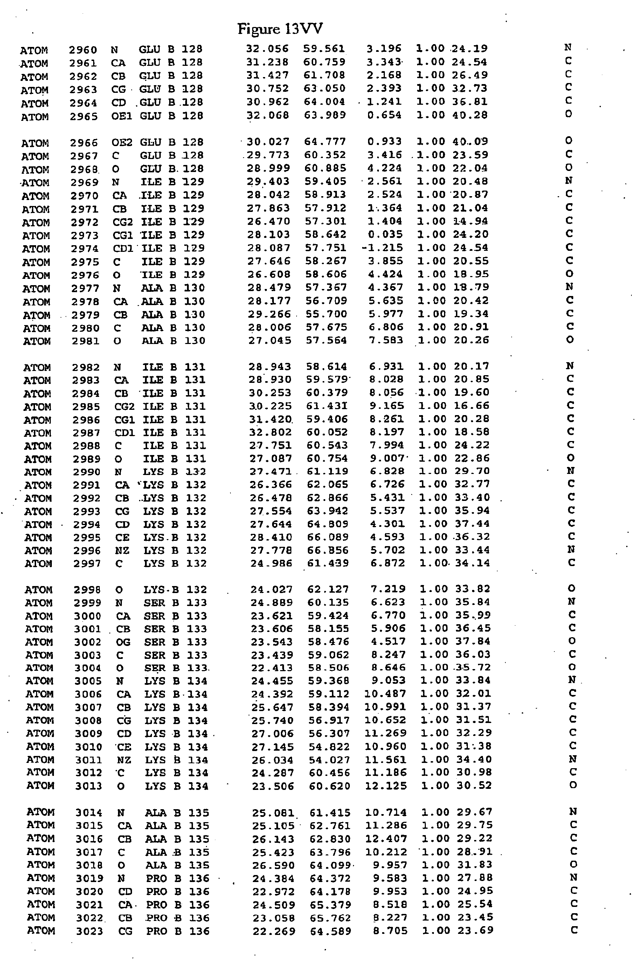

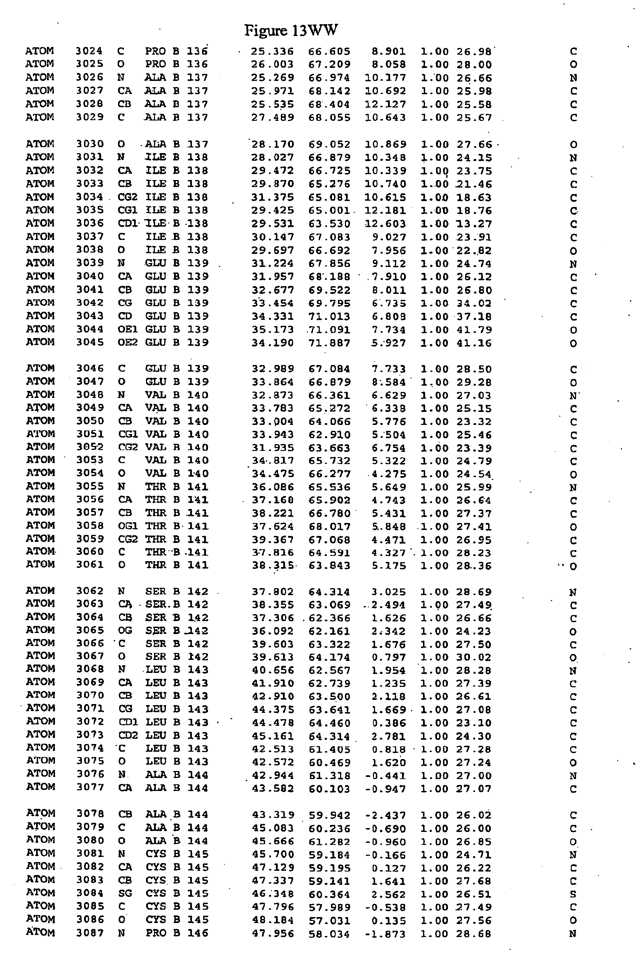

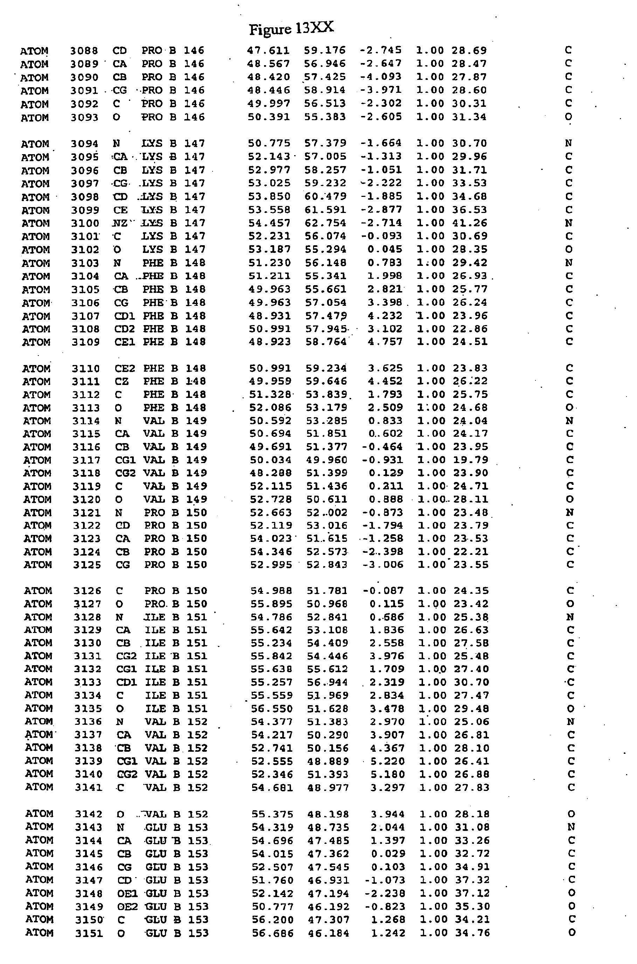

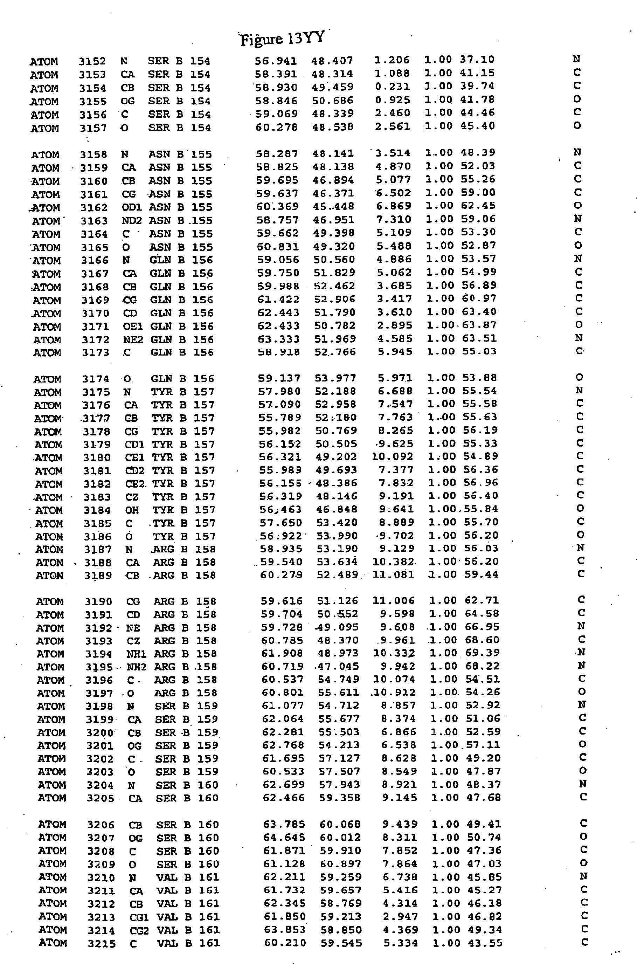

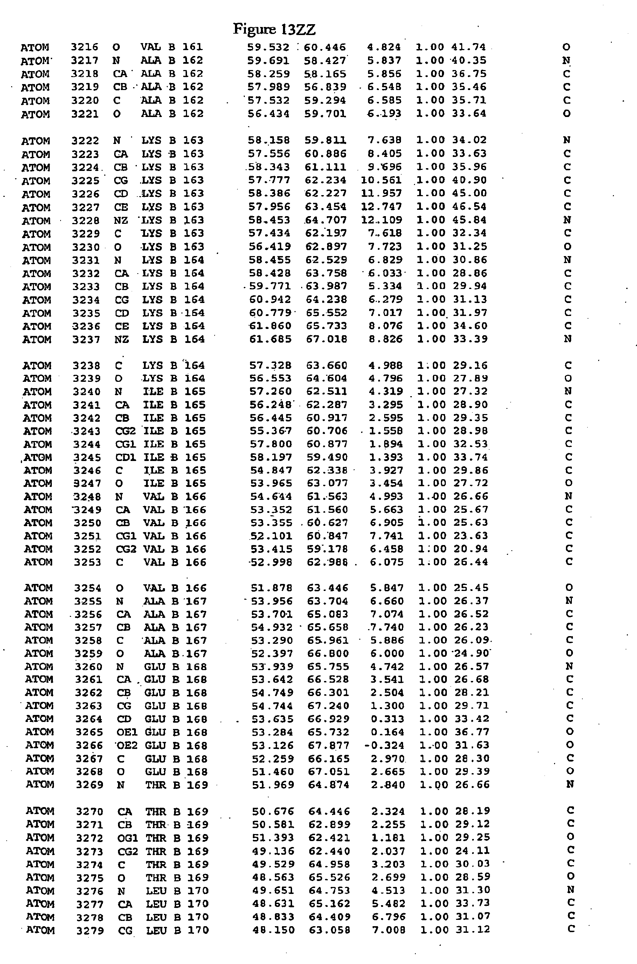

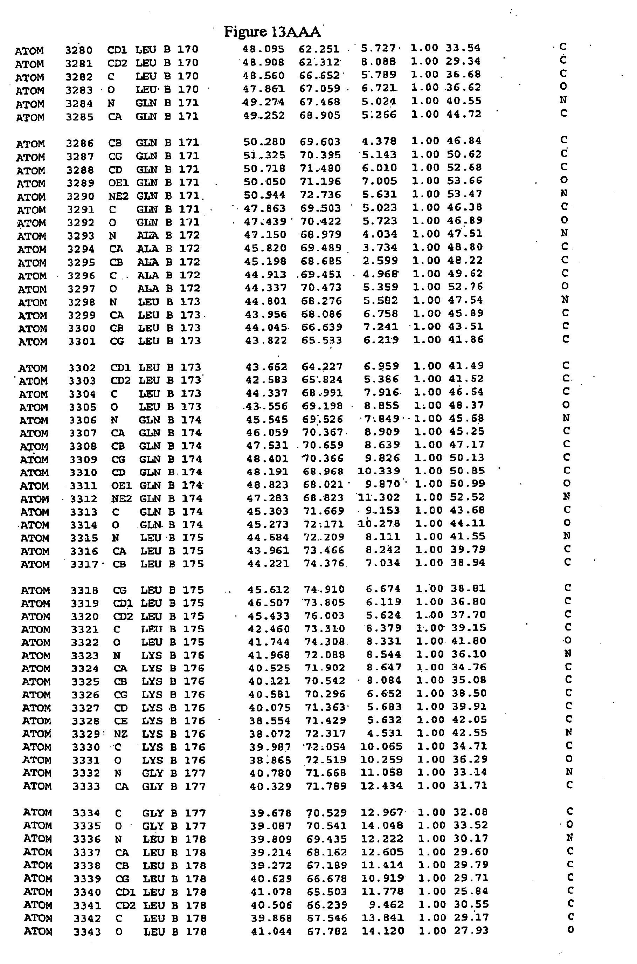

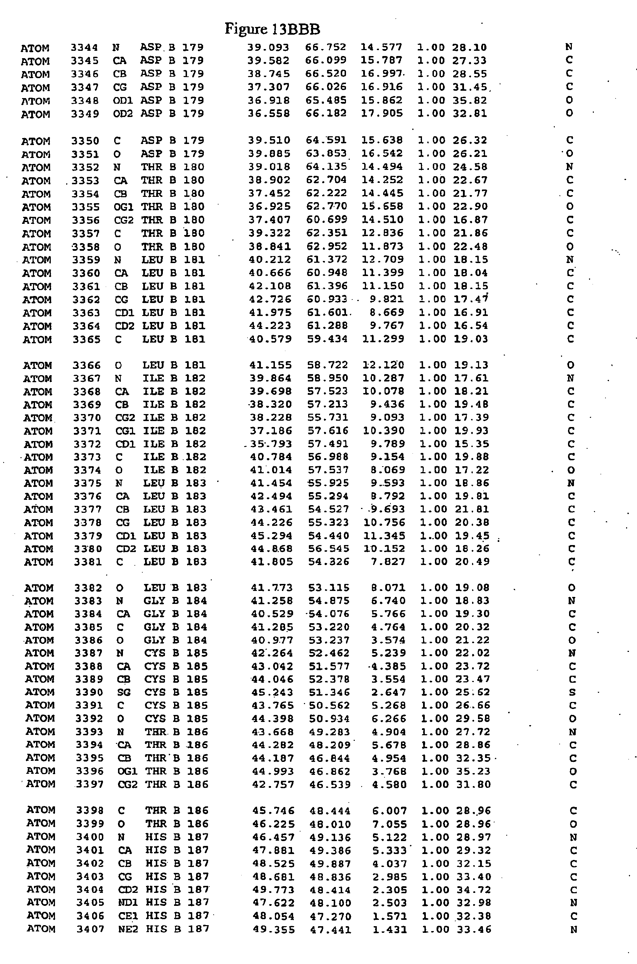

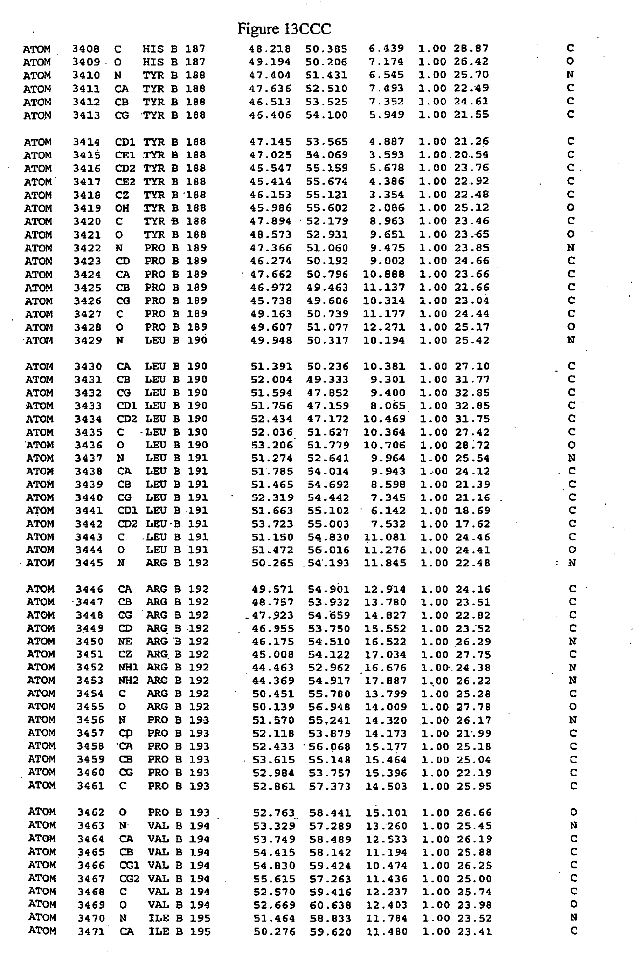

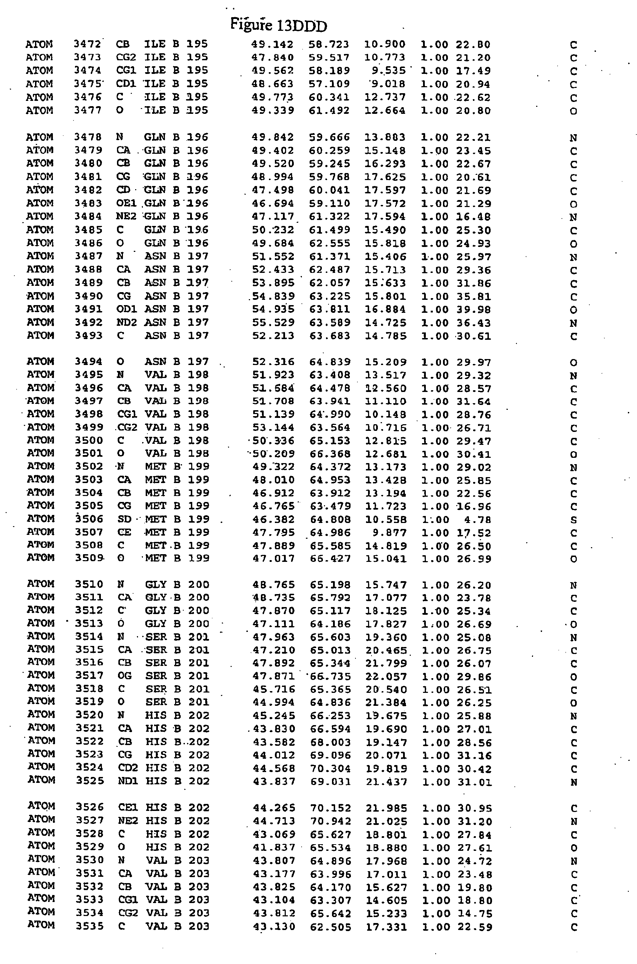

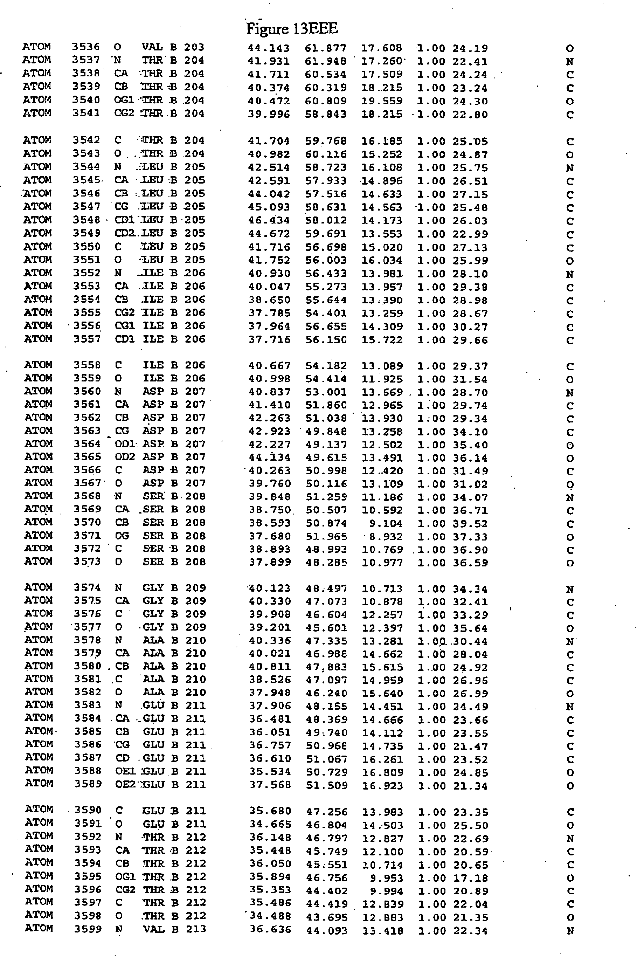

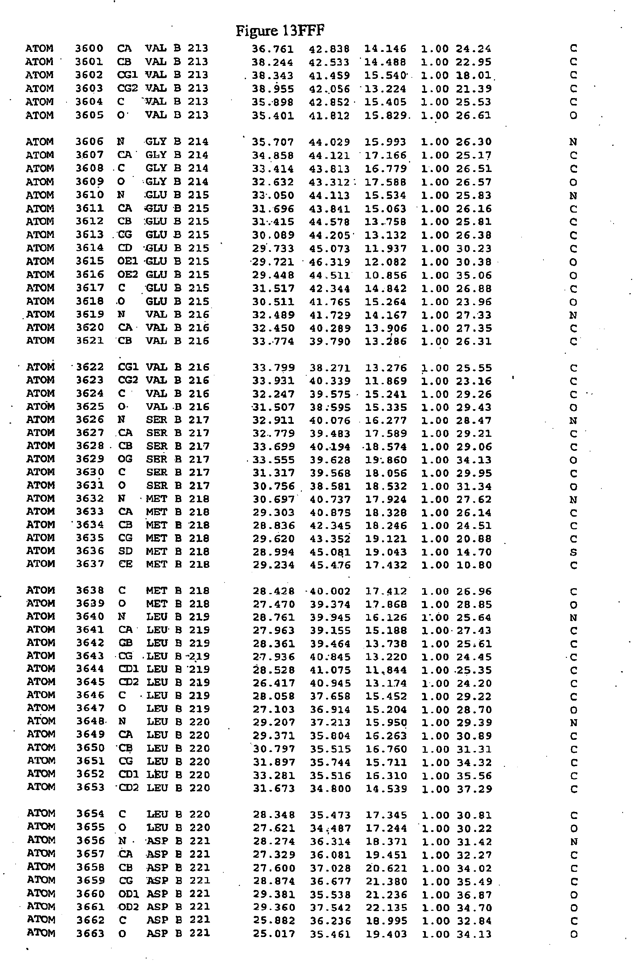

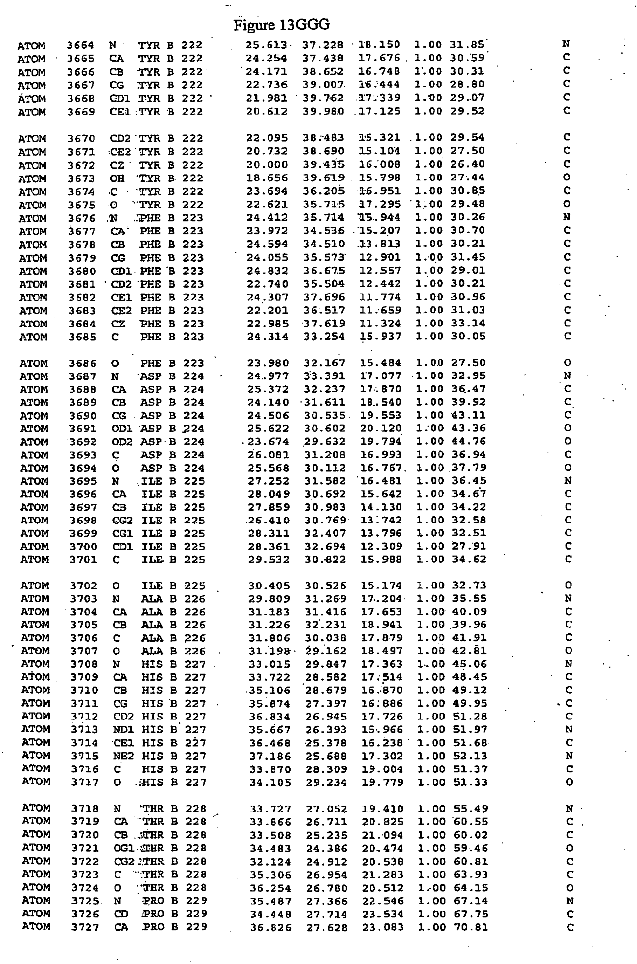

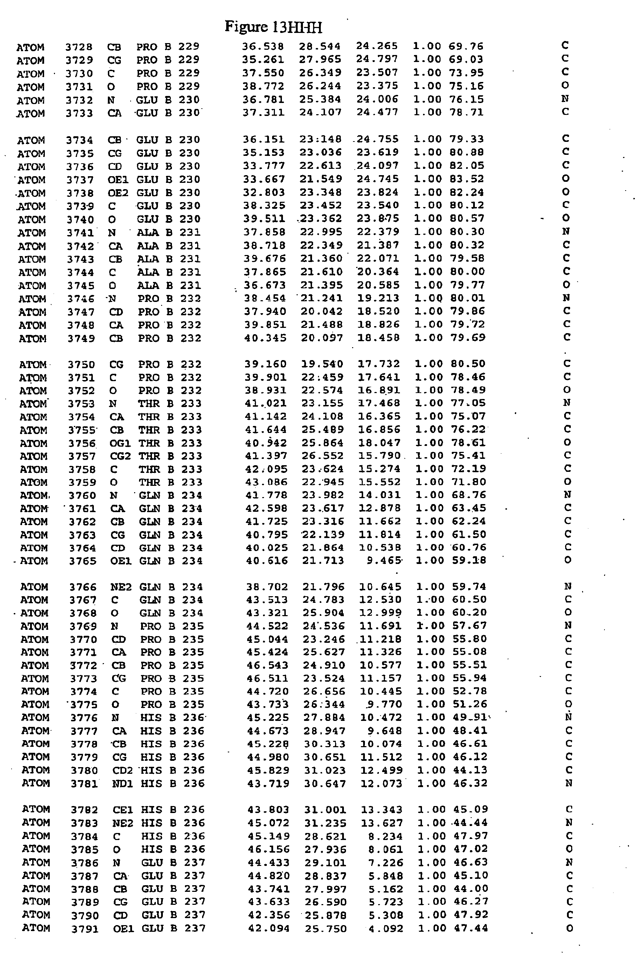

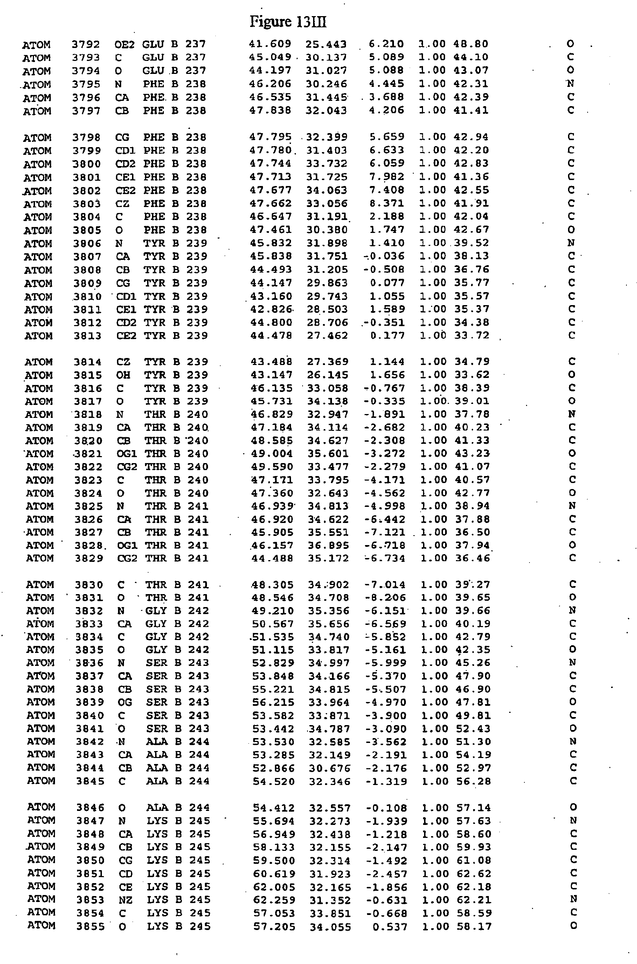

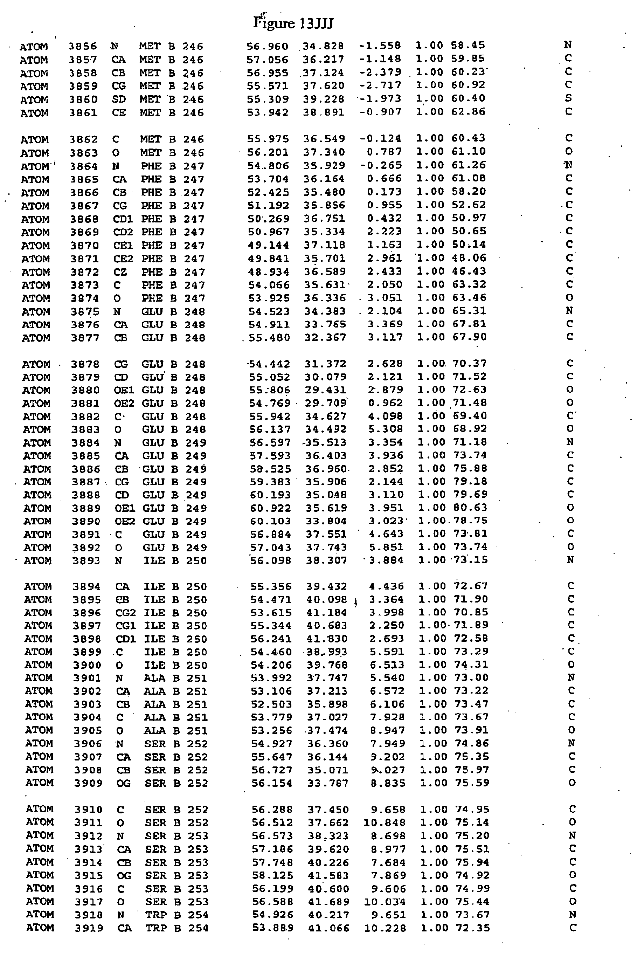

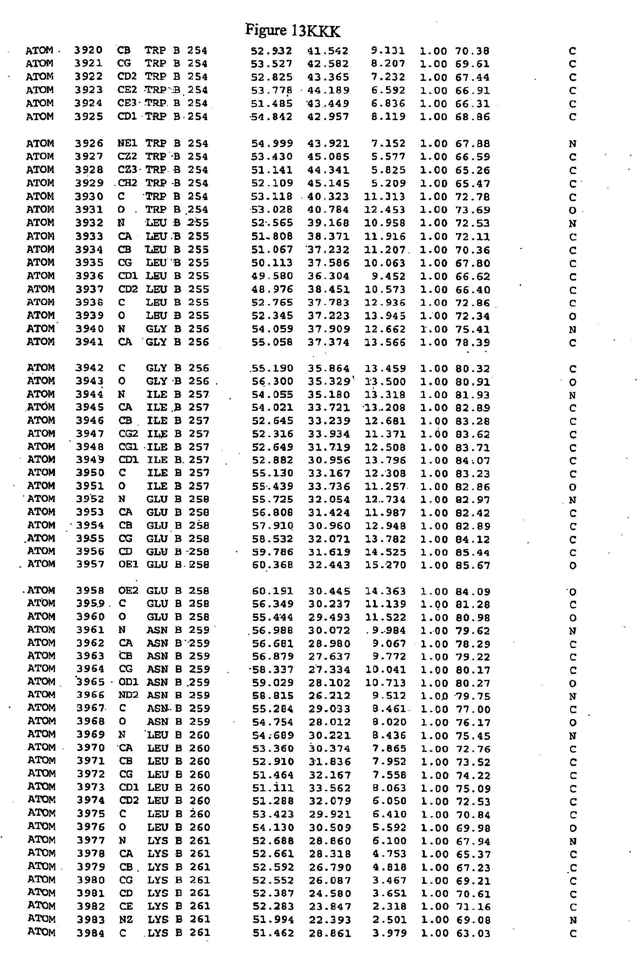

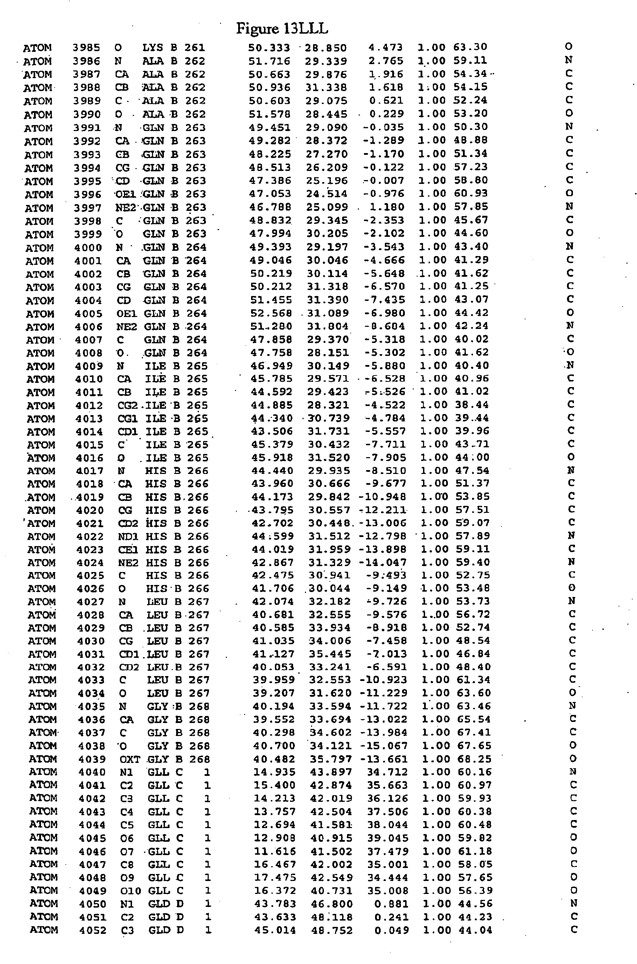

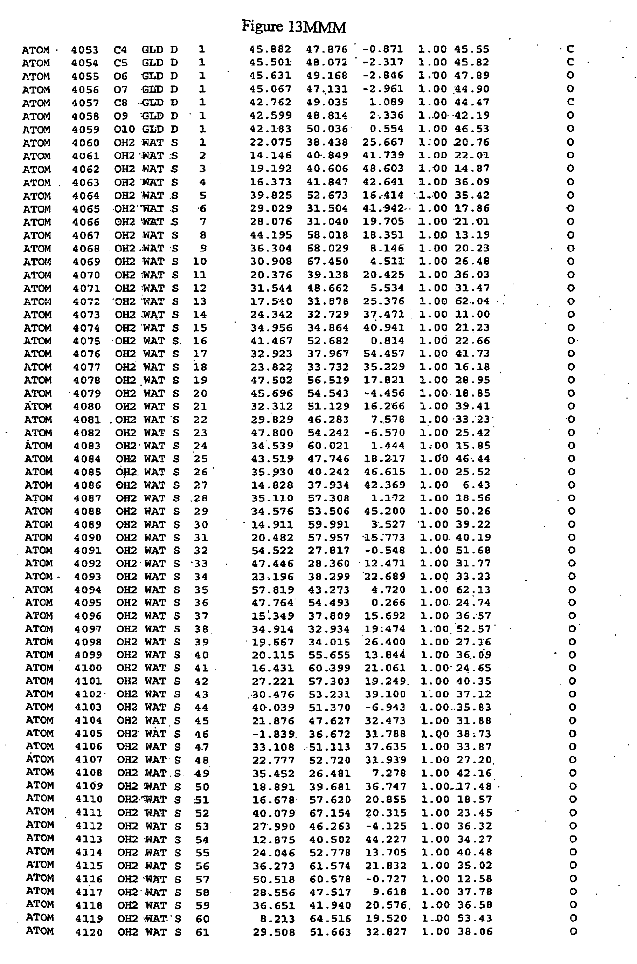

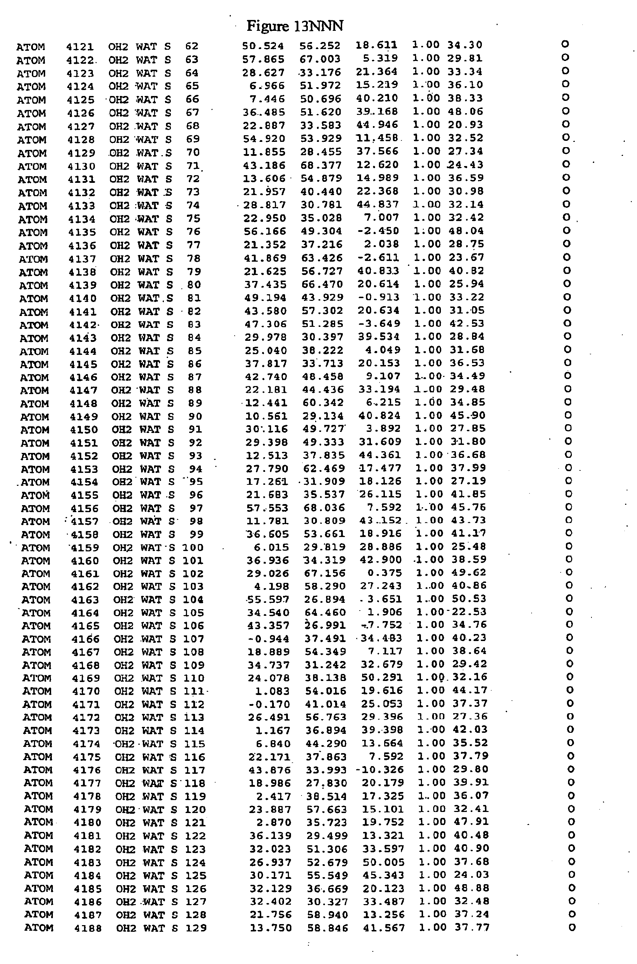

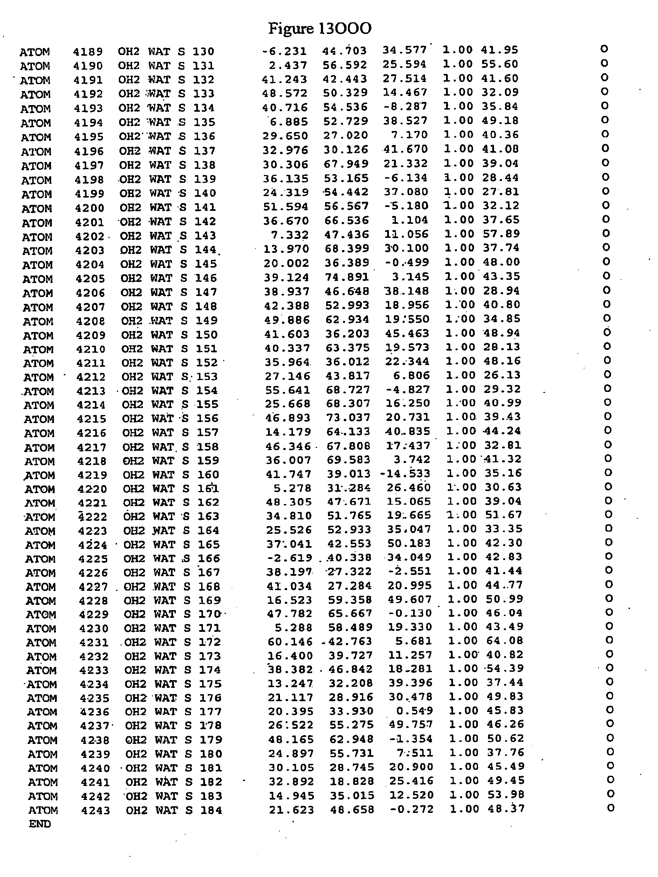

One embodiment relates to crystallized *E. faecalis* MurI characterized by the structural coordinates depicted in Figure 12. One embodiment discloses crystallized *E*. *faecalis* MurI complexed with D- and/or L-glutamate characterized by the structural coordinates depicted in Figure 13.

One embodiment of the crystallized complex of *E. faecalis* MurI and D- or L-glutamate is characterized as belonging to the orthorombic space group P2₁2₁2₁ and has cell dimensions of a = 60.29 Å, b = 82.08 Å and c = 115.57 Å, wherein α = 90°, β = 90°, and γ = 90°. This embodiment is encompassed by the structural coordinates of Figure 12, or in complex with D- and/or L-glutamate as encompassed by the structural coordinates of Figure 13.

One embodiment is a crystal of *E. faecalis* MurI complexed with the product (substrate) D- and/or L-glutamate wherein the MurI is at least 70%, 75%, 80%, 85%, 90%, 95%, 98%, or 99% homologous to the amino acid sequence represented by SEQ ID NO: 44, or portions thereof. The crystals may diffract from about 0.8 Å to about 3.5 Å.

One embodiment of the *E. faecalis* crystallized complex with D- and/or L-glutamate is characterized as belonging to the orthorombic space group P2₁2₁2₁ and having cell dimensions of a = 60.29 Å, b = 82.08 Å and c = 111.57 Å, wherein α = 90°, β = 90°, and γ = 90°, wherein the crystallized complex is produced by the process of preparing a first solution of about 5 mM D,L-glutamic acid, about 1 mM TCEP, about 200 mM ammonium acetate pH 7.4, about 10 mg/ml *E. faecalis* MurI; preparing a second solution of about 100 mM Tris pH 7.5, about 0.2 mM MgCl₂, and about 20-25% PEG 4000; combining the first solution and the second solution, thereby producing a combination; combining the first solution and the second solution, thereby producing a combination; and forming drops from the combination in a method of crystallization under conditions in which crystals of MurI are produced, whereby, crystals of MurI are produced.

A further embodiment relates to an *E*. *faecalis* glutamate racemase in which the binding site comprises two conserved cysteine residues, denoted Cys74 and Cys185 in the sequence represented as SEQ ID NO: 44. A further embodiment comprises a binding site of *E. faecalis* glutamate racemase wherein the binding site additionally comprises one or more of the following amino acid residues: Ser12, Thr76, Thr118, Thr121, Glu153, Thr186 and His187 as represented by the structural coordinates of Figure 12. In one embodiment, the binding site of the *E. faecalis* glutamate racemase is complexed with D- and/or L-glutamate and comprises amino acid residues Cys74 and Cys185 as well as amino acid residues within 5 Å of the Cys74 and Cys185 residues as represented by the structural coordinates of Figure 13. In one embodiment, the binding site of the *E. faecalis* glutamate racemase additionally comprises one or more of the following amino acid residues: Ser12, Thr76, Thr118, Thr121, Glu153, Thr186 and His187. In this embodiment, the binding site complexes with D- or L-glutamate and comprises amino acid residues Cys74 and Cys185 and at least one (i.e., one or more) of the following amino acid residues: Ser12, Thr76, Thr118, Thr121, Glu153, Thr186 and His187, which can be present in any combination. In a further embodiment, the binding site includes amino acid residues Ile10, Asp11, Ser12, Glyl3, Val14, Gly15, Gly16, Thr18, Val19, Tyr34, Asp37, Arg40, Cys41, Pro42, Tyr43, Gly44, Pro45, Arg46, Val51, Glu53, Ile72, Ala73, Cys74, Asn75, Thr76, Ala77, Ser78, Ala79, Val96, Ile116, Gly117, Thr118, Leu119, Gly120, Thr121, Ile122, Tyr127, Cys145, Pro146, Val149, Pro150, Leu183, Giy184, Cys185, Thr186, His187, Tyr188 and Ser208 of SEQ ID NO: 44.

In a further embodiment, the binding site of the glutamate racemase comprises two hydrogen bond TRIADS, which occur close to the conserved cysteine residues of the binding site. Specifically, on one side (Cys185) of the binding site, the TRIAD is Glu153-Thr186-His187 and on the other side (Cys74), the TRIAD is Thr76-Thr118-Thr121. Thus, in a specific embodiment, the binding site of *E. faecalis* glutamate racemase is complexed with D- and/or L-glutamate and comprises amino acid residues Cys74 and Cys185 and additionally comprises at least one (one or more) of the following: amino acid residues Ser12, Thr76, Thr118, Thr121, Glu153, Thr186 and His187.

The threonine residues have features of interest as they relate to the binding site of the *E. faecalis* glutamate racemase. On one side (Cys185) of the binding site, Thr186 is H-bonded to His187 and His187 is further H-bonded to Glu153. The hydroxyl oxygen (O) of Thr186 is 2.9 Å away from the amino nitrogen (N) of the substrate (glutamate) and 4.3 Å from the sulfur (S) atom of Cys185, which is 4.1 Å from the nitrogen (N) of His187.

On the other side of the binding site (Cys74), Thr76 is H-bonded to Thr118 and further H-bonded to Thr121. The hydroxyl O of Thr76 is H-bonded to one of the carboxylate oxygen atoms of the substrate and is 4.3 Å away from the S atom of Cys74. Analysis showed that the three hydroxyl oxygens form a triangle, all within less than 3.4 Å from one another.

The two TRIADs may play important roles in altering the pKa of the two binding site cysteine residues (in addition to that of the neighboring hydrophobic core), facilitating the proton transfer during catalysis or both.

Another embodiment is a crystal of glutamate racemase, comprising a binding site comprising the amino acid residues Cys74, Cys185, Ser12, Thr76, Thr118, Thr121, Glu153, Thr186 and His187 of the amino acid sequence represented by SEQ ID NO: 44. In one embodiment is a crystal of glutamate racemase complexed with D- or L-glutamate. Additionally, the binding site of the crystal complexed with D-glutamate can comprise the amino acid residues: Cys74, Cys185, and one or more of the following amino acid residues: Ser12, Thr76, Thr118, Thr121, Glu153, Thr186 and His187. A further embodiment is one in which the binding site additionally comprises at least one of the following amino acid residues: Asp11, Gly13, Val14, Gly15, Gly16, Arg40, Cys41, Pro42, Arg46, Ala73, Asn75, Ala77, Va196, Gly117, Val149, Gly184, and Tyr188. A further embodiment is one in which the binding site additionally comprises at least one of the following amino acid residues: Ile10, Thr18, Val19, Tyr34, Tyr43, Gly44, Pro45, Asp37, Val51, Ile72, Ser78, Ala79, Ile116, Leu119, Gly120, Ile122, Tyr127, Cys145, Pro146, Pro150, Leu183, and Ser208 of SEQ ID NO: 44. The crystals comprising binding sites represented by the above amino acid residues comprise the amino acid sequence is that represented by SEQ ID NO: 44; or an amino acid sequence is at least 75%, 80%, 85%, 90%, 95%, or 98% homologous to the amino acid sequence that is represented by SEQ ID NO: 44.

Location and geometry of a binding site of the enzyme are also defined. Two conserved cysteine (Cys) residues were identified as the residues responsible for the (de)protonation of the alpha-carbon of the substrate during catalysis, which is consistent with the two-base mechanism proposed for function of the enzyme in its role as a racemase. The two binding site cysteines are Cys74 and Cys185, which are about 7.0 Angstroms (Å) apart (Cα-Cα distance, which is the distance between Cα atoms). Other amino acid residues identified in the vicinity of the binding site include Ser12, Thr76, Thr118, Thr121, Glu153, Thr186 and His187. The bound substrate/product, D- or L-glutamate, is located between the two conserved cysteine residues. Further detail of the binding site is as follows: looking down the axis defined by the G1u153 along the γ and α carbon, the two cysteines exhibit a rather symmetrical environment; each has a hydrophobic core behind, with respect to the substrate and a neighboring threonine residue not far from the substrate C-alpha (3.7 - 3.4 Å when L-Glu is bound; and 3.4 - 4.3 Å when D-Glu is bound). Here, product is present in one site and a substrate in the other. There are conformational changes bringing the cysteines closer in the sub-unit with the substrate.

Analysis of the crystal structure of the *E. faecalis* glutamate racemase indicates that the following amino acid residues are within 10 Å of the bound D- or L-glutamate: 10-19, 34-46, 51, 72-78, 96-97, 116-122, 124, 127, 183-189, 208-209. Analysis of the crystal structure also shows that the following amino acid residues are within 4 Å of the bound D- or L-glutamate: 11-12 41-44, 74-76, 118, 121, and 186-187. Of interest is the fact that only one acidic amino acid residue (Asp11) is present in the structure surrounding the binding site Cys74, which serves as an anchoring point for the amino group of the substrate/product with a polar (charged) interaction between the amino nitrogen atom of D- or L-glutamate and the delta oxygen atom of Asp11.

### 2. E. faecium

We provide a method of making a crystal of *E. faecium* Murl complexes. The method comprises preparing a first solution of from about 2 to about 10 mM D,L-glutamate; from about 0.1 to about 10 mM reducing agent; from about 50 to about 500 mM salt pH 6.5-9.4; and about 10 mg/ml *E. faecium* MurI, wherein the glutamate, reducing agent, and salt are each present in sufficient concentration to bind to MurI, inhibit oxidation of the protein, and stabilize the protein and prevent aggregation; preparing a second solution of from about 50 to about 500 mM salt pH 4.5-9.0; wherein the salt is present in sufficient concentration to stabilize the protein, prevent aggregation and control the pH of the solution; combining the first solution and the second solution, thereby producing a combination; and forming drops from the combination under conditions in which crystals of MurI are produced.

In one embodiment, the substrate binding site of *E. faecium* Murl includes amino acid residues: Ser15, Gly16, Va117, Gly18, Pro45, Tyr46, Gly47, Cys77, Asn78, Thr79, Thr121, Ile122, Gly123, Thr124, Lys150, Phe151, Val152, Gly187, Cys188, Thr189, and His190 of SEQ ID NO: 48.

### 3. S. aureus

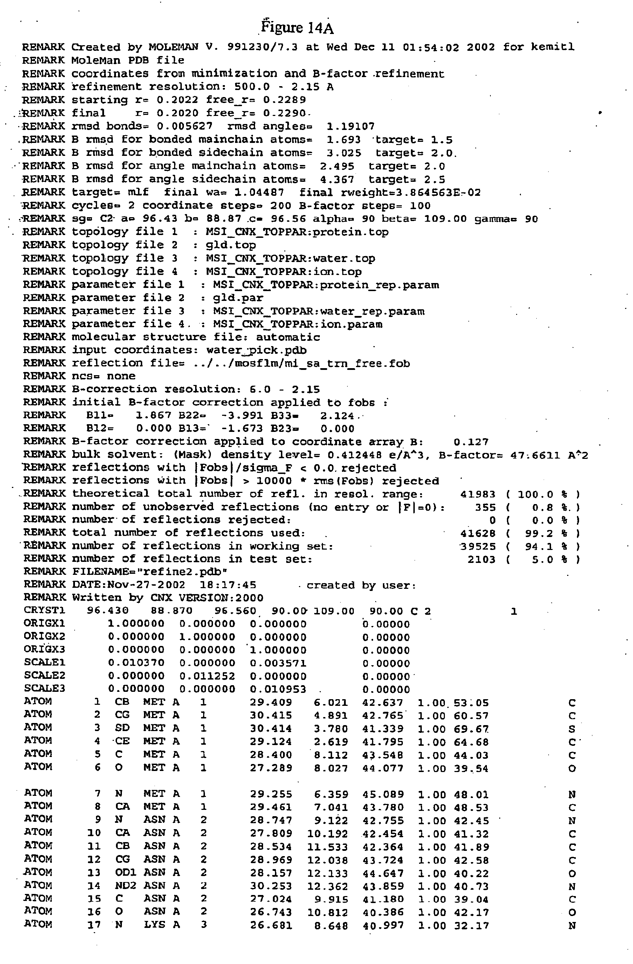

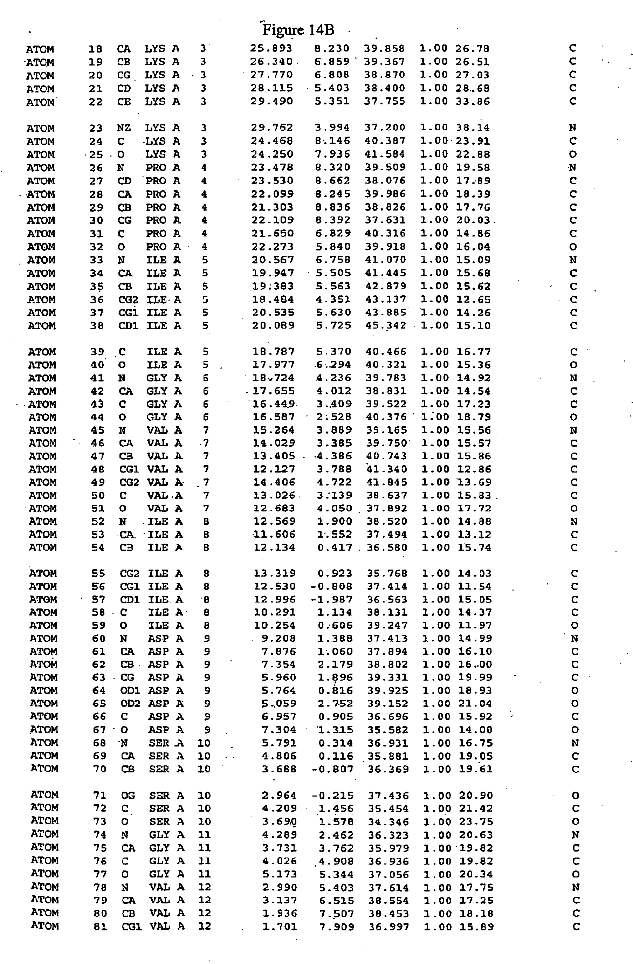

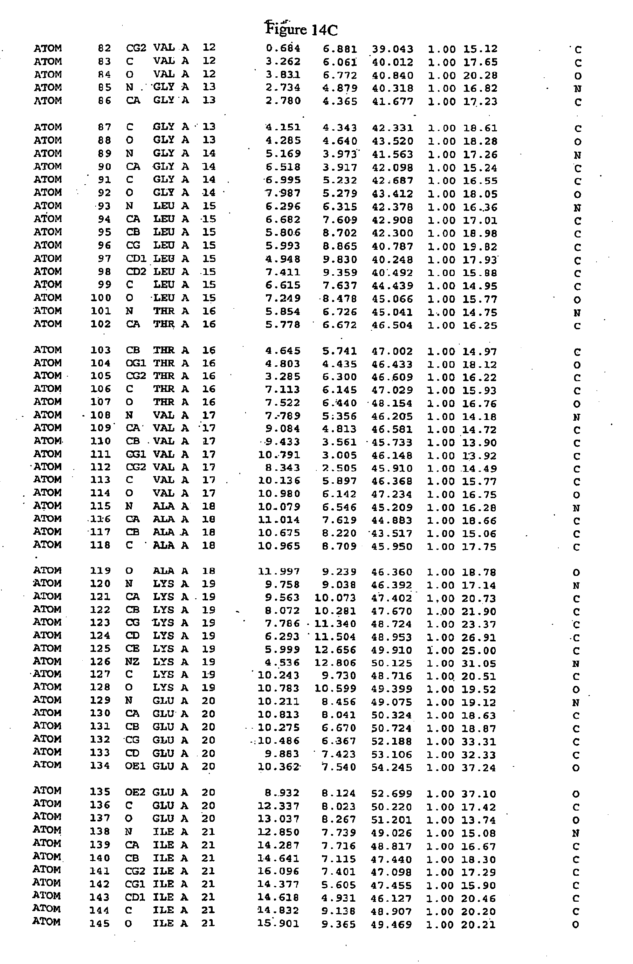

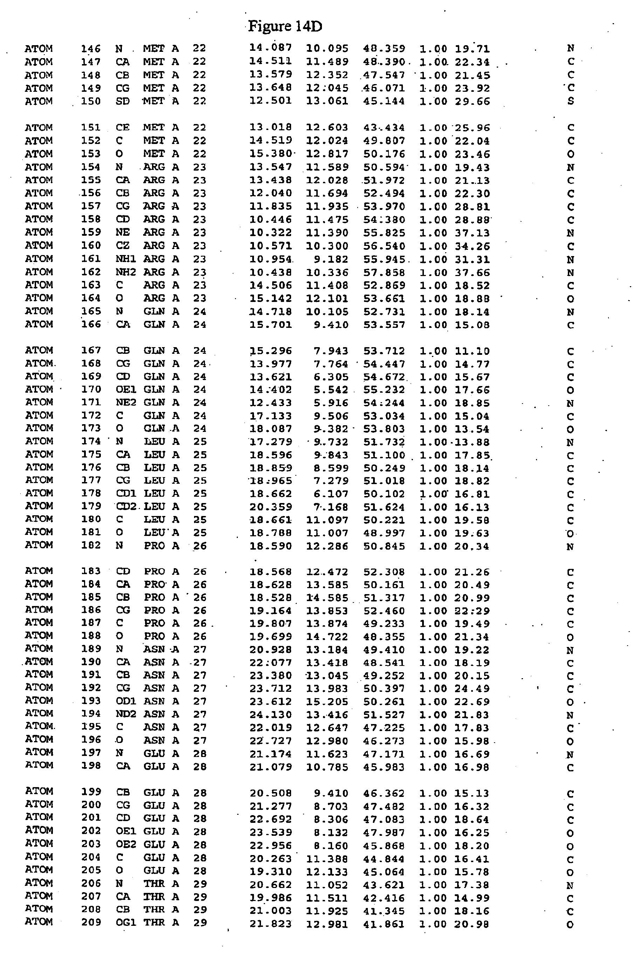

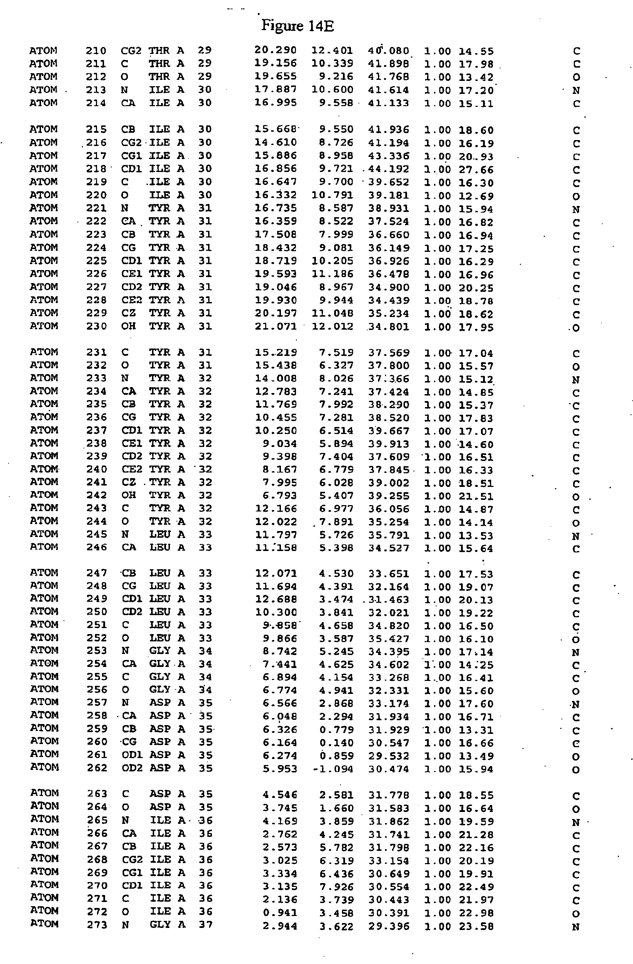

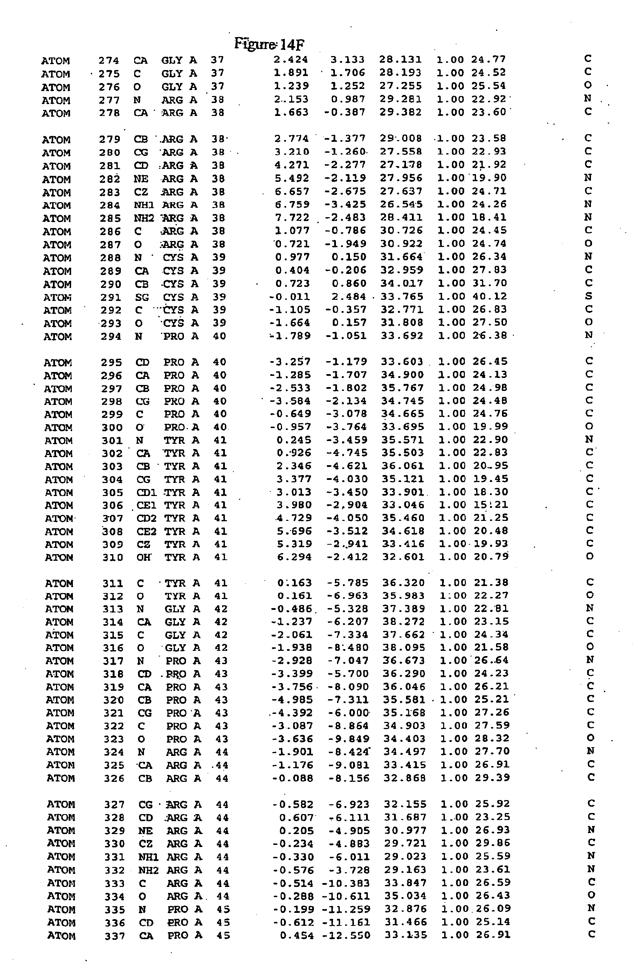

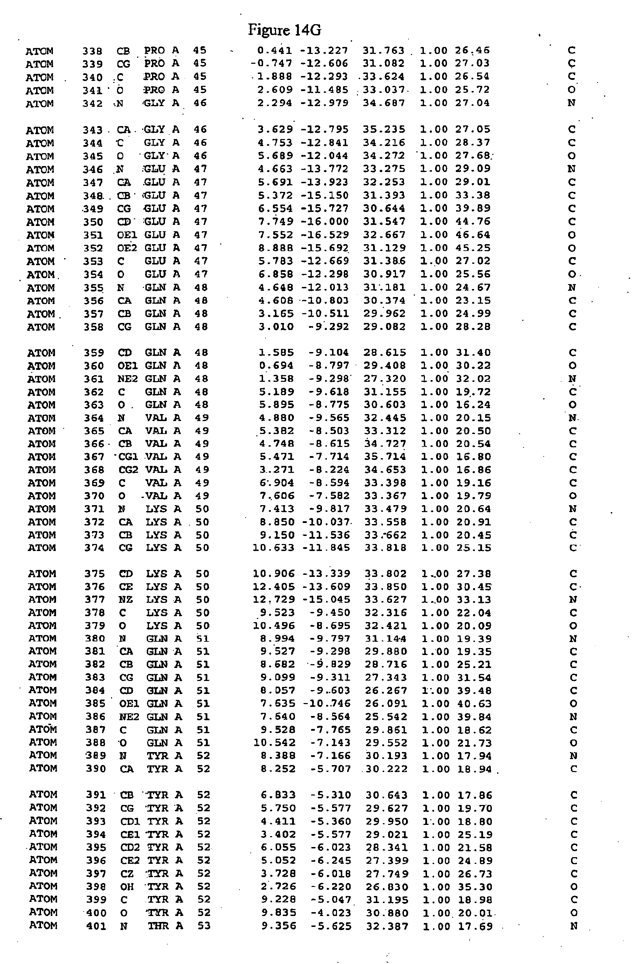

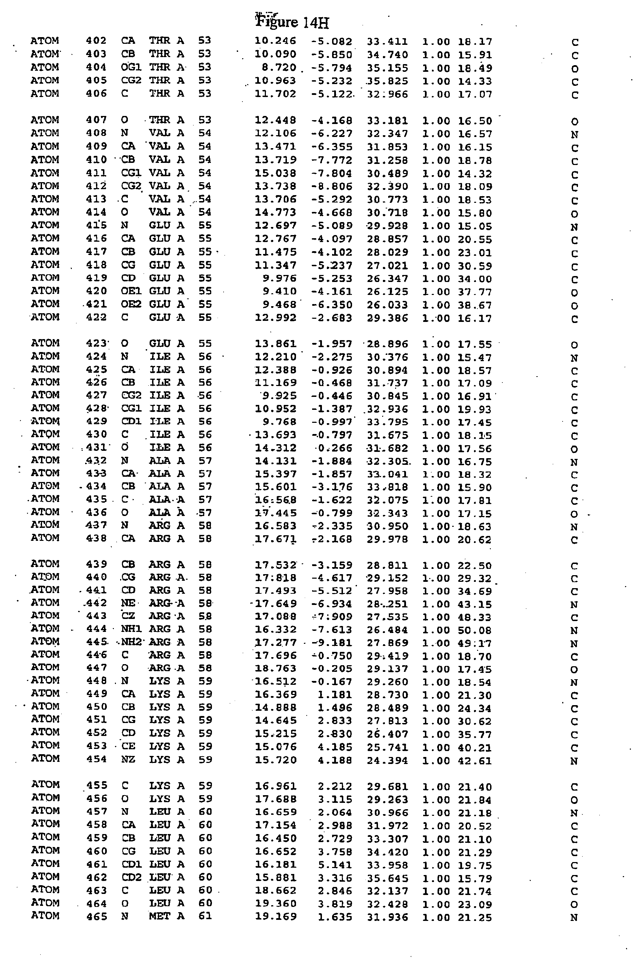

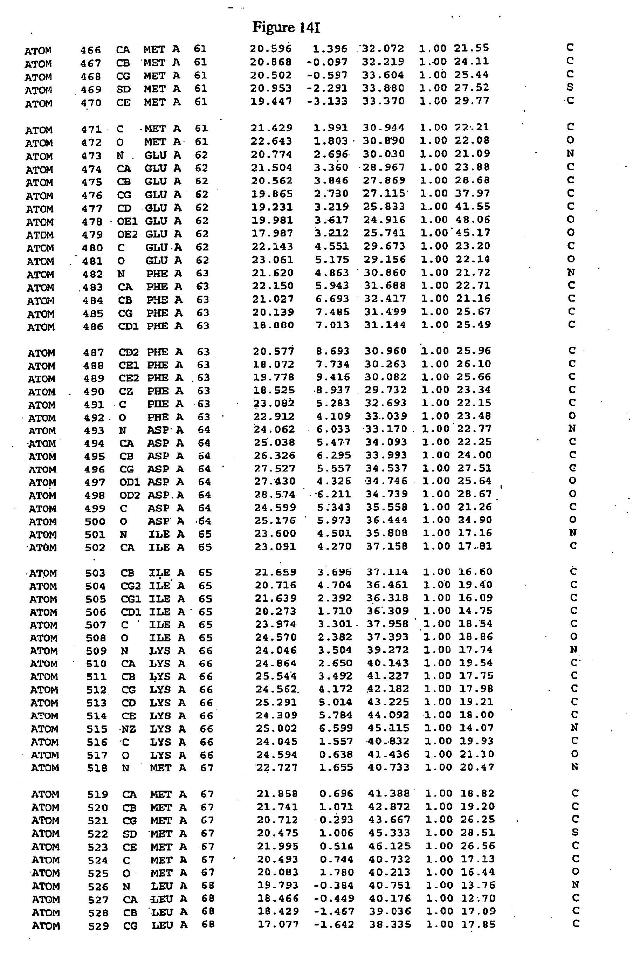

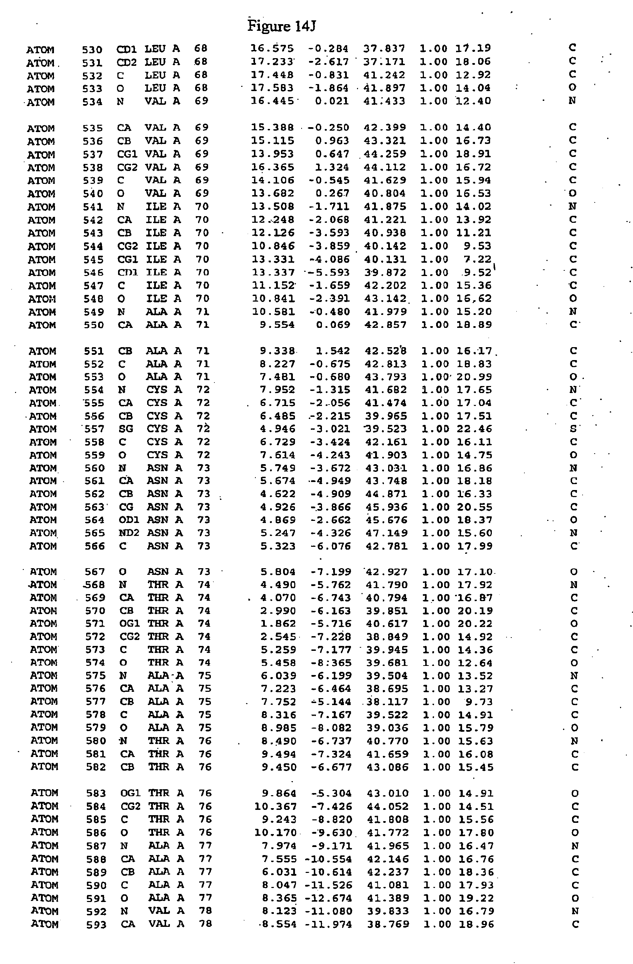

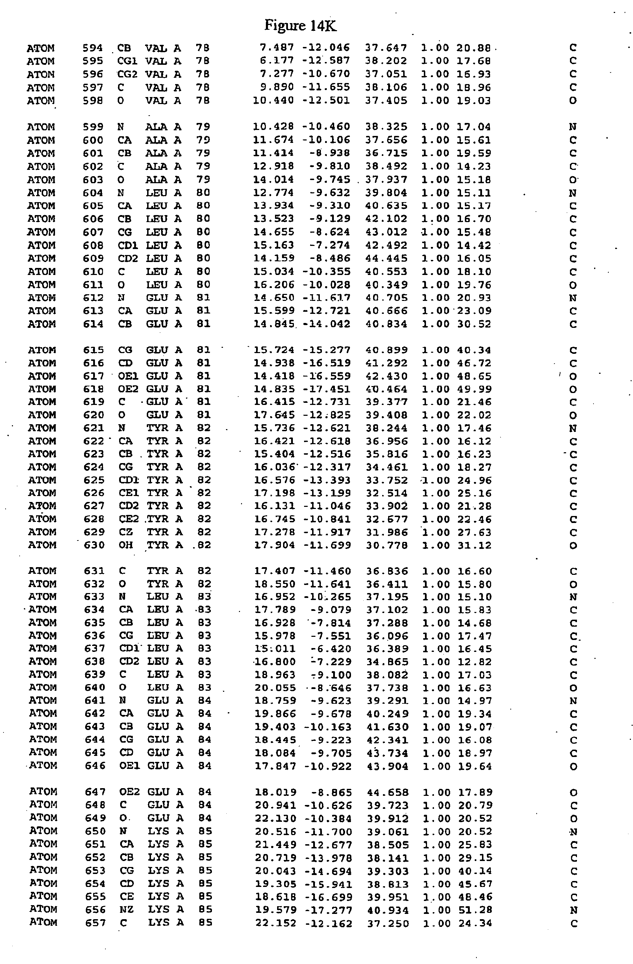

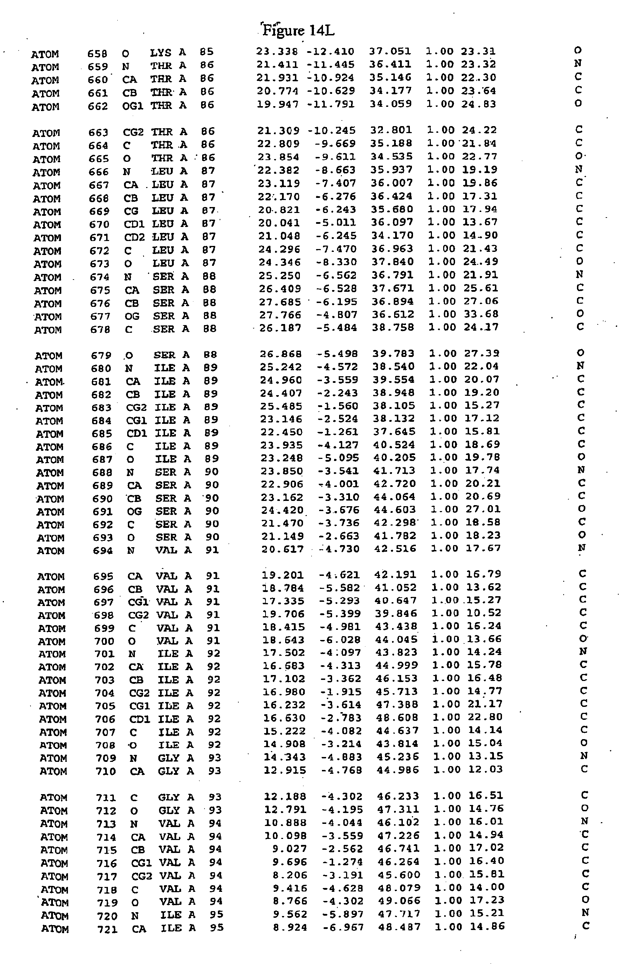

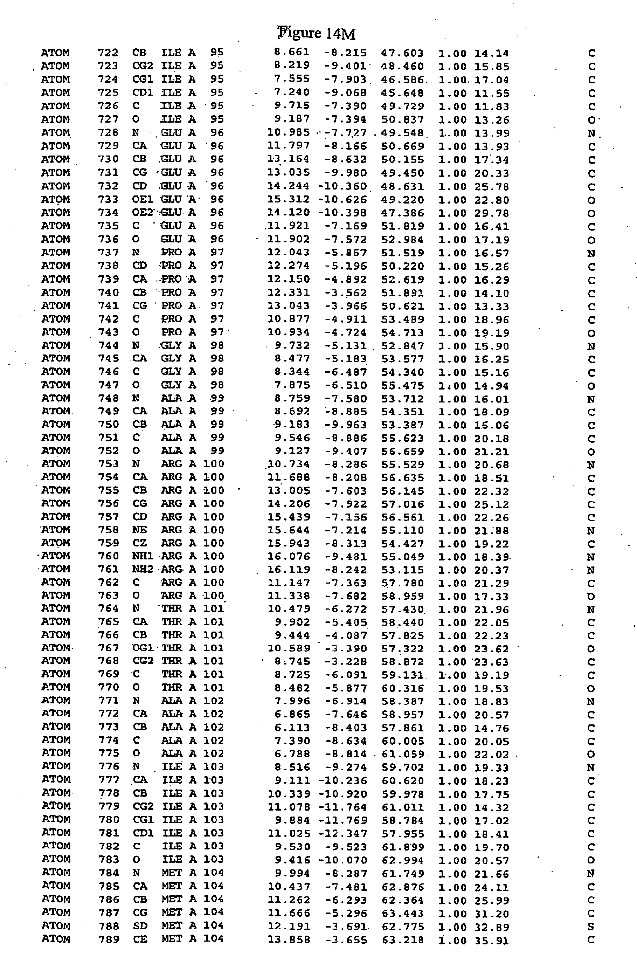

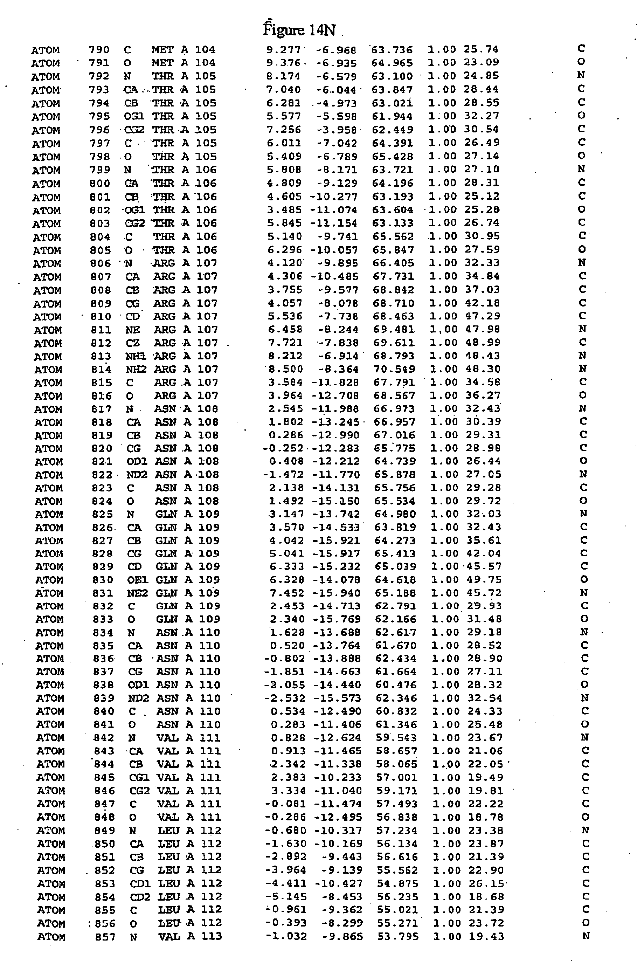

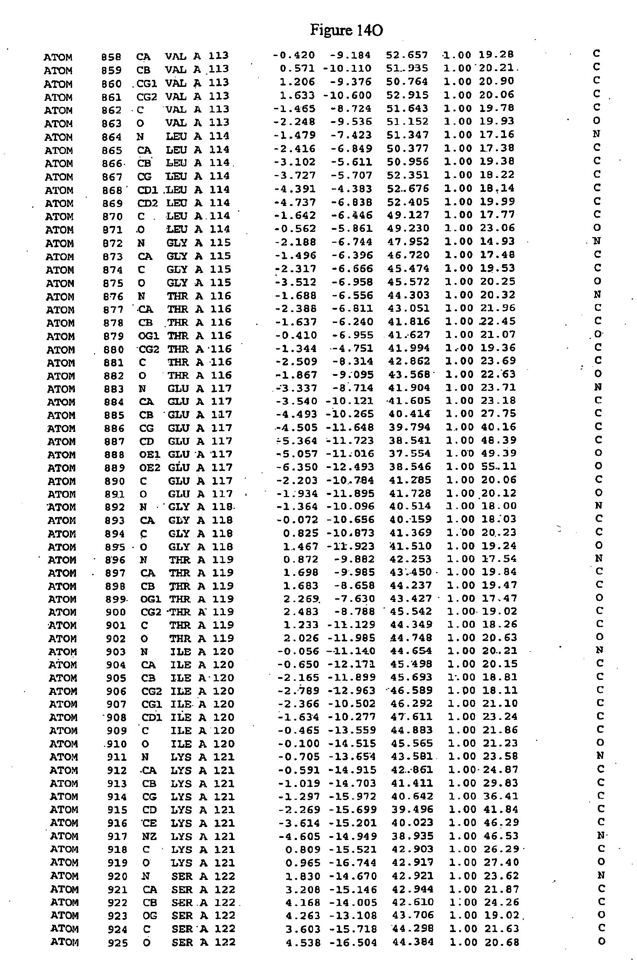

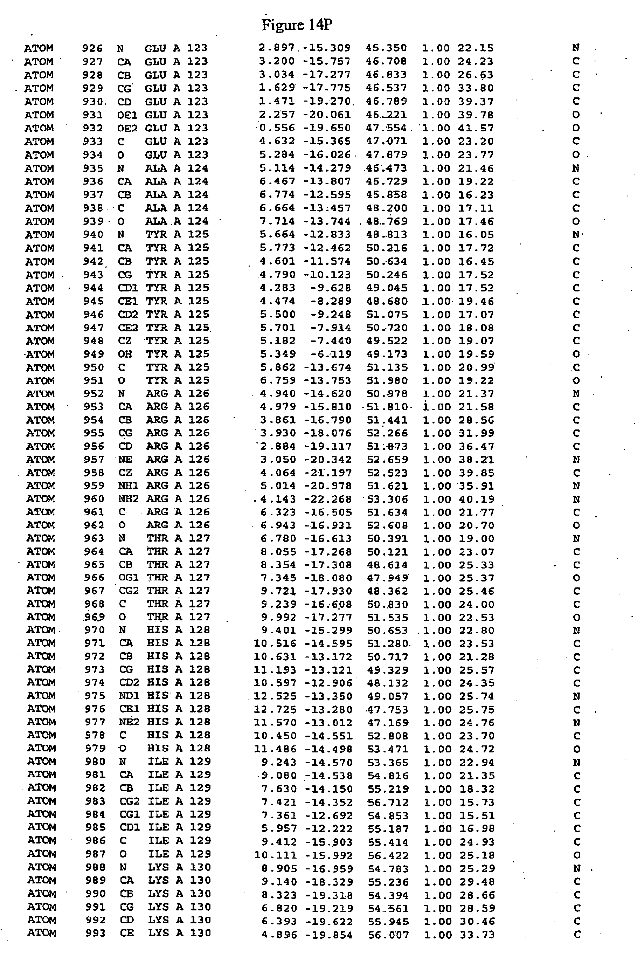

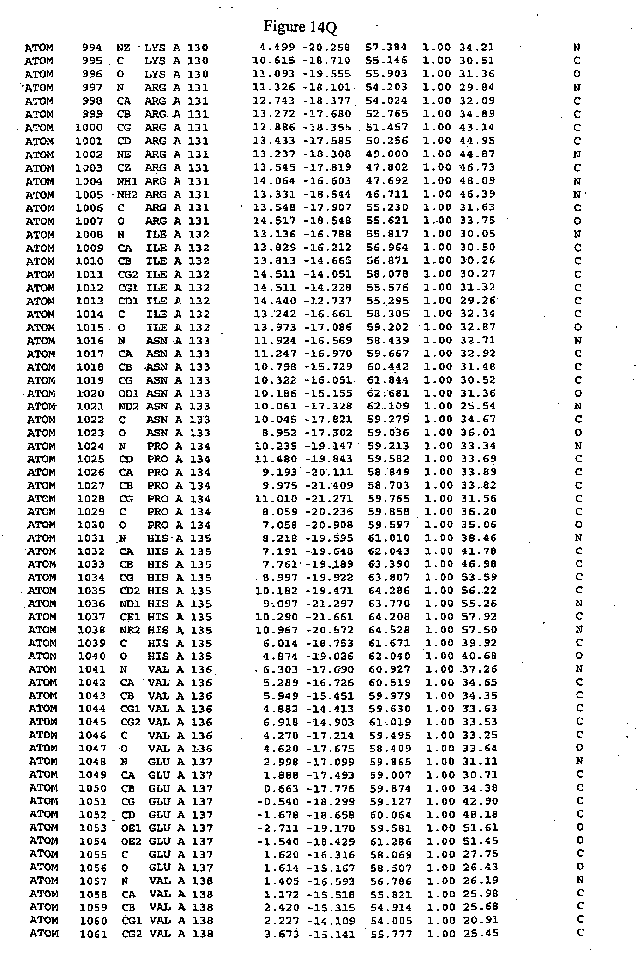

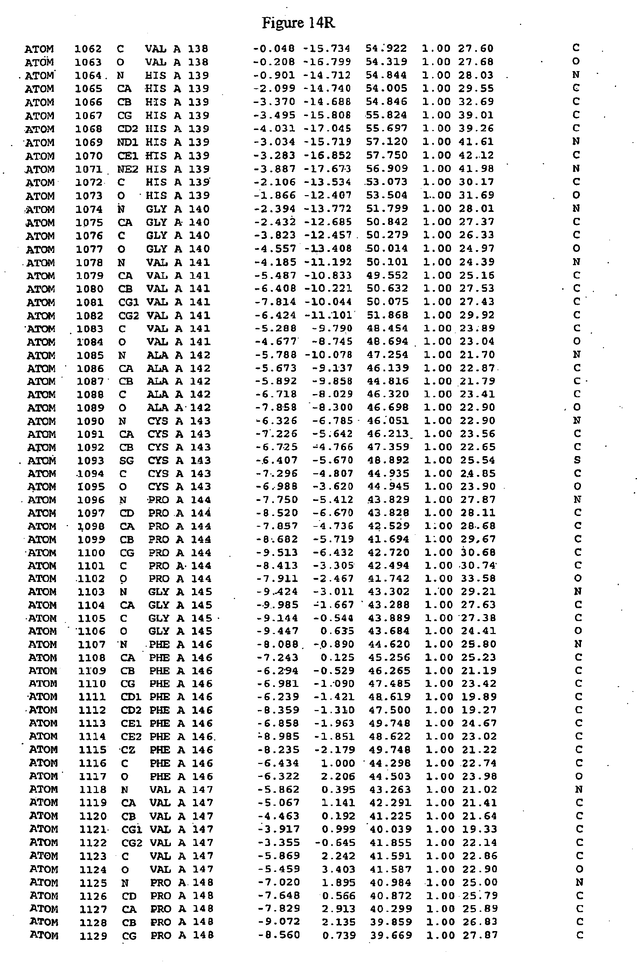

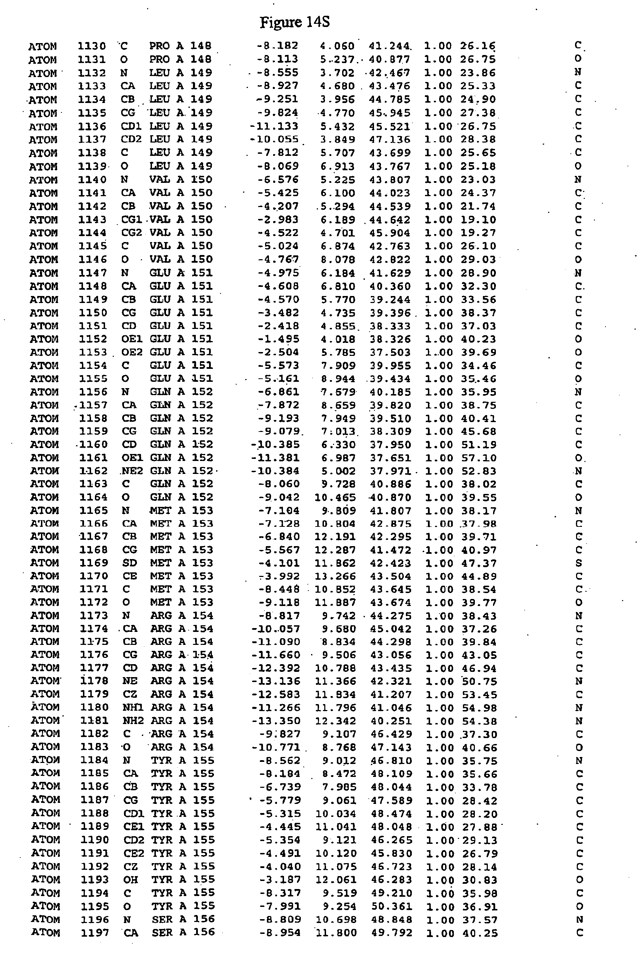

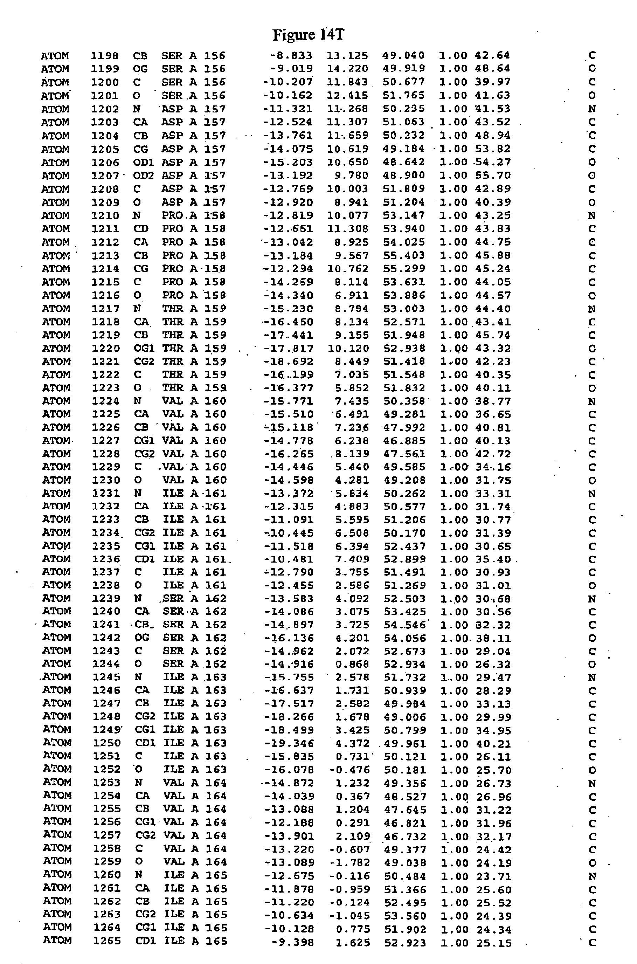

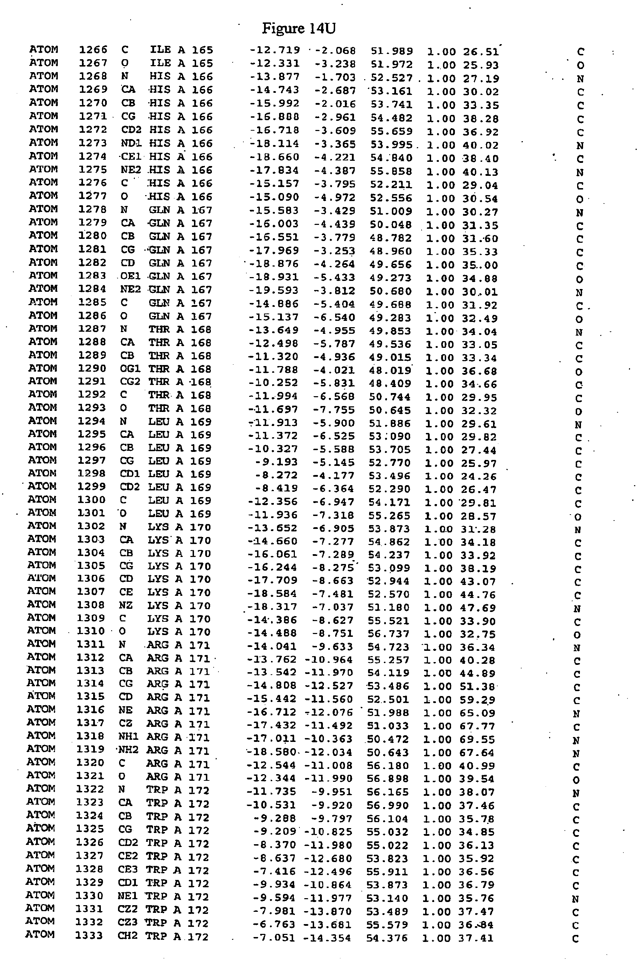

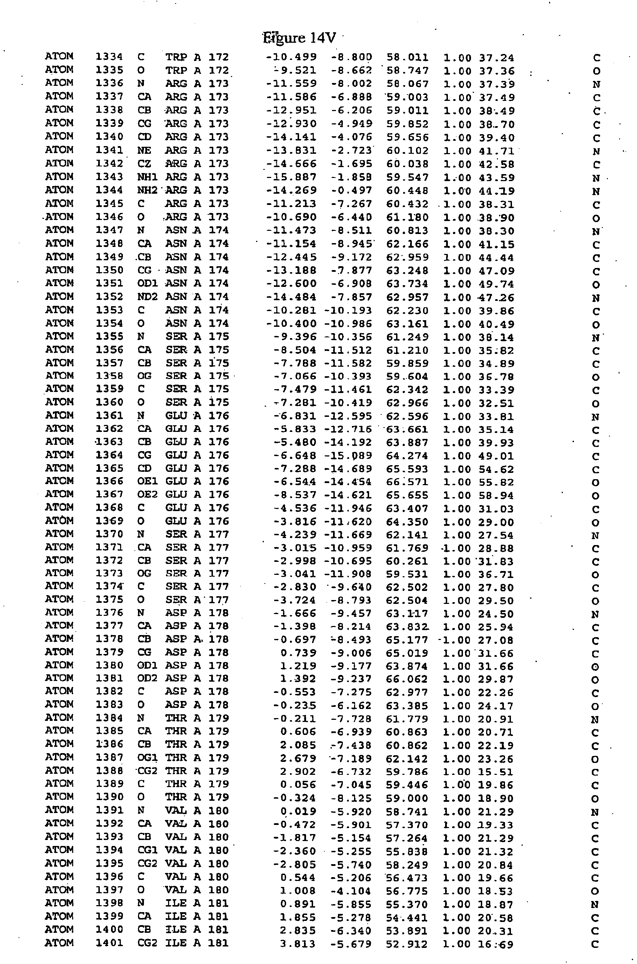

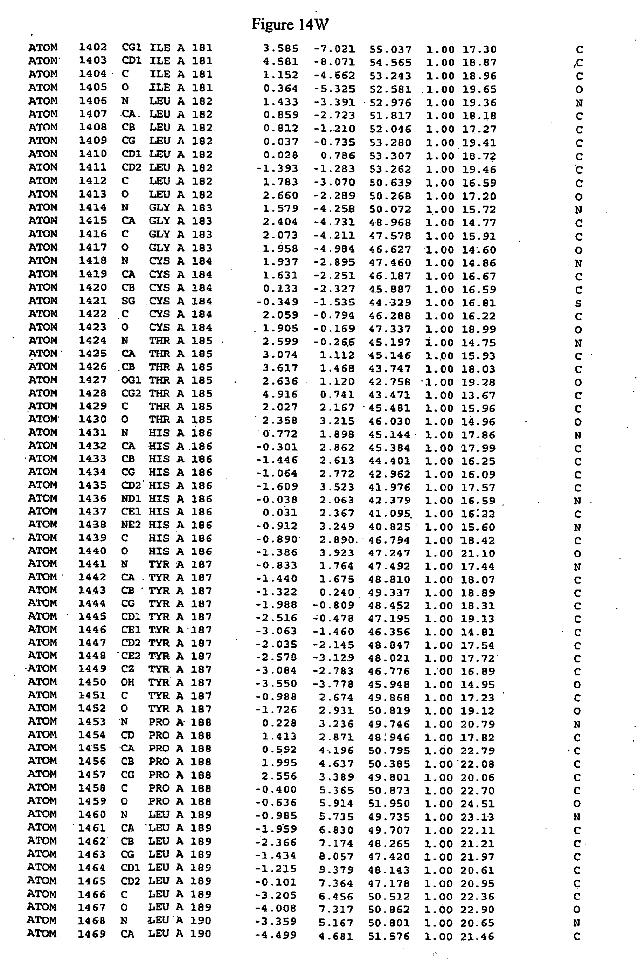

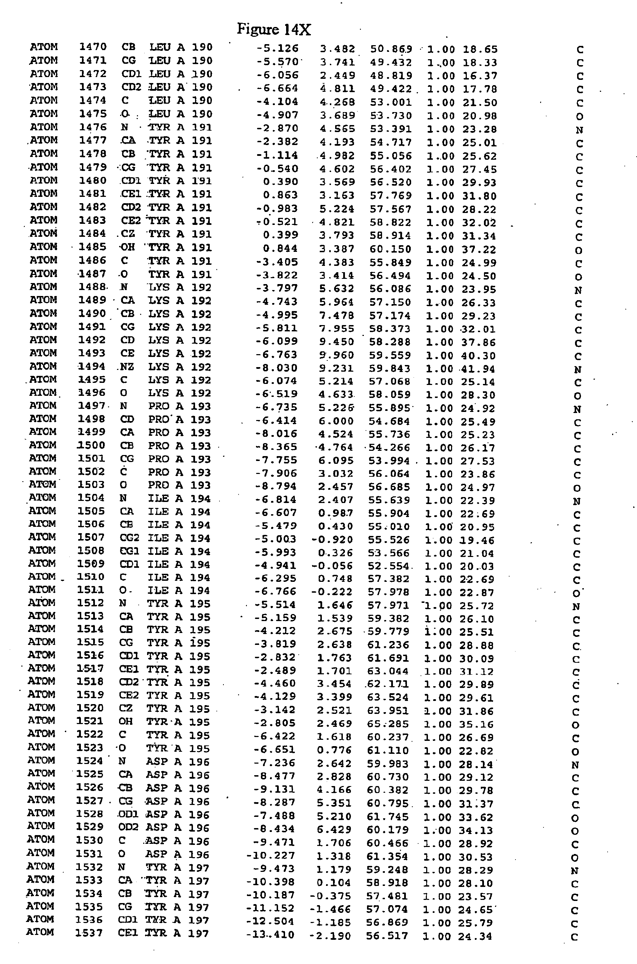

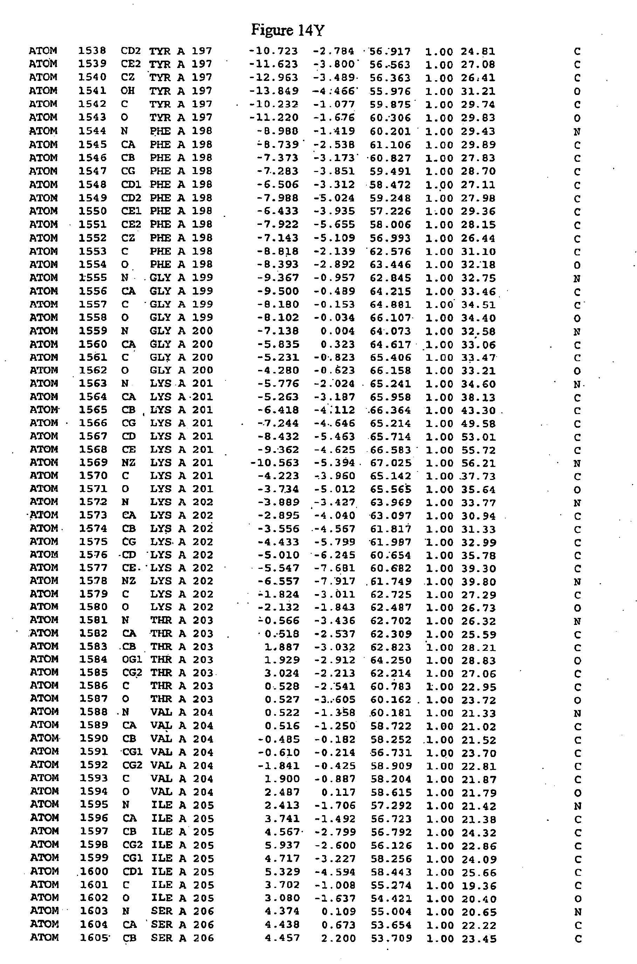

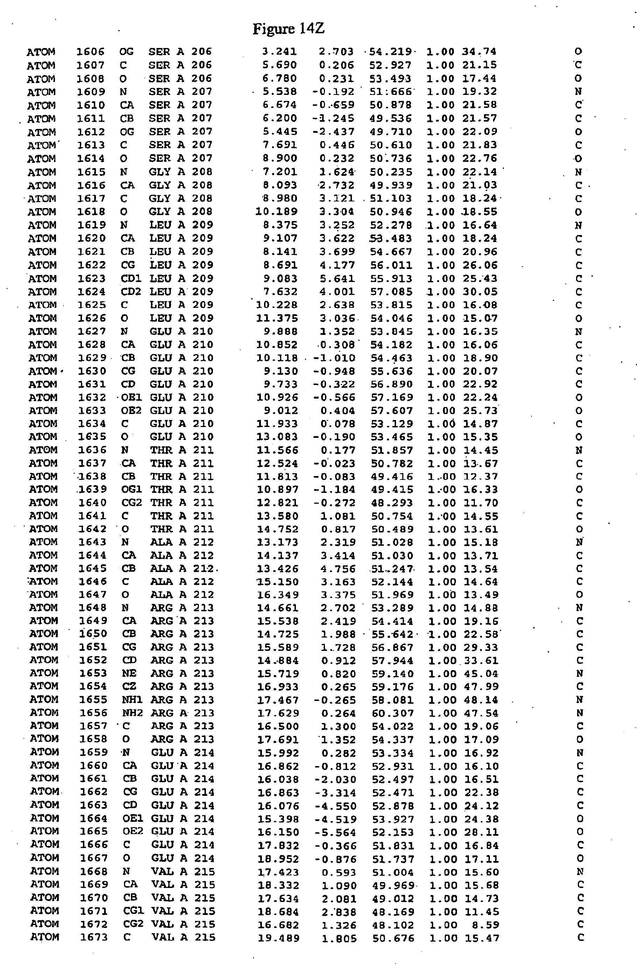

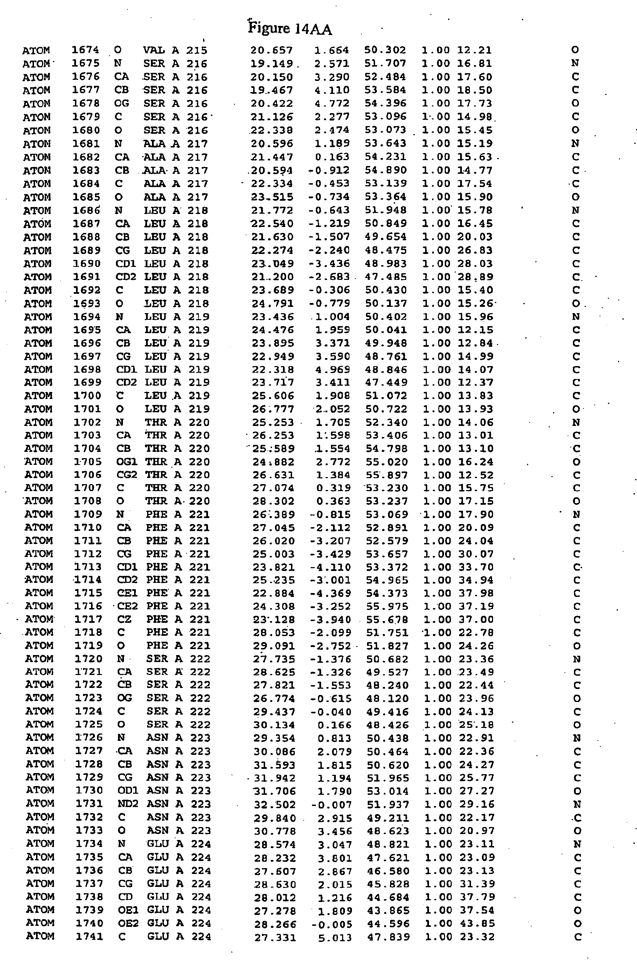

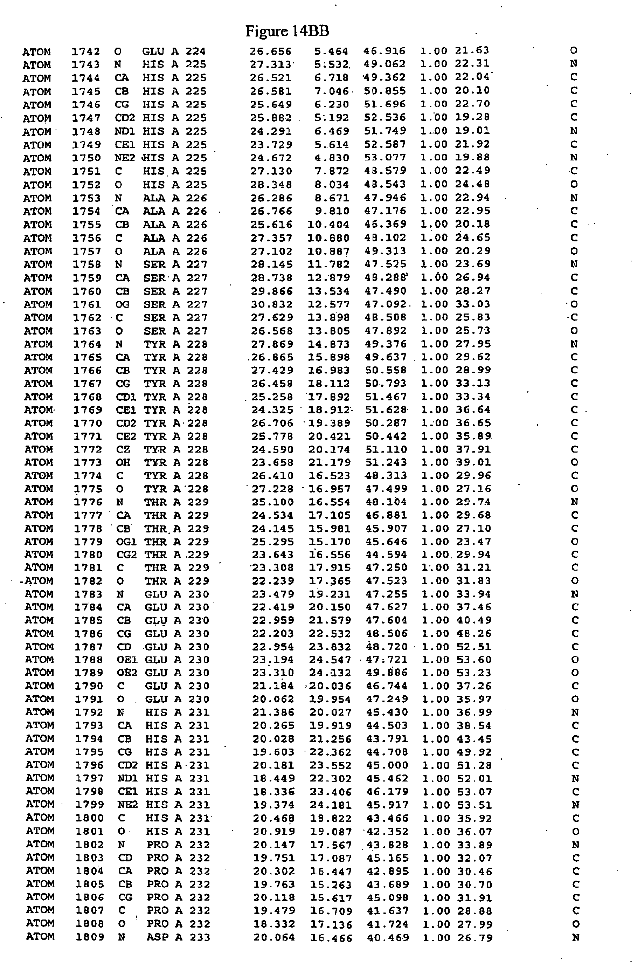

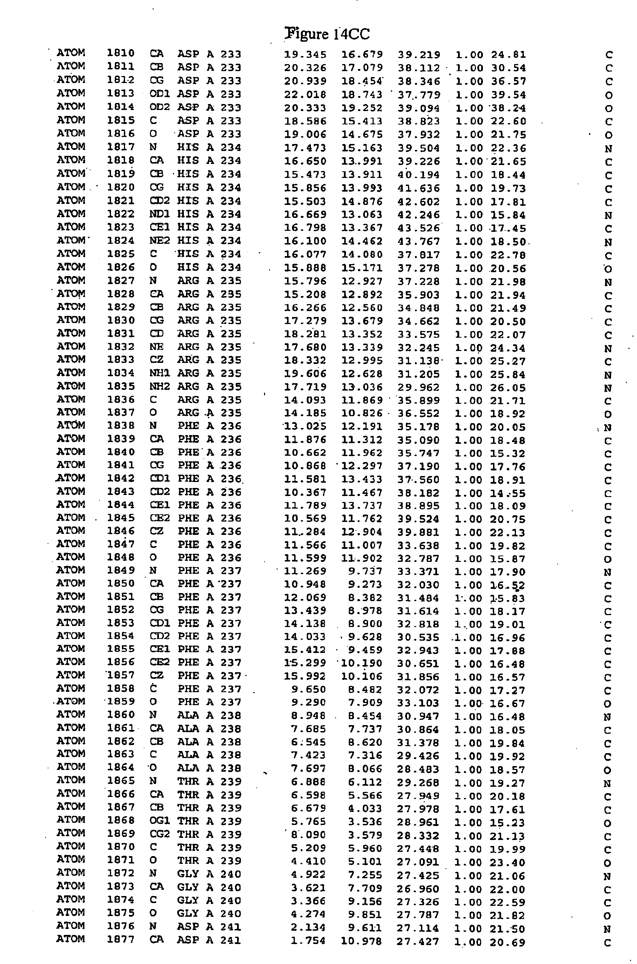

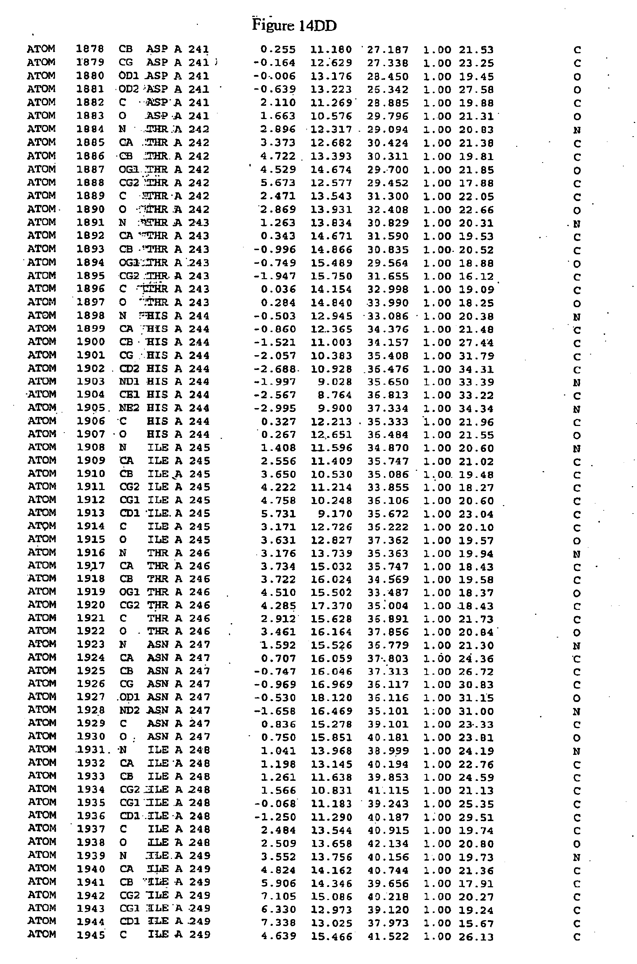

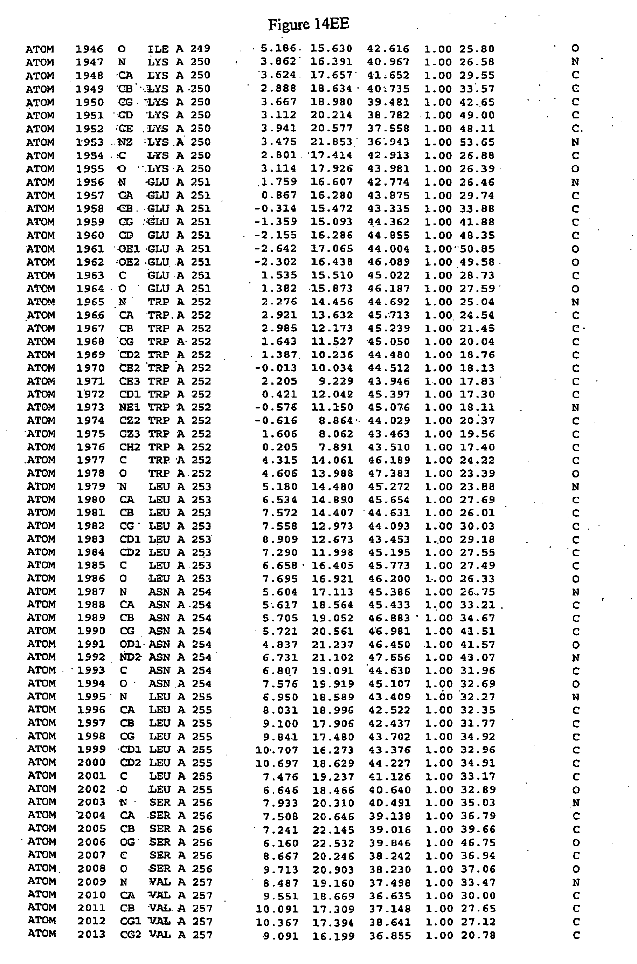

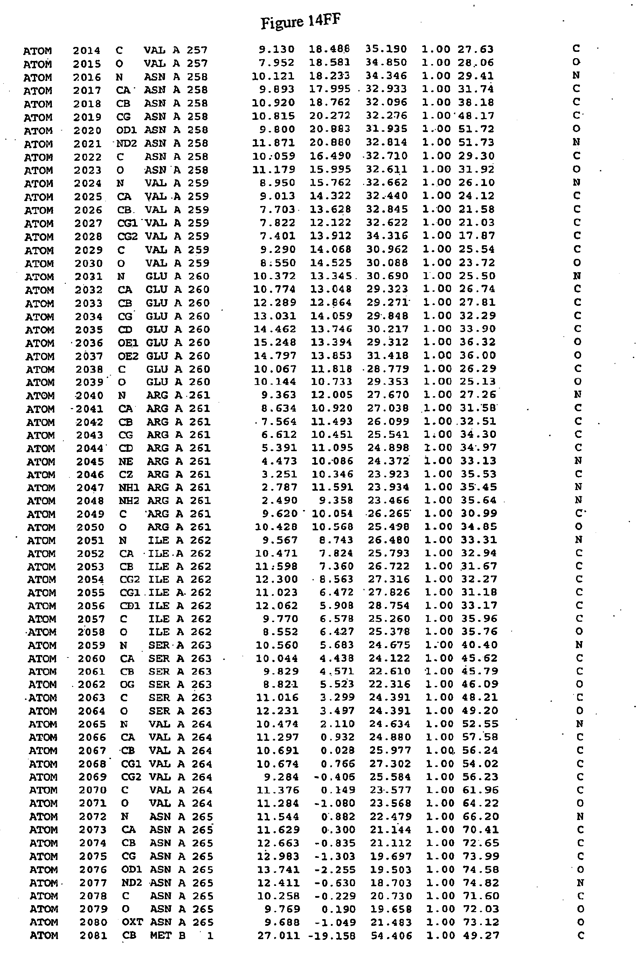

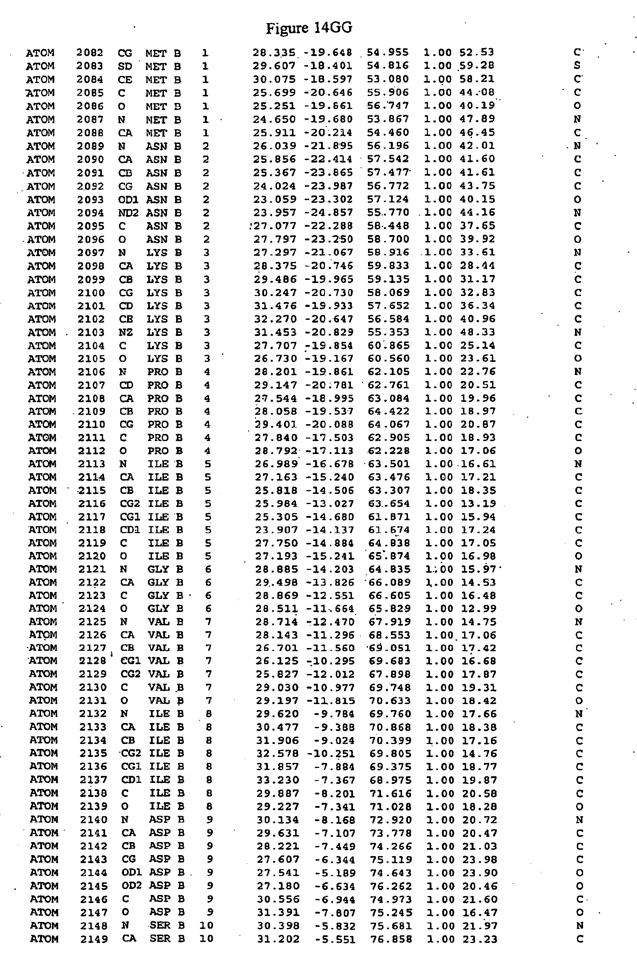

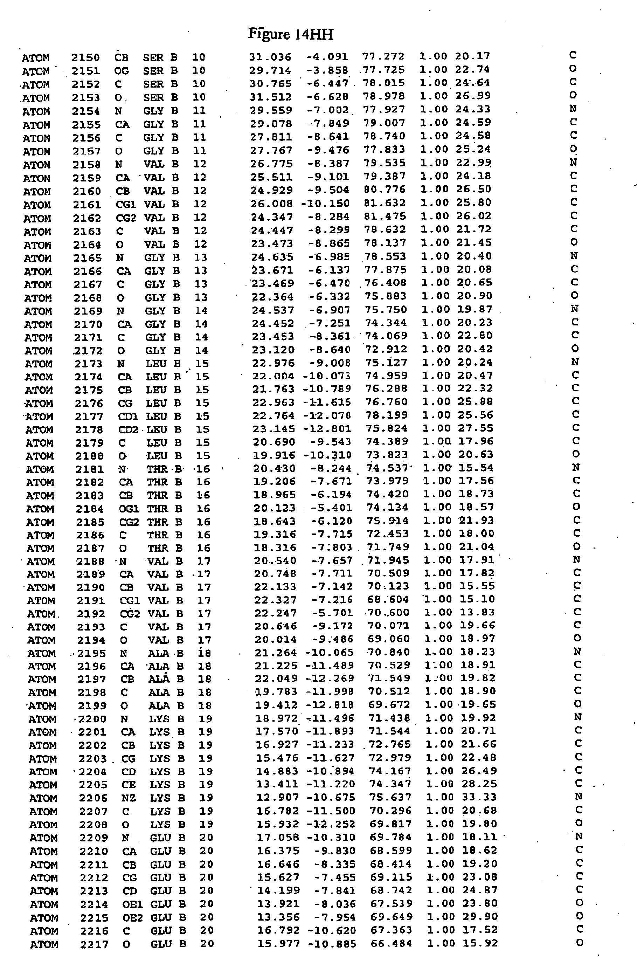

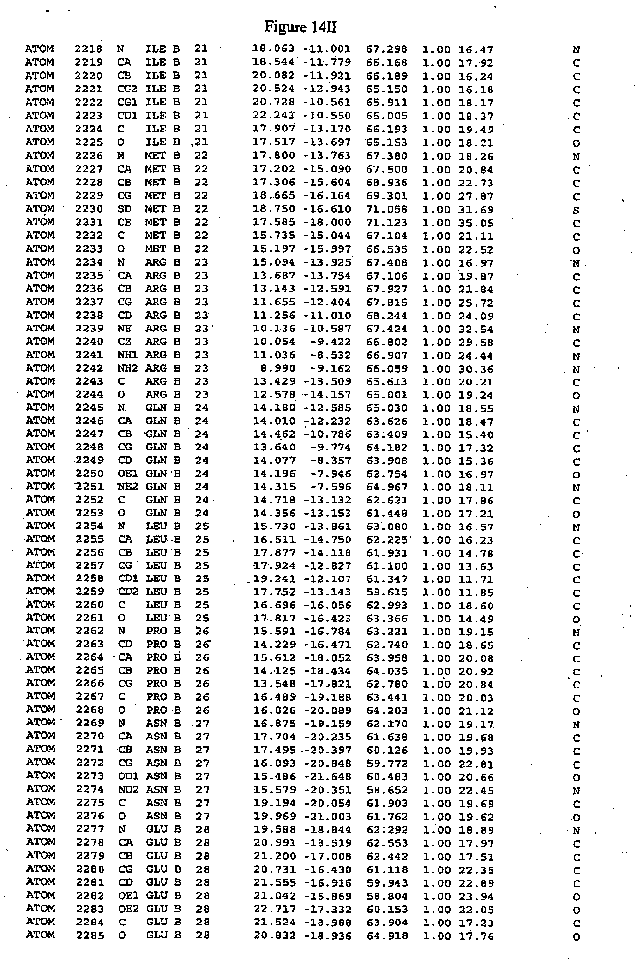

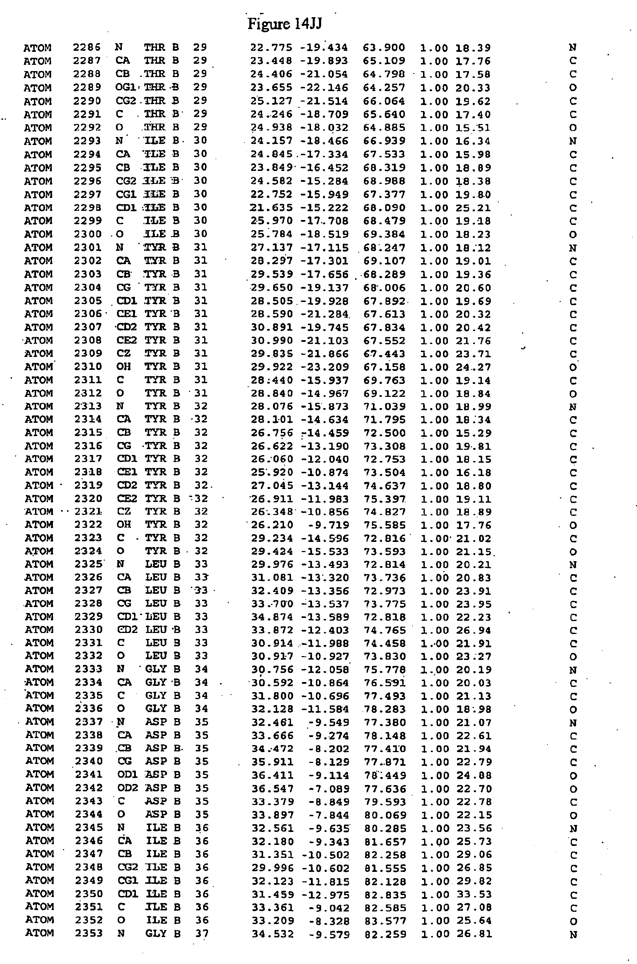

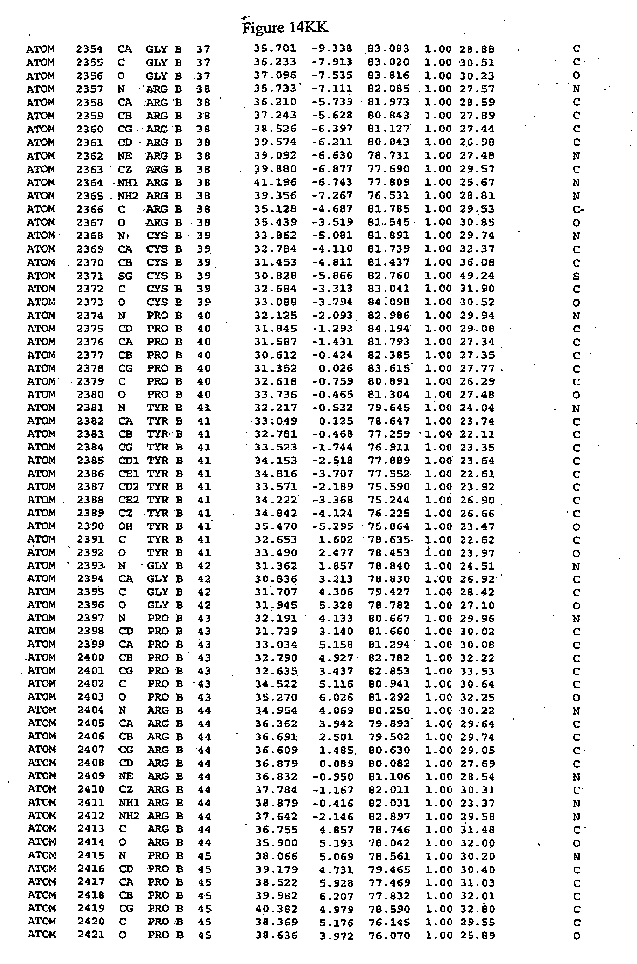

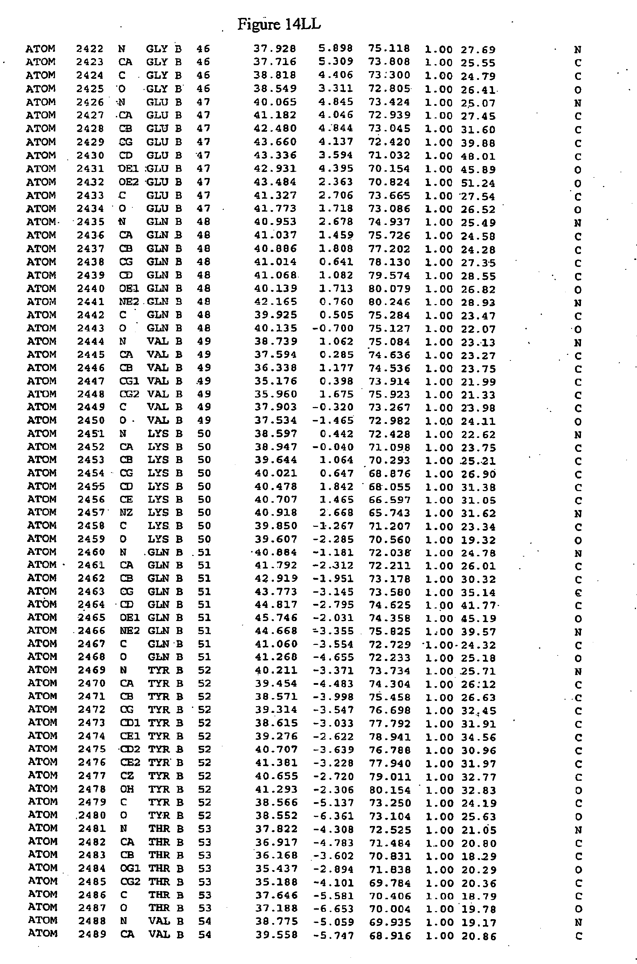

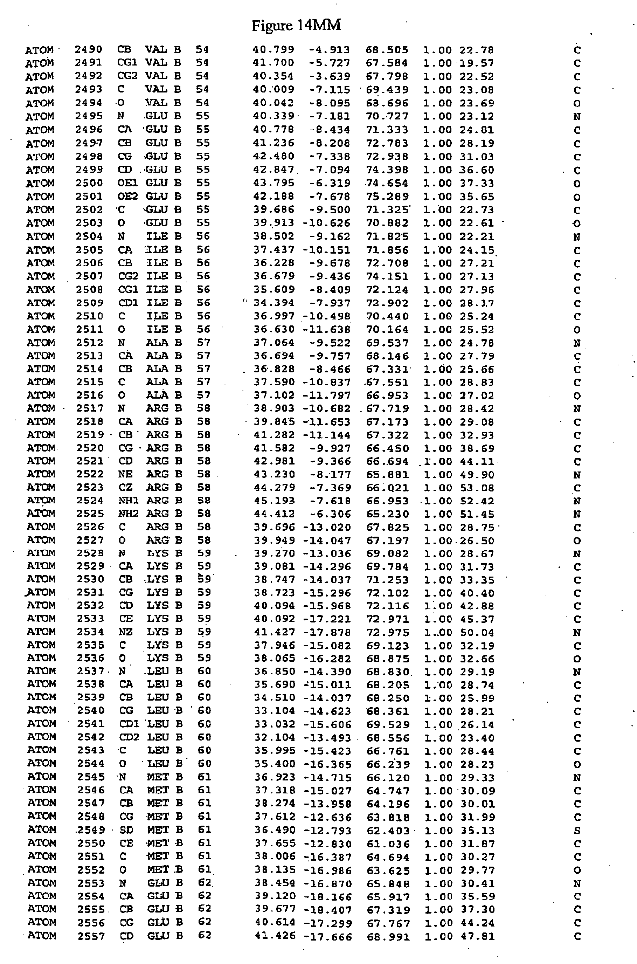

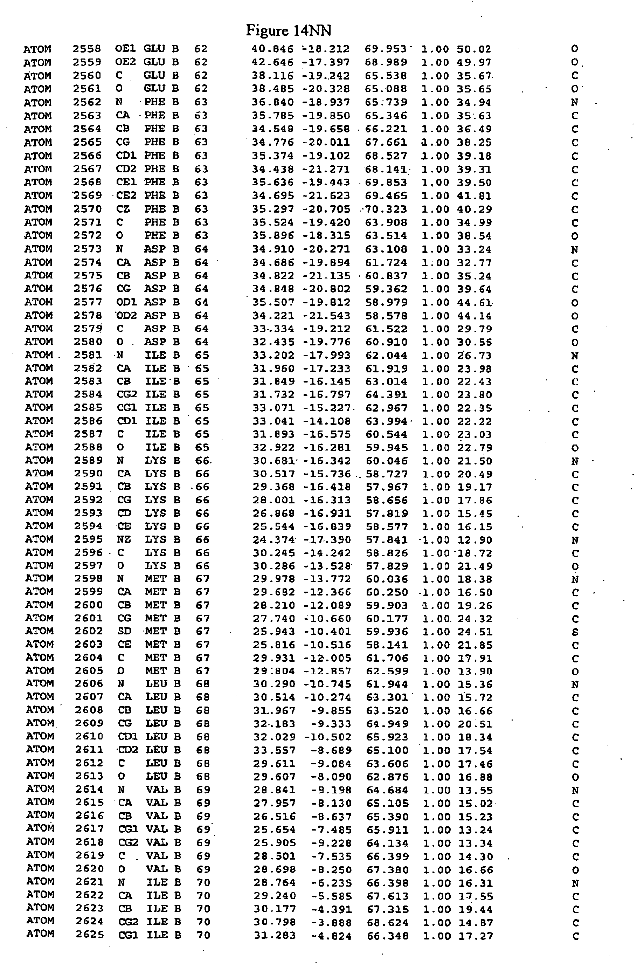

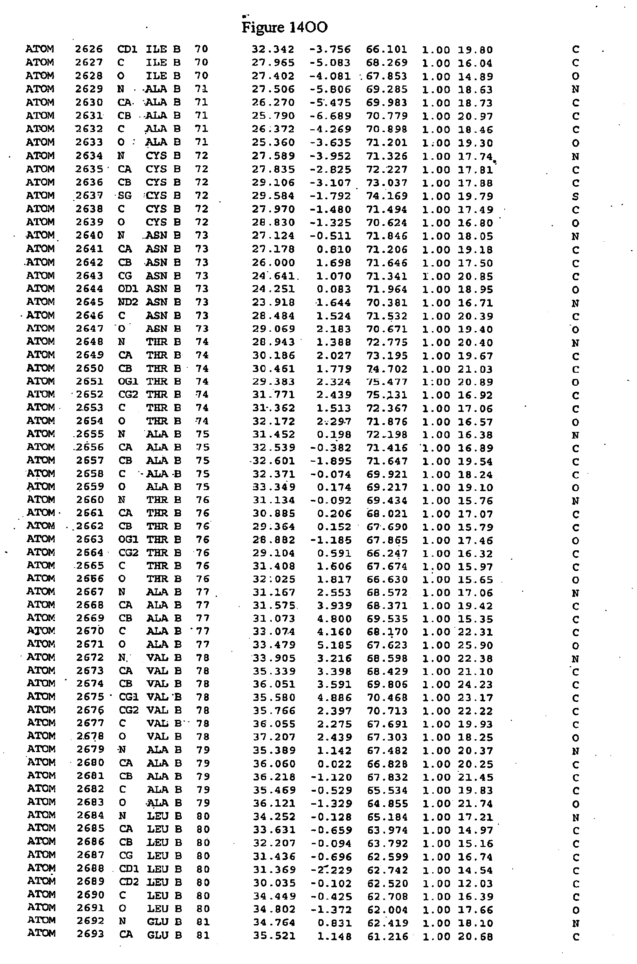

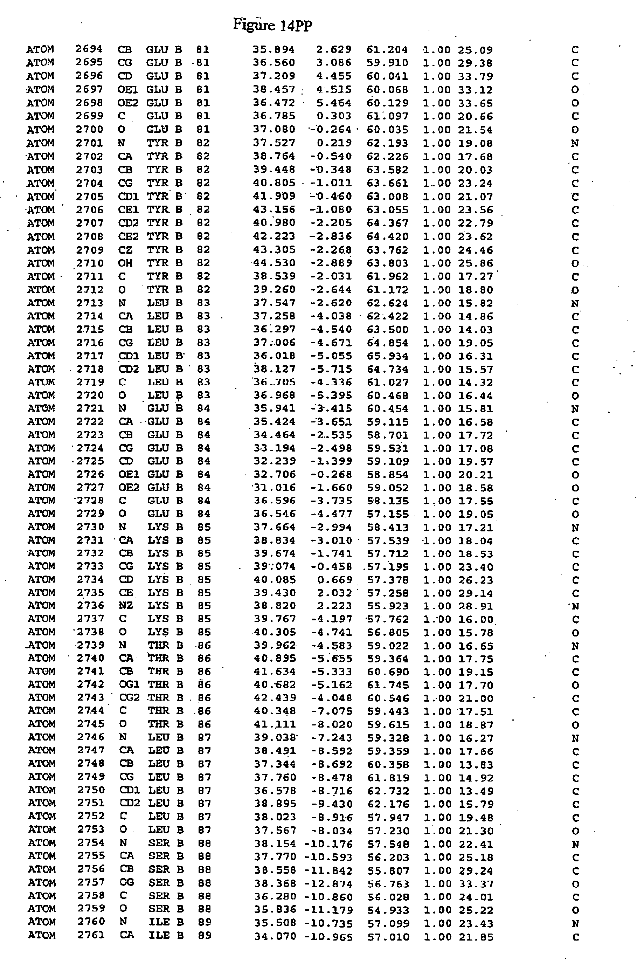

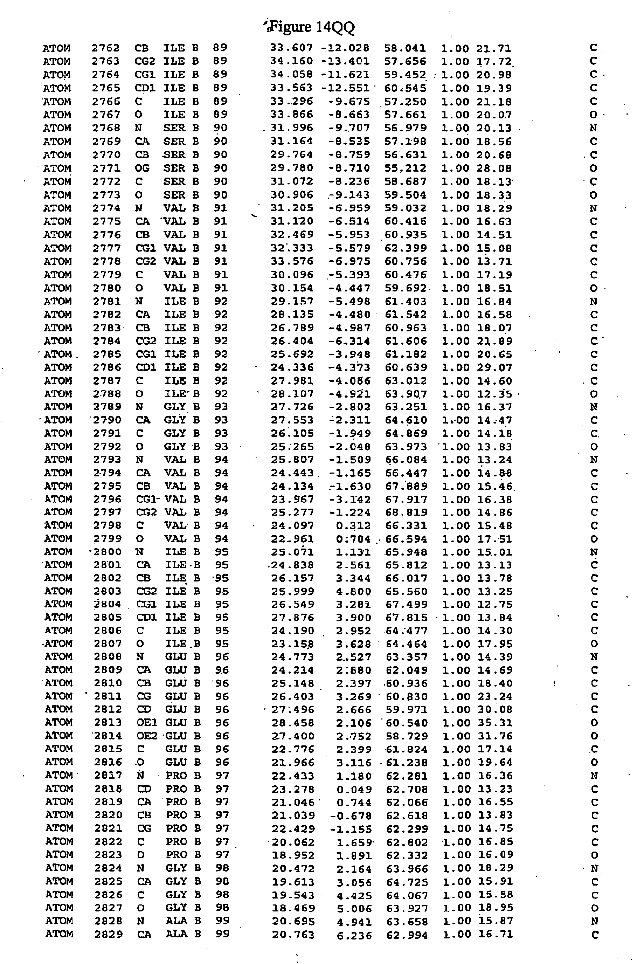

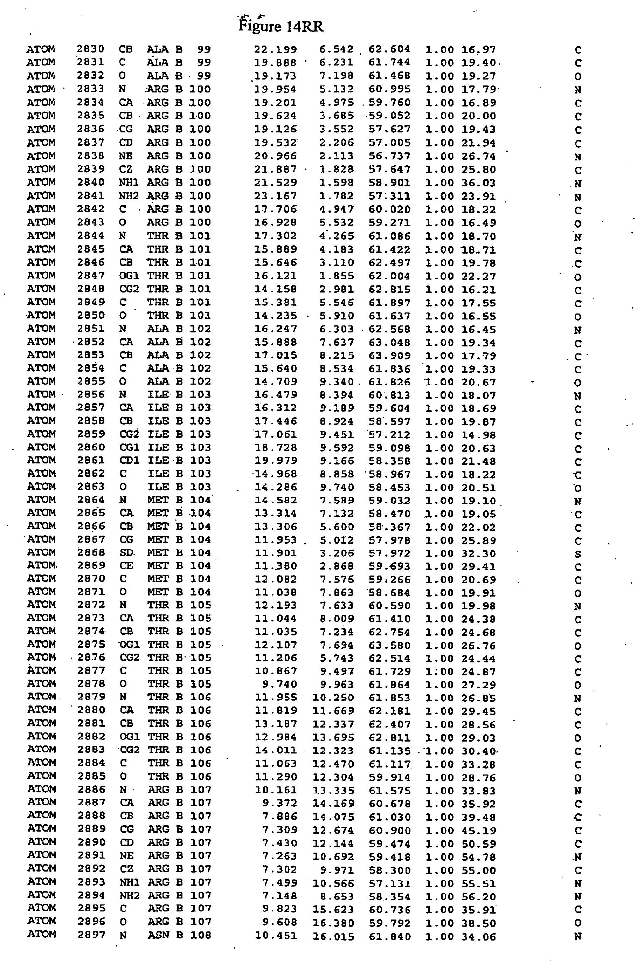

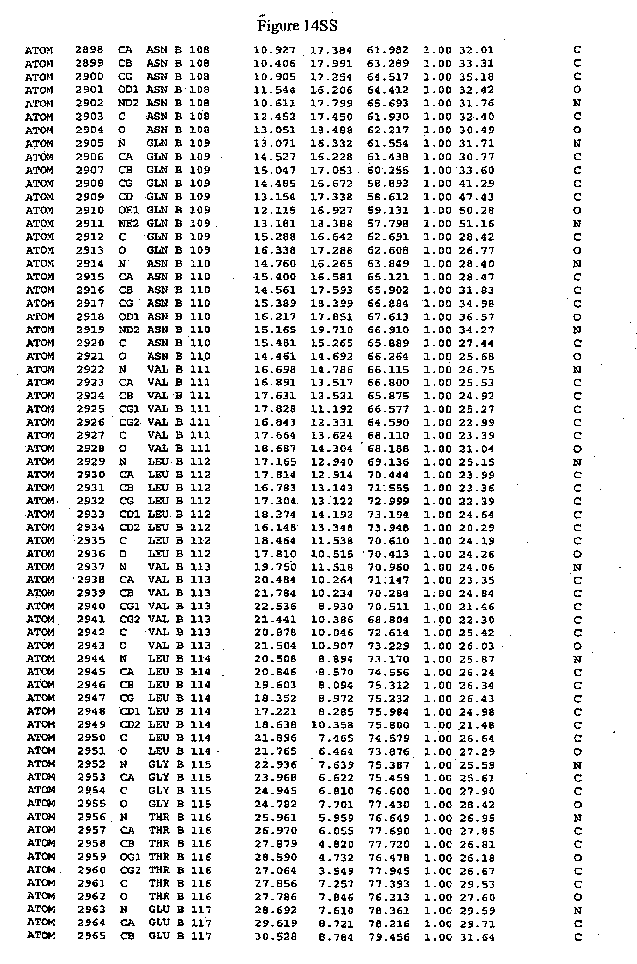

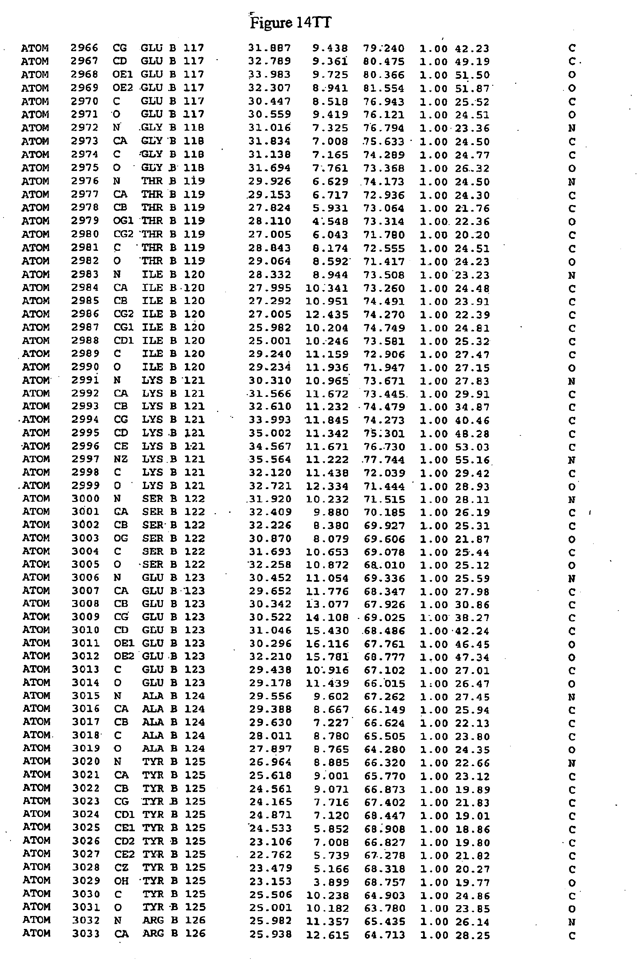

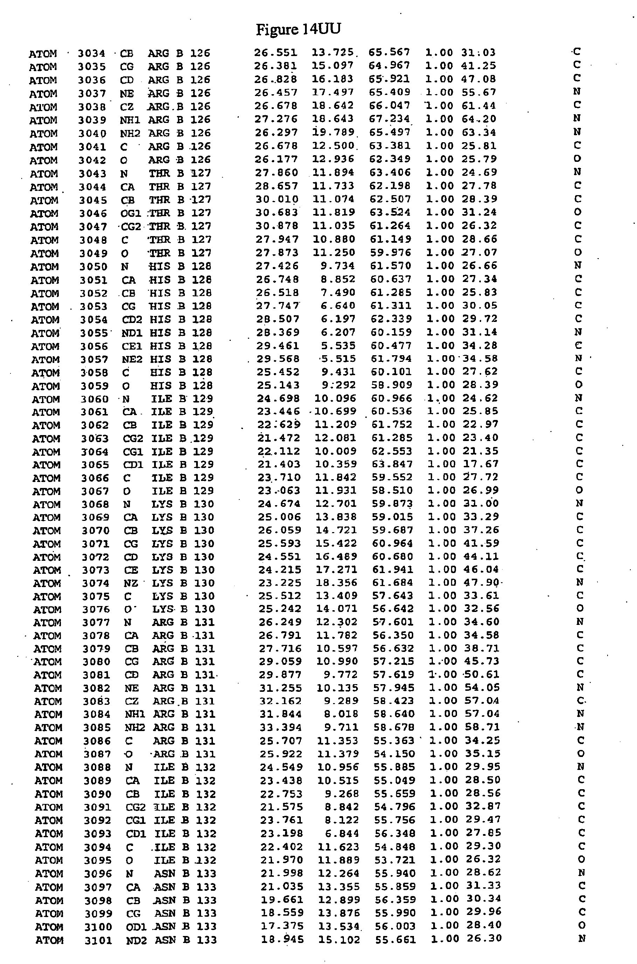

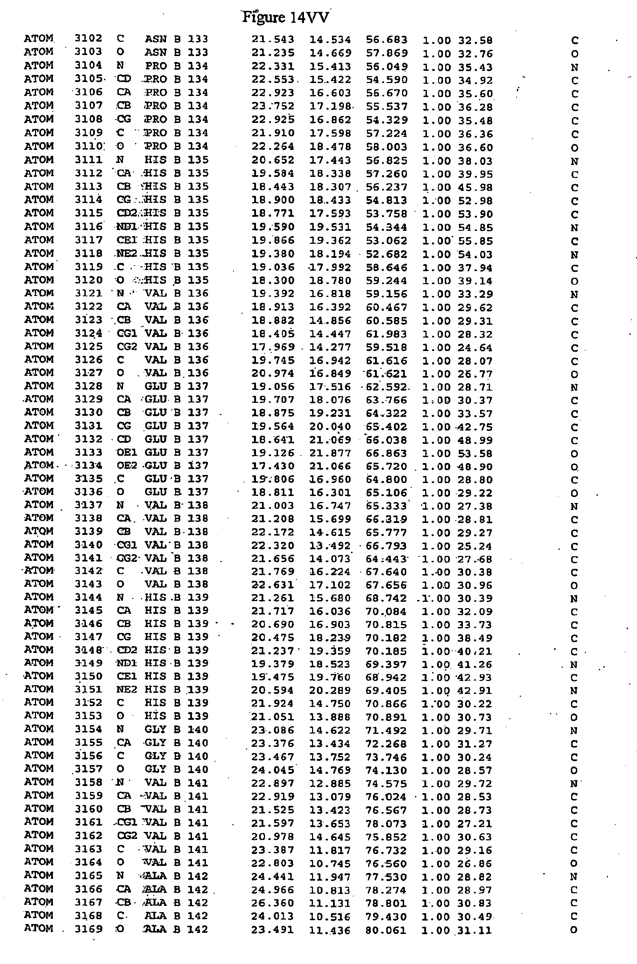

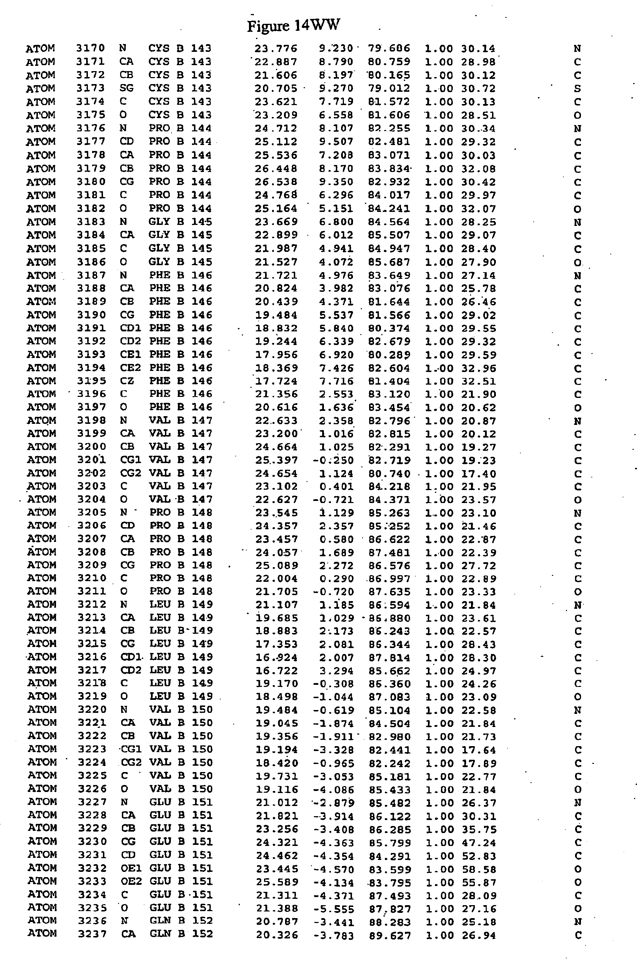

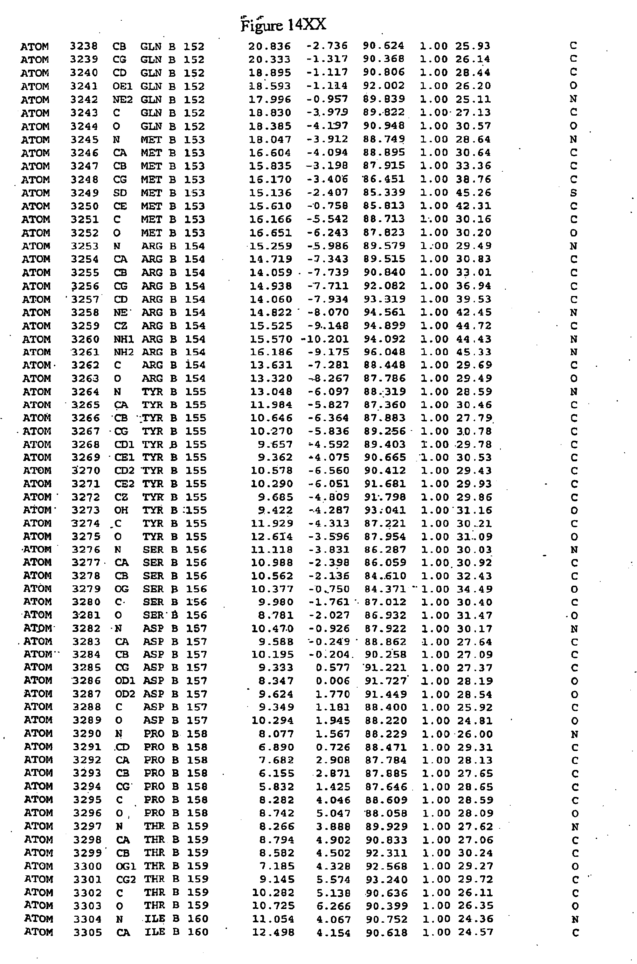

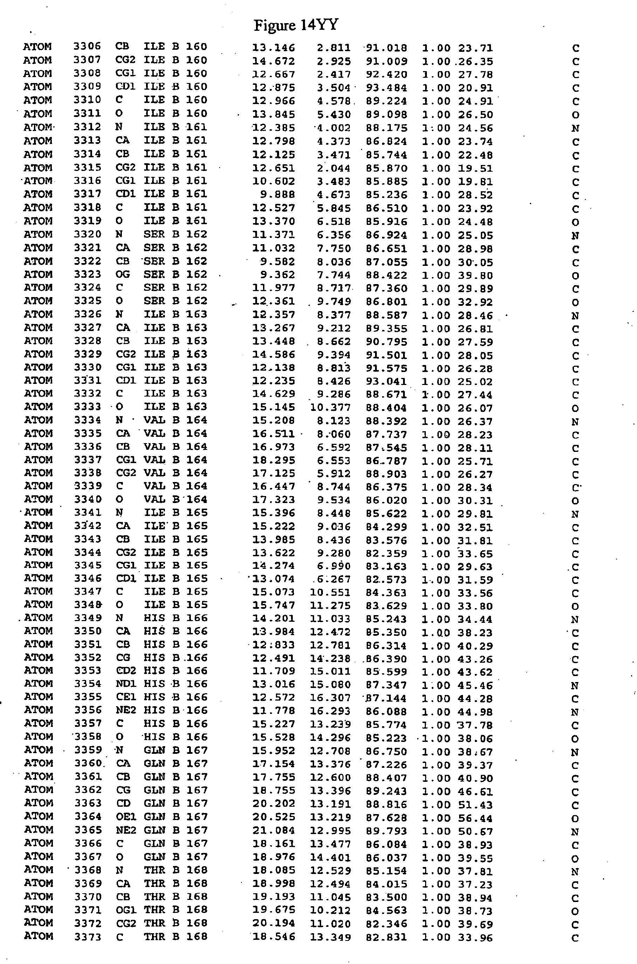

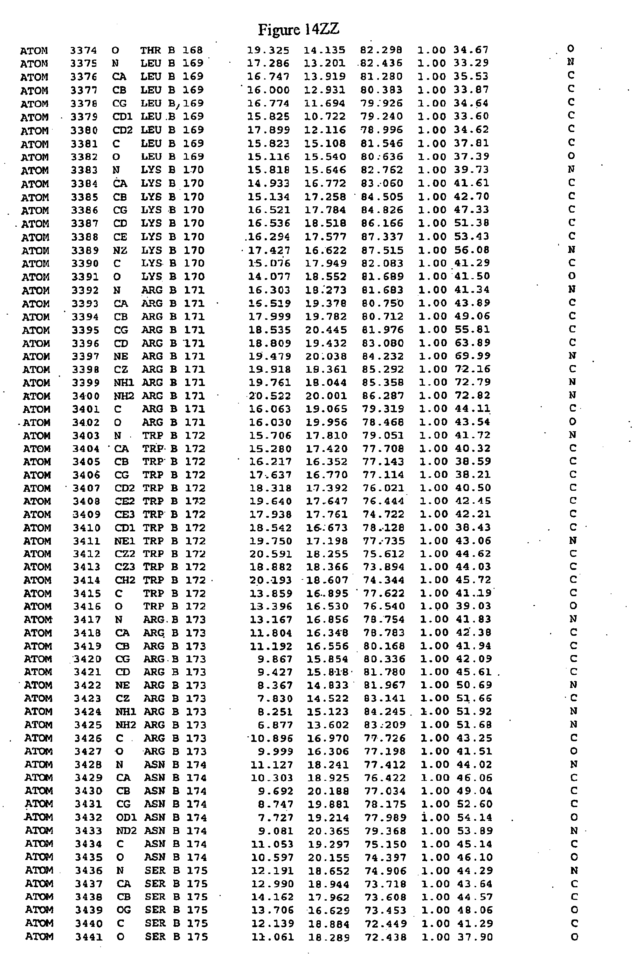

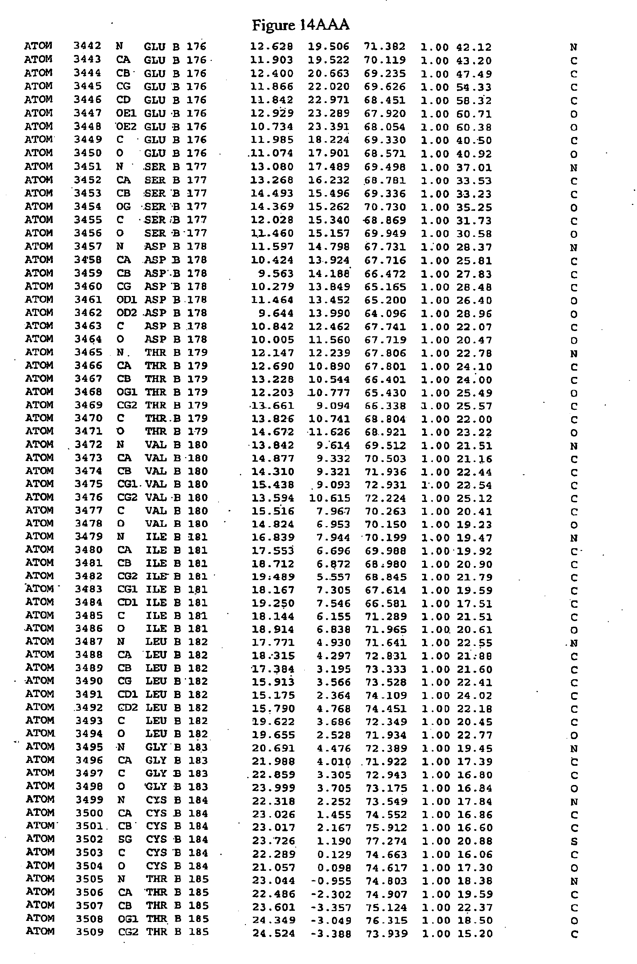

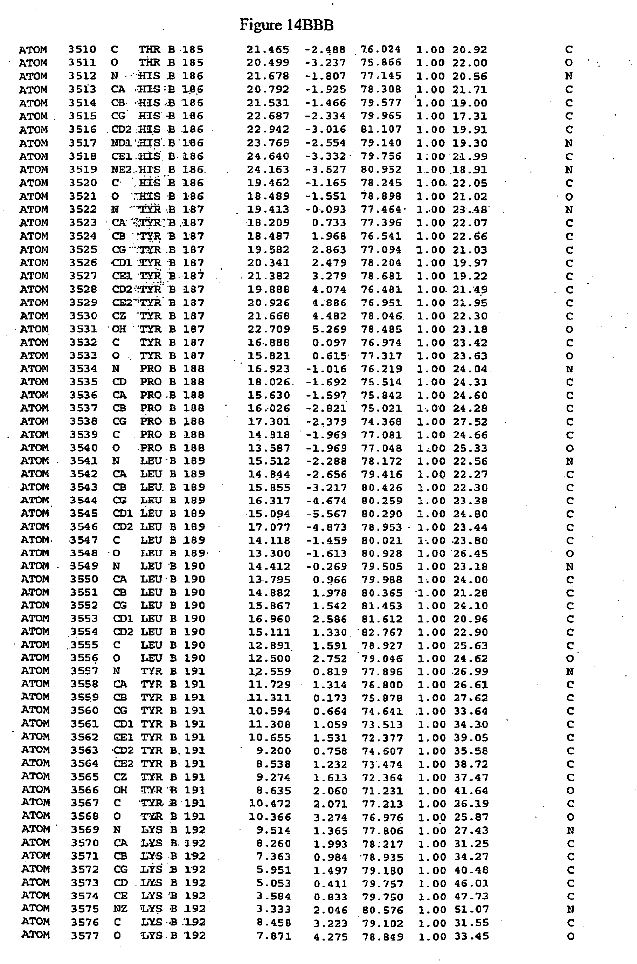

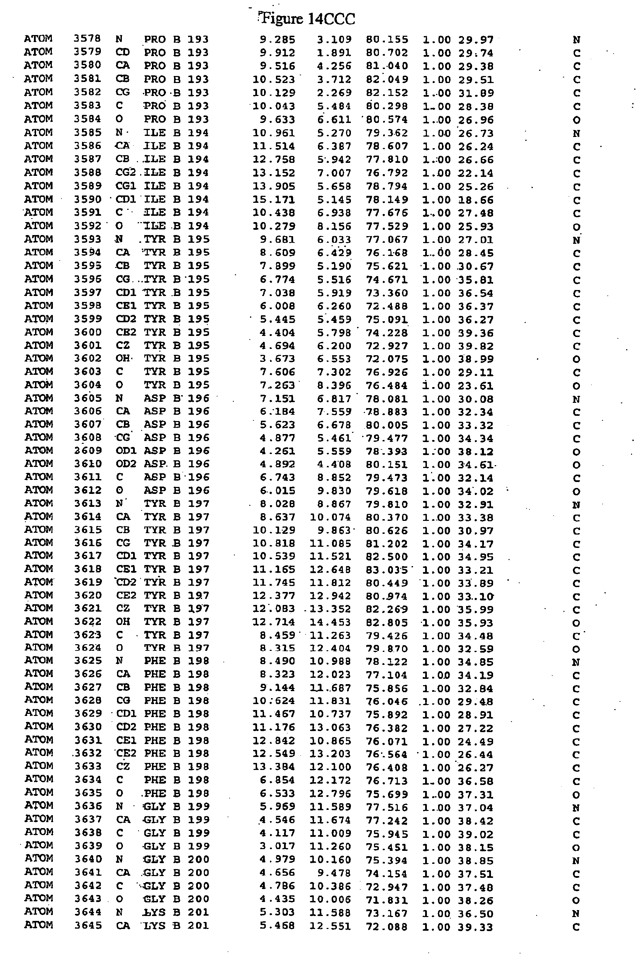

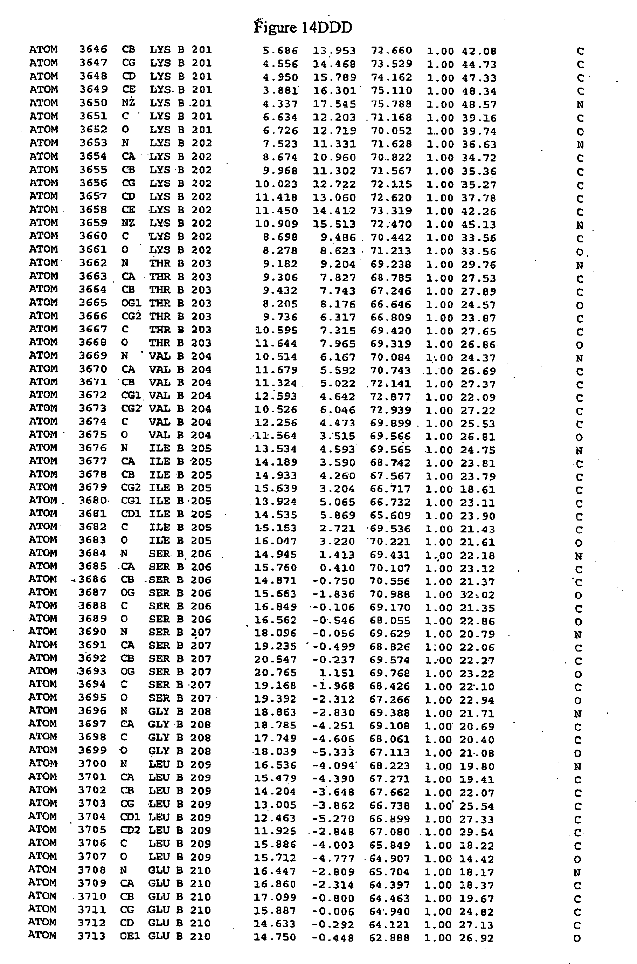

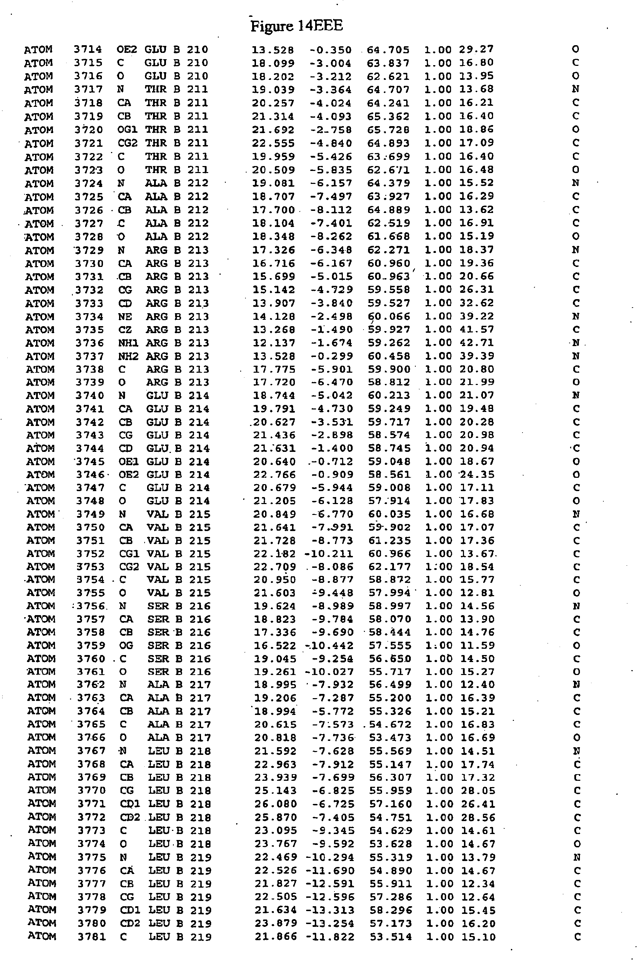

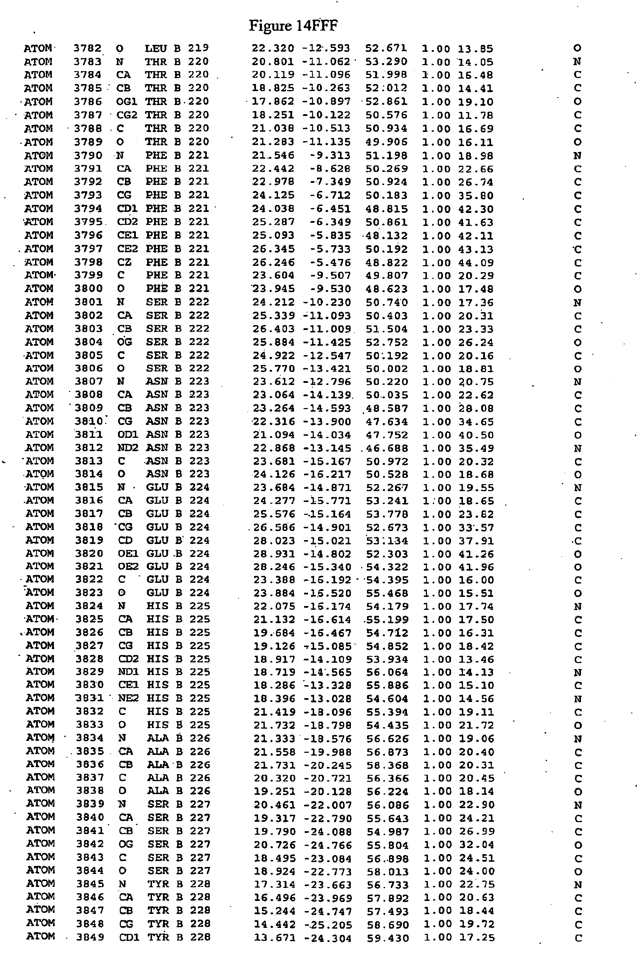

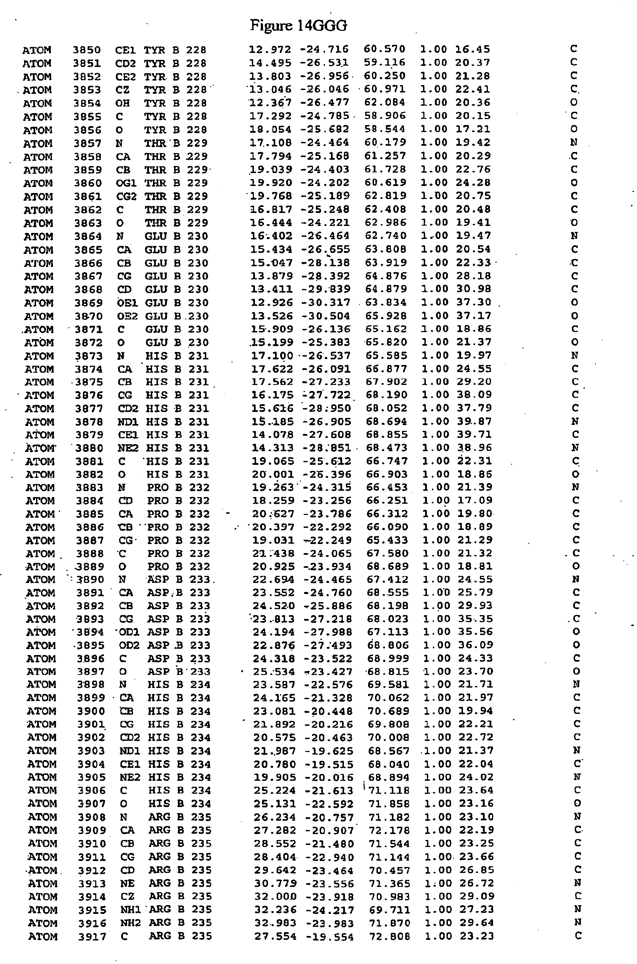

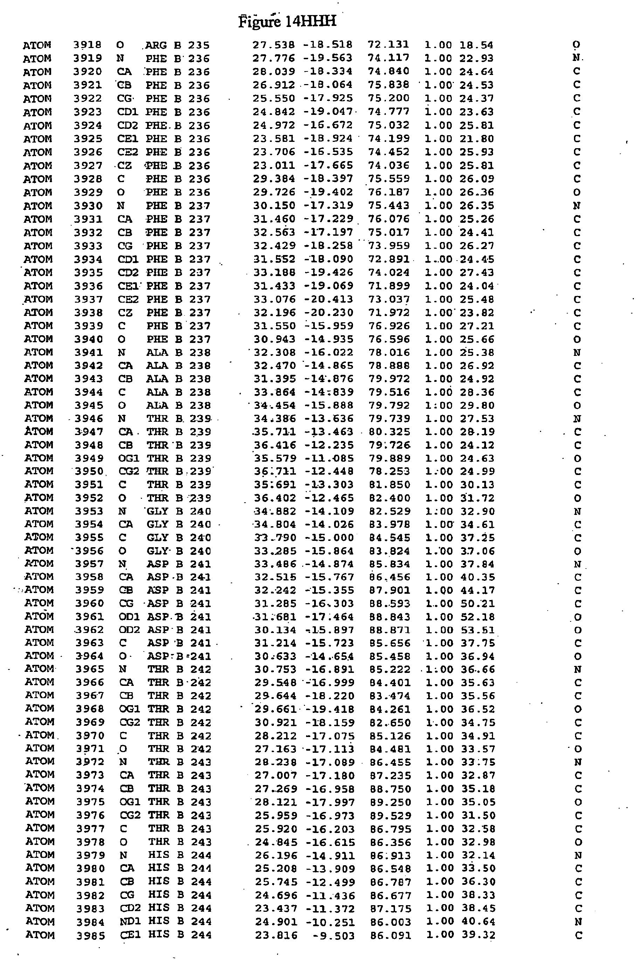

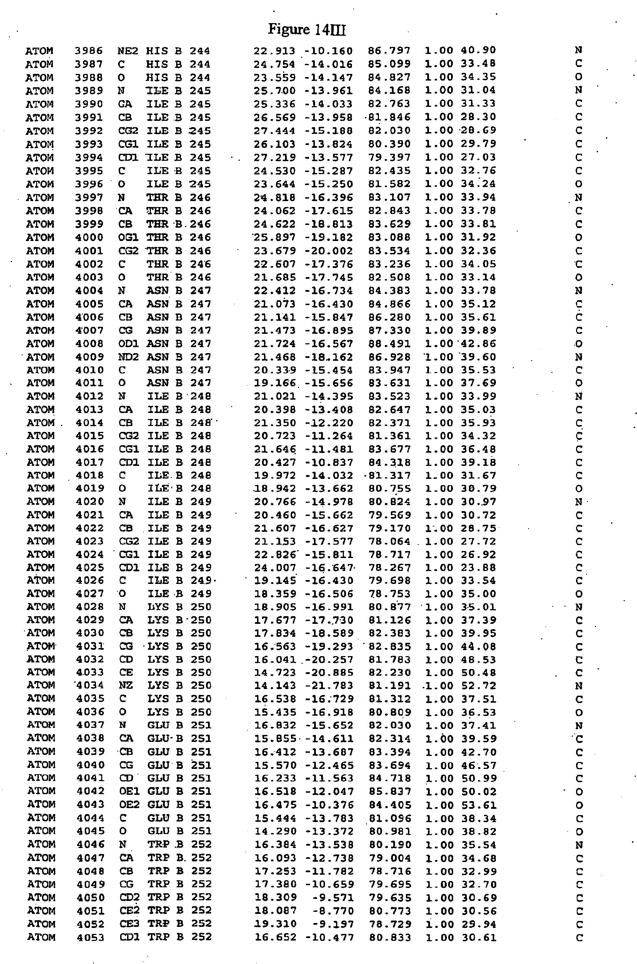

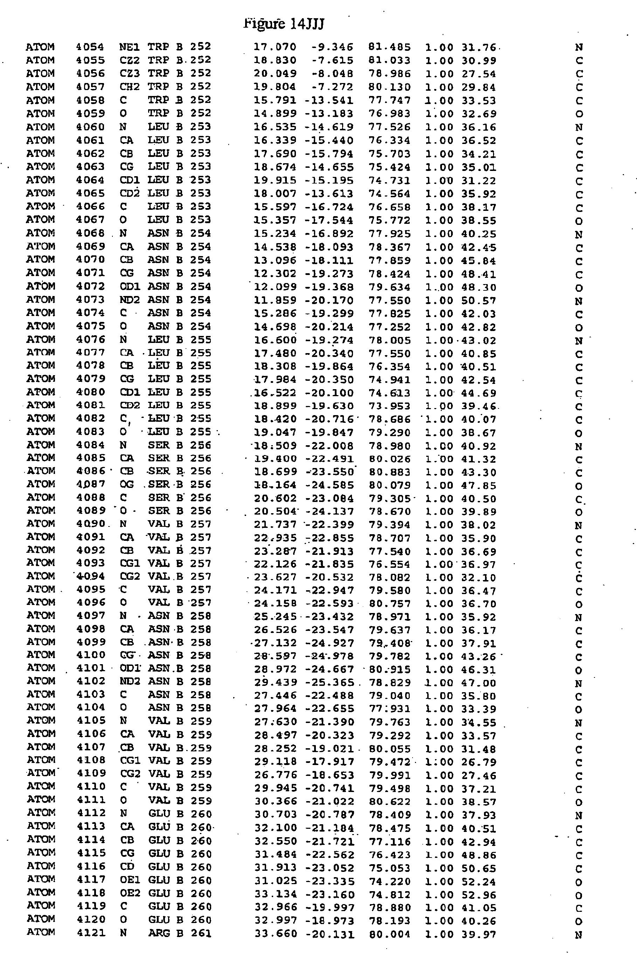

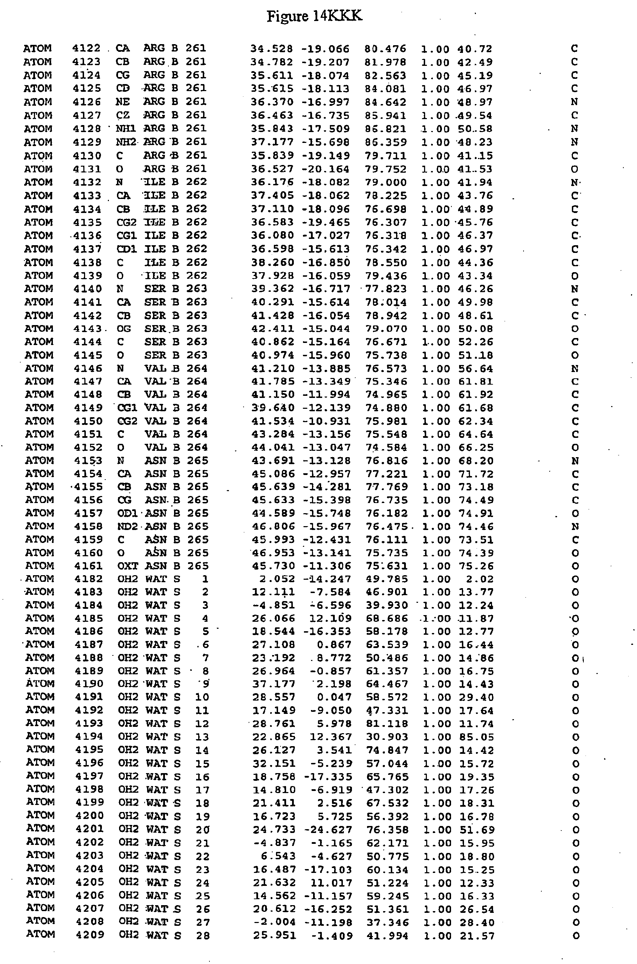

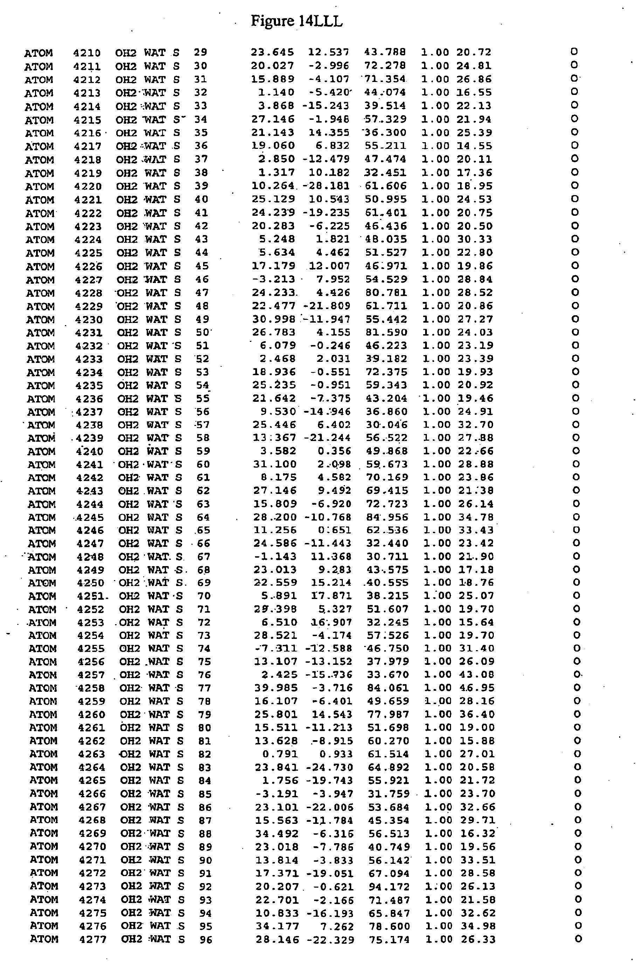

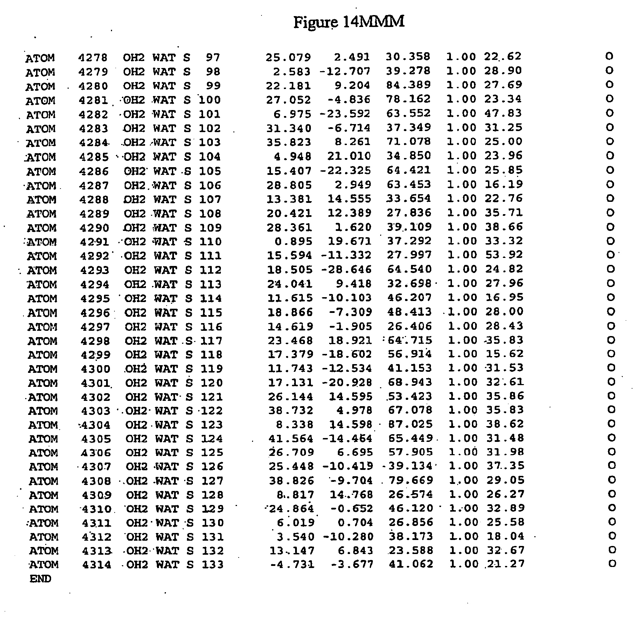

In one embodiment, we provide crystallized *S*. *aureus* MurI characterized by the structural coordinates depicted in Figure 14.

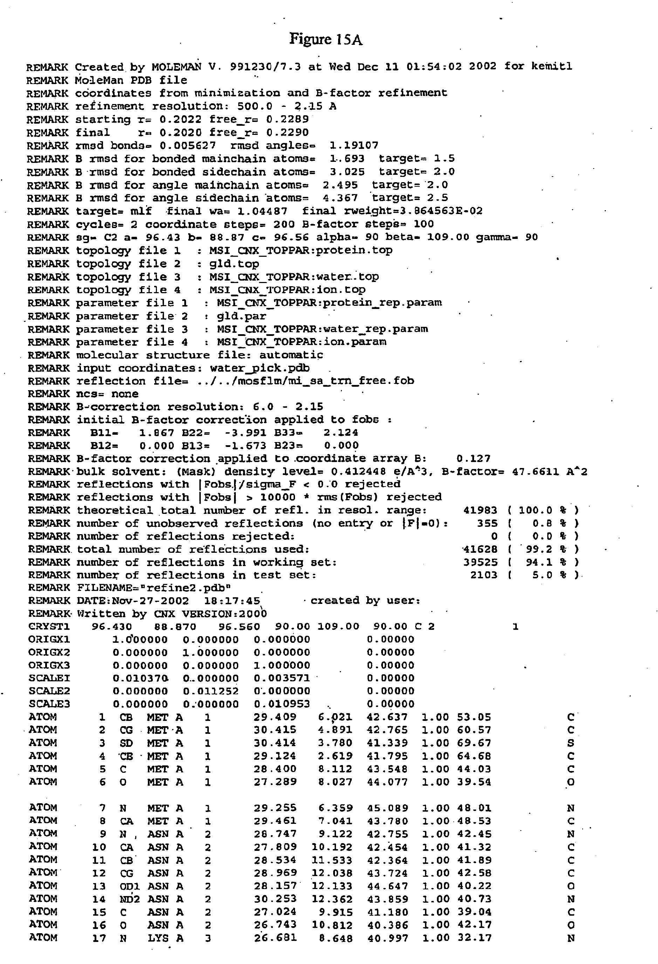

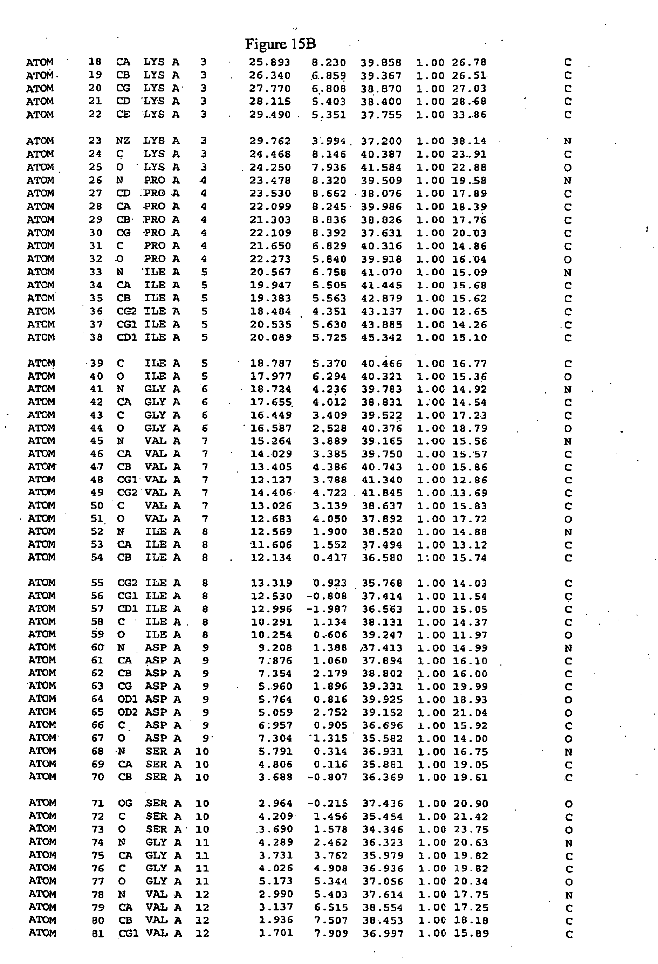

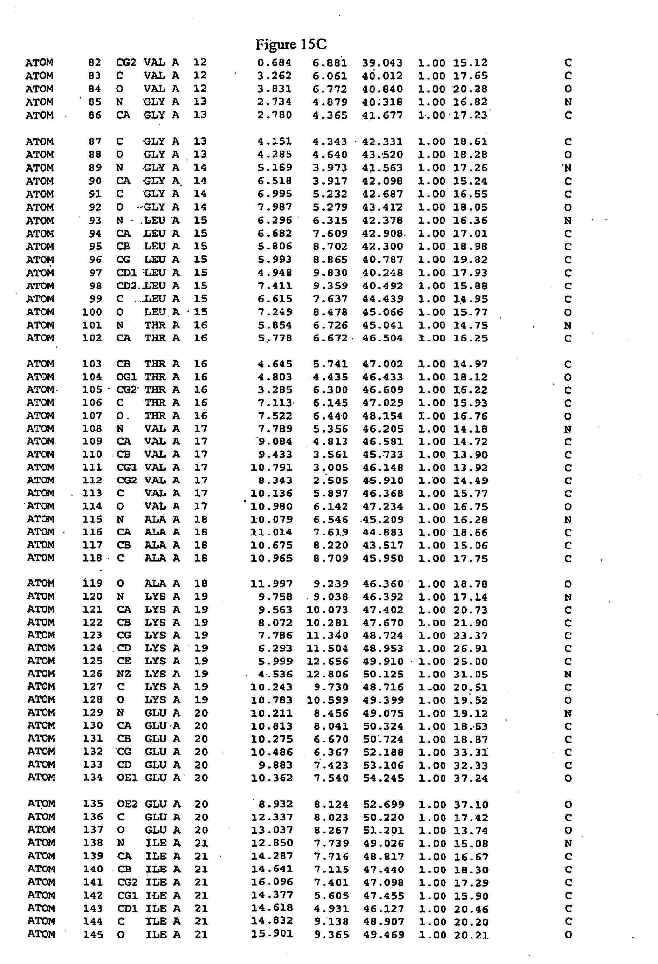

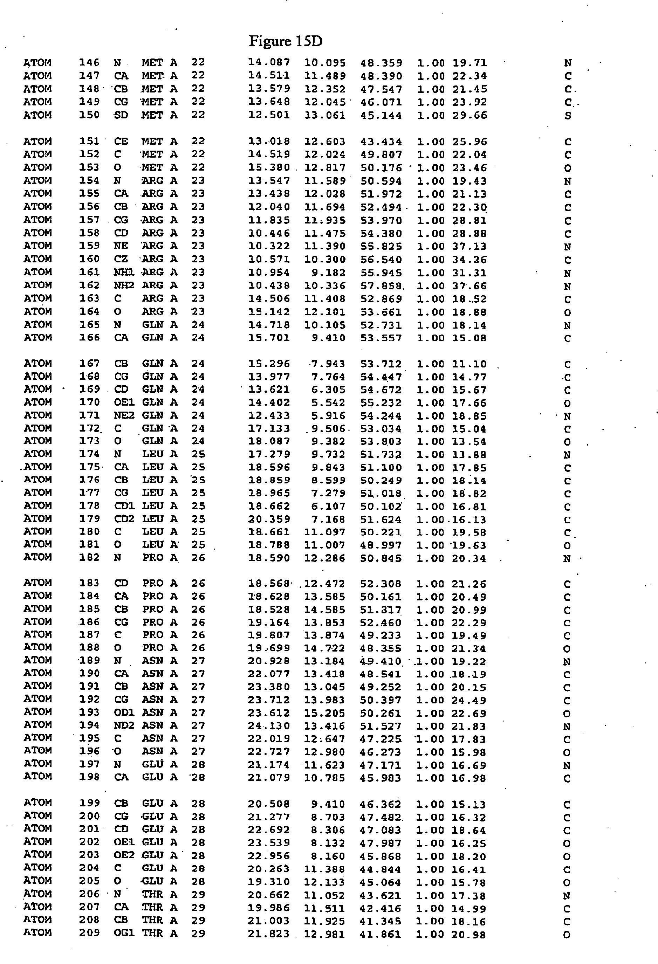

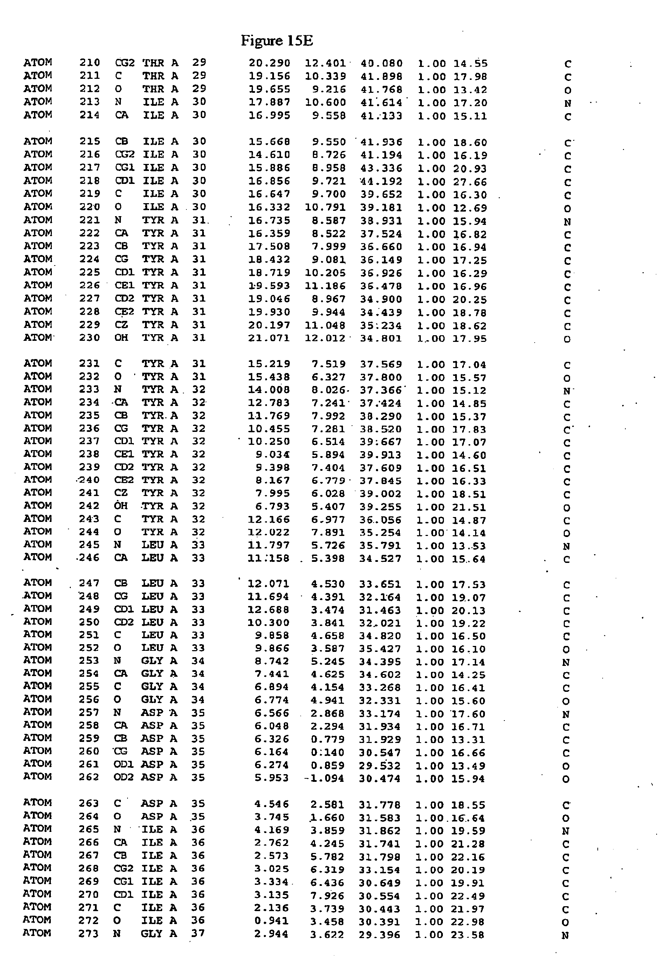

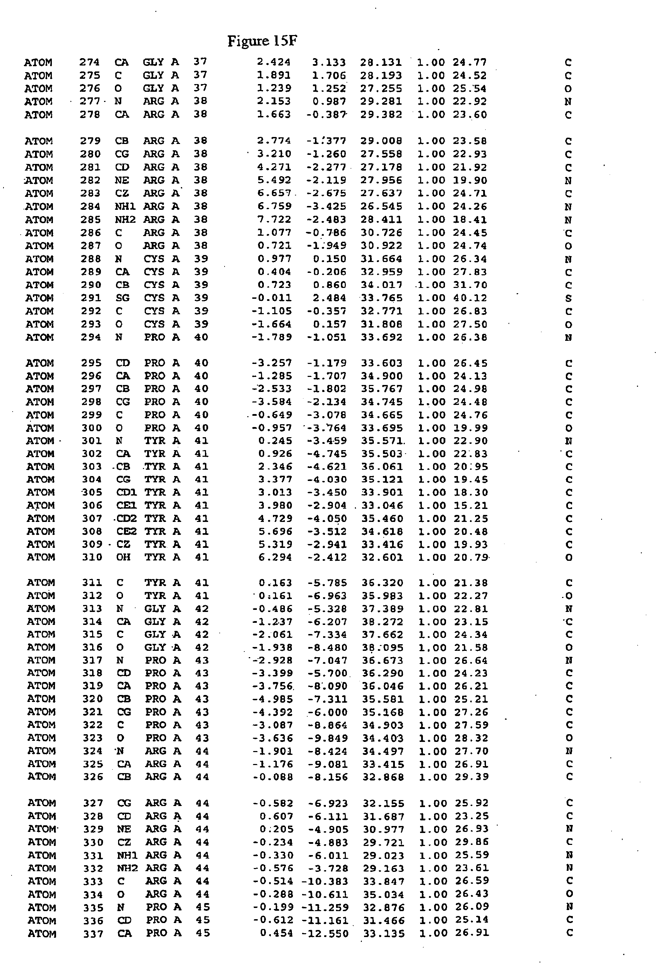

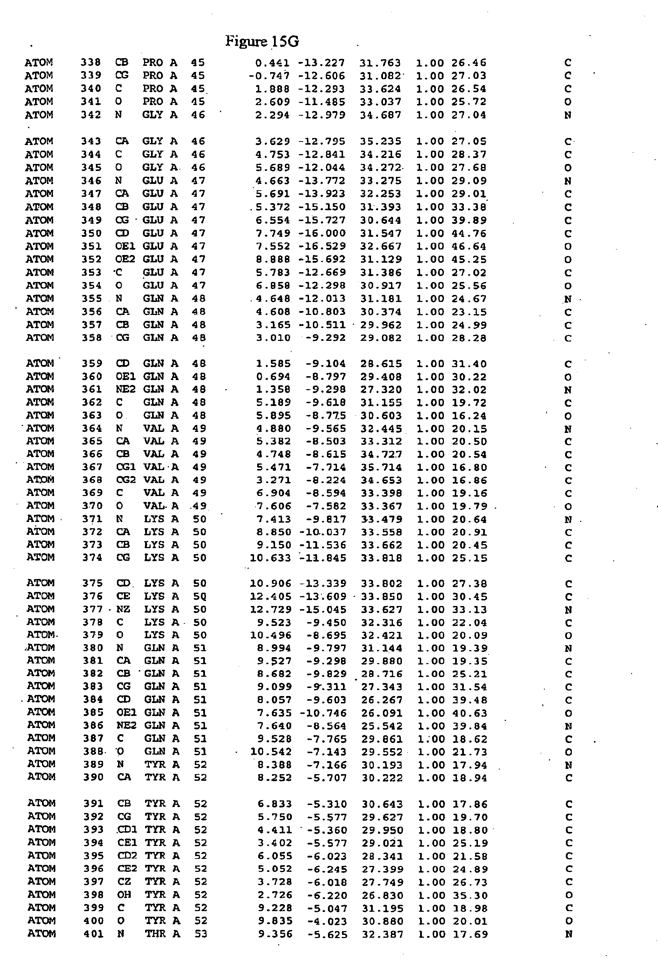

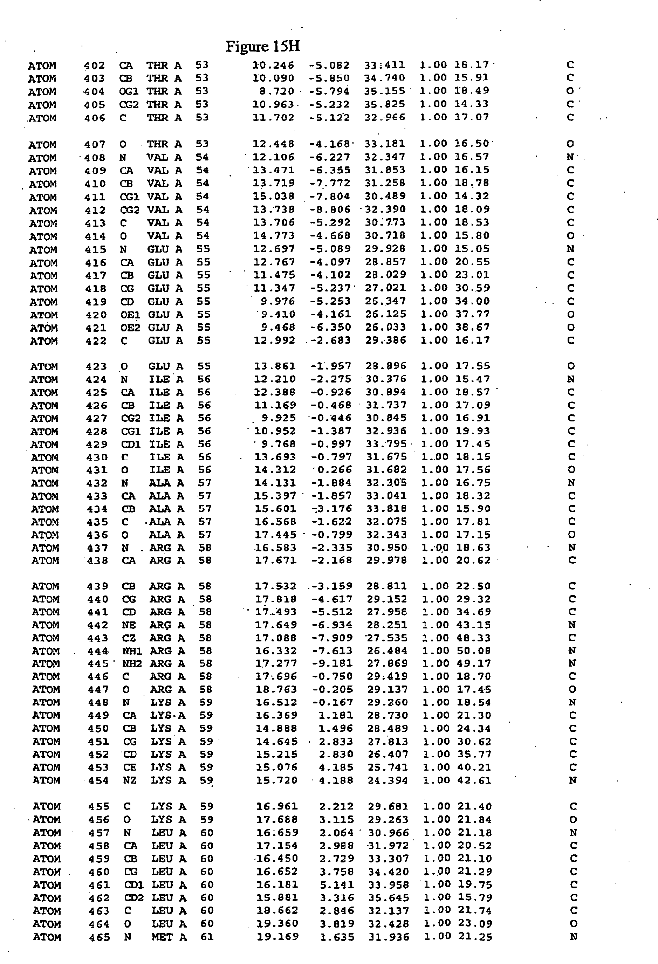

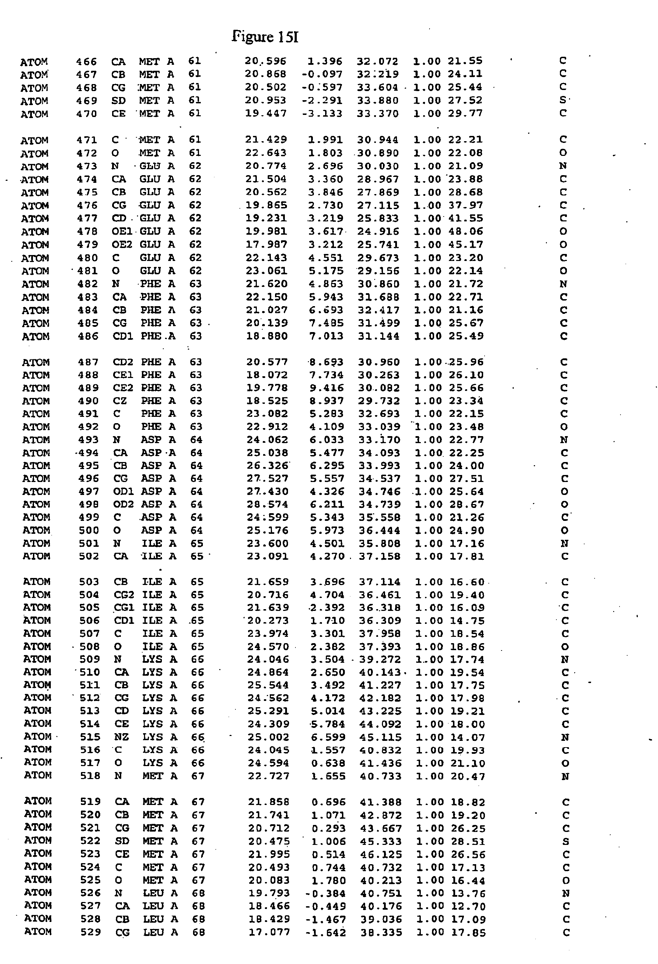

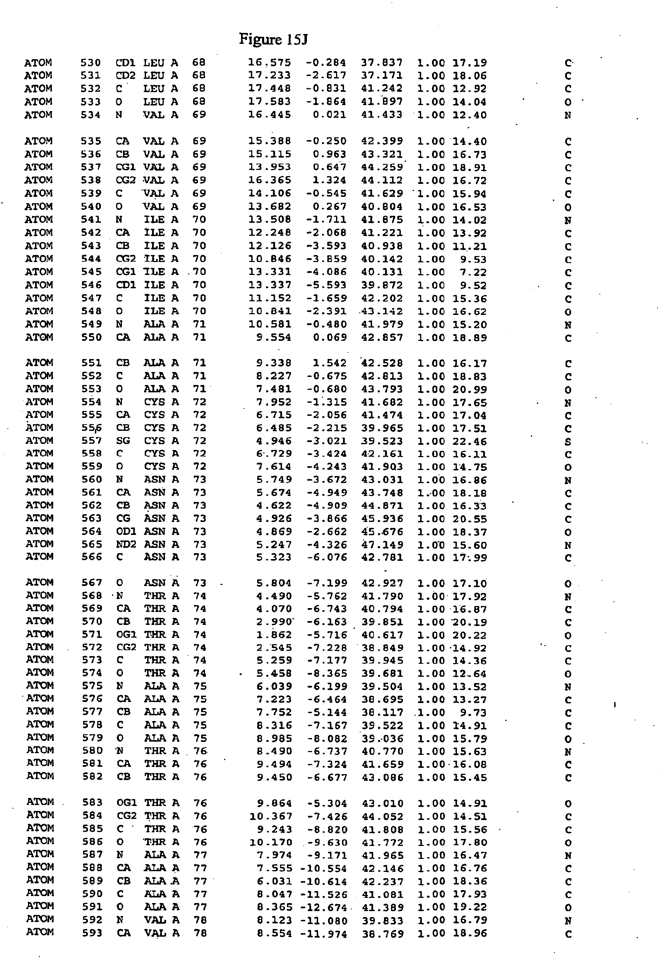

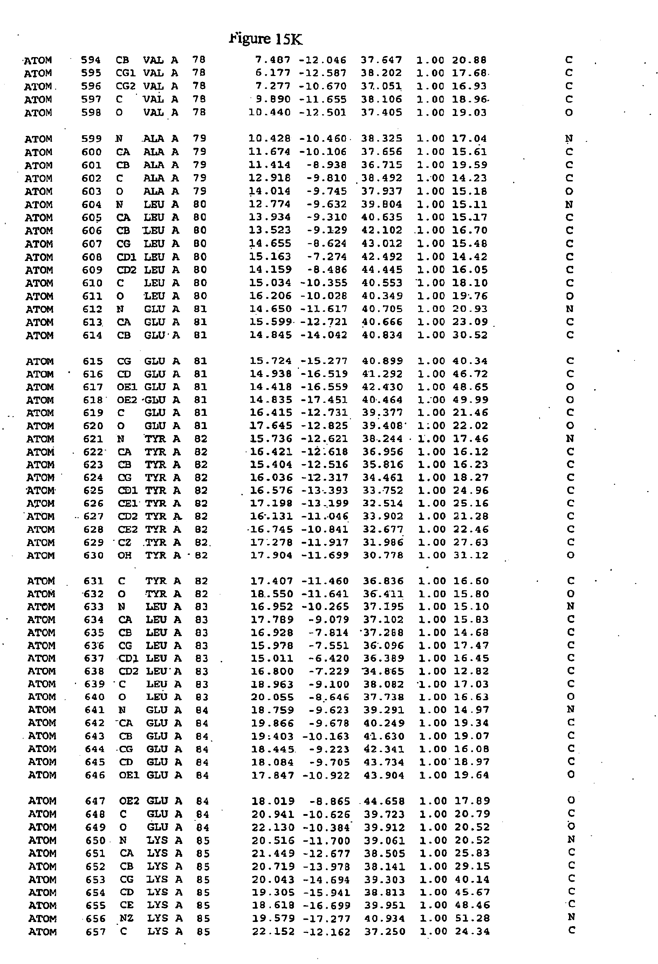

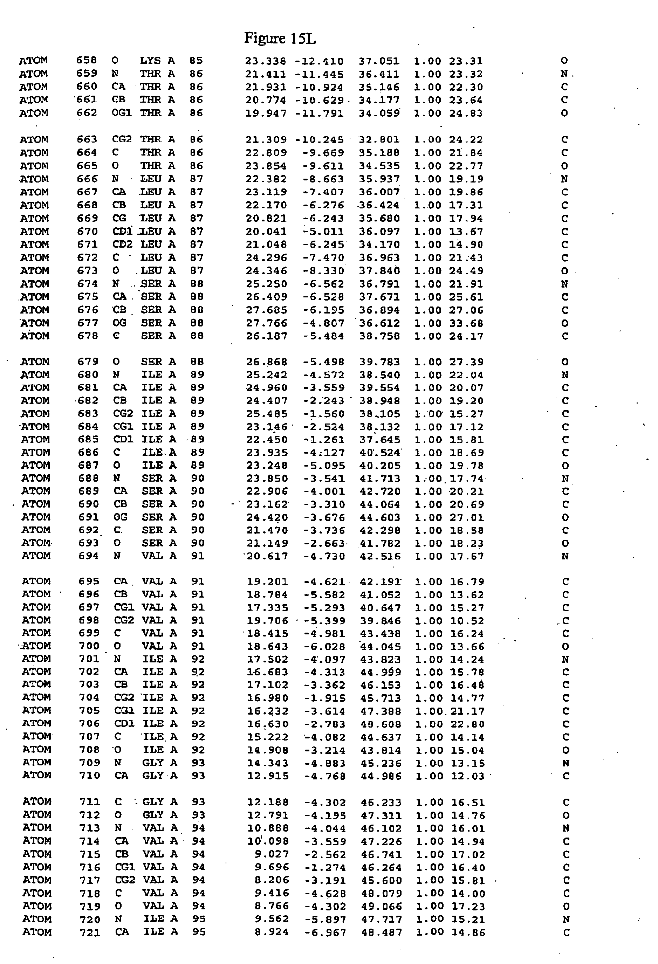

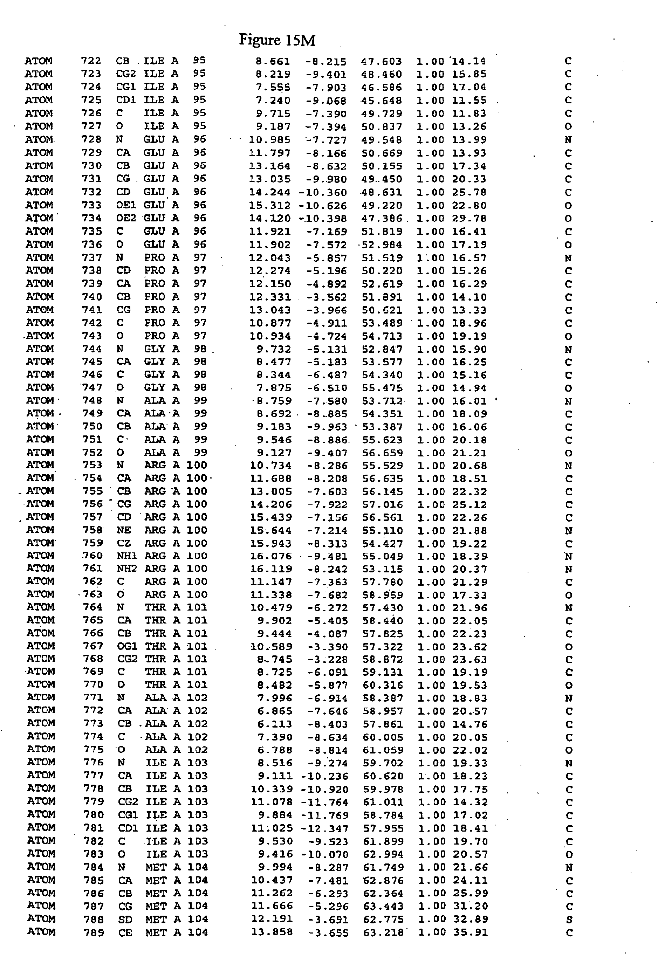

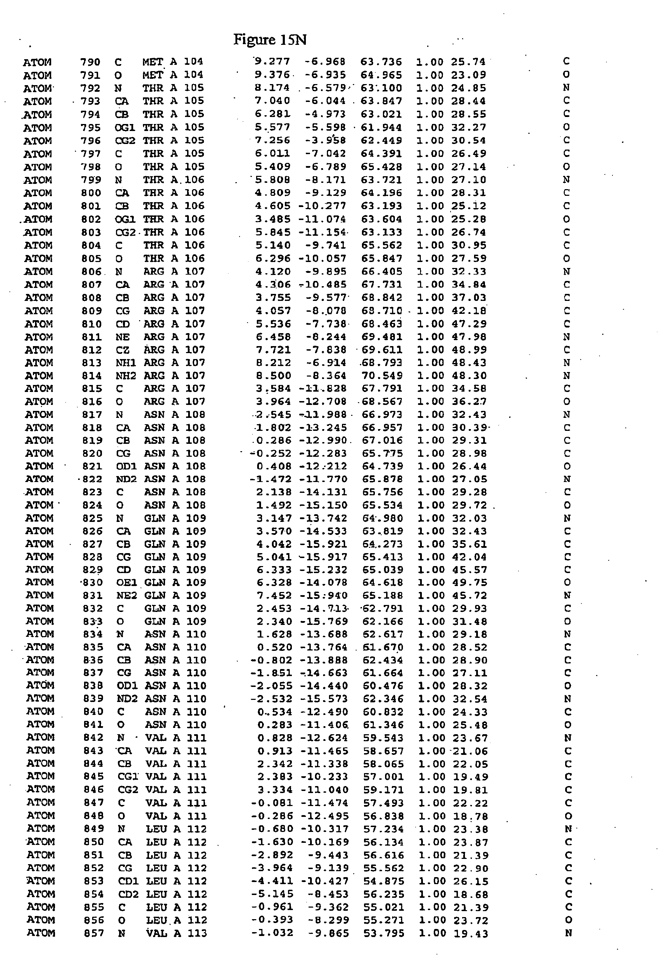

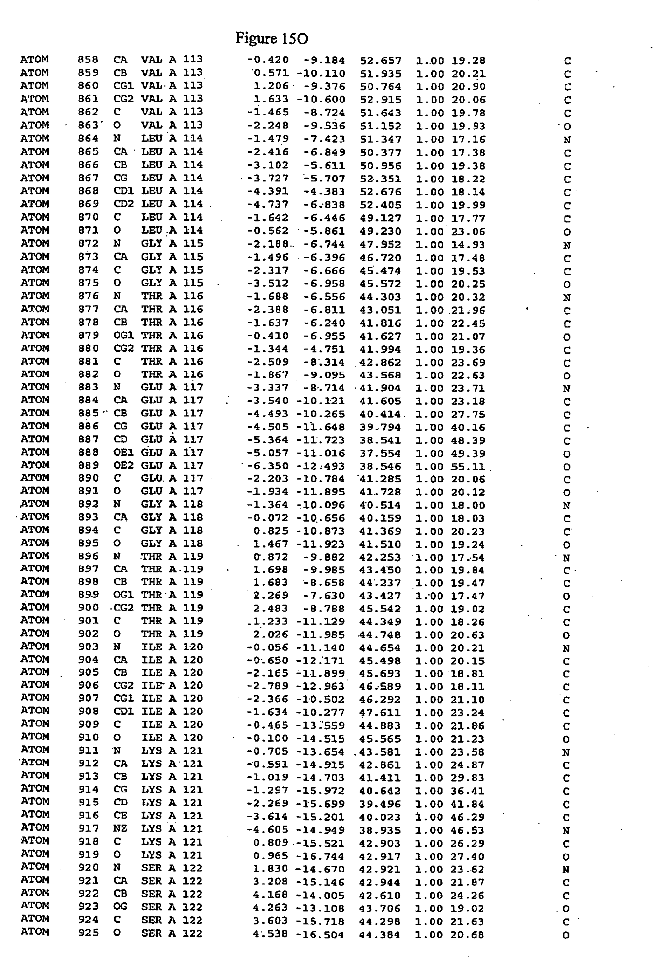

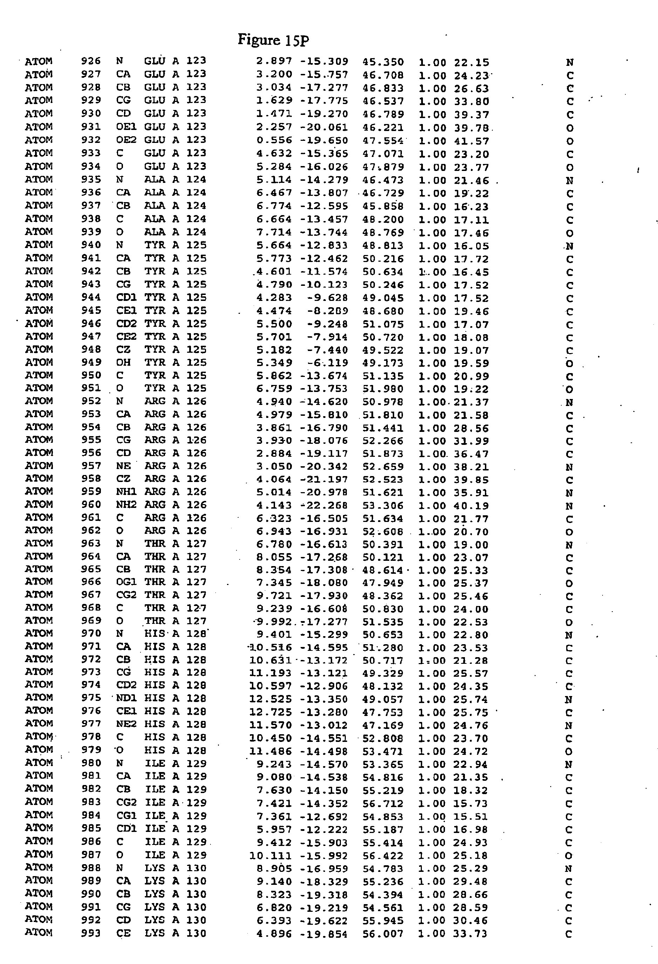

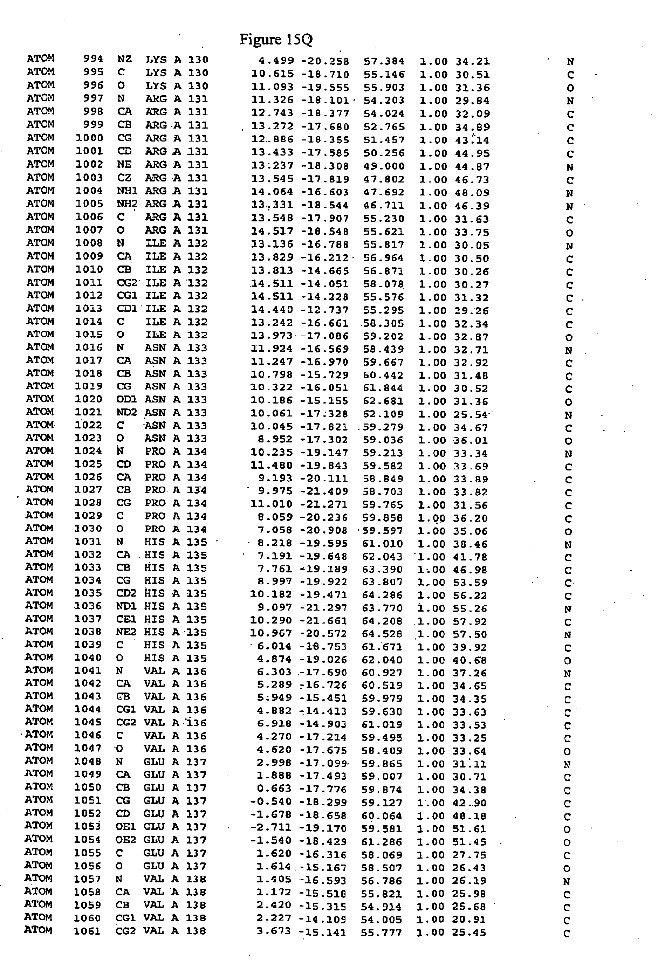

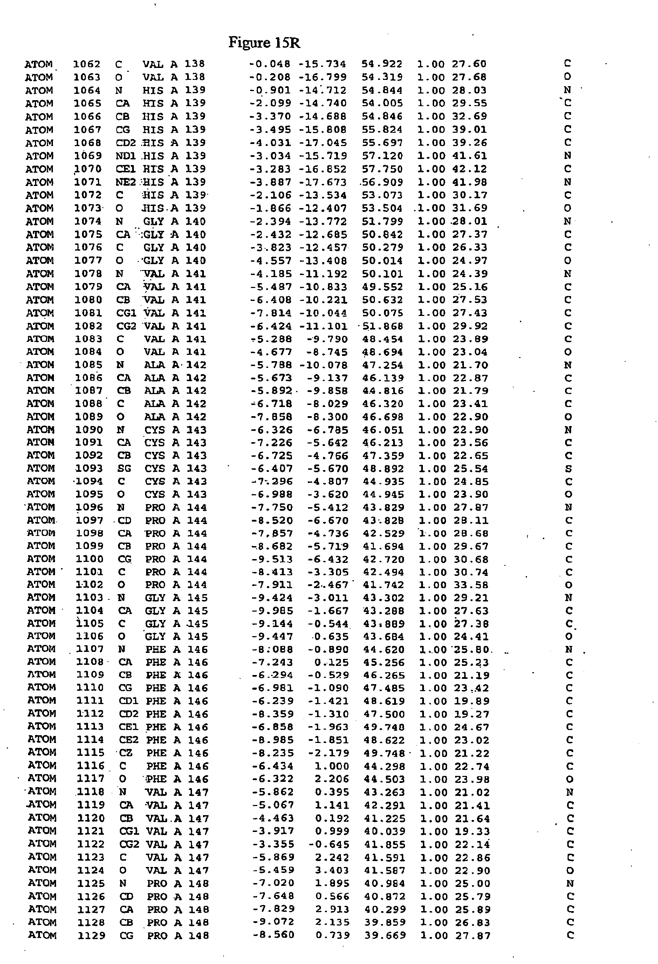

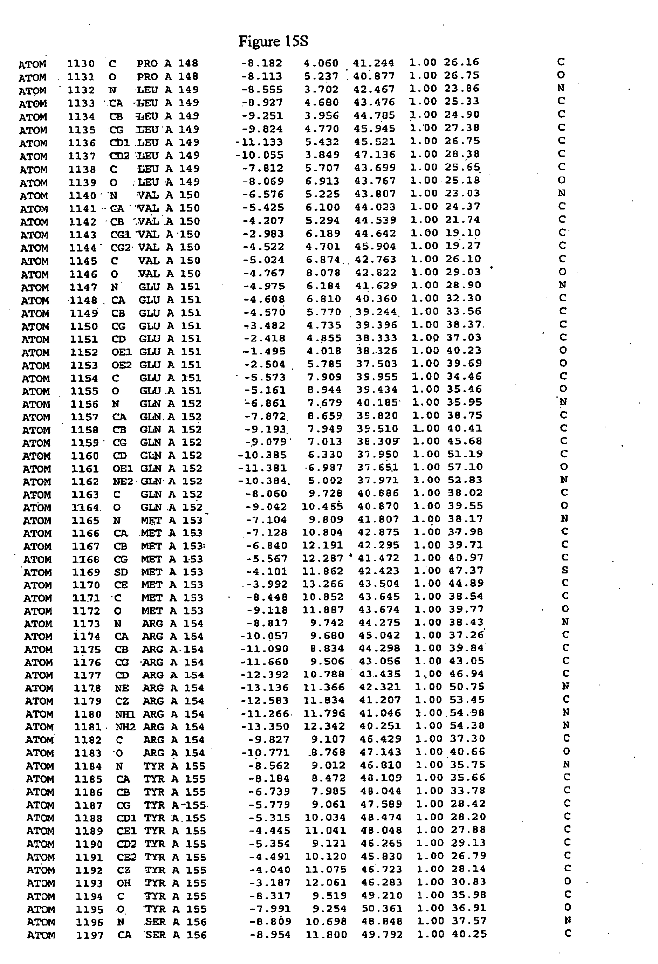

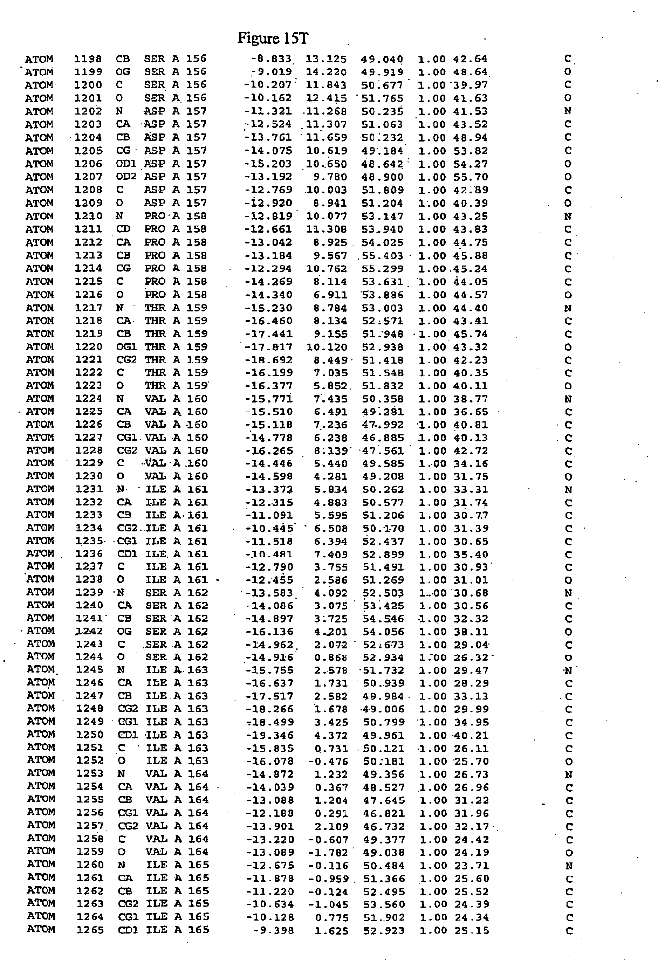

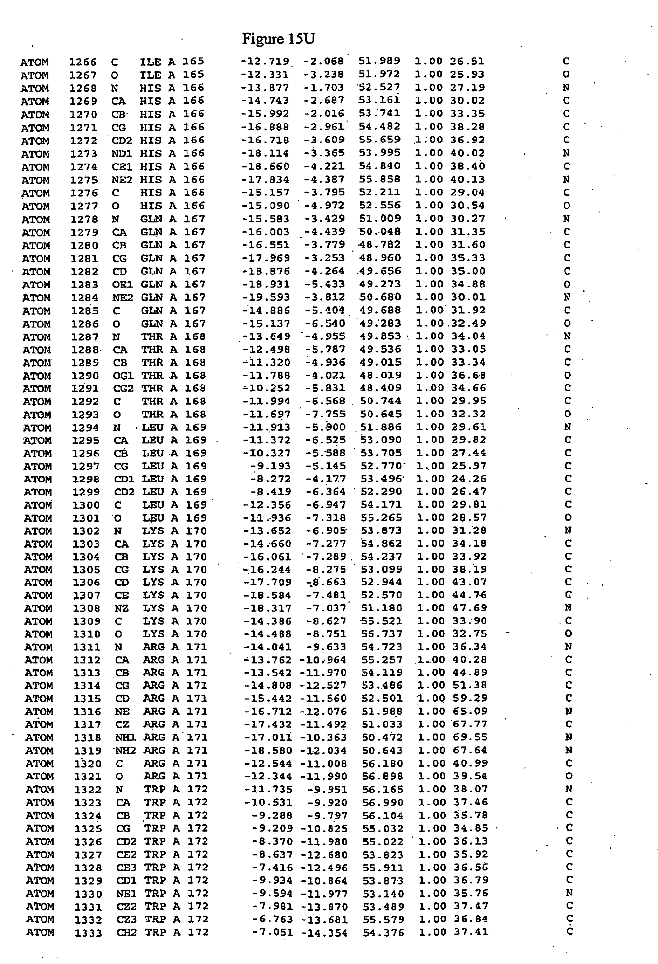

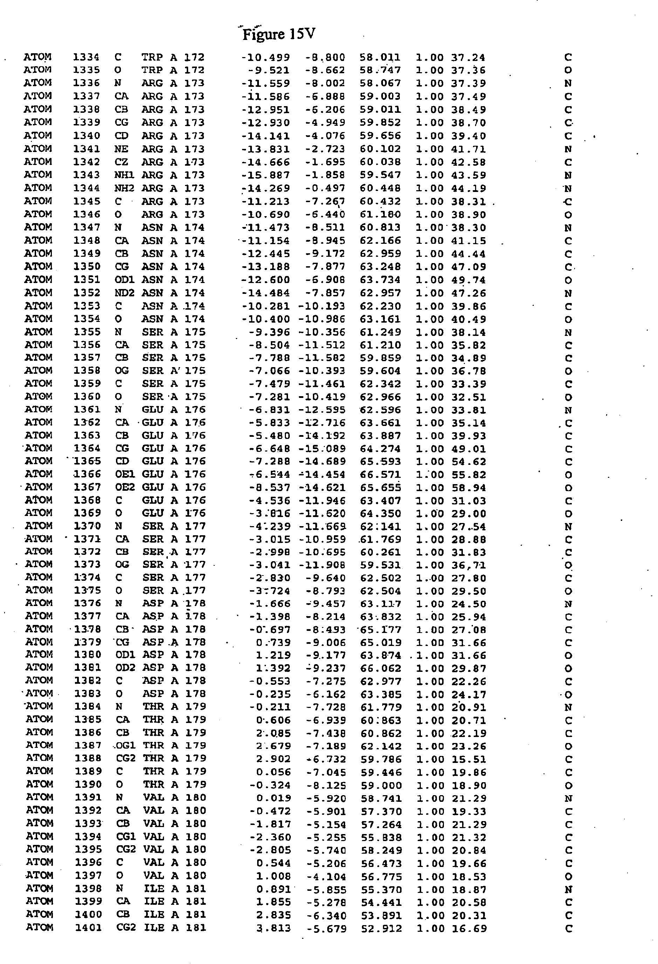

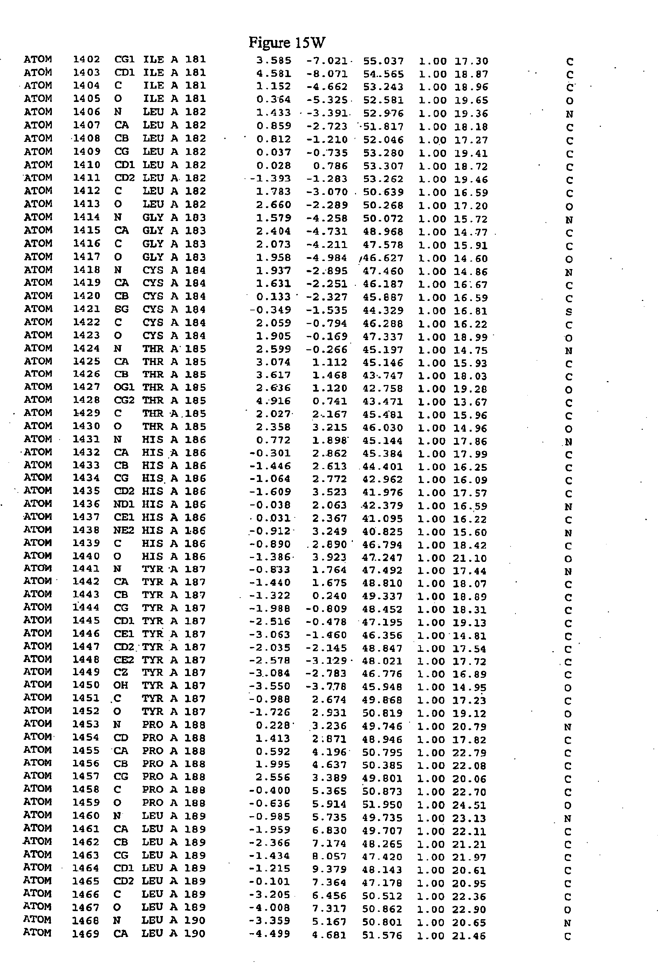

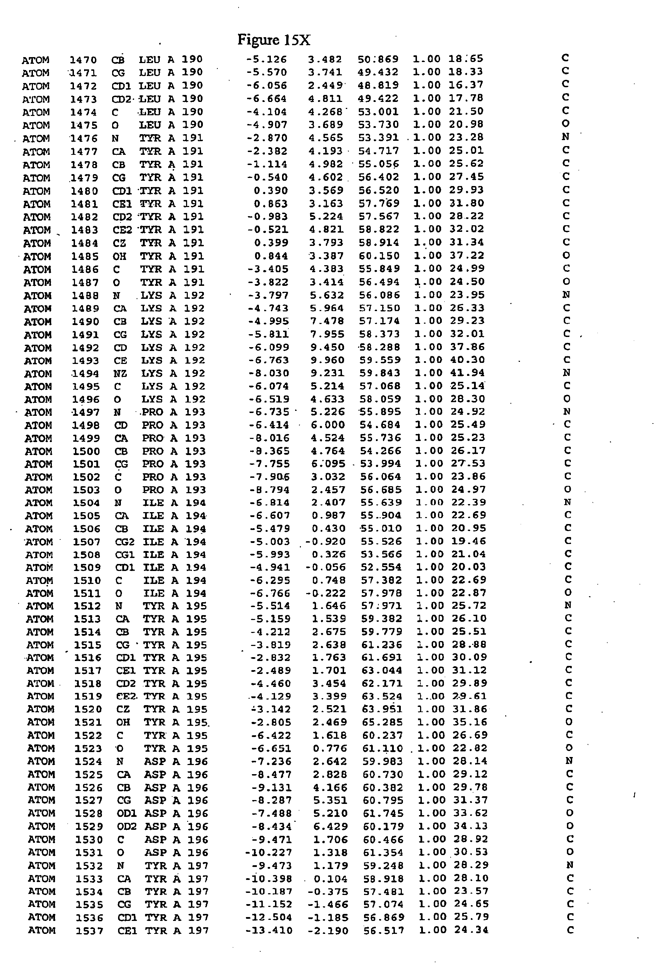

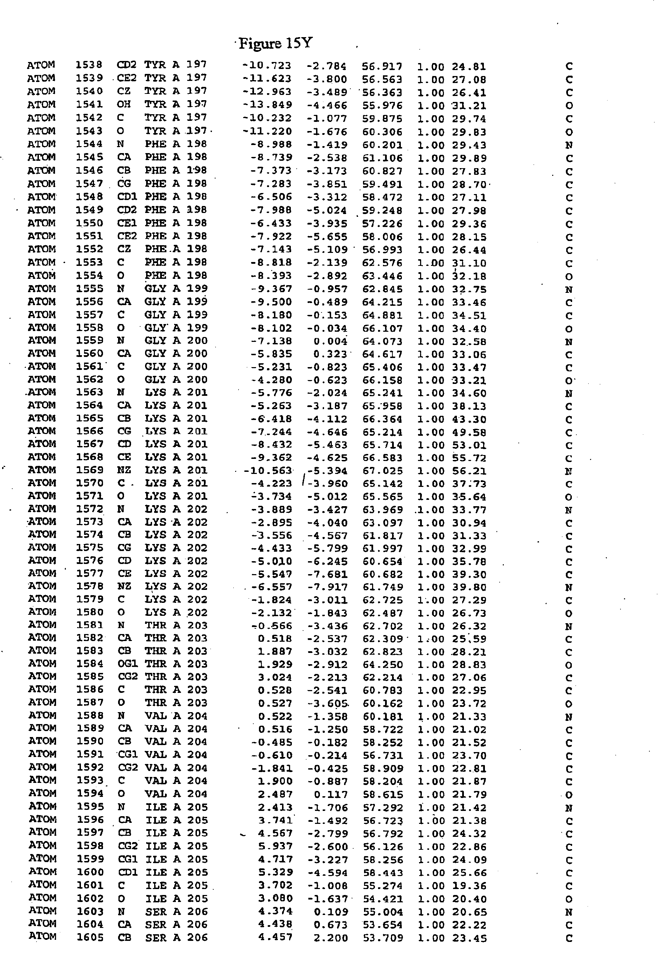

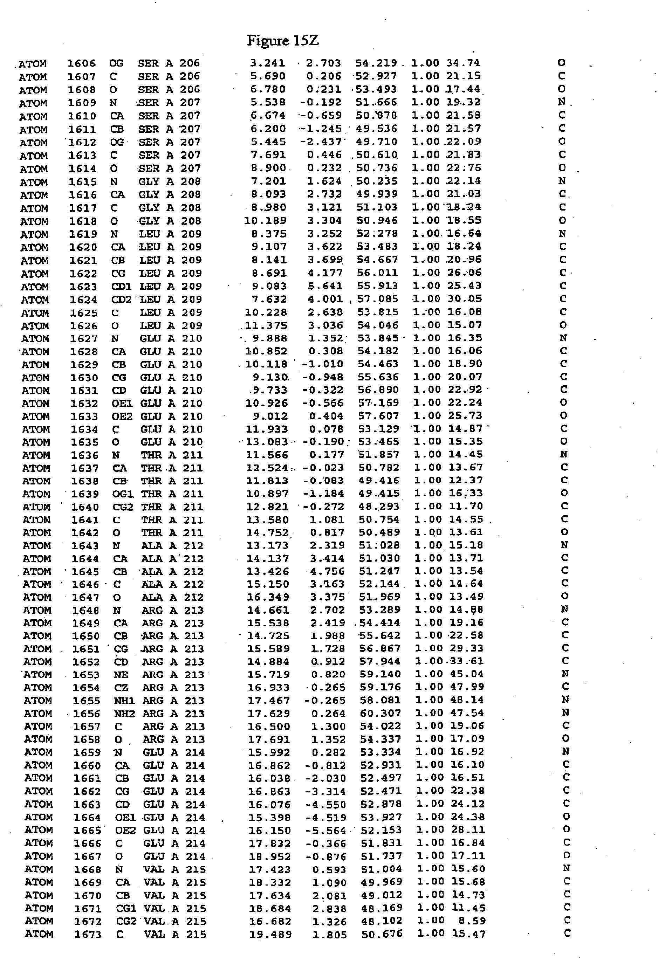

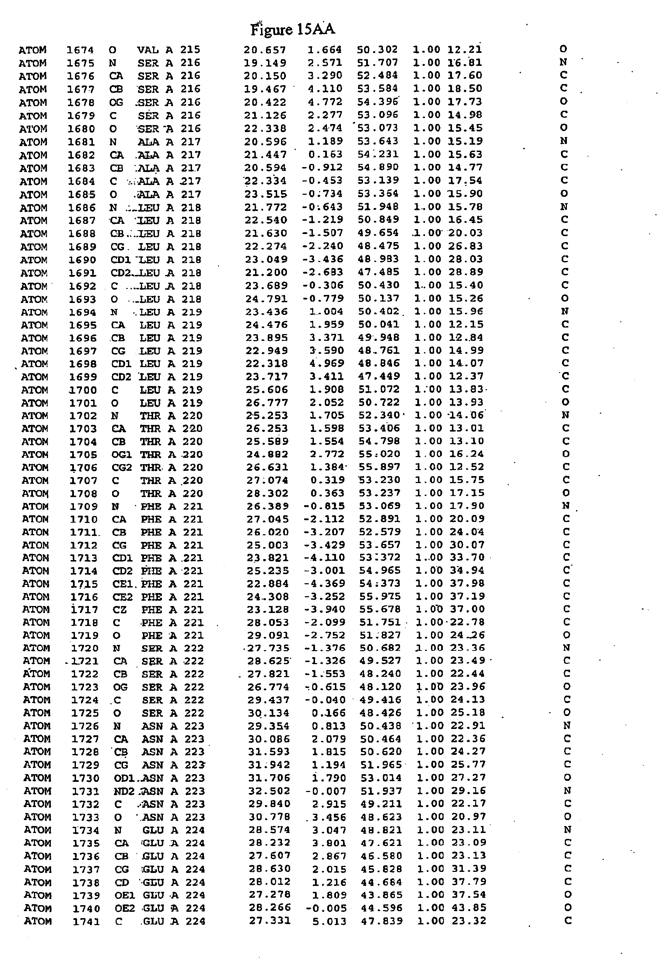

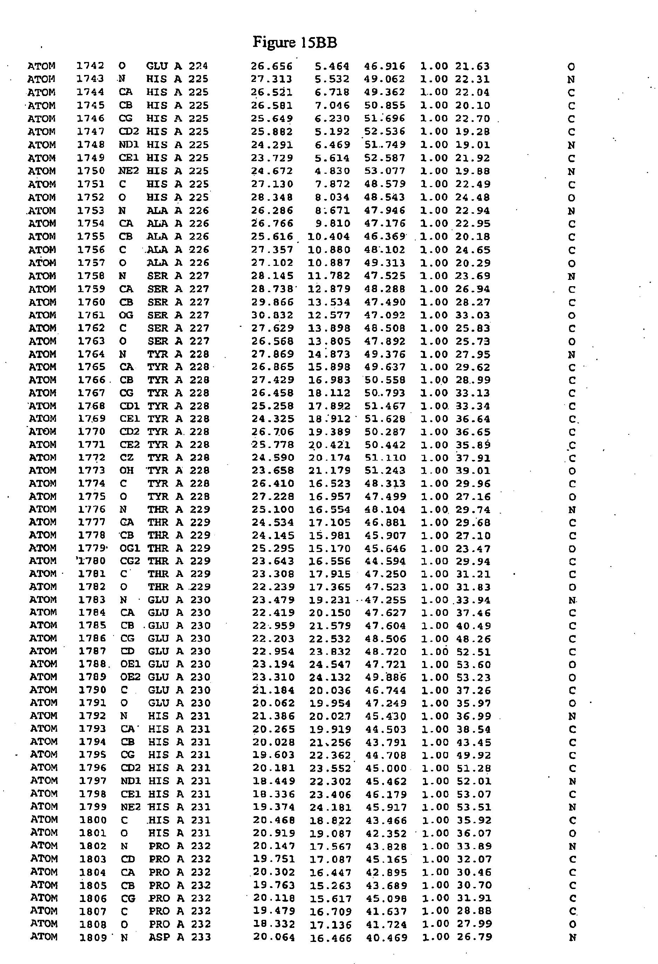

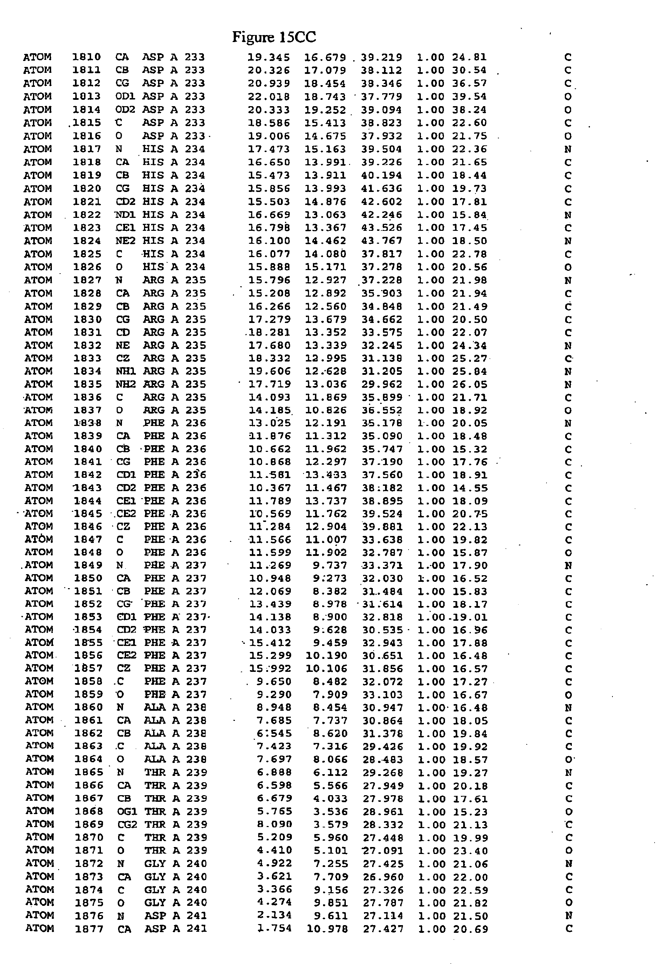

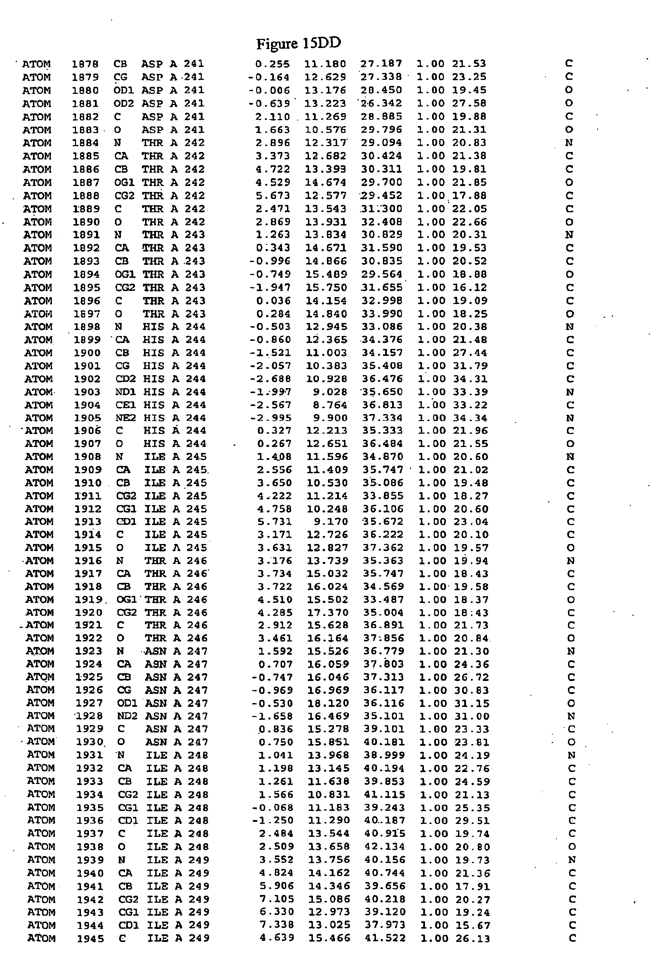

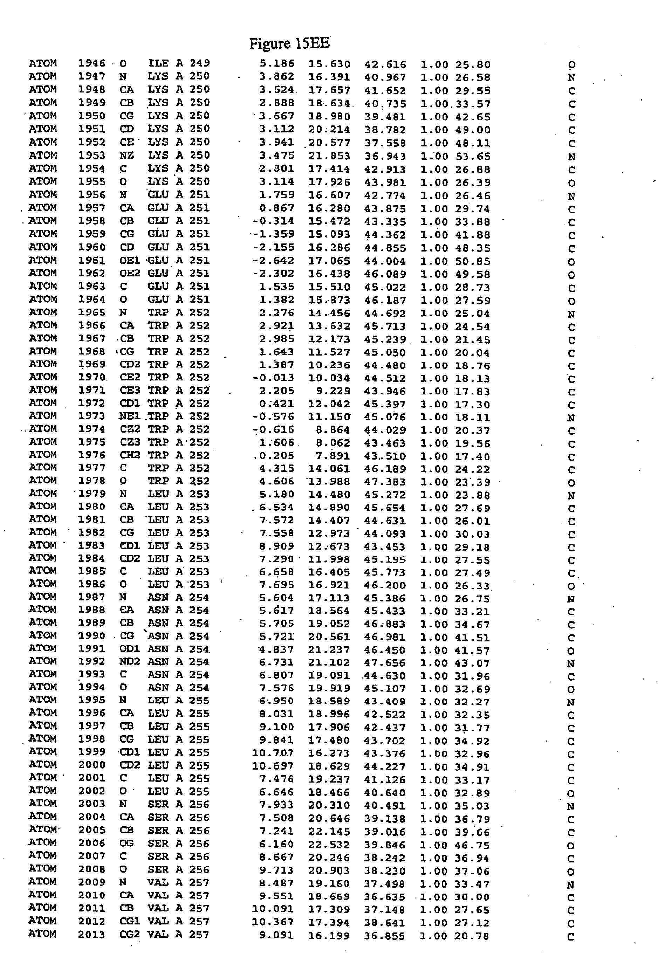

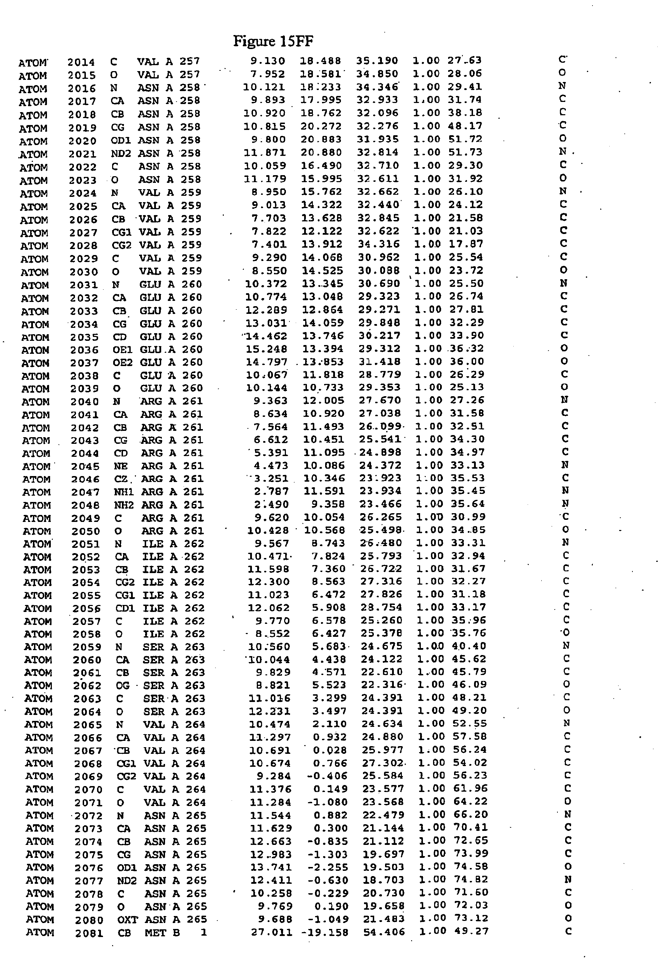

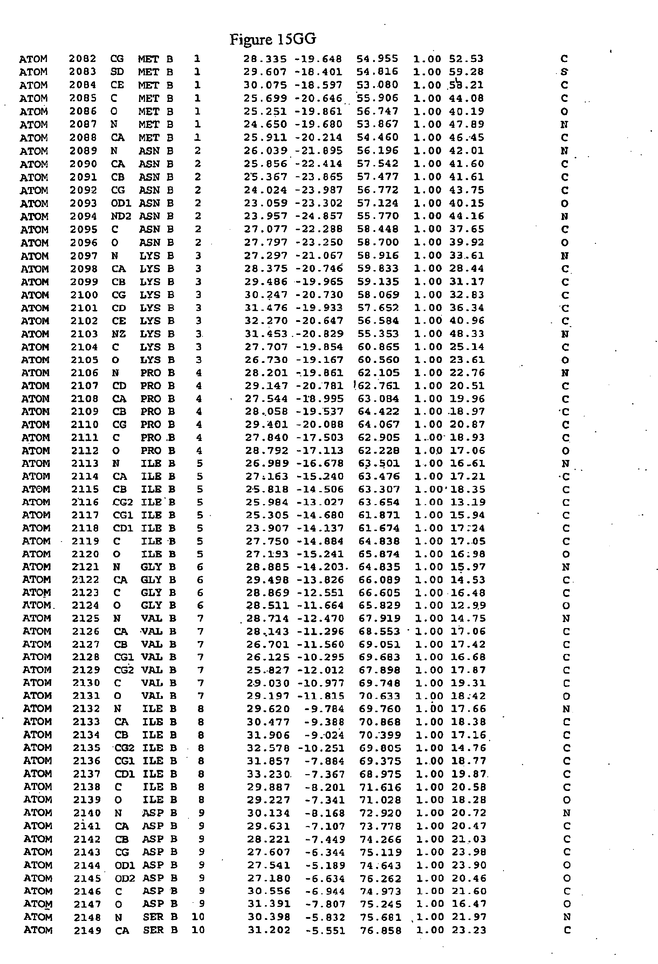

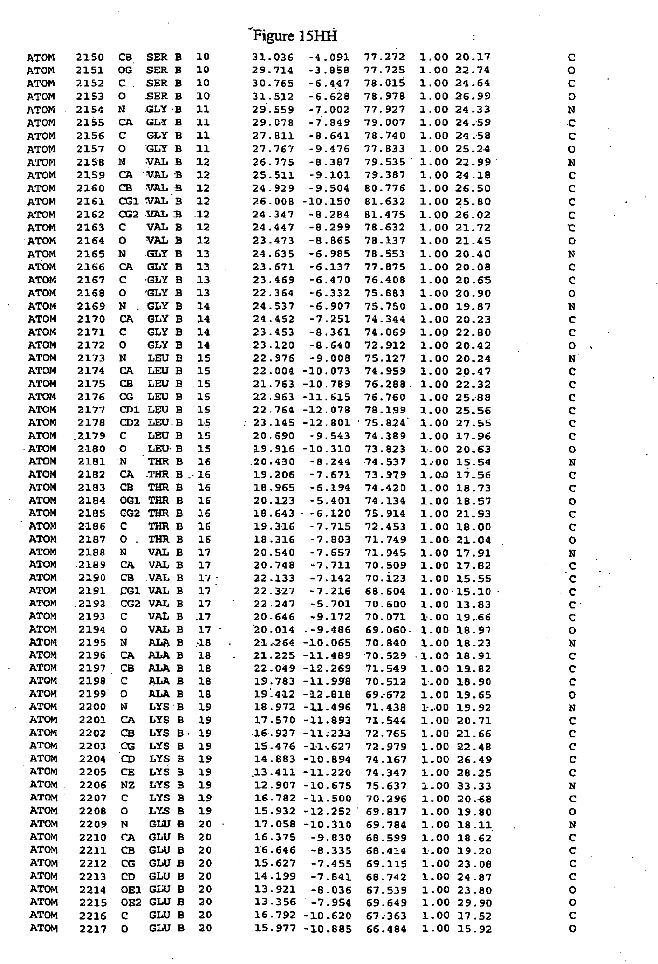

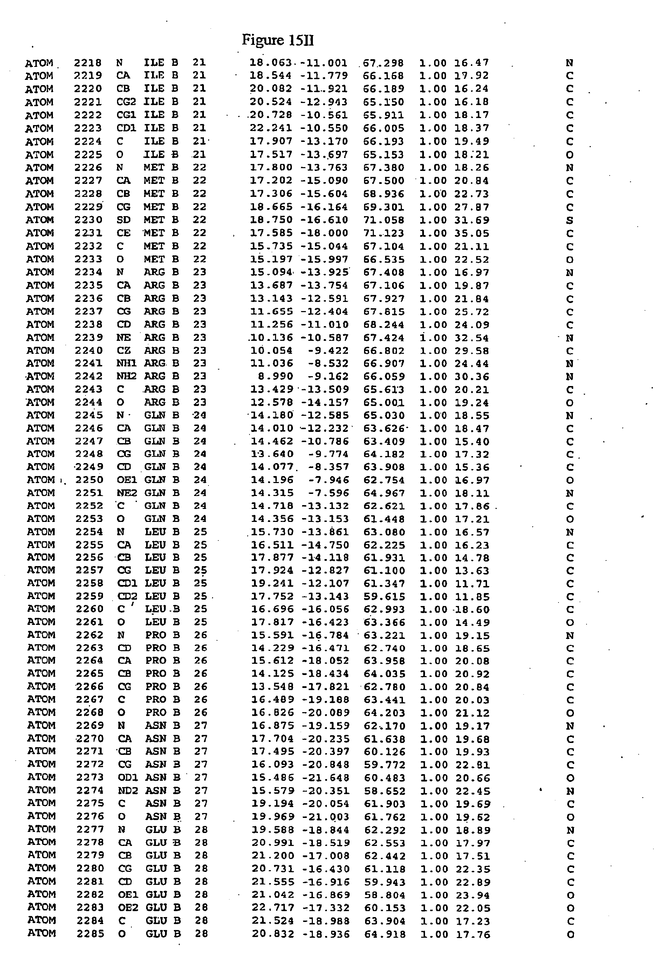

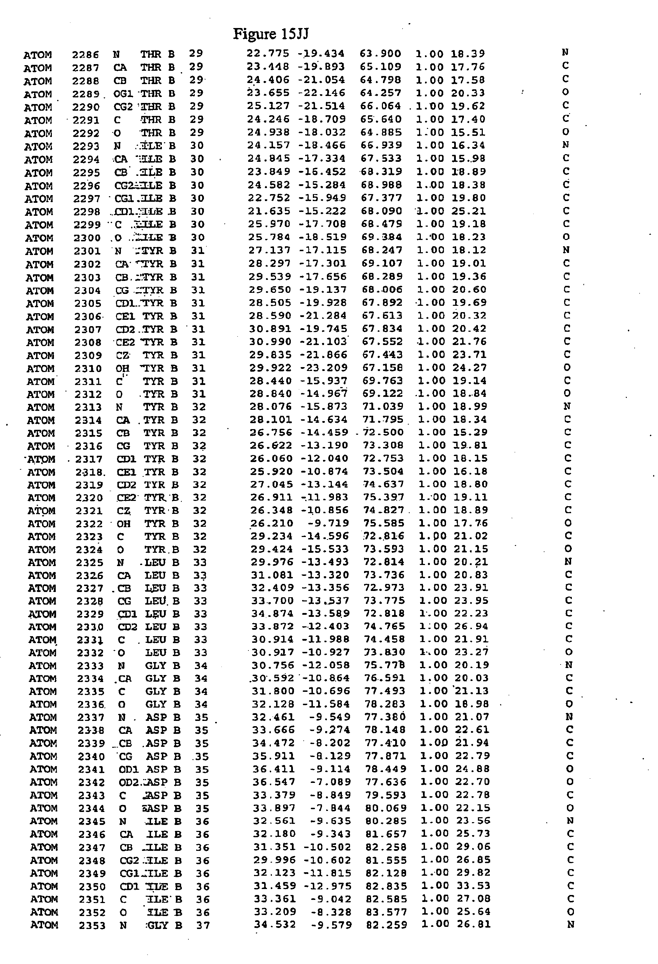

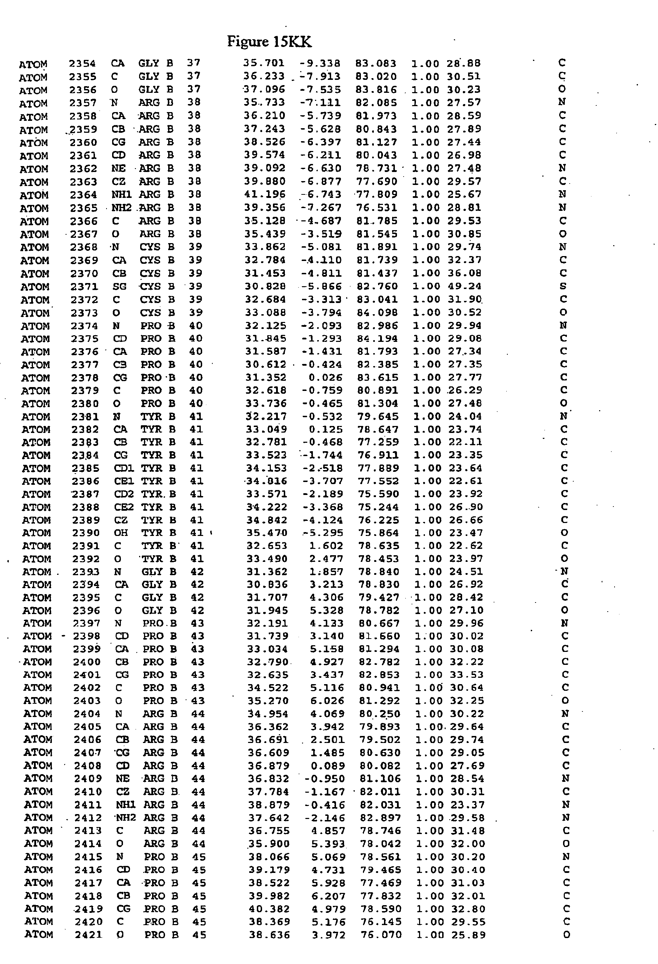

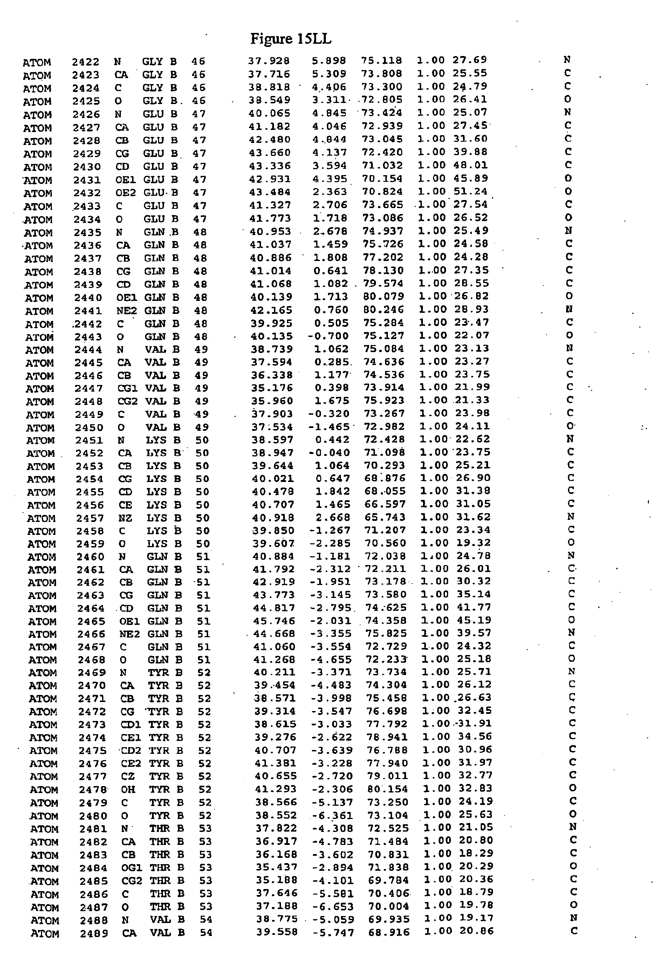

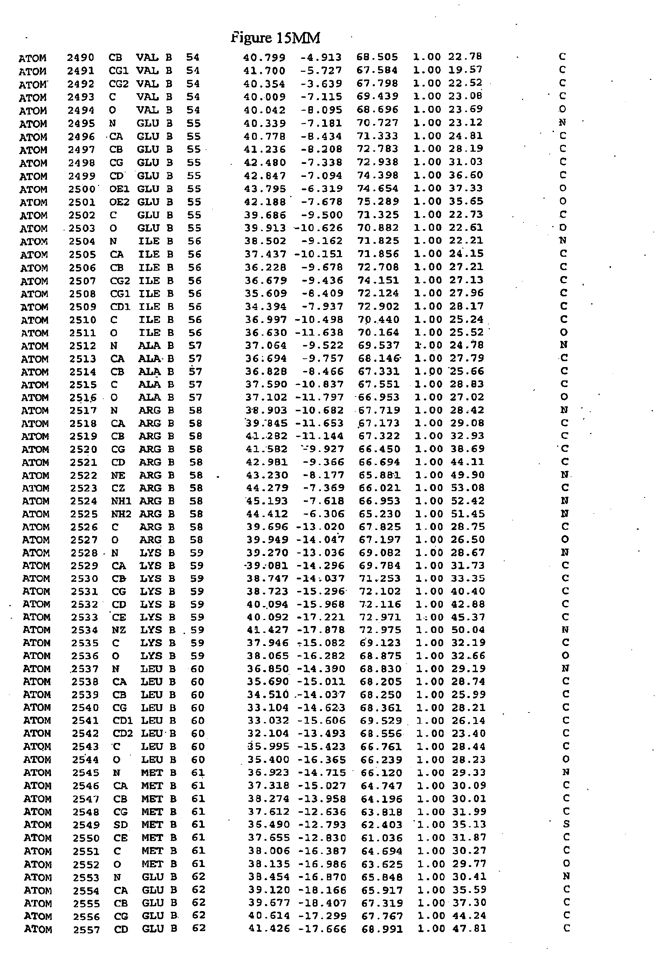

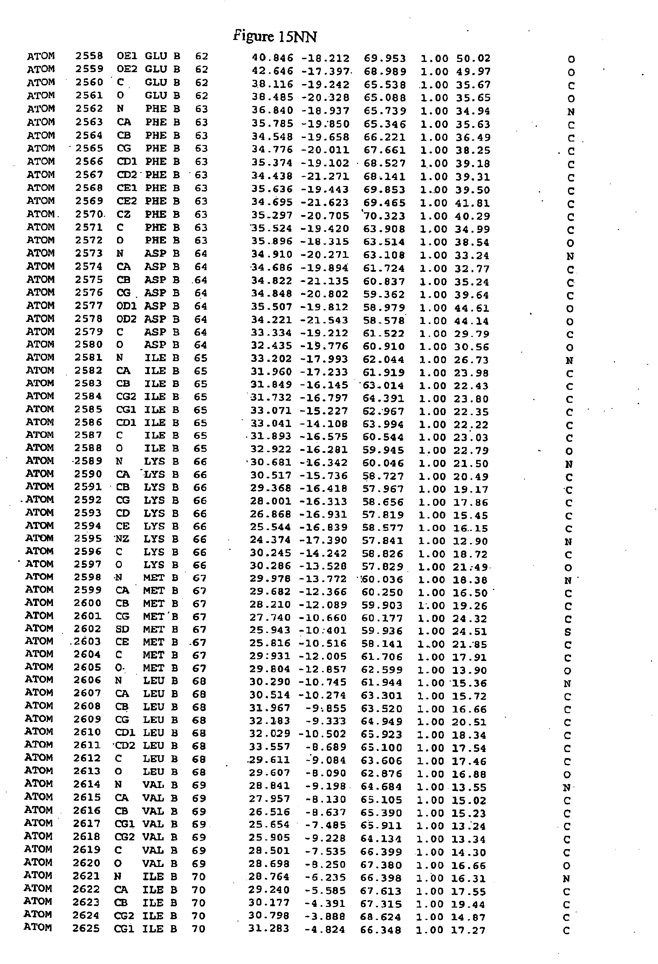

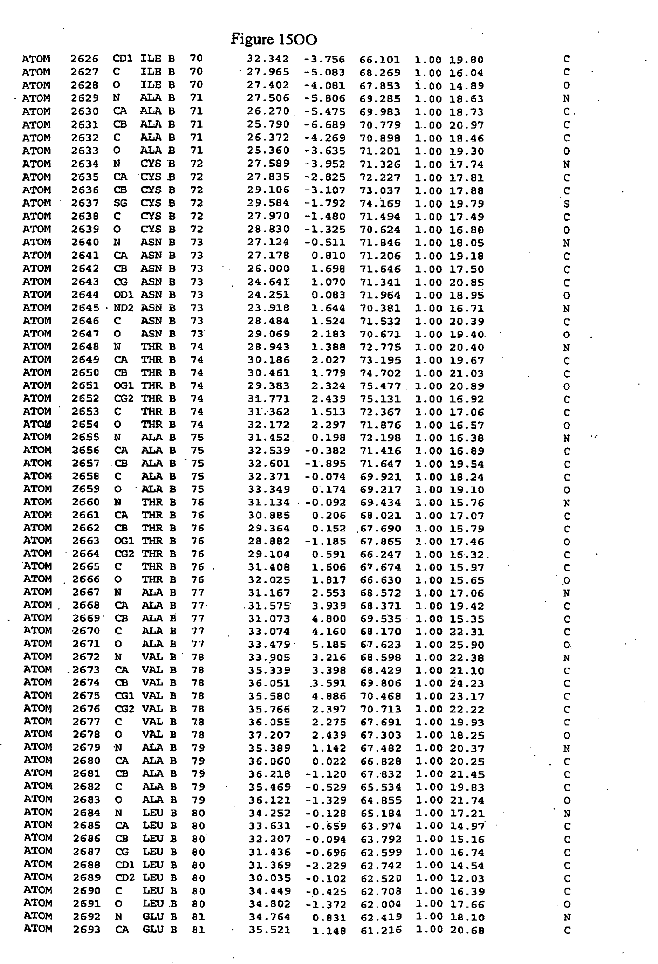

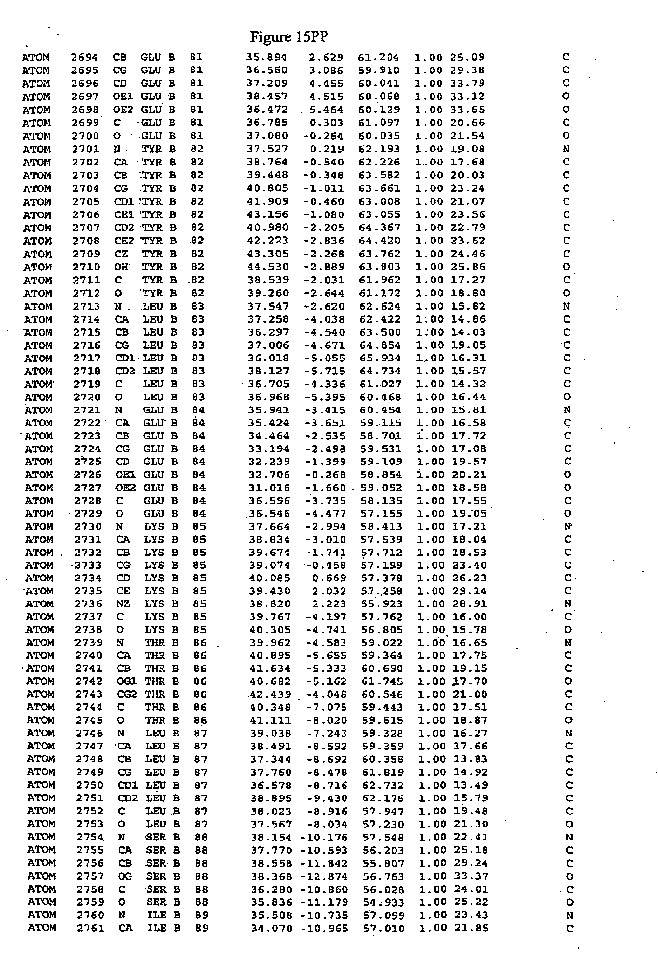

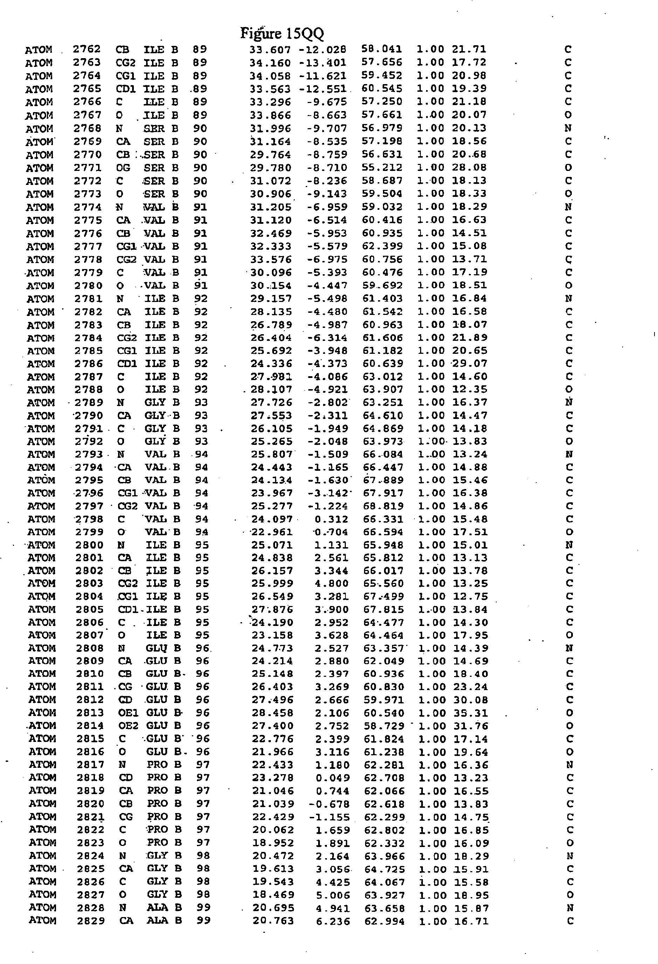

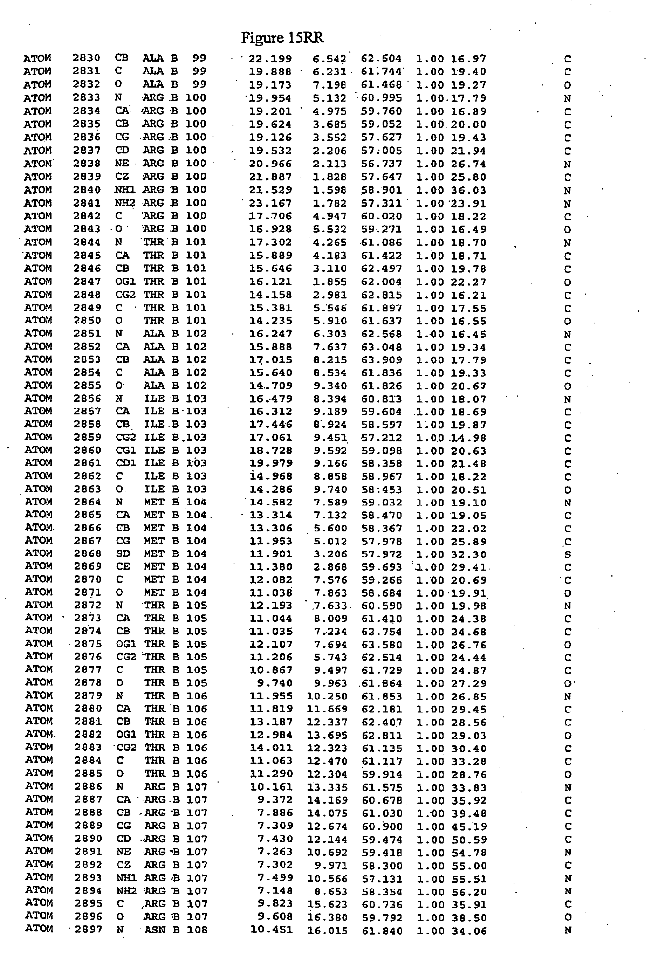

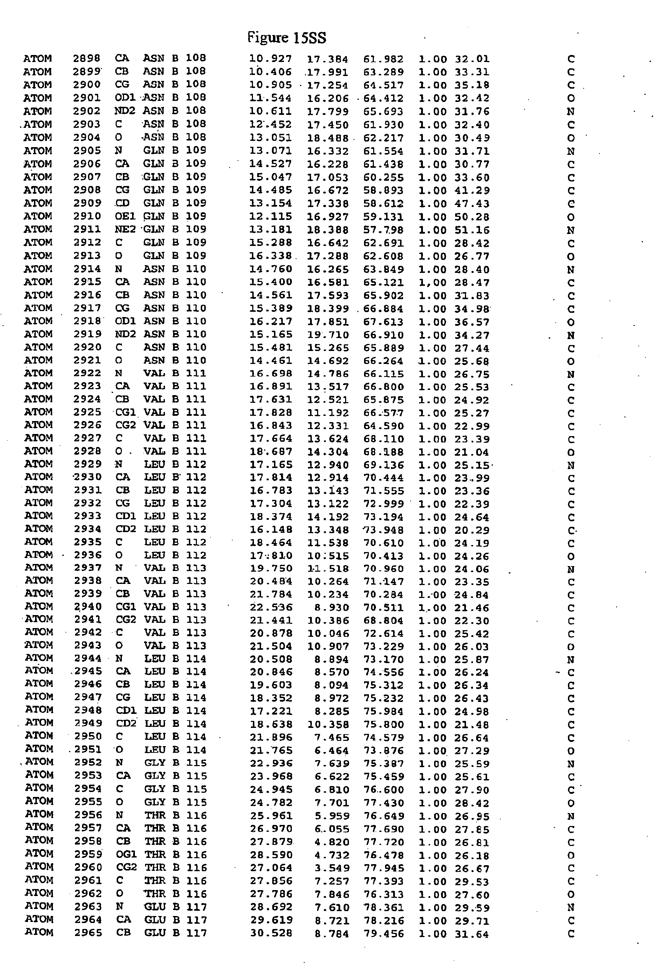

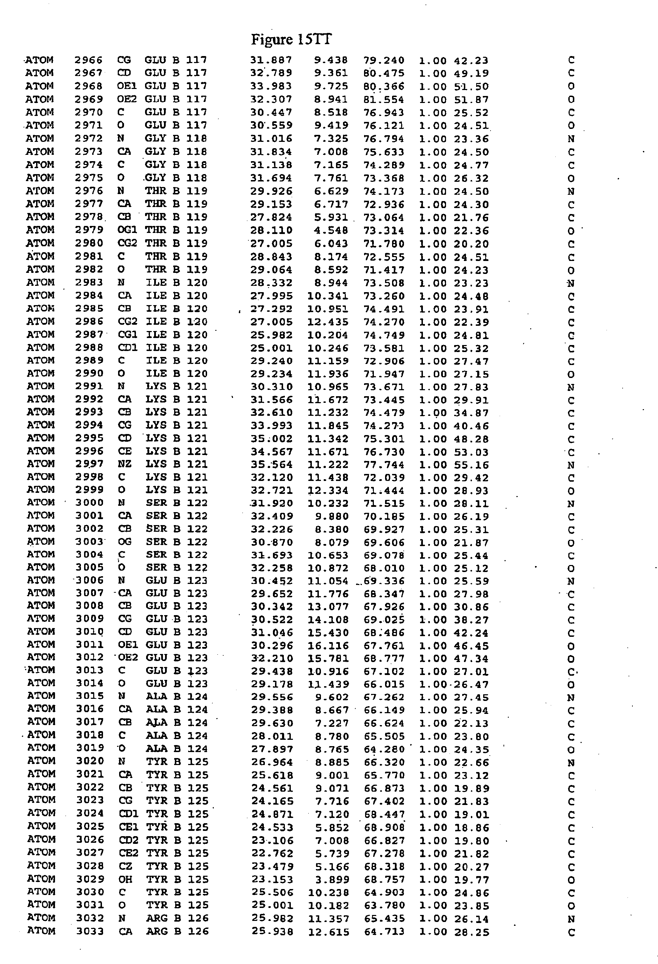

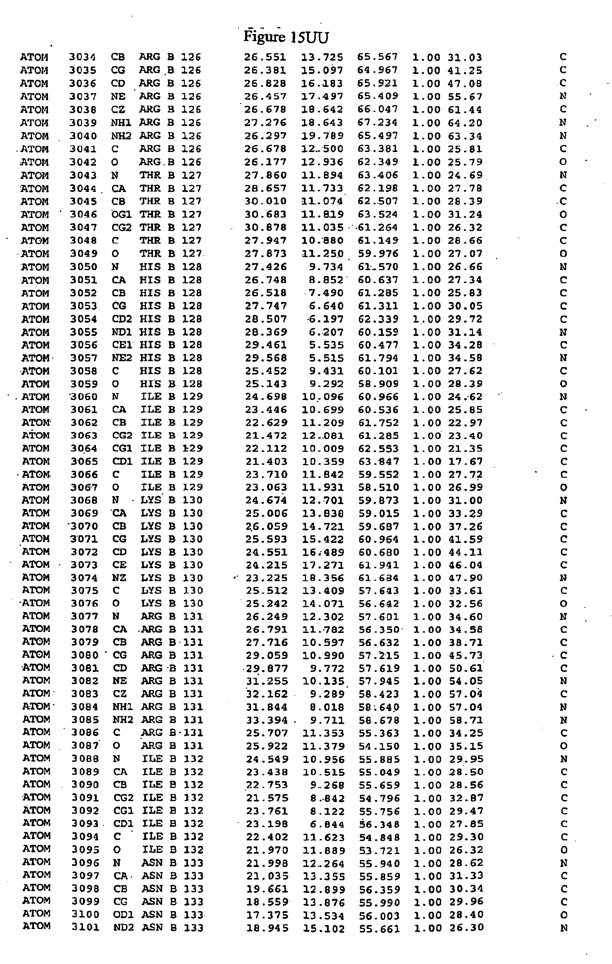

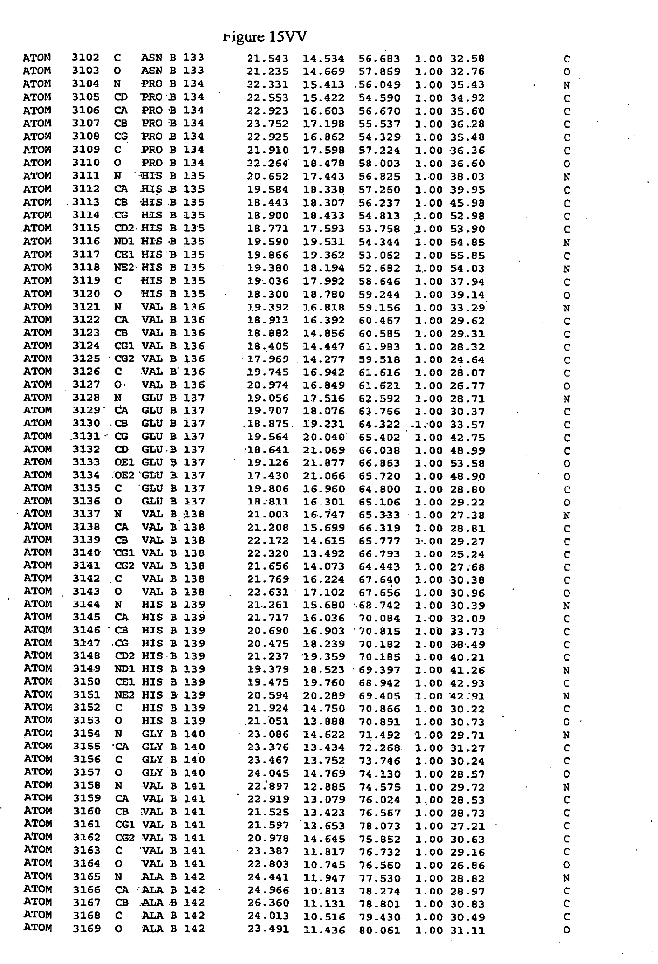

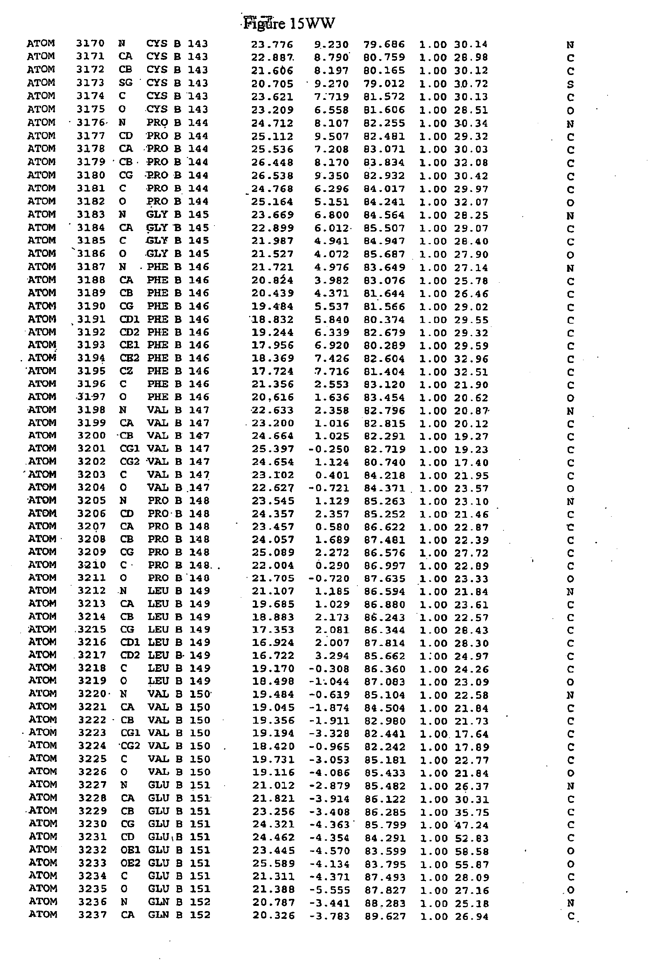

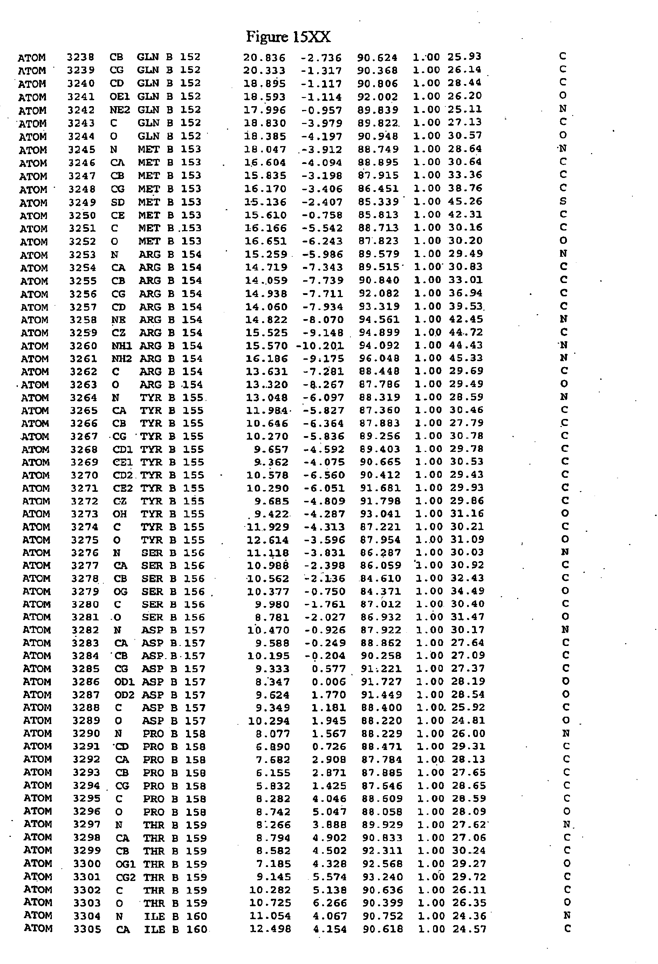

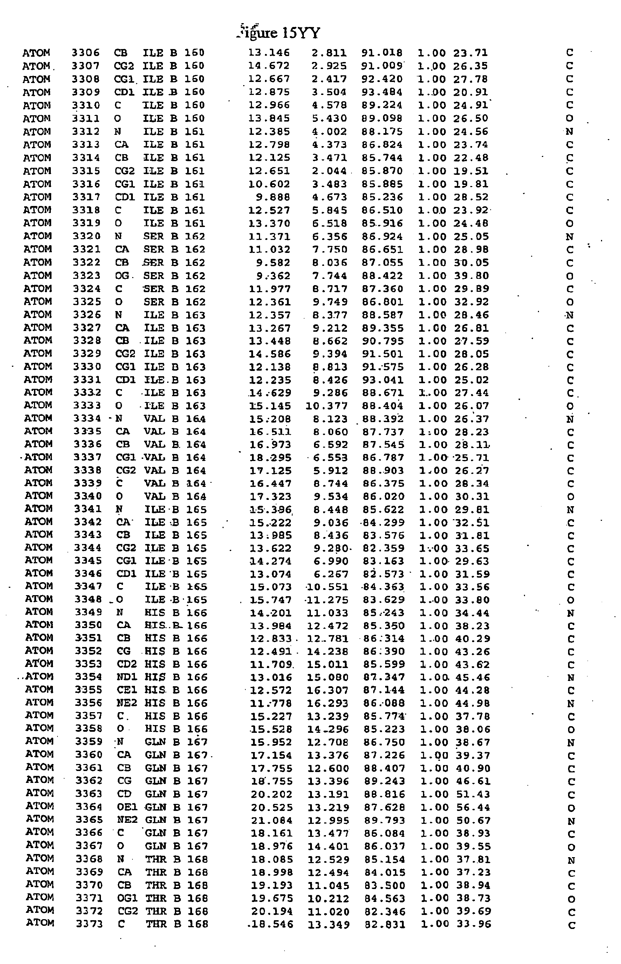

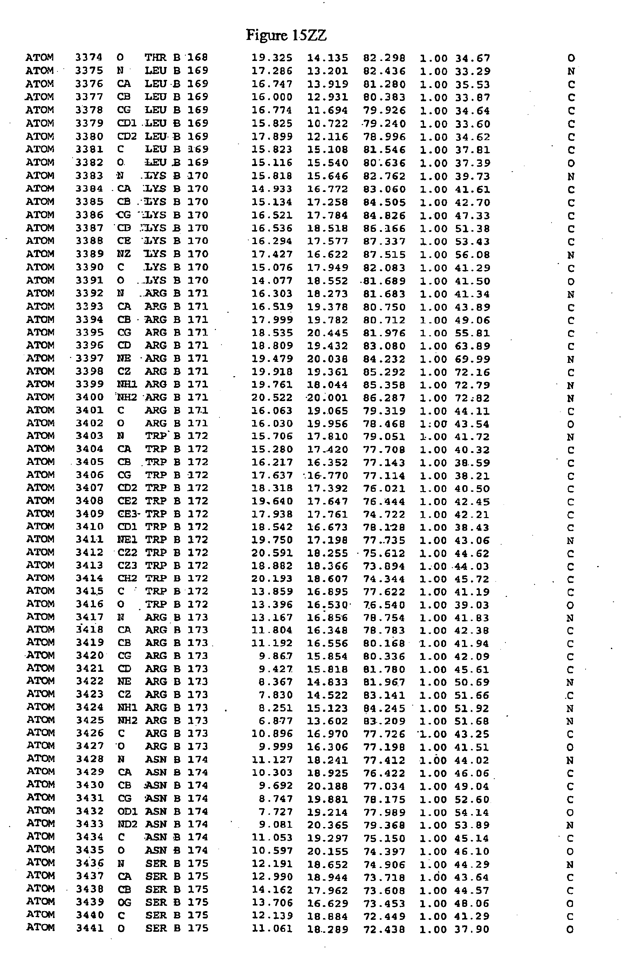

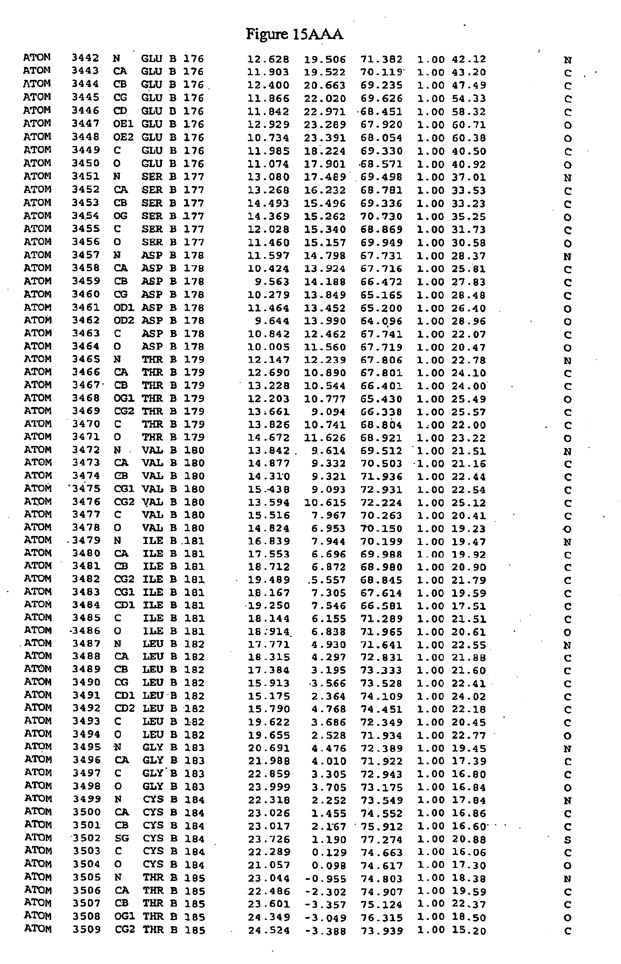

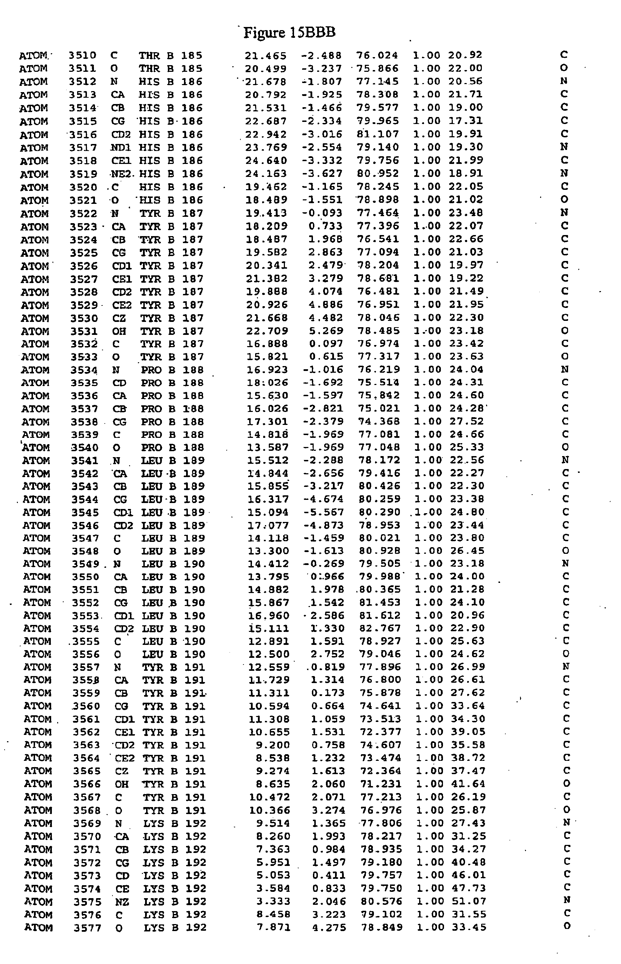

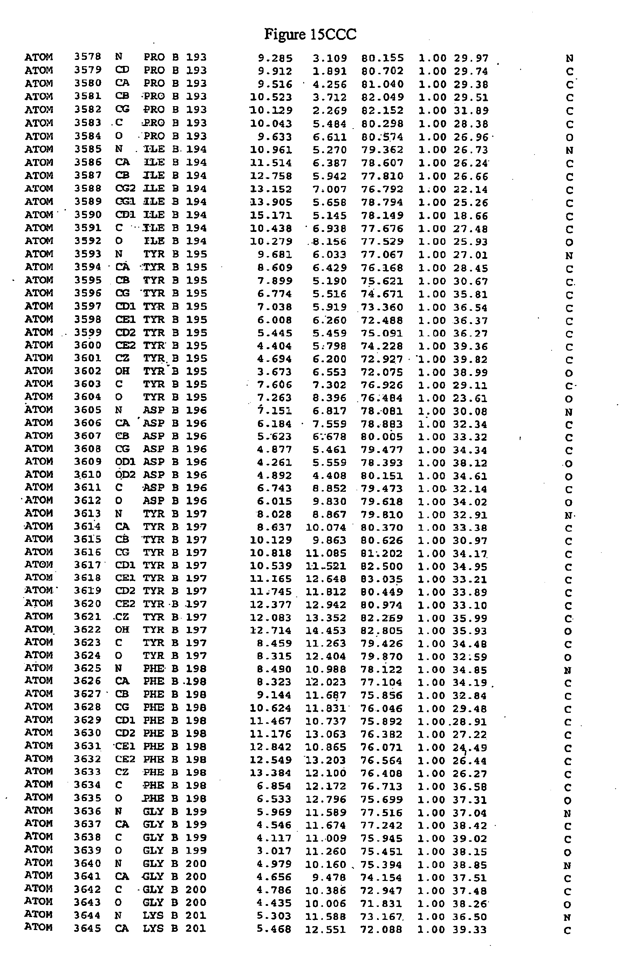

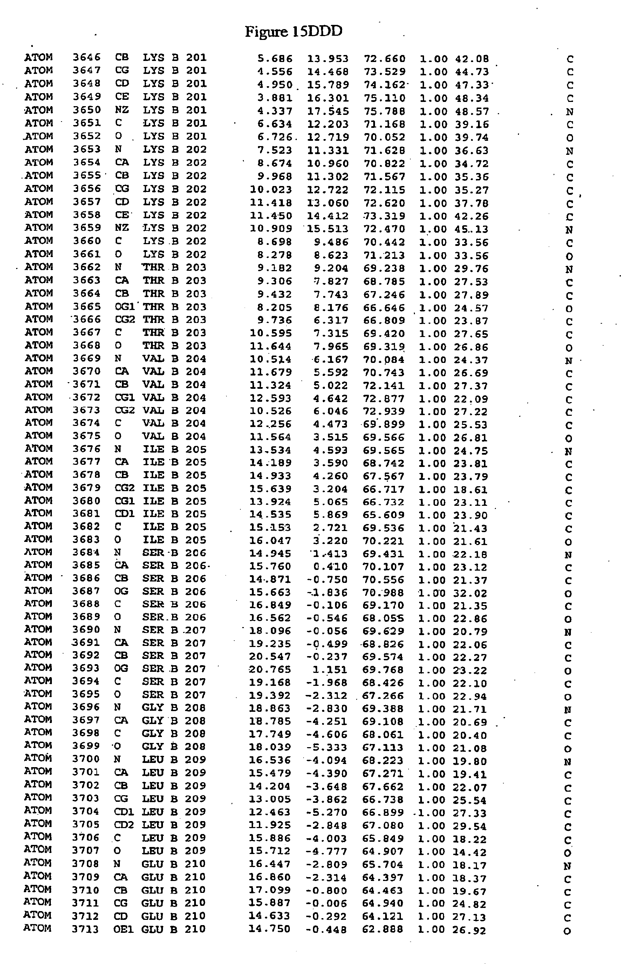

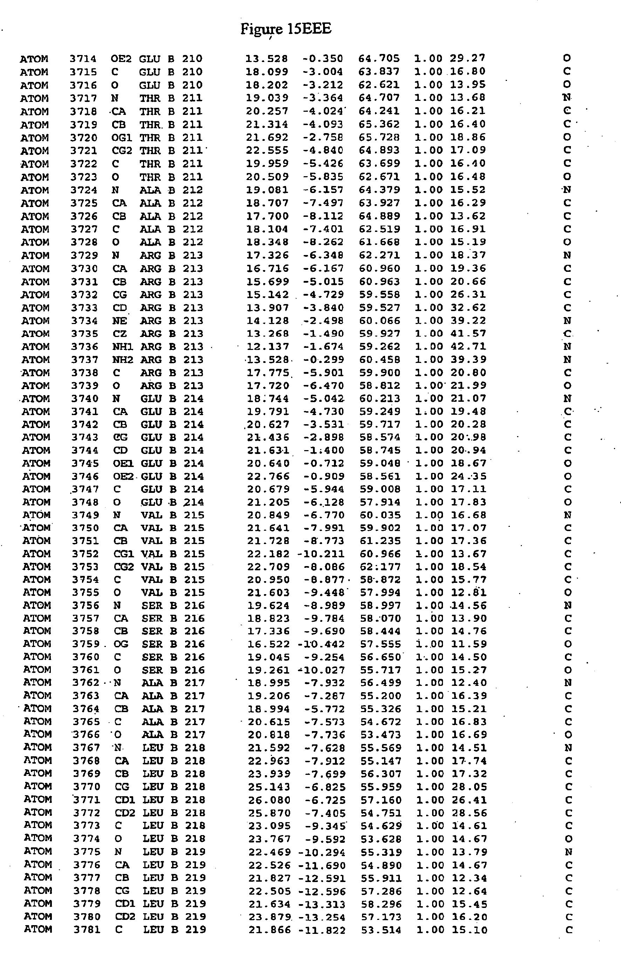

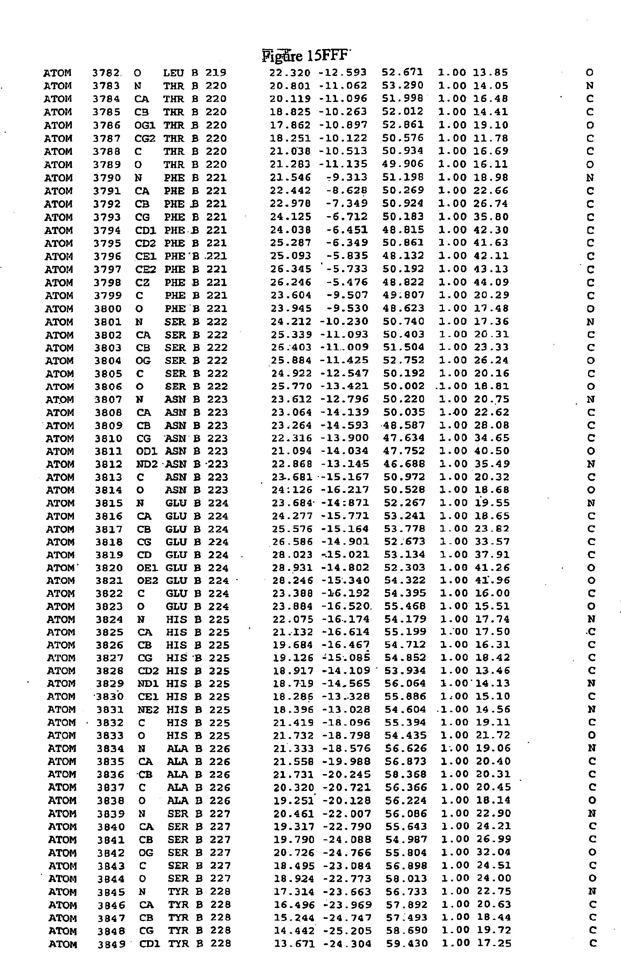

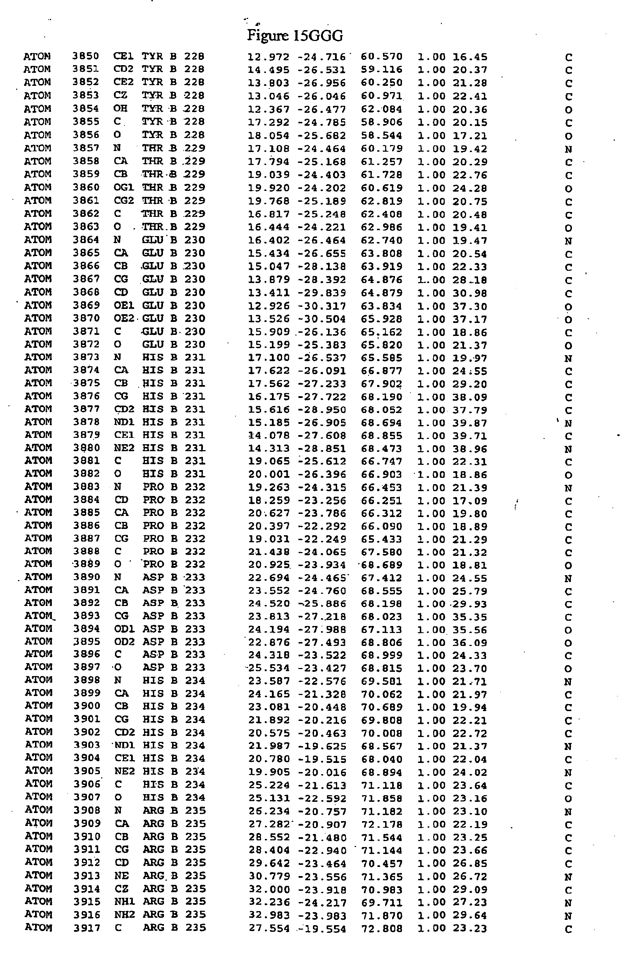

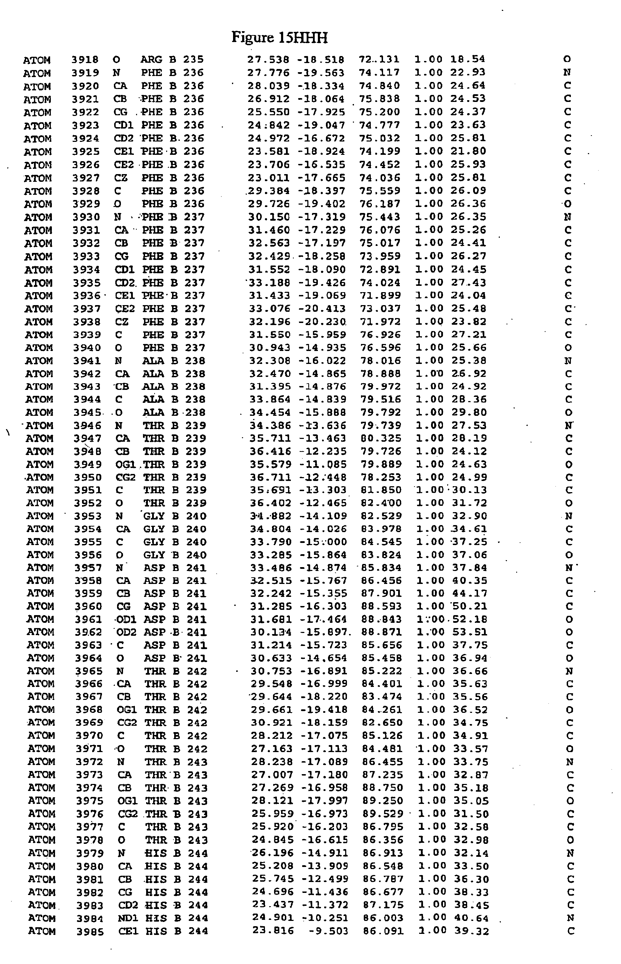

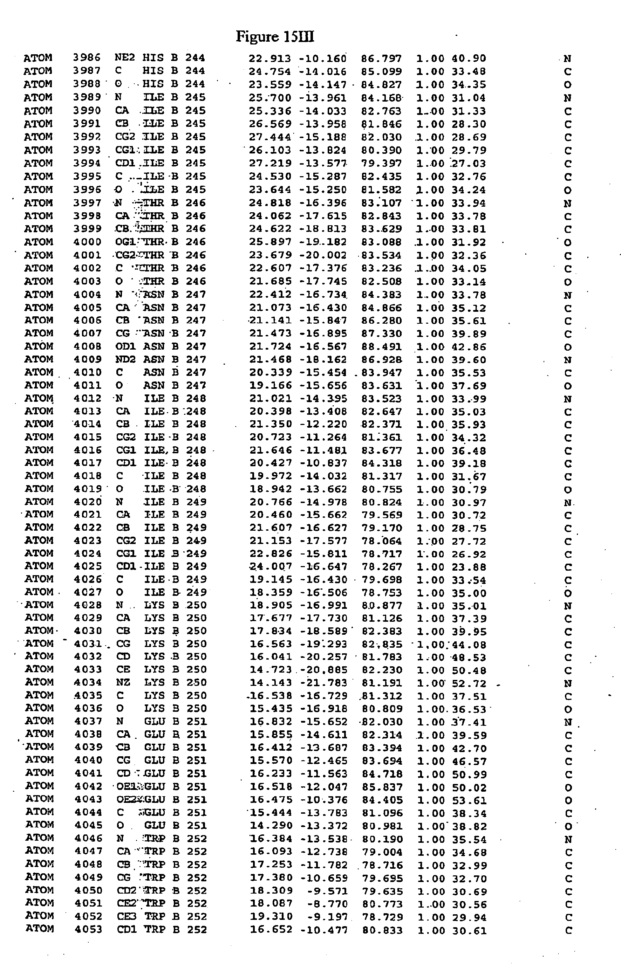

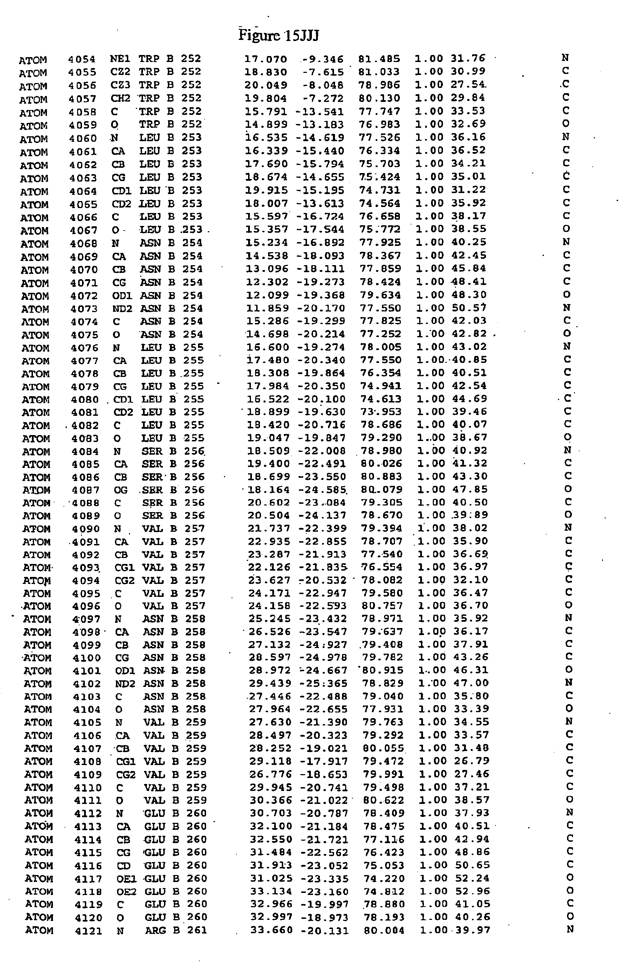

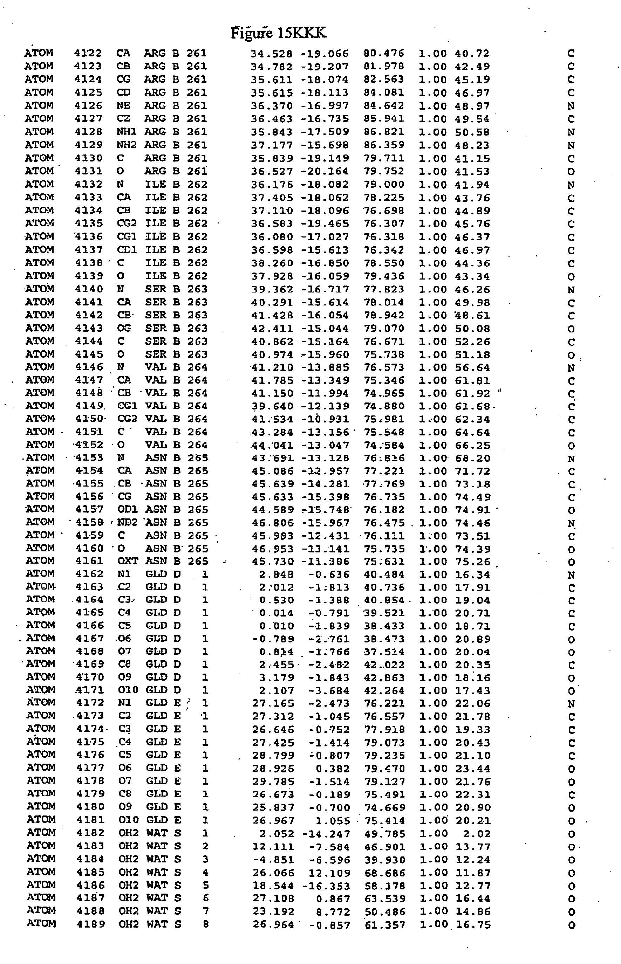

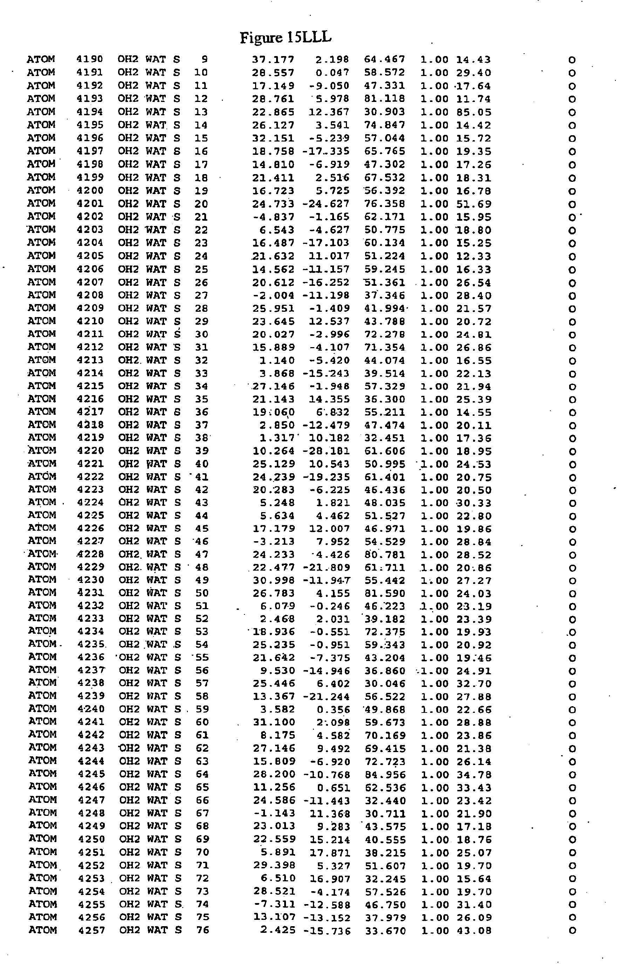

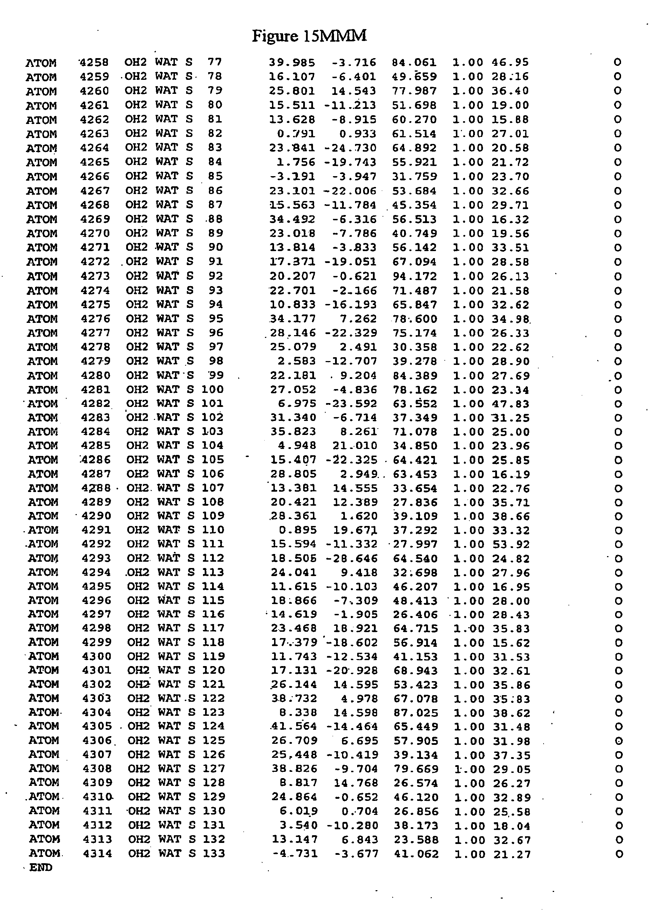

In one embodiment, we provide crystallized *S*. *aureus* MurI complexed with D-glutamate characterized by the structural coordinates depicted in Figure 15.

One embodiment we provide a crystal of *S. aureus* MurI complexed with the product (substrate) D-glutamate, wherein the MurI is at least 70%, 75%, 80%, 85%, 90%, 95%, 98%, or 99% homologous to the amino acid sequence represented by SEQ ID NO: 46, or portions thereof. The crystals may diffract from about 0.8 Å to about 3.5 Å.

One embodiment of the *S*. *aureus* crystal complex is characterized as belonging to the orthorombic space group C2 with cell dimensions a = 96.43 Å, b = 88.87 Å, c = 96.56 Å, α = 90°, β = 109.00°, and γ= 90°, wherein the crystallized complex is produced by the process of preparing a first solution of about 5 mM D,L-glutamic acid; about 1 mM TCEP; about 200 mM ammonium acetate pH 7.4; and about 10 mg/ml *S. aureus* MurI; preparing a second solution of about 0.17 M ammonium sulfate, and about 25% PEG 9000; combining the first solution and the second solution, thereby producing a combination; and forming drops from the combination under conditions in which crystals of MurI are produced.

A further embodiment relates to a *S*. *aureus* glutamate racemase in which the binding site comprises two conserved cysteine residues, denoted Cys72 and Cys184 in the sequence represented as SEQ ID NO: 46. A further embodiment comprises a binding site of *S*. *aureus* glutamate racemase wherein the binding site includes amino acid residues: Ser10, Pro40, Tyr41, Gly42, Cys72, Asn73, Thr74, Thr116, Thr119, Glu151, Cys184, Thr185 and His186 as represented by the structural coordinates of Figure 14. In one embodiment, the binding site of the *S*. *aureus* glutamate racemase complexes with D-glutamate and comprises the relative structural coordinates of amino acid residues Cys72 and Cys184 as well as amino acid residues within 5 Å of the Cys72 and Cys184 residues as represented by the structural coordinates of Figure 15. In one embodiment, the binding site of the S. *aureus* glutamate racemase additionally comprises at least one (one or more) of the following amino acid residues: Ser10, Pro40, Tyr41, Gly42, Asn73, Thr74, Thr116, Thr119, Glu151, Thr185 and His186. In this embodiment, the binding site complexes with D-glutamate and comprises amino acid residues Cys72 and Cys184 and at least one (one or more) of the following amino acid residues: Ser10, Pro40, Tyr41, Gly42, Asn73, Thr74, Thr116, Thr119, Glu151, Thr185 and His186, which can be present in any combination.

In a further embodiment, the binding site of the glutamate racemase comprises two hydrogen bond TRIADs, which occur close to the conserved cysteine residues of the binding site. Specifically, on one side (Cys184) of the binding site, the TRIAD is Gly151-Thr185-His186 and on the other side (Cys72), the TRIAD is Thr74-Thr1 16-Thr119. Thus, in a specific embodiment, the binding site of *S*. *aureus* glutamate racemase complexes with D-glutamate and comprises amino acid residues Cys72 and Cys184 and additionally comprises at least one (one or more) of the following: amino acid residues Ser10, Pro40, Tyr41, Gly42, Asn73, Thr74, Thr1 16, Thr119, Glu151, Thr185 and His186.

The threonine residues have features of interest as they relate to the binding site of the *S*. *aureus* glutamate racemase. On one side (Cys184) of the binding site, Thr185 is H-bonded to His186 and His186 is further H-bonded to Glu151. The hydroxyl oxygen (O) of Thr185 is 2.9 Å away from the amino nitrogen (N) of the substrate (i.e., glutamate) and 4.4 Å from the sulfur (S) atom of Cys184, which is 4.1 Å from the nitrogen of His186.

On the other side of the binding site (Cys72), Thr74 is H-bonded to Thr116 and further H-bonded to Thr119. The hydroxyl O of Thr74 is H-bonded to one of the carboxylate oxygen atoms of the substrate and is 4.3 Å away from the S atom of Cys72. Analysis showed that the three hydroxyl oxygens form a triangle, all within less than 3.4 Å from one another.

The two TRIADs may play important roles in altering the pKa of the two binding site cysteine residues (in addition to that of the neighboring hydrophobic core), facilitating the proton transfer during catalysis or both.

Another embodiment is a crystal of *S*. *aureus* glutamate racemase, comprising a binding site comprising the amino acid residues Cys72, Cys184, Ser10, Thr74, Thr116, Thr119, Glu151, Thr185 and His186 of the amino acid sequence represented by SEQ ID NO: 46. This crystal may be further complexed with D-glutamate. Additionally, the binding site of the crystal complexed with D-glutamate comprises the amino acid residues: Cys72, Cys184, and at least one of the following amino acid residues: Ser10, Pro40, Tyr41, Gly42, Asn73, Thr74, Thr116, Thr119, Glu151, Thr185 and His186. A further embodiment is one in which the binding site additionally comprises at least one of the following amino acid residues: Asp9, Gly11, Val12 Gly13, Gly14, Arg38, Cys39, Pro43, Arg44, Ala71, Ala75, Val94, Gly115, Val147, Gly183 and Tyr187. A further embodiment is one in which the binding site additionally comprises at least one of the following amino acid residues: He8, Thr16, Val17, Tyr32, Asp35, Va149, He70, Ser76, Ala77, Leu114, Gly118, Ile120, Tyr125, Pro144, Pro148, Leu182 and Ser207. The crystals comprising binding sites represented by the above amino acid residues comprise the amino acid sequence is that represented by SEQ ID NO: 46; or an amino acid sequence is at least 75%, 80%, 85%, 90%, 95%, 98%, or 99% homologous to the amino acid sequence that is represented by SEQ ID NO: 46.

Location and geometry of a binding site are of the enzyme are also defined. Two conserved cysteine (Cys) residues were identified as the residues responsible for the (de)protonation of the alpha-carbon of the substrate during catalysis, which is consistent with the two-base mechanism proposed for function of the enzyme in its role as a racemase. The two binding site cysteines are Cys72 and Cys184, which are about 7.3 Angstroms (Å) apart (Cα-Cα distance, which is the distance between Cα atoms). Other amino acid residues identified in the vicinity of the binding site include Ser10, Thr74, Thr116, Thr119, Glu151, Thr185 and His186. The bound substrate/product, D-glutamate, is located between the two conserved cysteine residues. Further detail of the binding site is as follows: looking down the axis defined by the Glu151 along the γ and α carbon, the two cysteines exhibit a rather symmetrical environment; each has a hydrophobic core behind, with respect to the substrate and a neighboring threonine residue not far from the substrate C-alpha (3.4 - 4.3 Å), respectively.

Analysis of the crystal structure of the *S*. *aureus* glutamate racemase indicates that the following amino acid residues are within 10 Å of the bound D-glutamate: 8-17, 32-44, 49, 52, 71-79, 94-95, 114-120, 122, 125, 184-190, and 207-208. Analysis of the crystal structure also shows that the following amino acid residues are within 4 Å of the bound D-glutamate: 9-10, 39-42, 72-74, 116, 119, and 184-186. Of interest is the fact that only one acidic amino acid residue (Asp9) is present in the structure surrounding the binding site Cys72, which serves as an anchoring point for the amino group of the substrate/product with a polar (charged) interaction between the amino nitrogen atom of D-glutamate and the delta oxygen atom of Asp9.

### C. Atypical gram negative bacteria

*H. pylori* MurI (MurI) has been crystallized, and crystal structures (three-dimensional structure) determined. The structures determined represent that of MurI alone, or in complex with the enzyme substrate, glutamate. NMR data suggests that multiple folded conformations of MurI can exist in the absence of substrate.

Results show that the asymmetric unit of the crystal consists of two copies of the polypeptide chain corresponding to a dimer; the active form of *H. pylori* MurI (MurI) is a dimer. Each monomer consists of two distinct and very similar alpha/beta type domains that are held together by a hinge situated at one end of the domain interface (around amino acid residues 92 and 210).

The two domains together form the active site that includes residues from only one of the monomers, despite the fact that the active site is situated close to the dimer interface. Both domains contribute important amino acid residues to the binding site. For example, the two conserved cysteines are from different domains.

The *H. pylori* MurI dimer is held together by a stable and conserved hydrophobic core created by a four helix bundle (e.g., two helices from each monomer, residues 143 to 169) that links the two domains and, as a result, the two monomers, rather rigidly together. This arrangement leaves the other two domains of each monomer more free to move which gives access to the two active sites at the dimer interface. Results show that the protein is a dimer also in the absence of a ligand, and is more flexible in this state.

The amino acid residues which make up the binding site of MurI are conserved in seventeen strains of *H. pylori* that have been sequenced and the amino acid sequences determined.

One of ordinary skill in the art would recognize that solving crystal structures of proteins such as *H. pylori* MurI (MurI) requires a stable, high-quality protein.

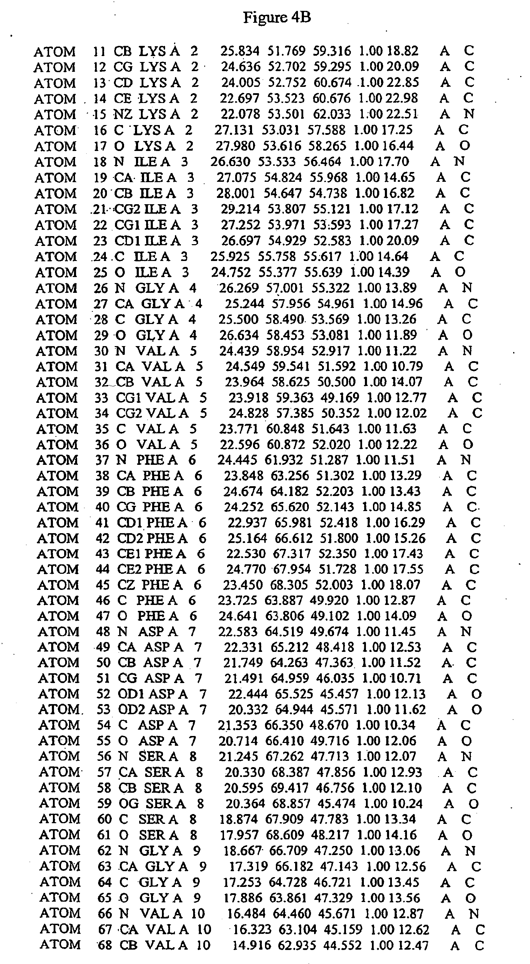

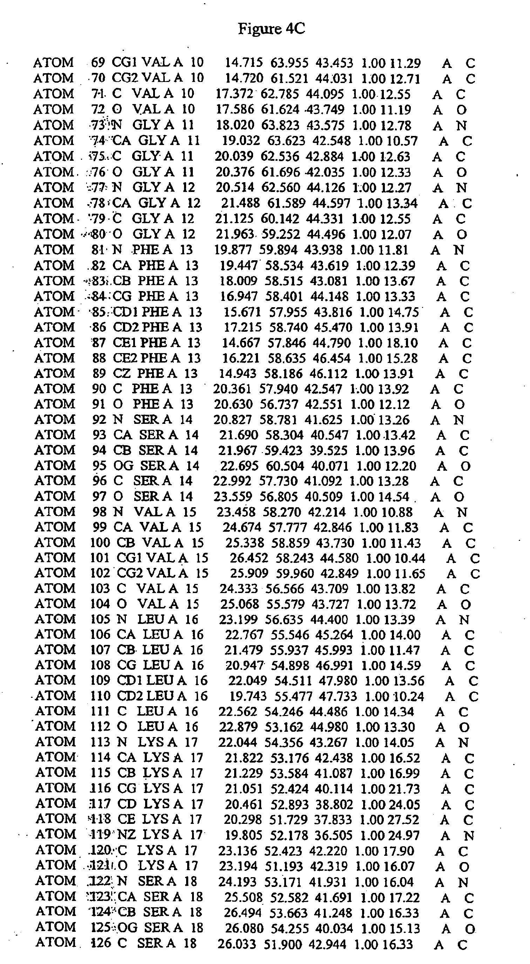

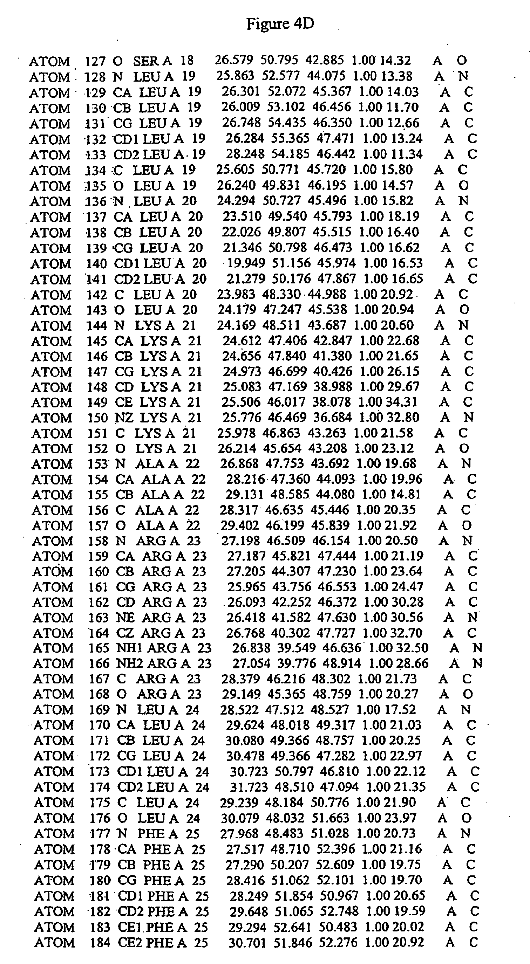

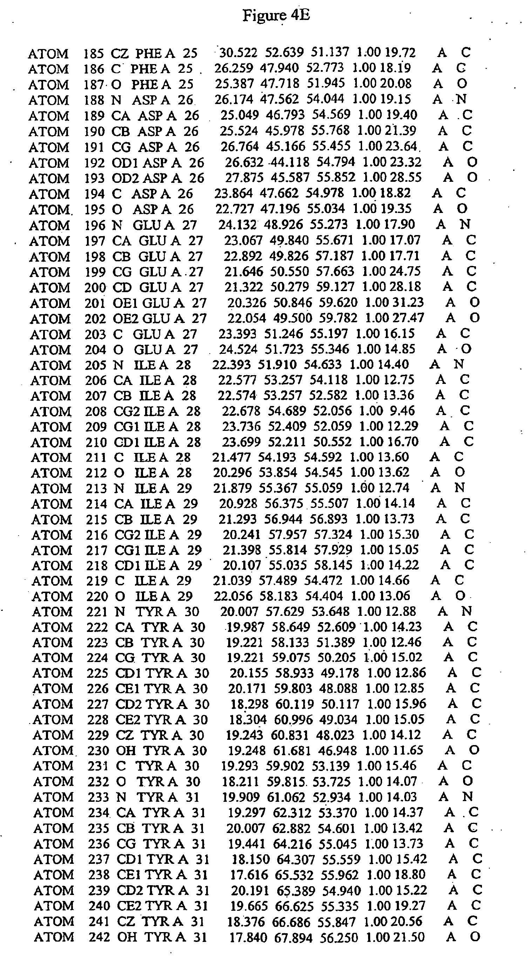

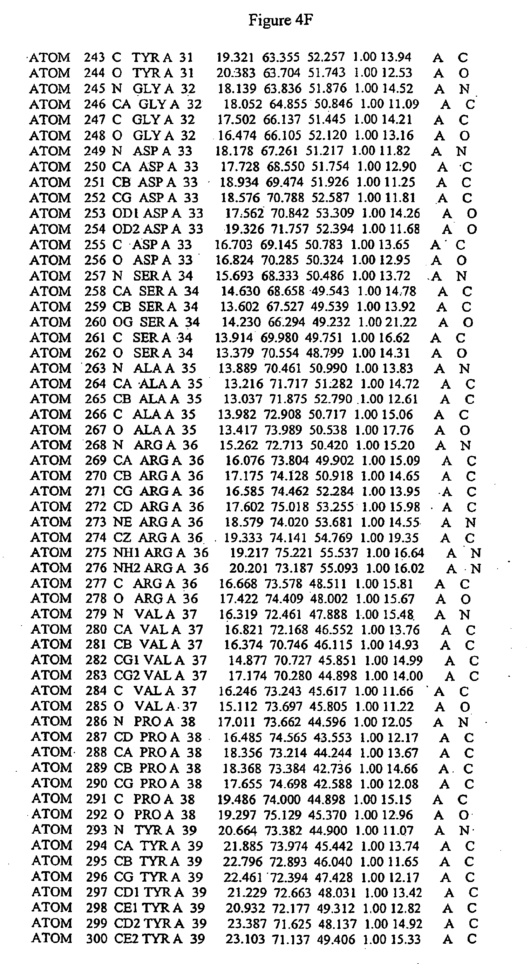

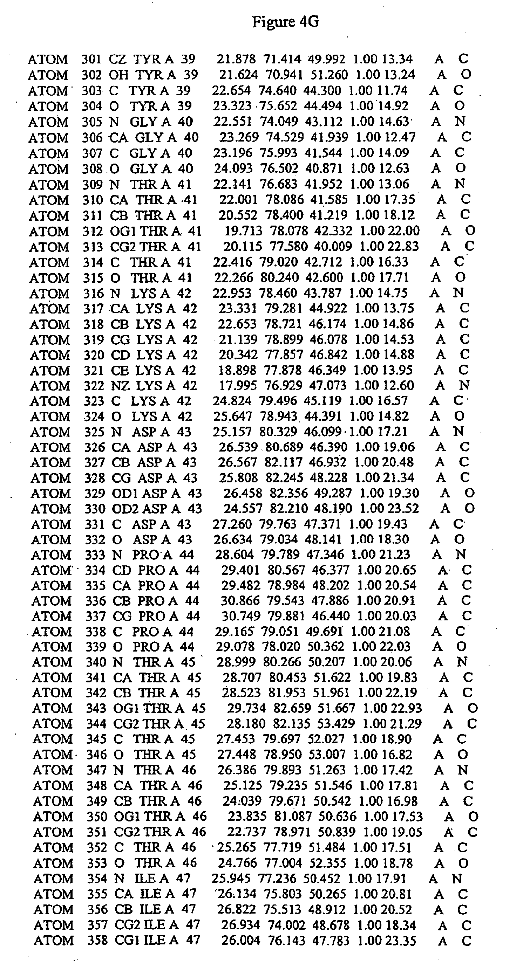

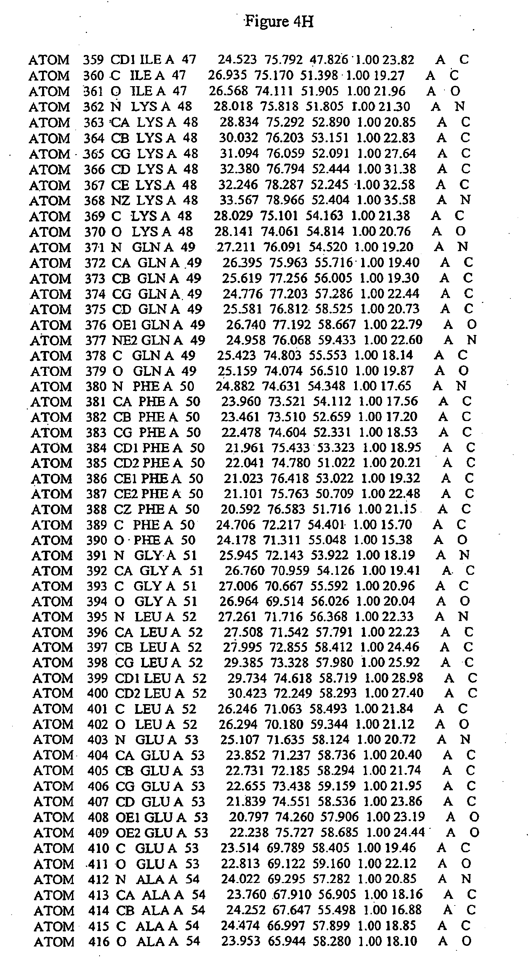

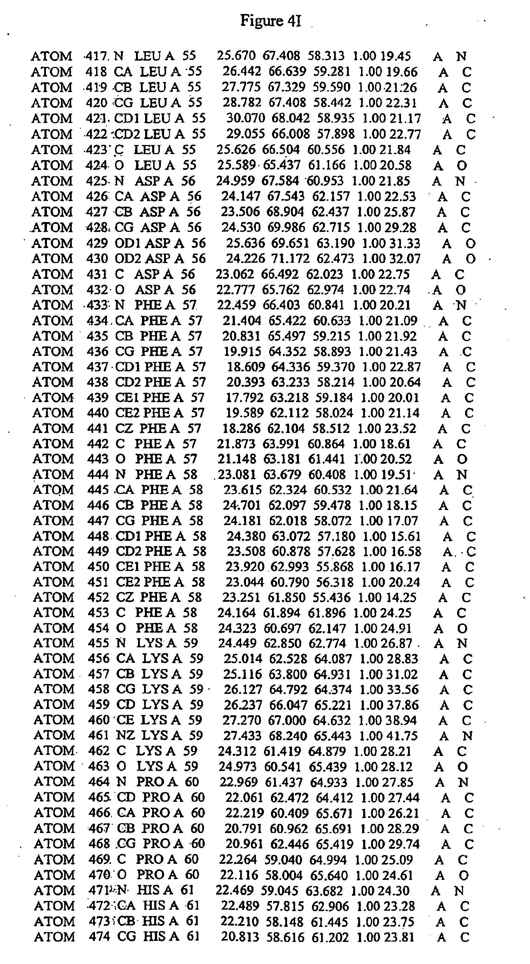

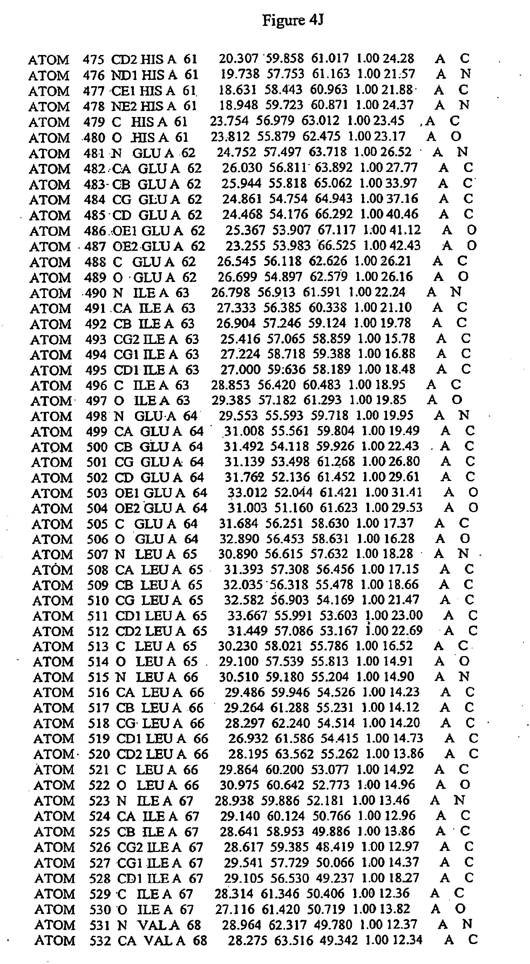

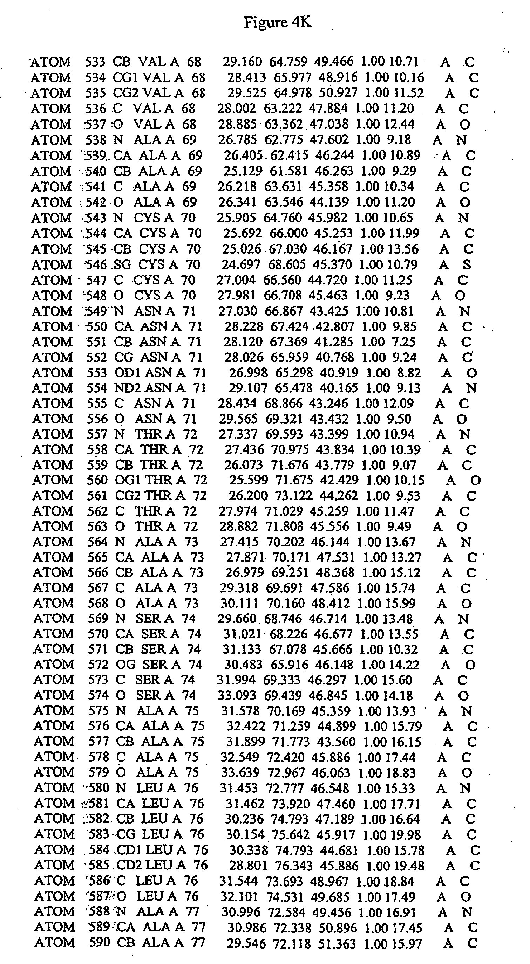

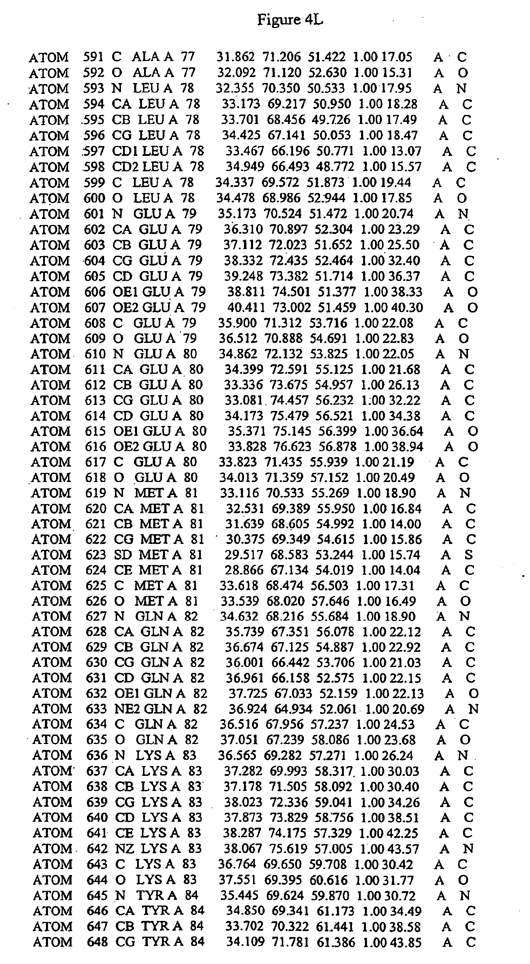

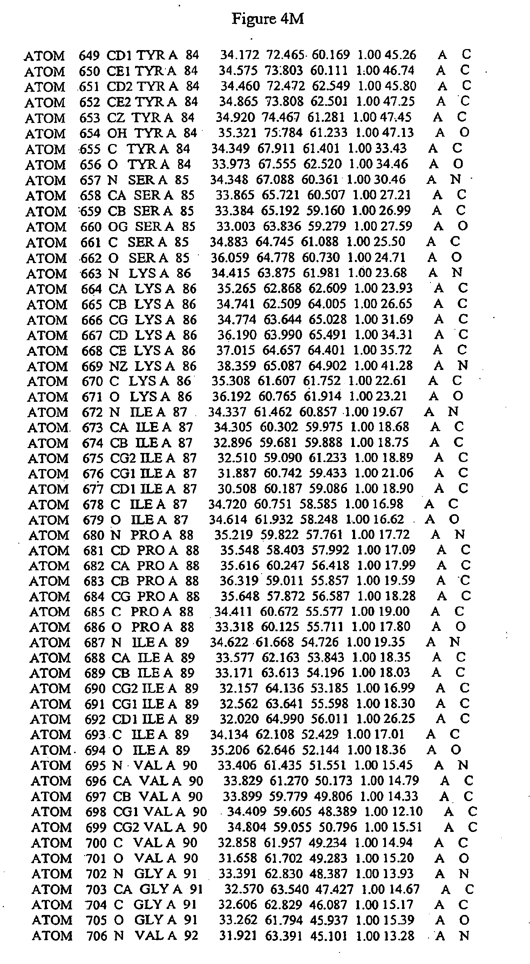

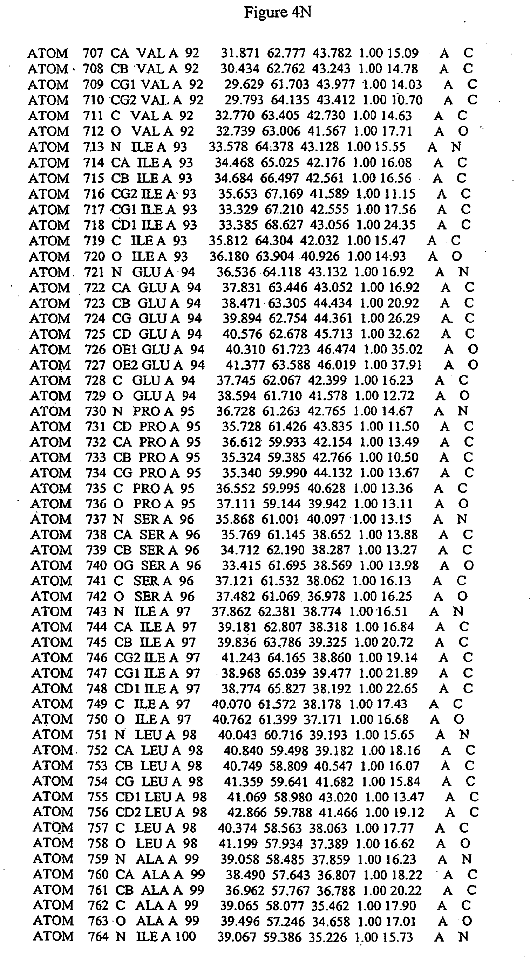

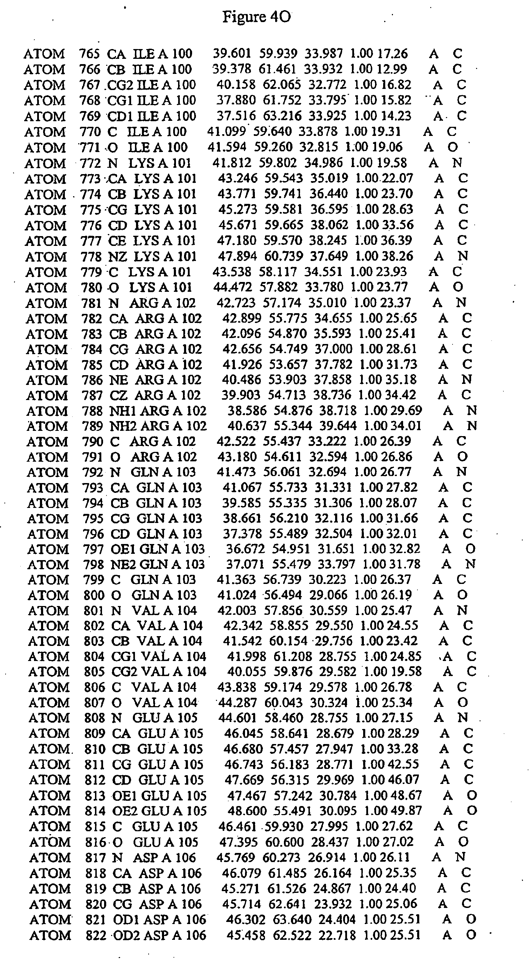

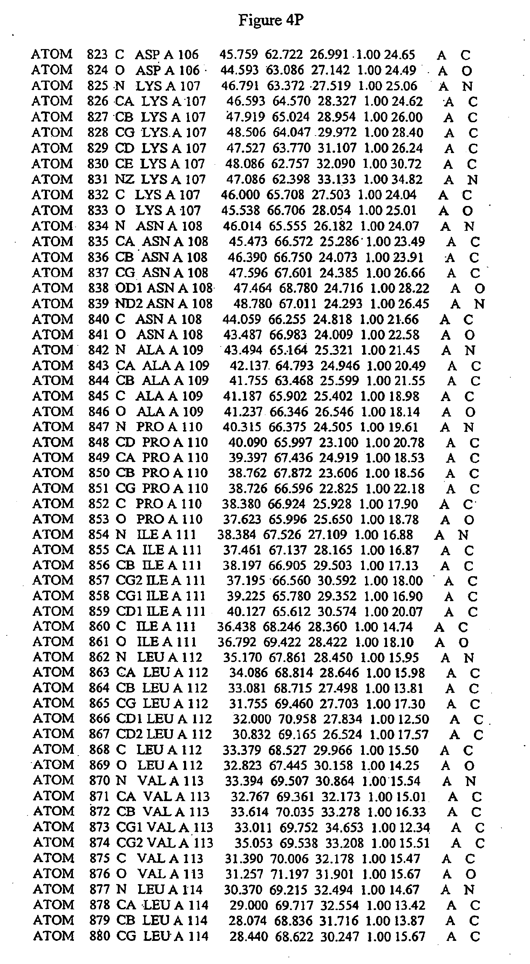

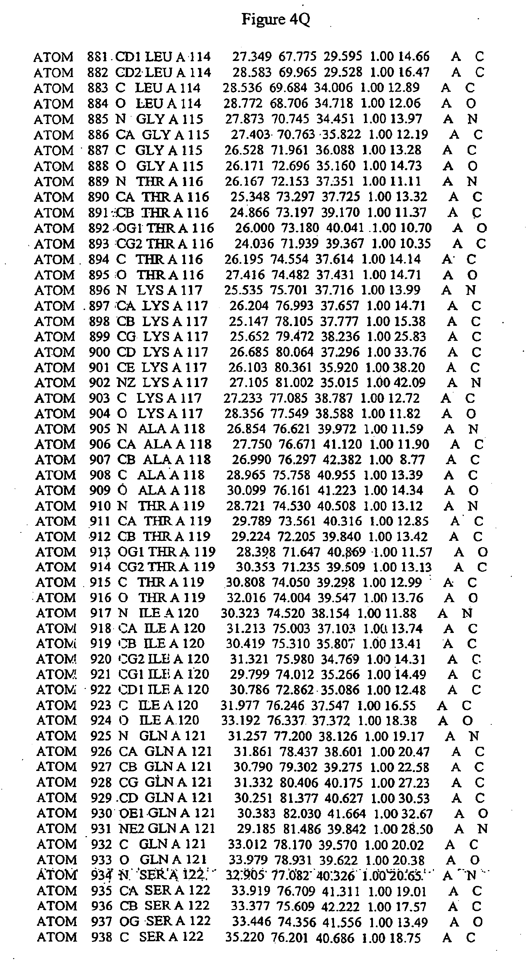

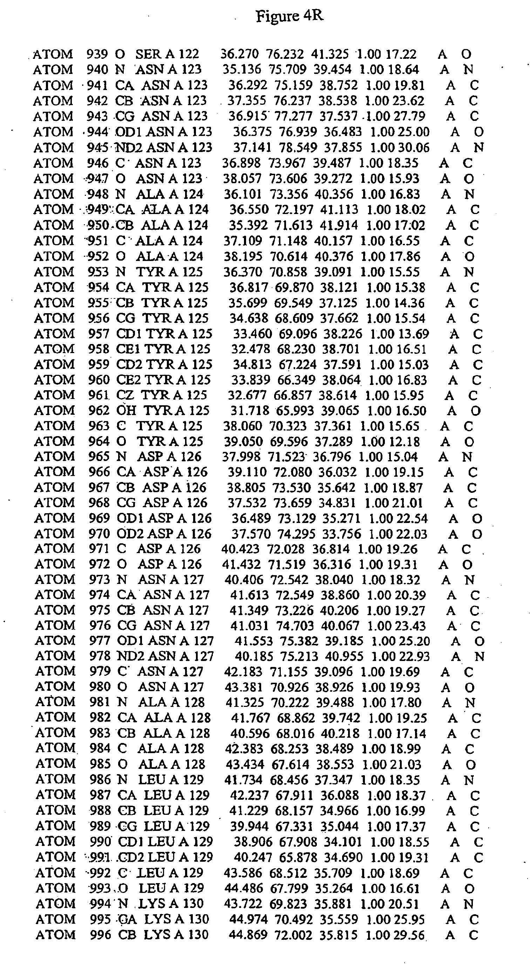

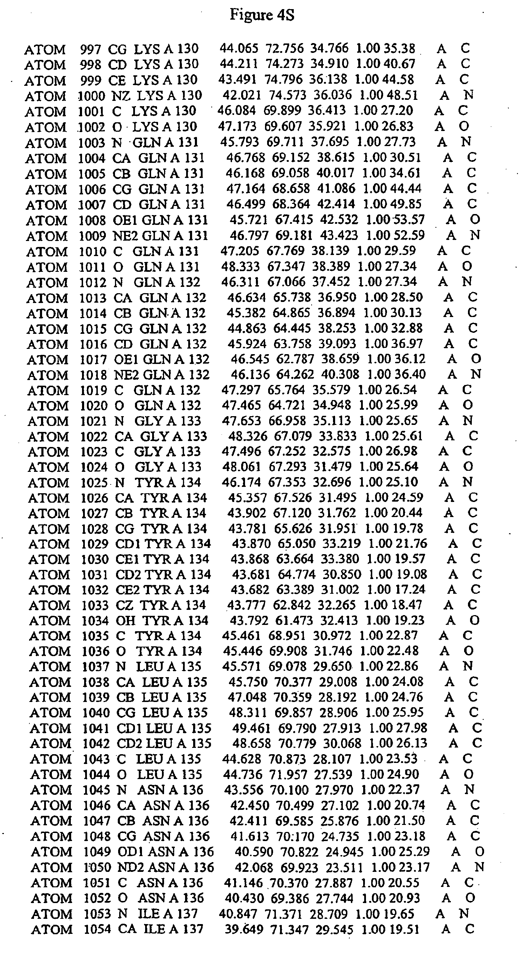

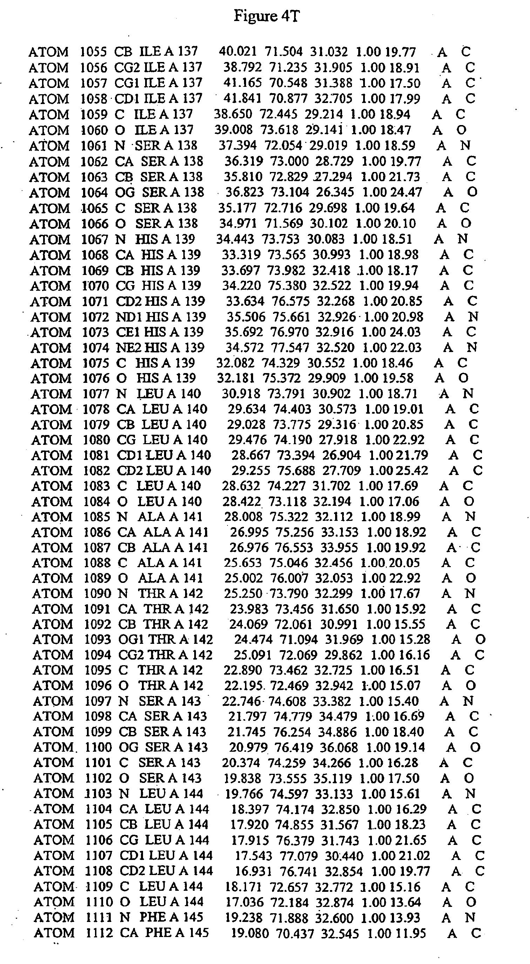

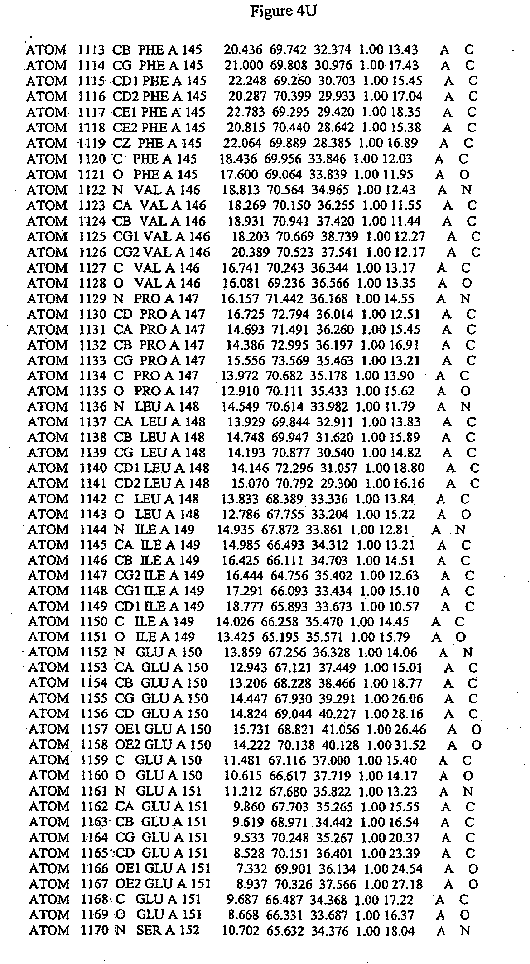

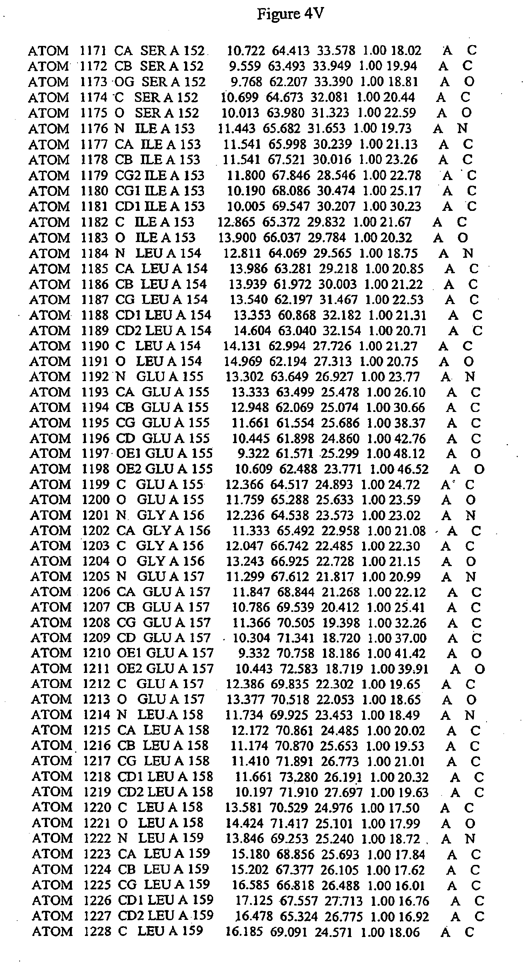

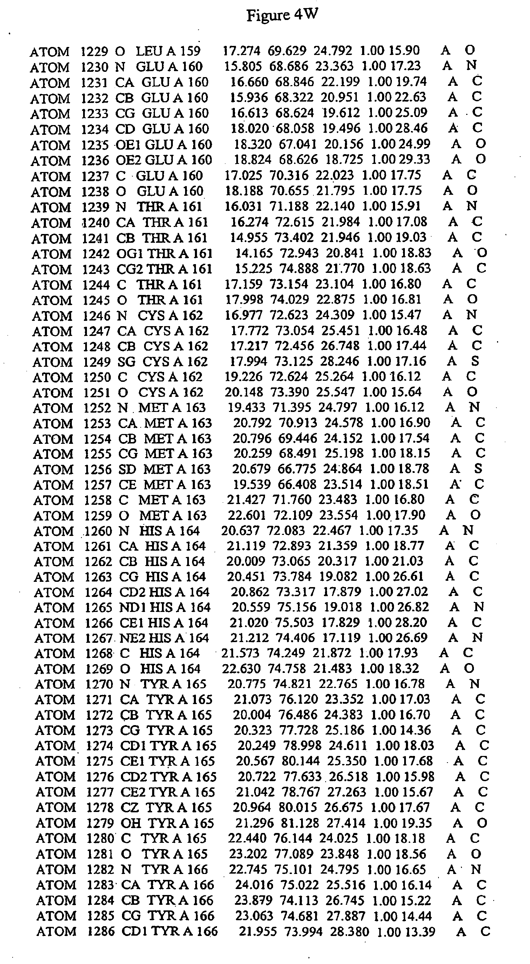

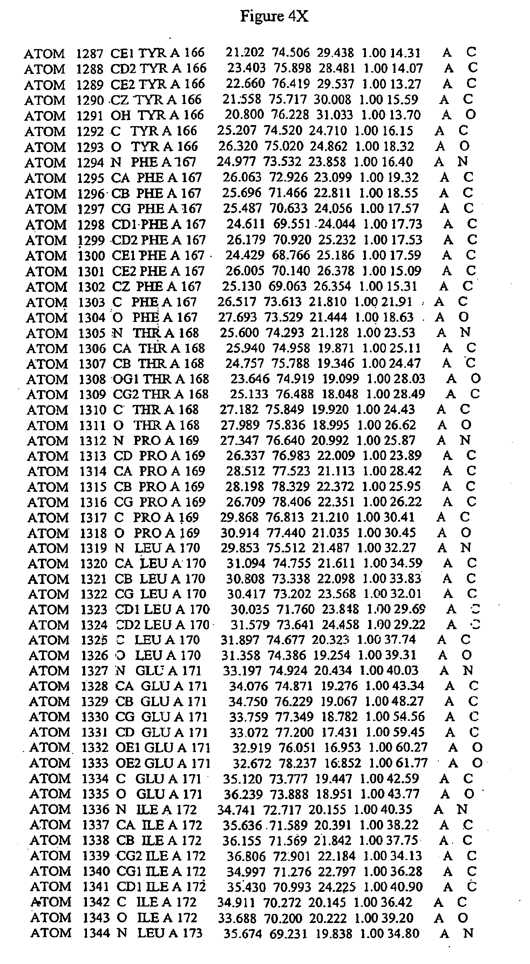

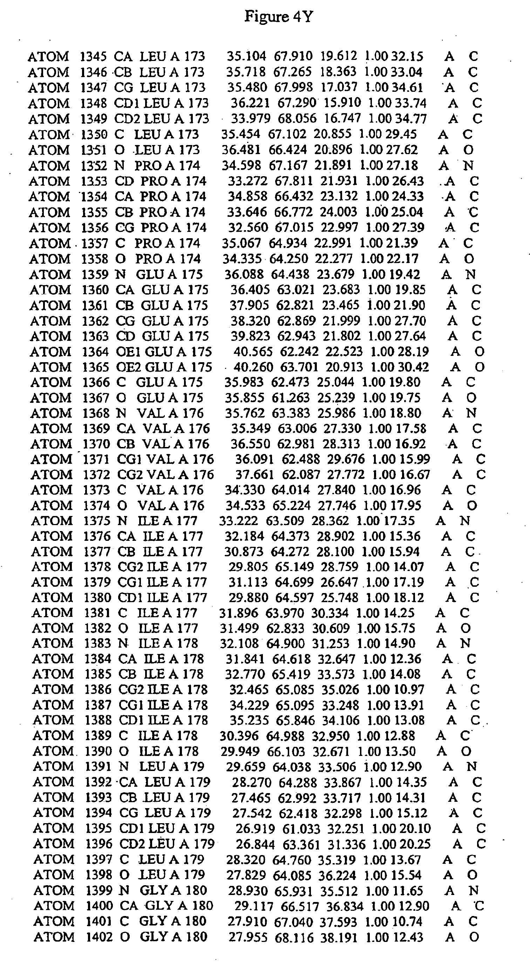

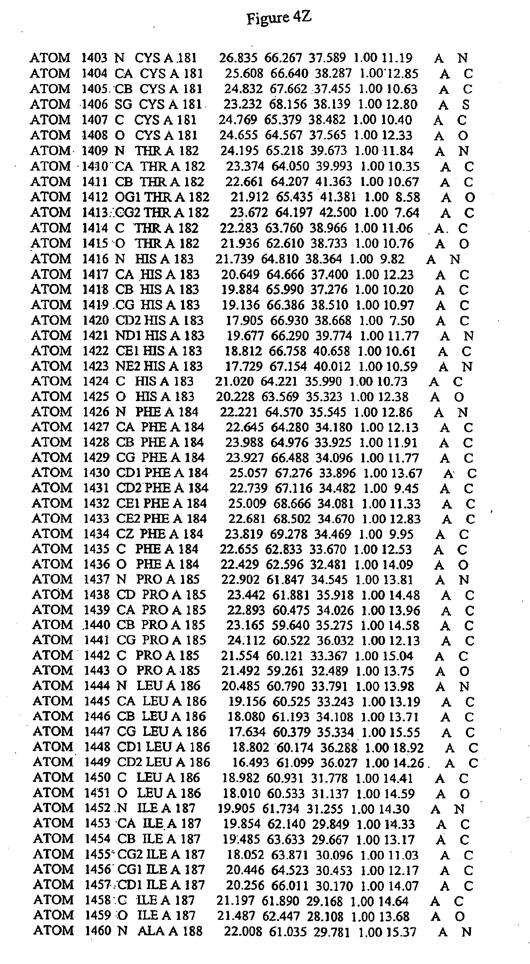

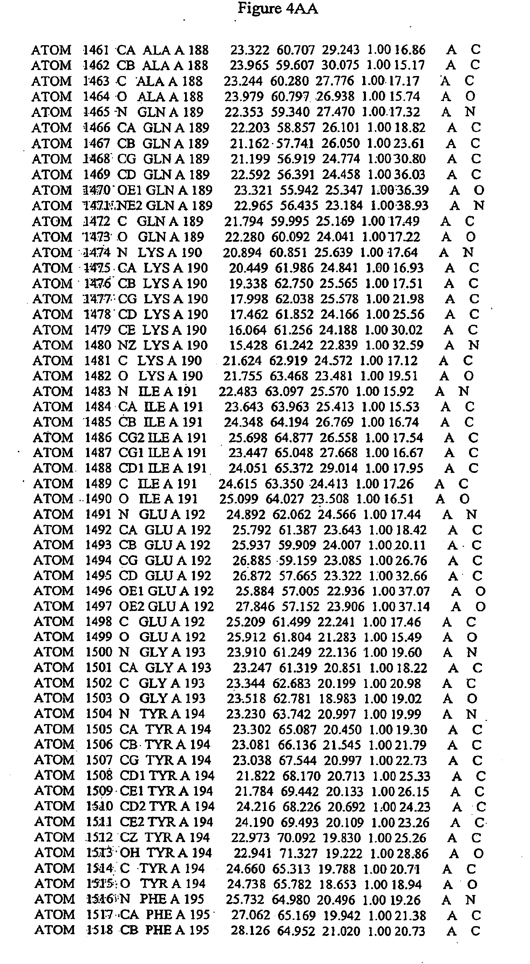

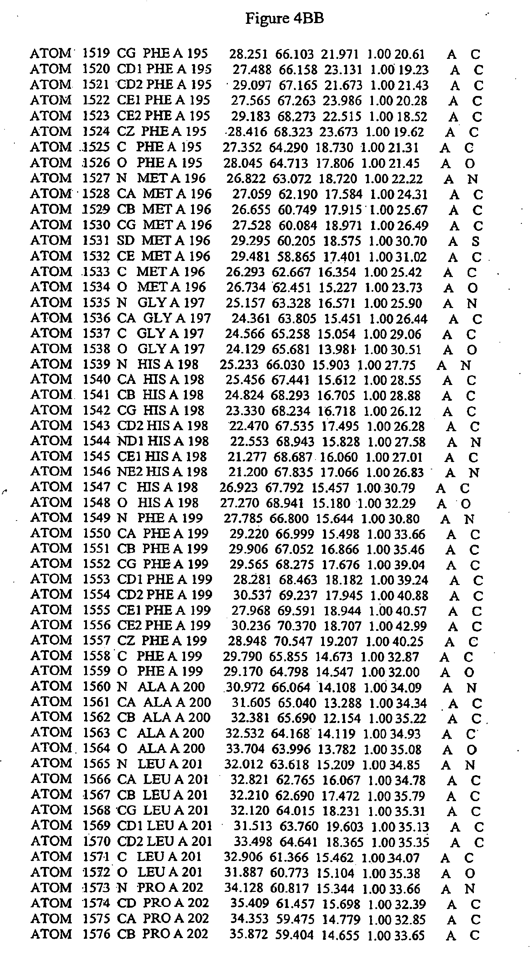

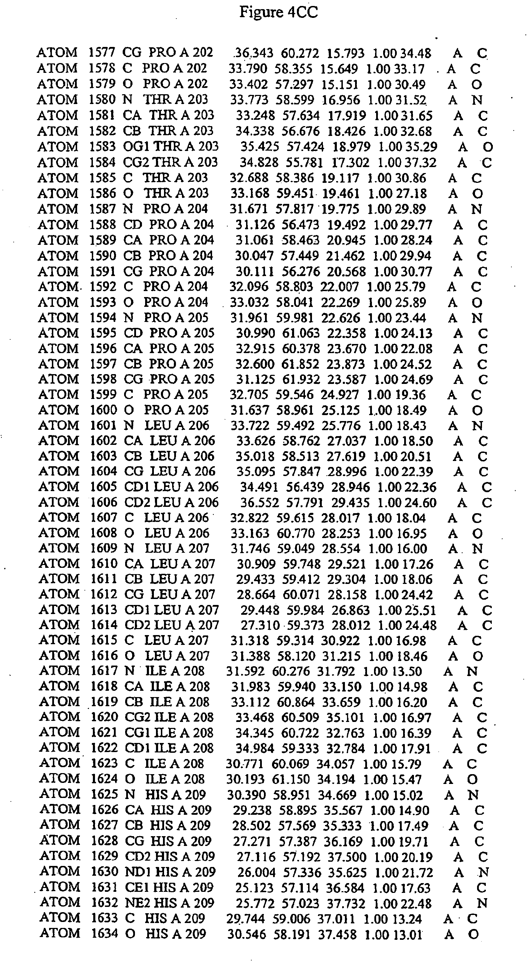

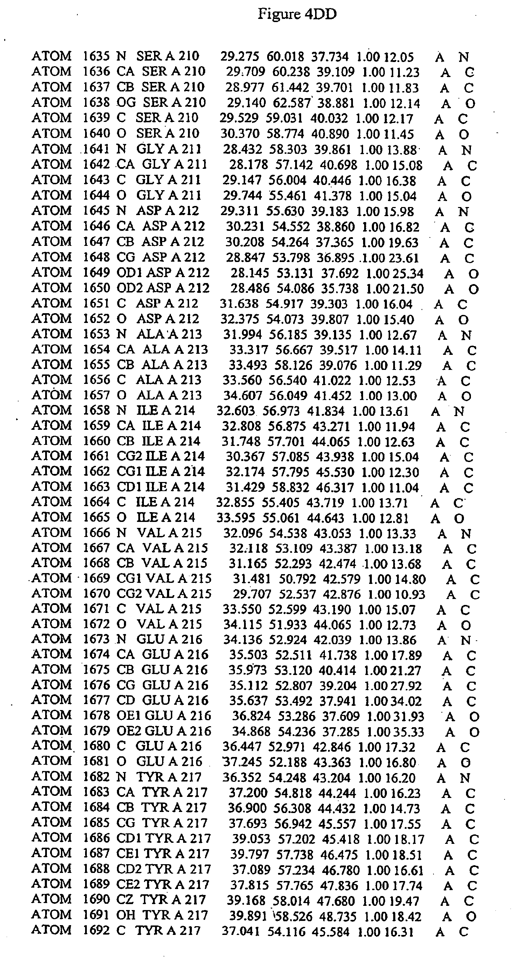

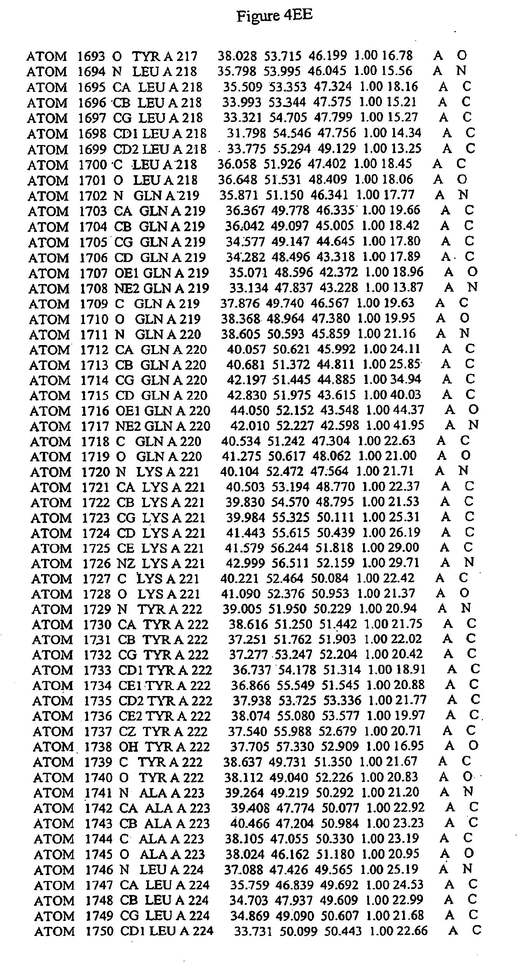

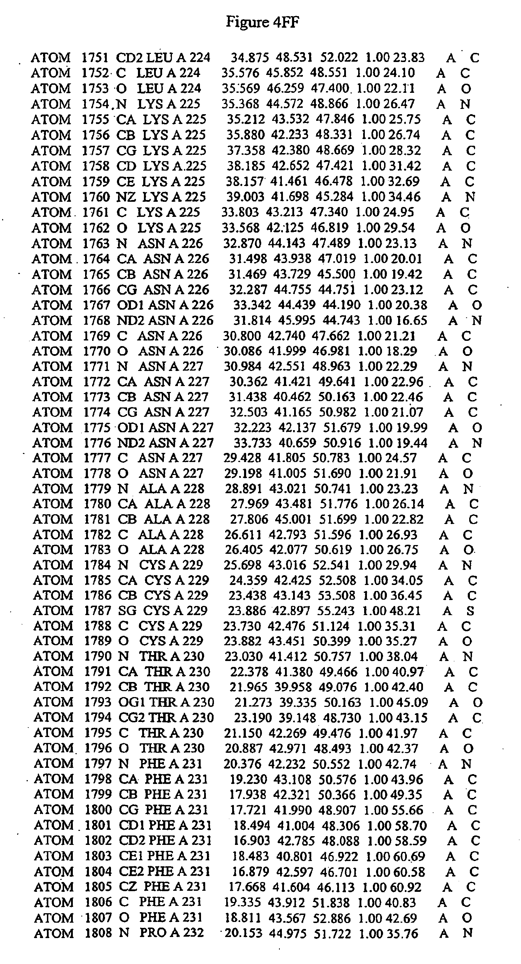

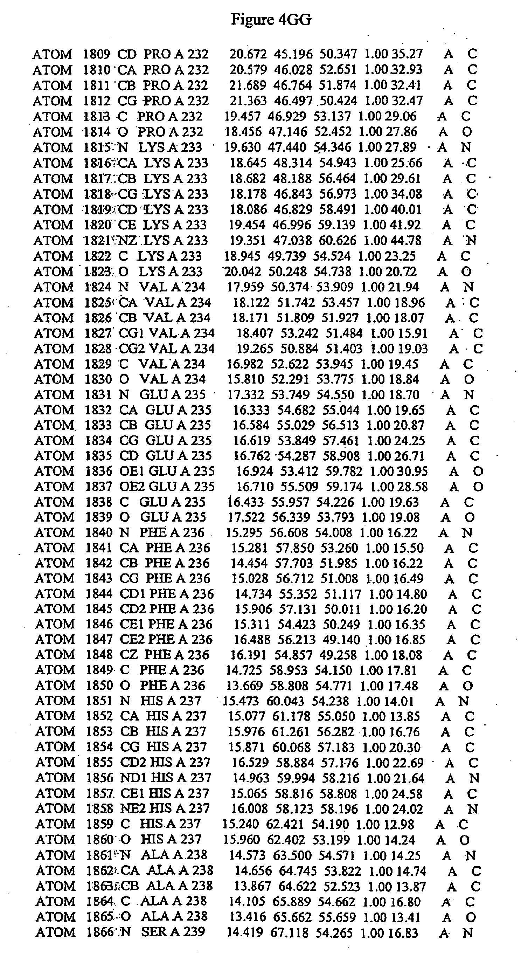

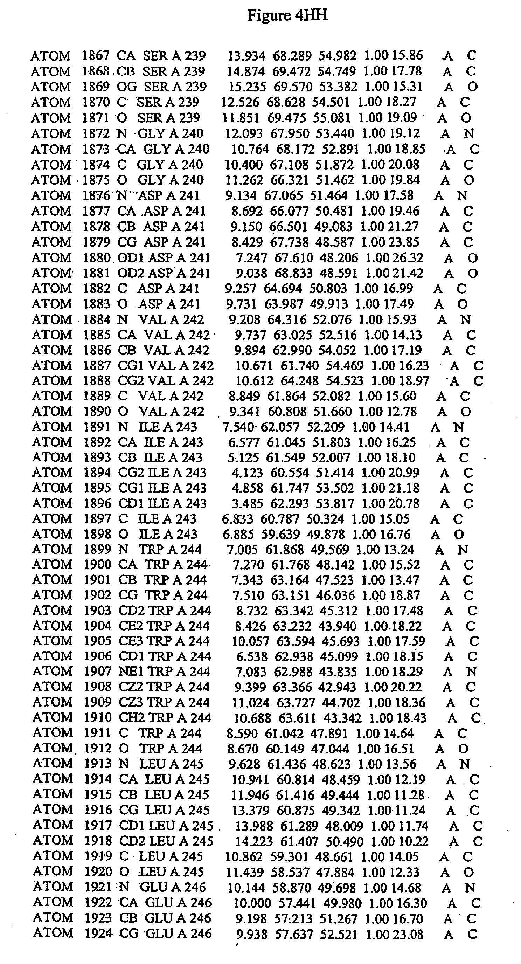

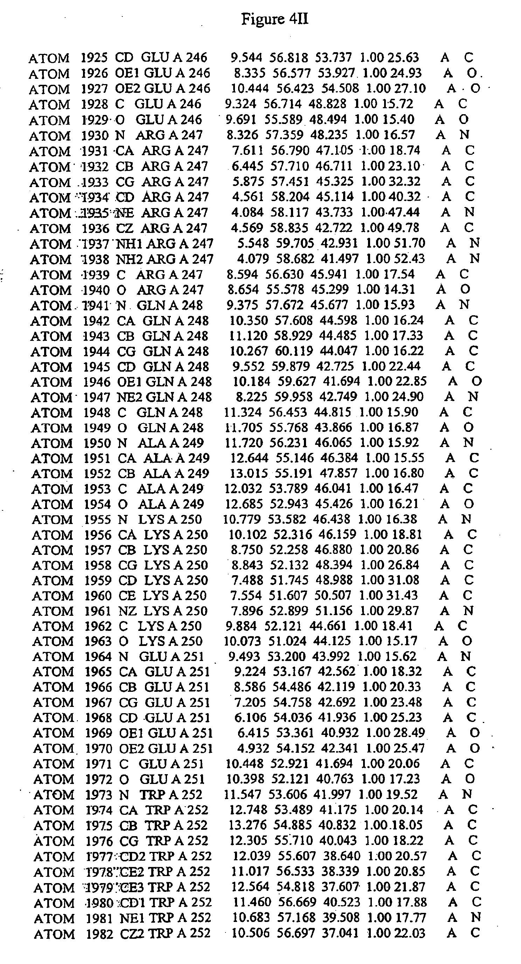

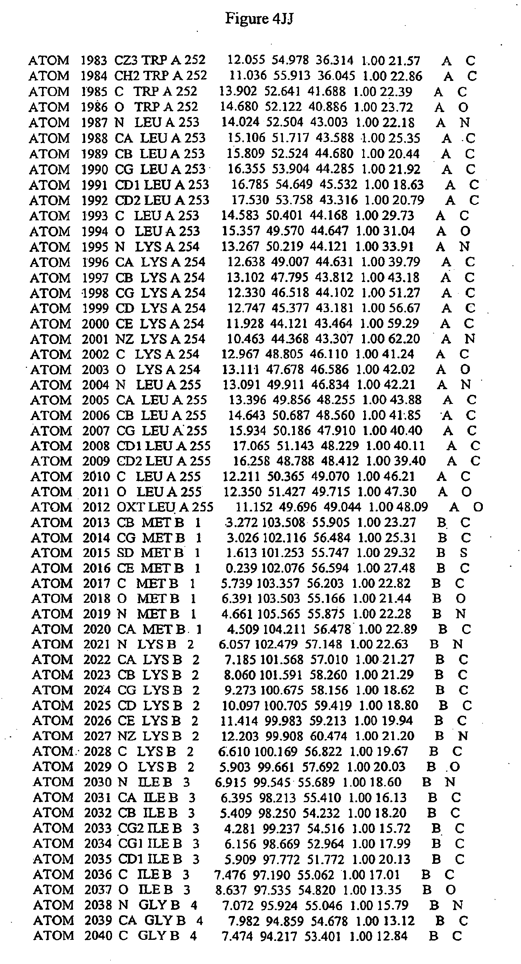

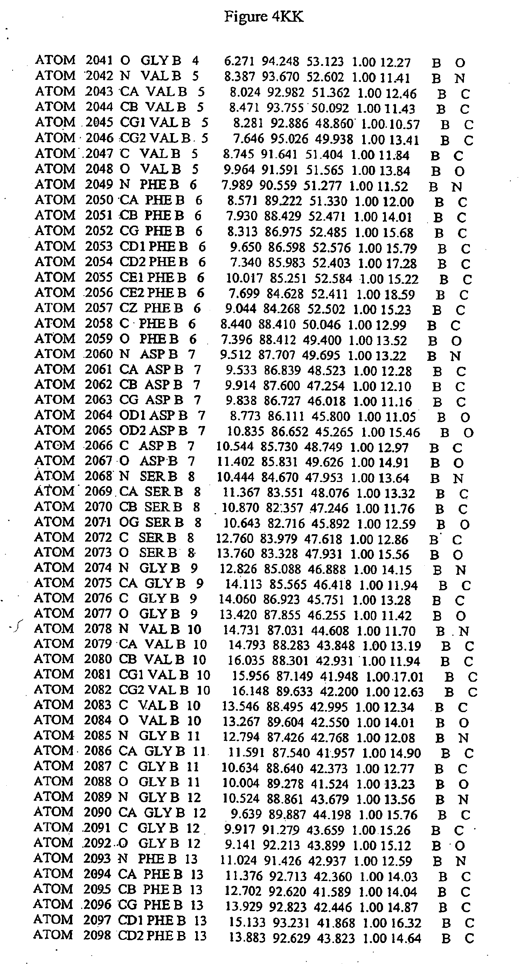

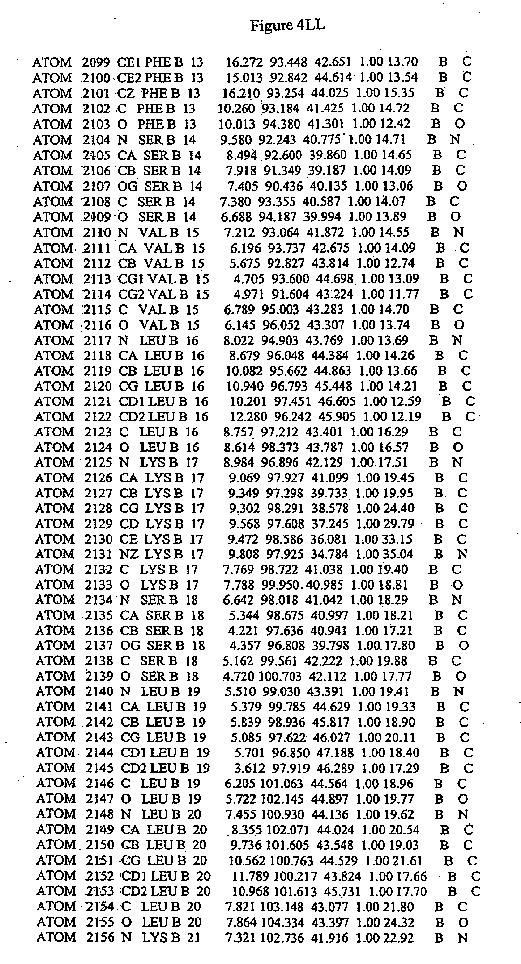

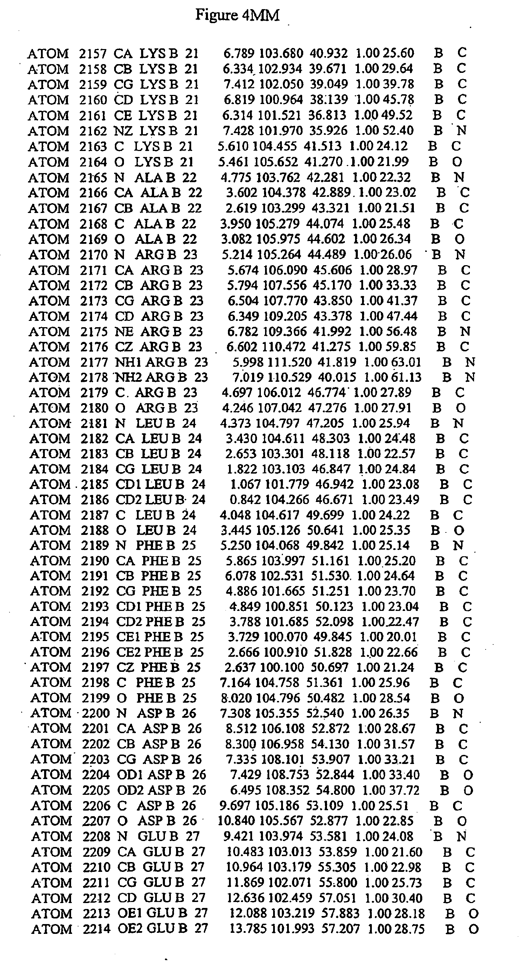

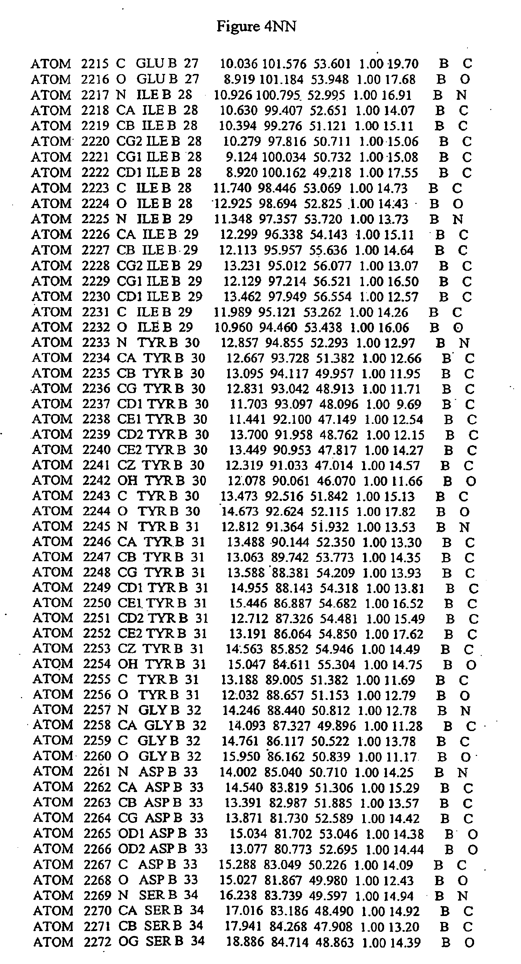

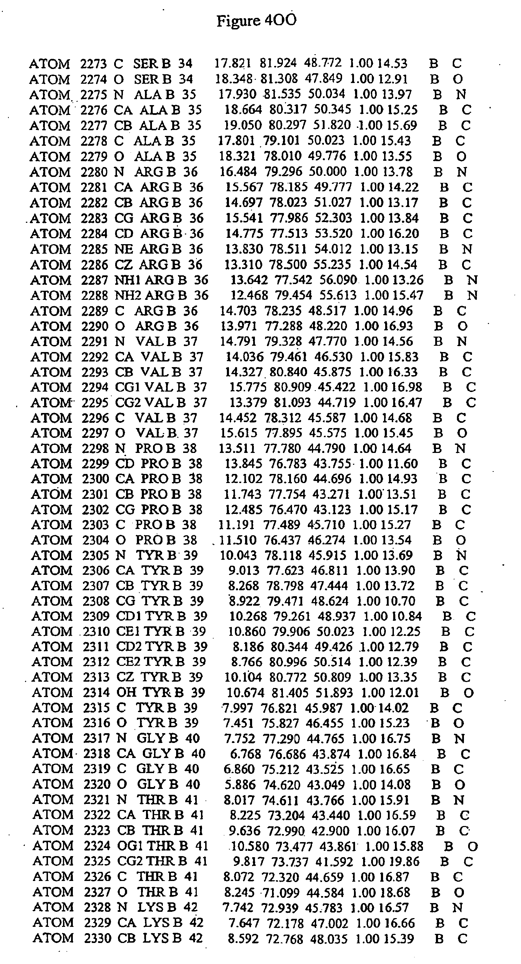

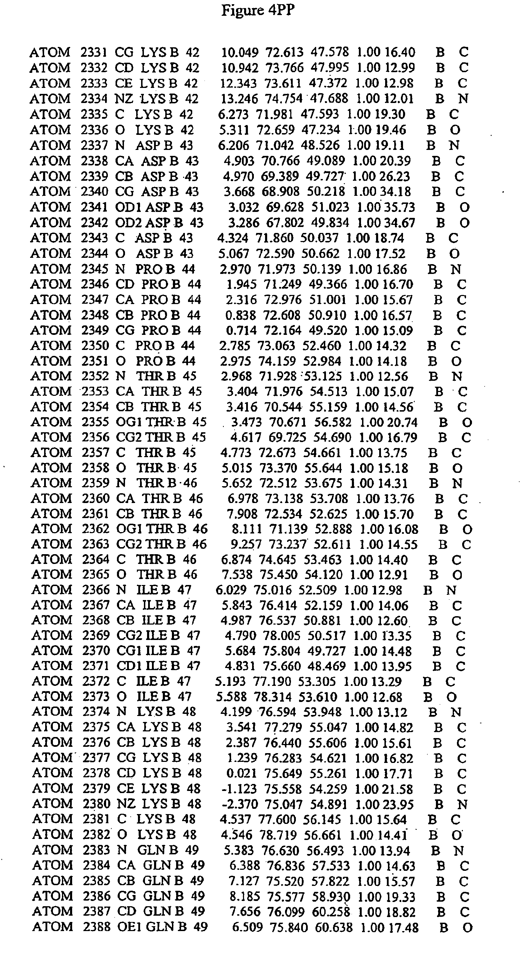

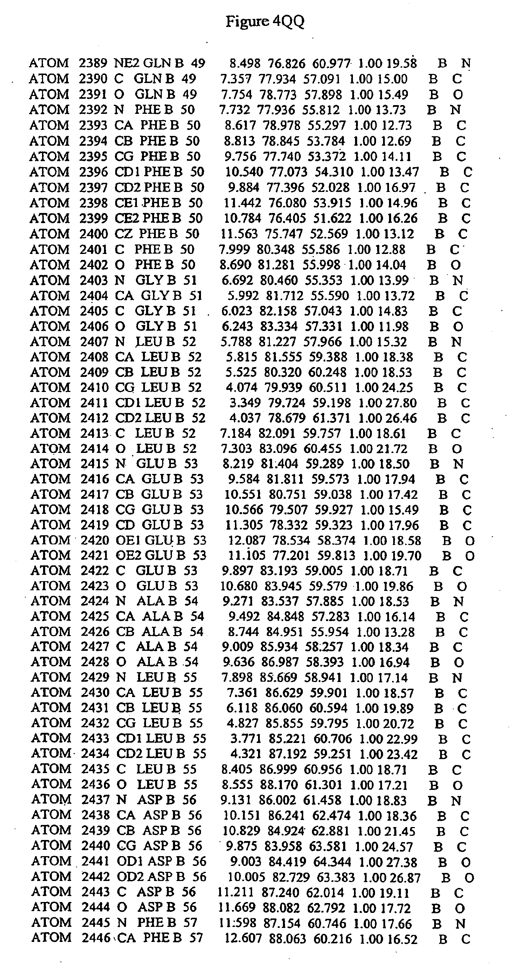

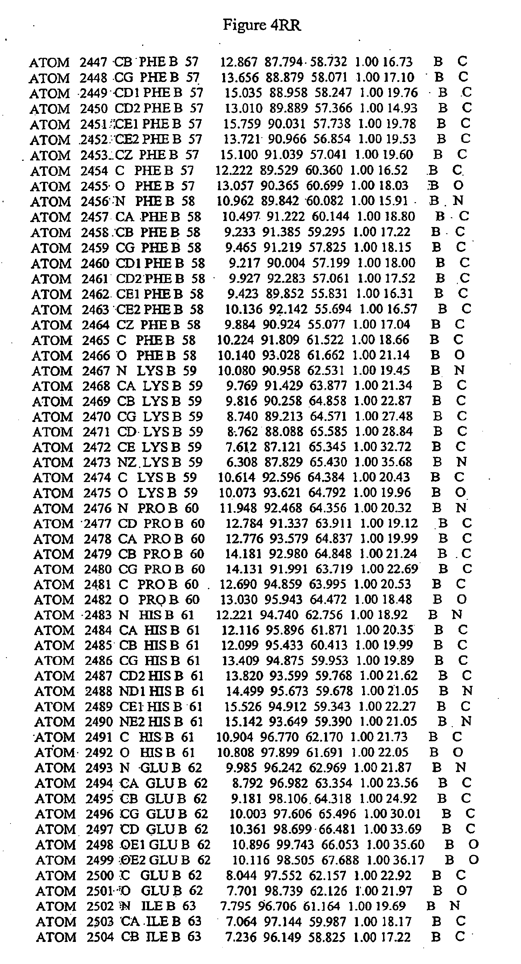

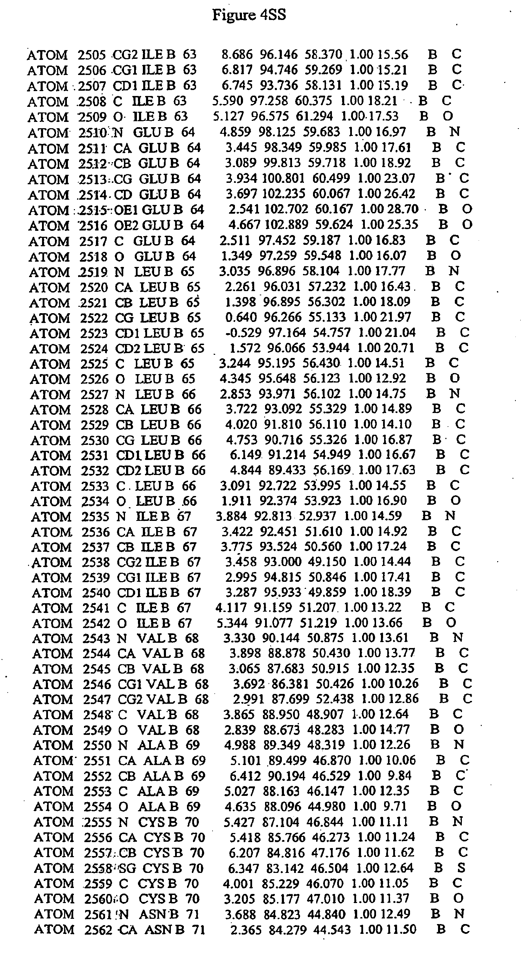

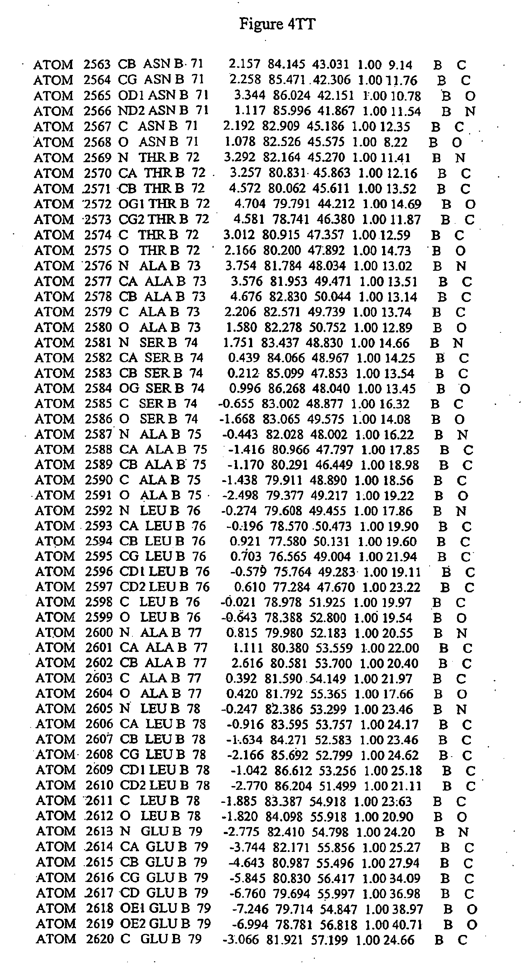

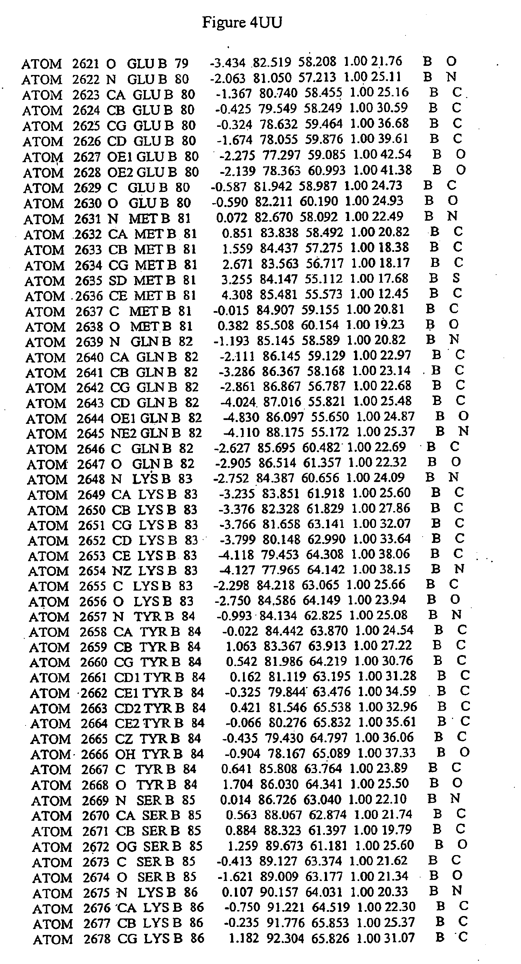

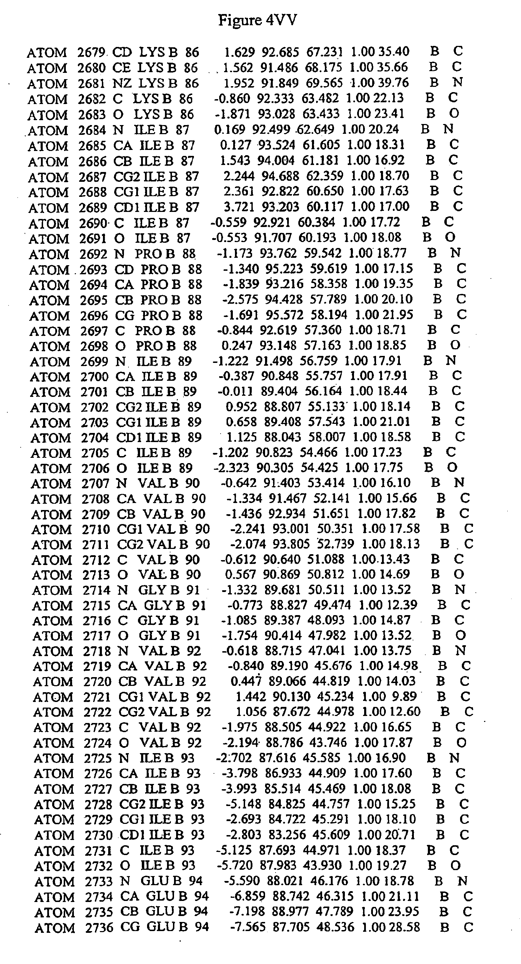

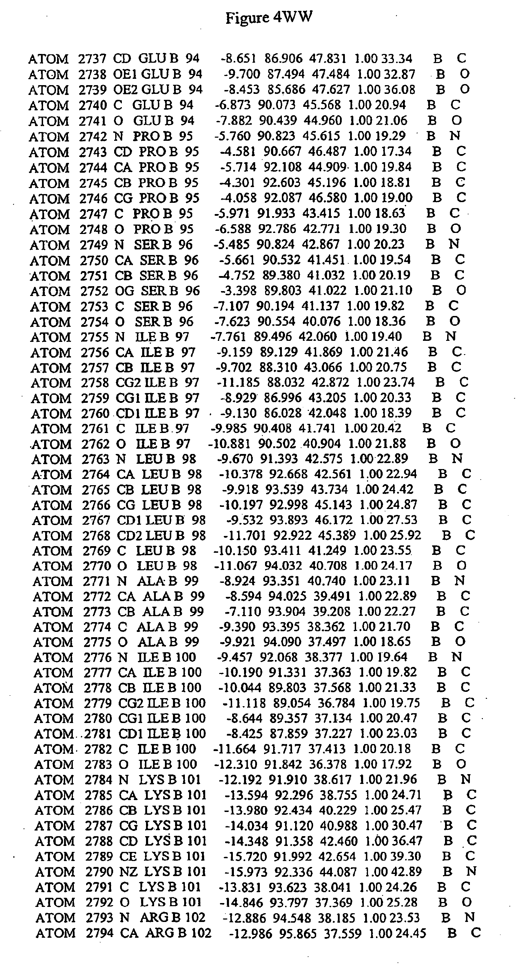

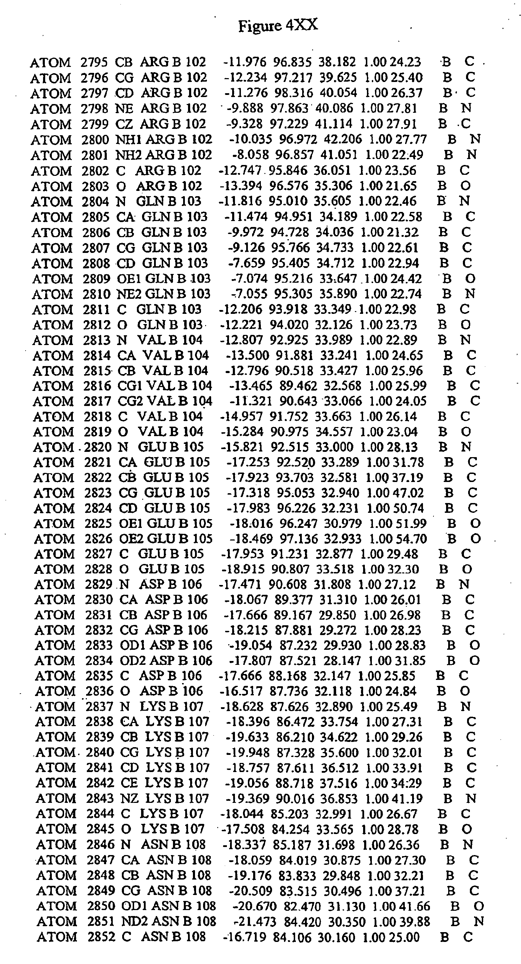

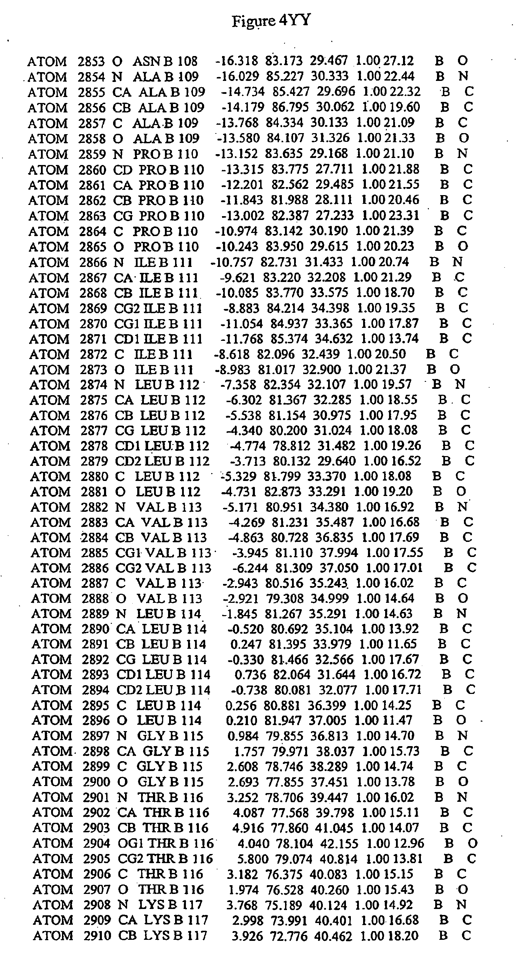

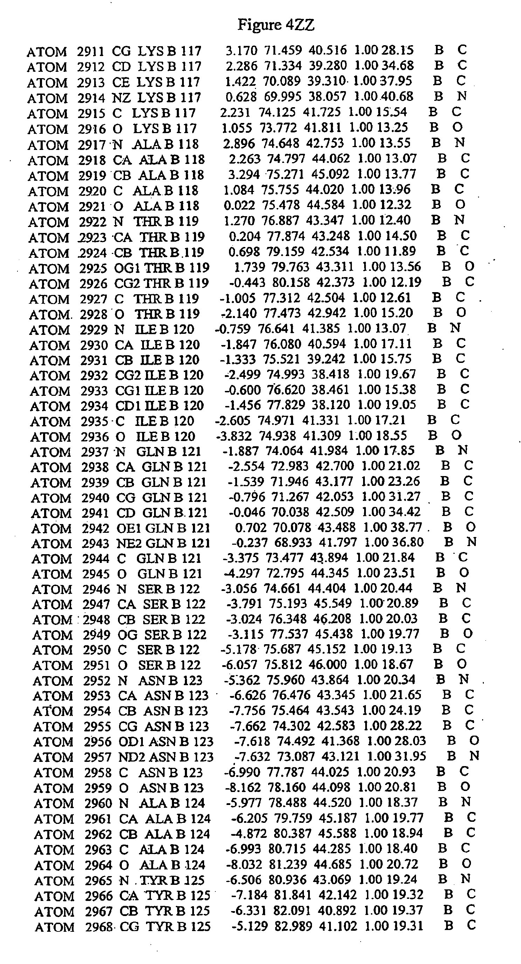

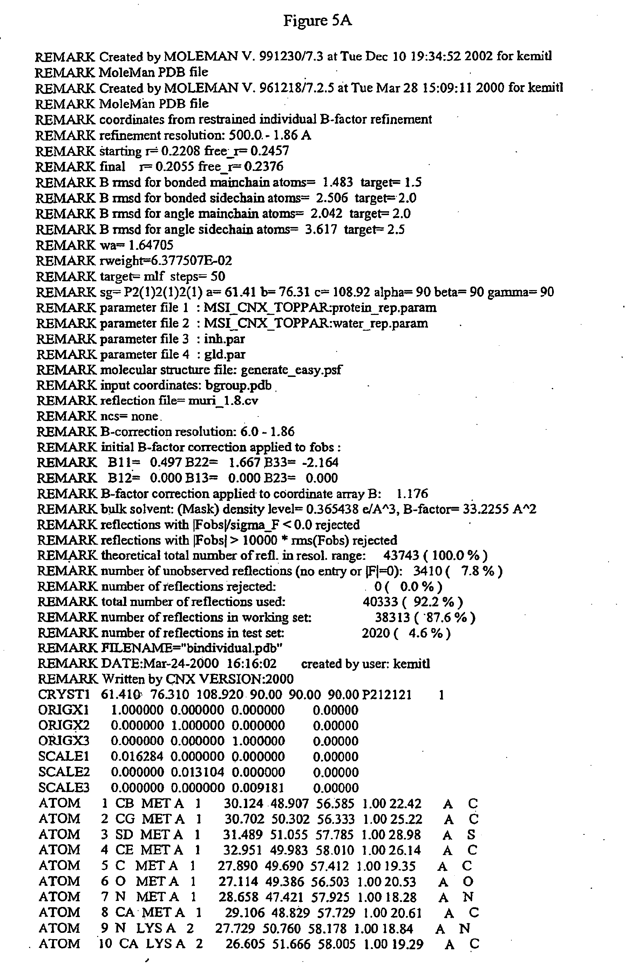

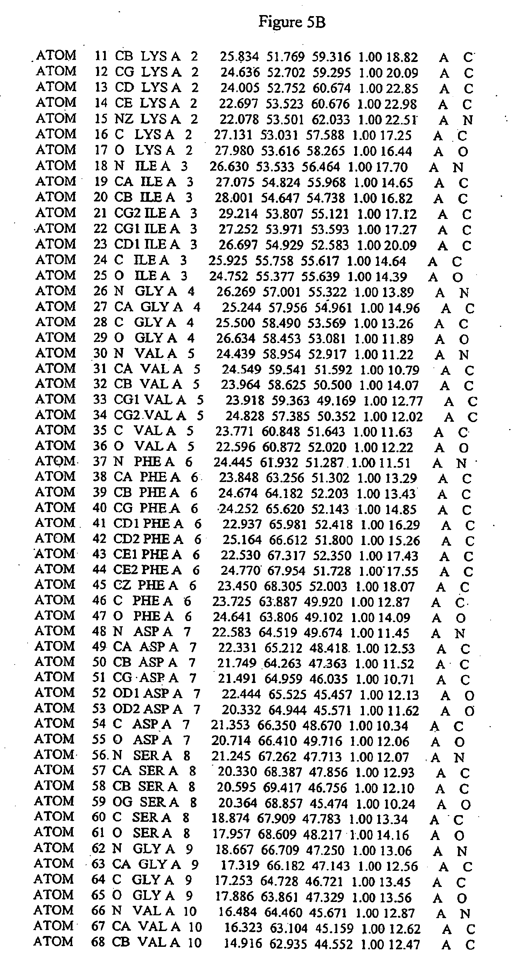

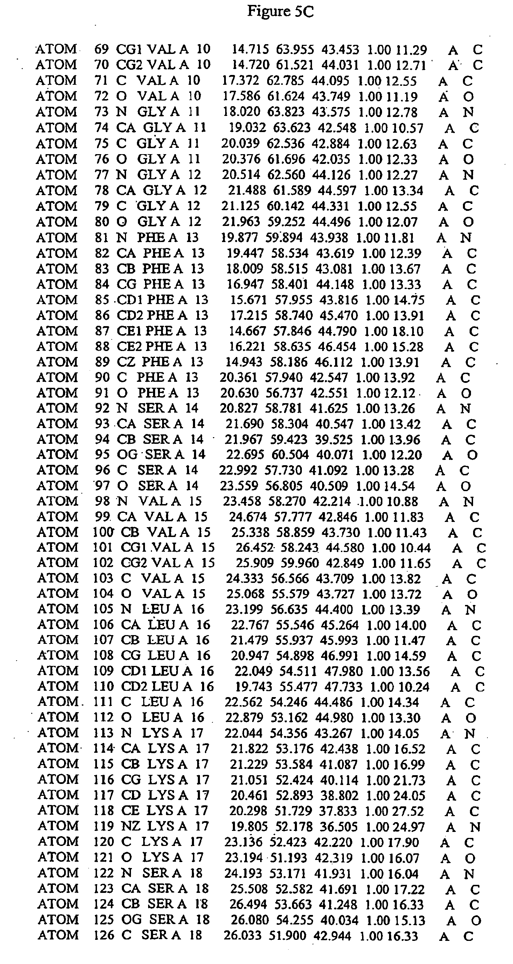

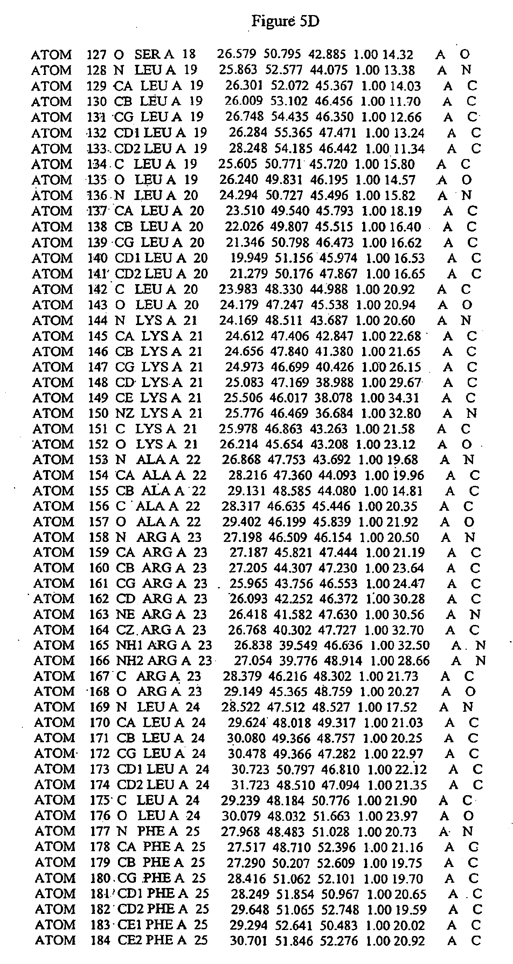

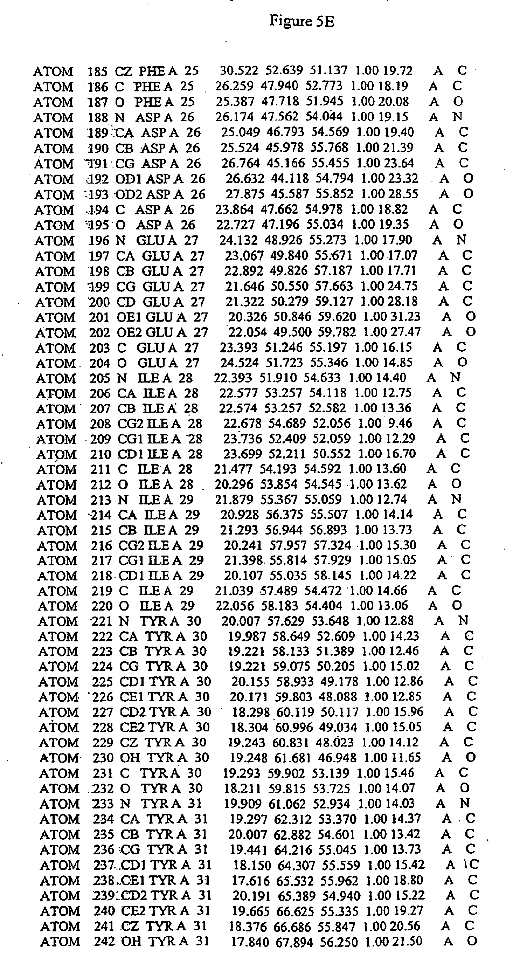

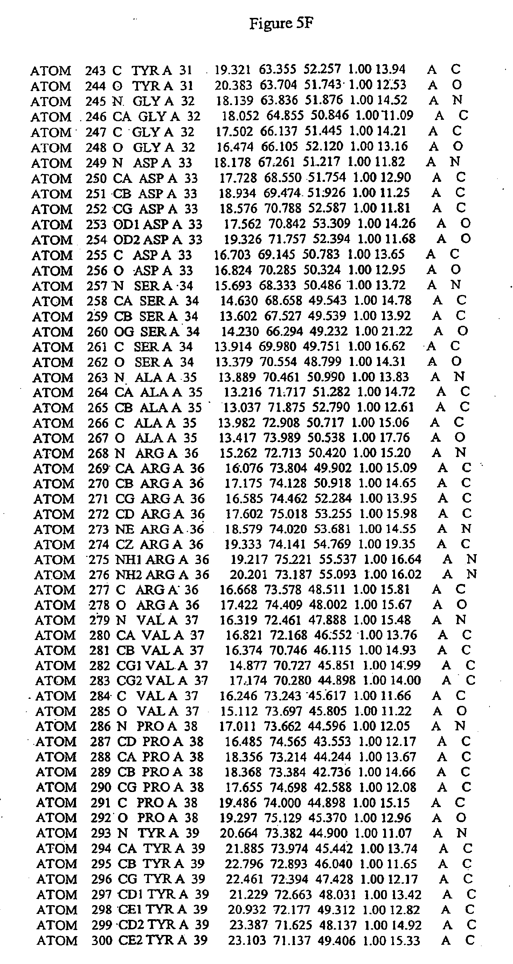

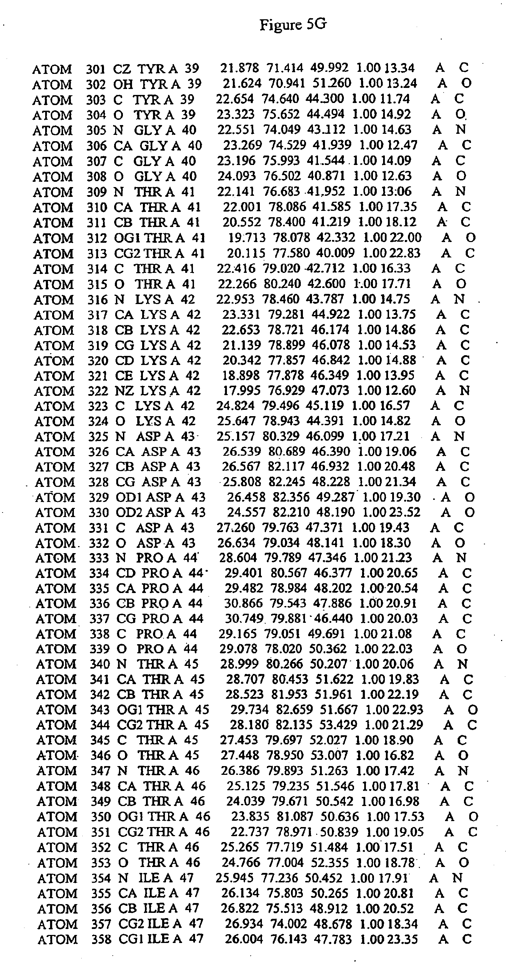

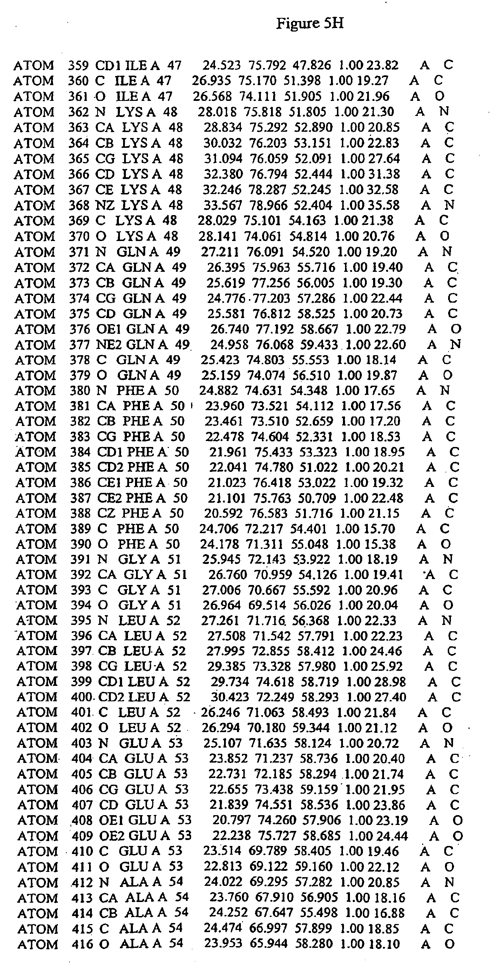

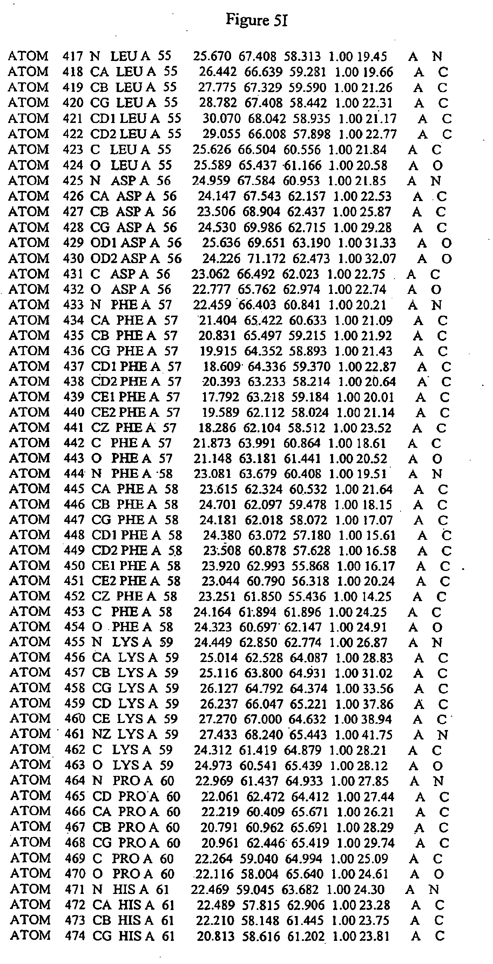

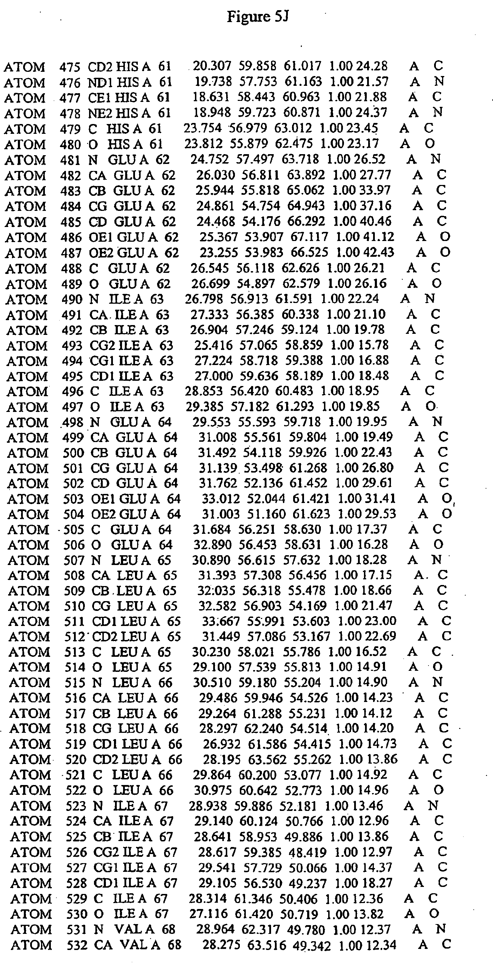

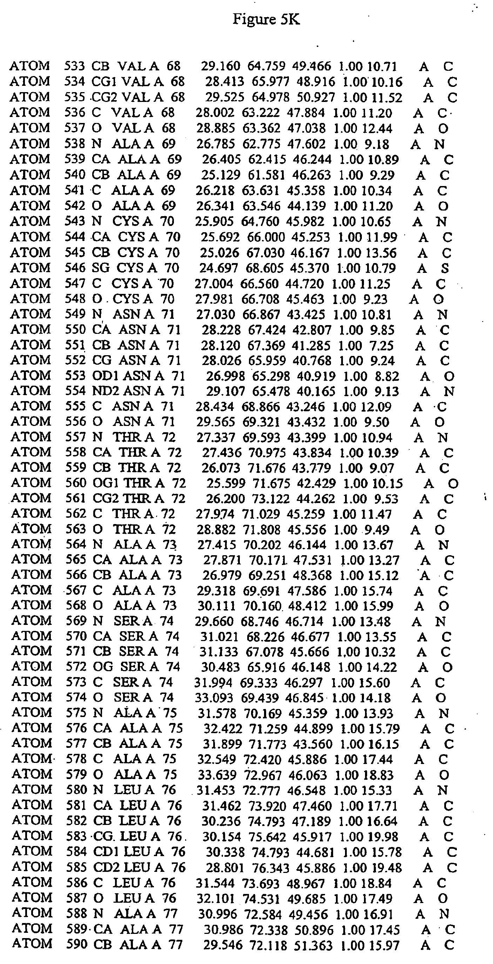

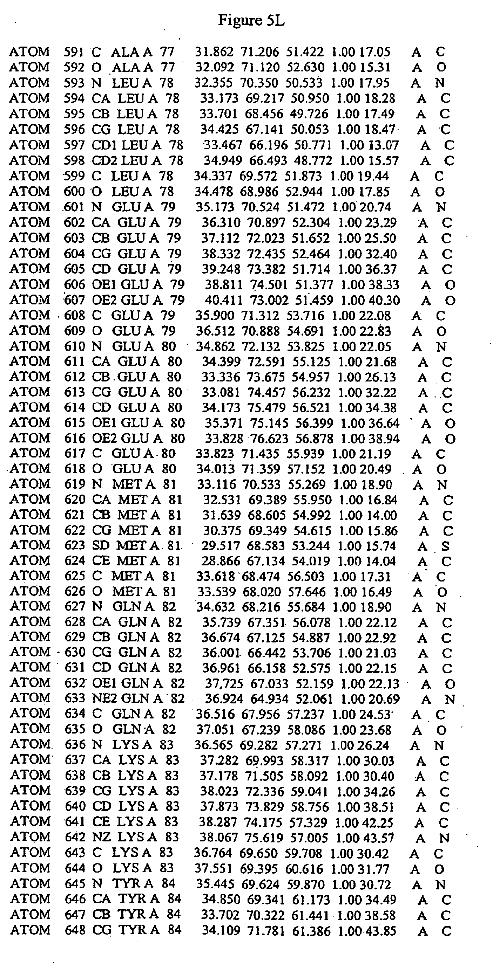

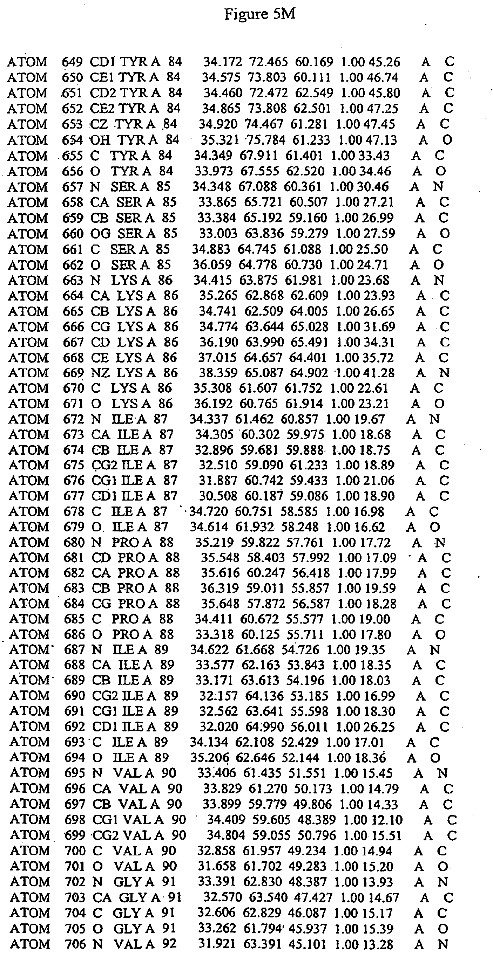

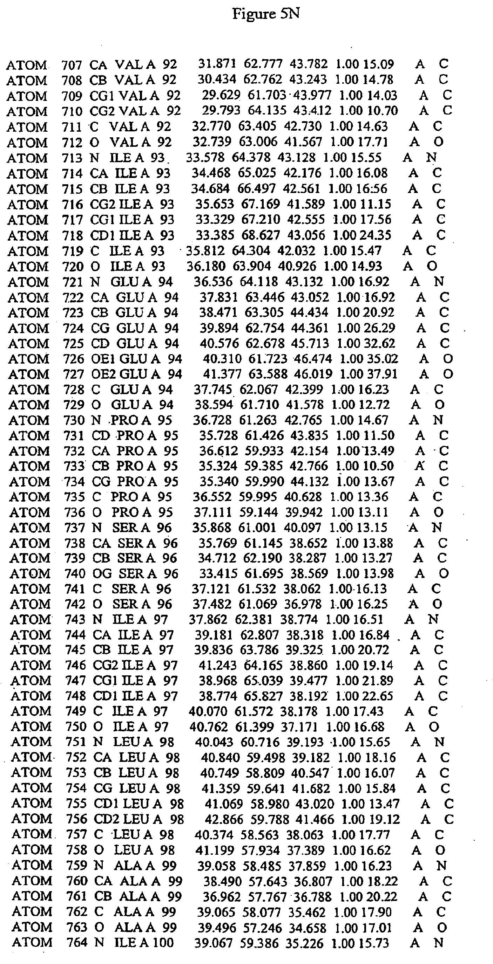

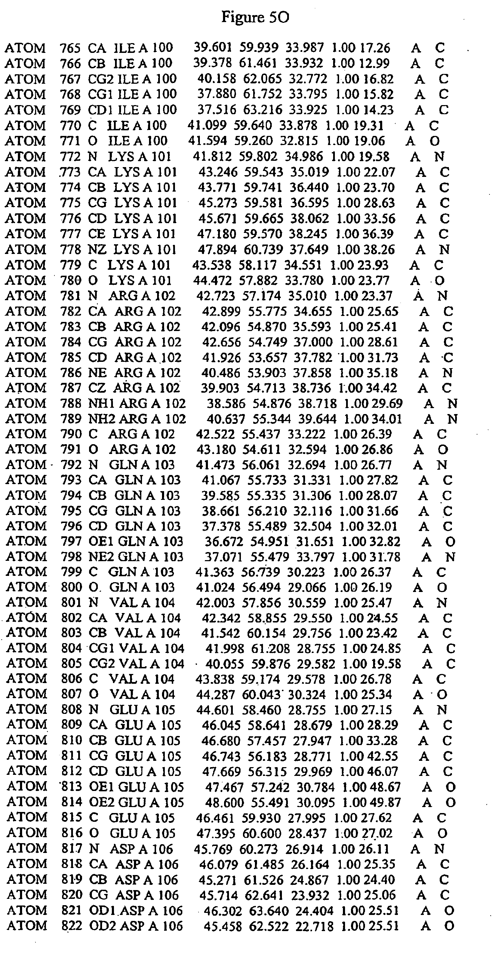

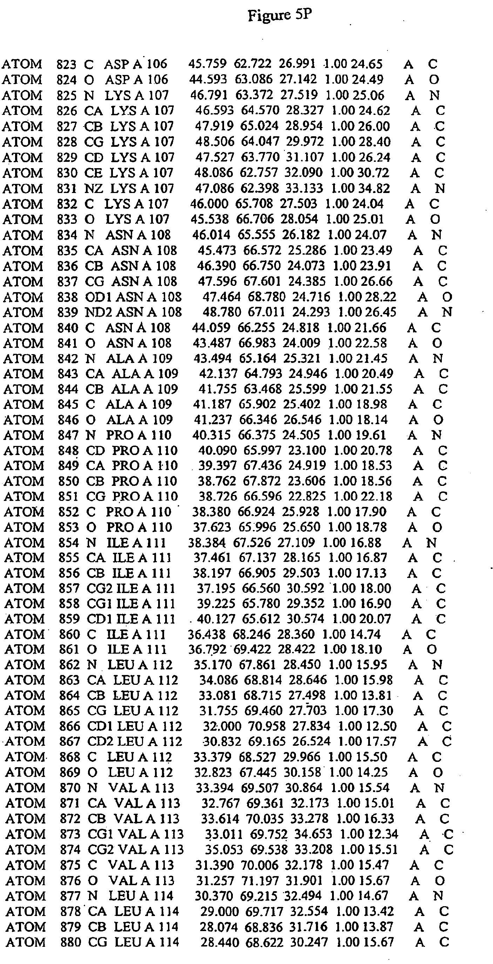

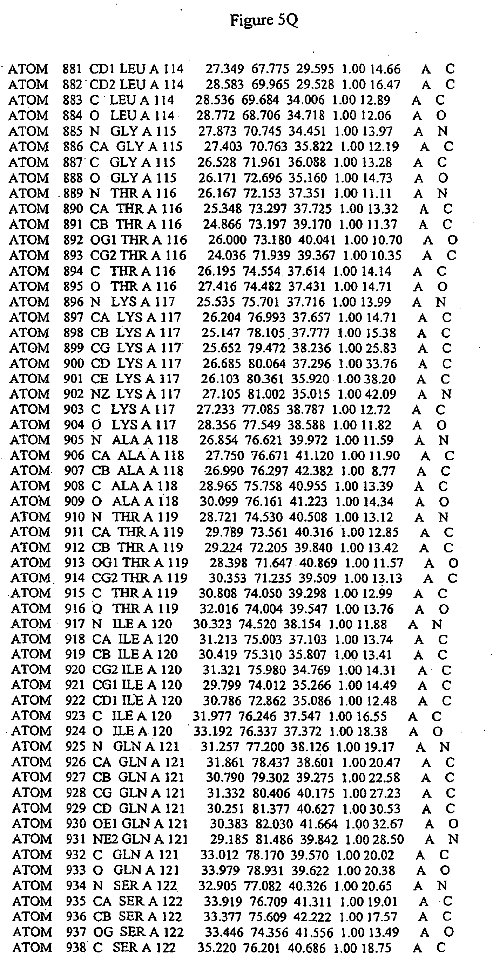

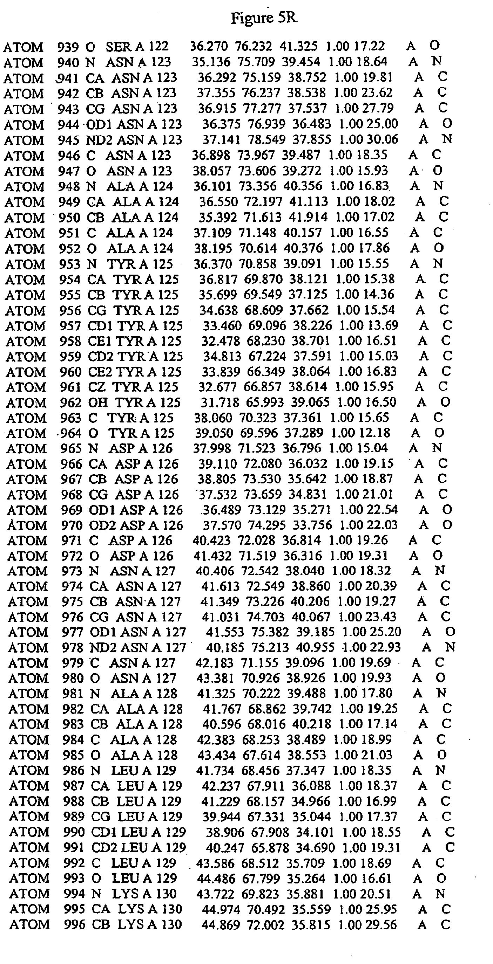

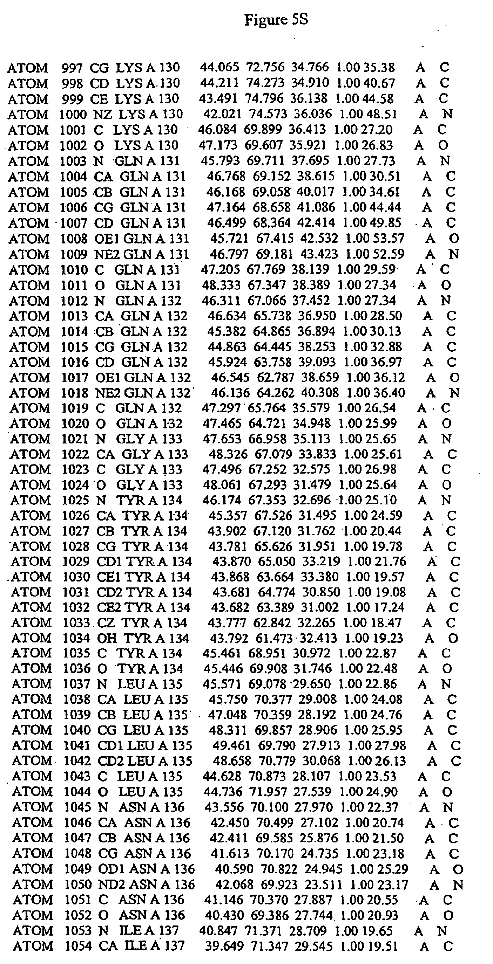

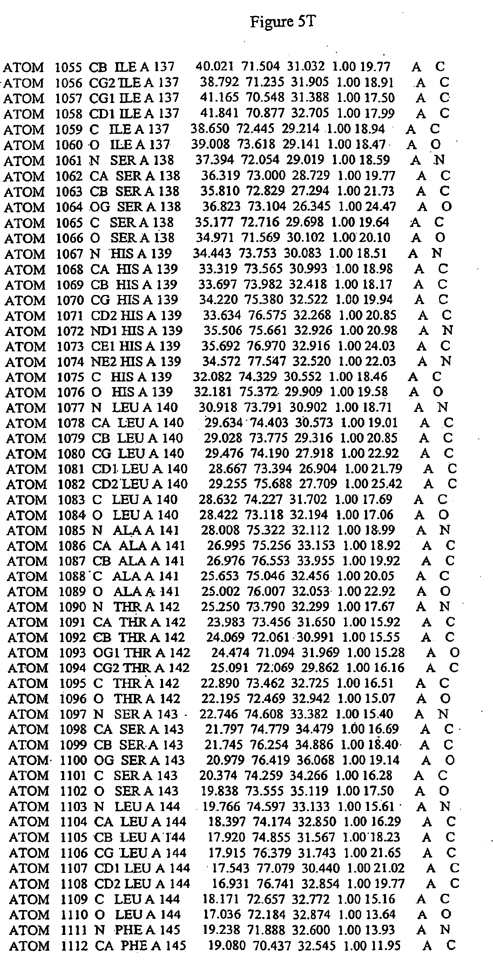

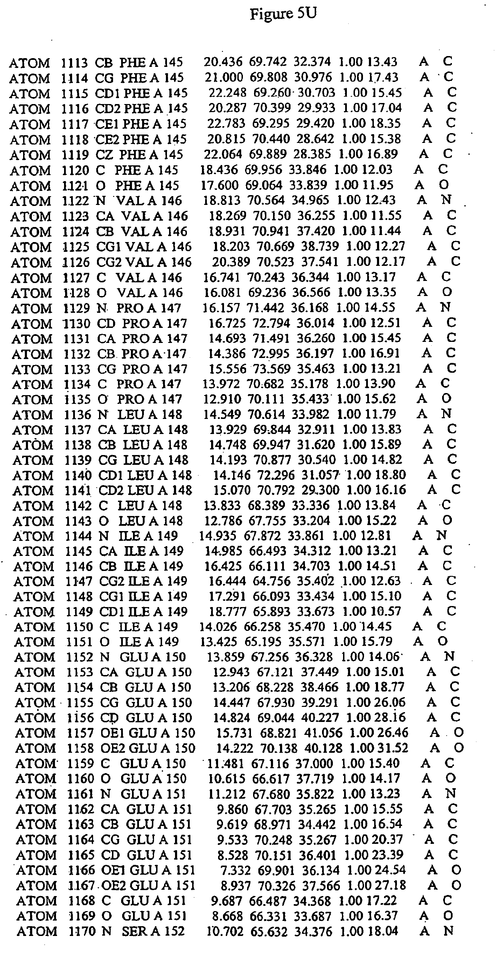

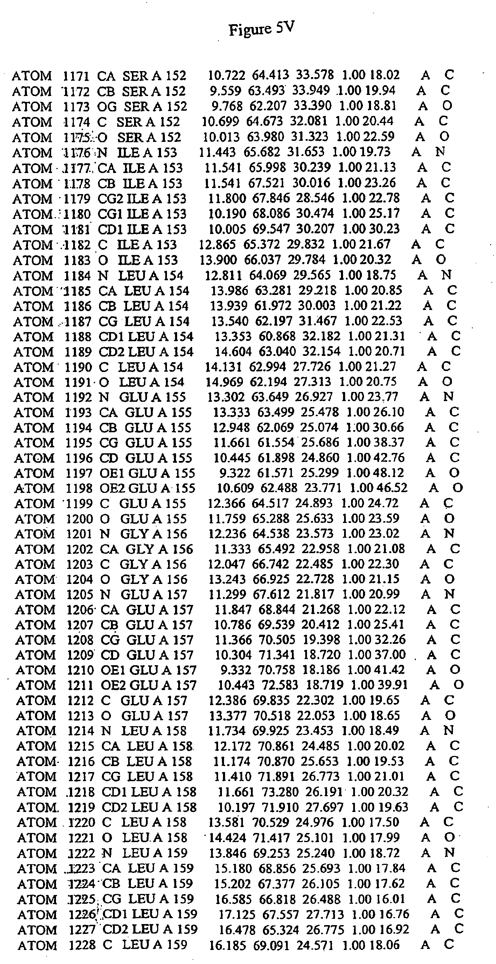

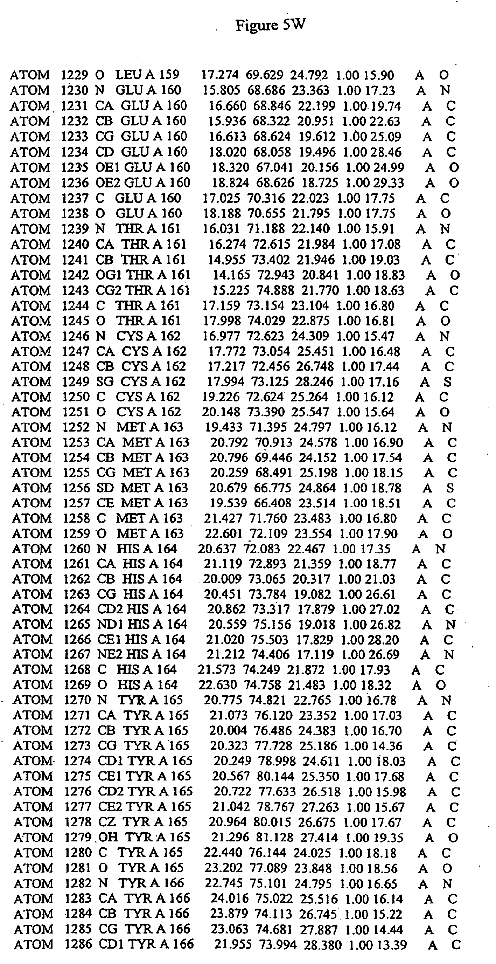

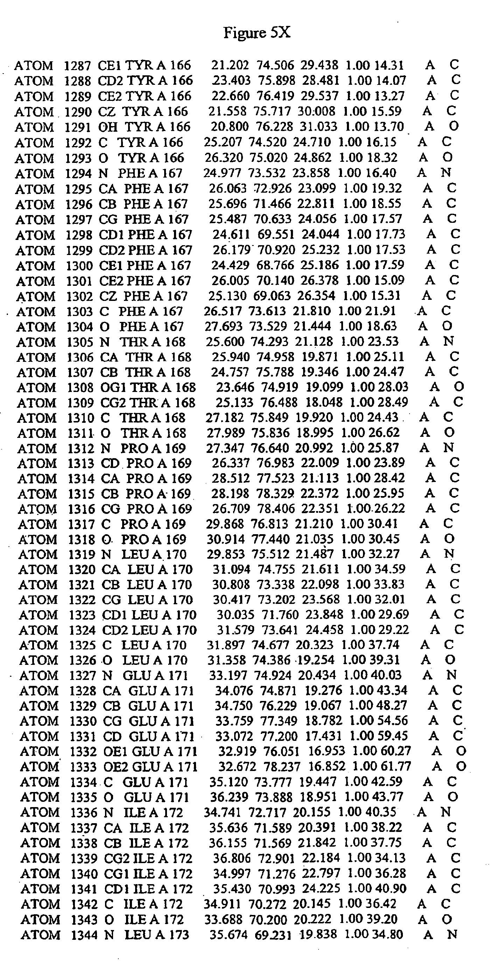

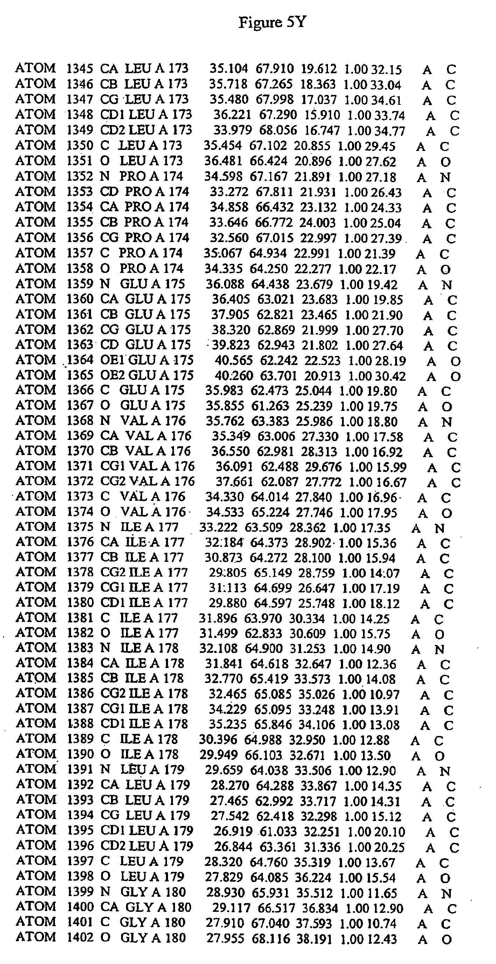

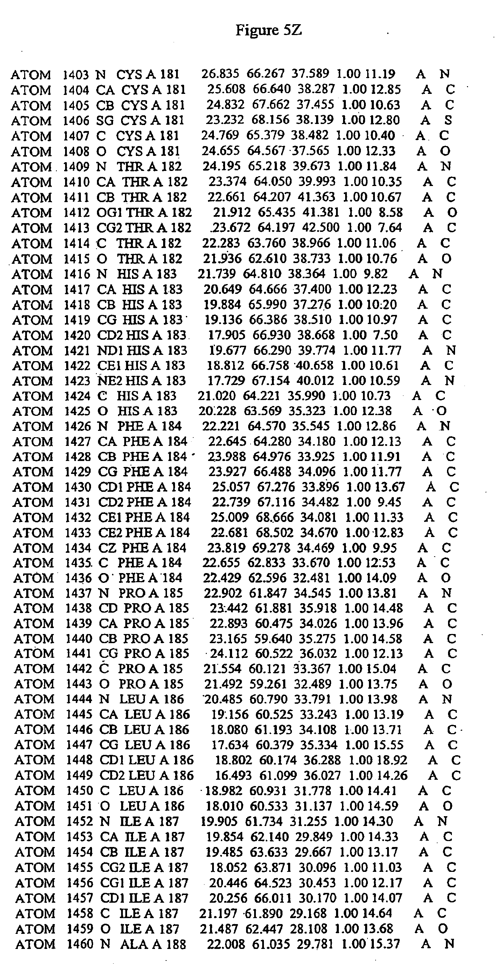

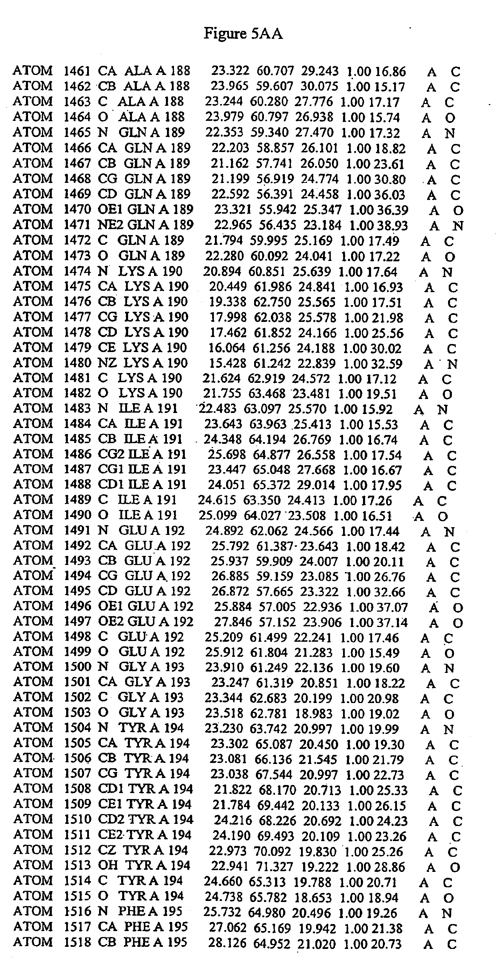

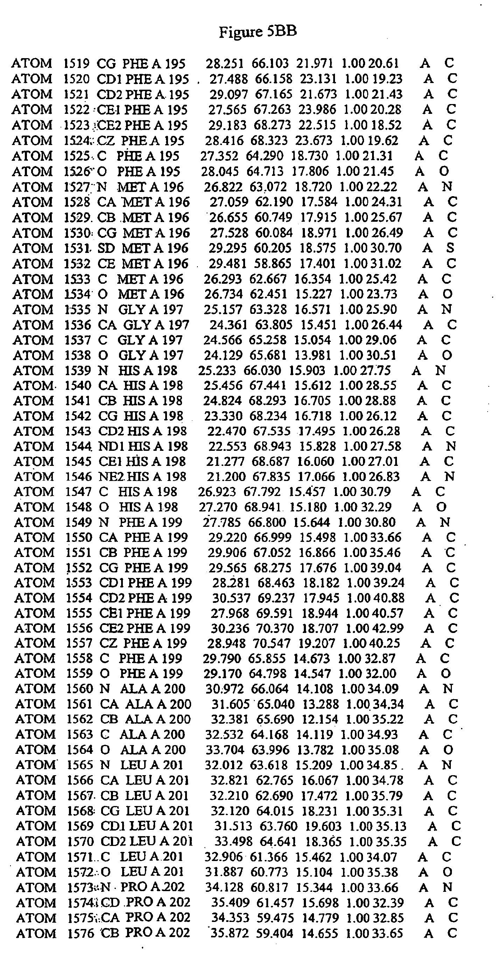

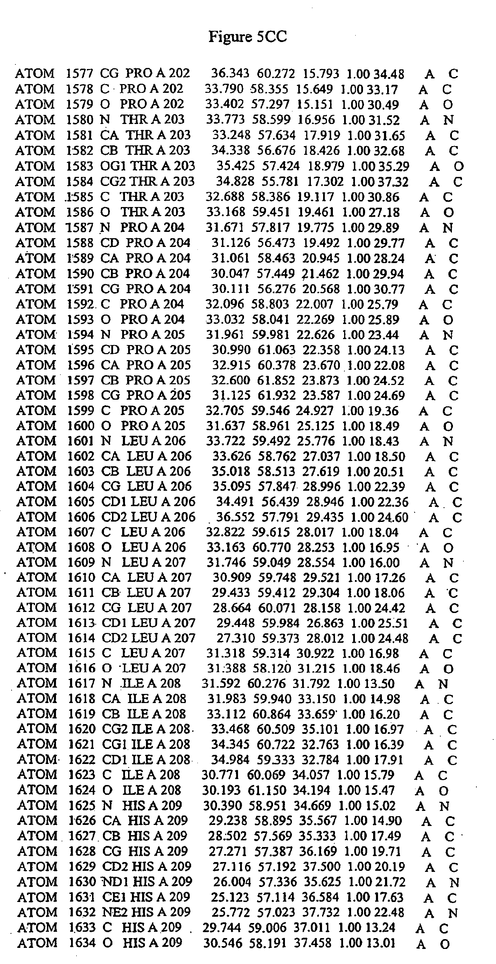

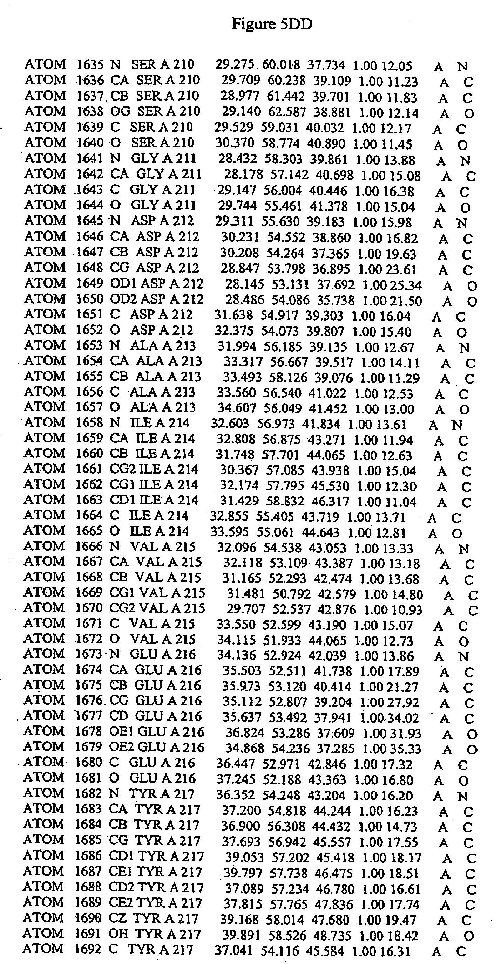

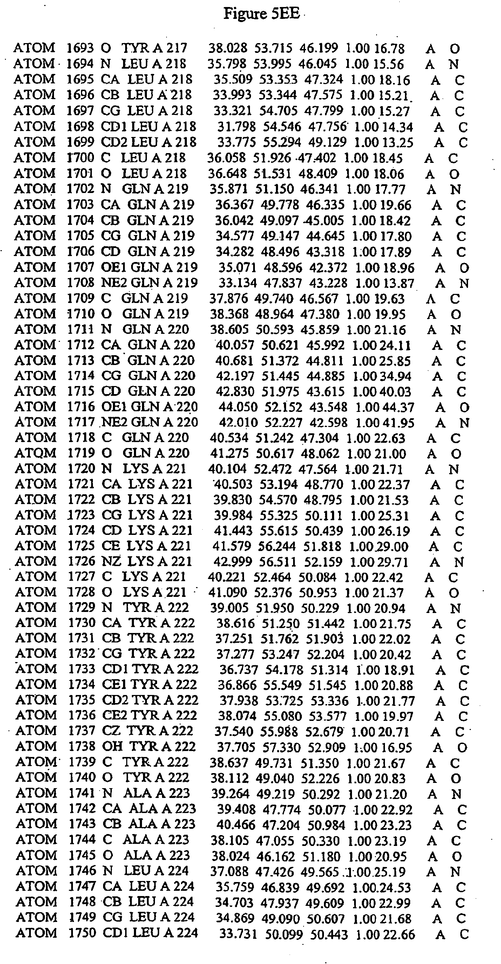

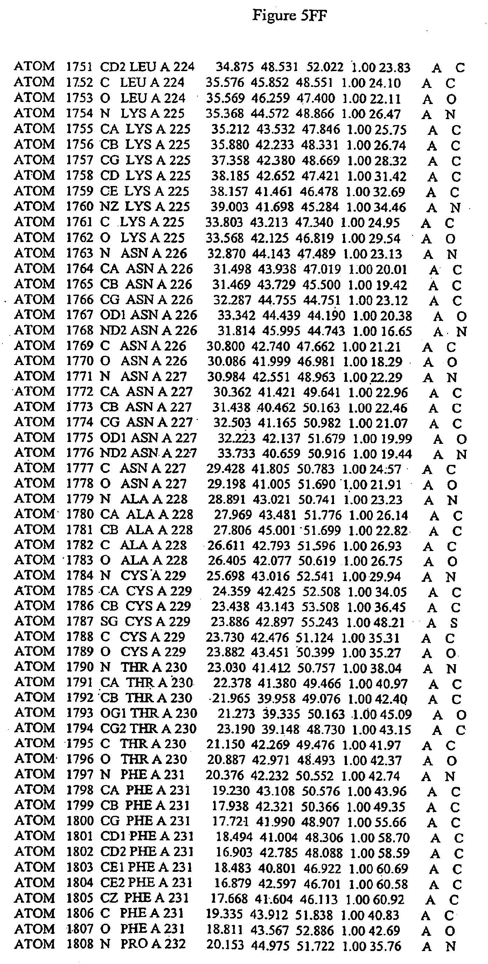

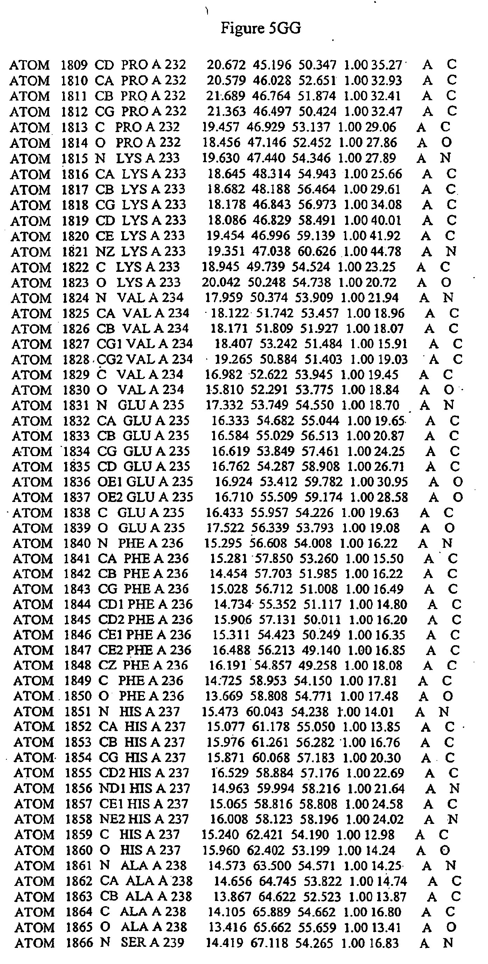

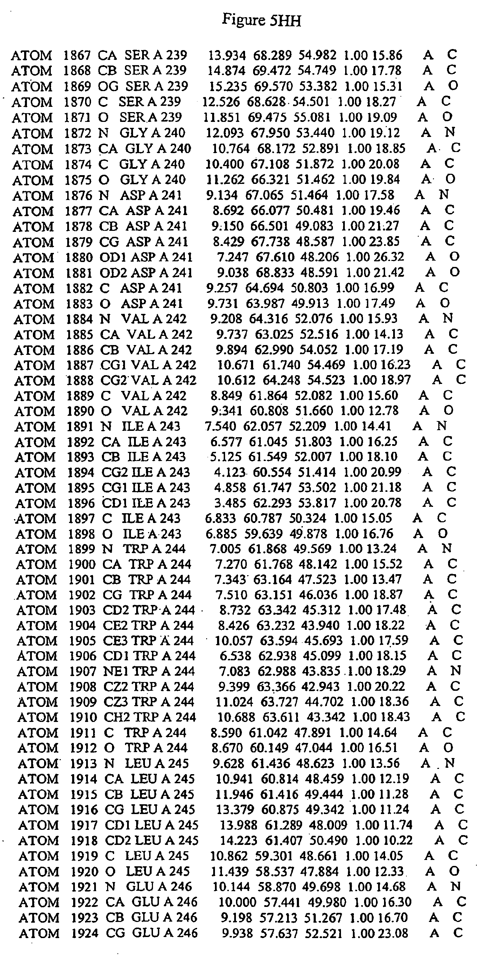

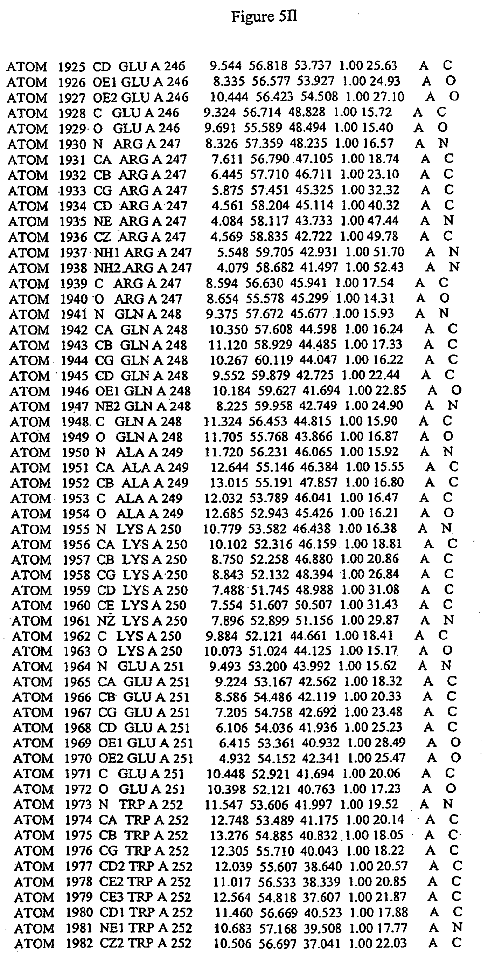

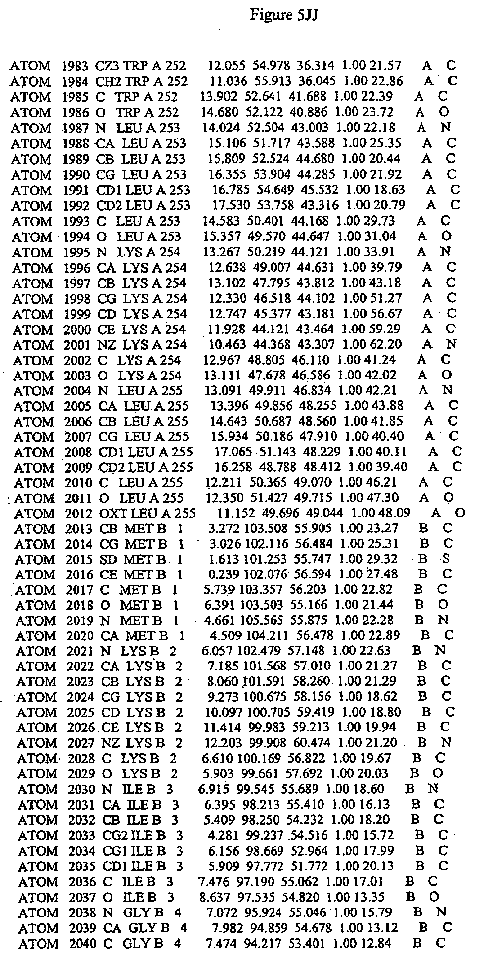

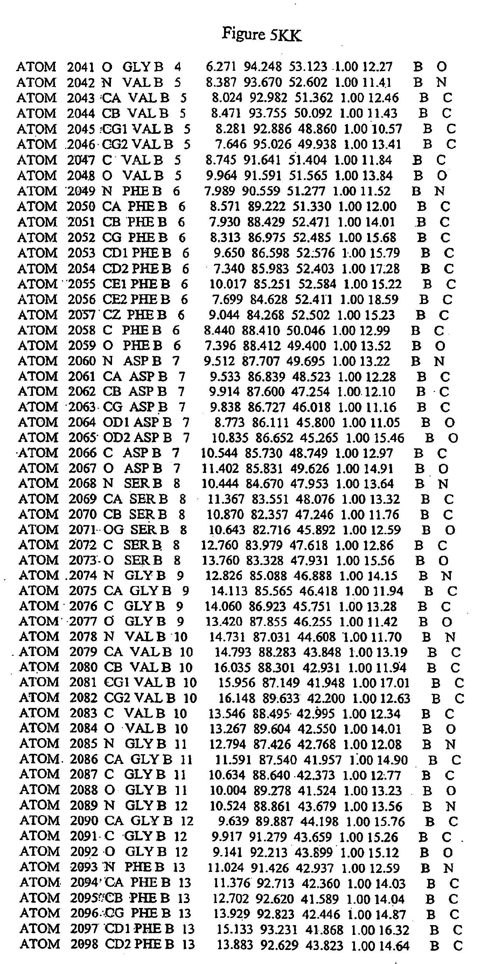

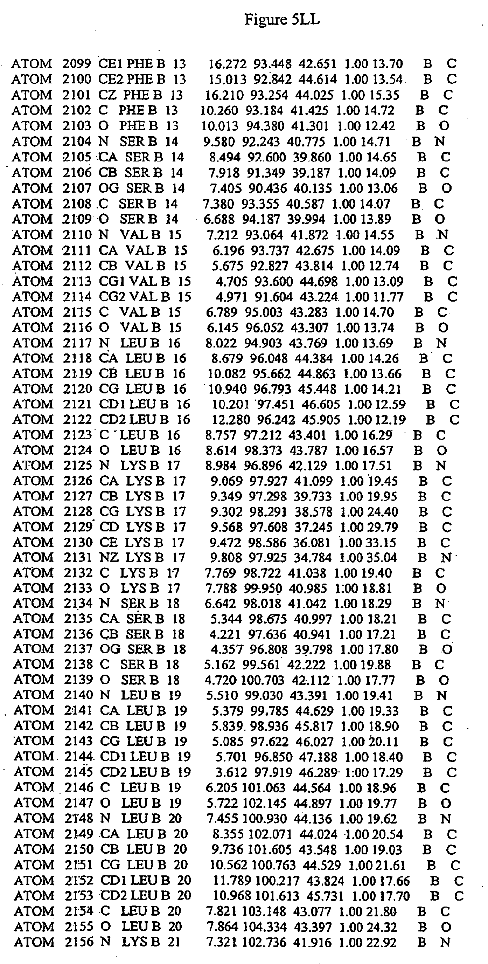

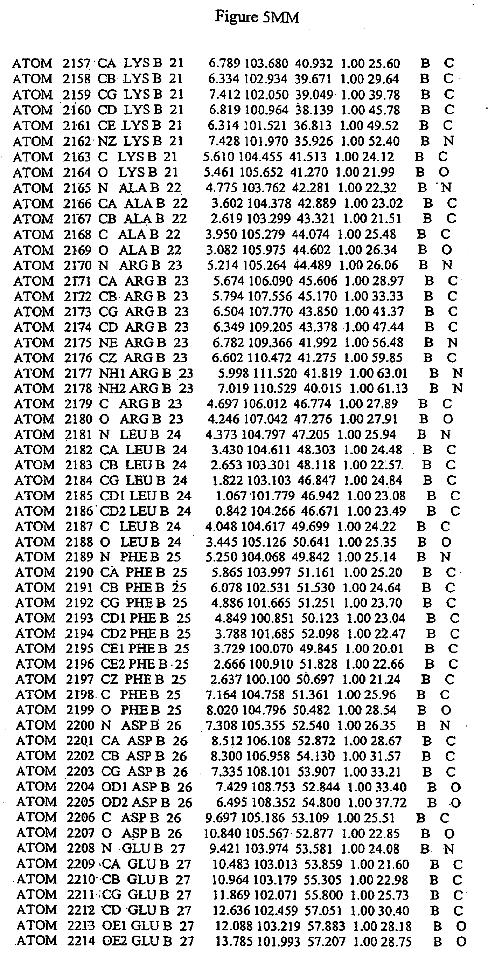

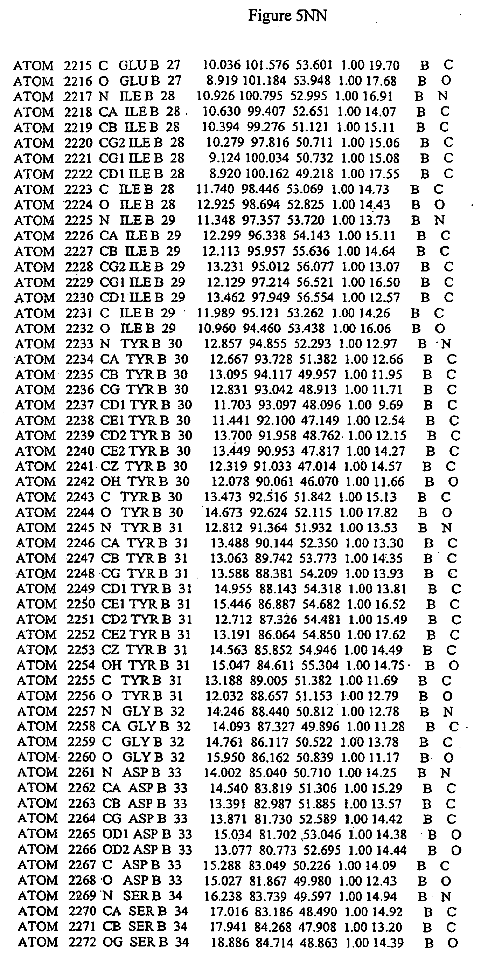

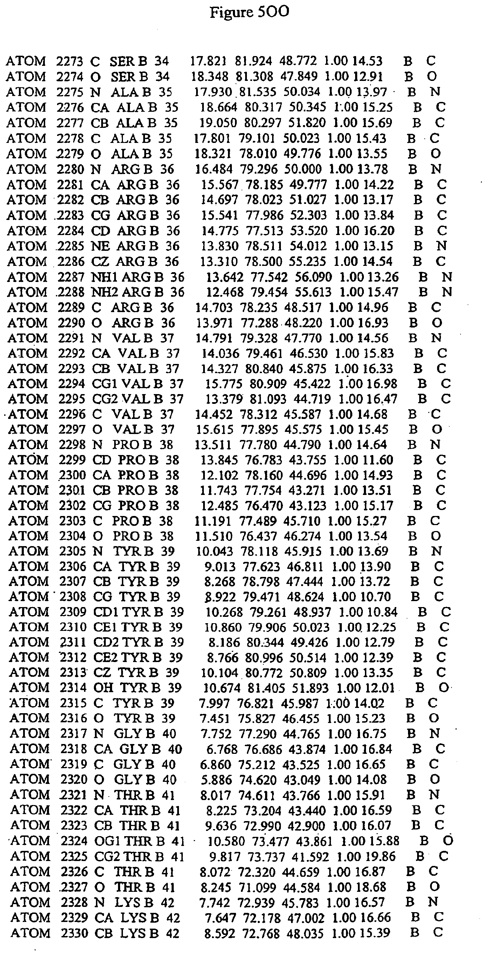

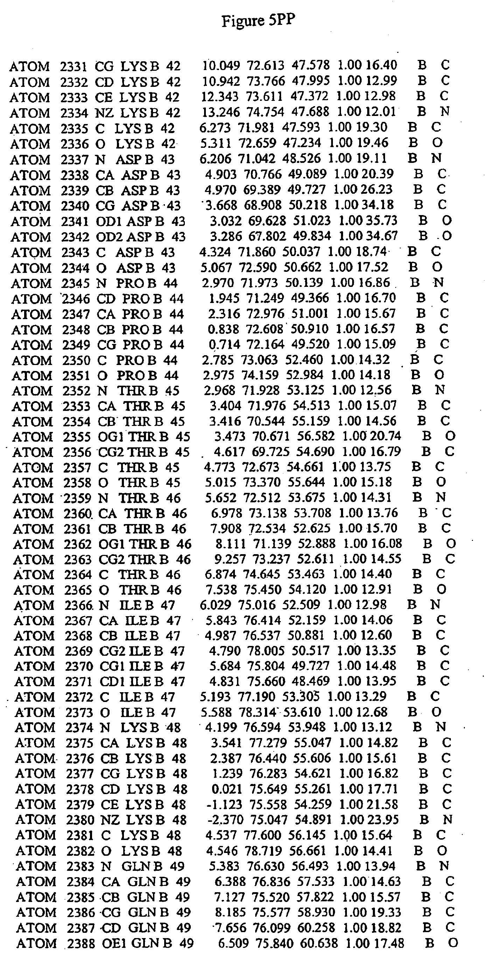

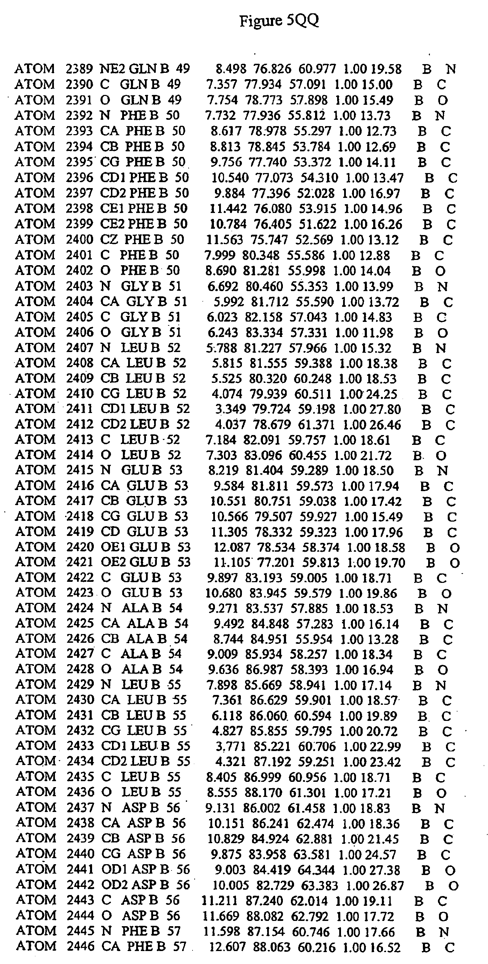

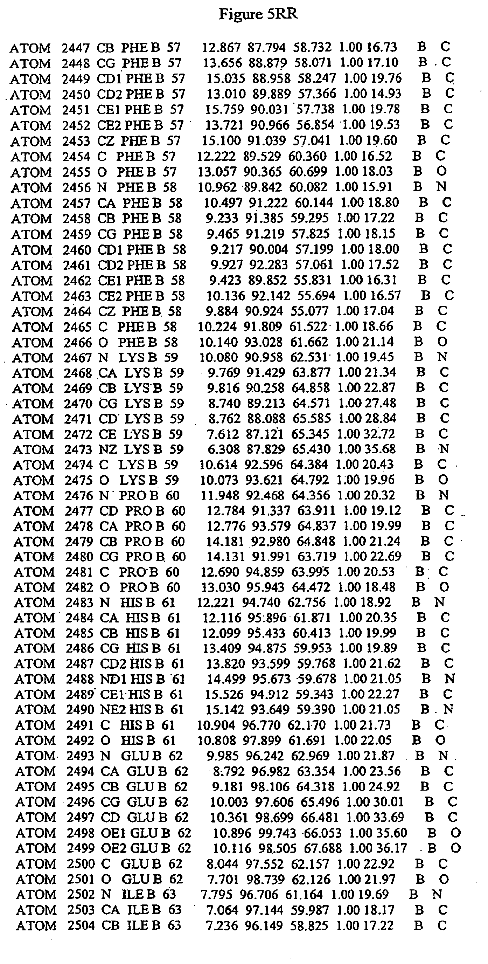

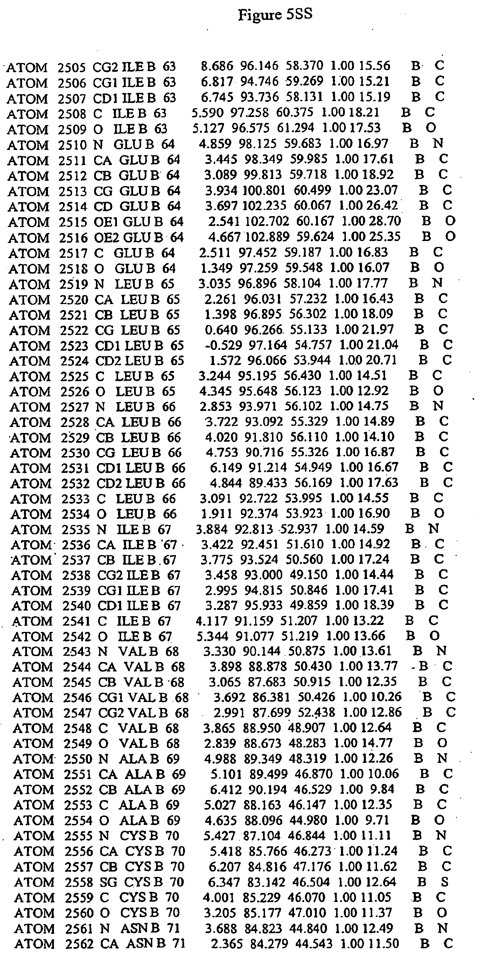

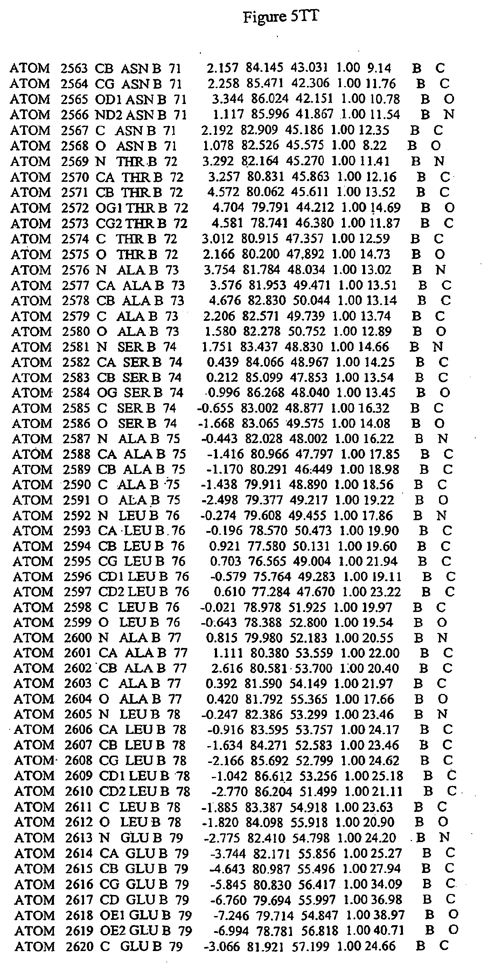

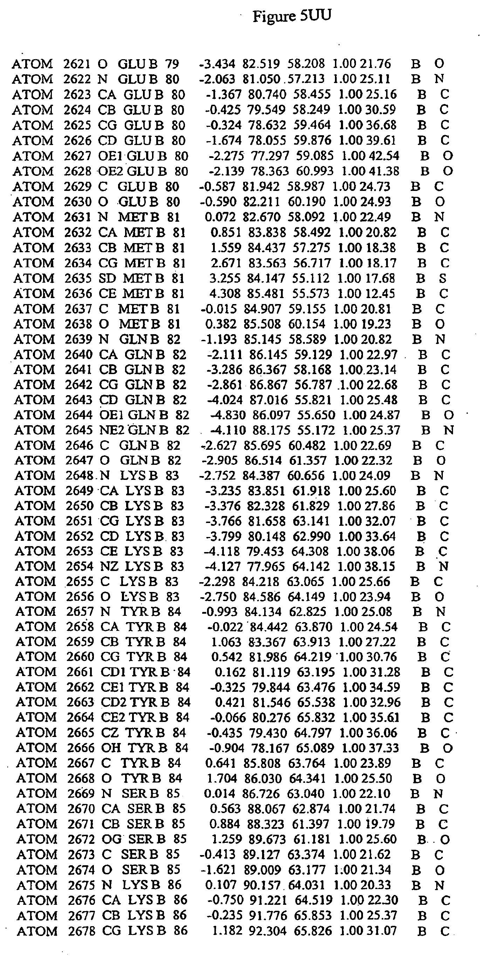

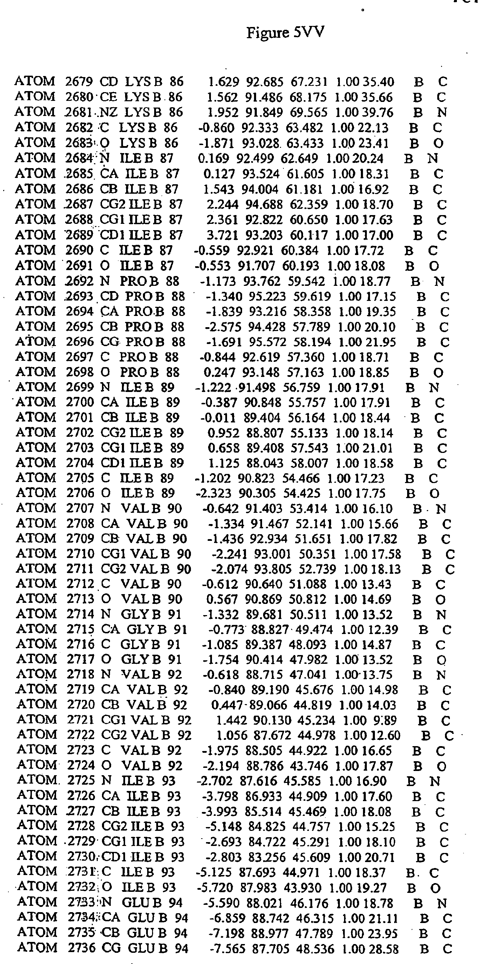

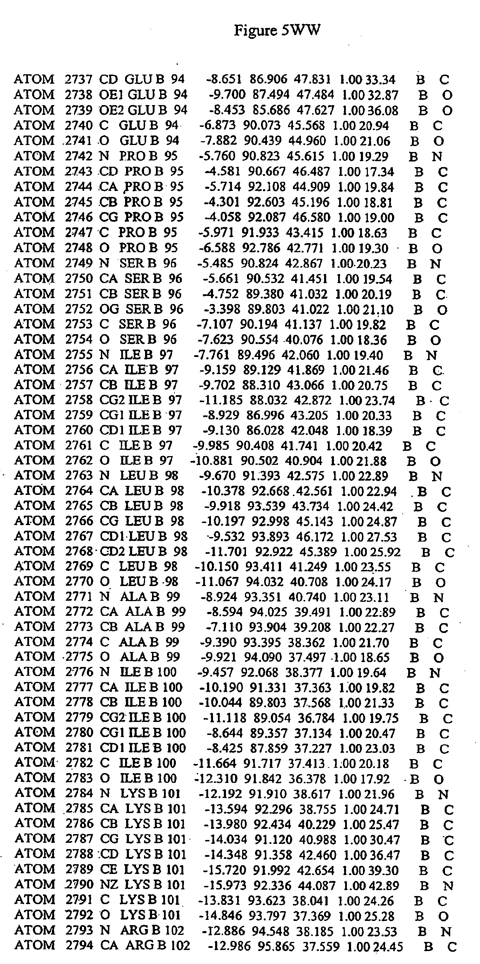

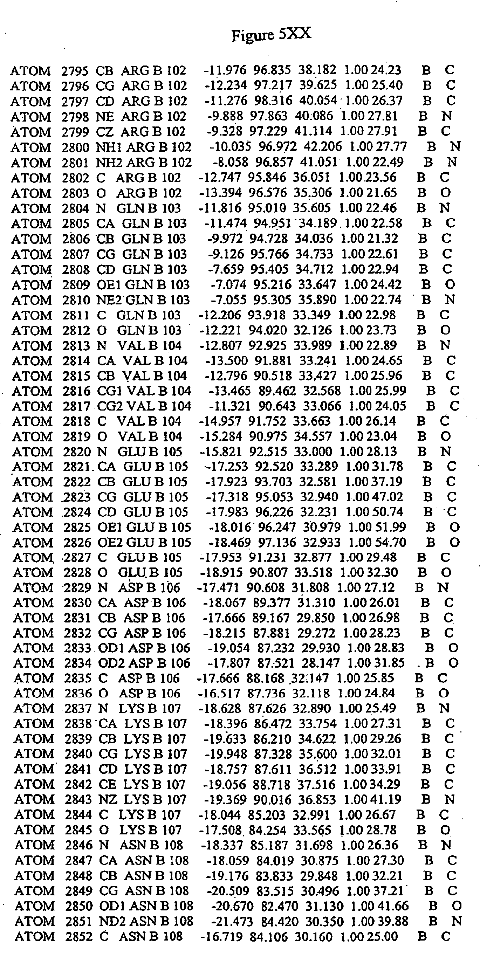

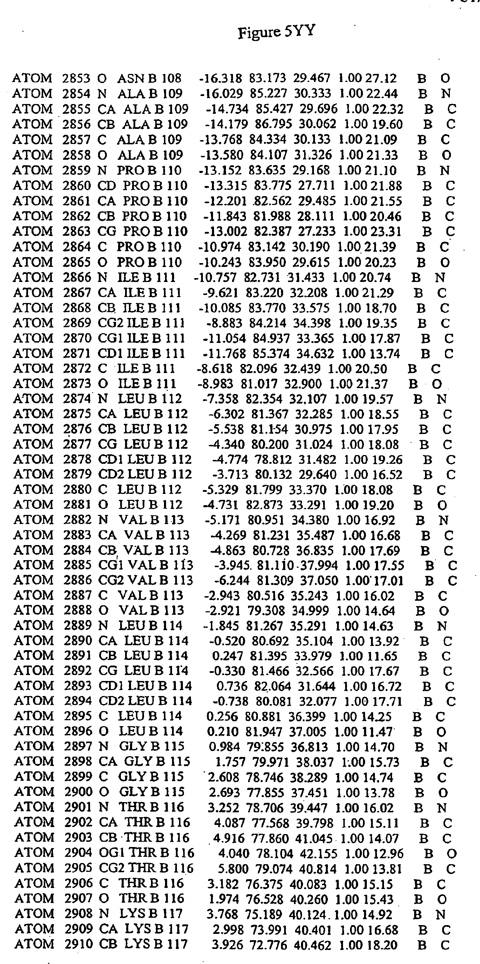

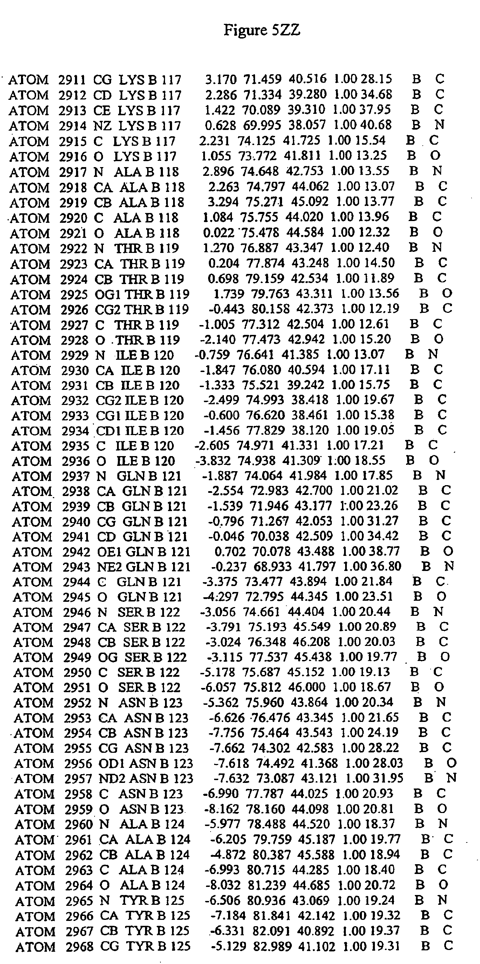

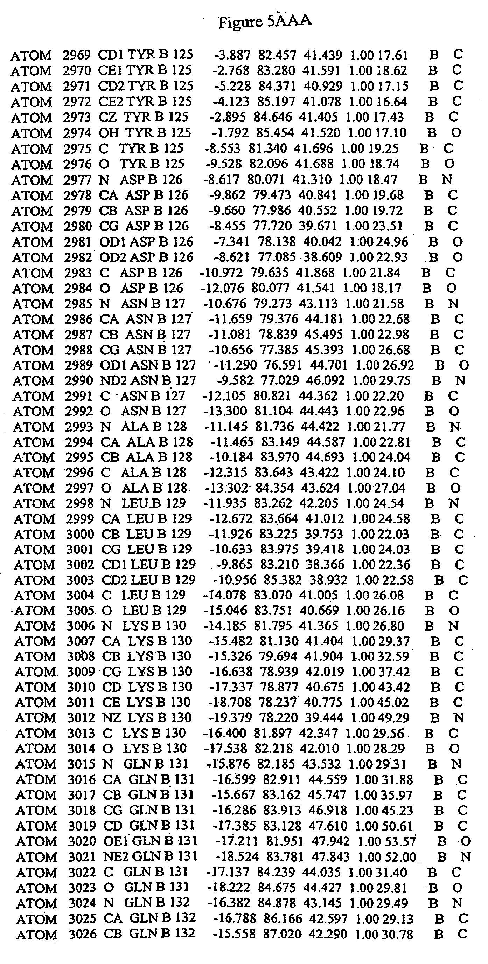

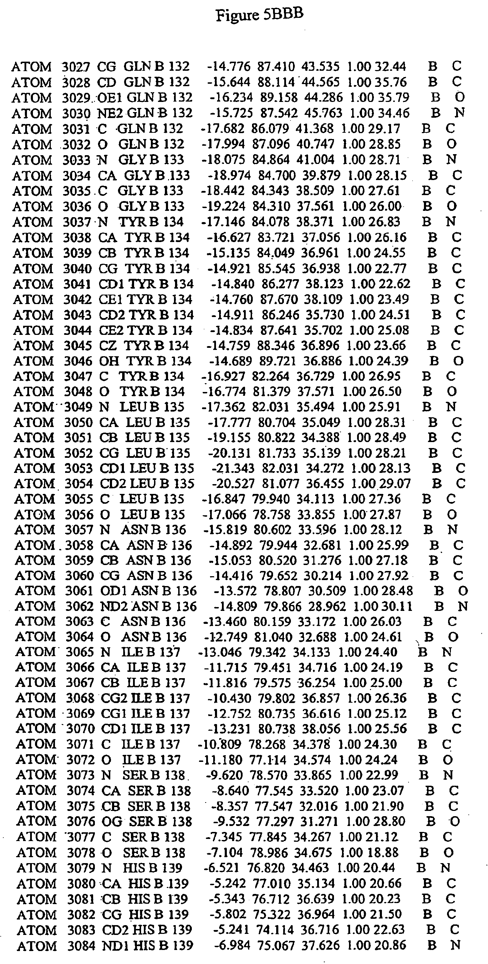

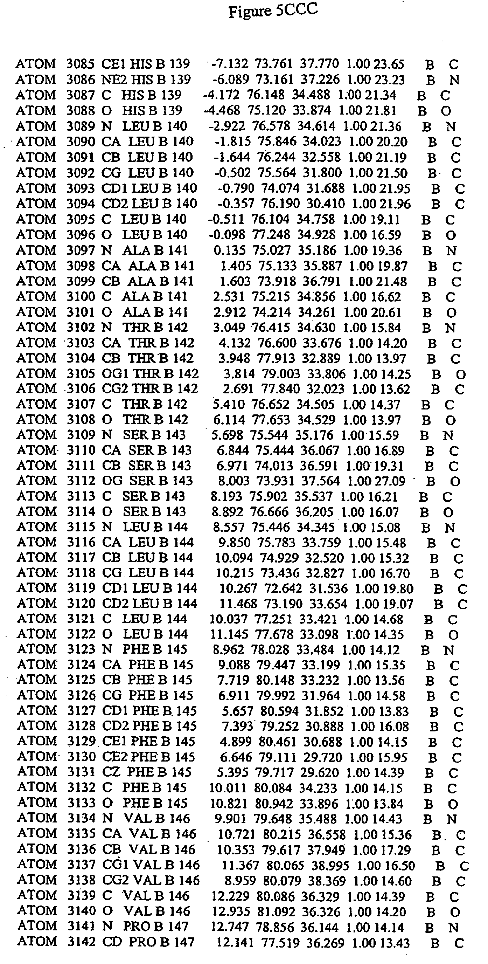

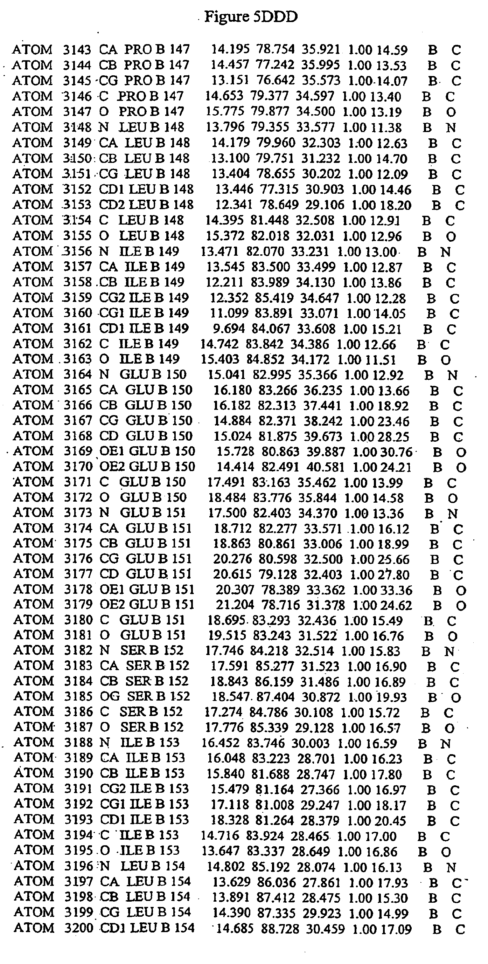

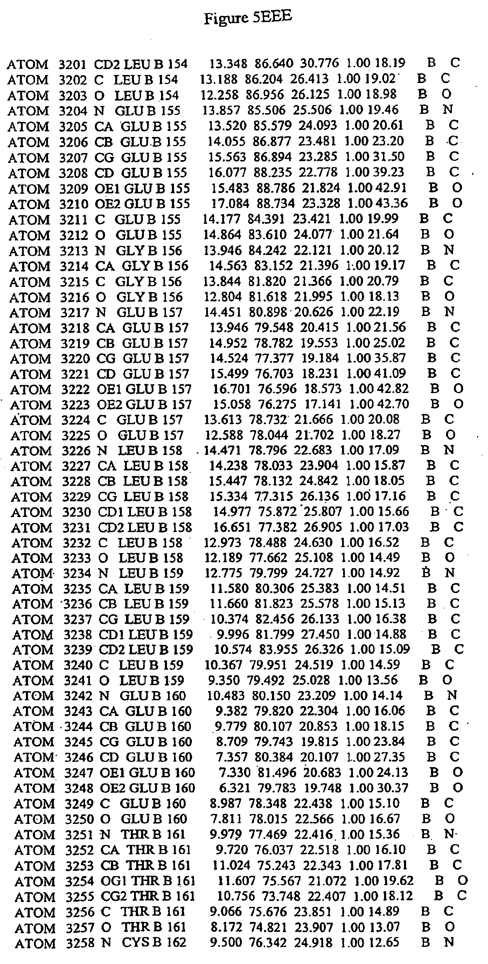

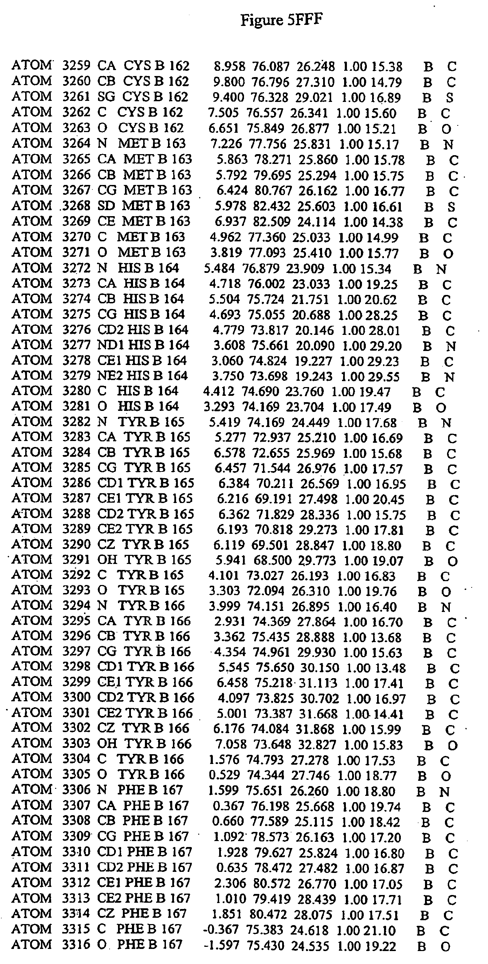

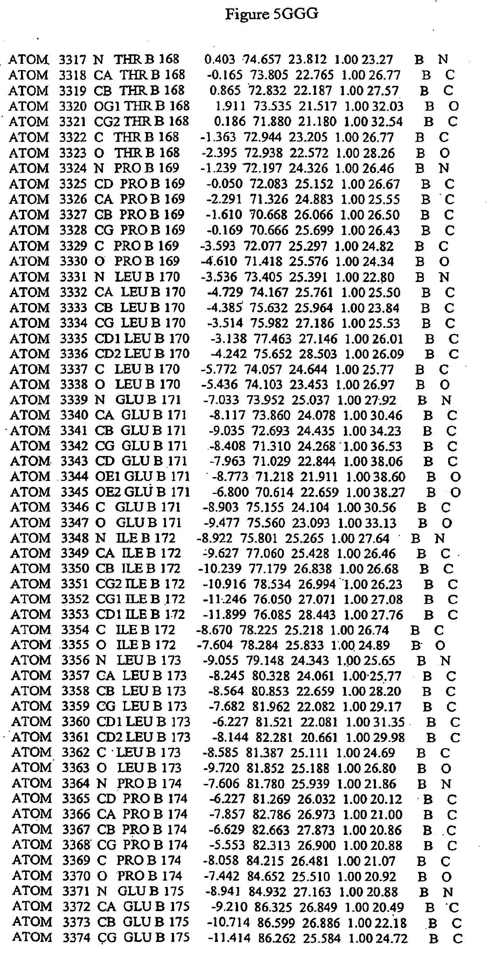

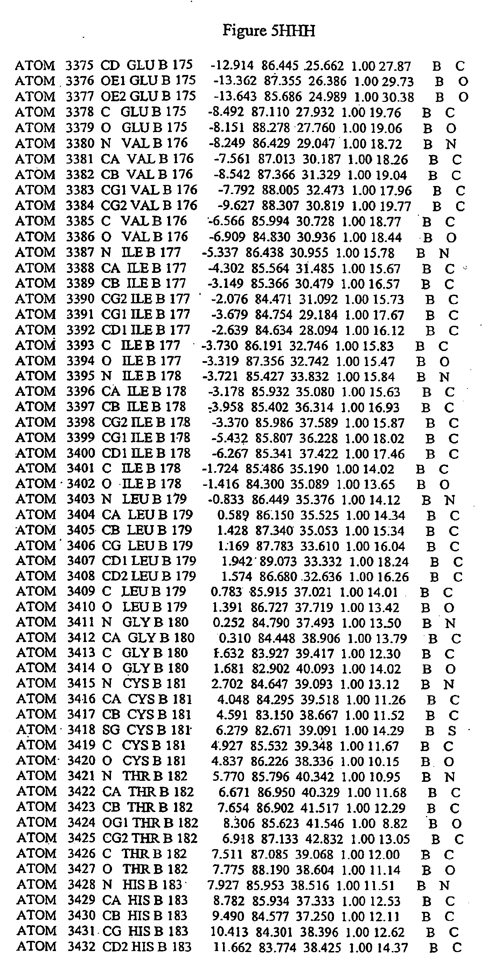

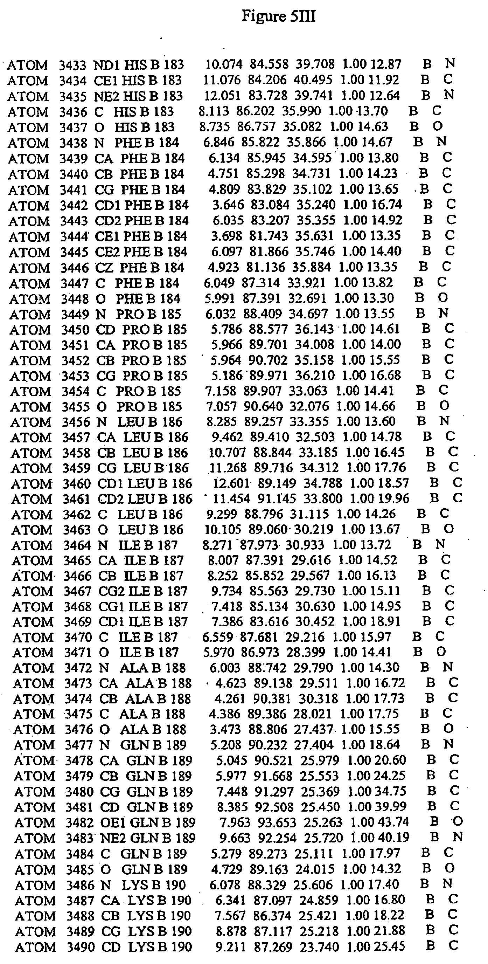

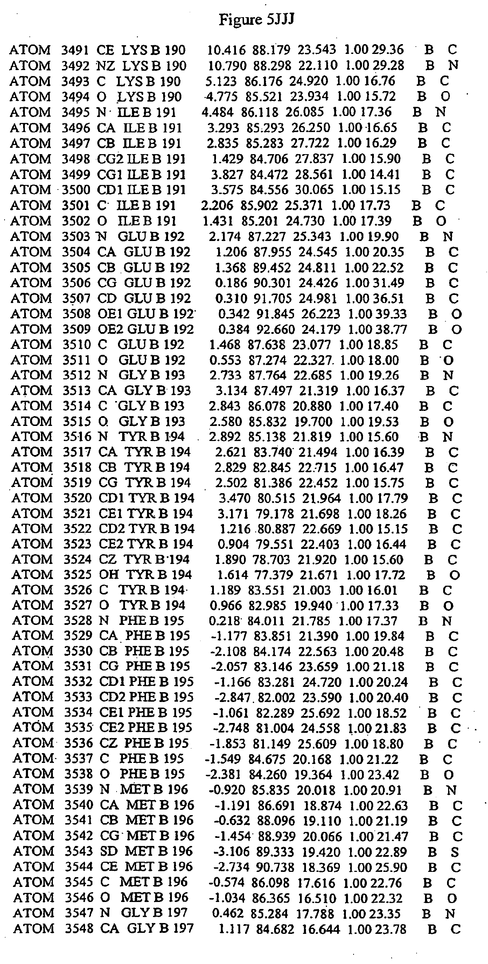

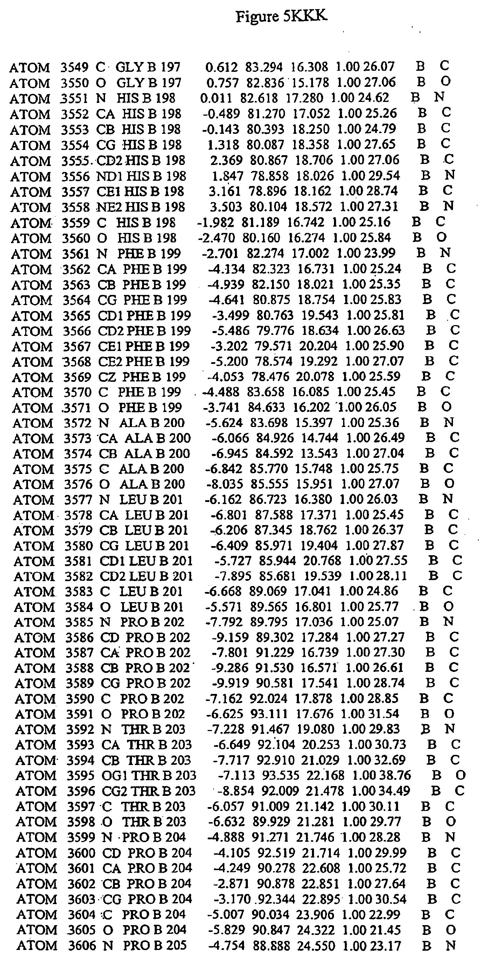

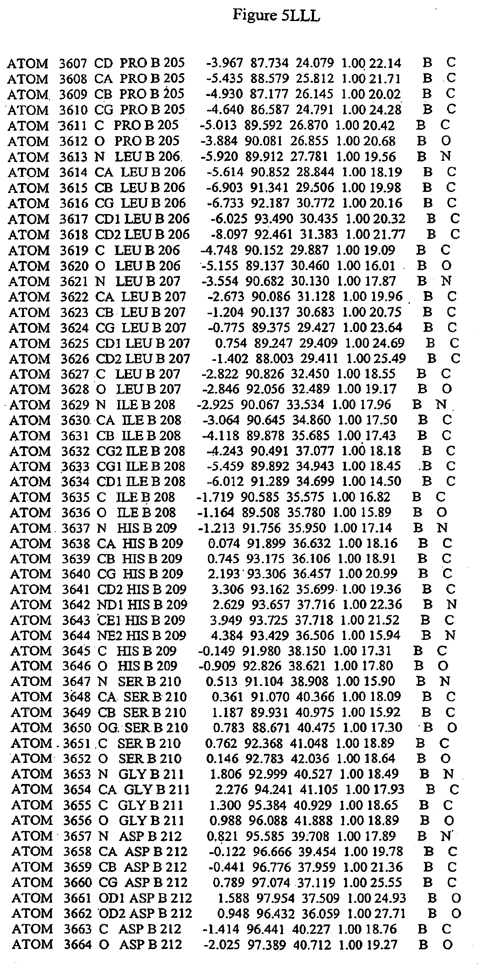

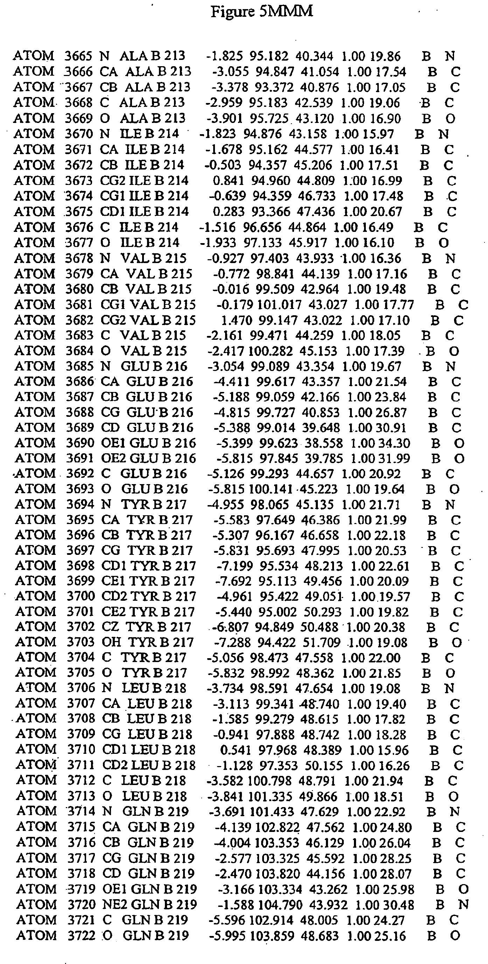

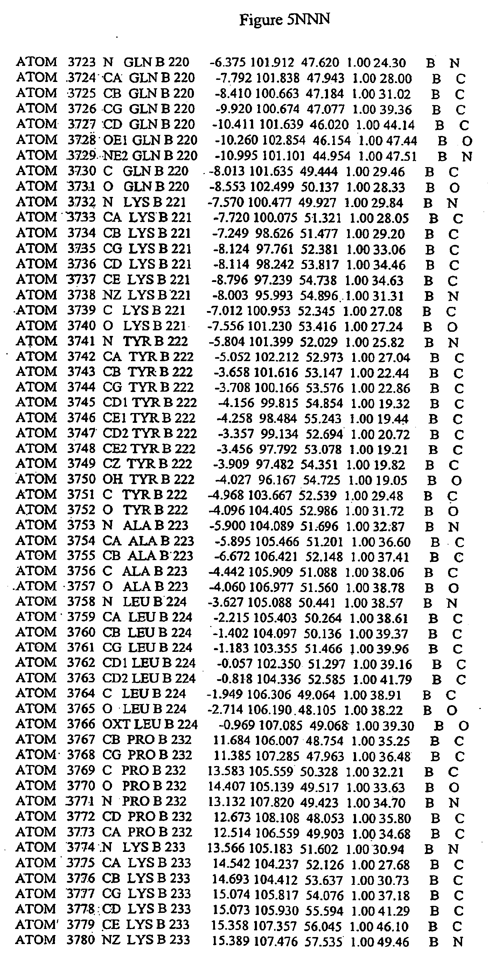

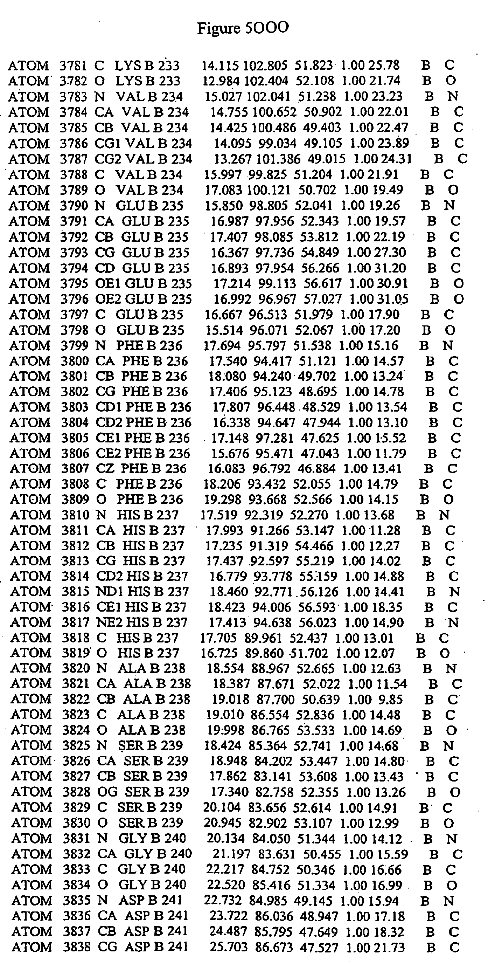

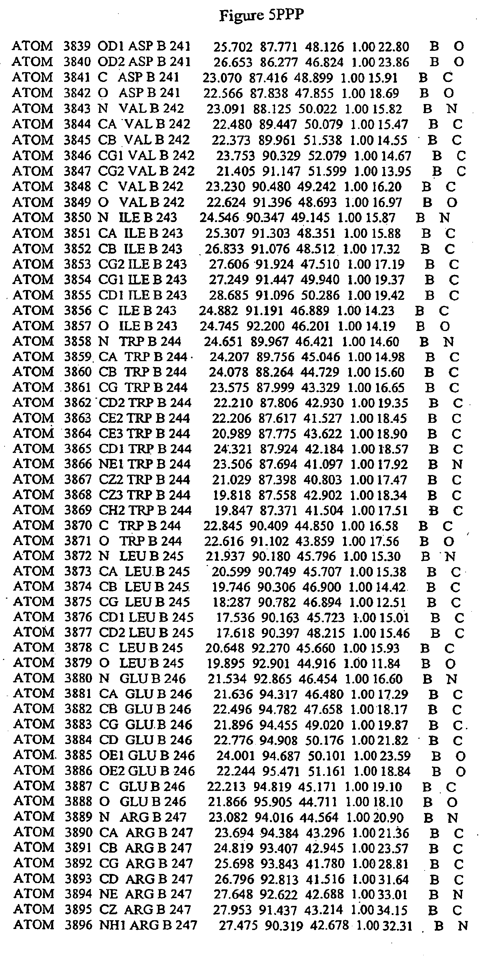

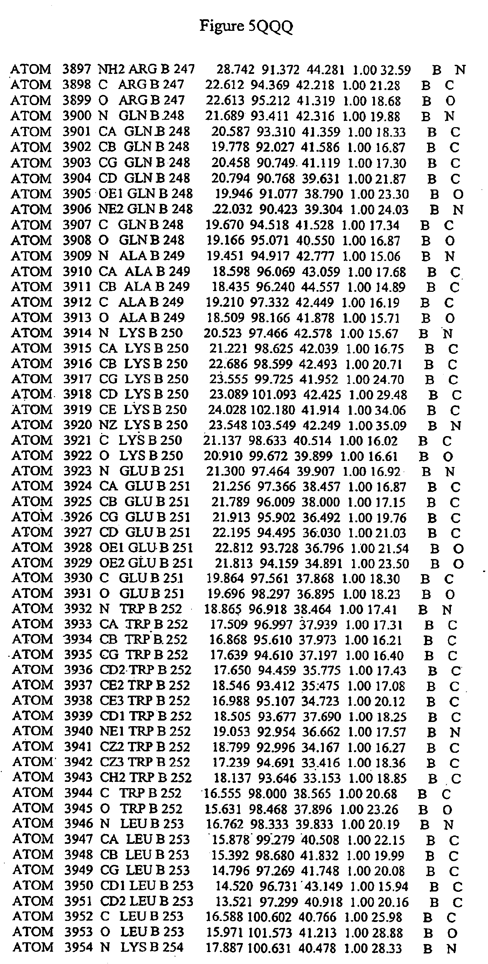

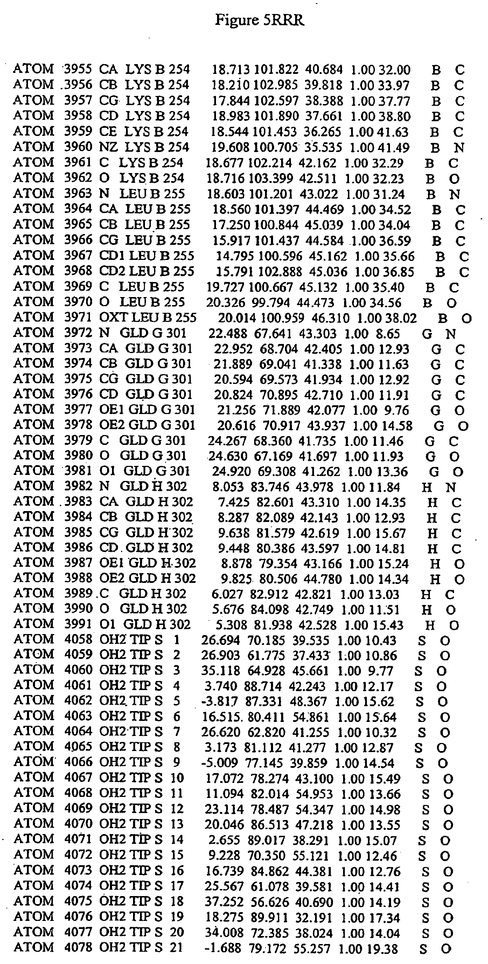

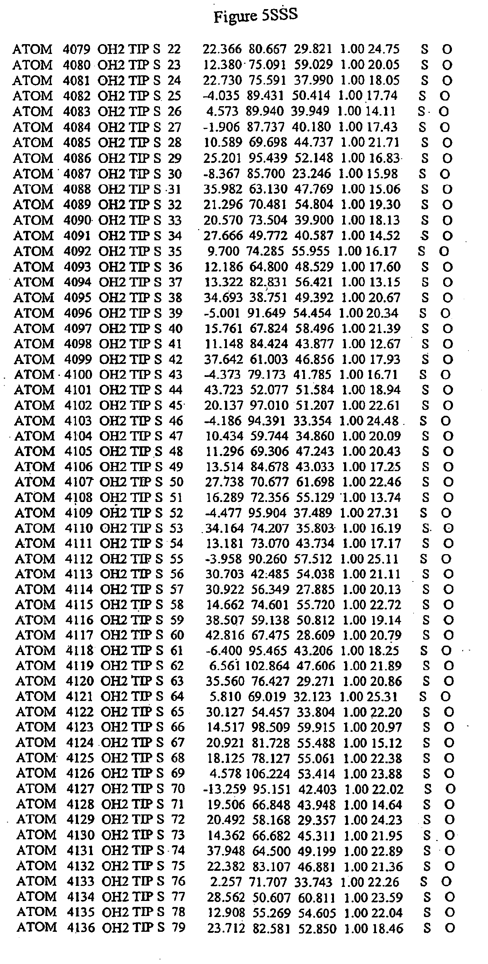

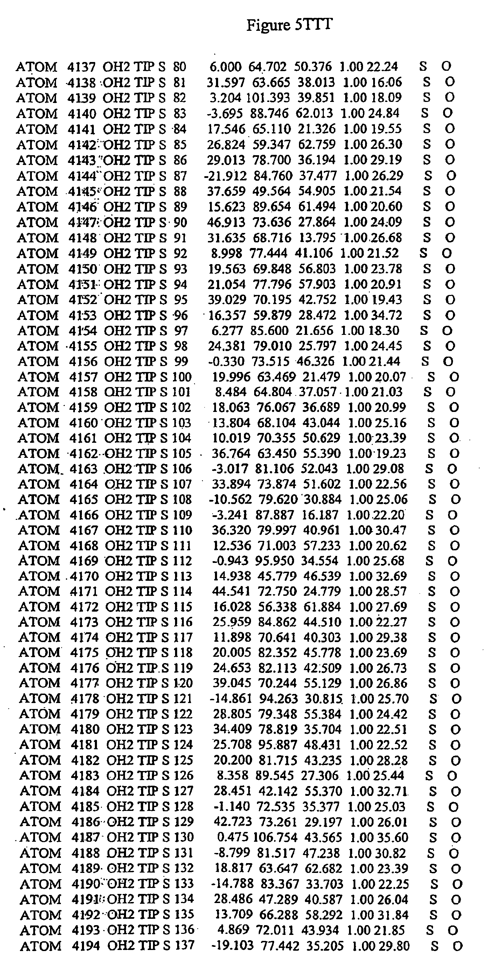

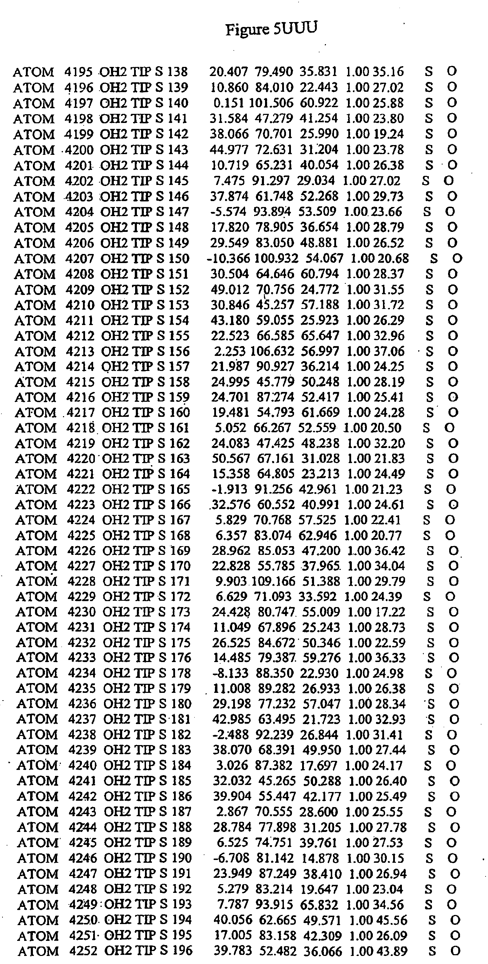

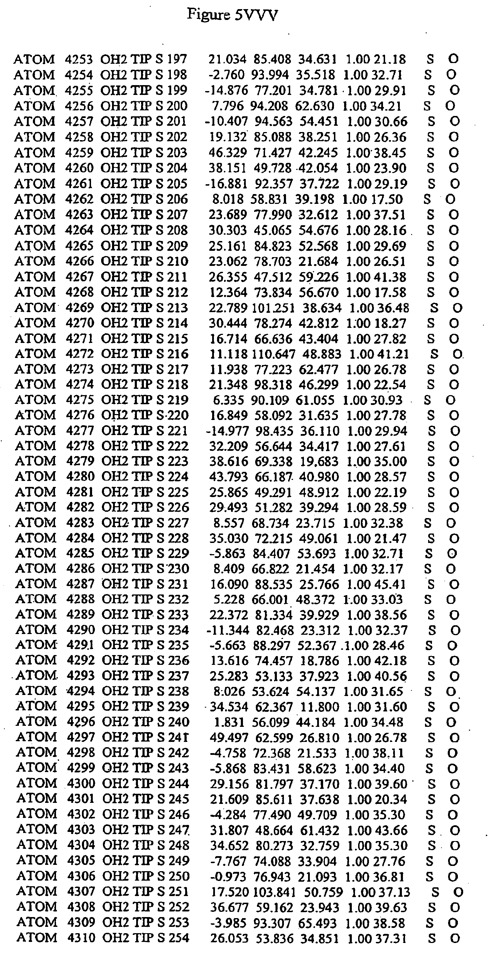

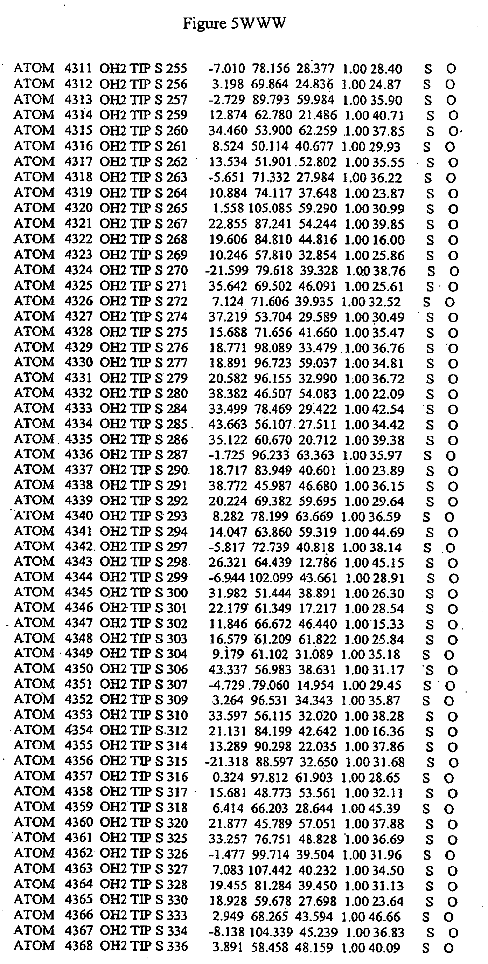

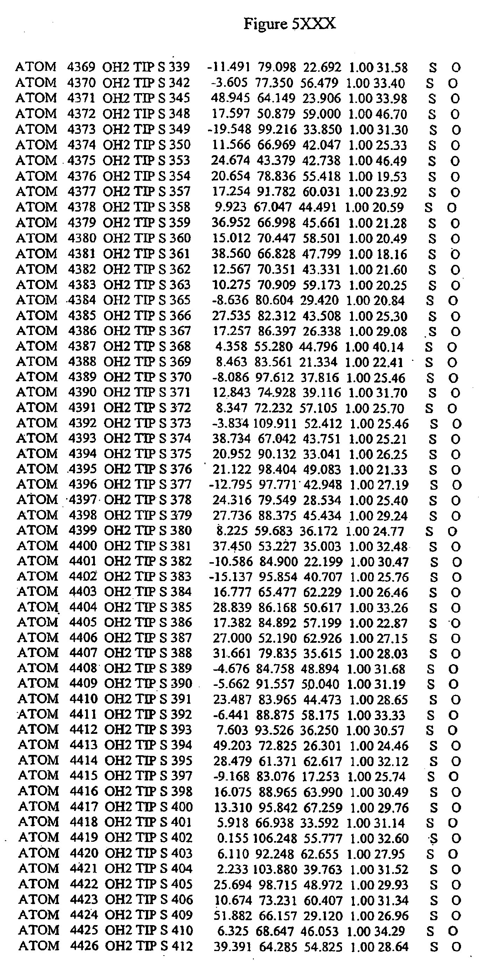

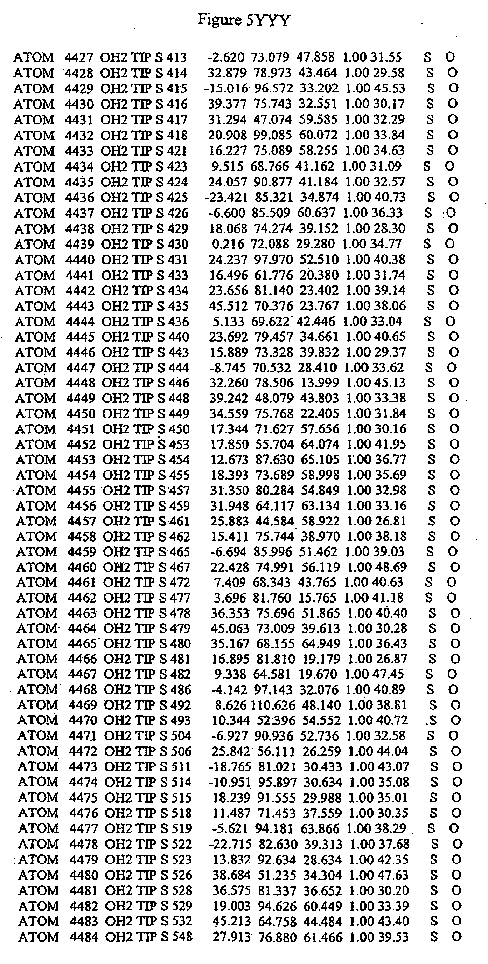

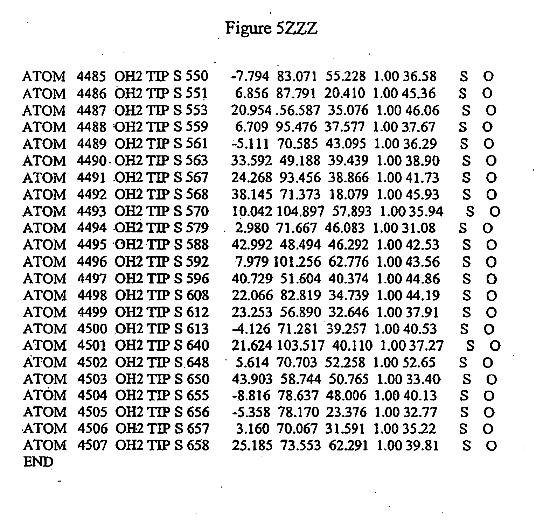

One embodiment relates to a crystal of MurI. In one embodiment, we provide crystallized *H. pylori* MurI characterized by the structural coordinates depicted in Figure 4. In one embodiment, we provide crystallized *H. pylori* MurI complexed with D-glutamate characterized by the structural coordinates depicted in Figure 5. One embodiment of the crystallized complex is characterized as belonging to the orthorombic space group P2₁2₁2₁ and has cell dimensions of a = 62.14 Å, b = 81.07 Å and c = 113.82 Å, wherein α = 90°, β = 90°, and γ = 90°. Another embodiment of the crystallized complex is characterized as belonging to the monoclinic space group P2₁ and has cell dimensions of a = 59.20 Å, b = 82.40 Å and c = 106.50 Å, wherein α = 90°, β= 92.15°, and γ = 90°. Another embodiment of the crystallized complex is characterized as belonging to the monoclinic space group P2₁ and has cell dimensions of a = 52.28 Å, b = 78.96 Å and c = 59.14 Å, wherein α = 90°, β = 92.64°, and γ = 90°. Another embodiment of the crystallized complex is characterized as belonging to the monoclinic space group P2₁ and has cell dimensions of a = 52.02 Å, b = 80.66 Å and c = 59.18 Å, wherein α = 90°, β= 92.65°, and γ = 90°. Another embodiment of the crystallized complex is characterized as belonging to the monoclinic space group P2₁ and has cell dimensions of a = 52.61 Å, b = 78.40 Å, and c = 59.43 Å, and wherein α = 90°, β = 92.33°, γ = 90°. Another embodiment of the crystallized complex is characterized as belonging to the monoclinic space group P2₁ and has cell dimensions of a = 62.9 Å, b = 81.8 Å, and c = 113.6 Å, and wherein α = 90°, β = 90°, γ = 90°. Each of these embodiments is encompassed by the structural coordinates of Figure 4, or in complex with D-glutamate as encompassed by the structural coordinates of Figure 5.

One embodiment is a crystal *of H. pylori* MurI complexed with the product (substrate) D-glutamate wherein the MurI is at least 70%, 75%, 80%, 85%, 90%, 95%, 98%, or 99% homologous to the amino acid sequence represented by SEQ ID NO: 2, or portions thereof. In the present invention, the crystals diffract from about 0.8 Å to about 3.5 Å.

One embodiment relates to a crystal of MurI in complex with an inhibitor (e.g., an antibacterial binding agent, drug, etc.). In one embodiment, we provide crystallized *H. pylori* MurI in complex with the substrate, D- glutamate. In a further embodiment, the crystallized complex is characterized by the structural coordinates depicted in Figure 4, wherein the determined structures presented represent that of MurI in complex with D-glutamate. In one embodiment, the present invention discloses crystallized *H. pylori* MurI in complex with an inhibitor and substrate (e.g., glutamate). In a further embodiment of the present invention, the crystallized complex is characterized by the structural coordinates depicted in Figure 5, wherein the determined structures presented represent that of MurI in complex with an inhibitor and glutamate. In a further embodiment, the antibacterial binding agent is a pyrimidinedione. In one embodiment of the present invention, the crystal complex is characterized by the structural coordinates depicted in Figure 4.

A further embodiment of the crystallized complex is characterized as belonging to the orthorhombic space group P2₁2₁2₁ and has cell dimensions of a = 61.41 Å, b = 76.31 Å and c = 108.92 Å, wherein α = 90°, β = 90°, and γ = 90°, wherein the crystallized complex is characterized by the structural coordinates depicted in Figure 5.

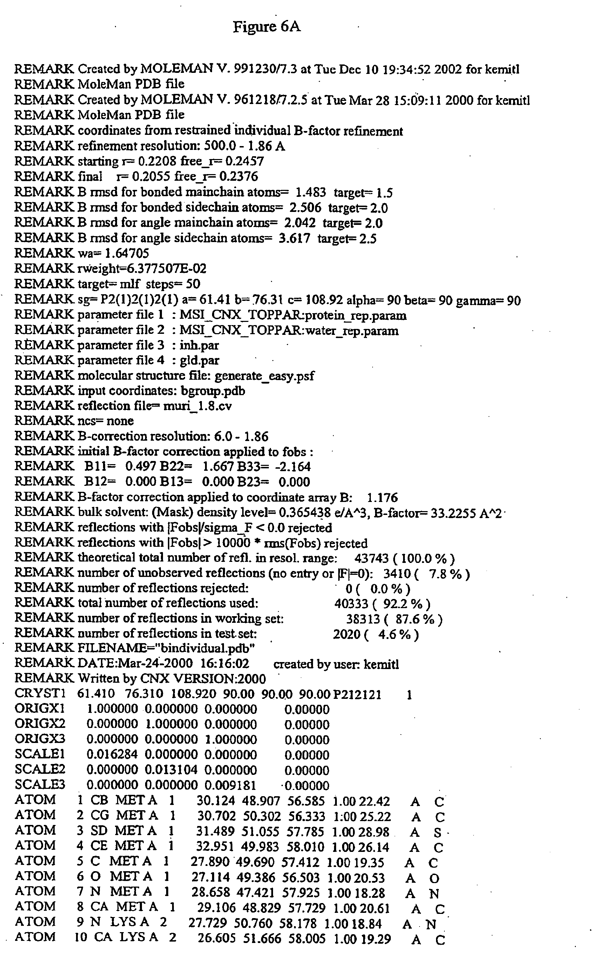

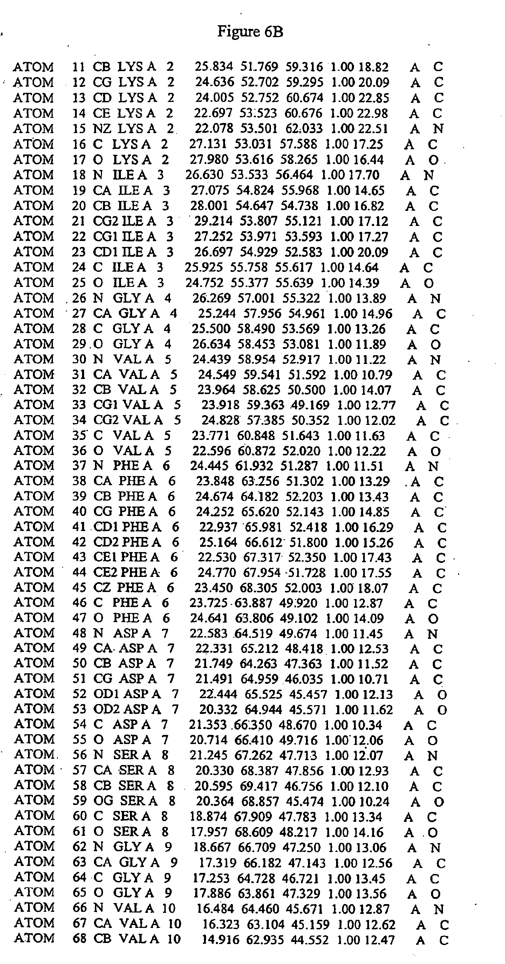

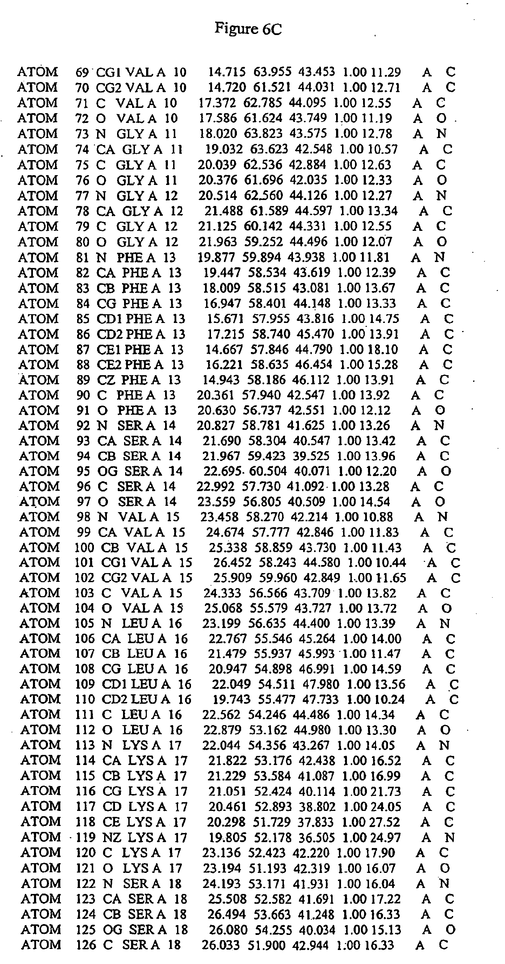

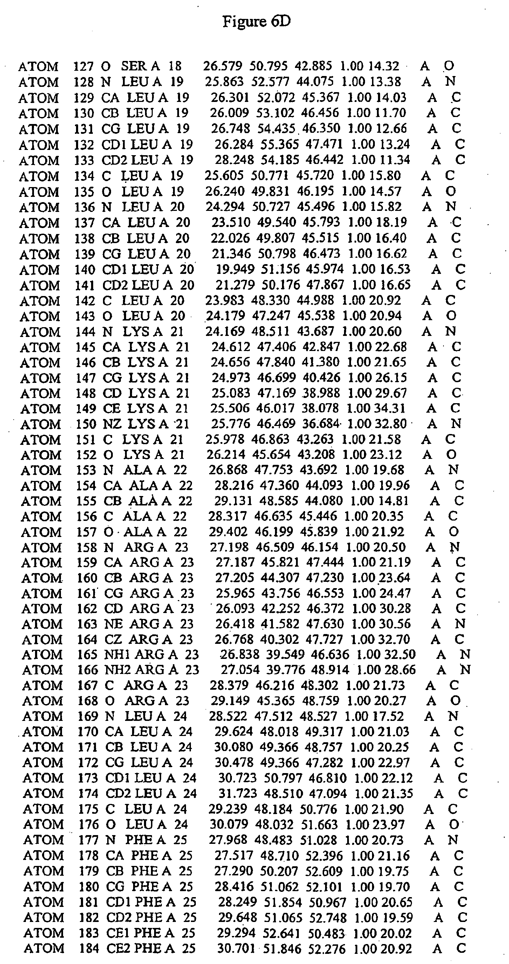

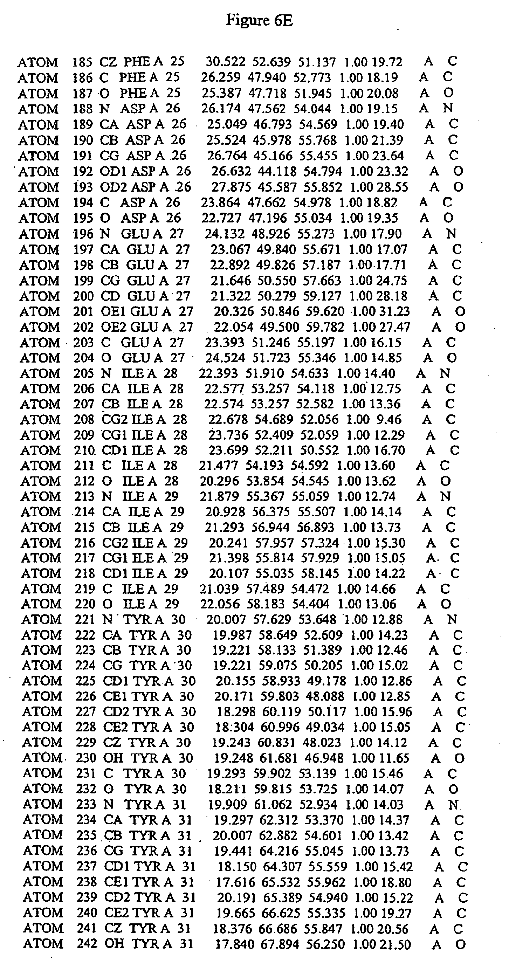

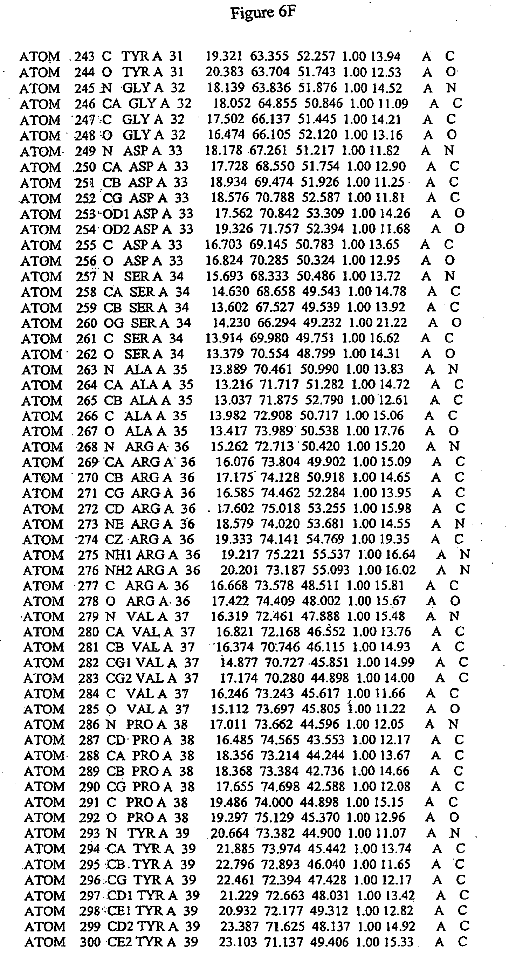

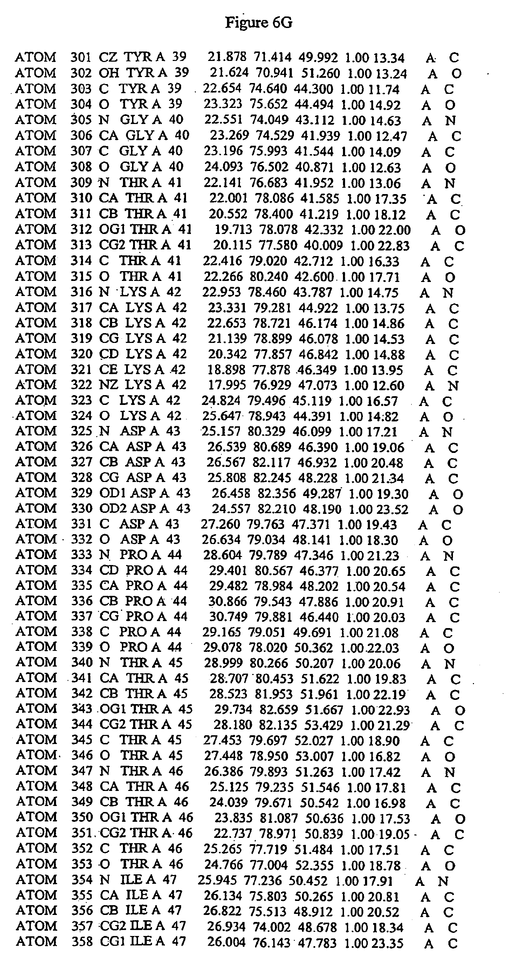

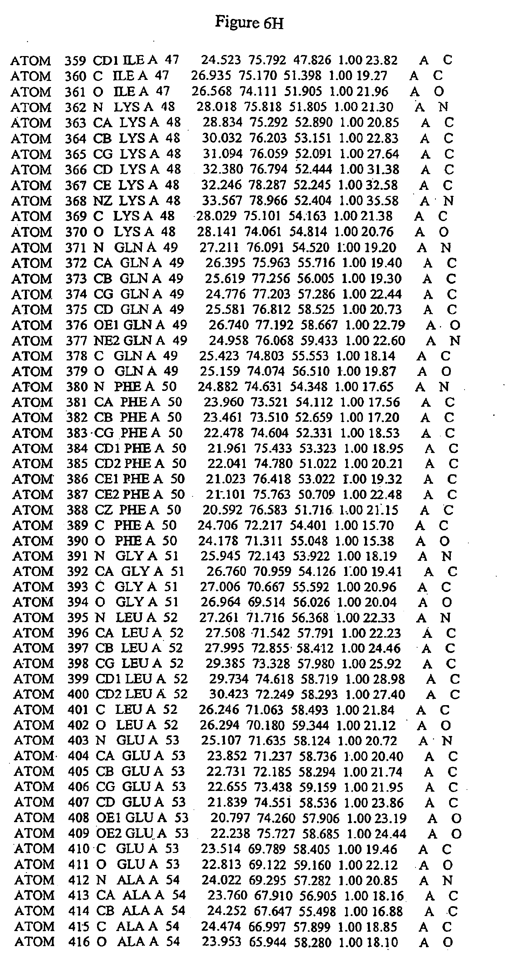

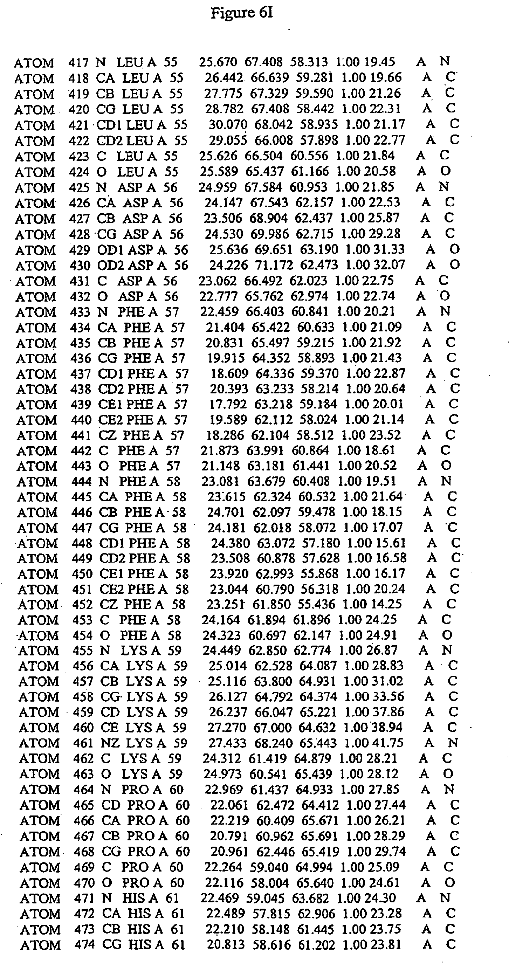

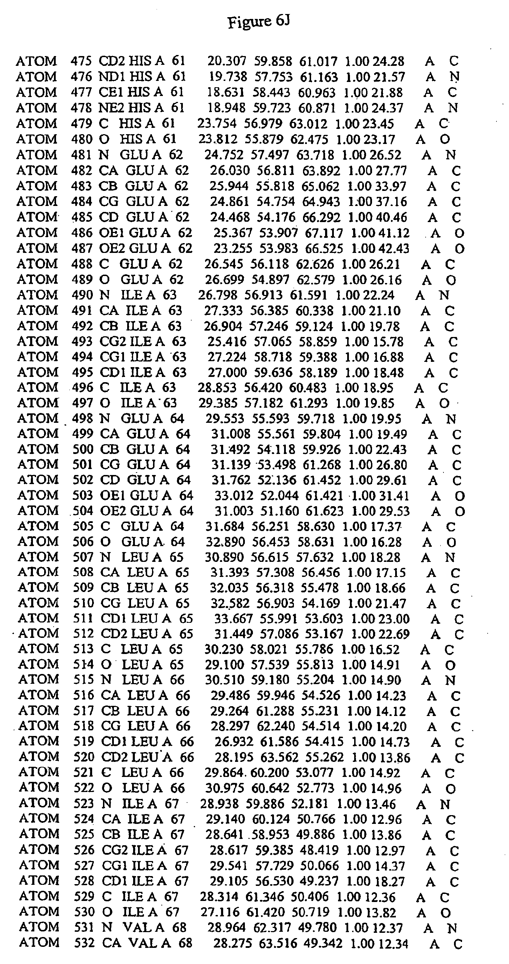

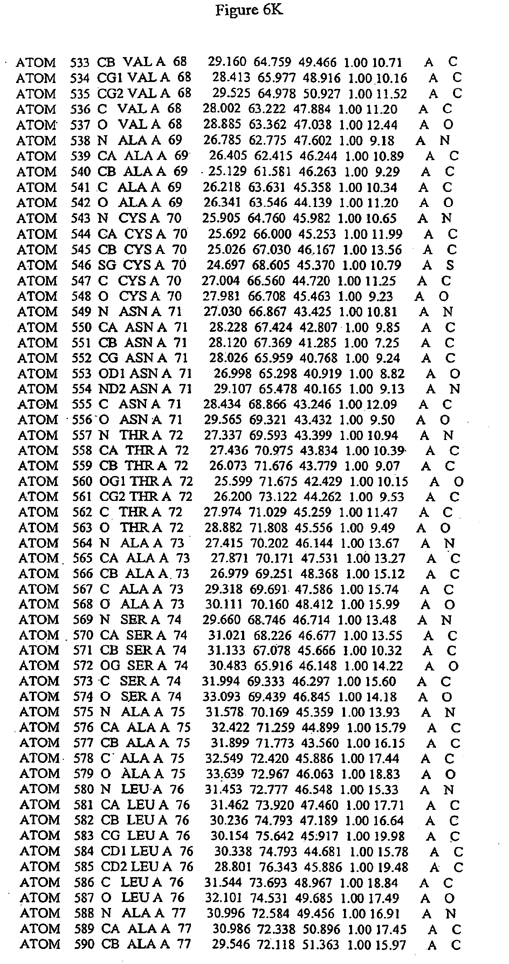

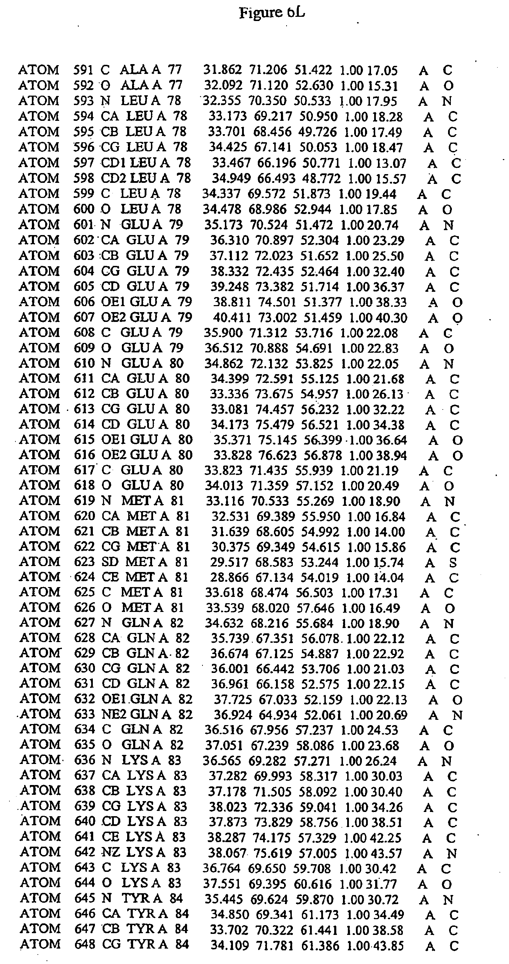

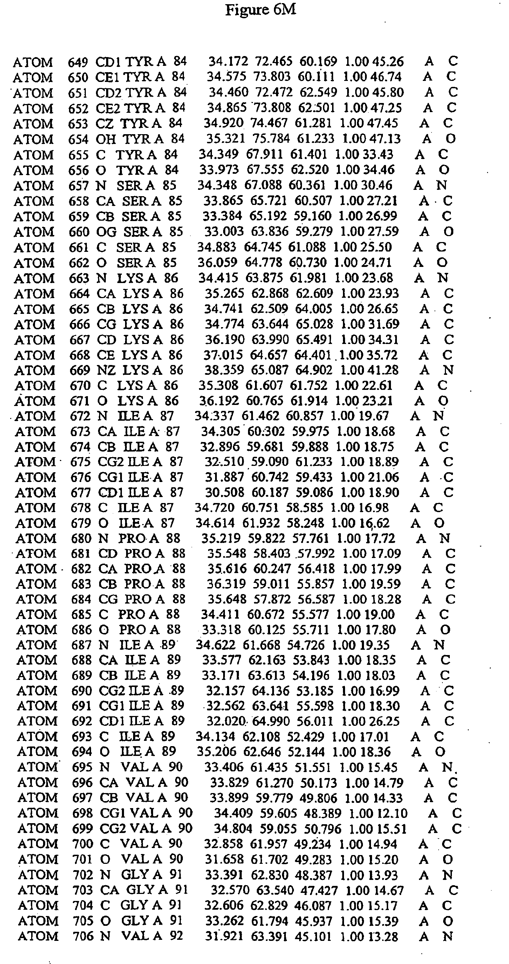

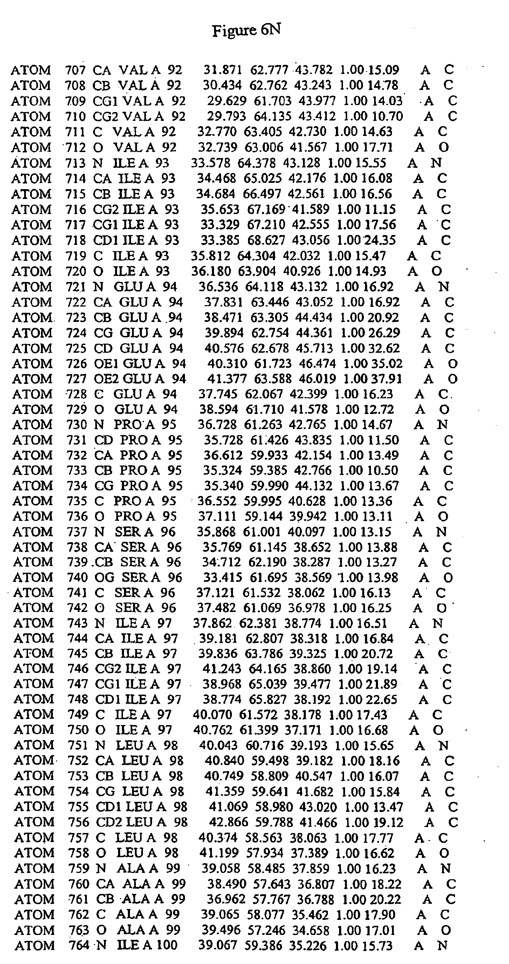

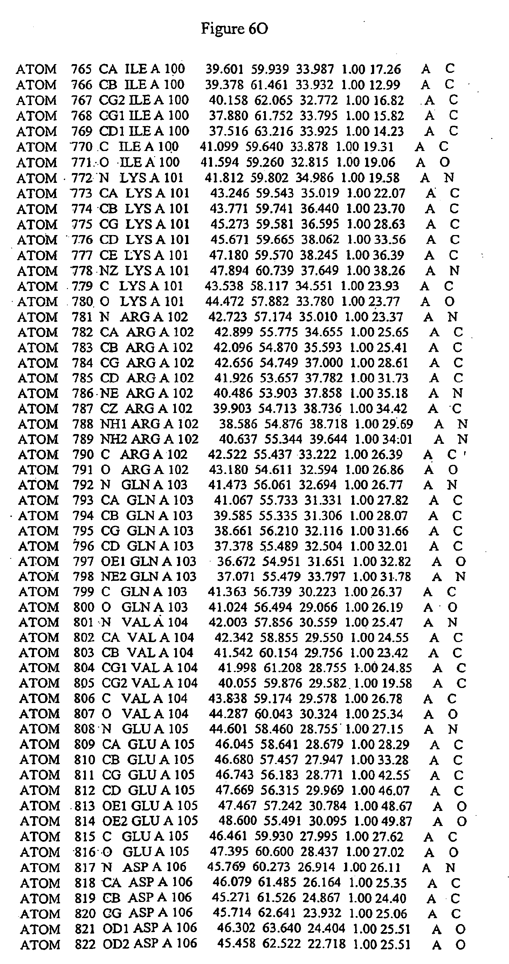

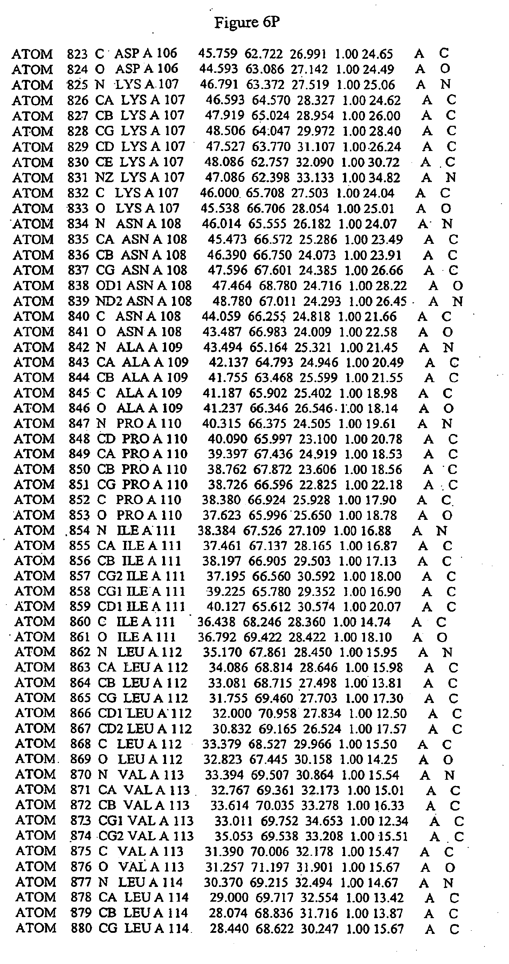

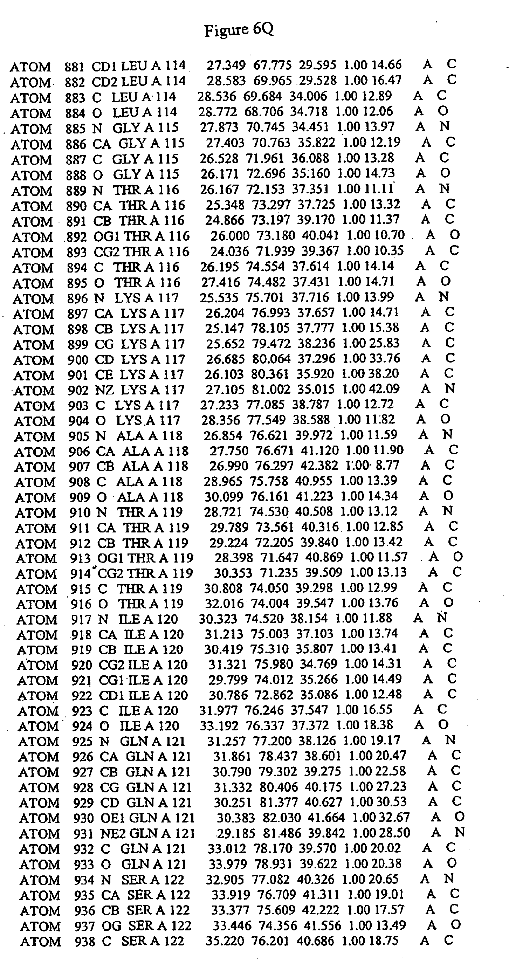

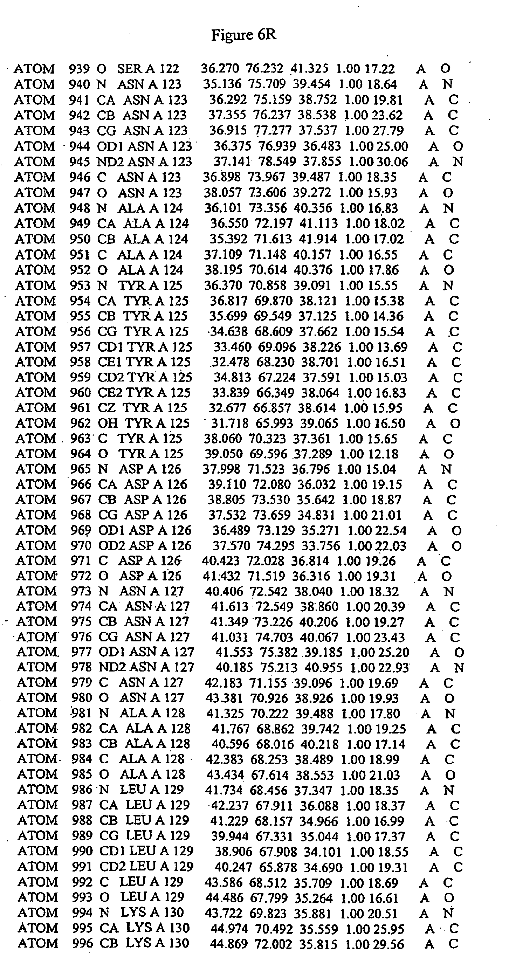

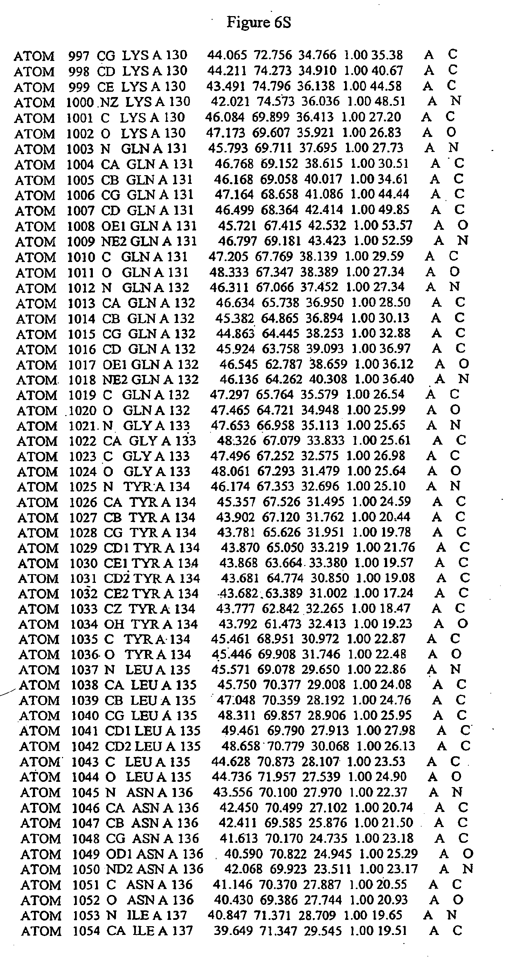

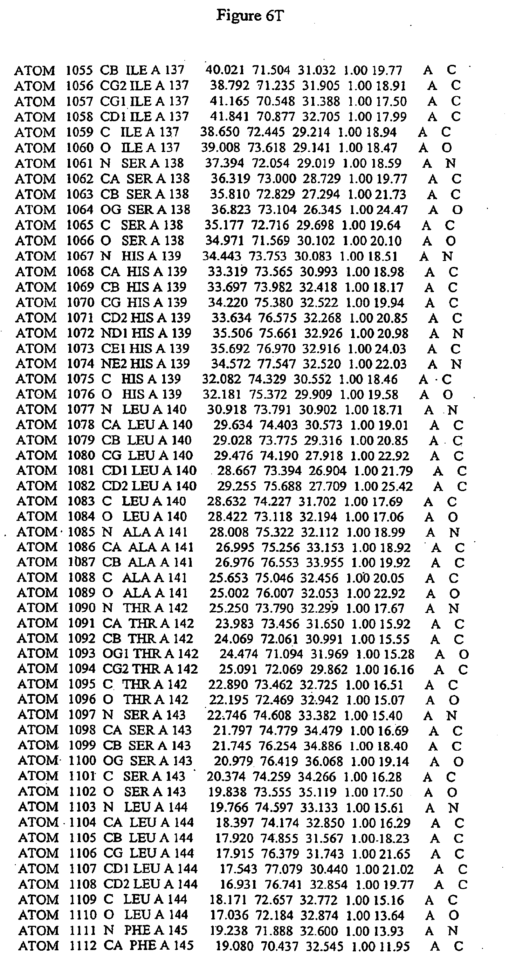

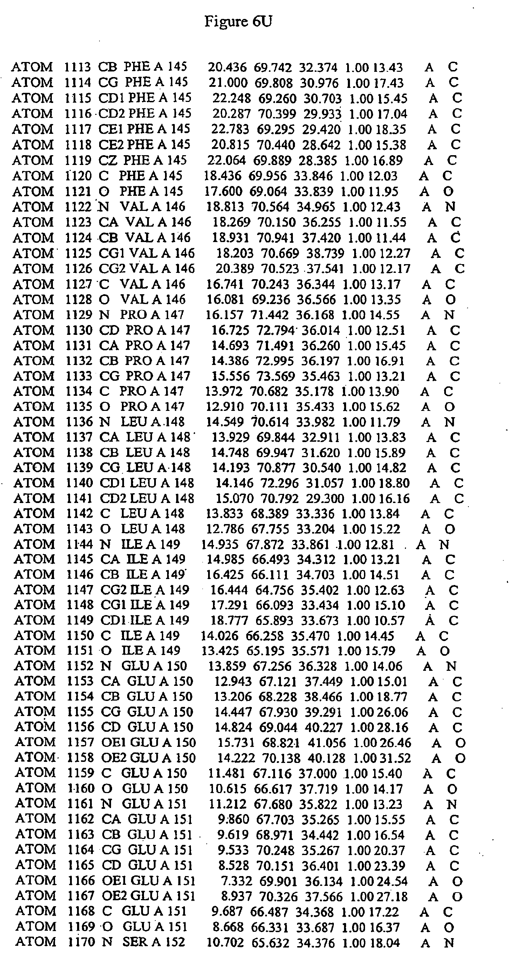

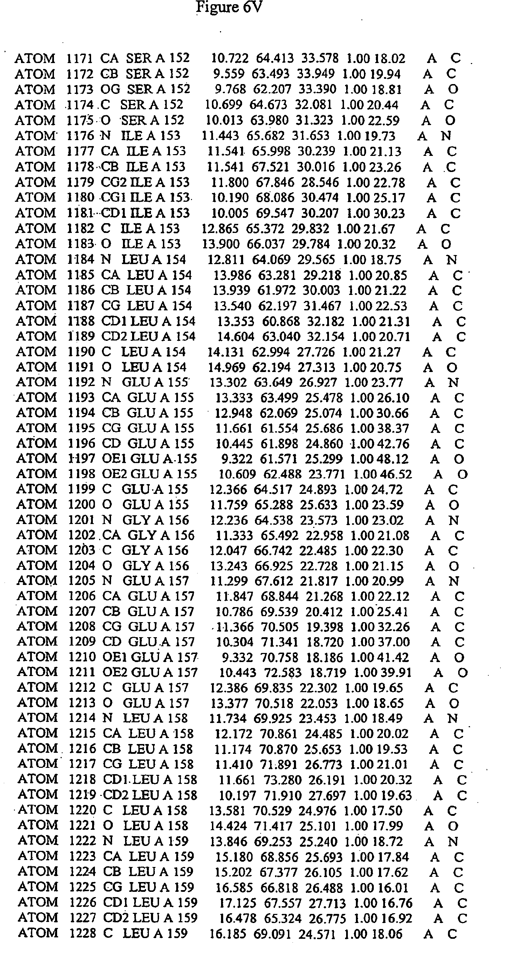

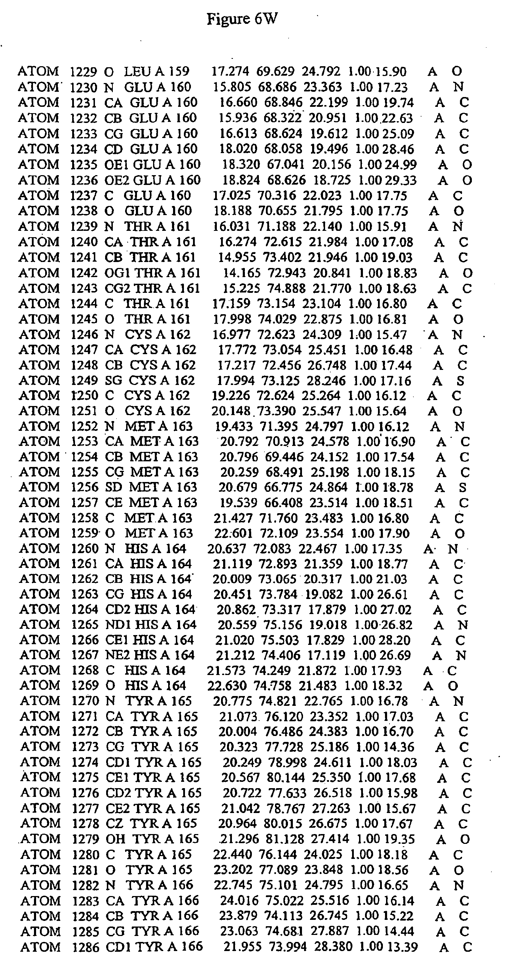

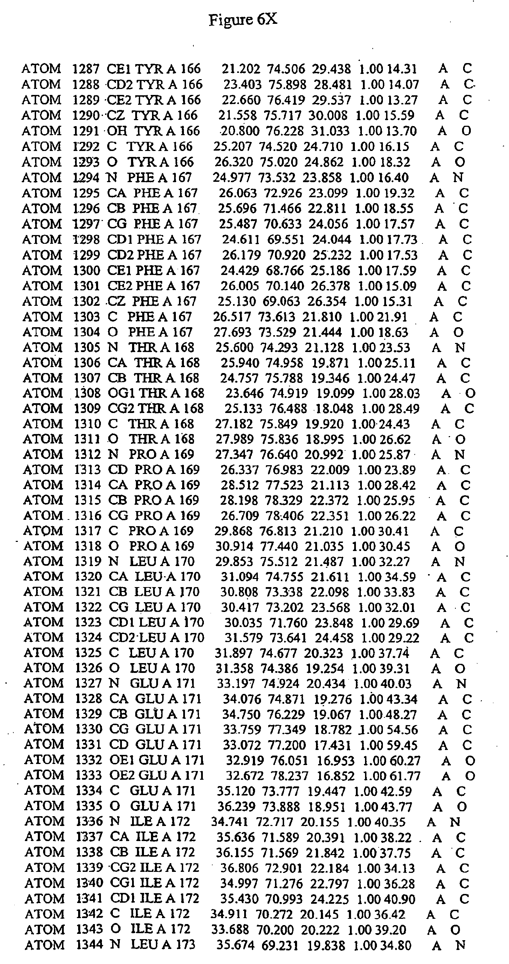

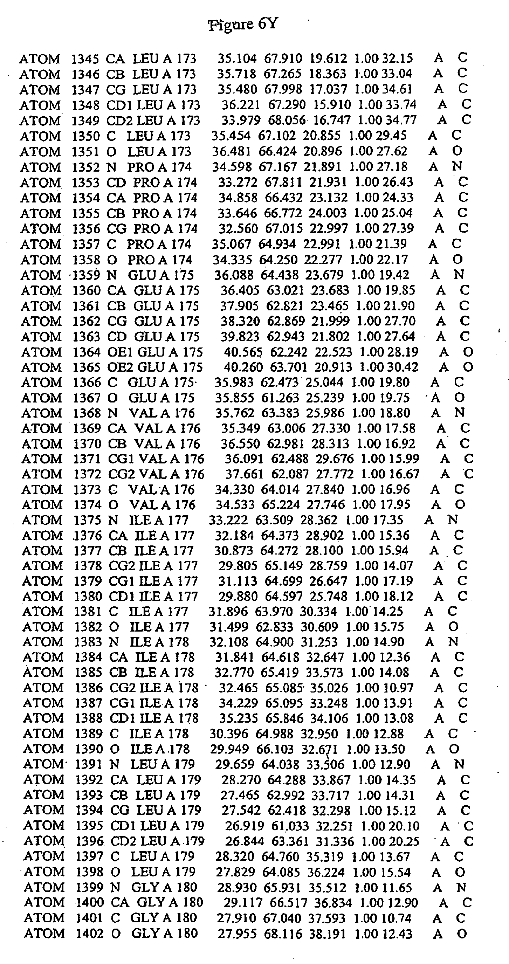

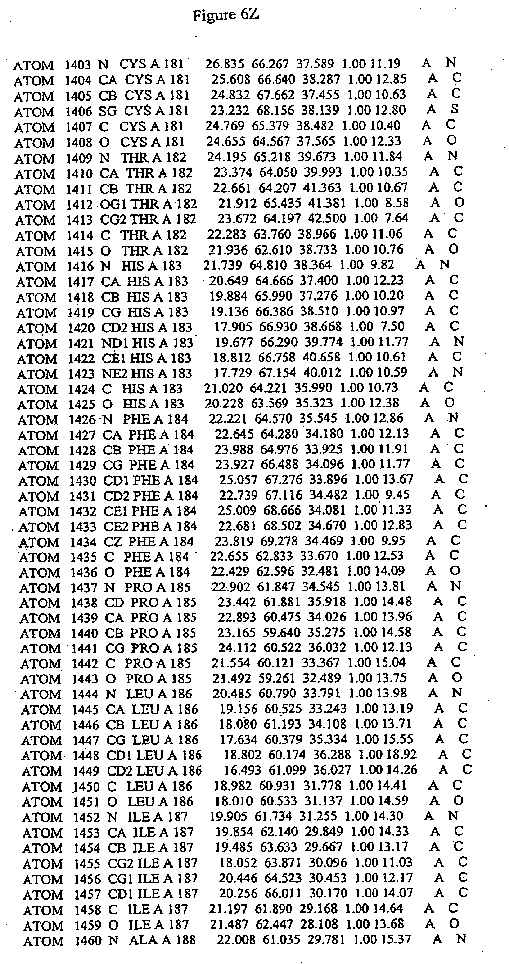

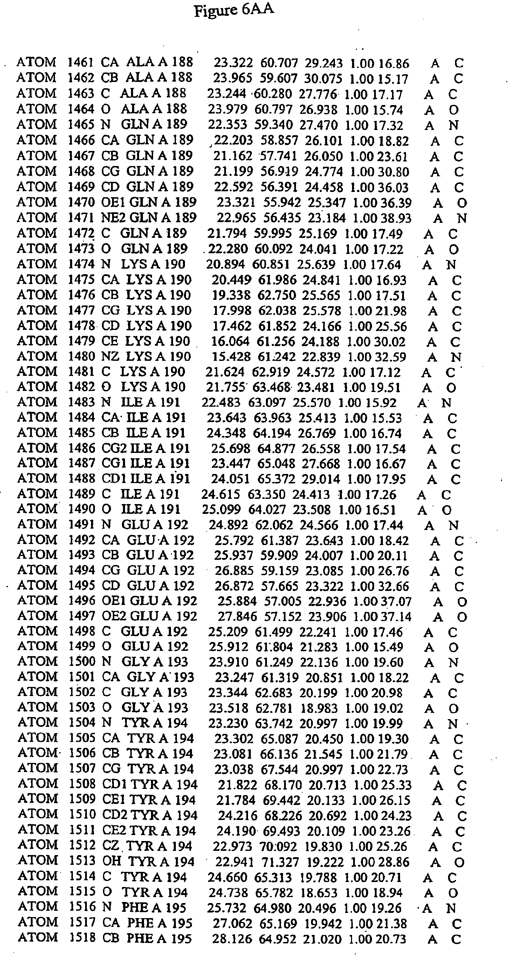

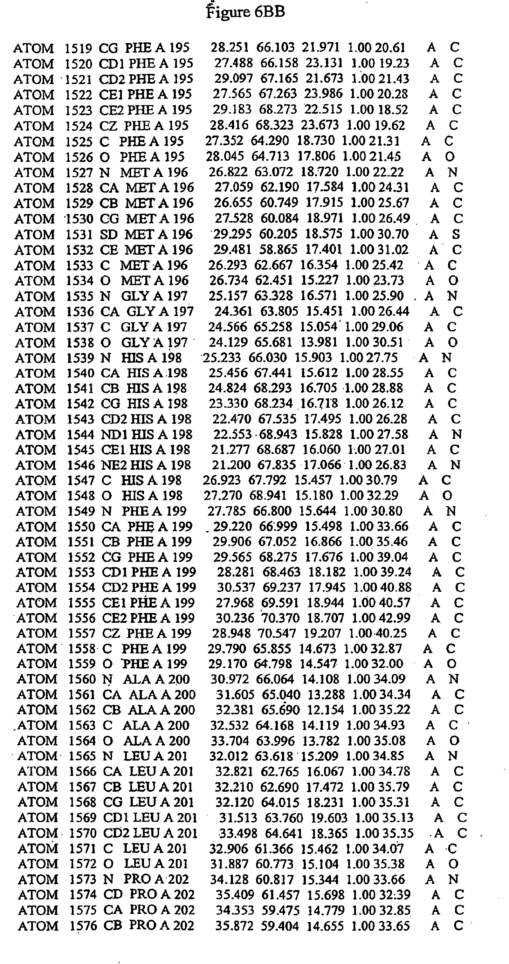

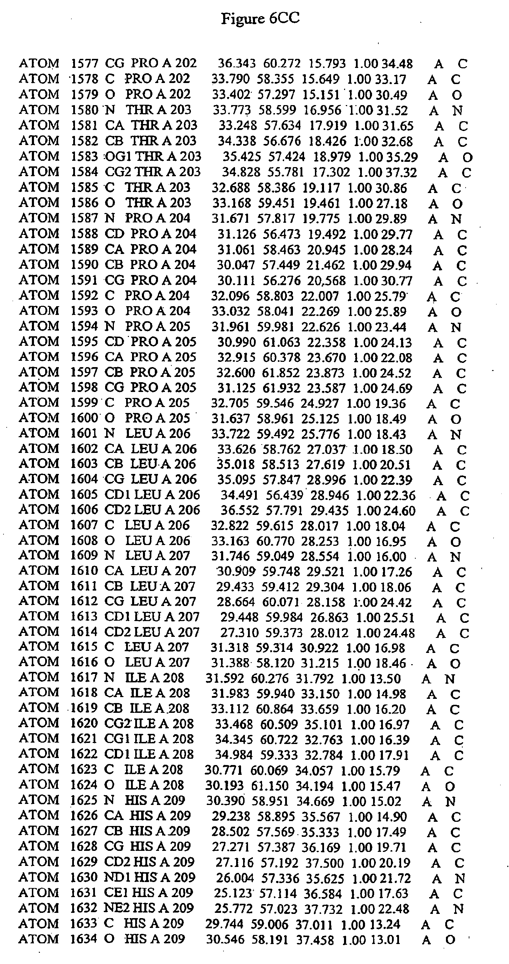

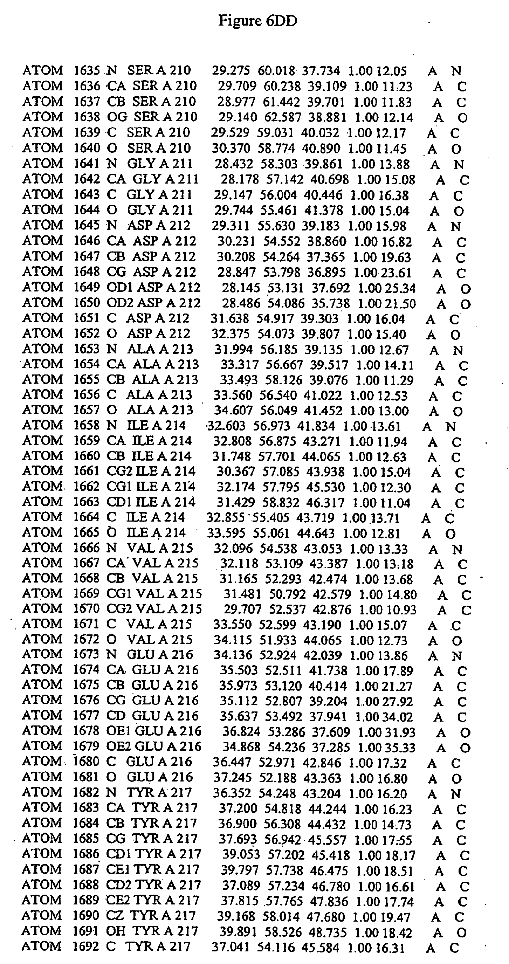

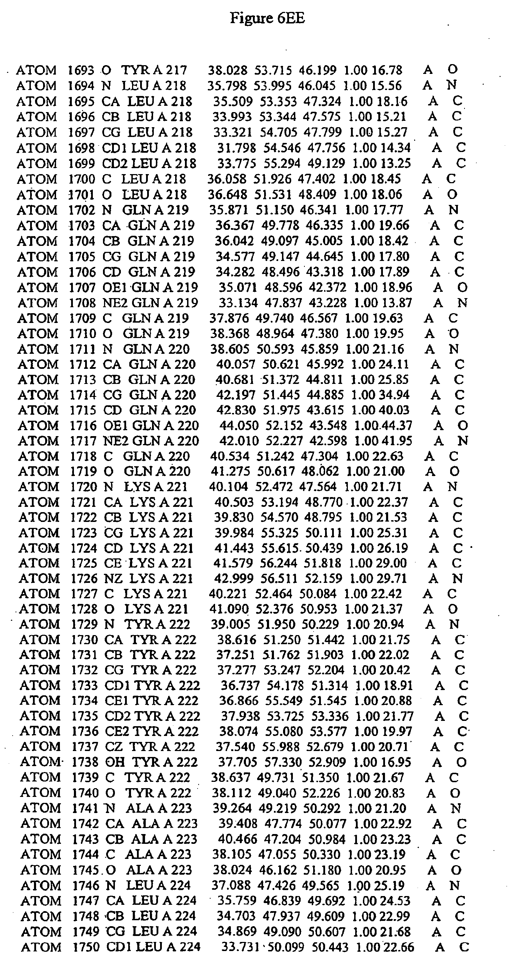

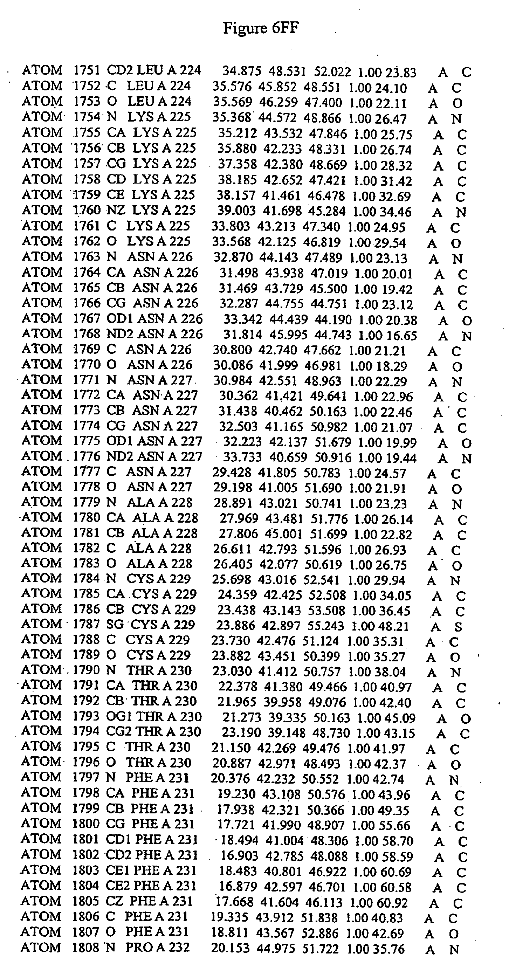

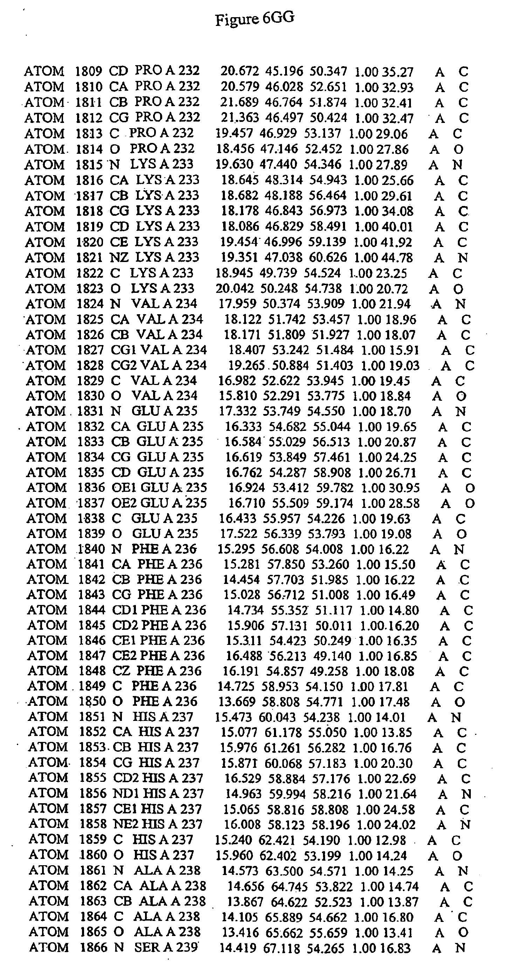

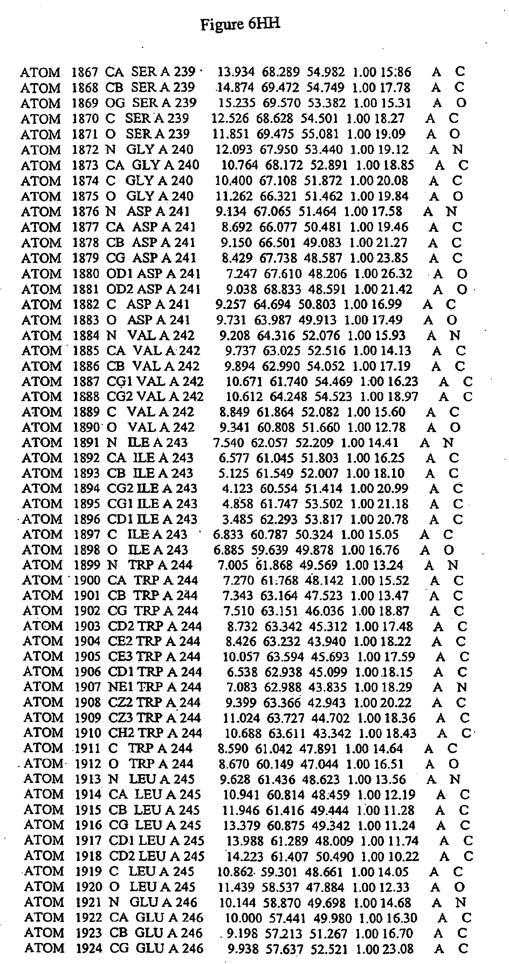

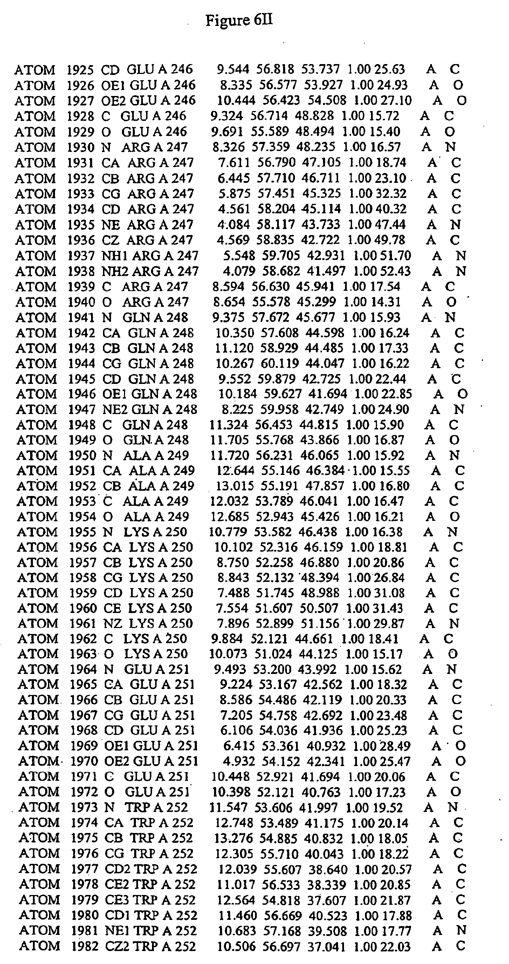

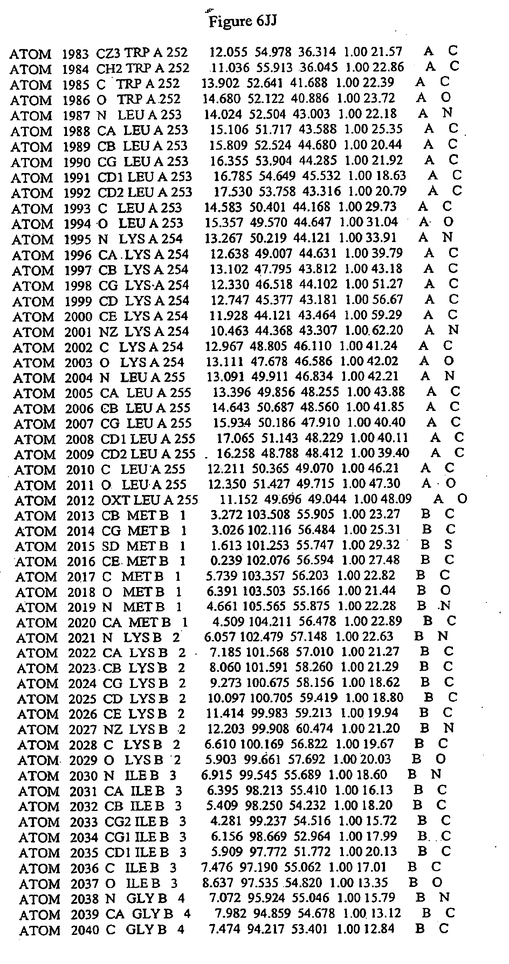

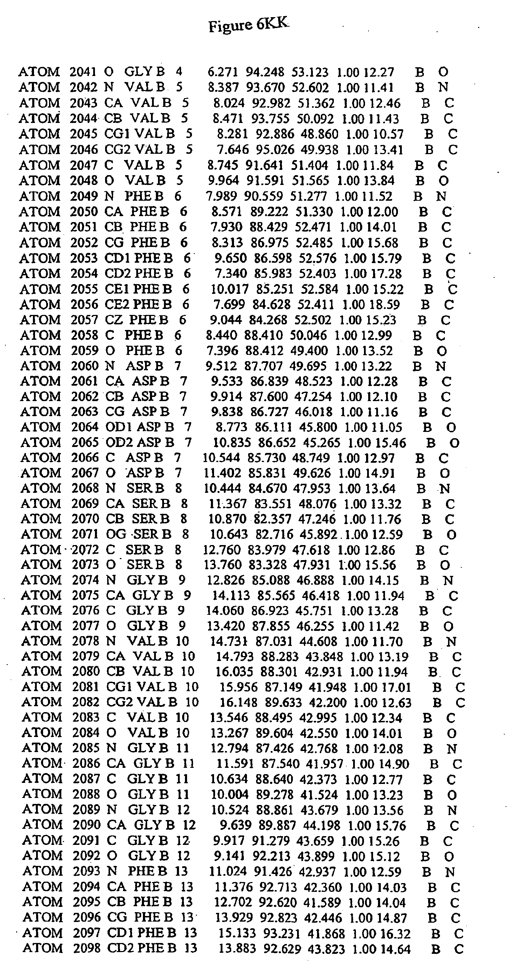

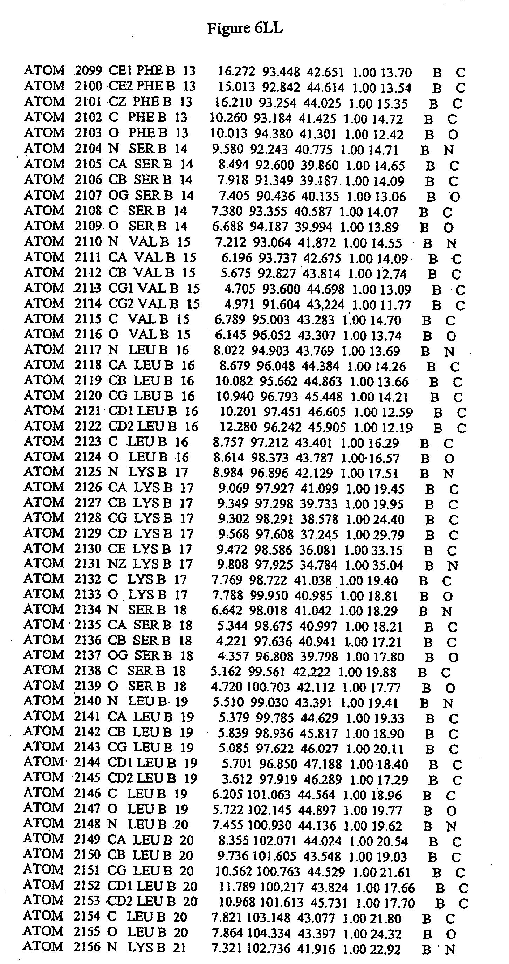

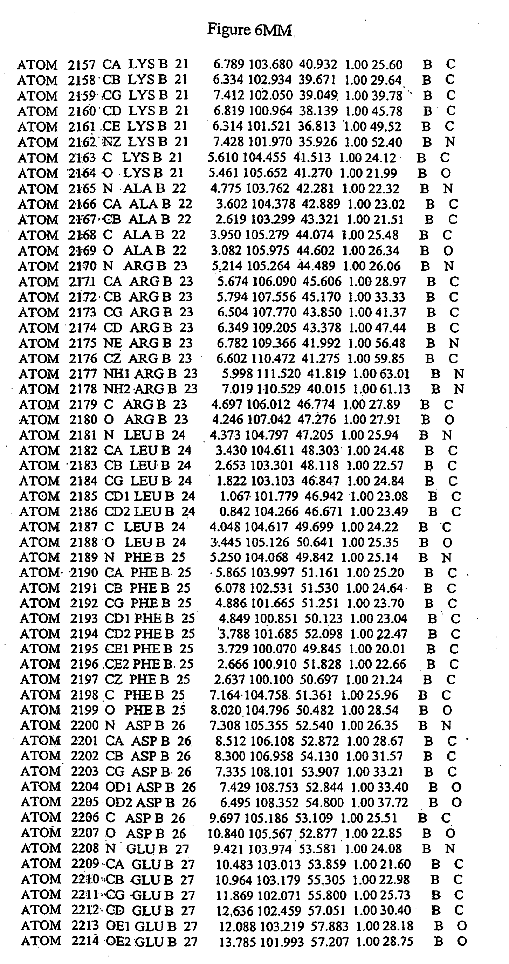

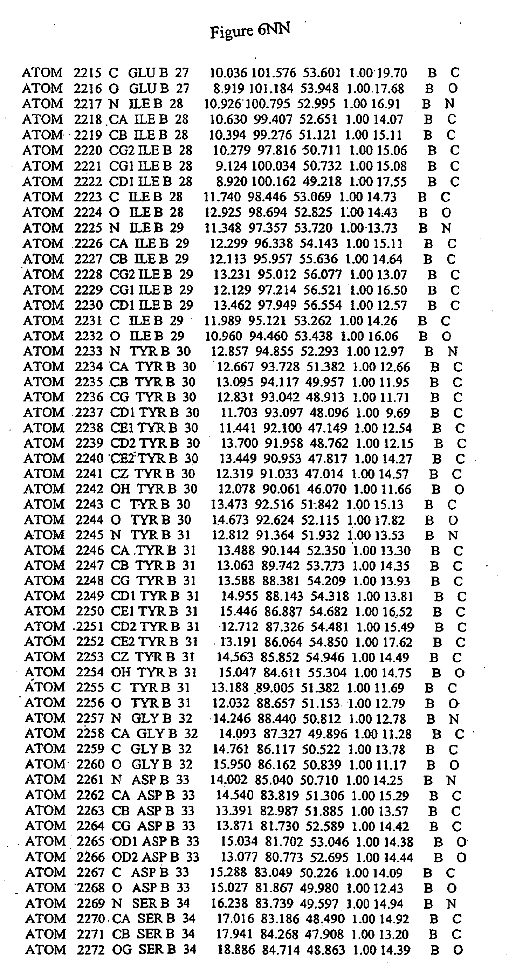

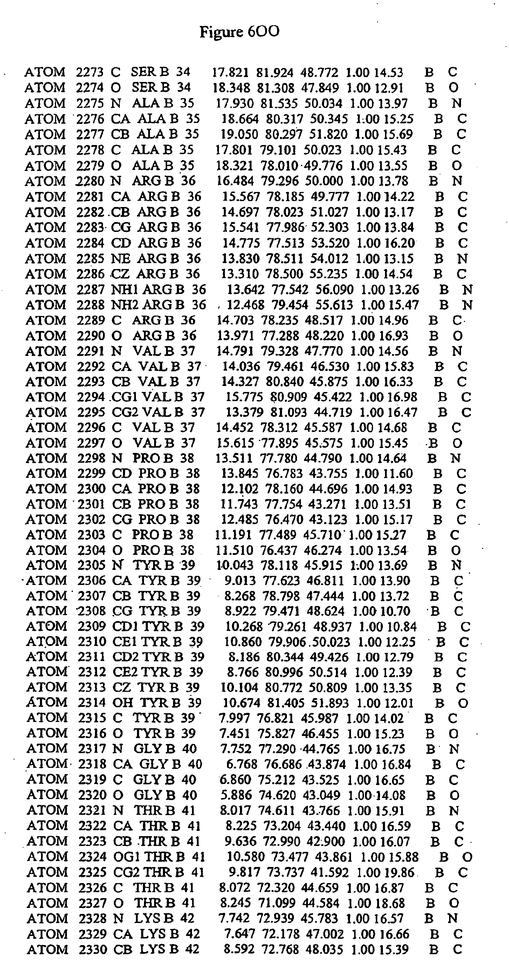

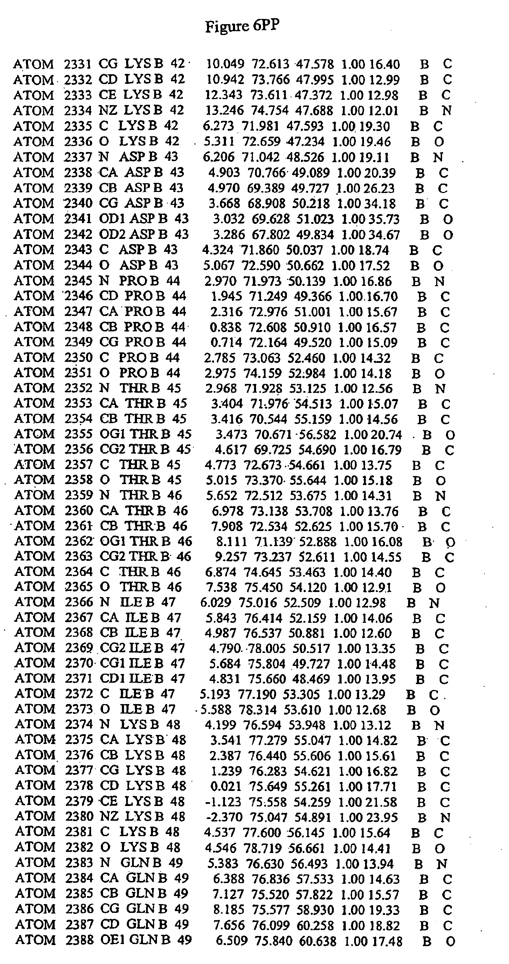

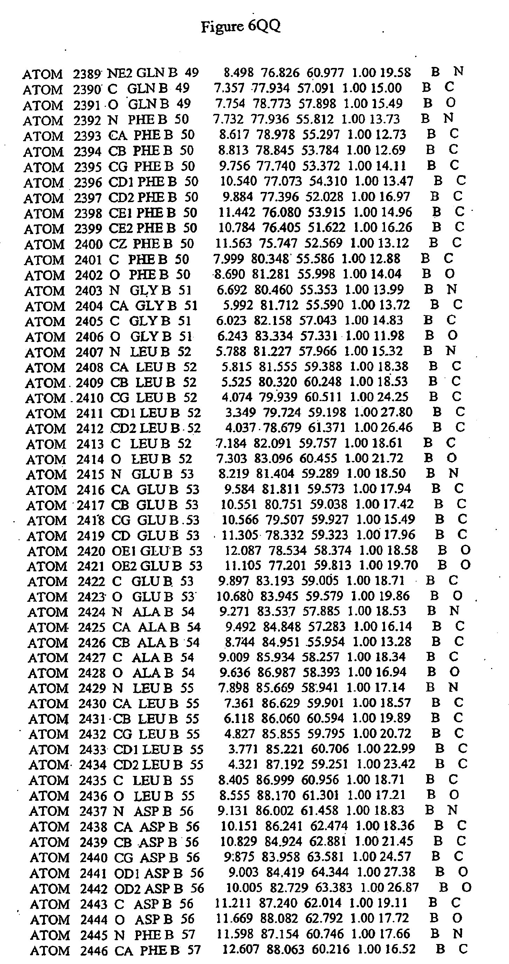

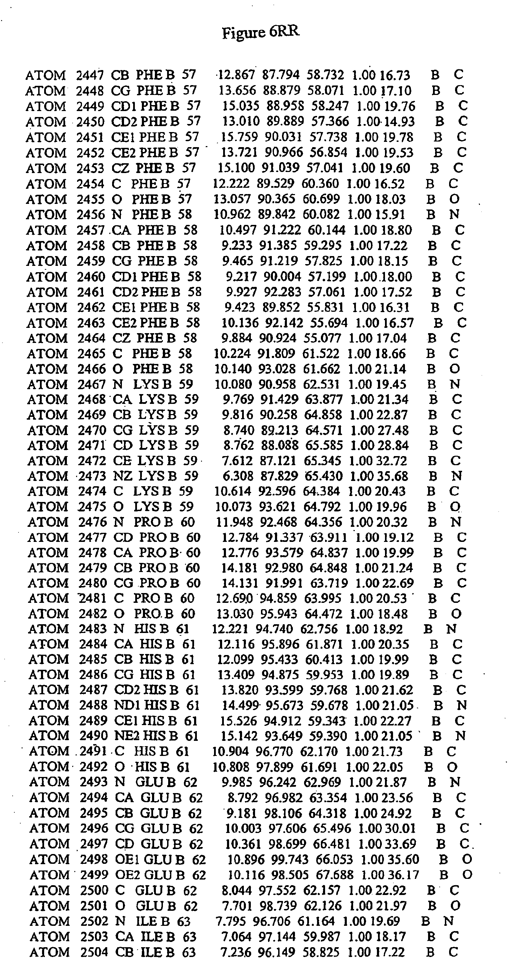

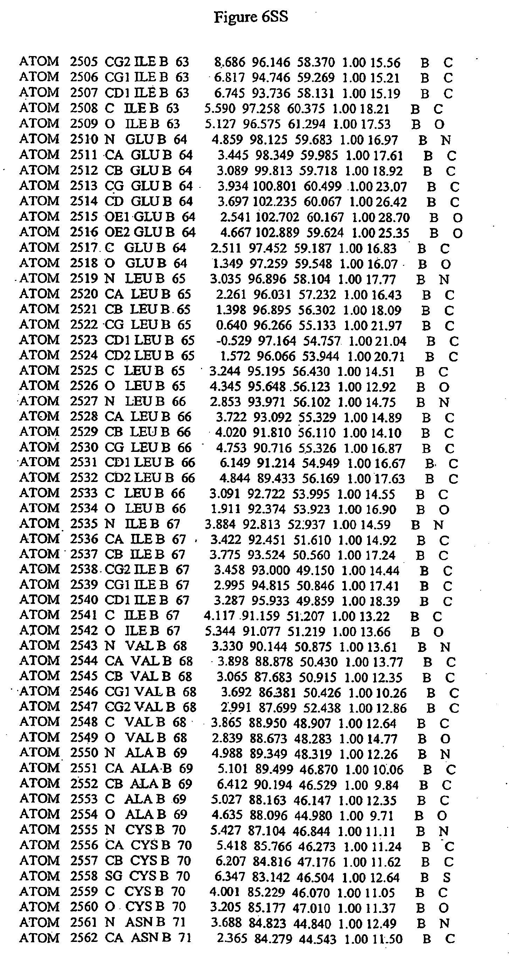

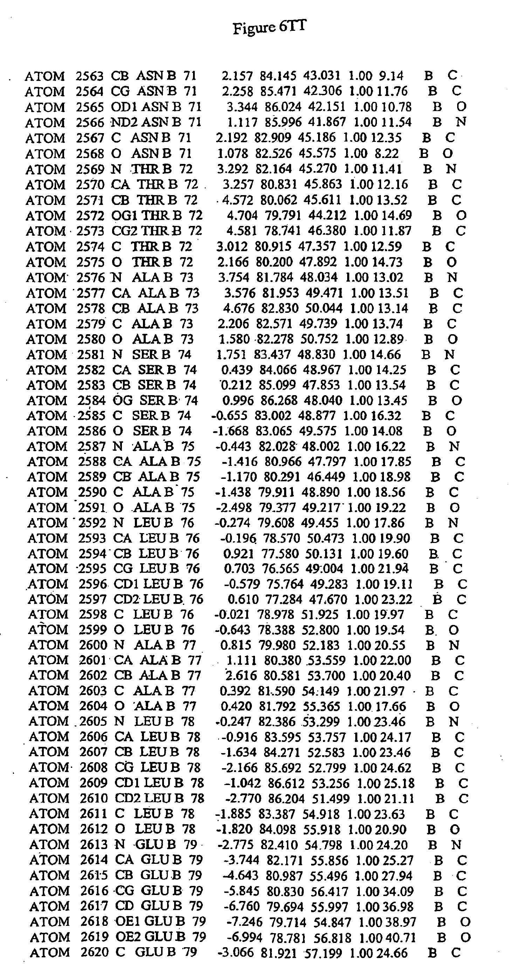

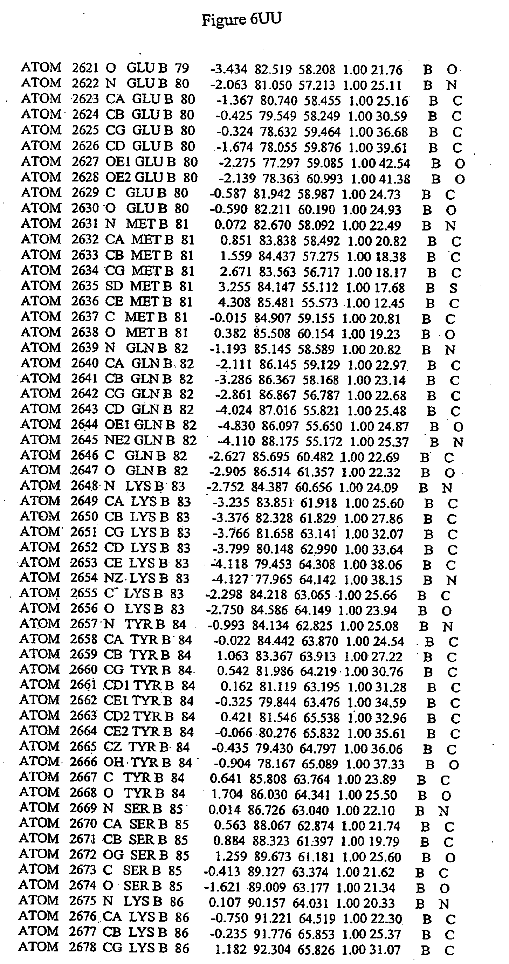

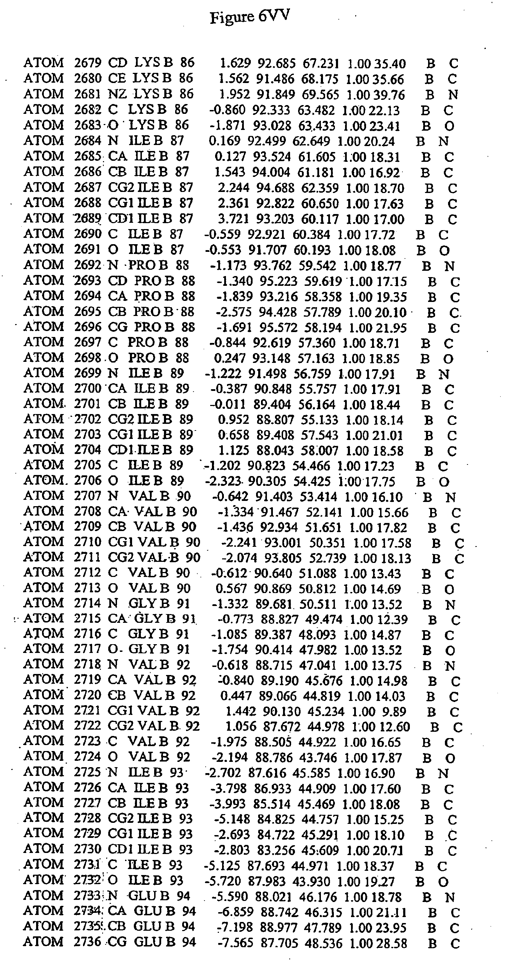

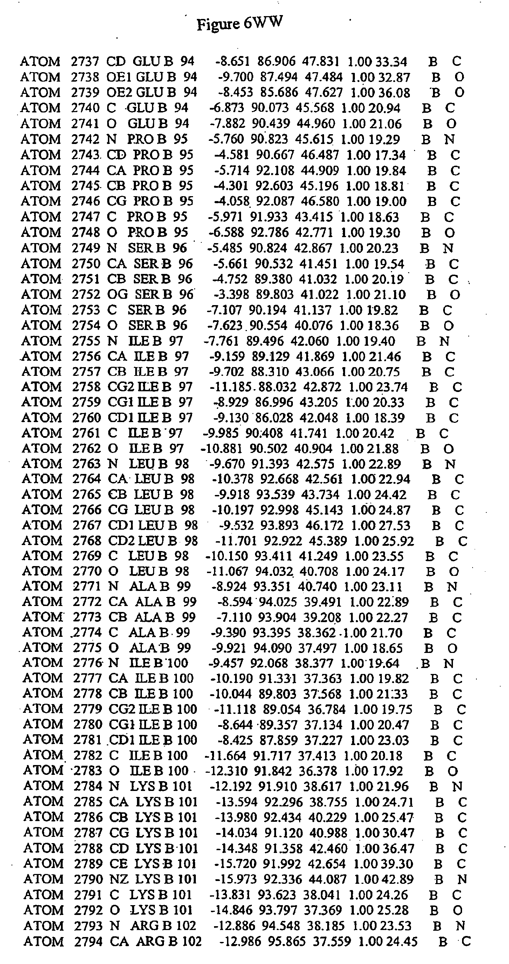

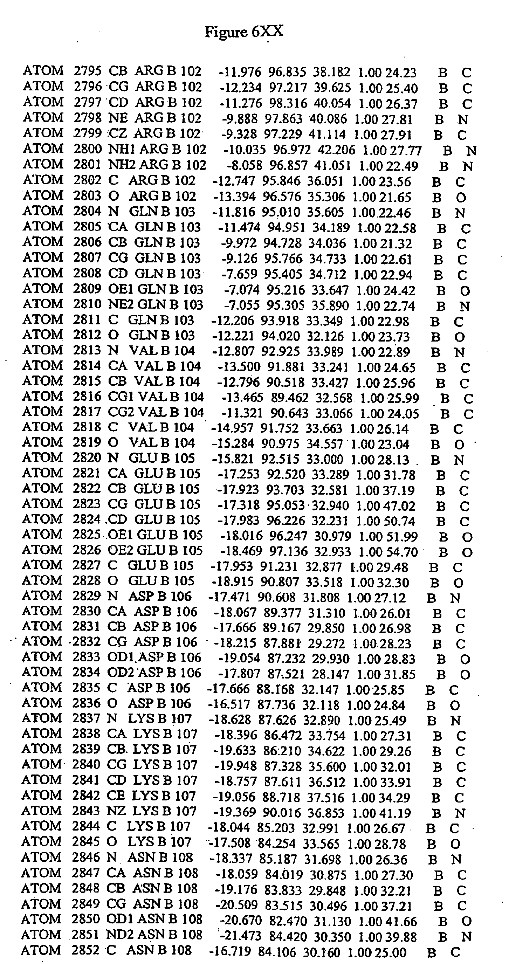

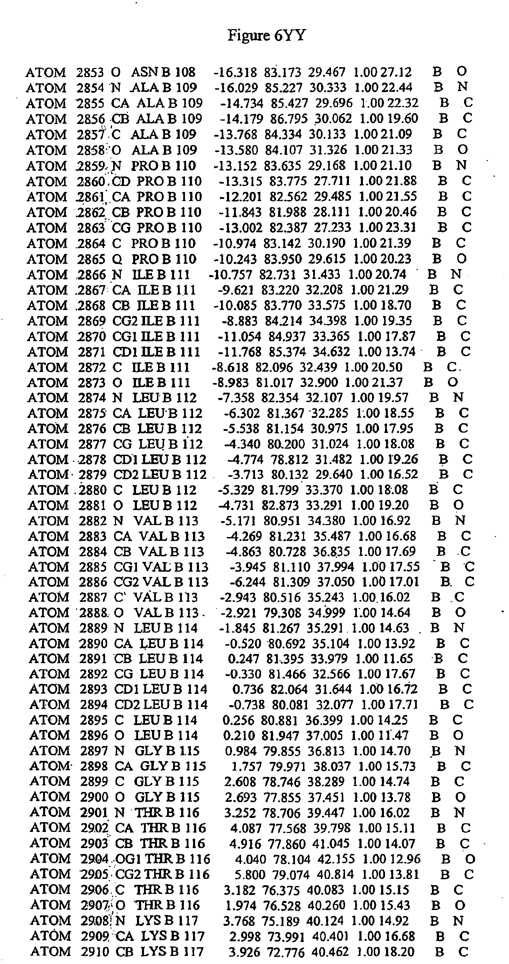

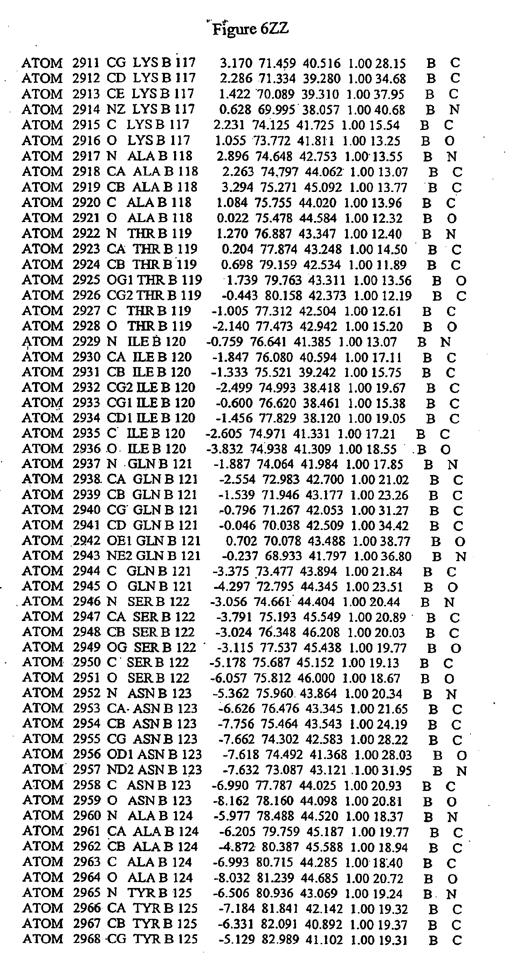

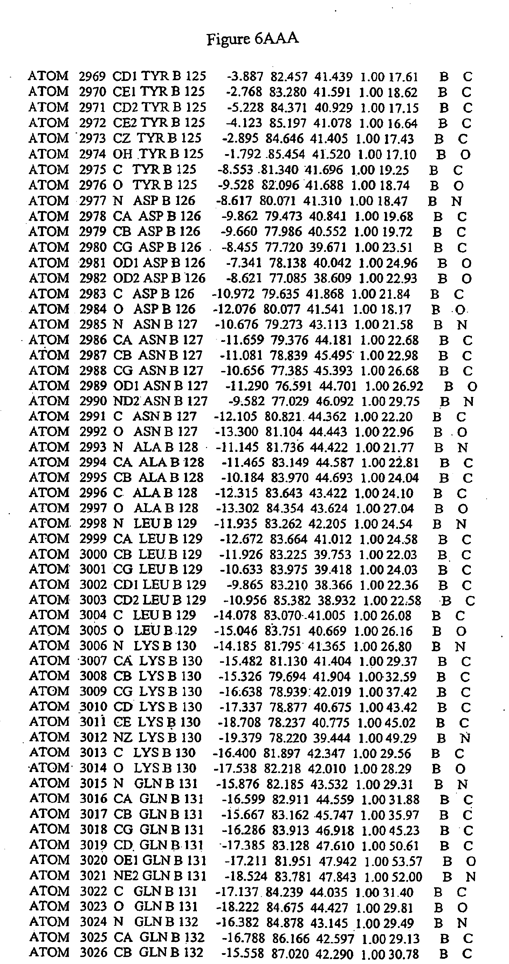

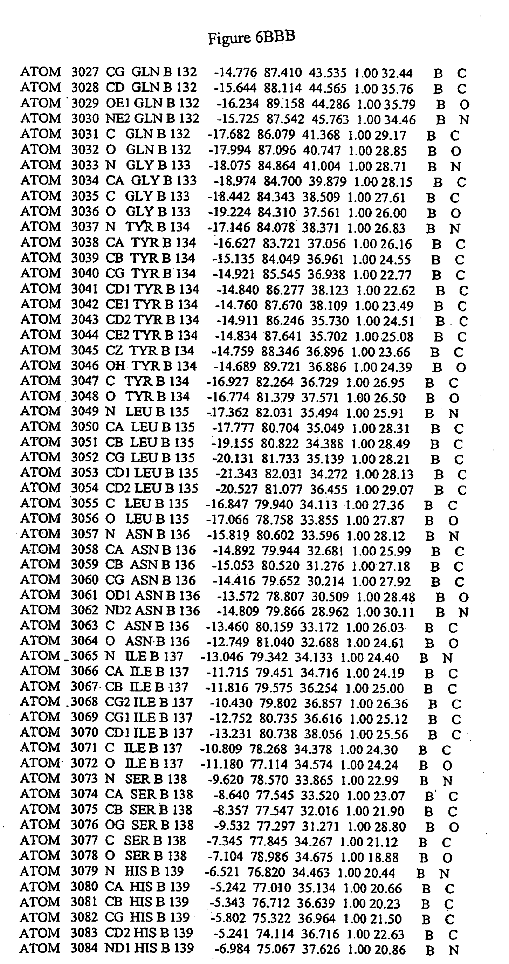

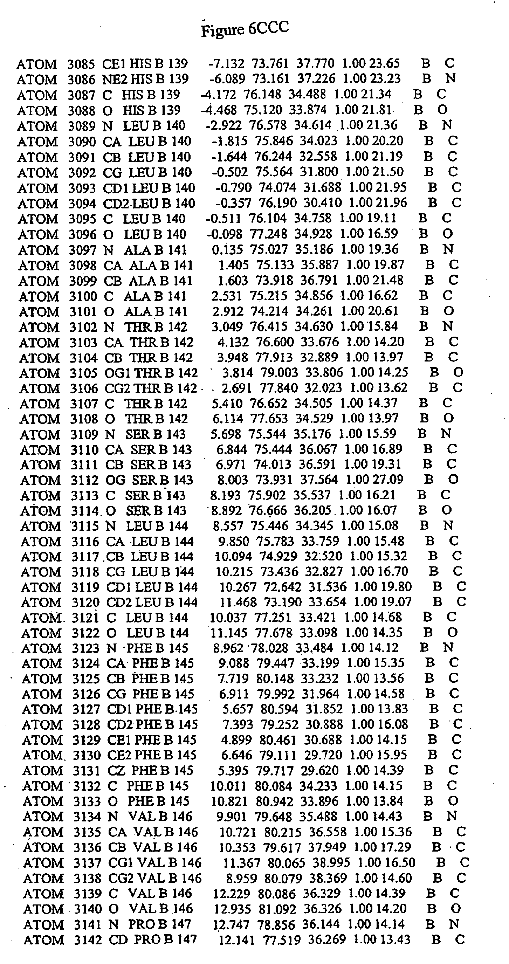

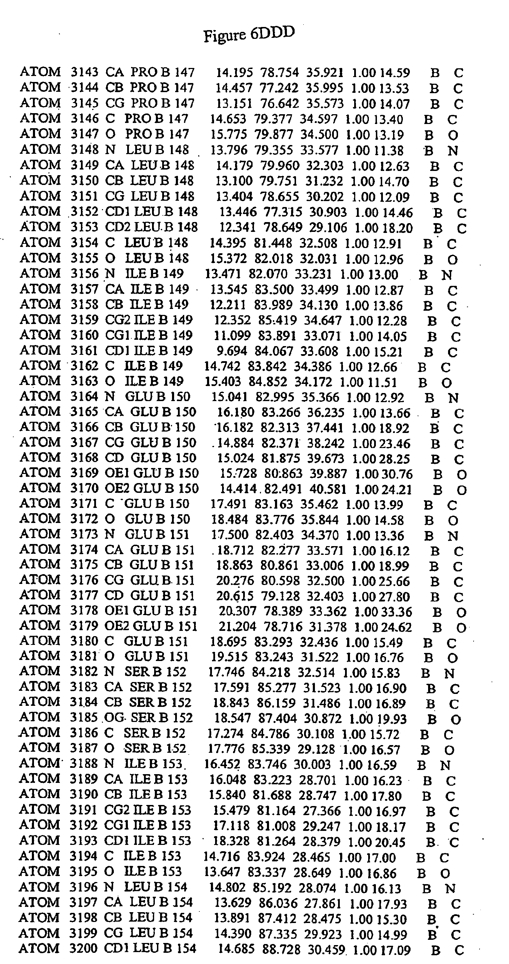

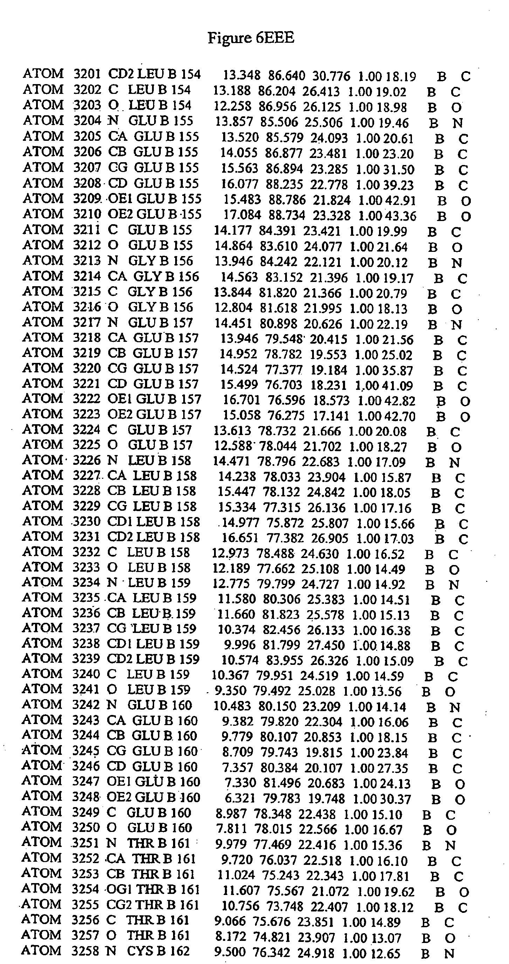

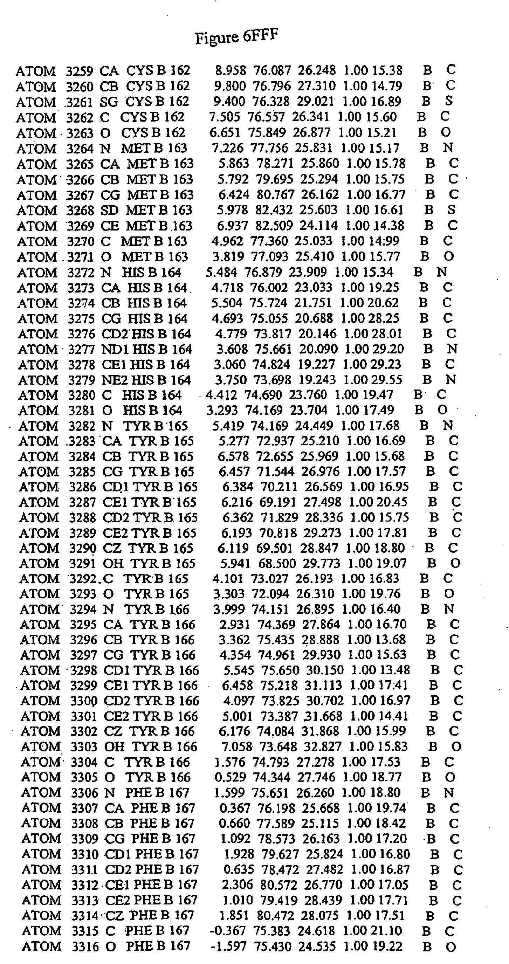

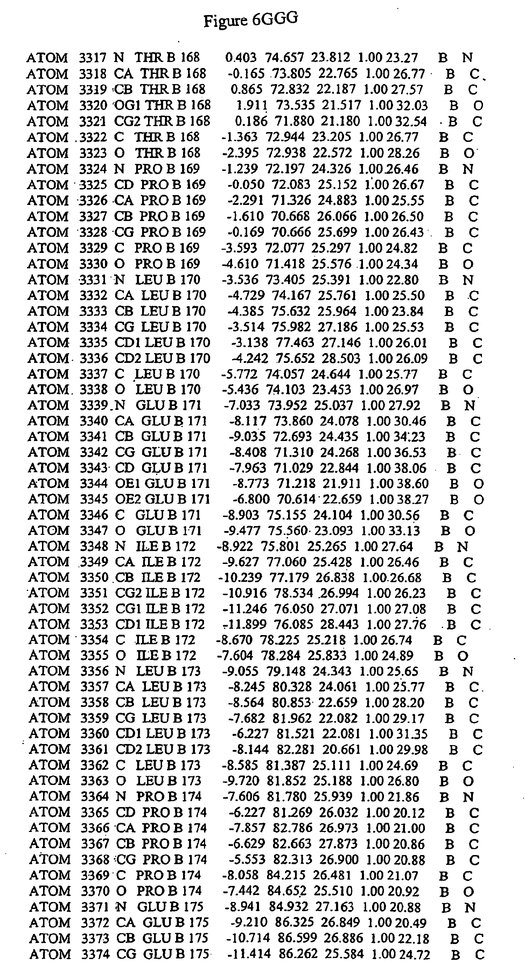

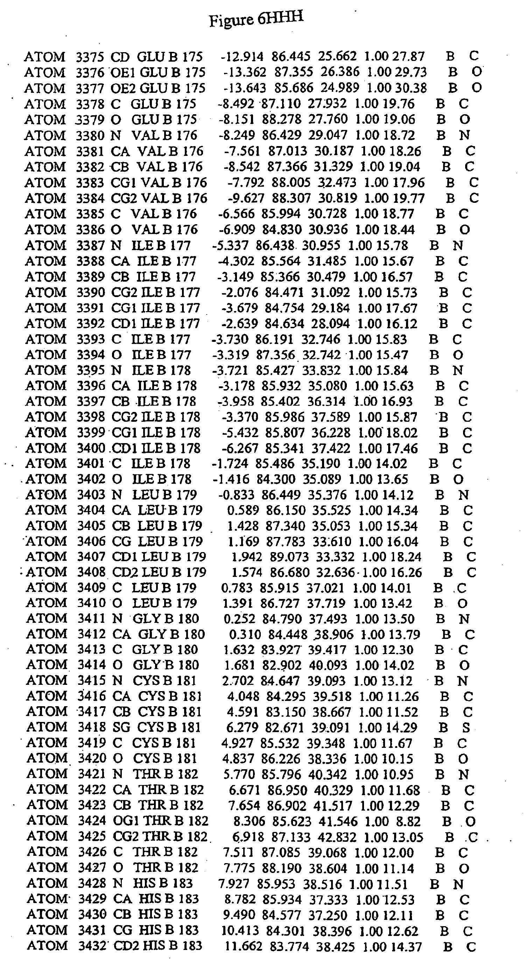

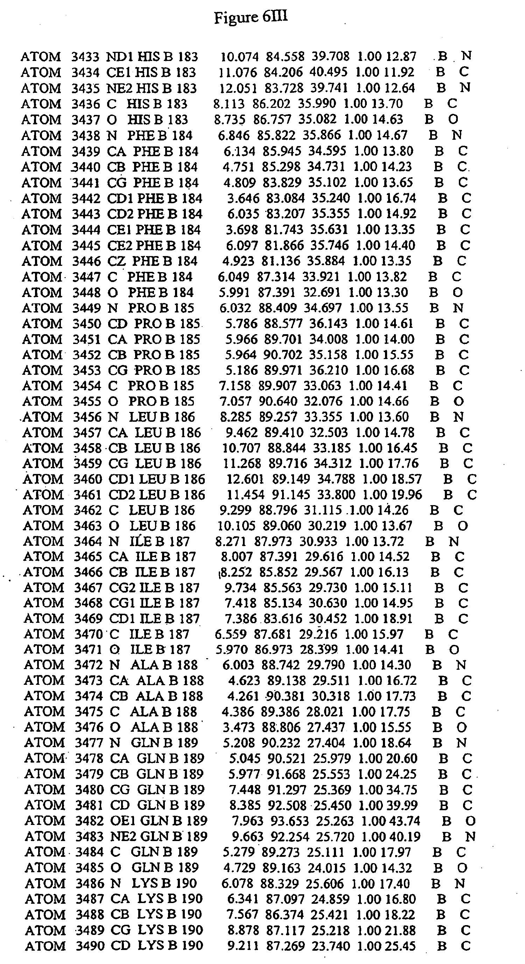

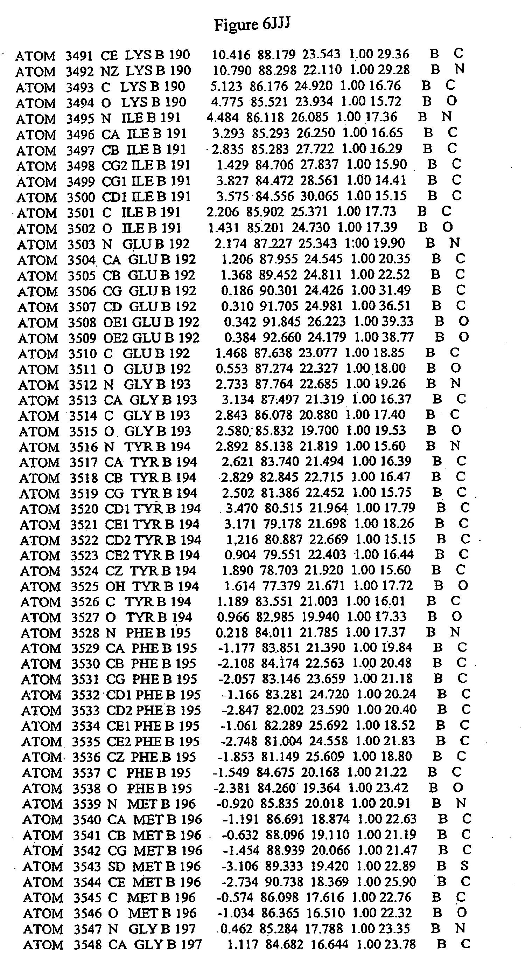

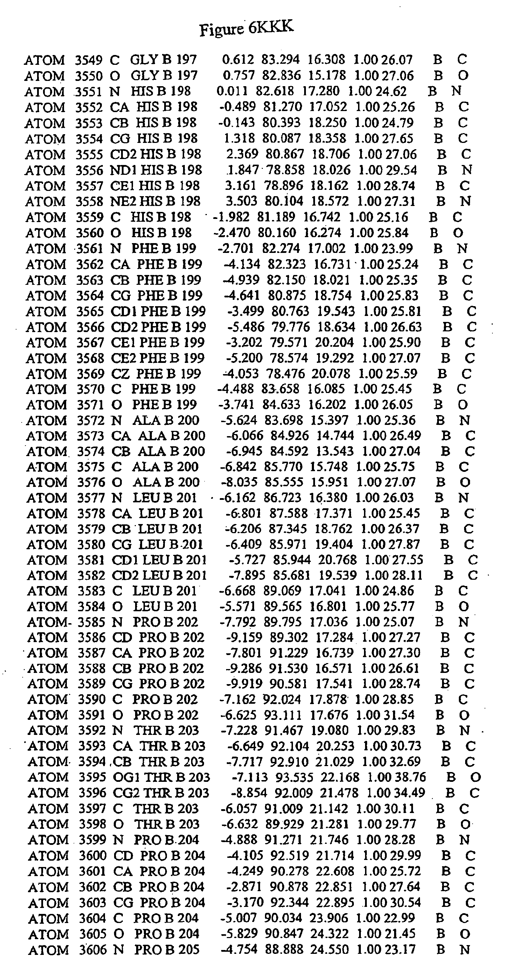

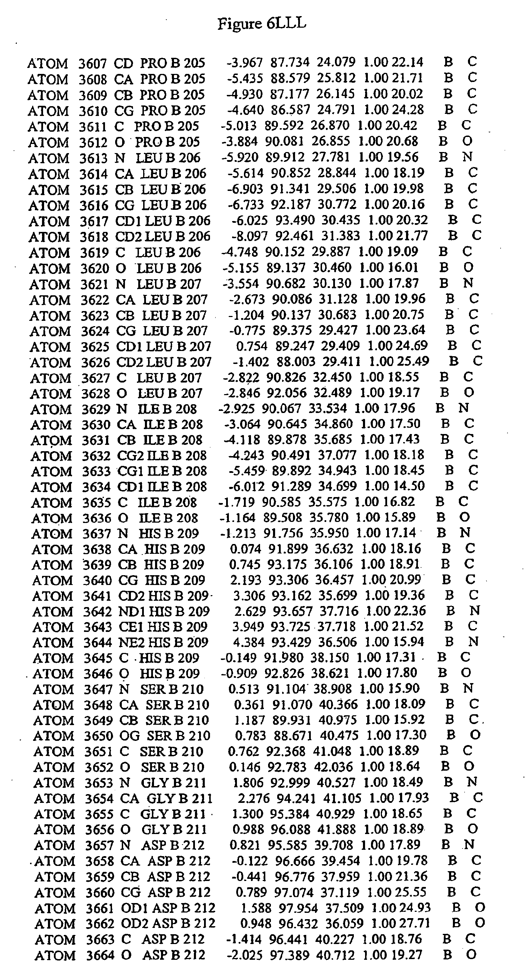

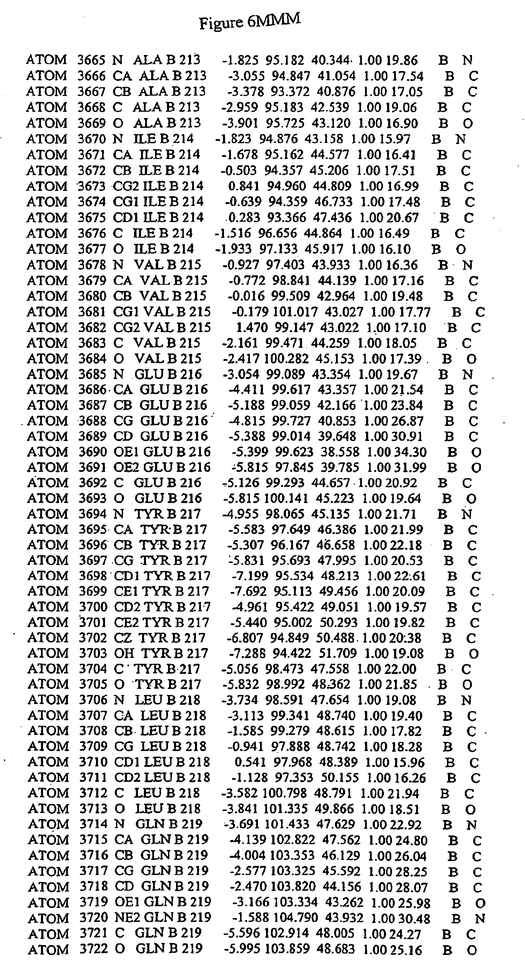

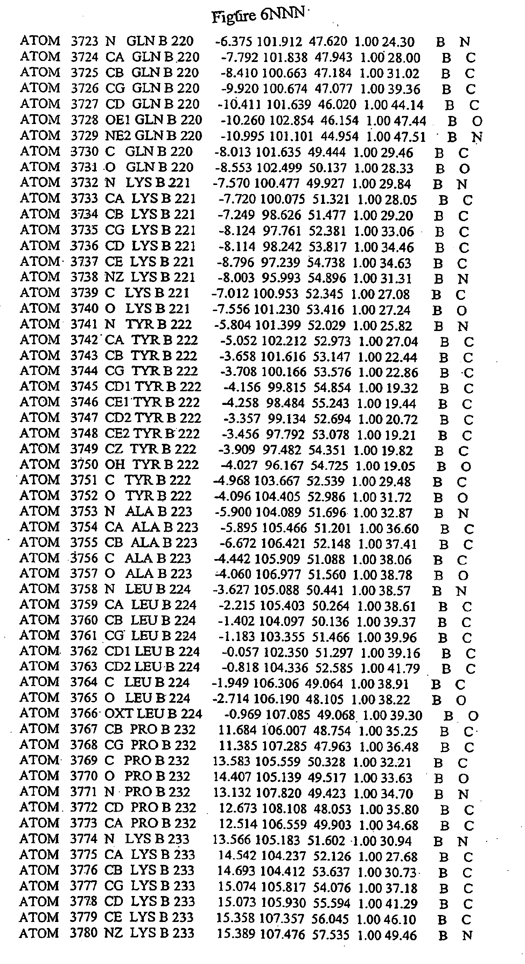

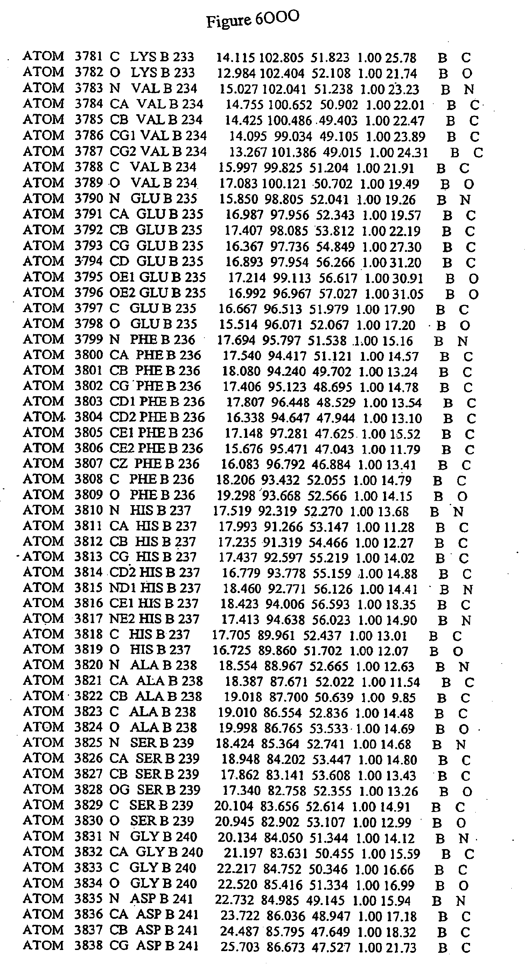

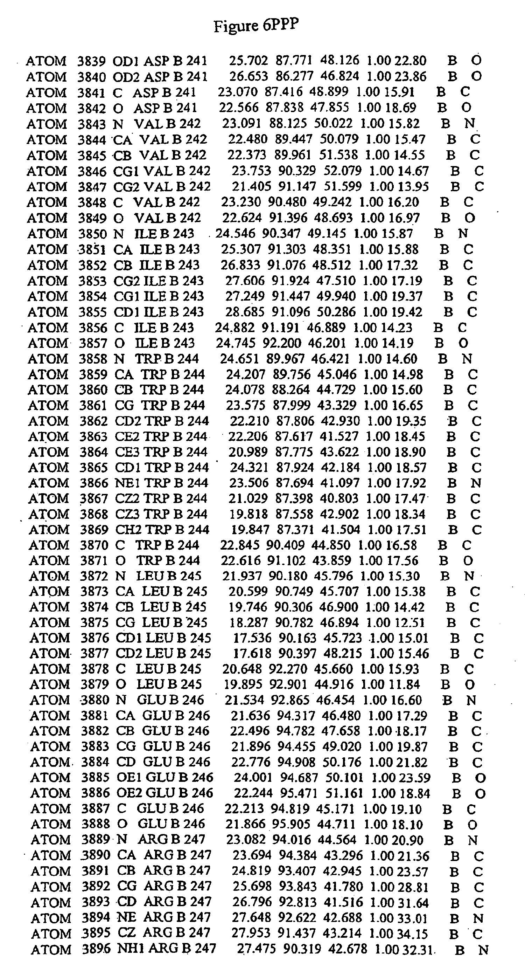

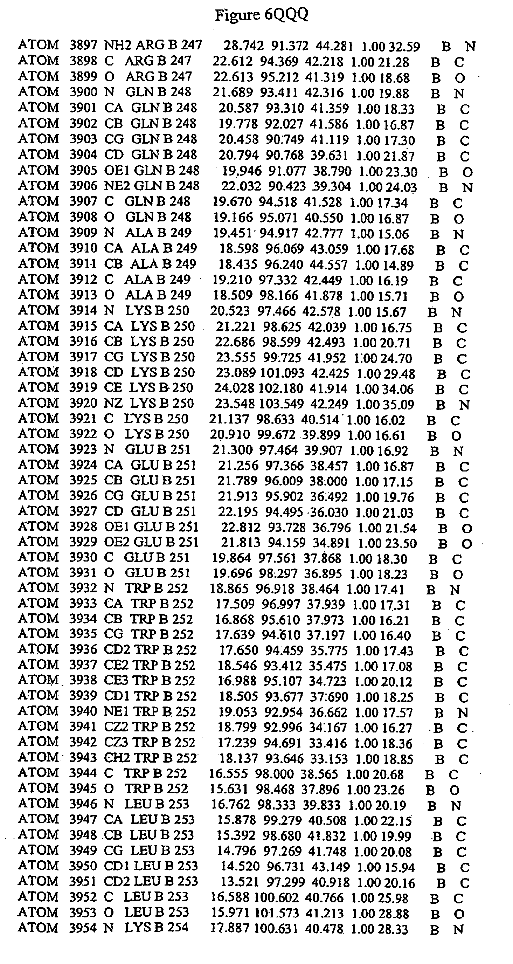

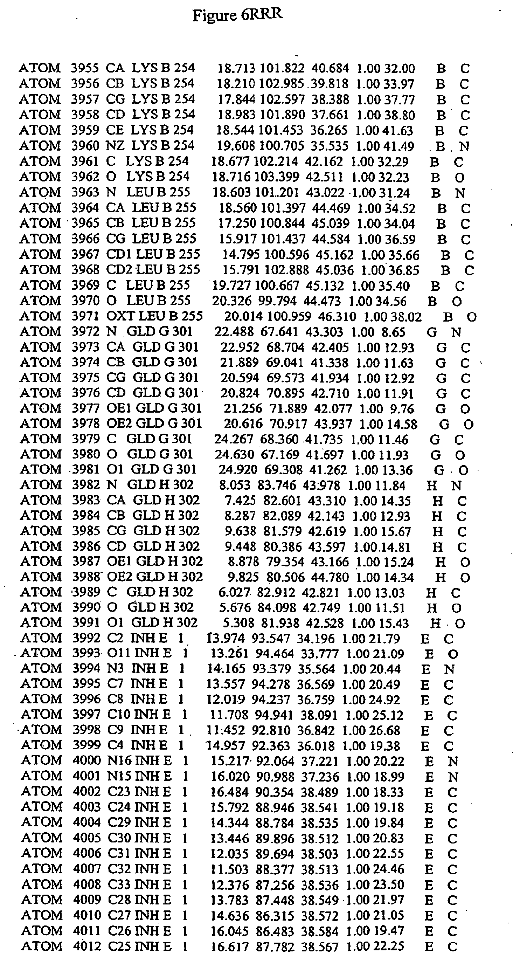

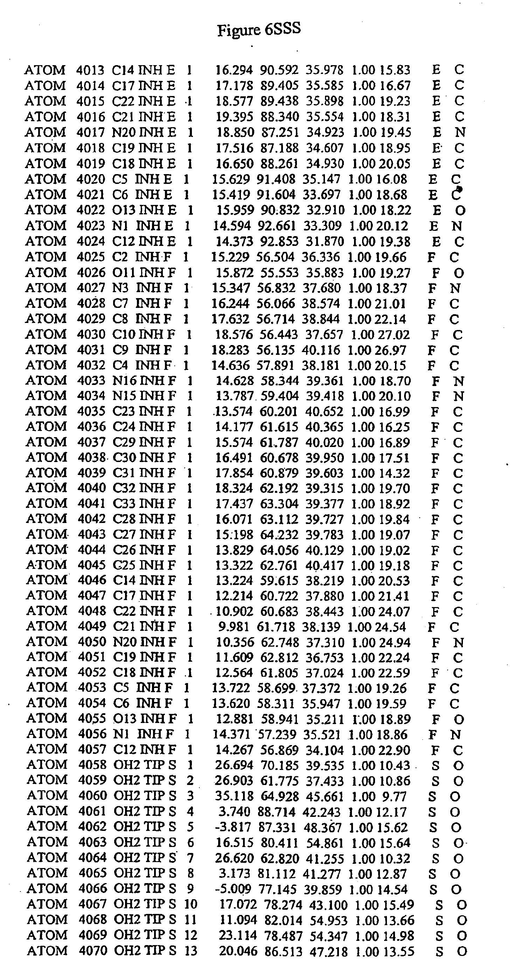

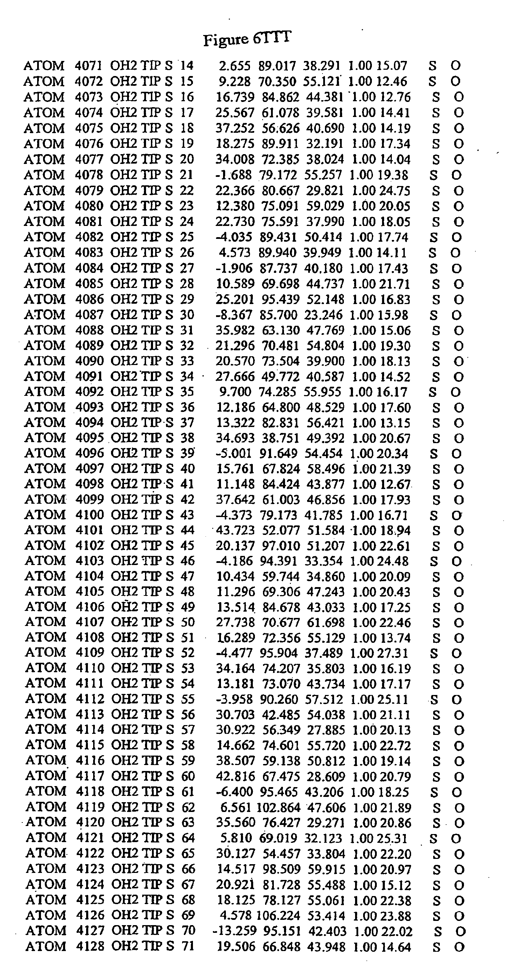

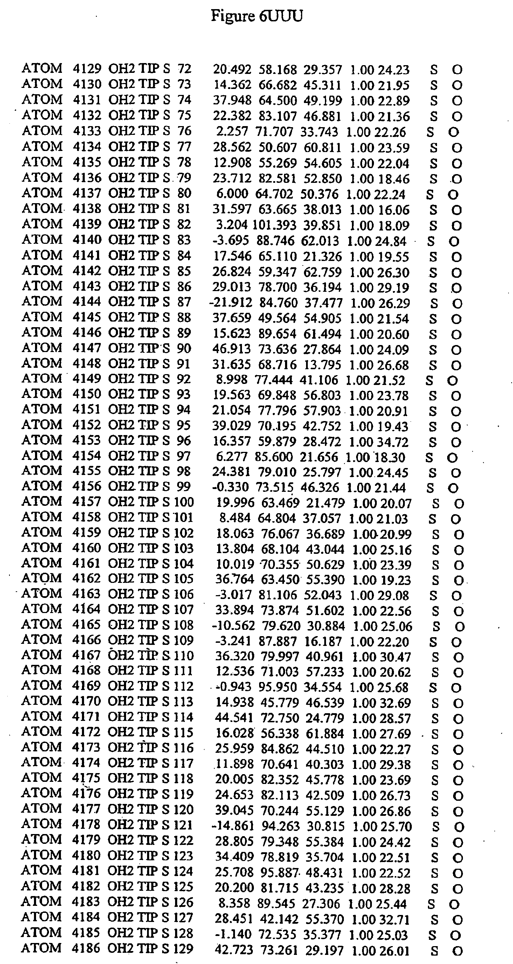

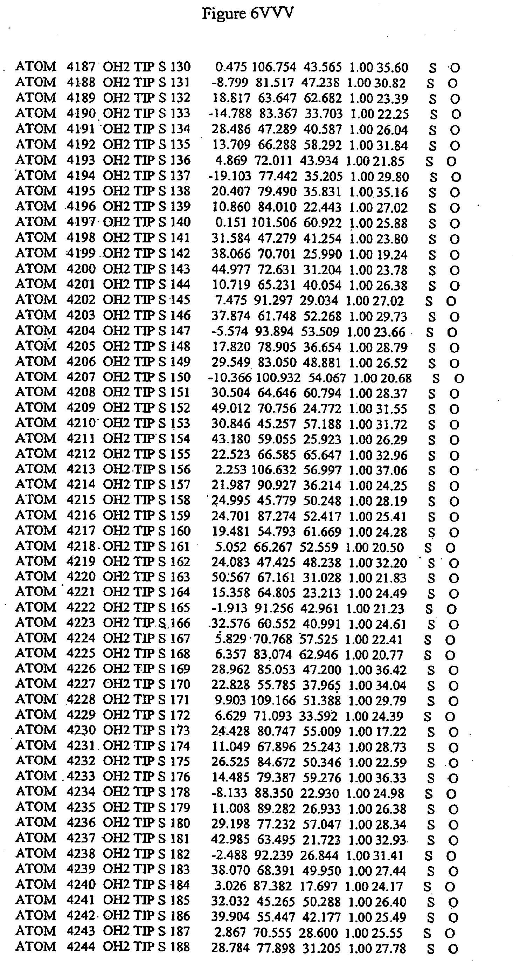

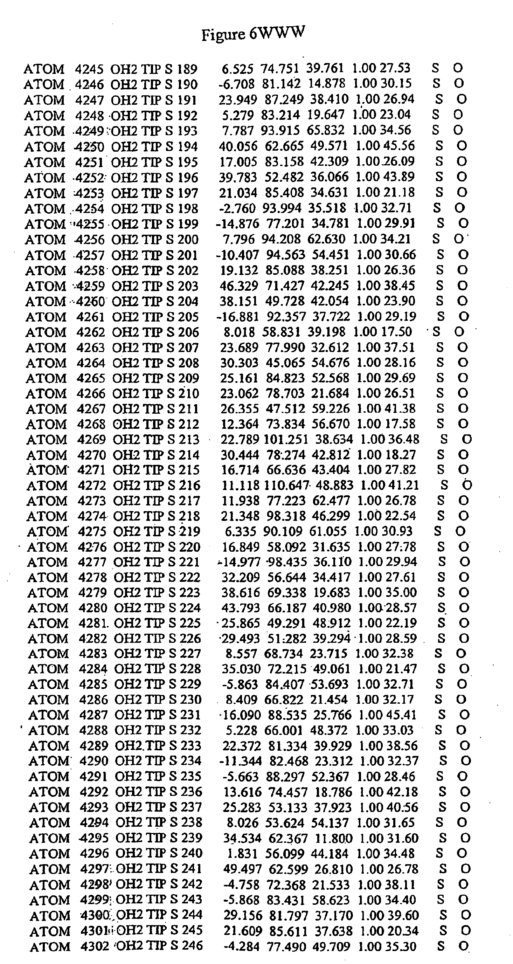

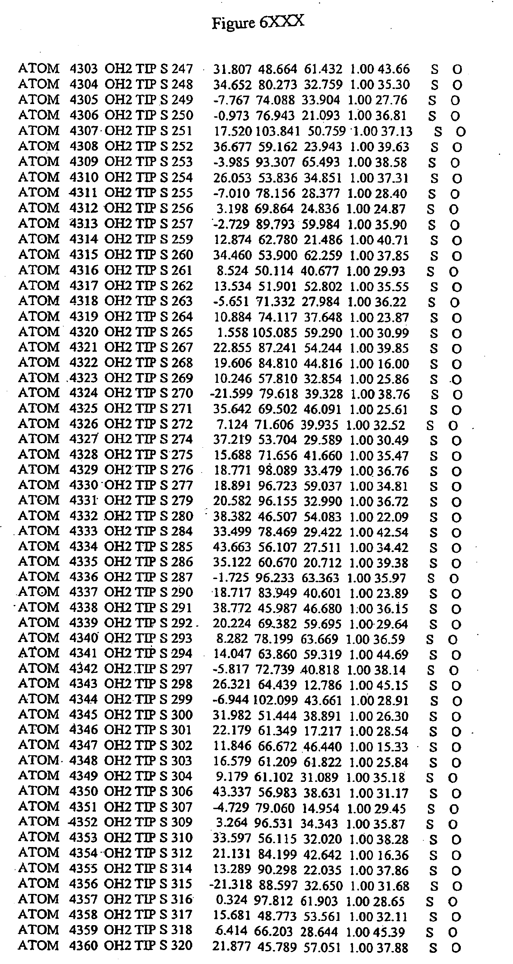

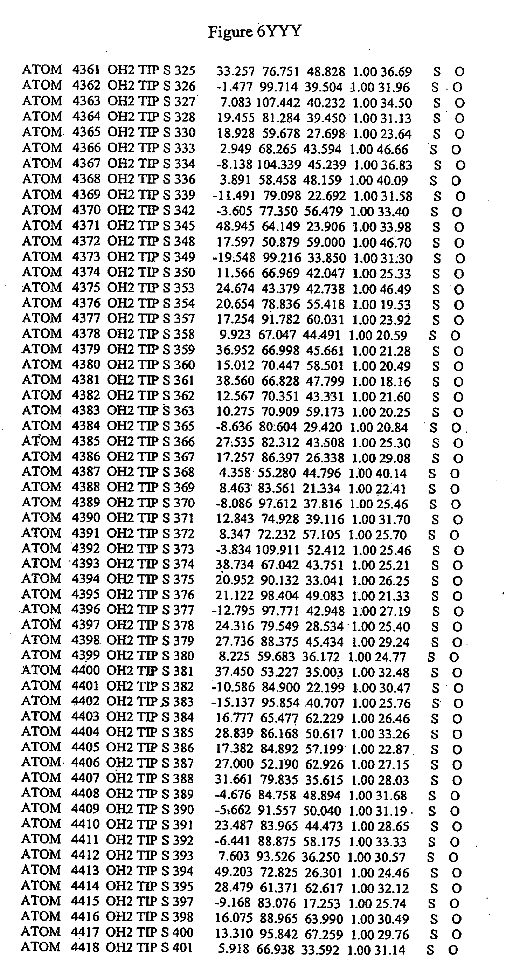

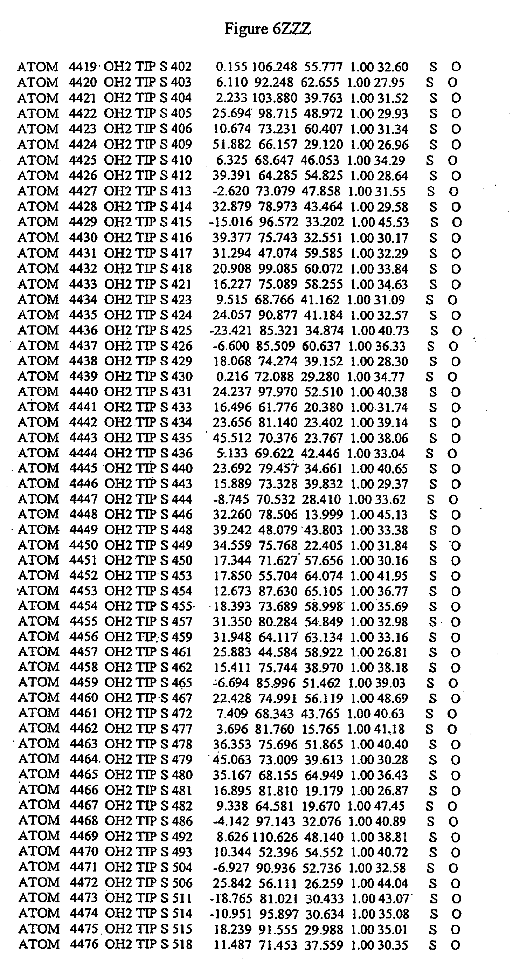

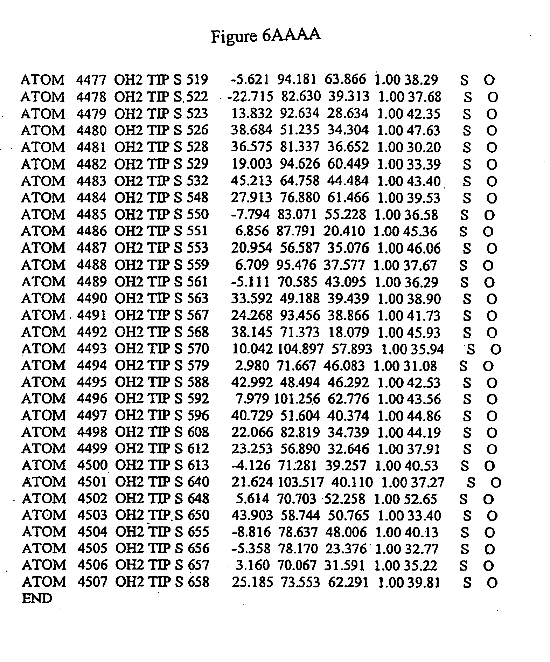

A further embodiment is crystallized *H. pylori* MurI. In a further embodiment of the present invention, the crystallized Murl is characterized by the structural coordinates depicted in Figure 6, wherein the determined structures presented represent that of MurI in complex with glutamate and the pyrimidinedione inhibitor, compound A.

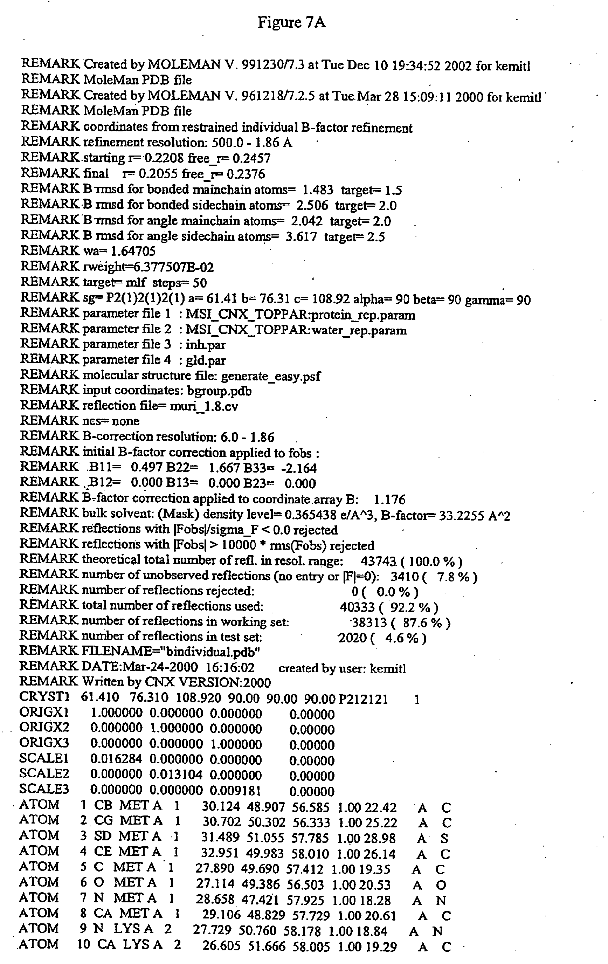

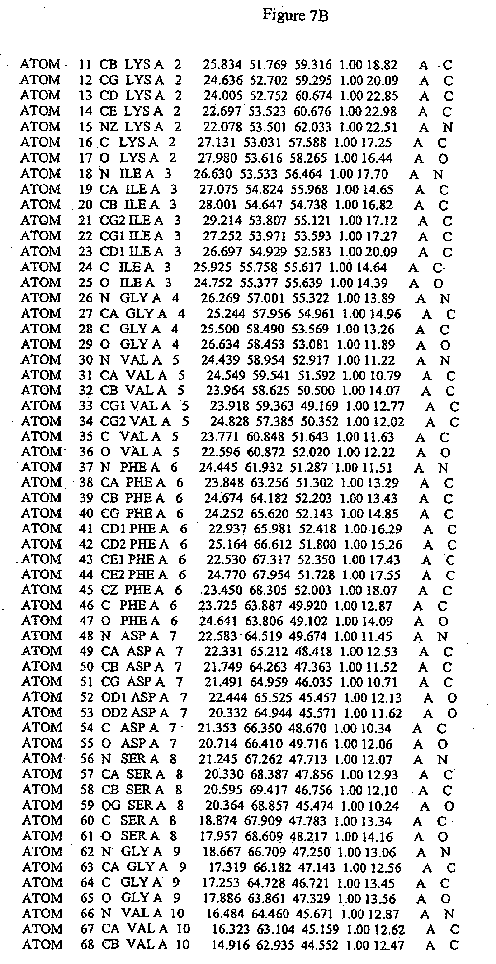

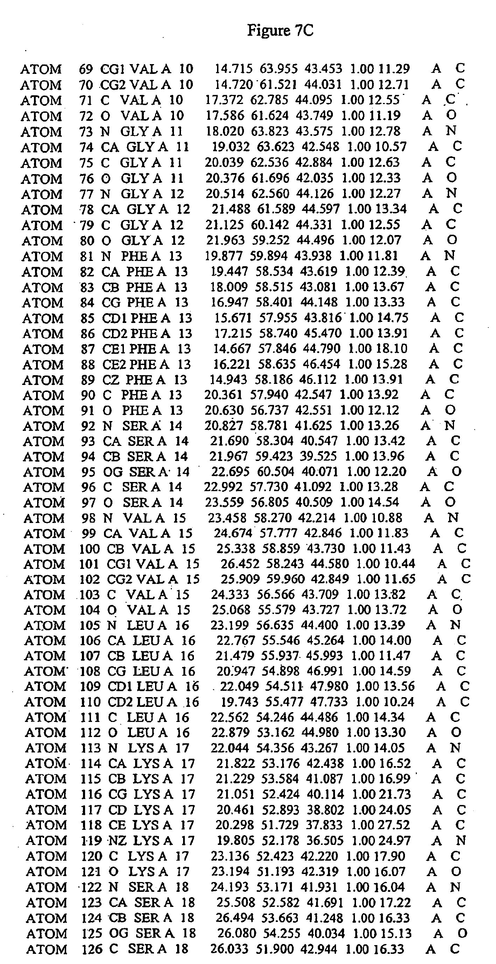

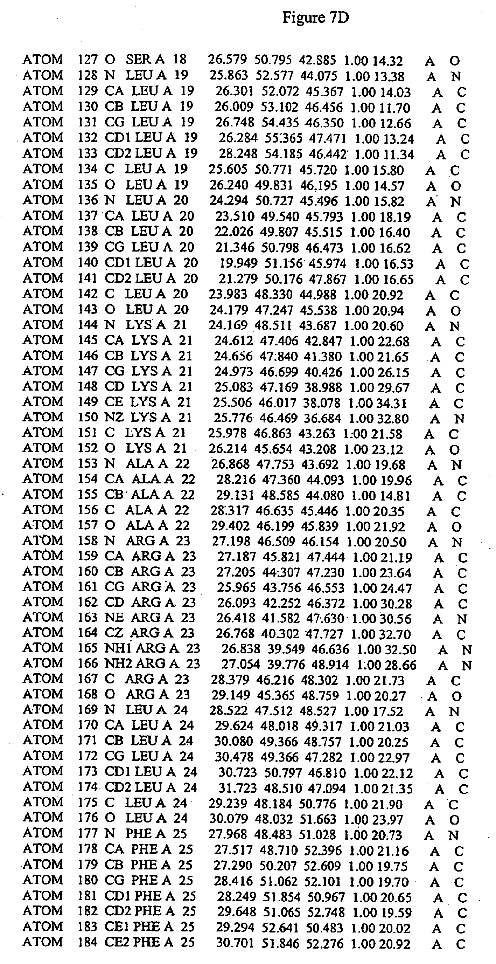

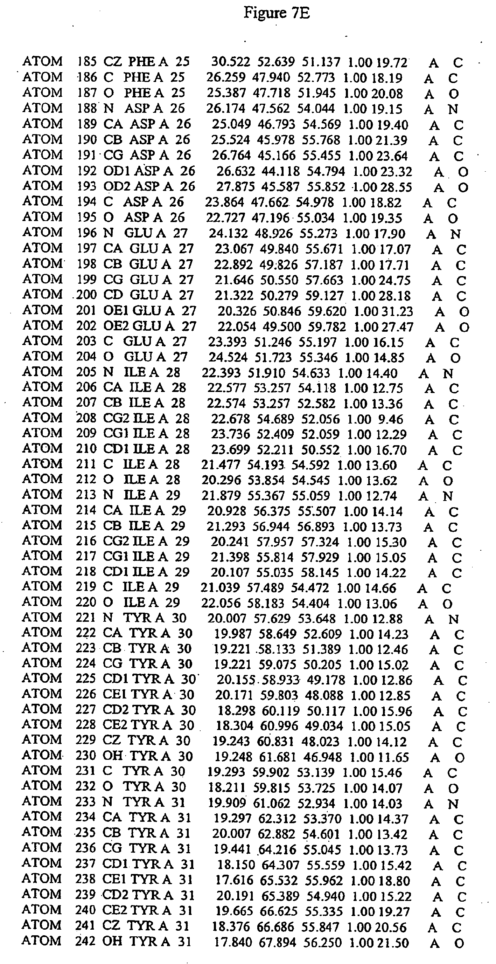

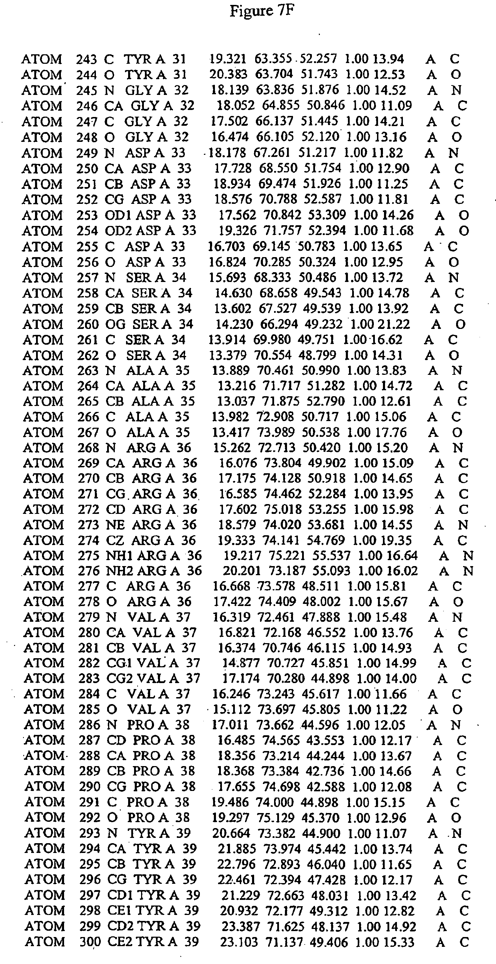

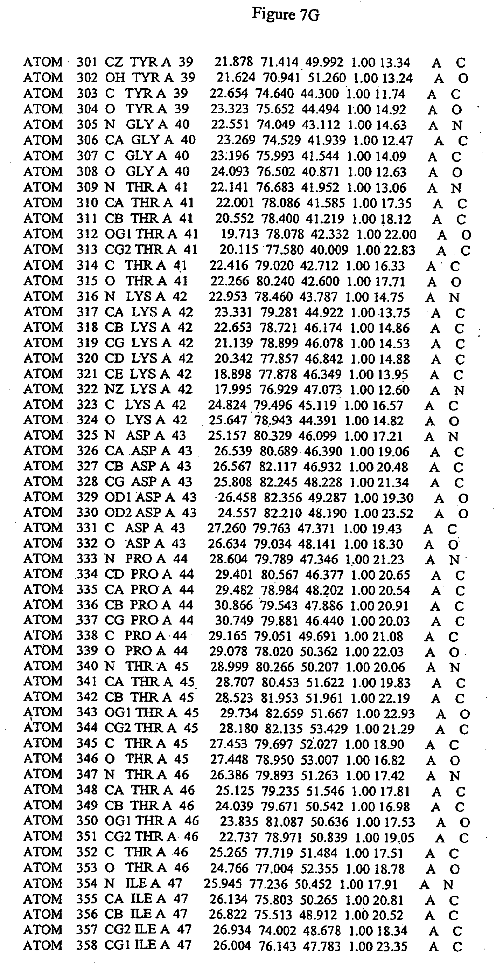

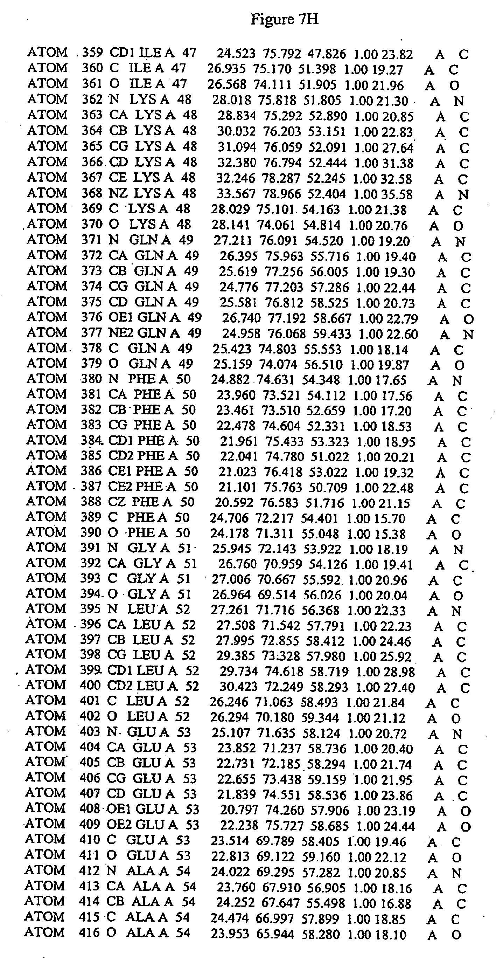

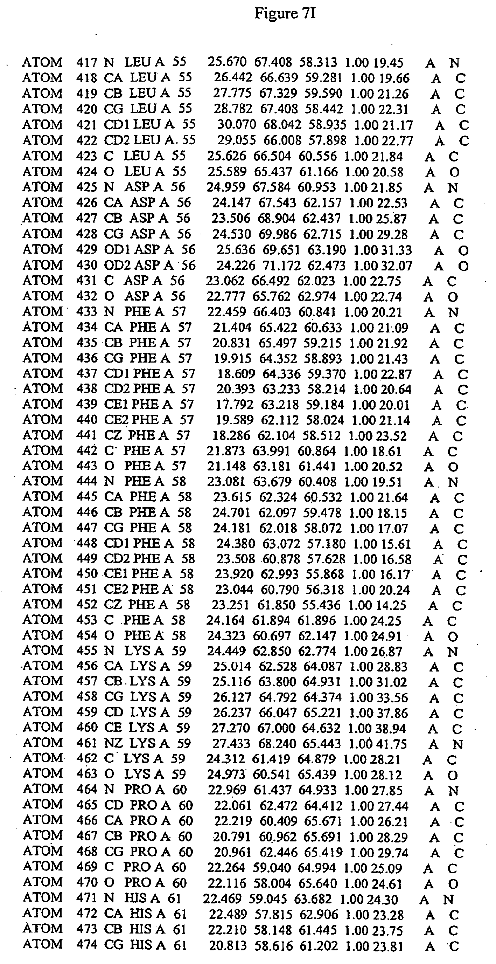

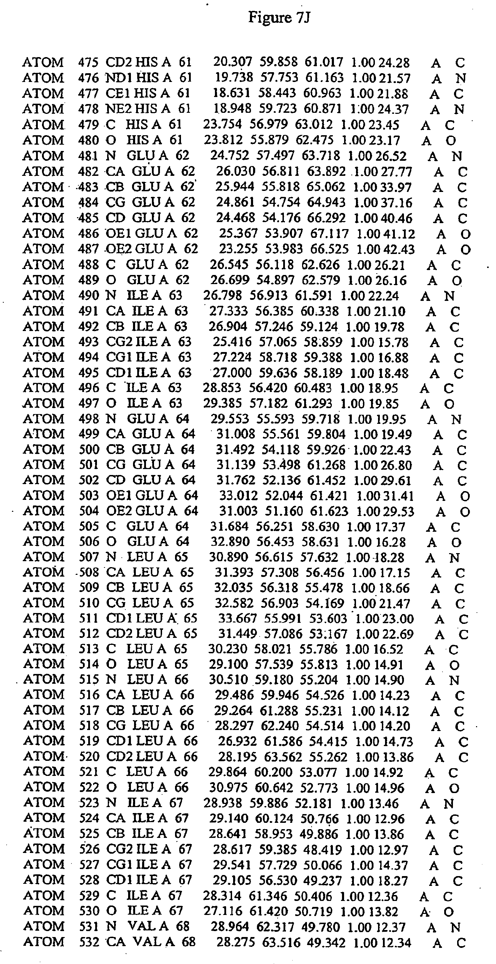

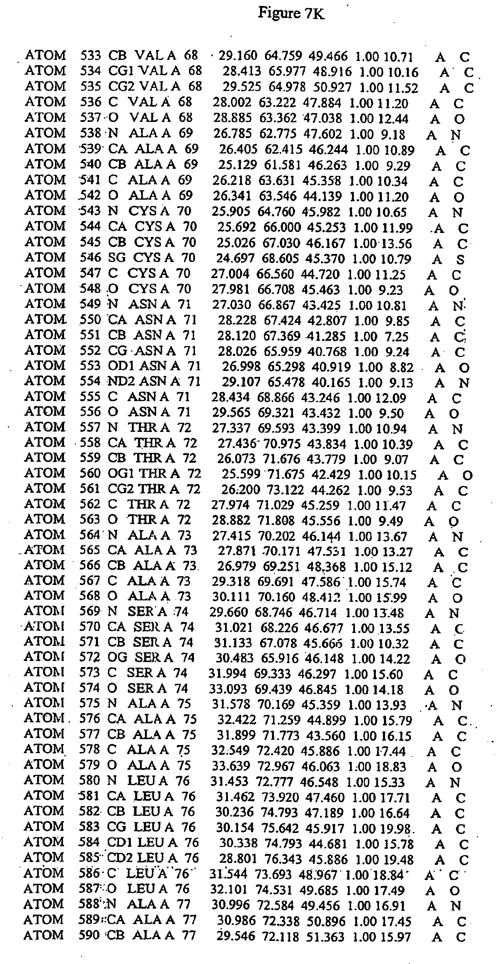

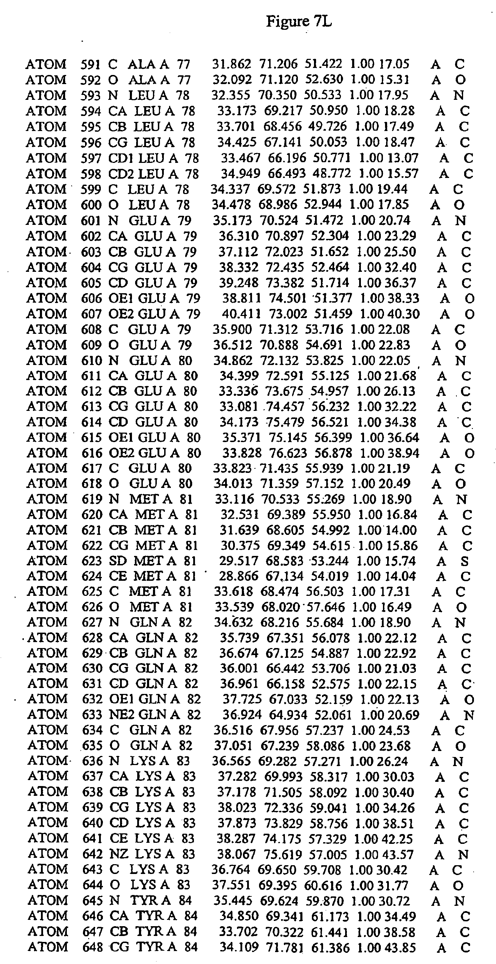

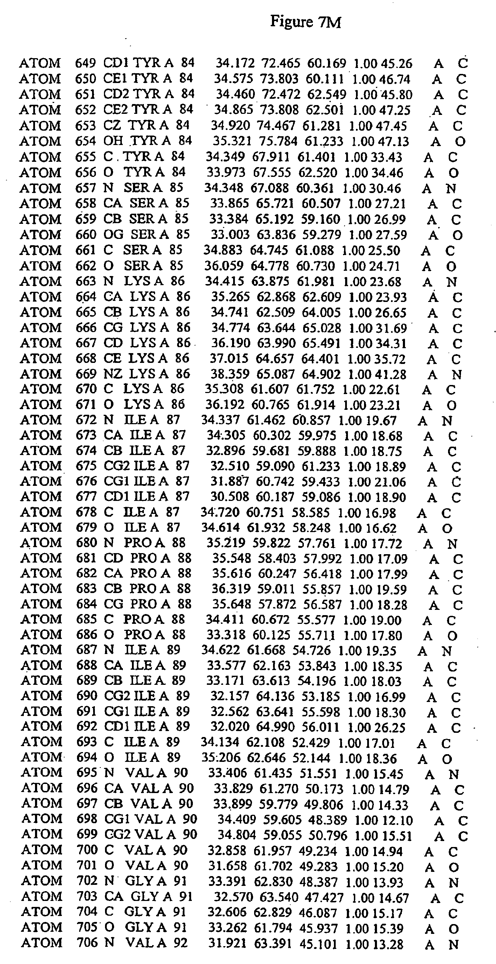

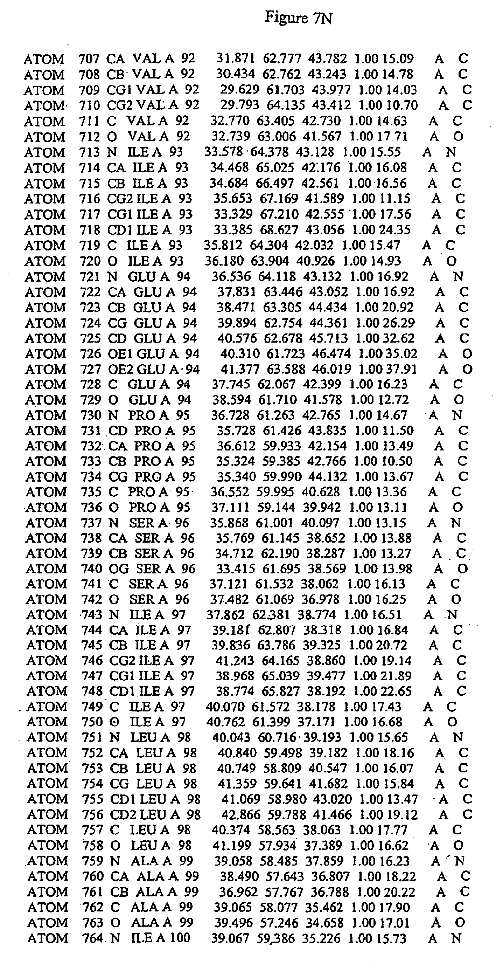

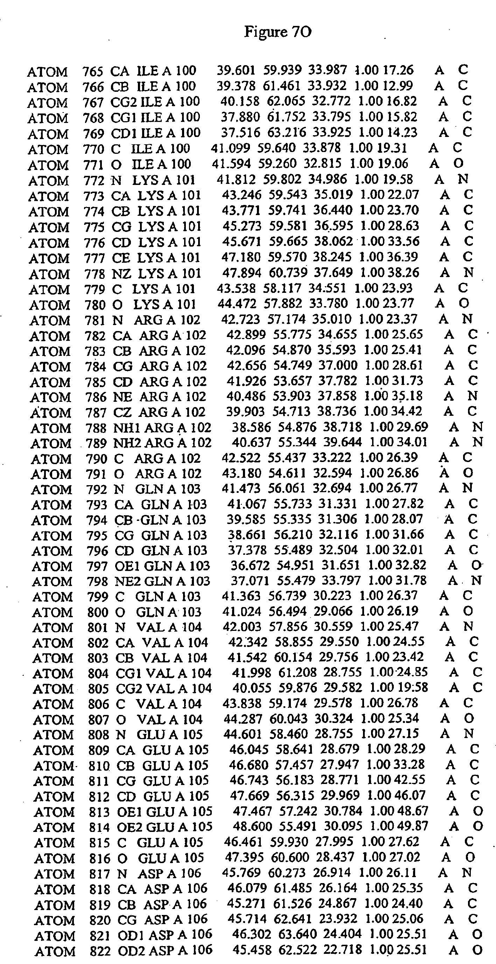

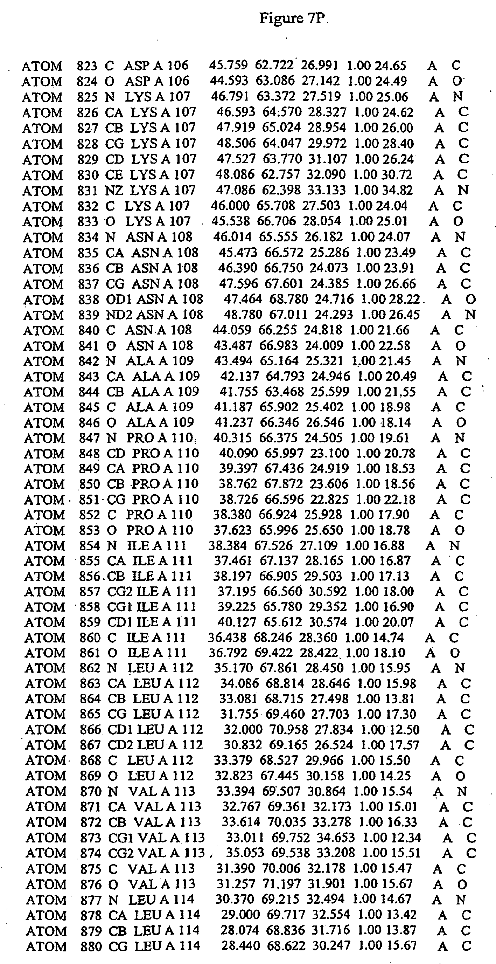

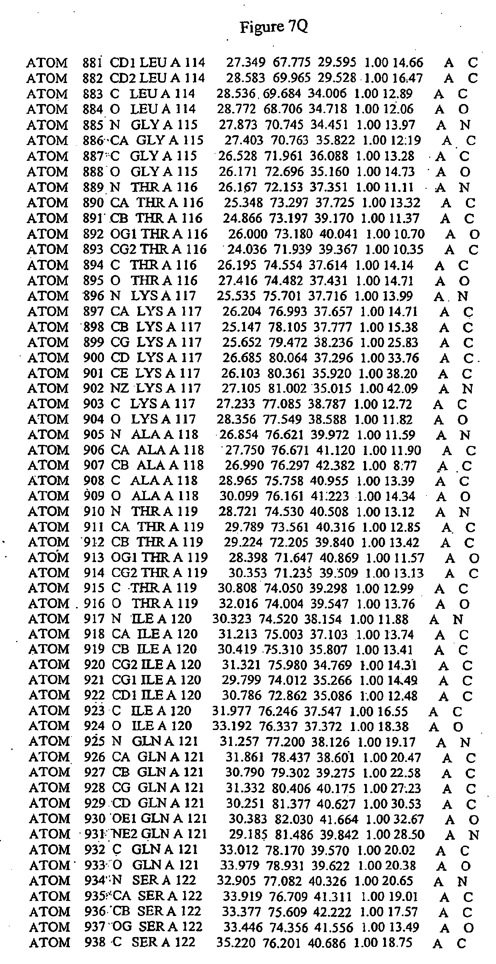

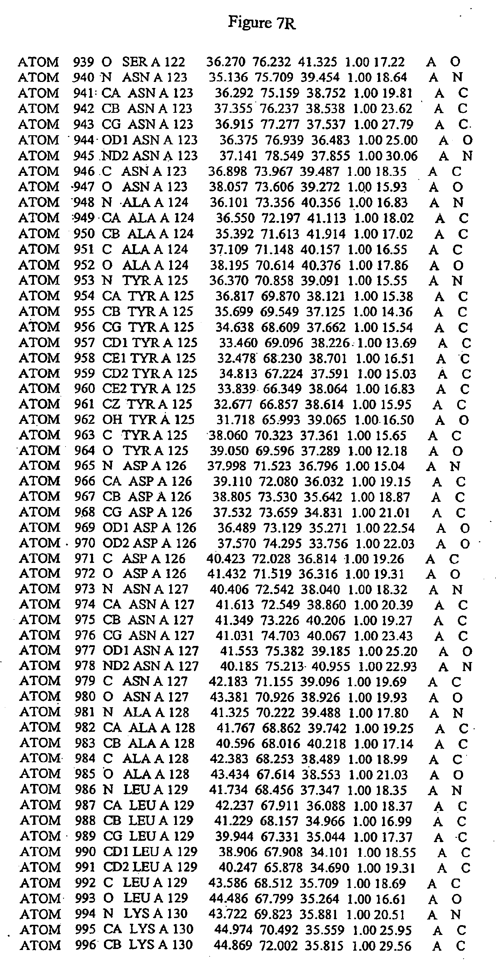

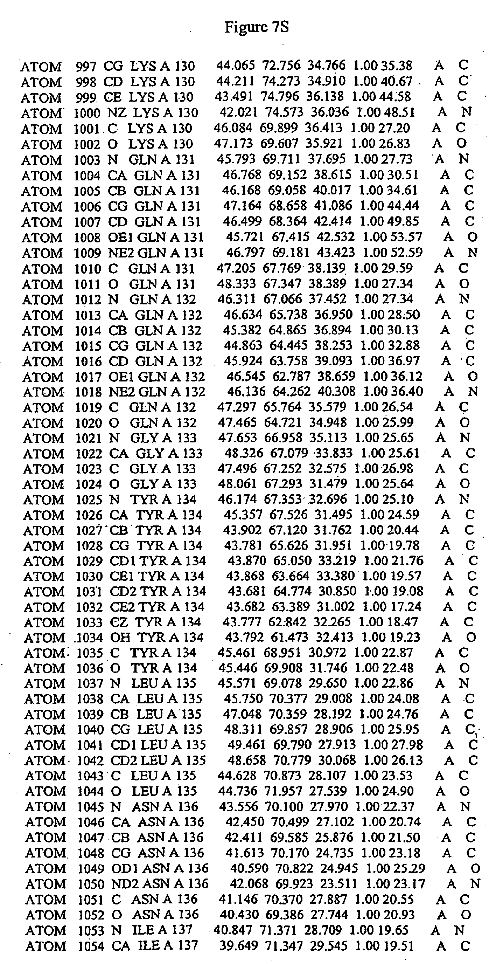

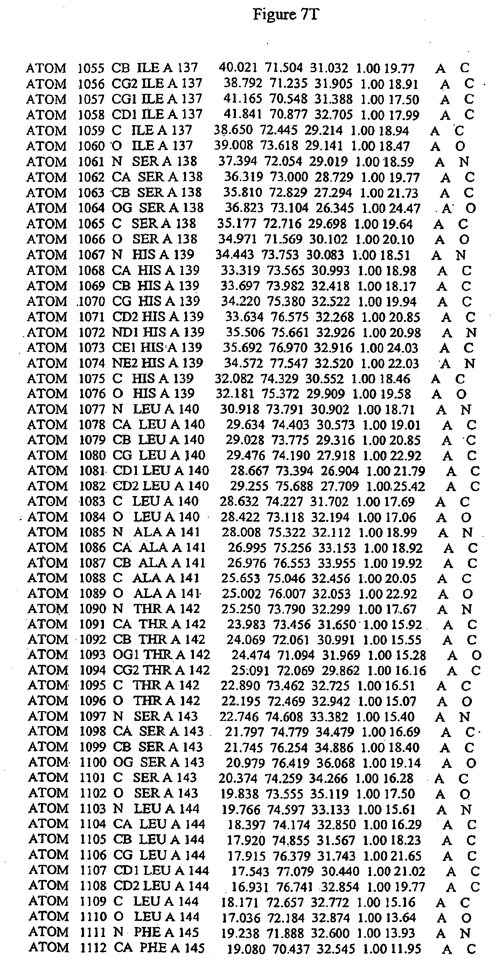

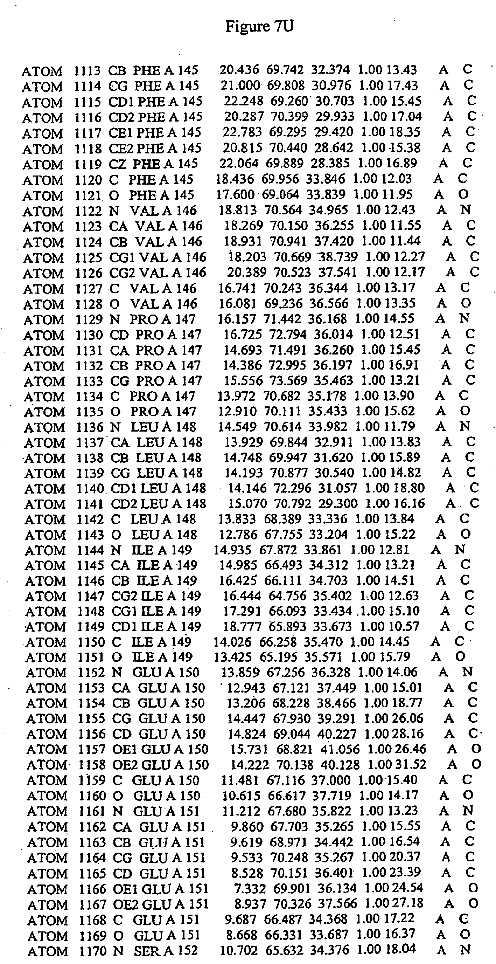

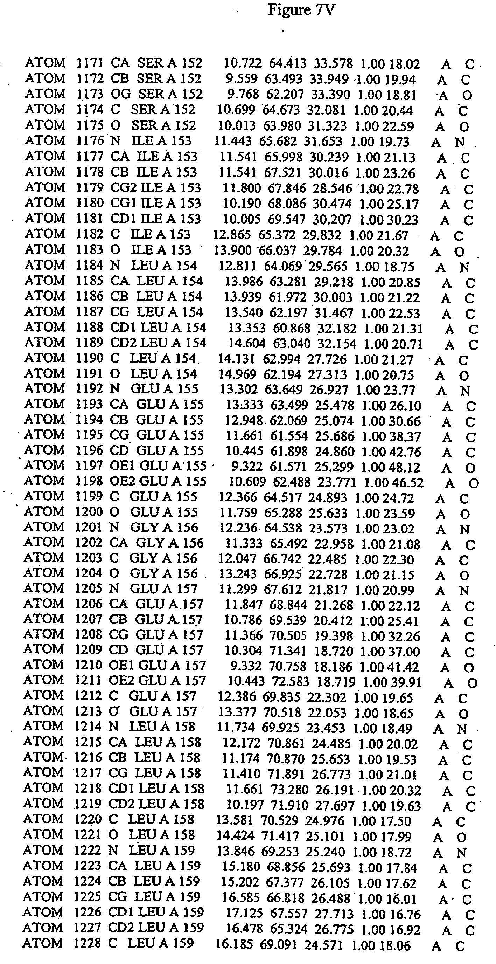

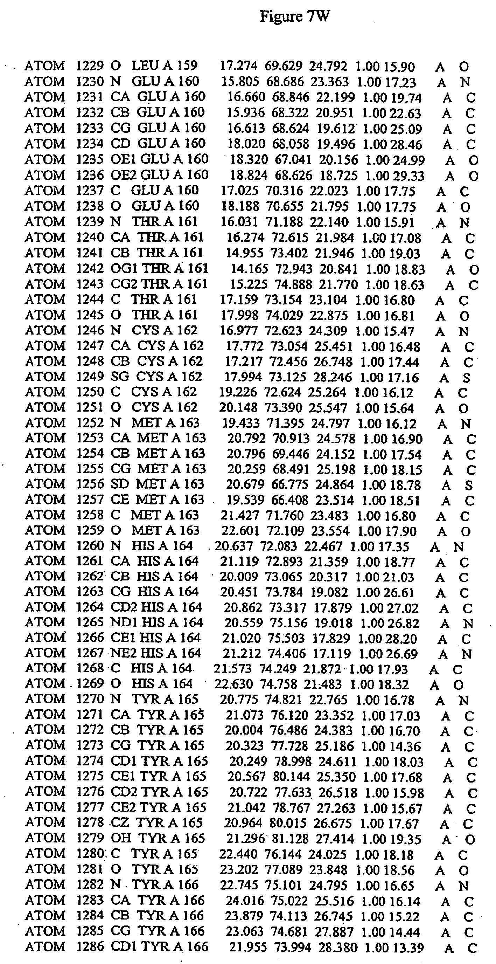

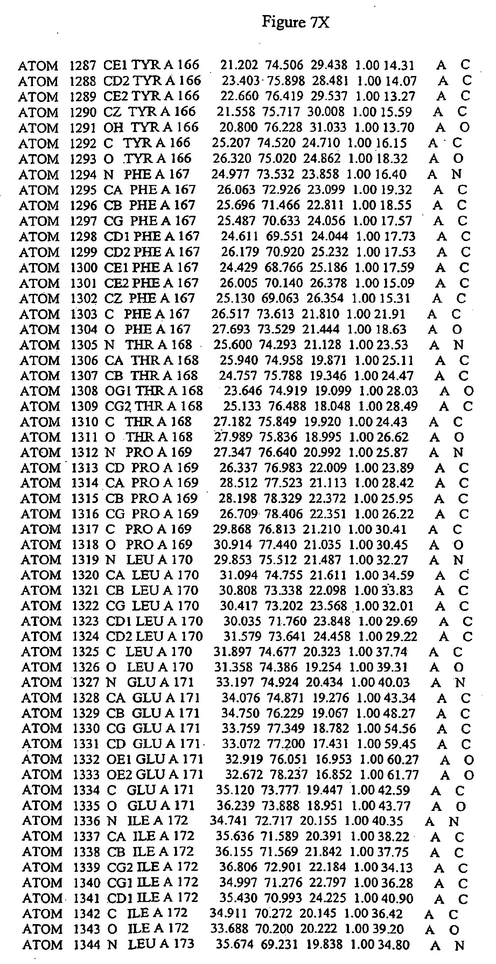

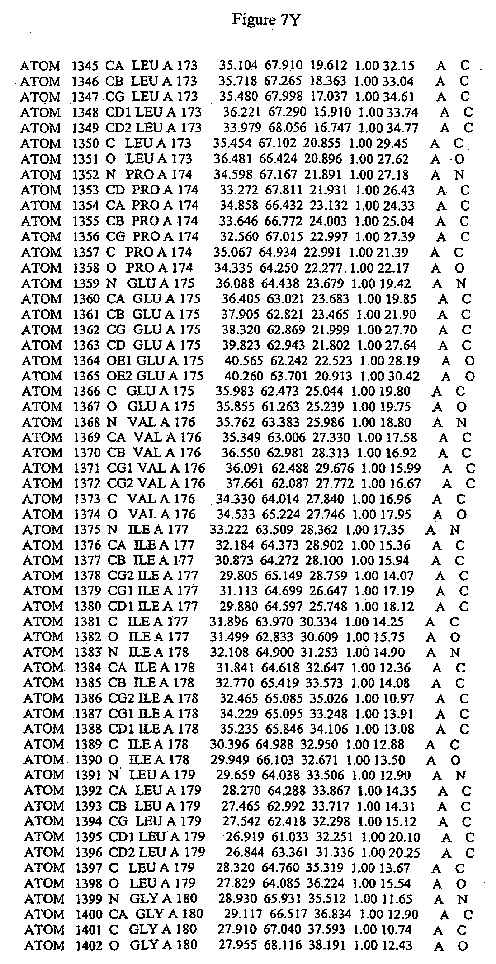

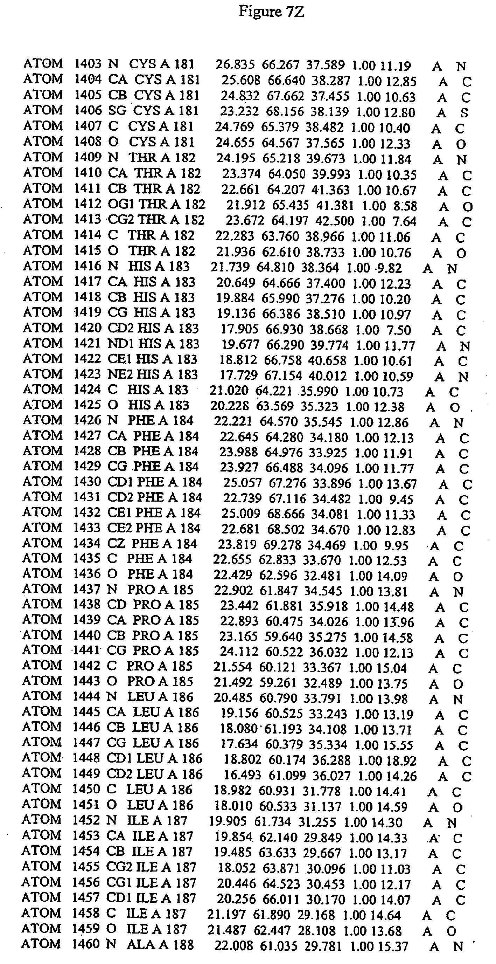

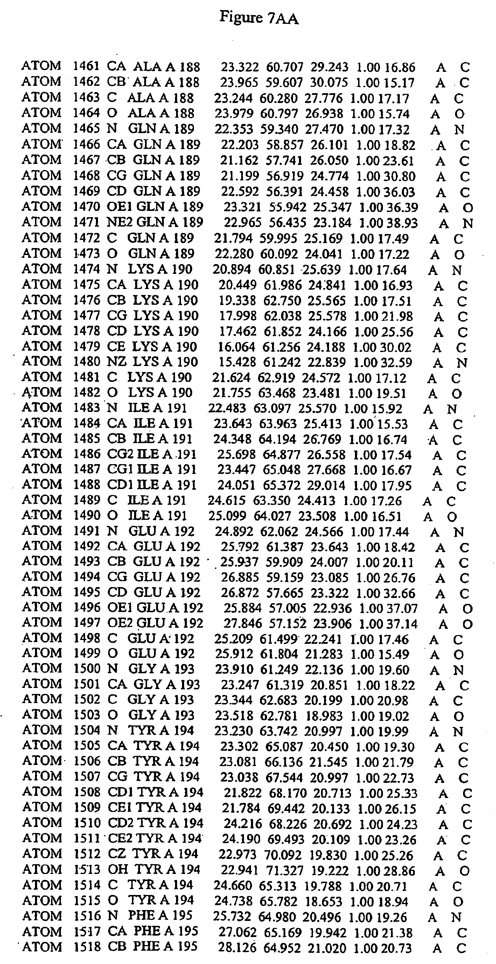

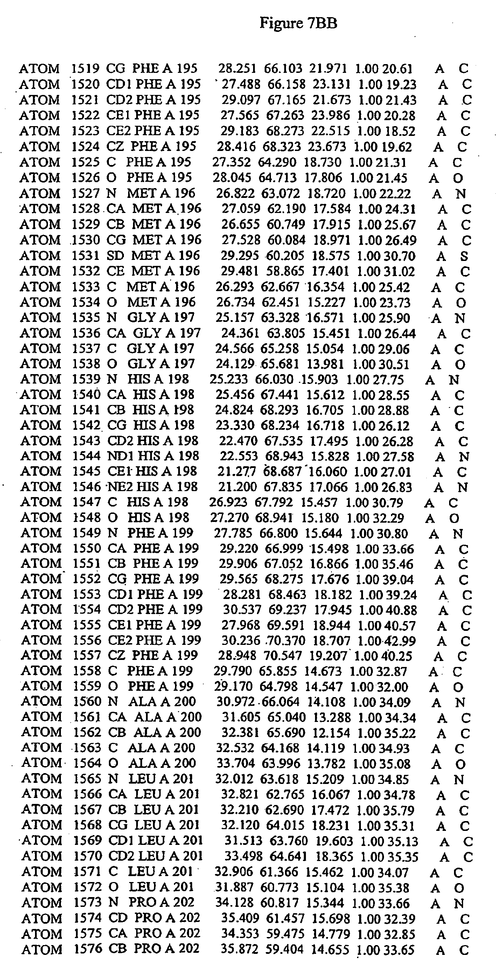

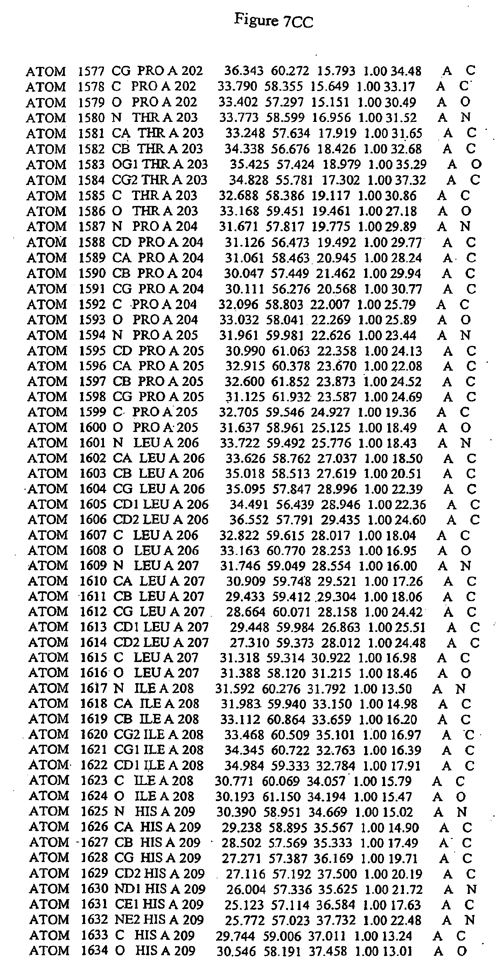

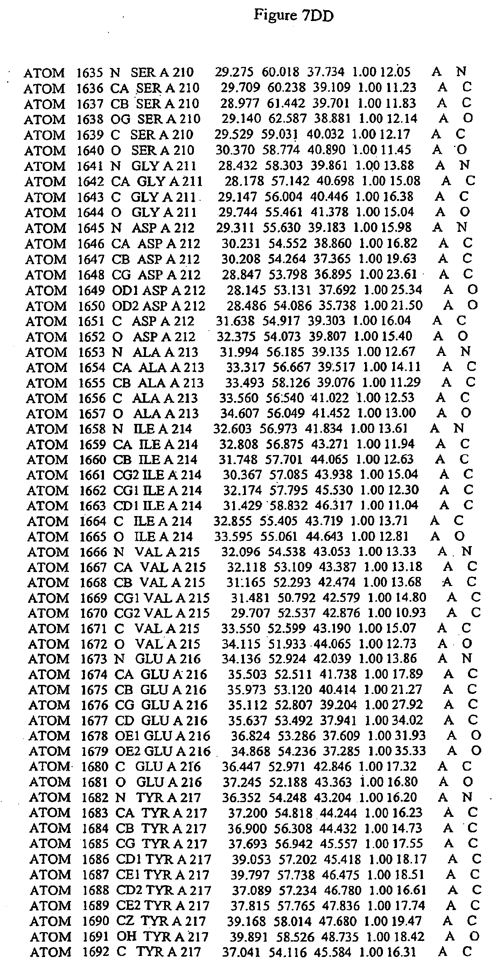

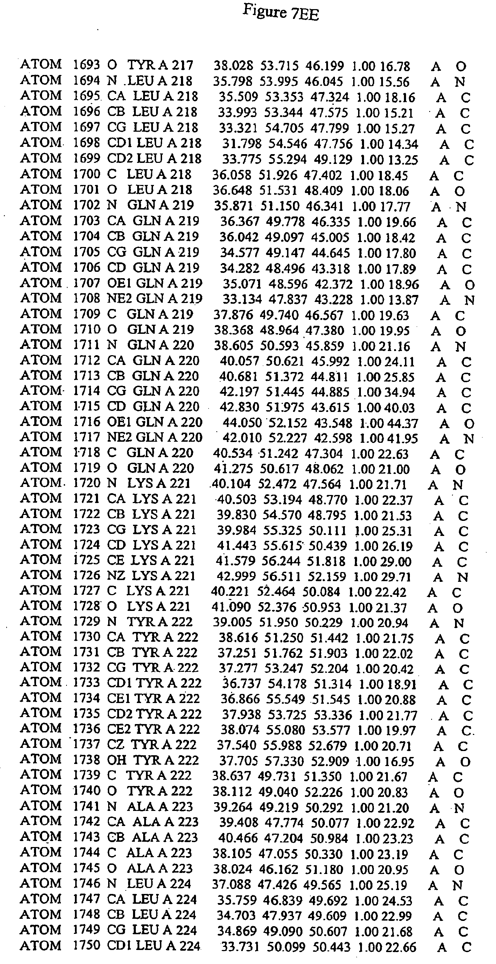

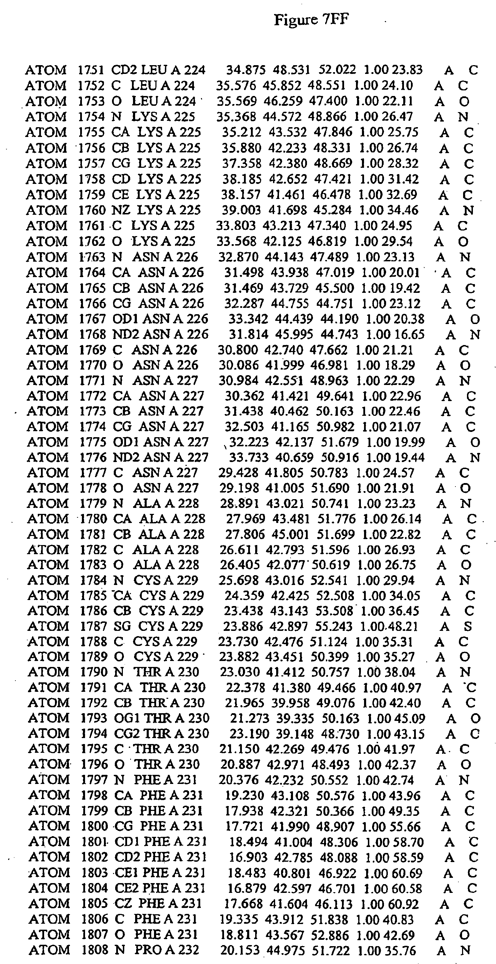

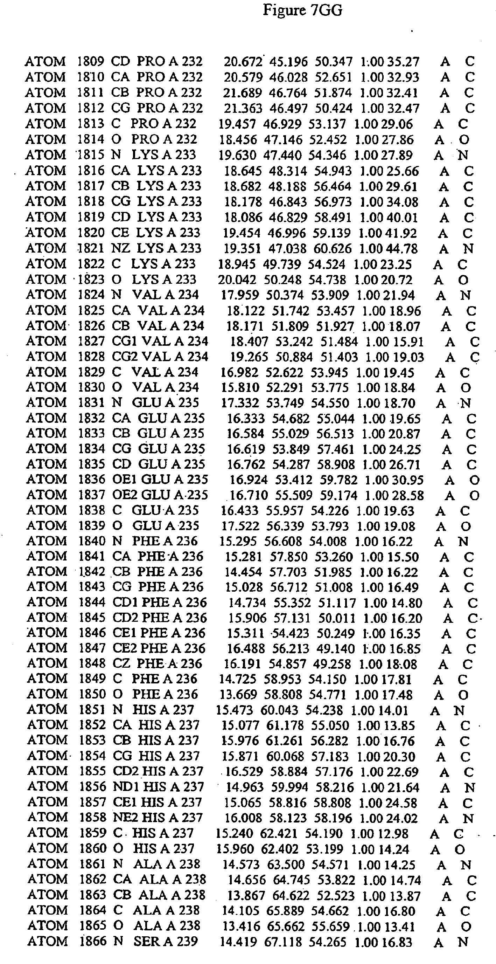

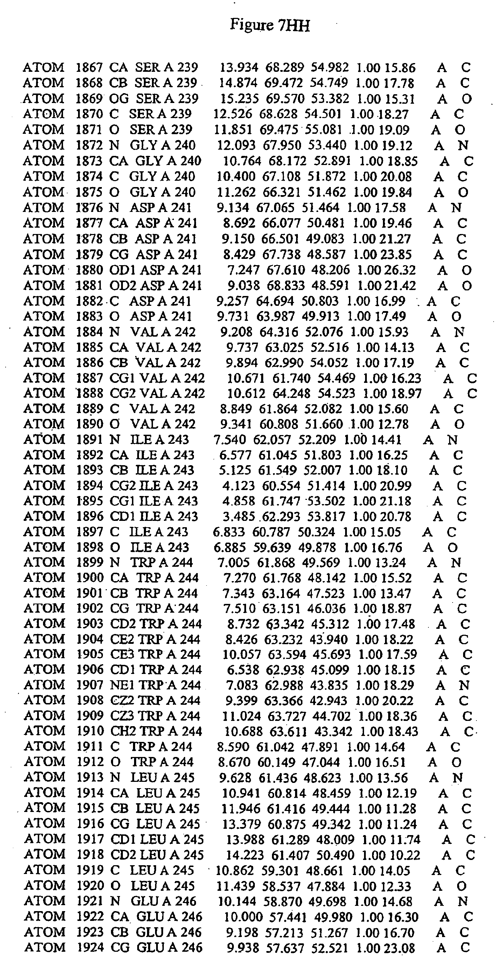

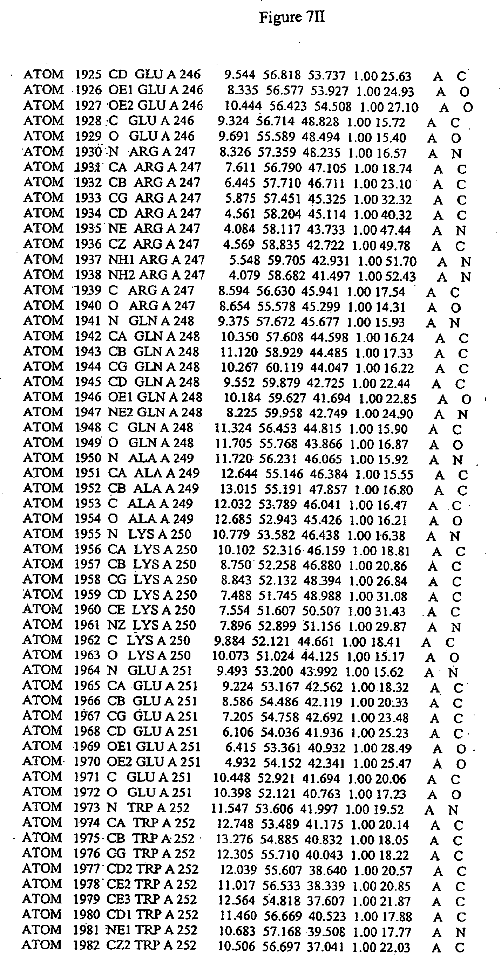

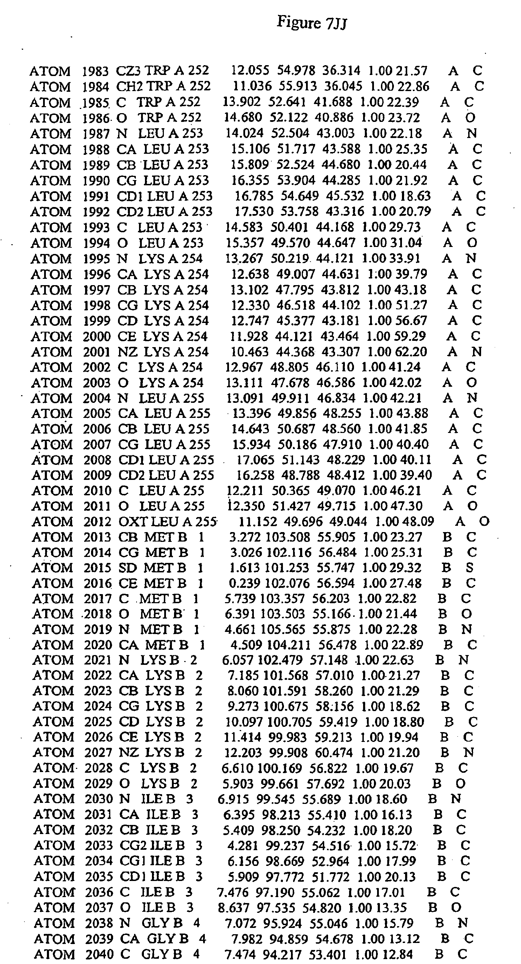

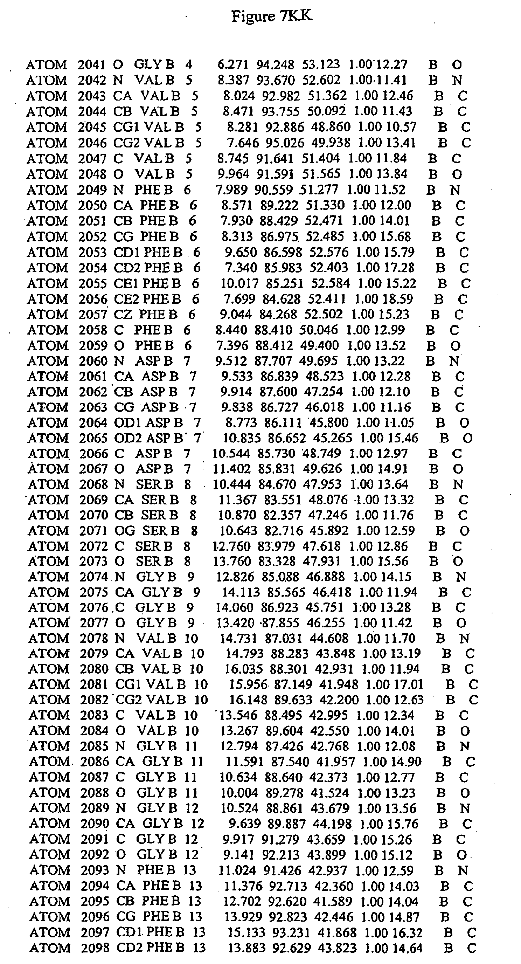

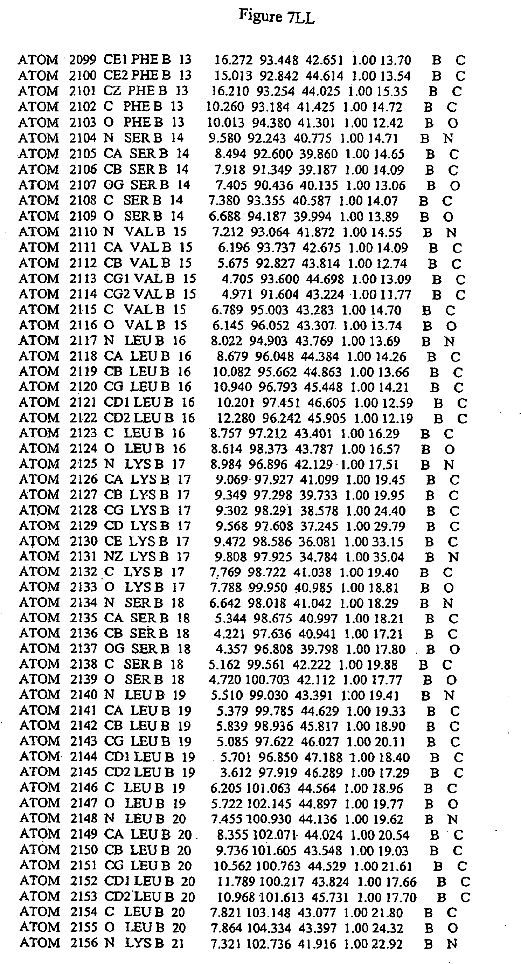

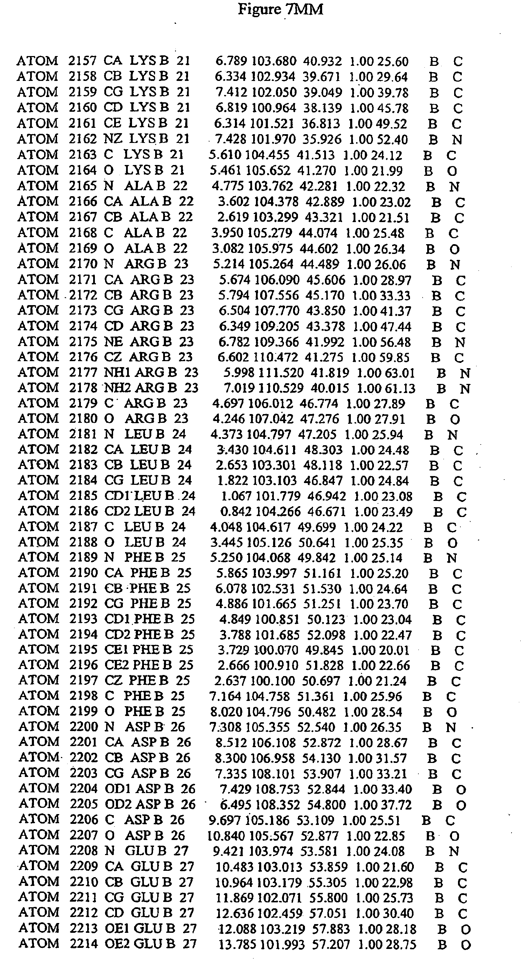

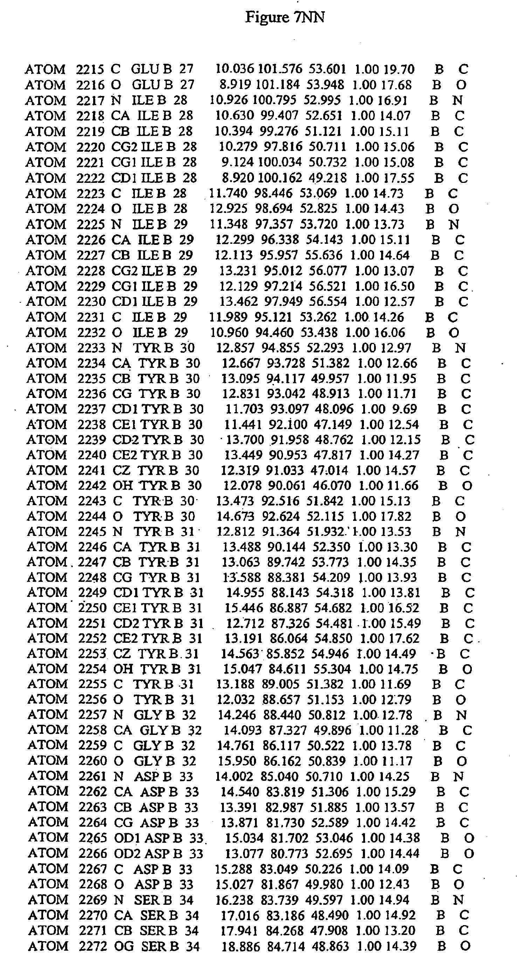

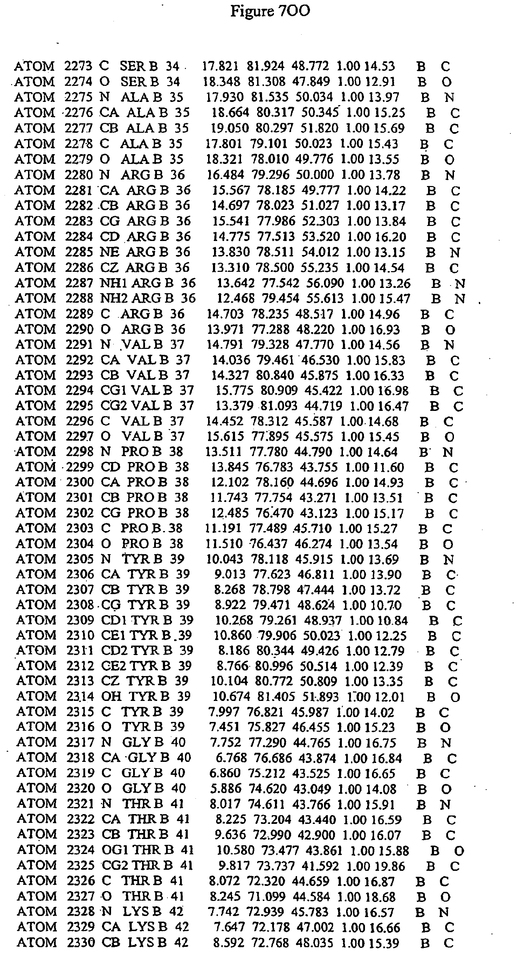

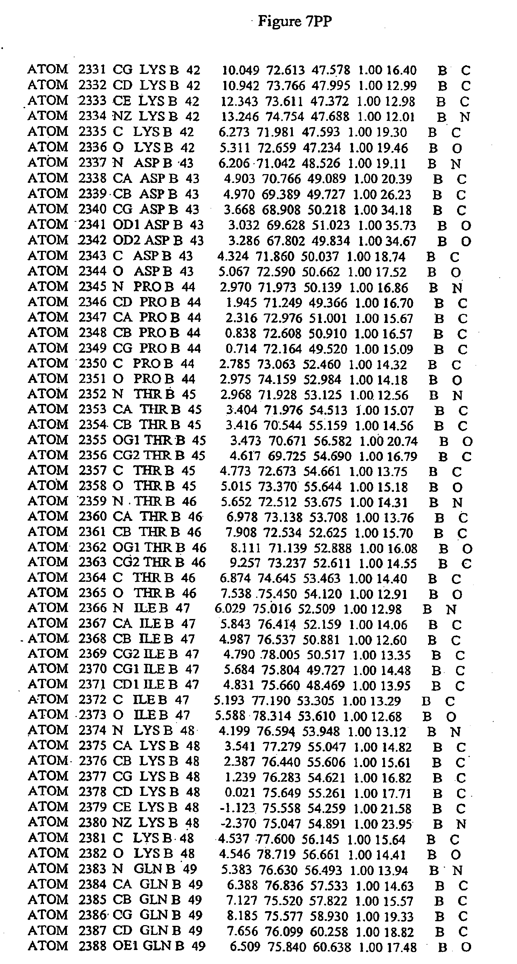

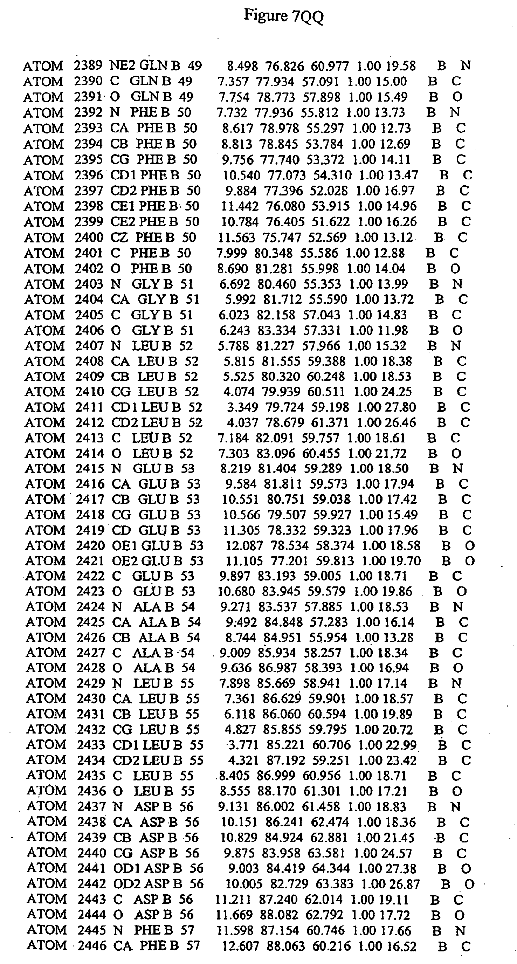

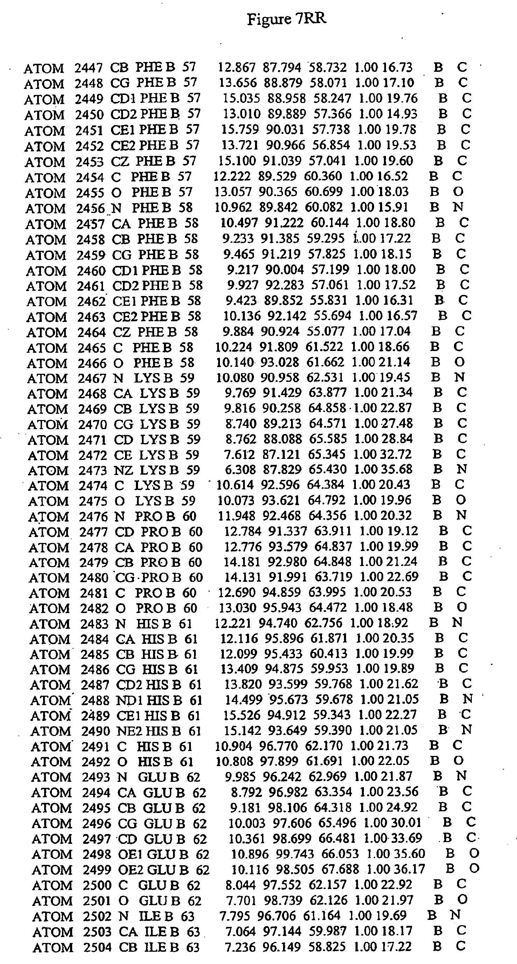

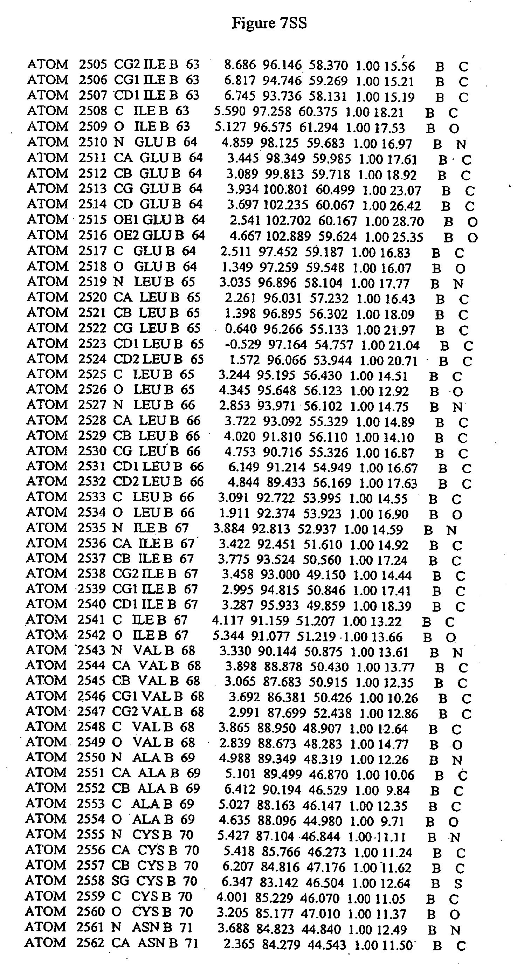

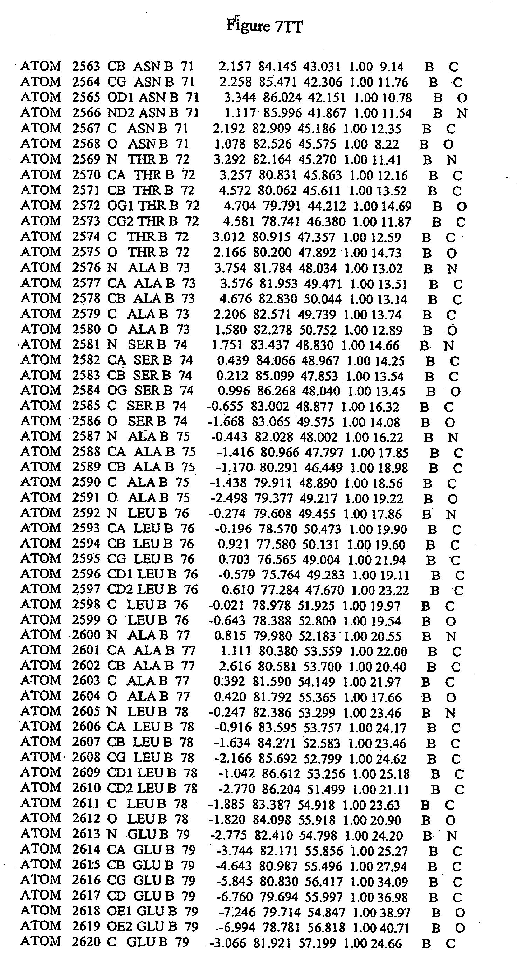

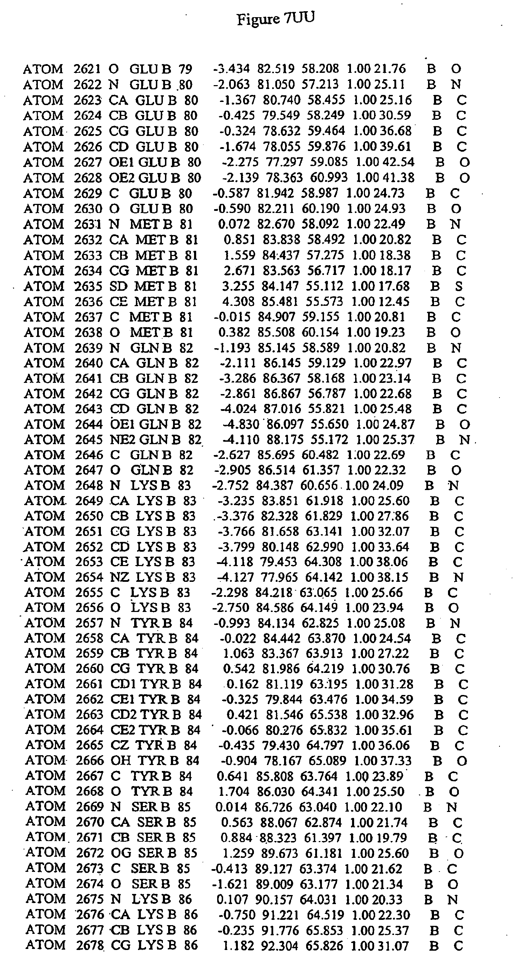

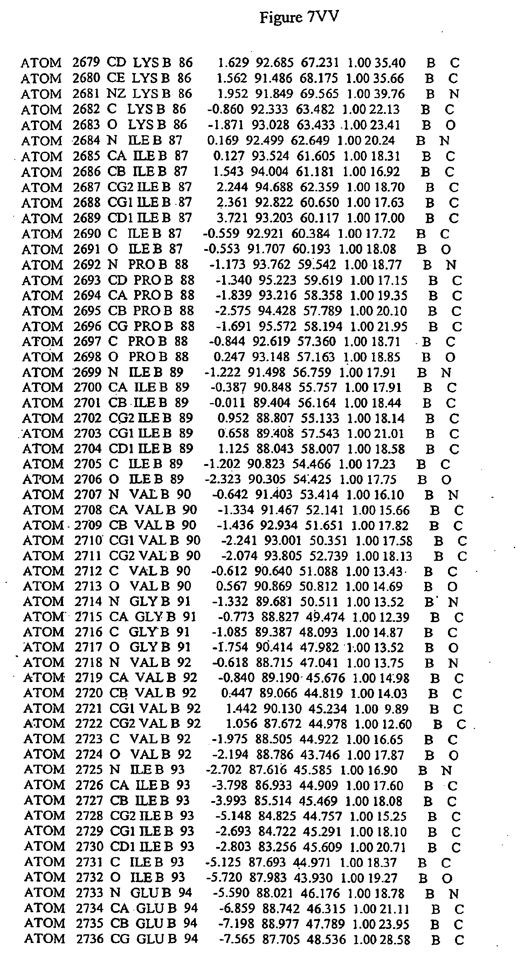

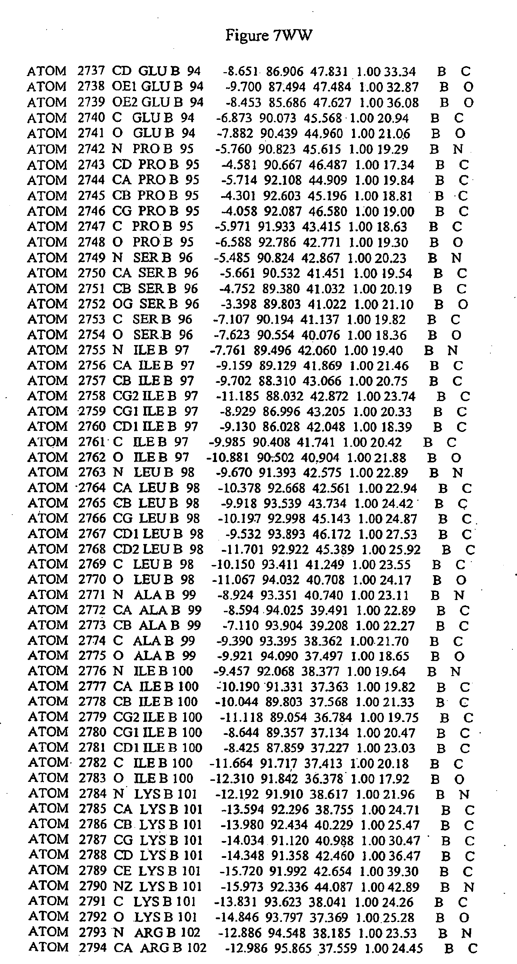

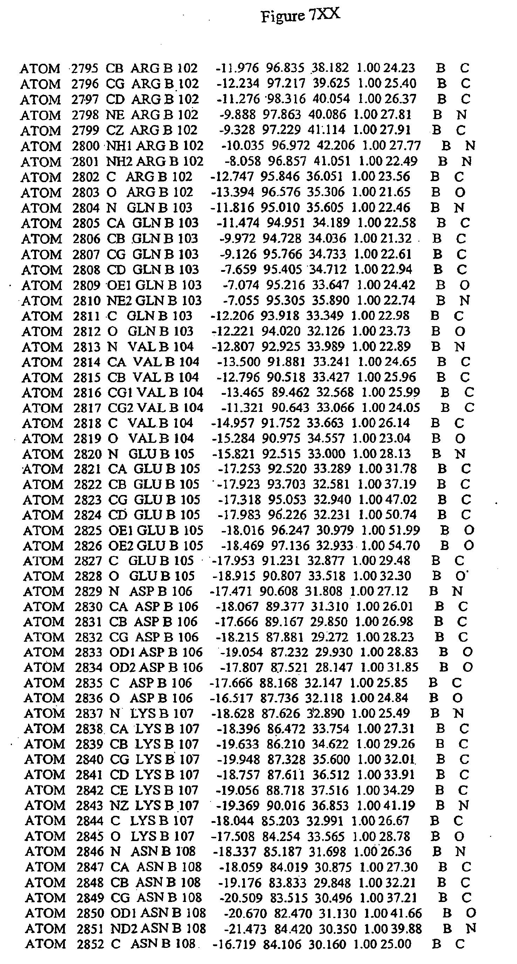

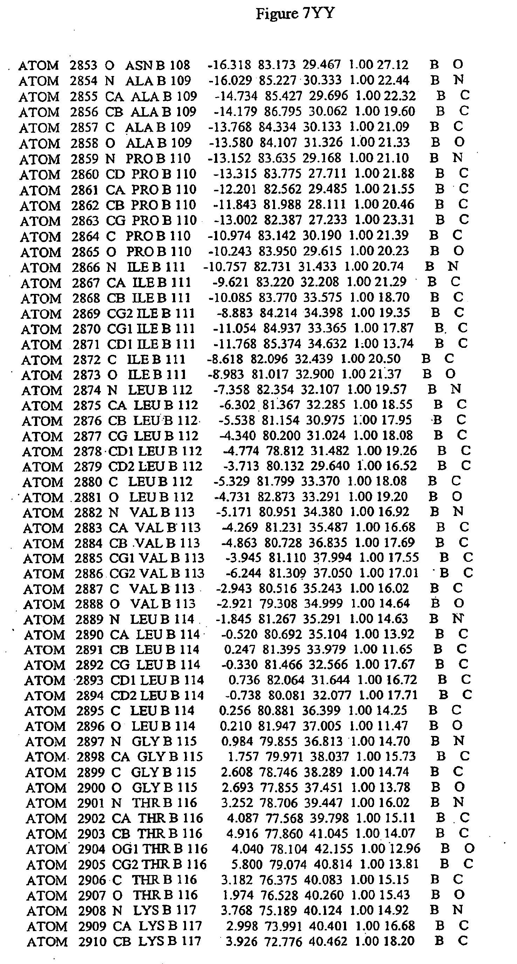

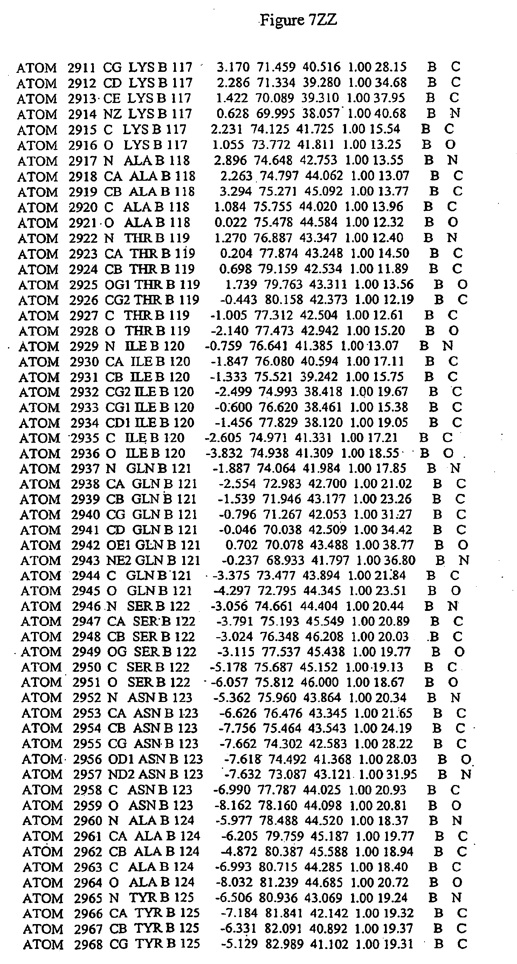

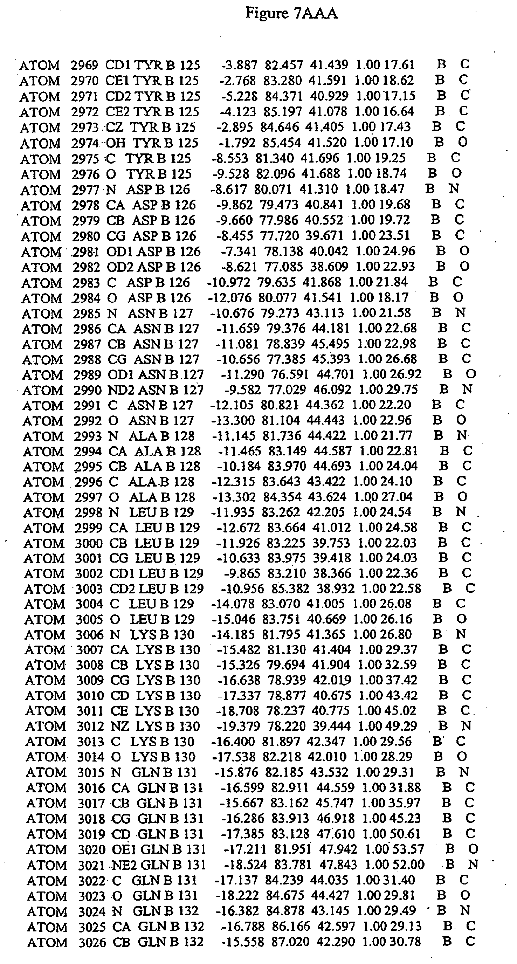

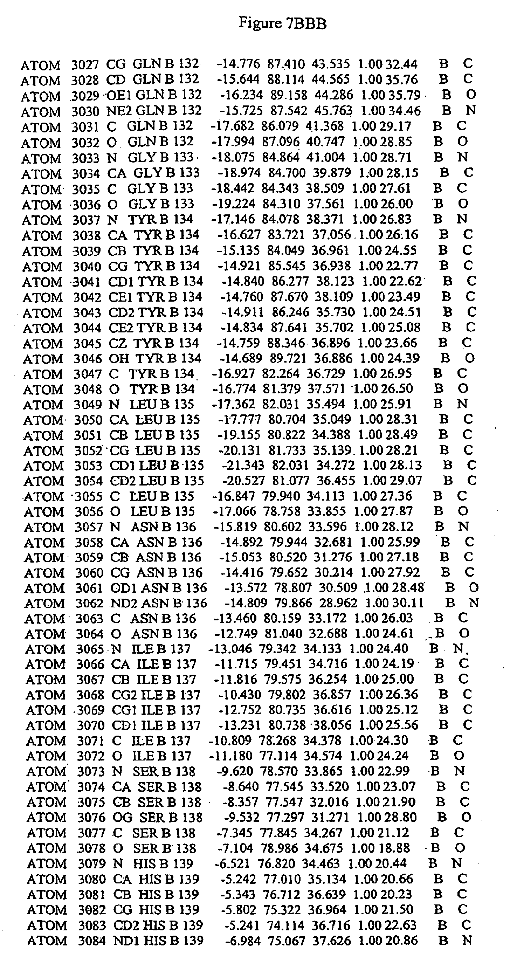

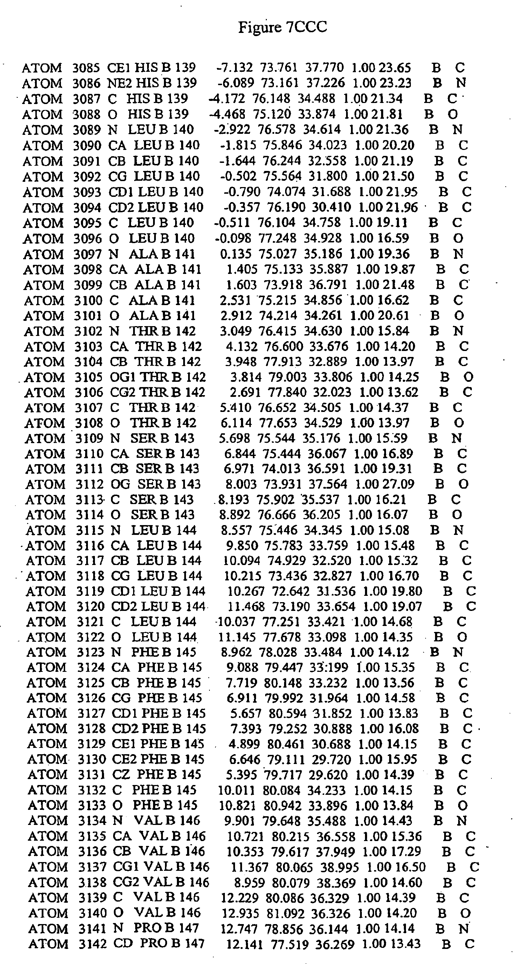

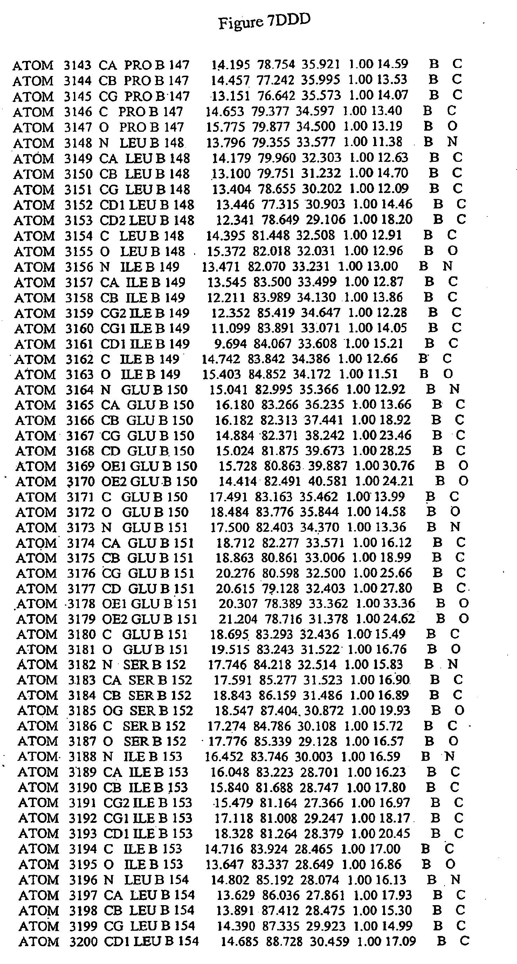

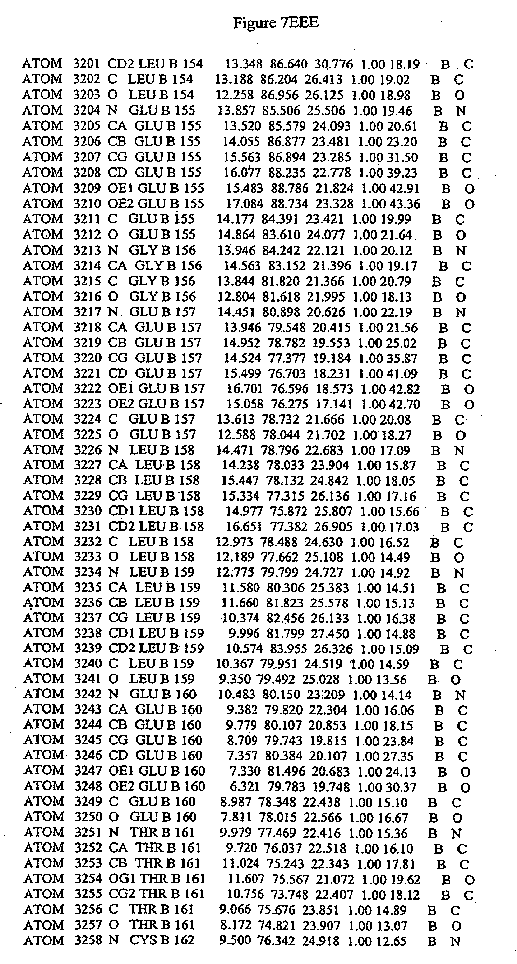

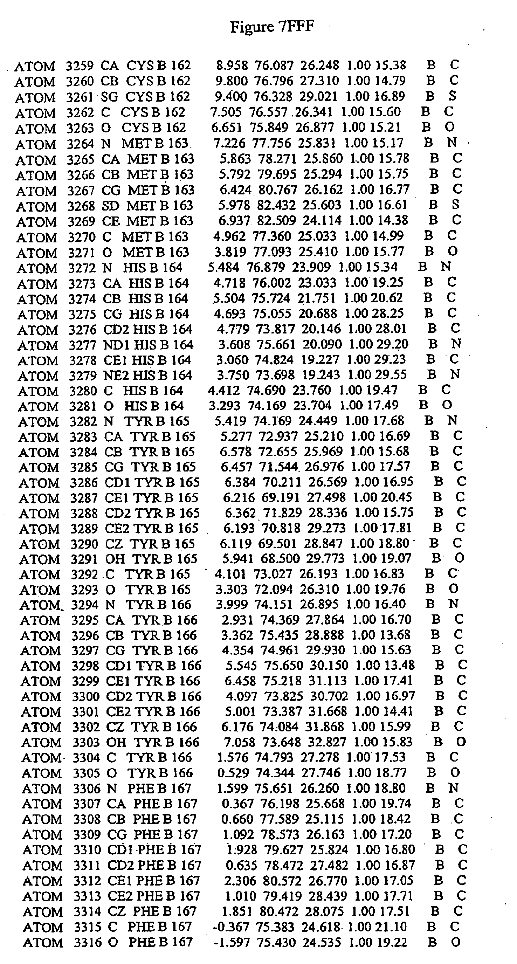

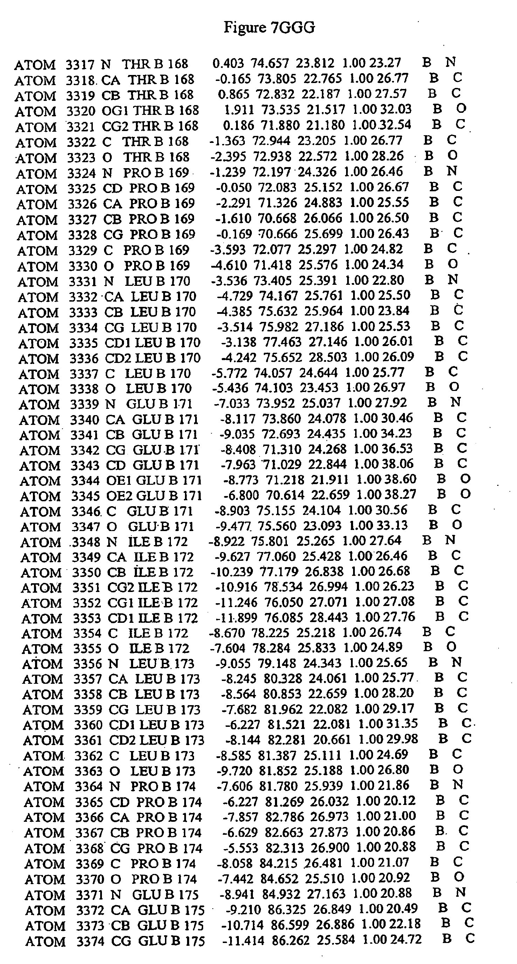

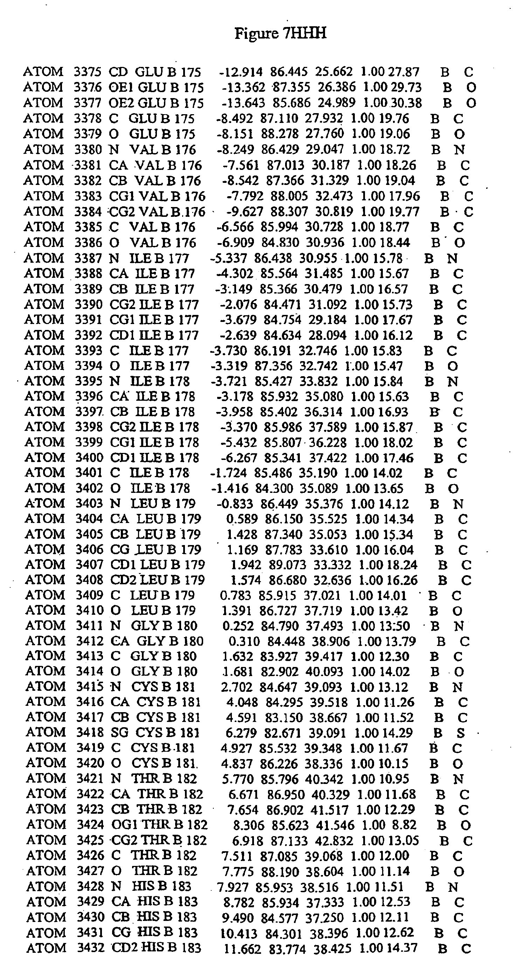

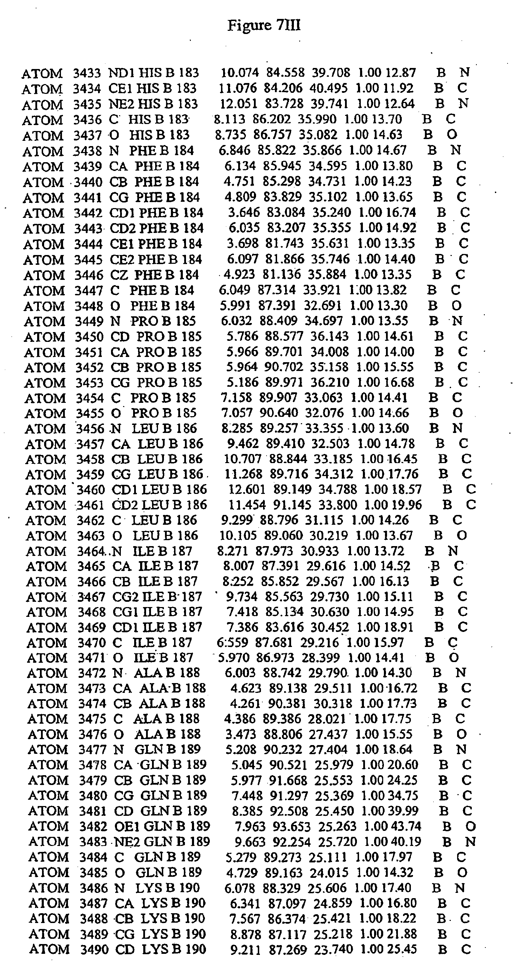

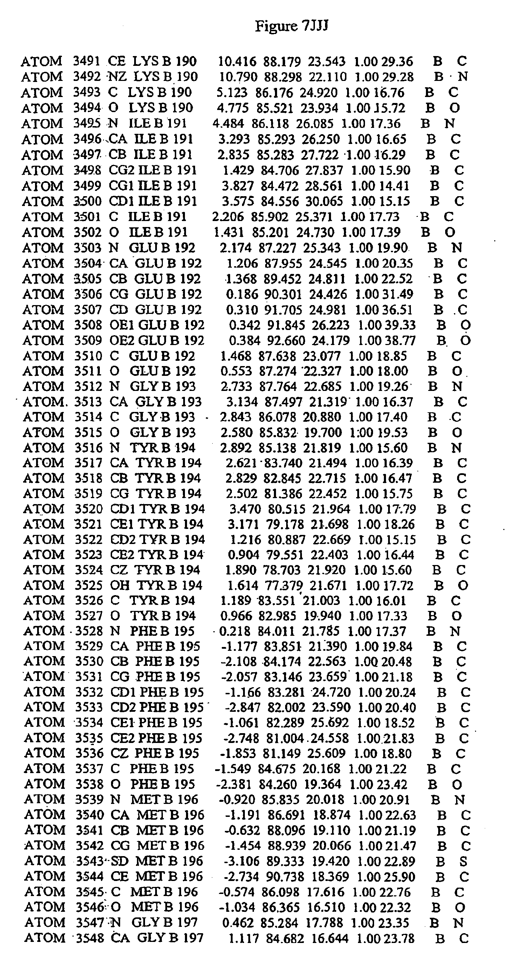

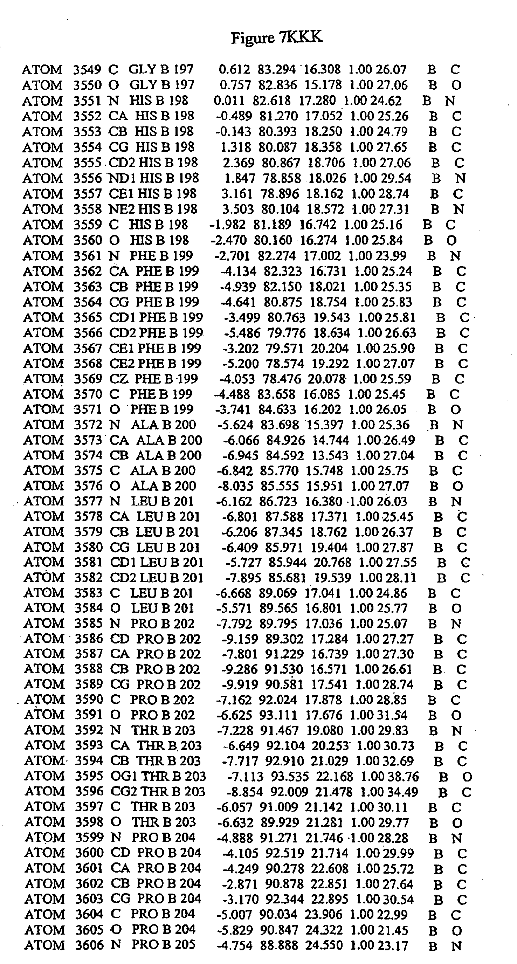

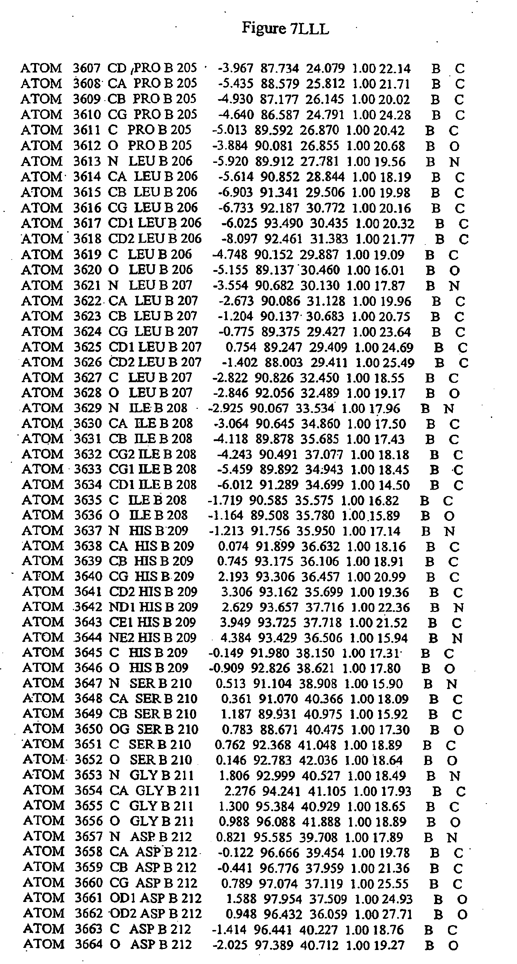

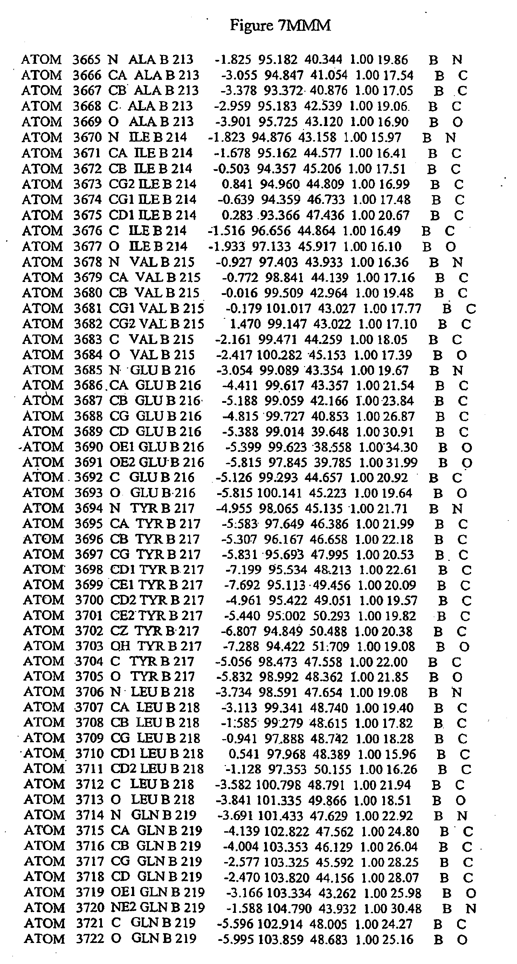

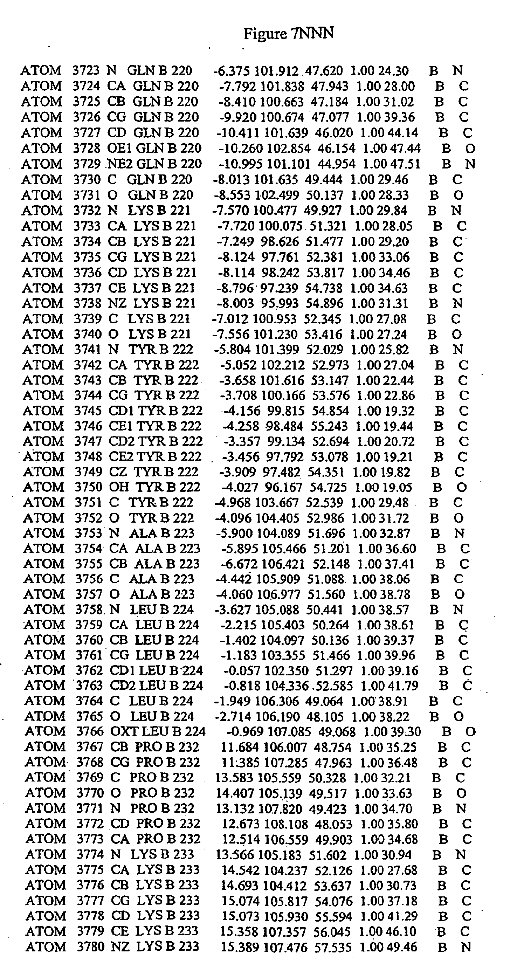

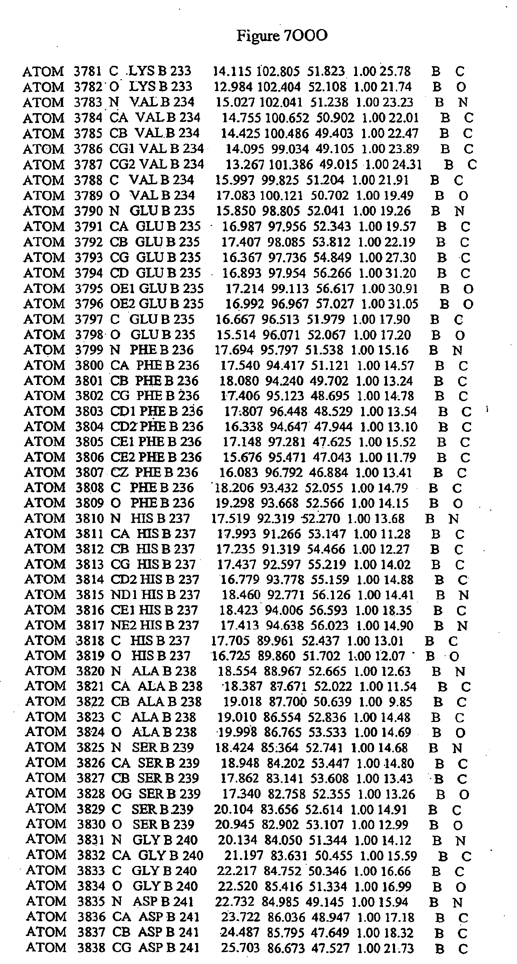

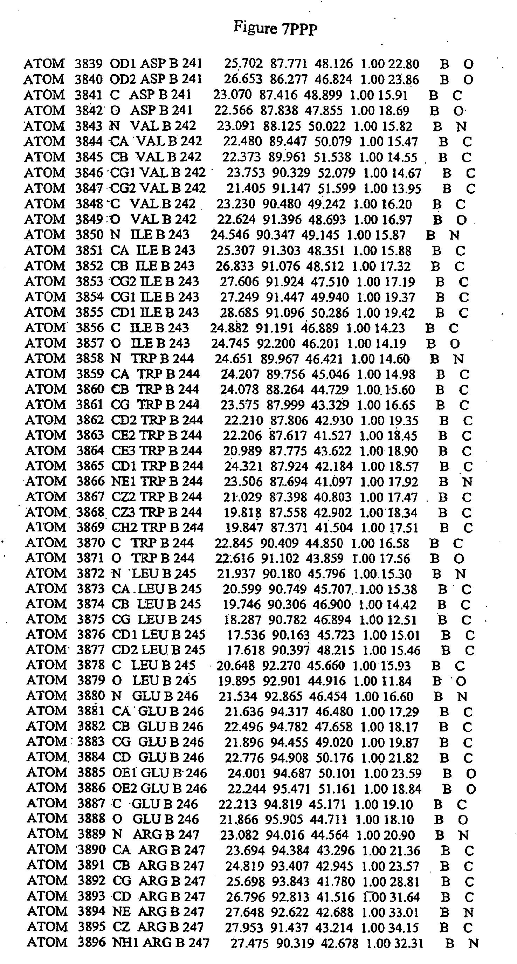

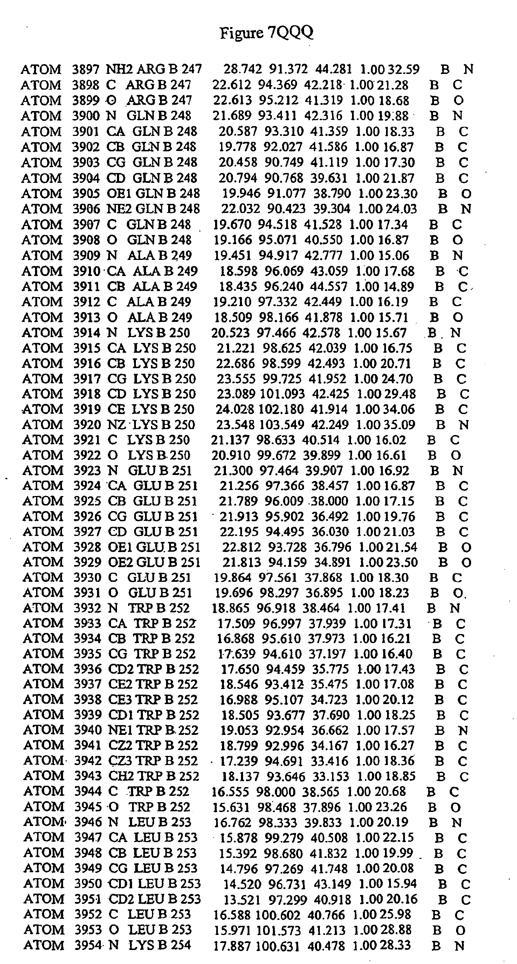

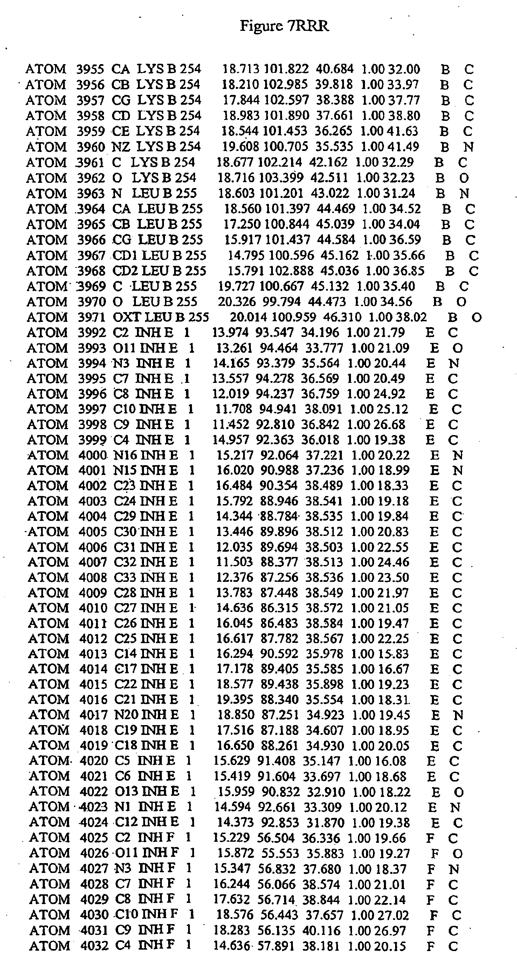

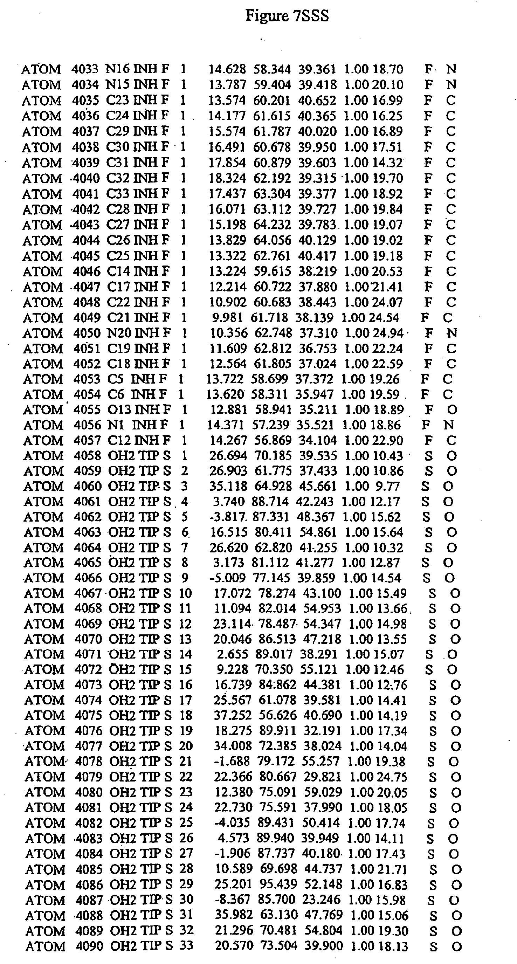

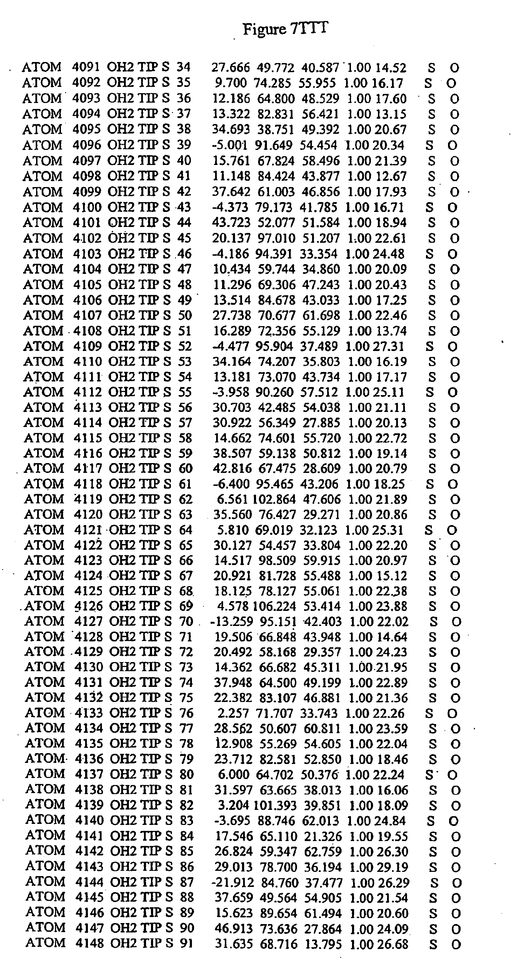

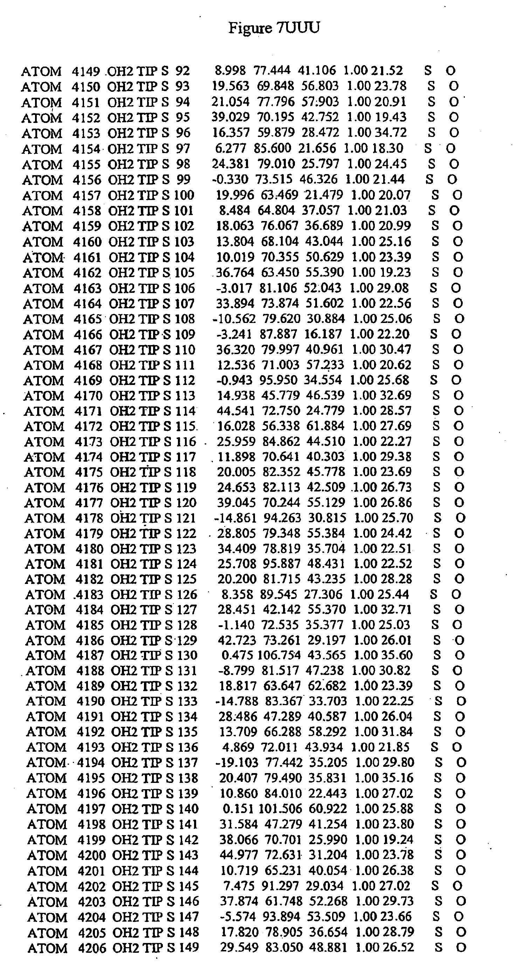

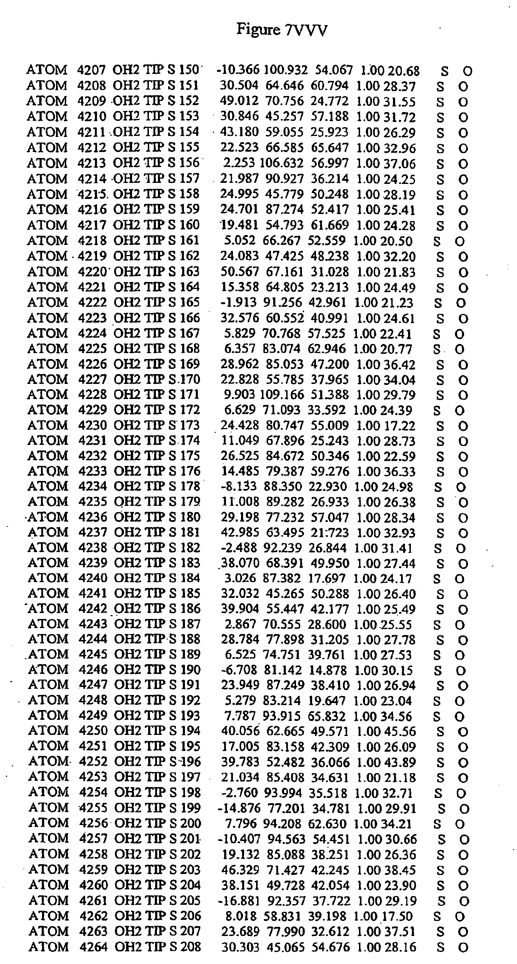

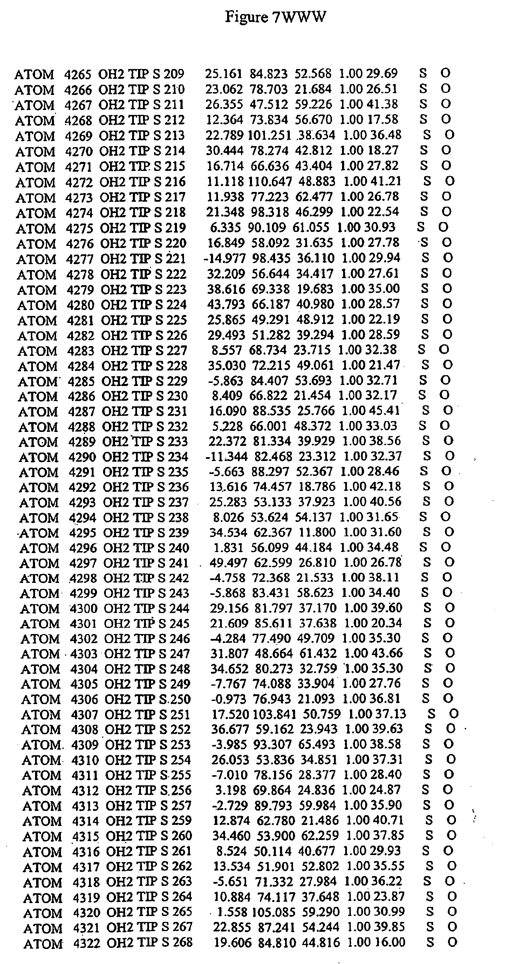

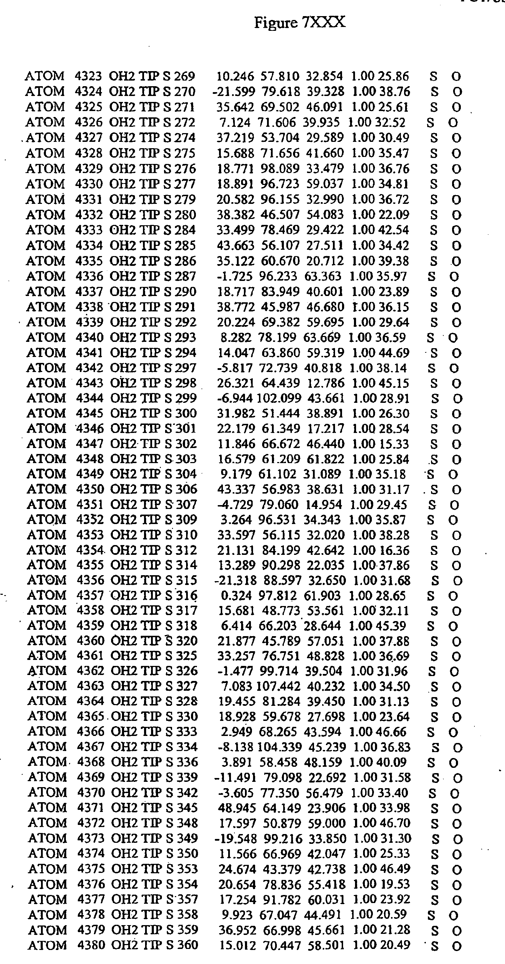

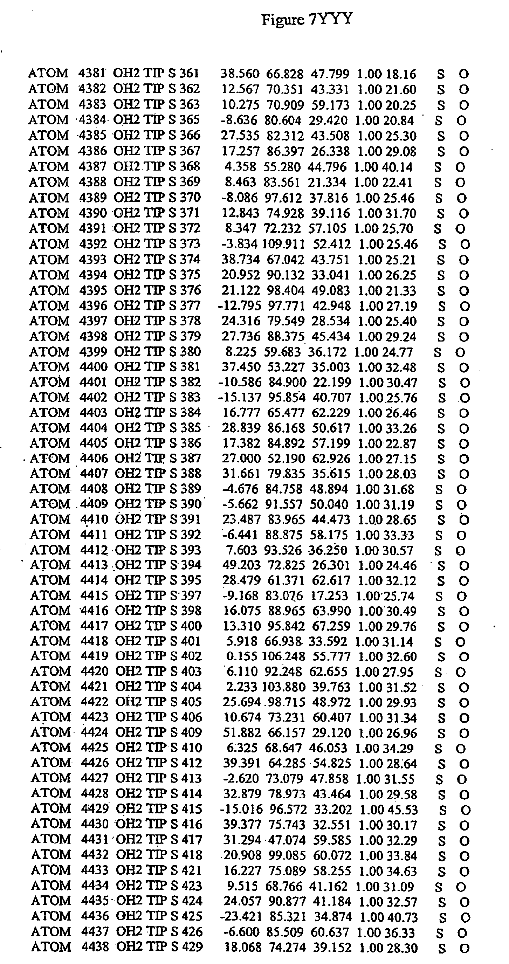

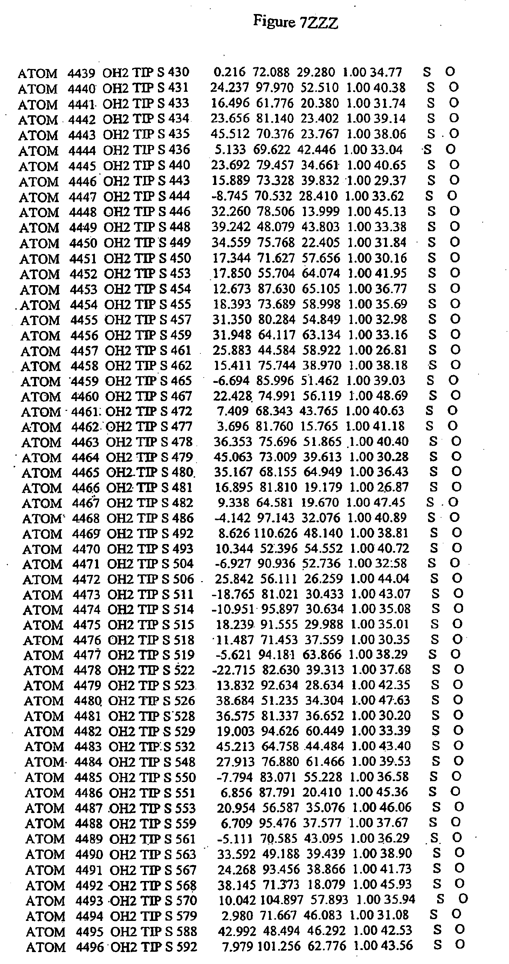

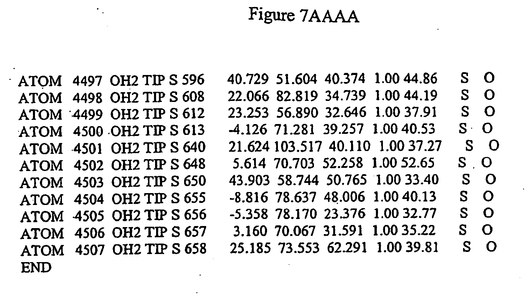

A further embodiment is crystallized *H. pylori* MurI in complex with an inhibitor (e.g., an antibacterial binding agent, drug, etc.). In a further embodiment of the present invention, the crystallized complex is characterized by the structural coordinates depicted in Figure 7, wherein the determined structures presented represent that of MurI in complex with compound A. In a further embodiment, the inhibitor is a pyrimidinedione, such as an imidazolyl pyrimidinedione, a thiophenyl-pyrimidinedione, a furanyl-pyrimidinedione, a pyrazolo-pyrimidinedione, or a pyrrolyl pyrimidinedione.

In a further embodiment, the pyrimidinedione is compound A, compound B, compound C, compound D, compound E, compound F, compound G, compound H, compound I, compound J, compound K, compound L, compound M, compound N, compound O, compound P, compound Q, compound R, compound S, compound T, compound U, compound V, compound W, compound X, compound Y, compound Z, compound AA, compound AB, compound AC, compound AD, compound AE, compound AF, compound AG, compound AH, compound AI, compound AJ, or compound AK, wherein the crystalline complexes are characterized by the space groups and cell dimensions depicted in Table 5.

One further embodiment of the present invention relates to a crystal of *H*. *pylori* MurI complexed with a pyrimidinedione having the orthorhombic space group P2₁2₁2, and having cell dimensions a = 60.7 Å, b = 77.5 Å, c = 56.6 Å, and α = 90°, β = 90°, and γ = 90°. A further embodiment encompasses this crystal made by the process of preparing a first solution of about 5 mM D-L Glutamate; about 1 mM TCEP; about 200 mM ammonium acetate; about 0.1 M Tris pH 7.4-8.5; about 500 micromolar of compound A; and about 10 mg/ml MurI from *H. pylori;* preparing a second solution of about 0.1 M Tris pH 7.4-8.5; about 20-25% PEG 3350; about 15-25% glycerol; and about 0.2 mM ammonium acetate; combining the first solution and the second solution, thereby producing a combination; and forming drops from the combination under conditions in which crystals of MurI are produced.

One further embodiment of the present invention relates to a crystal of *H*. *pylori* MurI complexed with a pyrimidinedione having the monoclinic space group P2₁, and having cell dimensions a = 57.1 Å, b = 78.0 Å, c = 58.55 Å, and α = 90°, β = 97.91°, and γ = 90°. A further embodiment encompasses this crystal made by the process of preparing a first solution of about 5 mM D-L Glutamate; about 1 mM TCEP; about 200 mM ammonium acetate; about 0.1 M Tris pH 7.4-8.5; about 500 micromolar of compound A; and about 10 mg/ml MurI from *H. pylori;* preparing a second solution with about 0.1 M Tris pH 7.4-8.5; about 20-25% PEG 3350; about 15-25% glycerol; and about 0.2 mM ammonium acetate; combining the first solution and the second solution, thereby producing a combination; and forming drops from the combination in a method of crystallization under conditions in which crystals of MurI are produced, whereby, crystals of MurI are produced.

### III. Variants

Variants of the present invention may have an amino acid sequence that is different by one or more amino acid substitutions from the sequence disclosed in SEQ ID NOS: 2 or 44, for example. Embodiments which comprise amino acid deletions and/or additions are also contemplated. The variant may have conservative changes (amino acid similarity), wherein a substituted amino acid has structural or chemical properties similar to those of the amino acid residue it replaces (e.g., the replacement of leucine with isoleucine). Guidance in determining which and how many amino acid residues may be substituted, inserted, or deleted without abolishing biological or pharmacological activity may be reasonably inferred in view of this disclosure and may further be found using computer programs well known in the art, for example, DNAStar® software.

Amino acid substitutions may be made, for instance, on the basis of similarity in polarity, charge, solubility, hydrophobicity, hydrophilicity, and/or the amphipathic nature of the residues as long as a biological and/or pharmacological activity of the native molecule is retained.

Negatively charged amino acids include aspartic acid and glutamic acid; positively charged amino acids include lysine and arginine; amino acids with uncharged polar head groups having similar hydrophilicity values include leucine, isoleucine, and valine; amino acids with aliphatic head groups include glycine, alanine; asparagine, glutamine, serine; and amino acids with aromatic side chains include threonine, phenylalanine, and tyrosine.

Example substitutions are set forth in Table 1 as follows:

**Table 1:**

| Original Residue | Example conservative substitutions |
|---|---|
| Ala (A) | Gly; Ser; Val; Leu; Ile; Pro |
| Arg (R) | Lys; His; Gln; Asn |
| Asn (N) | Gln; His; Lys; Arg |
| Asp (D) | Glu |
| Cys (C) | Ser |
| Gln (Q) | Asn |
| Glu (E) | Asp |
| Gly (G) | Ala; Pro |
| His (II) | Asn; Gln; Arg; Lys |
| Ile (I) | Leu; Val; Met; Ala; Phe |
| Leu (L) | Ile; Val; Met; Ala; Phe |
| Lys (K) | Arg; Gln; His; Asn |
| Met (M) | Leu; Tyr; Ile; Phe |
| Phe (F) | Met; Leu; Tyr; Val; Ile; Ala |
| Pro (P) | Ala; Gly |
| Ser (S) | Thr |
| Thr(T) | Ser |
| Trp(W) | Tyr; Phe |
| Tyr (Y) | Trp; Phe; Thr; Ser |
| Val (V) | Ile; Leu; Met; Phe; Ala |

In the present invention, "amino acid homology" is a measure of the identity of primary amino acid sequences. In order to characterize the homology, subject sequences are aligned so that the highest percentage homology (match) is obtained, after introducing gaps, if necessary, to achieve maximum percent homology. N- or C-terminal extensions shall not be construed as affecting homology. "Identity" *per se* has an art-recognized meaning and can be calculated using published techniques. Computer program methods to determine identity between two sequences include, for example, DNAStar® software (DNAStar Inc. Madison, WI); the GCG® program package (Devereux, J., et al. Nucleic Acids Research (1984) 12(1): 387); BLASTP, BLASTN, FASTA (Atschul, S.F. et al., J. Molec Biol (1990) 215: 403). Homology (identity) as defined herein is determined using the well-known computer program, BESTFIT® (Wisconsin Sequence Analysis Package, Version 8 for Unix, Genetics Computer Group, University Research Park, 575 Science Drive, Madison, WI, 53711). When using BESTFIT® or any other sequence alignment program (such as the Clustal algorithm from MegAlign software (DNAStar®) to determine whether a particular sequence is, for example, about 90% homologous to a reference sequence, according to the present invention, the parameters are set such that the percentage of identity is calculated over the full length of the reference nucleotide sequence or amino acid sequence and that gaps in homology of up to about 90% of the total number of nucleotides in the reference sequence are allowed.

Ninety percent homology is therefore determined, for example, using the BESTFIT® program with parameters set such that the percentage identity is calculated over the full length of the reference sequence, e.g., SEQ ID NOS: 2 or 44, and up to 10% of the amino acids in the reference sequence may be substituted with another amino acid. Percent homologies are likewise determined, for example, to identify preferred species, within the scope of the claims appended hereto, which reside within the range of about 70% to 100% homology to SEQ ID NOS: 2 or 4, as well as the binding site thereof. As noted above, N- or C-terminal extensions shall not be construed as affecting homology. When comparing two sequences, the reference sequence is generally the shorter of the two sequences. This means that, for example, if a sequence of 50 nucleotides in length has exact identity to a 50 nucleotide region within a 100 nucleotide polynucleotide, there is 100% homology for that region as opposed to only 50% homology.

Although the naturally polypeptides of a sequence such as SEQ ID NO: 2, and a variant polypeptide may be only 90% identical, they are actually likely to have a higher degree of similarity, depending on the number of dissimilar codons that are conservative changes. Conservative amino acid substitutions can frequently be made in a protein without altering either the conformation or function of the protein. Similarity between two sequences includes direct matches, as well as conserved amino acid substitutions which have similar structural or chemical properties, e.g., similar charge as described in Table 1.

Percentage similarity (conservative substitutions) between two polypeptides may also be scored by comparing the amino acid sequences of the two polypeptides by using programs well known in the art, including the BESTFIT program, by employing default settings for determining similarity.

A further embodiment is a crystal of MurI having an amino acid sequence represented by any one of SEQ ID NOS: 2-34, 40, 44, 46, 48, 50, 52, 54, 56, 58, 60, 62, 64, 66, and 74. A further embodiment is a crystal of MurI, wherein the amino acid sequence is at least 75%, 80%, 85%, 90%, 95%, 98%, or 99% homologous to the amino acid sequence represented by any one of SEQ ID NOS: 2-34, 40, 44, 46, 48, 50, 52, 54, 56, 58, 60, 62, 64, 66, and 74.

Mutations/substitutions in the present invention can be made using techniques well-known in the art (e.g. site-directed mutagenesis) using molecular based methods.

### IV. Structural Homology and Conserved Structural Elements

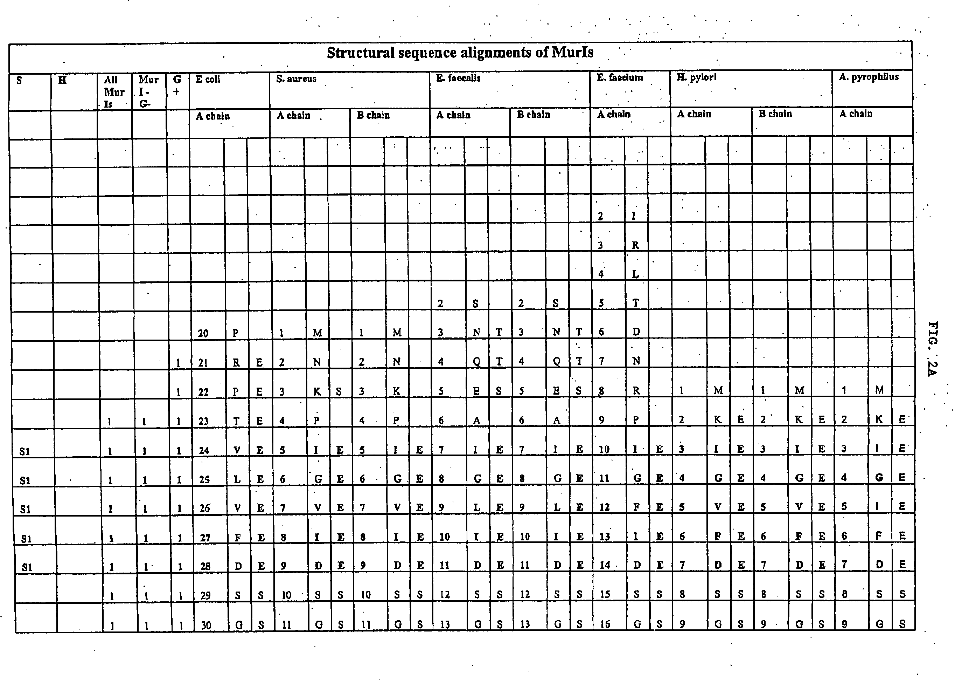

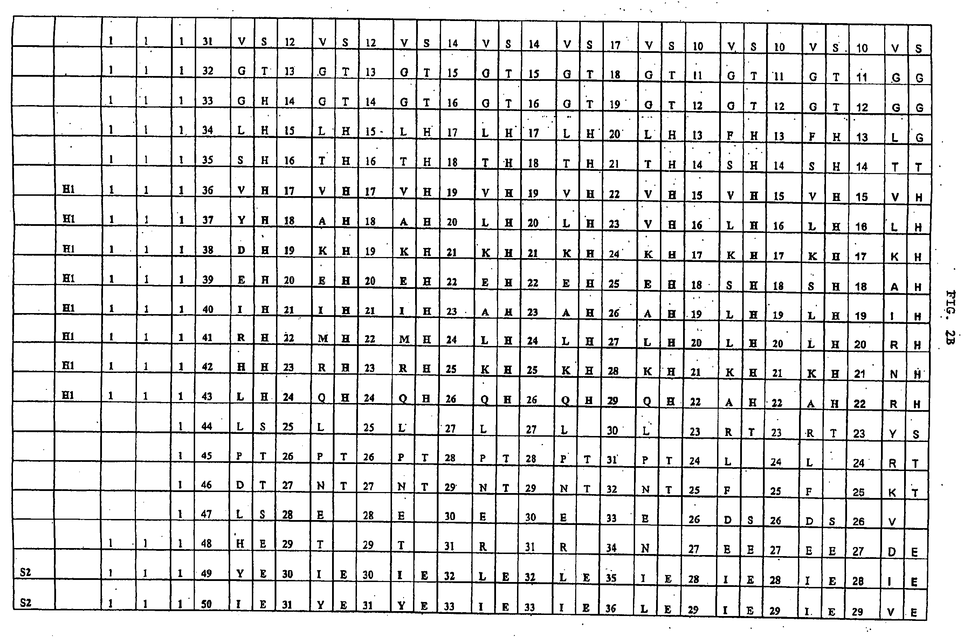

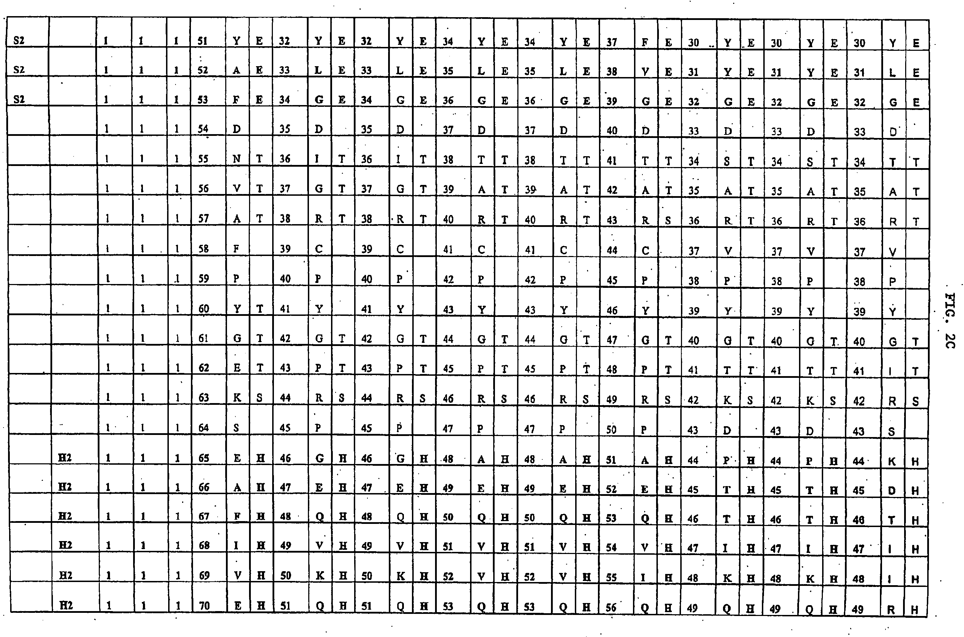

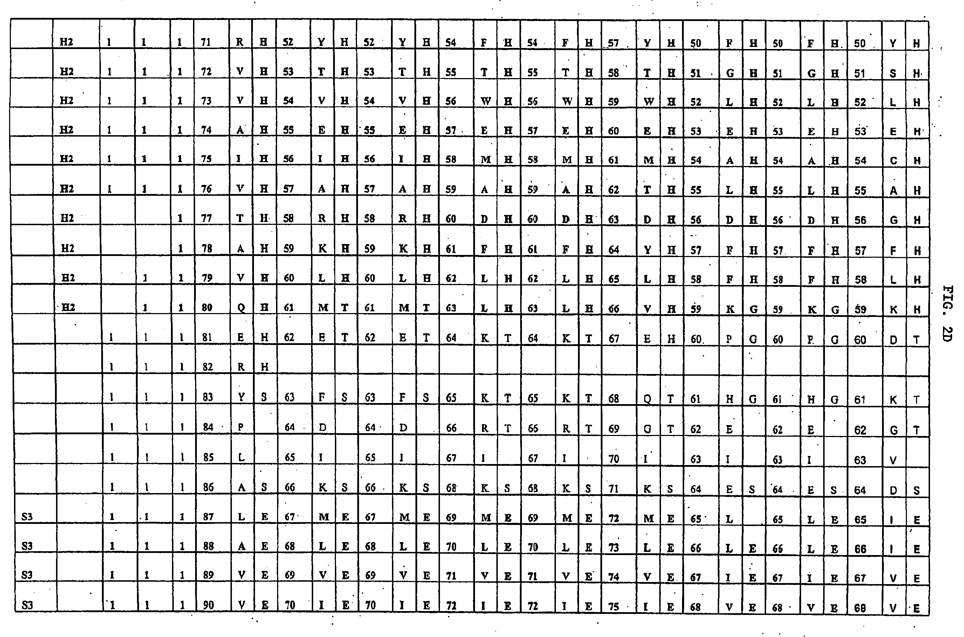

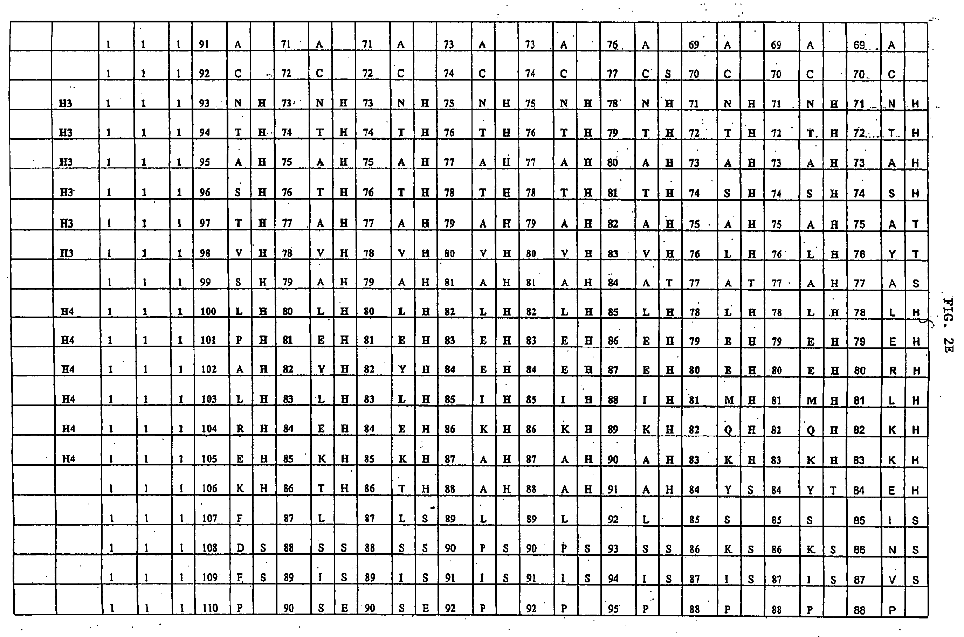

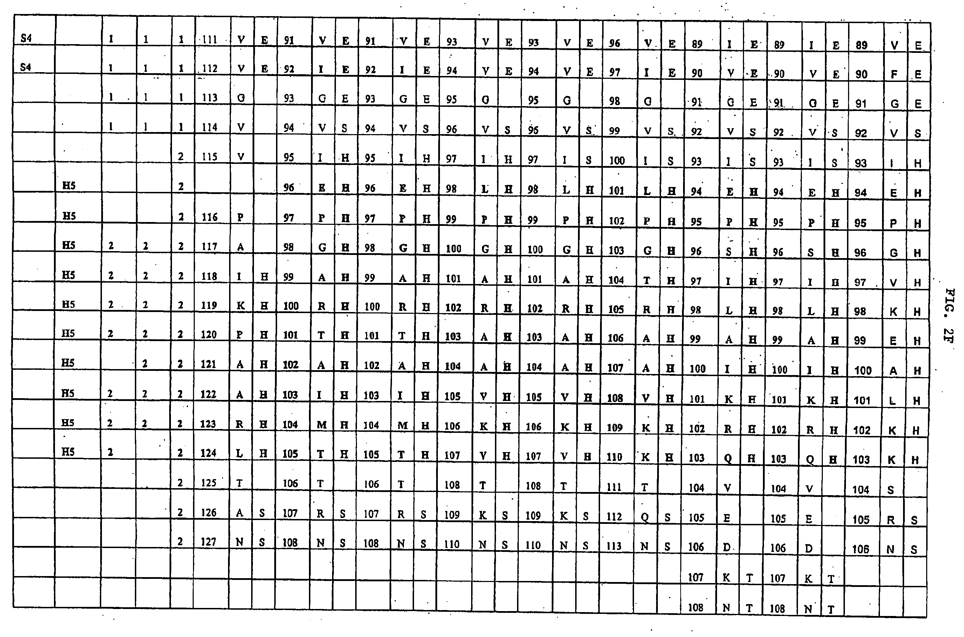

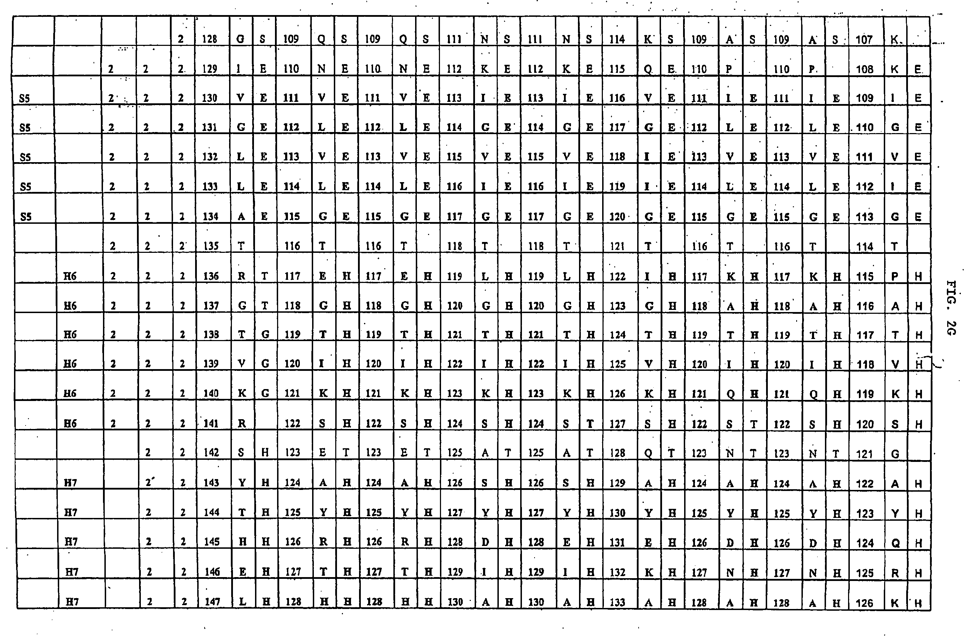

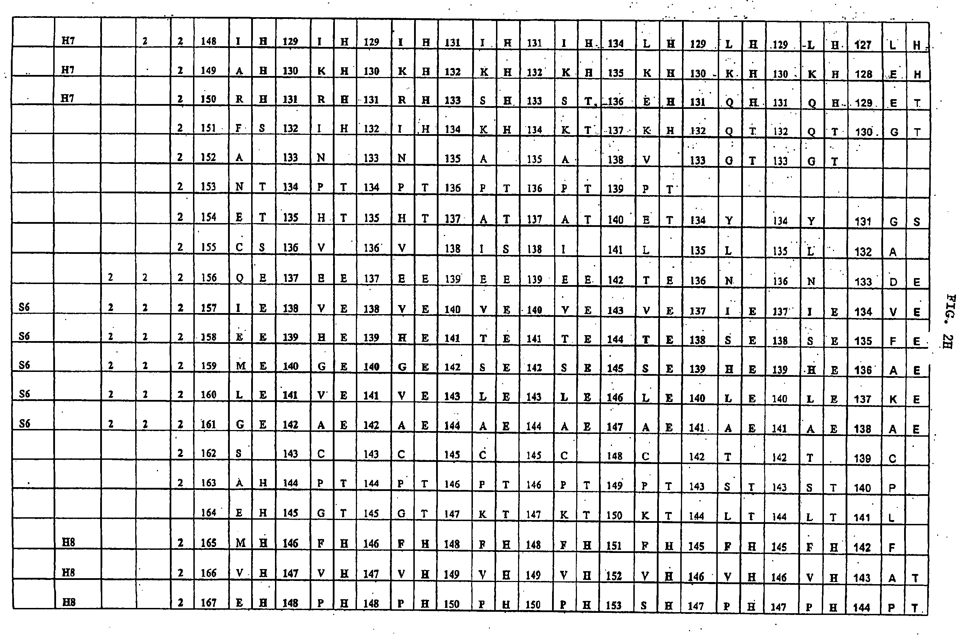

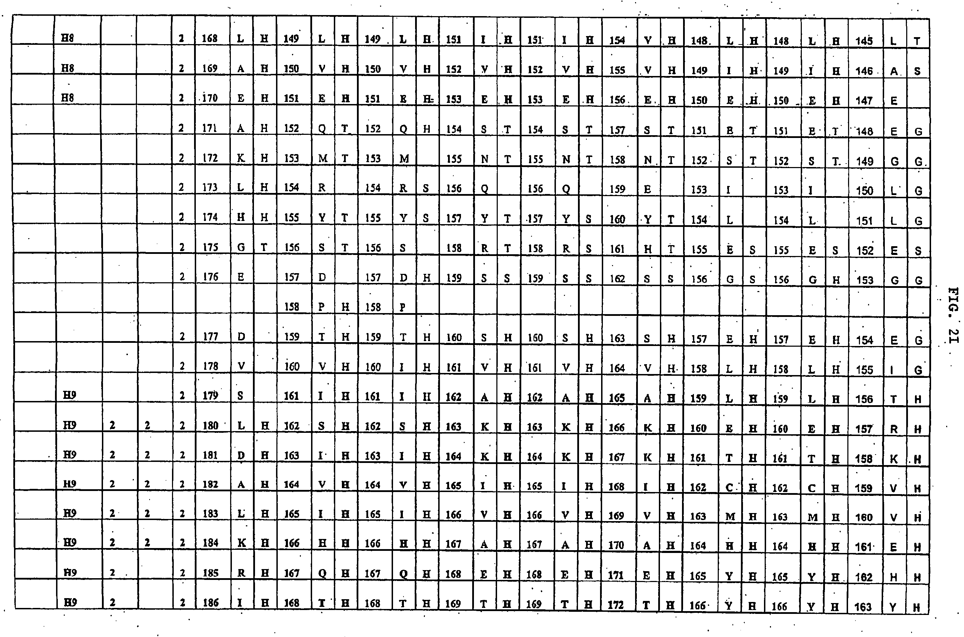

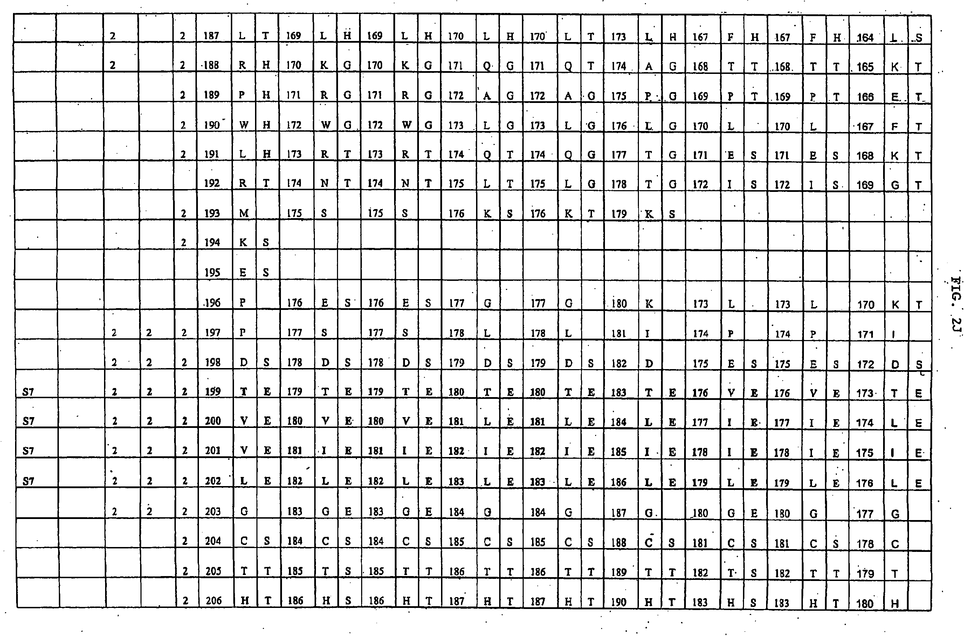

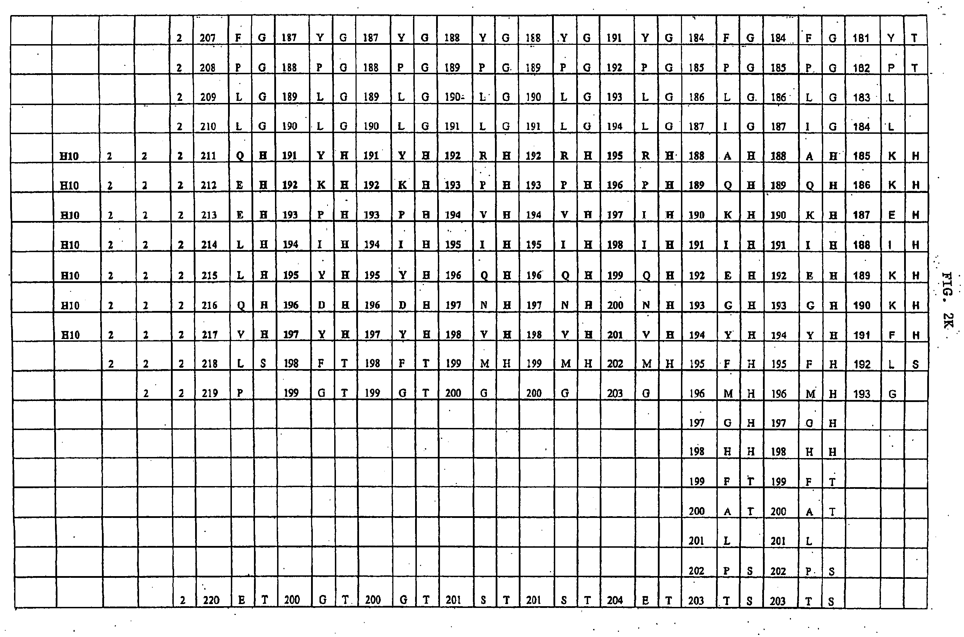

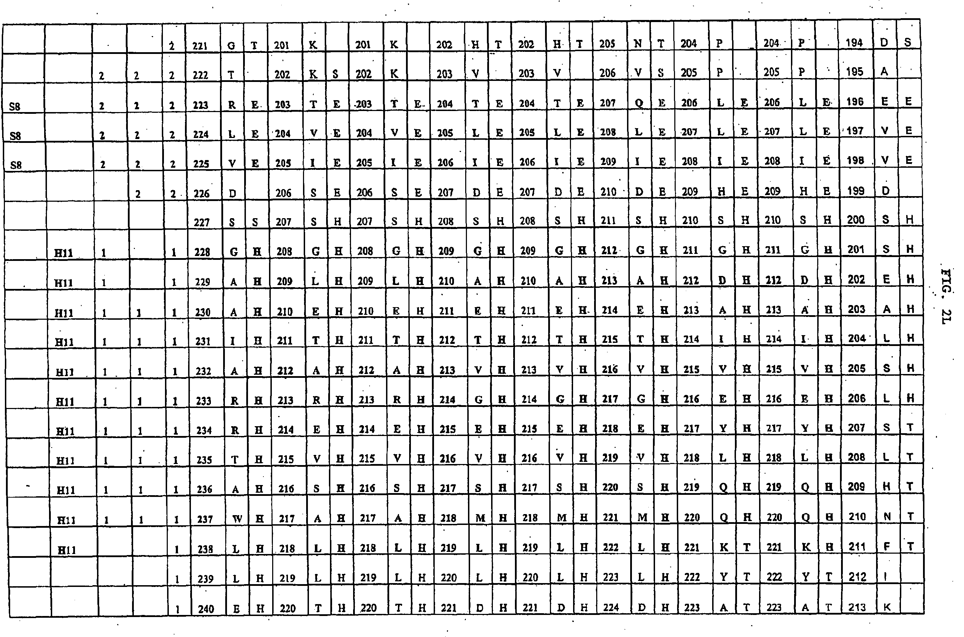

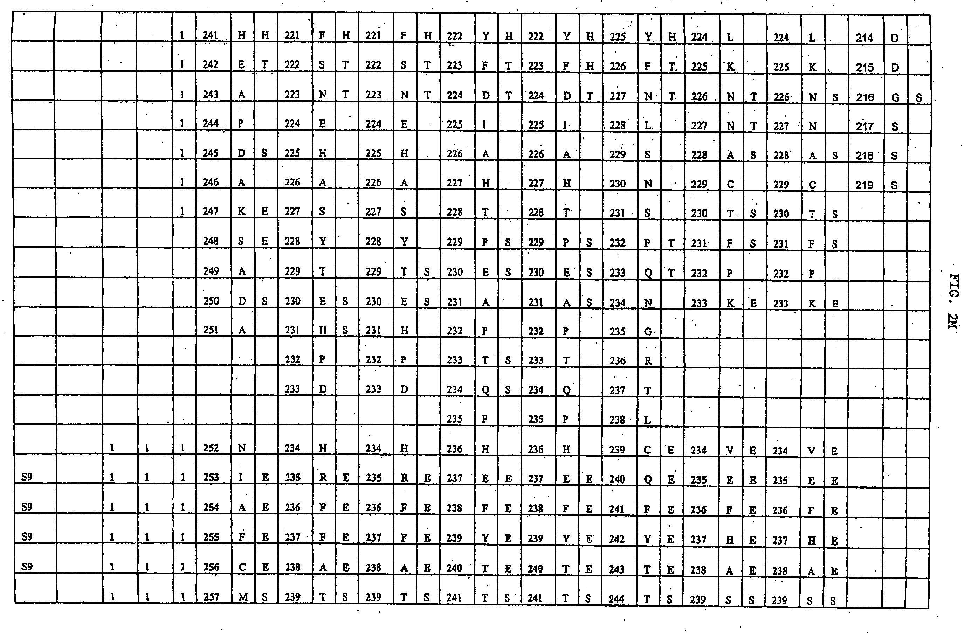

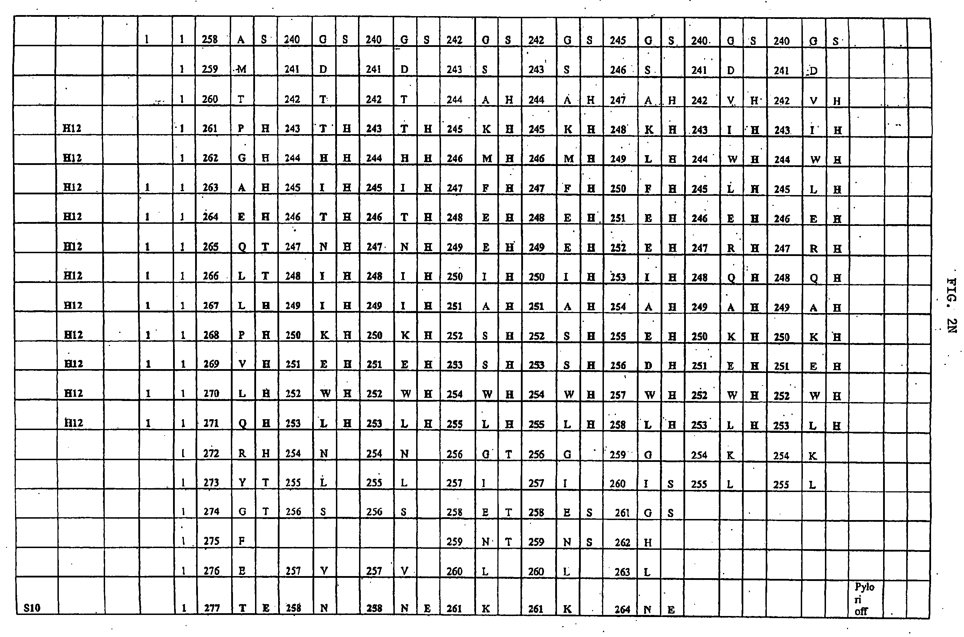

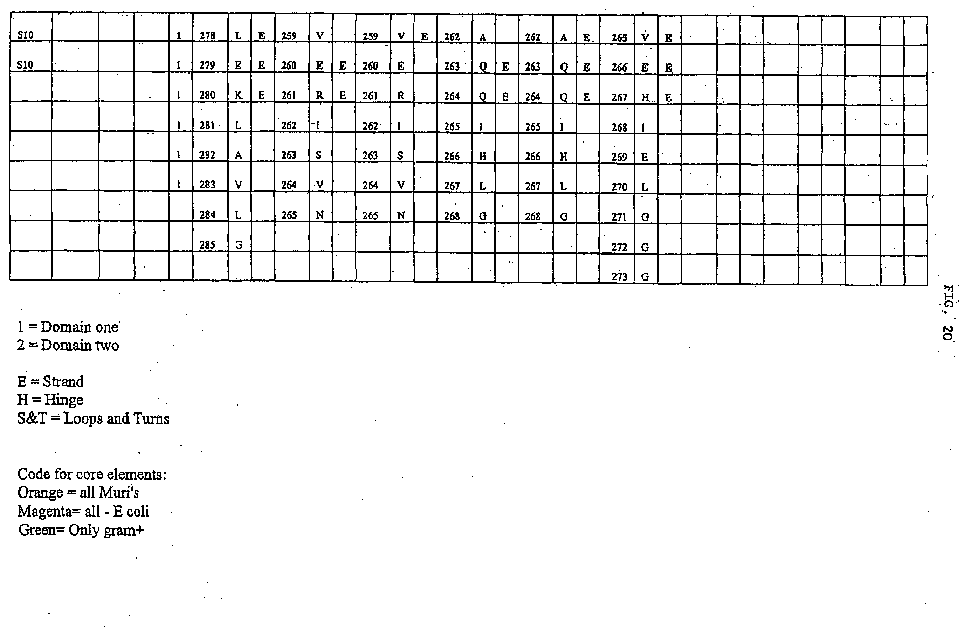

Applicants have determined that MurI from Gram negative, Gram positive, and atypical bacterium have common structural elements that are constant even in view of primary amino acid sequences that differ with respect to their length of sequences and overall content. These common structural elements are depicted in Figures 1 and 2. As used herein, the term "structural homology" refers to conserved structural motifs which may vary with respect to the primary amino acid sequence up to 75%, but have similar or identical tertiary structures.

Figure 1A illustrates a monomer of MurI depicting conserved structural elements. The topology of the conserved structural elements is illustrated in Figure 1B. One of ordinary skill in the art would recognize that all MurIs share certain structural features as indicated by the column headed: "All". Specific structural features are conserved among atypical and Gram positive bacterium ("MurI - G-"), and others are conserved within the genus of Gram positive bacterium ("G+"). Subsets of the conserved structural elements, (e.g., strands and helices) are depicted by the abbreviations H (helices), E (strands), S (loops) and T (turns). Structural elements conserved across all MurI are indicated by a number in the column entitled "All MurI"; structural elements conserved in MurI of Gram positive and atypical bacterium are indicated by a number in the column headed "MurI - *E*. *coli";* and structural elements conserved in MurI of Gram positive bacterium are indicated by a number in the column headed "G+". The number "1" indicates that the amino acid residue resides in domain 1 of MurI, whereas the number "2" indicates that the amino acid residue resides in domain 2 of MurI.

As used herein, the term "accessory site" means a combination of any of the conserved structural elements, a subset of any of the conserved structural elements, or part or all of a binding site of the present invention.

### A. Gram negative

Topology and conserved structural elements of *E*. *coli* MurI are illustrated in Figures 1A and 2, respectively. One embodiment are conserved structural elements of *E*. *coli* MurI represented by helices (H), beta sheets (E), loops (S), and turns (T). Exemplary examples of conserved structural elements of Gram negative bacterium of the present invention are S1: 24V-28D; H1: 36V-43L; S2: 49Y-53F; H2: 65E-80Q; S3: 87L-90V; H3: 93A-98V; H4: 100L-105E; S4: 111V-112V; H5: 118I-124L; S5: 130V-134A; H6: 136R-141R; H7: 143Y-150R; 56: 157I-161G; H8: 165M-170E; H9: 180L-186I; 57: 199T-202L; H10: 211Q-217V; S8: 223R-225V; HII: 228G-238L; 59: 2531-256C; H12: 261P-271Q; and S10: 227T-279E.

### B. Gram positive

### 1. E. faecalis

Conserved structural elements of *E. faecalis* MurI are illustrated in Figures 1A, 1B and 2. One embodiment are conserved structural elements of *E. faecalis* MurI represented by helices (H), beta sheets (E), loops (S), and turns (T). Exemplary examples of conserved structural elements of the present invention are S1: 7I-11D; H1: 19V-26Q; S2: 32L-36G; H2: 48A-63L; S3: 69M-72I; H3: 75N-80V; H4: 82L-87A; S4: 93V-94V; H5: 98L-107V; S5: 113I-117G; H6: 119L-124S; H7: 126S-133S; 56: 140V-144A; H8: 148F-153E; H9: 162A-169T; S7: 180T-183L; H10: 192r-198v; S8: 204T-206I; H11: 209G-219L; S9: 237E-240T; and H12: 245K-255L.

### 2. E. faecium

Conserved structural elements of *E. faecium* MurI are illustrated in Figures 1A, 1B and 2. One embodiment are conserved structural elements of *E. faecium* MurI represented by helices (H), beta sheets (E), loops (S), and turns (T). Exemplary examples of conserved structural elements of the present invention are S1: 101-14D; H1: 22V-29Q; S2: 351-39G; H2: 51A-66V; S3: 72M-751; H3: 78N-83V; H4: 85L-90A; S4: 96V-97I; H5: 101L-110A; S5: 116V-120G; H6: 122I-127S; H7: 129A-136E; S6: 143V-147A; H8: 151F-156E; H9: 166K-172T; S7: 183T-186L; H10: 195R-201V; S8: 207Q-209I; H11: 212G-222L; S9: 240Q-243T; H12: 248K-258L; and S10: 264E-266E.

### 3. S. aureus

Conserved structural elements of *S*. *aureus* MurI are illustrated in Figures 1A, 1B and 2. One embodiment are conserved structural elements of *S*. *aureus* MurI represented by helices (H), beta sheets (E), loops (S), and turns (T). Exemplary examples of conserved structural elements of the present invention are S1: 51-9D; H1: 17V-24Q; S2: 301-34C; H2: 46G-61M; S3: 67M-70I; H3: 73N-78V; H4: 80L-85K; S4:91V-92I; H5:96E-105T; S5: 111V-115G; H6:117E-122S; H7: 124A-131R; S6: 138V-142A; H8: 146F-151E; H9: 162S-168T; S7:179T-182L; H10: 191Y-197Y; S8: 203T-205I; and H11: 208G-218L.

### C. Atypical

### 1. H. pylori

Conserved structural elements *of H. pylori* MurI are illustrated in Figures 1A, 1B and 2. One embodiment of the present invention are conserved structural elements of *H. pylori* MurI represented by helices (H), beta sheets (E), loops (S), and turns (T). Exemplary examples of conserved structural elements of the present invention are S1: 3I-7D; H1: 15V-22A; S2: 28I-32G; H2: 44P-59K; S3: 65L-68V; H3: 71N-76L; H4: 78L-83K; S4: 89I-90V; H5: 94E-103Q; S5: 111I-115G; H6: 117K-122S; H7: 124A-131Q; S6: 137I-141A; H8: 145F-150E; H9: 160E-166Y; S7: 176V-179L; H10: 188A-194Y; S8: 206L-208F; H11: 211G-221K; S9: 235E-238A; and H12: 243I-253L.

### 2. A. pyrophilus

Conserved structural elements of *A*. *pyrophilus* MurI are illustrated in Figures 1A, 1B and 2. One embodiment are conserved structural elements *A*. *pyrophilus* MurI represented by helices (H), beta sheets (E), loops (S), and turns (T). Exemplary examples of conserved structural elements of the present invention are S1: 3I-7D; H1: 15V-22R; S2: 28I-32G; H2: 44K-59K; S3: 65I-68V; H3: 71N-76Y; H4: 78L,-83K; S4: 89V-90F; H5: 94E-103K; S5: 109I-113G; H6: 115P-120S; H7: 122A-129E; S6: 134V-138A; H8: 142F-147E; H9: 157R-163Y; S7:173T-176L; H10: 185K-191F; S8: 196E-198V; H11: 201S-211F; S9: 221E-224F; H12: 229P-239L; and S10: 245V-247L.

### C. Common structural elements for Gram positive and atypical MurI

Analysis of the atomic coordinates with reference to the 3-dimensional structure of MurI showed that MurI from Gram positive and atypical bacterium share conserved structural elements and fragments regardless of differences in primary amino acid sequence. The shared elements are illustrated below in Table 2. The thirteen shared elements of each conserved element are provided in terms of the primary amino acid sequence represented in Figure 2.

**Table 2**

| | 1 | 2 | 3 | 4 | 5 | 6 | 7 | 8 | 9 | 10 | 11 | 12 | 13 |
|---|---|---|---|---|---|---|---|---|---|---|---|---|---|
| *S. aureus* | 4P- 24Q | 29T- 57A | 60L- 94V | 98G- 104M | 110N-1291 | 137E-142A | 162S-166H | 177S- 183G | 191Y- 199G | 202K- 206S | 210E- 217A | 234H-240G | 245I-253L |
| *E. faecalis* | 6A- 26Q | 31R-59A | 62L-96V | 100G- 106K | 112K-131I | 139E-144A | 163K-167A | 178L, 184G | 192R- 200G | 203V- 207D | 211E- 218M | 236H-242G | 247F-255L |
| *E. faecium* | 9P- 29Q | 34N- 62T | 65L-99V | 103G- 109K | 115Q-134L | 142T-147A | 166K-170A | 18II- 187G | 195R- 203G | 206V- 210D | 214A-221M | 239C-245G | 250F-258L |
| *H. pylori* | 2K- 22A | 27E-55L | 59F- 92V | 96S- 102R | 110P-129L | 136N-141A | 160E-164H | 174P- 180G | 188A- 196M | 205P- 209H | 213A- 220Q | 234V-240G | 245L-253L |
| *A*. *pyrophilus* | 2K- 22B | 27D- 55A | 58L- 92V | 96G- 102K | 108K-127L | 133D-138A | 157R- 161E | 171I- 177G | 185K- 193G | 195A- 199D | 203A- 210N | 220L-226D | 231L-239L |

### D. Common structural elements for all MurI

Analysis of the atomic coordinates with reference to the 3-dimensional structure of MurI showed that all MurI share conserved structural elements and fragments regardless of differences in primary amino acid sequence. The shared elements are illustrated below in Table 3. The thirteen shared elements of each conserved element are provided in terms of the primary amino acid sequence represented in Figure 2.

**Table 3**

| | 1 | 2 | 3 | 4 | 5 | 6 | 7 | 8 | 9 | 10 | 11 | 12 | 13 |
|---|---|---|---|---|---|---|---|---|---|---|---|---|---|
| *E. coli* | 23T-43L | 48H-76V | 81E-114V | 117A-120R | 122A-124L | 1291-141R | 156Q-161G | 180L-188R | 197P-203G | 211Q- 218L | 222T-25V | 228G-237W | 252N-257M |
| *S. aureus* | 4P- 24Q | 29T-57A | 62E-94V | 98G- 101T | 103I-105T | 110N-122S | 137E-142A | 162S-170K | 177S-183G | 191Y- 198F | 202K-2051 | 208G-217A | 234H-239T |
| *E. faecalis* | 6A-26Q | 31R-59A | 64K-96V | 100G-103A | 105V-107V | 112K-124S | 139E, 144A | 163K-171Q | 178L-184G | 192R- 199M | 203V-2061 | 209G-218M | 236H-242T |
| *E.faecium* | 9P-29Q | 34N-62T | 67E-99V | 1030-106A | 108V-110K | 115Q-127S | 142T- 147A | 166K-174A | 18II-187G | 195R- 202M | 206V-2091 | 212G-221M | 239C-244T |
| *H. pylori* | 2K-22A | 27E-55L | 60P-92V | 96S- 99A | 101K-103Q | 110P-122S | 136N- 141A | 160E-168T | 174P-180G | 188A- 195F | 205P-2081 | 211G-220Q | 234V-239S |
| *A*. *pyrophilus* | 2K-22R | 27D-55A | 60D-92V | 96G- 99E | 101L-103K | 108K-120S | 133D- 138A | 157R-165K | 171I-177G | 185K- 192L | 195A-198V | 201S-210N | 220L-225T |

### E. Common structural elements for Gram positive MurI

Analysis of the atomic coordinates with reference to the 3-dimensional structure of MurI showed that MurI from Gram positive bacterium share conserved structural elements and fragments regardless of differences in primary amino acid sequence. The shared elements are illustrated below in Table 4. The seven shared elements of each conserved element are provided in terms of the primary amino acid sequence represented in Figure 2.

**Table 4**

| | 1 | 2 | 3 | 4 | 5 | 6 7 | |
|---|---|---|---|---|---|---|---|
| *S. aureus* | 2N-144P | 146F-157D | 159T-173R | 177S- 199G | 201K-206S | 208G-227S | 234H-24V |
| *E. faecalis* | 4Q-146P | 148F-159S | 160S-174Q | 178L- 2030 | 202H-207D | 209G-228T | 236H-27L |
| *E. faecium* | 7N-149P | 151F-162S | 163S- 177T | 18II- 2030 | 205N-210D | 212G-231S | 239C-20L |

### V. Binding Sites

MurI is highly flexible and depending on its conformation, can bind to a wide variety of structures. MurI from Gram positive bacterium, such as *E. faecalis, E. faecium,* and *S. aureus;* Gram negative bacterium, such as *E*. *coli;* and atypical bacterium, such as *H. pylori,* and *A. pyrophilus,* have been crystallized.

A study of the structural coordinates and biochemistry has revealed a molecular interface along an axis of MurI that is highly flexible and allows the enzyme to exist in multiple conformations which are determined by the substances that bind to the active site, intradomain interface, or intermolecular dimer interface, and in the case of MurI from Gram negative bacterium, the activator site. As such, the enzyme binds multiple substrates and inhibitors with a wide variety of structural features and sizes.

MurI from Gram positive and atypical bacterium comprise a dimeric structure of two monomers, each of which is comprised of 2 domains. The "molecular interface" of MurI in Gram positive and atypical bacterium has three domains: a substrate binding site, an intermolecular dimer interface, and an intradomain interface.

MurI from Gram negative bacterium is a monomer having three domains along the molecular interface: a substrate binding site, an activator binding site, and an intradomain interface. All MurI described herein have an "intradomain interface". When an inhibitor binds to the intradomain interface, it prevents movement of the enzyme, thereby inhibiting the enzyme.

MurI from Gram positive bacterium are dimers, each monomer of the dimer having two domains. Gram positive bacteria have an intermolecular dimer interface in which the two substrate binding sites face outward on opposite ends of the enzyme, such that MurI can simultaneously binds two substrate moieties.

MurI from atypical bacterium, such as *H. pylori,* are dimers having two monomers, each monomer having two domains, and each domain having a substrate binding site. The substrate binding site of *A. pyrophilus* MurI differs slightly in that the substrate is in contact with residues from each monomer, each containing key elements of the active site.

As used herein, the term "binding site" refers to a specific region (or atom) of MurI that enters into an interaction with a molecule that binds to MurI. A binding site can be, for example, a conserved structural element or a combination of several conserved structural elements, a substrate binding site, an activator binding site, an inhibitor binding site, an intradomain interface, or an intermolecular dimer interface.

As used herein, the term "substrate binding site" refers to a specific region (or atom) of MurI that interacts with a substrate, such as D-glutamate. In the present application, the terms substrate binding site and active site are used interchangeably. A substrate binding site may comprise, or be defined by, the three dimensional arrangement of two or more amino acid residues within a folded polypeptide.

In the present invention, a substrate can be a compound such as L-glutamate or D-glutamate which MurI reversibly converts from the R- to S-enantiomer. Thus, a substrate can also act a product. The substrate can be a naturally-occurring or artificial compound.

In the present invention, an inhibitor can be a compound which also may undergo a catalytic reaction, bind to the substrate binding site, or another site on MurI and which competes with substrate turnover of glutamate. Inhibitors of the present invention can be a compound such as-L-serine-O-sulfate, D-serine-O-sulfate, D-aspartate, L-aspartate, tartrate, citrate, phosphate, sulfate, aziridino-glutamate, N-hydroxyglutamate, or 3-.chloroglutamate. The inhibitor can be a naturally-occurring or artificial compound.

As used herein, the term "activator binding site" refers to a specific region (or atom) of MurI that interacts with an activator, such as UDP-MurNAc-Ala. An activator binding site may comprise, or be defined by, the three dimensional arrangement of one or more amino acid residues within a folded polypeptide. An activator can be a compound, such as UDP-MurNAc-Ala. The activator can be a naturally-occurring or an artificial compound. The structure of the activator is compact when it binds to MurI of Gram negative bacterium. When complexed with MurI, the activator (such as UDP-MurNAc-Ala) folds back on itself into a two-layered structure in which two phosphate groups act as a bridging link. The uridine ring of UDP-MurNAc-Ala stacks against the muramic acid ring. There is almost a perfect shape match between MurI and the activator because the activator resides on top of two connecting loops between the two domains, corresponding in sequence to residues 112-116 and 225-228. In the absence of activator, the two loops act as a hinge between the two domains. The binding of MurI and the activator is not entirely driven by favorable van der Waal interactions; there are a number of polar interactions and at least one strong salt bridge between Arg104 and the carboxylate group of the alanine motif of the activator. The salt bridge could be a key interaction for UDP-MurNAc-Ala to be able to lock the two domains into the proper orientation. This explains the need for the terminal alanine residue in order for the molecule to work as an activator. Other extensive polar interactions are found between the uracil ring of UDP-MurNAc-Ala and main chain atoms of MurI, residues 113-115, as well as a number of hydrogen bonds from the hydroxyl groups of the two sugar rings of UDP-MurNAc-Ala. Many interactions between activator and MurI are mediated by water molecules. In contrast to the uracil and sugar moieties, two phosphate groups of UDP-MurNAc-Ala make very little contact with the protein since they are facing towards the solution. On each side of the diphosphate group is a positively charged residue, Lys119 on one domain, and Arg233 from the other, which provides another example of domain-domain stabilizing interaction.

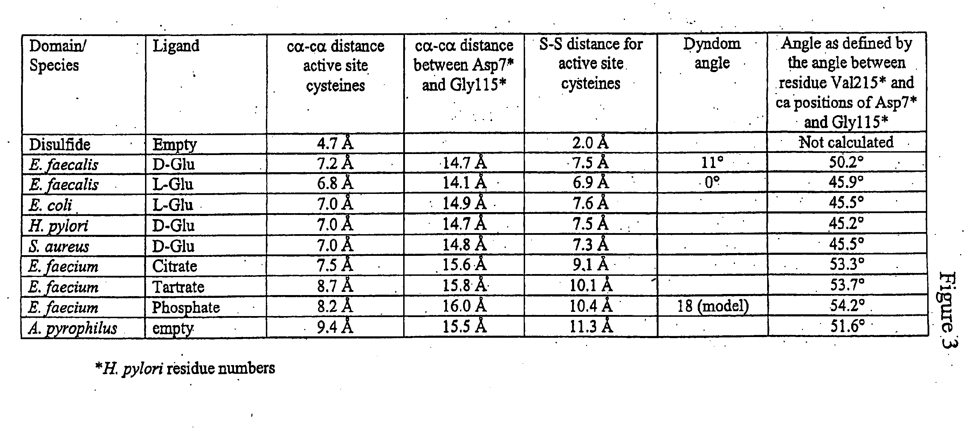

One part of the molecular interface is an "intermolecular dimer interface" that is present only in MurI dimers (Gram positive and atypical bacterium) and occurs at the interface of the monomers that make up the dimer. Analysis of this intermolecular dimer interface of multiple MurIs has revealed that the interface is highly flexible and also allows rotation of the monomers with respect to one another. The distance between the cα-cα active site cysteines present in the monomer and the extent of the angle of the opening can vary greatly (See Figure 1C, and Figure 3, column 3). They determine the structure of substrate or inhibitor that binds to different regions or components of the enzyme. Applicants have determined, for each species that has been crystallized, the amino acid residues present in the intermolecular dimer interface, and have compared the primary amino acid sequence across the species of MurI from Gram positive and atypical bacterium. Accordingly, Applicants have been able to determine the degree (in Å) of flexibility of MurI when bound to several substrates or inhibitors such that it would be expected that MurI of all Gram positive and atypical bacterium would be expected to behave in a similar, flexible manner (See Figure 3, column 7). Applicants have discovered several new inhibitors or substrates of MurI which bind to the active site. The active site of MurI is not static, and indeed, is highly flexible, making it possible for a variety of types of structures to bind to it and inhibit function of the enzyme.

As shown in Figure 3, Applicants have calculated the distance in Å between the active site cysteines of MurI when different compounds are bound to the substrate binding site. For example, the distance between cysteine residues of *E*. *faecium* can range from 7.5 Å to 8.7 Å, depending on whether tartrate, citrate, or phosphate is bound.

### A. Gram negative - E. coli

The coordinates determined represent those of MurI alone, and MurI in complex with the substrate (L-glutamate), in complex with activator (UDP-MurNAc-Ala), and in complex with substrate (L-glutamate) and activator (UDP-MurNAc-Ala). Crystallization of *E*. *coli* MurI is described in Example 2 and Figures 8-11. Results show that the unit of the crystal consists of one molecule corresponding to a monomer; the native form of MurI from Gram negative bacteria is a monomer. The monomer has two domains, which both have similar alpha/beta type folds. The binding of the substrate clearly identifies the binding site that is situated between the two domains (See left side of Figure 1H). The activator, UDP-MurNAc-Ala, binds at the opposite side of the protein (See right side of Figure 1H), possibly acting as a modulator of activity by inducing the correct conformation of the binding site by modulation of the relative position of the two domains of the protein. Thus, the hinge region located between the two domains is flexible such that when activator is bound, the conformation of the protein changes at the hinge region to make the substrate binding site available.

Gram negative bacteria include, for example, Escherichia species, *Haemophilus influenzae, Klebsiella pneumoniae, Moraxella catarrhalis, Vibrio cholerae, Proteus mirablis, Pasteruella multocida, Acinetobacter baumanii, Bacteroides fragilis,* Treponemal species, Borrelial species, Deinococcal species, Pseudomonas species, Salmonella species, Shigella species, Yersinia species and *Porphyromonas gingivalis.*

The crystal structure of the *E*. *coli* MurI reveals the three dimensional structure of the binding domains formed by the atoms of the amino acid residues listed in Figure 10.

### 1. Substrate Binding Site

A further embodiment relates to a *E*. *coli* MurI in which the substrate binding site comprises two conserved cysteine residues, denoted Cys92 and Cys204 in the amino acid sequence represented as SEQ ID NO: 40. A further embodiment comprises a substrate binding site of *E*. *coli* MurI wherein the binding site additionally comprises one or more of the following amino acid residues: Ser29, Thr94, Thr135, Thr138, Glu170, Thr205 and His206 as represented by the structural coordinates of Figure 10. In one embodiment, the substrate binding site of the *E*. *coli* MurI complexes with L-glutamate and comprises amino acid residues Cys92 and Cys204 as well as amino acid residues within 5 Å of the Cys92 and Cys204 residues as represented by the structural coordinates of Figure 8. In one embodiment, the substrate binding site of the *E. coli* MurI additionally comprises one or more of the following amino acid residues: Ser29, Thr94, Thr135, Thr138, Glu170, Thr205 and His206. In this embodiment, the substrate binding site complexes with L-glutamate and comprises amino acid residues Cys92 and Cys204 and at least one (i.e., one or more) of the following amino acid residues: Ser29, Thr94, Thr135, Thr138, Glu170, Thr205 and His206, which can be present in any combination. In a further embodiment, the substrate binding site includes amino acid residues Phe27, Asp28, Ser29, Gly30, Va131, Gly32, Gly33, Ser35, Va136, Asp54, Ala57, Ala57, Phe58, Pro59, Tyr60, Gly61, Glu62, Lys63, IIe68, Va190, Ala91, Cys92, Asn93, Thr94, Ala95, Ser96, Thr97, Val114, Va1115, Leu133, Ala134, Thr135, Arg136, Gly137, Thr138, Va1139, Thr144, Ala163, Va1166, Glu167, Glu170, Leu202, Gly203, Cys204, Thr205, His206, Phe207 and Ser227.

In a further embodiment, the substrate binding site of the MurI comprises two hydrogen bond TRIADs, which occur close to the conserved cysteine residues of the binding site. Specifically, on one side (Cys204) of the binding site, the TRIAD is Glu170-Thr20S-His206 and on the other side (Cys92), the TRIAD is Thr94-Thr135-Thr138. Thus, in a specific embodiment, the substrate binding site of *E. coli* MurI complexes with L-glutamate and comprises the relative structural coordinates of amino acid residues Cys92 and Cys204 and additionally comprises the relative structural coordinates of at least one (i.e., one or more) of the following: amino acid residues Ser29, Thr94, Thr135, Thr138, Glu170, Thr205 and His206.

The threonine residues have features of interest as they relate to the binding site of the *E*. *coli* MurI. On one side (Cys204) of the substrate binding site, Thr205 is H-bonded to His206 and His206 is further H-bonded to Glu170. The hydroxyl oxygen (O) of Thr205 is 2.8 Å away from the amino nitrogen (N) of the substrate (i.e., glutamate) and 5.3 Å from the sulfur (S) atom of Cys204, which is 4.3 Å from His206. On the other side of the substrate binding site (Cys92), Thr94 is H-bonded to Thr135 and further H-bonded to Thr138. The hydroxyl O of Thr94 is H-bonded to one of the carboxylate oxygen atoms of the substrate and is 3.3 Å away from the S atom of Cys92. Analysis showed that the three hydroxyl oxygens form a triangle, all within less than 3.2 Å from one another.

The two TRIADs may play important roles in altering the pKa of the two substrate binding site cysteine residues (in addition to that of the neighboring hydrophobic core), facilitating the proton transfer during catalysis or both.

Another embodiment is a crystal of *E*. *coli* MurI, comprising a substrate binding site comprising the amino acid residues Cys92, Cys204, Ser29, Thr94, Thr135, Thr138, Glu170, Thr205 and His206. This crystal may also comprise MurI complexed with L-glutamate. Additionally, the substrate binding site of the crystal complexed with L-glutamate can comprise the amino acid residues: Cys92, Cys204, and one or more of the following amino acid residues: Ser29, Thr94, Thr135, Thr138, Glu170, Thr205 and His206. A further embodiment is one in which the substrate binding site additionally comprises at least one of the following amino acid residues: Asp28, Gly30, Val131, Gly32, Gly33, Ala57, Phe58, Pro59, Tyr60, Gly61, Glu62, Lys63, Ala91, Asn93, Ala95, Val114, Ala134, Val166, Gly203 and Phe207. A further embodiment is one in which the substrate binding site additionally comprises at least one of the following amino acid residues: Phe27, Ser35, Val36, Tyr51, Asp54, De68, Val90, Ser96, Thr97, Val115, Leu133, Thr135, Arg136, Gly137, Val139, Thr144, Ala163, Glu167, Leu202, and Ser227. The crystals comprising substrate binding sites encompassed by the above amino acid residues comprise the amino acid sequence is that represented by SEQ ID NO: 40; or an amino acid sequence is at least 75%, 80%, 85%, 90%, 95%, 98%, or 99% homologous to the amino acid sequence that is represented by SEQ ID NO: 40.

Location and geometry of the substrate binding site of the enzyme are also defined. Two conserved cysteine (Cys) residues are identified as the residues responsible for the (de)protonation of the alpha-carbon of the substrate during catalysis, which is consistent with the two-base mechanism proposed for function of the enzyme in its role as a racemase. The two substrate binding site cysteines are Cys92 and Cys204, which are about 7.6 Angstroms (Å) apart (Cα-Cα distance, i.e., the distance between Ca atoms). Other amino acid residues identified in the vicinity of the substrate binding site include Ser29, Thr94, Thr135, Thr138, Glu170, Thr205 and His206. The bound substrate/product, L-glutamate, is located between the two conserved cysteine residues. Further detail of the binding site is as follows: looking down the axis defined by the Glu170 along the γ and α carbon, the two cysteines exhibit a rather symmetrical environment; each has a hydrophobic core with respect to the substrate and a neighboring threonine residue not far from the substrate C-alpha (4.5 - 3.4 Å), respectively.

Analysis of the crystal structure of the *E*. *coli* MurI indicates that the following amino acid residues are within 10 Å of the bound L-glutamate: 27-36, 51-63, 69, 52, 90-98, 227-228, 205-208, 133-139, 141, 114-118, 144, 58, 60, 61, and 65. Analysis of the crystal structure also shows that the following amino acid residues are within 4 Å of the bound L-glutamate: 28-29, 58-61, 92-94, 135, 138, and 204-206. Of interest is the fact that only one acidic amino acid residue (Asp28) is present in the structure surrounding the substrate binding site Cys92, which serves as an anchoring point for the amino group of the substrate/product with a polar (charged) interaction between the amino nitrogen atom of L-glutamate and the delta oxygen atom of Asp28.

### 2. Activator Binding Site

One embodiment relates to an activator binding site of *E*. *coli* MurI. As used herein, the term "activator binding site" refers to a specific region (or atom) of MurI that interacts with an activator, such as LTDP-MurNAc-Ala. In certain embodiments, a binding site may comprise, or be defined by, the three dimensional arrangement of one or more amino acid residues within a folded polypeptide.

A further embodiment relates to *E*. *coli* MurI in which the activator binding site comprises amino acid residues Arg104, Gly113, Val114, Val115, Pro116, Lys119, Ser227, Ala230, Ile231, Arg233, and Arg234 of SEQ ID NO: 40. A further embodiment comprises an activator binding site of *E*. *coli* MurI wherein the binding site additionally comprises at least one (i.e., one or more) of the following amino acid residues: Leu100, Va1112, Ala117 and Pro120 as represented by the structural coordinates of Figure 8. In a further embodiment, the activator binding site comprises at least one (i.e., one or more) of the following amino acid residues: Thr97, Pro101, Ala102, Glu105, Lys106, Phe107, Asp108, Phe109, Pro110, Val1 11, Gly113, Ile118, Arg123, Leu124, Ser142, Tyr143, Thr144, Glu146, Leu147, Arg150, Phe151, Asp226, Gly228, Ala229, Trp237, Leu238, Glu240 and His241.

### 3. Intradomain Binding Site

In one embodiment, *E*. *coli* Murl has an intradomain dimer interface in which the two domains of the monomer interact. The intradomain dimer interface of *E. coli* MurI comprises at least one of amino acid residues: Asp28, Ser29, Gly30, Va131, Gly32, Gly33, Leu34, Ser35, Val36, Asp38, Glu39, His42, Leu43, Val56, Ala57, Phe58, Pro59, Tyr60, Gly61, Glu62, Lys63, Ser64, Glu65, Ala66, Phe67, Ile68, Ala91, Cys92, Asn93, Thr94, Ala95, Ser96, Thr97, Va198, Va1114, Ala230, Ile231, Ala232, Arg233, Arg234, Trp237, Leu238, Pro261, Gly262, Gin265, Leu266, Pro268, Va1269, Leu270, Arg272, and Tyr273 of a first domain, and amino acid residues Va1115, Pro116. Ala117, Ile118, Lys119, Pro120, A1a121, Arg123, Leu124, Thr135, Arg136, Gly137, Thr138, Va1139, Lys140, Arg141, Ser142, Tyr143, Thr144, Glu146, Leu147, Arg150, Ser162, Ala163, Va1166, Gly167, Ala169, Glu170, Ala171, Lys172, Leu173, His174, Va1201, Leu202, Gly203, Cys204, Thr205, His206, Phe207, Pro208, Leu209, Leu210, Val225, Asp226, Ser227, Gly228, and Leu229 of a second domain, in which the amino acid residues are represented by SEQ ID NO: 40.

### B. Gram positive

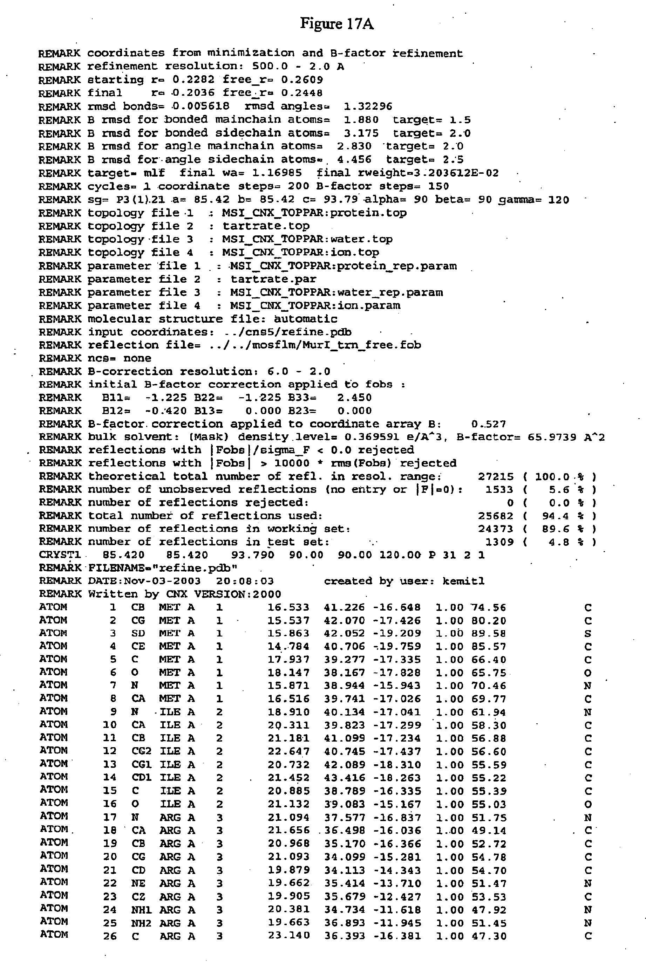

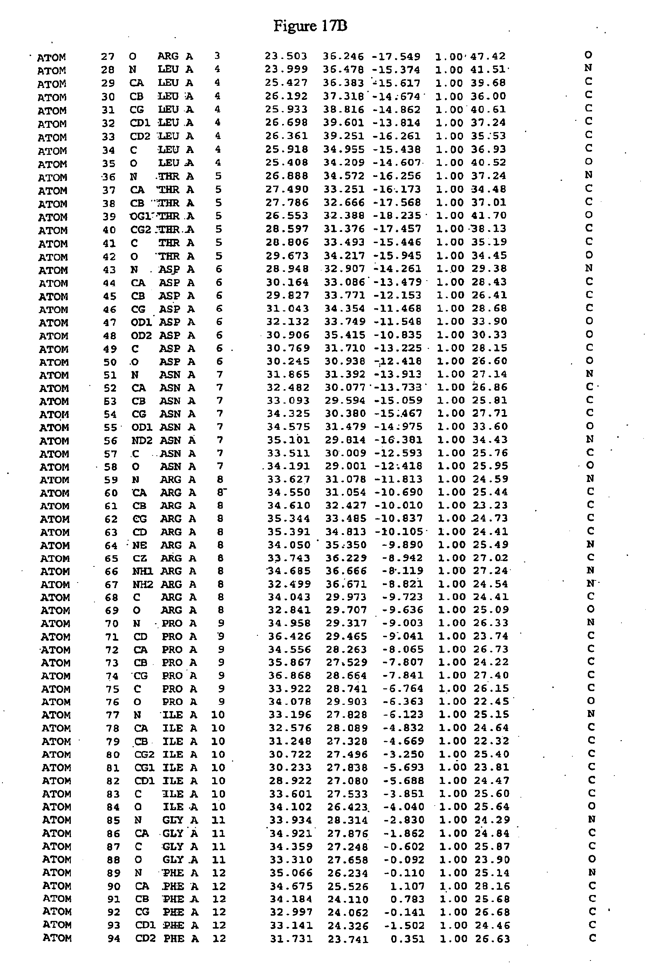

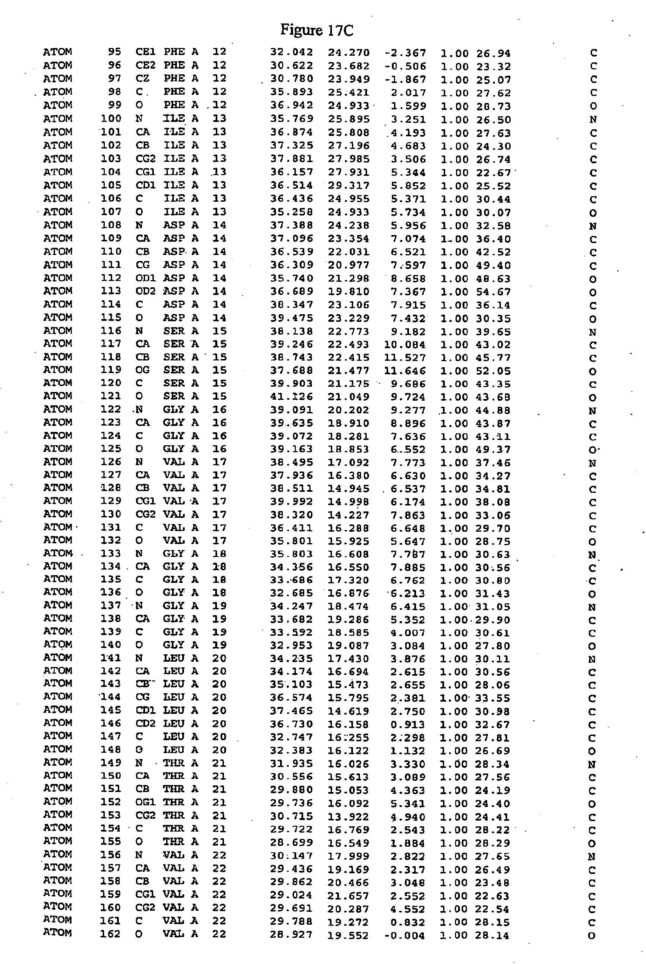

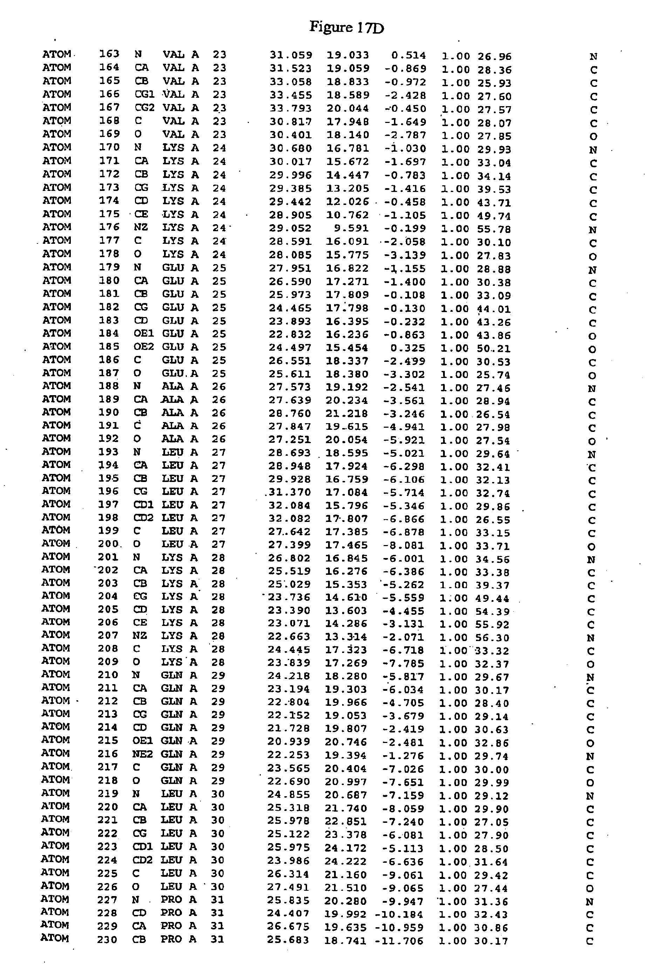

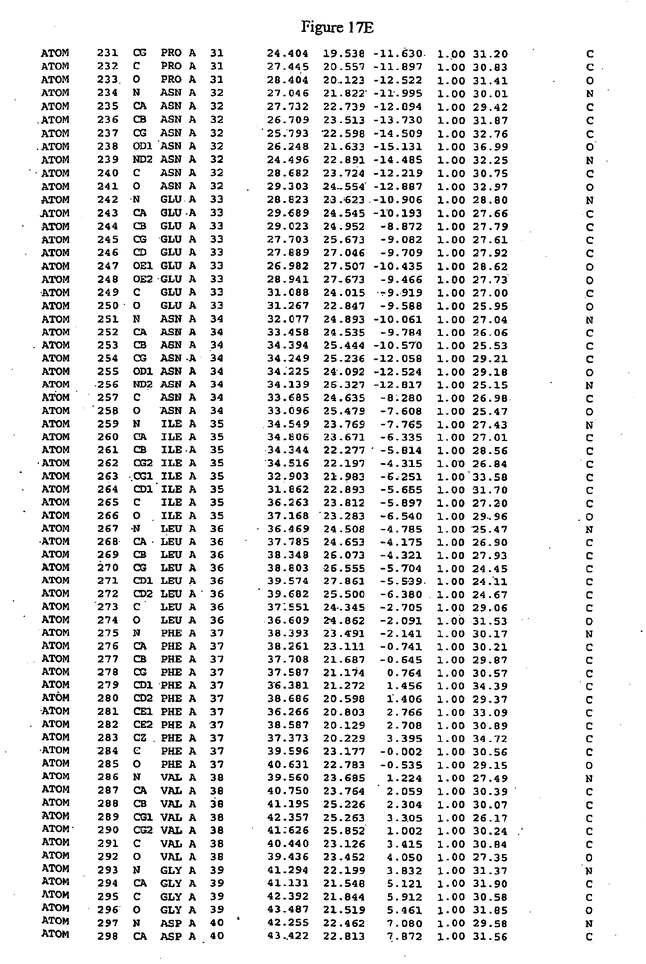

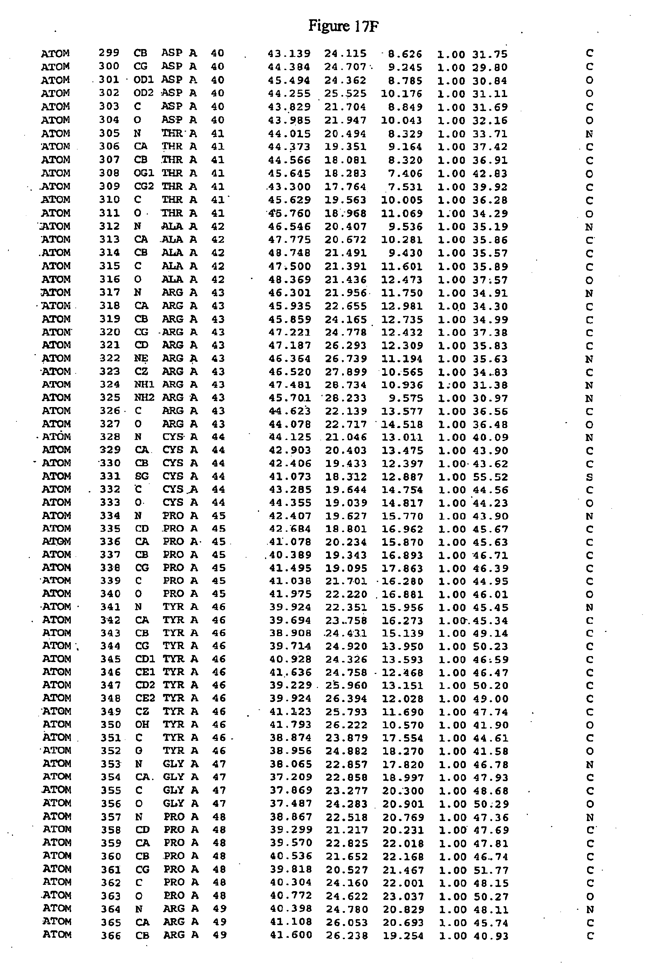

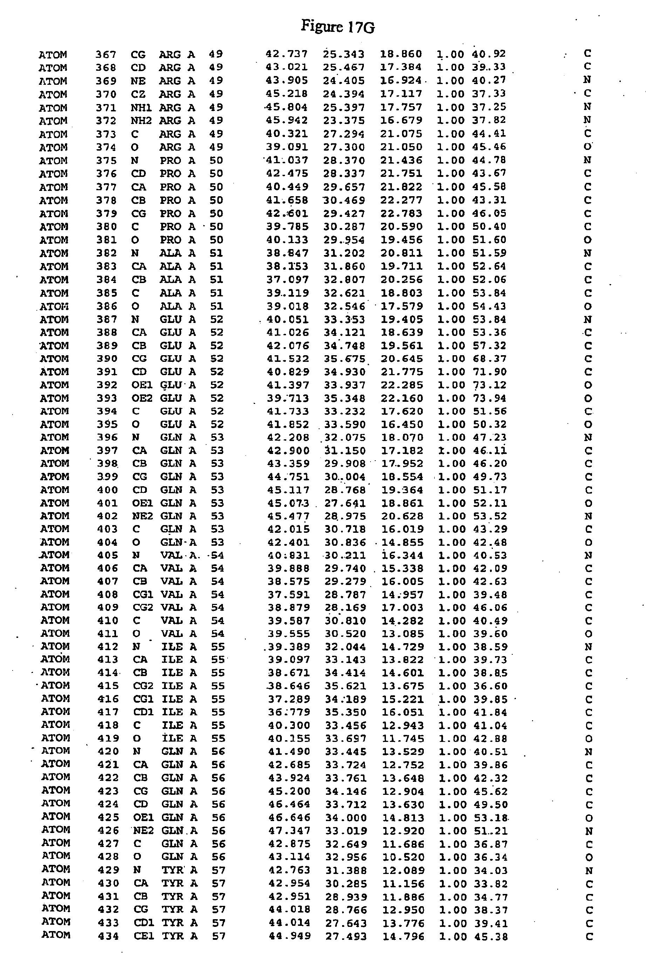

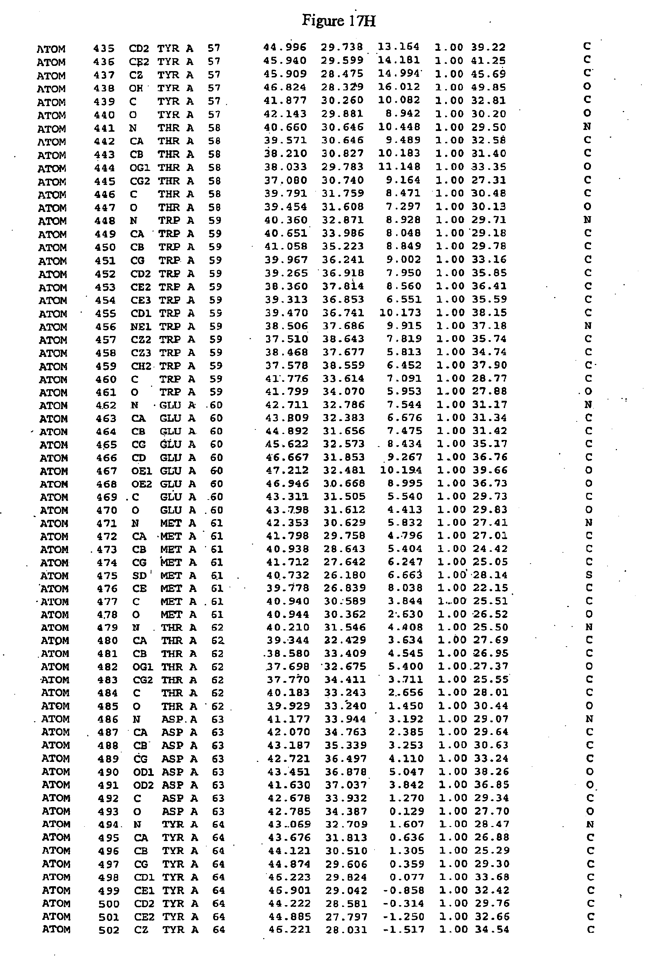

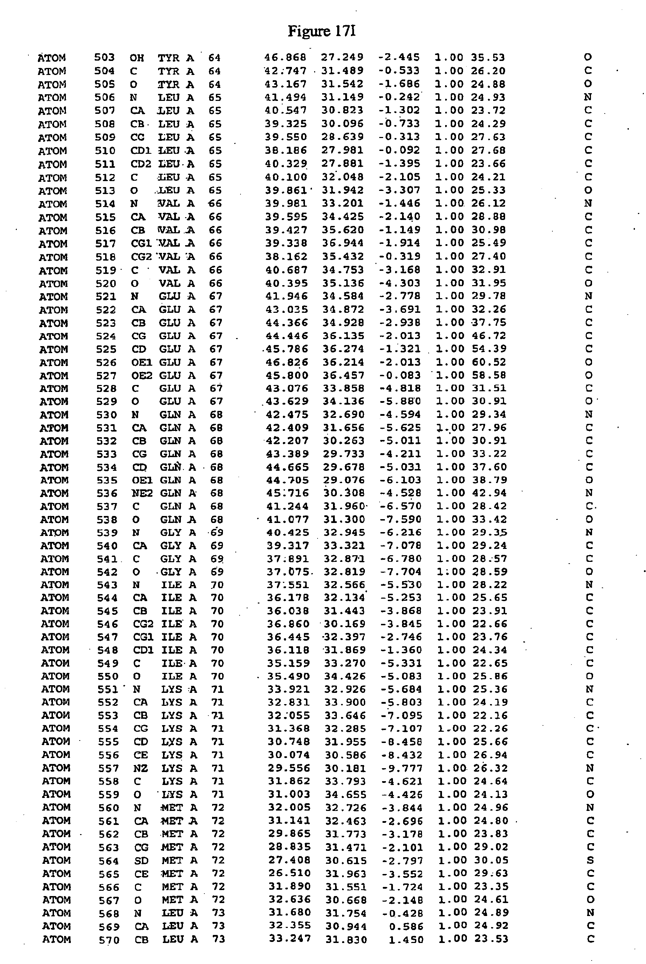

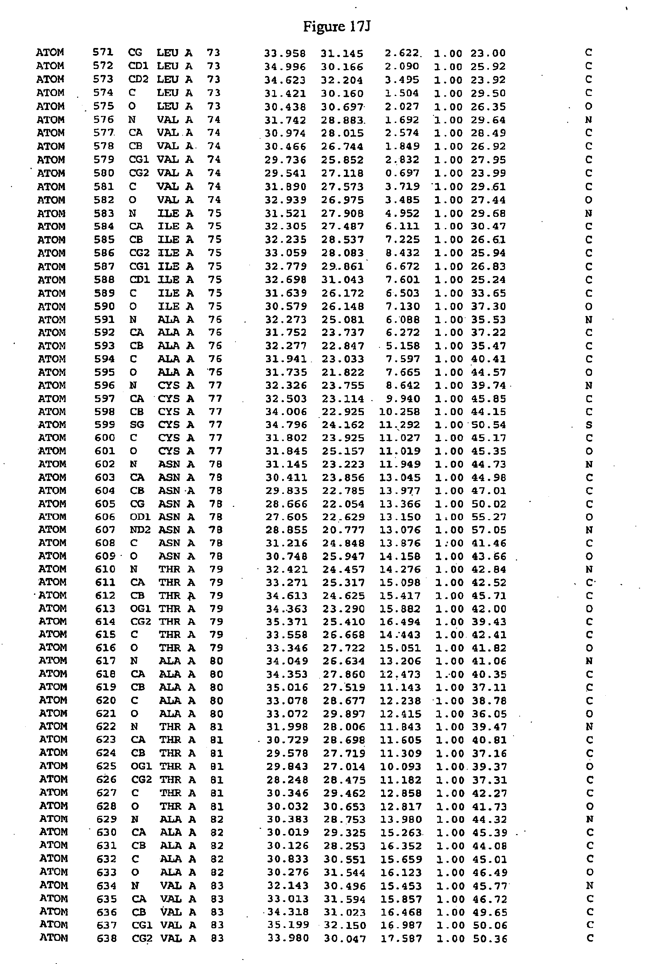

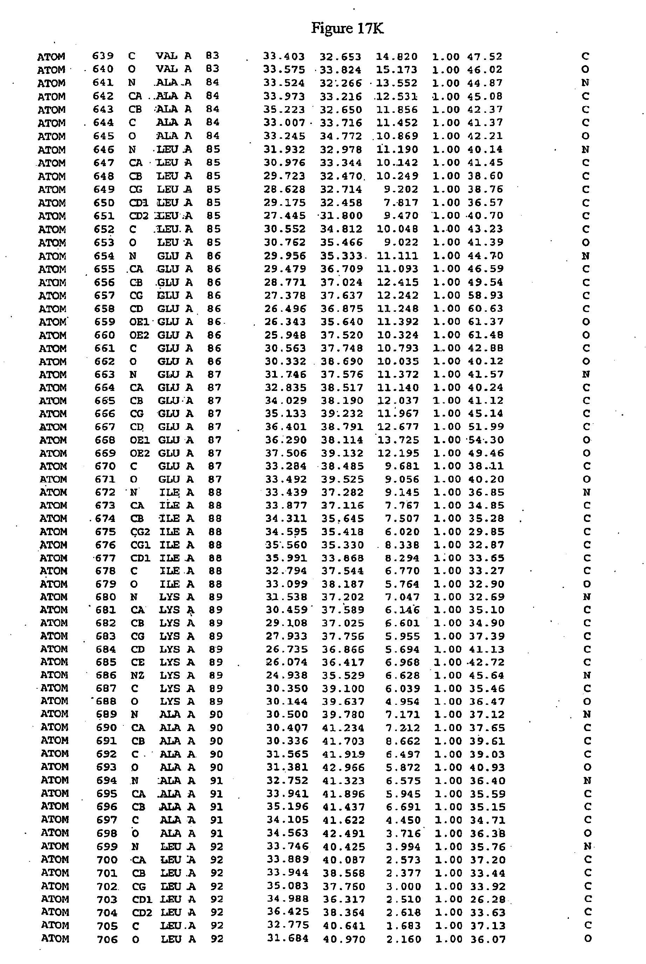

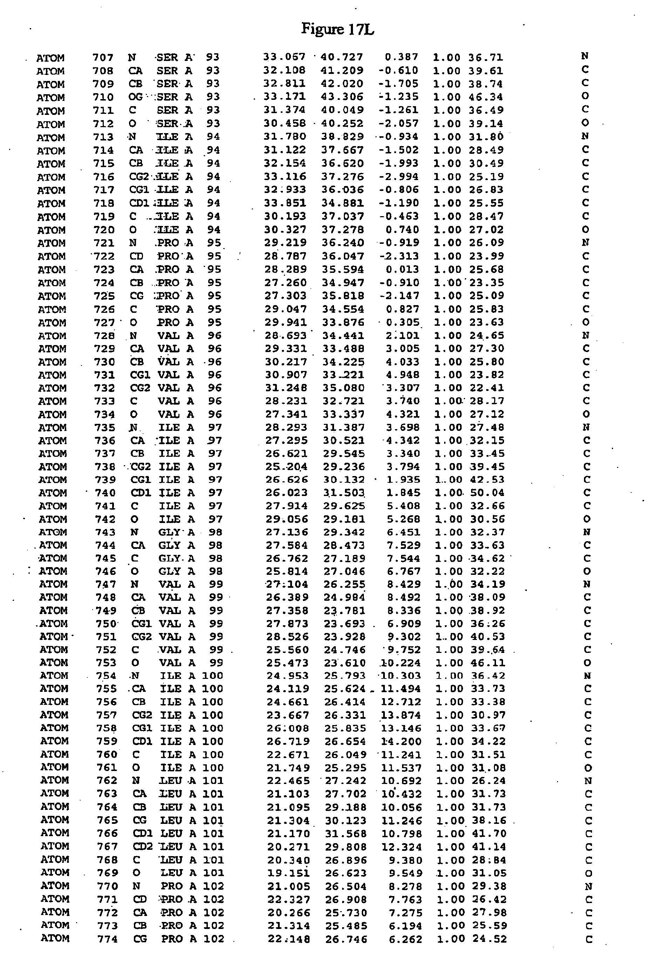

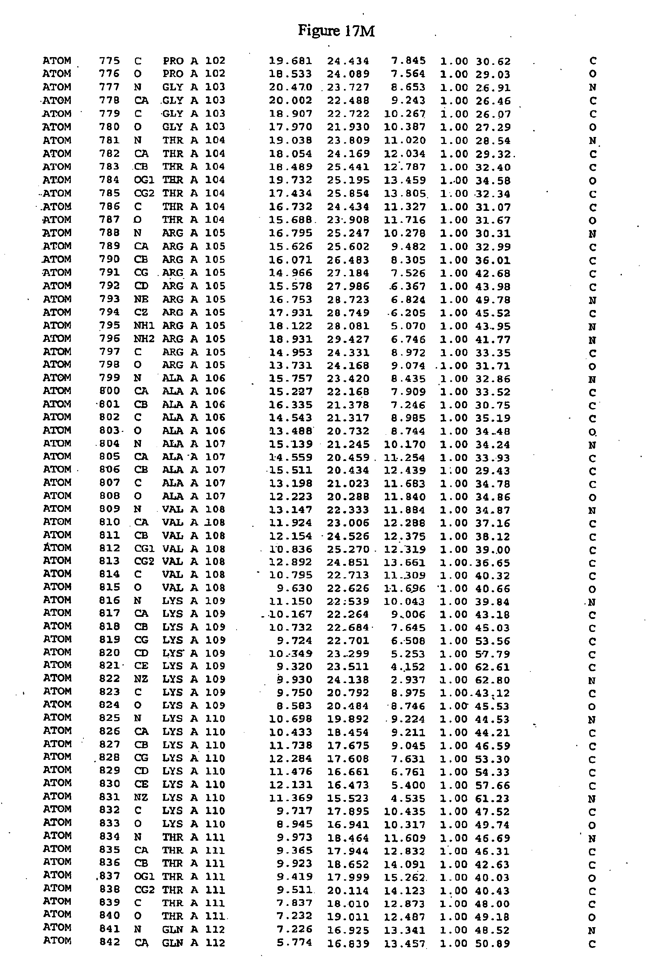

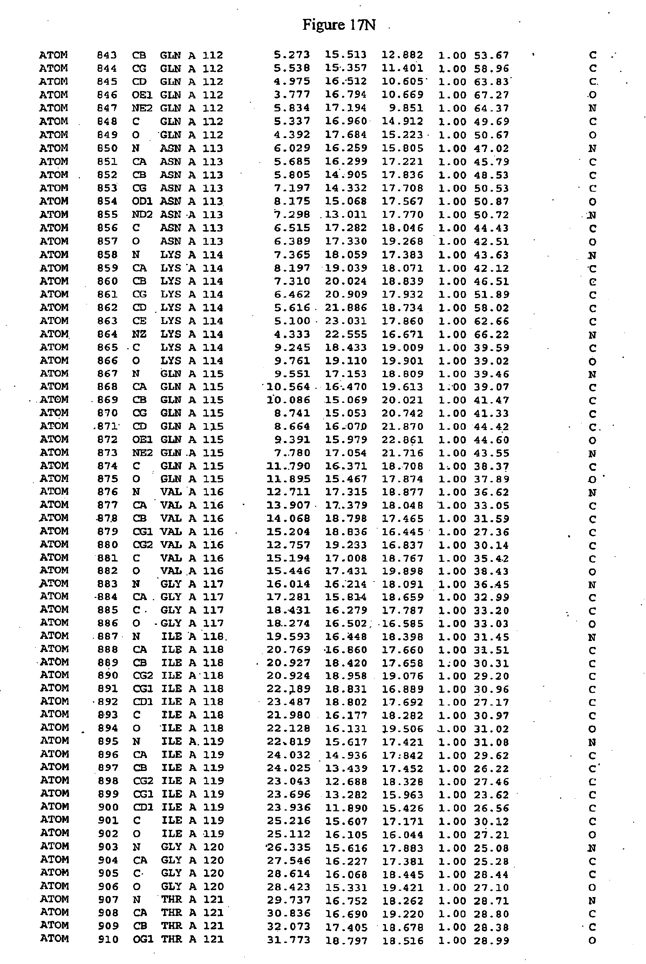

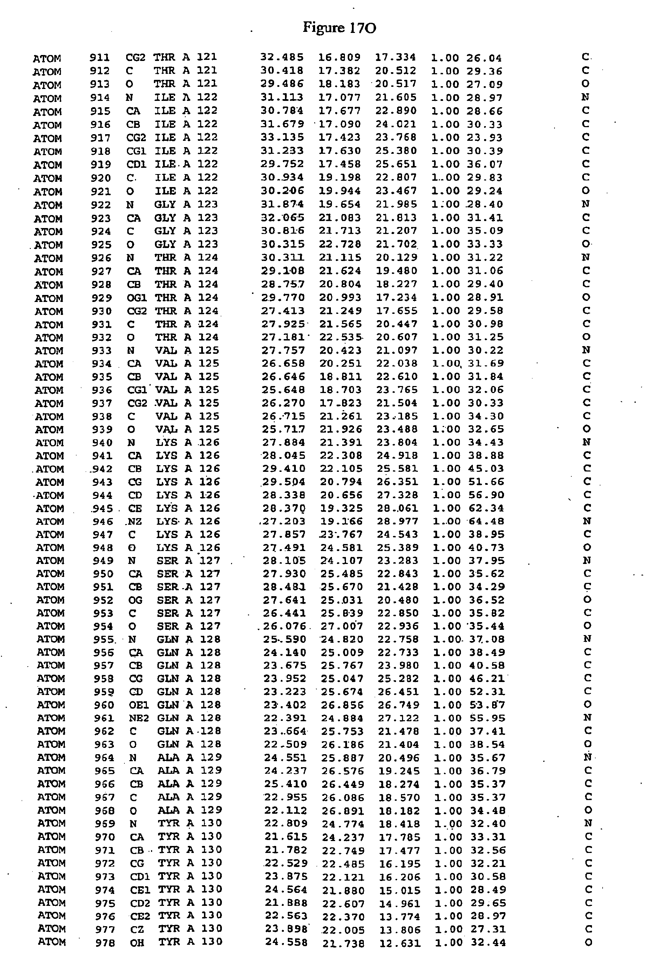

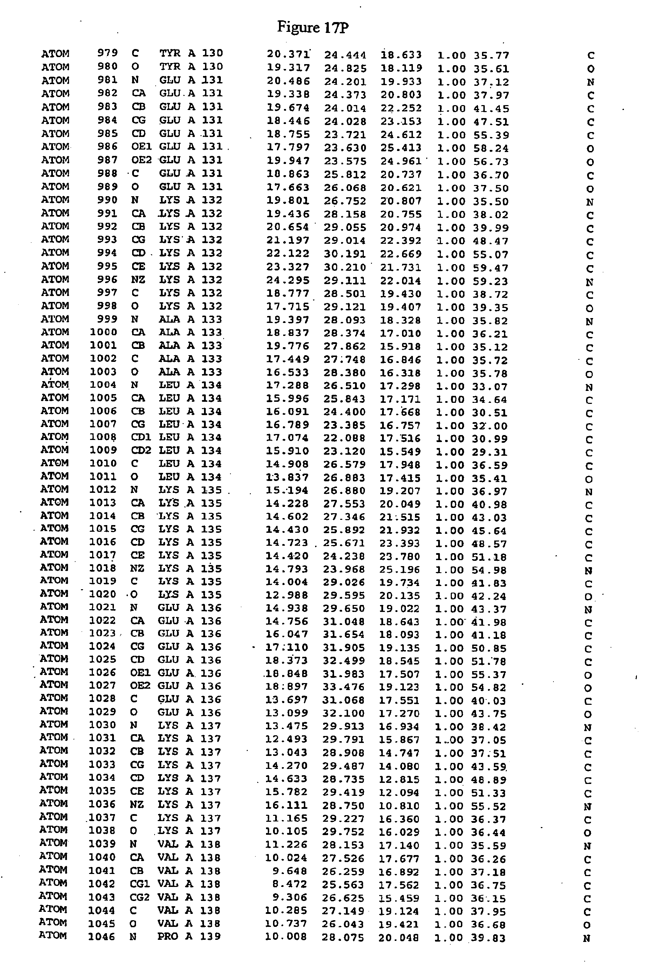

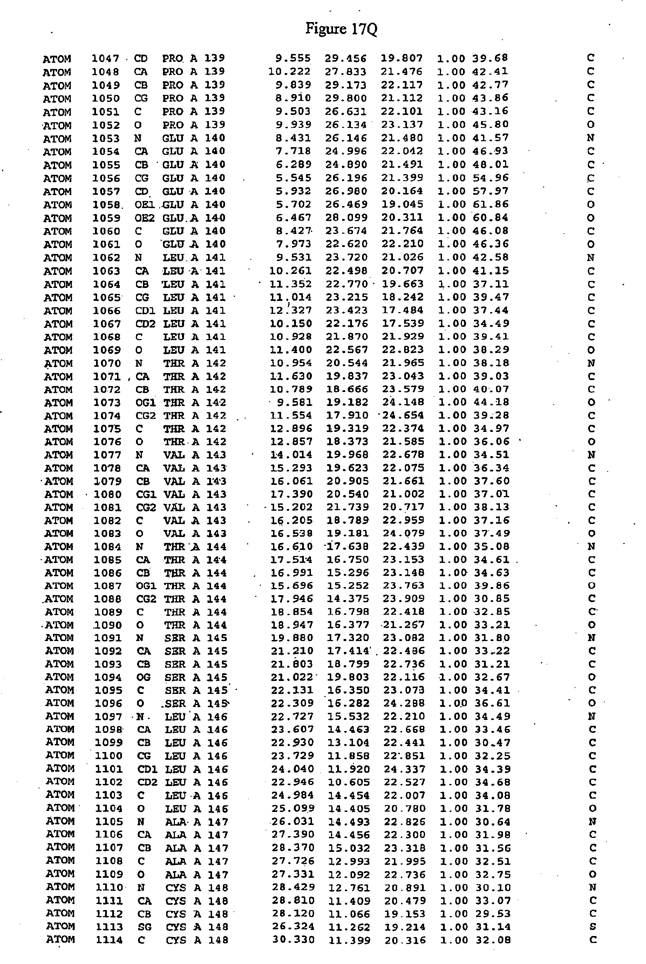

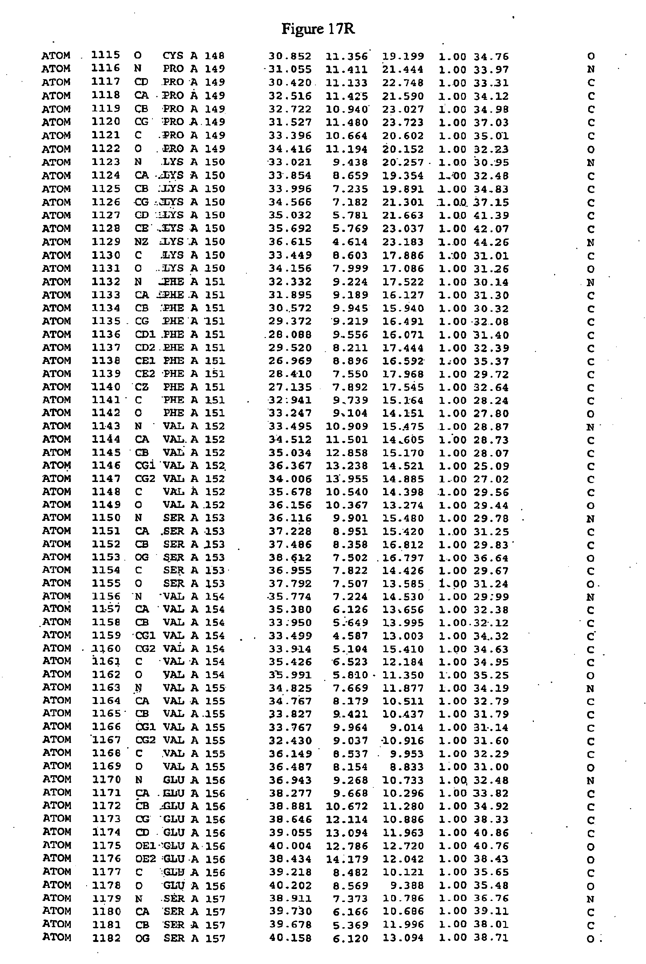

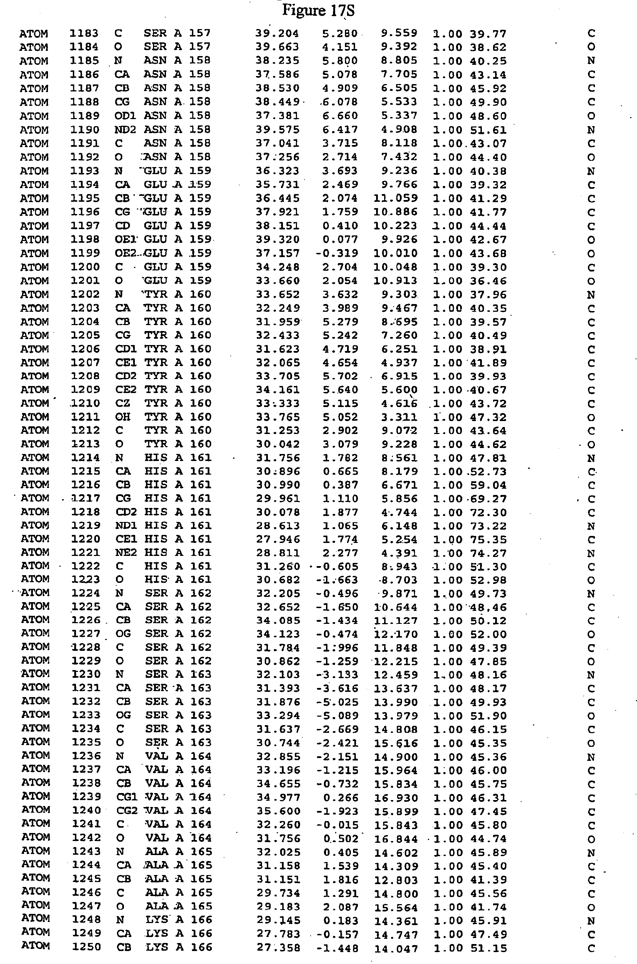

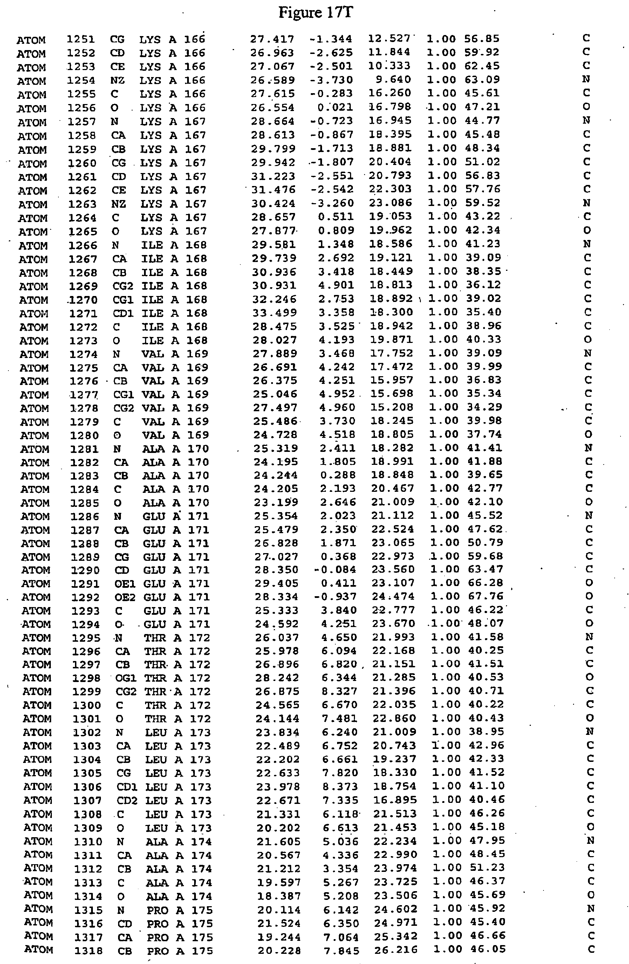

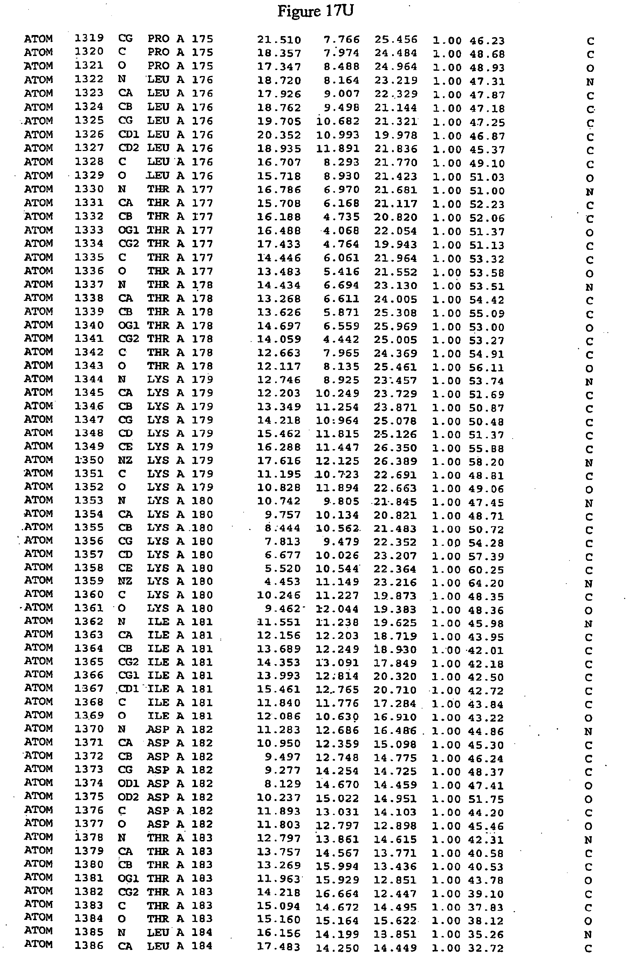

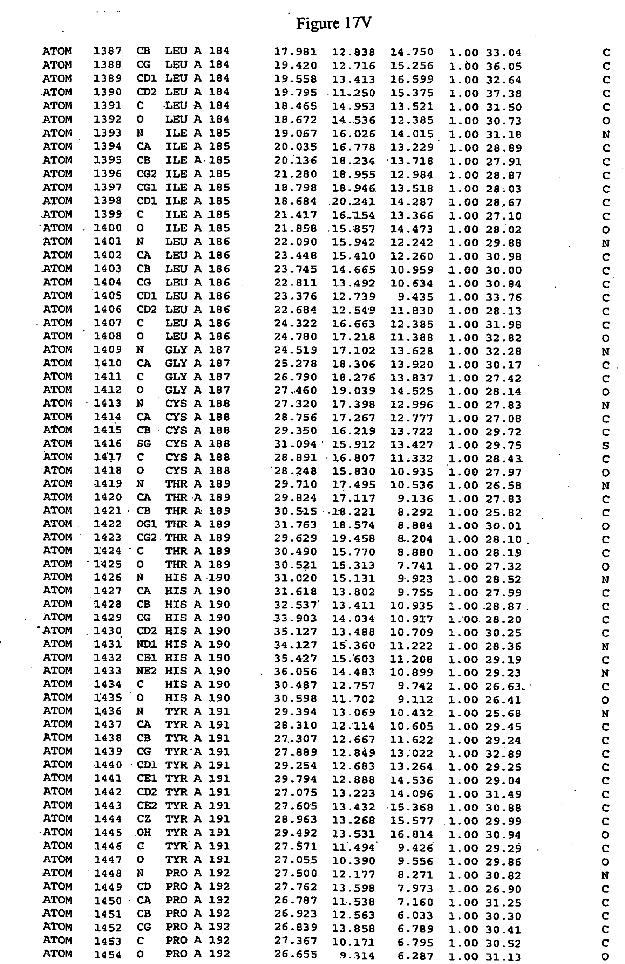

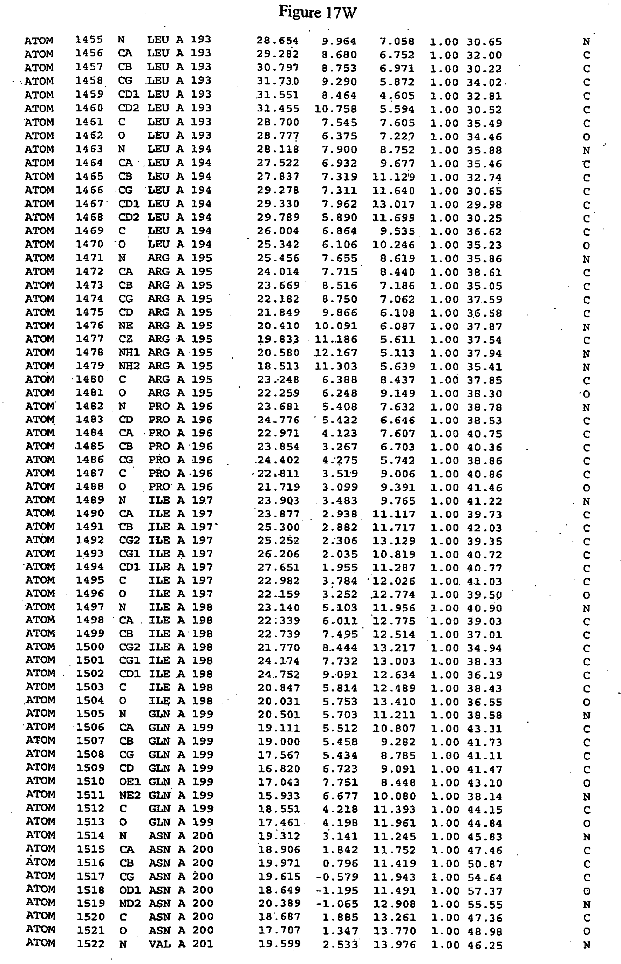

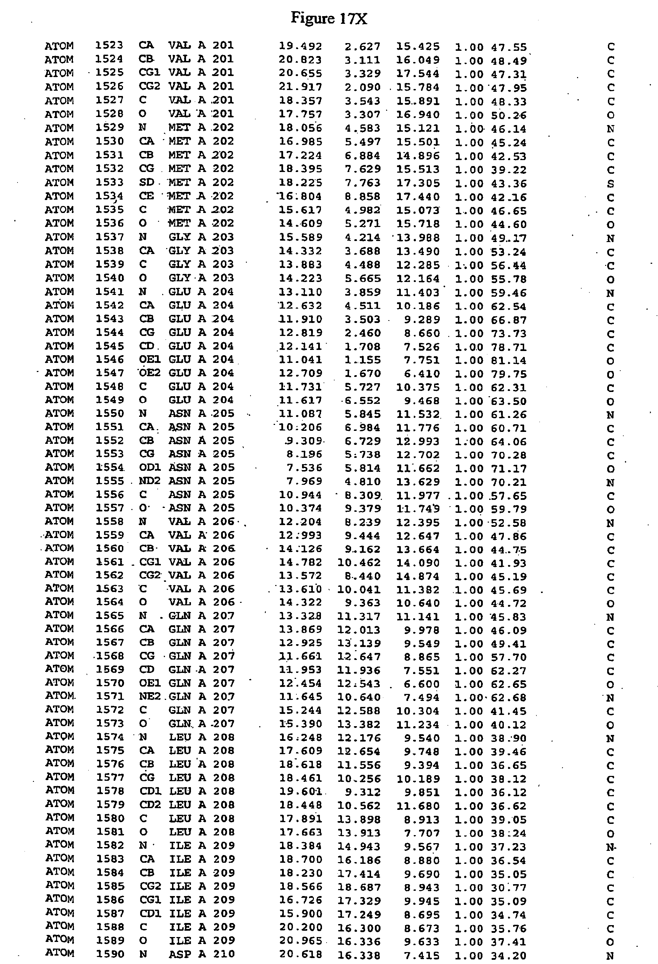

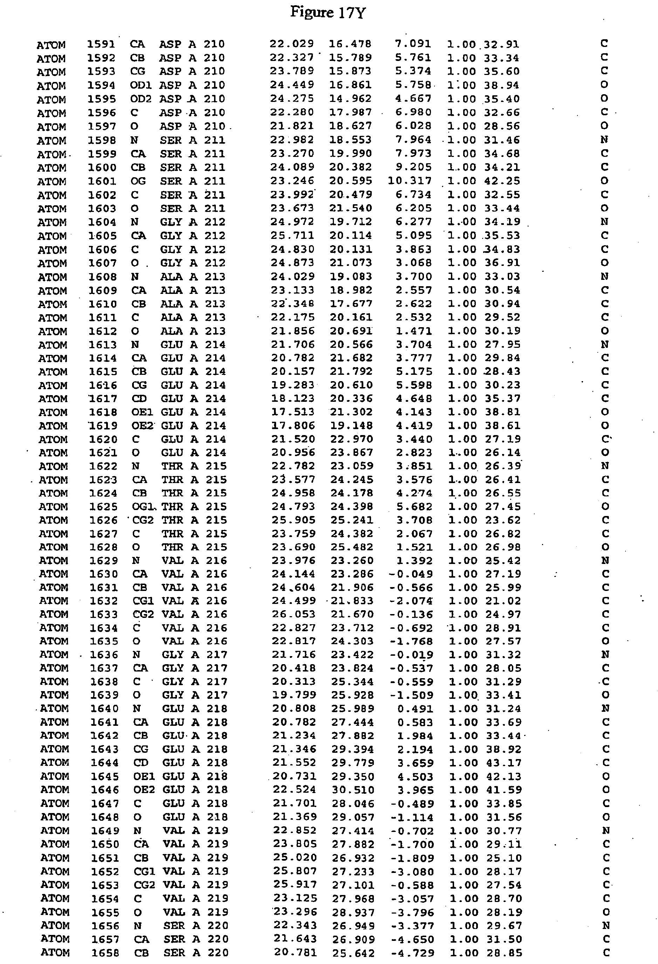

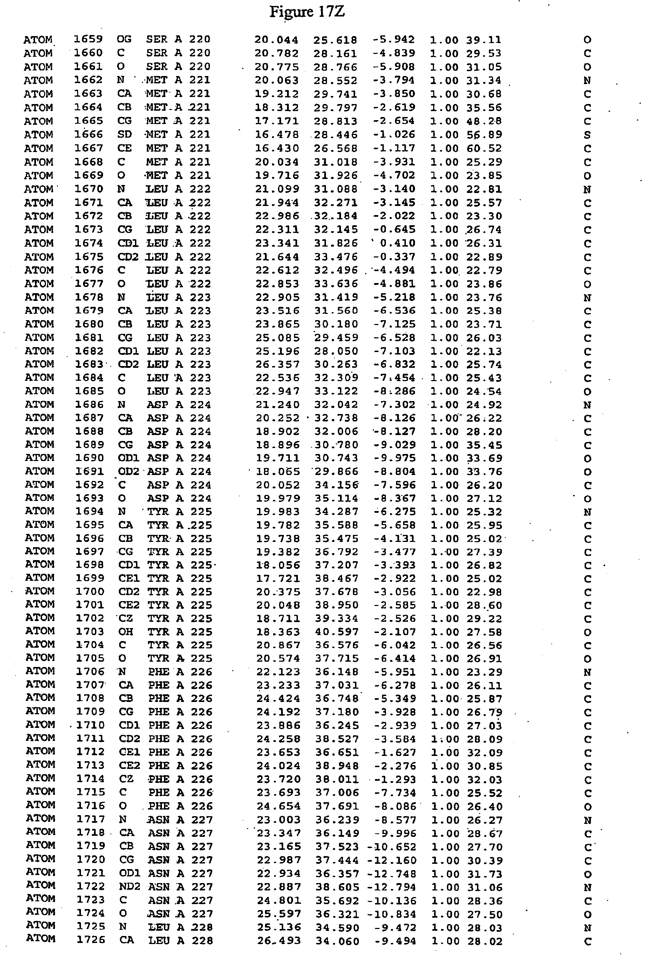

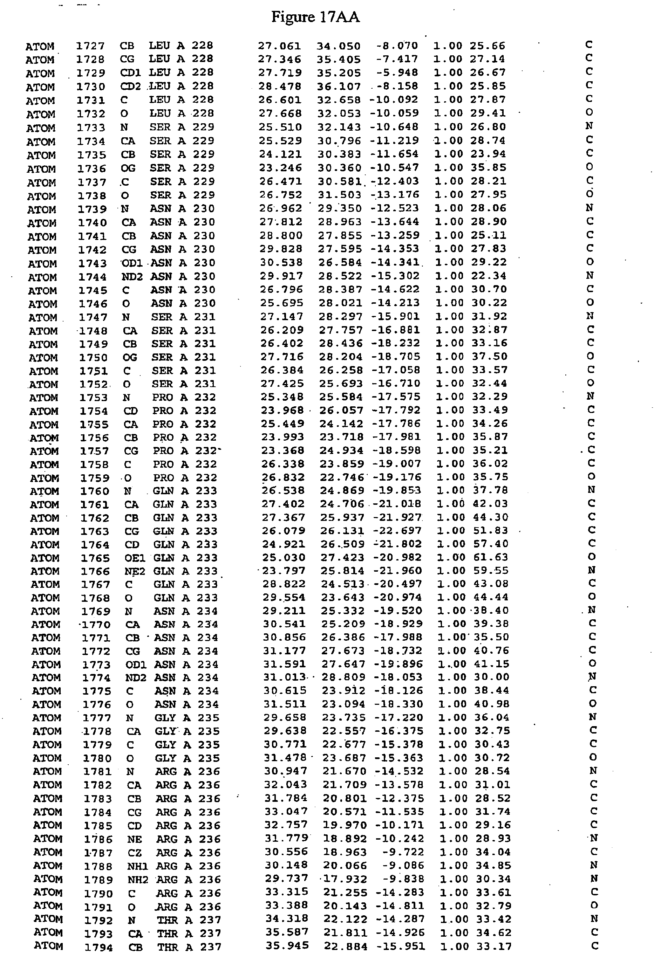

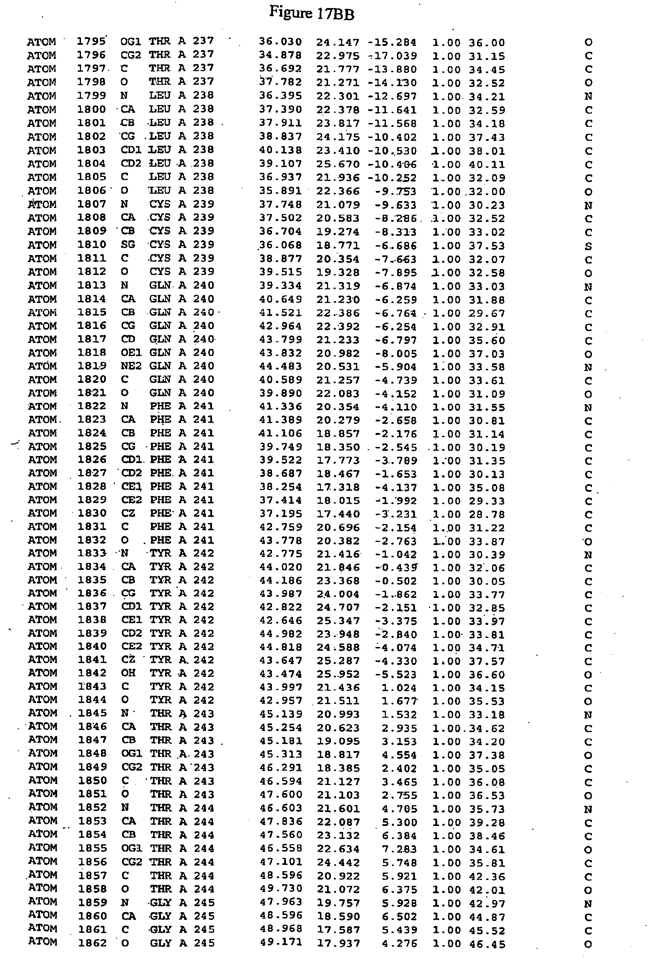

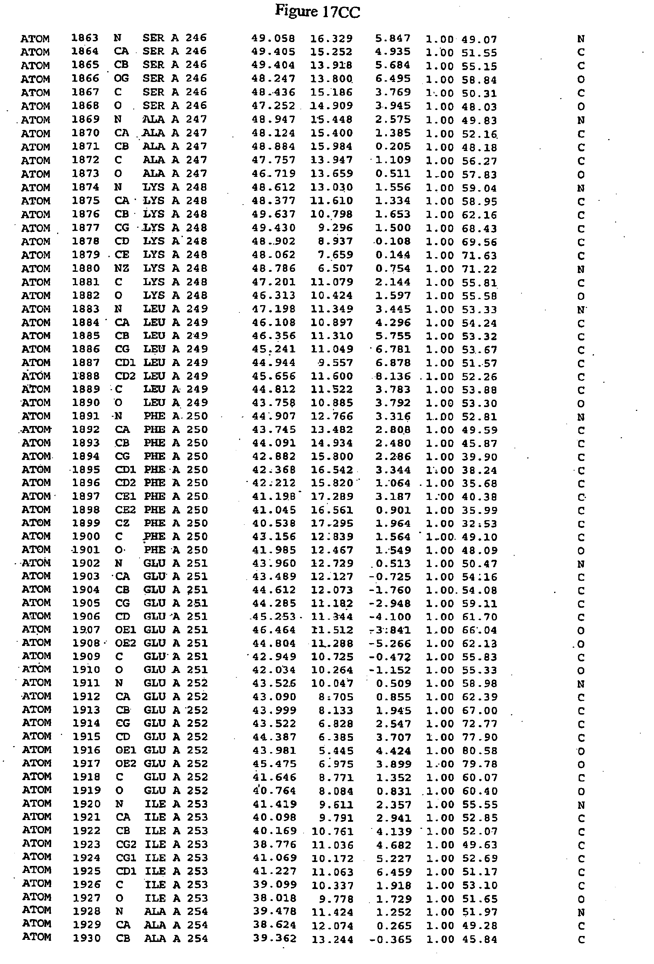

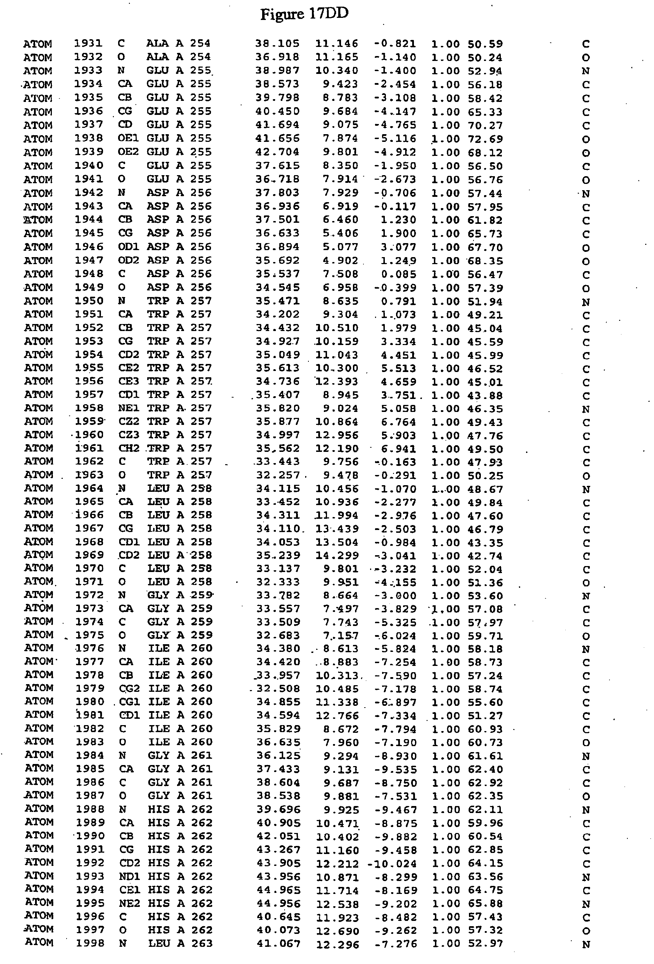

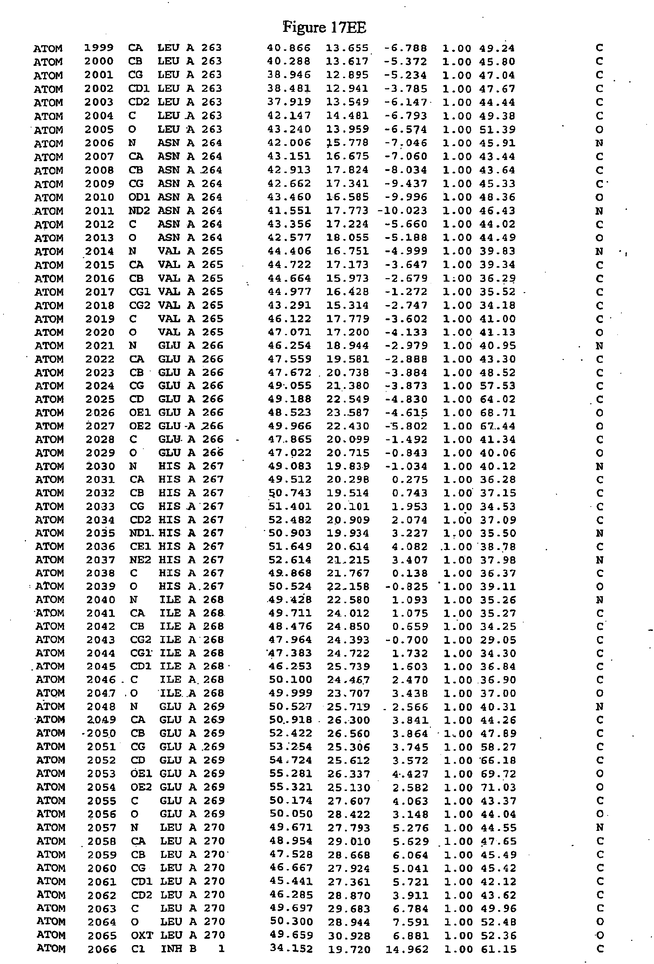

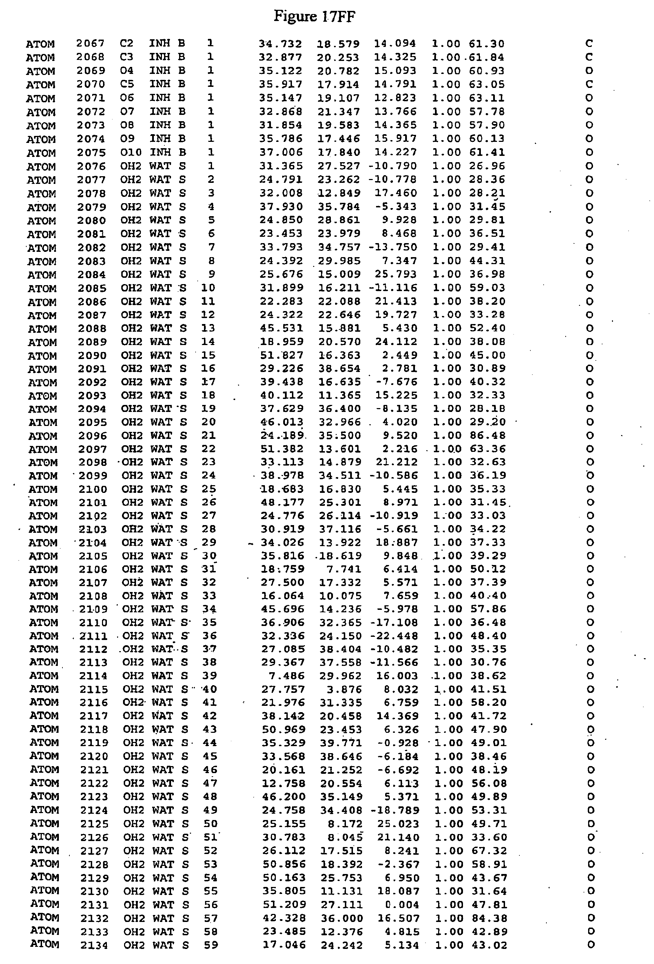

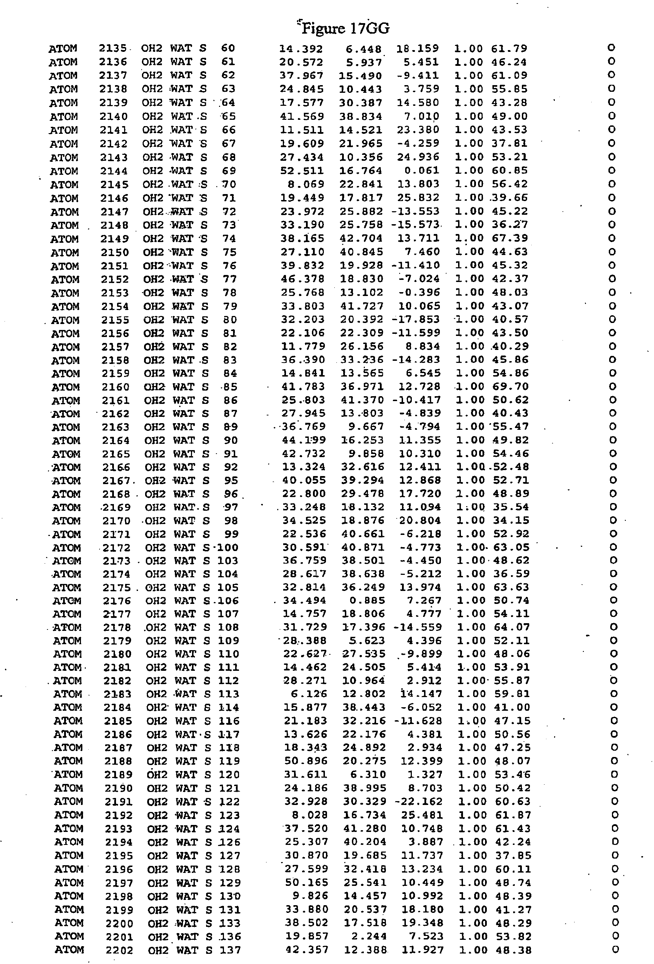

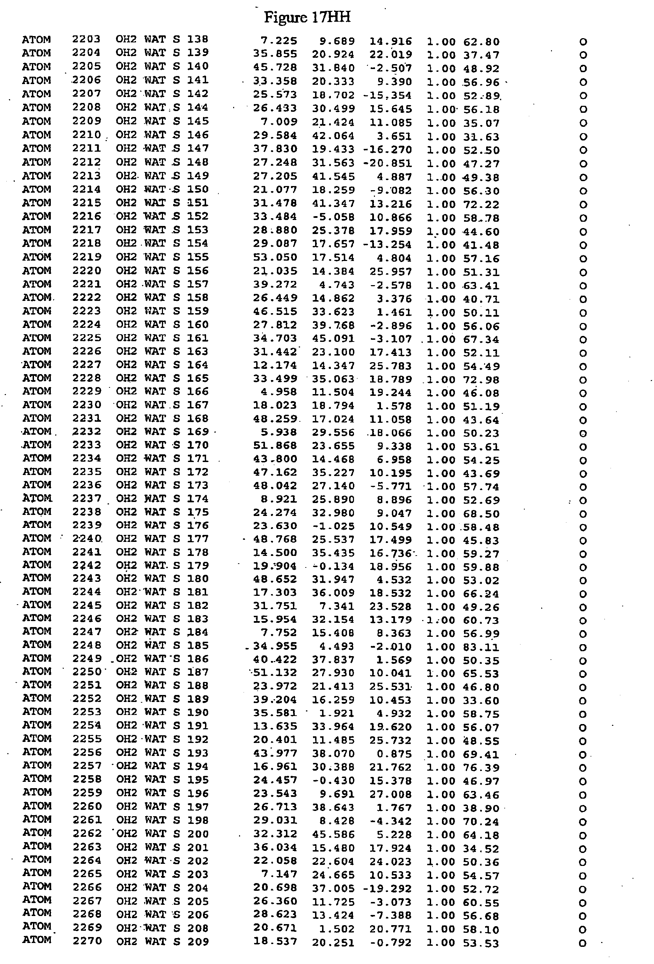

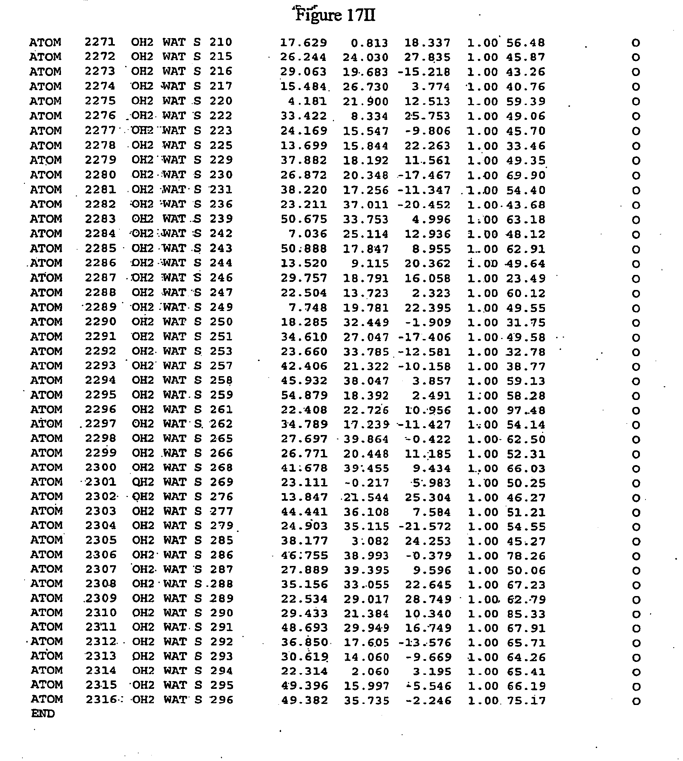

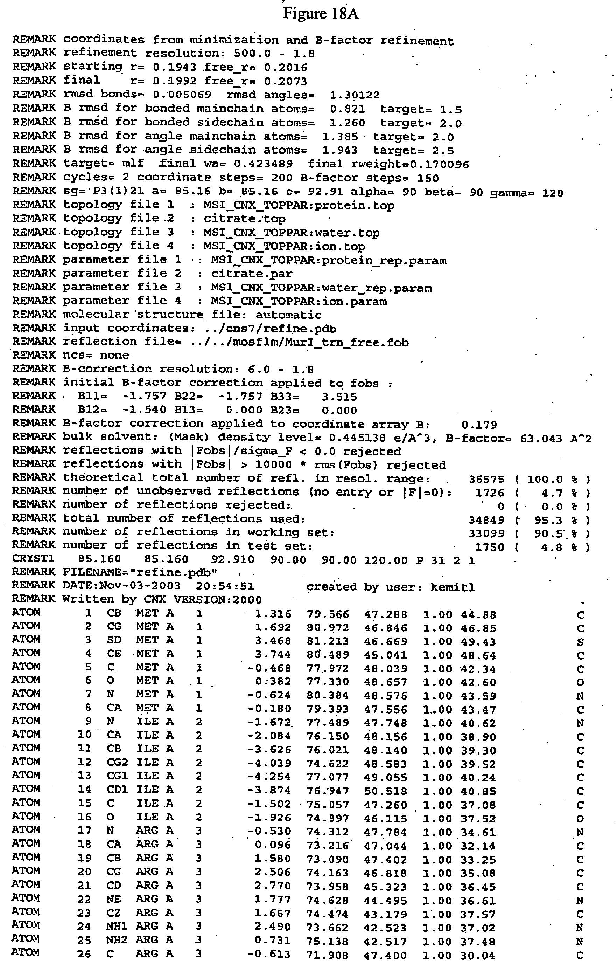

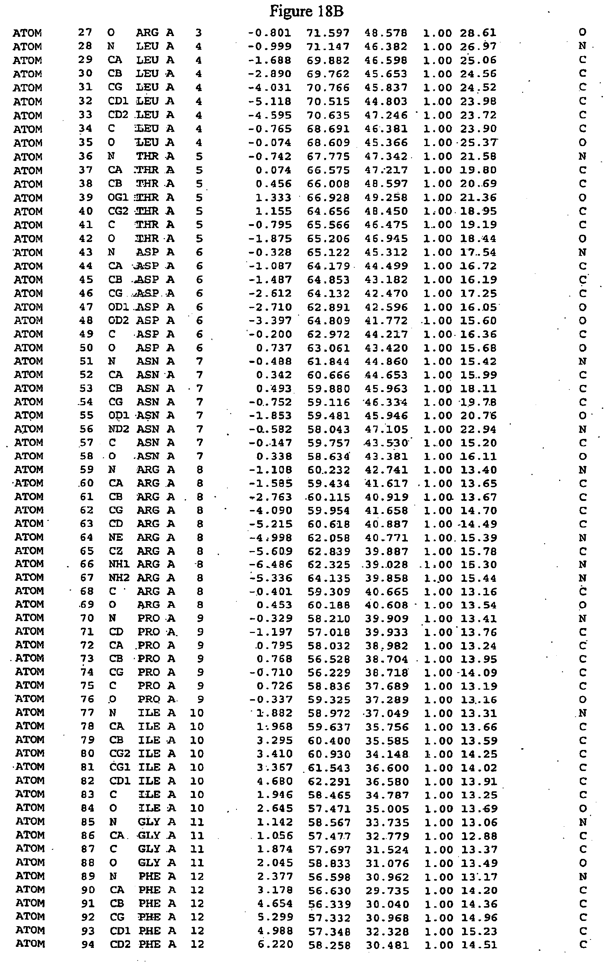

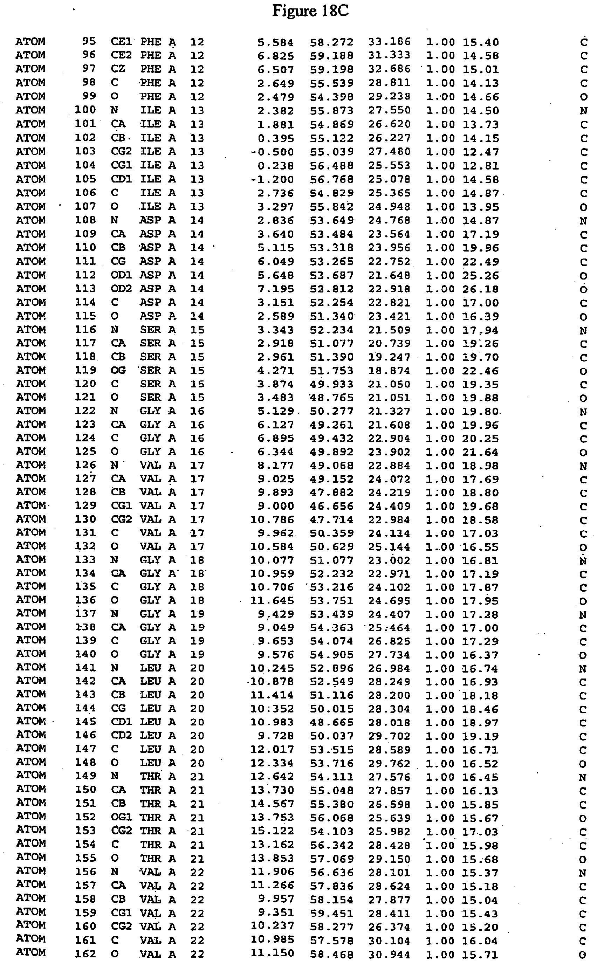

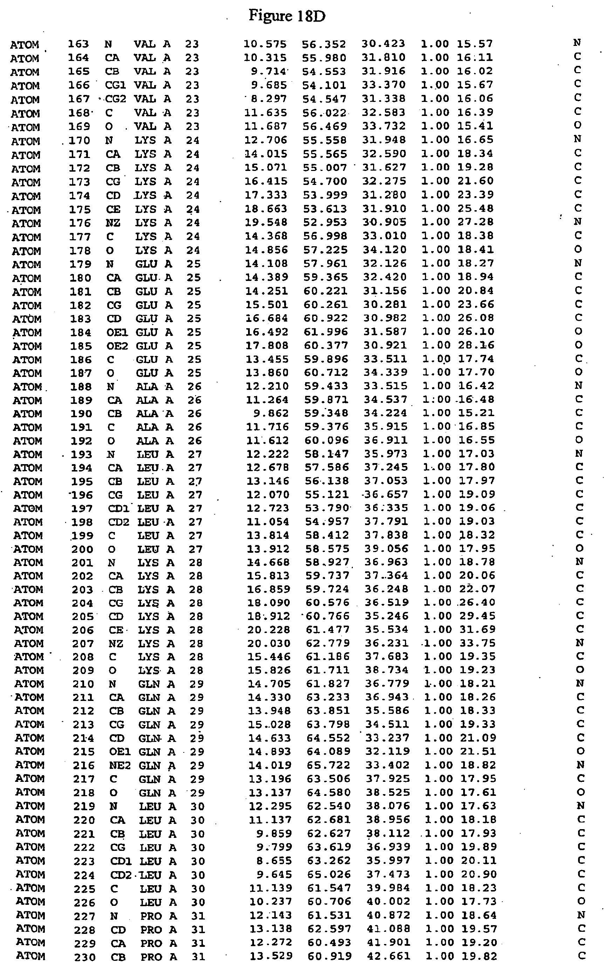

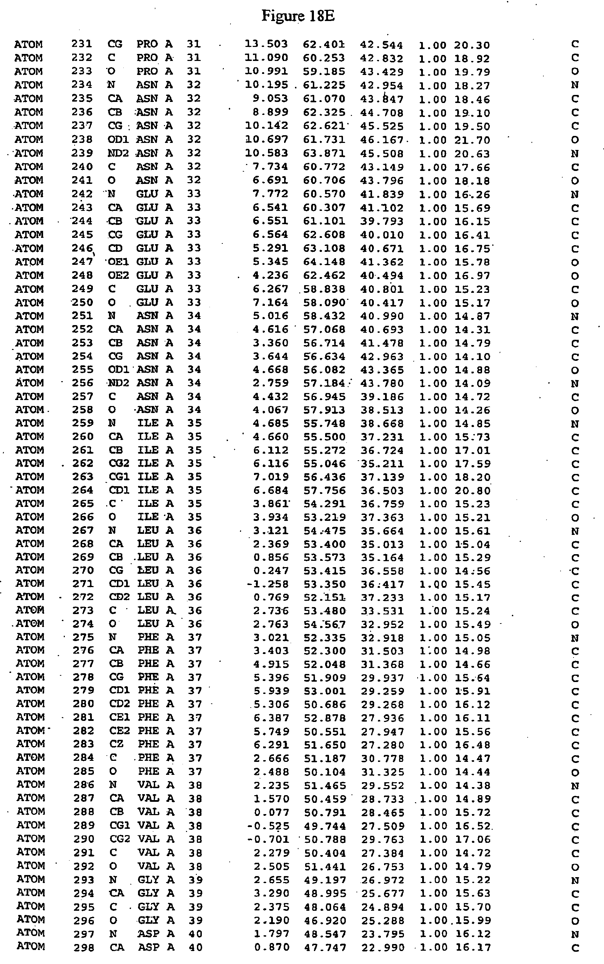

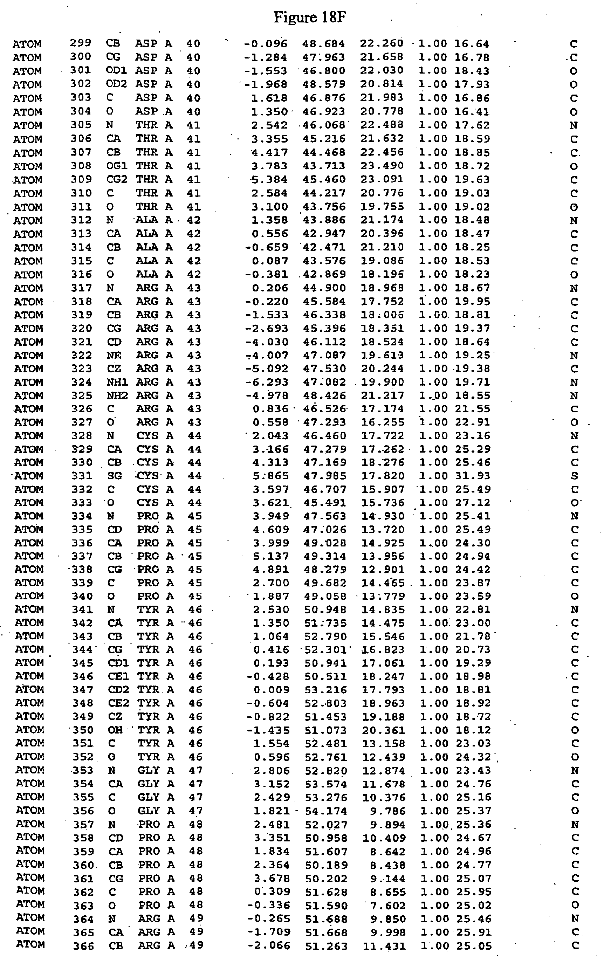

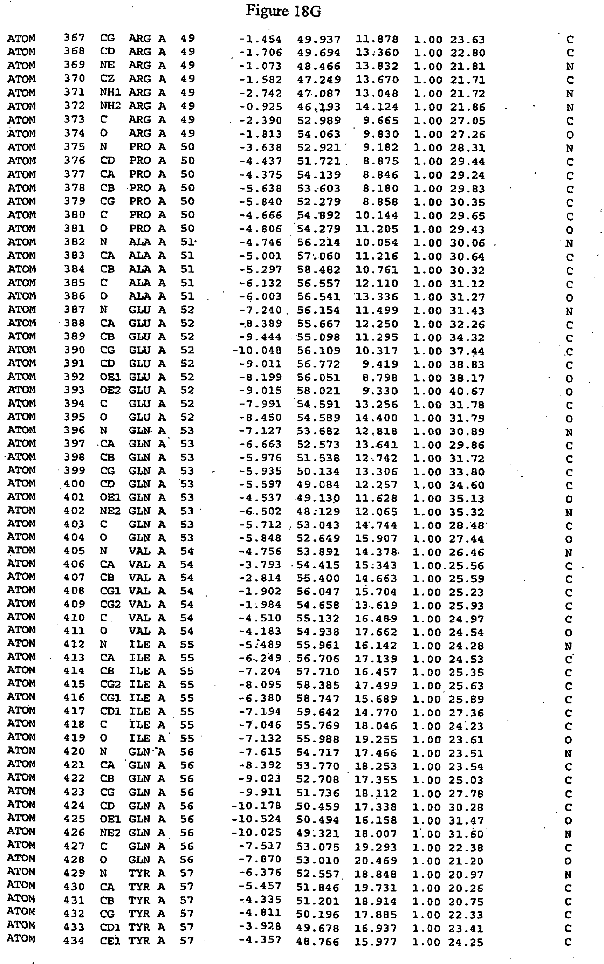

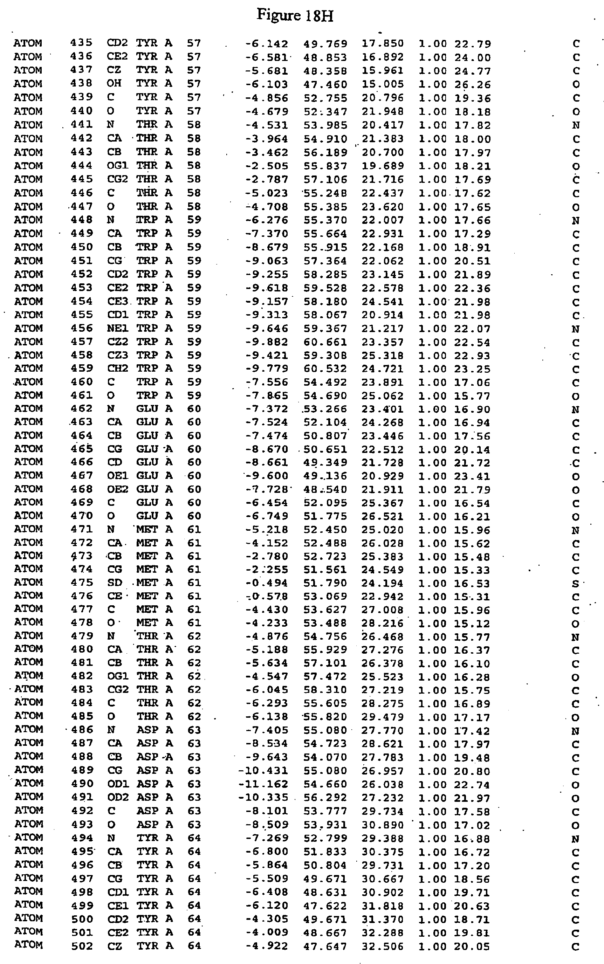

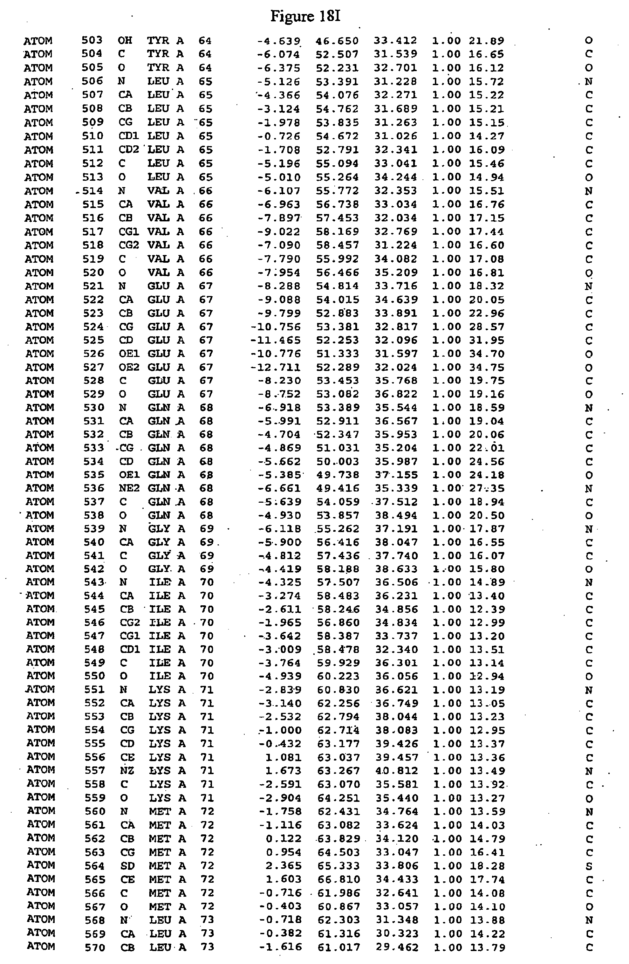

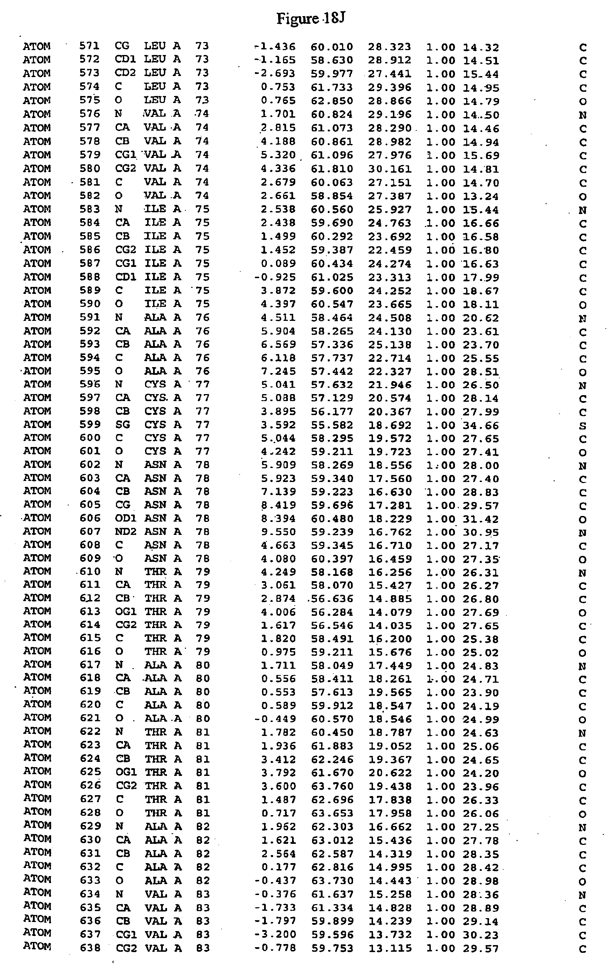

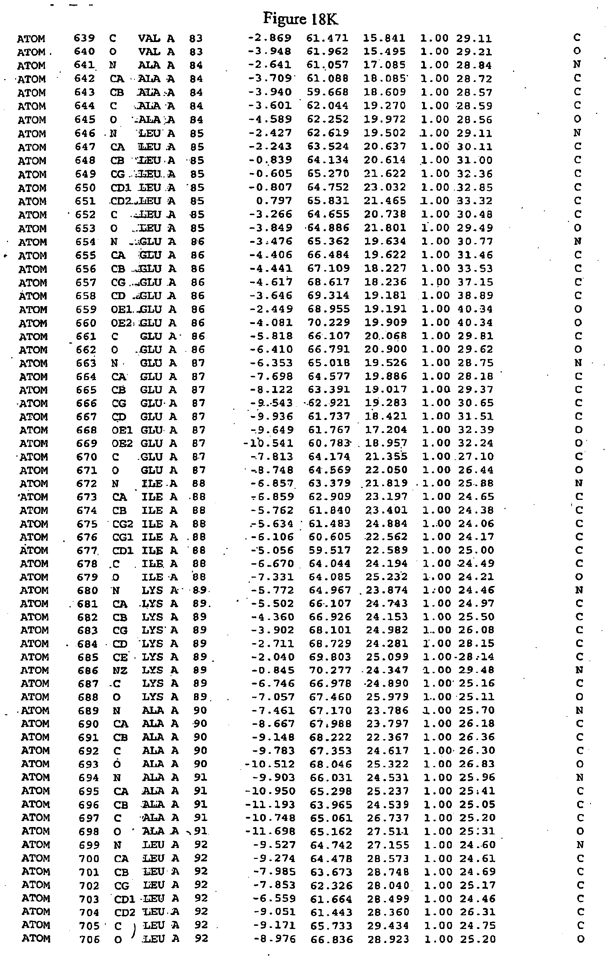

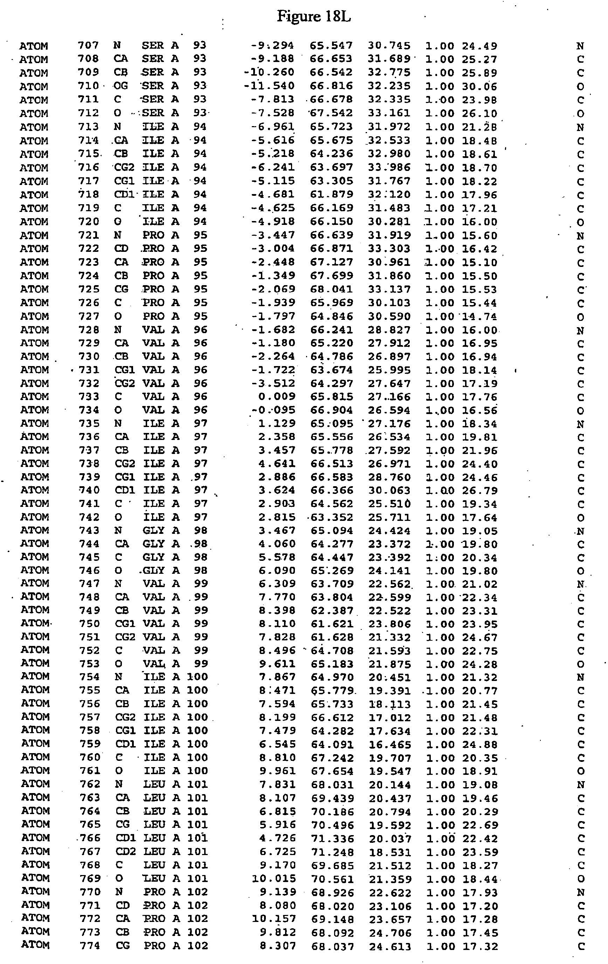

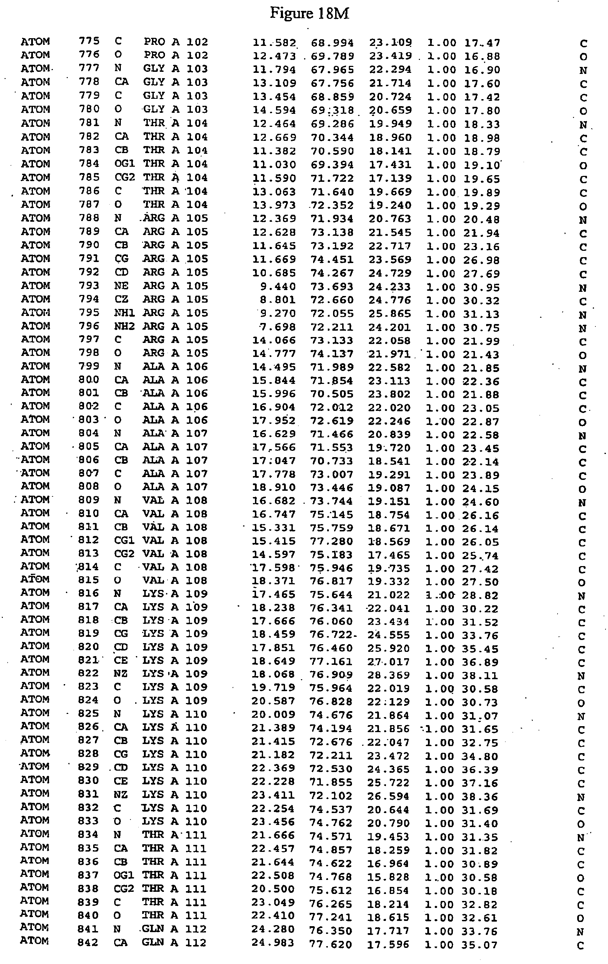

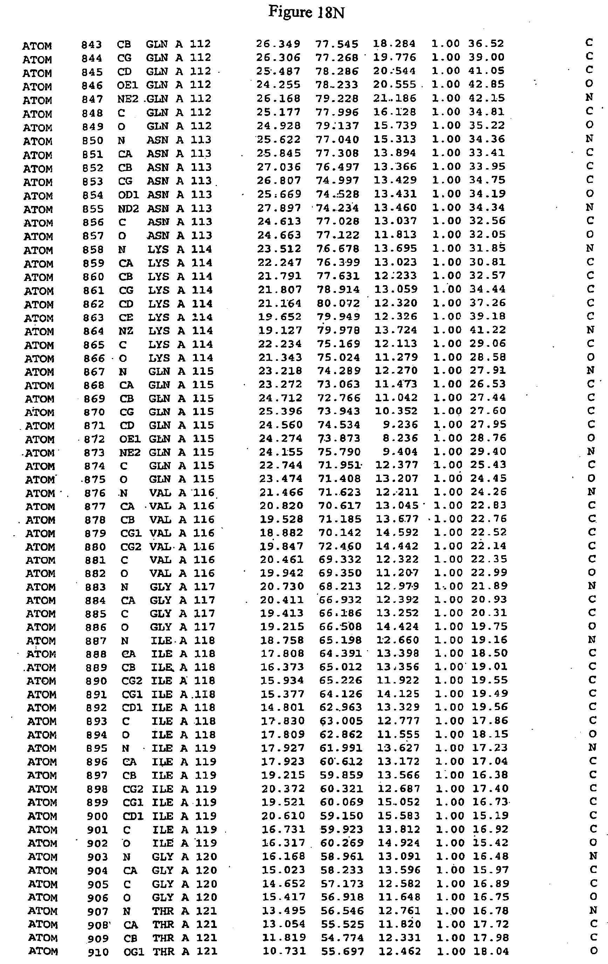

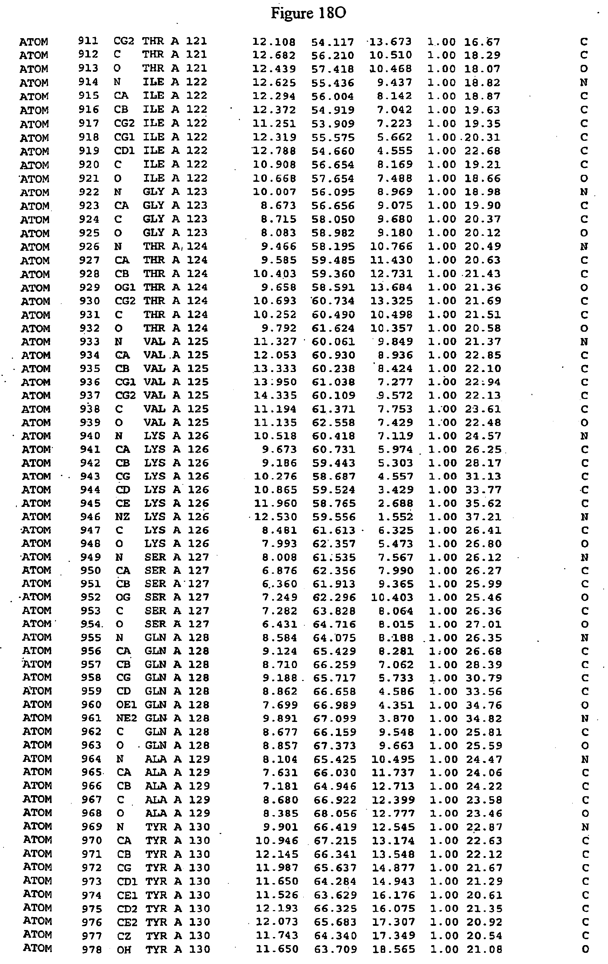

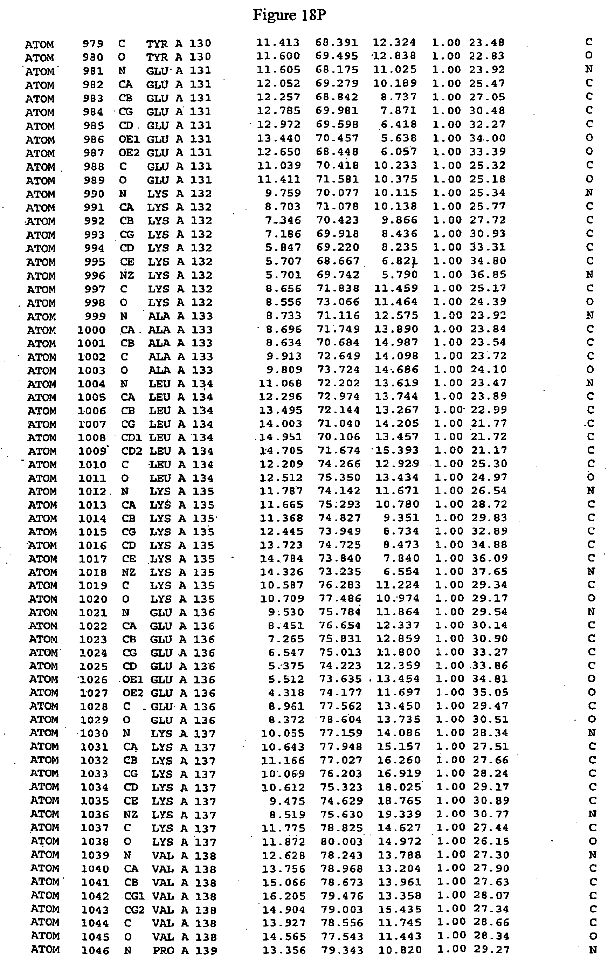

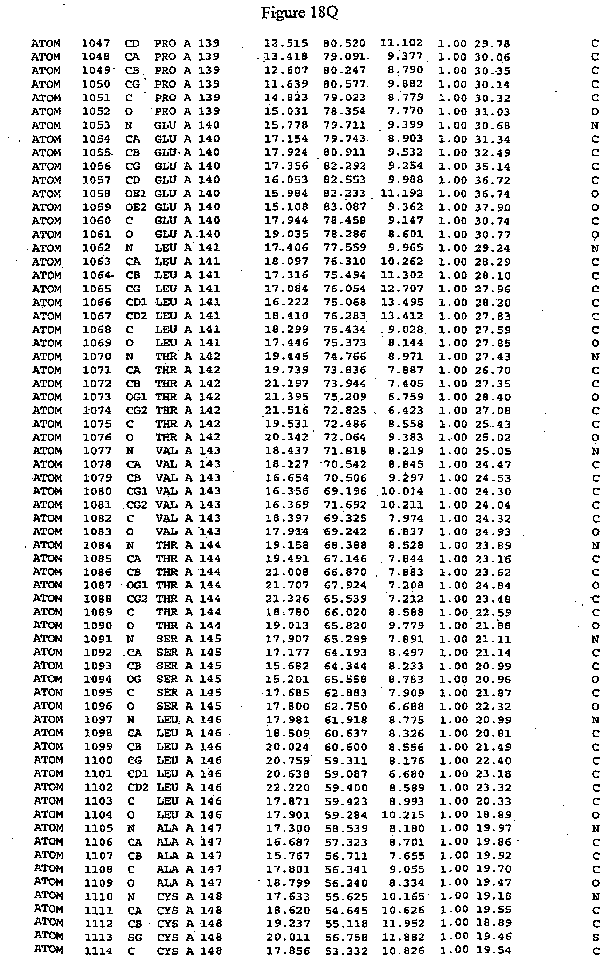

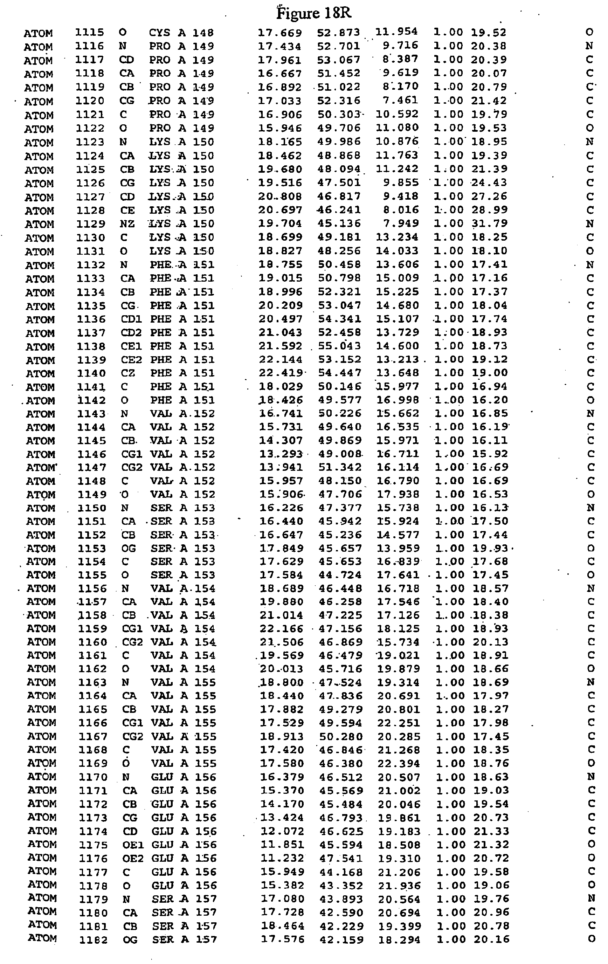

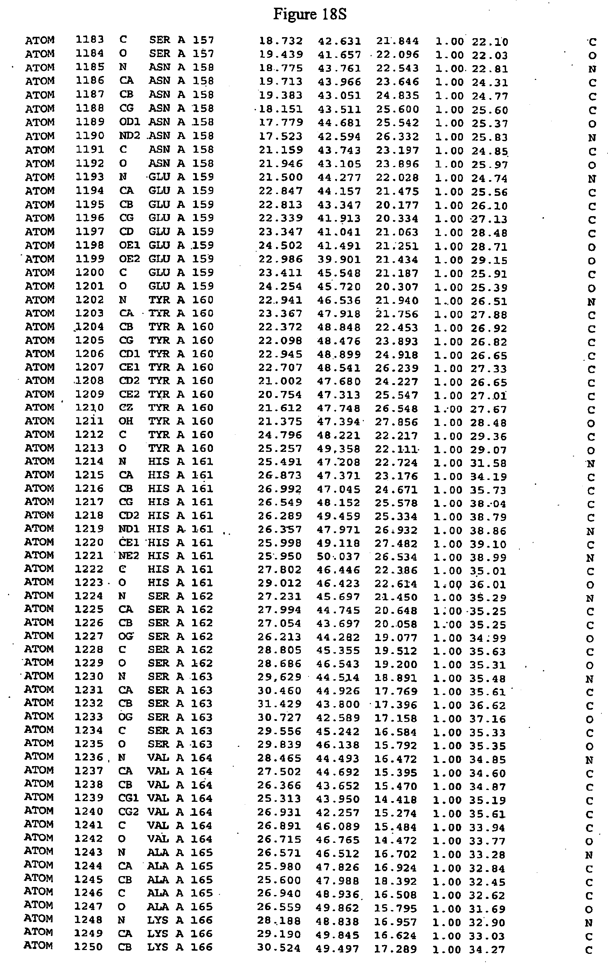

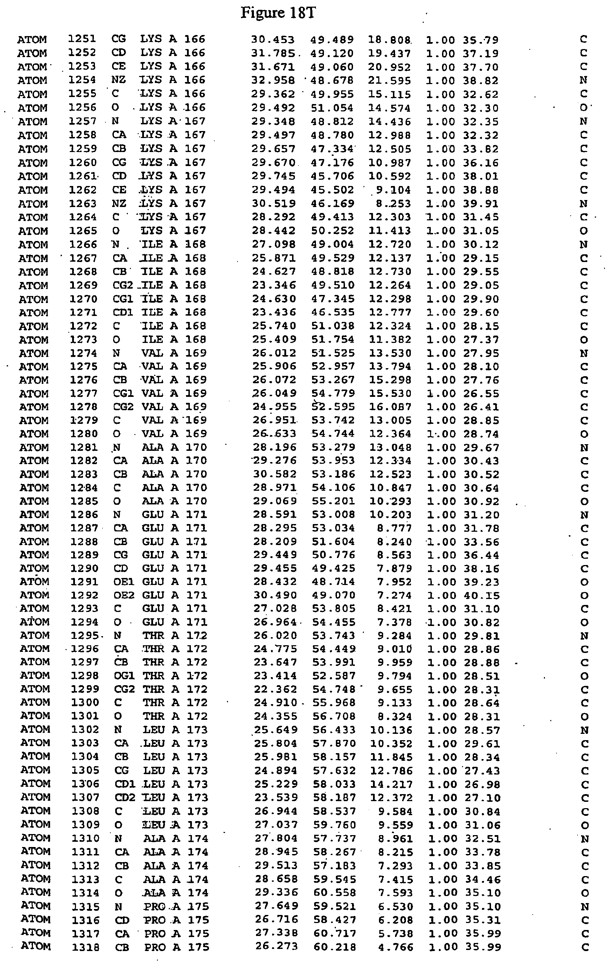

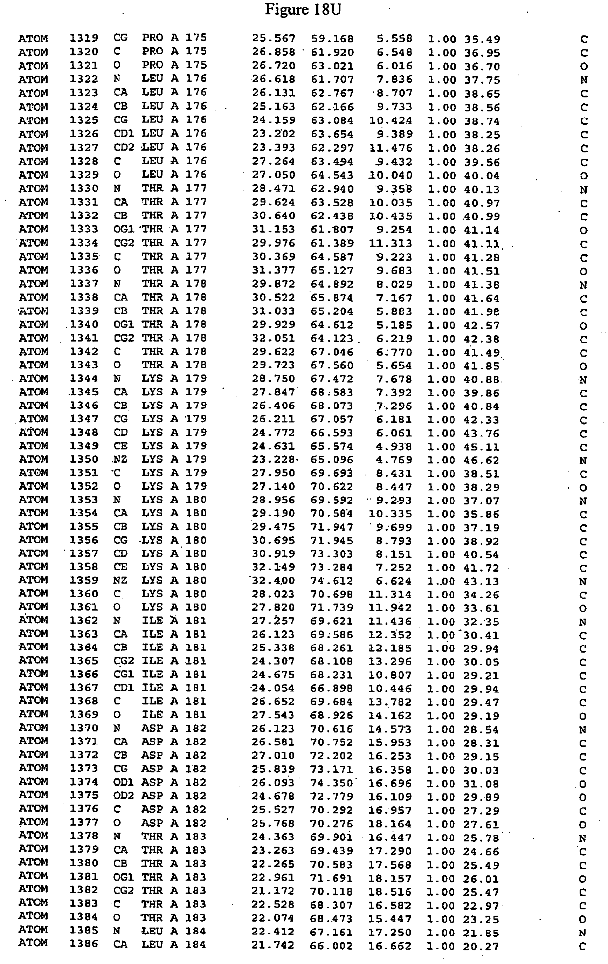

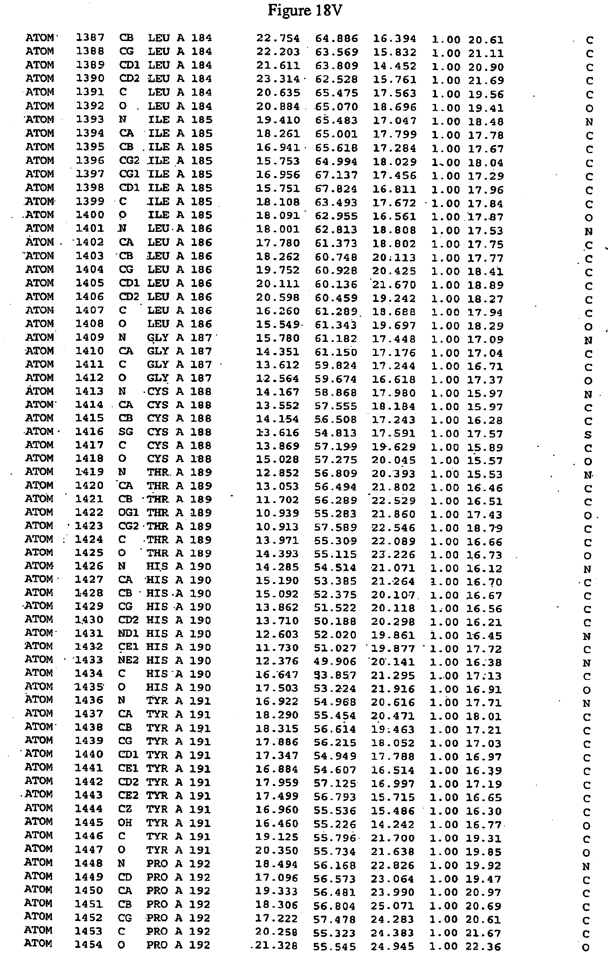

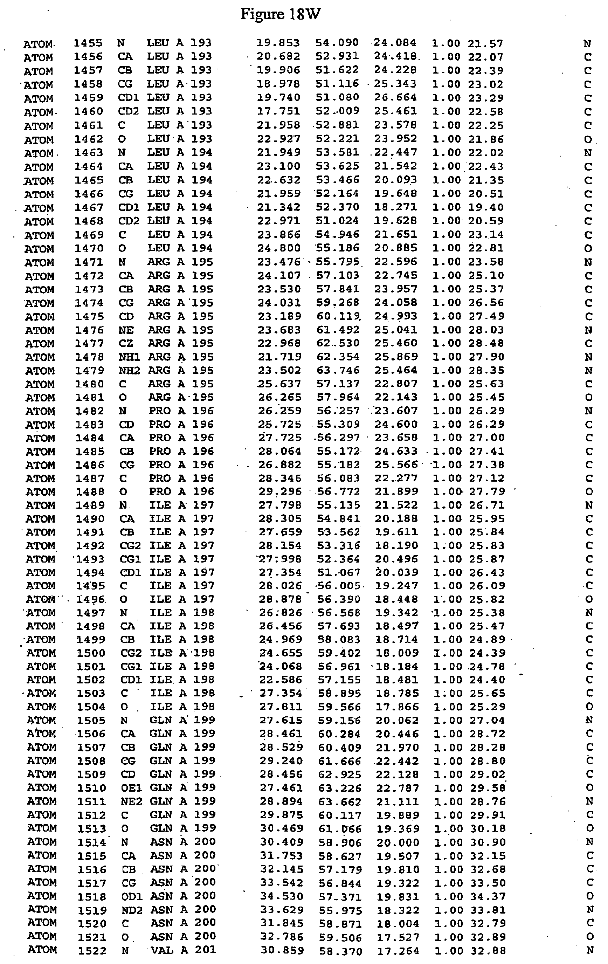

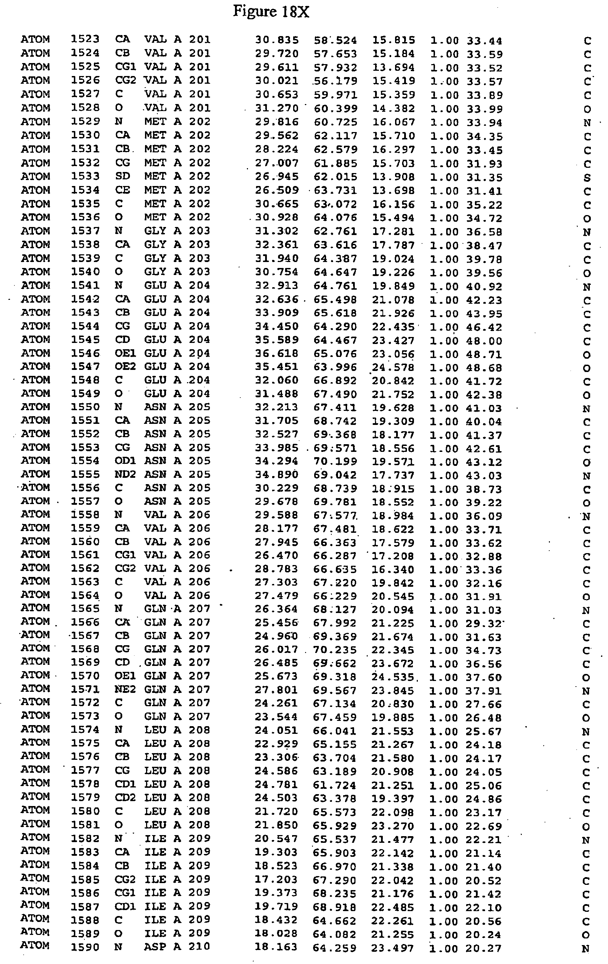

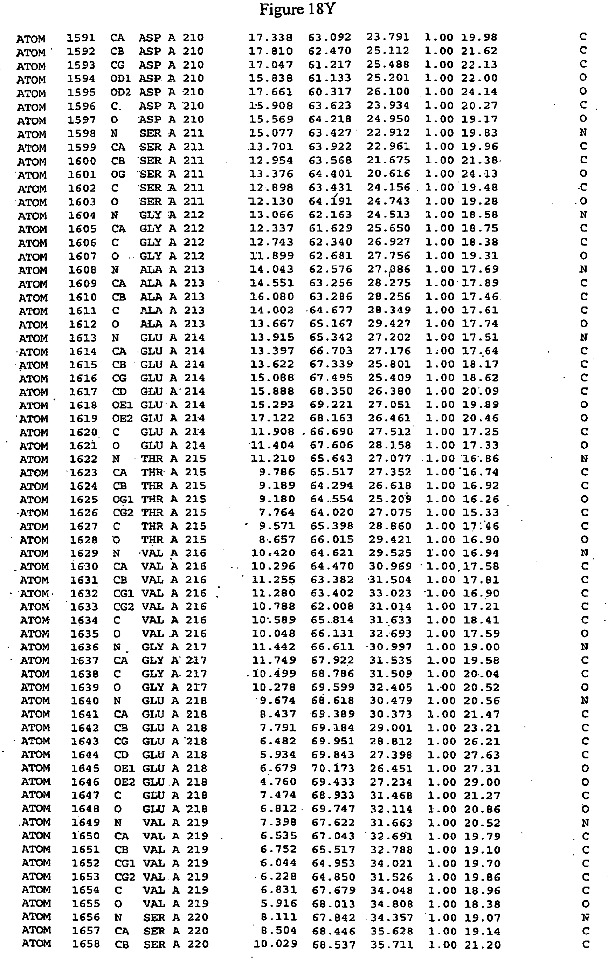

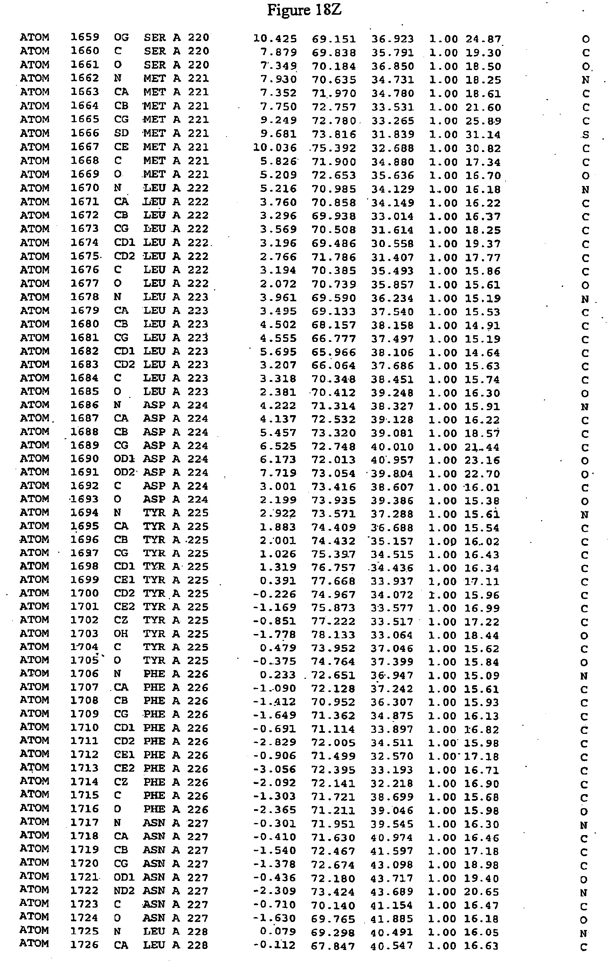

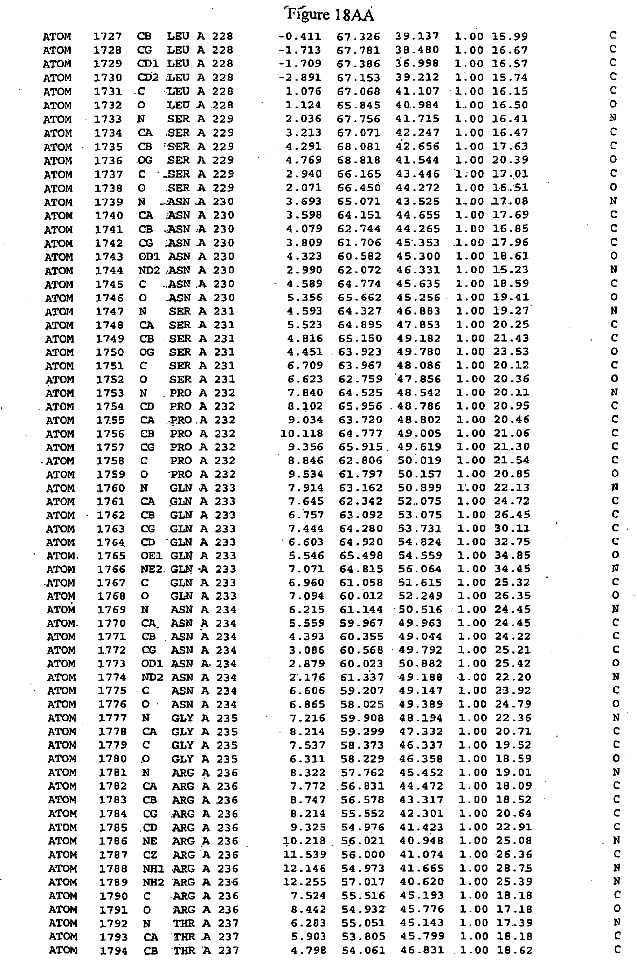

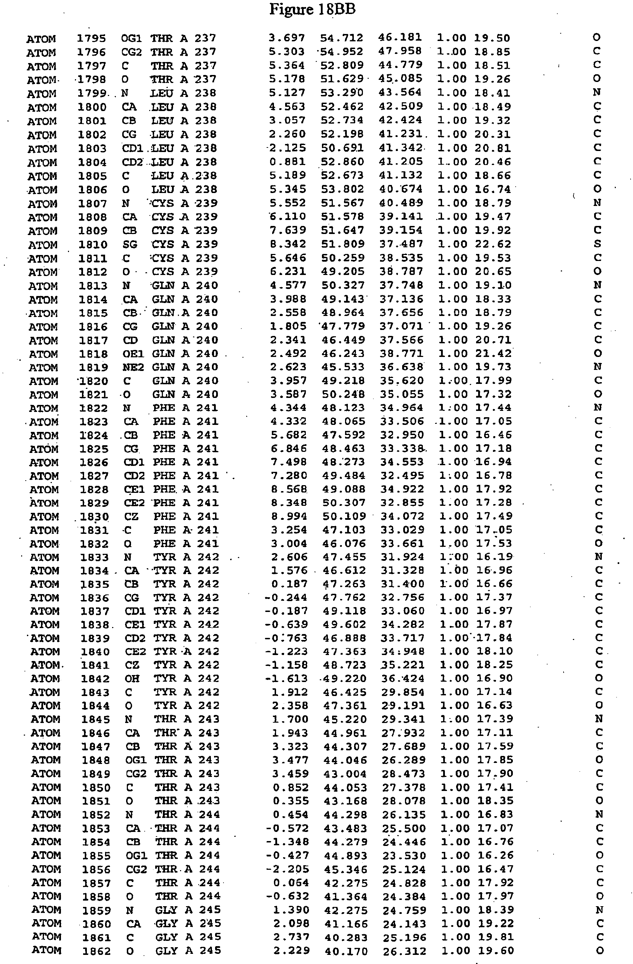

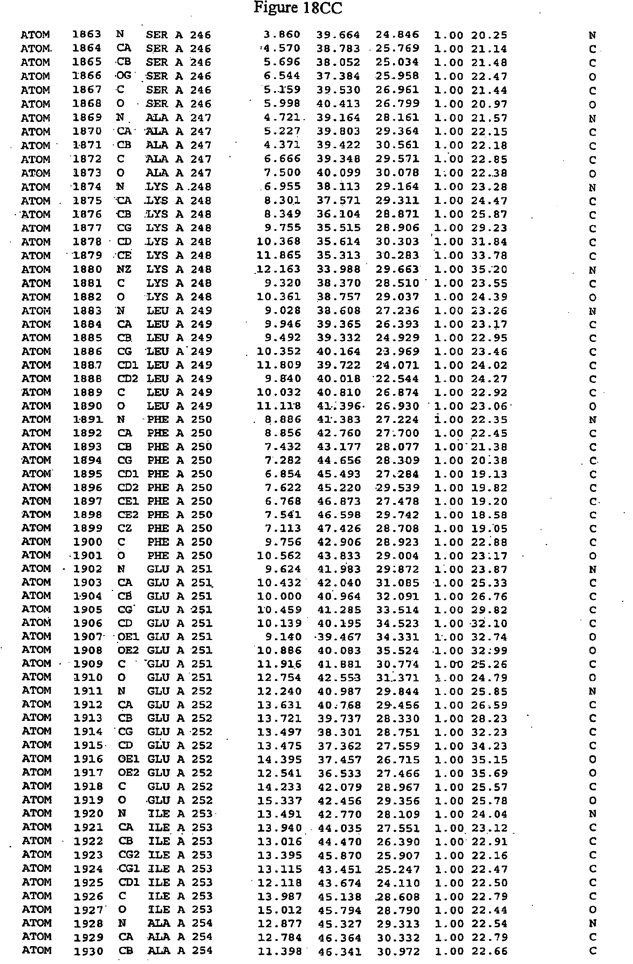

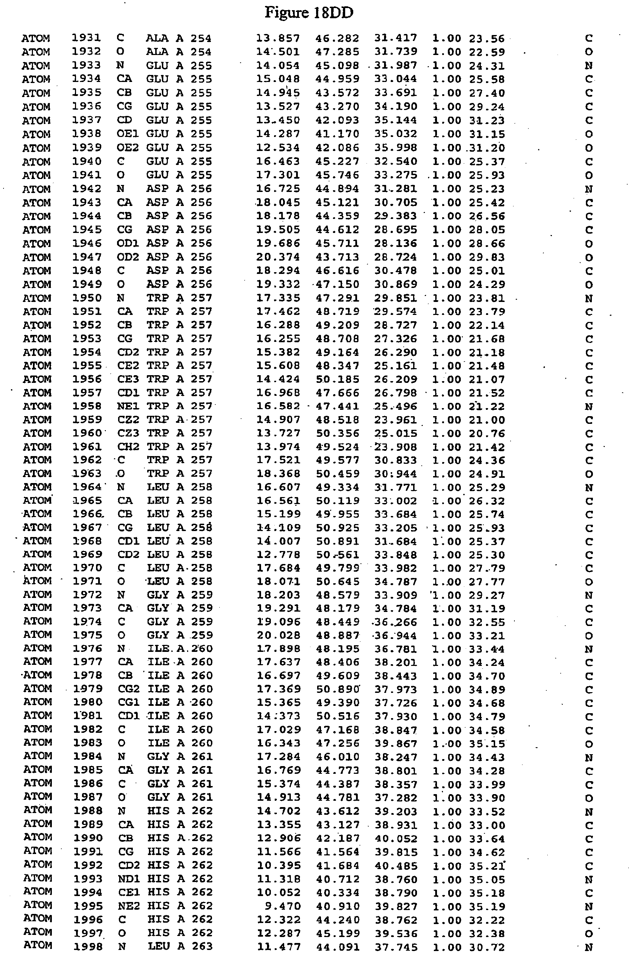

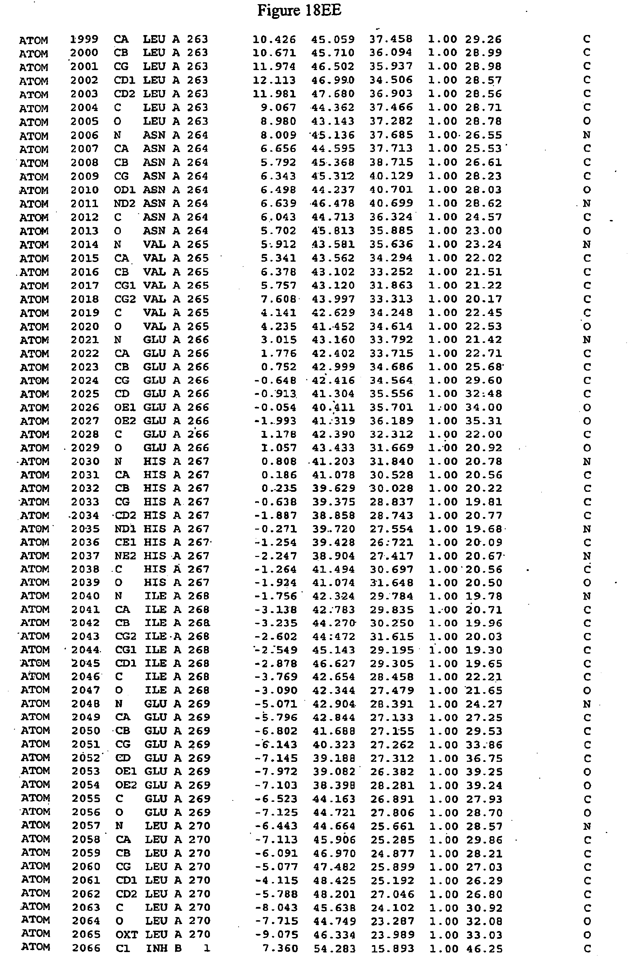

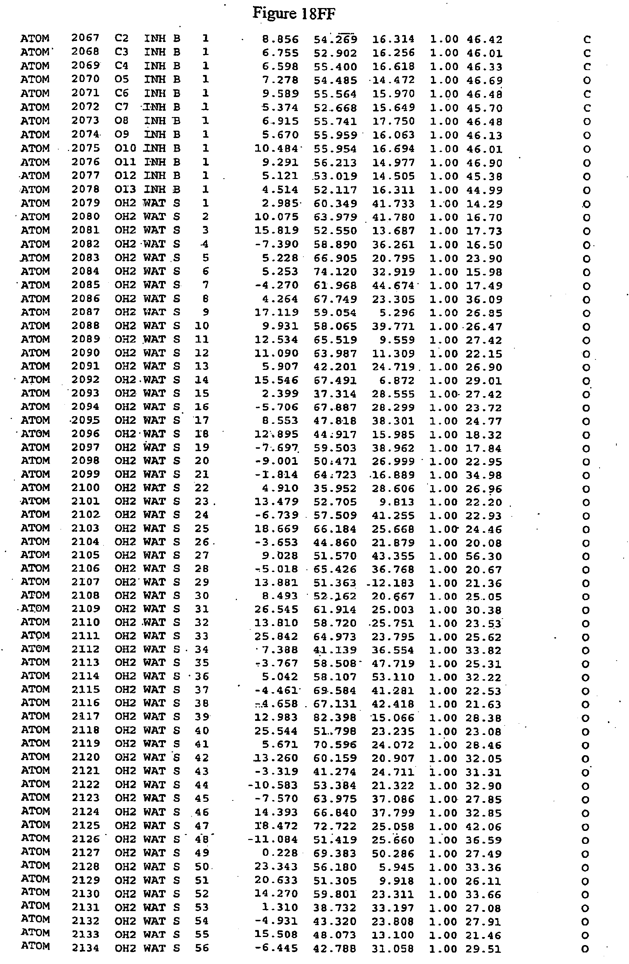

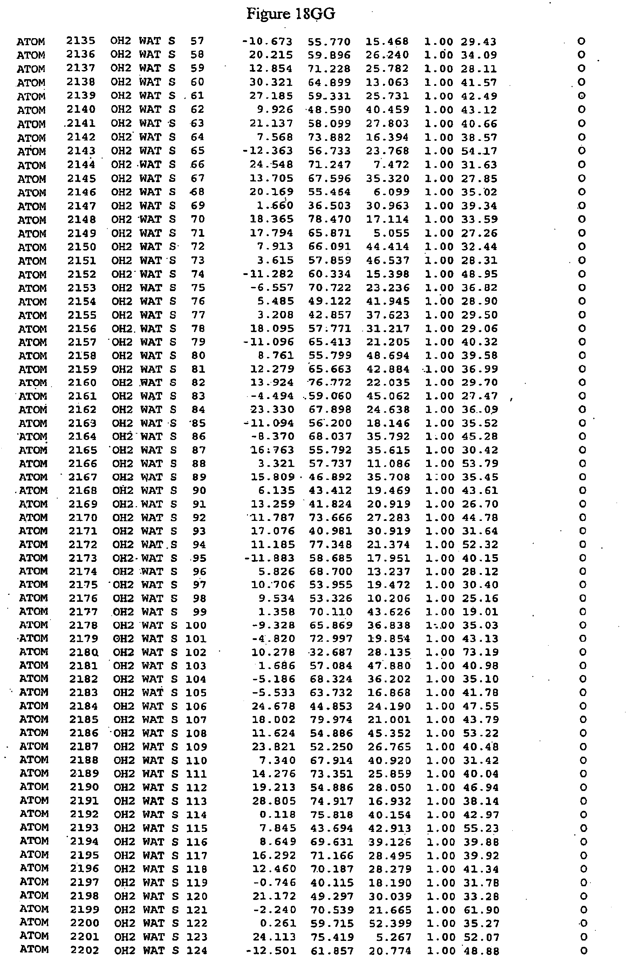

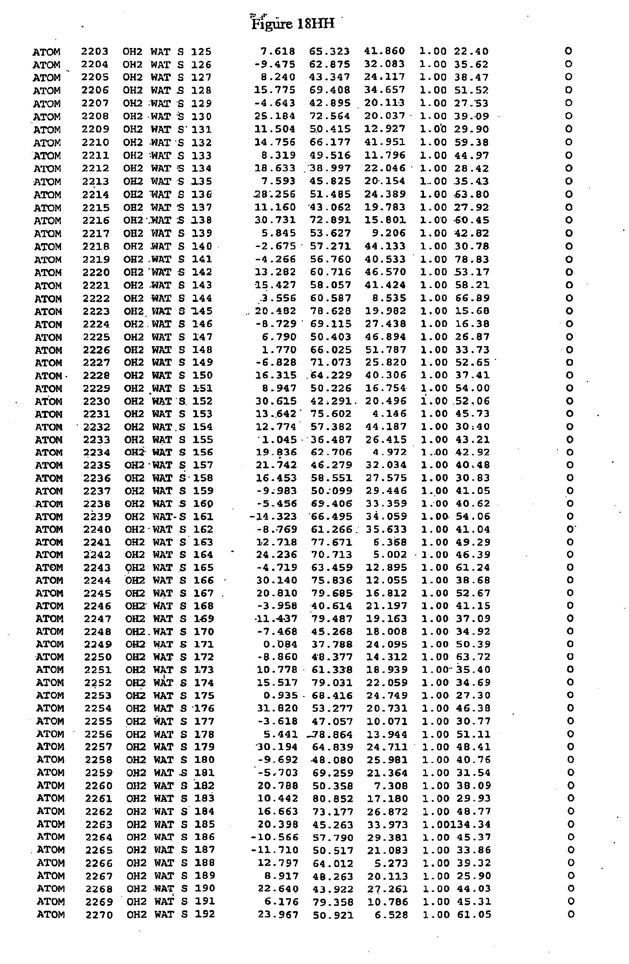

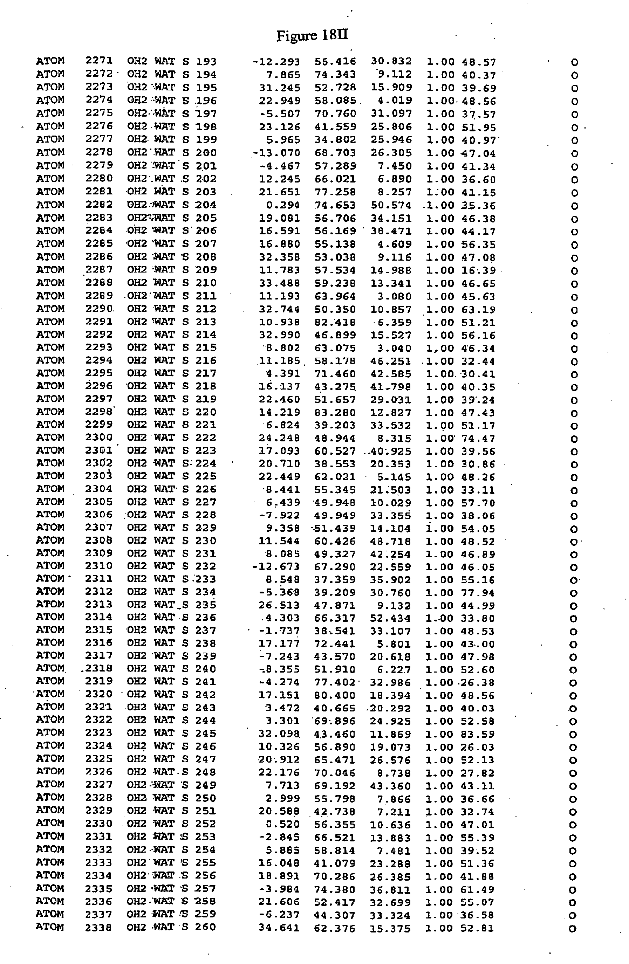

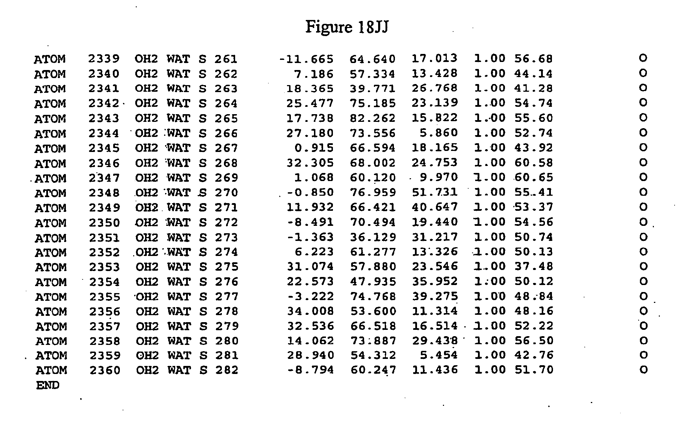

As described in the examples that follow, MurI of *E. faecalis, S. aureus,* and *E. faecium* have been crystallized and the crystal structure (three-dimensional structure) of each determined. The structures determined represent that of MurI alone, in complex with the enzyme product (substrate), such as D-glutamate or L-glutamate, or in complex with an inhibitor. Crystallization of *E. faecalis, E. faecium,* and *S. aureus* MurI is described in Examples 3-5, and Figures 12-18. Results show that the asymmetric unit of the crystal consists of a dimer which can exists in symmetrical (see Figure 1G) or non-symmetrical forms (see Figure 1H), depending on whether one or both of the substrate binding sites are occupied or open. The MurI protein is a four-domain structure in terms of overall folding; each domain has folds of the alpha/beta type. The molecular interface of Gram positive bacterium exists between two of the domains and functions as a flexible element by which a change in conformation opens the substrate binding site, allowing substrate to bind MurI.

Gram positive bacteria include the bacteria of Bacillus species, Staphylococcal spp., Streptococcal species, Enterococcal species, Lactobacilli, Pediococci, and Mycobacterial species. More specifically, Gram positive bacteria include, for example, *B. subtilis, S. aureus, E. faecalis,* and *E. faecium.*

### 1. E. faecalis

### a. Substrate Binding Site

A further embodiment relates to an *E. faecalis* MurI in which the binding site comprises two conserved cysteine residues, denoted Cys74 and Cys185 in the sequence represented as SEQ ID NO: 44. A further embodiment comprises a binding site of *E. faecalis* MurI wherein the binding site additionally comprises one or more of the following amino acid residues: Ser12, Thr76, Thr118, Thr121, Glu153, Thr186 and His187 as represented by the structural coordinates of Figure 12. In one embodiment, the binding site of the *E. faecalis* Murl complexes with D-or L-glutamate and comprises amino acid residues Cys74 and Cys185, as well as amino acid residues within 5 Å of the Cys74 and Cys185 residues as represented by the structural coordinates of Figure 13. In one embodiment, the binding site of the *E. faecalis* MurI additionally comprises one or more of the following amino acid residues: Ser12, Thr76, Thr118, Thr121, Glu153, Thr186 and His187. In this embodiment, the binding site complexes with D- or L-glutamate and comprises the amino acid residues Cys74 and Cys185 and at least one (one or more) of the following amino acid residues: Ser12, Thr76, Thr118, Thr121, Glu153, Thr186 and His187, which can be present in any combination. In a further embodiment, the binding site includes amino acid residues Ile10, Asp11, Ser12, Gly13, Val14, Gly15, Gly16, Thr18, Val19; Tyr34, Asp37, Arg40, Cys41, Pro42, Tyr43,Gly44, Pro45, , Arg46, Val51, Glu53, Ile72, Ala73, Cys74, Asn75, Thr76, Ala77, Ser78, Ala79, Val96, Ile116, Gly117, Thr118, Leu119, Gly120, Thr121, Ile122, Tyr127, Cys145, Pro146, Val149, Pro150, Leu183, Gly184, Cys185, Thr186, His187, Tyr188 and Ser208.

In a further embodiment, the binding site of the MurI comprises two hydrogen bond TRIADs, which occur close to the conserved cysteine residues of the binding site. Specifically, on one side (Cys185) of the binding site, the TRIAD is Glu153-Thr186-His187 and on the other side (Cys74), the TRIAD is Thr76-Thr118-Thr121. Thus, in a specific embodiment, the binding site of *E. faecalis* MurI is complexed with D- or L-glutamate and comprises amino acid residues Cys74 and Cys185 and additionally comprises at least one (i.e., one or more) of the following: amino acid residues Ser12, Thr76, Thr118, Thr121, G1u153, Thr186 and His187.

The threonine residues have features of interest as they relate to the binding site of *E. faecalis* MurI. On one side (Cys185) of the binding site, Thr186 is H-bonded to His187 and His187 is further H-bonded to Glu153. The hydroxyl oxygen (O) of Thr186 is 2.9 Å away from the amino nitrogen (N) of the substrate (i.e., glutamate) and 4.3 Å from the sulfur (S) atom of Cys185, which is 4.1 Å from the nitrogen (N) of His187.

On the other side of the binding site (Cys74), Thr76 is H-bonded to Thr1 18 and further H-bonded to Thr121. The hydroxyl O of Thr76 is H-bonded to one of the carboxylate oxygen atoms of the substrate and is 4.3 Å away from the S atom of Cys74. Analysis showed that the three hydroxyl oxygens form a triangle, all within less than 3.4 Å from one another.

The two TRIADs may play important roles in altering the pKa of the two binding site cysteine residues (in addition to that of the neighboring hydrophobic core), facilitating the proton transfer during catalysis or both.

Another embodiment is a crystal of MurI, comprising a binding site comprising the amino acid residues Cys74, Cys185, Ser12, Thr76, Thr118, Thr121, Glu153, Thr186 and His187 of the amino acid sequence represented by SEQ ID NO: 44. This crystal may be further complexed with D- or L-glutamate. Additionally, the binding site of the crystal complexed with D-glutamate can comprise the amino acid residues: Cys74, Cys185, and one or more of the following amino acid residues: Ser12, Thr76, Thr118, Thr121, Glu153, Thr186 and His187. A further embodiment is one in which the binding site additionally comprises at least one of the following amino acid residues: Asp11, Gly13, Val14, Gly15, Gly16, Arg40, Cys41, Pro42, Arg46, Ala73, Asn75, Ala77, Val96, Gly117, Val149, Gly184, and Tyr188. A further embodiment is one in which the binding site additionally comprises at least one of the following amino acid residues: Ile10. Thr18, Va119, Tyr34, Asp37, Tyr43, Gly44, Pro45, Va151, De72, Ser78, Ala79, Ile116, Leu119, Gly120, Ile122, Tyr127, Cys145, Pro146, Pro150, Leu183, and Ser208. The crystals comprising binding sites represented by the above amino acid residues comprise the amino acid sequence is that represented by SEQ ID NO: 44; or an amino acid sequence is at least 75%, 80%, 85%, 90%, 95%, or 98% homologous to the amino acid sequence that is represented by SEQ ID NO: 44.

Location and geometry of a binding site of the enzyme are also defined. Two conserved cysteine (Cys) residues were identified as the residues responsible for the (de)protonation of the alpha-carbon of the substrate during catalysis, which is consistent with the two-base mechanism proposed for function of the enzyme in its role as a racemase. The two binding site cysteines are Cys74 and Cys185. which are about 7.0 Angstroms (Å) apart (Cα-Cα distance, i.e., the distance between Cα atoms). Other amino acid residues identified in the vicinity of the binding site include Ser12, Thr76, Thr118, Thr121, Glu153, Thr186 and His187. The bound substrate/product, D- or L-glutamate, is located between the two conserved cysteine residues. Further detail of the binding site is as follows: looking down the axis defined by the GIu153 along the γ and α carbon, the two cysteines exhibit a rather symmetrical environment; each has a hydrophobic core behind, with respect to the substrate and a neighboring threonine residue not far from the substrate C-alpha (3.7 - 3.4 Å when L-Glu is bound; and 3.4 - 4.3 Å when D-Glu is bound), Here we have product in one site and a substrate in the other. Conformational changes bring the cysteines closer in the α sub-unit with the substrate), respectively.

Analysis of the crystal structure of the *E. faecalis* MurI indicates that the following amino acid residues are within 10 Å of the bound D- or L-glutamate: 10-19, 34-46, 51, 72-78, 96-97, 116-122, 124, 127, 183-189, and 208-209. Analysis of the crystal structure also shows that the following amino acid residues are within 4 Å of the bound D- or L-glutamate: 11-12 41-44, 74-76, 118, 121, and 186-187. Of interest is the fact that only one acidic amino acid residue (Asp11) is present in the structure surrounding the binding site Cys74, which serves as an anchoring point for the amino group of the substrate/product with a polar (charged) interaction between the amino nitrogen atom of D- or L-glutamate and the delta oxygen atom of Asp11.

### b. Intermolecular Dimer Interface

One embodiment is a crystal of *E*. *faecalis* MurI having an intermolecular interface comprising at least one of amino acid residues: Gln26, Leu27, Pro28, Asn29, Glu83, Lys86, Ala87, Ala88, Leu89, Pro90, Ile91, Pro92, Val93, Val94, Gly95, Val96, Ile97, Leu98, Pro99, Arg102, Ala103, Lys106, Ala130, Ser133, Lys134, Ala135, Pro136, Ala210, Glu211, Gly214, Glu215, Glu216, Ser217, Met218, Leu219, Asp221, Tyr222, Phe223, Asp224, Ile225, Ala226, His227, Thr228, and Pro229 of SEQ ID NO: 44.

### c. Intradomain Binding Site

One embodiment is crystallized *E. faecalis* MurI having an intradomain dimer interface comprising at least one of amino acid residues: Asp11, Ser12, Gly13, Val14, Gly15, Gly16, Leu17, Thr18, Val19, Lys21, Glu22, Lys25, Ala39, Arg40, Cys41, Pro42, Tyr43, Gly44, Pro45, Arg46, Pro47, Val51, Ala73, Cys74, Asn75, Thr76, Ala79, Val80, Val96, Ile97, Glu211, Thr212, G1y214, Glu215, Met218, Leu219, Asp221, and Tyr222 of a first domain, and amino acid residues Ile97, Leu98, Pro99, Gly100, A1a101, Arg102, Ala103 , Ala104, Val105 , Lys106, Va1107, Thr118, Leu119, Gly120, Thr121, Lys123, Ser124, Ala125, Ser126, Tyr127, Ile129, Ala130, Ser133, Lys134, Cys145, Pro146, Lys147, Phe148, Val149, Pro150, Ile151, Val152, Glu153, Ser154, Asn155, Ile182, Leu183, Gly184, Cys185, Thr186, His187, Tyr188, Pro189, Leu190, Ile206, Asp207, Ser208, Gly209, and Ala210 of a second domain, in which the amino acid residues are represented by SEQ ID NO: 44.

### 2. E. faecium

### a. Substrate Binding Site

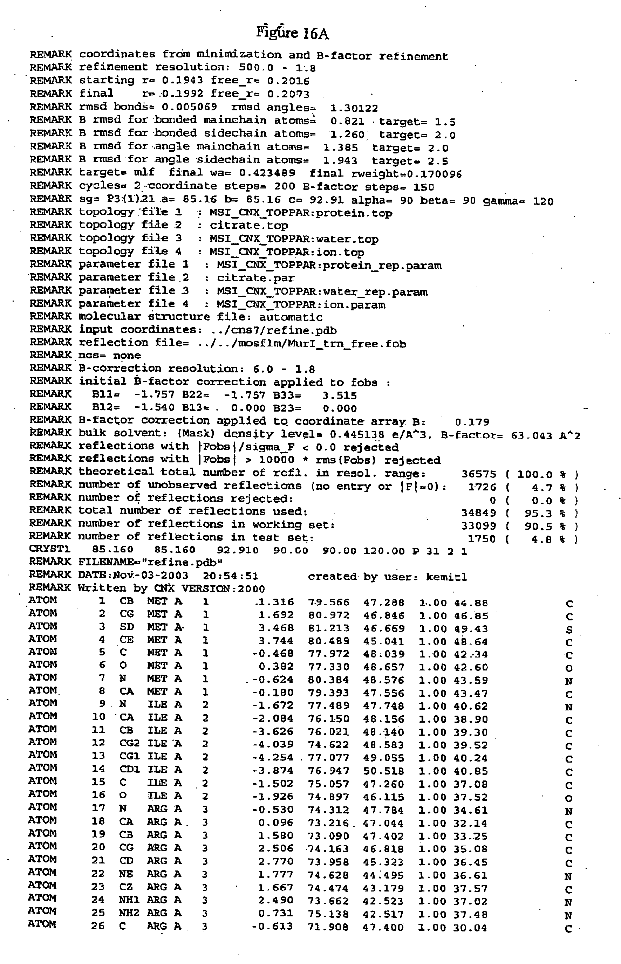

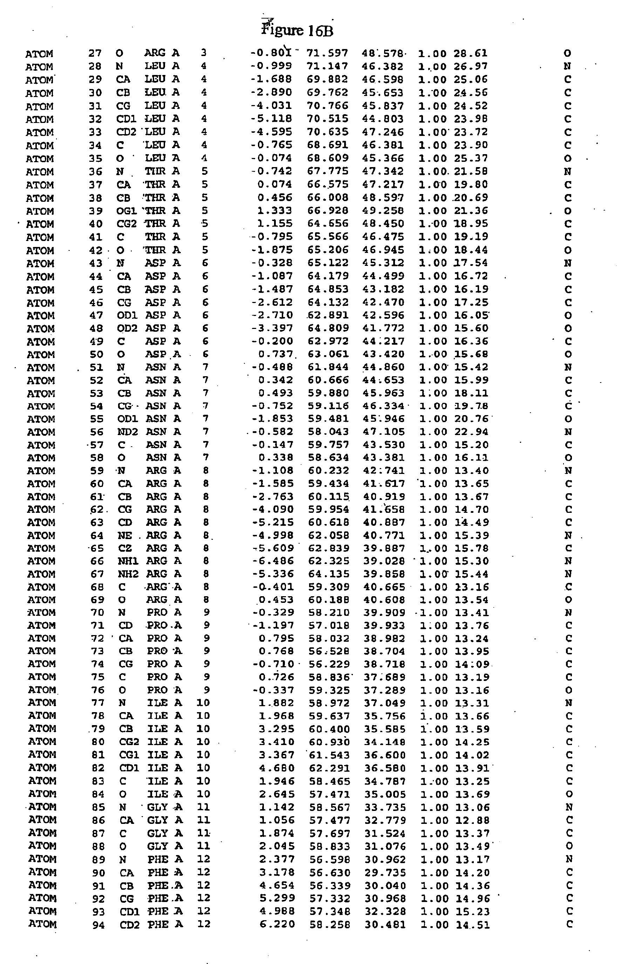

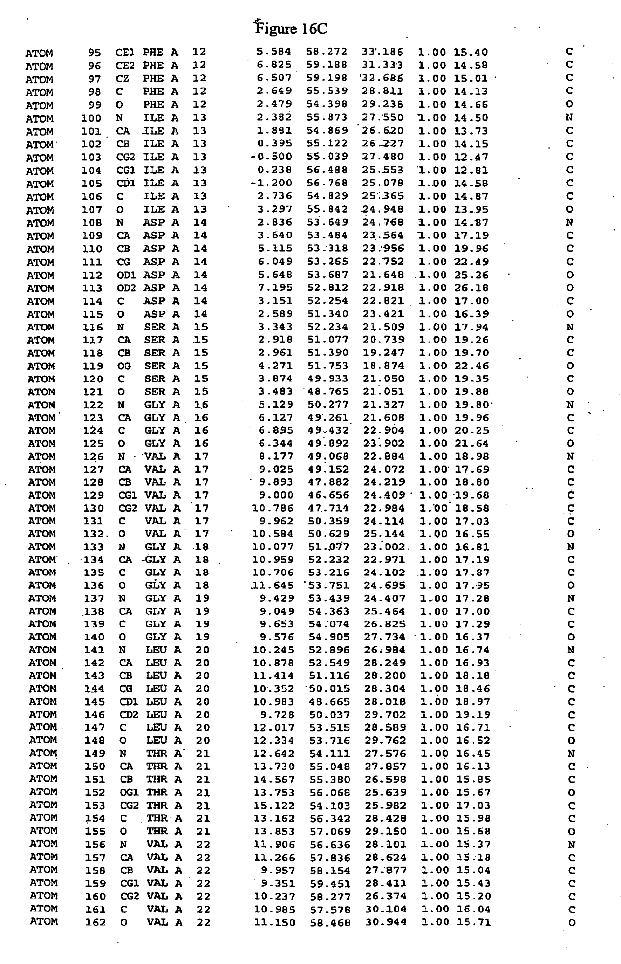

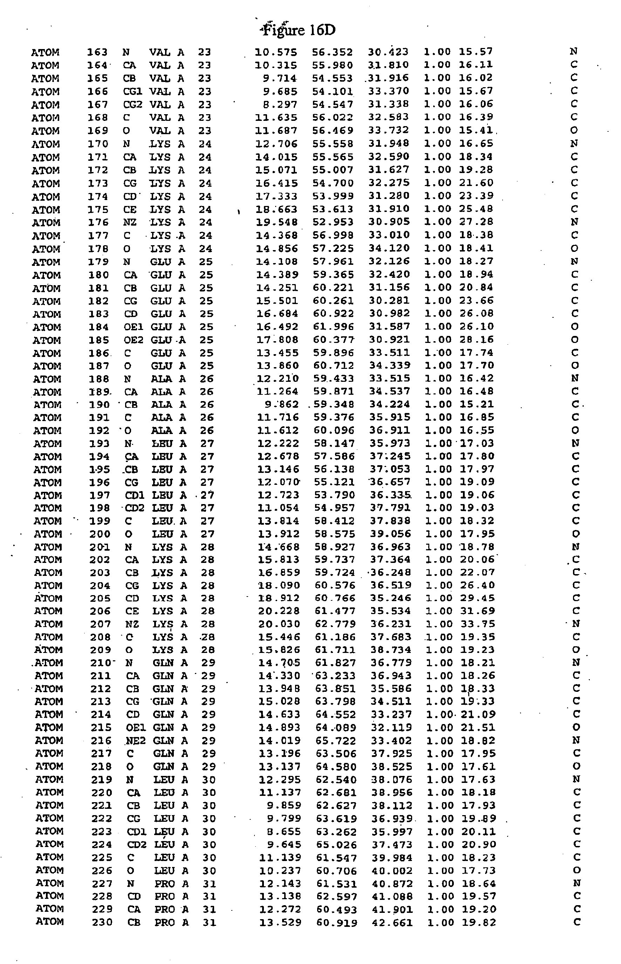

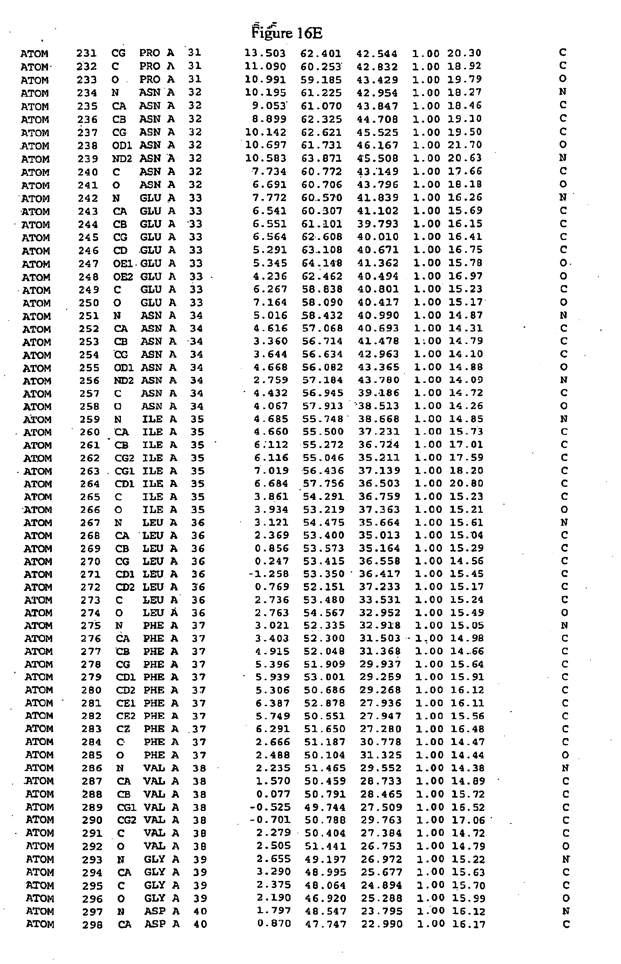

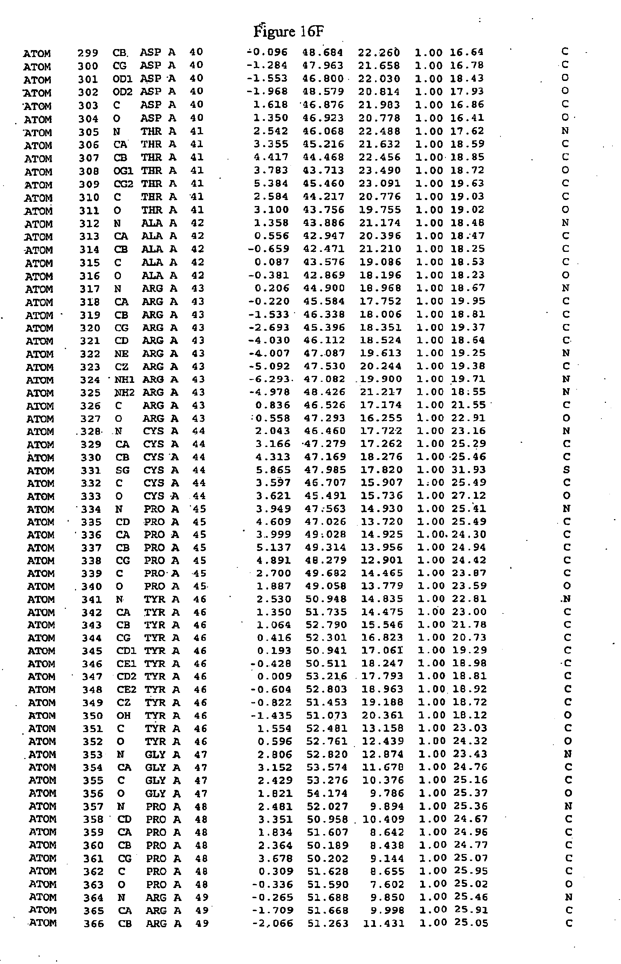

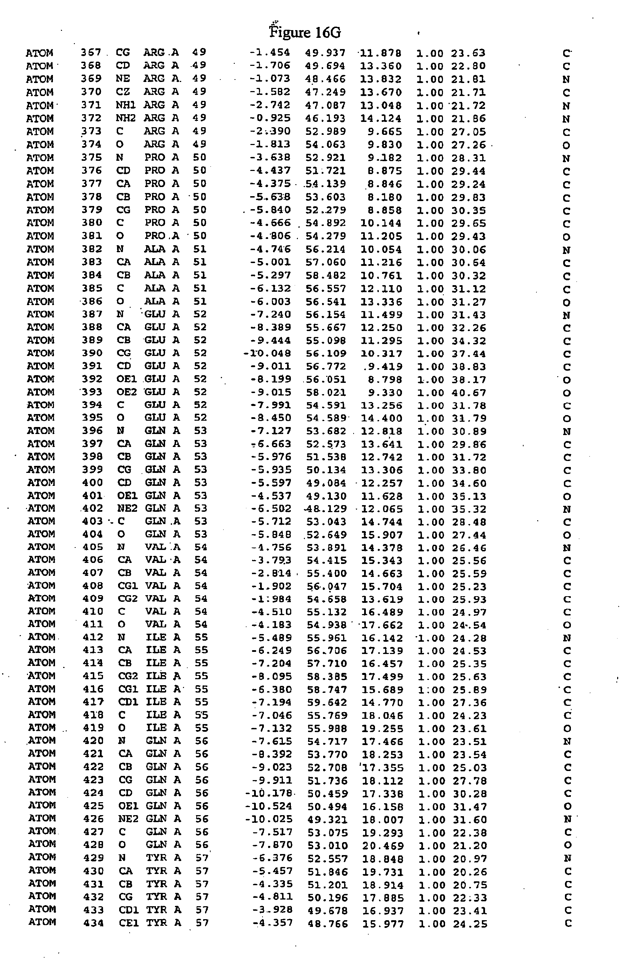

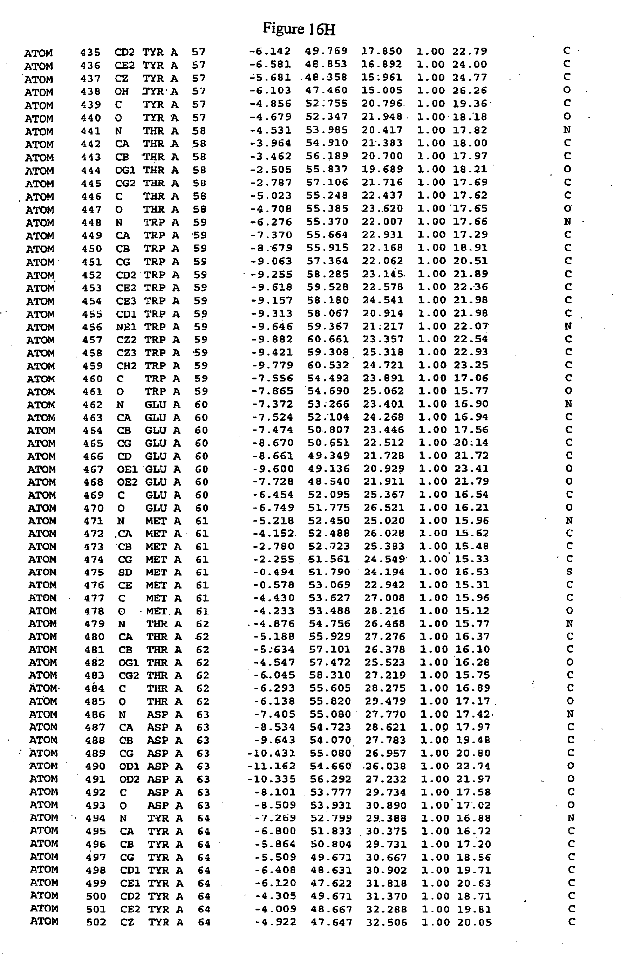

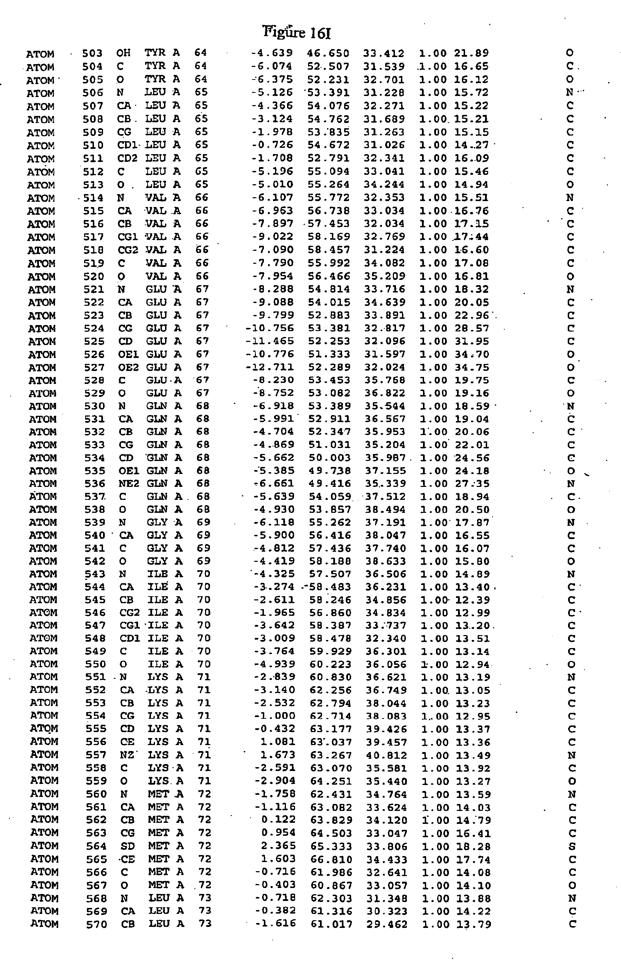

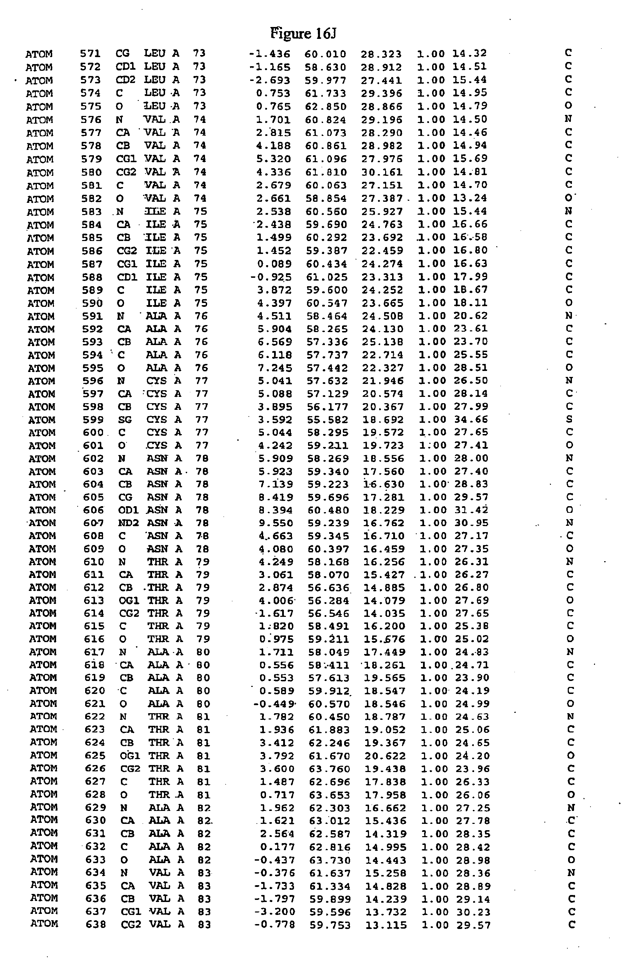

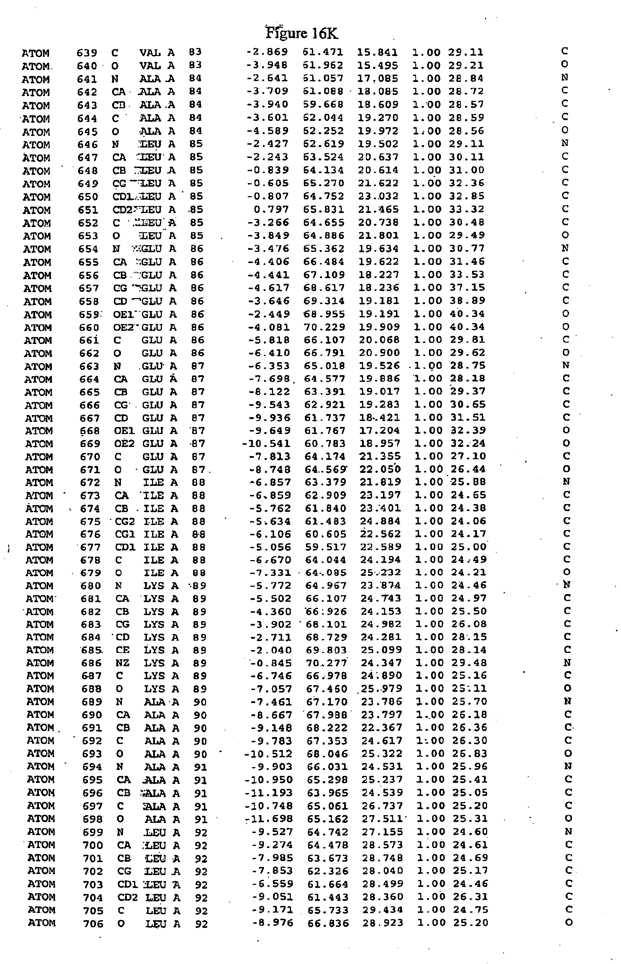

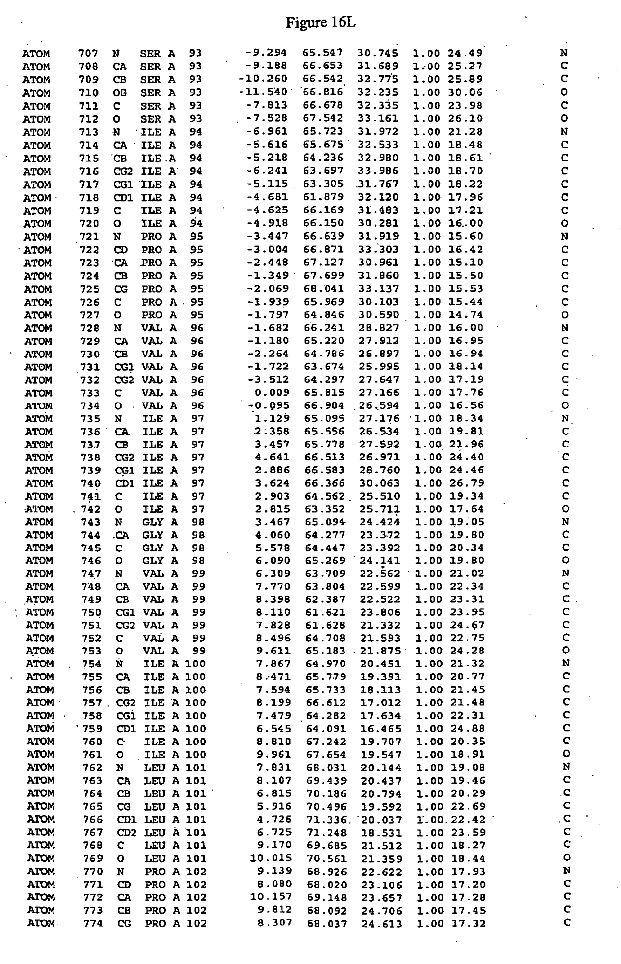

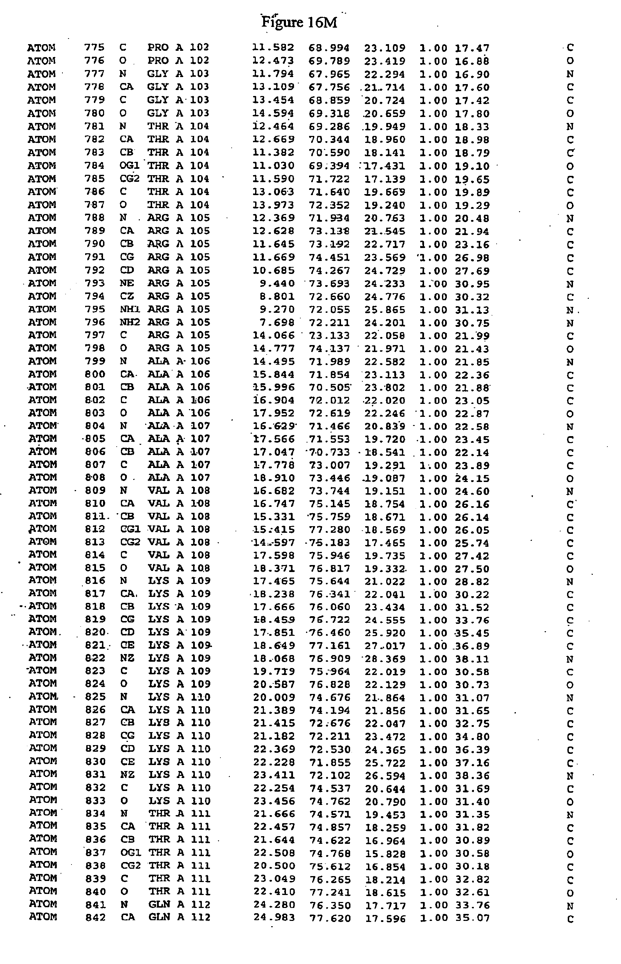

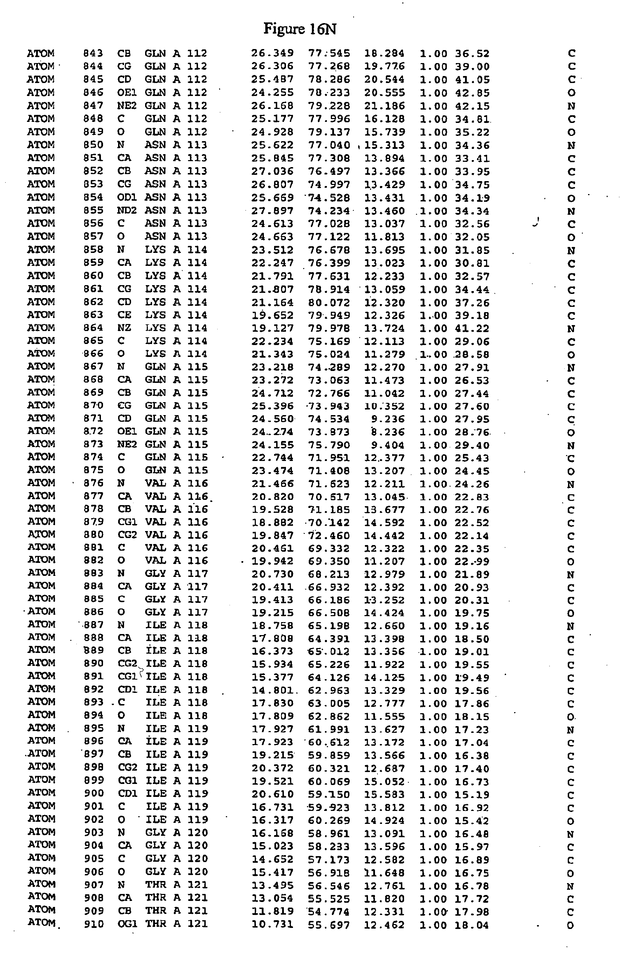

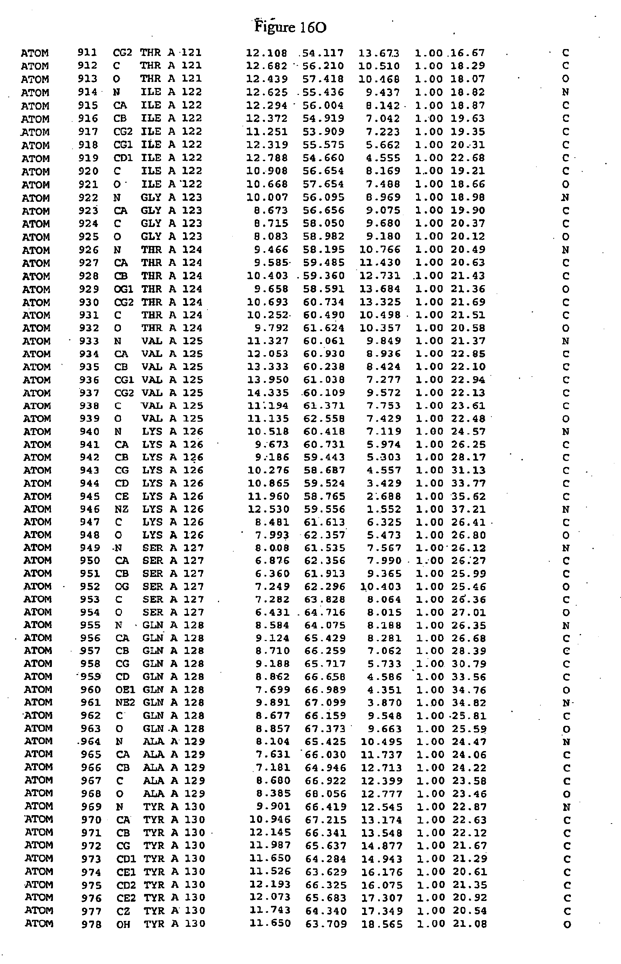

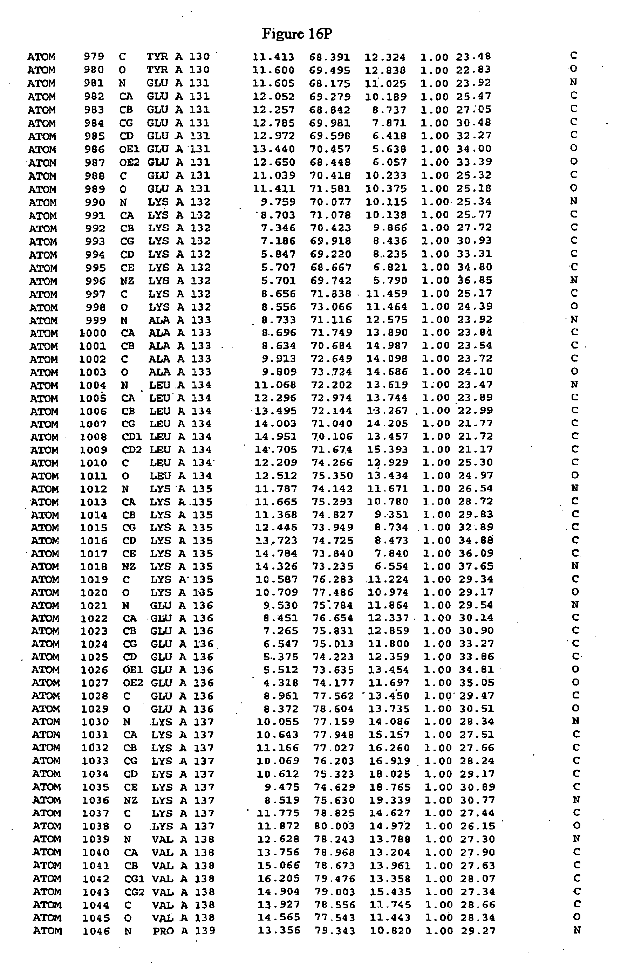

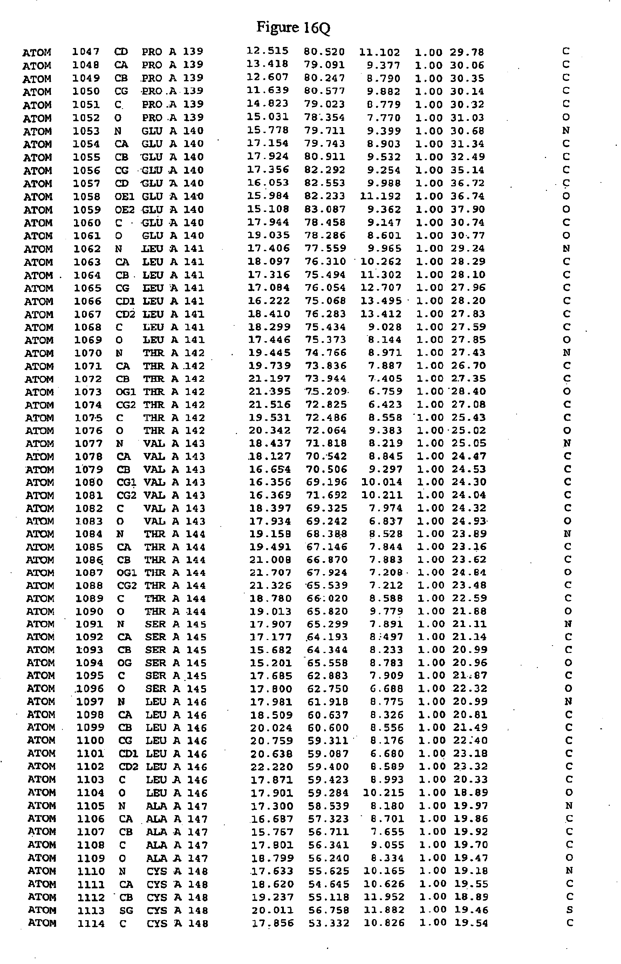

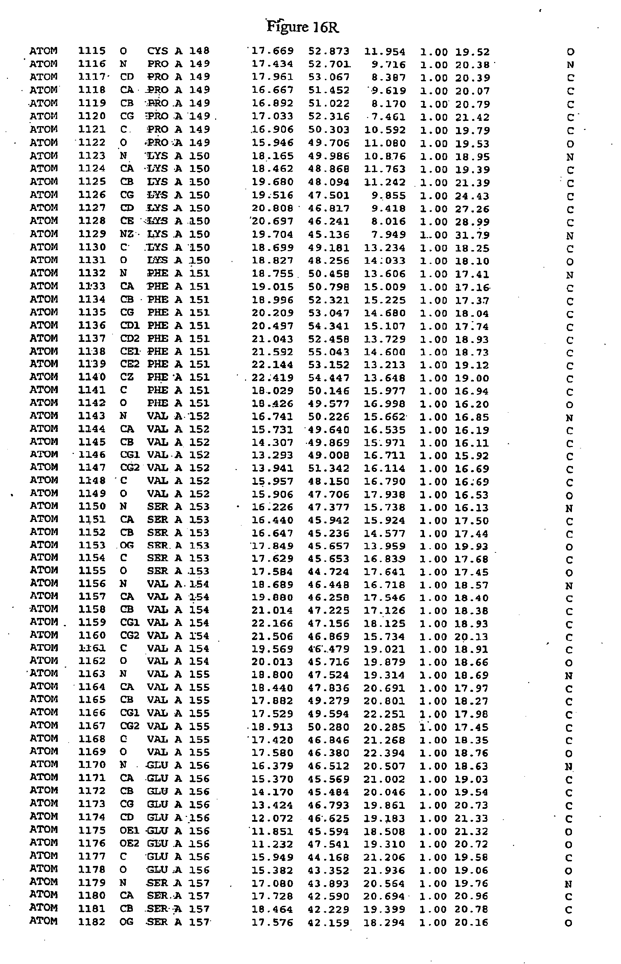

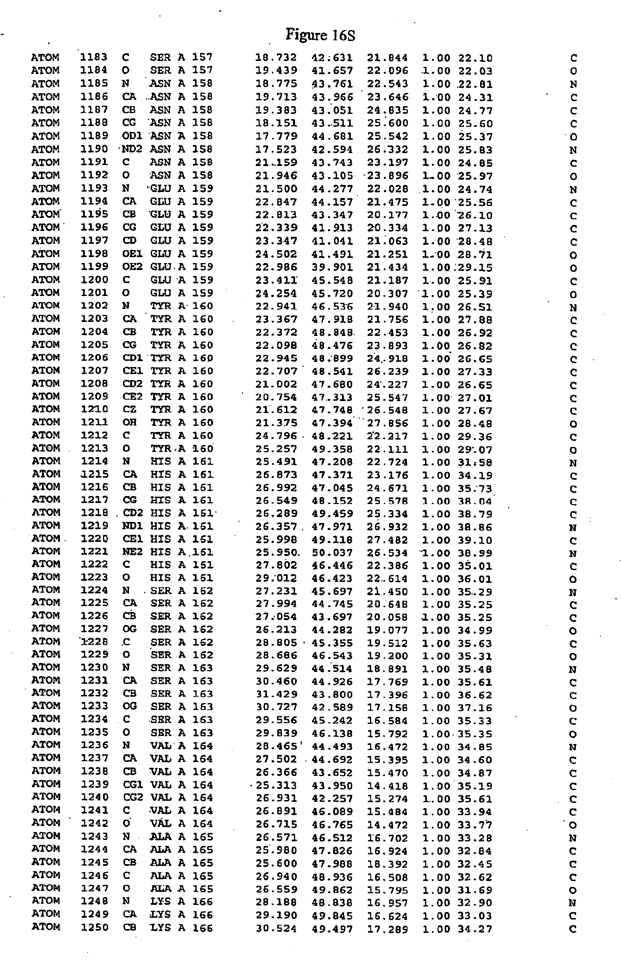

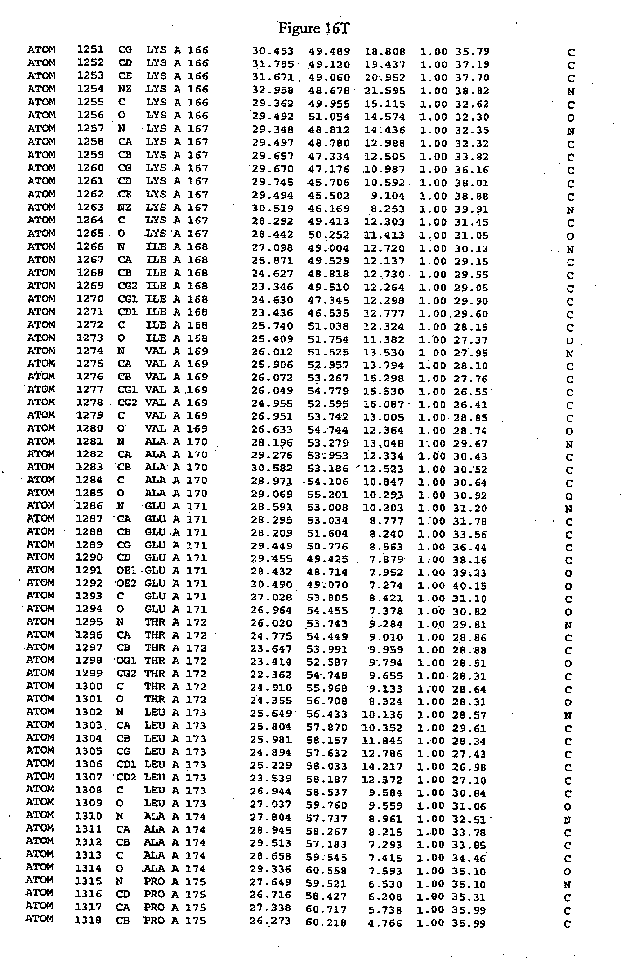

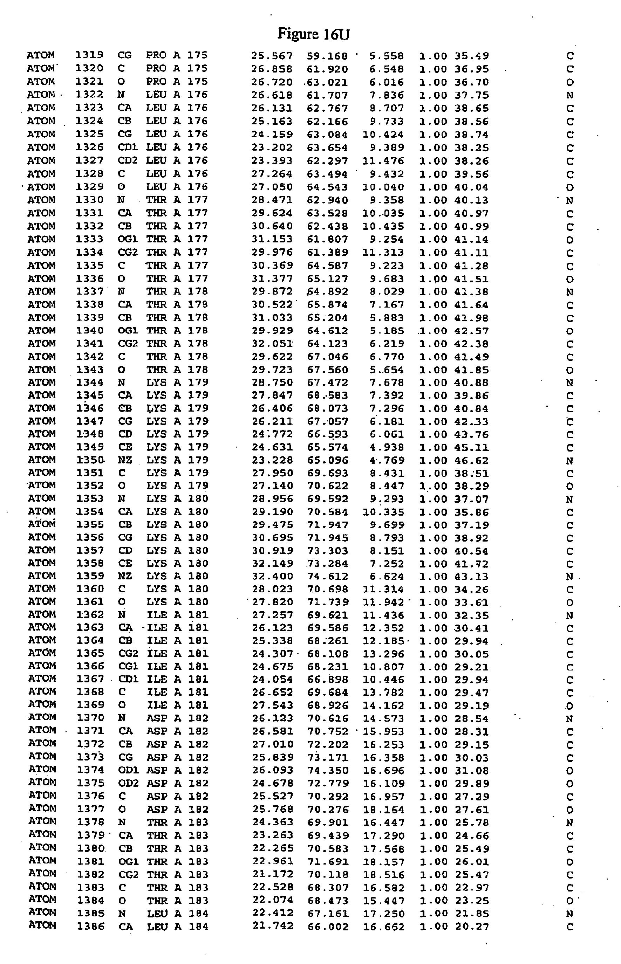

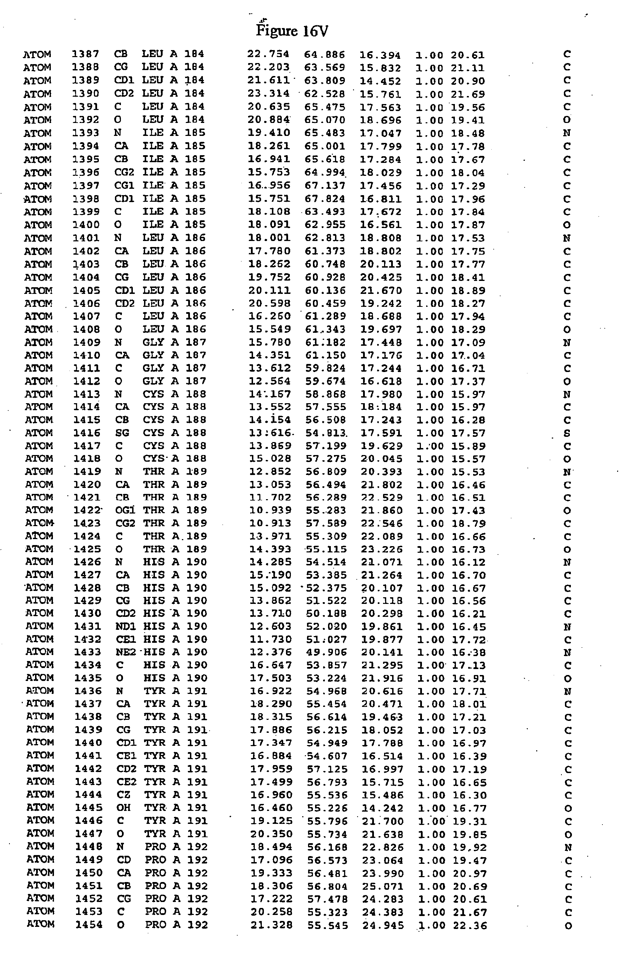

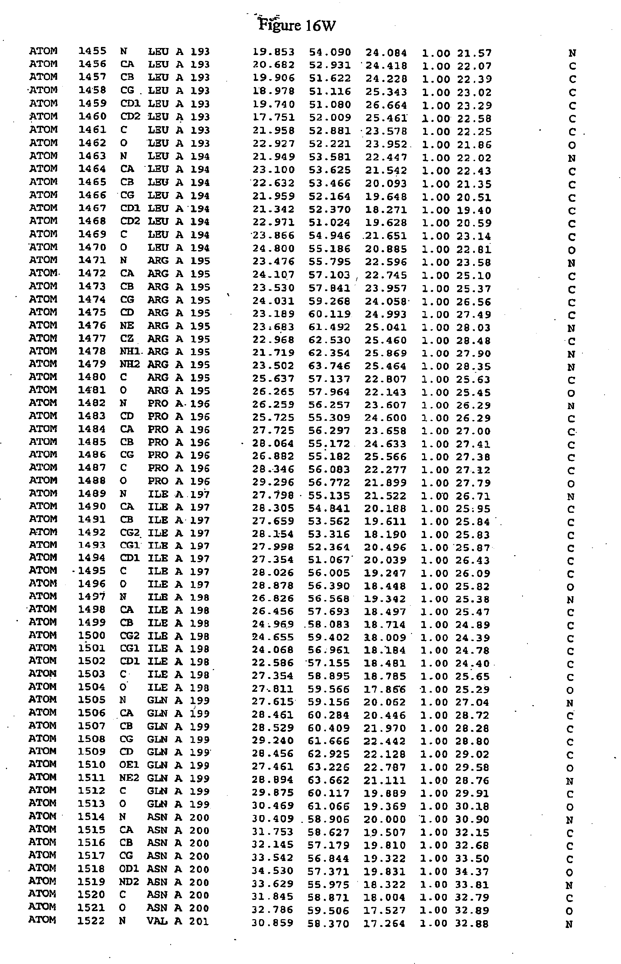

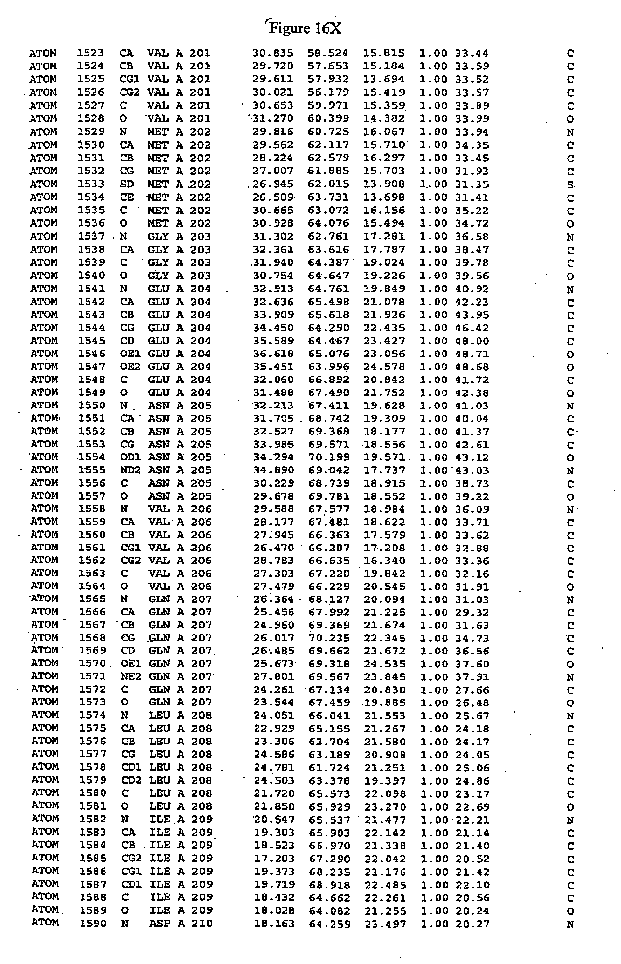

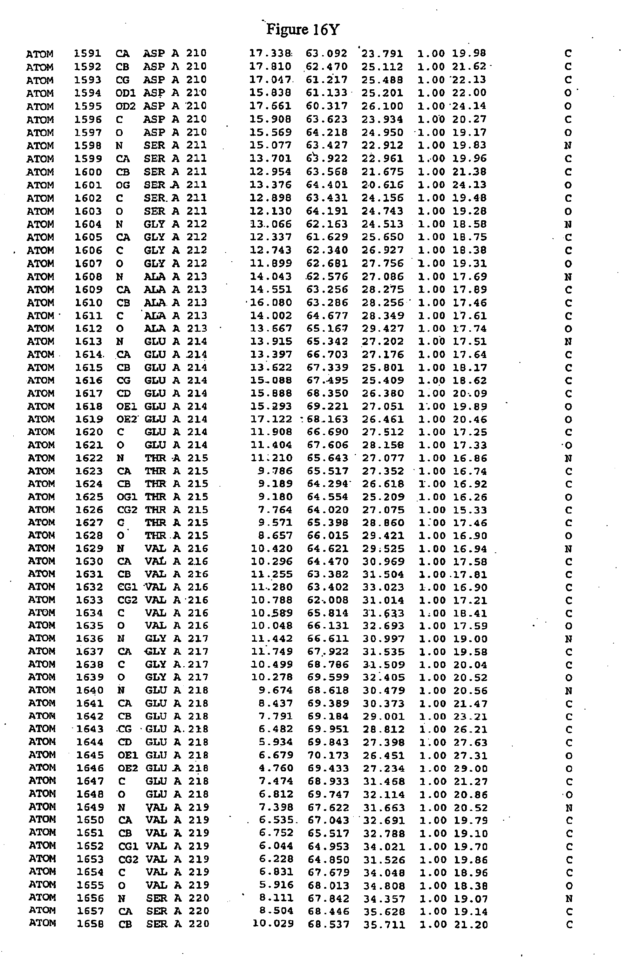

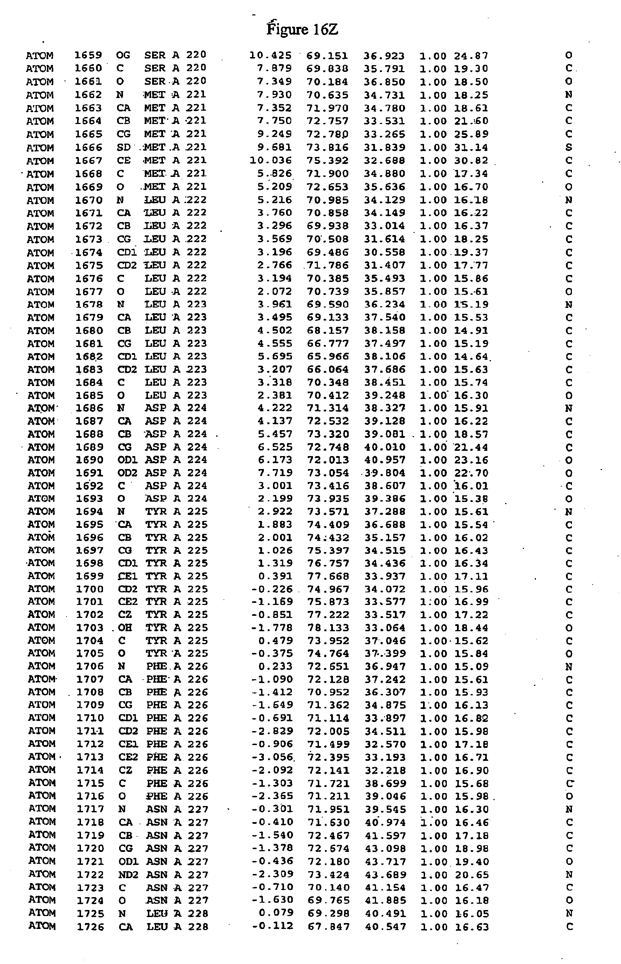

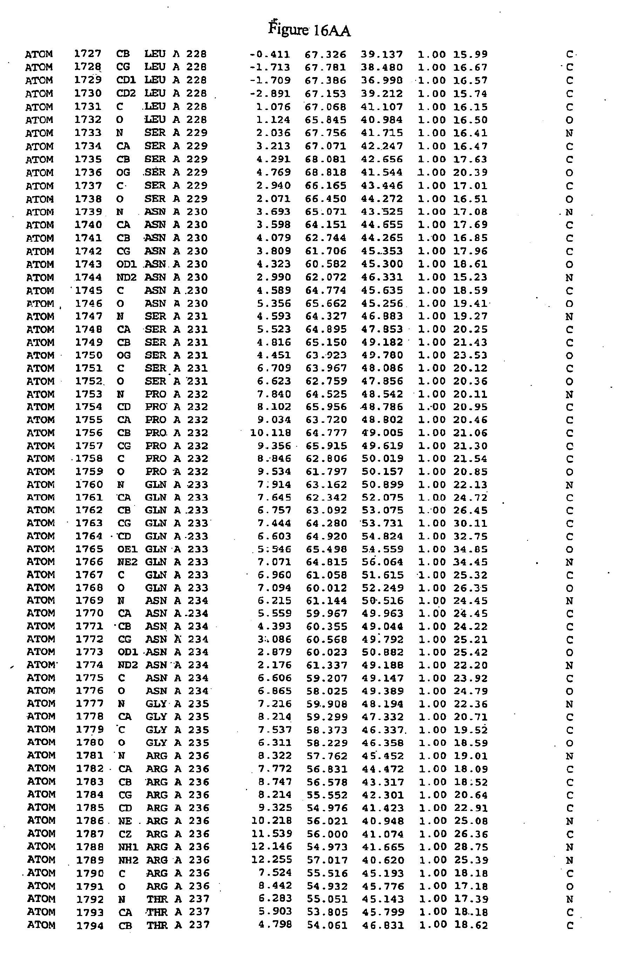

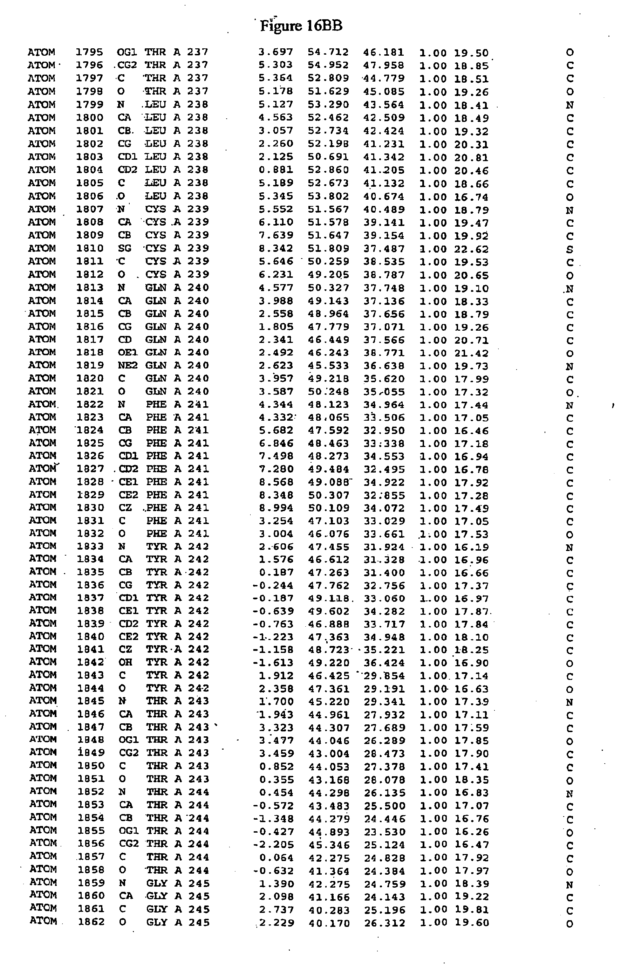

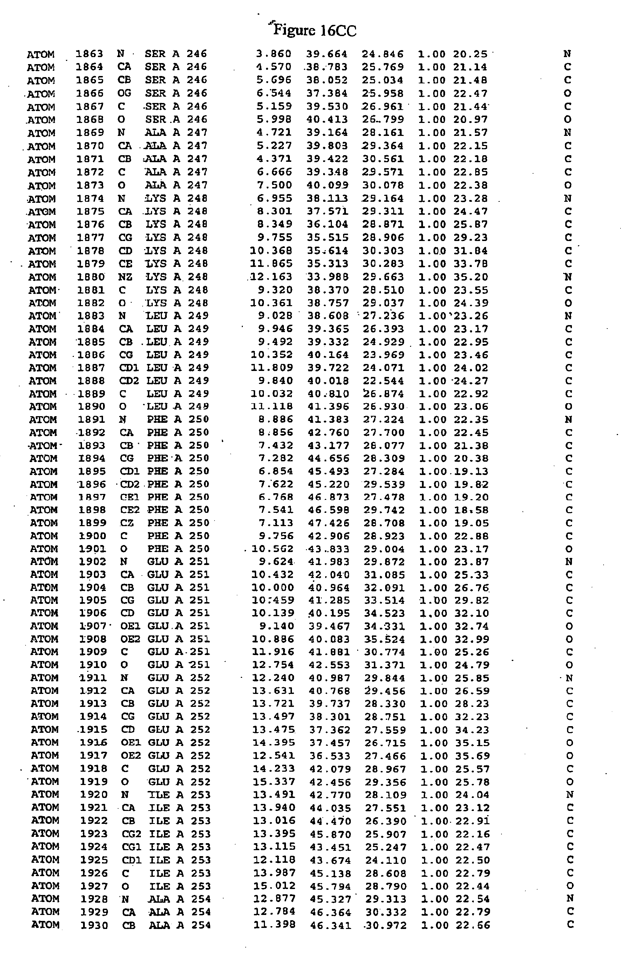

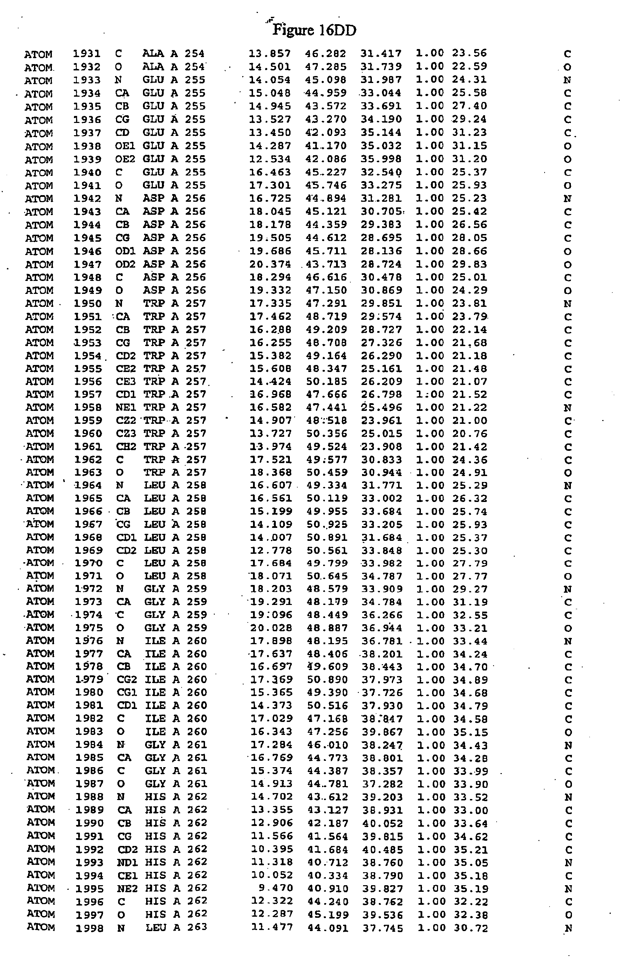

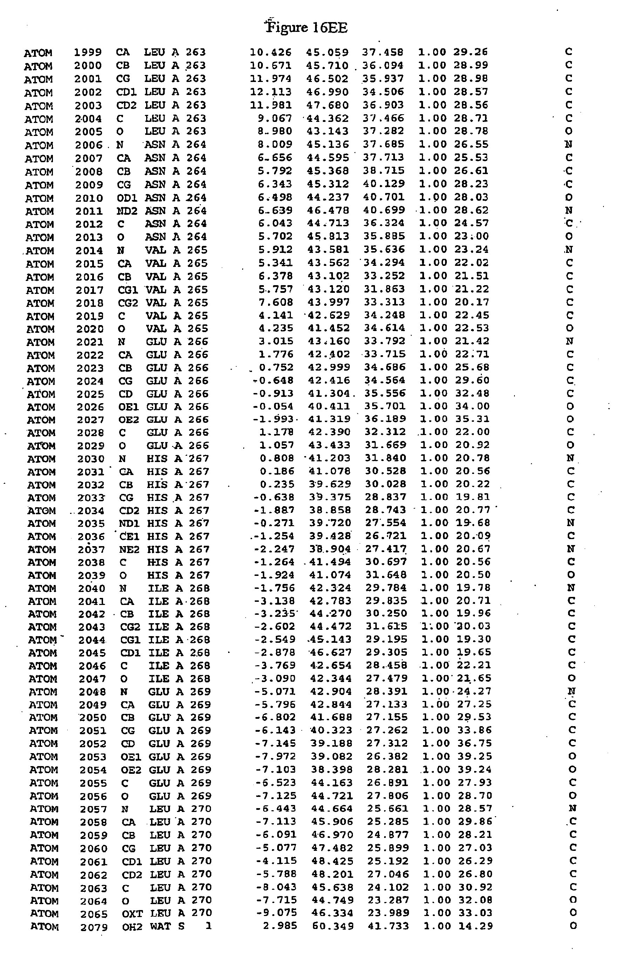

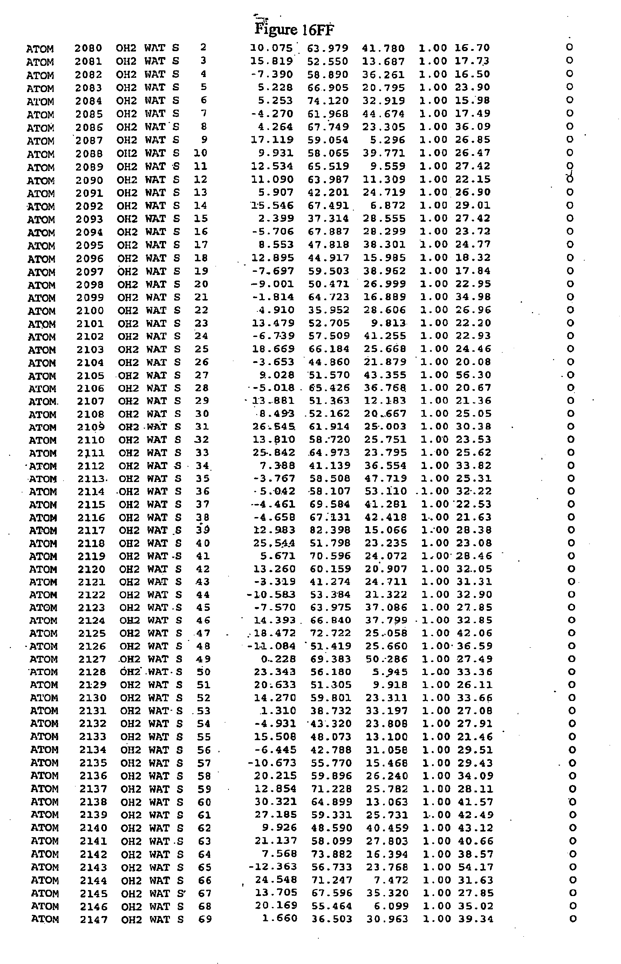

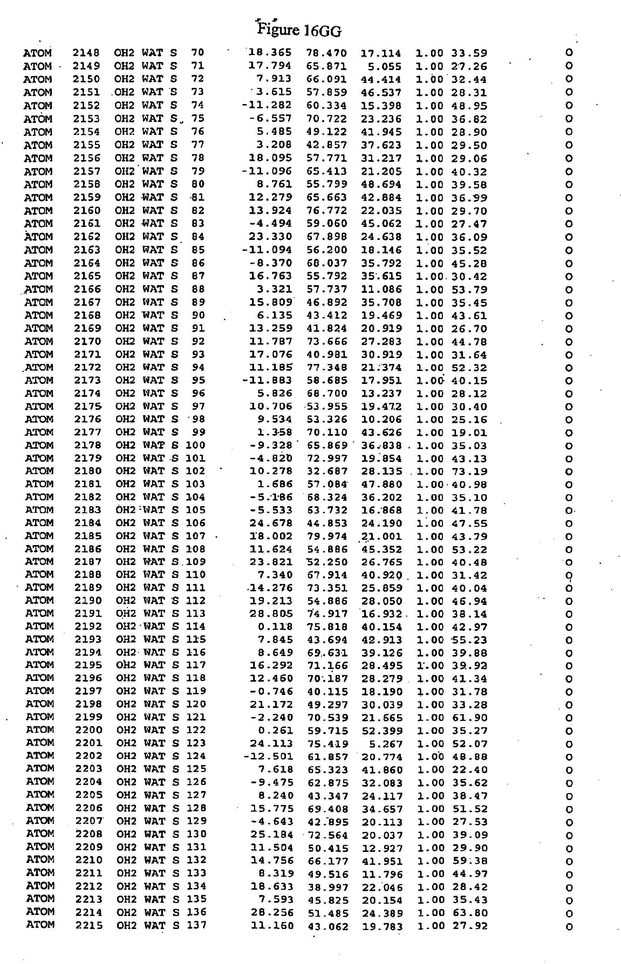

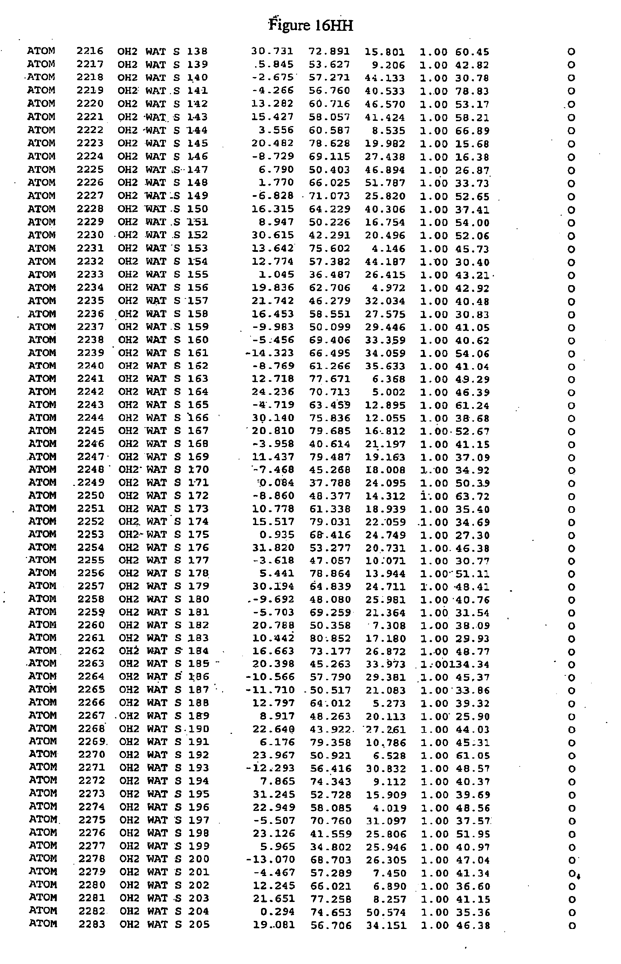

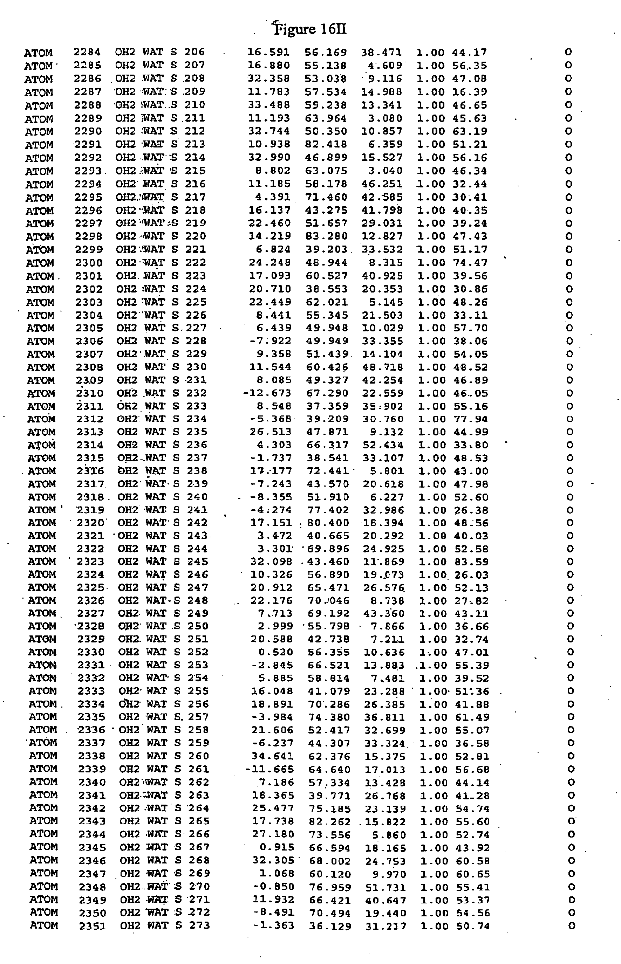

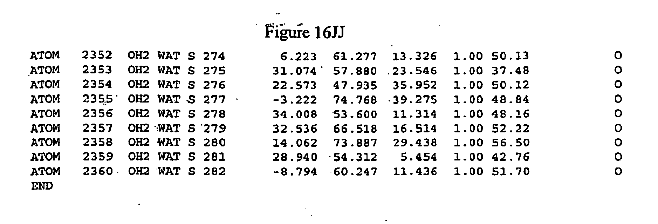

A further embodiment relates to a *E. faecium* MurI in which the substrate binding site comprises two conserved cysteine residues, denoted Cys77and Cys188 in the amino acid sequence represented as SEQ ID NO: 48. A further embodiment comprises a substrate binding site of *E*. *faecium* MurI wherein the binding site additionally comprises one or more of the following amino acid residues: Ser15, Thr79, Thr121, Thr124, Glu156, Thr189, and His190 as represented by the structural coordinates of Figure 16. In one embodiment, the substrate binding site of the *E. faecium* MurI includes amino acid residues within 5 Å of the Cys77and Cys188 residues as represented by the structural coordinates of Figure 16. In one embodiment, the substrate binding site of the *E. faecium* MurI additionally comprises one or more of the following amino acid residues: Ser15, Thr79, Thr121, Thr124, Glu156, Thr189, and His190. In this embodiment, the substrate binding site complexes with L-glutamate and comprises amino acid residues Cys77 and Cys188 and at least one (i.e., one or more) of the following amino acid residues: Ser15, Thr79, Thx121, Thr124, Glu156, Thr189, and His190, which can be present in any combination. In a further embodiment, the substrate binding site includes amino acid residues Ile13, Asp14, Ser15, Gly16, Va117, Gly18, Gly19, Thr21, Va122, Asp40, Arg43, Cys44, Pro45, Tyr46, Gly47, Phe48, Arg49, Va154, Met72, Ala76, Cys77, Asn78, Thr79, Ala80, Thr81, Ala82, Va199, Ile100, Ile119, Gly120, Thr121, Ile122, Gly123, Thr124, Val125, Tyr130, Phe149, Val152, Ser153, Glu156, Leu186, Gly187, Cys188, Thr189, His190, Tyr191 and Ser211 of SEQ ID NO: 48.

In a further embodiment, the substrate binding site of the MurI comprises two hydrogen bond TRIADs, which occur close to the conserved cysteine residues of the binding site. Specifically, on one side (Cys188) of the binding site, the TRIAD is Glu156-Thr189-His190 and on the other side (Cys77), the TRIAD is Thr79-Thr121-Thr124. Thus, in a specific embodiment, the substrate binding site of *E. faecium* MurI complexes with L-glutamate and comprises amino acid residues Cys77 and Cys188 and additionally includes least one (i.e., one or more) of the following: amino acid residues Ser15, Thr79, Thr121, Thr124, Glu156, Thr189, and His 190.

The threonine residues have features of interest as they relate to the binding site of the *E. faecium* MurI. On one side (Cys188) of the substrate binding site, Thr189 is H-bonded to His190 and His190 is further H-bonded to Glu156. On the other side of the substrate binding site (Cys77), Thr79 is H-bonded to Thr121 and further H-bonded to Thr124. The two TRIADs may play important roles in altering the pKa of the two substrate binding site cysteine residues (in addition to that of the neighboring hydrophobic core), facilitating the proton transfer during catalysis or both.

### b. Intermolecular Dimer Interface

One embodiment is crystallized *E. faecium* MurI having an intermolecular interface comprising at least one of amino acid residues: Gln29, Leu30, Pro31, Asn32, Glu86, Lys89, Ala90, Ala91, Leu92, Ser93, Ile94, Pro95, Val96, Ile97, Gly98, Val99, Ile100, Leu101, Pro102, Arg105, Lys109, Glu136, Lys137, Val138, Pro139, Glu214, Gly217, Glu218, Ser220, Met221, Leu222, Asp224, Tyr225, Phe226, Asn230, Ser231, and Pro232 of SEQ ID NO: 48.

### c. Intradomain Binding Site

One embodiment is crystallized *E. faecium* MurI having an intradomain dimer interface comprising at least one of amino acid residues: Asp14, Ser15, Gly16, Val17, Gly18, Gly19, Leu20, Thr21, Val22, Glu25, Lys28, Gln29, Arg43, Cys44, Pro45, Tyr46, Gly47, Pro48, Arg49, Pro50, Ala51, Val54, Ala76, Cys77, Asn78, Thr79, Ala82, Val83, Val99, Glu214, Thr215, Val216, Gly217, Glu218, Met221, Leu222, Leu249, Phe250, Glu252, Ile253, Asp256, and Trp257 of a first domain, and amino acid residues Ile100, Leu101, Pro102, Gly103, Thr104, Arg105, Ala106,Ala107, Val108, Arg109, Lys110, Thr121, Ile122, Gly123, Thr124, Ser127, Gln128, Ala129, Tyr130, Leu132, Ala133, Leu134, Gly136, Lys137, Pro149, Lys150, Phe151, Va1152, Va1155, Glu156, Ser157, Asn158, Ile185, Leu186, Gly187, Cys188, Thr189, His190, Tyr191, Pro192, Leu193, Ile209, Asp210, Ser211, Gly212, and Ala213 of a second domain, wherein the amino acid residues are represented by SEQ ID NO: 48.

### 3. S. aureus

### a. Substrate Binding Site

A further embodiment relates to *S*. *aureus* MurI in which the binding site comprises two conserved cysteine residues, denoted Cys72 and Cys184 in the sequence represented as SEQ ID NO: 46. A further embodiment comprises a binding site of *S*. *aureus* MurI wherein the binding site additionally comprises one or more of the following amino acid residues: Ser10, Thr74, Thr116, Thr119, G1u151, Thr1 85 and His186 as represented by the structural coordinates of Figure 14. In one embodiment, the binding site of *S*. *aureus* MurI complexes with D-glutamate and comprises amino acid residues Cys72 and Cys184 as well as amino acid residues within 5 Å of the Cys72 and Cys184 residues as represented by the structural coordinates of Figure 15. In one embodiment, the binding site of the *S*. *aureus* MurI additionally comprises one or more of the following amino acid residues: Ser10, Thr74, Thr116, Thr119, Glu151, Thr185 and His186. In this embodiment, the binding site complexes with D-glutamate and comprises amino acid residues Cys72 and Cys184 and at least one (i.e., one or more) of the following amino acid residues: Ser10, Thr74, Thr116, Thr119, G1u151, Thr185 and His186, which can be present in any combination. In a further embodiment, the binding site includes amino acid residues Ser10, Pro40, Tyr41, Gly42, Cys72, Asn73, Thr74, Thr116, Thr119, Glu151, Cys184, Thr185 and His186.

In a further embodiment, the binding site of the MurI comprises two hydrogen bond TRIADs, which occur close to the conserved cysteine residues of the binding site. Specifically, on one side (Cys184) of the binding site, the TRIAD is Gly151-Thr185-His186 and on the other side (Cys72), the TRIAD is Thr74-Thr116-Thr119. Thus, in a specific embodiment, the binding site of *S*. *aureus* MurI complexes with D-glutamate and comprises amino acid residues Cys72 and Cys184 and additionally comprises at least one (i.e., one or more) of the following: amino acid residues Ser10, Thr74, Thr116, Thr119, Glu151, Thr185 and His186.

The threonine residues have features of interest as they relate to the binding site of *S*. *aureus* Murl. On one side (Cys184) of the binding site, Thr185 is H-bonded to His186 and His186 is further H-bonded to Glu151. The hydroxyl oxygen (O) of Thr185 is 2.9 Å away from the amino nitrogen (N) of the substrate (i.e., glutamate) and 4.4 Å from the sulfur (S) atom of Cys184, which is 4.1 Å from the nitrogen of His186.

On the other side of the binding site (Cys72), Thr74 is H-bonded to Thr116 and further H-bonded to Thr119. The hydroxyl O of Thr74 is H-bonded to one of the carboxylate oxygen atoms of the substrate and is 4.3 Å away from the S atom of Cys72. Analysis showed that the three hydroxyl oxygens form a triangle, all within less than 3.4 Å from one another.

The two TRIADs may play important roles in altering the pKa of the two binding site cysteine residues (in addition to that of the neighboring hydrophobic core), facilitating the proton transfer during catalysis or both.

Another embodiment is crystallized *S*. *aureus* MurI, comprising a binding site comprising the amino acid residues Ser10, Pro40, Tyr41, Gly42, Cys72, Asn73, Thr74, Thr116; Thr1 19, Glu151, Cys184, Thr185 and His186 of the amino acid sequence represented by SEQ ID NO: 46. This crystal may be further complexed with D-glutamate. Additionally, the binding site of the crystal complexed with D-glutamate can comprise the amino acid residues: Cys72, Cys184, and one or more of the following amino acid residues: Ser10, Thr74, Thr116, Thr119, Glu151, Thr185 and His186. A further embodiment is one in which the binding site additionally comprises at least one of the following amino acid residues: Asp9, Gly11, Val12, Gly13, Gly14, Arg38, Cys39, Pro40, Tyr41, Gly42, Pro43, Arg44, Ala71, Asn73, Ala75, Va194, Gly115, Val147, Gly183 and Tyr187. A further embodiment is one in which the binding site additionally comprises at least one of the following amino acid residues: Ile8, Thr16, Val17, Tyr32, Asp35, Va149, Ile70, Ser76, Ala77, Lew114, Gly118, Ile120, Tyr125, Pro144, Pro148, Leu182 and Ser207. The crystals comprising binding sites represented by the above amino acid residues comprise the amino acid sequence is that represented by SEQ ID NO: 46; or an amino acid sequence is at least 75%, 80%, 85%, 90%, 95%, 98%, or 99% homologous to the amino acid sequence that is represented by SEQ ID NO: 46.

Location and geometry of a binding site of the enzyme are also defined. Two conserved cysteine (Cys) residues were identified as the residues responsible for the (de)protonation of the alpha-carbon of the substrate during catalysis, which is consistent with the two-base mechanism proposed for function of the enzyme in its role as a racemase. The two binding site cysteines are Cys72 and Cys184, which are about 7.3 Angstroms (Å) apart (Cα-Cα distance, i.e., the distance between Cα atoms). Other amino acid residues identified in the vicinity of the binding site include Ser10, Thr74, Thr116; Thr119, Glu151, Thr185 and His186. The bound substrate/product, D-glutamate, is located between the two conserved cysteine residues. Further detail of the binding site is as follows: looking down the axis defined by the Glu151 along the γ and α carbon, the two cysteines exhibit a rather symmetrical environment; each has a hydrophobic core with respect to the substrate and a neighboring threonine residue not far from the substrate C-α (3.4 - 4.3 Å), respectively.

Analysis of the crystal structure of the *S. aureus* MurI indicates that the following amino acid residues are within 10 Å of the bound D-glutamate: 8-17, 32-44, 49, 52, 71-79, 94-95, 114-120, 122, 125, 184-190, and 207-208. Analysis of the crystal structure also shows that the following amino acid residues are within 4 Å of the bound D-glutamate: 9-10, 39-42, 72-74, 116, 119, and 184-186. Of interest is the fact that only one acidic amino acid residue (Asp9) is present in the structure surrounding the binding site Cys72, which serves as an anchoring point for the amino group of the substrate/product with a polar (charged) interaction between the amino nitrogen atom of D-glutamate and the delta oxygen atom of Asp9.

### b. Intermolecular Dimer Interface

One embodiment is crystallized *S. aureus* MurI having an intermolecular interface comprising at least one of amino acid residues: Gln24, Leu25, Pro26, Asn27, Glu81, Glu84, Ser90, Va191, Ile92, Glu96, Pro97, Arg100, Thr101, Met104, Arg131, Ile132, Asn133, Pro134, Arg213, Glu214, Ser216, Ala217, Leu218, Thr220, Phe221, Ala226, Ser227, and Tyr228 of SEQ ID NO: 46.

### c. Intradomain Binding Site

One embodiment is crystallized *S. aureus* MurI having an intradomain dimer interface comprising at least one of amino acid residues: Asp9, Ser10, Gly11, Val12, Gly13, Gly14, Leu15, Thr16, Val17, Glu20, Cys39, Pro40, Tyr41, Gly42, Pro43, Arg44, Pro45, Gly46, Val49, Ala71, Cys72, Asn73, Thr74, Ala77, Val78, Val94, Ile95, Glu210, Thr211, Ala212, Arg213, Glu214, Ala217, Leu218, His244, Asn247, Ile248, Glu251, and Trp252 of a first domain and amino acid residues Ile95, Glu96, Pro97, Gly98, Ala99, Arg100, Thr101, Ala102, Ile103, Met104, Thr105, Thr116, Glu117, Gly118, Thr119, Ser122, Glu123, Ala124, Tyr125, His128, Arg131, De132, Pro144, Gly145, Phe146, Val147, Val150, Glu151, Gln152, Met153, De181, Leu182, Gly183, Cys184, Thr185, His186, Tyr187, Pro188, Leu189, Ile205, Ser206, Ser207, Gly208, and Leu209 of a second domain, wherein the amino acid residues are represented by SEQ ID NO: 46.

### C. Atypical Gram negative bacteria

As described in the examples that follow, MurI of *H. pylori* has been crystallized, and crystal structures (three-dimensional structure) determined. The structures determined represent that of MurI alone, and MurI in complex with an inhibitor or in complex with the enzyme substrate, glutamate, in which glutamate can be in the L- or D-form. NMR data suggests that MurI has stable structural elements in the absence of substrate. Crystallization of *H. pylori* MurI is described in Example 1 and Figures 4-7.

Experimental results show that the unit cell of crystals of *H. pylori* MurI can be a monomer, a symmetrical or nonsymmetrical dimer, or a multimer, depending upon the crystallization conditions and that the active form of MurI from atypical bacterium is a dimer. Each monomer consists of two distinct and very similar alpha/beta type domains that interact at a molecular interface.

The *H. pylori* MurI dimer is held together by a stable and conserved hydrophobic core created by a four helix bundle (e.g., two helices from each monomer, residues 143 to 169) that link the two domains and thereby the two monomers rather rigidly together. This arrangement leaves the other two domains of each monomer free to move in order to give access to the two active sites which occur at the dimer interface. NMR data shows that the protein is also a dimer in the absence of a ligand, and is more flexible in this state than when ligand is bound. Thus, the molecular interface of MurI from an atypical bacterium exists at the junctions of two domains and functions as a flexible element by which a change in conformation opens the substrate binding site, allowing substrate to bind MurI. The degree of movement of the molecular interface of *H. pylori* MurI is less flexible that of typical Gram negative bacteria due to the strict requirement of an activator for substrate binding and enzyme activity in typical Gram negative bacteria.

The amino acid residues which comprise the binding site of MurI (MurI) are conserved in seventeen strains of *H*. *pylori* that have been sequenced.

One embodiment relates to a binding site of a *H. pylori* MurI. As used herein, the term "binding site" refers to a specific region (or atom) of MurI that interacts with another molecular entity. In certain embodiments, a binding site may comprise, or be defined by, the three dimensional arrangement of one or more amino acid residues within a folded polypeptide.

In the present invention, a substrate can be a compound such as L-glutamate or D-glutamate which MurI reversibly converts from the R- to S-enantiomer. Thus, a substrate can also act a product. The substrate can be a naturally-occurring or artificial compound.

In the present invention, an inhibitor can be a compound which also may undergo a catalytic reaction, bind to the substrate binding site, or another site on MurI and which competes with substrate turnover of glutamate. Inhibitors of the present invention can be a compound such as L-serine-O-sulfate, D-serine-O-sulfate, D-aspartate, L-aspartate, tartrate, citrate, phosphate, sulfate, aziridino-glutamate, N-hydroxyglutamate, or 3-chloroglutamate. The inhibitor can be a naturally-occurring or artificial compound.

One embodiment relates to a binding site of *H. pylori* MurI, in which the binding site comprises two conserved cysteine residues, denoted Cys70 and Cys181 as represented by the amino acid sequence of SEQ ID NO: 2. A further embodiment comprises a binding site of *H. pylori* MurI wherein the binding site additionally comprises one or more of the following amino acid residues: Ser8, Thr72, Thr116, Thr119, Thr182, His183 and Glu150 as represented by the structural coordinates of Figure 4. In one embodiment, the binding site of the *H. pylori* MurI is complexed with D-glutamate and comprises amino acid residues Cys70 and Cys181 as well as amino acid residues within 5 Å of the Cys70 and Cys181 residues as represented by the structural coordinates of Figure 5. In one embodiment, the binding site of *H*. *pylori* MurI additionally comprises one or more of the following amino acid residues: Ser8, Thr72, Thr116, Thr119, Thr182, His183 and Glu150. In this embodiment, the binding site is complexed with D-glutamate and comprises amino acid residues Cys70 and Cys181 and at least one (i.e., one or more) of the following amino acid residues: Ser8, Thr72, Thr116, Thr119, Thr182, His183 and Glu150, which can be present in any combination. In a further embodiment, the binding site includes amino acid residues Asp7, Ser8, Gly9, Val10, Gly11, Gly12, Val37, Pro38, Tyr39, Gly40, Thr41, Ala69, Cys70, Asn71, Thr72, Ala73, Gly115, Thr116, Lys117, Ala118, Thr119, Val146, Ile149, Glu150, Gly180, Cys181, Thr182, and His183.

In a further embodiment, the binding site of the MurI comprises two hydrogen bond TRIADs, which occur close to the conserved cysteine residues of the binding site. Specifically, on one side (Cys181) of the binding site, the TRIAD consists essentially of Thr182-His183-Glu150 and on the other side (Cys70), the TRIAD consists essentially of Thr72-Thr116-Thr119. Thus, in a specific embodiment, the binding site of *H. pylori* MurI is complexed with D-glutamate and comprises amino acid residues Cys70 and Cys181 and additionally comprises at least one (i.e., one or more) of the following: amino acid residues Ser8. Thr72, Thr116, Thr119, Thr182, His183, and Glu150.

In addition, both TRIADs involve conserved residues throughout all available *H. pylori* MurI sequences. The threonine residues have features of interest as they relate to the binding site of the *H. pylori* MurI. On one side (Cys181) of the binding site, Thr182 is H-bonded His183 and His183 is further bonded to Glu150. All three residues are conserved for all MurI proteins in *H. pylori* strains tested. The hydroxyl oxygen (O) of Thr182 is 3 Å away from the amino nitrogen (N) of the substrate (i.e., glutamate) and 4.4 Å from the sulfur (S) atom of Cys 181.

On the other side of the binding site (Cys70), Thr72 is H-bonded to Thr116 and further H-bonded to Thr119. The hydroxyl oxygen (O) of Thr72 is 3.4 Å away from one of the carboxylate oxygen (O) atoms of the substrate and is 4.5 Å away from the sulfur (S) atom of Cys70. Analysis showed that the three hydroxyl oxygens of the threonines form a triangle, all within less than 3.2 Å from one another.

The two TRIADs may play important roles in altering the pKa of the two binding site cysteine residues (in addition to that of the neighboring hydrophobic core), facilitating the proton transfer during catalysis or both.

Another embodiment is crystallized MurI, comprising a binding site comprising the amino acid residues Asp7, Ser8, Gly9, Val10, Gly11, Gly12, Val37, Pco38, Tyr39, Gly40, Thr41, Ala69, Cys70, Asn71, Thr72, Ala73, Gly115, Thr116, Lys117, Ala118, Thr119, Val146, Ile149, Glu150, Gly180, Cys181, Thr182, and His183 of the amino acid sequence represented by SEQ ID NO: 2. This crystal may be further complexed with D-glutamate. Additionally, the binding site of the crystal complexed with D-glutamate can comprise the amino acid residues: Cys70, Cys181, and one or more of the following amino acid residues: Ser8, Thr72, Thr116, Thr119, Thr182, His183 and Glu150. A further embodiment is one in which the binding site additionally comprises at least one of the following amino acid residues: Asp7, Gly9, Va110, Gly11, Gly12, Arg36, Va137, Pro38, Tyr39, Gly40, Thr41, Lys42, Ala69, Asn71, Ala73, Val92, Glyl15. Va1146, Gly180, Phe184. A further embodiment is one in which the binding site additionally comprises at least one of the following amino acid residues: Phe6, Asp7, Gly9, Val10, Gly11, Gly12, Ser14, Val15, Tyr30, Asp33, Arg36, Val37, Pro38, Tyr39, Gly40, Thr41, Lys42, Ile47, Val68, Ala69, Asn71, Ala73, Ser74, Ala75, Va192, Leul14, Glyl15, Lysl17, Alal18, Ilel20, Tyrl25, Ser143, Val146, Prol47, Leu179, Gly180, Phe184, and Ser210.

The crystals with binding sites comprising the above amino acid residues comprise the amino acid sequence that is represented by SEQ ID NO: 2; or an amino acid sequence that is at least 75%, 80%, 85%, 90%, 95%, or 98% homologous to the amino acid sequence that is represented by SEQ ID NO: 2.

The crystals of the present invention diffract to about 1.86 Å to about 3.0 Å, and could be refined to about 1.5 Å.

Location and geometry of a binding site of the enzyme are also defined. Two conserved cysteine (Cys) residues are identified as the residues responsible for the (de)protonation of the alpha-carbon of the substrate (D-glutamate) during catalysis, which is consistent with the two-base mechanism proposed for how the enzyme functions in its role as a racemase. The two binding site cysteines are Cys70 and Cys181, which are about 7.5 Angstroms (Å) apart (Cα-Cα distance, i.e., the distance between Cα atoms). Other amino acid residues identified in the vicinity of the binding site include Ser8, Thr72, Thr182, His183 and Glu150. The bound substrate/product, D-glutamate, is located between the two conserved cysteine residues. Further detail of the binding site is as follows: looking down the axis defined by the Glu150 along the γ and α carbon, the two cysteines exhibit a rather symmetrical environment; each has a hydrophobic core behind, with respect to the substrate and a neighboring threonine residue not far from the substrate C-alpha (3.7 - 4 Å), respectively.

Analysis of the crystal structure of the *H. pylori* MurI indicates that the following amino acid residues are within 10 Å of the bound D-glutamate: 6-15, 30-42, 47, 50, 68-76, 210-211, 179-185, 114-120, 122, 92-93, 125, 35, 37, 38, and 42. Note that residues 35,37, 38 and 42 are from the other molecule of the dimer. Analysis of the crystal structure also shows that the following amino acid residues are within 4 Å of the bound D-glutamate: 7-8, 37-40, 70-72, 116, 119, and 181-183. Of interest is the fact that only one acidic amino acid residue (Asp7) is present in the structure surrounding the binding site Cys70, which serves as an anchoring point for the amino group of the substrate/product with a polar (charged) interaction between the amino nitrogen atom of D-glutamate and the delta oxygen atom of Asp7.

Location and geometry of the active site of the enzyme were also defined. Two key residues were identified as the residues responsible for creating the pocket for the inhibitor, interacting with the core ring system in its new position, and fixing the position of the central core. When inhibitor is bound, Cγ of Leu186 and Cβ of Trp252 are 8.9 Angstroms (Å) apart.

### 1. Substrate Binding Site

One embodiment is crystallized *H. pylori* MurI having a substrate binding site comprising the amino acid residues Cys70, Cys181, Ser8, Thr72, Thr116, Thr119, Thr182, His183 and Glu150 of the amino acid sequence represented by SEQ ID NO: 2. Additionally, the binding site of the crystal complexed with D-glutamate can comprise amino acid residues: Cys70, Cys181, and one or more of the following amino acid residues: Ser8, Thr72, Thr116, Thr119, Thr182, His183 and Glu150. A further embodiment is one in which the binding site additionally comprises at least one of the following amino acid residues: Asp7, Gly9, Val10, Gly11, Gly12, Arg36, Val37, Pro38, Tyr39, Gly40, Thr41, Lys42, Ala69, Asn71, Ala73, Val92, Gly115, Val146, Gly180, Phe184. A further embodiment is one in which the binding site additionally comprises at least one of the following amino acid residues: Phe6, Asp7, Gly9, Va110, Gly11, Gly12, Ser14, Val15, Tyr30, Asp33, Arg36, Val37, Pro38, Tyr39, Gly40, Thr41, Lys42, Ile47, Va168, Ala69, Asn71, Ala73, Ser74, Ala75, Va192, Leul14, Gly115, Lys117, Ala118, Ile120, Tyr125, Ser143, Val146, Pro147, Leu179, Gly180, Phe184, and Ser210.

### 2. Intermolecular Dimer Interface

One embodiment is crystallized *H. pylori* MurI having a intermolecular dimer interface comprising at least one of amino acid residues Ser34, Ala35, Arg36, Val37, Pro38, Tyr39, Gly40, Thr41, Lys42, Asp43, Pro44, Thr46, Phe50, Lys117. Asn121, Ser143, Leu144, Pro147, Leu148, Glu150, Glu151, Ser152, Ile153, Gly157, Leu158, Thyr161, Cys162, Tyr165, Tyr166, Ser239, Gly240, Asp241, and Trp244 of SEQ ID NO: 2.

### 3. Intradomain Binding Site

One embodiment is crystallized *H. pylori* MurI having a intradomain interface comprising at least one of amino acid residues Asp7, Ser8, Gly9, Val10, Gly11, Gly12, Phe13, Ser14, Val15, Ser18, Lys21, Ala22, Val37, Pro38, Tyr39, Gly40, Thr41, Lys42, Asp43., Pro44, Ile47, Ala69, Cys70, Asn71, Thr72, Ser74, Ala75, Leu76, Gly91, Val92, Gly211, Asp212, Ala213, Ile214, Val215, Glu216, Tyr217, Leu218; Gln219, Gln220, Lys221, Glu251, Trp252, Leu253, Lys254, and Leu255 of a first domain, and amino acid residues Ile93, Glu94, Pro95, Ser96, Ile97, Leu98, Ala99, Ile100, Arg102, Gln103, Thr116, Lys117, Ala118, Thr119, Ser122, Asn123, Ala124, Tyr125, Ala128, Gln131, Gln132, Ser143, Val146, Pro147, Ile149, Glu150, Glu151, Ser152, Ile178, Leu179, Gly180, Cys181, Thr182, His183, Phe184, Pro185, Leu186, Ile208, His209, Ser210, Gly211, and Asp212 of a second domain of SEQ ID NO: 2.

### 4. Inhibitor Binding Site

In the present invention, the amino acid residues to which the inhibitor binds an inhibitor binding site.

One embodiment is an inhibitor binding site of *H. pylori* MurI, in which the inhibitor binding site comprises two key residues, denoted as Leu186 and Trp252 in the amino acid sequence represented as SEQ ID NO: 2. In a further embodiment, the binding site of the *H. pylori* MurI is complexed with an inhibitor and comprises amino acid residues Leu186 and Trp252 as well as amino acid residues within 5 Å, 7 Å, 8 Å, 10 Å, 15 Å, or 20 Å of the Leu186 and Trp252 residues of SEQ ID NO: 2.

In one embodiment of the present invention, the inhibitor binding site of *H*. *pylori* MurI is complexed with an inhibitor, and the inhibitor binding site comprises amino acid residues Leu186 and Trp252, and additionally comprises at least one (i.e., one or more) of the following: amino acid residues Val10, Gly11, Phe13, Ile149, Glu151, Ser152, Trp244, and Gln248 of SEQ ID NO: 2. In a further embodiment, the inhibitor binding site additionally comprises amino acid residues of at least one (i.e., one or more) of the following: amino acid residues: Gly12, Ser14, Lys17, Glu150, Leu154, Thr182, and His183. In a further embodiment, the inhibitor binding site additionally comprises amino acid residues of at least one (i.e., one or more) of the following: amino acid residues: Phe13, Trp244, and Leu253. Alternatively, we provide a crystal of MurI, comprising an inhibitor binding site comprising the amino acid residues Val10, Gly11, Gly12, Phe13, Ser14, Lys17, Ile149, Glu150, Glu151, Ser152, Thr182, His183, Leu186, Trp244, Gln248, Trp252 and Leu253.

### VI. Inhibitors

Inhibitors (such as small molecules, proteins, polypeptides, and peptides, etc.) can be utilized to bind a binding domain of MurI or the immediate surrounding area and prevent the movement of MurI such that substrates could not bind the substrate binding site (SBS). In doing so, blockage of MurI would prevent building of the bacterial cell wall in a manner similar to antibiotics. However, since MurI is ubiquitous to all bacteria, it is expected that an inhibitor that blocks the molecular interface will be a broad spectrum inhibitor that can block an entire genera of bacteria. Inhibitors of the present invention partially, or fully, block the molecular interface of MurI.

The terms "small molecule" and "small compound" as used herein, are meant to refer to composition, that have a molecular weight of less than about 5 kD and most preferably less than about 2.5 kD. Small molecules and small compounds are used interchangeable herein. Small molecules can be nucleic acids, peptides, polypeptides, peptidomimetics, carbohydrates, lipids or other organic (carbon containing) or inorganic molecules. Many pharmaceutical companies have extensive libraries of chemical and/or biological mixtures comprising arrays of small molecules, often fungal, bacterial, or algal extracts, which can be screened to identify an inhibitor of MurI.

An example is crystallized MurI in complex with an inhibitor (e.g., an antibacterial binding agent, drug, etc.). A specific example is crystallized *H. pylori* MurI in complex with an inhibitor. In a further embodiment, the crystallized complex is characterized by the structural coordinates depicted in Figure 7, in which the determined structures presented represent that of MurI in complex with an inhibitor. In a further embodiment, the inhibitor is a pyrimidinedione, such as an imidizolyl pyrimidinedione, a thiophenyl pyrimidinedione, a furanyl pyrimidinedione, a pyrazolo pyrimidinedione, or a pyrrolyl pyrimidinedione.

In further embodiments, the pyrimidinedione is compound A, compound B, compound C, compound D, compound E, compound F, compound G, compound H, compound I, compound J, compound K, compound L, compound M, compound N, compound O, compound P, compound Q, compound R, compound S, compound T, compound U, compound V, compound W, compound X, compound Y, compound Z, compound AA, compound AB, compound AC, compound AD, compound AE, compound AF, compound AG, compound AH, compound AI, compound AJ, or compound AK. The crystalline complexes are characterized by the space groups and cell dimensions depicted in Table 5.

**Table 5.**

| Compound | Structure | Space Group | Cell Dimensions |
|---|---|---|---|
| A | 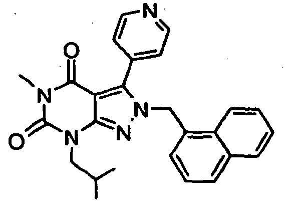 | P2₁2₁2₁ | a= 61.41 b= 76.31 c= 108.92 alpha= 90 beta= 90 gamma= 90 |
| B | 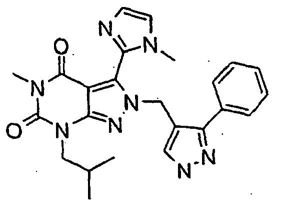 | P2₁ | a=57.27 b=76.59 c=60.00 alpha= 90 beta= 98.74 gamma= 90 |
| C | 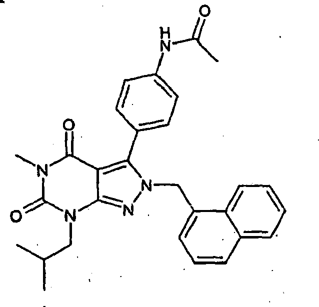 | P2₁2₁2₁ | a= 61.54 b= 75.80 c= 108.13 alpha= 90 beta= 90 gamma= 90 |
| D | 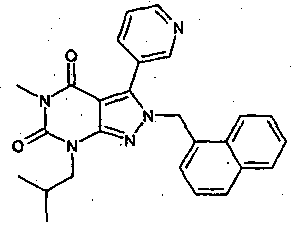 | P2₁2₁2₁ | a= 61.67 b= 75.63 c= 108.18 alpha= 90 beta= 90 gamma= 90 |
| E | 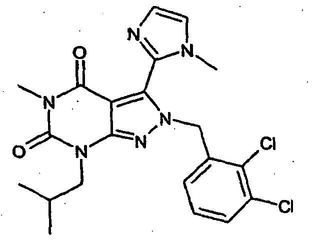 | P2₁ | a= 56.06 b= 62.10 c= 75.86 alpha= 90 beta= 93.46 gamma= 90 |
| F | 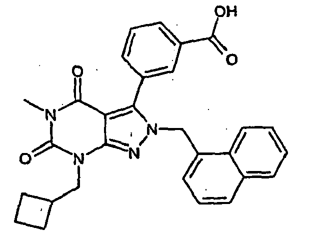 | P2₁2₁2 | a= 59.92 b= 78.21 c= 56.86 alpha= 90 beta= 90 gamma= 9 |
| G | 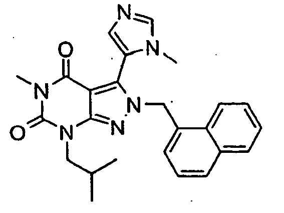 | P2₁2₁2₁ | a= 61.9 b= 76.0 c= 108.9 alpha= 90 beta= 90 gamma= 90 |
| H | 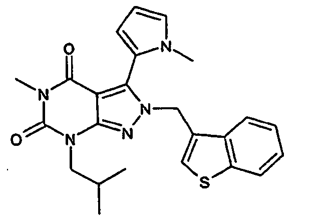 | P2₁2₁2 | a=61.0 b=78.7 c=57.0 alpha= 90 beta= 90 gamma= 90 |
| I | 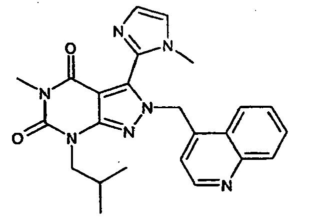 | P2₁ | a= 57.47 b= 77.03 c= 60.57 alpha= 90 beta= 98.96 gamma= 90 |
| J | 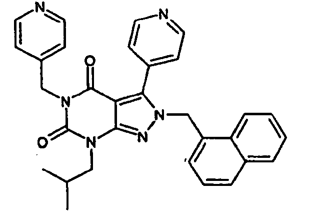 | P2₁2₁2₁ | a= 61.21 b= 75.19 c= 107.77 alpha= 90 beta= 90 gamma= 90 |
| K | 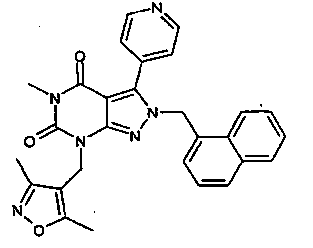 | P2₁ | a= 56.61 b= 76.45 c= 60.24 alpha= 90 beta= 99.05 gamma= 90 |
| L | 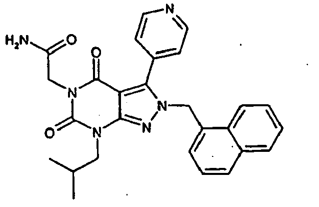 | P2₁2₁2₁ | a= 61.78 b= 75.59 c= 108.14 alpha= 90 beta= 90 gamma= 90 |
| M | 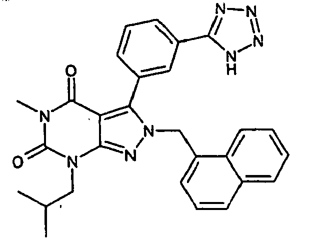 | P2₁ | a= 56.04 b= 76.19 c= 60.16 alpha= 90 beta= 98.56 gamma= 90 |
| N | 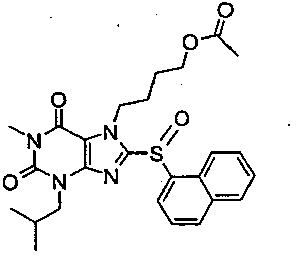 | P2₁2₁2₁ | a= 61.72 b=75.47 c= 108.23 alpha= 90 beta= 90 gamma= 90 |
| O | 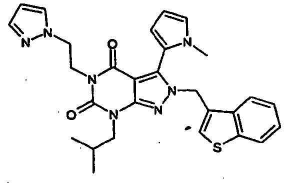 | P2₁ | a= 57.063 b= 77.958 c= 58.55 alpha= 90 beta= 97.91 gamma= 90 |
| P | 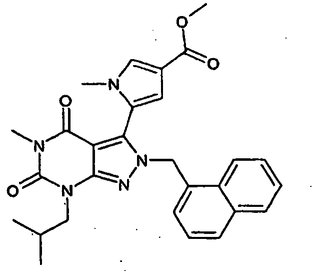 | P2₁2₁2 | a= 60.419 b= 78.322 c= 56.680 alpha= 90 beta= 90.0 gamma= 90 |
| Q | 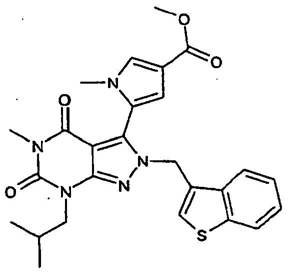 | P2₁ | a= 57.557 b= 77.694 c= 60.192 alpha= 90 beta= 98.999 gamma= 9 |
| R | 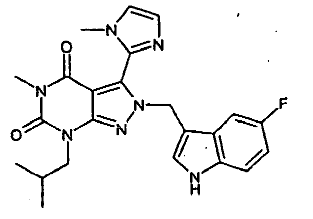 | P2₁ | a= 57.140 b= 62.179 c= 75.679 alpha= 90 beta= 94.045 gamma= 90 |
| S | 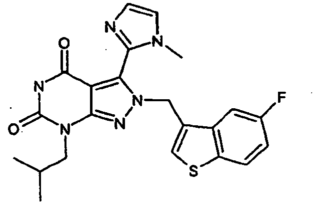 | P2₁ | a= 55.969 b= 62.338 c= 75.846 alpha= 90 beta= 93.431 gamma= 90 |
| T | 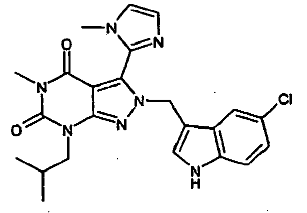 | P2₁ | a= 57.50 b= 77.08 c= 60.62 alpha= 90 beta= 98.93 gamma= 90 |
| U | 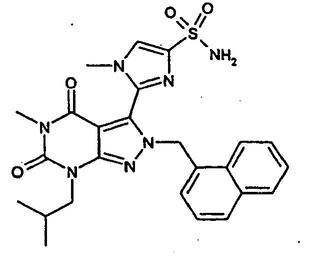 | P2₁ | a= 57.22 b= 62.07 c= 75.66 alpha= 90 beta= 93.99 gamma= 90 |
| V | 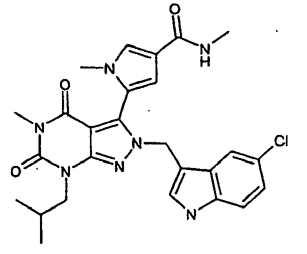 | P2₁2₁2 | a= 60.67 b= 77.47 c= 56.57 alpha= 90 beta= 90 gamma= 90 |
| X | 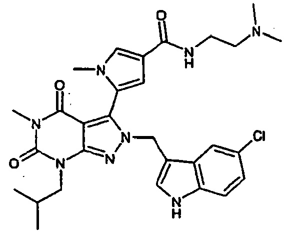 | P2₁2₁2 | a= 61.8 b= 75.9 c= 108.3 alpha= 90 beta= 90 gamma=90 |
| Y | 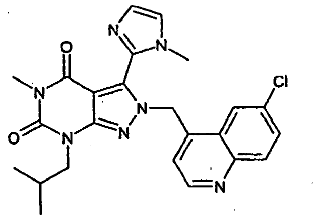 | P2₁ | a= 56.61 b= 76.45 c= 60.24 alpha= 90 beta= 99.05 gamma= 90 |
| Z | 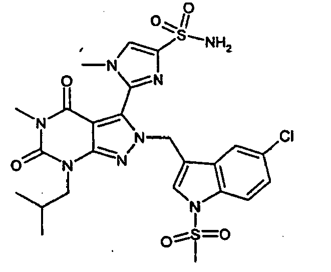 | P2₁ | a= 56.16 b= 61.83 c= 75.60 alpha= 90 beta= 90 gamma= 90 |
| AA | 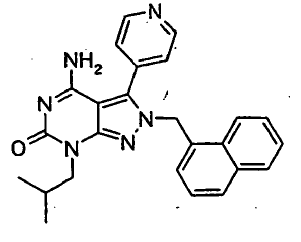 | P2₁2₁2 | a= 60.36 b= 77.34 c= 55.70 alpha= 90 beta= 90 gamma= 90 |
| AB | 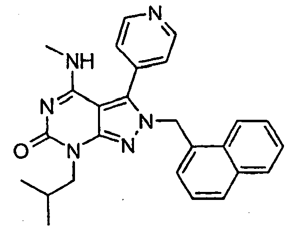 | P2₁2₁2 | a= 60.69 b= 77.90 c= 56.88 alpha= 90 beta= 90 gamma= 90 |
| AC | 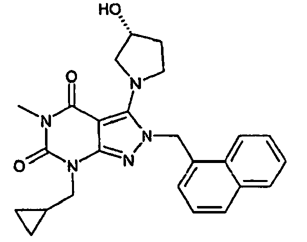 | P2₁2₁2 | a= 59.55 b= 79.29 c= 57.47 alpha= 90 beta= 90 gamma=90 |
| AD | 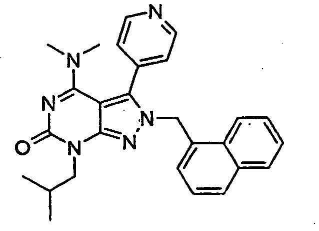 | P2₁2₁2 | a= 60.675 b= 77.47 c= 56.57 alpha= 90 beta= 90 gamma= 90 |
| AE | 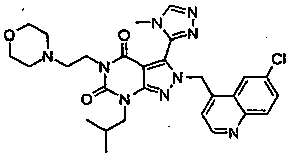 | P2₁2₁2₁ | a= 61.72 b= 75.48 c= 108.23 alpha= 90 beta= 90 gamma= 90 |

An exemplary example of the present invention is a crystal *of H. pylori* MurI complexed with an antibacterial binding agent and the product (substrate) D-glutamate wherein the MurI is at least 70%, 75%, 80%, 85%, 90%, 95%, 98%, or 99% homologous to the amino acid sequence represented by any one of SEQ ID NOS: 2-34, 40, 44, 46, 48, 50, 52, 54, 56, 58, 60, 62, 64, 66, and 74, or a portion thereof. In the present invention, the crystals diffract from about 0.8 Å to about 3.5 Å.

### VII. Computer-Assisted Methods of Identifying MurI Inhibitors

Also the.subject of this invention is a computer-assisted method for identifying a potential modifier, particularly a potential inhibitor, of MurI activity. The method comprises providing a computer modeling application with a set of relative structural coordinates of MurI, or a binding site thereof, wherein the set of relative structural coordinates is selected from a set of relative structural coordinates of MurI; supplying the computer modeling application with a set of structural coordinates of a candidate inhibitor of MurI; comparing the two sets of coordinates and determining whether the candidate inhibitor is expected to bind, or interfere with, the MurI, or a binding site thereof. Binding to, or interference with MurI, or a binding site thereof, is indicative of an inhibitor of MurI activity and, thus, indicative of an antibacterial agent. In most instances, determining whether the candidate inhibitor is expected to bind, or interfere with, the MurI, or a binding site thereof, includes performing a fitting operation or comparison between the candidate inhibitor and the MurI, or binding site thereof, followed by computational analysis of the outcome of the comparison in order to determine the association between, or the interference with, the candidate inhibitor and MurI or binding site. A candidate inhibitor identified by such methods is a candidate antibacterial drug. Optionally, a candidate drug can be synthesized or otherwise obtained and further assessed (e.g., *in vitro,* in cells or in an appropriate animal model) for.its ability to inhibit MurI.

In a specific embodiment, the computer-assisted method of identifying an agent that is a binding agent of MurI comprises the steps of (1) supplying the computer modeling application the coordinates of a known agent that binds a molecular interface of MurI and the coordinates of MurI; (2) quantifying the fit of an agent that binds the molecular interface of MurI to MurI; (3) supplying the computer modeling application with a set of structural coordinates of an agent to be assessed to determine if it binds a molecular interface of MurI; (4) quantifying the fit of the test agent in the molecular interface using a fit function; (5) comparing the fit calculation for the known agent with that of the test agent; and (6) selecting a test agent that has a fit that is better than, or approximates the fit of the known agent.

In a specific embodiment, the computer-assisted method of identifying an agent that is a binding agent of MurI comprises the steps of (I) supplying the computer modeling application the coordinates of an activator and/or a substrate, and a MurI, (2) quantifying the fit of a known binding agent of MurI to MurI, (3) supplying the computer modeling application with a set of structural coordinates of an agent to be assessed to determine if it binds a binding domain of Murl, (4) quantifying the fit of the test agent in the binding site using a fit function, (5) comparing the fit calculation for the known agent with that of the test agent, and (6) selecting a test agent that has a fit that is better than, or approximates the fit of the known agent.

One embodiment relates to a process which may be used to identify MurI inhibitors having the steps of:
(1) providing one or more molecular structures individually or as members of any suitable commercial or proprietary structure-searchable database of chemical compounds;
(2) selecting the molecular structures from step (1) to be converted into three-dimensional structures and placed into a binding/accessory site of MurI such that the moiety is constrained to make the appropriate hydrogen bond interactions;
(3) analyzing the remainder of the constrained molecular structure to determine if it contains any other suitably placed moiety or moieties which allows one to confirm whether the group(s) fit appropriately into the binding or accessory site of MurI;
(4) analyzing the molecular structures selected from step (3) to determine if the distances and the polar/non-polar surface areas are checked to determine whether they are within the specified ranges.

It would be apparent to one skilled in the art that the above steps do not need to be performed in the above order. Alternatively, one skilled in the art would recognize that fragments of the moiety or moieties described above could be sufficient to inhibit MurI activity if they substantially fill the binding domain (site) or accessory site, and such fragments could be tested *in vitro* for inhibitory activity. These fragments may be obtained by reference to the generic and specific examples provided in this application or by searching other structures that have the required inter-group distances and polar and non-polar surface areas.

Bacterial MurI inhibitors may also be obtained by modifying compound structures to include the pharmacophore features described above.

### VIII. Computer-Assisted Methods of Screening MurI Inhibitors

One skilled in the art may use one of several methods to screen chemical entities or fragments for their ability to associate with MurI and more particularly with the individual binding domains of MurI. This process may begin, for example, by visual inspection of the substrate binding site on the computer screen based on MurI coordinates. Selected fragments or chemical entities may then be positioned relative to the substrate binding site of MurI. Docking may be accomplished using software such as Quanta and Sybyl, followed by energy minimization and molecular dynamics with standard molecular mechanics forcefields, such as CHARMM and AMBER.

Specialized computer programs may also assist in the process of selecting fragments or chemical entities. These include:
- GRID [P.J. Goodford, "A Computational Procedure for Determining Energetically Favorable Binding Sites on Biologically Important Macromolecules', J. Med. Chem., 28:849-857 (1985)]. GRID is available from Oxford University, Oxford, UK.
- MCSS [A. Miranker and M. Karplus, "Functionality Maps of Binding Sites: A Multiple Copy Simultaneous Search Method", Proteins: Structure. Function and Genetics, 11:29-34 (1991)]. MCSS is available from Molecular Simulations, Burlington, Mass.
- AUTODOCK [D. S. Goodsell and A. J. Olsen, "Automated Docking of Substrates to Proteins by Simulated Annealing", Proteins: Structure. Function and Genetics, 8:195-202 (1990)]. AUTODOCK is available from Scripps Research Institute, La Jolla, Calif.
- DOCK [I. D. Kuntz et al, "A Geometric Approach to Macromolecule-Ligand Interactions", J. Mol. Biol., 161:269-288 (1982)]. DOCK is available from University of California, San Francisco, Calif.

Additional commercially available computer databases for small molecular compounds includes Cambridge Structural Database and Fine Chemical Database, for a review see Rusinko, A., (Chem, Des. Auto. News 8, 44-47 (1993)).

Once suitable chemical entities or fragments have been selected, they can be assembled into a single compound or inhibitor. Assembly may be proceeded by visual inspection of the relationship of the fragments to each other on the threedimensional image displayed on a computer screen in relation to the structure coordinates of MurI. This would be followed by manual model building using software such as Quanta or Sybyl.

Useful programs to aid one of skill in the art in connecting the individual chemical entities or fragments include:
- CAVEAT [P.A. Bartlett et al, "CAVEAT: A Program to Facilitate the Structure-Derived Design of Biologically Active Molecules", in Molecular Recognition in Chemical and Biological Problems", Special Pub., Royal Chem. Soc. 78, pp. 182-196 (1989)]. CAVEAT is available from the University of California, Berkeley, Calif.
- 3D Database systems such as MACCS-3D (MDL Information Systems, San Leandro, Calif) This area is reviewed in Y.C. Martin, "3D Database Searching in Drug Design", J. Med. Chem., 35:2145-2154 (1992).
- HOOK (available from Molecular Simulations, Burlington, Mass.).

Instead of proceeding to build a MurI inhibitor in a step-wise fashion one fragment or chemical entity at a time as described above, inhibitory or other type of binding compounds may be designed as a whole or "de novo" using either an empty active site or optionally including some portion(s) of a known inhibitor(s). These methods include:
- LUDI [H.-J. Bohm, "The Computer Program LUDI: A New Method for the De Novo Design of Enzyme Inhibitors", J. Comp. Aid. Molec. Design 6:61-78 (1992). LUDI is available from Biosym Technologies, San Diego, Calif.
- LEGEND [Y. Nishibata and A. Itai, Tetrahedron, 47:8985 (1991). LEGEND is available from Molecular Simulations, Burlington, Mass.
- LeapFrog (available from Tripos Associates, St. Louis, Mo.)

Other molecular modeling techniques may also be employed to screen for inhibitors of MurI. See, e.g., N.C. Cohen et al, "Molecular Modeling Software and Methods for Medicinal Chemistry", J. Med. Chem., 33:883-894 (1990). See also, M. A. Navia and M. A. Murcko, "'The Use of Structural Information in Drug Design", Current Opinions in Structural Biology, 2:202-210 (1992). For example, where the structures of test compounds are known, a model of the test compound may be superimposed over the model of the structure of the invention. Numerous methods and techniques are known in the art for performing this step. Any of these may be used. See, e.g., P.S. Farmer, Drug Design, Ariens, E. J., ed., Vol. 10, pp 119-143 (Academic Press, New York, 1980); U.S. Pat. No. 5, 331,573; U.S. Pat. No. 5,500,807; C. Verlinde, Structure, 2:577-587 (1994); and I. D. Kuntz, Science, 257:1078-1082 (1992). The model building techniques and computer evaluation systems described herein are not a limitation on the present invention.

A variety of conventional techniques may be used to carry out each of the above evaluations as well as the evaluations necessary in screening a candidate compound for ability to inhibit MurI. Generally, these techniques involve determining the location and binding proximity of a given moiety, the occupied space of a bound inhibitor, the amount of complementary contact surface between the inhibitor and protein, the deformation energy of binding of a given compound and some estimate of hydrogen bonding strength and/or electrostatic interaction energies. Examples of techniques useful in the above evaluations include: quantum mechanics, molecular mechanics, molecular dynamics, Monte Carlo sampling, systematic searches and distance geometry methods [G.R. Marshall, Ann. Rev. Pharmacal. Toxicol., 1987, 27: 193]. Specific computer software has been developed for use in carrying out these methods. Examples of programs designed for such uses include:
- Gaussian 92 [M.J. Frisch, Gaussian, Inc., Pittsburgh, PA. ©1993]
- AMBER [P.A. Kollman, University of California at San Francisco, ©1993]
- QUANTA/CHARMM [Molecular Simulations, Inc., San Diego, CA, ©1992]

Other hardware systems and software packages will be known and of evident applicability to those skilled in the art.

The concept of the pharmacophore has been well described in the literature [D. Mayer, C.B. Naylor, I. Motoc, and G.R. Marshall, J. Comp. Aided Molec. Design, 1987, 1: 3; A. Hopfinger and B.J. Burke, in Concepts and Applications of Molecular Similarity, 1990, M.A. Johnson and G.M. Maggiora, Ed., Wiley]. Different classes of MurI inhibitors of this invention may also use different scaffolds or core structures, but all of these cores will allow the necessary moieties to be placed in the active site such that the specific interactions necessary for binding result. These compounds are best defined in terms of their ability to match a pharmacophore, i.e., their structural identity relative to the shape and properties of the binding domain of the MurI.

Distances to or from any given group are calculated from the center of mass of that group. The term "center of mass" refers to a point in three-dimensional space which represents a weighted average position of the masses that make up an object. Distances between groups may readily be determined using any modeling software and other suitable chemical structure software. In addition, specialized, commercially-available pharmacophore modeling software enables one to determine pharmacophore models from a variety of structural information and data. The software may also be used to search a database of three-dimensional structures in order to identify compounds that meet specific pharmacophore requirements. Examples of this software include:
- DISCO [Martin, Y.C., Bures, M.G., Danaher, E.A., DeLazzer, J, Lico, A., Pavlik, P.A., J. Comput. Aided Mol. Design, 1993, 7:83] DISCO is available from Tripos Associates, St. Louis, MO.
- CHEM-X, which is developed and distributed by Chemical Design Ltd., Oxon, UK and Mahwah, NJ.
- APEX-3D, which is part of the Insight molecular modeling program, distributed by Molecular Simulations, Inc., San Diego, CA.
- CATALYST [Sprague, P.W., Perspectives in Drug Discovery and Design, 1995, 3:1; Müller, K., Ed., ESCOM, Leiden] CATALYST is distributed by Molecular Simulations, Inc., San Diego, CA.
- UNITY, which is available from Tripos Associates, St. Louis, MO.

A typical hydrogen bond acceptor (HBA) is an oxygen or nitrogen, especially an oxygen or nitrogen that is sp²-hybridized or an ether oxygen. A typical hydrogen bond donor (HBD) is an oxygen or nitrogen that bears a hydrogen. During binding, the pharmacophore features of the compounds will occupy certain regions or pockets of the binding site. In this region, the interaction of the compounds with the surrounding environment is primarily of a hydrophobic nature.

The pharmacophore features of the present compounds are not limited to distinct chemical moieties within the same compound. A chemical moiety may serve as parts of two pharmacophore features.

Thus, using these computer evaluation systems, a large number of compounds may be quickly and easily examined and expensive and lengthy biochemical testing avoided. Moreover, the need for actual synthesis of many compounds is effectively eliminated.

One design approach is to probe MurI of the invention with molecules composed of a variety of different chemical entities to determine optimal sites for interaction between candidate MurI binding agents and the enzyme. For example, high resolution X-ray diffraction data collected from crystals saturated with solvent allows the determination of where each type of molecule binds. Small molecules that bind tightly to those sites can then be designed and synthesized and tested for their MurI binding activity. (Bugg et al., Scientific American, December: 92-98 (1993); West et al., TIPS, 16: 67-74 (1995)).

This also enables the development of compounds that can isomerize to short-lived reaction intermediates in the chemical reaction of a substrate or other compound that binds MurI. Thus, it is possible to carry out time-dependent analysis of structural changes in MurI during its interaction with other molecules. The reaction intermediates of MurI can also be deduced from the reaction product in co-complex with MurI. Such information is useful to design improved analogues of known MurI inhibitors or to design novel classes of binding agents based on the reaction intermediates of the MurI enzyme and MurI binding agent co-complex.

Another approach, is to screen computationally small molecule databases for chemical entities or compounds that can bind in whole, or in part, to a binding site or an accessory site of Murl. In this screening, the quality of fit of such entities or compounds to the binding site may be judged either by shape complementarity [R.L. DesJarlais et al., J. Med. Chem. 31; 722-729 (1988)] or by estimated interaction energy [E. C. Meng et al., J. Comp. Chem., 13;505-524 (1992)].

Thus, the MurI structure provided herein permits the screening of known molecules and/or the designing of new molecules which bind to the MurI structure, particularly at the active site, via the use of computerized evaluation systems. In one example, the sequence of MurI, and the MurI structure (e.g., atomic coordinates of MurI and/or the atomic coordinate of the binding site cavity; bond angles, dihedral angles, distances between atoms in the binding site region, etc. as provided by Figure 4 may be input. Thus, a machine readable medium may be encoded with data representing the coordinates of Figure 7 in this process. The computer then generates structural details of the site into which a test compound should fit, thereby enabling the determination of the complementary structural details of the test compound/ inhibitor.

In one embodiment, the inhibitor belongs to the class of compounds known as pyrimidinediones found to inhibit *H. pylori* MurI. In specific embodiments, the pyrimidinedione is compound A, compound B, compound C, compound D, compound E, compound F, compound G, compound H, compound I, compound J, compound K, compound L, compound M, compound N, compound O, compound P, compound Q, compound R, compound S, compound T, compound U, compound V, compound W, compound X, compound Y, compound Z, compound AA, compound AB, compound AC, compound AD, compound AE, compound AF, compound AG, compound AH, compound AI, compound AJ, or compound AK. The crystalline complexes are characterized by the space groups and cell dimensions depicted in Table 5.

### IX. Computer-Assisted Methods of Designing and Making MurI Inhibitors

In one embodiment, we provide computer-assisted design of binding agents of MurI, including molecules that fit into or bind to the binding site of MurI, or a portion of MurI which acts as a binding site or accessory site. A binding agent of the present invention can be an antibacterial binding agent, such as a pyrimidinedione, that partially or totally inhibits the enzymatic activity of MurI.

One embodiment is a disk on which is stored the structural coordinates of MurI, wherein the structural coordinates may be used in a wide variety of computer-assisted methods, such as those described herein.

The crystal structure of MurI, and the binding site thereof described herein are useful for the design of agents, particularly selective inhibitory agents, which inhibit MurI, and, thus, could act as antibacterial agents. In a related embodiment, we provide a method for structure-based drug design for an agent that inhibits MurI activity.

More particularly, the design of compounds that inhibit MurI according to this invention generally involves consideration of two factors. First, the compound must be capable of physically and structurally associating with MurI via covalent and/or non-covalent interactions. Non-covalent molecular interactions important in the association of MurI with its substrate, or inhibitor, include hydrogen bonding, van der Waals and hydrophobic interactions.

Second, the compound must be able to assume a conformation that allows it to associate with Murl. Although certain portions of the compound will not directly participate in this association with MurI, those portions may still influence the overall conformation of the molecule. This, in turn, may have a significant impact on potency. Such conformational requirements include the overall three-dimensional structure and orientation of the chemical entity or compound in relation to all or a portion of a binding site, e.g., a substrate binding site, an activator binding site, an intradomain interface, an intermolecular dimer interface, or accessory site thereof, of MurI, or the spacing between functional groups of a compound comprising several chemical entities that directly interact with MurI.

The potential inhibitory effect of a chemical compound on MurI may be estimated prior to its synthesis and testing by the use of computer modeling techniques. If the theoretical structure of the given compound suggests insufficient interaction and association between it and MurI, synthesis and testing of the compound is obviated. However, if computer modeling indicates a strong interaction, the molecule may then be synthesized and tested for its ability to bind to MurI in a suitable assay. In this manner, synthesis of inactive compounds may be avoided.

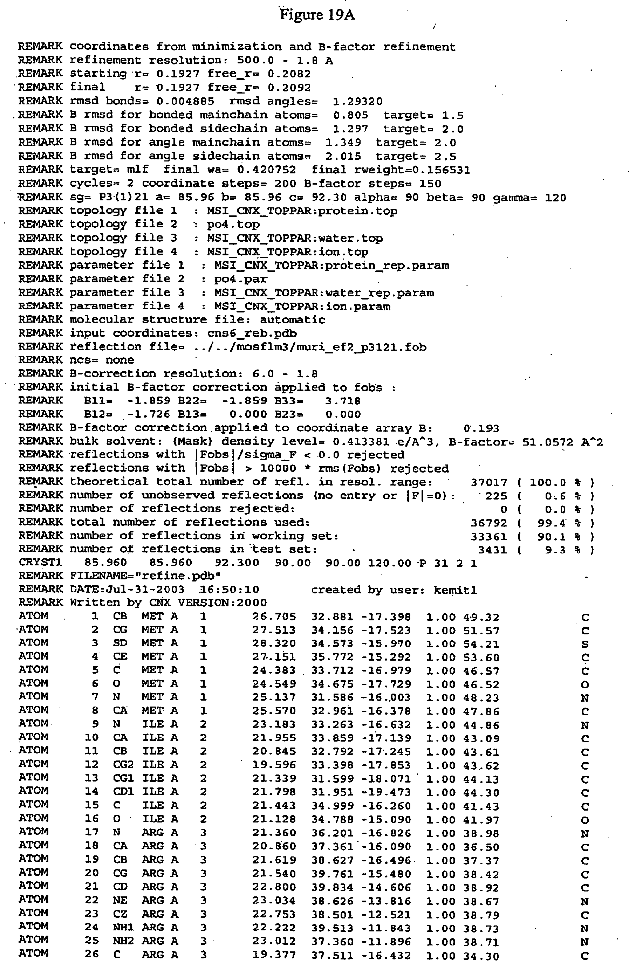

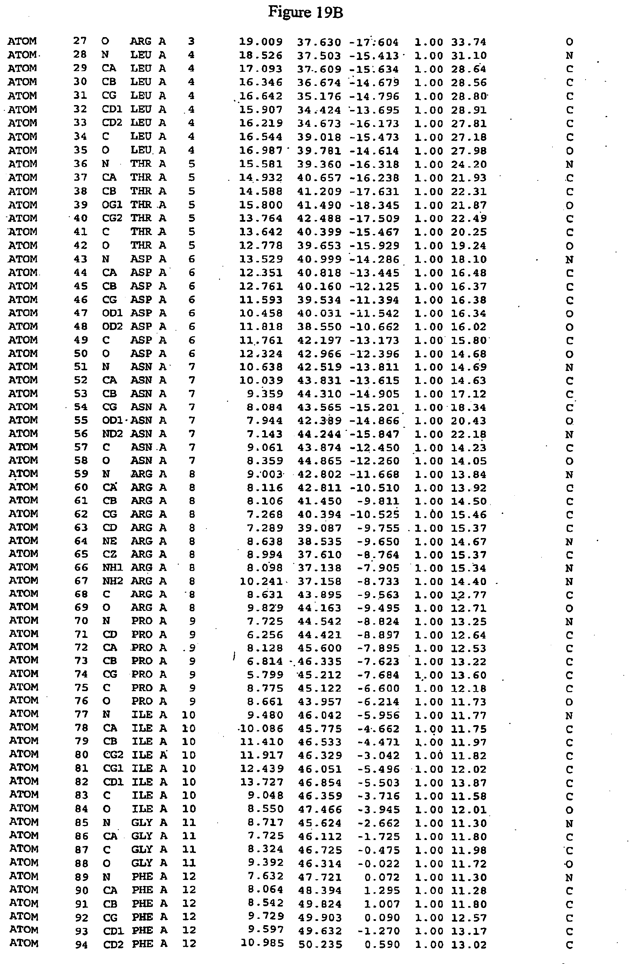

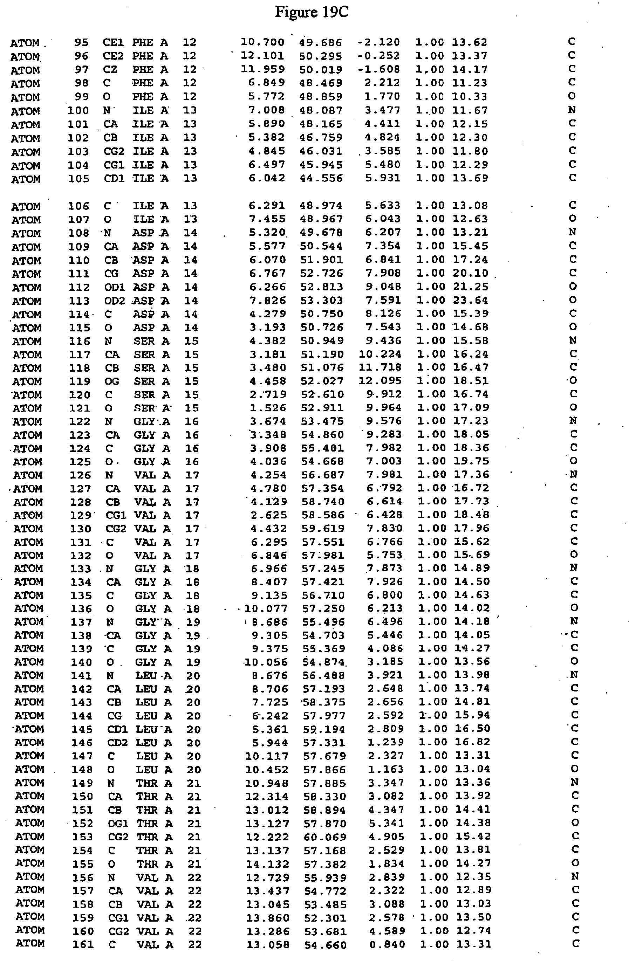

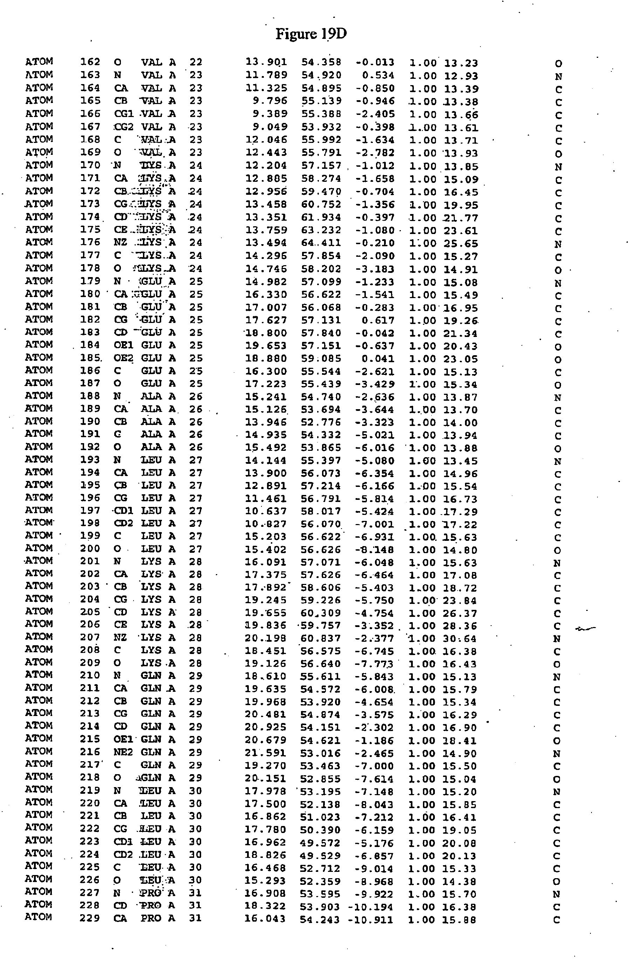

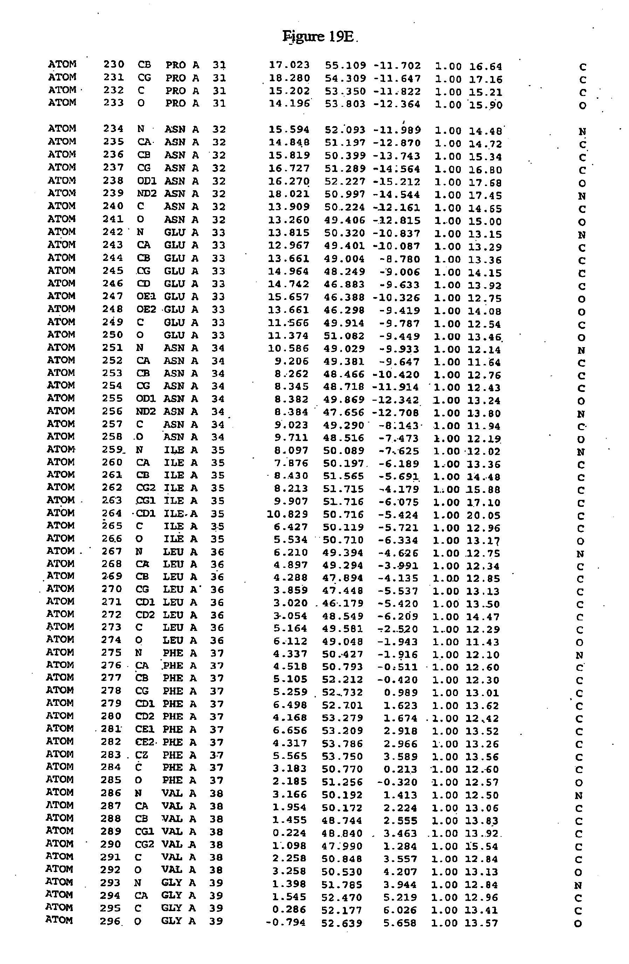

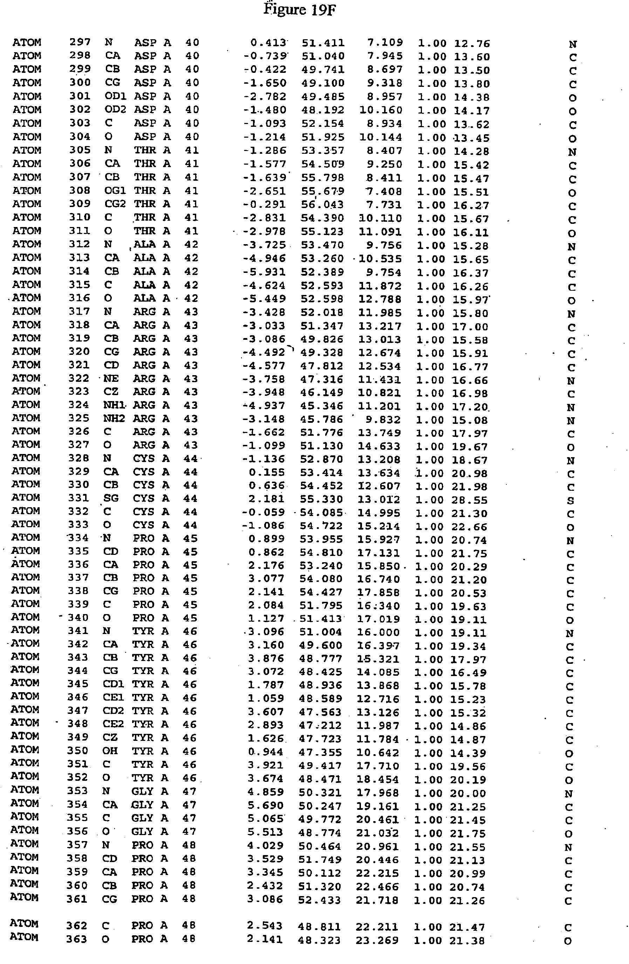

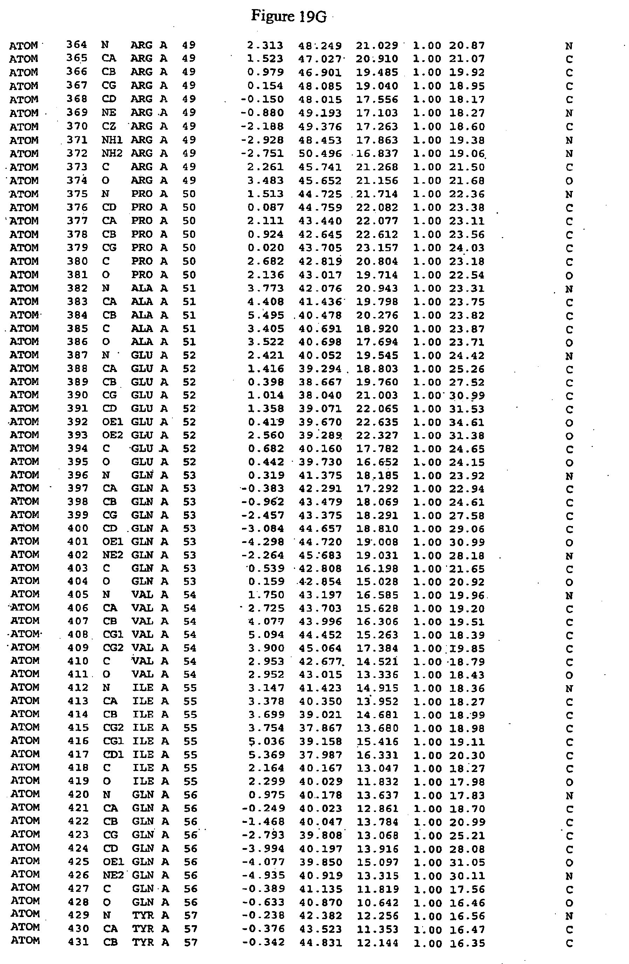

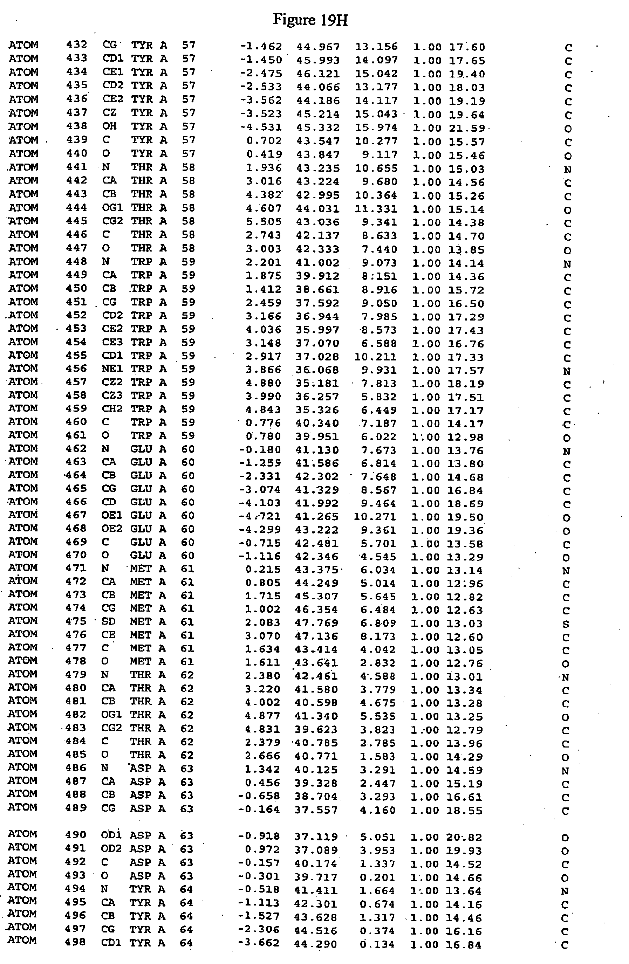

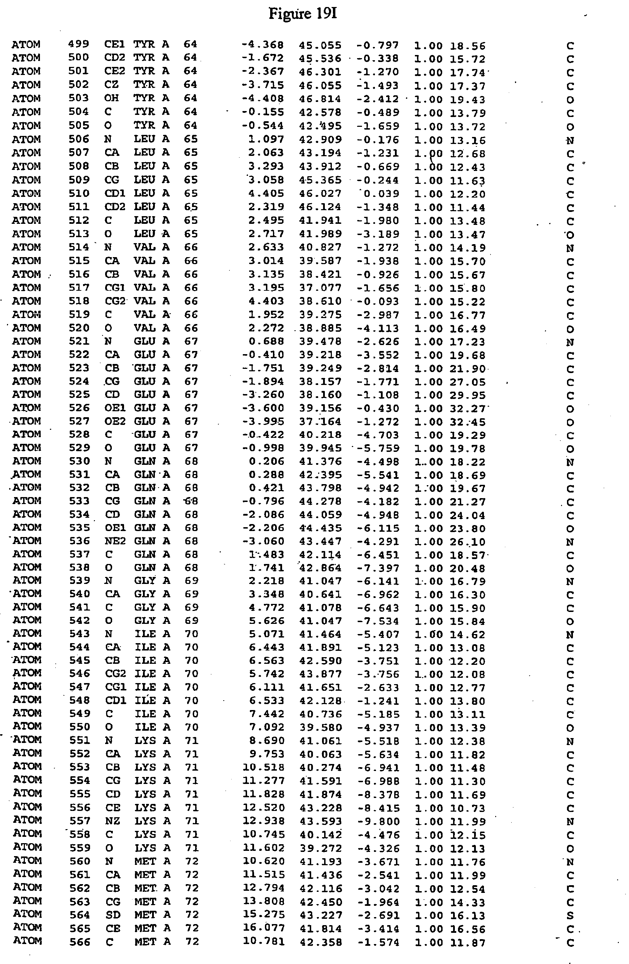

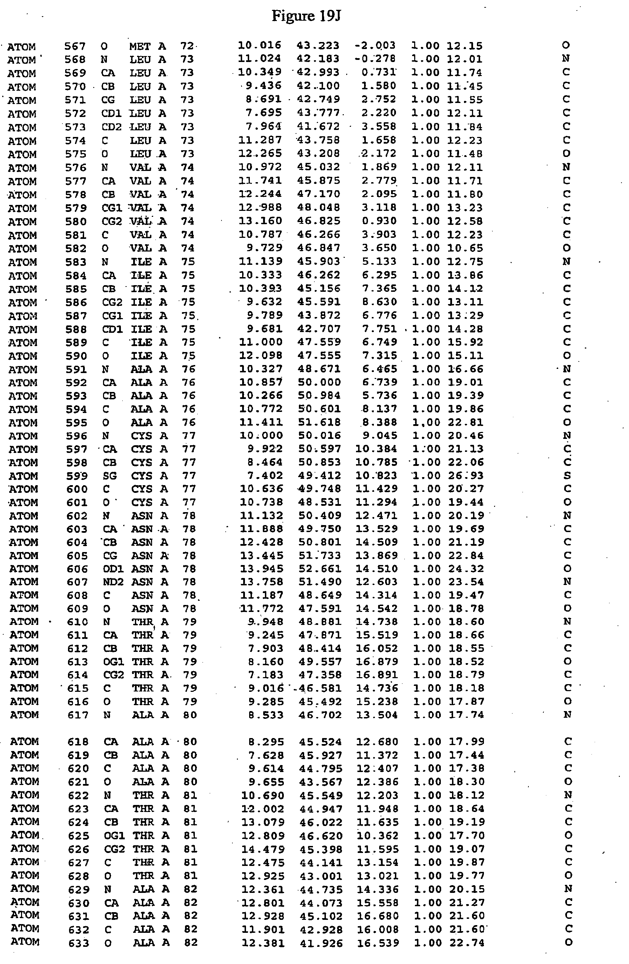

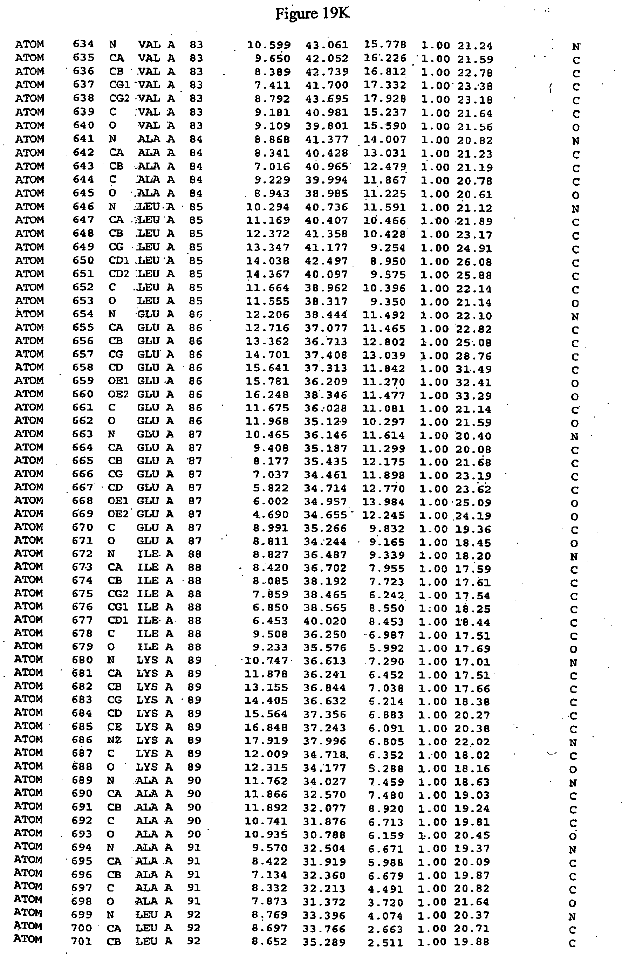

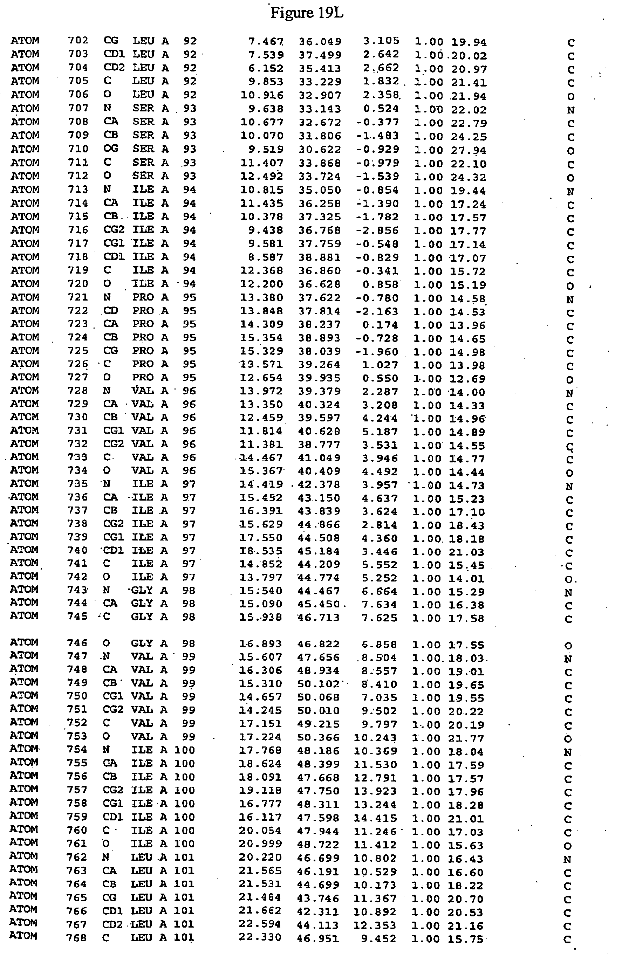

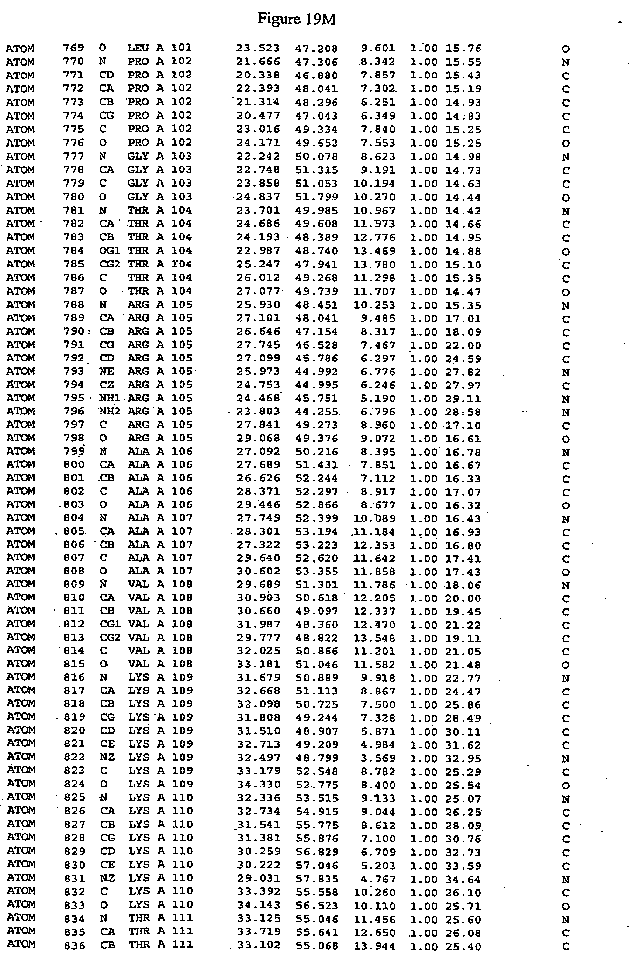

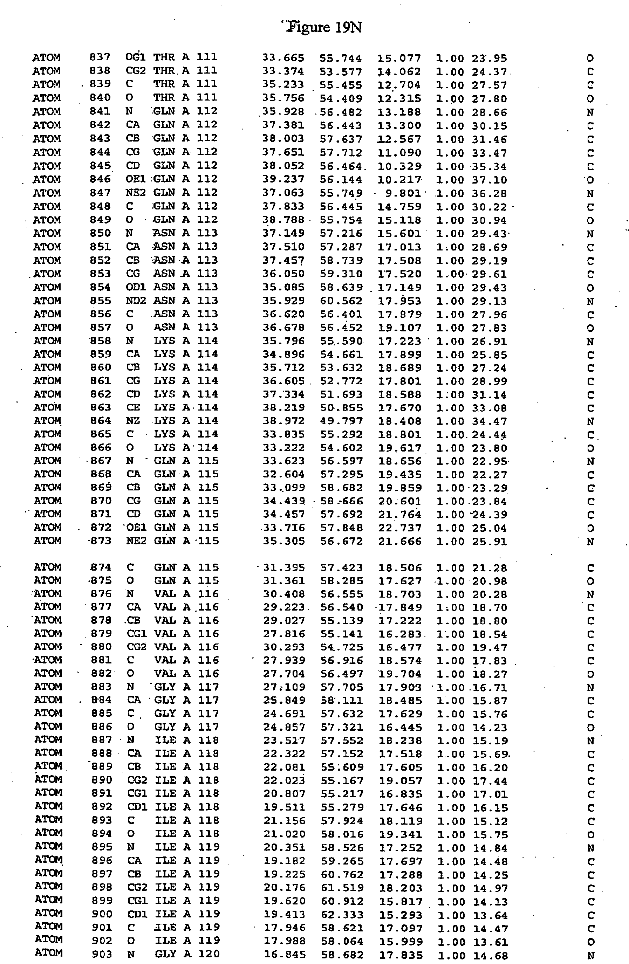

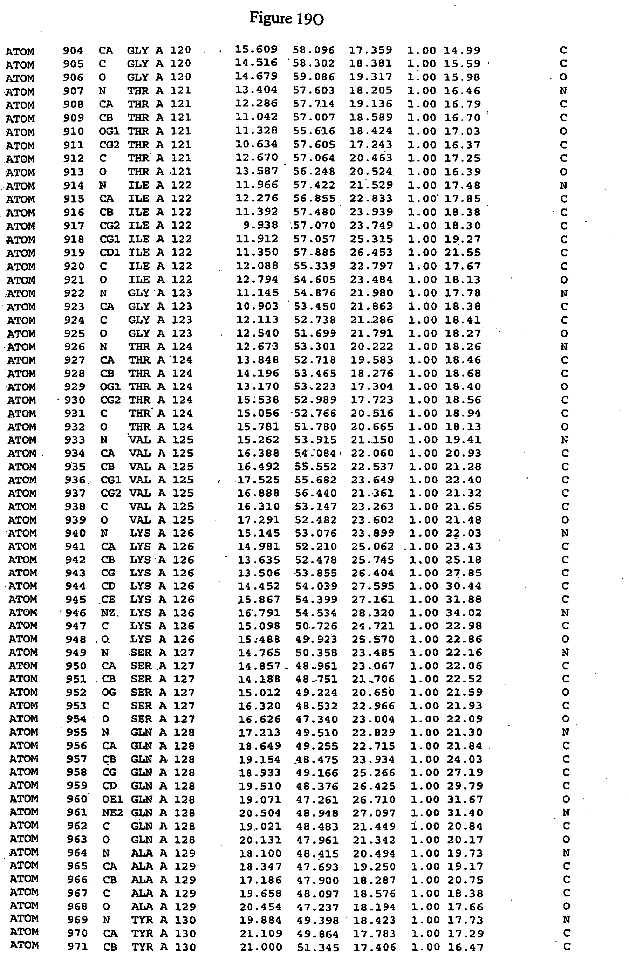

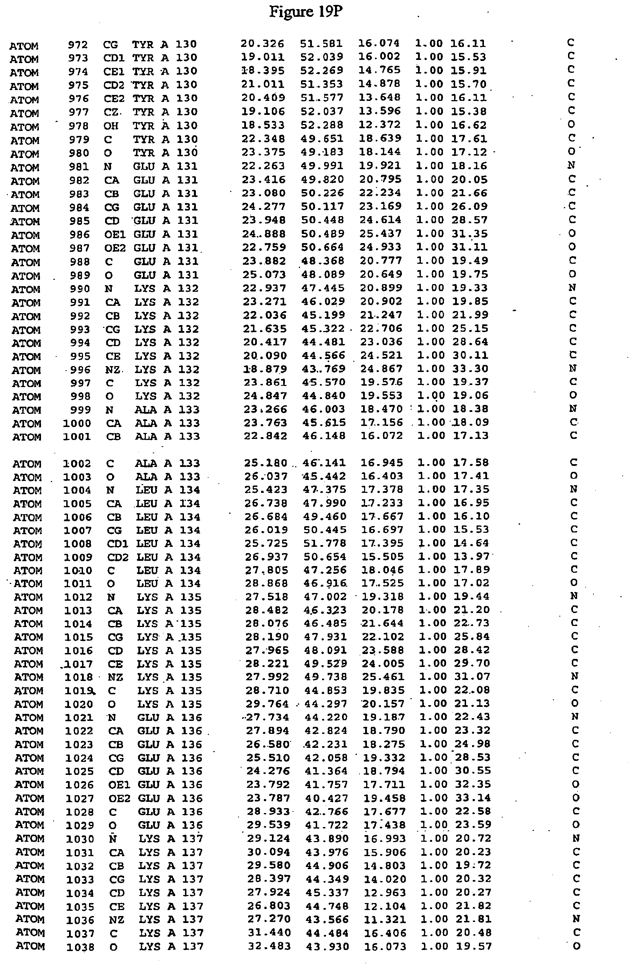

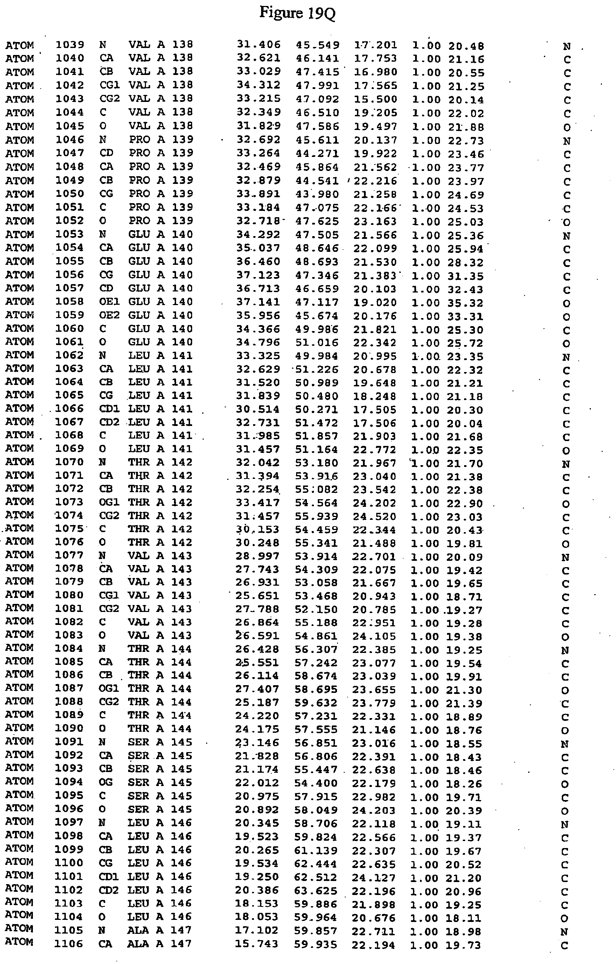

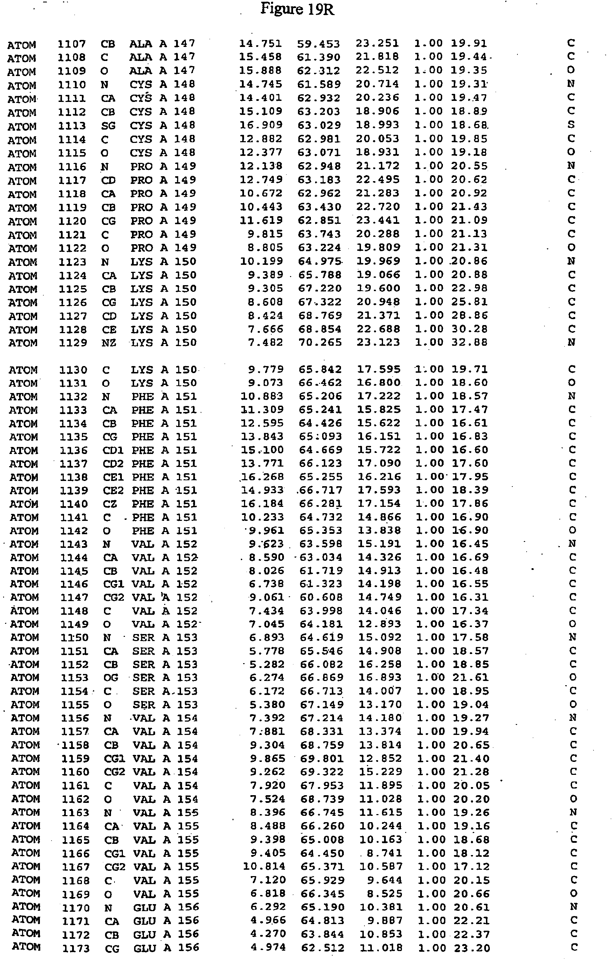

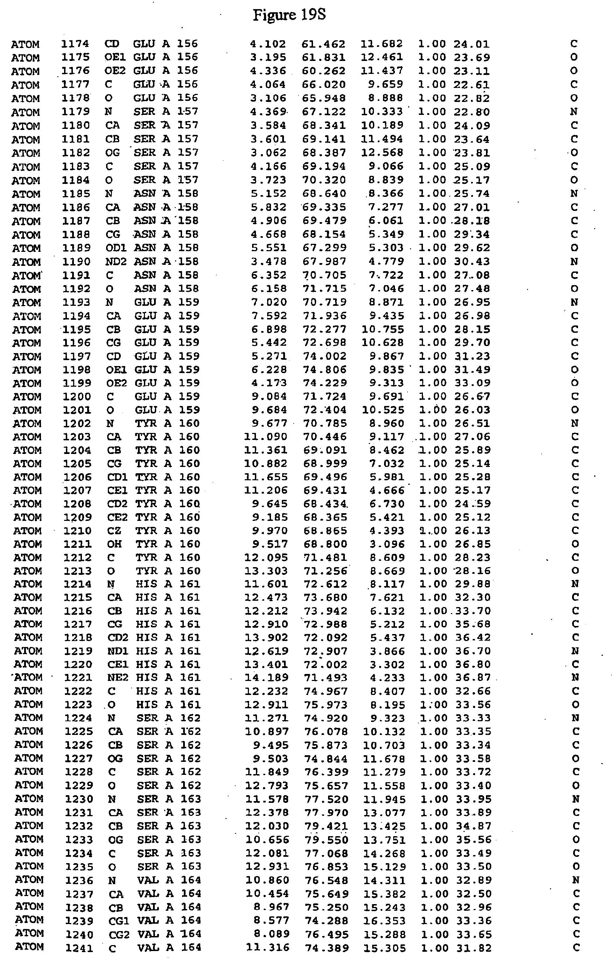

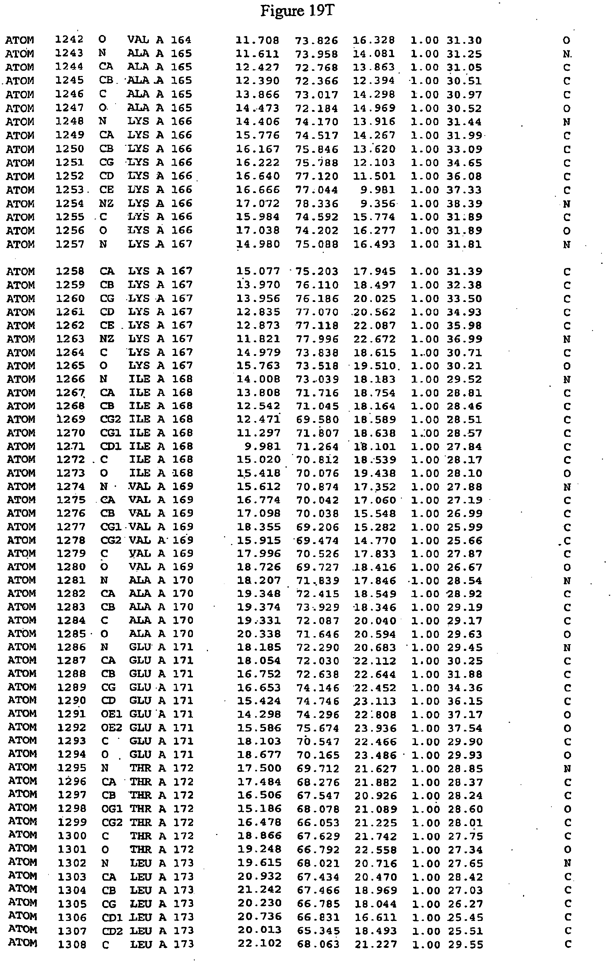

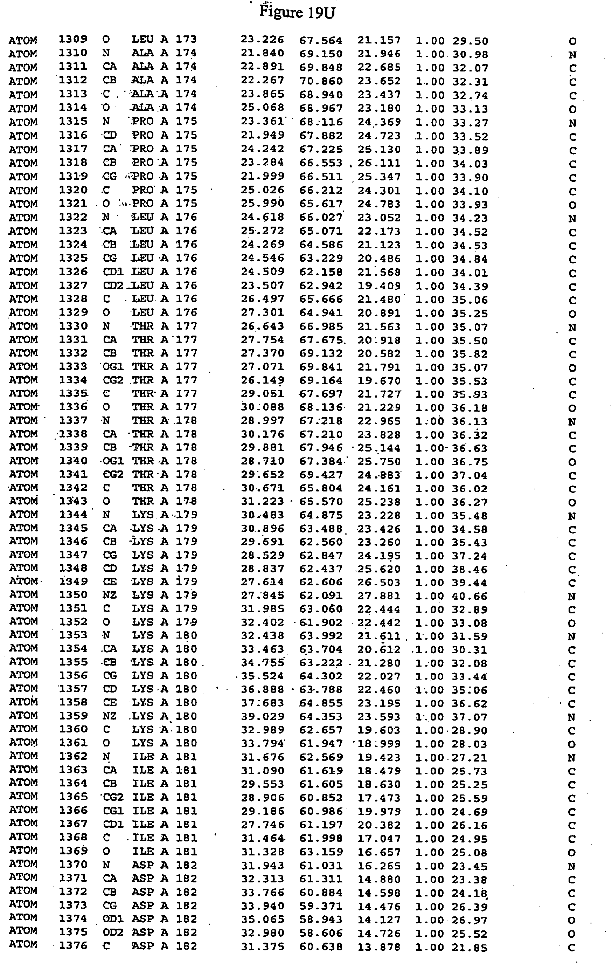

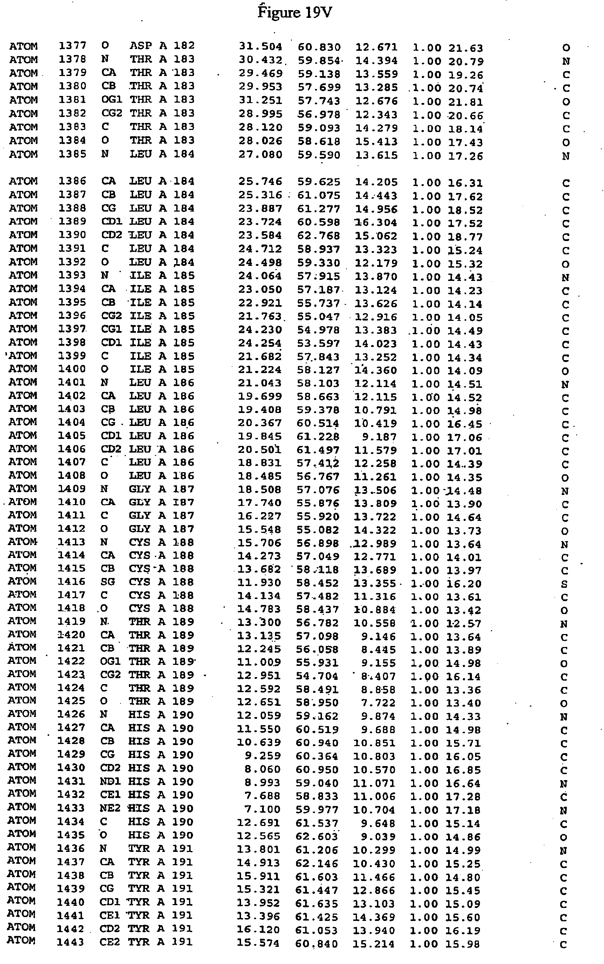

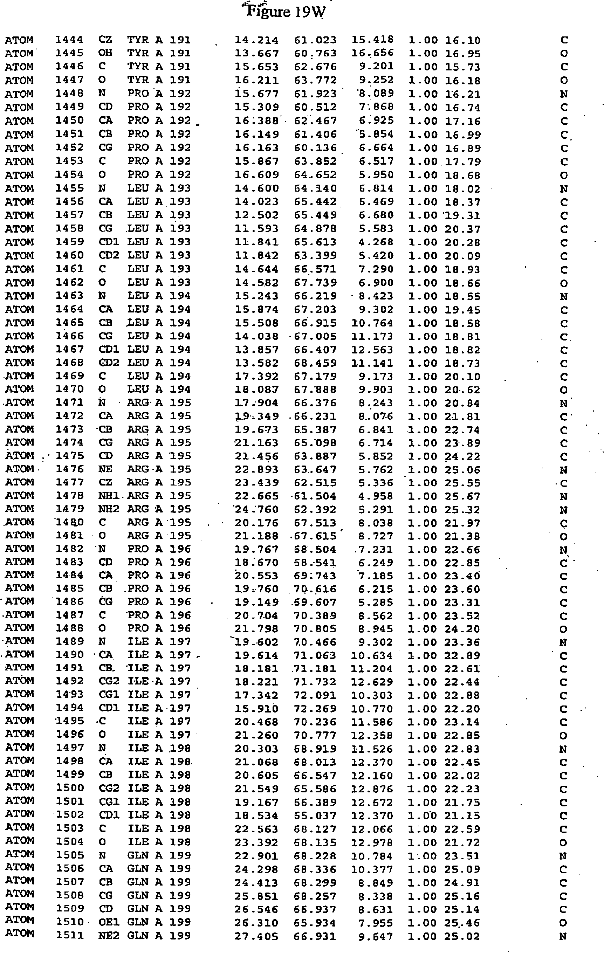

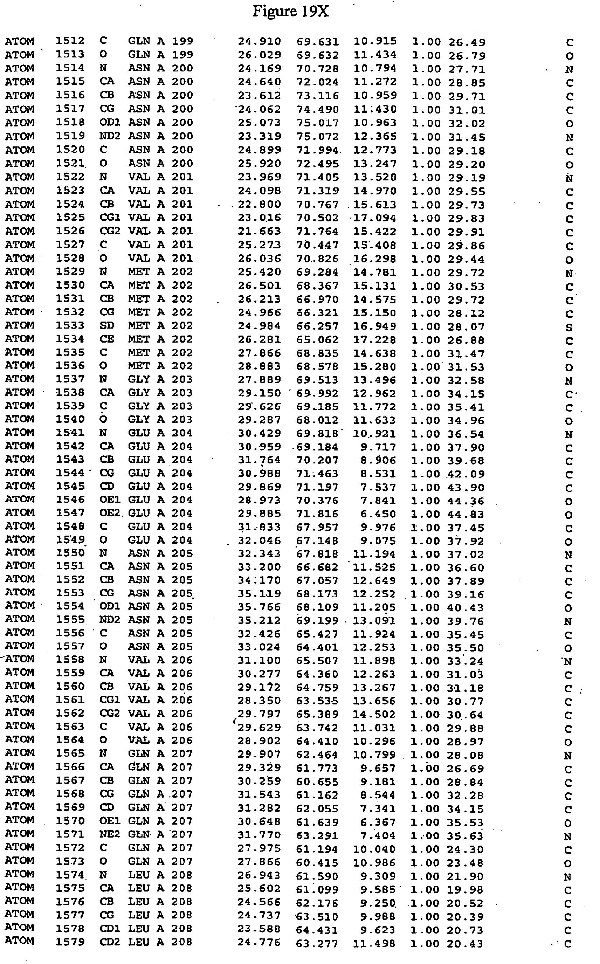

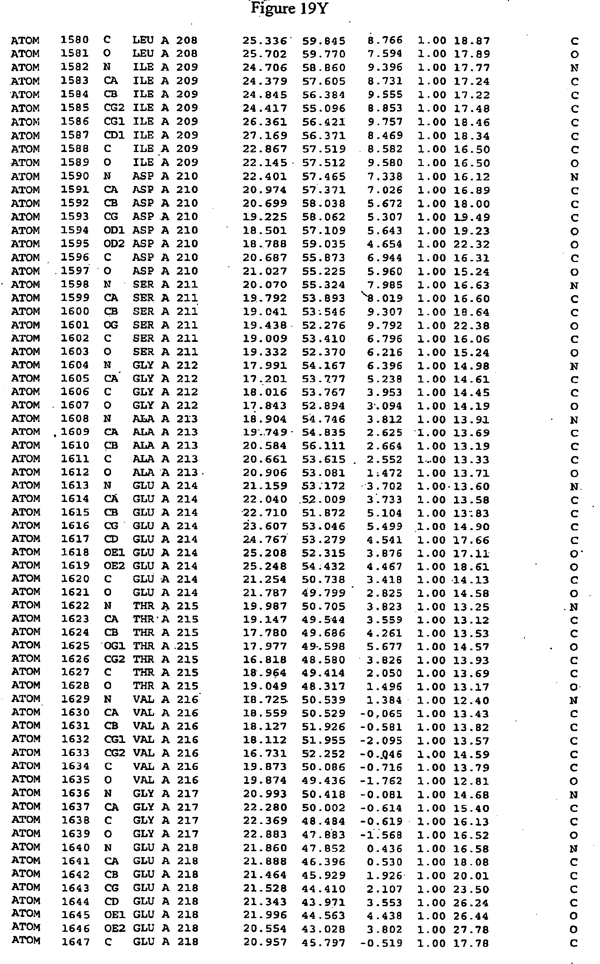

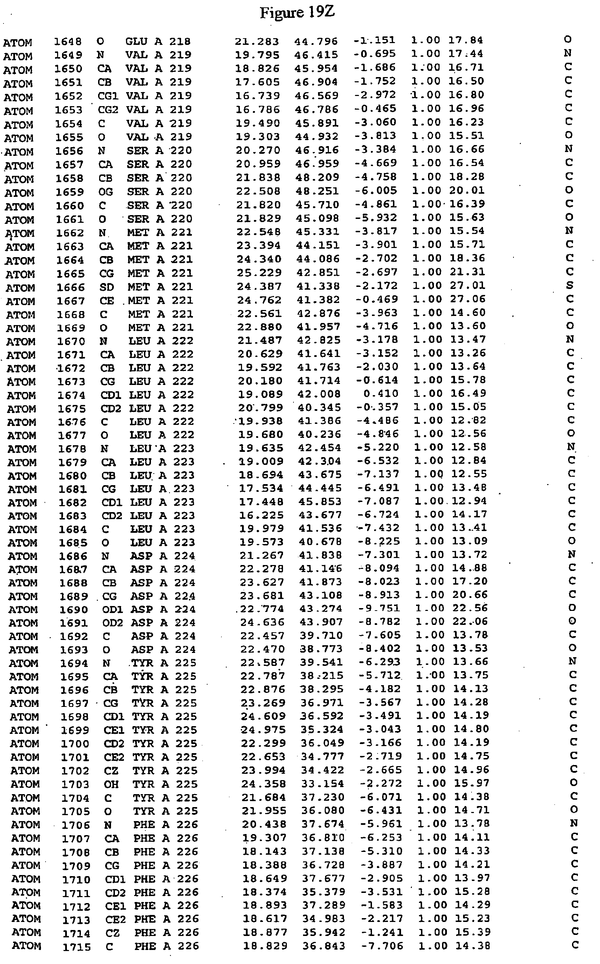

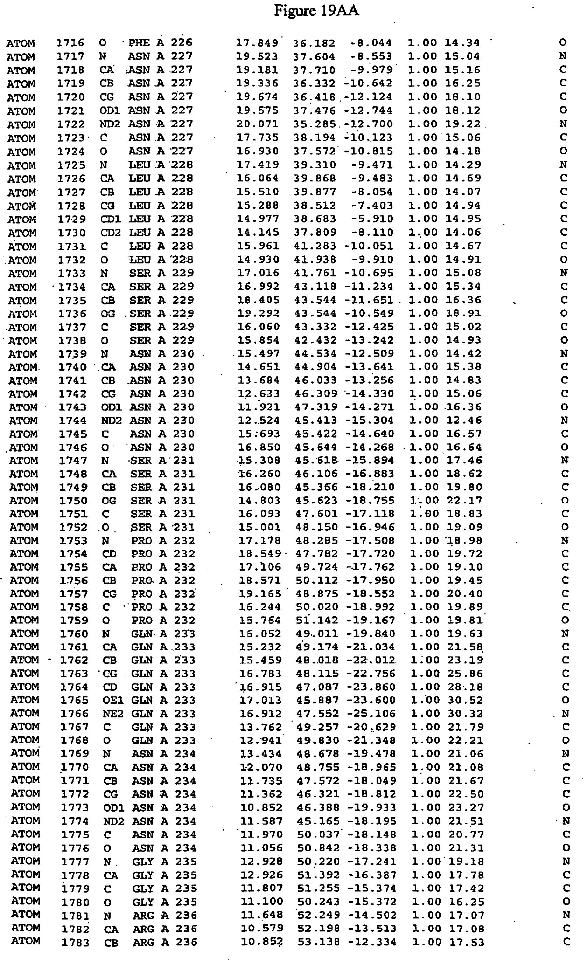

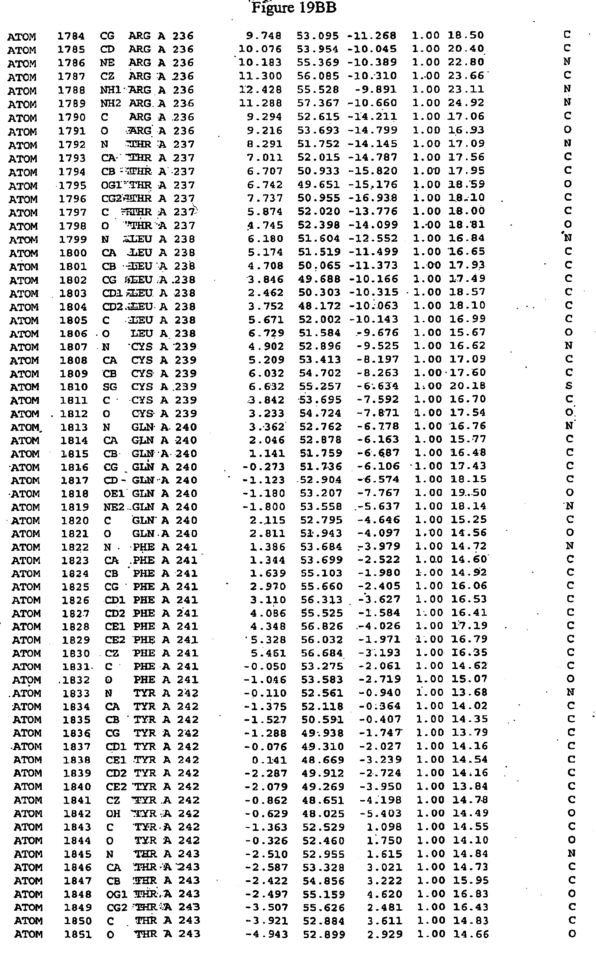

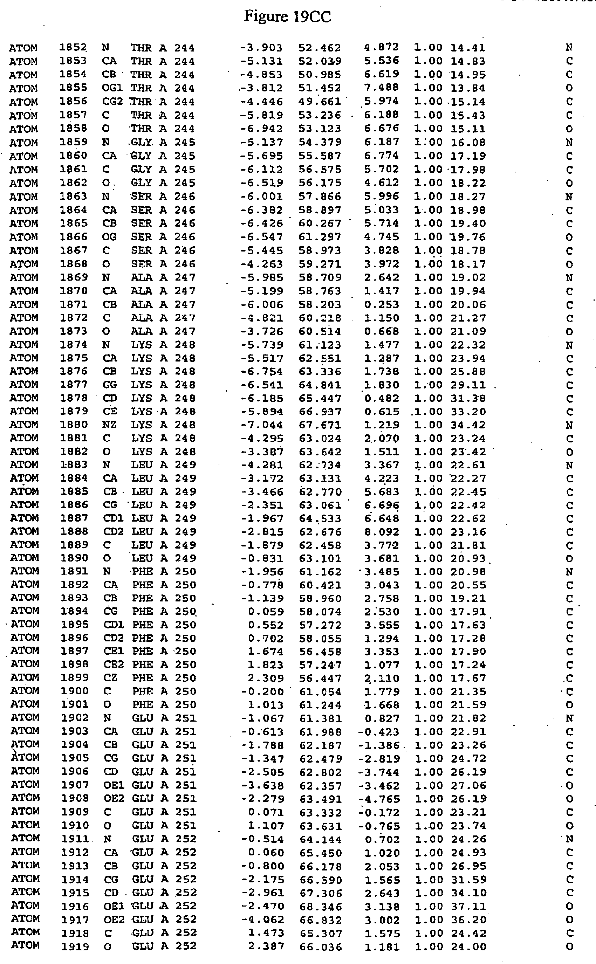

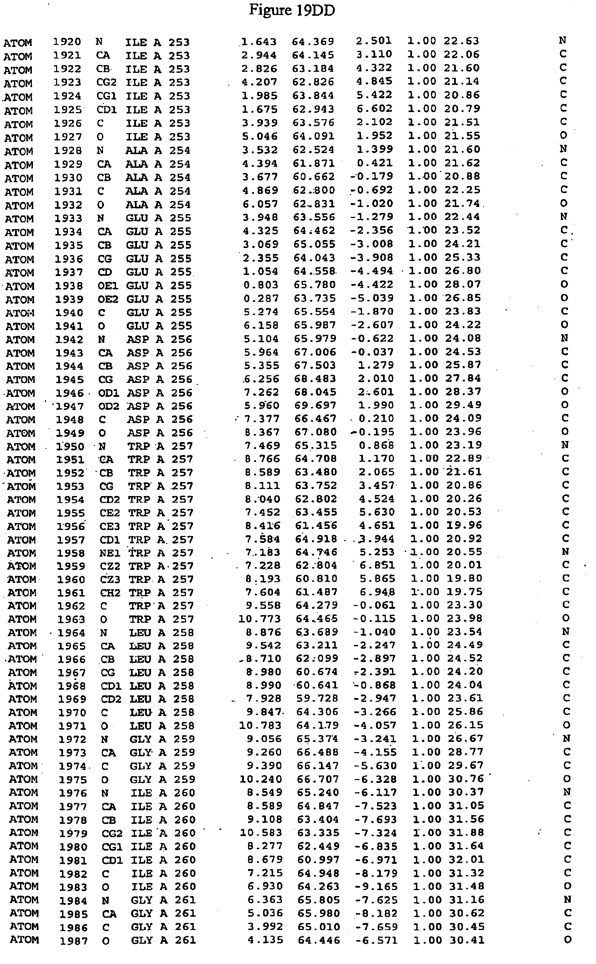

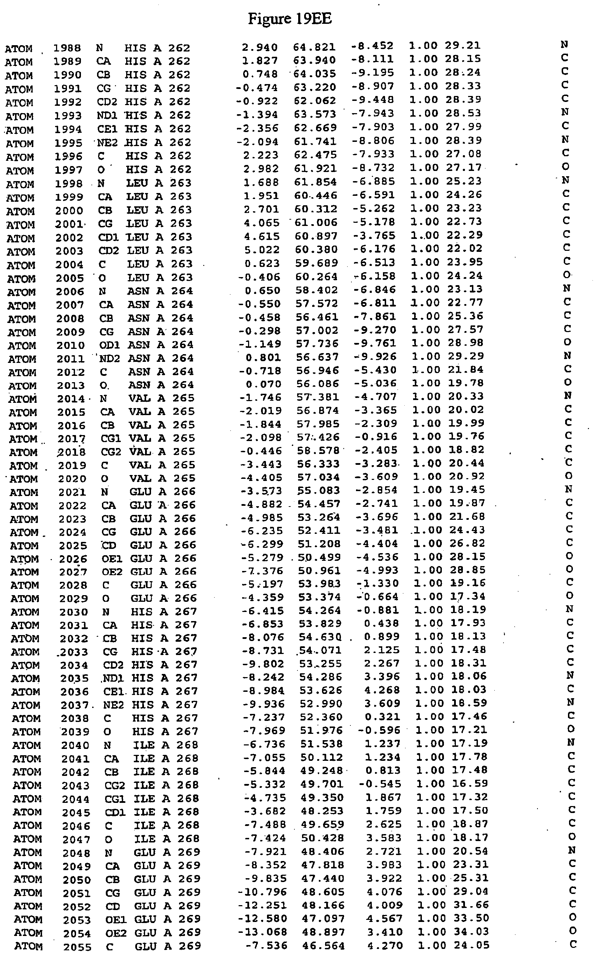

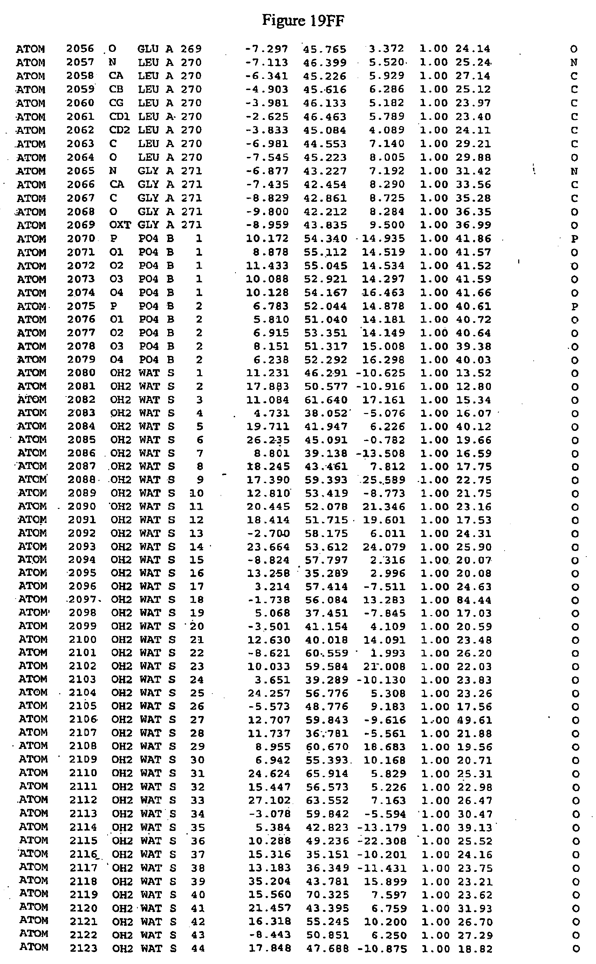

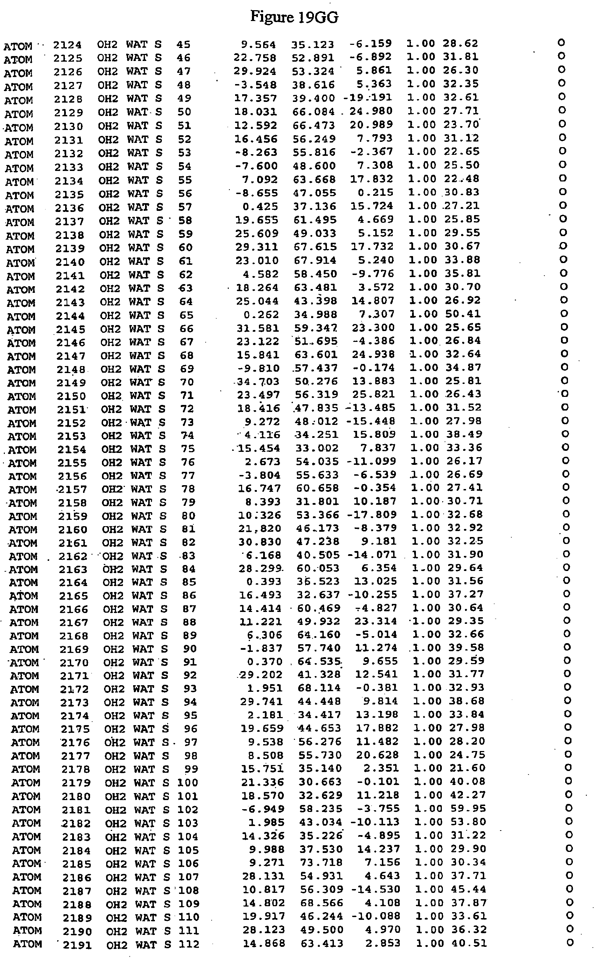

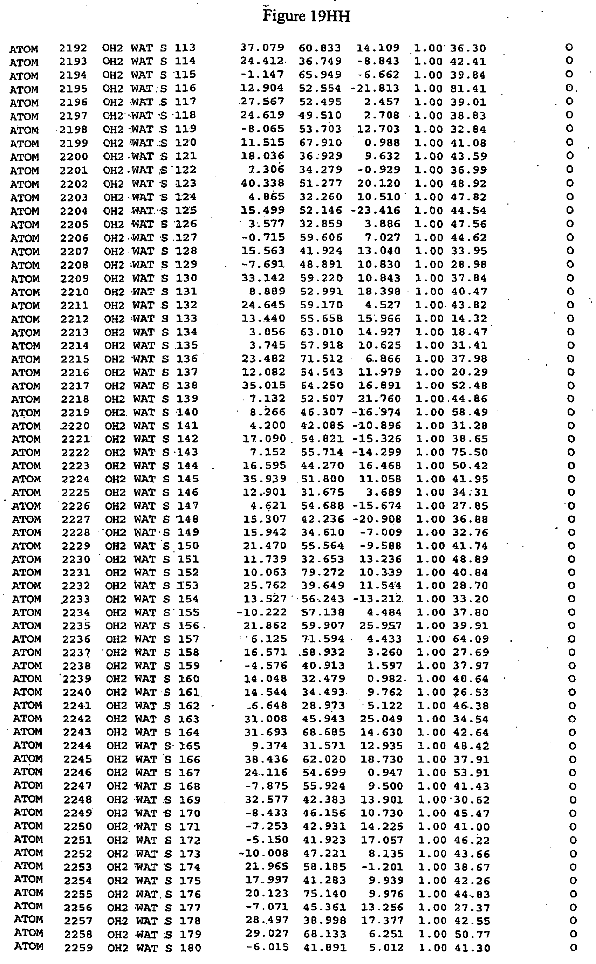

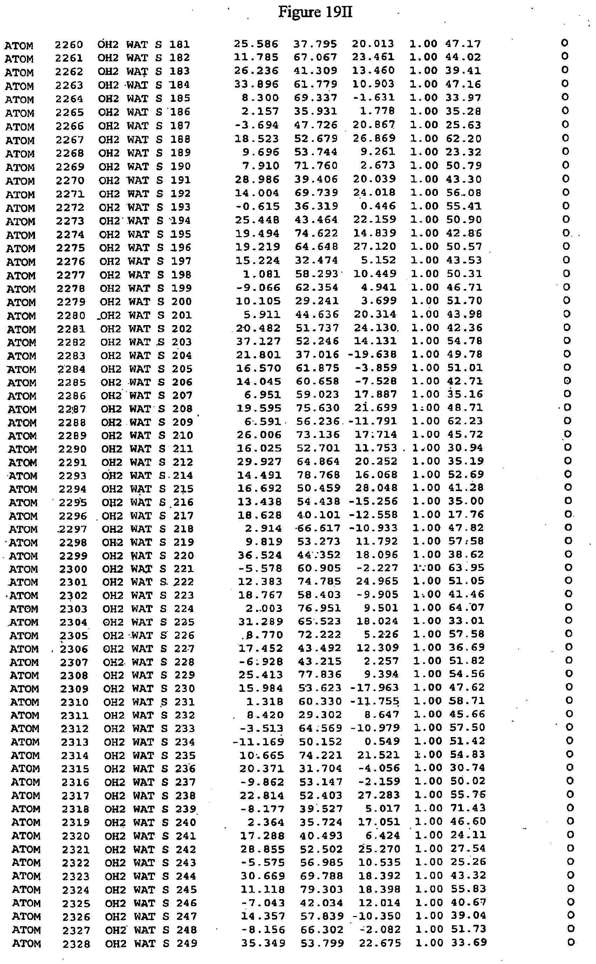

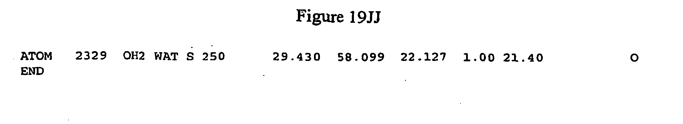

In another embodiment, the invention is a computer-assisted method for designing a candidate modifier, particularly a candidate inhibitor, of MurI. In the method, a computer modeling application is supplied with a set of relative structural coordinates of MurI or a binding domain thereof, and a set of structural coordinates of a candidate inhibitor of MurI. The set of relative structural coordinates is selected from sets of relative structural coordinates of a MurI as depicted in Figures 4-19, and with a set of structural coordinates of a candidate inhibitor of MurI. The potential interference of the candidate inhibitor with the activity of MurI is assessed and the candidate inhibitor is structurally modified as needed to produce a set of structural coordinates for a modified candidate inhibitor. The modified candidate inhibitor is further assessed, using computer-assisted techniques and, optionally, *in vitro* and/or *in vivo* testing and modified further, if needed, to produce a modified candidate inhibitor with enhanced properties (e.g., greater inhibitory activity than the starting candidate inhibitor).

We also enable the development of compounds that can isomerize to short-lived reaction intermediates in the chemical reaction of a substrate or other compound that binds to or with MurI. This makes it possible to carry out time-dependent analysis of structural changes in MurI during its interaction with other molecules. The reaction intermediates of MurI can also be deduced from the reaction product in complex with MurI. Such information is useful to design improved analogues of known racemase inhibitors, or to design novel classes of inhibitors based on the reaction intermediates of the MurI enzyme and MurUinhibitor co-complex. This provides a novel route for designing MurI inhibitors with both high specificity and stability.

An inhibitory or other binding compound of MurI may be computationally evaluated and designed by means of a series of steps in which chemical entities or fragments are screened and selected for their ability to associate with the individual binding domains (pockets) or other areas of MurI.

In a related embodiment, we encompass a method for structure-based drug design for an agent that inhibits MurI activity, comprising generating a compound structure using a crystalline form of MurI which can be used for X-ray or nuclear magnetic resonance (NMR) studies. In one embodiment, the coordinates of Figure 4 are used. Alternatively, coordinates having a root mean square deviation from the coordinates of Figure 4 with respect to conserved backbone atoms of the listed amino acid sequence of not more than 1.0 Å, a root mean square deviation of not more than 1.5 Å, a root mean square deviation of not more than 2.0 Å, or a root mean square deviation of not more than 2.5 Å. In a further embodiment, the method additionally comprises generating a conserved surface of the crystalline form of MurI on a computer screen, generating the spatial structure of test compounds on a computer screen, and determining if the compounds having a spatial structure fit the conserved surface. In the example, the conserved surface of the crystalline form of MurI has a binding site with a crystal structure which is created by atoms from the following amino acid residues: Asp7, Ser8, Gly9, Val10, Gly11, Gly12, Va137, Pro38, Tyr39, Gly40, Thr41, Ala69, Cys70, Asn71, Thr72, Ala73, Gly115, Thr116, Lys117, Ala118, Thr119, Va1146, Ile149, Glu150, Gly180, Cys181, Thr182, and His183of SEQ ID NO: 2.

In another embodiment, we provide a computer-assisted method of designing a candidate modifier, particularly a candidate inhibitor, of MurI. The method comprises supplying a computer modeling application with a set of relative structural coordinates of MurI, or a binding site thereof and a set of structural coordinates of a candidate inhibitor of MurI; computationally building an agent to be assessed for its ability to interfere with the MurI or a binding site thereof, wherein the resulting agent is represented by a set of structural coordinates; and determining whether the agent is expected to bind, or interfere with, the MurI, or the binding site, wherein if the agent is expected to bind, or interfere with, the MurI or the binding site, a candidate inhibitor has been designed.

In certain embodiments we provide a method for generating 3-D atomic coordinates of a protein homologue or a variant of MurI using the X-ray coordinates of MurI described in any one of Figures 4-19, comprising,
a. identifying one or more polypeptide sequences homologous to MurI;
b. aligning the sequences with the sequence of MurI which comprises a polypeptide with the amino acid sequence of any one of SEQ ID NOS: 2-34, 40, 44, 46, 48, 50, 52, 54, 56, 58, 60, 62, 64, 66, and 74;
c. identifying structurally conserved and structurally variable regions between the homologous sequence(s) and MurI;
d. generating 3-D coordinates for structurally conserved residues of the homologous sequence(s) from those of MurI using coordinates of MurI, such as those listed in any one of Figures 4-19;
e. generating conformations for helices, strands, loops, and/or turns in the structurally variable regions of the homologous sequence(s);
f. building side-chain conformations for the homologous sequence(s); and
g. combining the 3-D coordinates of the conserved residues, loops and side-chain conformations to generate full or partial 3-D coordinates for the homologous sequences. In certain embodiments the method further comprises refining and evaluating the full or partial 3-D coordinates for percent homology with MurI.

One embodiment relates to any computer-assisted method using known binding agents of MurI, such as L-glutamate, D-glutamate, L-serine-O-sulfate, and D-serine-O-sulfate, tartrate, citrate, phosphate, sulfate, D-aspartate, L-aspartate, aziridino-glutamate, N-hydroxyglutamate, 3-chloroglutamate, a pyrimidinedione, or UDP-MurNAc-Ala, to determine the fit of a known agent for comparison to a candidate inhibitor.

One embodiment is a disk on which is stored the structural coordinates of any one of Figures 4-19, wherein the structural coordinates may be used in a variety of computer-assisted methods, such as those described herein.

In a specific embodiment, the computer-assisted method of designing an agent that binds MurI comprises the steps of (1) supplying to a computer modeling application a set of relative structural coordinates of a crystal of MurI; (2) computationally building an agent represented by a set of structural coordinates; and (3) determining whether the agent is expected to bind, or interfere with MurI, wherein if the agent is expected to bind MurI, an agent that binds MurI has been designed. In a further embodiment of the present invention, the binding agent has been designed such that it directly binds to the amino acid residues forming a binding site of MurI or a surrounding area such that the enzyme is conformationally hindered and substrate cannot bind to the substrate binding site. The MurI can be from a Gram negative bacterium, a Gram positive bacterium, or an atypical bacterium.

In a related embodiment, we encompass a method for structure-based drug design of an agent that binds to MurI, comprising generating a compound structure using a crystalline form of MurI wherein the crystalline form of the MurI is capable of being used for X-ray studies. In one embodiment, the coordinates of Figure 4 are used. Alternatively, coordinates having a root mean square deviation from the coordinates of Figure 4 with respect to conserved backbone atoms of the listed amino acid sequence of not more than 1.0 Å, a root mean square deviation of not more than 1.5 Å, a root mean square deviation of not more than 2.0 Å, or a root mean square deviation of not more than 2.5 Å. In a further embodiment, the method additionally comprises generating a conserved surface of the crystalline form of MurI on, a computer screen, generating the spatial structure of test compounds on a computer screen, and determining if the compounds having a spatial structure fit the conserved surface. In a further embodiment, the method additionally comprises generating a conserved surface of the crystalline form of MurI on a computer screen, generating the spatial structure of test compounds on a computer screen, and determining if the compounds having a spatial structure fit the conserved surface.

In another aspect, the MurI structure permits the design and identification of synthetic compounds and/or other molecules which have a shape complimentary to the conformation of a MurI binding site described herein. Using known computer systems, the coordinates of the MurI structure of the invention may be provided in machine readable form, the test compounds designed and/or screened and their conformations superimposed on the structure of the MurI of the invention. Subsequently, suitable candidates identified as above may be screened for the MurI binding, bioactivity, and stability.

In another embodiment, we provide a method of making a candidate modifier of MurI by chemical, enzymatic or other synthetic method. Candidate modifiers identified or designed as described herein can be made using techniques known to those of skill in the art.

In another aspect, the MurI structure permits the design and identification of synthetic compounds and/or other molecules which have a shape complimentary to the conformation of the MurI binding site of the invention. Using known computer systems, the coordinates of the MurI structure of the invention may be provided in machine readable form, the test compounds designed and/or screened and their conformations superimposed on the structure of the MurI. Subsequently, suitable candidates identified as above may be screened for the desired MurI inhibitory bioactivity, and stability.

Once identified and screened for biological activity, these inhibitors may be used therapeutically or prophylactically to block MurI activity and to treat bacterial infections in a mammalian subject.

Once identified by the modeling techniques, the inhibitor may be tested for bioactivity using standard techniques. For example, the MurI structure may be used in binding assays using conventional formats to screen inhibitors. Suitable assays for use include, but are not limited to, the enzyme-linked immunosorbant assay (ELISA) or a fluorescence quench assay. Other assay formats may be used; these assay formats are not a limitation on the present invention.

### X. MAPS/Molecular Replacement/Dyndom

MurI may crystallize in more than one form. Therefore, the structural coordinates of MurI as described herein are particularly useful to solve the structure of additional crystal forms of MurI, or binding domains of additional crystal forms of MurI. Portions of MurI function as the active site (substrate binding site). They may also be used to solve the structure of MurI mutants, MurI complexes, or of the crystalline form of other proteins with significant amino acid sequence homology or structural homology to a functional domain of MurI. In one embodiment, significant amino acid sequence homology comprises at least 70%, 75%, 80%, 85%, 90%, 95%, 98%, or 99% homology to any functional domain of MurI.

One method that may be employed for this purpose is molecular replacement. In this method, the unknown crystal structure, whether it is another crystal form of MurI, a MurI, or a MurI co-complex, or the crystal of some other protein with significant amino acid sequence homology to any functional domain of MurI, may be determined using the MurI structure coordinates of this invention. This method will provide an accurate structural form for the unknown crystal more quickly and efficiently than attempting to determine such information *ab initio.*

MOLREP is an integrated molecular replacement program that finds molecular replacement solutions using a two-step procedure: (1) rotation function (RF) search orientation of model and (2) cross translation function (TF) and packing function (PF) search position of oriented model. The translation function checks several peaks of the rotation function by computing a correlation coefficient for each peak and sorting the result. The packing function is important in removing incorrect solutions that correspond to overlapping symmetry. MOLREP can be set to search for any number of molecules per asymmetric unit and will automatically stop when no further improvement of the solution can be achieved by adding additional molecules. The molecular replacement software is part of a CCP4 software package (Computer Computational Project, Number 4, 1994; "The CCP4 Suite: Programs for Protein Crystallography". Acta Cryst. D50: 760-763).

DynDom is a fully-automated program that determines protein domains, hinge axes and amino acid residues involved in enzyme movement. DynDom can be used with two conformations of the same protein. Structures can be two X-ray structures or structures generated using simulation techniques such as molecular dynamics or normal mode analysis. The application of DynDom provides a view of the conformational change and visualizes movement of domains as quasi-rigid bodies. DynDom was used to show that MurI goes through a conformational change that corresponds to two rigid domains that move relative to each other through a molecular interface movement. The two conformations seen in the *E. faecalis* structure provided the most convincing case as seen in Figure 3. DynDom illustrated that the different conformations seen with D-Glutamate versus L-Glutamate bound to the same protein and suggested that there is a domain movement associated with the catalytic cycle of the enzyme. The DynDom software is part of a CCP4 software package (Computer Computational Project, Number 4, 1994; "The CCP4 Suite: Programs for Protein Crystallography". Acta Cryst. D50: 760-763).

MAPS (Multiple Alignment of Protein Structures) is an automated program for comparisons of multiple protein structures based upon tertiary structures of crystal coordinates. When homologous proteins with common structural elements are available, the MAPS program can automatically superimpose the three-dimensional models, detect which residues are structurally equivalent among all the structures and provide the residue-to-residue alignment. The structurally equivalent residues are defined by the program according to the approximate position of both main-chain and side-chain atoms of all of the proteins. Thus, in cases in which different primary amino acid sequences are present, the program picks out common tertiary structures of the proteins. (See Figure 2, for example). Based on structure similarity, the program calculates a score of structure diversity which can be used to build a phylogenetic tree.

In another aspect, we provide a method involving molecular replacement to obtain structural information about a molecule or molecular complex of unknown structure using the software programs described above and the coordinates described herein.

### XI. Assay Methods

### A. In vitro

Another aspect involves a method for identifying inhibitors of MurI characterized by the crystal structure and novel binding domains described herein, and the inhibitors themselves. The novel MurI crystal structure of the invention permit the identification of inhibitors of MurI activity. Such inhibitors may bind to all, or a binding domain, of MurI, and may be competitive or non-competitive inhibitors. Once identified and screened for biological activity, these inhibitors may be used therapeutically or prophylactically to block bacterial growth and spread.

One design approach is to probe the MurI of the invention with molecules composed of a variety of different chemical entities to determine optimal sites for interaction between candidate MurI inhibitors and the enzyme. For example, high resolution X-ray diffraction and NMR data collected from crystals saturated with solvent makes it possible to determine where each type of solvent molecule resides within the protein. Small molecules that bind tightly to those sites can then be designed and synthesized and tested for their MurI inhibitor activity.

One embodiment of the present invention relates to a method of identifying an agent that inhibits MurI comprising combining the bacterium with a test agent under conditions suitable for binding of an agent to the active site, and determining whether the test agent inhibits MurI activity, wherein if inhibition occurs, the test agent is an inhibitor of MurI activity.

The present invention encompasses an *in vitro* assay to identify an inhibitor of MurI. The assay can be a single or double enzyme activity as described in detail in the Examples or an equivalent *in vitro* assay system wherein small molecules, proteins, or fragments thereof are added to bacterium prior to the addition of activator (in the case of Gram negative bacterium) and/or substrate. When growth of the bacterial cell wall is inhibited compared to the control (which lacks inhibitor) an inhibitor of MurI has been identified.

One embodiment includes a method of testing inhibitors that bind to the binding domain identified or produced by the computer-assisted models in an *in vitro* assay comprising culturing bacteria.

In one representative embodiment, *H. pylori* MurI was tested for inactivation with a suicide inhibitor, L-serine-O sulfate, which is known to inhibit MurI from *E. coli.* The enzyme was incubated in the presence of 20 mM L-serine-O sulfate, and at different time intervals, aliquots were removed to determine residual activity. The initial velocity of purified recombinant *H. pylori* MurI protein was determined in the single enzyme coupled assay following incubation with the inhibitor L-serine-O-sulfate (LSOS). The control was incubated in an identical manner but without LSOS.

In another representative embodiment, the assay has the steps of culturing a control collection of Gram negative bacteria, such as *E*. *coli,* in the appropriate liquid or solid agar in the presence activator and substrate, culturing a test collection of the Gram negative bacteria in the in the appropriate liquid or solid agar in the presence activator, substrate, and inhibitor, and comparing the growth of the bacteria in the test culture to the control culture wherein if growth is inhibited, an inhibitor of MurI has been identified.

In one exemplary embodiment, the assay has the steps of culturing a control collection of a Gram positive or atypical bacteria, such as *S. aureus* or *H. pylori,* culturing a control collection bacteria in the appropriate liquid or solid agar in the presence substrate, culturing a test collection of bacteria in the in the appropriate liquid or solid agar in the presence substrate, and inhibitor, and comparing the growth of the bacteria in the test culture to the control culture wherein if growth is inhibited, an inhibitor of the MurI hinge region has been identified.

### B. In vivo

One embodiment relates to an *in vivo* analysis of the antibacterial activity of the binding agents comprising infecting a mammalian subject (preferably a non-human primate or a rodent) with a clinically relevant amount of bacteria sufficient to establish an infection in the subject. After the bacteria has established an infection, the inhibitor (e.g., antibacterial binding agent) will be administered to the subject. A separate control group will be administered a placebo. Tissue, blood, and blood products can be collected at various time points to determine the course of infection, and those inhibitors which reduce (partially or totally) the extent of infection are determined to be effective inhibitors of infections.

### XII. Pharmaceutical Compositions/Preparations/Packages

Inhibitors of the invention can be formulated as a pharmaceutical composition for administration to mammalian subjects as a broad spectrum therapeutic for bacterial infections. In a preferred embodiment, the pharmaceutical composition is formulated for intravenous or oral administration.

The inhibitor can be present in a composition, such as a pharmaceutical composition, which also comprises, for example, a pharmaceutically acceptable carrier, a flavoring agent, or adjuvant. Pharmaceutically-acceptable carriers and their formulations are well-known and generally described in, for example, Remington's pharmaceutical Sciences (18th Edition, ed. A. Gennaro, Mack Publishing Co., Easton, PA, 1990). One exemplary pharmaceutically acceptable carrier is physiological saline. Other acceptable examples of pharmaceutically acceptable carriers include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilcate, polyvinyl pyrrolidone, cellulose-based substances, polyethylene glycol, sodium carboxymethylcellulose, polyacrylates, waxes, polyethylene-polyoxytpropylene-block polymers, wool fat and self-emulsifying drug delivery systems (SEDDS) such as α-tocopherol, polyethyleneglycol 1000 succinate, or other similar polymeric delivery matrices. The pharmaceutical compositions of the present invention may be administered to a patient, together with a compound of the present invention. The pharmaceutical compositions and methods will be useful generally for controlling, treating or reducing the advancement, severity or effects of bacterial infections *in vivo.* Such pharmaceutical compositions can comprise two or more antibacterial agents as described herein.

The compounds/compositions are also useful as commercial reagents which effectively bind to MurI. As commercial reagents, the compounds and their derivatives, may be used to block MurI activity in biochemical or cellular assays for bacterial MurI or its homologs or may be derivatized to bind to a stable resin as a tethered substrate for affinity chromatography applications. These and other uses which characterize commercial MurI inhibitors will be evident to those of ordinary skill in the art.

The compounds/compositions may be employed in a conventional manner for controlling bacterial infection levels *in vivo* and for treating diseases or reducing the advancement or severity of effects which are mediated by bacteria. Such methods of treatment, their dosage levels, modes of administration and requirements may be selected by one of ordinary skill in the art from available methods and techniques.

Alternatively, the compounds/compositions may be used in compositions and methods for treating or protecting individuals against bacterial infections or diseases over extended periods of time. The compounds may be employed in such compositions either alone or together with other compounds of this invention in a manner consistent with the conventional utilization of enzyme inhibitors in pharmaceutical compositions. For example, a compound may be combined with pharmaceutically acceptable adjuvants conventionally employed in vaccines and administered in prophylactically effective amounts to protect individuals over an extended period of time against bacterial infections or diseases.

One embodiment is a pharmaceutical package comprising a pharmaceutical composition of a MurI inhibitor that binds to the hinge region and instructions for its use in the treatment of a bacterial infection.

One embodiment is a pharmaceutical composition comprising a MurI inhibitor for the treatment of bacterial infections.

### XIII. Methods of Treating Bacterial Infections

One embodiment comprises a method of treating a subject having a bacterial infection comprising administering a pharmaceutical composition of the MurI inhibitor. In one embodiment, the bacterial infection is caused by a Gram negative, a Gram positive, or an atypical bacterium. This structure is clearly useful in the structure-based design of MurI inhibitors, which may be used as therapeutic agents against bacterial infections. In one embodiment, the bacterial infection is caused by *Helicobacter pylori, Campylobacter jejuni, Porphyromonas gingivalis, Pseudomonas aeruginosa, Deinococcus radiodurans, Borrelia burgdorferi, Treponema pallidum, Vibrio cholerae, Shewanella putrefaciens, Escherichia coli, Haemophilus influenzae, Mycobacterium tuberculosis, Mycobacterium leprae, Lactobacillus fermentum, Pediococcus, pentosacceus, Enterococcus fecalis, Enterococcus faecium, Streptococcus pyogenes, Streptococcus pneumoniae, Bacillus spaericus, Staphylococcus aureus, Staphylococcus haemolyticus, Bacillus anthracis,* or *Bacillus subtilus* infection.

Pharmaceutical compositions also include compositions (formulations) which may be administered orally, parenterally, by inhalation spray, topically, via ophthalmic solution or ointment, rectally, intranasally, buccally, vaginally, via an implanted reservoir, intramuscularly, intraperitoneally, and intravenously to the subject. A patient is preferably a mammal. In a further embodiment, the mammal is a human, a primate, a dog, a horse, a cow, a sheep, a rat, a mouse, a pig, etc.

In one embodiment, the MurI inhibitor is administered continuously to the subject. In one embodiment, the MurI inhibitor is administered to the subject every 1-24 hours. In one embodiment, administration continues until the bacterial infection is eradicated.

Dosage levels would be apparent to one of ordinary skill in the art and would be determined based on a variety of factors, such as body weight of the individual, general health, age, the activity of the specific compound employed, sex, diet, time of administration, rate of excretion, drug combination, the severity and course of the disease, and the patient's disposition to the disease and the judgment of the treating physician. The amount of active ingredient that may be combined with the carrier materials to produce a single dosage form with vary depending upon the host treated and the particular mode of administration. A typical compound preparation will contain from about 5% to about 95% active compound (w/w). Preferably, such preparations contain from about 20% to about 80% active compound.

Upon improvement of a patient's condition, a maintenance dose of a compound or composition of the present invention may be administered, if necessary; and the dosage, the dosage form, or frequency of administration, or a combination thereof, may be modified. In some cases, the subject may require intermittent treatment on a long-term basis upon any recurrence of disease symptoms.

By "treating" a patient suffering from a bacterial infection it is meant that the patient's symptoms are partially or totally alleviated, or remain static following treatment according to the invention. A patient who has been treated will exhibit a partial or total alleviation of symptoms and/or bacterial load. The term "treatment" is intended to encompass prophylaxis, therapy and cure.

A "patient" or "subject" to be treated by the subject method can mean either a human or non-human animal. The mammal can be a primate (e.g., a human, a chimpanzee, a gorilla, a monkey, etc.), a domesticated animal (e.g., a dog, a horse, a cat, a pig, a cow), or a rodent (e.g., a mouse or a rat), etc.

The phrase "therapeutically effective amount" as used herein means that amount of a compound, material, or composition comprising a compound of the present invention which is effective for producing a desired therapeutic effect by blocking cell wall synthesis of bacterial cells in a patient.

Each of the compositions of the present invention can be used as a composition when combined with a pharmaceutically acceptable carrier or excipient. "Carrier" and "excipient" are used interchangeably herein.

The phrase "pharmaceutically acceptable" is employed herein to- refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. "Pharmaceutically acceptable carrier" is defined herein as a carrier that is physiologically acceptable to the recipient and that does not interfere with, or destroy, the therapeutic properties of the MurI inhibitor with which it is administered.

### XIV. Methods for Conducting Business

One embodiment encompasses a method for conducting a pharmaceutical business having the steps of isolating one or more MurI inhibitors that bind to MurI expressed in bacteria; generating a composition comprising a MurI inhibitor, which composition has a minimal inhibitory concentration (MIC) or 8 µg/mL or less; conducting therapeutic profiling of the composition, for efficacy and toxicity in animals; preparing a package insert describing the composition for treatment of bacterial infections; and, marketing the composition for treatment of bacterial infections.

One embodiment encompasses a method for conducting a life science business having the steps of isolating one or more MurI inhibitors that bind to MurI expressed in bacteria; generating a composition comprising a MurI inhibitor, which composition has a minimal inhibitory concentration (MIC) of 8 µg/mL or less; licensing, jointly developing or selling, to a third party, the rights for selling the composition.

One embodiment encompasses a method for conducting pharmaceutical business having the steps of (a) isolating one or more MurI inhibitors that bind to MurI expressed in bacteria with a K_{d} of 1 µM or less; (b) generating a composition comprising said MurI inhibitors, which composition has a minimal inhibitory concentration (MIC) of 8 µg/mL or less; (c) conducting therapeutic profiling of the composition for efficacy and toxicity in animals; (d) preparing a package insert describing the use of the composition for antibacterial therapy; and, (e) marketing the composition for use as an antibacterial agent.

One embodiment encompasses a method for conducting a life science business having the steps of (a) isolating one or more MurI inhibitors that bind to MurI expressed in bacteria with a K_{d} of 1 µM or less; (b) generating a composition comprising said MurI inhibitors, which composition has a minimal inhibitory concentration (MIC) of 8 µg/mL or less; and (c) licensing, jointly developing or selling, to a third party, the rights for selling the composition.

### XV. Equivalents

It will be apparent to those skilled in the art that various modifications and variations can be made in the present invention without departing from the scope of the invention. Other embodiments of the invention will be apparent to those skilled in the art form consideration of the specification and practice of the invention disclosed herein. It is intended that the specification and examples be considered as exemplary only, with the true scope being indicated by the claims.

### EXAMPLES

The present invention is illustrated by the following examples which should not be construed as limiting in any way. The contents of all cited references (including literature references, issued patents, published patent applications as cited throughout this application) are hereby expressly incorporated by reference.

The practice of the present invention will employ, unless otherwise indicated, conventional techniques of cell biology, cell culture, molecular biology, microbiology and recombinant DNA, X-ray crystallography, and molecular modeling which are within the skill of the art. Such techniques are explained fully in the literature. See, for example, Molecular Cloning: A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); B. Perbal, A Practical Guide To Molecular Cloning (1984); the treatise, Methods In Enzymology (Academic Press, Inc., N.Y.); Methods In Enzymology, Vols. 154 and 155 (Wu et al. eds.), Crystallography made crystal clear: a guide for users of macromolecular models (Gale Rhodes, 2nd Ed. San Diego: Academic Press, 2000).

### Example 1: Cloning, Crystallization and Characterization of H. pylori MurI

### A. Cloning, purification, and characterization of the gene encoding the MurI of H. pylori

Cloning and purification of MurI has been previously described in U.S. Provisional Applications 60/435,087 and 60/435,527.

The *H. pylori* genome contains an open reading frame (ORF) of 255 amino acids that was found to have homology to the *Staphylococcus haemolyticus* MurI gene (dga) (NCBI Accession number U12405) and to the *E*. *coli murI* gene which encodes MurI activity in that organism. To evaluate whether this *H. pylori* ORF encodes a protein with MurI activity, the gene was isolated by polymerase chain reaction (PCR) amplification cloning, overexpressed in *E*. *coli,* and the protein purified to apparent homogeneity. A simple assay for MurI activity resulting in the isomerization ofD-glutamic acid to L-glutamic acid was developed to facilitate purification and for future use as a high-throughput drug screen. The ORF in *H*. *pylori* has been found by gene disruption studies to be essential for viability of *H. pylori* cells in laboratory culture.

### Cloning of H. pylori murl gene encoding MurI

A 765 base pair DNA sequence encoding the *murI* gene *of H. pylori* was isolated by polymerase chain reaction (PCR) amplification cloning. A synthetic oligonucleotide primer (5'-AAATAGTCATATGAAAATAGGCGTTTTTG -3' (SEQ ID NO: 35)) encoding an *NdeI* restriction site and the 5' terminus of the *murI* gene and a primer (5'-AGAATTCTATTACAATTTGAGCCATTCT -3' (SEQ ID NO: 36)) encoding an *EcoRI* restriction site and the 3' end of the *murI* gene were used to amplify the *murI* gene of *H. pylori* using genomic DNA prepared from the J99 strain *of H. pylori* as the template DNA for the PCR amplification reactions. (Current Protocols in Molecular Biology, John Wiley and Sons, Inc., F. Ausubel et al., editors, 1994). To amplify a DNA sequence containing the *murI* gene, genomic DNA (25 nanograms) was introduced into each of two reaction vials containing 1.0 micromole of each synthetic oligonucleotide primer, 2.0 mM MgCl₂, 0.2 mM of each deoxynucleotide triphosphate (dATP, dGTP, dCTP and dTTP), and 1.25 units of heat stable DNA polymerases (Amplitaq, Roche Molecular Systems. Inc., Branchburg, NJ, USA) in a final volume of 50 microliters. The following thermal cycling conditions were used to obtain amplified DNA products for the *murI* gene using a Perkin Elmer Cetus/ GeneAmp PCR System 9600 thermal cycler:
Conditions for amplification of *H. pylori murI* (SEQ ID NO: 1):
   Denaturation at 94°C for 2 minutes;
   2 cycles at 94°C for 15 seconds, 30°C for 30 seconds, and 72°C for 15 seconds;
   23 cycles at 94°C for 15 seconds, 53°C for 30 seconds, and 72°C for 15 seconds.

Reactions were concluded at 72°C for 20 minutes.

Upon completion of thermal cycling reactions, the amplified DNA was washed and purified using the Qiaquick Spin PCR purification kit (Qiagen, Gaithersburg, MD USA). The amplified DNA sample was subjected to digestion with the restriction-endonucleases, *NdeI* and *EcoRI* (New England Biolabs, Beverly, MA USA) (Current Protocols in Molecular Biology, *Ibid).* The DNA samples from each of two reaction mixtures were pooled and subjected to electrophoresis on a 1.0% SeaPlaque (FMC BioProducts, Rockland, ME, USA) agarose gel. DNA was visualized by exposure to ethidium bromide and long wave UV irradiation. Amplified DNA encoding the *H. pylori murI* gene was isolated from agarose gel slices and purified using the Bio 101 GeneClean Kit protocol (Bio 101 Vista, CA USA).

### Cloning of H. pylori DNA sequences into the pET-23 prokaryotic expression vector:

The pET-23b vector can be propagated in any *E. coli* K-12 strain, e.g., HMS174, HB101, JM109, DH5α, etc., for the purpose of cloning or plasmid preparation. Hosts for expression include *E*. *coli* strains containing a chromosomal copy of the gene for 17 RNA polymerase. These hosts are lysogens of bacteriophage DE3, a lambda derivative that carries the *lacI* gene, the lacUV5 promoter and the gene for T7 RNA polymerase. T7 RNA polymerase is induced by addition of isopropyl-B-D-thiogalactosidase (IPTG), and the T7 RNA polymerase transcribes any target plasmid such as pFT-28b, carrying its gene of interest. Strains used in our laboratory include: BL21(DE3) (Studier, F,W., Rosenberg, A.H., Dunn, J.J., and Dubendorff, LW. Meth. Enzymol. 185: 60-89, 1990). The pET-23b vector (Novagen, Inc., Madison, WI, USA) was prepared for cloning by digestion with *NdeI* and EcoRl (Current Protocols in Molecular Biology, *ibid*)*.* Following digestion, the amplified, agarose get-purified DNA fragment carrying the *murI* gene was cloned (Current Protocols in Molecular Biology, *ibid*) into the previously digested pET-23b expression vector. Products of the ligation reaction were then used to transform the BL21(DE3) strain of *E. coli.*

### Transformation of competent bacteria with recombinant plasmids:

Competent bacteria, *E. coli,* strain BL21 or strain BL21(DE3), were transformed with recombinant pET23- *murI* expression plasmid carrying the cloned *H. pylori* sequence according to standard methods (Current Protocols in Molecular, *ibid).* Briefly, 1 microliter of ligation reaction was mixed with 50 microliters of electrocompetent cells and subjected to a high voltage pulse, after which, samples were incubated in 0.45 milliliters SOC medium (0.5% yeast extract, 2.0% tryptone, 10 mM NaCl, 2.5 m KCl, 10mM MgCl₂, 10mM MgSO₄ and 20mM glucose) at 37°C with shaking for 1 hour. Samples were then spread on LB agar plates containing 100 microgram/ml ampicillin for growth overnight. Transformed colonies of BL21 were then picked and analyzed to evaluate cloned inserts as described below. Identification of recombinant pET expression plasmids carrying *H. pylori* sequences:

Individual BL2I clones transformed with recombinant pET-23- *murI* were analyzed by PCR amplification of the cloned inserts using the same forward and reverse primers specific for each *H. pylori* sequence that were used in the original PCR amplification cloning reactions. Successful amplification verified the integration of the *H. pylori* sequences in the expression vector (Current Protocols in Molecular Biology, *ibid).*

### Isolation and Preparation of plasmid DNA from BL21 transformants:

Colonies carrying pET-23- *murI* vectors were picked and incubated in 5 mls of LB broth plus 100 microgram/ml ampicillin overnight. The following day plasmid DNA was isolated and purified using the Qiagen plasmid purification protocol (Qiagen Inc., Chatsworth, CA, USA).

### Cloning and expression of the E. coli groE operon:

It has been demonstrated that co-expression of the *E. coli murI* gene with the genes in the *E. coli groE* operon reduces the formation of insoluble inclusion bodies containing recombinant MurI (Ashiuchi, M., Yoshimura, 1., Kitamura, T., Kawata, Y., Nagai, J., Gorlatov, S., Esaki, N. and Soda, K. 1995, J. Biochem. 117: 495-498). The *groE* operon encodes two proteins, *GroES* (97 amino acids) and *GroEL* (548 amino acids), which are molecular chaperones. Molecular chaperones cooperate to assist the folding of new polypeptide chains (F. Ulrich Hartl, 1996, Nature London 381: 571-580).

The 2210 bp DNA sequence encoding the *groE* operon of *E*. *coli* (NCBI Accession number X07850) was isolated by polymerase chain reaction (PCR) amplification cloning. A synthetic oligonucleotide primer (5'-GCGAATTCGATCAGAATTTTTTTTCT (SEQ ID NO: 37)) encoding an *EcoRI* restriction site and the 5' terminus of the *groE* operon containing the endogenous promoter region of the groE operon and a primer (5'-ATAAGTACTTGTGAATCTTATACTAG -3' (SEQ ID NO: 38)) encoding a *ScaI* restriction site and the 3' end of the *groEL* gene contained in the *groE* operon were used to amplify the *groE* operon of *E*. *coli* using genomic DNA prepared from *E*. *coli* strain MG1655 as the template DNA for the PCR amplification reactions (Current Protocols in Molecular Biology, *Ibid*)*.* to amplify a DNA sequence containing the *E. coli groE* operon genomic DNA (12.5 nanograms) was introduced into each of two reaction vials containing 0.5 micromoles of each synthetic oligonucleotide primer, 1.5 mM MgCl₂, 0.2 mM each deoxynucleotide triphosphate (dATP, dGTP, dCTP and dTTP) and 2.6 units heat stable DNA polymerases (Expanded High Fidelity PCR System, Boehringer Mannheim, Indianapolis, Indiana) in a final volume of 50 microliters. The following thermal cycling conditions were used to obtain amplified DNA products for the *groE* operon using a Perkin Elmer Cetus/ GeneAmp PCR System 9600 thermal cycler: culture at a final concentration of 1.0 mM. Cells were cultured overnight to induce gene expression of the *H. pylori* recombinant DNA constructions.

After induction of gene expression with IPTG, bacteria were pelleted by centrifugation in a Sorvall RC-3B centrifuge at 3,000 x g for 20 minutes at 4°C. Pellets were re-suspended in 50 milliliters of cold 10mM Tris-HCl, pH 8.0, 0.1 M NaCl and 0.1 mM EDTA (STE buffer). Cells were then centrifuged at 2000 x g for 20 min at 4°C. Pellets were weighed (average wet weight 6 grams/liter) and processed to purify recombinant protein as described below.

### Purification of soluble MurI:

All steps were carried out at 4°C. Cells were suspended in 4 volumes of lysis buffer (50mM Potassium phosphate, p 7.0, 100mM NaCl, 2mM EDTA, 2mM EGTA, 10% glycerol, 10 mM D,L-glutamic acid, 0.1 % β-mercaptoethanol, 200 micrograms/ml lysozyme, 1 mM PMSF, and 10 µg/ml each of leupeptin, aprotinin, pepstatin, L-1-chloro-3-(4-tosylamido]-7-amino-2-heptanone (TLCK), L-1-chloro-3-phenyl-2-butanone (TPCK), and soybean trypsin inhibitor, and ruptured by three passages through a small volume microfluidizer (Model M-110S, Microfluidics International Corporation, Newton, MA). The resultant homogenate was diluted with one volume of buffer A (10 mM Tris-HCl pH 7.0, 0.1 mM EGTA, 10 % glycerol, 1 mM D,L-Glutamic acid, 1 mM PMSF, 0.1 % beta-mercaptoethanol), and 0.1 % Brij-35, and centrifuged (100,000 x g, 1 h) to yield a clear supernatant (crude extract).

After filtration through a 0.80-µm filter, the extract was loaded directly onto a 20 ml Q-Sepharose column pre-equilibrated in buffer A containing 100 mM NaCl and 0.02% Brij-35. The column was washed with 100 ml (5 bed volumes) of Buffer A containing 100 mM NaCl and 0.02% Brij-35, then developed with a 100-ml linear gradient of increasing NaCl (from 100 to 500 mM) in Buffer A. A band of Mᵣ = 28,000 kD corresponding to MurI, the product of the recombinant *H. pylori murI* gene, eluted at a gradient concentration of approximately 200-280 mM NaCl. Individual column fractions were then characterized for MurI activity (see below for description of assay) and the protein profile of the fractions were analyzed on 12% acrylamide SDS-PAGE gels.

Fractions containing MurI were pooled, brought to 70% saturation with solid (NH₄)₂SO₄, stirred for 20 minutes, and then centrifuged at 27,000 x g for 20 minutes. The resulting pellet was re-suspended in lysis buffer to a final volume of 8 ml and loaded directly onto a 350-ml column (2.2 x 92 cm) of Sephacryl S-100HR gel filtration medium equilibrated in buffer B (10 mM Hepes pH 7.5, 150 mM NaCl, 0.1 mM EGTA, 10% glycerol, 1 mM D,L-glutamic acid, 0.1 mM PMSF, 0.1% beta-mercaptoethanol) and run at 30 ml/hour. Fractions found to contain a MurI activity were pooled, and 0.5 volume of buffer C (10 mM Tris pH 7.5, 0.1 mM EGTA,10% glycerol, 1 mM D,L-glutamic acid, 0.1 mM PMSF, 0.1 % beta-mercaptoethanol) was added (to reduce the NaCl concentration to 100 mM) and loaded onto a MonoQ 10/10 high- pressure liquid chromato graphy column equilibrated in buffer C containing 100 mM NaCl. The column was washed with 2 bed volumes of this buffer and developed with a 40 mL linear gradient of increasing NaCl (from 100 to 500). MurI eluted as a sharp peak at 310 mM NaCl. Fractions containing a MurI activity were pooled, concentrated by dialysis against storage buffer [50% glycerol, 10 mM 3-(N-morpholino-propanesulfonic acid (MOPS) pH 7.0,150 mM NaCl, 0.1 mM EGTA, 0.02% Brij-35, 1 mM dithiothreitol (DTT)], and stored at -20°C.

### Preparation of glutamate-free H. pylori MurI

1 mL of 1-25mg/ml *H. pylori* MurI stored in the presence of 1-10 mM Glutamate and 10- 50% glycerol were dialyzed 3 times against 1L of 100 mM KH₂PO₄, 100 mM Na₂SO₄, 5 mM DTT and 1 mM EDTA, pH 8.2 for 3 hours at 4°C.

No glutamate was observable for the dialyzed protein by 1D-NMR or by using L-glutamate dehydrogenase in a photometric enzyme assay. The specific activity of the glutamate free *H. pylori* MurI was unaltered (k_{cat} =1.8/min) compared to an enzyme stored in the presence of glutamate.

### B. Assays for MurI activity

### 1. Conversion of D-glutamate to L-glutamate (single enzyme coupled assay):

In this assay, the conversion of D-glutamic acid to L-glutamic acid is coupled to the conversion of L-glutamic acid and NAD+ by L-glutamate dehydrogenase to 2-oxoglutarate, ammonia. The production of NADH is measured as an increase of absorbance at 340 nm (the reduction ofNAD+ to NADH) at 37°C. The standard assay mixture (adapted from Choi, S-Y,, Esaki, N., Yoshimura, T., and Soda, K. 1991, Protein Expression and Purification 2: 90-93) contained 10 mM Tris-HCI, pH 7.5, 5 mM NAD+, 5 Units/ml L-glutamate dehydrogenase, varying concentrations of the substrate D-glutamic Acid (0.063 mM to 250 mM and the purified recombinant *H. pylori* enzyme MurI (1 µg to 50 µg). The reaction was started by the addition of either the substrate D-glutamate or the recombinant MurI after a pre-incubation at 37°C for 5 minutes with all of the other assay ingredients. The change in absorbance at 340 nm was measured in a Spectra MAX 250. Initial velocities were derived from the initial slopes.

### 2. Conversion of D-glutamate to L-glutamate (two enzyme coupled assay)

The activity of MurI interconversion of the enantiomers of glutamic acid, can be measured using D-glutamic acid as substrate as described by the methods of Gallo and Knowles (Gallo, K.A. and Knowles, J.R., 1993, Biochemistry 32, 3981-3990). The assay originally was used to measure the MurI activity of *Lactobacillus fermenti* and can be adapted for the measurement of MurI activity of the *H. pylori murl* gene product isolated as a recombinant protein from *E*. *coli.* In this assay, the measurement of the activity of MurI is linked to an OD change in the visible range in a series of coupled reactions to the activities of L-glutamate dehydrogenase (reduction of NAD to NADH). Initial rates were determined by following the increase in absorbance at 340 nm in a reaction volume of 200 ml containing 50 mM Tris-HCl pH 7.8, 4% v/v glycerol, 10 mM NAD, 60 Units/ml L-glutamate dehydrogenase, and varying concentrations of either substrate (from 0.063 mM to 250 mM D-glutamic acid) or purified enzyme (from 1 µg to 50 µg). After a pre-incubation of all reagents except either the substrate (D-glutamate) or the enzyme (*murI* gene product) for a period of 5 minutes, reactions were initiated by adding the missing ingredient (i.e., the enzyme or the substrate, as required), and the increase in optical density at 500 nm was measured in a Microplate Spectophotometer System (Molecular Devices, Spectra MAX 250). Measurements were followed for 20 minutes, and initial velocities were derived by calculating the maximum slope for the absorbance increases.

### 3. Conversion of L-Glutamate to D-Glutamate (two enzyme coupled assay)

The activity of MurI interconversion of the enantiomers of glutamic acid was measured using L-Glutamic acid as a substrate. In this assay, the conversion of L-Glutamate to D-Glutamate was monitored spectrophotometrically through a two enzyme coupling system wherein, the production of D-glutamate is coupled to the incorporation of D-Glutamate into UDP-MurNAc-Ala-D-Glu by recombinant *E*. *faecalis* MurD, with concomitant hydrolysis of ATP to ADP and inorganic phosphate. The inorganic phosphate produced in this reaction is subsequently consumed by the enzyme purine-nucleoside phosphorylase (PNP) reacting with the chromogenic substrate 2-amino-6-mercapto-7-methylpurine ribonucleoside (MESG) which has a spectral band at 360nm as described in Webb, M.R, Proc. Natl. Acad. Sci USA, 89: 4884-4887 (1992). Initial rates of reaction were determined by following the increase of absorbance at 360nm in a 100 µL, reaction volume containing 85 µL of reaction buffer (58 mM Tris, pH=8.0, 23.5 mM ammonium acetate, 23.5 mM magnesium acetate, 2.94 mM dithiothreitol, 2.94 mM adenosine triphosphate, 0.47 mM UDP-MurNAc-Ala, 0.47 mM MESG, 1.17 units./mL PNP, 16 µg/mL *E. faecalis* MurD, and 30nM MurI) and 15 µL of L-Glutamate stock (0.05 -10 mM). The increase in optical density at 360nM was measured continuously in at 96 well microtitre plate in a Microplate Spectrophotometer System (Molecular Devices, SpectraMAX 250). Measurements were followed for 20min and initial velocities were derived by calculating the maximum slope for the absorbance increases.

### Results

### Kinetic properties of recombinant H. pylori enzyme:

Kinetic constant for recombinant MurI were estimated by assaying its activity at various concentrations of protein and D-glutamic acid as described above. Purified recombinant *H. pylori* MurI exhibits a Vmax of about 300 nanomoles/min/mg protein (kcat = 8.6 min -1) and a Km of 100 micromolar for D-glutamate. Severe substrate inhibition was observed. Although the Vmax value is lower than that observed for highly purified MurI from some other bacterial species, the Km for D-glutamic acid is lower than that observed for the enzyme from most other species, resulting in a catalytic efficiency (kcat/Km) which is typical of purified preparation from *E*. *coli* and *P*. *pentococcus.*

### C. Crystallization methodology

One method of crystallizing *H. pylori* Murl complexed with glutamate includes the steps of: (a) preparing a first solution comprising reducing agent, substrate, salt, bacteriocide, buffer pH 6.5-9.5, and MurI, wherein the reducing agent, substrate, salt, bacteriocide and buffer pH 6.5-9.5, is each present in sufficient concentration to inhibit oxidation of the solution, bind to the MurI, stabilize the protein and prevent aggregation, inhibit bacterial growth, and control the pH of the solution; (b) preparing a second solution comprising salt and buffer pH 6.5-9.5, wherein the salt and buffer pH 6.5-9.5 is each present in sufficient concentration to stabilize the protein and prevent aggregation, and control the pH of the solution; (c) combining the first solution and the second solution, thereby producing a combination; and (d) forming drops from the combination in a method of crystallization under conditions in which crystals of MurI are produced, whereby, crystals of MurI are produced. The first solution can additionally include glycerol in sufficient concentration to facilitate freezing of the crystals and stabilize the protein, and the second solution can additionally include a precipitant, an organic additive, and a reducing agent, each in sufficient concentration to sequester water and force protein molecules out of solution, precipitate the protein and effect the dielectric of the solution, and inhibit oxidation of the solution, respectively.

Batch, vapor diffusion, or dialysis methods of crystallization can be employed to crystallize MurI.

The need for a reducing agent can be relieved by performing the crystallization under anaerobic conditions; such as under oil or in an anaerobic box.

Organic additives of the present invention include, for example, methanol, ethanol, or 2-methyl-2,4-pentanediol (MPD).

One skilled in the art would recognize that a wide variety of well-known buffers (e.g., HEPES, Tris, MOPS, etc.) could be used to buffer the solutions at the proper pH.

Examples of a bacteriocide is ethylene glycol bis (92-aminoethyl ether)-N,N,N',N'-tetraacetic acid (EGTA) or ethylenediaminetetraacetic acid (EDTA) or sodium azide.

Salts in the second solution can be, for example, magnesium chloride, magnesium sulfate, magnesium foramate, lithium sulfate, lithium chloride, ammonium acetate, ammonium sulfate, lithium acetate, ammonium citrate, or lithium citrate.

Reducing agents in the first or second solutions can be, for example, dithiothreitol (DTT), Tri(2-carboxyethyl)phosphine hydrochloride (TCEP), or beta-mercaptoethanol, to prevent, or retard oxidation of the protein.

Precipitants in the second solution can range from about 0% to about 55%, polyethylene glycol (PEG), or a derivative thereof (e.g., mono-methyl-ether poly-ethylene glycol (MME PEG)). More specifically can range from about 5% to about 40%, more specifically can range about 15% to about 30% PEG, or from about 20% to about 25% PEG. A variety of precipitants, such as PEG (e.g., PEG 500 to 20,000, or any intermediate PEG), or a derivative thereof, can be used. More specifically the precipitant in the second solution is PEG 1,000-10,000, or PEG 2,000-6,000.

One crystal of *H. p.* MurI was made by the process of: (a) preparing a first solution of from about one to about 100 mM D,L-glutamic acid, from about 0.1 mM to about 5 mM reducing agent, about 0-30% glycerol, about 1-500 mM buffer pH 6.5-9.5, about 50-500 mM salt, about 0.1 mM bacteriocide, and from about 1 mg/ml to about 50 mg/ml *H. pylori* MurI; (b) preparing a second solution of about 50-150 mM salt, about 0-55% precipitant, from about 0-15% organic additive, about 0-30% glycerol, about 5-200 mM buffer pH 6.5-9.5, and about 0-50 mM reducing agent; and (c) combining the first solution and the second solution into drops thereby, producing a combination used in a method of crystallization.

One embodiment of the present invention relates to selected crystals that were flash frozen in cold nitrogen stream and tested on in-house detector.

One *H. pylori* MurI crystal of the present invention was made by the process of preparing a first solution of from about one to about 100 mM D,L-glutamic acid, from about 1 mM to about 5 mM reducing agent, from about 0 to about 30% glycerol, from bacteriocide 1 to about 100 mM buffer pH 6.5-9.5, from about 50 to about 500 mM salt, about 0.1 mM EGTA, and from about 1 mg/ml to about 50 mg/ml *H. pylori* MurI; preparing a second solution of about 50-150 mM salt, from about 0 to about 55% precipitant, from about 0 to about 15% organic additive, from about 0 to about 30% glycerol, from about 5 to about 200 mM buffer pH 6.5-9.5, and from about 1 to about 10 mM reducing agent; combining the first solution and the second solution into drops thereby producing a combination; and forming drops from the combination in a method of crystallization under conditions in which crystals of MurI are produced, whereby crystals of MurI were produced.

One crystallized complex of the present invention is characterized as belonging to the orthorhombic space group P2₁2₁2₁ and having cell dimensions of a = 62.18 Å, b = 81.07 Å and c =113.82 Å, wherein α = 90°, β = 90°, and γ = 90°, wherein the crystallized complex was made by the process of preparing a first solution of about 10 mM D,L-glutamic acid, about 1 mM DTT, about 10% glycerol, about 10 mM MOPS pH 7.0, about 50 mM NaCl, about 0.1 mM EGTA, and about 7 mg/ml *H. pylori* MurI; preparing a second solution of about 80 mM MgCl₂, about 25% PEG 3350, from about 8% methanol, about 10% glycerol, about 100 mM Tris pH 8.0, and about 5 mM DTT; combining the first solution and the second solution, thereby producing a combination; and forming drops from the combination in a method of crystallization under conditions in which crystals of MurI were produced.

Another crystallized complex of the present invention is characterized as belonging to the monoclinic space group P2₁ and having cell dimensions of a = 59.20 Å, b = 82.40 Å and c = 106.50 Å, wherein α = 90°, β = 92.15°, and γ = 90°, wherein the crystallized complex was made by the process of preparing a first solution of about 5 mM D,L-glutamic acid, about 1 mM TCEP, about 200 mM ammonium acetate pH 7.4, and about 10 mg/ml *H. pylori* MurI; preparing a second solution of about 0.2 mM MgCl₂, about 20-25% PEG 4000, about 20% glycerol, about 100 mM Tris pH 8.5; combining the first solution and the second solution, thereby producing a combination; and forming drops from the combination in a method of crystallization under conditions in which crystals of MurI were produced.

Another crystallized complex of the present invention is characterized as belonging to the monoclinic space group P2₁ and having cell dimensions of a = 52.28 Å, b = 78.96 Å and c = 59.15 Å, wherein α = 90°, β = 92.64°, and γ = 90°, wherein the crystallized complex was made by the process of preparing a first solution of about 5 mM D,L-glutamic acid, about 1 mM TCEP, about 20-25% PEG 4000, about 200 mM ammonium acetate pH 7.4, and about 10 mg/ml *H. pylori* MurI; preparing a second solution of about 200 mM MgSO₄, about 100 mM Tris pH 8.5, about 25% PEG 4000; combining the first solution and the second solution, thereby producing a combination; and forming drops from the combination in a method of crystallization under conditions in which crystals of MurI were produced.

Another crystallized complex of the present invention is characterized as belonging to the monoclinic space group P2₁ and having cell dimensions of a = 52.02 Å, b = 80.66 Å and c = 59.18 Å, wherein α = 90°, β = 92.65°, and γ = 90°, wherein the crystallized complex was made by the process of preparing a first solution of about 5 mM D,L-glutamic acid, about 1 mM TCEP, about 200 mM ammonium acetate pH 7.4, and about 10 mg/ml *H. pylori* MurI; preparing a second solution of about 200 mM sodium acetate, about 100 mM Tris pH 8.5, about 25% PEG 4000, and about 15% glycerol; combining the first solution and the second solution, thereby producing a combination; and forming drops from the combination in a method of crystallization under conditions in which crystals of MurI were produced.

Another crystallized complex of the present invention is characterized as belonging to the monoclinic space group P2₁ and having cell dimensions of a = 52.61 Å, b = 78.40 Å, and c = 59.43 Å, and wherein α = 90°, β = 92.33°, γ = 90°, wherein the crystallized complex was made by the process of preparing a first solution of about 5 mM D,L-glutamic acid, about 1 mM TCEP, about 200 mM ammonium acetate pH 7.4, and about 10 mg/ml *H. pylori* MurI; preparing a second solution of about 200 mM LiSO₄, about 100 mM Tris pH 8.5, and about 25% PEG 3350; combining the first solution and the second solution, thereby producing a combination; and forming drops from the combination in a method of crystallization under conditions in which crystals of MurI were produced.

One crystallized complex of the present invention is characterized as belonging to the orthorombic space group P2₁2₁2₁ and having cell dimensions of a = 62.9 Å, b = 80.8 Å and c =113.6 Å, wherein α = 90°, β = 90°, and γ = 90°, wherein the crystallized complex was made by the process of preparing a first solution of about 10 mM D,L-glutamic acid, about 1 mM DTT, about 10% glycerol, about 10 mM MOPS pH 7.0, about 50 mM NaCl, about 0.1 mM EGTA, and about 7 mg/ml *H. pylori* MurI; preparing a second solution of about 80 mM MgCl₂, about 25% PEG 3350, from about 8% methanol, about 10% glycerol, about 100 mM Tris pH 8.0, and about 5 mM DTT; combining the first solution and the second solution, thereby producing a combination; and forming drops from the combination in a method of crystallization under conditions in which crystals of MurI were produced.

Crystals having the atomic coordinate of Figure 5 were obtained by vapor diffusion using the hanging drop method. (''Protein Crystallization", Terese M. Bergfors (Ed.), International University Line, pages 7-15, 1999.) Protein had been stored at -80 degrees at a concentration of 10 mg/ml in a buffer containing 200 mM ammonium acetate pH 7.4, 5 mM D,L-glutamate, and 1 mM TCEP. The reservoir solution typically contained 25-35% PEG 4000, 200 mM magnesium sulfate, 100 mM Tris pH 8.0-9.0, and 1 mM TCEP. Hanging drops were set up by mixing 4 microliters of protein solution with either 2 or 4 microliters of the reservoir solution. Plate shaped crystals appear overnight and reached a typical size of 0.2 x 0.2 x 0.2-0.6 (mm³) in a couple of days. Selected crystals were transferred into a modified reservoir solution now containing 20% glycerol for a few seconds and flash frozen in a cold nitrogen stream and tested on an in-house Mar345 detector (MarResearch Hamburg, Germany) prior to data collection at a synchrotron.

### Crystallization of H. pylori MurI with glutamate and pyrimidinedione inhibitors.

One embodiment of the present invention relates to a crystal of *H. pylori* glutamate racemase complexed with a pyrimidinedione having the orthorhombic space group P2₁2₁2₁, and having cell dimensions a = 61.4 Å, b = 76.3 Å, c = 108.9 Å, and α = 90°, β = 90°, and γ = 90°. A further embodiment encompasses this crystal made by the process of preparing a first solution of About 5 mM D-L Glutamate; about 1 mM TCEP; about 200 mM ammonium acetate; about 0.1 M Tris pH 7.4-8.5; about 500 micromolar of compound A; and about 10 mg/ml glutamate racemase from *H. pylori;* preparing a second solution with about 0.1 M Tris pH 7.4-8.5; about 20-25% PEG 3350; about 15-25% glycerol; and about 0.2 mM ammonium acetate; combining the first solution and the second solution, thereby producing a combination; and forming drops from the combination in a method of crystallization under conditions in which crystals of glutamate racemase are produced.

One embodiment of the present invention is crystallized *H. pylori* glutamate racemase complexed with a pyrimidinedione having the orthorhombic space group P2₁2₁2, and having cell dimensions a = 60.7 Å, b = 77.5 Å, c = 56.6 Å, and α = 90°, β = 90°, and γ = 90°. A further embodiment encompasses this crystal made by the process of preparing a first solution of about 5 mM D-L Glutamate; about 1 mM TCEP; about 200 mM ammonium acetate; about 0.1 M Tris pH 7.4-8.5; about 500 micromolar of compound A; and about 10 mg/ml glutamate racemase from *H*. *pylori;* preparing a second solution with about 0.1 M Tris pH 7.4-8.5; about 20-25% PEG 3350; about 15-25% glycerol; and about 0.2 mM ammonium acetate; combining the first solution and the second solution, thereby producing a combination; and forming drops from the combination in a method of crystallization under conditions in which crystals of glutamate racemase are produced.

Another embodiment of the present invention is crystallized *H. pylori* glutamate racemase complexed with a pyrimidinedione having the monoclinic space group P2₁, and having cell dimensions a = 57.1 Å, b = 78.0 Å, c = 58.55 Å, and α = 90°, β = 97.91°, and γ = 90°. A further embodiment encompasses this crystal made by the process of preparing a first solution of about 5 mM D-L Glutamate; about 1 mM TCEP; about 200 mM ammonium acetate; about 0.1 M Tris pH 7.4-8.5; about 500 micromolar of compound A; and about 10 mg/ml glutamate racemase from *H*. *pylori;* preparing a second solution with about 0.1 M Tris pH 7.4-8.5; about 20-25% PEG 3350; about 15-25% glycerol; and about 0.2 mM ammonium acetate; combining the first solution and the second solution, thereby producing a combination; and forming drops from the combination in a method of crystallization under conditions in which crystals of glutamate racemase are produced.

### D. Data Collection

Crystals diffracted to about 2.8 Å resolution using in-house X-ray source (MarResearch 345 mm image-plate detector system with X-ray generated on Rigaku RU300HB rotating anode operated at 50 kV and 100m Å). A complete data set was collected at ID2, ESRF to 2.3 Å resolution. MAD (MAD = Multiwavelength Anomalous Diffraction phasing methods) (Se-Met) data were collected at BM14, ESRF. Three data sets were collected at three different wavelengths (0.91833 Å, 0.97804 Å, and 0.97821 Å). The data were processed using Denzo (Z. Otwinowski and W. Minor, "Processing of X-ray Diffraction Data Collected in Oscillation Mode", Methods in Enzymology, Volume 276: Macromolecular Crystallography, part A, p.307-326, 1997,C.W. Carter, Jr. & R. M. Sweet, Eds., Academic Press, New York). Statistics of the MAD data collection are shown in Table 6.

A high resolution data set with a diffraction of 1.9 Å resolution was collected from a crystal grown in the presence of 0.1 M Tris pH 8.5, 0.2 M MgSO₄, and 25% PEG 4000 that belonged to space group P2₁ (a = 52.28 Å, b = 78.96 Å, c = 59.14 Å, α = 90°, β = 92.64°, and γ = 90°) at beam line 711 at MaxLab. The data was processed, scaled and merged using MOSFLM, SCALA, and TRUNCATE (The CCP4 Suite: "Programs for Protein Crystallography", Acta Cryst. D50: 760-763). The data set comprised 144510 measurements of 34987 unique reflections giving a multiplicity of 4.1, a completeness of 92.3% and an overall R-merge of 8.1%. Additional cycles of refinement with the program CNS and model building with the program ONO gave a final model consisting of two polypeptide chains of 255 amino acid residues (residues 1-255), two D-glutamic acids and 377 ordered water molecules. The final R-values were R = 0.2061 and R-free = 0.2458.

One crystal belongs to the orthorhombic space group P2₁2₁2₁ and has cell dimensions of a = 62.18 Å, b = 81.07 Å and c =113.82 Å, wherein α = 90°, β = 90°, and γ = 90°. One crystal belongs to the to the monoclinic space group P2₁ and has cell dimensions of a = 59.20 Å, b = 82.40 Å and c = 106.50 Å, wherein α = 90°, β = 92.15°, and γ = 90°. One crystal belongs to the monoclinic space group P2₁ and has cell dimensions of a = 59.67 Å, b = 78.82 Å and c = 59.34 Å, wherein α = 90°, β = 92.35°, and γ = 90°. One crystal belongs to the monoclinic space group P2i and has cell dimensions of a = 52.02 Å, b = 80.66 Å and c = 59.18 Å, wherein α = 90°, β = 92.65°, and γ = 90°. One crystal belongs to the monoclinic space group P2₁ and has cell dimensions of a = 52.48 Å, b = 80.71 Å, and c = 59.42 Å, and wherein α = 90°, β = 91.68°, γ = 90°. Each of these space groups are encompassed by the structural coordinates of Figure 6.

**Table 6: MAD Data Collected at ESRF.BM14; Resolution 2.8 Å (CCD detector/DENZO)**

| Dataset | Wave-length (Å) | Nₘₑₐₛ | N_{ref} | %poss | Multiplicity | R_{fac} | Rₐₙₒₘ |
|---|---|---|---|---|---|---|---|
| PK | 0.97805 | 47166 | 13965 | 98.6 | 3.4 | 0.048 | 0.052 |
| PI | 0.97821 | 49507 | 14015 | 98.9 | 3.5 | 0.045 | 0.036 |
| RM | 0.91834 | 47118 | 13976 | 98.6 | 3.4 | 0.047 | 0.032 |

| | | | | | | | |
|---|---|---|---|---|---|---|---|
| R_{fac}=Sum\|<I>-Ij\|/Sum \|Ij\| Rₐₙₒₘ= Sum\|<I+>-<I->\|/ Sum\|<I+>-<I->\| | | | | | | | |

### E. Phase Determination by MAD

The selenium sites were found by the program RANTAN (The CCP4 Suite: Programs for Protein Crystallography". *Acta. Cryst.* D50, 760-763, Yao Jia-xing, (1981). *Acta. Cryst.* A37, 642-644) and verified by difference Fourier.

Heavy atom refinement and phasing were carried out with MLPHARE (The CCP4 Suite: "Programs for Protein Crystallography". Acta. Cryst. D50, 760-763 Z.Otwinowski: Daresbury Study Weekend proceedings, 1991). Clear protein and solvent boundary was observed after solvent flattening. Phasing statistics using MLPHARE are depicted in Table 7.

Further heavy atom refinement was carried out using the program SHARP (de la Fortelle, E. and Bricogne, G. (1997)). Maximum likelihood heavy-atom parameter refinement for multiple isomorphous replacement and multiwavelength anomalous diffraction methods. In Carter, C.W. and Sweet, R.M. (ed.) Methods in Enzymology vol. 276, Academic Press, Orlando, Fl: pp. 472-494, 1997), which resulted in a slightly improved electron density map.

It was already known that the protein exists as a homo-dimer but inspection of the electron density map identified additional molecular symmetry. Each monomer has a pseudo two-fold symmetry that divides the monomer into two domains with very similar alpha/beta type folds. The binding site is found between the two domains. Thus, the active form of the protein is a four-domain structure, with two seemingly independent binding sites.

Phase improvement using two-fold density averaging (DM, The CCP4 Suite: "Programs for Protein Crystallography". Acta. Cryst. D50, 760-763, 1995) yielded an electron density map with much better quality. The polypeptide chain was easily traced using the improved map and guided by the 8 Seleno-methionine sites. More than 80% of the amino acid sequence was traced with relatively high confidence. The co-location of the two proposed active-site cysteine residues (Cys70 and Cys181, Cα-Cα distance 7.5 Å) validated the chain tracing. Further analysis of this tentative active site, confirmed that indeed a high number of conserved residues mapped into the same site. In addition, a piece of significant electron density was found that did not belong to any of the protein side chains. This density was interpreted and confirmed as arising from the bound substrate, D-glutamate.

**Table 7: Phasing statistics using MLPHARE (3.0 Å)**

| Dataset | Wave-length (Å) | R_Cul acen | RhPow acen | R_Cul cent | RhPow cent | R_Culan om |
|---|---|---|---|---|---|---|
| PI (native) | 0.97821 | - | - | - | - | 0.86 |
| RM | 0.91834 | 0.84 | 1.12 | 0.70 | 0.85 | 0.84 |
| PK | 0.97805 | 0.97 | 0.40 | 0.96 | 0.27 | 0.75 |

| | | | | | | |
|---|---|---|---|---|---|---|
| FOM 0.4470 (acent: 0.4479, cent: 0.4417) Phase improved by two-fold density averaging (DM) | | | | | | |

### F. Refinement of the Crystal Structure

A first round of refinement using REFMAC (The CCP4 Suite: Programs for Protein Crystallography". Acta Cryst. D50: 760-763, "Refinement of Macromolecular Structures by the Maximum-Likelihood Method". G.N. Murshudov, A.A.Vagin and E.J.Dodson, (1997) in Acta Cryst. D53: 240-255) after completing model building for protein atoms yielded an R-value of 0.26 and an R-free value of 0.37. The R-value describes the discrepancy between the observed data and synthetic data calculated from the model. The R-free is the same calculated from a test set of reflections, usually 4% of total, that one sets aside at the beginning of the refinement and serves as an unbiased reference to avoid over-fitting of the data. The R-value is resolution dependent but should be below 25 and the Rfree normally not more than 5% higher.

At this point of the refinement a high resolution native data set was introduced. The data was not completely isomorphous so molecular replacement (MR) was performed using the model refined against the Multiwavelength Anomalous Diffraction (MAD) data. MR was performed using AMORE (The CCP4 Suite: Programs for Protein Crystallography", Acta. Cryst. D50: 760-763, J. Navaza, Acta Cryst. A50: 157-163 (1994) on the earlier native data (2.3 Å resolution), and resulted in a clear solution. Rigid body and NCS restrained refinement using REFMAC was carried out. D-glutamate was built in the active site and crystallographic R/Rfree of 0.27/0.34 was obtained.

In the later stage of refinement, simulated annealing with torsion angle dynamics was employed using program the CNS (Crystallography & NMR System, Acta. Cryst. D54: 905-921 (1998)), which yielded a lower R-free value and an improved electron density map.

The refinement statistics for CNS are as follows:
Refinement resolution: 50-2.3 Å;
Final R= 0.2283 R-free = 0.2808;
Total number of reflections used: 22893 (87.2%);
Number of reflections in working set: 21719 (82.7%); and
Number of reflections in test set: 1174 (4.5%).

### G. Nuclear magnetic resonance (NMR) of H. pylori MurI

Nuclear magnetic resonance (NMR) provides a method by which the structure and conformation of the amino acid residues of the protein can be visualized in solution. NMR experiments were performed at 303K on a Bruker Avance 800 MHz system equipped with a triple-resonance (¹H/¹³C/¹⁵N) single-gradient 5-mm probe. Samples were at pH 7.5 in a 100 mM phosphate buffer containing 100 mM K₂SO₄, 5 mM DTT and 1 mM EDTA. Protein concentration was 0.3 mM. [¹⁵N, ¹H] correlation experiments were recorded to follow proton stability with increasing D-glutamate concentration. Evolution times were around 90 minutes in the protein dimensions, and 30 minutes in the nitrogen dimension, with a total acquisition time of 2.5 hours. Data sets were processed and analyzed with the program nmrPipe (Delaglio, F., Grzesiek, S., Vuister, G., Zhu, G., Pfeifer, H., and Bax, A. "NMRPipe: a multidimensional spectral processing system based on UNIX Pipes", J. Biomol. NMR 6: 277-293 (1995)).

### Production of isotopically labeled Murl protein for NMR studies.

### Plate pre-growth and adaptation

Fresh *H. pylori* J99 MurI transformants were grown to the size of small pinheads for 30-36 hours on plates prepared as follows:

0.2 grams of agar in 19 mLs D₂O is melted by mild heating (e.g., microwave). The melted agar is cooled to around 60°C and 2 mL of BioExpress^{®}-1000 media (U-D, 98%, U-15N, 96-99% for ¹⁵N/²D labeling orU-13C, 97-98%, U-15N, 96-99%, U-D, 98%, for ¹⁵N/²D/¹³C labeling; Cambridge lostope Labs, Andover, MA, USA) and the respective antibodies were labeled.

### Cell growth and inoculation

The colonies on the plate were suspended using 2 mL of culture media (prepared as described below) and the OD₆₀₀ of the solution was determined. 1L of culture medium (prepared as described below) and was inoculated with a total of 0.1 OD₆₀₀. At OD₆₀₀ = 0.5-0.8 (about 14-20 hours of growth time) *H. pylori* MurI protein production was induced using 0.5 mM IPTG. At the time of induction, 4 mL/L of BioExpress^{®}-1000 media (U-D, 98%, U-15N, 96-99% for ¹⁵N/²D labeling or U-13C, 97-98%, U-15N, 96-99%, U-D, 98%, for ¹⁵N/²D/¹³C labeling) were added.

### Culture medium preparation: M9 Minimal Media spiked with BioExpress^{®}-1000 media (1L total volume)

Na₂HPO₄ (7.26g), KH₂PO₄ (3.0g), NaCl (0.5g), NH₄Cl (1.0g; ¹⁵N labeled), 10 ml MgSO₄ (100mM in D₂O), 10 ml CaCl₂ (10 mM in D₂O), 10 mL glucose (20% in D₂O), 10 ml thiamine (0.1% in D₂O), 760 mL D₂O, 16 mL BioExpress^{®}-1000 media (U-D, 98%, U-15N, 96-99% for ¹⁵N/²D labeling or U-13C, 97-98%, U-15N, 96-99%, U-D, 98%, for ¹⁵N/²D/¹³C labeling).

The protein was purified and characterized as described above.

### Example 2: Crystallization and Characterization of MurI from E. coli

Cloning and characterization of MurI from *E. coli* has been described in detail in U.S. Provisional Application 60/435,167.

Cloning, overexpression, purification, and biochemical characterization of *E*. *coli* MurI has been reported H.T. Ho et al. (Biochemistry 34: 2464-2470 (1995)).

### A. Crystallization conditions and space groups

One method of crystallizing *E. coli* MurI complexed with glutamate includes the steps of: (a) preparing a first solution comprising a reducing agent, a substrate, an activator, a salt pH 6.5-9.5, and MurI, wherein the reducing agent, substrate, salt pH 6.5-9.5, is each present in sufficient concentration to inhibit oxidation of the solution, bind to the MurI, stabilize the protein and prevent aggregation while controlling the pH of the solution, respectively; (b) preparing a second solution comprising salt pH 4.5-9.5, wherein the salt pH 4.5-9.5 is present in sufficient concentration to stabilize the protein and prevent aggregation while controlling the pH of the solution; (c) combining the first solution and the second solution, thereby producing a combination; and (d) forming drops from the combination in a method of crystallization under conditions in which crystals of MurI are produced, whereby, crystals of MurI are produced.

The first solution can additionally include glycerol in sufficient concentration to facilitate freezing of the crystals and stabilize the protein, and the second solution additionally comprises a precipitant, each in sufficient concentration to force the protein out of solution, and inhibit oxidation of the solution, respectively.

Batch, vapor diffusion, or dialysis methods of crystallization can be employed.

The need for a reducing agent can be relieved by performing the crystallization under anaerobic conditions, such as under oil or in an anaerobic box.

Salts can be, for example, magnesium chloride, magnesium sulfate, magnesium foramate, lithium sulfate, lithium chloride, ammonium acetate, ammonium sulfate, lithium acetate, ammonium citrate, or lithium citrate.

Reducing agents in the first or second solutions can be, for example, Tri(2-carboxyethyl)phosphine hydrochloride (TCEP), beta-mercaptoethanol, or dithiothreitol (DTT), to prevent or retard oxidation of the protein.

In a further embodiment, the precipitant in the second solution is from about 0% to about 55% polyethylene glycol (PEG), or a derivative thereof (e.g., mono-methyl-ether poly-ethylene glycol (MME PEG)). In a further embodiment, the precipitant in the second solution comprises from about 5% to about 40% PEG. In an additional embodiment, the precipitant in the second solution comprises from about 15% to about 30% PEG. In another embodiment, the precipitant in the second solution comprises from about 20% to about 25% PEG.

A variety of precipitants, such as PEG (e.g., PEG 500 to 20,000, or any intermediate PEG), or a derivative thereof, can be used. In a further embodiment, the precipitant in the second solution is PEG 1,000-10,000. Alternatively, the precipitant in the second solution is PEG 2,000-6,000.

One crystal of *E*. *coli* MurI was made by the process of preparing a first solution of from about one to about 100 mM D,L-glutamic acid; from about 0.1 mM to about 5 mM reducing agent; from about 0 to about 30% glycerol; from about 0 to about 500 mM buffer pH 6.5-9.5; from about 50 to about 500 mM salt; about 0.6 mM UDP-MurNAc-Ala; and from about 1 mg/ml to about 50 mg/ml *E*. *coli* MurI; preparing a second solution of from about 50 to about 500 mM salt pH 4.5-7.5; from about 0 to about 55% precipitant; and from about 0 to about 30% glycerol; and combining the first solution and the second solution, thereby producing a combination; and forming drops from the combination in a method of crystallization under conditions in which crystals of MurI were produced.

Selected crystals that were flash frozen in cold nitrogen stream and tested on in-house detector.

One crystal of E. coli was made by the process of preparing a first solution of from about one to about 100 mM D,L-glutamic acid; from about 0.1 mM to about 5 mM reducing agent; from about 0 to about 30% glycerol; from about 50 to about 500 mM salt pH 6.5-9.5; about 0.6 mM UDP-MurNAc-Ala; and from about 1 mg/ml to about 50 mg/ml *E*. *coli* MurI; preparing a second solution of from about 50 to about 500 mM salt pH 4.5-7.5; and from about 0 to about 55% precipitant; combining the first solution and the second solution, thereby producing a combination; and forming drops from the combination in a method of crystallization under conditions in which crystals of MurI are produced, whereby, crystals of MurI were produced.

One embodiment of the present invention comprises making an *E*. *coli* MurI crystal by the process described herein, wherein the crystal is characterized by the structural coordinates of Figure 11. One embodiment of the present invention comprises a method of preparing an *E*. *coli* MurI crystal complexed with glutamate by the process described herein, wherein the crystal is characterized by the structural coordinates of Figure 8.

One crystallized complex of the present invention is characterized as belonging to the orthorombic space group C222₁ and having cell dimensions of a = 83.05 Å, b = 112.82 Å and c = 74.12 Å, wherein α = 90°,β = 90°, and γ = 90°, wherein the crystallized complex was produced by the process of preparing a first solution of about 5 mM D,L-glutamic acid; about 1 mM TCEP; about 200 mM ammonium acetate pH 7.4; about 10 mg/ml *E*. *coli* MurI; and about 0.6 mM of the activator, UDP-MurNAc-Ala; preparing a second solution of about 100 mM sodium acetate pH 4.5; about 5-10% MME 2000; about 30% glycerol; combining the first solution and the second solution, thereby producing a combination; and forming drops from the combination in a method of crystallization under conditions in which crystals of MurI were produced.

One crystallized complex of the present invention is characterized as belonging to the monoclinic space group P2₁ and having cell dimensions of a = 70.04 Å, b = 74.13 Å and c = 70.10 Å, wherein α = 90°, β = 107.15°, and γ = 90°, wherein the crystallized complex was produced by the process of preparing a first solution of about 5 mM D,L-glutamic acid; about 1 mM TCEP; about 200 mM ammonium acetate pH 7.4; about 10 mg/ml *E*. *coli* MurI; and about 0.6 mM of the activator UDP-MurNAc-Ala; preparing a second solution of about 100 mM sodium acetate pH 4.5; about 5-10% MME 2000; about 30% glycerol; combining the first solution and the second solution, thereby producing a combination; and forming drops from the combination in a method of crystallization under conditions in which crystals of MurI were produced.

Crystals were obtained by vapor diffusion using the hanging drop method. ("Protein Crystallization" Terese M. Bergfors (Editor), International University Line, pages 7-15, 1999). Protein had been stored in 50 microliters aliquots at -80 degrees at a concentration of 10 mg/ml in a buffer containing 200 mM ammonium acetate pH 7.4, 5 mM D,L-glutamic acid and 1 mM TCEP. Prior to crystallization, the activator, UDP-MurNAc-Ala was added to the solution at a final concentration of 0.6 mM. The reservoir solution typically contained 100 mM sodium acetate pH 4.5, 25% polyethylene glycol mono-methyl ether (MME) 2000, and 30% glycerol. Drops were set up by mixing 2 microliters of protein solution with 2 microliters of reservoir solution. Plate-shaped crystals appeared over night and reached a typical size of 0.4 x 0.3 x 0.2 (mm³) in a week. Se-Met substituted MurI was produced and crystallized under similar conditions as for the wild-type protein. The substitution of the Se-Met was verified by Mass spectroscopy. Selected crystals were flash frozen in a cold nitrogen stream and tested on an in-house detector. The crystal belongs to the orthorombic space group C222₁ with cell dimensions of a = 83.05 Å, b =112.82 Å and c = 74.12 Å, wherein α = 90°, β = 90°, and γ = 90°. Crystals can be flash-cooled to 95K directly from the drop without ice formation.

### B. Data Collection

Crystals diffracted to about 2.3 Å resolution using in-house X-ray source (MarResearch 345 mm image-plate detector system with X-ray generated on Rigaku RU300HB rotating anode operated at 50 kV and 100m Å). A complete data set was collected at ID 14.4 ESRF to 1.9 Å resolution. MAD (MAD = Multiwavelength Anomalous Diffraction phasing methods) (Se-Met) data were collected at BM14, ESRF. Three data sets were collected at three different wavelengths (0.9786 Å, 0.9789 Å, and 0.9184 Å).

The data were processed, scaled, and merged using MOSFLM, SCALA and TRUNCATE (The CCP4 Suite: "Programs for Protein Crystallography", *Acta Cryst.* D50: 760-763) and then scaled together using SCALEIT (The CCP4 Suite: "Programs for Protein Crystallography", *Acta Cryst.* D50: 760-763). Statistics of the MAD data collected are shown in Table 8.

**Table 8: MAD Data Collected at ESRF.BM14; Resolution 2.2 Å (CCD detector/DENZO)**

| Dataset | Wave-length (Å) | Nₘₑₐₛ | N_{ref} | %poss | Multiplicity | R_{fac} | Rₐₙₒₘ |
|---|---|---|---|---|---|---|---|
| PK | 0.978 | 119109 | 17758 | 99.5 | 6.7 | 5.1 | 4.8 |
| PI | 0.9778 | 120976 | 17788 | 99.5 | 6.8 | 5.2 | 3.0 |
| RM | 0.9184 | 101650 | 18008 | 100.0 | 7.3 | 7.3 | 4.0 |

| | | | | | | | |
|---|---|---|---|---|---|---|---|
| R_{fac}=Suml<I>-Ij\|/Sum \|Ij\| Rₐₙₒₘ = Sum\|<I+>-<I->\|/ Sum\|<I+>-<I->\| | | | | | | | |

### C. Phase Determination by MAD

The crystal has one monomer per asymmetric unit and all 5 of the seleno-methionine sites could be easily found by the program SOLVE (Terwilliger, T.C. and J. Berendzen, "Automated MAD and MIR structure solution", Acta Cryst. D55: 849-861 (1999)). The following statistics were obtained from the same program as shown in Table 9.

**Table 9: Figure of Merit (FOM) with and estimates of lack-of closure (LOC) residuals as a function of resolution from the solution found with SOLVE.**

| | | | | | | | | | |
|---|---|---|---|---|---|---|---|---|---|
| DMIN: | Total | 7.85 | 4.98 | 3.90 | 3.31 | 2.92 | 2.65 | 2.44 | 2.27 |
| N: | 17999 | 911 | 1500 | 1919 | 2216 | 2506 | 2755 | 3000 | 3192 |
| MEAN FOM: | 0.62 | 0.88 | 0.90 | 0.87 | 0.82 | 0.69 | 0.58 | 0.46 | 0.28 |
| CENTRIC LOC: | | 150.6 | 34.5 | 23.8 | 20.7 | 18.4 | 18.9 | 21.9 | 39.4 |
| CORR ERR: | | 1.4 | 1.0 | 0.6 | 0.4 | 0.8 | 0.7 | 0.4 | 0.9 |

The resulting electron density map could only marginally be improved by density modification as implemented in the program DM (The CCP4 Suite: "Programs for Protein Crystallography". *Acta. Cryst.* D50, 760-763 (1991)).

The polypeptide chain was easily traced using the improved map and guided by the 5 Seleno-methionine sites. More than 90% of the amino acid sequence was traced with relatively high confidence. The co-location of the two proposed active-site cysteine residues (Cys82 and Cys204, Ca-Ca distance 7.6 Å) validated the chain tracing. Further analysis of this tentative active site, confirmed that indeed a high number of conserved residues mapped into the same site. In addition, the electron density corresponding to the bound substrate, L-glutamate, was found between the two conserved cysteines.

### D. Refinement of the Crystal Structure

A first round of refinement was performed using the program CNS (Crystallography & NMR System, Acta. Cryst. D54: 905-921 (1998)). After completing model building for protein atoms, an R-value of 0.24 and an R-free value of 0.27 was achieved. At this point of the refinement, a high resolution native data set to 1.9 Å was introduced. Additional refinement gave a final model building consisting of a polypeptide chain of 265 amino acids (residues 20-285), one L-glutamate, one UDP-MurNAc-Ala, and 221 ordered water molecules. The final value was R = 0.2185 and R-free = 0.2440.

### Example 3: Cloning and Characterization of MurI from E. faecalis and S. aureus, and E. faecium

The genomes of *E. faecalis* and *S*. *aureus,* and *E. faecium* contain open reading frames (ORF) with homology to the *Staphylococcus haemolyticus* MurI gene (dga) (NCBI Accession number U12405) and to the *E*. *coli* MurI gene which encodes MurI activity in that organism.

To evaluate whether these ORFs encode a protein with MurI activity, the gene was isolated by polymerase chain reaction (PCR) amplification cloning, overexpressed in *E*. *coli,* and the proteins purified to apparent homogeneity. A simple assay for MurI activity resulting in the isomerization of D-glutamic acid to L-glutamic acid was developed to facilitate purification and for future use as a high-throughput drug screen.

### Cloning of E. faecalis and S. aureus murI gene encoding MurI:

An 822 base pair DNA sequence encoding the *murI* gene of *E. faecalis* was isolated by polymerase chain reaction (PCR) amplification cloning. A synthetic oligonucleotide primer (5'-AAATAGTCATATGAAAATAGGCGTTTTTG -3' (SEQ ID NO: 67)) encoding an *NdeI* restriction site and the 5' terminus of the *murI* gene and a primer (5'-AGAATTCTATTACAATTTGAGCCATTCT -3' (SEQ ID NO: 68)) encoding an *EcoRI* restriction site and the 3' end of the *murl* gene were used to amplify the MurI gene of *E*. *faecalis* using genomic DNA prepared from the ATCC 29212 type strain of *E*. *faecalis* as the template DNA for the PCR amplification reactions. (Current Protocols in Molecular Biology, John Wiley and Sons, Inc., F. Ausubel et al., editors, 1994).

An 801 base pair DNA sequence encoding the *murI* gene of S. *aureus* was isolated by polymerase chain reaction (PCR) amplification cloning. A synthetic oligonucleotide primer (5'-AAATAGTCATATGAAAATAGGCGTTTTTG -3' (SEQ ID NO: 67)) encoding an *NdeI* restriction site and the 5' terminus of the *murI* gene and a primer (5'-AGAATTCTATTACAATTTGAGCCATTCT -3' (SEQ ID NO: 68)) encoding an *EcoRI* restriction site and the 3' end of the *murI* gene were used to amplify the MurI gene of *S. aureus* using genomic DNA prepared from the ATCC 25923 type strain of *S*. *aureus* as the template DNA for the PCR amplification reactions. (Current Protocols in Molecular Biology, John Wiley and Sons, Inc., F. Ausubel et al., editors, 1994).

A 913 base pair DNA sequence encoding the *murI* gene of *E. faecium* was isolated by polymerase chain reaction (PCR) amplification cloning. A synthetic oligonucleotide primer (5'-AAATAGTCATATGAAAATAGGCGTTTTTG -3' (SEQ ID NO: 67)) encoding an *Ndel* restriction site and the 5' terminus of the *murI* gene and a primer (5'-AGAATTCTATTACAATTTGAGCCATTCT -3' (SEQ ID NO: 68)) encoding an *EcoRI* restriction site and the 3' end of the *murI* gene were used to amplify the MurI gene of *E. faecium* using genomic DNA prepared from the ATCC 19434 type strain of *E*. *faecium* as the template DNA for the PCR amplification reactions. (Current Protocols in Molecular Biology, John Wiley and Sons, Inc., F. Ausubel et al., editors, 1994).

To amplify a DNA sequence containing the *murI* gene, genomic DNA (25 nanograms) was introduced into each of two reaction vials containing 1.0 micromole of each synthetic oligonucleotide primer, 2.0 mM MgCl₂, 0.2 mM of each deoxynucleotide triphosphate (dATP, dGTP, dCTP and dTTP), and 1.25 units of heat stable DNA polymerases (Amplitaq, Roche Molecular Systems. Inc., Branchburg, NJ, USA) in a final volume of 50 microliters. The following thermal cycling conditions were used to obtain amplified DNA products for the MurI gene using a Perkin Elmer Cetus/ GeneAmp PCR System 2400 thermal cycler:
Conditions for amplification of *E. faecalis and S. aureus murI:*
   Denaturation at 94°C for 2 minutes;
   30 cycles at 94°C for 10 seconds, 55°C for 30 seconds, and 72°C for 90 seconds;
   Reactions were concluded at 72°C for 7 minutes.
Conditions for amplification of *E. faecium murI*:
   Denaturation at 94°C for 5 minutes;
   25 cycles at 94°C for 30 seconds, 55°C for 30 seconds, and 72°C for 60 seconds;

Reactions were concluded at 72°C for 7 minutes.

Upon completion of thermal cycling reactions, the amplified DNA was washed and purified using the Qiaquick Spin PCR purification kit (Qiagen, Gaithersburg, MD USA). The amplified DNA sample was subjected to digestion with the restriction-endonucleases, *Ndel* and *EcoRI* (New England Biolabs, Beverly, MA USA) (Current Protocols in Molecular Biology, *Ibid).* The DNA samples from each of two reaction mixtures were pooled and subjected to electrophoresis on a 1.0% SeaPlaque (FMC BioProducts, Rockland, ME, USA) agarose gel. DNA was visualized by exposure to ethidium bromide and long wave UV irradiation. Amplified DNA encoding the *H. pylori* MurI gene was isolated from agarose gel slices and purified using the Bio 101 GeneClean Kit protocol (Bio 101 Vista, CA USA).

### Cloning of E. faecalis, S. aureus, and E. faecium murI DNA sequences into the pET-23 prokaryotic expression vector:

The pET-23b vector can be propagated in any *E*. *coli* K-12 strain, e.g., HMS174, HB101, JM109, DH5α, etc., for the purpose of cloning or plasmid preparation. Hosts for expression include *E*. *coli* strains containing a chromosomal copy of the gene for 17 RNA polymerase. These hosts are lysogens of bacteriophage DE3, a lambda derivative that carries the l*acl* gene, the lacUV5 promoter and the gene for T7 RNA polymerase. T7 RNA polymerase is induced by addition of isopropyl-B-D-thiogalactosidase (IPTG), and the T7 RNA polymerase transcribes any target plasmid such as pFT-28b, carrying its gene of interest. Strains used in our laboratory include: BL21 (DE3) (Studier, F,W., Rosenberg, A.H., Dunn, J.J., and Dubendorff, LW. Meth. Enzymol. 185: 60-89, 1990). The pET-23b vector (Novagen, Inc., Madison, WI, USA) was prepared for cloning by digestion with *NdeI* and *EcoRl* (Current Protocols in Molecular Biology, ibid). Following digestion, the amplified, agarose get-purified DNA fragment carrying the *murI* gene was cloned (Current Protocols in Molecular Biology, *ibid)* into the previously digested pET-23b expression vector. Products of the ligation reaction were then used to transform the BL21(DE3) strain of *E*. *coli.*

### Transformation of competent bacteria with recombinant plasmids:

Competent bacteria, *E. coli,* strain BL21 or strain BL21(DE3), were transformed with recombinant pET23- *murI* expression plasmid carrying the cloned *E. faecalis* or S. *aureus* sequence according to standard methods (Current Protocols in Molecular, *ibid*). Briefly, 1 microliter of ligation reaction was mixed with 50 microliters of electrocompetent cells and subjected to a high voltage pulse, after which, samples were incubated in 0.45 milliliters SOC medium (0.5% yeast extract, 2.0% tryptone, 10 mM NaCl, 2.5 m KCl, 10mM MgCl₂, 10mM MgSO₄ and 20mM glucose) at 37°C with shaking for 1 hour. Samples were then spread on LB agar plates containing 100 microgram/ml ampicillin for growth overnight. Transformed colonies of BL21 were then picked and analyzed to evaluate cloned inserts as described below.

### Identification of recombinant pET expression plasmids carrying murI sequences:

Individual BL2I clones transformed with recombinant pET-23- *murI* were analyzed by PCR amplification of the cloned inserts using the same forward and reverse primers specific for each sequence that were used in the original PCR amplification cloning reactions. Successful amplification verified the integration of the *murI* sequences in the expression vector (Current Protocols in Molecular Biology, *ibid*)*.*

### Isolation and Preparation of plasmid DNA from BL21 transformants:

Colonies carrying pET-23- *murI* vectors were picked and incubated in 5 mls of LB broth plus 100 microgram/ml ampicillin overnight. The following day plasmid DNA was isolated and purified using the Qiagen plasmid purification protocol (Qiagen Inc., Chatsworth, CA, USA).

### Cloning and expression of the E. coli groE operon:

It has been demonstrated that co-expression of the *E*. *coli murI* gene with the genes in the *E*. *coli groE* operon reduces the formation of insoluble inclusion bodies containing recombinant MurI (Ashiuchi, M., Yoshimura, 1., Kitamura, T., Kawata, Y., Nagai, J., Gorlatov, S., Esaki, N. and Soda, K. 1995, J. Biochem. 117: 495-498). The *groE* operon encodes two proteins, *GroES* (97 amino acids) and *GroEL* (548 amino acids), which are molecular chaperones. Molecular chaperones cooperate to assist the folding of new polypeptide chains (F. Ulrich Hartl, 1996, Nature London 381:571-580).

The 2210 bp DNA sequence encoding the *groE* operon of *E. coli* (NCBI Accession number X07850) was isolated by polymerase chain reaction (PCR) amplification cloning. A synthetic oligonucleotide primer (5'-GCGAATTCGATCAGAATTTTTTTTCT (SEQ ID NO: 69)) encoding an *EcoRI* restriction site and the 5' terminus of the *groE* operon containing the endogenous promoter region of the groE operon and a primer (5'- , ATAAGTACTTGTGAATCTTATACTAG -3' (SEQ ID NO: 70)) encoding a *Scal* restriction site and the 3' end of the *groEL* gene contained in the *groE* operon were used to amplify the *groE* operon, of *E*. *coli* using genomic DNA prepared from *E*. *coli* strain MG1655 as the template DNA for the PCR amplification reactions (Current Protocols in Molecular Biology, *Ibid).* to amplify a DNA sequence containing the *E. coli groE* operon genomic DNA (12.5 nanograms) was introduced into each of two reaction vials containing 0.5 micromoles of each synthetic oligonucleotide primer, 1.5 mM MgCl₂, 0.2 mM each deoxynucleotide triphosphate (dATP, dGTP, dCTP and dTTP) and 2.6 units heat stable DNA polymerases (Expanded High Fidelity PCR System, Boehringer Mannheim, Indianapolis, Indiana) in a final volume of 50 microliters. The following thermal cycling conditions were used to obtain amplified DNA products for the *groE* operon using a Perkin Elmer Cetus/ GeneAmp PCR System 9600 thermal cycler: culture at a final concentration of 1.0 mM. Cells were cultured overnight to induce gene expression of the *H. pylori* recombinant DNA constructions.

After induction of gene expression with IPTG, bacteria were pelleted by centrifugation in a Sorvall RC-3B centrifuge at 3,000 x g for 20 minutes at 4°C. Pellets were re-suspended in 50 milliliters of cold 10mM Tris-HCl, pH 8.0, 0.1 M NaCl and 0.1 mM EDTA (STE buffer). Cells were then centrifuged at 2000 x g for 20 min at 4°C. Pellets were weighed (average wet weight 6 grams/liter) and processed to purify recombinant protein as described below.

### Purification procedure for recombinant MurI proteins:

The cell pellet form 2L culture was suspended in 50 mls lysis buffer (50 mM Tris pH 8, 150 mM NaCI, 1 mM DL glutamate, 0.1 mM phenylmethanesulfonyl fluoride) and lysed by French Press (10,000-15;000 psi). The lysates was centrifuged at 4°C (10,000 x g, 30 min) and the supernatant was loaded on a 5 mL HiTrap chelating agarose column (Amersham, Piscutaway, MH, USA) charged with NiSO₄. The column was washed with 50 mL column buffer 19100 mM Tris pH 9, 300 mM NaCl, 2 mM DL glutamate). Bound protein was eluted using a step gradient of imadazole concentration in column buffer and pure (>95%) MurI protein eluted at 100-300 mM imadazole. Fractions containing MurI were brought to 1 mM dithio-DL-threitol (DTT), concentrated and dialyzed (4x, 2-4L each) with dialysis buffer (10 mM Tris pH 8, 0.1 mM ethylene glycol-bis-(2-aminoethylether-N,N,N',N'-tetraacetic acid (EGTA), 1 mM DL glutamate, 150 mM NaCl, 1 mM tricarboxyethyl phosphine (TCEP)). The final dialysis buffer contained glycerol (10-50% wt/vol) for storage at -80°C.

### Amplification of internal fragments of the murI gene from 9 Enterococcal species.

In order to obtain nucleotide sequence from the *murI* genes from other Enterococcal species, it was necessary to amplify and de novo sequence DNA as genome sequences have not been elucidated for these species. The approach used was to design synthetic oligonucleotide primers based on the *E. faecalis murI* sequence and use the possibility of nucleotide homology between species of the same genus to amplify *murI* from taxonomically related Enterococcus species.

360 base pair fragments of the *murI* coding sequence from 9 species from the genus Enterococcus were generated by polymerase chain reaction (PCR) amplification cloning. Two synthetic oligonucleotide primers derived from the *E*. *faecalis murI* gene (5'-AAAATGCTAGTAATCGCATGTAATACCGC-3' (SEQ ID NO: 71) and (5'-TGGGTACAACCTAAAATCAACGTATC-3' (SEQ ID NO: 72) were used to amplify the murI gene fragment from *E. saccharolyticus* (ATCC 43076), *E. mundtii* (ATCC 43186), *E. casseliflavus* (ATCC 25788), *E. favescens* (ATCC 49996), *E. cecorum* (ATCC 43198), *E. raffinosus* (ATCC 49427), *E*. *malodoratus* (ATCC 43197), *E. solitarus* (ATCC 49428), and *E. hirae* (ATCC 48043) using genomic DNA as the template for the PCR amplification reactions. (Current Protocols in Molecular Biology, John Wiley and Sons, Inc., R. Ausubel et al., eds., 1994). To amplify a DNA sequence containing the *murI* gene, genomic DNA (50 nanograms) was introduced into a reaction vial containing 1.0 micromole of each synthetic oligonucleotide primer, 2.0 mM MgCl2,25 microliters of High Fidelity Platinum PCR Supermix (Invitrogen, Carlsbad, CA 92008, USA) to a final volume of 50 microliters. The following thermal cycling conditions were used to obtain amplified DNA products for the murI gene using a Perkin Elmer Cetus/GeneAmp PCR System 2400 thermal cycler:
Conditions for amplification of Enterococcal *murI* fragments:
   Denaturation at 94°C for 5 minutes;
   30 cycles at 94°C for 30 seconds, 50°C for 30 seconds, and 72°C for 30 seconds.

Reactions were concluded at 72°C for 30 minutes.

Upon completion of thermal cycling reactions, the amplified DNA was washed and purified using the Qiaquick Spin PCR purification kit (Qiagen, Gaitherburg, MD, 20876, USA). Purified fragments were sequenced using the BigDye Terminator Cycle Sequencing Ready Reaction kit (PE Biosystems, Foster City, CA, 94404, USA) and reactions run on an ABI 3100 sequencer (Applied Biosystems, Inc., Foster City, CA 94404, USA).

### Example 4: Crystallization and Characterization of MurI from E. Faecalis

Cloning and purification of MurI from *E. faecalis* has been previously described in U.S. Provisional Application 60/435,272.

### A. Crystallization of MurI from E. faecalis

Crystals were obtained by vapor diffusion using the hanging drop method. ("Protein Crystallization" Terese M. Bergfors (Editor), International University Line, pages 7-15, 1999).

Protein had been stored in 50 microliters aliquots at -80 degrees at a concentration of 10 mg/ml in a buffer containing 0.2 M ammonium acetate pH 7.4; 5 mM D-,L-glutamic acid and 1 mM Tri(2-carboxyethyl)phosphine hydrochloride (TCEP).

The reservoir solution typically contained 200 mM magnesium chloride, 100 mM Tris pH 7.5, and 20-25% PEG 4000.

Drops were set up by mixing 2 microliters of protein solution with 2 microliters of reservoir solution. Plate-shaped crystals appeared over night and reached a typical size of 0.2 x 0.2 x 0.2 mm, in a week. SE-Met substituted MurI was produced and crystallized under similar conditions as for the wild-type (wt) protein. The substitution of the Se-Met was verified by Mass spectroscopy. Selected crystals were incubated for 5 seconds in a solution containing 100 mM Tris pH 7.5, 200 mM ammonium sulphate, 25% PEG 4000 and 25% glycerol and then flash frozen in a cold nitrogen stream and tested on an in-house detector.

### B. Data Collection

Crystals diffracted to about 2.2 Å resolution using in-house X-ray source (MarResearch 345 mm image-plate detector system with X-ray generated on Rigaku RU300HB rotating anode operated at 50 kV and 100m Å). MAD (MAD = Multiwavelength Anomalous Diffraction phasing methods) (Se-Met) data were collected at BM14 at ESRF on a crystal grown from Se-Met modified material. Three data sets were collected at three different wavelengths (0.9786 Å, 0.8789 Å, and 0.9184 Å). The data were processed, scaled and merged using the programs MOSFLM, SCALA, TRUNCATE and SCALEIT (The CCP4 Suite: Programs for Protein Crystallography". Acta. Cryst. D50, 760-763, Yao Jia-xing, (1981). Acta. Cryst. A37, 642-644). Statistics of the MAD data collection are shown in Table 10.

The crystal belongs to the orthorombic space group P2₁2₁2₁ and has cell dimensions of a = 60.29 Å, b = 82.08 Å and c = 115.57 Å, wherein α = 90°, β = 90°, and γ = 90°. This space group is encompassed by the structural coordinates of Figure 13.

**Table 10: MAD Data Collected at ESRF.BM14; Resolution 2.2 Å (CCD detector/MOSFLM)**

| Dataset | Wave-length (Å) | Nₘₑₐₛ | N_{ref} | %poss | Multiplicity | R_{fac} | Rₐₙₒₘ |
|---|---|---|---|---|---|---|---|
| PK | 0.9786 | 64292 | 14241 | 93.7 | 4.3 | 9.0 | 7.2 |
| PI | 0.8789 | 69592 | 14463 | 94.1 | 4.5 | 9.4 | 5.3 |
| RM | 0.9184 | 81414 | 15837 | 95.6 | 5.0 | 10.5 | 6.2 |

| | | | | | | | |
|---|---|---|---|---|---|---|---|
| R_{fac}=Sum\|<I>-Ij\|/Sum \|Ij\| Rₐₙₒₘ= Sum\|<I+>-<I->\|/Sum\|<I+>-<I->\| | | | | | | | |

### C. Phase Determination by MAD

The selenium sites were found by the program SOLVE (Terwilliger, T.C. and J. Berendzen. "Automated MAD and MIR structure solution:. Acta Cryst. D55: 849-861 (1999)). The program found a solution based on nine sites that gave the phasing statistics described in Table 11. The resulting map was readily interpreted and the polypeptide could easily be traced.

**Table 11: Phasing statistics using MLPHARE**

| | | | | | | | | | |
|---|---|---|---|---|---|---|---|---|---|
| Figure of Merit < | | 0.1 | 0.2 | 0.3 | 0.4 | 0.5 | 0.6 | 0.7 | 0.8 |
| 0.9 | 1.0 | | | | | | | | |
| # reflections | | 1510 | 1498 | 1426 | 1421 | 1338 | 1373 | 1495 | 1733 |
| 2249 | 2654 | | | | | | | | |
| | | | | | | | | | |
| Figure of Merit with Resolution | | | | | | | | | |
| DMIN: | Total | 8.31 | 5.61 | 4.49 | 3.85 | 3.42 | 3.11 | 2.87 | 2.68 |
| N: | 16697 | 937 | 1416 | 1761 | 2056 | 2300 | 2554 | 2735 | 2938 |
| MEAN FOM: | 0.55 | 0.79 | 0.77 | 0.68 | 0.63 | 0.56 | 0.52 | 0.46 | 0.34 |

More than 90% of the amino acid sequence was traced with relatively high confidence from the first map using the program O (T.A. Jones, J.Y. Zou, S.W. Cowan & M. Kjeldgaard, "Improved methods for building protein models in electron density maps and the location of errors in these models:, Acta Cryst. A47: 110-119 (1991)). The co-location of the two proposed binding site cysteine residues (Cys76 and Cys185, Cα-Cα distance 7.5 Å) as well as the 9 easily identified Seleno-Methionine sites validated the chain tracing.

The two monomers were built independently so that differences in the relative position of the two main domains as well as secondary structural elements could be readily observed.

The monomer is a two domain structure wherein each domain has an alphabeta type fold. The binding site residues reside in the interface between the two domains.

*E. faecalis* has two binding sites, each on opposing sites of the dimer face. That this is a biologically relevant dimer was confirmed by the conservation of the residues located in the dimer interface within the family of Gram positive bacteria. Of interest is that the one binding site was found to bind D-glutamate, whereas the other binding site bound L-glutamate. The binding of L-glutamate seems to induce significant conformational differences in the active site that seemingly propagates throughout the structure. The protein has amino acids that are used to fold the polypeptide chain back onto the structure to almost form an additional strand on one of the beta sheets.

### D. Refinement of the Crystal Structure

The initial model was refined with a simulated annealing protocol using torsion angle dynamics as implemented in the program CNS (Crystallography & NMR System, *Acta. Cryst.* D54: 905-921 (1998)). After the first round of refinement, the model had an R-value of 28.6% and an R-free value of 36.8%. Further model building and refinement yielded a model comprising 4243 atoms corresponding to 2 x 268 amino acids, one D-glutamate, one L-glutamate, and 184 ordered water molecules. The final refinement statistics for CNS are as follows: Final R = 0.2045 and R-free = 0.2567.

### Example 5: Crystallization and Characterization of MurI from S. aureus

Cloning and purification of MurI from S. *aureus* has been previously described in U.S. Provisional Application 60/435,272.

### A. Crystallization of MurI from S. aureus

Crystals were obtained by vapor diffusion using the hanging drop method. ("Protein Crystallization" Terese M. Bergfors (Editor), International University Line, pages 7-15, 1999).

Protein had been stored in 50 microliters aliquots at -80 degrees at a concentration of 10 mg/ml in a buffer containing 5 mM D,L-glutamic acid; about 1 mM TCEP; and about 200 mM ammonium acetate pH 7.4.

The reservoir solution typically contained 150 mM ammonium sulfate and 25% PEG 8000, and 15% glycerol.

Drops were set up by mixing 2 microliters of protein solution with 2 or 4 microliters of reservoir solution. Box-shaped crystals appeared within a couple of days and reached a typical size of 0.3 x 0.2 x 0.2 (mm³), in a week. Selected crystals were flash frozen in a cold nitrogen stream and tested on an in-house detector.

The crystal belongs to the monoclinic space group C2 and has cell dimensions of a = 96.43 Å, b = 88.87 Å, c = 96.56 Å, α = 90°, β =109.00°, and γ= 90°.

### B. Data Collection on S. aureus MurI

Crystals diffracted to about 3.0 Å resolution using in-house X-ray source (MarResearch 345 mm image-plate detector system with X-ray generated on Rigaku RU300HB rotating anode operated at 50 kV and 100m Å). The *S. aureus* structure was solved with molecular replacement using the *E. faecalis* model, so only a single data set was collected at beam line 711 at MaxLab to 2.15 Å resolution. The data sets were processed, scaled and merged using the programs MOSFLM, SCALA, TRUNCATE and SCALEIT (The CCP4 Suite: Programs for Protein Crystallography". Acta. Cryst. D50, 760-763, Yao Jia-xing, (1981). Acta. Cryst. A37, 642-644). The data sets comprised 169158 measurements of 41453 unique reflections giving a multiplicity of 4.0, a completeness of 98.2% and an overall R-merge of 9.9% (34.3% in the highest resolution bin).

### C. Phase Determination of S. aureus MurI by molecular replacement

The structure was solved by molecular replacement using the program MOLREP (The CCP4 Suite: "Programs for Protein Crystallography", Acta Cryst. D50: 760-763, J. Navaza, Acta Cryst. A50: 157-163 (1994)) and a pruned model of a monomer from the previously solved structure of *E. faecalis* as a search model. From the two peaks in the rotation function, the *S. aureus* MurI protein was determined to be an asymmetric dimer. The final solution at 4 Å had an R-value of 0.44 and a correlation of 0.49. The map showed clear density for the side chains that are different between the two species confirming a correct solution.

The model was easily corrected using the software ONO (T.A. Jones et al. Acta Cryst. A47: 110-119 (1991)) and a model corresponding to residues 3 to 268 could be constructed from the first map.

### D. Refinement of the S. aureus Crystal Structure

The initial model was refined with a simulated annealing protocol using torsion angle dynamics as implemented in the program CNS (Crystallography & NMR System, Acta. Cryst. D54: 905-921 (1998)). After one cycle of simulated annealing an R-value of 0.27 and an R-free value of 0.30 was achieved. Additional refinement and model building gave a final model consisting of two polypeptide chains of 262 amino acids (residues 1-262), two D-glutamates, and 133 ordered water molecules. The final values were R = 0.2020 and R-free = 0.2290.

### Example 6: Crystallization and Characterization of MurI from E. faecium

Cloning and purification of MurI from *E. faecium* has been previously described in U.S. Provisional Application 60/435,272.

### A. Crystallization

Crystals were obtained by vapour diffusion using the hanging drop method. (Protein Crystallization, Terese M. Bergfors (Ed.), Published 1999).

Protein was stored at -80 °C at a concentration of 10 mg/ml in a buffer containing 200 mM ammonium acetate pH 7.4, 5 mM D,L-glutamic acid, and 1 mM TCEP.

The reservoir solution typically contained 100 mM sodium di-hydrogen citrate pH 5.6, and (1) 0.6-0.7M ammonium di-hydrogen phosphate, (2) 200 mM trisodium citrate dehydrate and 20% polyethylene glycol 3350, or (3) 200 mM disodium tartrate dehydrate and 20% polyethylene glycol 3350.

Two microliters of the protein solution were mixed with 2 or 4 microliters of the reservoir solution to make drops. Bipyramidal crystals appeared within a day, and reached a typical size of 0.3 x 0.3 x 0.2 mm³ within one week.

One crystal belongs to the centered monoclinic space group P3₁2₁ with cell dimensions of (phosphate) a = b = 85.82 Å and c = 92.25 Å, α = 90 °, β = 90 ° and γ = 120 °; (citrate) a = b = 85.42 Å and c = 92.91 Å, α = 90 °, β = 90 ° and γ = 120 °; and (tartrate) a = b = 85.16 Å and c = 96.56 Å, α = 90 °, β =109 ° and γ = 90 °. Crystals can be flash-cooled to 95K directly from the drop without ice formation.

### B. Data Collection

Crystals were checked for diffraction using an in-house X-ray source (MarResearch 345 mm image-plate detector system with X-ray generated on Rigaku RU300HB rotating anode operated at 50 kilovolts and 100 milliamps). Complete data sets were collected at beam line 711 at MaxLab to 1.8 Å (phosphate and citrate) and 2.0 Å (tartrate) resolution. The data sets were processed, scaled and merged using MOSFLM, SCALA and TRUNCATE (The CCP4 Suite: "Programs for Protein Crystallography", Acta Cryst. D50, 760-763). The data sets include:
Phosphate: 732191 measurements of 36792 unique reflections giving a multiplicity of 19.9, a completeness of 99.4% and an overall R-merge of 8.0%;
Citrate: 151895 measurements of 34849 unique reflections giving a multiplicity of 4.4, a completeness of 95.3% and an overall R=merge of 4.9%; and
Tartrate: 85092 measurements of 25682 unique reflections giving a multiplicity of 3.3, a completeness of 94.4% and an overall R-merge of 6.6%.

### C. Phase determination by Molecular Replacement (MR)

The structure was solved by molecular replacement using the phosphate data set and the program MOLREP (The CCP4 Suite: "Programs for Protein Crystallography", Acta Cryst. D50, 760-763, J. Navaza, Acta Cryst. A50, 157-163 (1994) and a pruned model of a monomer from the previously solved structure of *E*. *faecalis* as a search model. From the single major peak in the rotation function it was obvious that there was only a monomer in the asymmetric unit but with a twofold crystallographic axis creating the same dimer as seen in the solved *E*. *faecalis* and *S. aureus* structures. The final solution at 4 Å had an R-value of 0.448 and a correlation of 0.504. The map showed clear density for the side chains that are different between *E. faecium* and *E. faecalis* confirming a correct solution.

The incomplete model was corrected manually using the software ONO (T.A. Hones, J.Y. Zou, S.W. Cowan & M. Kjeldgaard, "Improved methods for building protein models in electron density maps and the location of errors in these models", Acta Cryst. A47, 110-119 (1991)) and a model corresponding to residues 2-273 was constructed from the first map.

### D. Refinement of the Crystal Structure

A first round of refinement was performed using the program CNS (Crystallography & NMR System. Acta Cryst. (1998) D54: 905-921). After one cycle of simulated annealing, an R-value of 0.27 and an R-free value of 0.30 was achieved. Additional refinement and model building gave a final model consisting of one polypeptide chain of 271 amino acid residues (residues 1-271 of SEQ ID NO: 48), two phosphate ions and 250 ordered water molecules. The final R-values were R = 0.1927 and R-free = 0.2092.

The citrate and tartrate structures were solved by molecular replacement using MOLREP and the phosphate model as a search model. They were refined to 1.8 Å and 2.0 Å resolution for citrate and tartrate, respectively. The citrate structure consists of one polypeptide chain of 271 amino acids (residues 1-271 of SEQ ID NO: 48), one citrate molecule and 282 ordered water molecules. The final R-values were R = 0.1991 and R-free 0.2073. The tartrate structure consists of one polypeptide chain of 271 amino acids (residues 1-271 of SEQ ID NO: 48), one tartrate molecule, and 296 ordered water molecules. The final R-values were R = 0.2036 and R-free = 0.244.

### Example 7: Isolation and Identification of Mutant MurI Proteins

Two-fold serial dilutions of MurI inhibitors (AR-B051082, AR-B051076, and AR-B052184), ranging from 0.25 to 32 x MIC were prepared in agar media in triplicate. After transfer of *H. pylori* cells (>10⁸ cfu) onto the plates, the plates were incubated at 37 °C in a tri-gas incubator (5% oxygen, 85% nitrogen, 10% carbon dioxide) for seven (7) days. Colonies isolated from plates with compound concentrations above MIC levels were selected and transferred to agar media without inhibitor, prior to growth on agar media containing inhibitor to confirm acquisition of a stable resistance phenotype.

Chromosomal DNA was extracted from representative colonies and the *murI* gene was amplified using polymerase chain reaction (PCR) with template primers TGATGCAACAAATGGACGA (SEQ ID NO: 75) and TTACAATTTGAGCCATTC (SEQ ID NO: 76). Mutations in the *murI* gene were identified using DNA sequencing. Samples of the mutant *murI* PCR products were transformed into wild-type *H. pylori* and subsequently grown on agar media containing MurI inhibitors at concentrations above the MIC of the wild-type strain.

Confluent growth on those plates relative to control on media containing inhibitor showing the presence of mutated MurI confirmed that resistance was mediated by expression of mutant MurI proteins.

### Mutant Protein Overexpression and Characterization:

Cloned J99 *murI* was altered to encode either mutation A75T (G223A) or E151K (G451A) using site-directed mutagenesis in the expression vector pET23a. The mutated clones were shown by transformation to confer resistance to AI1244 *acrB⁻.* For overexpression, the above constructs were co-transformed with a plasmid encoding GroEL into BL21(DE3) and grown in LB with D/L glutamate. Induction was done using 0.5 mM IPTG at room temperature overnight. The expression levels of the mutated MurI proteins were the same as wild-type MurI and with about 50-80% solubility. The expressed proteins were purified using the procedure described for the wild-type MurI protein (>20mg, >99% pure). Kinetic analysis of MurI resistance mutant proteins were measures in the forward (L-Glu→ D-Glu) and reverse direction (D-Glu → L-Glu). In the forward direction, no substrate inhibition by L-Glu was observed and the turnover numbers were very similar to the wild-type enzyme (*k*_{cat} ~90 min⁻¹), but the K_{M} for L-Glu was elevated by 10-fold, giving an overall drop in catalytic efficiency (*k*_{cat}/K_{M}) of 10-fold relative to the native enzyme. In the reverse direction (D-Glu → L-Glu) the mutant enzyme exhibits substrate inhibition by D-glutamate, but the inhibition constant is shifted > 120-fold relative to the wild-type enzyme (i.e. K_{I,D-Glu A75T} - 680 µM, K_{I,D-Glu WT} - 5 µM). Further, the K_{M} for D-Glu for the mutant enzyme is elevated relative to the wild-type enzyme

### (i.e. K_{M,D-Glu A75T} - 280 µM, K_{M,D-Glu WT} - 70 µM).

### Example 8: MAPS

MAPS has been described in detail above and was used, for example, to find common structural units among Gram positive MurI from three bacterial species. Abbreviations used in the MAPS example: Sa = *S. aureus,* Ef = *E. faecalis,* and Ef2 = *E. faecium.*
Automatic mode of the program:
Least matching rate for the two molecules 0.33
Least second matching rate 0.90 superimposed structure will be written out
Shortest residues of one fragment 3
Maximum distance between Ca atoms of aligned residues 3.800000

**Total 3 models will be used for 3d comparison**

| | | | |
|---|---|---|---|
| Model 1 | Residues 113 | Name: SA | PDB file: muri_s_aureus_patent 1 |
| Model 2 | Residues 112 | Name: EF | PDB file: muri_e_faecalis_patent 114 |
| Model 3 | Residues 111 | Name: EF2 | PDB file: ef2_z.pdb 226 |
| Maximum | residue number | 113 | |

| | | | |
|---|---|---|---|
| * Secondary structure assignment completed 113 Ca atoms read 6 helixes 4 strands in file muri_s_aureus_patent_z.pdb * Secondary structure assignment completed 112 Ca atoms read 6 helixes 4 strands in file muri_e_faecalis_patent_ z.pdb 1 chains in Mo12 | | | |

| | | | | | |
|---|---|---|---|---|---|
| Chain A Nr | 1 | 10 | | | |
| Matching residues | 111 | Identical residues | 51 | Identity | 45.9% |
| Match Rate(1) | 98.2% | r.m.s. of atoms | 0.92 | Mean distance | 0.71 |
| structure diversity | 0.95 | with | 111 | residues in match 1 | of muri_e_ |
| | | | | | |
| <<<Total 1 way to align the new structure to muri_e_faecalis_patent_z.pdb | | | | | |
| muri_e_faecalis_patent_z.pdb<->muri_s_aureus_patent_z.pdb Max Align: 10 Max Match: 10 | | | | | |
| Best Topological Diversity 3.2 with 10 matched SS to muri_e_ | | | | | |
| Best Structure Diversity 0.95 with 111 matched residues to muri_e_ | | | | | |
| #Ca 111 RMS: 0.9 dist. 0.7 str.div: 1.0 top.div 3.2 | | | | | |

| | | | | | |
|---|---|---|---|---|---|
| * Secondary structure assignment completed 111 Ca atoms read 6 helixes 4 strands in file ef2_z.pdb 1 chains in Mol2 | | | | | |

| | | | | | |
|---|---|---|---|---|---|
| Chain A Nr | 1 | 10 | | | |
| Matching residues | 109 | Identical residues | 45 | Identity | 41.3% |
| Match Rate(1) | 96.5% | r.m.s. of atoms | 0.94 | Mean distance | 0.76 |
| structure diversity | 1.01 | with 109 residues in match 1 of ef2_z | | | |
| | | | | | |
| <<<Total 1 way to align the new structure to ef2_z.pdb | | | | | |
| ef2_z.pdb<->muri_s_aureus_patent_z.pdb Max Align: 10 Max Match: 10 | | | | | |
| Best Topological Diversity 4.5 with 10 matched SS to ef2_z | | | | | |
| Best Structure Diversity 1.01 with 109 matched residues to ef2_z | | | | | |
| #Ca 109 RMS: 0.9 dist. 0.8 str.div: 1.0 top.div 4.5 | | | | | |

| | | | | | |
|---|---|---|---|---|---|
| * Secondary structure assignment completed 112 Ca atoms read 6 helixes 4 strands in file muri_e_faecalis_patent_z.pdb * Secondary structure assignment completed 111 Ca atoms read 6 helixes 4 strands in file ef2_z.pdb 1 chains in Mo12 | | | | | |

| | | | | | |
|---|---|---|---|---|---|
| Chain A Nr | 1 | 10 | | | |
| Matching residues | 111 | Identical residues | 78 | Identity | 70.3% |
| Match Rate(1) | 99.1% | r.m.s. of atoms | 0.49 | Mean distance | 0.41 |
| structure diversity | 0.50 | with 111 residues in match 1 of ef2_z | | | |
| | | | | | |
| <<<Total 1 way to align the new structure to ef2_z.pdb | | | | | |
| ef2_z.pdb<->muri_e_faecalis_atent_z.pdb Max Align: 10 Max Match: 10 | | | | | |
| Best Topological Diversity 2.0 with 10 matched SS to ef2_z | | | | | |
| Best Structure Diversity 0.50 with 111 matched residues to el2_z | | | | | |
| #Ca 111 RMS:0.5 dist. 0.4 str.div: 0.5 top.div 2.0 | | | | | |

### Comparisons between --- S. aureus and E. faecalis

| begin end | Sequence Matching |
|---|---|
| | |

| | | | | | |
|---|---|---|---|---|---|
| Matching residues | 111 | Identical residues | 51 | Identity | 45.9% |
| Match Rate(1) | 99.1% | r.m.s. of Ca | 0.92 | Mean distance | 0.71 |

| | |
|---|---|
| SA | EF2 |

Comparisons between---- SA & EF2

| begin end | Sequence Matching |
|---|---|
| | |

| | | | | | |
|---|---|---|---|---|---|
| Matching residues | 109 | Identical residues | 45 | Identity | 41.3% |
| Match Rate(1) | 98.2% | r.m.s. of Ca | 0.94 | Mean distance | 0.76 |

| | | |
|---|---|---|
| EF | EF2 | |

Comparisons between--- EF & EF2

| begin end | Sequence Matching |
|---|---|
| | |
| | |

| | | | | | |
|---|---|---|---|---|---|
| Matching residues | 111 | Identical residues | 78 | Identity | 70.3% |
| Match Rate(1) | 100.0% | r.m.s. of Ca | 0.49 | Mean distance | 0.41 |

### Matrix of fitting residues

| | | | | |
|---|---|---|---|---|
| | SA | | EF | EF2 |
| SA | | | | |
| EF | | | | |
| | 111. | | | |
| EF2 | | | | |
| | 109. | 111. | | |

### Sequence identities of aligned residues

| | | | |
|---|---|---|---|
| | SA | EF | EF2 |
| SA | | | |
| EF | | | |
| | 0.4595 | | |
| EF2 | | | |
| | 0.4128 | 0.7027 | |

### Matrix for fitting scores

| | | | | |
|---|---|---|---|---|
| | SA | EF | | EF2 |
| SA | | | | |
| EF | | | | |
| | 0.71 | | | |
| EF2 | | | | |
| | 0.77 | 0.40 | | |

Refine all the pairwise alignment....
RMS deviation 0.809 Mean distance 0.626 with 3 structures
Search equivalent residues among all the structures ....
Cycle= 1 RMS 0.816 Mean-distance 0.628 with 110 aligned residues
Cycle= 2 RMS 0.816 Mean-distance 0.628 with 110 aligned residues
Total 3 fragment with 110 residues are
equivalent residues in this set of the structures

| Fragment | 1 length | 63 |
|---|---|---|
| | | |

| Fragment | 2 length | 15 |
|---|---|---|
| | | |
| | | |

| Fragment | 3 length | 32 |
|---|---|---|
| | | |

Total 43 residues are identical among all 3 structures
Rate of overall identity 0.391
Statistics for residues which share least identity

| | | |
|---|---|---|
| SA | 52 | 0.473 |
| EF | 85 | 0.773 |
| EF2 | 80 | 0.727 |

Generate the superposed models based on the multiple alignment

| | |
|---|---|
| EF2 | not changed |

File: muri_e_fecalis_patent_z.pdb_maps for EF
File: ef2_z.pdb_maps for EF2

## Claims

1. A crystal of *Helicobacter pylori (H. pylori)* MurI complexed with an inhibitor and/or a substrate.

2. A crystal according to claim 1, wherein the inhibitor is bound to a molecular interface of MurI.

3. A crystal according to claim 2, wherein the molecular interface of MurI is selected from the group consisting of a substrate binding site, an activator binding site, an intermolecular dimer interface, an intradomain interface, an inhibitor binding site, and a combination thereof.

4. A crystal according to claim 3, wherein the substrate binding site of *H, pylori* MurI comprises amino acid residues: Ser8, Cys70, Thr72, Thr116, Thr119, Glu150, Cys181, Thr182, and His183 of SEQ ID NO: 2

5. A crystal according to claim 3, wherein the intermolecular dimer interface of *H. pylori* MurI comprises amino acid residues: Ser34, Ala35, Arg36, Val37, Pro38, Tyr39, Gly40, Thr41, Lys42, Asp43, Pro44, Thr46, Phe50, Lys117, Asn121, Ser143, Leu144, Pro147, Leu148, Glu150, Glu151, Ser152, Ile153, Gly157, Leu158, Thyr161, Cys162, Tyr165, Tyr166, Ser239, Gly240, Asp241, and Trp244 of SEQ ID NO: 2.

6. A crystal according to claim 3, wherein the intradomain interface comprises amino acid residues: Asp7, Ser8, Gly9, Va110, Gly11, Gly12, Phe13, Ser14, Val15, Ser18, Lys21, Ala22, Val37, Pro38, Tyr39, Gly40, Thr41, Lys42, Asp43, Pro44, Ile47, Ala69, Cys70, Asn71, Thr72, Ser74, Ala75, Leu76, Gly91, Val92, Gly211, Asp212, Ala213, Ile214, Val215, Glu216, Tyr217, Leu218, Gln219, Gln220, Lys221, Glu251, Trp252, Leu253, Lys254, and Leu255 of a first domain, and amino acid residues Ile93, Glu94, Pro95, Ser96, Ile97, Leu98, Ala99, Ile100, Arg102, Gln103, Thr116, Lys117, Ala118, Thr119, Ser122, Asn123, Ala124, Tyr125, Ala128, Gln131, Gln132, Ser143, Val146, Pro147, Ile149, Glu150, Glu151, Ser152, Ile178, Leu179, Gly180, Cys181, Thr182, His183, Phe184, Pre185, Leu186, Ile208, His209, Ser210, Gly211, and Asp212 of a second domain of SEQ ID NO: 2.

7. A crystal according to claim 3, wherein the inhibitor binding site comprises amino acid residues Va110, Gly11, Phe13, De149, Glu151, Ser152, Leu186, Trp244, Gln248, and Trp252 of SEQ ID NO: 2.

8. A computer-assisted method of identifying an agent that is an inhibitor of Murl, comprising:
(a) providing a computer modeling application with a set of relative structural coordinates of a crystal of *H. pylori* Murl as shown in any of Figures 5-7, or of a molecular interface thereof;
(b) supplying the computer modeling application with a set of structural coordinates of an agent to be assessed to determine if it binds a molecular interface of MurI;
(c) comparing the two sets of coordinates and;
(d) determining whether the agent is expected to bind a molecular interface of MurI;
wherein if the agent is expected to bind a molecular interface of MurI, an agent that is an inhibitor of Murl activity has been identified.

9. A computer-assisted method for designing an inhibitor of MurI activity, comprising:
(a) supplying to a computer modeling application a set of relative structural coordinates of *H. pylori* MurI as shown in any of Figures 5-7, or a molecular interface thereof;
(b) computationally building an agent represented by a set of structural coordinates; and
(c) determining whether the agent is expected to interfere with MurI, or a molecular interface thereof,
wherein if the agent is expected to interfere with the MurI or a molecular interface thereof, an inhibitor has been designed.

10. A method of identifying a molecule that binds to MurI comprising:
(a) applying a 3-dimensional molecular modeling algorithm to the atomic coordinates of a molecular interface of *H. pylori* MurI as shown in any of Figures 5-7; and
(b) electronically screening the stored spatial coordinates of a set of candidate compounds against the spatial coordinates of the molecular interface of MurI to identify compounds that bind to the molecular interface of MurI.

11. A method for structure-based design of a compound that fits a conserved surface of MurI comprising:
(a) producing a computer-generated representation of a conserved surface of a crystalline form of MurI, wherein the conserved surface of a crystalline form of Murl is represented by the relative structural coordinates of any of Figures 5-7;
(b) producing a computer-generated representation of a library of compounds to be assessed for their ability to fit the conserved surface; and
(c) determining if the compounds from step (b) fit the conserved surface,
wherein the crystalline form of the MurI is suitable for use in X-ray studies.

12. A method of assessing the *in vitro* binding of a MurI inhibitor identified in the computer-assisted method of claims 8 or 9 comprising the steps of :
(a) culturing a test culture comprising a MurI substrate, the inhibitor, and a bacterium having MurI;
(b) culturing the test culture for a period of time such that the bacterium having MurI in the culture expand; and
(d) comparing growth of the test culture to growth of an appropriate control culture,
whereby if growth of bacterium having MurI in the test culture is inhibited, an agent that binds MurI has been identified, wherein the agent that binds MurI is an inhibitor.

13. The method of claim 12, wherein the control culture is cultured simultaneously with the test culture.

14. The method of claim 12, wherein the control culture is cultured prior to the test culture.

15. The method of claim 12, wherein the control culture is cultured after the test culture.

16. The method of any of claims 8-15 and further comprising testing the agent that binds MurI to determine if it inhibits MurI, such method comprising the steps of:
(a) preparing a test culture of a substrate, a test inhibitor, and an atypical bacterium;
(b) preparing a control culture of a substrate and an atypical bacterium;
(c) culturing the atypical bacterial cultures for a period of time such that the atypical bacterium in the control culture expand; and
(d) comparing the growth of the control atypical bacterium to the growth of the test culture atypical bacterium,
whereby if atypical bacterial growth in the test culture is inhibited, an agent that binds MurI has been identified, wherein the agent that binds MurI is an inhibitor.

17. A crystal as claimed in claim 1 wherein the complex comprises the orthorombic space group P2₁2₁2₁ and has cell dimensions of a = 62.14 Å, b = 81.07 Å and c = 113.82 Å, wherein α = 90°, β = 90°, and γ = 90°.

18. A crystal as claimed in claim 1 wherein the complex comprises the monoclinic space group P2₁ and has cell dimensions of a = 59.20 Å, b = 82.40 Å and c = 106.50 Å, wherein α = 90°, β = 92.15°, and γ = 90°.

19. A crystal as claimed in claim 1 wherein the complex comprises the monoclinic space group P2₁ and has cell dimensions of a = 52.28 Å, b = 78.96 Å and c = 59.14 Å, wherein α = 90°, β = 92.64°, and γ = 90°.

20. A crystal as claimed in claim 1 wherein the complex comprises the monoclinic space group P2₁ and has cell dimensions of a =52.02 Å, b = 80.66 Å and c = 59.18 Å, wherein α = 90°, β = 92.65°, and γ = 90°.

21. A crystal as claimed in claim 1 wherein the complex comprises the monoclinic space group P2₁ and has cell dimensions of a = 52.61 Å, b = 78.40 Å, and c = 59.43 Å, wherein α = 90°, β = 92.33°, γ = 90°.

22. A crystal as claimed in claim 1 wherein the complex comprises the monoclinic space group P2₁ and has cell dimensions, of a = 62.9 Å, b = 81.8 Å, and c = 113.6 Å, wherein α = 90°, β = 90°, γ = 90°.

23. A crystal as claimed in claim 1 wherein *Helicobacter pylori* glutamate racemase (MurI) complexed with an inhibitor and substrate comprises the orthorhombic space group P2₁2₁2₁ and has cell dimensions of a = 61.41 Å, b = 76.31 Å and c = 108.92 Å, wherein α = 90°, β = 90°, and γ = 90°.

24. A crystal as claimed in claim 1 wherein *Helicobacter pylori* glutamate racemase (MurI) complexed with an inhibitor and substrate comprises the orthorhombic space group P2₁2₁2₁ and has cell dimensions a=60.7 Å, b=77.5 Å, c= 56.6 Å, wherein α=90°, β=90° and γ=90°.

25. A crystal as claimed in claim 1 wherein *Helicobacter pylori* glutamate racemase (MurI) complexed with an inhibitor and substrate comprises monoclinic space group P2₁, and has cell dimensions a =57.1 Å, b =78.0 Å, c =58.55 Å, wherein α = 90°, β = 97.91°, and γ = 90°.

26. A crystal as claimed in claim 1 wherein *Helicobacter pylori* glutamate racemase (MurI) complexed with a substrate is represented by the structure coordinates in Figure 5.

27. A crystal as claimed in claim 1 wherein *Helicobacter pylori* glutamate racemase (MurI) complexed with a substrate and inhibitor is represented by the structure coordinates in Figure 6.

## Patentansprüche

1. Kristall von *Helicobacter pylori (H. pylori)* MurI, das mit einem Hemmstoff und/oder einem Substrat komplexiert ist.

2. Kristall nach Anspruch 1, wobei der Hemmstoff an eine molekulare Grenzfläche von MurI gebunden ist.

3. Kristall nach Anspruch 2, wobei die molekulare Grenzfläche von MurI aus der Gruppe bestehend aus einer Substratbindungsstelle, einer Aktivatorbindungsstelle, einer intramolekularen Dimer-Grenzfläche, einer intradomänen Grenzfläche, einer Hemmstoffbindungsstelle und einer Kombination davon ausgewählt ist.

4. Kristall nach Anspruch 3, wobei die Substratbindungsstelle von *H. pylori* MurI die Aminosäurereste Ser8, Cys70, Thr72, thr116, Thr119, Glu150, Cys181, Thr182 und His183 von SEQ ID NO: 2 umfaßt.

5. Kristall nach Anspruch 3, wobei die intermolekulare Dimer-Grenzfläche *H. pylori* MurI die Aminosäurereste Ser34, Ala35, Arg36, Val37, Pro38, Tyr39, Gly40, Thr41, Lys42, Asp43, Pro44, Thr46, Phe50, Lys117, Asn121, Ser143, Leu144, Pro147, Leu148, Glu150, Glu151, Ser152, Ile153, Gly157, Leu158, Thyr161, Cys162, Tyr165, Tyr166, Ser239, Gly240, Asp241 und Trp244 von SEQ ID NO: 2 umfaßt.

6. Kristall nach Anspruch 3, wobei die Intradomänengrenzfläche die Aminosäurereste Asp7, Ser8, Gly9, Val10, Gly12, Phe13, Ser14, Val15, Ser18, Lys21, Ala22, Val37, Pro38, Tyr39, Gly40, Thr41, Lys42, Asp43, Pro44, Ile47, Ala69, Cys70, Asn71, Thr72, Ser74, Ala75, Leu76, Gly91, Val92, Gly211, Asp212, Ala213, Ile214,Val215, Glu216, Tyr217, Leu218, Gln219, Gln220, Lys221, Glu251, Trp252, Leu253, Lys254 und Leu255 einer ersten Domäne und die Aminosäurereste Ile93, Glu94, Pro95, Ser96, Ile97, Leu98, Ala99, Ile100, Arg102, Gln103, Thr116, Lys117, Ala118, Thr119, Ser122, Asn123, Ala124, Tyr125, Ala128, Gln131, Gln132, Ser143, Val146, Pro147, Ile149, Glu150, Glu151, Ser152, Ile178, Leu179, Gly180, Cys181, Thr182, His183, Phe184, Pro185, Leu186, Ile208, His209, Ser210, Gly211 und Asp212 einer zweiten Domäne von SEQ ID NO: 2 umfaßt.

7. Kristall nach Anspruch 3, wobei die Hemmstoffbindungsstelle die Aminosäurereste Val10, Gly11, Phe13, Ile149, Glu151, Ser152, Leu186, Trp244, Gln248 und Trp252 von SEQ ID NO: 2 umfaßt.

8. Computergestütztes Verfahren zum Identifizieren eines Agens, bei dem es sich um einen MurI-Hemmstoff handelt, das folgendes umfaßt:
(a) Bereitstellen einer Computer-Modeling-Anwendung mit einem Satz von relativen Strukturkoordinaten eines Kristalls von H. pylori MurI nach einer der Figuren 5-7 oder von einer molekularen Grenzfläche davon,
(b) Beliefern der Computer-Modeling-Anwendung mit einem Satz von Strukturkoordinaten eines zu beurteilenden Agens, um zu bestimmen, ob es an eine molekulare Grenzfläche von MurI bindet,
(c) Vergleichen der beiden Koordinatensätze und
(d) Bestimmen, ob zu erwarten ist, daß das Agens an eine molekulare Grenzfläche von MurI bindet,
wobei, wenn zu erwarten ist, daß das Agens an eine molekulare Grenzfläche von MurI bindet, ein Agens identifiziert worden ist, bei dem es sich um einen Hemmstoff der MurI-Aktivität handelt.

9. Computergestütztes Verfahren zum Entwickeln eines Hemmstoffs der MurI-Aktivität, das folgendes umfaßt:
(a) Beliefern einer Computer-Modeling-Anwendung mit einem Satz von relativen Strukturkoordinaten von *H. pylori* MurI nach einer der Figuren 5 bis 7 oder von einer molekularen Grenzfläche davon,
(b) rechnerisches Bilden eines Agens, das einem Satz von Strukturkoordinaten entspricht und
(c) Bestimmen, ob zu erwarten ist, ob das Agens auf MurI oder eine molekulare Grenzfläche davon störend wirkt,
wobei, wenn von dem Agens erwartet wird, daß es auf MurI oder eine molekulare Grenzfläche davon störend wirkt, ein Hemmstoff entwickelt worden ist.

10. Verfahren zum Identifizieren eines Moleküls, das an MurI bindet, wobei das Verfahren folgendes umfaßt:
(a) Anwenden eines 3-dimensionalen Molecular-Modeling-Algorithmus gemäß der Atomkoordinaten einer molekularen Grenzfläche von *H. pylori* MurI gemäß einer der Figuren 5-7 und
(b) elektronisches Durchmustern der gespeicherten Raumkoordinaten eines Satzes von Kandidatenverbindungen im Vergleich zu den Raumkoordinaten der molekularen Grenzfläche von MurI, um Verbindungen zu identifizieren, die an die molekulare Grenzfläche von MurI binden.

11. Verfahren für das strukturbegründete Design einer Verbindung, die mit einer konservierten Oberfläche von MurI zusammenpaßt, wobei dieses Verfahren folgendes umfaßt:
(a) Erstellen einer computergenerierten Darstellung einer konservierten Oberfläche einer kristallinen Form von MurI, wobei die konservierte Oberfläche einer kristallinien Form von MurI durch die relativen Strukturkoordinaten nach einer der Figuren 5-7 repräsentiert wird,
(b) Erstellen einer computergenerierten Darstellung einer Bibliothek von Verbindungen, die auf ihre Fähigkeit, mit der konservierten Oberfläche zusammenzupassen, zu beurteilen sind und
(c) Bestimmen, ob die Verbindungen aus Schritt (b) mit der konservierten Oberfläche zusammenpassen,
wobei sich die kristalline Form von MurI für Röntgenstudien eignet.

12. Verfahren zur Beurteilung der in-vitro-Bindung eines in dem computergestützten Verfahren der Ansprüche 8 oder 9 identifizierten MurI-Hemmstoffs, wobei das Verfahren die folgenden Schritte umfaßt:
(a) Züchten einer Testkultur, die ein MurI-Substrat, den Hemmstoff und ein Bakterium mit MurI umfaßt,
(b) Züchten der Testkultur über solch einen Zeitraum, daß sich das Bakterium mit MurI in der Kultur ausbreitet und
(d) Vergleichen des Wachstums der Testkultur mit dem Wachstum einer entsprechenden Kontrollkultur,
wobei, wenn das Wachstum des Bakteriums mit MurI in der Testkultur gehemmt ist, ein Agens, das an MurI bindet, identifiziert worden ist, wobei es sich bei dem Agens, das an MurI bindet, um einen Hemmstoff handelt.

13. Verfahren nach Anspruch 12, wobei die Kontrollkultur gleichzeitig mit der Testkultur gezüchtet wird.

14. Verfahren nach Anspruch 12, wobei die Kontrollkultur vor der Testkultur gezüchtet wird.

15. Verfahren nach Anspruch 12, wobei die Kontrollkultur nach der Testkultur gezüchtet wird.

16. Verfahren nach einem der Ansprüche 8-15, das weiterhin umfaßt, daß das Agens, das an MurI bindet, getestet wird, um zu bestimmen, ob es MurI hemmt, wobei solch ein Verfahren die folgenden Schritte umfaßt:
(a) Herstellen einer Testkultur eines Substrats, eines Testhemmstoffs und eines untypischen Bakteriums,
(b) Herstellen einer Kontrollkultur eines Substrats und eines untypischen Bakteriums,
(c) Züchten der untypischen Bakterienkulturen über solch einen Zeitraum, daß sich das untypische Bakterium in der Kontrollkultur ausbreitet und
(d) Vergleichen des Wachstums des untypischen Kontrollbakteriums mit dem Wachstum des untypischen Testkulturbakteriums,
wobei, wenn das Wachstum des untypischen Bakteriums in der Testkultur gehemmt ist, ein Agens, das an MurI bindet, identifiziert worden ist, wobei es sich bei dem Agens, das an MurI bindet, um einen Hemmstoff handelt.

17. Kristall nach Anspruch 1, wobei der Komplex die orthorhombische Raumgruppe P2₁2₁2₁ aufweist und die Zelldimensionen a = 62,14 Å, b = 81,07 Å und c = 113,82 Å aufweist, wobei α = 90°, β = 90° und γ = 90°.

18. Kristall nach Anspruch 1, wobei der Komplex die monokline Raumgruppe P2₁ aufweist und die Zelldimensionen a = 59,20 Å, b = 82,40 Å und c = 106,50 Å aufweist, wobei α = 90°, β = 92,15° und γ = 90°.

19. Kristall nach Anspruch 1, wobei der Komplex die monokline Raumgruppe P2₁ aufweist und die Zelldimensionen a = 52,28 Å, b = 78,96 Å und c = 59,14 Å aufweist, wobei α = 90°, β = 92,64° und γ = 90°.

20. Kristall nach Anspruch 1, wobei der Komplex die monokline Raumgruppe P2₁ aufweist und die Zelldimensionen a = 52,02 Å, b = 80,66 Å und c = 59,18 Å aufweist, wobei α = 90°, β = 92,65° und γ = 90°.

21. Kristall nach Anspruch 1, wobei der Komplex die monokline Raumgruppe P2₁ aufweist und die Zelldimensionen a = 52,61 Å, b = 78,40 Å und c = 59,43 Å aufweist, wobei α = 90°, β = 92,33° und γ = 90°.

22. Kristall nach Anspruch 1, wobei der Komplex die monokline Raumgruppe P2₁ aufweist und die Zelldimensionen a = 62,9 Å, b = 81,8 Å und c = 113,6 Å aufweist, wobei α = 90°, β = 90° und γ = 90°.

23. Kristall nach Anspruch 1, wobei die mit einem Hemmstoff und Substrat komplexierte Glutamatracemase (MurI) aus *Helicobacter pylori* die orthorhombische Raumgruppe P2₁2₁2₁ aufweist und die Zelldimensionen a = 61,41 Å, b = 76,31 Å und c = 108,92 Å aufweist, wobei α = 90°, β = 90° und γ = 90°.

24. Kristall nach Anspruch 1, wobei die mit einem Hemmstoff und Substrat komplexierte Glutamatracemase (MurI) aus *Helicobacter pylori* die orthorhombische Raumgruppe P2₁2₁2₁ aufweist und die Zelldimensionen a = 60,7 Å, b = 77,5 Å und c = 56,6 Å aufweist, wobei α = 90°, β = 90° und γ = 90°.

25. Kristall nach Anspruch 1, wobei die mit einem Hemmstoff und Substrat komplexierte Glutamatracemase (MurI) aus *Helicobacter pylori* die monokline Raumgruppe P2₁ aufweist und die Zelldimensionen a = 57,1 Å, b = 78,0 Å und c = 58,55 Å aufweist, wobei α = 90°, β = 97,91° und γ = 90°.

26. Kristall nach Anspruch 1, wobei die mit einem Substrat komplexierte Glutamatracemase (MurI) aus *Helicobacter pylori* den Strukturkoordinaten in Figur 5 entspricht.

27. Kristall nach Anspruch 1, wobei die mit einem Substrat und einem Hemmstoff komplexierte Glutamatracemase (MurI) aus *Helicobacter pylori* den Strukturkoordinaten in Figur 6 entspricht.

## Revendications

1. Cristal de MurI d'*Helicobacter pylori (H. pylori*) complexé à un inhibiteur et/ou à un substrat.

2. Cristal selon la revendication 1, selon lequel l'inhibiteur est lié à une interface moléculaire de MurI.

3. Cristal selon la revendication 2, selon lequel l'interface moléculaire de MurI est choisie dans le groupe constitué d'un site de liaison d'un substrat, d'un site de liaison d'un activateur, d'une interface dimérique intermoléculaire, d'une interface intra-domaine, d'un site de liaison d'un inhibiteur et de combinaisons de ceux-ci.

4. Cristal selon la revendication 3, selon lequel le site de liaison du substrat de MurI d'*H*. *pylori* comprend les résidus d'acides aminés : Ser8, Cys70, Thr72, thrl16, Thrl19, Glu150, Cys181, Thr182 et His183 de SEQ ID NO : 2.

5. Cristal selon la revendication 3, selon lequel l'interface dimérique intermoléculaire de MurI d'*H. pylori* comprend les résidus d'acides aminés : Ser34, Ala35, Arg36, Val37, Pro38, Tyr39, Gly40, Thr41, Lys42, Asp43, Pro44, Thr46, Phe50, Lys117, Asn121, Ser143, Leu144, Pro147, Leu148, Glu150, Glu151, Ser152, Ile153, Gly157, Leu158, Thyr161, Cys162, Tyr165, Tyr166, Ser239, Gly240, Asp241 et Trp244 de SEQ ID NO : 2.

6. Cristal selon la revendication 3, selon lequel l'interface intra-domaine comprend les résidus d'acides aminés : Asp7, Ser8, Gly9, Val10, Gly11, Gly12, Phe13, Ser14, Val15, Ser18, Lys21, Ala22, Val37, Pro38, Tyr39, Gly40, Thr41, Lys42, Asp43, Pro44, Ile47, Ala69, Cys70, Asn71, Thr72, Ser74, Ala75, Leu76, Gly91, Val92, Gly211, Asp212, Ala213, Ile214, Val215, Glu216, Tyr217, Leu218, Gln219, Gln220, Lys221, Glu251, Trp252, Leu253, Lys254 et Leu255 d'un premier domaine, et les résidus d'acides aminés Ile93, Glu94, Pro95, Ser96, Ile97, Leu98, Ala99, Ile100, Arg102, Gln103, Thr116, Lys117, Ala118, Thr119, Ser122, Asn123, Ala124, Tyr125, Ala128, Gln131, Gln132, Ser143, Val146, Pro147, Ile149, Glu150, Glu151, Ser152, Ile178, Leu179, Gly180, Cys181, Thr182, His183, Phe184, Pro185, Leu186, Ile208, His209, Ser210, Gly211 et Asp212 d'un deuxième domaine de SEQ ID NO : 2.

7. Cristal selon la revendication 3, selon lequel le site de liaison d'un inhibiteur comprend les résidus d'acides aminés : Val10, Gly11, Phe13, Ile149, Glu15l, Ser152, Leu186, Trp244, Gln248 et Trp252 de SEQ ID NO : 2.

8. Méthode assistée par ordinateur permettant l'identification d'un agent qui est un inhibiteur de MurI, comprenant les étapes consistant à :
(a) fournir à une application de modélisation par ordinateur un ensemble de coordonnées structurales relatives d'un cristal de MurI d'*H*. *pylori*, comme présenté dans l'une quelconque des figures 5 à 7, ou d'une interface moléculaire de celui-ci ;
(b) fournir à l'application de modélisation par ordinateur un ensemble de coordonnées structurales d'un agent à évaluer pour déterminer s'il se lie à une interface moléculaire de MurI ;
(c) comparer les deux ensembles de coordonnées et ;
(d) déterminer si l'agent est susceptible se lier à une interface moléculaire de MurI ;
méthode selon laquelle, si l'agent est susceptible se lier à une interface moléculaire de MurI, un agent qui est un inhibiteur de l'activité de MurI a été identifié.

9. Méthode assistée par ordinateur de conception d'un inhibiteur de l'activité de MurI, comprenant les étapes consistant à :
(a) fournir à une application de modélisation par ordinateur un ensemble de coordonnées structurales relatives de MurI d'*H*. *pylori*, comme présenté dans l'une quelconque des figures 5 à 7, ou d'une interface moléculaire de celui-ci ;
(b) construire informatiquement un agent représenté par un ensemble de coordonnées structurales ; et
(c) déterminer si l'agent est susceptible d'interférer avec MurI, ou avec une interface moléculaire de celui-ci ;
méthode selon laquelle, si l'agent est susceptible d'interférer avec MurI ou avec une interface moléculaire de celui-ci, un inhibiteur a été conçu.

10. Méthode d'identification d'une molécule qui se lie à MurI comprenant les étapes consistant à :
(a) appliquer un algorithme de modélisation moléculaire tridimensionnelle aux coordonnées atomiques d'une interface moléculaire de MurI d'*H*. *pylori*, comme présenté dans l'une quelconque des figures 5 à 7 ; et
(b) cribler électroniquement les coordonnées spatiales stockées d'un ensemble de composés candidats contre les coordonnées spatiales de l'interface moléculaire de MurI pour identifier des composés qui se lient à l'interface moléculaire de MurI.

11. Méthode de conception basée sur la structure d'un composé qui s'adapte à une surface conservée de MurI comprenant les étapes consistant à :
(a) produire une représentation générée par ordinateur d'une surface conservée d'une forme cristalline de MurI, la surface conservée d'une forme cristalline de MurI étant représentée par les coordonnées structurales relatives de l'une quelconque des figures 5 à 7 ;
(b) produire une représentation générée par ordinateur d'une banque de composés à évaluer quant à leur aptitude à s'adapter à la surface conservée ; et
(c) déterminer si les composés de l'étape (b) s'adaptent à la surface conservée,
la forme cristalline de MurI étant appropriée à une utilisation dans des études par rayons X.

12. Méthode d'évaluation de la liaison *in vitro* d'un inhibiteur de MurI identifié par la méthode assistée par ordinateur des revendications 8 ou 9, comprenant les étapes consistant à :
(a) mettre en culture une culture à l'essai comprenant un substrat MurI, l'inhibiteur et une bactérie ayant MurI ;
(b) mettre en culture la culture à l'essai pendant une durée telle que la bactérie ayant MurI dans la culture se développe ; et
(c) comparer la croissance de la culture à l'essai à la croissance d'une culture témoin appropriée,
méthode selon laquelle, si la croissance de la bactérie ayant MurI dans la culture à l'essai est inhibée, un agent qui se lie à MurI a été identifié, l'agent qui se lie à MurI étant un inhibiteur.

13. Méthode selon la revendication 12, selon laquelle la culture témoin est mise en culture simultanément avec la culture à l'essai.

14. Méthode selon la revendication 12, selon laquelle la culture témoin est mise en culture avant la culture à l'essai.

15. Méthode selon la revendication 12, selon laquelle la culture témoin est mise en culture après la culture à l'essai.

16. Méthode selon l'une quelconque des revendications 8 à 15 et comprenant en outre l'étape consistant à tester l'agent qui se lie à MurI pour déterminer s'il inhibe MurI, une telle méthode comprenant les étapes consistant à :
(a) préparer une culture à l'essai d'un substrat, d'un inhibiteur à l'essai et d'une bactérie atypique ;
(b) préparer une culture témoin d'un substrat et d'une bactérie atypique ;
(c) mettre en culture les cultures de bactéries atypiques pendant une durée telle que la bactérie atypique dans la culture témoin se développe ; et
(d) comparer la croissance de la bactérie atypique témoin à la croissance de la bactérie atypique de la culture à l'essai, méthode selon laquelle, si la croissance de la bactérie atypique dans la culture à l'essai est inhibée, un agent qui se lie à MurI a été identifié, l'agent qui se lie à MurI étant un inhibiteur.

17. Cristal selon la revendication 1, selon lequel le complexe comprend le groupe d'espace orthorhombique P2₁2₁2₁ et présente des dimensions de maille de a = 62,14 Å, b = 81,07 Å et c = 113,82 Å, où α = 90°, β = 90° et γ = 90°.

18. Cristal selon la revendication 1, selon lequel le complexe comprend le groupe d'espace monoclinique P2₁ et présente des dimensions de maille de a = 59,20 Å, b = 82,40 Å et c = 106,50 Å, où α = 90°, β = 92,15° et γ = 90°.

19. Cristal selon la revendication 1, selon lequel le complexe comprend le groupe d'espace monoclinique P2₁ et présente des dimensions de maille de a = 52,28 Å, b = 78,96 Å et c = 59,14 Å, où α = 90°, β = 92,64° et γ = 90°.

20. Cristal selon la revendication 1, selon lequel le complexe comprend le groupe d'espace monoclinique P2₁ et présente des dimensions de maille de a = 52,02 Å, b = 80,66 Å et c = 59,18 Å, où α = 90°, β = 92,65° et γ = 90°.

21. Cristal selon la revendication 1, selon lequel le complexe comprend le groupe d'espace monoclinique P2₁ et présente des dimensions de maille de a = 52,61 Å, b = 78,40 Å et c = 59,43 Å, où α = 90°, β = 92,33° et γ = 90°.

22. Cristal selon la revendication 1, selon lequel le complexe comprend le groupe d'espace monoclinique P2₁ et présente des dimensions de maille de a = 62,9 Å, b = 81,8 Å et c = 113,6 Å, où α = 90°, β = 90° et γ = 90°.

23. Cristal selon la revendication 1, selon lequel la glutamate racémase (MurI) d'*Helicobacter pylori* complexée à un inhibiteur et à un substrat comprend le groupe d'espace orthorhombique P2₁2₁2₁ et présente des dimensions de maille de a = 61,41 Å, b = 76,31 Å et c = 108,92 Å, où α = 90°, β = 90° et γ = 90°.

24. Cristal selon la revendication 1, selon lequel la glutamate racémase (MurI) d'*Helicobacter pylori* complexée à un inhibiteur et à un substrat comprend le groupe d'espace orthorhombique P2₁2₁2₁, et présente des dimensions de maille de a = 60, 7 Å, b = 77, 5 Å et c = 56, 6 Å, où α = 90°, β = 90° et γ = 90°.

25. Cristal selon la revendication 1, selon lequel la glutamate racémase (MurI) d'*Helicobacter pylori* complexée à un inhibiteur et à un substrat comprend le groupe d'espace monoclinique P2₁ et présente des dimensions de maille de a = 57,1 Å, b = 78,0 Å et c = 58,55 Å, où α = 90°, β = 97,91° et γ = 90°.

26. Cristal selon la revendication 1, selon lequel la glutamate racémase (MurI) d'*Helicobacter pylori* complexée à un substrat est représentée par les coordonnées de structure de la figure 5.

27. Cristal selon la revendication 1, selon lequel la glutamate racémase (MurI) d'*Helicobacter pylori* complexée à un substrat et à un inhibiteur est représentée par les coordonnées de structure de la figure 6.
